# EUROPEAN PATENT APPLICATION

(11) **EP 4 159 739 A1**
(43) Date of publication of application: **05.04.2023**
(21) Application number: 21813120.9
(22) Date of filing: 27.05.2021
(51) Int. Cl.: C07D 498/14, A61K 31/553, A61P 35/00

(54) **FUSED TRICYCLIC COMPOUND, PHARMACEUTICAL COMPOSITION THEREOF, AND USE THEREOF**

(30) Priority: 27.05.2020 CN 202010461834; 11.09.2020 CN 202010956281; 30.12.2020 CN 202011627054; 11.05.2021 CN 202110513435
(71) Applicant: Genfleet Therapeutics (Shanghai) Inc., Shanghai 201203 (CN); Zhejiang Genfleet Therapeutics Co., Ltd., Shaoxing Binhai New City, Zhejiang 312000 (CN)
(72) Inventor: ZHOU, Fusheng, Shanghai 201203 (CN); JIANG, Tao, Shanghai 201203 (CN); LIU, Yingtao, Shanghai 201203 (CN); ZHAO, Jichen, Shanghai 201203 (CN); HE, Wan, Shanghai 201203 (CN); LIU, Zhubo, Shanghai 201203 (CN); PENG, Ling, Shanghai 201203 (CN); CAI, Lijian, Shanghai 201203 (CN); ZHANG, Leitao, Shanghai 201203 (CN); LIN, Chonglan, Shanghai 201203 (CN); YANG, Huabin, Shanghai 201203 (CN); ZHANG, Tao, Shanghai 201203 (CN); LAN, Jiong, Shanghai 201203 (CN)
(74) Representative: Mewburn Ellis LLP
(86) International application number: PCT/CN2021/096424
(87) International publication number: WO 2021/239058

(57) **Abstract**

Provided are a fused tricyclic compound having the structure shown in formula (I), a pharmaceutical composition thereof, and a use thereof. The fused tricyclic compound serves as a selective inhibitor of KRAS mutation, and has high activity, good selectivity, and reduced toxic side effects.

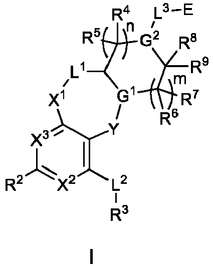

## Description

The present application claims the priority of Chinese patent application 202010461834.8 filed on May 27, 2020, the priority of Chinese patent application 202010956281.3 filed on September 11, 2020, the priority of Chinese patent application 202011627054.2 filed on December 30, 2020 and the priority of Chinese patent application 202110513435.6 filed on May 11, 2021. The contents of the above Chinese patent applications are incorporated herein by reference in their entireties.

### TECHNICAL FIELD

The present disclosure relates to a tricyclic fused ring compound, a preparation method therefor and a pharmaceutical use.

### BACKGROUND

Lung cancer is the cancer with the highest incidence in the world. The incidence of lung cancer in China ranks first among all cancers, and it is also the cancer with the highest incidence and mortality in China. According to the data released by the American Cancer Society in 2016, about 1.8 million people suffer from lung cancer in one year, of which nearly 80% are non-small cell lung cancer (NSCLC).

RAS is a group of closely related monomeric globular proteins (molecular weight of 21 kDa) with 188-189 amino acids and bound to guanosine diphosphate GDP or guanosine triphosphate GTP. Members of the RAS subfamily include HRAS, KRAS and NRAS. RAS acts as a molecular switch, and when RAS contains bound GDP, it is in a dormant or off position and is "inactive". When cells are exposed to certain growth-promoting stimuli, RAS is induced to convert its bound GDP to GTP, and when binding to GTP, RAS is "turned on" and is able to interact with other downstream target proteins and activate these proteins. The inherent ability of the RAS protein itself to hydrolyze GTP back to GDP (thus switching itself to the off state) is extremely low. The exogenous protein GTPase activating protein (GAP) is required to restore it to the off state, and the interaction of GAP with RAS greatly accelerates the conversion of GTP into GDP.

Any mutation in RAS will affect the interaction of RAS with GAP and the ability of GTP to be converted into GDP, and this mutation will lead to prolonged activation of the protein, thereby prolonging cell signal transduction, which in turn leads to continued growth and division of cells. Since this signal transduction causes cells to grow and divide, overactive RAS signal transduction can ultimately lead to cancer.

In lung cancer, about 32% of lung cancers have confirmed the mutation of RAS gene, and mutations in any of the three main subtypes of RAS (HRAS, NRAS or KRAS) gene can lead to the occurrence of human tumors. It has been reported that the KRAS gene has the highest mutation frequency in the RAS gene, and KRAS mutations are detected in 25-30% of tumors. In contrast, the incidence of carcinogenic mutations in NRAS and HRAS family members is much lower (8% and 3% respectively). The most common KRAS mutations are found in residues G12 and G13 and residue Q61 in the P-loop. G12C mutation is the frequent mutation of KRAS gene (glycine-12 mutation to cysteine). This mutation has been found in about 13% of cancers, about 43% of lung cancers, and almost 100% of MYH-associated polyposis (familial colon cancer syndrome).

Therefore, it is a good direction to develop inhibitors that selectively inhibit KRAS mutation. In order to improve the inhibitory activity of KRAS mutation and reduce the inhibitory activity of wild-type KRAS, it is of great significance to develop new selective inhibitors of RAS mutant with higher activity, better selectivity and lower toxicity.

### CONTENT OF THE PRESENT INVENTION

The present disclosure provides a tricyclic fused ring compound, which acts as a selective inhibitor of KRAS mutation and has the advantages of high activity, good selectivity and low toxicity and side effects, etc.

The first aspect of the present disclosure provides a compound of formula (I), a pharmaceutically acceptable salt thereof, a stereoisomer thereof, a solvate thereof or a prodrug thereof: 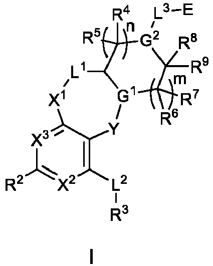 in the formula,
X¹ is O, S, NR^{x1} or CR^{x2}R^{x3}; wherein, R^{x1} is hydrogen or C₁₋₆ alkyl; R^{x2}, R^{x3} are each independently hydrogen, deuterium, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy;
X² is N or CR^{x4}; wherein, R^{x4} is hydrogen, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy;
X³ is N or CR¹; wherein, R¹ is hydrogen, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, C₂₋₆ alkenyl, C₂₋₆ alkynyl, -C₁₋₄ alkyl-hydroxyl, -C₁₋₄ alkyl-cyano, -C₁₋₄ alkyl-C₁₋₆ alkoxy, -C₁₋₄ alkyl-C₃₋₆ cycloalkoxy, -C₁₋₄ alkyl-halo C₁₋₆ alkyl, -C₁₋₄ alkyl-halo C₁₋₆ alkoxy or -C₁₋₄ alkyl-NR^{a}R^{b};
L¹ is a bond, (CR^{L1}R^{L2})ₜ, C(=O), C(=O)C(R^{L1}R^{L2}) or C(R^{L1}R^{L2})C(=O); wherein, R^{L1}, R^{L2} are each independently hydrogen, deuterium, halogen or C₁₋₆ alkyl; t is 1 or 2;
L³ is a bond, NR^{L6} or C₁₋₆ alkyl; wherein, R^{L6} is hydrogen, cyano, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R² is C₆₋₁₄ aryl, 5- or 6-membered monocyclic heteroaryl or 8- to 10-membered bicyclic heteroaryl; wherein, the 5- or 6-membered monocyclic heteroaryl has 1, 2 or 3 heteroatoms selected from N, O and S as ring atoms; the 8- to 10-membered bicyclic heteroaryl has 1, 2, 3, 4 or 5 heteroatoms selected from N, O and S as ring atoms; and the C₆₋₁₄ aryl, the 5- or 6-membered monocyclic heteroaryl and/or the 8- to 10-membered bicyclic heteroaryl are each independently unsubstituted or substituted by 1, 2, 3 or 4 groups independently selected from Rₛ₁;
   or
R² is a structure shown in formula (B): 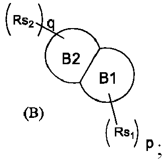
wherein, ring B1 is benzene ring or 5- or 6-membered monocyclic heteroaryl ring; ring B2 is a fused 5- or 6-membered monocyclic heterocycloalkyl ring or fused 5- or 6-membered monocyclic cycloalkyl ring; wherein, the 5- or 6-membered monocyclic heteroaryl ring or the fused 5- or 6-membered monocyclic heterocycloalkyl ring has 1, 2 or 3 heteroatoms selected from N, O and S as ring atoms;
(Rₛ₁)ₚ means that hydrogen on ring B1 is substituted by p Rₛ₁, p is 0, 1, 2 or 3, each Rₛ₁ is the same or different;
(Rₛ₂)_{q} means that hydrogen on ring B2 is substituted by q Rₛ₂, q is 0, 1, 2 or 3, each Rₛ₂ is the same or different;
Rₛ₁, Rₛ₂ are each independently halogen, cyano, nitro, hydroxyl, -C₁₋₆ alkyl, -C₁₋₆ alkoxy, -halo C₁₋₆ alkyl, -halo C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; wherein, the 3- to 6-membered heterocyclyl has 1, 2 or 3 heteroatoms selected from N, O and S as ring atoms;
G¹, G² are each independently N or CH;
   1) when G¹ is N,
      Y is -C(=O)-, -C(=S)-, -S(=O)z- or -S(=O)-;
      L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
      R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl has 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl;
   2) when G¹ is N,
      Y is -C(R^{L7}R^{L8})-; wherein, R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
      L² is O, S or NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
      R³ is C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl has 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl has 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl;
   3) when G¹ is N,
      Y is -C(R^{L7}R^{L8})-; wherein, R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
      L² is NH(C=O), S(=O)₂ or C(=O);
      R³ is C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7-to 11-membered spiroheterocyclyl, 5- to 11-membered bridged cycloalkyl, 5- to 11-membered bridged heterocyclyl, -L⁴-C₆₋₁₄ aryl, -L⁴-5-or 6-membered monocyclic heteroaryl, -L⁴-3-to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl has 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; and when R³ is NR^{e}R^{f}, R^{e}, R^{f} are not simultaneously H;
   4) when G¹ is N,
      Y is -C(R^{L7}R^{L8})-; wherein, R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
      L² is a bond or (CR^{L3}R^{L4})ₘ₁; wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy or -C₁₋₄ alkyl-NR^{a}R^{b}; or R^{L3}, R^{L4} combining with the carbon atoms to which they are attached form a C₃₋₆ monocyclic cycloalkyl;
      R³ is NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5-or 6-membered monocyclic heteroaryl, -L⁴-3-to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl;
         or
   5) when G¹ is CH,
      Y is NR^{L9}, O or S; wherein, R^{L9} is hydrogen, cyano, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
      L² is a bond, O, S, CR^{L3}R^{L4} , NR^{L5}, NH(C=O), S(=O)₂ or C(=O); wherein, R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy or -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
      R³ is C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7-to 11-membered spiroheterocyclyl, 5- to 11-membered bridged cycloalkyl, 5- to 11-membered bridged heterocyclyl, -L⁴-C₆₋₁₄ aryl, -L⁴-5-or 6-membered monocyclic heteroaryl, -L⁴-3-to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl has 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl;
      the substituents of each group S1 are each independently oxo (=O), halogen, cyano, nitro, hydroxyl, carboxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, C₆₋₁₄ aryl, 5- or 6-membered monocyclic heteroaryl, C₃₋₆ monocyclic cycloalkoxy, -O-3- to 6-membered monocyclic heterocyclyl, -O-5- to 11-membered fused cycloalkyl, -O-5- to 11-membered fused heterocyclyl, -O-6- to 11-membered spirocycloalkyl, -O-7- to 11-membered spiroheterocyclyl, -O-5- to 11-membered bridged cycloalkyl, -O-5- to 11-membered bridged heterocyclyl, -O-C₆₋₁₄ aryl, -O-5- or 6-membered monocyclic heteroaryl, NR^{c}R^{d}, C(O)C₁₋₆ alkyl, C(O)C₂₋₆ alkenyl, C(O)C₃₋₆ monocyclic cycloalkyl, C(O)NR^{c}R^{d}, -SO₂C₁₋₃alkyl, -SOzhalo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, - C₁₋₄ alkyl-deuterated C₁₋₆ alkoxy, -C₁₋₄ alkyl-3- to 6-membered monocyclic heterocyclyl, -C₁₋₄ alkyl-NR^{c}R^{d}, -O-C₁₋₄alkyl-NR^{c}R^{d},-C₁₋₄ alkyl-C(O)NR^{c}R^{d},-C₁₋₄ alkyl-SO₂C₁₋₃ alkyl, carboxyl, -C₁₋₄ alkyl-carboxyl, C₂₋₆ alkenyl or C₂₋₆ alkynyl; the 3- to 6-membered monocyclic heterocyclyl or 5- or 6-membered monocyclic heteroaryl has 1 or 2 heteroatoms selected from N, O and S as ring atoms; the 3- to 6-membered monocyclic heterocyclyl or 5- or 6-membered monocyclic heteroaryl is optionally substituted by 1 or 2 groups selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl;
      m, n are each independently selected from 0, 1, 2 and 3;
      in m R⁶, R⁷, R⁶, R⁷ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or one or more pairs of R⁶, R⁷ combining with the carbon atoms to which they are attached form a C₃₋₆ monocyclic cycloalkyl;
      in n R⁴, R⁵, R⁴, R⁵ are each independently hydrogen, deuterium, C₁₋₆ alkyl, -C₁₋₄ alkyl-hydroxyl, - C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or one or more pairs of R⁴, R⁵ combining with the carbon atoms to which they are attached form a C₃₋₆ monocyclic cycloalkyl; R⁸, R⁹ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or R⁸, R⁹ combining with the carbon atoms to which they are attached form a C₃₋₆ monocyclic cycloalkyl;
      or in n R⁴, R⁵, one R⁴ combining with R⁸ to form (CH₂)ₙ₁; n1 is 1, 2, 3 or 4; R⁹ is hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; R⁵ and the rest R⁴ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or one or more of the rest R⁴ and R⁵ combining with the same carbon atom each independently form a C₃₋₆ monocyclic cycloalkyl together with the attached carbon atom; the rest R⁵, R⁴ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy;
      E is an electrophilic addition fragment that is able to form a covalent bond with the cysteine at position 12 of KRAS, HRAS, NRAS G12C mutant proteins;
      the R^{a}, R^{b} are each independently H or C₁₋₆ alkyl or R^{a}, R^{b} combining with the nitrogen atoms to which they are attached form a 3- to 6-membered nitrogen-containing heterocyclyl;
      the R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3-to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by - CH₂CH₂- to form a cycloalkyl.

In an embodiment, the compound is shown in formula I-1 or formula I-2: 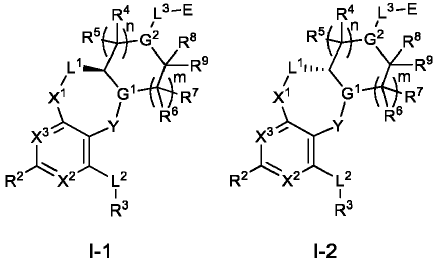 in each formula, X¹, X², X³, L¹, L², L³, R², R³, R⁴, R⁵, R⁶, R⁷, R⁸, R⁹, E, m, n, G₁, G₂ are as defined in the formula (I) of the first aspect.

In an embodiment, E is 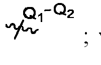 wherein, Q₁ is C(=O) or S(=O)₂; Q₂ is 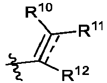 or 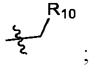 wherein, 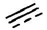 refers to a double bond or triple bond; R¹⁰, R¹¹, R¹² are each independently hydrogen, halogen, cyano, C₁₋₃ alkyl or N(C₁₋₃ alkyl)₂.

In an embodiment, E is 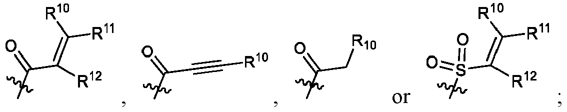 wherein, R¹⁰, R¹¹, R¹² are each independently hydrogen, halogen, cyano, C₁₋₃ alkyl or N(C₁₋₃ alkyl)₂.

In an embodiment, E is 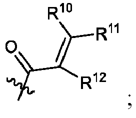 wherein, R¹² is hydrogen, halogen or cyano; R¹⁰, R¹¹ are each independently hydrogen or N(C₁₋₃ alkyl)₂.

In an embodiment, E is 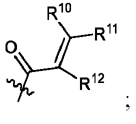 wherein, R¹⁰, R¹¹, R¹² are hydrogen.

In an embodiment, L³ is a bond.

In an embodiment, G¹ is N.

In an embodiment, G² is N.

In an embodiment, L¹ is (CR^{L1}R^{L2})ₜ; wherein, R^{L1}, R^{L2} are each independently hydrogen, deuterium, halogen or C₁₋₆ alkyl; t is 1 or 2.

In an embodiment, L¹ is CH₂ or CD₂ (preferably CH₂).

In an embodiment, X¹ is O.

In an embodiment, X² is N.

In an embodiment, X³ is CR¹; wherein, R¹ is hydrogen, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, C₂₋₆ alkenyl, C₂₋₆ alkynyl, -C₁₋₄ alkyl-hydroxyl, -C₁₋₄ alkyl-cyano, -C₁₋₄ alkyl-C₁₋₆ alkoxy, -C₁₋₄ alkyl-C₃₋₆ cycloalkoxy, -C₁₋₄ alkyl-halo C₁₋₆ alkyl, -C₁₋₄ alkyl-halo C₁₋₆ alkoxy or -C₁₋₄ alkyl-NR^{a}R^{b}.

In an embodiment, X³ is CR¹; wherein, R¹ is fluorine or chlorine.

In an embodiment, L² is a bond.

In an embodiment, L² is O or S.

In an embodiment, L² is (CR^{L3}R^{L4})ₘ₁; wherein, R^{L3}, R^{L4} are each independently hydrogen, deuterium, C₁₋₃ alkyl, deuterated C₁₋₃ alkyl, halo C₁₋₃ alkyl or cyclopropyl; or R^{L3}, R^{L4} combining with the carbon atoms to which they are attached form a cyclopropyl; m1 is 1, 2 or 3.

In an embodiment, L² is NR^{L5}; wherein, R^{L5} is hydrogen, C₁₋₃ alkyl or deuterated C₁₋₃ alkyl.

In an embodiment, L² is NH(C=O).

In an embodiment, L² is S(=O)z.

In an embodiment, L² is C(=O).

In an embodiment, when the R^{e}, R^{f} combining with the nitrogen atoms to which they are attached form a 3- to 6-membered nitrogen-containing heterocyclyl; the 3- to 6-membered nitrogen-containing heterocyclyl can have 1 nitrogen atom and optionally 1 or 2 heteroatoms selected from N, O and S as ring atoms; the 3- to 6-membered nitrogen-containing heterocyclyl can be unsubstituted or substituted by 1, 2, 3 or 4 substituents independently selected from group S1. In an embodiment, when the R^{a}, R^{b} combining with the nitrogen atoms to which they are attached form a 3 - to 6-membered nitrogen-containing heterocyclyl; the 3- to 6-membered nitrogen-containing heterocyclyl can have 1 nitrogen atom and optionally 1 or 2 heteroatoms selected from N, O and S as ring atoms.

In an embodiment, when G¹ is N, Y is -C(=O)- or -C(=S)-; L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5-to 11-membered bridged heterocyclyl, -L⁴-C₆₋₁₄ aryl, -L⁴-5- or 6-membered monocyclic heteroaryl, -L⁴-3-to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, when G¹ is N, Y is -C(=O)- or -C(=S)-; L² is O; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, when G¹ is N, Y is -C(=O)- or -C(=S)-; L² is NR^{L5}; wherein, R^{L5} is hydrogen or C₁₋₆ alkyl; R³ is 5- or 6-membered monocyclic heteroaryl, 8- to 10-membered bicyclic heteroaryl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3-to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, when G¹ is N, Y is -C(=O)- or -C(=S)-; L² is a bond; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1.

In an embodiment, when G¹ is N, Y is -C(=O)- or -C(=S)-; L² is a bond; R³ is 3- to 6-membered monocyclic heterocyclyl, 5- to 11-membered fused heterocyclyl, 7- to 11-membered spiroheterocyclyl or 5-to 11-membered bridged heterocyclyl; wherein, the 3- to 6-membered monocyclic heterocyclyl has 1, 2 or 3 heteroatoms selected from N, O and S as ring atoms; the 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the 3- to 6-membered monocyclic heterocyclyl, 5- to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1.

In an embodiment, when G¹ is N, Y is -C(=O)- or -C(=S)-; L² is a bond; R³ is 3- to 6-membered monocyclic heterocyclyl, 5- to 11-membered fused heterocyclyl, 7- to 11-membered spiroheterocyclyl or 5-to 11-membered bridged heterocyclyl; wherein, the 3- to 6-membered monocyclic heterocyclyl, 5- to 11-membered fused heterocyclyl, 7- to 11-membered spiroheterocyclyl, 5- to 11-membered bridged heterocyclyl have at least 1 N as a ring atom and R³ is attached to the parent ring through this N atom; the 3- to 6-membered monocyclic heterocyclyl, 5- to 11-membered fused heterocyclyl, 7- to 11-membered spiroheterocyclyl, 5- to 11-membered bridged heterocyclyl also optionally have 1 or 2 heteroatoms selected from N, O and S as ring atoms; the 3- to 6-membered monocyclic heterocyclyl, 5- to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1.

In an embodiment, when G¹ is N, Y is -C(=O)- or -C(=S)-; L² is a bond; R³ is 5-membered monocyclic heterocyclyl or 6-membered monocyclic heterocyclyl; wherein, the 5-membered monocyclic heterocyclyl or 6-membered monocyclic heterocyclyl has at least 1 N as a ring atom and R³ is attached to the parent ring through this N atom; the 5-membered monocyclic heterocyclyl or 6-membered monocyclic heterocyclyl also optionally has 1 or 2 heteroatoms selected from N, O and S as ring atoms; the 5-membered monocyclic heterocyclyl or 6-membered monocyclic heterocyclyl is each independently unsubstituted or substituted by 1, 2 or 3 substituents independently selected from group S1.

In an embodiment, when G¹ is N, Y is -C(=O)- or -C(=S)-; L² is O or S; R³ is hydrogen, C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5-to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L^{4_}C₁₋₆ alkoxy, -L 4-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3-to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1.

In an embodiment, when G¹ is N, Y is -C(=O)- or -C(=S)-; L² is O; R³ is C₆₋₁₄ aryl, 3- to 6-membered monocyclic heterocyclyl or -L⁴-3- to 6-membered monocyclic heterocyclyl; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 3- to 6-membered monocyclic heterocyclyl are each independently unsubstituted or substituted by 1, 2, 3 or 4 substituents independently selected from group S1.

In an embodiment, when G¹ is N, Y is -C(=O)- or -C(=S)-; L² is (CR^{L3}R^{L4})ₘ₁; wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{a}, R^{b} are each independently H or C₁₋₆ alkyl or R^{a}, R^{b} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, when G¹ is N, Y is -C(=O)- or -C(=S)-; L² is NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is hydrogen, C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{a}, R^{b} are each independently H or C₁₋₆ alkyl or R^{a}, R^{b} combining with the nitrogen atoms to which they are attached form a 3- to 6-membered nitrogen-containing heterocyclyl; the 3-to 6-membered nitrogen-containing heterocyclyl has 1 nitrogen atom and optionally 1 or 2 heteroatoms selected from N, O and S as ring atoms; the R^{e}, R^{f} are each independently H or C₁₋₆ alkyl or R^{e}, R^{f} combining with the nitrogen atoms to which they are attached form a 3 - to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, when G¹ is N, Y is -C(=O)- or -C(=S)-; L² is NR^{L5}; wherein, R^{L5} is hydrogen, C₁₋₃ alkyl or deuterated C₁₋₃ alkyl; R³ is C₆₋₁₄ aryl, 5- or 6-membered monocyclic heteroaryl, 3- to 6-membered monocyclic heterocyclyl, 5- to 11-membered bridged heterocyclyl or -L⁴-C₆₋₁₄ aryl; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 5- or 6-membered monocyclic heteroaryl, 3- to 6-membered monocyclic heterocyclyl, 5- to 11-membered bridged heterocyclyl are each independently unsubstituted or substituted by 1, 2, 3 or 4 substituents independently selected from group S1.

In an embodiment, when G¹ is N, Y is -C(=O)- or -C(=S)-; L² is NH(C=O), S(=O)₂ or C(=O); R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7-to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, the L⁴ is unsubstituted or wherein 1, 2, 3, or 4 hydrogen atoms are independently substituted by a substituent selected from methyl, ethyl, halomethyl, haloethyl, deuterated methyl, deuterated ethyl.

In an embodiment, R^{L3}, R^{L4} are each independently hydrogen, deuterium, C₁₋₃ alkyl, deuterated C₁₋₃ alkyl, halo C₁₋₃ alkyl or C₃₋₆ monocyclic cycloalkyl; or R^{L3}, R^{L4} combining with the carbon atoms to which they are attached form a cyclopropyl.

In an embodiment, R^{L3}, R^{L4} are each independently hydrogen, deuterium, methyl, deuterated methyl, halomethyl or cyclopropyl; or R^{L3}, R^{L4} combining with the carbon atoms to which they are attached form a cyclopropyl.

In an embodiment, R^{L5} is hydrogen, C₁₋₃ alkyl, deuterated C₁₋₃ alkyl, halo C₁₋₃ alkyl or C₃₋₆ monocyclic cycloalkyl.

In an embodiment, R^{L5} is hydrogen, methyl, deuterated methyl, halomethyl or cyclopropyl.

In an embodiment, G² is N.

In an embodiment, X¹ is O; L¹ is CH₂ or CDz; X³ is CR¹; wherein, R¹ is hydrogen, halogen (preferably chlorine or fluorine) or C₁₋₆ alkyl; X² is CR^{x4}; wherein, R^{x4} is hydrogen or halogen; G¹ is N; G² is N; Y is -C(=O)-.

In an embodiment, X¹ is O; L¹ is CH₂ or CDz; X³ is CR¹; wherein, R¹ is hydrogen, halogen (preferably chlorine or fluorine) or C₁₋₆ alkyl; X² is N; G¹ is N; G² is N; Y is -C(=O)-.

In an embodiment, X¹ is O; L¹ is CH₂ or CDz; X³ is CR¹; wherein, R¹ is hydrogen, halogen (preferably chlorine or fluorine) or C₁₋₆ alkyl; X² is CR^{x4}; wherein, R^{x4} is hydrogen or halogen; G¹ is N; G² is N; Y is -C(R^{L7}R^{L8})-; wherein, R^{L7}, R^{L8} are each independently hydrogen or deuterium.

In an embodiment, X¹ is O; L¹ is CH₂ or CDz; X³ is CR¹; wherein, R¹ is hydrogen, halogen (preferably chlorine or fluorine) or C₁₋₆ alkyl; X² is N; G¹ is N; G² is N; Y is -C(R^{L7}R^{L8})-; wherein, R^{L7}, R^{L8} are each independently hydrogen or deuterium.

In an embodiment, the compound is shown in formula II: 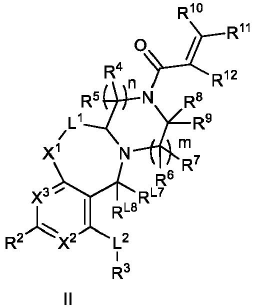
in the formula, X¹, X², X³, L¹, R², R⁴, R⁵, R⁶, R⁷, R⁸, R⁹, m, n are as defined in the corresponding groups in formula (I) of the first aspect;
R¹⁰, R¹¹, R¹² are each independently hydrogen, halogen, cyano, C₁₋₃ alkyl or N(C₁₋₃ alkyl)₂;
R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
L² is O, S or NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1);
   or
L² is NH(C=O), S(=O)₂ or C(=O); R³ is C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; and when R³ is NR^{e}R^{f}, R^{e}, R^{f} are not simultaneously H; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1);
   or
L² is a bond or (CR^{L3}R^{L4})ₘ₁; wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy or -C₁₋₄ alkyl-NR^{a}R^{b}; or R^{L3}, R^{L4} combining with the carbon atoms to which they are attached form a C₃₋₆ monocyclic cycloalkyl; R³ is NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1 (in a preferred embodiment, the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3-to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1);
the R^{a}, R^{b} are each independently H or C₁₋₆ alkyl or R^{a}, R^{b} combining with the nitrogen atoms to which they are attached form a 3- to 6-membered nitrogen-containing heterocyclyl (in a preferred embodiment, the 3- to 6-membered nitrogen-containing heterocyclyl has 1 nitrogen atom and optionally 1 or 2 heteroatoms selected from N, O and S as ring atoms).

In an embodiment, the compound is shown in formula II-1 or II-2: 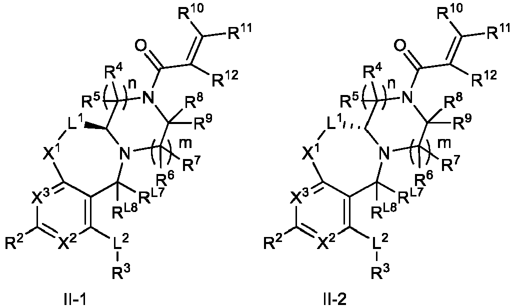 in the formula, X¹, X², X³, L¹, L², R², R³, R⁴, R⁵, R⁶, R⁷, R⁸, R⁹, R¹⁰, R¹¹, R¹², R^{L7}, R^{L8}, m, n are as defined in the corresponding groups in formula II.

In an embodiment, R^{L7}, R^{L8} are each independently hydrogen or deuterium.

In an embodiment, the compound is shown in formula III: 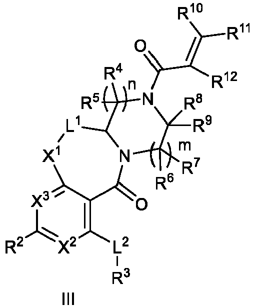
in the formula, X¹, X², X³, L¹, R², R⁴, R⁵, R⁶, R⁷, R⁸, R⁹, m, n are as defined in the corresponding groups in the formula (I) of the first aspect;
R¹⁰, R¹¹, R¹² are each independently hydrogen, halogen, cyano, C₁₋₃ alkyl or N(C₁₋₃ alkyl)₂;
L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
the R^{a}, R^{b} are each independently H or C₁₋₆ alkyl or R^{a}, R^{b} combining with the nitrogen atoms to which they are attached form a 3- to 6-membered nitrogen-containing heterocyclyl (in a preferred embodiment, the 3- to 6-membered nitrogen-containing heterocyclyl has 1 nitrogen atom and optionally 1 or 2 heteroatoms selected from N, O and S as ring atoms);
the R^{e}, R^{f} are each independently H or C₁₋₆ alkyl or R^{e}, R^{f} combining with the nitrogen atoms to which they are attached form a 3- to 6-membered nitrogen-containing heterocyclyl (in a preferred embodiment, the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, the compound is shown in formula III-1 or III-2: 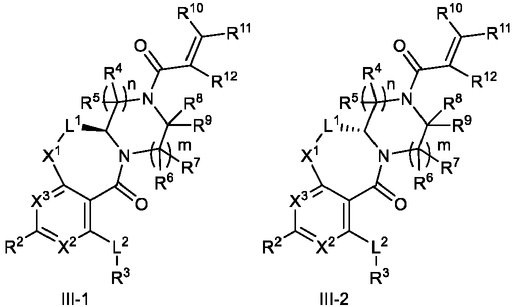 in the formula, X¹, X², X³, L¹, L², R², R³, R⁴, R⁵, R⁶, R⁷, R⁸, R⁹, R¹⁰, R¹¹, R¹², m, n are as defined in the corresponding groups in formula III.

In an embodiment, the compound is shown in formula IV: 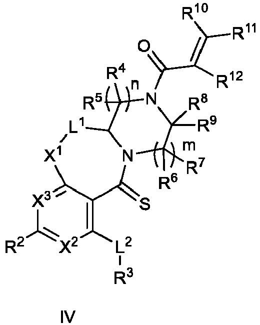
in the formula, X¹, X², X³, L¹, R², R⁴, R⁵, R⁶, R⁷, R⁸, R⁹, m, n are as defined in the corresponding groups in the formula (I) of the first aspect;
R¹⁰, R¹¹, R¹² are each independently hydrogen, halogen, cyano, C₁₋₃ alkyl or N(C₁₋₃ alkyl)₂;
L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
the R^{a}, R^{b} are each independently H or C₁₋₆ alkyl or R^{a}, R^{b} combining with the nitrogen atoms to which they are attached form a 3- to 6-membered nitrogen-containing heterocyclyl (in a preferred embodiment, the 3- to 6-membered nitrogen-containing heterocyclyl has 1 nitrogen atom and optionally 1 or 2 heteroatoms selected from N, O and S as ring atoms);
the R^{e}, R^{f} are each independently H or C₁₋₆ alkyl or R^{e}, R^{f} combining with the nitrogen atoms to which they are attached form a 3- to 6-membered nitrogen-containing heterocyclyl (in a preferred embodiment, the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1).

In an embodiment, the compound is shown in formula IV-1 or IV-2: 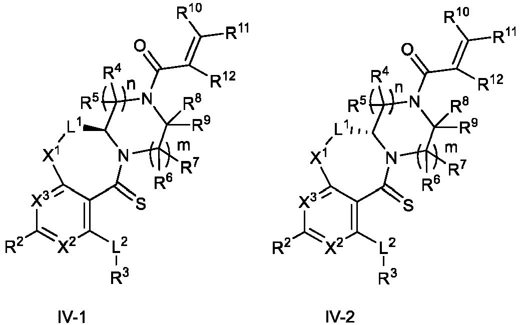 in the formula, X¹, X², X³, L¹, L², R², R³, R⁴, R⁵, R⁶, R⁷, R⁸, R⁹, R¹⁰, R¹¹, R¹², m, n are as defined in the corresponding groups in formula IV

In an embodiment, in the formula II, formula II-1, formula II-2, formula III, formula III-1, formula III-2, formula IV, formula IV-1, formula IV-2, R¹⁰, R¹¹, R¹² are each independently hydrogen, halogen, cyano, C₁₋₃ alkyl or N(C₁₋₃ alkyl)₂.

In an embodiment, in the formula II, formula II-1, formula II-2, formula III, formula III-1, formula III-2, formula IV, formula IV-1, formula IV-2, R¹² is each independently hydrogen, halogen or cyano; R¹⁰, R¹¹ are each independently hydrogen or N(C₁₋₃ alkyl)₂.

In an embodiment, in the formula II, formula II-1, formula II-2, formula III, formula III-1, formula III-2, formula IV, formula IV-1, formula IV-2, R¹⁰, R¹¹, R¹² are each independently hydrogen.

In an embodiment, the compound is shown in formula III-1-1: 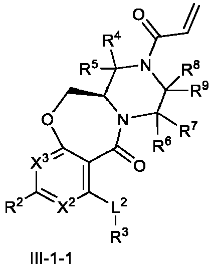 in the formula, X², X³, L², R², R³, R⁴, R⁵, R⁶, R⁷, R⁸, R⁹ are as defined in the corresponding groups in formula III.

In an embodiment, the compound is shown in formula III-1-2: 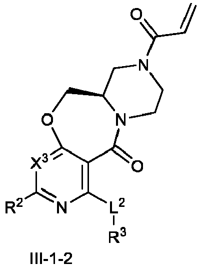 in the formula, X³, L², R², R³are as defined in the corresponding groups in formula III.

In an embodiment, in the formula II, formula II-1, formula II-2, formula III, formula III-1, formula III-2, formula III-1-1, formula III-1-2, formula IV-1, formula IV-2 or formula IV, when L² is a bond; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7-to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl.

In an embodiment, in the formula II, formula II-1, formula II-2, formula III, formula III-1, formula III-2, formula III-1-1, formula III -1-2, formula IV-1, formula IV-2 or formula IV, when L² is a bond; R³ is 8-to 10-membered bicyclic heteroaryl; the 8- to 10-membered bicyclic heteroaryl is 8- to 10-membered heteroaryl-heterocyclyl; the 8- to 10-membered bicyclic heteroaryl is unsubstituted or substituted by 1, 2, 3 or 4 substituents independently selected from group S1. For example, the 8- to 10-membered heteroaryl - heterocyclyl is 8- to 10-membered fused bicyclic heteroaryl formed by 5- or 6-membered monocyclic heteroaryl ring fused with 5- or 6-membered monocyclic heterocyclyl ring. For another example, the 8- to 10-membered heteroaryl-heterocyclyl is pyrrolo 5-membered monocyclic heteroaryl ring or pyrrolo 6-membered monocyclic heteroaryl ring.

In an embodiment, in the formula II, formula II-1, formula II-2, formula III, formula III-1, formula III-2, formula III-1-1, formula III-1-2, formula IV-1, formula IV-2 or formula IV, when L² is a bond; R³ is 3-to 6-membered monocyclic heterocyclyl, 5- to 11-membered fused heterocyclyl, 7- to 11-membered spiroheterocyclyl or 5- to 11-membered bridged heterocyclyl; wherein, the 3- to 6-membered monocyclic heterocyclyl has 1, 2 or 3 heteroatoms selected from N, O and S as ring atoms; the 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the 3- to 6-membered monocyclic heterocyclyl, 5-to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1.

In an embodiment, in the formula II, formula II-1, formula II-2, formula III, formula III-1, formula III-2, formula III-1-1, formula III-1-2, formula IV-1, formula IV-2 or formula IV, when L² is a bond; R³ is 3-to 6-membered monocyclic heterocyclyl, 5- to 11-membered fused heterocyclyl, 7- to 11-membered spiroheterocyclyl or 5- to 11-membered bridged heterocyclyl; wherein, the 3- to 6-membered monocyclic heterocyclyl, 5- to 11-membered fused heterocyclyl, 7- to 11-membered spiroheterocyclyl, 5- to 11-membered bridged heterocyclyl have at least 1 N as a ring atom and R³ is attached to the parent ring through this N atom; the 3- to 6-membered monocyclic heterocyclyl, 5- to 11-membered fused heterocyclyl, 7- to 11-membered spiroheterocyclyl, 5- to 11-membered bridged heterocyclyl also optionally have 1 heteroatom selected from N, O and S as ring atom; the 3- to 6-membered monocyclic heterocyclyl, 5- to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1.

In an embodiment, in the formula II, formula II-1, formula II-2, formula III, formula III-1, formula III-2, formula III-1-1, formula III-1-2, formula IV-1, formula IV-2 or formula IV, when L² is a bond; R³ is 5-membered monocyclic heterocyclyl or 6-membered monocyclic heterocyclyl; wherein, the 5-membered monocyclic heterocyclyl or 6-membered monocyclic heterocyclyl has at least 1 N as a ring atom and R³ is attached to the parent ring through this N atom; the 5-membered monocyclic heterocyclyl or 6-membered monocyclic heterocyclyl also optionally has 1 heteroatom selected from N, O and S as a ring atom; the 5-membered monocyclic heterocyclyl or 6-membered monocyclic heterocyclyl is each independently unsubstituted or substituted by 1, 2 or 3 substituents independently selected from group S1.

In an embodiment, in the formula II, formula II-1, formula II-2, formula III, formula III-1, formula III-2, formula III-1-1, formula III-1-2, formula IV-1, formula IV-2 or formula IV, when L² is O or S; R³ is hydrogen, C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl.

In an embodiment, in the formula II, formula II-1, formula II-2, formula III, formula III-1, formula III-2, formula III-1-1, formula III-1-2, formula IV-1, formula IV-2 or formula IV, when L² is O; R³ is C₆₋₁₄ aryl, 3- to 6-membered monocyclic heterocyclyl or -L⁴-3- to 6-membered monocyclic heterocyclyl; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 3- to 6-membered monocyclic heterocyclyl are each independently unsubstituted or substituted by 1, 2, 3 or 4 substituents independently selected from group S1.

In an embodiment, in the formula II, formula II-1, formula II-2, formula III, formula III-1, formula III-2, formula III-1-1, formula III-1-2, formula IV-1, formula IV-2 or formula IV, when L² is (CR^{L3}R^{L4})ₘ₁, wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{a}, R^{b} are each independently H or C₁₋₆ alkyl or R^{a}, R^{b} combining with the nitrogen atoms to which they are attached form a 3- to 6-membered nitrogen-containing heterocyclyl; the R^{e}, R^{f} are each independently H or C₁₋₆ alkyl or R^{e}, R^{f} combining with the nitrogen atoms to which they are attached form a 3- to 6-membered nitrogen-containing heterocyclyl.

In an embodiment, in the formula II, formula II-1, formula II-2, formula III, formula III-1, formula III-2, formula III-1-1, formula III-1-2, formula IV-1, formula IV-2 or formula IV, when L² is NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is hydrogen, C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{a}, R^{b} are each independently H or C₁₋₆ alkyl or R^{a}, R^{b} combining with the nitrogen atoms to which they are attached form a 3- to 6-membered nitrogen-containing heterocyclyl; the R^{e}, R^{f} are each independently H or C₁₋₆ alkyl or R^{e}, R^{f} combining with the nitrogen atoms to which they are attached form a 3- to 6-membered nitrogen-containing heterocyclyl.

In an embodiment, in the formula II, formula II-1, formula II-2, formula III, formula III-1, formula III-2, formula III-1-1, formula III-1-2, formula IV-1, formula IV-2 or formula IV, when L² is NR^{L5}; wherein, R^{L5} is hydrogen, C₁₋₃ alkyl or deuterated C₁₋₃ alkyl; R³ is C₁₋₈ alkyl, C₆₋₁₄ aryl, 5- or 6-membered monocyclic heteroaryl, C₃₋₆ monocyclic cycloalkyl, 3- to 6-membered monocyclic heterocyclyl, 5- to 11-membered bridged heterocyclyl, -L⁴-3- to 6-membered monocyclic heterocyclyl, -L⁴-5- or 6-membered monocyclic heteroaryl, -L⁴-C₆₋₁₄ aryl or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 5- or 6-membered monocyclic heteroaryl, C₃₋₆ monocyclic cycloalkyl, 3- to 6-membered monocyclic heterocyclyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl.

In an embodiment, in the formula II, formula II-1, formula II-2, formula III, formula III-1, formula III-2, formula III-1-1, formula III-1-2, formula IV-1, formula IV-2 or formula IV, when L² is NR^{L5}; wherein, R^{L5} is hydrogen, C₁₋₃ alkyl or deuterated C₁₋₃ alkyl; R³ is C₆₋₁₄ aryl, 5- or 6-membered monocyclic heteroaryl, 3- to 6-membered monocyclic heterocyclyl, 5- to 11-membered bridged heterocyclyl or -L⁴-C₆₋₁₄ aryl; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 5- or 6-membered monocyclic heteroaryl, 3- to 6-membered monocyclic heterocyclyl, 5- to 11-membered bridged heterocyclyl are each independently unsubstituted or substituted by 1, 2, 3 or 4 substituents independently selected from group S1.

In an embodiment, in the formula II, formula II-1, formula II-2, formula III, formula III-1, formula III-2, formula III-1-1, formula III-1-2, formula IV-1, formula IV-2 or formula IV, when L² is NH(C=O), S(=O)₂ or C(=O); R³ is hydrogen, C₁₋₈ alkyl, NR^{e}R^{f}, C₆₋₁₄ aryl, 5- or 6-membered monocyclic heteroaryl, 8-to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl.

In an embodiment, the L⁴ is unsubstituted or wherein 1, 2, 3, or 4 hydrogen atoms are independently substituted by a substituent selected from methyl, ethyl, halomethyl, haloethyl, deuterated methyl, deuterated ethyl.

In an embodiment, R^{L3}, R^{L4} are each independently hydrogen, deuterium, C₁₋₃ alkyl, deuterated C₁₋₃ alkyl, halo C₁₋₃ alkyl or C₃₋₆ monocyclic cycloalkyl; or R^{L3}, R^{L4} combining with the carbon atoms to which they are attached form a cyclopropyl.

In an embodiment, R^{L3}, R^{L4} are each independently hydrogen, deuterium, methyl, deuterated methyl, halomethyl or cyclopropyl; or R^{L3}, R^{L4} combining with the carbon atoms to which they are attached form a cyclopropyl.

In an embodiment, R^{L5} is hydrogen, C₁₋₃ alkyl, deuterated C₁₋₃ alkyl, halo C₁₋₃ alkyl or C₃₋₆ monocyclic cycloalkyl.

In an embodiment, R^{L5} is hydrogen, methyl, deuterated methyl, halomethyl or cyclopropyl.

In an embodiment, m is 1.

In an embodiment, n is 1.

In an embodiment, X¹ is O.

In an embodiment, X¹ is S.

In an embodiment, X¹ is NR^{x1}; wherein, R^{x1} is hydrogen, C₁₋₃ alkyl or deuterated C₁₋₃ alkyl.

In an embodiment, X¹ is NR^{x1}; wherein, R^{x1} is hydrogen, methyl, deuterated methyl, ethyl or isopropyl.

In an embodiment, X¹ is CR^{x2}R^{x3}; wherein, R^{x2}, R^{x3} are each independently hydrogen, deuterium, halogen, cyano, C₁₋₃ alkyl, deuterated C₁₋₃ alkyl, C₁₋₃ alkoxy, halo C₁₋₃ alkyl or halo C₁₋₃ alkoxy.

In an embodiment, X¹ is CR^{x2}R^{x3}; wherein, R^{x2}, R^{x3} are each independently hydrogen, deuterium, halogen, cyano, methyl, deuterated methyl, methoxy, halomethyl, halomethoxy, cyclopropyl or cyclopropoxy.

In an embodiment, X² is N or CR^{x4}; wherein, R^{x4} is hydrogen, halogen, cyano, C₁₋₃ alkyl, deuterated C₁₋₃ alkyl, halo C₁₋₃ alkyl or cyclopropyl.

In an embodiment, X² is N.

In an embodiment, X² is CR^{x4}; wherein, R^{x4} is hydrogen.

In an embodiment, X³ is N or CR¹; wherein, R¹ is hydrogen, halogen, cyano, C₁₋₃ alkyl, deuterated C₁₋₃ alkyl, C₁₋₃ alkoxy, halo C₁₋₃ alkyl, halo C₁₋₃ alkoxy or cyclopropyl.

In an embodiment, X³ is N.

In an embodiment, X³ is CR¹; wherein, R¹ is hydrogen or halogen (preferably chlorine or fluorine).

In an embodiment, X² is N and X³ is CR¹, wherein, R¹ is defined as above; or X² is CR^{x4} and X³ is N, R^{x4} is defined as above, R^{x4} is preferably H or halogen.

In an embodiment, L¹ is (CR^{L1}R^{L2})ₜ or C(=O); wherein, R^{L1}, R^{L2} are each independently hydrogen, deuterium or C₁₋₃ alkyl; t is 1 or 2.

In an embodiment, L¹ is (CR^{L1}R^{L2})ₜ; wherein, R^{L1} and R^{L2} are independently hydrogen, deuterium or C₁₋₃ alkyl; t is 1 or 2.

In an embodiment, L¹ is CH₂ or CD₂ (preferably CH₂).

In an embodiment, X¹ is O; L¹ is CH₂ or CD₂ (preferably CH₂).

In an embodiment, X¹ is NR^{x1}; wherein, R^{x1} is hydrogen, C₁₋₃ alkyl or deuterated C₁₋₃ alkyl; L¹ is C(=O).

In an embodiment, X¹ is O; L¹ is CH₂ or CD₂ (preferably CH₂); X³ is CR¹; wherein, R¹ is hydrogen, halogen (preferably chlorine or fluorine) or C₁₋₆ alkyl; X² is CR^{x4}; wherein, R^{x4} is hydrogen or halogen.

In an embodiment, X¹ is O; L¹ is CH₂ or CD₂ (preferably CH₂); X³ is CR¹; wherein, R¹ is hydrogen, halogen (preferably chlorine or fluorine) or C₁₋₆ alkyl; X² is N.

In an embodiment, L³ is a bond.

In an embodiment, R⁴, R⁵, R⁶, R⁷, R⁸, R⁹ are each independently hydrogen or deuterium (e.g., hydrogen).

In an embodiment, in R², the C₆₋₁₄ aryl is phenyl, naphthyl, 9- or 10-membered phenyl-heterocyclyl or 9- or 10-membered phenyl-cycloalkyl (preferably phenyl or naphthyl).

In an embodiment, in R², the C₆₋₁₄ aryl is phenyl.

In an embodiment, in R², the 5- or 6-membered monocyclic heteroaryl is selected from the following groups: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine and pyrazine.

In an embodiment, in R², the 5- or 6-membered monocyclic heteroaryl is pyridine.

In an embodiment, in R², the 8- to 10-membered bicyclic heteroaryl is a bicyclic group formed by a monocyclic aryl ring fused with a monocyclic heteroaryl ring, a bicyclic group formed by a monocyclic heteroaryl ring fused with a monocyclic heteroaryl ring, 8- to 10-membered heteroaryl-heterocyclyl or 8- to 10-membered heteroaryl-cycloalkyl.

In an embodiment, the 8- to 10-membered bicyclic heteroaryl is selected from the following groups: benzo[*d*]isoxazole, 1*H*-indole, isoindole, 1*H*-benzo[*d*]imidazole, benzo[*d*]isothiazole, 1*H-*benzo[*d*][1,2,3]triazole, benzo[*d*]oxazole, benzo[*d*]thiazole, indazole, benzofuran, benzo[*b*]thiophene, quinoline, isoquinoline, quinazoline, quinoxaline, cinnoline, pyrido[3,2-*d*]pyrimidine, pyrido[2,3-*d*]pyrimidine, pyrido[3,4-*d*]pyrimidine, pyrido[4,3-*d*]pyrimidine, 1,8-naphthyridine, 1,7-naphthyridine, 1,6-naphthyridine, 1,5-naphthyridine, pyrazolo[1,5-*a*]pyrimidine, imidazo[1,2-*b*]pyridazine, 1,4,5,6-tetrahydropyrrolo[3,4-c]pyrazole, 2,4,5,6-tetrahydropyrrolo[3,4-c]pyrazole, 5,6,7,8-tetrahydro-[1,2,4]triazolo[1,5-*a*]pyrazine, 4,5,6,7-tetrahydro-[1,2,3]triazolo[1,5-*a*]pyrazine, 4,5,6,7-tetrahydro-pyrazolo [1,5-*a*]pyrazine (preferably

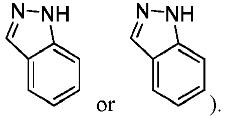

In an embodiment, in R², when the C₆₋₁₄ aryl, the 5- or 6-membered monocyclic heteroaryl and the 8- to 10-membered bicyclic heteroaryl are each independently substituted by 1, 2 or 3 groups independently selected from Rₛ₁; Rₛ₁ is independently halogen, cyano, hydroxyl, -C₁₋₆ alkyl, -C₁₋₆ alkoxy, -halo C₁₋₆ alkyl, -halo C₁₋₆ alkoxy, -C₃₋₆ monocyclic cycloalkyl or -NR^{c}R^{d}; wherein R^{c}, R^{d} are each independently H or C₁₋₆ alkyl (Rₛ₁ is independently and preferably -F, -Cl, -OH, -CN, -CH₃, -CH₂CH₃, -OCH₃, -CHF₂, -CF₃, cyclopropyl or -NH₂).

In an embodiment, R² is 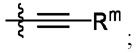 wherein R^{m} is H or C₁₋₆ alkyl (R^{m} is for example -CH₃).

In an embodiment, R² is phenyl, naphthyl or benzopyrazole; and the phenyl, naphthyl or benzopyrazole is unsubstituted or substituted by 1, 2 or 3 groups independently selected from Rₛ₁; Rₛ₁ is halogen, cyano, hydroxyl, -C₁₋₃ alkyl, -C₁₋₃ alkoxy, -halo C₁₋₃ alkyl, -halo C₁₋₃ alkoxy or -cyclopropyl.

In an embodiment, R² is selected from the following structures: 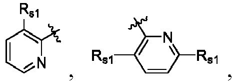 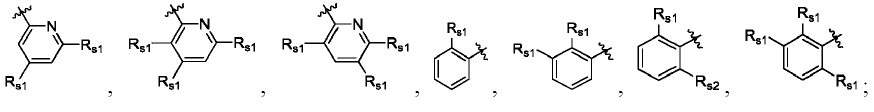 wherein Rₛ₁ of each position is the same or different; Rₛ₁, Rₛ₂ are the same or different; Rₛ₁, Rₛ₂ are each independently halogen, cyano, nitro, hydroxyl, -C₁₋₆ alkyl, -C₁₋₆ alkoxy, -halo C₁₋₆ alkyl, -deuterated C₁₋₆ alkyl, -halo C₁₋₆ alkoxy, -deuterated C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; R^{c}, R^{d} are defined as above.

In an embodiment, R² is 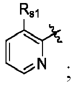 wherein Rₛ₁ is halogen, -C₁₋₆ alkyl, -halo C₁₋₆ alkyl, -deuterated C₁₋₆ alkyl or -NR^{c}R^{d}; R^{c}, R^{d} are defined as above. Preferably, Rₛ₁ is halogen or -halo C₁₋₆ alkyl (e.g., -F, - Cl or -CF₃).

In an embodiment, R² is 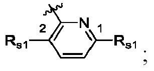 wherein Rₛ₁ at position 1 is -NR^{c}R^{d}; R^{c}, R^{d} are defined as above (preferably, R^{c}, R^{d} are independently H or -C₁₋₆ alkyl, e.g., H or -CH₃); Rₛ₁ at position 2 is halogen, -C₁₋₆ alkyl or -halo C₁₋₆ alkyl (preferably halogen or -halo C₁₋₆ alkyl, e.g., F, Cl, -CF₃ or -CH₂CF₃).

In an embodiment, R² is 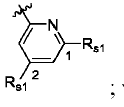 wherein Rₛ₁ at position 1 is -NR^{c}R^{d}; R^{c}, R^{d} are defined as above (preferably, R^{c}, R^{d} are H); Rₛ₁ at position 2 is halogen, -C₁₋₆ alkyl or -halo C₁₋₆ alkyl (preferably -C₁₋₆ alkyl, e.g., -CH₃).

In an embodiment, R² is 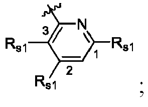 wherein Rₛ₁ at position 1 is -NR^{c}R^{d}; R^{c}, R^{d} are defined as above (preferably, R^{c}, R^{d} are H); Rₛ₁ at position 2 is halogen, -C₁₋₆ alkyl or -halo C₁₋₆ alkyl (preferably - C₁₋₆ alkyl, e.g., -CH₃ or -CH₂CH₃); Rₛ₁ at position 3 is halogen, -C₁₋₆ alkyl or -halo C₁₋₆ alkyl (preferably - halo C₁₋₆ alkyl, e.g., -CF₃ or -CH₂CF₃).

In an embodiment, R² is 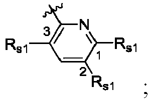 wherein Rₛ₁ at position 1 is -NR^{c}R^{d}; R^{c}, R^{d} are defined as above; Rₛ₁ at position 2 is halogen, -C₁₋₆ alkyl or -halo C₁₋₆ alkyl; Rₛ₁ at position 3 is halogen, -C₁₋₆ alkyl or -halo C₁₋₆ alkyl.

In an embodiment, R² is 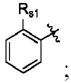 wherein, Rₛ₁ is halogen, cyano, -C₁₋₆ alkyl or -halo C₁₋₆ alkyl (preferably F, Cl, cyano, -CH₃ or -CF₃).

In an embodiment, R² is 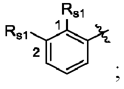 wherein Rₛ₁ at position 1 is halogen (e.g., F); Rₛ₁ at position 2 is halogen, -C₁₋₆ alkyl or -halo C₁₋₆ alkyl, preferably halogen or -C₁₋₆ alkyl (e.g., For -CH₃).

In an embodiment, R² is 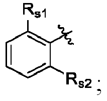 wherein, Rₛ₁ is halogen, cyano, nitro, hydroxyl, -C₁₋₆ alkyl, - C₁₋₆ alkoxy, -halo C₁₋₆ alkyl, -halo C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; R^{c}, R^{d} are defined as above; Rₛ₂ is halogen. Preferably, Rₛ₁ is halogen, hydroxyl, -C₁₋₆ alkoxy, -C₁₋₆ alkyl, -halo C₁₋₆ alkyl, -C₃₋₆ monocyclic cycloalkyl or -NR^{c}R^{d}, preferably, R^{c}, R^{d} are H; Rₛ₂ is halogen or (e.g., For Cl) or -halo C₁₋₆ alkoxy. More preferably, Rₛ₁ is F, Cl, -OH, -OCH₃, -CH₃, -CF₃, 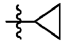 or -NH₂; Rₛ₂ is F, Cl or -CF₃.

In an embodiment, R² is 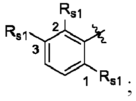 wherein Rₛ₁ at position 1 is halogen and Rₛ₁ at position 2 is hydroxyl, or Rₛ₁ at position 1 is hydroxyl and Rₛ₁ at position 2 is halogen; and Rₛ₁ at position 3 is halogen, cyano, nitro, hydroxyl, -C₁₋₆ alkyl, -C₁₋₆ alkoxy, -halo C₁₋₆ alkyl, -halo C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, - C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; R^{c}, R^{d} are defined as above. In another embodiment, Rₛ₁ at position 1 is halogen and Rₛ₁ at position 2 is -NR^{c}R^{d}, R^{c}, R^{d} are defined as above; or Rₛ₁ at position 1 is -NR^{c}R^{d}, R^{c}, R^{d} are defined as above; and Rₛ₁ at position 2 is halogen; and Rₛ₁ at position 3 is halogen. Preferably, Rₛ₁ at position 1 is F or Cl and Rₛ₁ at position 2 is hydroxyl or -NH₂, or Rₛ₁ at position 1 is hydroxyl or -NH₂ and Rₛ₁ at position 2 is F or Cl; and Rₛ₁ at position 3 is F or Cl.

In an embodiment, R² is 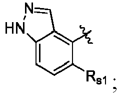 Rₛ₁ is -C₁₋₆ alkyl, e.g., -CH₃.

In an embodiment, R² is 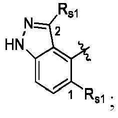 Rₛ₁ at position 1 is -C₁₋₆ alkyl, e.g., -CH₃; Rₛ₁ at position 2 is -C₃₋₆ monocyclic cycloalkyl, e.g.,

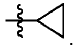

In an embodiment, R² is 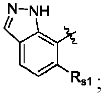 Rₛ₁ is -C₁₋₆ alkyl or halogen, e.g., -CH₃ or F.

In an embodiment, R² is selected from the following structures: 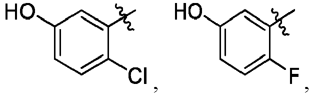 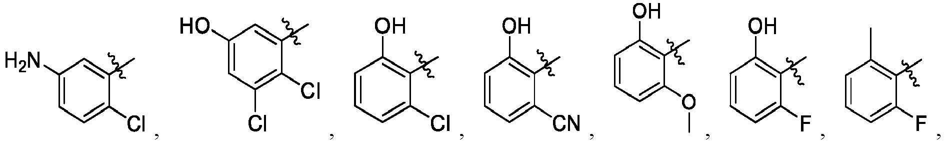 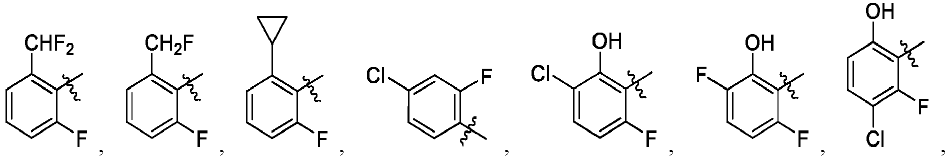 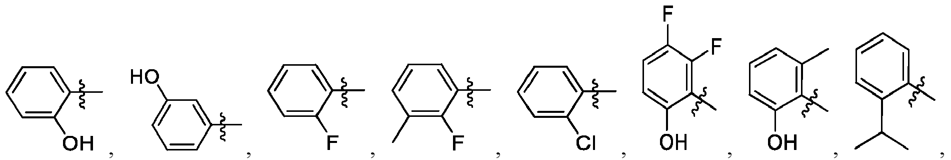 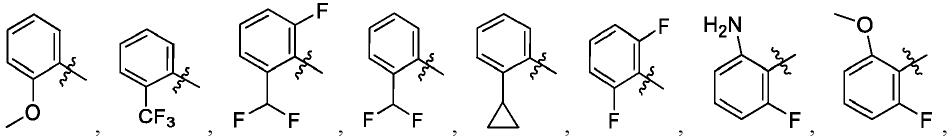 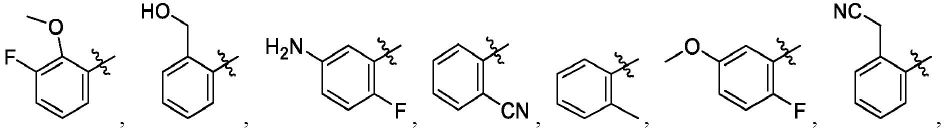 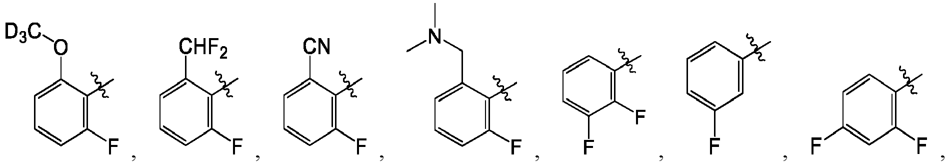 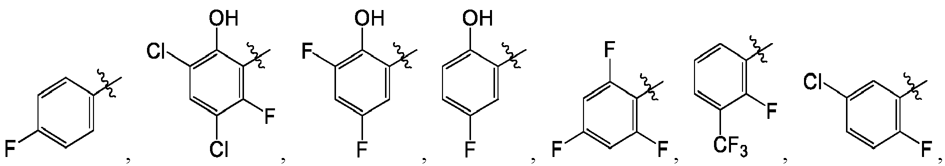 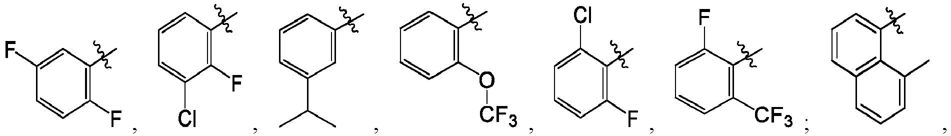 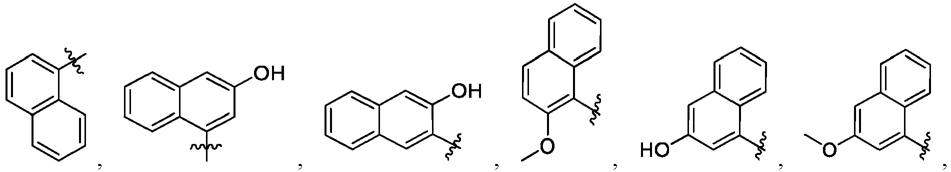 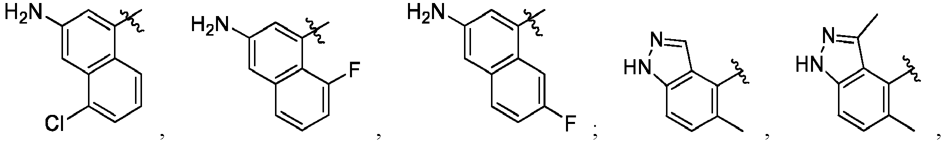 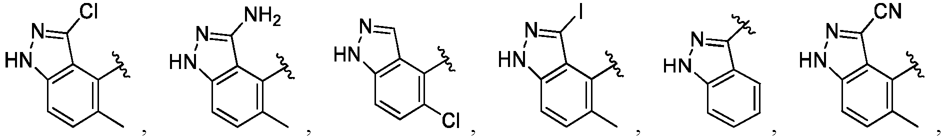 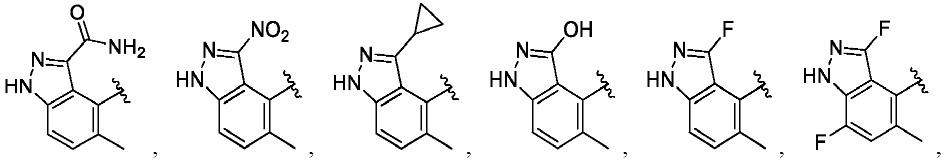 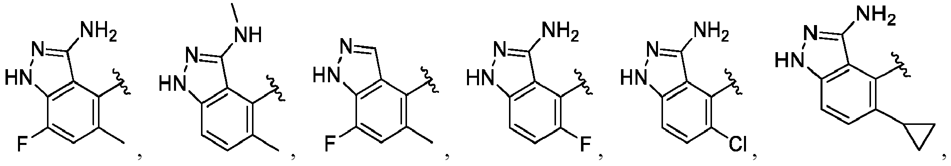 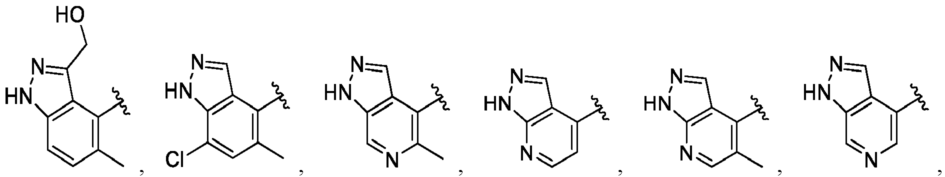 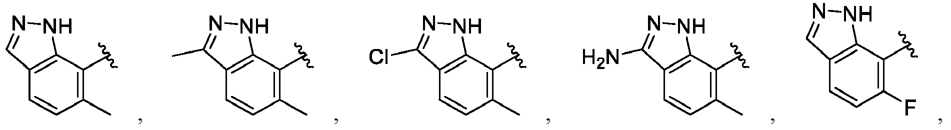 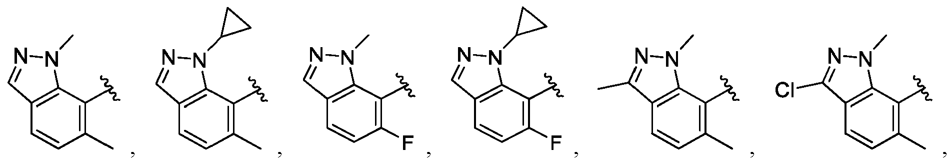 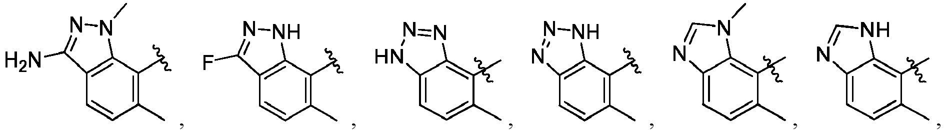 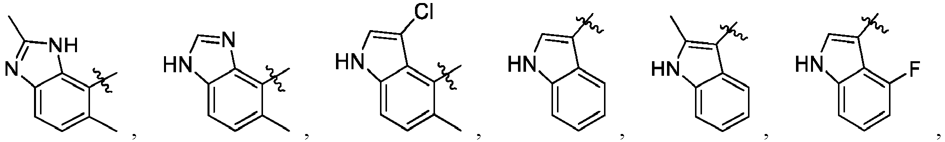 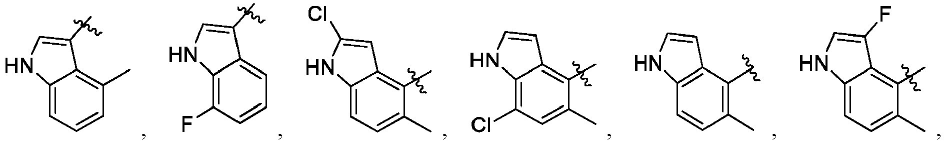 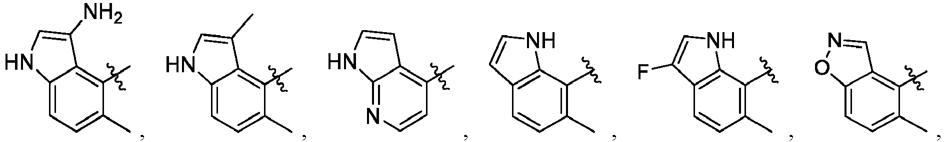 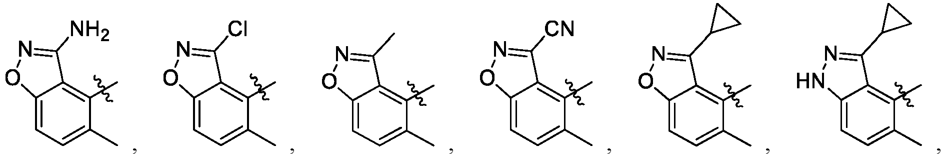 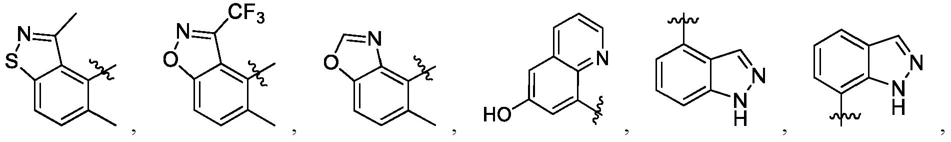 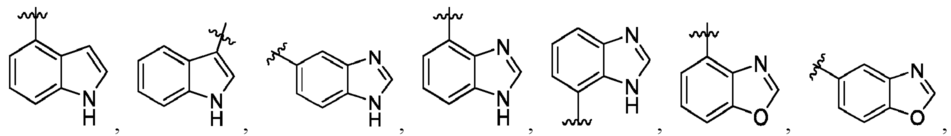 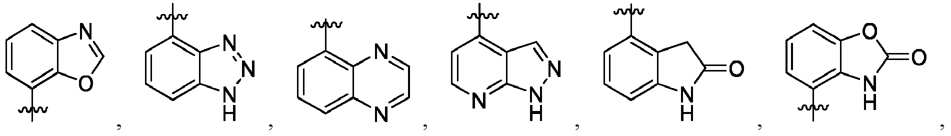 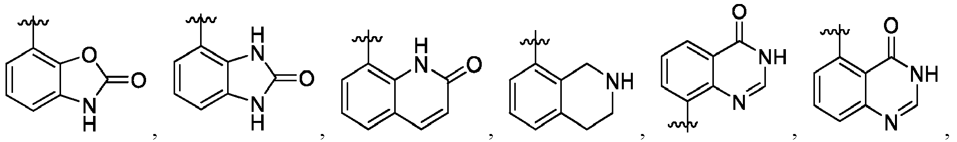 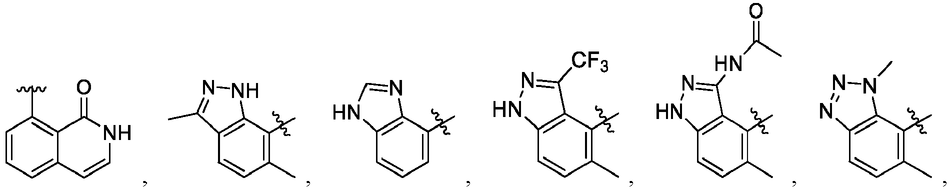 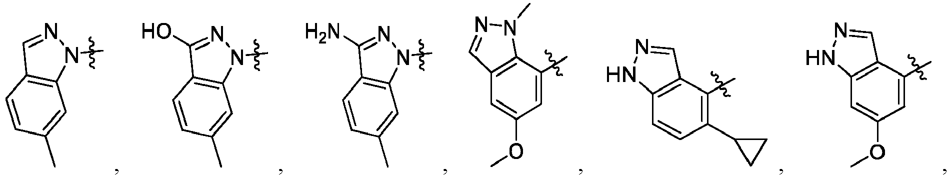 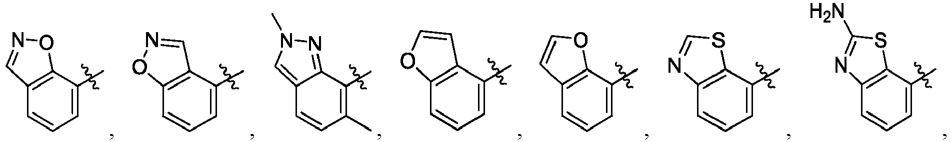 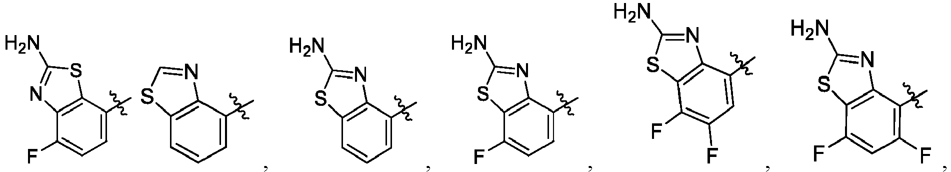 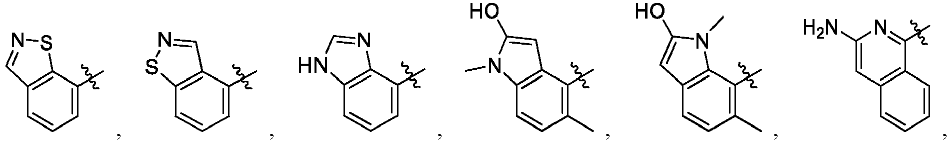 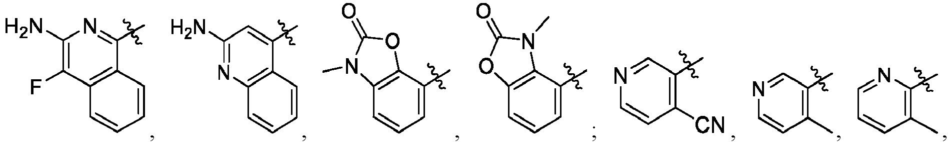 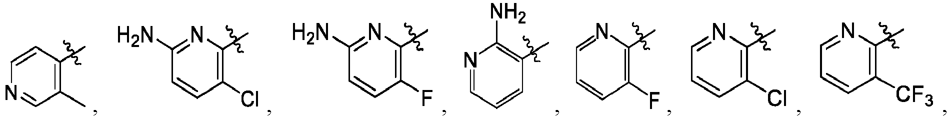 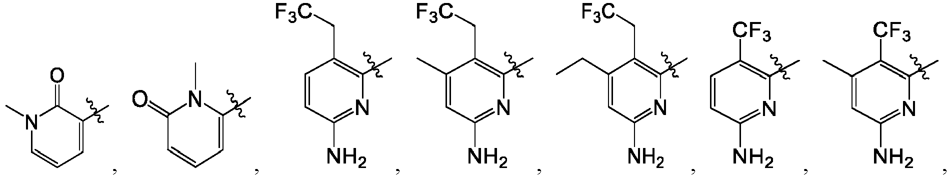 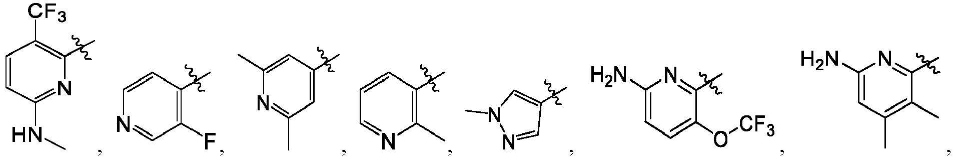 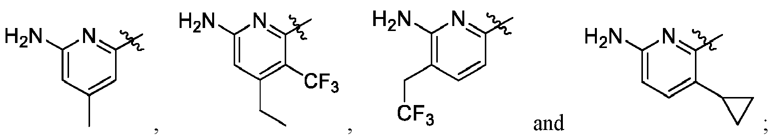 further preferably

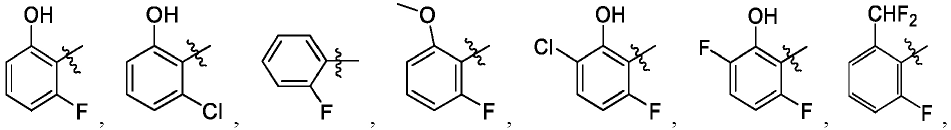

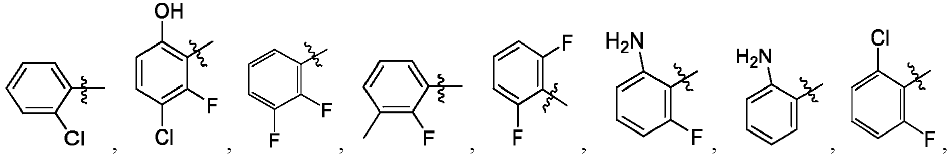

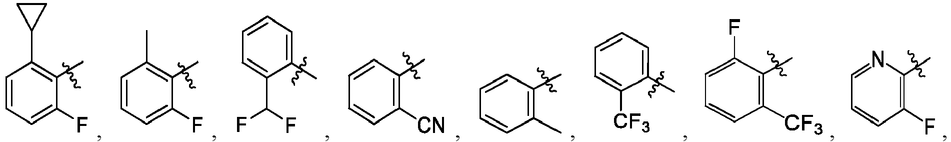

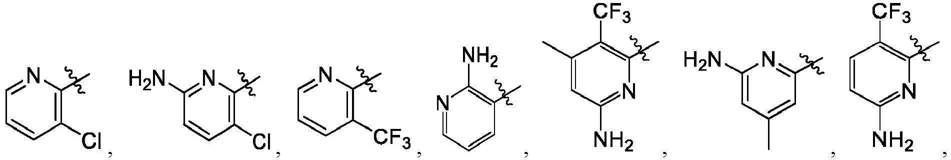

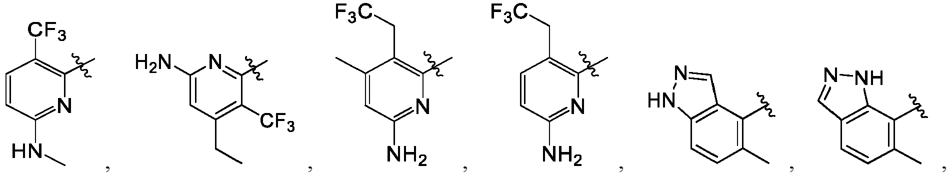

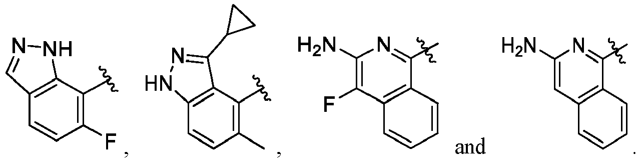

In an embodiment, R² is selected from the following structures:

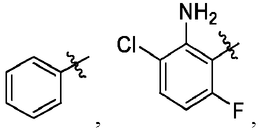

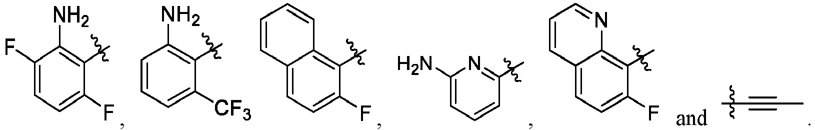

In an embodiment, in R³, the C₆₋₁₄ aryl is phenyl, naphthyl, 9- or 10-membered phenyl-heterocyclyl or 9- or 10-membered phenyl-cycloalkyl.

In an embodiment, in R³, the 5- or 6-membered monocyclic heteroaryl is selected from the following groups: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine and pyrazine.

In an embodiment, in R³, the 8- to 10-membered bicyclic heteroaryl is a bicyclic group formed by a monocyclic aryl ring fused with a monocyclic heteroaryl ring, a bicyclic group formed by a monocyclic heteroaryl ring fused with a monocyclic heteroaryl ring, 8- to 10-membered heteroaryl-heterocyclyl or 8- to 10-membered heteroaryl-cycloalkyl.

In an embodiment, the 8- to 10-membered bicyclic heteroaryl is selected from the following groups: benzo[*d*]isoxazole, 1*H*-indole, isoindole, 1*H*-benzo[*d*]imidazole, benzo[*d*]isothiazole, 1*H-*benzo[*d*][1,2,3]triazole, benzo[*d*]oxazole, benzo[*d*]thiazole, indazole, benzofuran, benzo[*b*]thiophene, quinoline, isoquinoline, quinazoline, quinoxaline, cinnoline, pyrido[3,2-*d*]pyrimidine, pyrido[2,3-*d*]pyrimidine, pyrido[3,4-*d*]pyrimidine, pyrido[4,3-*d*]pyrimidine, 1,8-naphthyridine, 1,7-naphthyridine, 1,6-naphthyridine, 1,5-naphthyridine, pyrazolo[1,5-*a*]pyrimidine, imidazo[1,2-*b*]pyridazine, 1,4,5,6-tetrahydropyrrolo[3,4-c]pyrazole, 2,4,5,6-tetrahydopyrrolo[3,4-*c*]pyrazole, 5,6,7,8-tetrahydro-[1,2,4]triazolo[1,5-*a*]pyrazine, 4,5,6,7-tetrahydro-[1,2,3]triazolo[1,5-*a*]pyrazine, 4,5,6,7-tetrahydropyrazolo[1,5-*a*]pyrazine.

In an embodiment, in R³, the C₃₋₆ monocyclic cycloalkyl is selected from the following groups: cyclopropyl, cyclobutyl, cyclopentyl and cyclohexyl.

In an embodiment, in R³, the 3- to 6-membered monocyclic heterocyclyl is selected from the following groups: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3*H*)-one, dihydrofuran-2,5-dione, piperidin-2-one, piperidin-2,6-dione, tetrahydro-2*H*-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxacyclobutadiene, 2,5-dihydro-1*H*-pyrrole, 2,5-dihydrofuran, 2,3-dihydrofuran, 2,3-dihydro-1*H*-pyrrole, 3,4-dihydro-2*H*-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1*H*)-one, 1,4-dioxan-2-one, 5,6-dihydro-2*H*-pyran-2-one, 5,6-dihydropyrimidin-4(3*H*)-one, 3,4-dihydropyridin-2(1*H*)-one, 5,6-dihydropyridin-2(1*H*)-one, 5,6-dihydropyrimidin-4(1*H*)-one, pyrimidin-4(3*H*)-one, pyrimidin-4(1*H*)-one, 4,5-dihydro-1*H*-imidazole, 2,3-dihydro-1*H*-imidazole, 2,3-dihydrooxazole, 1,3-dioxacyclopentene, 2,3-dihydrothiophene, 2,5-dihydrothiophene, 3,4-dihydro-2*H*-1,4-oxazine, 3,4-dihydro-2*H*-1,4-thiazine 1,1-dioxide, 1,2,3,4-tetrahydropyrazine, 1,3-dihydro-2*H*-pyrrol-2-one, 1,5-dihydro-2*H*-pyrrol-2-one, 1*H*-pyrrol-2,5-dione, furan-2(3H)-one, furan-2(5*H*)-one, 1,3-dioxacyclopenten-2-one, oxazol-2(3H)-one, 1,3-dihydro-2*H*-imidazol-2-one, furan-2,5-dione, 3,6-dihydropyridin-2(1*H*)-one, pyridin-2,6-(1H, 3H)-dione, 5,6-dihydro-2*H*-pyran-2-one, 3,6-dihydro-2H-pyran-2-one, 3,4-dihydro-2/7-1,3-oxazine, 3,6-dihydro-2*H*-1,3-oxazine, 1,2,3,4-tetrahydropyrimidine.

In an embodiment, in R³, the 5- to 11-membered fused cycloalkyl is selected from the following groups: fused cycloalkyl formed by 3- to 6-membered monocyclic cycloalkyl ring and 4- to 6-membered monocyclic cycloalkyl ring.

In an embodiment, in R³, the 5- to 11-membered fused cycloalkyl is selected from the following groups: 6-membered fused cycloalkyl formed by 5-membered monocyclic cycloalkyl ring and 3-membered monocyclic cycloalkyl ring, 7-membered fused cycloalkyl formed by 5-membered monocyclic cycloalkyl ring and 4-membered monocyclic cycloalkyl ring, 8-membered fused cycloalkyl formed by 5-membered monocyclic cycloalkyl ring and 5-membered monocyclic cycloalkyl ring, 7-membered fused cycloalkyl formed by 6-membered monocyclic cycloalkyl ring and 3-membered monocyclic cycloalkyl ring, 8-membered fused cycloalkyl formed by 6-membered monocyclic cycloalkyl ring and 4-membered monocyclic cycloalkyl ring, 9-membered fused cycloalkyl formed by 6-membered monocyclic cycloalkyl ring and 5-membered monocyclic cycloalkyl ring, 10-membered fused cycloalkyl formed by 6-membered monocyclic cycloalkyl ring and 6-membered monocyclic cycloalkyl ring.

In an embodiment, in R³, the 5- to 11-membered fused heterocyclyl is selected from the following groups: fused heterocyclyl formed by 3- to 6-membered monocyclic cycloalkyl ring and 4- to 6-membered monocyclic heterocyclyl ring, fused heterocyclyl formed by 3- to 6-membered monocyclic heterocyclyl ring and 4- to 6-membered monocyclic cycloalkyl ring, fused heterocyclyl formed by 3- to 6-membered monocyclic heterocyclyl ring and 4- to 6-membered monocyclic heterocyclyl ring.

In an embodiment, in R³, the 5- to 11-membered fused heterocyclyl is selected from the following groups: 6-membered fused heterocyclyl formed by 5-membered monocyclic heterocyclyl ring and 3-membered monocyclic cycloalkyl ring, 7-membered fused heterocyclyl formed by 5-membered monocyclic heterocyclyl ring and 4-membered monocyclic cycloalkyl ring, 7-membered fused heterocyclyl formed by 5-membered monocyclic heterocyclyl ring and 4-membered monocyclic heterocyclyl ring, 8-membered fused heterocyclyl formed by 5-membered monocyclic cycloalkyl ring and 5-membered monocyclic heterocyclyl ring, 8-membered fused heterocyclyl formed by 5-membered monocyclic heterocyclyl ring and 5-membered monocyclic heterocyclyl ring, 7-membered fused heterocyclyl formed by 6-membered monocyclic heterocyclyl ring and 3-membered monocyclic cycloalkyl ring, 8-membered fused heterocyclyl formed by 6-membered monocyclic heterocyclyl ring and 4-membered monocyclic cycloalkyl ring, 8-membered fused heterocyclyl formed by 6-membered monocyclic heterocyclyl ring and 4-membered monocyclic heterocyclyl ring, 9-membered fused heterocyclyl formed by 6-membered monocyclic heterocyclyl ring and 5-membered monocyclic heterocyclyl ring.

In an embodiment, in R³, the 6- to 11-membered spirocycloalkyl is selected from the following groups: spirocycloalkyl formed by 3- to 6-membered monocyclic cycloalkyl ring and 4- to 6-membered monocyclic cycloalkyl ring.

In an embodiment, in R³, the 6- to 11-membered spirocycloalkyl is selected from the following groups: 7-membered spirocycloalkyl formed by 4-membered monocyclic cycloalkyl ring and 4-membered monocyclic cycloalkyl ring, 7-membered spirocycloalkyl formed by 5-membered monocyclic cycloalkyl ring and 3-membered monocyclic cycloalkyl ring, 8-membered spirocycloalkyl formed by 5-membered monocyclic cycloalkyl ring and 4-membered monocyclic cycloalkyl ring, 9-membered spirocycloalkyl formed by 5-membered monocyclic cycloalkyl ring and 5-membered monocyclic cycloalkyl ring, 8-membered spirocycloalkyl formed by 6-membered monocyclic cycloalkyl ring and 3-membered monocyclic cycloalkyl ring, 9-membered spirocycloalkyl formed by 6-membered monocyclic cycloalkyl ring and 4-membered monocyclic cycloalkyl ring, 10-membered spirocycloalkyl formed by 6-membered monocyclic cycloalkyl ring and 5-membered monocyclic cycloalkyl ring, 11-membered spirocycloalkyl formed by 6-membered monocyclic cycloalkyl ring and 6-membered monocyclic cycloalkyl ring.

In an embodiment, in R³, the 7- to 11-membered spiroheterocyclyl is selected from the following groups: spiroheterocyclyl formed by 3- to 6-membered monocyclic cycloalkyl ring and 4- to 6-membered monocyclic heterocyclyl ring, spiroheterocyclyl formed by 3- to 6-membered monocyclic heterocyclyl ring and 4- to 6-membered monocyclic cycloalkyl ring, spiroheterocyclyl formed by 3- to 6-membered monocyclic heterocyclyl ring and 4- to 6-membered monocyclic heterocyclyl ring.

In an embodiment, in R³, the 7- to 11-membered spiroheterocyclyl is selected from the following groups: 7-membered spiroheterocyclyl formed by 4-membered monocyclic heterocyclyl ring and 4-membered monocyclic heterocyclyl ring, 7-membered spiroheterocyclyl formed by 4-membered monocyclic heterocyclyl ring and 4-membered monocyclic cycloalkyl ring, 7-membered spiroheterocyclyl formed by 5-membered monocyclic heterocyclyl ring and 3-membered monocyclic cycloalkyl ring, 8-membered spiroheterocyclyl formed by 5-membered monocyclic heterocyclyl ring and 4-membered monocyclic cycloalkyl ring, 8-membered spiroheterocyclyl formed by 5-membered monocyclic heterocyclyl ring and 4-membered monocyclic heterocyclyl ring, 9-membered spiroheterocyclyl formed by 5-membered monocyclic heterocyclyl ring and 5-membered monocyclic cycloalkyl ring, 9-membered spiroheterocyclyl formed by 5-membered monocyclic heterocyclyl ring and 5-membered monocyclic heterocyclyl ring, 8-membered spiroheterocyclyl formed by 6-membered monocyclic heterocyclyl ring and 3-membered monocyclic cycloalkyl ring, 9-membered spiroheterocyclyl formed by 6-membered monocyclic heterocyclyl ring and 4-membered monocyclic cycloalkyl ring, 9-membered spiroheterocyclyl formed by 6-membered monocyclic heterocyclyl ring and 4-membered monocyclic heterocyclyl ring.

In an embodiment, R³ is a structure shown in formula (B): 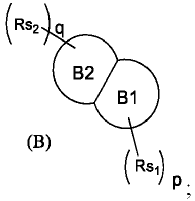
wherein, ring B1 is a benzene ring or 5- or 6-membered monocyclic heteroaryl ring ; ring B2 is a fused 5- or 6-membered monocyclic heterocycloalkyl ring or fused 5- or 6-membered monocyclic cycloalkyl ring; wherein, the 5- or 6-membered monocyclic heteroaryl ring or the fused 5- or 6-membered monocyclic heterocycloalkyl ring has 1, 2 or 3 heteroatoms selected from N, O and S as ring atoms;
(Rₛ₁)ₚ means that the hydrogen on ring B1 is substituted by p Rₛ₁, p is 0, 1, 2 or 3, each Rₛ₁ is the same or different;
(Rₛ₂)_{q} means that the hydrogen on ring B2 is substituted by q Rₛ₂, q is 0, 1, 2 or 3, each Rₛ₂ is the same or different;
Rₛ₁, Rₛ₂ are each independently halogen, cyano, nitro, hydroxyl, -C₁₋₆ alkyl, -C₁₋₆ alkoxy, -halo C₁₋₆ alkyl, -halo C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; wherein, the 3- to 6-membered heterocyclyl has 1, 2 or 3 heteroatoms selected from N, O and S as ring atoms.

In the present disclosure, the structure shown in formula (B) can be independently attached to other parts of the molecule through a ring atom on the ring B1; or can be attached to other parts of the molecule through a ring atom on the ring B2.

In an embodiment, R³ is 8- to 10-membered bicyclic heteroaryl; the 8- to 10-membered bicyclic heteroaryl is 8- to 10-membered heteroaryl-heterocyclyl; the 8- to 10-membered bicyclic heteroaryl is unsubstituted or substituted by 1, 2, 3 or 4 substituents independently selected from group S 1. For example, the 8- to 10-membered heteroaryl-heterocyclyl is the 8- to 10-membered fused bicyclic heteroaryl formed by 5- or 6-membered monocyclic heteroaryl ring fused with 5- or 6-membered monocyclic heterocyclyl ring. For another example, the 8- to 10-membered heteroaryl-heterocyclyl is pyrrolo 5-membered monocyclic heteroaryl ring or pyrrolo 6-membered monocyclic heteroaryl ring.

In an embodiment, R³ is 3- to 6-membered monocyclic heterocyclyl, 5- to 11-membered fused heterocyclyl, 7- to 11-membered spiroheterocyclyl or 5- to 11-membered bridged heterocyclyl; wherein, the 3- to 6-membered monocyclic heterocyclyl has 1, 2 or 3 heteroatoms selected from N, O and S as ring atoms; the 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the 3- to 6-membered monocyclic heterocyclyl, 5- to 11-membered fused heterocyclyl, 7- to 11-membered spiroheterocyclyl, 5-to 11-membered bridged heterocyclyl are each independently unsubstituted or substituted by 1, 2, 3 or 4 substituents independently selected from group S1.

In an embodiment, R³ is 3- to 6-membered monocyclic heterocyclyl, 5- to 11-membered fused heterocyclyl, 7- to 11-membered spiroheterocyclyl or 5- to 11-membered bridged heterocyclyl; wherein, the 3- to 6-membered monocyclic heterocyclyl, 5- to 11-membered fused heterocyclyl, 7- to 11-membered spiroheterocyclyl, 5- to 11-membered bridged heterocyclyl have at least 1 N as a ring atom and R³ is attached to the parent ring through this N atom; the 3- to 6-membered monocyclic heterocyclyl, 5- to 11-membered fused heterocyclyl, 7- to 11-membered spiroheterocyclyl, 5- to 11-membered bridged heterocyclyl also optionally have 1 heteroatom selected from N, O and S as a ring atom; the 3- to 6-membered monocyclic heterocyclyl, 5- to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1.

In an embodiment, R³ is 5-membered monocyclic heterocyclyl; wherein, the 5-membered monocyclic heterocyclyl has at least one N atom as a ring atom and R³ is attached to the parent ring through this N atom; the 5-membered monocyclic heterocyclyl also optionally has 1 heteroatom selected from N, O and S as a ring atom; the 5-membered monocyclic heterocyclyl is unsubstituted or substituted by 1, 2, 3 or 4 substituents independently selected from group S1.

In an embodiment, R³ is 5-membered monocyclic heterocyclyl; wherein, the 5-membered monocyclic heterocyclyl has one N atom as a ring atom and R³ is attached to the parent ring through this N atom; the 5-membered monocyclic heterocyclyl is substituted by 2, 3 or 4 substituents independently selected from group S1.

In an embodiment, R³ is 5-membered monocyclic heterocyclyl; wherein, the 5-membered monocyclic heterocyclyl has one N atom as a ring atom and R³ is attached to the parent ring through this N atom; the 5-membered monocyclic heterocyclyl is substituted by 2 methyl and another substituent selected from group S1.

In an embodiment, R³ is 5-membered monocyclic heterocyclyl; wherein, the 5-membered monocyclic heterocyclyl has one N atom as ring atom and R³ is attached to the parent ring through this N atom; and in the 5-membered monocyclic heterocyclyl, the carbon atom (C*) in the ortho position to the N atom is substituted by 1 or 2 methyl and the carbon atom (C**) in the meta position to the carbon atom (C*) is substituted by 1 substituent selected from group S1.

In an embodiment, R³ is 6-membered monocyclic heterocyclyl; wherein, the 6-membered monocyclic heterocyclyl has at least one N atom as a ring atom and R³ is attached to the parent ring through this N atom; the 6-membered monocyclic heterocyclyl also optionally has 1 heteroatom selected from N, O and S as a ring atom; the 6-membered monocyclic heterocyclyl is unsubstituted or substituted by 1, 2, 3 or 4 substituents independently selected from group S1.

In an embodiment, R³ is 6-membered monocyclic heterocyclyl; wherein, the 6-membered monocyclic heterocyclyl has at least one N atom as a ring atom and R³ is attached to the parent ring through this N atom; the 6-membered monocyclic heterocyclyl also has one N atom as a ring atom ; the 6-membered monocyclic heterocyclyl is substituted by 2 or 3 substituents independently selected from group S1.

In an embodiment, R³ is 6-membered monocyclic heterocyclyl; wherein, the 6-membered monocyclic heterocyclyl has at least one N atom as a ring atom and R³ is attached to the parent ring through this N atom; the 6-membered monocyclic heterocyclyl also has one N atom as a ring atom; the 6-membered monocyclic heterocyclyl is substituted by 2 methyl and another substituent selected from group S1.

In an embodiment, R³ is 6-membered monocyclic heterocyclyl; wherein, the 6-membered monocyclic heterocyclyl has at least 1 N atom (N*) as a ring atom and R³ is connected to the parent ring through this N atom (N*); and in the 6-membered monocyclic heterocyclyl, the carbon atom (C*) in the meta position to the N atom (N*) is substituted by 1 or 2 methyl and the 6-membered monocyclic heterocyclyl is further substituted by 1 substituent selected from group S1. Preferably, the 6-membered monocyclic heterocyclyl also has one N atom as a ring atom.

In an embodiment, R³ is selected from the following structures: 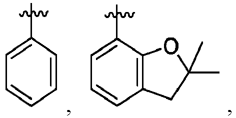 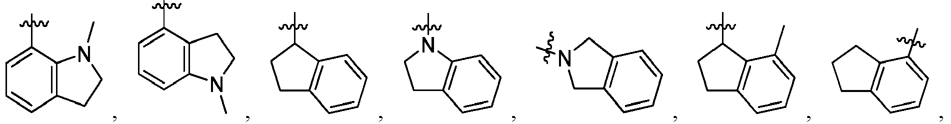 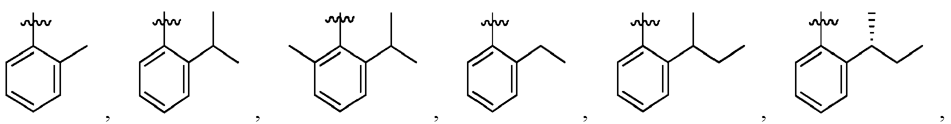 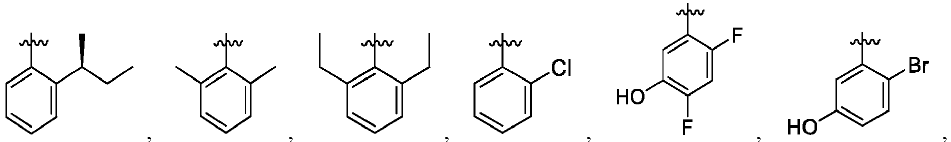 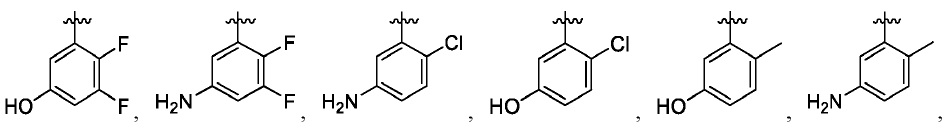 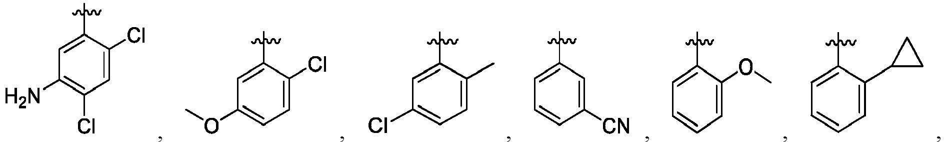 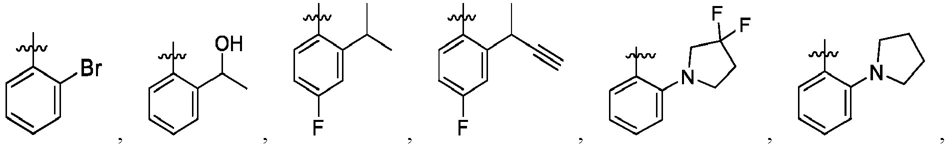 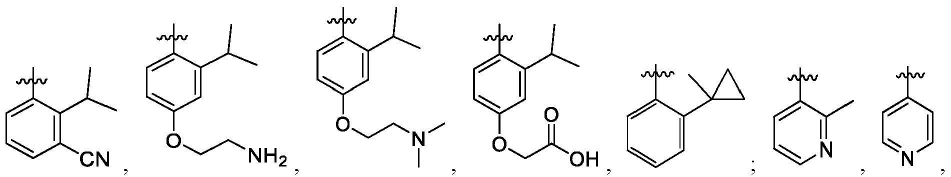 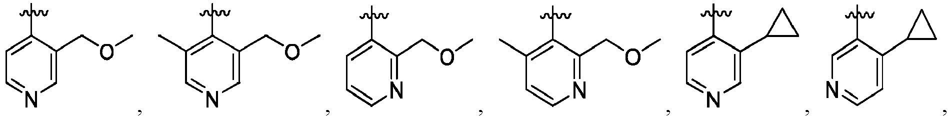 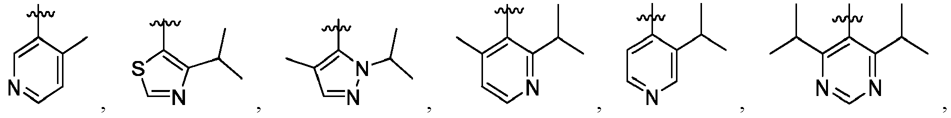 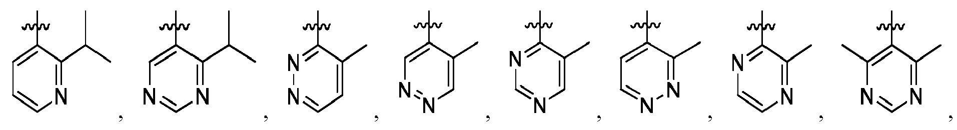 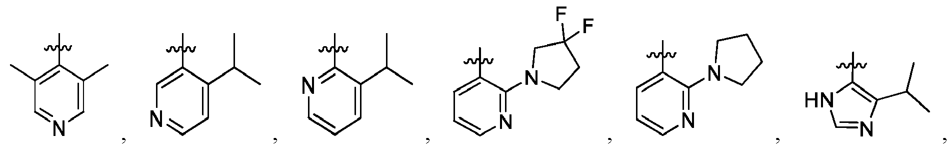 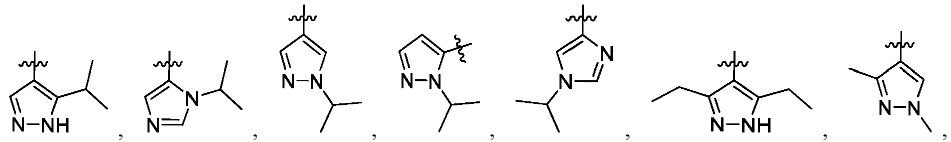 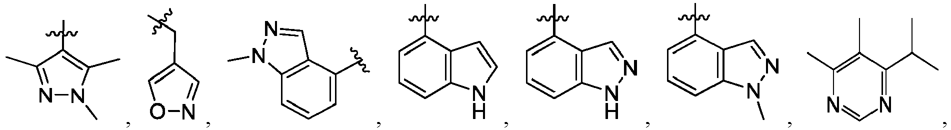 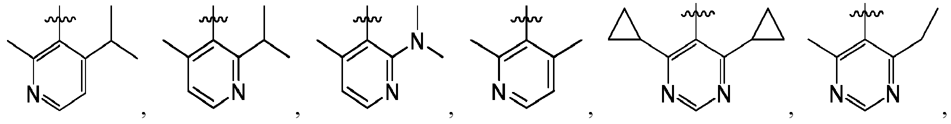 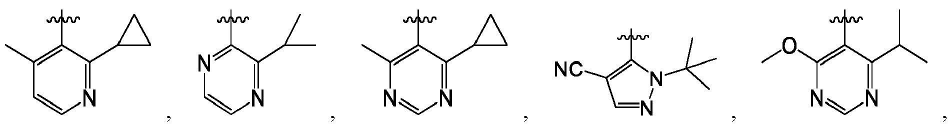 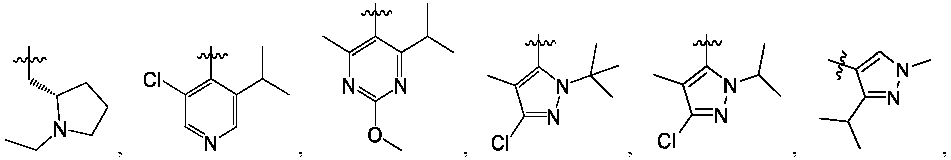 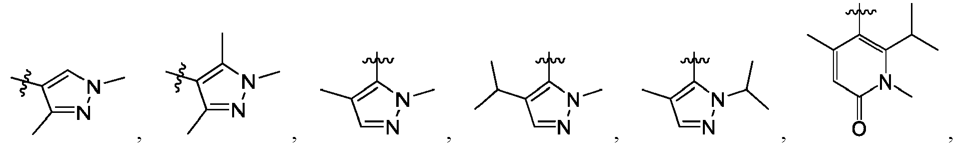 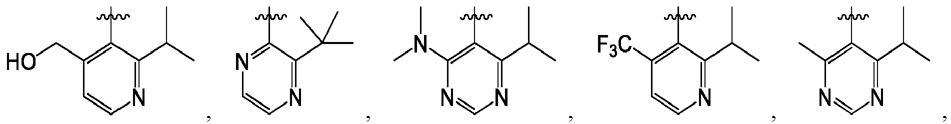 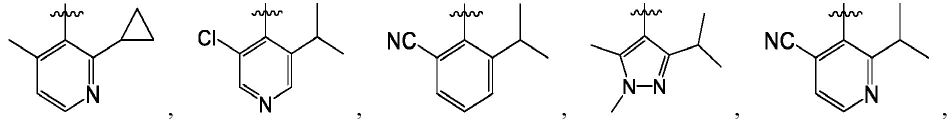 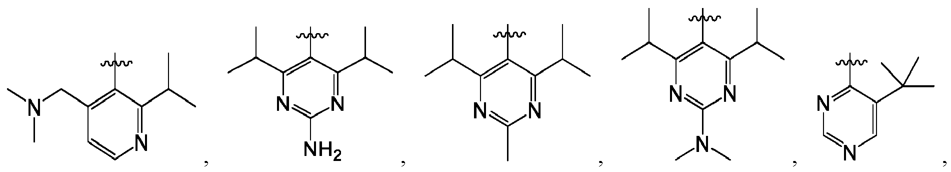 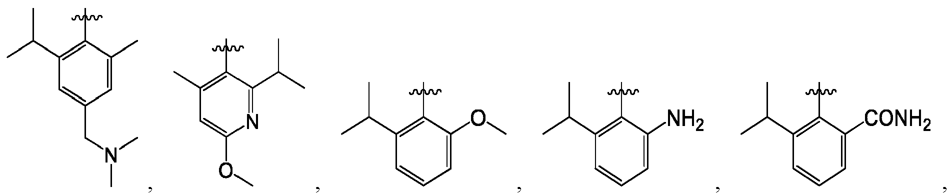 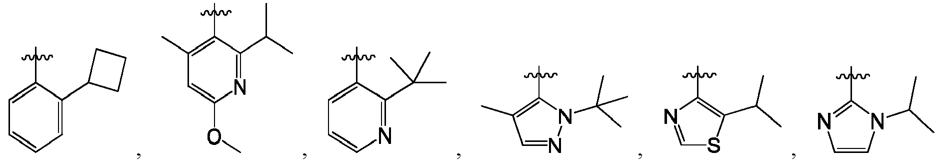 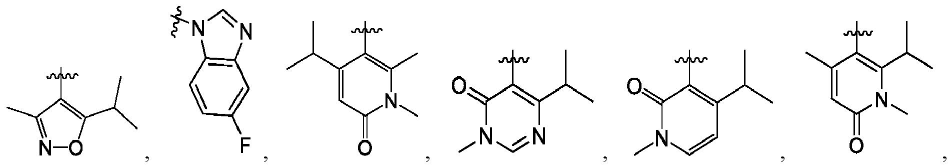 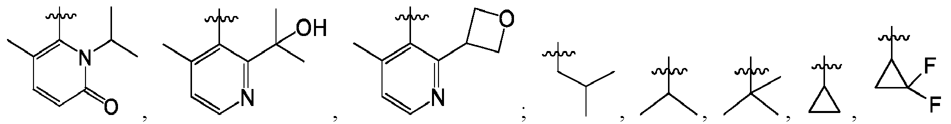 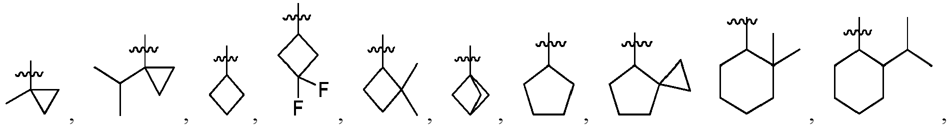 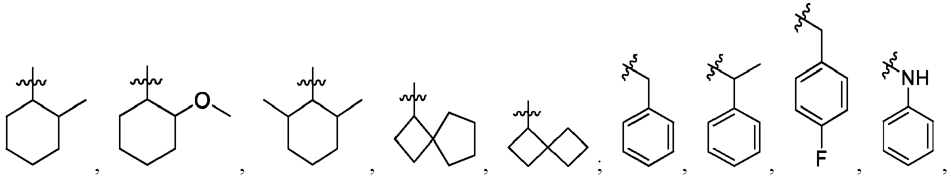 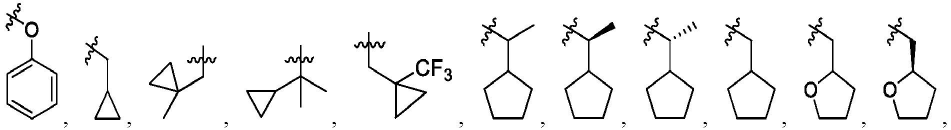 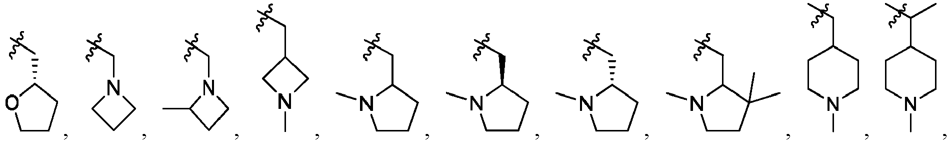 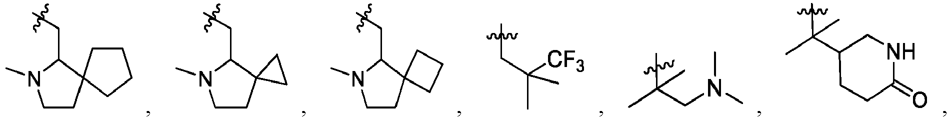 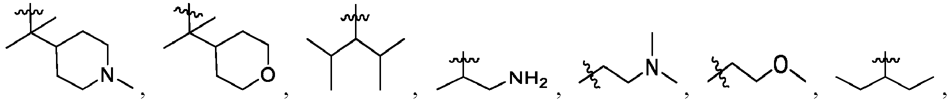 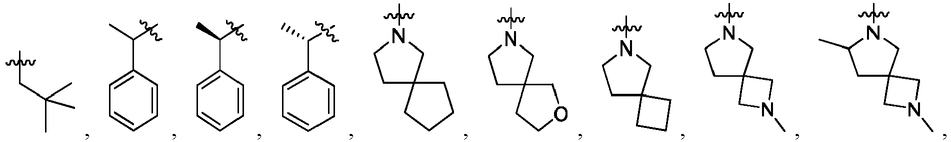 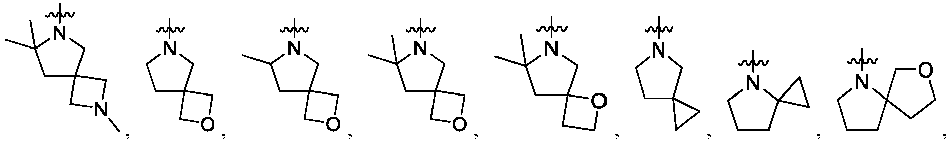 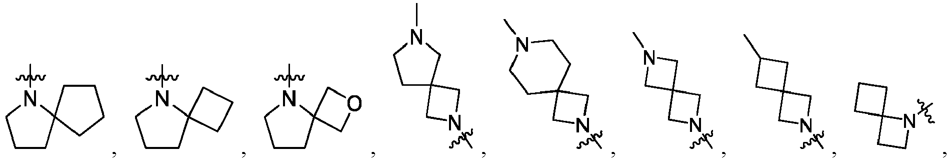 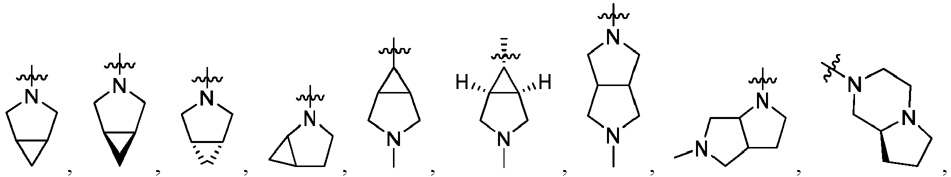 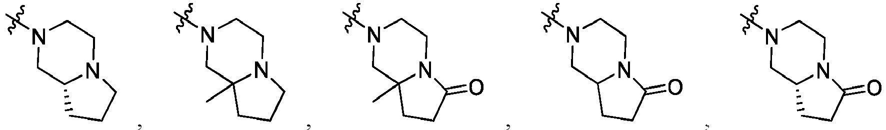 and

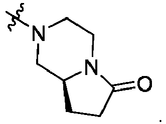

In an embodiment, R³ is selected from the following structures:

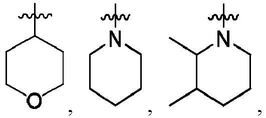

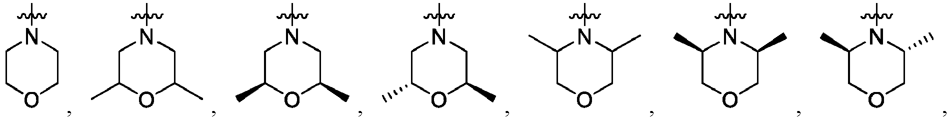

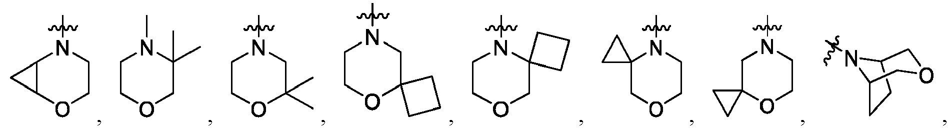

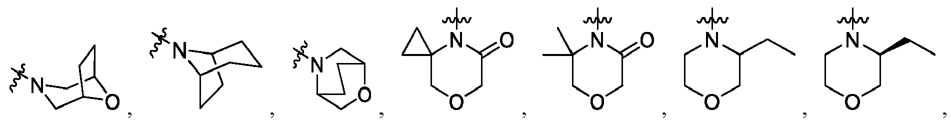

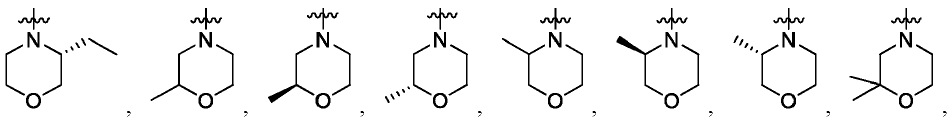

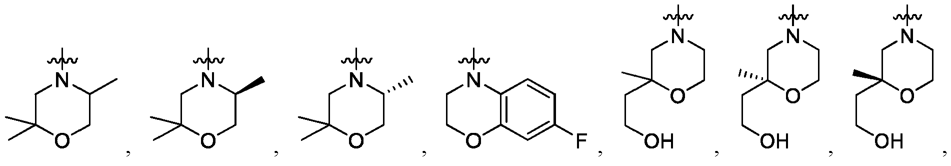

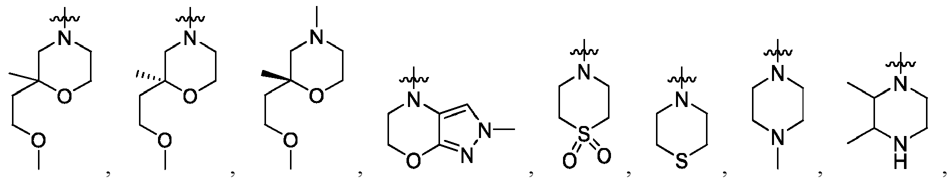

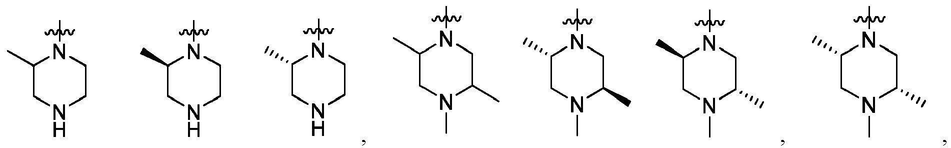

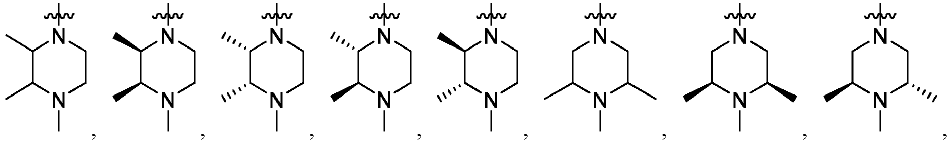

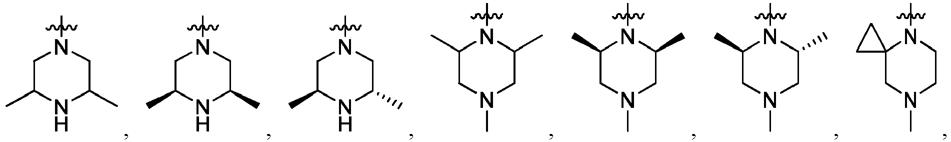

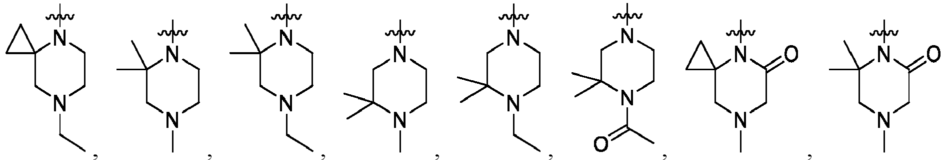

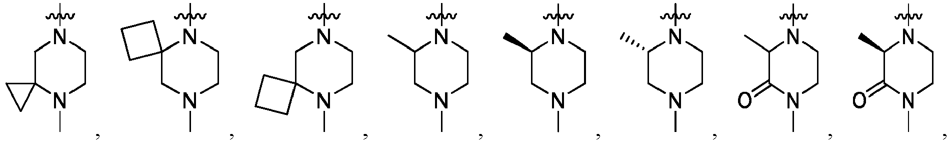

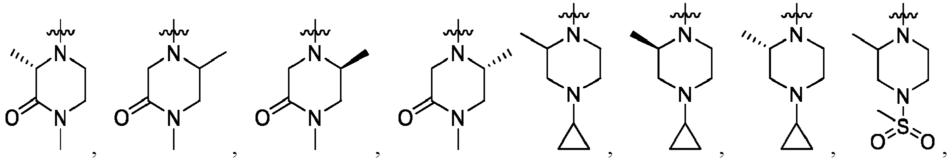

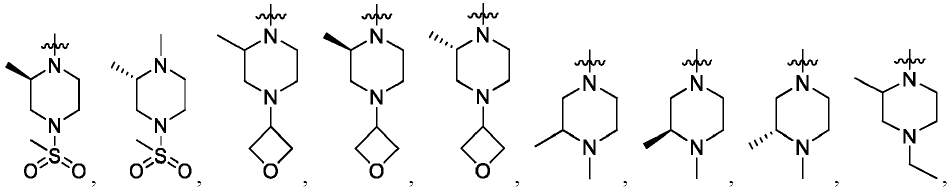

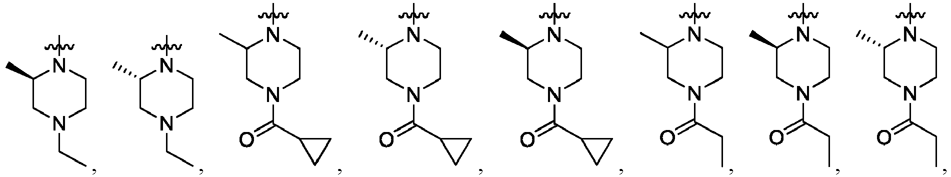

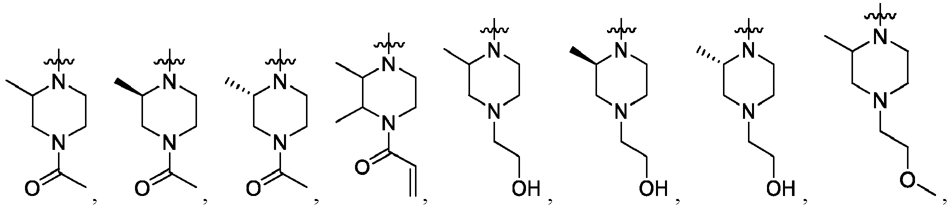

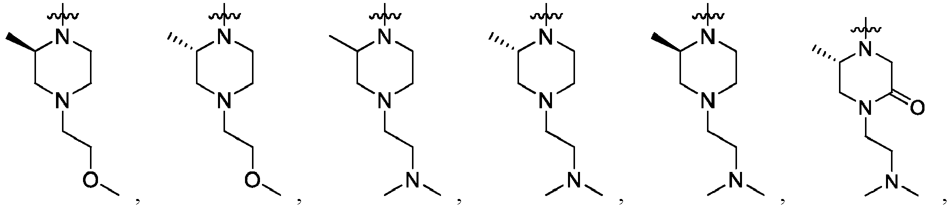

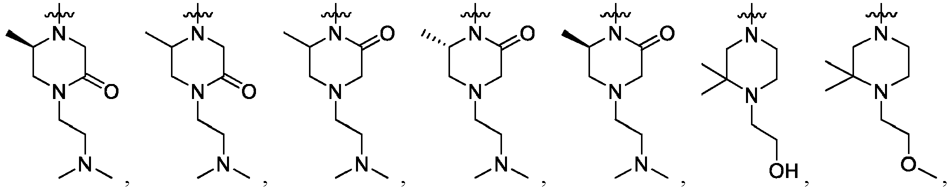

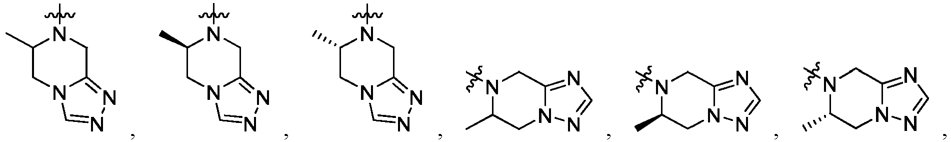

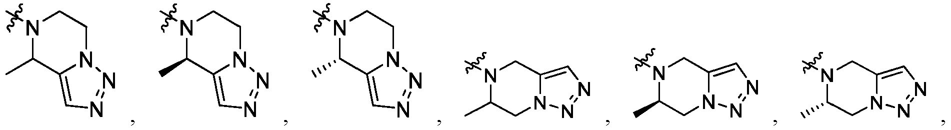

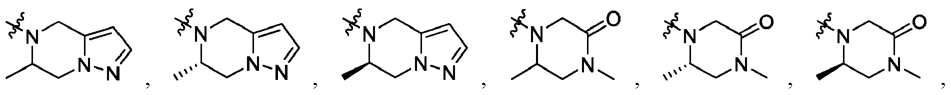

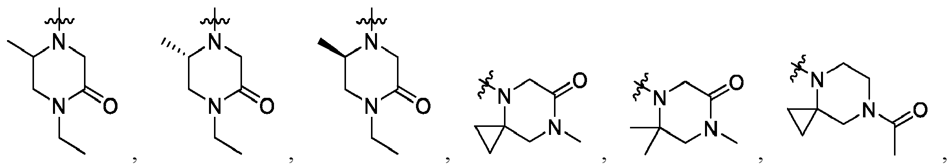

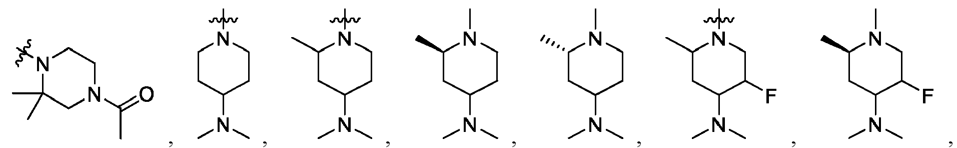

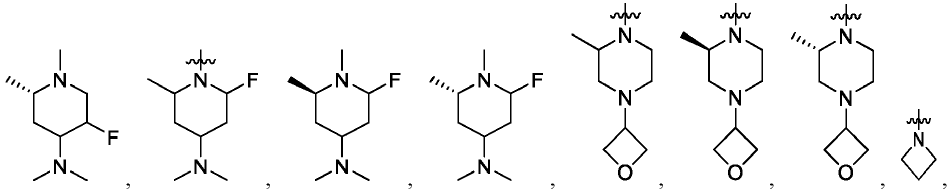

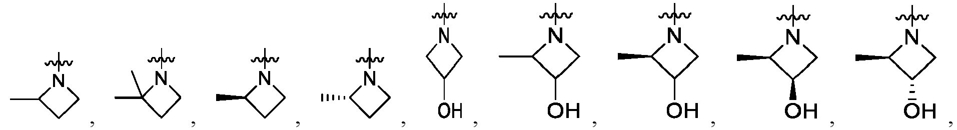

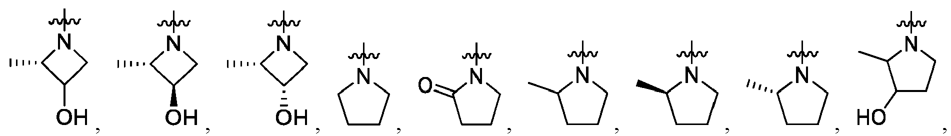

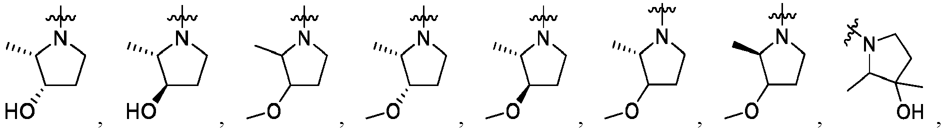

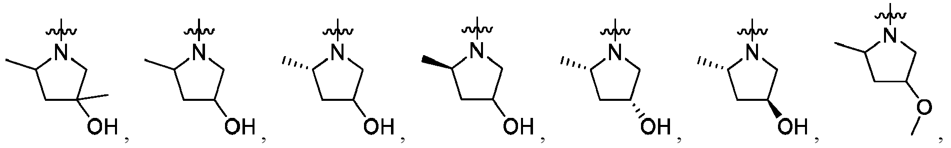

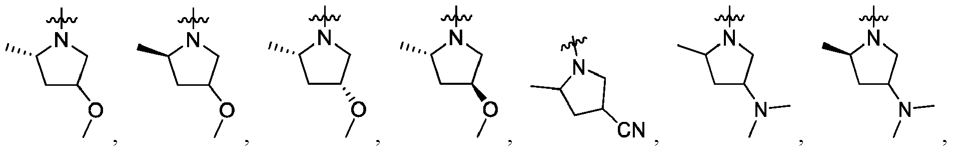

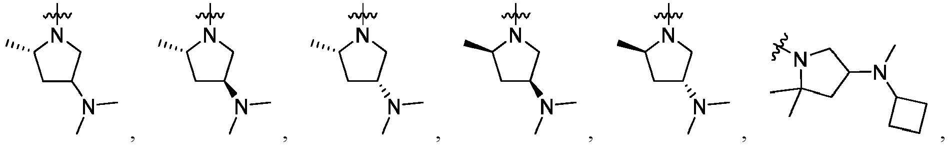

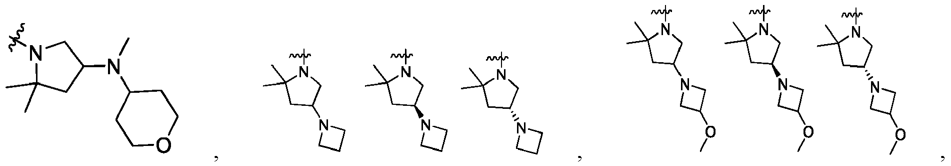

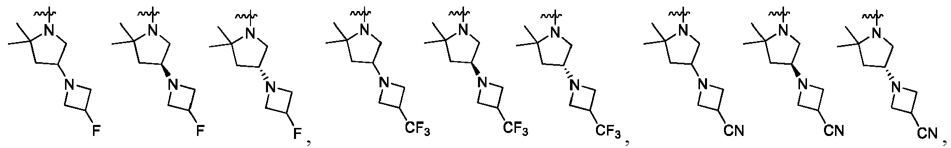

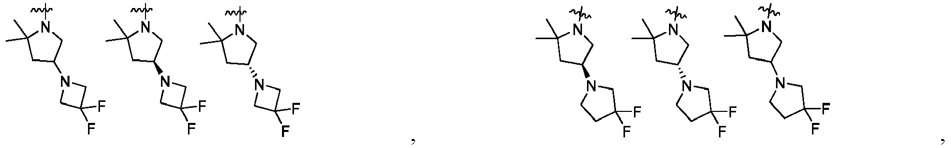

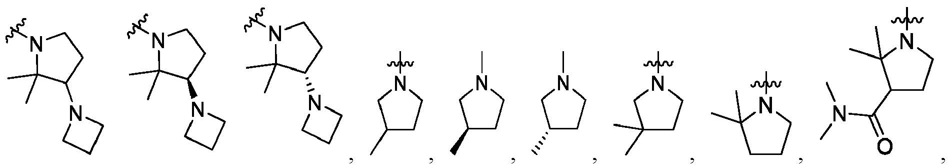

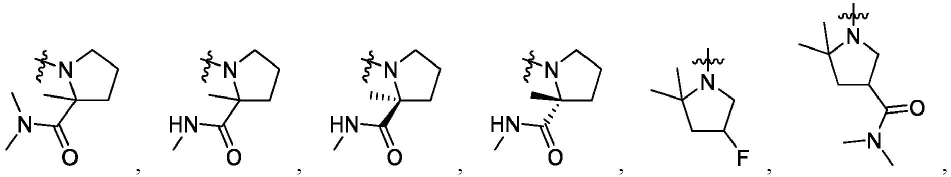

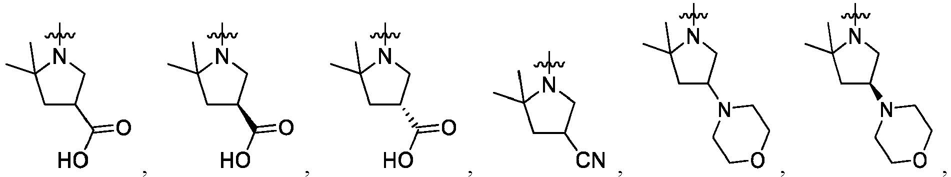

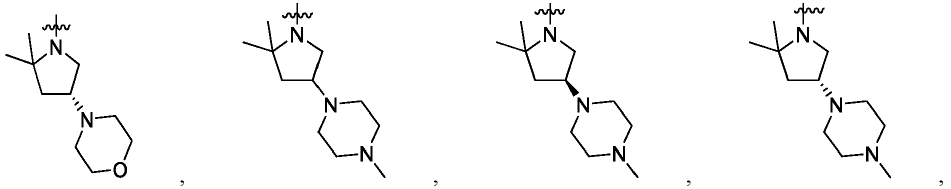

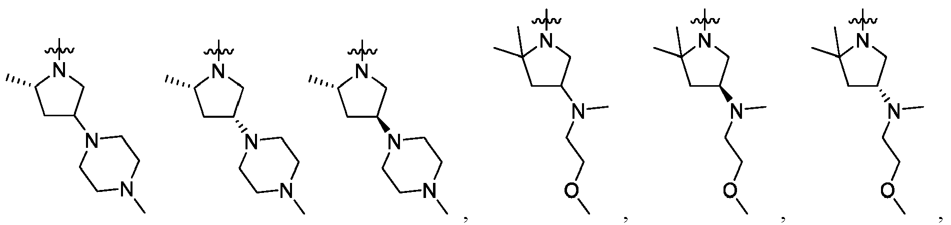

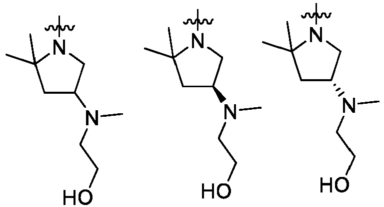

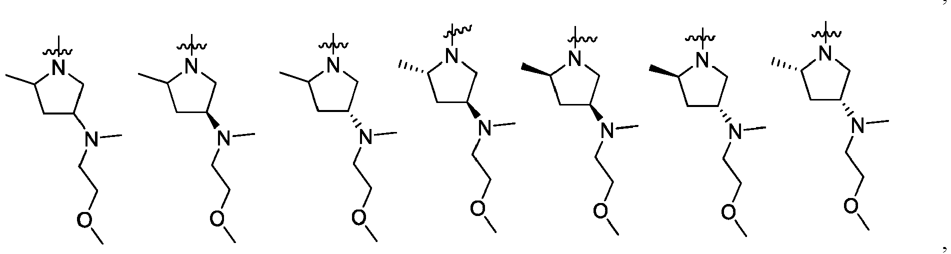

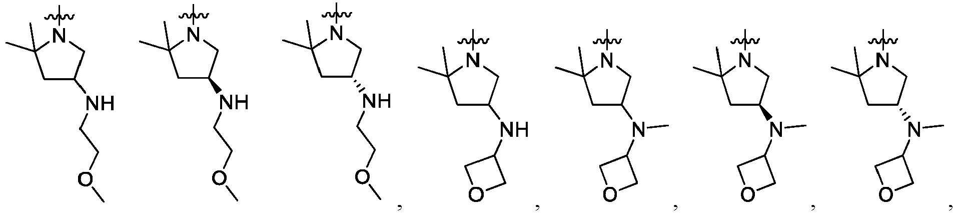

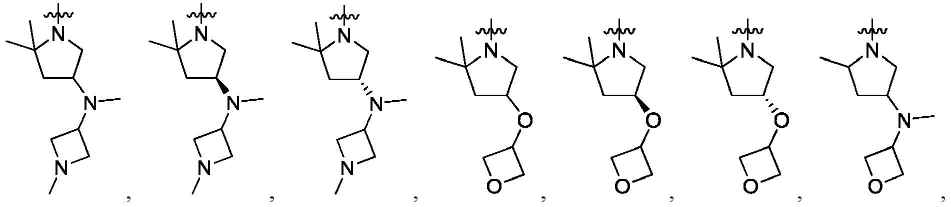

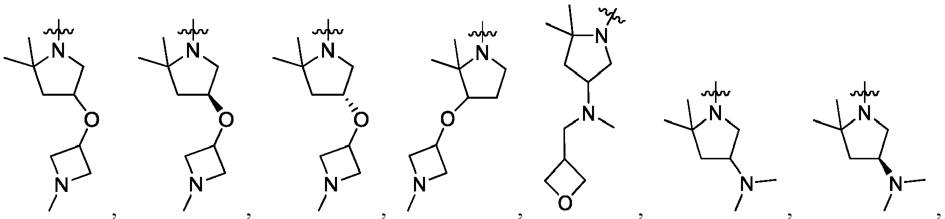

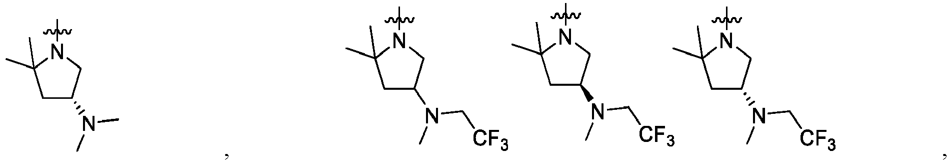

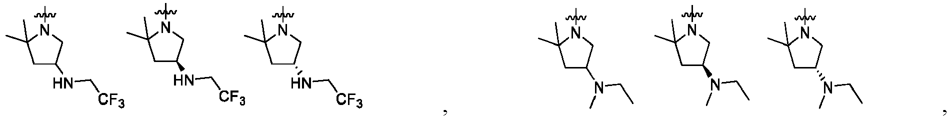

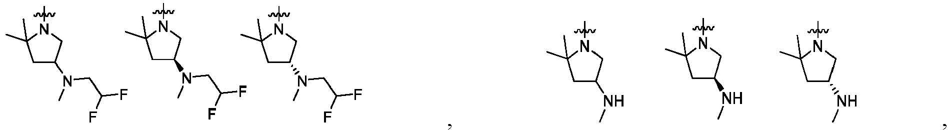

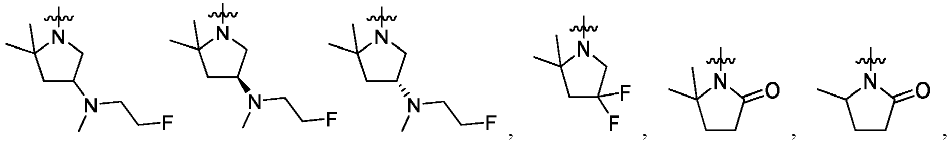

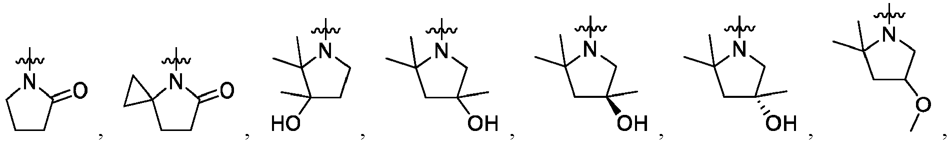

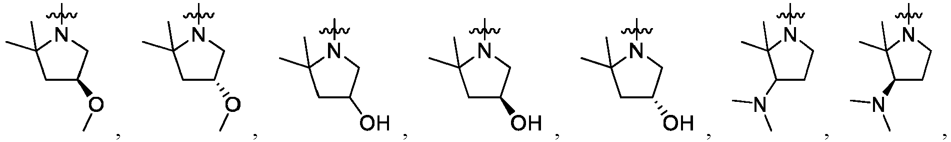

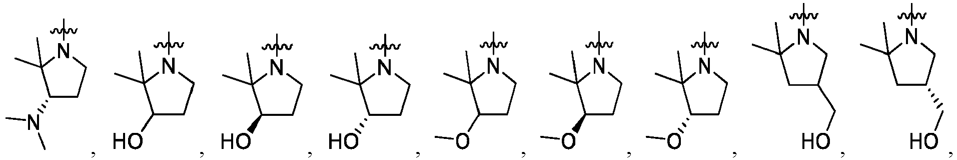

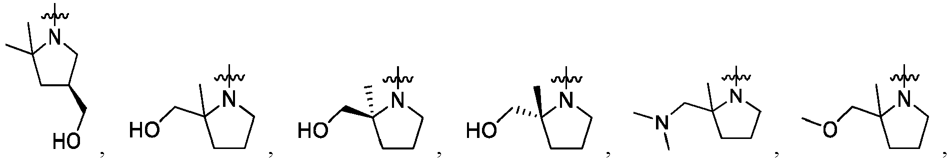

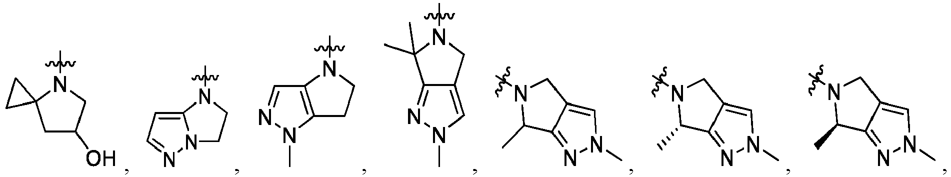

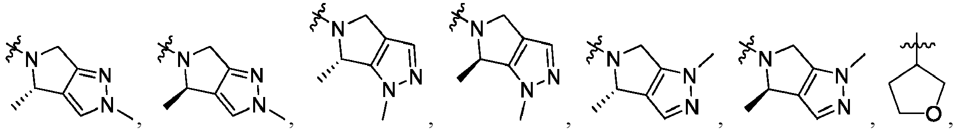

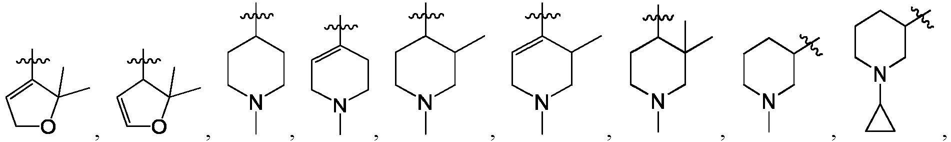

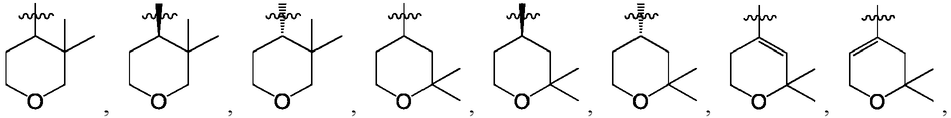

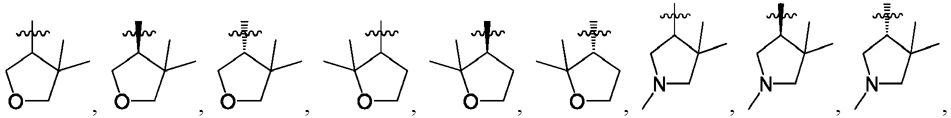

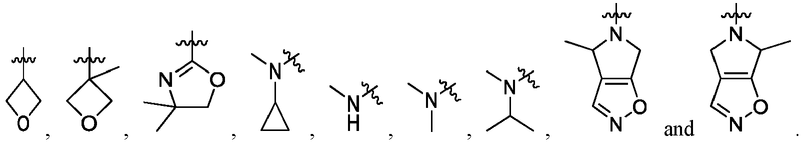

In an embodiment, R³ is selected from the following structures: H, NH₂, -CH₃, -C(CH₃)₃, - CH(CH₃)₂,

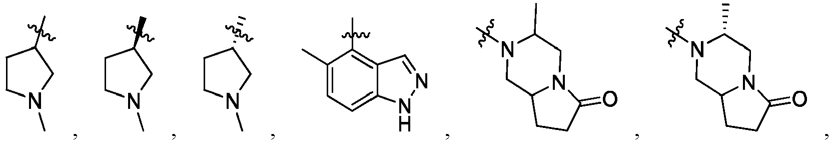

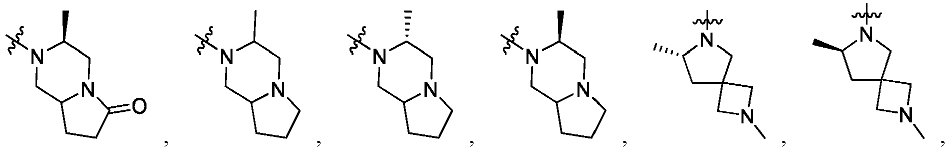

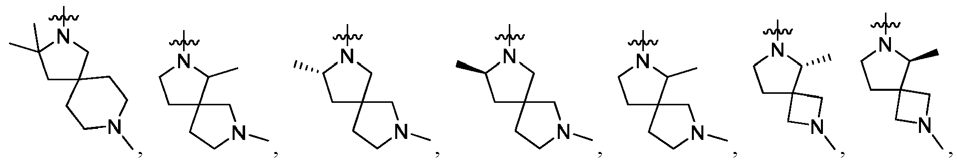

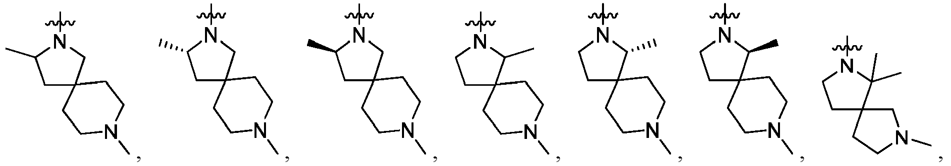

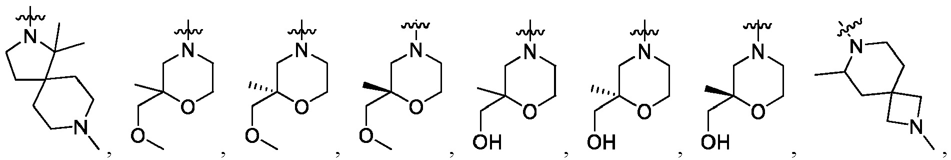

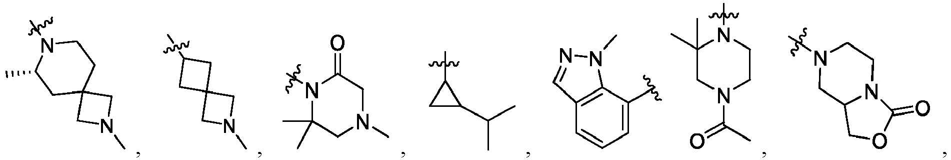

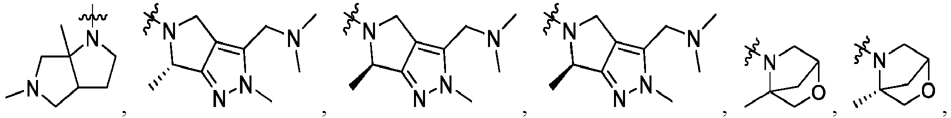

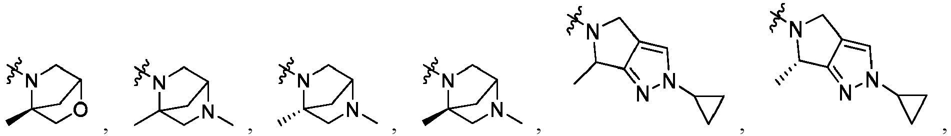

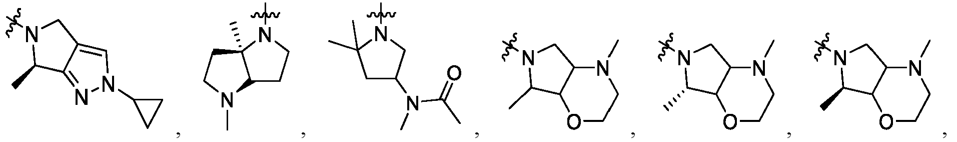

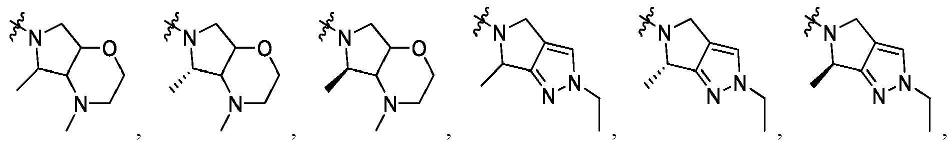

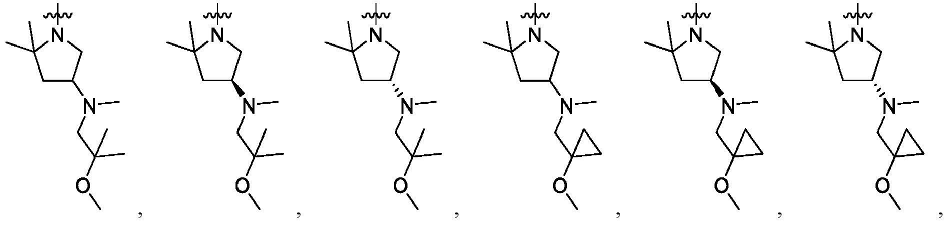

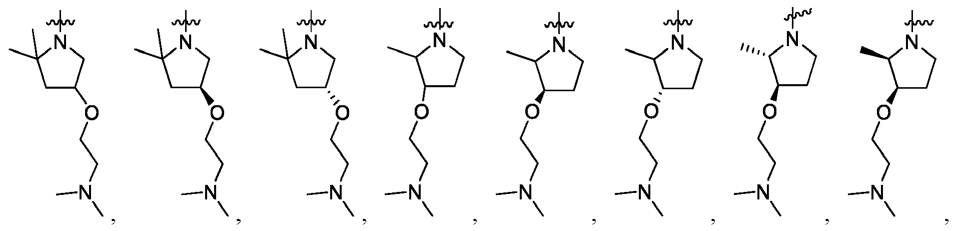

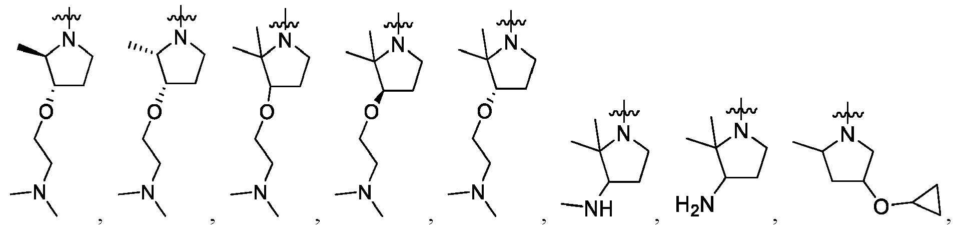

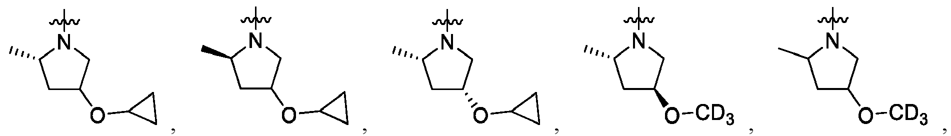

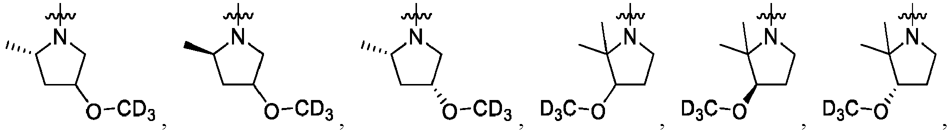

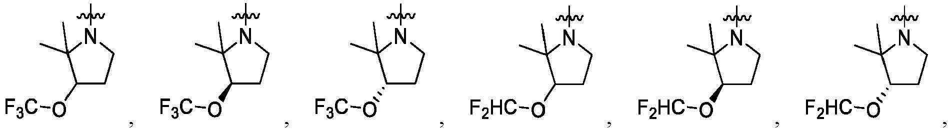

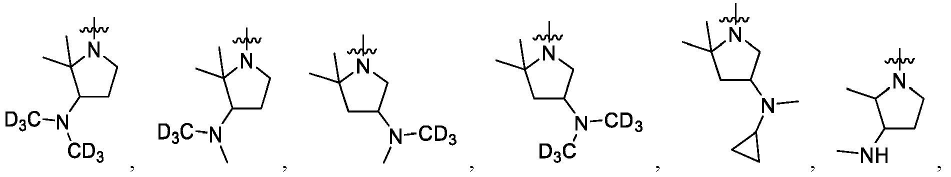

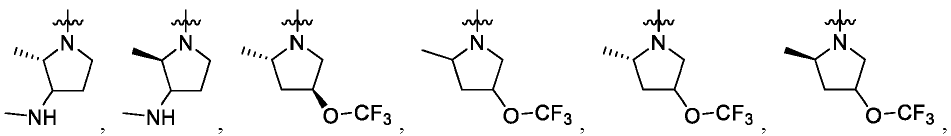

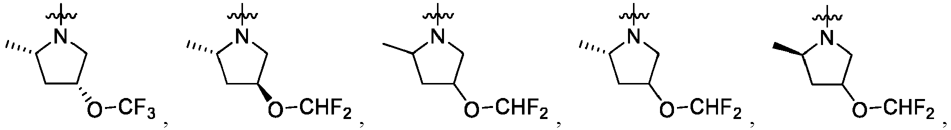

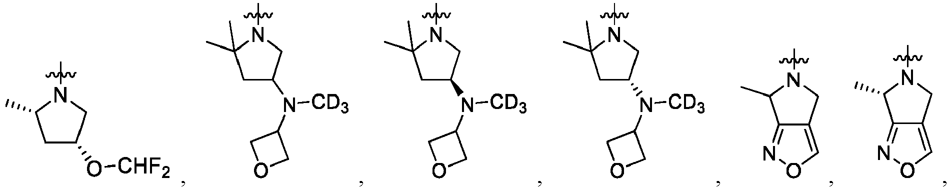

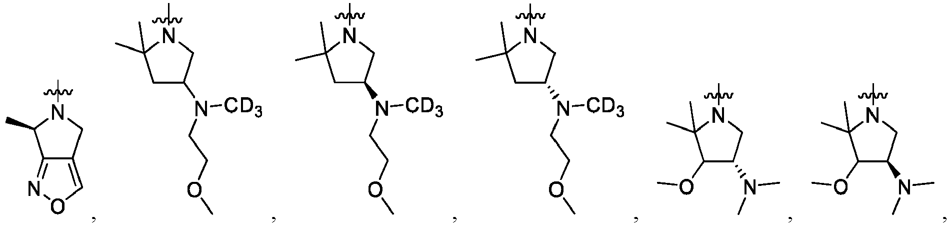

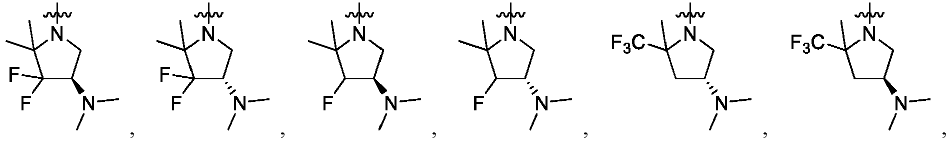

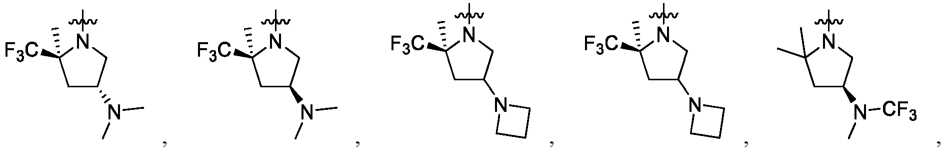

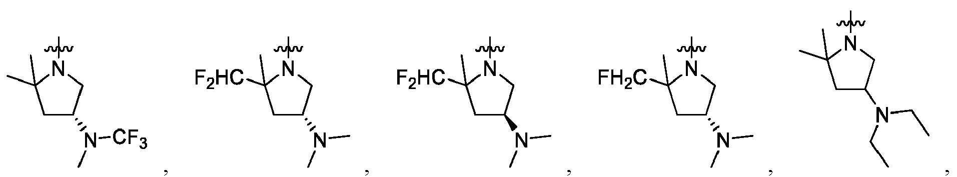

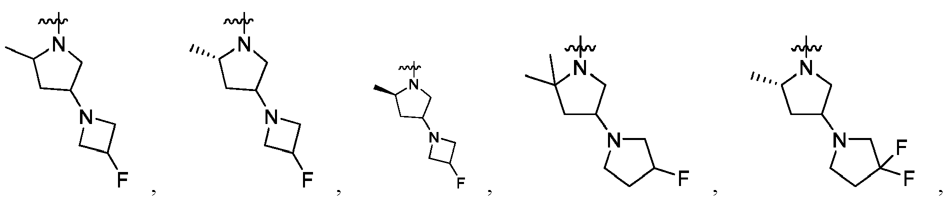

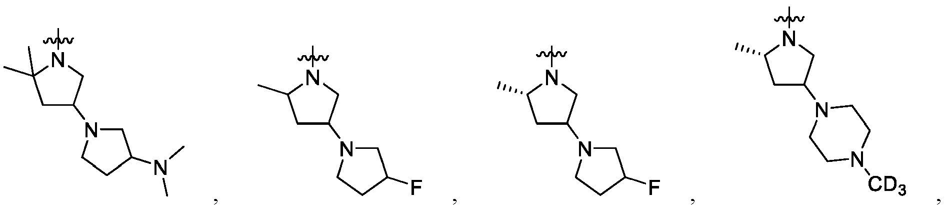

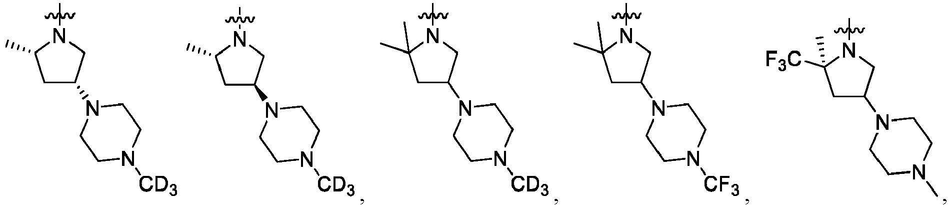

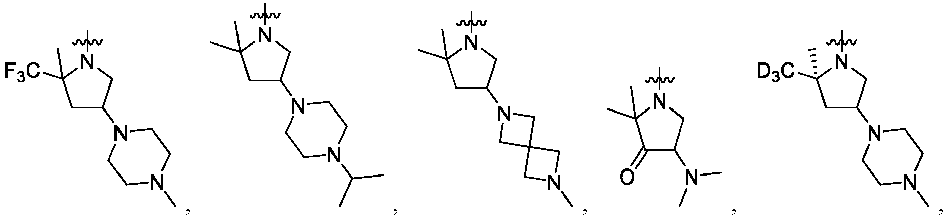

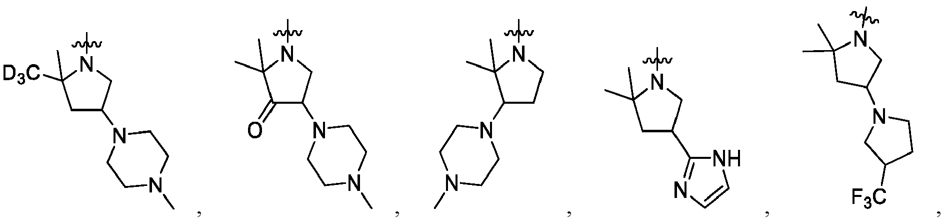

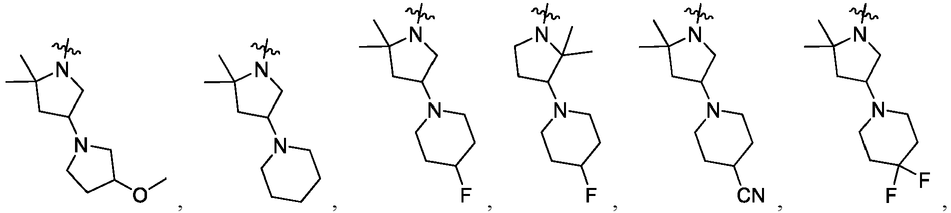

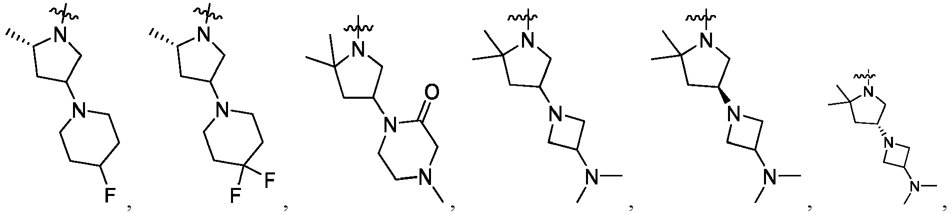

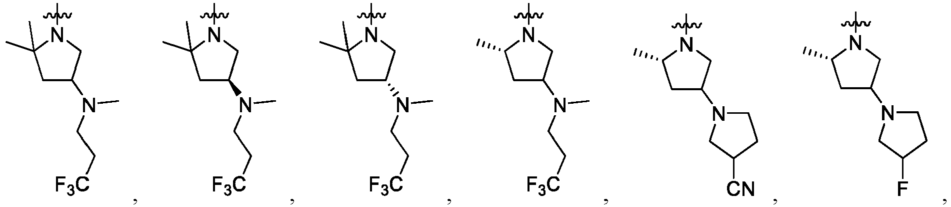

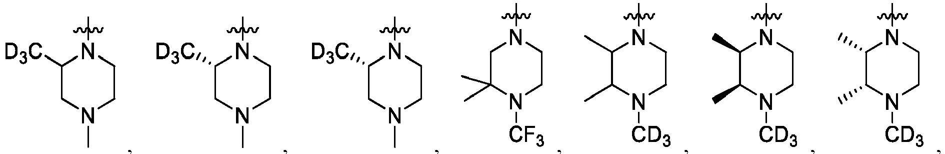

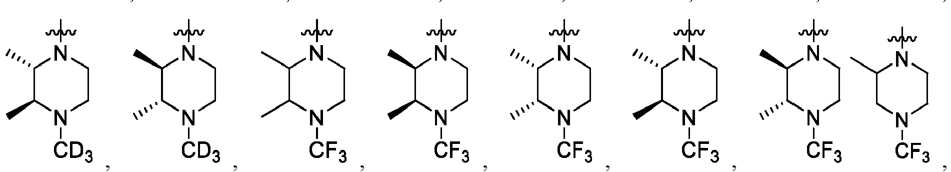

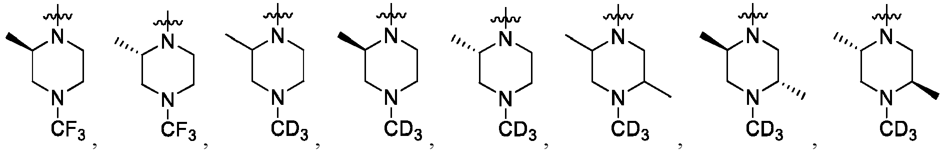

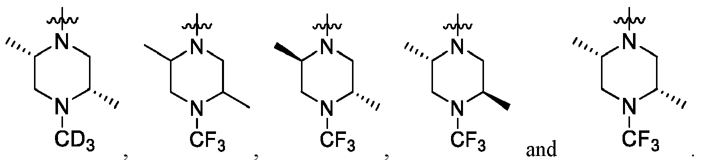

In an embodiment:
G¹ is N;
Y is -C(=O)-;
L² is NR^{L5}, R^{L5} is defined as above (R^{L5} is preferably H, C₁₋₆ alkyl or deuterated C₁₋₆ alkyl, e.g., H, -CH₃, isopropyl or -CD₃); and
R³ is C₁₋₈ alkyl (e.g., isopropyl or tert-butyl), C₆₋₁₄ aryl, 5- or 6-membered monocyclic heteroaryl, C₃₋₆ monocyclic cycloalkyl, 3- to 6-membered monocyclic heterocyclyl, 5- to 11-membered bridged cycloalkyl, -L⁴-C₆₋₁₄ aryl, -L⁴-3- to 6-membered monocyclic heterocyclyl or -L⁴-C₃₋₆ monocyclic cycloalkyl, and the C₆₋₁₄ aryl, 5- or 6-membered monocyclic heteroaryl, C₃₋₆ monocyclic cycloalkyl, 3- to 6-membered monocyclic heterocyclyl and 5- to 11-membered bridged cycloalkyl are independently unsubstituted or substituted by 1, 2, 3 or 4 substituents independently selected from group S1; the substituents of group S1 and L^{4f} are defined as above. Preferably, the substituents of group S1 are oxo, halogen, hydroxyl, C₁₋₆ alkyl, C₁₋₆ alkoxy or NR^{c}R^{d}, R^{c} and R^{d} are defined as above; L⁴ is preferably -CH₂-, -CHCH₃-, C(CH₃)₂ or C(CH₃)₂CH₂-; R^{c} and R^{d} are independently and preferably H or C₁₋₆ alkyl.

In an embodiment:
G¹ is N;
Y is -C(=O)-;
L² is a bond;
R³ is H, NR^{e}R^{f}, C₆₋₁₄ aryl, 5- or 6-membered monocyclic heteroaryl, 8- to 10-membered bicyclic heteroaryl, 3- to 6-membered monocyclic heterocyclyl, 5- to 11-membered fused heterocyclyl, 5- to 11-membered bridged heterocyclyl, 7- to 11-membered spiroheterocyclyl, -L⁴-C₆₋₁₄ aryl, -L⁴-3- to 6-membered monocyclic heterocyclyl, -L₄-7- to 11-membered spiroheterocyclyl; the C₆₋₁₄ aryl, 5- or 6-membered monocyclic heteroaryl, 8- to 10-membered bicyclic heteroaryl, 3- to 6-membered monocyclic heterocyclyl, 5- to 11-membered fused heterocyclyl, 5- to 11-membered bridged heterocyclyl and 7- to 11-membered spiroheterocyclyl are independently unsubstituted or substituted by 1, 2, 3 or 4 substituents independently selected from group S1; the substituents of group S1, L₄, R^{e} and R^{f} are defined as above.

In an embodiment:
G¹ is N;
Y is -C(=O)-;
L² is O;
R³ is C₁₋₈ alkyl (e.g., -CH₃), C₆₋₁₄ aryl, 8- to 10-membered bicyclic heteroaryl, 5- to 11-membered fused heterocyclyl, 3- to 6-membered monocyclic heterocyclyl, -L⁴-3- to 6-membered monocyclic heterocyclyl or -L⁴-NR^{e}R^{f}; the C₆₋₁₄ aryl, 8- to 10-membered bicyclic heteroaryl, 3- to 6-membered monocyclic heterocyclyl and 5- to 11-membered fused heterocyclyl are independently unsubstituted or substituted by 1, 2, 3 or 4 substituents independently selected from group S1; the substituents of group S1, L₄, R^{e} and R^{f} are defined as above. Preferably, the substituents of group S1 are C₁₋₆ alkyl; L₄ is CH₂ or CHCH₃; R^{e} and R^{f} are H or C₁₋₆ alkyl (e.g., -CH₃).

In an embodiment:
G¹ is N;
Y is -C(=O)-;
L² is NH(C=O);
R³ is C₁₋₈ alkyl (e.g., -CH₂(CH₃)₂, -CH₂C(CH₃)₃ or CH(CHCH₃CH₃)₂).

In an embodiment, R³ is 5- or 6-membered monocyclic heterocyclyl; wherein the 5- or 6-membered monocyclic heterocyclyl has 1 or 2 heteroatoms selected from N, O, S as ring atoms; the 5- or 6-membered monocyclic heterocyclyl is unsubstituted or substituted by 1, 2, 3 or 4 substituents independently selected from group S1; the 5- or 6-membered monocyclic heterocyclyl is saturated or partially unsaturated (e.g., having one double bond or two double bonds on the ring).

In an embodiment, L² is a bond; R³ is 3- to 6-membered monocyclic heterocyclyl; wherein the 3-to 6-membered monocyclic heterocyclyl has 1 or 2 heteroatoms selected from N, O, S as ring atoms; the 3-to 6-membered monocyclic heterocyclyl is unsubstituted or substituted by 1, 2, 3 or 4 substituents independently selected from group S1; the 3- to 6-membered monocyclic heterocyclyl is saturated or partially unsaturated (e.g., having one double bond or two double bonds on the ring).

In an embodiment, L² is a bond; R³ is 5- or 6-membered monocyclic heterocyclyl; wherein the 5or 6-membered monocyclic heterocyclyl has 1 or 2 heteroatoms selected from N, O as ring atoms; the 5- or 6-membered monocyclic heterocyclyl is unsubstituted or substituted by 1, 2, 3 or 4 substituents independently selected from group S1; the 5- or 6-membered monocyclic heterocyclyl is saturated or partially unsaturated (e.g., having one double bond or two double bonds on the ring).

In an embodiment, L² is a bond; R³ is 5- to 11-membered fused heterocyclyl, 7- to 11-membered spiroheterocyclyl or 5- to 11-membered bridged heterocyclyl; wherein the 5- to 11-membered fused heterocyclyl, 7- to 11-membered spiroheterocyclyl or 5- to 11-membered bridged heterocyclyl has 1, 2 or 3 heteroatoms selected from N, O, S as ring atoms; the 5- to 11-membered fused heterocyclyl, 7- to 11-membered spiroheterocyclyl or 5- to 11-membered bridged heterocyclyl are each independently unsubstituted or substituted by 1, 2, 3 or 4 substituents independently selected from group S1; the 5- or 6-membered monocyclic heterocyclyl is saturated or partially unsaturated (e.g., having one double bond or two double bonds on the ring).

In an embodiment, L² is NR^{L5}; wherein, R^{L5} is hydrogen, C₁₋₃ alkyl or deuterated C₁₋₃ alkyl; R₃ is C₁₋₆ alkyl, C₃₋₆ monocyclic cycloalkyl or 5- or 6-membered monocyclic heteroaryl; the 5- or 6-membered monocyclic heteroaryl has 1 or 2 heteroatoms selected from N, O and S as ring atoms; the C₃₋₆ monocyclic cycloalkyl, 5- or 6-membered monocyclic heteroaryl are each independently unsubstituted or substituted by 1, 2, 3 or 4 substituents independently selected from group S1.

In an embodiment, L² is O; R₃ is C₆₋₁₄ aryl; the C₆₋₁₄ aryl is unsubstituted or substituted by 1, 2, 3 or 4 substituents independently selected from group S1.

In an embodiment, the compound is shown in formula III-1-3: 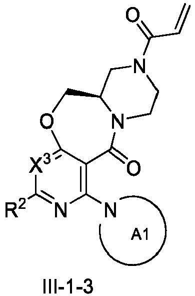
in the formula III-1-3,
R² is selected from the following structures: 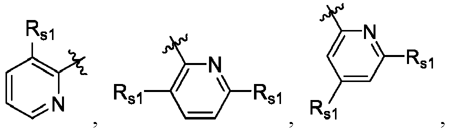 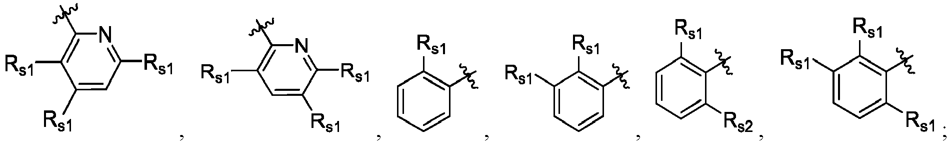 wherein Rₛ₁ of each position is the same or different; Rₛ₁, Rₛ₂ are the same or different; Rₛ₁, Rₛ₂ are each independently halogen, cyano, nitro, hydroxyl, -C₁₋₆ alkyl, -C₁₋₆ alkoxy, -halo C₁₋₆ alkyl, -deuterated C₁₋₆ alkyl, -halo C₁₋₆ alkoxy, -deuterated C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; R^{c}, R^{d} are defined as above;
X³ is CR¹; wherein, R¹ is chlorine or fluorine: 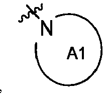 is 3- to 6-membered nitrogen-containing heterocyclyl and the 3- to 6-membered nitrogen-containing heterocyclyl is attached to the rest of the molecule via the nitrogen atom; the 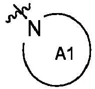 is unsubstituted or substituted by 1, 2, 3 or 4 substituents independently selected from group S1.

In an embodiment, R² is 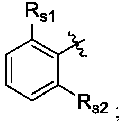 Rₛ₁ is fluorine; Rₛ₂ is hydroxyl; X³ is CR¹; wherein, R¹ is chlorine or fluorine; 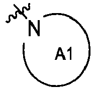 is 3- to 6-membered nitrogen-containing heterocyclyl and the 3- to 6-membered nitrogen-containing heterocyclyl is attached to the rest of the molecule via the nitrogen atom the 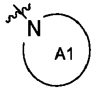 is unsubstituted or substituted by 1, 2, 3 or 4 substituents independently selected from group S1.

In an embodiment, 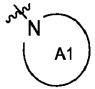 is azetidinyl, tetrahydropyrrolyl, hexahydropyridinyl, morpholinyl or piperazinyl, and the azetidinyl, tetrahydropyrrolyl, hexahydropyridinyl, morpholinyl or piperazinyl is attached to the rest of the molecule via the nitrogen atom; and the azetidinyl, tetrahydropyrrolyl, hexahydropyridinyl, morpholinyl or piperazinyl is each independently unsubstituted or substituted by 1, 2, 3 or 4 substituents independently selected from group S1.

In an embodiment, 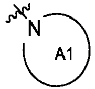 is azetidinyl, tetrahydropyrrolyl, hexahydropyridinyl, morpholinyl or piperazinyl, and the azetidinyl, tetrahydropyrrolyl, hexahydropyridinyl, morpholinyl or piperazinyl is attached to the rest of the molecule via the nitrogen atom and one hydrogen atom on the ortho carbon atom of the nitrogen atom is substituted by methyl; and the hydrogen atoms in the rest positions of the azetidinyl, tetrahydropyrrolyl, hexahydropyridinyl, morpholinyl or piperazinyl are each optionally and independently substituted by 1, 2 or 3 substituents independently selected from group S1.

In an embodiment, 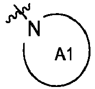 is azetidinyl, tetrahydropyrrolyl, hexahydropyridinyl, morpholinyl or piperazinyl, and the azetidinyl, tetrahydropyrrolyl, hexahydropyridinyl, morpholinyl or piperazinyl is attached to the rest of the molecule via the nitrogen atom and two hydrogen atoms on the same ortho carbon atom of the nitrogen atom are both substituted by methyl; and the hydrogen atoms in the rest positions of the azetidinyl, tetrahydropyrrolyl, hexahydropyridinyl, morpholinyl or piperazinyl are each optionally and independently substituted by 1 or 2 substituents independently selected from group S1.

In an embodiment, in the present disclosure, R³ or 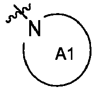 is each independently selected from the following groups: 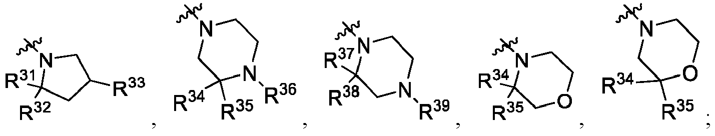 in each formula, R³¹, R³², R³³, each R³⁴, each R³⁵, R³⁶, R³⁷, R³⁸, R³⁹ are each independently selected from group S1. In an embodiment, the substituents of group S1 comprise H, C₁₋₆ alkyl, halo C₁₋₆ alkyl, deuterated C₁₋₆ alkyl, hydroxyl, NR^{c}R^{d}; wherein, R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by - CH₂CH₂- to form a cycloalkyl. In an embodiment, the substituents of group S1 comprise H, methyl, hydroxyl, NR^{c}R^{d}; wherein, R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3-to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by - CH₂CH₂- to form a cycloalkyl. In an embodiment, the substituents of group S1 comprise H, methyl, hydroxyl, NR^{c}R^{d}; wherein, R^{c}, R^{d} are each independently H, methyl, halomethyl (e.g., trifluoromethyl, difluoromethyl, monofluoromethyl), ethyl, deuterated ethyl, haloethyl (e.g., trifluoroethyl, difluoroethyl, monofluoroethyl); or R^{c}, R^{d} combining with the nitrogen atoms to which they are attached form a piperazinyl, N-methylpiperazinyl, azetidinyl or tetrahydropyrrolyl; and the piperazinyl, *N*-methylpiperazinyl, azetidinyl or tetrahydropyrrolyl is optionally substituted by 1 or 2 substituents selected from the following groups: methyl, deuterated methyl, halomethyl, ethyl, deuterated ethyl, haloethyl.

In an embodiment, 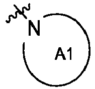 is morpholinyl or piperazinyl, and the morpholinyl or piperazinyl is attached to the rest of the molecule via the nitrogen atom and two hydrogen atoms on the same meta carbon atom of the nitrogen atom are both substituted by methyl; and the hydrogen atoms in the rest positions of the morpholinyl or piperazinyl are each optionally and independently substituted by 1 or 2 substituents independently selected from group S1.

In an embodiment, the substituents of group S1 are each independently oxo (=O), halogen, cyano, nitro, hydroxyl, carboxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7-to 11-membered spiroheterocyclyl, 5- to 11-membered bridged cycloalkyl, 5- to 11-membered bridged heterocyclyl, C₆₋₁₄ aryl, 5- or 6-membered monocyclic heteroaryl, C₃₋₆ monocyclic cycloalkoxy, -O-3- to 6-membered monocyclic heterocyclyl, -O-5- to 11-membered fused cycloalkyl, -O-5- to 11-membered fused heterocyclyl, -O-6- to 11-membered spirocycloalkyl, -O-7- to 11-membered spiroheterocyclyl, -O-5- to 11-membered bridged cycloalkyl, -O-5- to 11-membered bridged heterocyclyl, -O-C₆₋₁₄ aryl, -O-5- or 6-membered monocyclic heteroaryl, NR^{c}R^{d}, C(O)C₁₋₆ alkyl, C(O)C₂₋₆ alkenyl, C(O)C₃₋₆ monocyclic cycloalkyl, C(O)NR^{c}R^{d}, -SO₂C₁₋₃ alkyl, -SOzhalo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, -C₁₋₄ alkyl-3- to 6-membered monocyclic heterocyclyl, -C₁₋₄ alkyl-NR^{c}R^{d}, -O-C₁₋₄ alkyl-NR^{c}R^{d}, -C₁₋₄ alkyl-C(O)NR^{c}R^{d}, -C₁₋₄ alkyl-SO₂C₁₋₃ alkyl, carboxyl, -C₁₋₄ alkyl-carboxyl, C₂₋₆ alkenyl or C₂₋₆ alkynyl; the 3- to 6-membered monocyclic heterocyclyl or 5- or 6-membered monocyclic heteroaryl has 1 or 2 heteroatoms selected from N, O and S as ring atoms; the 3- to 6-membered monocyclic heterocyclyl or 5- or 6-membered monocyclic heteroaryl is optionally substituted by 1 or 2 groups selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl;
the R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3-to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by - CH₂CH₂- to form a cycloalkyl.

In an embodiment, the substituents of group S1 are each independently NR^{c}R^{d}; the R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on - C₁₋₄ alkyl- are optionally substituted by C₁₋₆ alkyl or two hydrogen atoms on the same carbon on -C₁₋₄ alkylare simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5-to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl.

In an embodiment, the substituents of group S1 are each independently selected from the following groups: methyl, hydroxyl and NR^{c}R^{d}; the R^{c}, R^{d} are each independently H, C₁₋₆ alkyl, halo C₁₋₆ alkyl or deuterated C₁₋₆ alkyl; or R^{c}, R^{d} combining with the nitrogen atoms to which they are attached form a piperazine; the piperazine is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; wherein, R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl.

In an embodiment, the substituents of group S1 are each independently selected from: oxo (=O), COOH, fluorine, hydroxyl, cyano, methyl, ethyl, isopropyl, methoxy, ethoxy, isopropoxy, CHF₂, CH₂F, CF₃, deuterated methyl, deuterated methoxy, OCHF₂, OCH₂F, OCF₃, cyclopropyl, cyclobutyl, cyclopropoxy, cyclobutoxy, oxiranyl, aziridinyl, oxetanyl, azetidinyl, tetrahydrofuranyl, tetrahydropyrrolyl, piperazinyl, N-methyl-piperazinyl, C(O)CH₃, C(O)CH₂CH₃, C(O)CH₂CH₂CH₃, C(O)CH=CH₂, C(O)-cyclopropane, - SO₂CH₃, -SO₂CH₂CH₃, -SO₂CH₂CH₂CH₃, NH₂, NH(CH)₃, N(CH₃)₂, NH(CH₂CH₃), NH(CH₂CF₃), NH(CH₂CHF₂), NH(CH₂CH₂F), N(CH₂CH₃)₂, N(CH₂CH₃)(CH₃), N(CH₂CF₃)(CH₃), N(CH₂CHF₂)(CH₃), N(CH₂CH₂F)(CH₃), N(CH₂CH₂CH₃)(CH₃), N(CH₂CH₂CH₂CH₃)(CH₃), N(CH₃)(CH₂CH₂OH), N(CH₃)(CH₂CH₂OCH₃), N(CH₃)(CH₂CH₂CH₂OCH₃), N(CH₃)(CH₂CH₂CH₂CH₂OCH₃), N(CH₃)(CH₂C(CH₃)₂OCH₃), N(CH₃)(CH₂C(CH₂)₂OCH₃), N(CD₃)(CH₂C(CH₂)₂OCH₃), NH(CH₂CH₂OH), NH(CH₂CH₂OCH₃), N(cyclobutane)(CH₃), N(azetidine)(CH₃), N(1-methylazetidine)(CH₃), NH(oxetane), N(oxetane)(CH₃), N(oxacycloheptane)(CH₃), N(CH₂-azetidine)(CH₃), N(CH₂(1-methylazetidine))(CH₃), NH(CH₂-oxetane), N(CH₂-oxetane)(CH₃), N(oxetane)(CD₃), N(CD₃)₂, N(CH₃)(CD₃), N(C(O) CH₃)(CH₃), CH₂N(CH₃)₂, CH₂CH₂N(CH₃)₂, -OCH₂CH₂(CH₃)₂, -OCH₂CH₂CH₂(CH₃)₂, -OCH₂CH₂CH₂CH₂(CH₃)₂, -O-oxetane, -O-azetidine, -O-(1-methylazetidine), morpholinyl, CH₂OH, CH₂CH₂OH, CH₂OCH₃, CH₂CH₂OCH₃, -OCH₂CH₂N(CH₃)₂, -CH₂CH₂N(CH₃)₂, -CH₂N(CH₃)₂.

In an embodiment, the substituents of each group S1 are each independently oxo (=O), halogen, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C₃₋₆ monocyclic cycloalkyl, 3- to 6-membered monocyclic heterocyclyl, C₃₋₆ monocyclic cycloalkoxy, -O-3- to 6-membered monocyclic heterocyclyl, NR^{c}R^{d}, C(O) C₁₋₆ alkyl, C(O) C₂₋₆ alkenyl, C(O) C₃₋₆ monocyclic cycloalkyl, C(O)NR^{c}R^{d}, -SO₂C₁₋₃ alkyl, -SOzhalo C₁₋₃ alkyl, -SO₂NR^{c}R^{d}, - C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered monocyclic heterocyclyl, -C₁₋₄ alkyl-NR^{c}R^{d}, -O-C₁₋₄ alkyl-NR^{c}R^{d}, -C₁₋₄ alkyl-C(O)NR^{c}R^{d}, -C₁₋₄ alkyl-SO₂C₁₋₃ alkyl, carboxyl, -C₁₋₄ alkyl-carboxyl, C₂₋₆ alkenyl or C₂₋₆ alkynyl; the 3- to 6-membered monocyclic heterocyclyl has 1 or 2 heteroatoms selected from N, O and S as ring atoms; the 3- to 6-membered monocyclic heterocyclyl is optionally substituted by 1 or 2 groups selected from the following groups: C₁₋₆ alkyl.

In an embodiment, the substituents of group S1 are each independently oxo (=O), halogen, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, C₃₋₆ monocyclic cycloalkyl, 3- to 6-membered monocyclic heterocyclyl, C₃₋₆ monocyclic cycloalkoxy, NR^{c}R^{d}, C(O) C₁₋₆ alkyl, C(O)NR^{c}R^{d}, -SO₂C₁₋₃ alkyl, -SOzhalo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl, C₂₋₆ alkenyl or C₂₋₆ alkynyl.

In an embodiment, the R^{c}, R^{d} are each independently H, C₁₋₆ alkyl, deuterated C₁₋₆ alkyl, C₃₋₆ monocyclic cycloalkyl, -C₁₋₄ alkyl-C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms.

In an embodiment, the R^{c}, R^{d} are each independently H, C₁₋₆ alkyl or -C₁₋₄ alkyl-C₁₋₆ alkoxy or R^{c}, R^{d} combining with the nitrogen atoms to which they are attached form a 3- to 6-membered nitrogen-containing heterocyclyl.

In an embodiment, 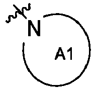 is selected from the following groups:

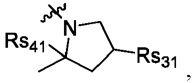

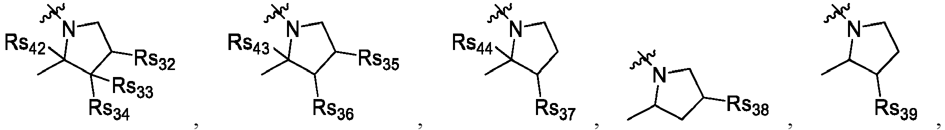

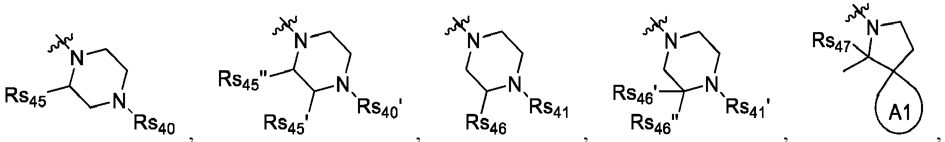

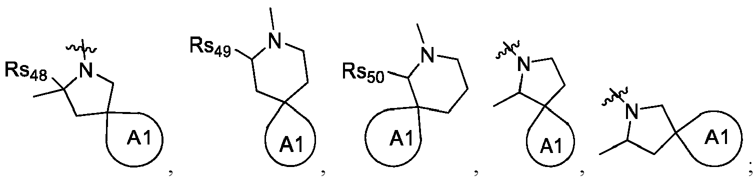

in each formula, Rₛ₄₁, Rₛ₄₂, Rₛ₄₃, Rₛ₄₄, Rₛ₄₅, R_{s45'}, R_{s45"}, Rₛ₄₆, R_{s46'}, R_{s46"}, Rₛ₄₇, Rₛ₄₈, Rₛ₄₉, Rₛ₅₀ are each independently selected from the following groups: hydrogen, C₁₋₆ alkyl, halo C₁₋₆ alkyl, deuterated C₁₋₆ alkyl; Rₛ₃₃, Rₛ₃₄, Rₛ₃₆ are each independently selected from the following groups: hydrogen, oxo (=O), halogen, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy; Rₛ₃₁, Rₛ₃₂, Rₛ₃₅, Rₛ₃₇, Rₛ₃₈, Rₛ₃₉, Rₛ₄₀, R_{s40'}, Rₛ₄₁, R_{s41'} are each independently selected from the following groups: hydrogen, halogen, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-NR^{c}R^{d}, C(O)C₁₋₆ alkyl, 5- or 6-membered monocyclic heteroaryl, NR^{c}R^{d}; the R^{c}, R^{d} are each independently C₁₋₆ alkyl, halo C₁₋₆ alkyl, deuterated C₁₋₆ alkyl or C₃₋₆ monocyclic cycloalkyl; or R^{c}, R^{d} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms and the 3- to 6-membered nitrogen-containing heterocyclyl is optionally substituted by 1 or 2 substituents selected from the following groups: oxo (=O), C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, N(C₁₋₆ alkyl)₂, N(deuterated C₁₋₆ alkyl)₂, N(deuterated C₁₋₆ alkyl)(C₁₋₆ alkyl), halogen; 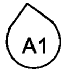 is 4- to 6-membered heterocyclyl, the 4- to 6-membered heterocyclyl has 1 or 2 heteroatoms selected from N, O and S as ring atoms and is optionally substituted by 1 or 2 substituents selected from the following groups: C₁₋₆ alkyl, halogen.

In an embodiment, Rₛ₄₁, Rₛ₄₂, Rₛ₄₃, Rₛ₄₄, Rₛ₄₅, R_{s45'}, R_{s45"}, Rₛ₄₆, R_{s46'}, R_{s46"}, Rₛ₄₇, Rₛ₄₈, Rₛ₄₉, Rₛ₅₀ are each independently selected from the following groups: methyl, deuterated methyl or fluoromethyl.

In an embodiment, 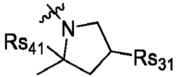 is

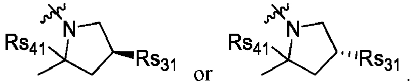

In an embodiment, 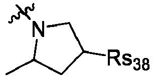 is

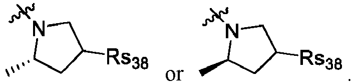

In an embodiment, 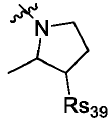 is

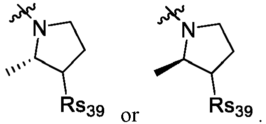

In an embodiment, 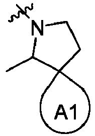 is

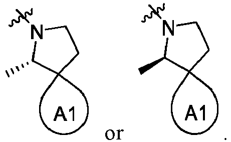

In an embodiment, 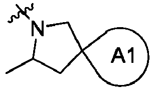 is

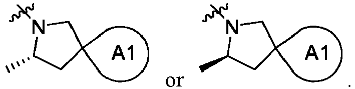

In an embodiment, the compound is shown in formula (III-1-4): 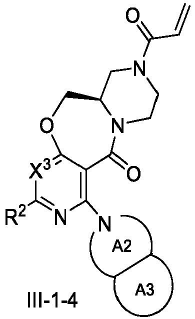 in the formula (III-1-4), R² is 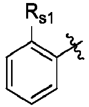 or; Rₛ₁ is fluorine or chlorine; Rₛ₂ is hydroxyl; X³ is CR¹; wherein, R¹ is chlorine or fluorine; 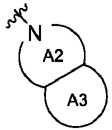 is 8- to 10-membered heteroaryl-heterocyclyl and the 8- to 10-membered heteroaryl-heterocyclyl is attached to the rest of the molecule via the nitrogen atom in ring A2, wherein, ring A2 is 5- or 6-membered nitrogen-containing heterocyclyl, ring A3 is 5- or 6-membered monocyclic heteroaryl; the 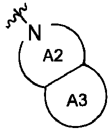 is unsubstituted or substituted by 1, 2, 3 or 4 substituents independently selected from group S1.

In an embodiment, 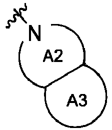 is 8-membered fused bicyclic heteroaryl formed by the 5-membered monocyclic heteroaryl ring fused with one 5-membered monocyclic heterocyclyl ring, or 9-membered fused bicyclic heteroaryl formed by the 5-membered monocyclic heteroaryl ring fused with one 6-membered monocyclic heterocyclyl ring; and 8-membered fused bicyclic heteroaryl formed by the 5-membered monocyclic heteroaryl ring fused with one 5-membered monocyclic heterocyclyl ring is attached to the rest of the molecule via the nitrogen atom in 5-membered monocyclic heterocyclyl ring; 9-membered fused bicyclic heteroaryl formed by the 5-membered monocyclic heteroaryl ring fused with one 6-membered monocyclic heterocyclyl ring is attached to the rest of the molecule via the nitrogen atom in 6-membered monocyclic heterocyclyl ring; and 8-membered fused bicyclic heteroaryl formed by the 5-membered monocyclic heteroaryl ring fused with one 5-membered monocyclic heterocyclyl ring, or 9-membered fused bicyclic heteroaryl formed by the 5-membered monocyclic heteroaryl ring fused with one 6-membered monocyclic heterocyclyl ring is each independently unsubstituted or substituted by 1, 2, 3 or 4 substituents independently selected from group S1.

In an embodiment, 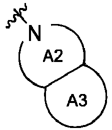 is tetrahydropyrrolopyrazolyl or piperazinotriazole; and the tetrahydropyrrolopyrazolyl is attached to the rest of the molecule via the nitrogen atom in tetrahydropyrrole ring; the piperazinotriazole is attached to the rest of the molecule via the nitrogen atom in piperazine ring; and the tetrahydropyrrolopyrazolyl or piperazinotriazole is unsubstituted or substituted by 1, 2, 3 or 4 substituents independently selected from group S1.

In an embodiment, 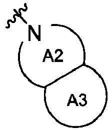 is tetrahydropyrrolopyrazolyl or piperazinotriazole; the tetrahydropyrrolopyrazolyl is attached to the rest of the molecule via the nitrogen atom in tetrahydropyrrole ring and one hydrogen atom on the ortho carbon atom of the nitrogen atom is substituted by methyl; the piperazinotriazole is attached to the rest of the molecule via the nitrogen atom in piperazine ring and one hydrogen atom on the ortho carbon atom of the nitrogen atom is substituted by methyl; and the hydrogen atoms in the rest positions of the tetrahydropyrrolopyrazolyl or piperazinotriazole are optionally substituted by 1, 2 or 3 substituents independently selected from group S1.

In an embodiment, 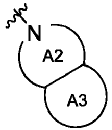 is tetrahydropyrrolopyrazolyl or piperazinotriazole; the tetrahydropyrrolopyrazolyl is attached to the rest of the molecule via the nitrogen atom in tetrahydropyrrole ring and two hydrogen atoms on the same ortho carbon atom of the nitrogen atom are both substituted by methyl; the piperazinotriazole is attached to the rest of the molecule via the nitrogen atom in piperazine ring and two hydrogen atoms on the same ortho carbon atom of the nitrogen atom are both substituted by methyl; and the hydrogen atoms in the rest positions of the tetrahydropyrrolopyrazolyl or piperazinotriazole are optionally substituted by 1 or 2 substituents independently selected from group S1.

In an embodiment, Rₛ₁, Rₛ₂ are each independently halogen, cyano, nitro, hydroxyl, -C₁₋₃ alkyl, - C₁₋₃ alkoxy, -halo C₁₋₃ alkyl, -halo C₁₋₃ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₂ alkyl-hydroxyl, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy, -C₁₋₂ alkyl-3- to 6-membered heterocyclyl, -C₁₋₂ alkyl-NR^{c}R^{d}, -C₁₋₂ alkyl-C(O)NR^{c}R^{d}, -C₁₋₂ alkyl-SO₂C₁₋₃ alkyl or C₂₋₄ alkynyl; wherein, the 3- to 6-membered heterocyclyl has 1, 2 or 3 heteroatoms selected from N, O and S as ring atoms.

In an embodiment, Rₛ₁, Rₛ₂ are each independently fluorine, chlorine, cyano, nitro, NH₂, NHCH₃, hydroxyl, methyl, ethyl, methoxy, halomethyl, haloethyl, halomethoxy, haloethoxy, cyclopropyl.

In an embodiment, in each embodiment, the substituents of group S1 each independently comprise oxo (=O), halogen, cyano, nitro, hydroxyl, carboxyl, C₁₋₃ alkyl, C₁₋₃ alkoxy, halo C₁₋₃ alkyl, deuterated C₁₋₃ alkyl, halo C₁₋₃ alkoxy, deuterated C₁₋₃ alkoxy, C₃₋₆ monocyclic cycloalkyl, 3- to 6-membered monocyclic heterocyclyl, C₃₋₆ monocyclic cycloalkoxy, -O-3- to 6-membered monocyclic heterocyclyl, NR^{c}R^{d}, C(O)C₁₋₃ alkyl, C(O)C₂₋₄ alkenyl, C(O)C₃₋₄ monocyclic cycloalkyl, C(O)NR^{c}R^{d}, -SO₂C₁₋₃ alkyl, -SOzhalo C₁₋₃ alkyl, -SO₂NR^{c}R^{d}, -C₁₋₂ alkyl-hydroxyl, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy, -C₁₋₂ alkyl-3- to 6-membered monocyclic heterocyclyl, -C₁₋₂ alkyl-NR^{c}R^{d}, -O-C₁₋₂ alkyl-NR^{c}R^{d}, -C₁₋₂ alkyl-C(O)NR^{c}R^{d}, -C₁₋₂ alkyl-SO₂C₁₋₃ alkyl, carboxyl, -C₁₋₂ alkyl-carboxyl; the R^{c}, R^{d} are each independently H, C₁₋₆ alkyl, deuterated C₁₋₆ alkyl, C₃₋₆ monocyclic cycloalkyl, -C₁₋₄ alkyl-C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms.

In an embodiment, the substituents of group S1 each independently comprise oxo (=O), COOH, chlorine, fluorine, nitro, hydroxyl, cyano, carboxyl, methyl, ethyl, isopropyl, methoxy, ethoxy, isopropoxy, fluoromethyl (e.g., CHF₂, CH₂F, CF₃), fluoroethyl (e.g., CF₂CF₃, CH₂CF₃), deuterated methyl(e.g., CD₃), deuterated ethyl (e.g., CH₂CD₃), deuterated methoxy (e.g., OCD₃), deuterated ethoxy (e.g., OCH₂CD₃), fluoromethoxy (e.g., OCHF₂, OCH₂F, OCF₃), fluoroethoxy (e.g., OCF₂CF₃, OCH₂CF₃), cyclopropyl, cyclobutyl, cyclopentyl, cyclopropoxy, cyclobutoxy, cyclopentyloxy, oxiranyl, aziridinyl, oxetanyl, azetidinyl, tetrahydrofuranyl, tetrahydropyrrolyl, C(O)CH₃, C(O)CH₂CH₃, C(O)CH₂CH₂CH₃, C(O)CH=CH₂, C(O)-cyclopropane, -SO₂CH₃, -SO₂CH₂CH₃, -SO₂CH₂CH₂CH₃, NH₂, NH(CH)₃, N(CH₃)₂, NH(CH₂CH₃), N(CH₂CH₃)₂, N(CH₂CH₃)(CH₃), N(CH₂CH₂CH₃)(CH₃), N(CH₂CH₂CH₂CH₃)(CH₃), N(CH₃)(CH₂CH₂OH), N(CH₃)(CH₂CH₂OCH₃), N(CH₃)(CH₂CH₂CH₂OCH₃), N(CH₃)(CH₂CH₂CH₂CH₂OCH₃), N(CH₃)(CH₂C(CH₃)₂OCH₃), N(CH₃)(CH₂C(CH₂)₂OCH₃), N(CD₃)(CH₂C(CH₂)₂OCH₃), NH(CH₂CH₂OH), NH(CH₂CH₂OCH₃), N(cyclobutane)(CH₃), N(azetidine)(CH₃), N(1-methylazetidine)(CH₃), NH(oxetane), N(oxetane)(CH₃), N(oxacycloheptane)(CH₃), N(CH₂-azetidine)(CH₃), N(CH₂(1-methylazetidine))(CH₃), NH(CH₂-oxetane), N(CH₂-oxetane)(CH₃), N(oxetane)(CD₃), N(CD₃)₂, N(CH₃)(CD₃), N(C(O)CH₃)(CH₃), CH₂N(CH₃)₂, CH₂CH₂N(CH₃)₂, - OCH₂CH₂(CH₃)₂, -OCH₂CH₂CH₂(CH₃)₂, -OCH₂CH₂CH₂CH₂(CH₃)₂, -O-oxetane, -O-azetidine, -O-(1-methylazetidine), morpholinyl, CHzOH, CHzCHzOH, CH₂OCH₃, CH₂CH₂OCH₃, -OCH₂CH₂N(CH₃)₂, - CH₂CH₂N(CH₃)₂, -CH₂N(CH₃)₂.

In an embodiment, the substituents of group S1 each independently comprise methyl, methoxy, hydroxyl.

In an embodiment, X¹, X², X³, L¹, L², L³, R², R³, R⁴, R⁵, R⁶, R⁷, R⁸, R⁹, E, m, n, G₁, G₂ are each independently the corresponding groups in each specific compound in the embodiments.

In an embodiment, the compound of formula (I) is selected from the compounds prepared in the embodiments of the present disclosure, for example, selected from compounds Z1 to Z662 and isomer compounds of these compounds.

In another aspect, the present disclosure provides a pharmaceutical composition, comprising the compound, the pharmaceutically acceptable salt thereof, the stereoisomer thereof, the solvate thereof or the prodrug thereof and a pharmaceutically acceptable carrier.

As used herein, the term "pharmaceutically acceptable carrier" refers to any formulation or a carrier representative of carrier media capable of delivering an effective amount of the active substance of the present disclosure without interfering with the biological activity of the active substance and with no toxic side effects to a host or a subject, including water, oils, vegetables and minerals, cream bases, lotion bases, ointment bases, etc. Such bases include suspending agents, tackifiers, transdermal penetration enhancer, etc. Their formulations are well known to those skilled in the field of cosmetics or topical pharmaceuticals.

In embodiments of the present disclosure, the pharmaceutical composition can be administered in any of the following ways: orally, by spray inhalation, rectally, nasally, bucally, topically, parenterally, e.g., injected or inputted subcutaneously, intravenously, intramuscularly, intraperitoneally, intrathecally, intraventricularly, intrasternally and intracranially, or administered by means of an external reservoir. Where, administered orally, intraperitoneally or intravenously is preferred. When administered orally, the compounds of the present disclosure can be prepared into any orally acceptable formulation, including but not limited to tablets, capsules, aqueous solutions, or aqueous suspensions. Carriers for tablets typically include lactose and cornstarch. In addition, lubricants such as magnesium stearate can also be added. Diluents used in capsule formulations typically include lactose and dried cornstarch. Aqueous suspension formulations are usually prepared by mixing active ingredients with suitable emulsifiers and suspending agents. If desired, some sweeteners, flavoring agents, or colorants may also be added to the oral formulations. When administered topically, particularly in the treatment of affected surfaces or organs prone to topical application such as eye, skin or lower intestinal neurological diseases, the compounds of the present disclosure can be prepared into different topical pharmaceutics according to different affected surfaces or organs. When administered topically to eyes, the compounds of the present disclosure can be formulated in the form of micronized suspensions or solutions. The carrier used is sterile saline at an isotonic pH, to which a preservative such as benzyl alkanol chloride may or may not be added. For eye use, the compounds can also be prepared into ointments such as Vaseline ointments. When administered topically to the skin, the compounds of the present disclosure can be prepared into suitable ointment, lotion or cream formulations, with the active ingredients being suspended or dissolved in one or more carriers. Carriers that can be used in ointment formulations include but are not limited to mineral oils, liquid Vaseline, white Vaseline, propylene glycol, polyethylene oxide, polypropylene oxide, emulsified wax, and water. Carriers that can be used in lotions or creams include but are not limited to mineral oils, sorbitan monostearate, Tween 60, cetyl esters wax, hexadecen-aryl alcohol, 2-octyldodecanol, benzyl alcohol, and water. The compounds of the present disclosure can also be administered in the form of sterile injection formulations, including sterile injectable water or oil suspensions or sterile injectable solutions. Carriers and solvents that can be used include water, Ringer's solution, and isotonic sodium chloride solution. In addition, sterile non-volatile oils can also be used as solvents or suspending media, e.g., monoglycerides or diglycerides.

In another aspect, the present disclosure provides use of the compound, the pharmaceutically acceptable salt thereof, the stereoisomer thereof, the solvate thereof or the prodrug thereof in the manufacture of a medicament for the treatment and/or prevention of cancer.

In another aspect, the present disclosure provides a method of treating cancer, comprising administering to a subject in need thereof a therapeutically effective amount of the compound, the pharmaceutically acceptable salt thereof, the stereoisomer thereof, the solvate thereof or the prodrug thereof, or any combination thereof or comprising a step of administering the pharmaceutical composition.

In an embodiment, the cancer is pancreatic ductal carcinoma, colorectal cancer, multiple myeloma, lung cancer, cutaneous melanoma, endometrial carcinoma, uterine cancer sarcoma, thyroid cancer, acute myeloid leukemia, bladder urothelial carcinomas, gastric cancer, cervical cancer, head and neck squamous cell carcinoma, diffuse large B-cell lymphoma, esophageal cancer, chronic lymphocytic leukemia, lung squamous cell carcinoma, small cell lung cancer, renal papillary cell carcinoma, adenoid cystic carcinoma, chromophobe renal cell carcinoma, liver cancer, breast invasive carcinoma, cervical squamous cell carcinoma, ovarian serous adenocarcinoma, adrenocortical carcinoma, prostate cancer, neuroblastoma, low grade brain glioma, glioblastoma, medulloblastoma, esophageal squamous cell carcinoma, renal clear cell carcinoma, osteosarcoma, ovarian small cell carcinoma, rhabdoid tumour, sarcoma, small intestine neuroendocrine tumour, T cell prolymphocytic leukemia.

In an embodiment, the cancer is lung cancer, preferably non-small cell lung cancer.

In another aspect, the present disclosure provides use of the compound, the pharmaceutically acceptable salt thereof, the stereoisomer thereof, the solvate thereof or the prodrug thereof in the manufacture of a KRAS mutation inhibitor (preferably, the KRAS mutation is KRAS G12C mutation).

As used herein, the term "subject" refers to an animal, especially a mammal, preferably human.

As used herein, the term "effective amount" or "therapeutically effective amount" refers to a sufficient amount of nontoxic drugs or pharmaceutics that can achieve the expected effects. In embodiments of the present disclosure, when treating a patient according to the present disclosure, the amount of a given drug depends on many factors, such as the specific dosage regimen, the disease or condition type and its severity, and the uniqueness (e.g., body weight) of the subject or host in need of treatment. However, depending on the particular circumstances, including, for example, the adopted specific drug, administration route, the treated condition, and the treated subject or host, the administered dosage can be conventionally determined by the known method in the art. Usually, for a dosage used for treating an adult, the administered dosage is typically in a range of 0.02-5000 mg/day, for example, about 1-1500 mg/day. The desired dosage can be conveniently shown as a single dose, or divided doses administered simultaneously (or in short time) or at appropriate intervals, for example, two, three, four or more divided doses each day. It will be understood by a person skilled in the art that although the dosage range is given, the specific effective amount can be adjusted appropriately according to the patient's condition in combination with the doctor's diagnosis.

As used herein, the term "pharmaceutically acceptable salt" refers to a salt of a compound of the present disclosure that is pharmaceutically acceptable and has the pharmacological activity of the parent compound. Such salts include: acid addition salts formed with inorganic acids such as nitric acid, phosphoric acid, and carbonic acid, or organic acids such as propionic acid, hexanoic acid, cyclopentanoic acid, glycolic acid, pyruvic acid, gluconic acid, stearic acid, and muconic acid; or salts formed by replacing acidic protons present on the parent compounds with metal ions, such as alkali metal ions or alkaline earth metal ions; or coordination compounds formed with organic bases such as ethanolamine, diethanolamine, triethanolamine, and *N*-methylglucamine. The pharmaceutically acceptable salts of the present disclosure can be synthesized from the parent compounds containing acidic radicals or basic radicals by a conventional chemical method. In general, such salts are prepared by reacting these compounds in the form of free acids or bases with a stoichiometric amount of an appropriate base or acid in water or an organic solvent or a mixture thereof. Generally, non-aqueous media such as ether, ethyl acetate, ethanol, isopropyl alcohol, or acetonitrile are preferred. In addition to the form of salts, the compounds provided by the present disclosure also exist in the form of prodrugs. Prodrugs of the compounds described herein are prone to chemical changes under physiological conditions and thus converted to the compounds of the present disclosure. In addition, prodrugs can be converted to the compounds of the present disclosure by a chemical or biochemical method in the *in vivo* environment.

As used herein, the terms "solvent compound" and "solvate" refer to a substrate formed by a compound of the present disclosure combined with a pharmaceutically acceptable solvent. Solvates include stoichiometric solvates and non-stoichiometric solvates. Pharmaceutically acceptable solvents include water, ethanol, acetic acid, etc. The solvent compound includes a stoichiometric amount of a solvent compound and a non-stoichiometric amount of a solvent compound, preferably a hydrate. Certain compounds of the present disclosure can be present in unsolvated or solvated forms, including hydrated forms. In general, the solvated form and the non-solvated form are equivalent and both included in the scope of the present disclosure.

As used herein, the term "stereisomer" includes a conformational isomer and a configurational isomer, wherein the configurational isomer mainly includes a *cis-trans* isomer and an optical isomer. The compounds of the present disclosure can be present in the form of stereisomers and thus encompass all possible stereisomer forms, including but not limited to *cis-trans* isomers, tautomers, enantiomers, diastereoisomers, atropisomers, etc. The compounds of the present disclosure can also be present in the form of any combination or any mixture of the stereisomers, for example, a mixture of equal amounts of a mesomer, a raceme, and an atropisomer. For example, each compound can be present as a single enantiomer, a single diastereoisomer or a mixture thereof, or a single atropisomer or a mixture thereof. When containing an olefinic double bond, the compounds of the present disclosure include *cis* isomers and *trans* isomer and any combination thereof unless specified otherwise. The atropisomers of the present disclosure are stereoisomers based on axial or planar chirality resulting from restricted intramolecular rotation. The compounds of the present disclosure each have two atropisomers originated from axial dissymmetry, which are derived from steric hindrance formed by restricting the rotation of the bond linkage between the substituent R₂ or R₃ being a cyclic group such as C₆₋₁₄ aryl, 5- or 6-membered monocyclic heteroaryl, 8- to 10-membered bicyclic heteroaryl, or pyridonyl (particularly when there is a substituent at a position ortho to both ends of the linking bond) with the the parent ring such as pyridine. In regard to the atropisomers of the present disclosure, the compound have a structure of formula (I), or the compound of formula (I) has isomers derived from asymmetric carbon, etc., which represents any one of a pair of atropisomers of each isocompound. As drugs, atropisomers having excellent activity are preferred. The compound of formula (I) has optical isomers derived from asymmetric carbon, axial dissymmetry, etc, and a single isomer can, if desired, be obtained by resolution by methods known in the art, e.g., crystallization or chiral chromatography. The atropisomers of the compounds of the present disclosure can be denoted as P- or M-configuration, and can also be denoted in other ways which are well-known and commonly used in the art.

As used herein, the term "alkyl" refers to a linear or branched saturated aliphatic hydrocarbyl group. The term "C₁₋₂₀ alkyl" refers to linear or branched alkyl with 1 to 20 carbon atoms, preferably C₁₋₁₀ alkyl, more preferably C₁₋₆ alkyl (i.e., linear or branched alkyl with 1, 2, 3, 4, 5 or 6 carbon atoms), further C₁₋₄ alkyl and still further preferably C₁₋₃ alkyl. Specific examples of alkyl include but are not limited to methyl, ethyl, *n*-propyl, isopropyl, *n*-butyl, isobutyl, *tert*-butyl, *sec-*butyl, *n*-amyl, 1,1-dimethylpropyl, 1,2-dimethylpropyl, 2,2-dimethylpropyl, 1-ethylpropyl, 2-methylbutyl, 3-methylbutyl, *n-*hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylamyl, 3-methylamyl, 4-methylamyl, 2,3-dimethylbutyl, and various branched isomers therof, etc.

As used herein, the term "alkoxy" refers to a group with an -O-alkyl structure, wherein the alkyl is defined as above. The term "C₁₋₁₀ alkoxy" refers to alkoxy with 1 to 10 carbon atoms, preferably C₁₋₆ alkoxy, more preferably C₁₋₄ alkoxy, and further preferably C₁₋₃ alkoxy. Specific examples of alkoxy include but are not limited to methoxy, ethoxy, *n*-propoxy, isopropoxy, *n*-butoxy, *tert*-butoxy, isobutoxy, *n-*pentyloxy, etc.

As used herein, the term "thioalkyl" refers to a group with an -S-alkyl structure, wherein the alkyl is defined as above. The term "C₁₋₁₀ thioalkyl" refers to thioalkyl with 1 to 10 carbon atoms, preferably C₁₋₆ thioalkyl, more preferably C₁₋₄ thioalkyl, and further preferably C₁₋₃ thioalkyl. Specific examples of thioalkyl include but are not limited to thiomethyl, thioethyl, thiopropyl, thioisopropyl, thiobutyl, thio-*tert-*butyl, thioisobutyl, thioamyl, etc.

As used herein, the term "alkenyl " refers to alkyl defined as above with one or more C-C double bonds at any site of the chain, and the term "C₂₋₈ alkenyl " refers to alkenyl with 2 to 8 carbon atoms and at least one C-C double bond, preferably C₂₋₆ alkenyl (i.e., alkenyl with 2 to 6 carbon atoms and 1 to 2 C-C double bonds), more preferably C₂₋₄ alkenyl (i.e., alkenyl with 2 to 4 carbon atoms and 1 to 2 C-C double bonds). Specific examples of alkenyl include but are not limited to ethenyl, 1-propenyl, 2-propenyl, 1-, 2- or 3-butenyl, pentenyl, hexenyl, butadienyl, etc.

As used herein, the term "alkynyl" refers to alkyl defined as above with one or more C-C triple bonds at any site of the chain, and the term "C₂₋₈ alkynyl" refers to alkynyl with 2 to 8 carbon atoms and at least one C-C triple bond (e.g., 1 or 2), preferably C₂₋₆ alkynyl (i.e., alkynyl with 2 to 6 carbon atoms and 1 to 2 C-C triple bonds), more preferably C₂₋₄ alkynyl (i.e., alkynyl with 2 to 4 carbon atoms and 1 to 2 C-C triple bonds). Specific examples of alkynyl include but are not limited to ethynyl, 1-propinyl, 2-propinyl, 1-, 2- or 3-butynyl, etc.

As used herein, the term "halogen" refers to fluorine, chlorine, bromine or iodine.

As used herein, the term "halo" refers to fluoro, chloro, bromo or iodo.

As used herein, the term "haloalkyl" refers to alkyl in which one or more (e.g., 1, 2, 3, 4, or 5) hydrogen atoms are substituted by halogen, wherein the alkyl is defined as above. The term "C₁₋₁₀ haloalkyl" refers to haloalkyl with 1 to 10 carbon atoms, preferably halo C₁₋₆ alkyl, more preferably halo C₁₋₄ haloalkyl, and further preferably halo C₁₋₃ alkyl. Specific examples of haloalkyl include but are not limited to monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, etc.

As used herein, the term "haloalkoxy" refers to alkoxy in which one or more (e.g., 1, 2, 3, 4 or 5) hydrogen atoms are substituted by halogen, wherein the alkoxy is defined as above. The term "C₁₋₁₀ haloalkoxy" refers to haloalkoxy with 1 to 10 carbon atoms, preferably halo C₁₋₆ alkoxy, more preferably halo C₁₋₄ haloalkoxy, and more preferably halo C₁₋₃ haloalkoxy. Specific examples of haloalkoxy include but are not limited to trifluoromethoxy, trifluoroethoxy, monofluoromethoxy, monofluoroethoxy, difluoromethoxy, difluoroethoxy, etc.

As used herein, the terms "cycloalkyl" and "cycloalkyl ring" can be used interchangeably, which refer to saturated monocyclic or polycyclic cyclohydrocarbyl, including, for example, monocyclic cycloalkyl, spirocycloalkyl, fused cycloalkyl, and bridged cycloalkyl. The ring carbon atoms of the cycloalkyl in the present disclosure can optionally be substituted by 1, 2 or 3 oxo to form a cycloketone structure. The term "3- to 20-membered cycloalkyl" or "C₃₋₂₀ cycloalkyl" refers to cycloalkyl with 3 to 20 ring carbon atoms, including monocyclic cycloalkyl, spirocycloalkyl, fused cycloalkyl and bridged cycloalkyl, preferably C₃₋₁₂ cycloalkyl. The terms "C₃₋₈ monocyclic cycloalkyl" and "3- to 8-membered monocyclic cycloalkyl" refer to saturated monocyclic cyclohydrocarbyl with 3 to 8 ring carbon atoms, preferably C₃₋₆ monocyclic cycloalkyl (i.e., 3- to 6-membered monocyclic cycloalkyl) or C₄₋₆ monocyclic cycloalkyl (i.e., 4- to 6-membered monocyclic cycloalkyl), more preferably C₃, C₄, C₅ or C₆ monocyclic cycloalkyl. Specific examples of monocyclic cycloalkyl include but are not limited to cyclopropyl, cyclobutyl, cyclopentyl, cyclohexyl, cycloheptyl, cyclooctyl, etc.

As used herein, the terms "spirocycloalkyl" and "spirocycloalkyl ring" refer to polycyclic cyclohydrocarbyl formed with two or more monocycles sharing one carbon atom (called a spiro-atom). Spirocycloalkyl is classified as monospirocycloalkyl, bispirocycloalkyl or polyspirocycloalkyl depending on the number of spiro-atoms shared between rings. The term "5- to 20-membered spirocycloalkyl" or "C₅₋₂₀ spirocycloalkyl" refers to polycyclic cyclohydrocarbyl with 5 to 20 ring carbon atoms, wherein the monocycles sharing a spiro-atom are 3- to 8-membered monocyclic cycloalkyl rings, preferably 6- to 14-membered (i.e., C₆₋₁₄) spirocycloalkyl, more preferably 6- to 14-membered monospirocycloalkyl, further preferably 7- to 11-membered (i.e., C₇₋₁₁) spirocycloalkyl, still further preferably 7- to 11-membered monospirocycloalkyl, and most preferably 7-membered (4-membered monocyclic cycloalkyl ring/4-membered monocyclic cycloalkyl ring), 8-membered (4-membered monocyclic cycloalkyl ring/5-membered monocyclic cycloalkyl ring), 9-membered (4-membered monocyclic cycloalkyl ring/6-membered monocyclic cycloalkyl ring, 5-membered monocyclic cycloalkyl ring/5-membered monocyclic cycloalkyl ring), 10-membered (5-membered monocyclic cycloalkyl ring/6-membered monocyclic cycloalkyl ring) or 11-membered (6-membered monocyclic cycloalkyl ring/6-membered monocyclic cycloalkyl ring) monospirocycloalkyl. Specific examples of spirocycloalkyl include but are not limited to:

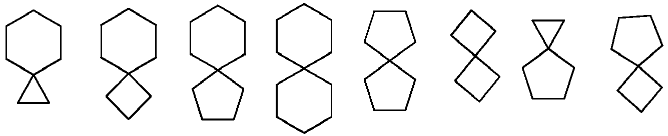

These spirocycloalkyl can be attached to the rest of the molecule through any one of the ring atoms.

As used herein, the terms "fused cycloalkyl" and "fused cycloalkyl ring" refer to polycyclic cyclohydrocarbyl formed by two or more monocycles sharing an adjacent pair of carbon atoms. According to the number of formed rings, fused cycloalkyl can be divided into bicyclic, tricyclic, tetracyclic or polycyclic fused cycloalkyl. The term "5- to 20-membered fused cycloalkyl" or "C₅₋₂₀ fused cycloalkyl" refers to a polycyclic cyclohydrocarbyl with 5 to 20 ring carbon atoms, wherein the monocycle sharing adjacent carbon atom pairs is a 3- to 8-membered monocyclic cycloalkyl ring. Preferably 6- to 14-membered (i.e., C₆₋₁₄) fused cycloalkyl, more preferably 6- to 14-membered bicyclic fused cycloalkyl, further preferably 7- to 10-membered (i.e., C₇₋₁₀) fused cycloalkyl, still further preferably 7- to 10-membered bicyclic cycloalkyl. Most preferably 8-membered (formed by 5-membered monocyclic cycloalkyl ring fused with 5-membered monocyclic cycloalkyl ring), 9-membered (formed by 5-membered monocyclic cycloalkyl ring fused with 6-membered monocyclic cycloalkyl ring) or 10-membered (formed by 6-membered monocyclic cycloalkyl ring fused with 6-membered monocyclic cycloalkyl ring) bicyclic fused cycloalkyl. Specific examples of fused cycloalkyl include, but are not limited to:

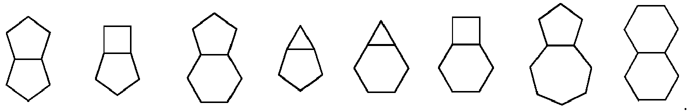

These fused cycloalkyl can be attached to the rest of the molecule through any one of the ring atoms.

As used herein, the terms "bridged cycloalkyl" and "bridged cycloalkyl ring" refer to polycyclic cyclohydrocarbyl formed between two or more monocycles by sharing two carbon atoms that are not directly connected. According to the number of rings formed, it can be divided into bicyclic, tricyclic, tetracyclic or polycyclic bridged cycloalkyl. The terms "5- to 20-membered bridged cycloalkyl" and "C₅₋₂₀ bridged cycloalkyl" refer to polycyclic cyclohydrocarbyl with 5 to 20 ring carbon atoms, wherein any two rings share two carbon atoms that are not directly connected. Preferably 6- to 14-membered (i.e., C₆₋₁₄) bridged cycloalkyl, more preferably 7- to 10-membered (i.e., C₇₋₁₀) bridged cycloalkyl. Specific examples of bridged cycloalkyl include, but are not limited to:

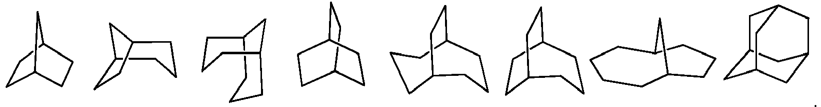

These bridged cycloalkyl can be attached to the rest of the molecule through any one of the ring atoms.

The cycloalkyl ring can be fused to an aryl, heteroaryl or heterocyclyl ring, wherein the ring attached to the parent structure is a cycloalkyl ring, non-limiting examples include indanyl, tetrahydronaphthyl, benzocycloheptyl, etc. In the present disclosure, the above types of cycloalkyl can be optionally substituted, and when substituted, the substituents are preferably one or more of the substituent groups described in the present disclosure.

As used herein, the term "halocycloalkyl" refers to cycloalkyl in which one or more (e.g., 1, 2, 3, 4 or 5) hydrogen atoms are substituted by halogen, wherein cycloalkyl is defined as above. The term "halo C₃₋₈ cycloalkyl" refers to halocycloalkyl with 3 to 8 ring carbon atoms, preferably halo C₃₋₆ cycloalkyl, more preferably halo C₃, halo C₄, halo C₅ or halo C₆ cycloalkyl. Specific examples include but are not limited to trifluorocyclopropyl, monofluocyclopropyl, monofluocyclohexyl, difluorocyclopropyl, difluorocyclohexyl, etc.

As used herein, the terms "heterocyclyl" and "heterocyclyl ring" can be used interchangeably and refer to saturated or partially unsaturated monocyclic or polycyclic cyclohydrocarbyl, including, for example, monocyclic heterocyclyl, spiroheterocyclyl, fused heterocyclyl and bridged heterocyclyl. The ring carbon atoms of the heterocyclyl in the present disclosure can be optionally substituted by 1, 2 or 3 oxo to form a cycloketone, cyclolactone or cyclolactam structure. The term "3- to 20-membered heterocyclyl" refers to saturated or partially unsaturated monocyclic or polycyclic cyclohydrocarbyl with 3 to 20 ring atoms, wherein one or more (preferably 1, 2, 3 or 4) ring atoms are heteroatoms selected from nitrogen, oxygen or S(=O)_{m'} (wherein m' is an integer from 0 to 2), but excluding ring moieties of -O-O-, -O-S- or -S-S-, and the rest ring atoms are carbon. Where when the ring atom is a nitrogen atom, it may be substituted or unsubstituted (i.e., N or NR, R is hydrogen or other substituents already defined herein). The 3- to 20-membered heterocyclyl of the present disclosure includes monocyclic heterocyclyl, spiroheterocyclyl, fused heterocyclyl and bridged heterocyclyl.

As used herein, the terms "3- to 8-membered monocyclic heterocyclyl" and "3- to 8-membered monocyclic heterocyclyl ring" refer to saturated or partially unsaturated monocyclic cyclohydrocarbyl with 3 to 8 ring atoms, wherein 1, 2, or 3 ring atoms are heteroatoms selected from nitrogen, oxygen, or S(=O)_{m'} (wherein m' is an integer from 0 to 2). Preferably 3- to 6-membered monocyclic heterocyclyl with 3 to 6 ring atoms, wherein 1 or 2 ring atoms are heteroatoms. More preferably 4- or 6-membered monocyclic heterocyclyl with 4 or 6 ring atoms, wherein 1 or 2 ring atoms are heteroatoms. Further preferably 5- or 6-membered monocyclic heterocyclyl with 5 or 6 ring atoms, wherein 1 or 2 ring atoms are heteroatoms. When the heteroatom is nitrogen atom, the nitrogen atom can be substituted or unsubstituted (i.e., N or NR, R is hydrogen or other substituents already defined herein). When the heteroatom is sulfur atom, the sulfur atom can be optionally oxidated (i.e., S(=O)_{m'}, m' is an integer from 0 to 2). The ring carbon atoms of the monocyclic heterocyclyl can be each optionally substituted by 1, 2, or 3 oxo to form cycloketone, cyclolactone or cyclolactam structures. Specific examples of monocyclic heterocyclyl include but are not limited to aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3*H*)-one, dihydrofuran-2,5-dione, piperidin-2-one, piperidin-2,6-dione, tetrahydro-2*H*-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxacyclobutadiene, 2,5-dihydro-1*H*-pyrrole, 2,5-dihydrofuran, 2,3-dihydrofuran, 2,3-dihydro-1*H-*pyrrole, 3,4-dihydro-2*H*-pyran, 1,2,3,4-tetrahydropyridine, 3,6-dihydro-2*H*-pyran, 1,2,3,6-tetrahydropyridine, 1,3-oxazinane, hexahydropyrimidine, 1,4-dioxane, tetrahydropyrimidin-2(1*H*)-one, 1,4-dioxan-2-one, 5,6-dihydro-2*H*-pyran-2-one, 5,6-dihydropyrimidin-4(3*H*)-one, 3,4-dihydropyridin-2(1*H*)-one, 5,6-dihydropyridin-2(1*H*)-one, 5,6-dihydropyrimidin-4(1*H*)-one, pyrimidin-4(3*H*)-one, pyrimidin-4(1*H*)-one, 4,5-dihydro-1*H*-imidazole, 2,3-dihydro-1*H*-imidazole, 2,3-dihydrooxazole, 1,3-dioxacyclopentene, 2,3-dihydrothiophene, 2,5-dihydrothiophene, 3,4-dihydro-2*H*-1,4-oxazine, 3,4-dihydro-2*H*-1,4-thiazine 1,1-dioxide, 1,2,3,4-tetrahydropyrazine, 1,3-dihydro-2*H*-pyrrol-2-one, 1,5-dihydro-2*H-*pyrrol-2-one, 1*H*-pyrrol-2,5-dione, furan-2(3*H*)-one, furan-2(5*H*)-one, 1,3-dioxacyclopenten-2-one, oxazol-2(3*H*)-one, 1,3-dihydro-2*H*-imidazol-2-one, furan-2,5-dione, 3,6-dihydropyridin-2(1*H*)-one, pyridin-2,6-(1*H*, 3*H*)-dione, 5,6-dihydro-2*H*-pyran-2-one, 3,6-dihydro-2*H*-pyran-2-one, 3,4-dihydro-2*H*-1,3-oxazine, 3,6-dihydro-2*H*-1,3-oxazine, 1,2,3,4-tetrahydropyrimidine, etc.

The term "3- to 6-membered nitrogen-containing heterocyclyl" refers to saturated or partially unsaturated monocyclic cyclohydrocarbyl with 3 to 6 ring atoms, wherein one ring atom is a nitrogen atom and the other one or two ring atoms are heteroatoms selected from nitrogen, oxygen or S(=O)_{m'} (wherein m' is an integer of 0 to 2). Specific examples include, but are not limited to, aziridinyl, azetidinyl, azacyclopentyl (i.e., tetrahydropyrrole), azacyclohexyl (i.e., hexahydropyridine), morpholinyl, piperazinyl, oxazolidine.

The term "3- to 8-membered monocyclic heterocycloalkyl" refers to saturated monocyclic cyclohydrocarbyl with 3 to 8 ring atoms, wherein 1 or 2 ring atoms are heteroatoms. Preferably 3- to 6-membered monocyclic heterocycloalkyl, i.e., saturated monocyclic cyclohydrocarbyl with 3 to 6 ring atoms, wherein 1 or 2 ring atoms are heteroatoms. Specific examples of heterocycloalkyl include, but are not limited to, aziridinyl, oxiranyl, azetidinyl, oxetanyl, oxazolidinyl, 1,3-dioxolanyl, dioxanyl, imidazolidinyl, tetrahydrofuranyl, tetrahydrothienyl, tetrahydropyrrolyl, piperidinyl, piperazinyl, morpholinyl, thiomorpholinyl, thiomorpholin-1,1-dioxide, tetrahydropyranyl, 1,4-oxazacycloheptyl, 1,3-oxazacycloheptyl, 1,3-oxazinanyl, hexahydropyrimidinyl, 1,4-dioxanyl.

The two ring atoms attached to the monocyclic heterocyclyl ring, including C-C, N-C, can both optionally fuse with a cycloalkyl such as monocyclic cycloalkyl ring, monocyclic heterocyclyl ring, monocyclic aryl ring, 5- or 6-membered monocyclic heteroaryl ring, heterocyclyl, aryl or heteroaryl defined in the present disclosure to form a fused polycyclic ring. The two ring atoms attached to the monocyclic heterocyclyl forming a fused ring with other rings are preferably C-C.

As used herein, the terms "spiroheterocyclyl" and "spiroheterocyclyl ring" refer to polycyclic heterocyclyl formed by two or more saturated or partially unsaturated monocycles sharing a carbon atom (called a spiro atom), wherein one or more (e.g., 1, 2 or 3) ring atoms are heteroatoms selected from nitrogen, oxygen or S(=O)_{m'} (wherein m' is an integer from 0 to 2) and the rest ring atoms are carbon. When the heteroatom is a nitrogen atom, the nitrogen atom can be substituted or unsubstituted (i.e., N or NR, R is hydrogen or other substituents already defined herein). Each monocycle can contain one or more double bonds, but no ring has a fully conjugated π-electron system. According to the number of shared spiro atoms between rings, the spiroheterocyclyl can be divided into monospiroheterocyclyl, bispiroheterocyclyl or polyspiroheterocyclyl. The term "5- to 20-membered spiroheterocyclyl" refers to spiroheterocyclyl with 5 to 20 ring atoms, wherein one of the monocycles sharing the spiro atoms is a 3- to 8-membered monocyclic heterocyclyl ring, and the other monocycle is a 3 to 8-membered monocyclic heterocyclyl ring or a 3 to 8-membered monocyclic cycloalkyl ring. Preferably 6- to 14-membered spiroheterocyclyl with 6 to 14 ring atoms, wherein 1 or 2 ring atoms are heteroatoms, and more preferably 7- to 11-membered spiroheterocyclyl with 7 to 11 ring atoms, wherein 1 or 2 ring atoms are heteroatoms. Most preferably 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cyclocycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring) or 11-membered (6-membered monocyclic heterocyclyl ring/6-membered monocyclic heterocyclyl ring) monospiroheterocyclyl. Specific examples of spiroheterocyclyl include, but are not limited to:

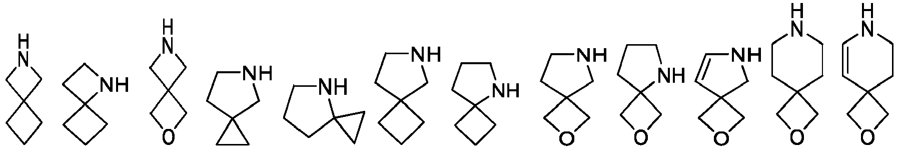

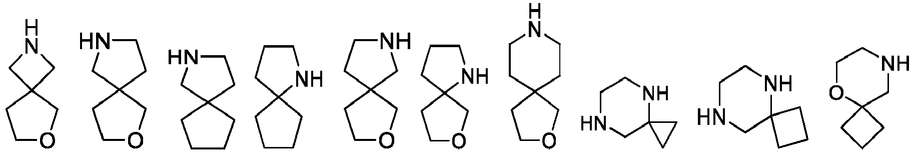

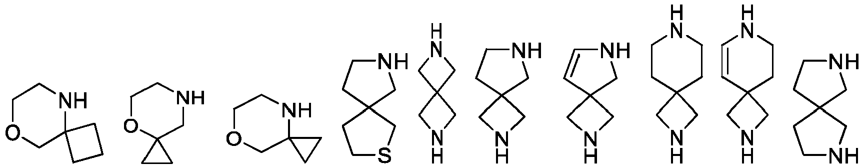

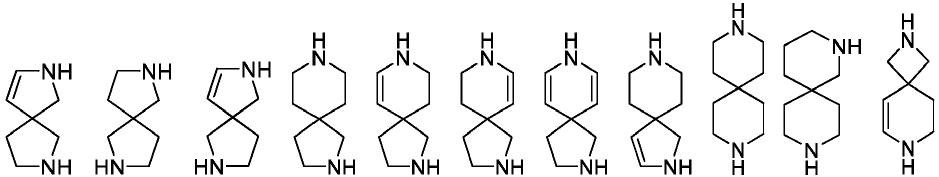

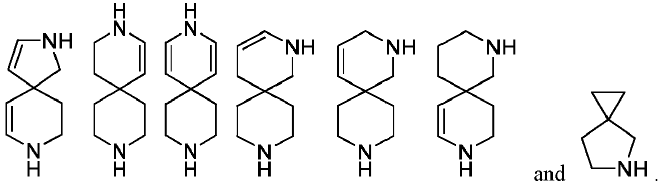

These spiroheterocyclyl can be attached to the rest of the molecule by any one of the suitable ring atoms.

As used herein, the terms "fused heterocyclyl" and "fused heterocyclyl ring" refer to polycyclic heterocyclyl formed by two or more saturated or partially unsaturated monocycles sharing an adjacent pair of ring atoms, wherein one or more (e.g., 1, 2 or 3) ring atoms are heteroatoms selected from nitrogen, oxygen or S(=O)_{m'} (where m' is an integer from 0 to 2) and the rest ring atoms are carbon. When the heteroatom is a nitrogen atom, the nitrogen atom can be substituted or unsubstituted (i.e., N or NR, R is hydrogen or other substituents already defined herein). Each monocycle can contain one or more double bonds, but no ring has a fully conjugated π-electron system. The shared adjacent ring atom pair can be C-C or N-C. According to the number of formed rings, they can be divided into bicyclic, tricyclic, tetracyclic or polycyclic fused heterocyclyl. The term "5- to 20-membered fused heterocyclyl" refers to fused heterocyclyl with 5 to 20 ring atoms, wherein the monocycles sharing adjacent ring atom pair are 3- to 8-membered monocyclic heterocyclyl rings. Preferably 6- to 14-membered fused heterocyclyl with 6 to 14 ring atoms, wherein 1 or 2 ring atoms are heteroatoms; more preferably 6- to 10-membered fused heterocyclyl with 6 to 10 ring atoms, wherein 1 or 2 ring atoms are heteroatoms; more preferably 8- to 10-membered fused heterocyclyl with 8 to 10 ring atoms, wherein 1 or 2 ring atoms are heteroatoms. Most preferably 8-membered (formed by 5-membered monocyclic heterocyclyl ring fused with 5-membered monocyclic heterocyclyl ring), 9-membered (formed by 5-membered monocyclic heterocyclyl ring fused with 6-membered monocyclic heterocyclyl ring) or 10-membered (formed by 6-membered monocyclic heterocyclyl ring fused with 6-membered monocyclic heterocyclyl ring) bicyclic fused heterocyclyl. Specific examples of fused heterocyclyl include, but are not limited to:

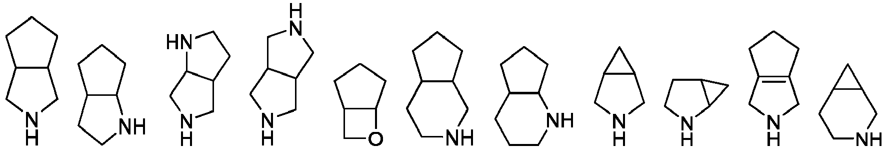

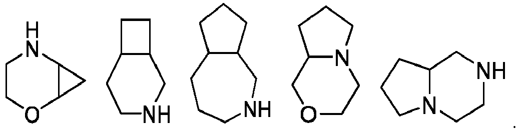

These fused heterocyclyl can be attached to the rest of the molecule by any one of the suitable ring atoms.

As used herein, the terms "bridged heterocyclyl" and "bridged heterocyclyl ring" refer to polycyclic heterocyclyl formed by two or more saturated or partially unsaturated monocycles by sharing two ring atoms that are not directly connected, wherein one or more (e.g., 1, 2 or 3) ring atoms are heteroatoms selected from nitrogen, oxygen or S(=O)_{m'} (wherein m' is an integer from 0 to 2), and the rest ring atoms are carbon. According to the number of formed rings, they can be divided into bicyclic, tricyclic, tetracyclic or polycyclic bridged cycloalkyl. The term "5- to 20-membered bridged heterocyclyl" refers to saturated or partially unsaturated polycyclic heterocyclyl with 5 to 20 ring atoms, wherein any two rings share two ring atoms that are not directly connected, and each monocycle can contain one or more double bonds, but none of the rings has a fully conjugated π-electron system. Preferably 6- to 14-membered bridged heterocyclyl, more preferably 7- to 10-membered bridged heterocyclyl. Specific examples of bridged heterocyclyl include, but are not limited to:

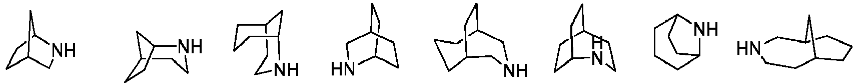

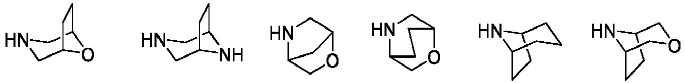

These bridged heterocyclyl can be attached to the rest of the molecule through any one of the suitable ring atoms.

In the present disclosure, the various heterocyclyl can be optionally substituted, and when substituted, the substituent is preferably one or more substituent groups described in the present disclosure.

As used herein, the terms "aryl", "aryl ring" and "aromatic ring" can be used interchangeably, and refer to all-carbon monocyclic all-carbon non-fused polycyclic (rings and rings are connected by covalent bonds, non-fused) or all-carbon fused polycyclic (i.e., rings sharing adjacent carbon atom pairs) groups. At least one ring in the group is aromatic, that is, has a conjugated π-electron system. The term "C₆₋₁₄ aryl" refers to aryl with 6 to 14 ring atoms, preferably C₆₋₁₀ aryl. C₆₋₁₄ aryl in the present disclosure includes monocyclic aryl, non-fused polycyclic aryl and aromatic fused polycyclic ring, wherein examples of monocyclic aryl include phenyl, and examples of non-fused polycyclic aryl include biphenyl, etc.

In the present disclosure, when C₆₋₁₄ aryl is an aromatic fused polycyclic ring, the aromatic fused polycyclic ring can be a polycyclic group formed by a monocyclic aryl ring fused with one or more monocyclic aryl rings, and non-limiting examples thereof include naphthyl, anthracyl, etc.

In some embodiments of the present disclosure, when C₆₋₁₄ aryl is an aromatic fused polycyclic ring, the aromatic fused polycyclic ring can also be a polycyclic group formed by a monocyclic aryl ring (such as phenyl) fused with one or more non-aromatic rings, wherein the ring attached to the parent structure is aromatic or non-aromatic ring. The non-aromatic rings include, but are not limited to, 3- to 6-membered monocyclic heterocyclyl ring (preferably 5- or 6-membered monocyclic heterocyclyl ring, wherein the ring carbon atom of the monocyclic heterocyclyl ring can be substituted by 1 to 2 oxo to form a cyclolactam or cyclolactone structure), 3- to 6-membered monocyclic cycloalkyl ring (preferably 5- or 6-membered monocyclic cycloalkyl ring, wherein the ring carbon atom of the monocyclic cycloalkyl ring can be substituted by 1 or 2 oxo to form a cycloketone structure). The polycyclic group formed by the monocyclic aryl rings fused with one or more non-aromatic rings can be connected to other groups or to the parent structure through a nitrogen atom or a carbon atom, and the ring attached to the parent structure is a monocyclic aryl ring or non-aromatic ring.

As used herein, the term "9-or 10-membered phenyl-heterocyclyl" or "9-or 10-membered phenyl-heterocyclyl ring" refers to a 9-or 10-membered aromatic fused bicyclic ring formed by a benzene ring fused with one 5- or 6-membered monocyclic heterocyclyl ring, i.e., a fused 5-or 6-membered monocyclic heterocyclyl ring formed by the two adjacent substituent groups on the phenyl fused with the ring atoms to which they are attached, and the 5- or 6-membered monocyclic heterocyclyl ring is defined as above.

As used herein, the term "9- or 10-membered phenyl-cycloalkyl" or "9- or 10-membered phenyl-cycloalkyl ring" refers to a 9- or 10-membered aromatic fused bicyclic ring formed by a benzene ring fused with one 5- or 6-membered monocyclic cycloalkyl ring, i.e., a fused 5- or 6-membered monocyclic cycloalkyl ring formed by the two adjacent substituent groups on the phenyl fused with the ring atoms to which they are attached, and the 5- or 6-membered monocyclic cycloalkyl ring is defined as above.

Non-limiting examples of "9- or 10-membered phenyl-heterocyclyl" or "9- or 10-membered phenyl-cycloalkyl" include: 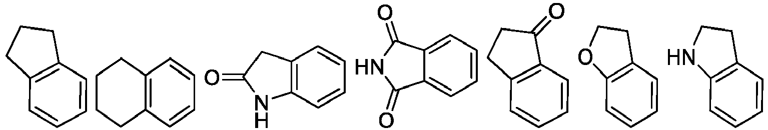 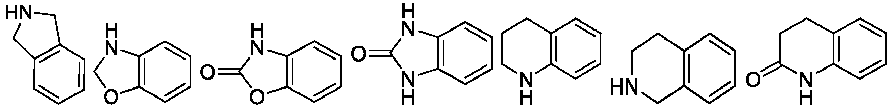 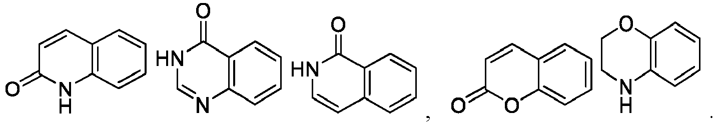 These groups can be attached to the rest of the molecule by any one of the suitable ring atoms.

In the present disclosure, the above types of aryl can be substituted or unsubstituted, and when substituted, the substituents are preferably one or more of the substituents described in the present disclosure.

As used herein, the terms "heteroaryl", "heteroaryl ring" and "heteroaromatic ring" can be used interchangeably, which refer to a monocyclic or fused polycyclic (i.e., sharing a pair of adjacent ring atoms which can be C-C or N-C) group with a ring atom being substituted by at least one heteroatom independently selected from nitrogen, oxygen, or sulfur, wherein nitrogen and sulfur atoms can be each optionally oxidated, and the nitrogen atom can be optionally quaternized. The heteroaryl has shared 6, 10 or 14 π electrons, and at least one ring in the group is aromatic. The term "5- to 14-membered heteroaryl" refers to heteroaryl with 5 to 14 ring atoms, wherein 1, 2, 3, or 4 ring atoms are heteroatoms selected from nitrogen, oxygen or S(=O)_{m'} (wherein m' is an integer from 0 to 2). Preferably 5- to 10-membered heteroaryl with 5 to 10 ring atoms, wherein 1, 2, 3, or 4 ring atoms are heteroatoms. In the present disclosure, 5- to 14-membered heteroaryl can be monocyclic heteroaryl, fused bicyclic heteroaryl or fused tricyclic heteroaryl.

As used herein, the term "5- or 6-membered monocyclic heteroaryl" refers to monocyclic heteroaryl with 5 or 6 ring atoms, wherein 1, 2 or 3 ring atoms are heteroatoms selected from nitrogen, oxygen or S(=O)_{m'} (wherein m' is an integer from 0 to 2). Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, etc.

As used herein, the terms "8- to 10-membered bicyclic heteroaryl" refers to fused bicyclic heteroaryl with 8 to 10 ring atoms, wherein 1, 2, 3, 4, or 5 ring atoms are heteroatoms selected from nitrogen, oxygen or S(=O)_{m'} (wherein m' is an integer from 0 to 2). The fused bicyclic heteroaryl can be a bicyclic group (preferably a 9- or 10-membered bicyclic heteroaryl ring) formed by a monocyclic aryl ring (e.g., phenyl) fused with a monocyclic heteroaryl ring (preferably a 5- or 6-membered monocyclic heteroaryl ring), or a bicyclic group formed by a monocyclic heteroaryl ring (preferably a 5- or 6-membered monocyclic heteroaryl ring) fused with a monocyclic heteroaryl ring (preferably a 5- or 6-membered monocyclic heteroaryl ring).

Any two linked ring atoms, including C-C, N-C and N-N, on the monocyclic heteroaryl ring can be fused with cycloalkyl such as a monocyclic cycloalkyl ring, a monocyclic heterocyclyl ring, a monocyclic aryl ring and a 5- or 6-membered monocyclic heteroaryl ring, heterocyclyl, aryl or heteroaryl, as defined in the present disclosure, to form fused polycycles. The two linked ring atoms on the monocyclic heteroaryl ring that forms a fused ring with other ring are preferably C-C, non-restrictively including the following forms:

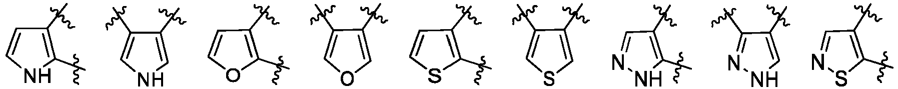

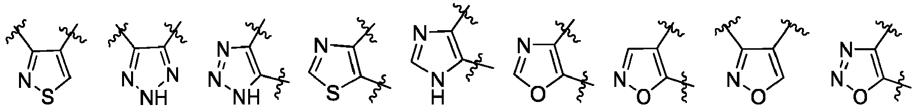

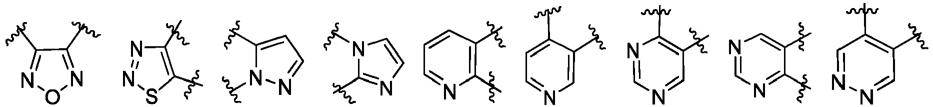

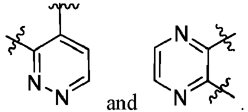

The ring atoms marked by " 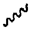 " in the groups are attached to the rest of the molecule.

Non-limiting examples of 8- to 10-membered bicyclic heteroaryl include: benzo[*d*]isoxazole, 1*H-*indole, isoindole, 1*H*-benzo[*d*]imidazole, benzo[*d*]isothiazole, 1*H*-benzo[*d*][1,2,3]triazole, benzo[*d*]oxazole, benzo[*d*]thiazole. indazole, benzofuran, benzo[*b*]thiophene, quinoline, isoquinoline, quinazoline, quinoxaline, cinnoline, pyrido[3,2-*d*]pyrimidine, pyrido[2,3-*d*]pyrimidine, pyrido[3,4-*d*]pyrimidine, pyrido[4,3-*d*]*p*yrimidine, 1,8-naphthyridine, 1,7-naphthyridine, 1,6-naphthyridine, 1,5-naphthyridine, pyrazolo[1,5-*α*]pyrimidine, imidazo[1,2-*b*]pyridazine, etc.

The monocyclic heteroaryl, or the bicyclic heteroaryl formed by a benzene ring fused with a monocyclic heteroaryl ring, or the bicyclic heteroaryl formed by a monocyclic heteroaryl ring fused with a monocyclic heteroaryl ring can be attached to other groups or parent structures by nitrogen or carbon atoms. Specific examples of bicyclic heteroaryl include, but are not limited to: 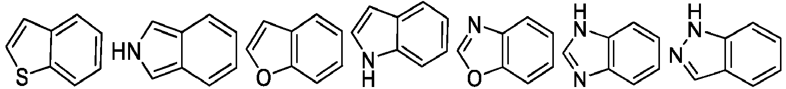 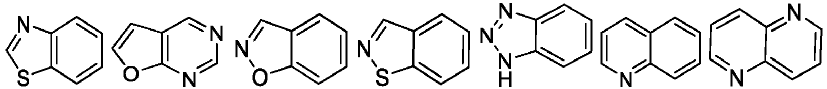 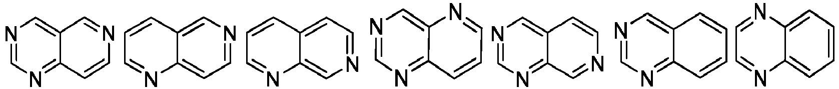 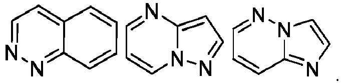 These groups can be attached to the rest of the molecule by any one of the suitable ring atoms. The ring attached to the parent structure can be a monocyclic heteroaryl ring or a benzene ring.

In some embodiments of the present disclosure, the fused bicyclic heteroaryl or the fused tricyclic heteroaryl can also be a polycyclic group formed by a monocyclic heteroaryl ring (preferably a 5- or 6-membered monocyclic heteroaryl ring) fused with one or more non-aromatic rings, wherein the ring attached to the parent structure is a monocyclic heteroaryl ring or a non-aromatic ring. The non-aromatic rings include but are not limited to 3- to 6-membered monocyclic heterocyclyl rings (preferably a 5- or 6-membered monocyclic heterocyclyl ring, wherein the ring carbon atoms of the monocyclic heterocyclyl ring can be substituted by 1 to 2 oxo, forming a cyclolactam or cyclolactone structure), 3- to 6-membered monocyclic cycloalkyl rings (preferably a 5- or 6-membered monocyclic cycloalkyl ring, wherein the ring carbon atoms of the monocyclic cycloalkyl ring can be substituted by 1 or 2 oxo, forming a cycloketone structure), etc. The polycyclic group formed by a monocyclic heteroaryl ring fused with one or more non-aromatic rings can be attached to other group or the parent structure by a nitrogen atom or a carbon atom, with the ring attached to the parent structure being a monocyclic heteroaryl ring or a non-aromatic ring.

As used herein, the term "8- to 10-membered heteroaryl-heterocyclyl" or "8- to 10-membered heteroaryl-heterocyclyl ring" refers to an 8- to 10-membered fused bicyclic heteroaryl formed by a 5- or 6-membered monocyclic heteroaryl ring fused with a 5- or 6-membered monocyclic heterocyclyl ring, that is, a fused 5- or 6-membered monocyclic heterocyclyl ring is formed by two adjacent substituent groups on the 5- or 6-membered monocyclic heteroaryl with the ring atom to which they are attached, and the 5- or 6-membered monocyclic heterocyclyl ring is defined as above.

As used herein, the term "8- to 10-membered heteroaryl-cycl*o*alkyl" or "8- to 10-membered heteroaryl-cycloalkyl ring" refers to an 8- to 10-membered fused bicyclic heteroaryl formed by a 5- or 6-membered monocyclic heteroaryl ring fused with a 5- or 6-membered monocyclic cycloalkyl ring, that is, a fused 5- or 6-membered monocyclic cycloalkyl ring is formed by two adjacent substituent groups on the 5- or 6-membered monocyclic heteroaryl with the ring atom to which they are attached, and the 5- or 6-membered monocyclic cycloalkyl ring is defined as above.

Non-limiting examples of "8- to 10-membered heteroaryl-heterocyclyl" or "8-to 10-membered heteroaryl-cycloalkyl" include: 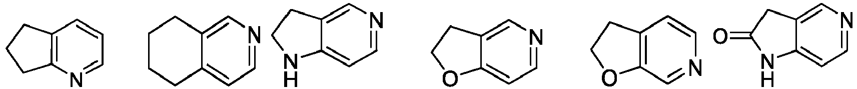 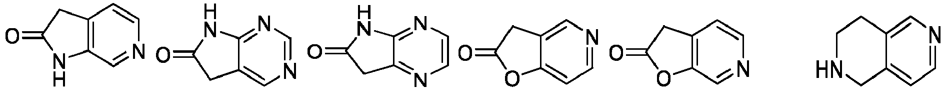 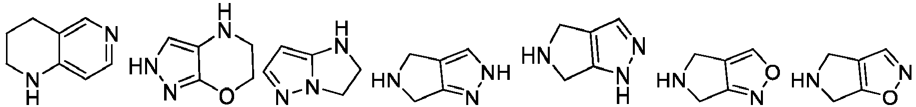 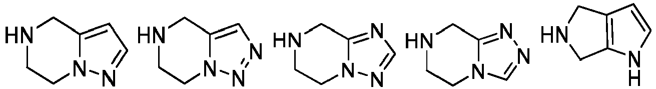 . The groups can be attached to the rest of the molecule by any one of the suitable ring atoms.

In the present disclosure, the various heteroaryl can be substituted or unsubstituted, and when substituted, the substituent is preferably one or more substituent groups described in the present disclosure.

As used herein, "3- to 6-membered saturated or partially unsaturated monocycle" refers to a saturated or partially unsaturated all-carbon monocycle containing 3 to 6 ring atoms. The ring carbon atoms of the monocycle can be optionally substituted by 1, 2 or 3 oxo to form a cycloketone structure. Examples of 3- to 6-membered saturated or partially unsaturated monocycle include (but are not limited to): cyclopropyl ring, cyclobutyl ring, cyclopentyl ring, cyclopentenyl ring, cyclohexyl ring, cyclohexenyl ring, cyclohexenyl ring, cyclohexadienyl ring, cyclobutanone, cyclobutan-1,2-dione, cyclopentanone, cyclopentan-1,3-dione, cyclohexanone, cyclohexan-1,3-dione, etc.

As used herein, "3- to 6-membered saturated or partially unsaturated monocyclic heterocycle" refers to a 3- to 6-membered monocycle in which 1, 2 or 3 carbon atoms are substituted by heteroatoms selected from nitrogen, oxygen or S(=O)_{m'} (wherein m' is an integer from 0 to 2), excluding ring moieties of -O-O-, -O-S- or -S-S-, and the rest ring atoms are carbon; preferably 4- to 6-membered, more preferably 5-to 6-membered. The ring carbon atoms of the monocyclic heterocycle can be optionally substituted by 1, 2 or 3 oxo to form a cycloketone, cyclolactone or cyclolactam structure. Examples of 3- to 6-membered saturated or partially unsaturated monocyclic heterocycles include (but are not limited to) aziridine, oxirane, azetidine, oxetane, tetrahydrofuran, tetrahydrothiophene, tetrahydropyrrole, piperidine, pyrroline, oxazolidine, piperazine, dioxolane, dioxane, morpholine, thiomorpholine, thiomorpholine-1,1-dioxide, tetrahydropyran, 1,2-dihydroazacyclobutadiene, 1,2-dihydrooxacyclobutadiene, 2,5-dihydro-1*H*-pyrrole, 2,5-dihydrofuran, 2,3-dihydrofuran, 2,3-dihydro-1*H*-pyrrole, 3,4-dihydro-2*H*-pyran, 1,2,3,4-tetrahydropyridine, 3,6-dihydro-2*H*-pyran, 1,2,3,6-tetrahydropyridine, etc.

As used herein, the term "hydroxyl" refers to -OH.

As used herein, the term "hydroxymethyl" refers to -CH₂OH, and "hydroxyethyl" refers to - CH₂CH₂OH or -CH(OH)CH₃.

As used herein, the term "cyanomethyl" refers to -CH₂CN, and "cyanoethyl" refers to -CH₂CH₂CN or -CHCNCH₃.

As used herein, the term "amino" refers to -NH₂.

As used herein, the term "cyano" refers to -CN.

As used herein, the term "nitro" refers to -NO₂.

As used herein, the term "benzyl" refers to -CH₂-benzene.

As used herein, the term "oxo" refers to =O.

As used herein, the term "carboxyl" refers to -C(O)OH.

As used herein, the term "carboxylate group" refers to -C(O)O(alkyl) or -C(O)O(cycloalkyl).

As used herein, the term "acetyl" refers to -COCH₃

As used herein, the term "substituted" refers to any one or more hydrogen atoms at a particular atom being substituted by a substituent, which can include heavy hydrogen and variants of hydrogen as long as the valence state of the particular atom is normal and the substituted compound is stable. When the substituent is an oxo (i.e., =O), it means that two hydrogen atoms are substituted. The substitution of oxo will not occur on aryl. The term "optionally substituted" or "optionally be substituted" means that a group may be substituted and may also not be substituted. Unless stated otherwise, the types and the number of substituents can be chosen arbitrarily provided that they can be achieved chemically.

When any variable (e.g., R) occurs in the composition or structure of a compound once or more than once, it is independently defined at each occurrence. Therefore, for example, if a group is substituted by 0 to 2 R groups, the group can be optionally substituted by two R groups at most, and R can be independently selected in each case. In addition, combinations of substituents and/or variants thereof are allowable only when such combinations can result in stable compounds.

### Preparation method

The compound of formula (I) of the present disclosure can be prepared by using a synthetic method known in the art or a combination of a method known in the art and the method described in the present disclosure. The solvents, temperatures and other reaction conditions given herein are exemplary and can be varied according to methods well known in the art. The compounds of the embodiments described in the present disclosure can be synthesized according to their specific structures using appropriate starting materials according to the methods described in the embodiments, and can also be synthesized by using methods similar to those described in the embodiments. The starting materials used to synthesize the compounds of the embodiments of the present disclosure can be prepared by known synthetic methods or analogous methods described in the literature or obtained from commercial sources. Compounds can be further resolved into stereoisomers by methods well known in the art, such as crystallization, chromatography, etc., as required, and the resolution conditions can be easily obtained by those skilled in the art through conventional means or limited experiments.

For example, compound 8 can be synthesized by the following routes.

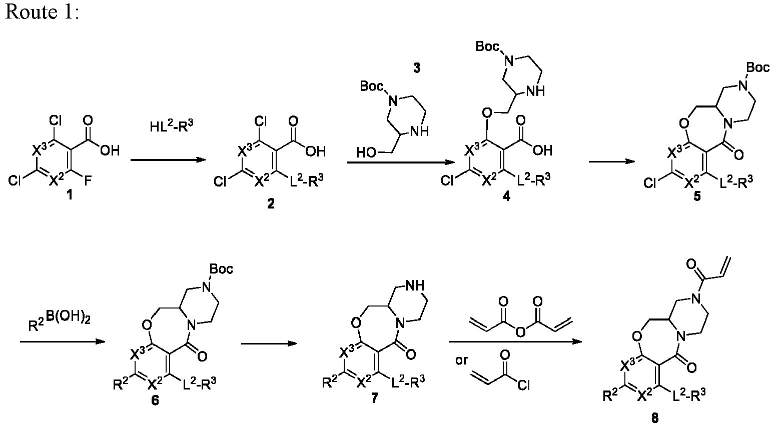

First, carrying out a condensation reaction between compound 1 and HL²R³ to form compound 2; then carrying out a condensation reaction between compound 2 and compound 3 to form compound 4; then carrying out a cyclization reaction of compound 4 to form compound 5; then carrying out a coupling reaction between compound 5 and R²B(OH)₂ to form compound 6; finally, carrying out a deprotection reaction of compound 6 to form compound 7, and then carrying out a displacement reaction between compound 7 and acryloyl chloride or acrylic anhydride to form compound 8. In each formula, X², X³, L², R², R³ are defined as above.

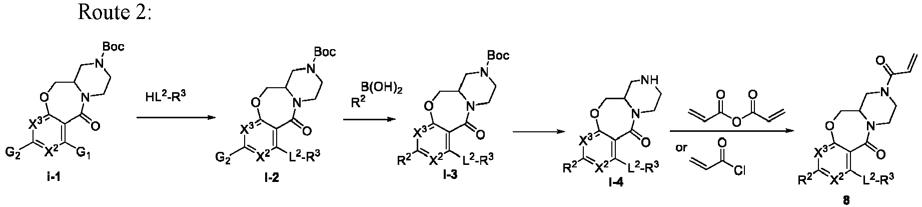

First, carrying out a condensation reaction between compound i-1 and HL²R³ to form compound i-2; then carrying out a coupling reaction between compound i-2 and R²B(OH)₂ to form compound i-3; finally, carrying out a deprotection reaction of compound i-3 to form compound i-4, and then carrying out a displacement reaction between compound i-4 and acryloyl chloride or acrylic anhydride to form compound 8. In each formula, G₂, G₁ are each independently leaving groups, such as fluorine, chlorine or bromine; X², X³, L², R², R³ are defined as above.

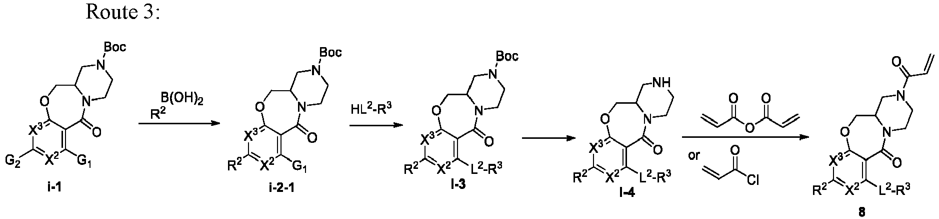

First, carrying out a coupling reaction between compound i-1 and R²B(OH)₂ to form compound i-2-1; then carrying out a condensation reaction between compound i-2-1 and HL²R³ to form compound i-3; finally, carrying out a deprotection reaction of compound i-3 to form compound i-4, and then carrying out a displacement reaction between compound i-4 and acryloyl chloride or acrylic anhydride to form compound 8. In each formula, G₂, G₁ are each independently leaving groups, such as fluorine, chlorine or bromine; X², X³, L², R², R³ are defined as above.

For another example, compound 12 can be synthesized by the following route.

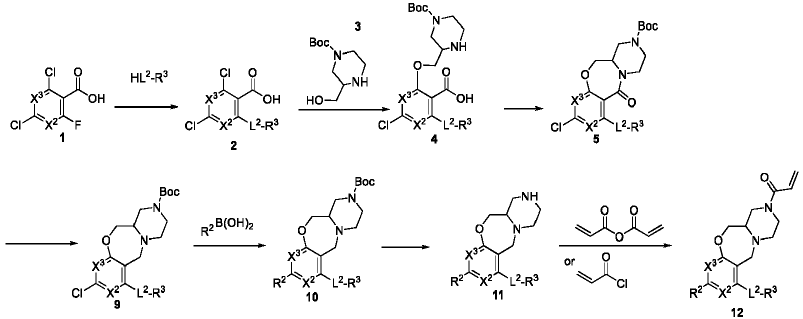

First, carrying out a condensation reaction between compound 1 and HL²R³ to form compound 2; then carrying out a condensation reaction between compound 2 and compound 3 to form compound 4; then carrying out a cyclization reaction of compound 4 to form compound 5; then carrying out a reduction reaction of compound 5 with a reducing agent to form compound 9; then carrying out a coupling reaction between compound 9 and R²B(OH)₂ to form compound 10; finally, carrying out a deprotection reaction of compound 10 to form compound 11, and then carrying out a displacement reaction between compound 11 and acryloyl chloride or acrylic anhydride to form compound 12. In each formula, X², X³, L², R², R³ are defined as above.

All the above steps are carried out in inert solvent. The so-called inert solvent means that the solvent only serves as a reaction medium and does not participate in the reaction. The choice of the inert solvent can be conventionally selected for each reaction.

As a further illustration, the compound of formula (I) of the present disclosure can be synthesized by the following method, wherein the solvent, temperature and other reaction conditions in each step can be the same or similar to those described in the following embodiments, or use reaction conditions known in the art.

### DETAILED DESCRIPTION OF THE PREFERRED EMBODIMENT

The compounds of the present disclosure can be prepared by a variety of synthetic methods known to those skilled in the art, including the specific implementations listed below, the implementations formed by their combination with other chemical synthesis methods, and equivalent alternatives known to those skilled in the art, preferred implementations include but are not limited to the embodiments of the present disclosure.

The present disclosure will be described in detail below by way of embodiments, but the scope of the present disclosure is not limited thereto. The present disclosure is described in detail herein, and specific embodiments thereof have also been disclosed. For those skilled in the art, it is obvious that various changes and improvements can be made to the specific embodiments of the present disclosure without departing from the spirit and scope of the present disclosure. Embodiments without indicating specific conditions are carried out according to the conventional conditions or conditions suggested by the manufacturer. The reagents or instruments used without indicating the manufacturer are conventional products that are commercially available.

Unless otherwise specified, the configurations of the isomers obtained by resolution (e.g., chiral resolution, chromatographic plate, etc.) in the following embodiments are arbitrarily specified. TLC in the following embodiments indicates thin-layer chromatography plate. Unless otherwise specified, the preparative HPLC used in the following embodiments may use the following conditions: column: Ultimate XB-C18, 50^{∗}250 mm, 10 µm; mobile phase system: A: purified water (0.1% NH₄HCO₃); B: pure acetonitrile (0.1% NH₄HCO₃); flow rate: 80 mL/min; gradient: B/A=20%-100%; column temperature: room temperature.

The reagents used in the following embodiments are abbreviated as follows: THF: tetrahydrofuran; DMSO: dimethyl sulfoxide; PE: petroleum ether; EtOAc: ethyl acetate; DCM: dichloromethane; MeOH: methanol; SPhos is 2-dicyclohexylphosphino-2',6'-dimethoxy-1,1'-biphenyl; SPhos-Pd-G2 is chloro(2-dicyclohexylphosphino-2',6'-dimethoxy-1,1'-biphenyl)[2-(2'-amino-1,1'-biphenyl)]palladium II; ACN is acetonitrile; IPA is isopropylamine; DMA is dimethylamine; TFA is trifluoroacetic acid; NH₄Cl is ammonium chloride; NaHMDS is sodium bis(trimethylsilyl)amide; LiHMDS is lithium bis(trimethylsilyl)amide.

### Preparation embodiment 1 Synthesis of intermediates 1a and 1b

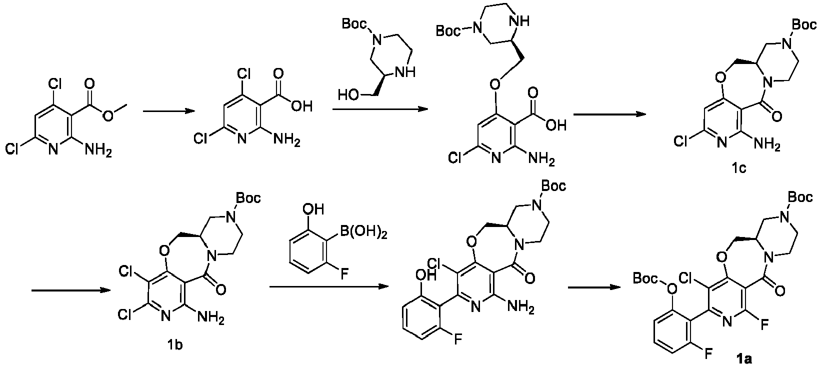

Step 1: Methyl 2-amino-4,6-dichloronicotinate (10 g, 45.2 mmol), 30 mL of THF, 30 mL of methanol and 30 mL of water were added to a 250 mL flask, stirred at room temperature for 3 hours. After the reaction was completed, the mixture was cooled in an ice-water bath. Dilute hydrochloric acid (3 M, 35 mL) was added dropwise to the reaction solution to adjust pH to 3-4, and a solid was precipitated. The mixture was filtered, and the solid was dissolved in ethyl acetate, washed with water and saturated brine in turn, and dried over anhydrous sodium sulfate. The mixture was concentrated and dried to obtain 2-amino-4,6-dichloronicotinic acid (8.3 g, crude product). ES-API: [M+H]⁺=206.9.

Step 2: 60% sodium hydride (4.11 g, 103 mmol), *tert*-butyl (R)-3-(hydroxymethyl)piperazine-1-carboxylate (5.55 g, 25.7 mmol) and THF (100 mL) were added to a 250 mL flask. The reaction was cooled to 0°C, and after stirring, a THF suspension (30 mL) of 2-amino-4,6-dichloronicotinic acid (5.3 g, 25.7 mmol) was added dropwise thereto. The reaction was stirred in an oil bath at 70°C for 3 hours. The completion of reaction was detected by LC-MS. The reaction solution was poured into ice-water, and diluted hydrochloric acid (3M) was added dropwise to the reaction solution to adjust pH to 7. The mixture was concentrated and then added with 100 mL of THF, filtered after stirring, and the filtrate was dried and concentrated to obtain (*R*)-2-amino-4-((4-(*tert*-butoxycarbonyl)piperazin-2-yl)methoxy)-6-chloronicotinic acid (11 g, crude product) as a yellow solid. ES-API: [M+H]⁺=387.2.

Step 3: (*R*)-2-Amino-4-((4-(*tert*-butoxycarbonyl)piperazin-2-yl)methoxy)-6-chloronicotinic acid (11 g, crude product), diisopropylethylamine (14 mL) and dichloromethane (120 mL) were added to a 250 mL flask. Propylphosphonic anhydride solution (28.5 mL, 50%w/w ethyl acetate solution) was added dropwise thereto. The mixture was reacted at room temperature for 1 hour. The completion of reaction was detected by LC-MS. 50 mL of dichloromethane was added to the reaction, then the mixture was washed with 100 mL of water and 100 mL of sodium bicarbonate, respectively. The organic phase was dried, concentrated, and purified by silica gel column chromatography to obtain (*R*)-1-amino-8-*tert-*butoxycarbonyl-3-chloro-6,6a,7,8,9,10-hexahydro-12*H*-pyrazino[2,1-*c*]pyrido[3,4-*f*][1,4]oxazepin-12-one (5.2 g, 3-step yield of 31%) as a white solid. ES-API: [M+H]⁺=369.3.

Step 4: (*R*)-1-Amino-8-*tert*-butoxycarbonyl-3-chloro-6,6a,7,8,9,10-hexahydro-12*H*-pyrazino[2,1-*c*]pyrido[3,4-*f*][1,4]oxazepin-12-one (1.6 g, 4.35 mmol), N-chlorosuccinimide (694 mg, 5.22 mmol) and acetonitrile (50 mL) were added to a 100 mL flask. The reaction was stirred at 90°C for 2 hours. The reaction solution was added with aqueous sodium thiosulfate solution, extracted with ethyl acetate, washed with sodium bicarbonate and saturated brine in turn, and the organic phase was dried and concentrated to obtain (*R*)-1-amino-8-*tert*-butoxycarbonyl-3,4-dichloro-6,6a,7,8,9,10-hexahydro-12*H*-pyrazino[2,1-c]pyrido[3,4-*f*][1,4]oxazepin-12-one (1.8 g, crude product). ES-API: [M+H]⁺=403.1.

Step 5: (*R*)-1-Amino-8-*tert*-butoxycarbonyl-3,4-dichloro-6,6a,7,8,9,10-hexahydro-12*H-*pyrazino[2,1-*c*]pyrido[3,4-*f*][1,4]oxazepin-12-one (1.8 g, 4.48 mmol), 2-fluoro-6-hydroxyphenylboronic acid (2.79 g, 17.9 mmol), Pd(PPh₃)₄ (52 mg, 0.448 mmol), potassium carbonate (1.85 g, 13.44 mmol), 100 mL of dioxane and 20 mL of water were added to a 100 mL reaction flask. The reaction was stirred in an oil bath at 90°C for 1 hour, and the reaction was stopped. The reaction solution was added with 150 mL of water, extracted three times with 150 mL of ethyl acetate, and the organic phase was dried, concentrated, and purified by flash silica gel column (EtOAc/PE: 0-60%) to obtain (6a*R*)-1-amino-8-tert-butoxycarbonyl-4-chloro-3-(2-fluoro-6-hydroxyphenyl)-6,6a,7,8,9,10-hexahydro-12*H*-pyrazino [2,1-c]pyrido [3,4-*f*][1,4]oxazepin-12-one (1.71 g, 2-step yield of 80%) as a yellow solid. ES-API: [M+H]⁺=479.1.

Step 6: (6a*R*)-1-Amino-8-*tert*-butoxycarbonyl-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (2.3 g, 4.803 mmol) and 70% pyridine hydrofluoric acid solution (4.6 mL) were added to a 50 mL reaction flask. Sodium nitrite (0.66 g, 9.605 mmol) was added in batches under an ice-water bath, and the mixture was stirred for 20 minutes under an ice-water bath to complete the reaction. 5 mL of ice-cold THF was added thereto to dilute, and the pre-configured mixed solution of NaOH (9.2 g) and (Boc)₂O (3 mL) in water (30 mL) and THF (30 mL) was slowly added dropwise thereto under the cooling of ice-water bath. After the dropwise addition was completed, the mixture was stirred at room temperature for 1 hour. The mixture was extracted with ethyl acetate (2×50 mL), and the organic phases were combined, washed with water, 1M hydrochloric acid, sodium bicarbonate and saturated brine in turn, dried over anhydrous sodium sulfate, filtered and concentrated, and then subjected to silica gel column chromatography (EtOAc/PE: 0-30%) to obtain (6a*R*)-8-*tert-*butoxycarbonyl-3-(2-(((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (1.3 g, 46%). ES-API: [M+H]⁺=582.1.

### Preparation embodiment 2 Synthesis of intermediate 2a

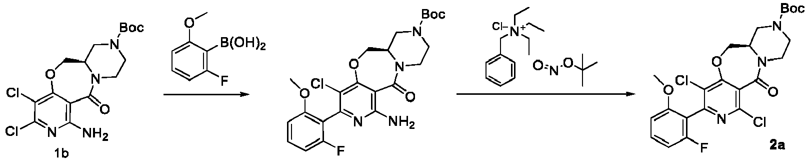

Step 1: tert-Butyl (R)-1-amino-3,4-dichloro-12-oxo-6a,7,9,10-tetrahydro-12H-pyrazino[2,1-c]pyrido[3,4-*f*][1,4]oxazepine-8(6*H*)-carboxylate (4.06 g, 10.099 mmol), (2-fluoro-6-methoxyphenyl)boronic acid (6.01 g, 35.34 mmol), tetrakis(triphenylphosphine)palladium (1.15 g, 0.9956 mmol), sodium carbonate (5.0 g, 47.17 mmol), dioxane (100 mL) and water (20 mL) were added to a microwave tube, and reacted at 100°C for 5 hours under nitrogen protection. After the reaction was completed, the mixture was cooled to room temperature, added with ethyl acetate (200 mL), washed with saturated brine (2^{∗}80 mL). The organic phase was dried over anhydrous sodium sulfate, filtered, and the filtrate was concentrated and purified by flash silica gel column (EtOAc/PE: 0-100%) to obtain *tert*-butyl (6a*R*)-1-amino-4-chloro-3-(2-fluoro-6-methoxyphenyl)-12-oxo-6a,7,9,10-tetrahydro-12*H*-pyrazino[2,1-c]pyrido[3,4-*f*][1,4]oxazepine-8(6*H*)-carboxylate (4.05 g, yield of 81.5%). ES-API: [M+H]⁺=493.1.

Step 2: *tert*-Butyl (6a*R*)-1-amino-4-chloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (4.05g, 8.230mmol) was added to dichloromethane (80 mL) at room temperature. After dissolution was completed, benzyltriethylammonium chloride (18.74 g, 82.30 mmol) was added thereto, stirred at room temperature for 5 minutes, and then *tert*-butyl nitrite (3.82 g, 37.03 mmol) was added thereto, stirred at room temperature overnight. After the reaction was completed, dichloromethane (100 mL) was added to the system, and washed with saturated aqueous sodium bicarbonate solution (2^{∗}80 mL). The organic phase was dried over anhydrous sodium sulfate, concentrated and purified by flash silica gel column (EtOAc/PE: 0-50%) to obtain *tert*-butyl (6a*R*)-1,4-dichloro-3-(2-fluoro-6-methoxyphenyl)-12-oxo-6a,7,9,10-tetrahydro-12*H-*pyrazino[2,1-*c*]pyrido[3,4-*f*][1,4]oxazepine-8(6*H*)-carboxylate (3.17 g, yield of 75%). ES-API: [M+H]⁺=512.2.

### Preparation embodiment 3 Synthesis of intermediate 3,5,5-trimethylpyrrolidin-3-ol

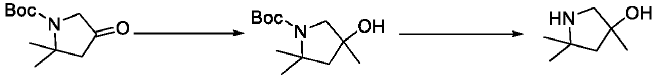

Step 1: *tert*-Butyl 2,2-dimethyl-4-oxopyrrolidine-1-carboxylate (311 mg, 1.46 mmol) and anhydrous THF (20 mL) were added to a flask, and methylmagnesium bromide (1.46 mL, 4.38 mmol, 3 M) was added under an ice-water bath under nitrogen protection. The mixture was reacted overnight at room temperature. 10 mL of saturated ammonium chloride solution was added dropwise to the reaction solution under an ice-water bath and extracted three times with ethyl acetate. The organic phase was dried and concentrated, and the crude product was purified by flash silica gel column (EtOAc/PE: 0-50%) to obtain *tert*-butyl 4-hydroxy-2,2,4-trimethylpyrrolidine-1-carboxylate (330 mg, 98%) as a white solid.

Step 2: *tert*-Butyl 4-hydroxy-2,2,4-trimethylpyrrolidine-1-carboxylate (330 mg, 1.44 mmol), 3 mL of dichloromethane and 3 mL of trifluoroacetic acid were added to a flask. The mixture was stirred at room temperature for 1 hour. The reaction solution was concentrated to obtain 3,5,5-trimethylpyrrolidin-3-ol (423 mg, crude product), and the crude product was directly subjected to the next step of reaction.

### Preparation embodiment 4 Synthesis of intermediate (3S)-3-methylhexahydropyrrolo[1,2-a]pyrazin-6(2H)-one

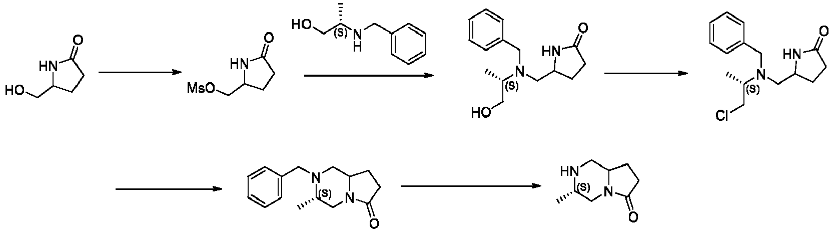

Step 1: Triethylamine (1.3 g, 13.04 mmol) was added to a solution of 5-(hydroxymethyl)pyrrolidin-2-one (1 g, 8.69 mmol) in dichloromethane (20 mL), and then methanesulfonyl chloride (1.2 g, 10.43 mmol) was added thereto at 0°C. The mixture was reacted at 0°C for 1 hour, added with water (20 mL) to quench the reaction, extracted with dichloromethane (20 mL^{∗}3), and the organic phases were combined, dried, and concentrated to obtain (5-oxopyrrolidin-2-yl)methyl methanesulfonate (1080 mg, 64%). ES-API: [2M+H]⁺= 387.16.

Step 2: (5-Oxopyrrolidin-2-yl)methyl methanesulfonate (880 mg, 4.56 mmol) and (S)-2-(benzylamino)-1-propanol (3 g, 18.24 mmol) were reacted at 130°C for 1 hour under microwave irradiation, and water (10 mL) was added to quench the reaction, and the mixture was extracted with dichloromethane (10 mL^{∗}3). The organic phases were combined, dried, concentrated, and the crude product was purified by column chromatography (DCM:MeOH=95/5) to obtain 5-((benzyl((*S*)-1-hydroxypropan-2-yl)amino)methyl)pyrrolidin-2-one (612 mg, 51%). ES-API: [M+H]⁺=263.3.

Step 3: Triethylamine (1.3 g, 13.04 mmol) was added to a solution of 5-((benzyl((*S*)-1-hydroxypropan-2-yl)amino)methyl)pyrrolidin-2-one (612 mg, 2.33 mmol) in dichloromethane (10 mL), and then methanesulfonyl chloride (1.2 g, 10.43 mmol) was added dropwise thereto. After reacting at room temperature for 4 hours, the solvent was removed and water (10 mL) was added to quench the reaction. The mixture was extracted with dichloromethane (10 mL^{∗}3), and the organic phases were combined and dried, and concentrated to obtain 5-((benzyl((*S*)-1-chloropropan-2-yl)amino)methyl)pyrrolidin-2-one (650 mg, crude product, yellow oil). ES-API: [M+H]⁺= 281.1.

Step 4: Sodium hydride (241 mg, 6.03 mmol) was added to a solution of 5-((benzyl((*S*)-1-chloropropan-2-yl)amino)methyl)pyrrolidin-2-one (650 mg, 2.32 mmol) in acetonitrile (10 mL). Then the mixture was reacted at 80°C for 4 hours and quenched with ice-water. The reaction solution was extracted with ethyl acetate (10 mL^{∗}3), and the combined organic phases were washed with saturated brine, dried and concentrated to obtain the crude product. The crude product was purified by column chromatography (dichloromethane/methanol=10:1) to obtain (3*S*)-2-benzyl-3-methylhexahydropyrrolo[1,2-*a*]pyrazin-6(2*H*)-one (265 mg, 47%, yellow oil). ES-API: [M+H]⁺=245.2.

Step 5: Palladium hydroxide (44 mg) and a few drops of glacial acetic acid were added to a solution of (3*S*)-2-benzyl-3-methylhexahydropyrrolo[1,2-*a*]pyrazin-6(2*H*)-one (220 mg, 0.902 mmol) in methanol (10 mL). The reaction was carried out at 45°C for 3 hours under a hydrogen atmosphere. The mixture was filtered and concentrated to obtain (3*S*)-3-methylhexahydropyrrolo[1,2-*a*]pyrazin-6(2*H*)-one (245 mg, crude product, yellow oil), which was directly used in the synthesis of Z330-1. ES-API: [M+H]⁺=155.1.

### Preparation embodiment 5 Synthesis of a mixture of intermediates 3a and 3b

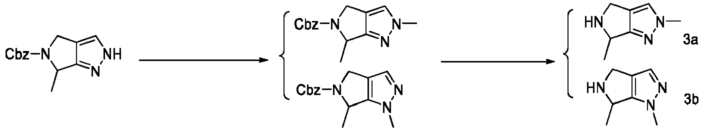

Step 1: Methyl (*S*)-2-(((benzyloxy)carbonyl)amino)propionate (5 g, 21.09 mmol), methyl acrylate (2.0 g, 23.2 mg) and dry THF (80 mL) were added to a 250 mL reaction flask, cooled to 0°C in an ice-water bath. Sodium hydride (844 mg, 21.09 mmol) was slowly added thereto, stirred for 30 minutes, raised to room temperature and heated to 80°C to react for 1 hour. After the reaction was completed, the mixture was cooled to room temperature, poured into ice-water, extracted with ethyl acetate, washed with water and saturated brine in turn, dried over anhydrous sodium sulfate, filtered, concentrated, and purified by silica gel column to obtain 1-benzyl 3-methyl 5-methyl-4-oxopyrrolidine-1,3-dicarboxylate (3 g, yield of 50%). ES-API: [M+H]⁺=292.1.

Step 2: 1-Benzyl 3-methyl 5-methyl-4-oxopyrrolidine-1,3-dicarboxylate (3 g, 10.3 mmol), sodium chloride (2.39 g, 41.2 mg), DMF (20 mL) and water (7 mL) were added to a 100 mL reaction flask, heated to 120°C and reacted for 5 hours. After the reaction was completed, the mixture was cooled to room temperature, poured into ice-water, extracted with ethyl acetate, washed with water and saturated brine in turn, dried over anhydrous sodium sulfate, filtered, concentrated, and purified by silica gel column to obtain benzyl 2-methyl-3-oxopyrrolidine-1-carboxylate (1.2 g, yield of 52%). ES-API: [M+H]⁺=234.1.

Step 3: Benzyl 2-methyl-3-oxopyrrolidine-1-carboxylate (1.2 g, 5.15 mmol) and *N,N-*dimethylformamide dimethyl acetal (6 mL) were added to a 25 mL reaction flask, heated to 80°C and reacted for 3 hours. The reaction was completed, and the mixture was concentrated, and purified by silica gel column to obtain benzyl 4-((dimethylamino)methylene)-2-methyl-3-oxopyrrolidine-1-carboxylate (1.1 g, yield of 74%). ES-API: [M+H]⁺=289.3.

Step 4: Benzyl 4-((dimethylamino)methylene)-2-methyl-3-oxopyrrolidine-1-carboxylate (1.1 g, 3.82 mmol), 80% hydrazine hydrate (0.5 mL) and ethanol (8 mL) were added to a 20 mL microwave reaction flask, heated to 90°C and reacted for 2 hours under microwave irradiation. After the reaction was completed, the mixture was cooled to room temperature, poured into ice-water, extracted with ethyl acetate, washed with water and saturated brine in turn, dried over anhydrous sodium sulfate, filtered, concentrated to obtain benzyl 6-methyl-4,6-dihydropyrrolo[3,4-c]pyrazole-5(2*H*)-carboxylate (1.0 g, crude product). ES-API: [M+H]⁺=258.1.

Step 5: Benzyl 6-methyl-4,6-dihydropyrrolo[3,4-*c*]pyrazole-5(2*H*)-carboxylate (1.0 g, crude product) was added to a 50 mL reaction flask, cooled to 0°C in an ice-water bath, and sodium hydride (776 mg, 19.4 mmol) was slowly added thereto. After stirring for 30 minutes, iodomethane (2.76 g, 19.4 mmol) was added thereto, and the mixture was heated to room temperature and reacted for 1 hour. After the reaction was completed, the mixture was poured into ice-water, extracted with ethyl acetate, washed with water and saturated brine in turn, dried over anhydrous sodium sulfate, filtered, concentrated, and purified by silica gel column to obtain a mixture of benzyl 2,6-dimethyl-4,6-dihydropyrrolo[3,4-*c*]pyrazole-5(2*H*)-carboxylate and benzyl 1,6-dimethyl-4,6-dihydropyrrolo[3,4-*c*]pyrazole-5(1*H*)-carboxylate (550 mg, yield of 54%). ES-API: [M+H]⁺=272.1.

Step 6: The above mixture (550 mg), 10% palladium on carbon (250 mg) and anhydrous methanol (20 mL) were added to a 50 mL reaction flask, and reacted at room temperature for 1 hour under hydrogen protection. After the reaction was completed, the mixture was filtered and concentrated to obtain a mixture of 2,6-dimethyl-2,4,5,6-tetrahydropyrrolo[3,4-c]pyrazole and 1,6-dimethyl-1,4,5,6-tetrahydropyrrolo[3,4-*c*]pyrazole (190 mg, yield of 68%), which was directly used to synthesize a mixture of Z291-2 and Z292-2. ES-API: [M+H]⁺=138.1.

### Preparation embodiment 6 Synthesis of intermediates INT-A, INT-B

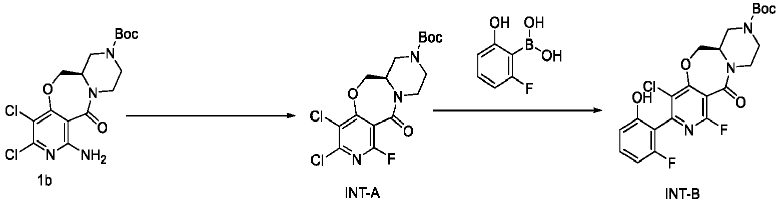

Step 1: *tert*-Butyl (R)-1-amino-3,4-dichloro-12-oxo-6a,7,9,10-tetrahydro-12*H*-pyrazino[2,1-*c*]pyrido[3,4-*f*][1,4]oxazepine-8(6*H*)-carboxylate (12 g, 29.8 mmol) and 24 mL of pyridine hydrofluoride solution were added to a 50 mL flask, dissolved, and then sodium nitrite solid (2.6 g, 37.7 mmol) was slowly added thereto under an ice-water bath, stirred for 10-20 minutes to detect that the reaction was completed, added to a pre-configured solution (48 g NaOH, 200 mL of water, 36 mL of BOCzO, 200 mL of THF) that has been cooled in advance during stirring. The mixture was stirred at room temperature for 30 minutes to 1 hour, and the reaction was detected as complete by mass spectrometry. The mixture was extracted with ethyl acetate, and the pH of the aqueous phase was adjusted to 6-7, and then the organic phase was dried, filtered, concentrated and subjected to column chromatography to obtain *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (INT-A, 9.77 g, 24 mmol). Yield: 80%. ES-API: [M+H]⁺=406.2.

Step 2: *tert*-Butyl (R)-3,4-dichloro-1-fluoro-12-oxo-6a,7,9,10-tetrahydro-12*H*-pyrazino[2,1-*c*]pyrido[3,41][1,4]-8(6*H*)-carboxylate (406 mg, 1.0 mmol), (2-hydroxy-6-fluorophenyl)boronic acid (624 mg, 4.0 mmol), Pd(PPh₃)₄ (115.6 mg, 0.1 mmol), sodium carbonate (324 mg, 3.0 mmol), 10 mL of dioxane and 2 mL of water were added to a 50 mL flask. Under nitrogen protection, the reaction was carried out at 100°C for 2 hours. The reaction solution was added with 30 mL of ethyl acetate, washed with 30 mL of saturated brine for 3 times, dried and concentrated, and purified by flash silica gel column to obtain *tert-*butyl (6a*R*)-4-chloro-1-fluoro-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (INT-B, 350 mg, yield of 73%). ES-API: [M+H]⁺=482.1.

### Preparation embodiment 7 Synthesis of intermediates INT-C, INT-D

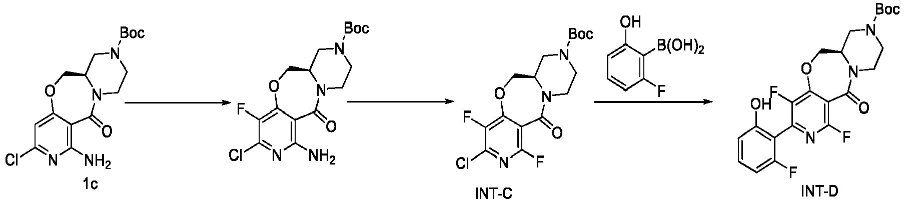

Step 1: tert-Butyl (R)-1-amino-3-chloro-12-oxo-6a,7,9,10-tetrahydro-12H-pyrazino[2,1-*c*]pyrido[3,4-*f*][1,4]oxazepine-8(6*H*)-carboxylate (3 g, 8.17 mmol) was dissolved in 100 mL of mixed solvent (acetonitrile:dichloromethane = 1:1), and 1-chloromethyl-4-fluoro-1,4-diazoniabicyclo[2.2.2]octane bis(tetrafluoroborate) (6 g, 16.5 mmol) was added thereto. The reaction was carried out at room temperature for 16 hours. The mixture was washed with 50 mL of saturated NaHCO₃ aqueous solution and 40 mL of saturated brine, dried, concentrated and filtered through a silica gel column to obtain a crude product (2.35 g, purity of 33%) of *tert*-butyl (*R*)-1-amino-3-chloro-4-fluoro-12-oxo-6a,7,9,10-tetrahydro-12*H*-pyrazino[2,1-*c*]pyrido[3,4-*f*][1,4]oxazepine-8(6*H*)-carboxylate. ES-API: [M+H]⁺=387.1.

Step 2: The crude product (2.35 g) of *tert*-butyl (*R*)-1-amino-3-chloro-4-fluoro-12-oxo-6a,7,9,10-tetrahydro-12*H*-pyrazino[2,1-*c*]pyrido[3,4-*f*][1,4]oxazepine-8(6*H*)-carboxylate and 4.7 mL of pyridine hydrofluoride solution were added to a 50 mL flask, dissolved, and then sodium nitrite solid (500 mg, 7.24 mmol) was slowly added thereto under an ice-water bath, stirred for 10-20 minutes to detect that the reaction was complete, added to a pre-configured solution (9.8 g of NaOH, 40 mL of water, 7 mL of BOC₂O, 40 mL of THF) that has been cooled in advance during stirring. The mixture was stirred at room temperature for 30 minutes to 1 hour, and the reaction was detected as complete by mass spectrometry. The mixture was extracted with ethyl acetate, and the pH of the aqueous phase was adjusted to 6-7, and then the organic phase was dried, concentrated and subjected to column chromatography to obtain *tert*-butyl (6a*R*)-3-chloro-1,4-difluoro-12-oxo-6a,7,9,10-tetrahydro-12*H*-pyrazino[2,1-*c*]pyrido[3,4-*f*][1,4]oxazepine-8(6*H*)-carboxylate (INT-C, 0.45 g, 1.15 mmol), with a 2-step yield of 14%. ES-API: [M-56]⁺ =334.0.

Step 3: *tert*-Butyl (6a*R*)-3-chloro-1,4-difluoro-12-oxo-6a,7,9,10-tetrahydro-12*H*-pyrazino[2,1-*c*]pyrido[3,4-*f*][1,4]oxazepine-8(6*H*)-carboxylate (174 mg, 0.448 mmol), 2-fluoro-6-hydroxyphenylboronic acid (0.279 g, 1.79 mmol), Pd(PPh₃)₄ (5.2 mg, 0.045 mmol), potassium carbonate (0.185 g, 1.34 mmol), 10 mL of dioxane and 2 mL of water were added to a 100 mL reaction flask. After reacting at 100°C for 2 hours, the mixture was added with 15 mL of water, extracted three times with 15 mL of ethyl acetate, and the organic phase was dried, concentrated, and purified by a flash silica gel column (EtOAc/PE: 0-60%) to obtain *tert*-butyl (*R*)-1,4-difluoro-3-(2-fluoro-6-hydroxyphenyl)-12-oxo-6a,7,9,10-tetrahydro-12*H-*pyrazino[2.1-*c*]pyrido|3.4-*f*][1.4|oxazepine-8(6*H*)-carboxylatc (INT-D, 0.171 g, 82%). Yellow solid, ES-API: [M+H]⁺=466.1.

### Preparation embodiment 8 Synthesis of a mixture of intermediates 4a and 4b

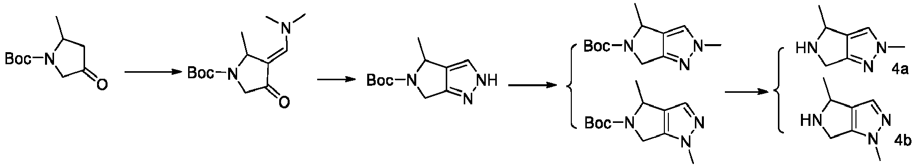

Step 1: *tert*-Butyl 2-methyl-4-oxopyrrolidine-1-carboxylate (1.0 g, 5.02 mmol) and *N,N-*dimethylformamide dimethyl acetal (5 mL) were added to a 25 mL reaction flask, reacted at 80°C for 3 hours. The reaction was completed, and the mixture was concentrated, and purified by column chromatography to obtain *tert*-butyl 3-((dimethylamino)methylene)-2-methyl-4-oxopyrrolidine-1-carboxylate (700 mg, yield of 55%). ES-API: [M+H]⁺=255.2.

Step 2: *tert*-Butyl 3-((dimethylamino)methylene)-2-methyl-4-oxopyrrolidine-1-carboxylate (700 mg, 2.75 mmol), 80% hydrazine hydrate (0.5 mL) and ethanol (5 mL) were added to a 20 mL microwave reaction flask, reacted at 90°C for 2 hours under microwave irradiation. After the reaction was completed, the mixture was cooled to room temperature, poured into ice-water, extracted with ethyl acetate, washed with water and saturated brine in turn, dried over anhydrous sodium sulfate, filtered, concentrated to obtain a crude product of *tert*-butyl 4-methyl-4,6-dihydropyrrolo[3,4-*c*]pyrazole-5(4*H*)-carboxylate (370 mg, yield of 60%). ES-API: [M+H]⁺=224.1.

Step 3: *tert*-Butyl 4-methyl-4,6-dihydropyrrolo[3,4-*c*]pyrazole-5(2*H*)-carboxylate (370 mg, 1.65 mmol) was added to a 50 mL reaction flask, cooled to 0°C in an ice-water bath, and 60% sodium hydride (330 mg, 8.25 mmol) was slowly added thereto. After stirring for 30 minutes, iodomethane (1.17 g, 8.25 mmol) was added thereto, reacted at room temperature for 1 hour. After the reaction was completed, the mixture was poured into ice-water, extracted with ethyl acetate, washed with water and saturated brine in turn, dried over anhydrous sodium sulfate, filtered, concentrated, and purified by silica gel column to obtain a mixture of *tert*-butyl 1,4-dimethyl-4,6-dihydropyrrolo[3,4-*c*]pyrazole-5(4*H*)-carboxylate and *tert*-butyl 2,4-dimethyl-4,6-dihydropyrrolo[3,4-*c*]pyrazole-5(1*H*)-carboxylate (312 mg, yield of 80%). ES-API: [M+H]⁺=238.1.

Step 4: The above mixture (312 mg, 1.31 mmol) was added to a 25 mL reaction flask, and 4 M HCl (5 mL, MeOH solution) was added thereto, and reacted at room temperature for 1 hour. After the reaction was completed, the mixture was concentrated to obtain a mixture of 1,4-dimethyl-1,4,5,6-tetrahydropyrrolo[3,4-*c*]pyrazole and 2,4-dimethyl-2,4,5,6-tetrahydropyrrolo[3,4-c]pyrazole (161 mg, yield of 90%), which was directly used to synthesize a mixture of Z345 and Z346. ES-API: [M+H]⁺=138.1.

### Preparation embodiment 9 Synthesis of a mixture of intermediates 5a and 5b

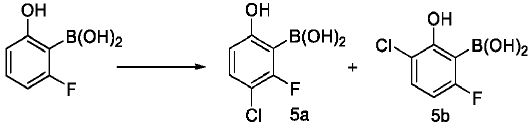

2-Fluoro-6-hydroxyphenylboronic acid (5 g, 32 mmol) was dissolved in acetonitrile (50 mL), and N-chlorosuccinimide (5 g, 32.6 mmol) was added thereto, and the mixture was stirred at room temperature overnight. Water was added to quench the reaction, and the reaction solution was extracted with ethyl acetate, and the organic phases were combined, dried over anhydrous sodium sulfate, concentrated and separated by column chromatography (EtOAc/PE: 0-100%) to obtain a mixture of 3-chloro-2-fluoro-6-hydroxyphenylboronic acid and 3-chloro-6-fluoro-2-hydroxyphenylboronic acid (4.2 g, 68%), which was directly used to synthesize a mixture of Z340 and Z341.

### Preparation embodiment 10 Synthesis of intermediate 6

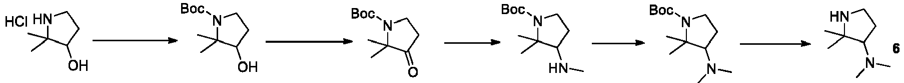

Step 1: 2,2-Dimethylpyrrolidin-3-ol hydrochloride (1 g, 6.6 mmol) was dissolved in dichloromethane (20 mL), and triethylamine (2 g, 19.8 mmol) and di-*tert*-butyl dicarbonate (2.8 g, 13.2 mmol) were added thereto, reacted at room temperature for 2 hours. The reaction solution was extracted with dichloromethane and water, and the organic phase was dried, evaporated to dryness, and purified by column chromatography (PE/EtOAc=5/1) to obtain a product (1.4 g, yield: 95%). (R_{f}=0.6, PE/EtOAc=3/1). ES-API: [M+H-56]⁺=160.2.

Step 2: *tert*-Butyl 3-hydroxy-2,2-dimethylpyrrolidine-1-carboxylate (1.3 g, 6.0 mmol) was dissolved in dichloromethane (30 mL), and Dess-Martin reagent (7.7 g, 18.1 mmol) was added thereto and reacted at room temperature for 2 hours. The reaction solution was quenched with aqueous sodium thiosulfate solution, extracted with dichloromethane, and the organic phase was dried, evaporated to dryness, and purified by column chromatography (PE/EtOAc=20/1, R_{f}=0.5) to obtain a product (1.3 g, yield of 95%).

Step 3: *tert-*Butyl 2,2-dimethyl-3-oxopyrrolidine-1-carboxylate (500 mg, 2.35 mmol) was added to titanium tetraisopropanolate (2 g, 7.0 mmol), and a solution of methylamine (5.9 mL, 11.7 mmol, 2 M) in tetrahydrofuran was added thereto, and the tube was sealed and heated to 60°C and reacted for 8 hours, then reacted at room temperature overnight. Ethanol (6 mL) and sodium cyanoborohydride (739 mg, 11.7 mmol) were added to the reaction solution, and the reaction was carried out at room temperature for 2 hours. The reaction solution was quenched with water, extracted with ethyl acetate, and the organic phase was dried, evaporated to dryness, and purified by column chromatography (ethyl acetate) to obtain a product (67 mg, yield of 12%). (R_{f}=0.3, ethyl acetate). ES-API: [M+H]⁺=229.3.

Step 4: *tert*-Butyl 2,2-dimethyl-3-(methylamino)pyrrolidine-1-carboxylate (67 mg, 0.29 mmol) was dissolved in methanol (3 mL), and then 37% aqueous formaldehyde solution (0.6 mL) and sodium cyanoborohydride (56 mg, 0.88 mmol) were added thereto and reacted at room temperature overnight. The reaction solution was quenched with water, extracted with ethyl acetate, and the organic phase was dried and evaporated to dryness to obtain a crude product (70 mg). (R_{f}=0.6, ethyl acetate). ES-API: [M+H]⁺=243.3.

Step 5: *tert*-Butyl 3-(dimethylamino)-2,2-dimethylpyrrolidine-1-carboxylate (86 mg, 0.35 mmol) was dissolved in dichloromethane (2 mL), and then trifluoroacetic acid (1 mL) was added thereto and reacted at room temperature for 2 hours. The reaction solution was directly evaporated to dryness to obtain a crude product (intermediate 6), which was directly used in the next reaction.

### Preparation embodiment 11 Synthesis of intermediate 7

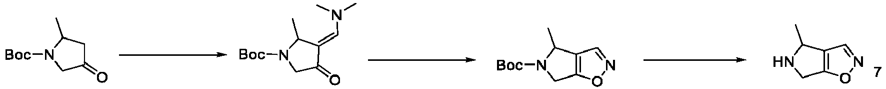

Step 1: *tert-*Butyl 2-methyl-4-oxopyrrolidine-1-carboxylate (1.0 g, 5.02 mmol) and *N,N-*dimethylformamide dimethyl acetal (6 mL) were added to a 25 mL reaction flask, heated to 80°C and reacted for 3 hours. The reaction was completed, and the mixture was concentrated, and purified by silica gel column to obtain a target product of *tert*-butyl (*Z*/*E*)-3-((dimethylamino)methylene)-2-methyl-4-oxopyrrolidine-1-carboxylate (600 mg, yield of 47%). ES-API: [M+H]⁺=255.2.

Step 2: *tert*-Butyl (*Z*/*E*)-3-((dimethylamino)methylene)-2-methyl-4-oxopyrrolidine-1-carboxylate (550 mg, 2.17 mmol), hydroxylamine hydrochloride (298 mg, 4.34 mmol), DIPEA (560 mg, 4.34 mmol) and ethanol (8 mL) were added to a 20 mL microwave reaction flask, heated to 85°C under microwave irradiation and reacted for 1 hour. After the reaction was completed, the mixture was cooled to room temperature, poured into ice-water, extracted with ethyl acetate, washed with water and saturated brine in turn, dried over anhydrous sodium sulfate, filtered, concentrated to obtain a crude target product of *tert*-butyl 4-methyl-4*H*-pyrrolo[3,4-*d*]isoxazole-5(6*H*)-carboxylate (50 mg, yield of 10.2%). ES-API: [M+H]⁺=225.1.

Step 3: *tert*-Butyl 4-methyl-4*H*-pyrrolo[3,4-*d*]isoxazole-5(6*H*)-carboxylate (50 mg) was added to dichloromethane (2 mL), and 2 mL of trifluoroacetic acid was added dropwise thereto, and the reaction was carried out at room temperature for 1 hour. After the reaction was completed, the mixture was concentrated to obtain 4-methyl-5,6-dihydro-4*H*-pyrrolo[3,4-*d*]isoxazole hydrochloride (intermediate 7, 28 mg, yield of 100%). ES-API: [M+H]⁺=125.1.

### Embodiment 1 Synthesis of two isomer compounds (Z1 and Z2) of 2-acryloyl-10-chloro-7-((2-isopropylphenyl)amino)-9-(5-methyl-1H-indazol-4-yl)-1,2,3,4,12,12a-hexahydro-6H-benzo[f]pyrazino[2,1-c][1,4]oxazepin-6-one

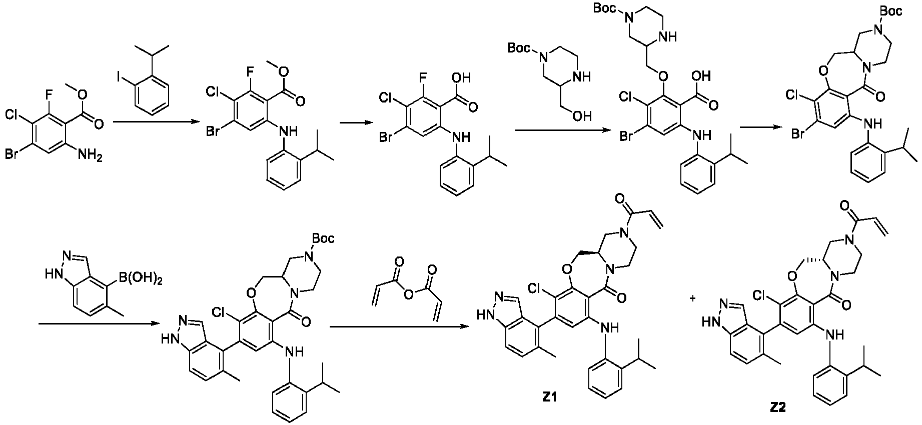

Step 1: Methyl 6-amino-4-bromo-3-chloro-2-fluorobenzoate (1.1 g, 3.89 mmol), 1-iodo-2-isopropylbenzene (1.44 g, 5.84 mmol), Pd₂(dba)₃ (0.36 g, 0.39 mmol), xantphos (0.56 g, 0.97 mmol), cesium carbonate (2.55 g, 7.78 mmol) and 20 mL of dioxane were added to a 100 mL flask. Under nitrogen protection, the reaction was carried out at 70°C for 16 hours. The completion of reaction was detected by LC-MS. 30 mL of water was added to the reaction. The mixture was extracted three times with 30 mL of ethyl acetate, and the organic phase was dried and concentrated. The crude product was purified by flash silica gel column (EtOAc/PE: 0-5%) to obtain 380 mg of methyl 4-bromo-3-chloro-2-fluoro-6-((2-isopropylphenyl)amino)benzoate with a yield of 24%. ES-API: [M+H]⁺=400.1.

Step 2: Methyl 4-bromo-3-chloro-2-fluoro-6-((2-isopropylphenyl)amino)benzoate (380 mg, 0.95 mmol), sodium hydroxide (114 mg, 2.84 mmol), methanol (5 mL), THF (10 mL) and water (5 mL) were added to a 100 mL flask. The mixture was reacted at room temperature for 3 hours. The completion of reaction was detected by LC-MS. 30 mL of water was added to the reaction, and the pH of the reaction was adjusted to 7 with 1 *M* aqueous hydrochloric acid solution. The mixture was extracted three times with 30 mL of ethyl acetate, and the organic phase was dried and concentrated to obtain 367 mg of a crude product of 4-bromo-3-chloro-2-fluoro-6-((2-isopropylphenyl)amino)benzoic acid. ES-API: [M+H]⁺=386.1.

Step 3: 60% sodium hydride (185 mg, 4.62 mmol), *tert*-butyl 3-(hydroxymethyl)piperazine-1-carboxylate (252 mg, 1.16 mmol) and THF (8 mL) were added to a flask. A THF suspension (4 mL) of 4-bromo-3-chloro-2-fluoro-6-((2-isopropylphenyl)amino)benzoic acid (300 mg, 0.77 mmol) was added dropwise thereto at 0°C. The reaction was stirred in an oil bath at 70°C for 2 hours. The completion of reaction was detected by LC-MS. The reaction solution was poured into 30 mL of saturated aqueous ammonium chloride solution. The mixture was extracted 4 times with 40 mL of ethyl acetate. The organic phase was dried and concentrated to obtain 4-bromo-2-((4-(*tert*-butoxycarbonyl)piperazin-2-yl)methoxy)-3-chloro-6-((2-isopropylphenyl)amino)benzoic acid (370 mg, 82%) as a yellow solid. ES-API: [M+H]⁺=582.0.

Step 4: At 0°C, 4-bromo-2-((4-(*tert*-butoxycarbonyl)piperazin-2-yl)methoxy)-3-chloro-6-((2-isopropylphenyl)amino)benzoic acid (370 mg, 0.63 mmol), HATU (482 mg, 1.27 mmol), *N,N-*diisopropylethylamine (244 mg, 1.89 mmol) and dichloromethane (10 mL) were added to a flask in turn. The mixture was reacted at room temperature for 3 hours. The reaction solution was added with water, extracted three times with dichloromethane, and the organic phase was dried, concentrated, and purified by flash silica gel column (EtOAc/PE: 5%-20%) to obtain *tert*-butyl 9-bromo-10-chloro-7-((2-isopropylphenyl)amino)-6-oxo-3,4,12,12a-tetrahydro-6*H*-benzo-[*f*]pyrazino[2,1-*c*][1,4]oxazepine-2(1*H-*carboxylate (320 mg, 89%) as a yellow solid. ES-API: [M+H]⁺=564.1.

Step 5: *tert*-Butyl 9-bromo-10-chloro-7-((2-isopropylphenyl)amino)-6-oxo-3,4,12,12a-tetrahydro-6*H*-benzo[*f*]pyrazino[2,1-*c*][1,4]oxazepine-2(1*H*)-carboxylate (290 mg, 0.51 mmol), (5-methyl-1*H*-indazol-4-yl)boronic acid (135 mg, 0.77 mmol), Sphos (20 mg, 0.051 mmol), sphos-Pd-G2 (36 mg, 0.051 mmol), potassium phosphate (324 mg, 1.53 mmol), 6 mL of dioxane and 1 mL of water were added to a 100 mL flask. The reaction was stirred at 105°C for 1 hour under microwave irradiation, and the reaction was stopped. The reaction solution was added with 20 mL of water, extracted three times with 20 mL of ethyl acetate, and the organic phase was dried, concentrated, and purified by flash silica gel column (EtOAc/PE: 0-50%) to obtain *tert*-butyl 10-chloro-7-((2-isopropylphenyl)amino)-9-(5-methyl-1*H*-indazol-4-yl)-6-oxo-3,4,12,12a-tetrahydro-6*H*-benzo[*f*]pyrazino[2,1-*c*][1,4]oxazepine-2(1*H*)-carboxylate (280 mg, 80%) as a yellow solid. ES-API: [M+H]⁺=616.2.

Step 6: *tert*-Butyl 10-chloro-7-((2-isopropylphenyl)amino)-9-(5-methyl-1*H*-indazol-4-yl)-6-oxo-3,4,12,12a-tetrahydro-6*H*-benzo[*f*]pyrazino[2,1-*c*][1,4]oxazepine-2(1*H*)-carboxylate (100 mg, 0.16 mmol), methanol (1 mL), and 4 *M* hydrogen chloride/dioxane solution (3 mL) were added to a flask. After stirring at room temperature for 1 hour, the reaction solution was concentrated to obtain a yellow solid. The yellow solid was dissolved in 3 mL of dichloromethane and triethylamine (81 mg, 0.80 mmol). At 0°C, a solution of acrylic anhydride in dichloromethane (16 mg, 0.13 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried and concentrated, and the crude product was purified by preparative HPLC to obtain two isomer compounds.

One isomer with a structure arbitrarily designated as (12a*R*)-2-acryloyl-10-chloro-7-((2-isopropylphenyl)amino)-9-(5-methyl-1*H*-indazol-4-yl)-1,2,3,4,12,12a-hexahydro-6*H-*benzo[*f*]pyrazino[2,1-*c*][1,4]oxazepin-6-one (Z1, 7.52 mg, 8%) as a white solid. HPLC retention time of the first peak: 7.374 min. ¹H NMR (400MHz, CDCl₃) δ 8.44 (s, 1*H*), 7.68 (s, 1H), 7.55-7.52 (m, 1H), 7.39-7.38 (m, 1H), 7.24-7.23 (m, 2H), 7.08-6.98 (m, 2H), 6.68 (s, 1H), 6.55-6.41 (m, 2H), 5.83-5.80 (m, 1H), 4.31-3.83 (m, 9H), 3.20-3.16 (m, 1H), 2.29 (s, 3H), 1.23 (d, J= 6.8 Hz 3H), 1.18 (d, J= 6.8 Hz, 3H); ES-API: [M+H]⁺=570.1.

Another isomer with a structure arbitrarily designated as (12aS)-2-acryloyl-10-chloro-7-((2-isopropylphenyl)amino)-9-(5-methyl-1*H*-indazol-4-yl)-1,2,3,4,12,12a-hexahydro-6*H-*benzo[*f*]pyrazino[2,1-*c*][1,4]oxazepin-6-one (Z2, 9.96 mg, 11%) as a white solid. HPLC retention time of the second peak: 7.754 min. ¹H NMR (400MHz, CDCl₃) δ 8.40 (s, 1H), 7.81 (s, 1H), 7.48-7.46 (m, 1H), 7.24-7.20 (m, 3H), 7.08-6.98 (m, 2H), 6.68 (s, 1H), 6.55-6.42 (m, 2H), 5.83-5.80 (m, 1H), 4.31-3.80 (m, 9H), 3.20-3.15 (m, 1H), 2.16 (s, 3H), 1.23 (d, J=6.8 Hz 3H), 1.16 (d, J= 6.8 Hz, 3H); ES-API: [M+H]⁺=570.1.

### Embodiment 2 Synthesis of 2-acryloyl-10-chloro-9-(5-methyl-1H-indazol-4-yl)-1,2,3,4,12,12a-hexahydro-6H-benzo[f]pyrazino[2,1-c][1,4]oxazepin-6-one Z3

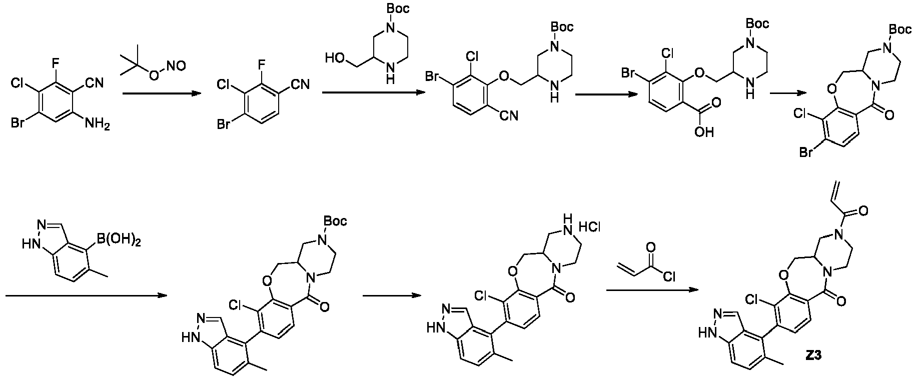

Step 1: 6-Amino-4-bromo-3-chloro-2-fluorobenzonitrile (415 mg, 1.66 mmol) was dissolved in THF (20.0 mL), cooled to 0°C, and a solution of *tert*-butyl nitrite (1.80 g, 16.6 mmol) in THF was added to the above solution. The reaction solution was stirred at 50°C for 16 hours. The reaction solution was cooled to 25°C and concentrated. Water and ethyl acetate were then added to the resulting crude product. The aqueous phase was extracted with ethyl acetate (20 mL^{∗}3). The organic phase was washed with saturated brine, dried over anhydrous sodium sulfate, and concentrated. The resulting crude product was purified by normal phase silica gel column (PE/EtOAc = 200/1-50/1) to obtain 4-bromo-3-chloro-2-fluorobenzonitrile (300 mg, 75%) as a yellow solid. ¹H NMR (400 MHz, DMSO-*d*₆) δ 8.44 (d, *J* = 8.0 Hz, 1H), 8.26 (d, *J* = 8.0 Hz, 1H).

Step 2: *tert*-Butyl 3-(hydroxymethyl)piperazine-1-carboxylate (280 mg, 1.28 mmol) was dissolved in THF (20 mL), and then sodium hydride (80 mg, 1.92 mmol, 60%) was added slowly in batches at 0°C. The reaction solution was stirred at 0°C for 15 minutes, and then 4-bromo-3-chloro-2-fluorobenzonitrile (300 mg, 1.28 mmol) was added thereto. The reaction solution was stirred at 50°C for 16 hours. After the reaction solution was concentrated, water and ethyl acetate were added to the resulting crude product at 0°C. The aqueous phase was extracted with ethyl acetate (20 mL^{∗}3). The organic phase was washed with saturated brine, dried over anhydrous sodium sulfate, and concentrated. The resulting crude product was purified by normal phase silica gel column (DCM/MeOH = 100/1-50/1) to obtain *tert*-butyl 3-((3-bromo-2-chloro-6-cyanophenoxy)methyl)piperazine-1-carboxylate (400 mg, 90%) as a white solid. ES-API: [M+H]⁺=430.2.

Step 3: *tert*-Butyl 3-((3-bromo-2-chloro-6-cyanophenoxy)methyl)piperazine-1-carboxylate (400 mg, 0.928 mmol) was dissolved in ethanol/water (10 mL/5 mL), and sodium hydroxide (0.93 g, 23.2 mmol) was added thereto; the reaction solution was heated and refluxed for 16 hours. After the reaction solution was concentrated, water and ethyl acetate were added to the resulting crude product at 0°C, and the phases were separated, and then the organic phase was discarded. The pH of the aqueous phase was adjusted to 2-3 with dilute hydrochloric acid, and the aqueous phase was extracted three times with ethyl acetate. The organic phases were combined, washed with saturated brine, dried over anhydrous sodium sulfate, concentrated, and the resulting crude product was purified by normal phase silica gel column (DCM/ MeOH = 100/1-10/1) to obtain 4-bromo-2-((4-*tert*-butoxycarbonyl)piperazin-2-yl)methoxy)-3-chlorobenzoic acid (350 mg, 40%) as a white solid. ES-API: [M+H]⁺=449.2.

Step 4: 4-Bromo-2-((4-*tert*-butoxycarbonyl)piperazin-2-yl)methoxy)-3-chlorobenzoic acid (210 mg, 0.467 mmol), 1-ethyl-(3-dimethylaminopropyl)carbodiimide hydrochloride (270 mg, 1.40 mmol), 1-hydroxybenzotriazole (65 mg, 0.467 mmol) and *N*-methylmorpholine (190 mg, 1.786 mmol) were added to dichloromethane (20.0 mL). The reaction solution was stirred at 40°C for 2 hours and concentrated. The resulting crude product was purified by normal phase silica gel column (PE/EtOAc = 10/1-8/1) to obtain *tert*-butyl 9-bromo-10-chloro-6-oxo-3,4,12,12a-tetrahydro-6*H*-benzo[*f*]pyrazino[2,1-*c*][1,4]oxazepine-2(6*H*)-carboxylate (115 mg, 50%) as an off-white solid. ES-API: [M-56+H]⁺= 375.2.

Step 5: *tert*-Butyl 9-bromo-10-chloro-6-oxo-3,4,12,12a-tetrahydro-6*H*-benzo[*f*]pyrazino[2,1-*c*][1,4]oxazepine-2(6*H*)-carboxylate (160 mg, 0.37 mmol), (5-methyl-1*H*-indazol-4-yl)boronic acid (98 mg, 0.55 mmol), sodium carbonate (80 mg, 0.742 mmol), chloro(2-dicyclohexylphosphino-2',6'-dimethoxy-1,1'-biphenyl)[2-(2'-amino-1,1'-biphenyl)]palladium(II) (30 mg, 0.037 mmol) were added to dioxane (10.0 mL) and water (4.0 mL), and the system was replaced with argon for three to five times. Then, the reaction solution was heated to 100° C under microwave irradiation, stirred for 60 minutes, cooled to room temperature, and filtered. The filtrate was extracted with ethyl acetate (10.0 mL^{∗}3). The organic phase was washed with water and saturated brine in turn, and then dried over anhydrous sodium sulfate. The resulting crude product was purified to obtain *tert*-butyl 10-chloro-9-(5-methyl-1*H*-indazol-4-yl)-6-oxo-3,4,12,12a-tetrahydro-6*H*-benzo[*f*]pyrazino[2,1-*c*][1,4]oxazepine-2(1*H*)-carboxylate (80 mg, 40%) as a yellow solid. ES-API: [M-56+H]⁺= 427.3.

Step 6: *tert*-Butyl 10-chloro-9-(5-methyl-1*H*-indazol-4-yl)-6-oxo-3,4,12,12a-tetrahydro-6*H-*benzo[*f*]pyrazino[2,1-*c*][1,4]oxazepine-2(1*H*)-carboxylate (80.0 mg, 0.16 mmol) and dioxane hydrochloride (4.0 mL) were added to methanol (15 mL), and the reaction solution was stirred at 25°C for 2 hours. The reaction solution was concentrated to obtain 10-chloro-9-(5-methyl-1*H*-indazol-4-yl)-3,4,12,12a-tetrahydro-1*H*-benzo[*f*]pyrazino[2,1-*c*][1,4]oxazepin-6-one hydrochloride (70 mg, 100%) as a white solid. ES-API: [M+H]⁺=383.3.

Step 7: 10-Chloro-9-(5-methyl-1*H*-indazol-4-yl)-3,4,12,12a-tetrahydro-1*H*-benzo[*f*]pyrazino[2,1-*c*][1,4]oxazepin-6-one hydrochloride (70 mg, 0.167 mmol) was dissolved in dichloromethane (10.0 mL), and then *N*,*N*-diisopropylethylamine was added thereto to adjust the pH of the reaction solution to 6-7. Then acryloyl chloride (15 mg, 0.167 mmol) was added thereto at -70°C. The reaction solution was stirred at -70°C for 30 minutes, quenched by adding methanol, and the reaction solution was concentrated. The resulting crude product was purified by normal phase silica gel column (DCM/MeOH = 10/1) to obtain 2-acryloyl-10-chloro-9-(5-methyl-1*H*-indazol-4-yl)-1,2,3,4,12,12a-hexahydro-6*H*-benzo[*f*]pyrazino[2,1-*c*][1,4]oxazepin-6-one (Z3, 40 mg, 50%) as an off-white solid. ¹H NMR (400 MHz, DMSO-*d*₆) δ 13.12 (s, 1H), δ 7.98 (m, 1H), 7.57 (m, 2H), 7.32 (d, *J* = 8.0 Hz, 1H), 7.07 (s, 1H), 6.79 (m, 1H), 6.20 (m, 1H), 5.77 (m, 1H), 4.36 (m, 2H), 4.15 (d, 3H), 3.77 (m, 4H), 2.17 (m, 3H). ES-API: [M+H]⁺=437.3.

### Embodiment 3 Synthesis of 2-acryloyl-7-amino-10-chloro-9-(5-methyl-1H-indazol-4-yl)-1,2,3,4,12,12a-hexahydro-1H-benzo[f]pyrazino[2,1-c][1,4]oxazepin-6-one Z4

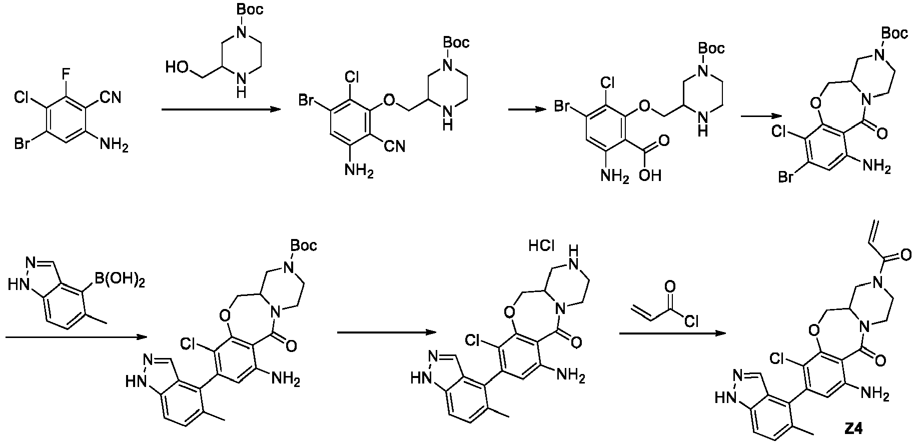

Step 1: *tert*-Butyl 3-(hydroxymethyl)piperazine-1-carboxylate (238 mg, 1.1 mmol) and sodium hydride (60 mg, 1.5 mmol) were added to anhydrous THF (5 mL), and stirred at room temperature for 30 minutes under nitrogen protection. Then, 6-amino-4-bromo-3-chloro-2-fluorobenzonitrile (250 mg, 1.0 mmol) was added thereto, and the mixture was heated and stirred at 50°C for 18 hours. The mixture was cooled to room temperature, quenched with water and extracted with ethyl acetate. The organic phase was dried over anhydrous sodium sulfate, concentrated and purified by silica gel column (PE:EtOAc=1:1) to obtain *tert*-butyl 3-((3-amino-5-bromo-6-chloro-2-cyanophenoxy)methyl)piperazine-1-carboxylate (370 mg, 83%) as a yellow solid. ES-API: [M+H]⁺=445.0.

Step 2: *tert*-Butyl 3-((3-amino-5-bromo-6-chloro-2-cyanophenoxy)methyl)piperazine-1-carboxylate (65 mg, 0.14 mmol) was dissolved in DMSO/isopropanol/water (5 mL/2 mL/2 mL), and potassium hydroxide (0.20 g, 3.5 mmol) was added thereto; the reaction solution was heated and refluxed for 16 hours. After the reaction solution was concentrated, water and ethyl acetate were added thereto at 0°C, and the phases were separated, and the organic phase was discarded. The pH of the aqueous phase was adjusted to 2-3 with dilute hydrochloric acid, and the aqueous phase was extracted three times with ethyl acetate. The organic phases were combined, washed with saturated brine, dried over anhydrous sodium sulfate, concentrated, and the resulting crude product was purified by normal phase silica gel column (DCM/MeOH = 100/1-10/1) to obtain 6-amino-4-bromo-2-((4-(*tert*-butoxycarbonyl)piperazin-2-yl)methoxy)-3-chlorobenzoic acid (10 mg, 15%) as a white solid. ES-API: [M+H]⁺=464.2.

Step 3: 6-Amino-4-bromo-2-((4-(*tert*-butoxycarbonyl)piperazin-2-yl)methoxy)-3-chlorobenzoic acid (10 mg, 0.021 mmol), 1-ethyl-(3-dimethylaminopropyl)carbodiimide hydrochloride (13 mg, 0.064 mmol), 1-hydroxybenzotriazole (3 mg, 0.021 mmol) and *N*-methylmorpholine (10 mg, 0.083 mmol) were added to dichloromethane (10.0 mL). The reaction solution was stirred at 40°C for 2 hours and concentrated. The resulting crude product was purified by normal phase silica gel column (PE/EtOAc = 10/1-8/1) to obtain *tert*-butyl 7-amino-9-bromo-10-chloro-6-oxo-3,4,12,12a-tetrahydro-1*H-*benzo[*f*]pyrazino[2,1-*c*][1,4]oxazepine-2(6*H*)-carboxylate (3.5 mg, 50%) as an off-white solid. ES-API: [M-56+H]⁺= 390.2.

Step 4: *tert*-Butyl 7-amino-9-bromo-10-chloro-6-oxo-3,4,12,12a-tetrahydro-1*H-*benzo[*f*]pyrazino[2,1-*c*][1,4]oxazepine-2(6*H*)-carboxylate (12 mg, 0.026 mmol), (5-methyl-1*H*-indazol-4-yl)boronic acid (8 mg, 0.040 mmol), sodium carbonate (6 mg, 0.053 mmol), chloro(2-dicyclohexylphosphino-2',6'-dimethoxybiphenyl)(2'-aminobiphenyl-2-yl)palladium(II) (2 mg, 0.0026 mmol) were added to dioxane (4.0 mL) and water (2.0 mL), and the system was replaced with argon for three to five times. Then the reaction solution was placed in a microwave apparatus, stirred at 100°C for 60 minutes, cooled to room temperature, and filtered. The filtrate was extracted with ethyl acetate (10.0 mL^{∗}3). The organic phase was washed with water and saturated brine in turn, and then dried over anhydrous sodium sulfate. The resulting crude product was purified to obtain *tert*-butyl 7-amino-10-chloro-9-(5-methyl-1*H-*indazol-4-yl)-6-oxo-3,4,12,12a-tetrahydro-1*H*-benzo[*f*]pyrazino[2,1-c][1,4]oxazepine-2(6*H*)-carboxylate (4.5 mg, 40%) as an off-white solid. ES-API: [M-56+H]⁺= 498.4.

Step 5: *tert*-Butyl 7-amino-10-chloro-9-(5-methyl-1*H*-indazol-4-yl)-6-oxo-3,4,12,12a-tetrahydro-1*H*-benzo[*f*]pyrazino[2,1-*c*][1,4]oxazepine-2(6*H*)-carboxylate (4.0 mg, 0.008 mmol) and dioxane hydrochloride (1.0 mL) were added to methanol (5 mL), and the reaction solution was stirred at 25°C for 2 hours. The reaction solution was concentrated to obtain 7-amino-10-chloro-9-(5-methyl-1*H*-indazol-4-yl)-3,4,12,12a-tetrahydro-1*H*-benzo[*f*]pyrazino[2,1-*c*][1,4]oxazepin-6(2*H*)-hydrochloride (3.5 mg, 100%) as a white solid. ES-API: [M+H]⁺=398.4.

Step 6: 7-Amino-10-chloro-9-(5-methyl-1*H*-indazol-4-yl)-3,4,12,12a-tetrahydro-1*H-*benzo[*f*]pyrazino[2,1-*c*][1,4]oxazepin-6(2*H*)-hydrochloride (3.5 mg, 0.008 mmol) was dissolved in dichloromethane (10.0 mL), and then *N*,*N*-diisopropylethylamine was added thereto to adjust the pH of the reaction solution to 6-7. Acryloyl chloride (0.8 mg, 0.008 mmol) was then added thereto at -70°C. The reaction solution was stirred at -70°C for 30 minutes, then methanol was added to quench the reaction, and the reaction solution was concentrated. The resulting crude product was purified by normal phase silica gel column (DCM/MeOH = 10/1) to obtain 2-acryloyl-7-amino-10-chloro-9-(5-methyl-1*H*-indazol-4-yl)-1,2,3,4,12,12a-hexahydro-1*H*-benzo[*f*]pyrazino[2,1-*c*][1,4]oxazepin-6-one (Z4, 1.00 mg, 25%) as an off-white solid. ES-API: [M+H]⁺=452.3.

### Embodiment 4 Synthesis of (6aR)-8-acryloyl-4-chloro-3-(2-fluoro-6-hydroxyphenyl)-1-((2-isopropyl-4-methylpyridin-3-yl)amino)-6,6a,7, 8,9,10-hexahydro-12H-pyrazino [2,1-c]pyrido [3,4-f][1,4]oxazepin-12-one Z5

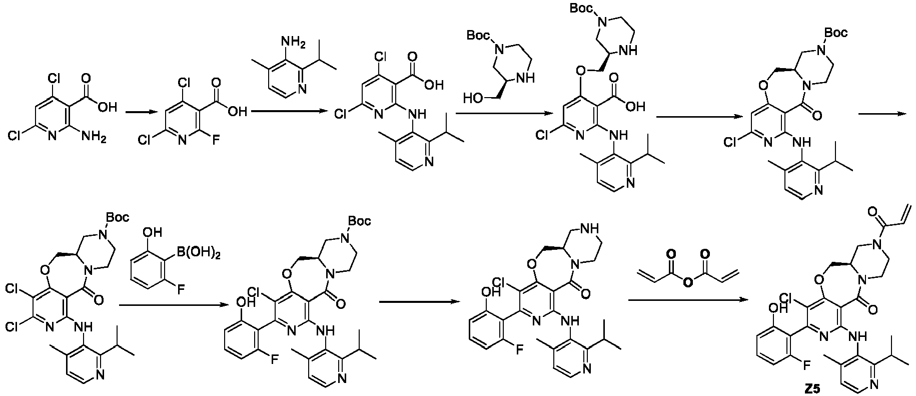

Step 1: 2-Amino-4,6-dichloronicotinic acid (3.3 g, 15.9 mmol) was added to 40 mL of pyridine hydrofluoride solution, cooled to 0 to 5°C in an ice-water bath, and sodium nitrite (2.2 g, 31.9 mmol) was added thereto in batches. Under nitrogen protection, the reaction was carried out at 0 to 5°C for about 1 hour, and the completion of reaction was detected by LCMS. The reaction solution was poured into about 100 mL of ice-water, extracted with ethyl acetate (80 mL^{∗}2), and the ethyl acetate phase was washed with saturated brine (100 mL^{∗}3), dried over anhydrous sodium sulfate, and the filtrate was evaporated to dryness by rotary evaporation to obtain 4,6-dichloro-2-fluoronicotinic acid (3 g, crude product). ES-API: [M+H]⁺=209.9.

Step 2: 2-Isopropyl-4-methylpyridin-3-amine (714 mg, 4.76 mmol) and 20 mL of THF were added to a 100 mL flask. Sodium bis(trimethylsilyl)amide (7.1 mL, 2 M of THF solution, 14.2 mmol) was added dropwise to the reaction solution at 0°C. After the dropwise addition, the reaction was carried out at 0°C for 10 minutes. Then, a solution of 4,6-dichloro-2-fluoronicotinic acid (1 g, 4.76 mmol) in THF (6 mL) was added dropwise to the reaction solution. After the dropwise addition, the reaction was carried out at 0° C for 20 minutes, then the reaction solution was poured into ice-water, and the aqueous phase was extracted with ethyl acetate to remove impurities. The pH of the aqueous phase was adjusted to 3 with 6 M hydrochloric acid, and a solid was precipitated in the system. The mixture was filtered, and the filter cake was washed with water and dried to obtain 4,6-dichloro-2-((2-isopropyl-4-methylpyridin-3-yl)amino)nicotinic acid (1 g, 61%). ES-API: [M+H]⁺=340.1.

Step 3: 60% sodium hydride (206 mg, 5.14 mmol), *tert*-butyl (*R*)-3-(hydroxymethyl)piperazine-1-carboxylate (381 mg, 1.76 mmol) and THF (8 mL) were added to a 100 mL flask. A THF suspension (4 mL) of 4,6-dichloro-2-((2-isopropyl-4-methylpyridin-3-yl)amino)nicotinic acid (500 mg, 1.47 mmol) was added dropwise thereto at 0°C. The reaction was carried out in an oil bath at 60°C for 5 hours. The completion of reaction was detected by LC-MS. 30 mL of saturated aqueous NH₄Cl solution was poured into the reaction solution. The mixture was extracted 4 times with 40 mL of ethyl acetate. The organic phase was dried and then concentrated to obtain (*R*)-4-((4-(*tert*-butoxycarbonyl)piperazin-2-yl)methoxy)-6-chloro-2-((2-isopropyl-4-methylpyridin-3-yl)amino)nicotinic acid (760 mg) as a yellow solid. ES-API: [M+H]⁺=520.3.

Step 4: (R)-4-((4-(*tert*-Butoxycarbonyl)piperazin-2-yl)methoxy)-6-chloro-2-((2-isopropyl-4-methylpyridin-3-yl)amino)nicotinic acid (760 mg, 1.45 mmol), *N*,*N*-diisopropylethylamine (4 mL) and dichloromethane (15 mL) were added to a 100 mL flask. Propylphosphonic anhydride solution (4 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of hydrochloric acid (1 M), and 30 mL of saturated aqueous sodium bicarbonate solution, respectively. The organic phase was dried and concentrated to obtain 725 mg of a crude product of *tert*-butyl (R)-3-chloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate as a yellow solid. ES-API: [M+H]⁺=502.3.

Step 5: *tert*-Butyl (*R*)-3-chloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (1.4 g, 2.79 mmol), N-chlorosuccinimide (750 mg, 5.58 mmol) and acetonitrile (20 mL) were added to a 100 mL flask. The reaction was stirred at 80°C for 1 hour. Aqueous sodium thiosulfate solution was added to the reaction solution and extracted with ethyl acetate. The organic phase was dried, concentrated and then purified by flash silica gel column (EtOAc/PE: 0-50%) to obtain *tert*-butyl (*R*)-3,4-dichloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (860 mg, 57%). ES-API: [M+H]⁺=536.2.

Step 6: *tert-*Butyl (*R*)-3,4-dichloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (300 mg, 0.56 mmol), 2-fluoro-6-hydroxyphenylboronic acid (262 mg, 1.68 mmol), Sphos (23 mg, 0.056 mmol), sphos-Pd-G2 (40 mg, 0.056 mmol), potassium phosphate (356 mg, 1.68 mmol), 10 mL of dioxane and 2 mL of water were added to a 100 mL reaction flask. The reaction was stirred in an oil bath at 70°C for 3 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-3-(2-fluoro-6-hydroxyphenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (160 mg, 56%) as a yellow solid. ES-API: [M+H]⁺=612.2.

Step 7: *tert*-Butyl (6a*R*)-4-chloro-3-(2-fluoro-6-hydroxyphenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (160 mg, 0.26 mmol), 1 mL of methanol and 4 M hydrogen chloride/dioxane solution (3 mL) were added to a 50 mL flask. The mixture was stirred at room temperature for 1 hour, and the completion of reaction was detected by LC-MS. The reaction solution was concentrated to obtain a crude product of (6aR)-4-chloro-3 -(2-fluoro-6-hydroxyphenyl)-1-((2-isopropyl-4-methylpyridin-3-yl)amino)-6,6a,7, 8,9,10-hexahydro-12*H*-pyrazino[2,1-*c*]pyrido[3,4-*f*][1,4]oxazepin-12-one (130 mg) as a yellow solid. ES-API: [M+H]⁺=512.2.

Step 8: (6a*R*)-4-Chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (130 mg, 0.25 mmol), 5 mL of dichloromethane and triethylamine (166 mg, 1.65 mmol) were added to a 50 mL flask. At 0°C, a solution of acrylic anhydride in dichloromethane (30 mg, 0.24 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (Z5, 56 mg, 39%) as a white solid. ¹H NMR (500MHz, CDCl₃) 9.35-9.32(m, 1H), 8.69 (s, 1H), 8.44 (s, 1H), 7.18-7.15 (m, 2H), 6.65-6.45 (m, 4H), 5.87-5.85 (m, 1H), 4.52-4.50 (m, 2H), 4.21-3.92 (m, 7H), 3.35-3.34 (m, 1H), 2.26-2.25 (m, 3H), 1.26-1.24 (m, 6H). ES-API: [M+H]⁺=566.2.

### Embodiment 5 Synthesis of (6aR)-8-acryloyl-4-chloro-3-(2-fluoro-6-hydroxyphenyl)-1-((2-isopropyl-4-methylpyridin-3-yl)(methyl)amino)-6,6a,7, 8,9,10-hexahydro-12H-pyrazino [2,1-c]pyrido [3,4-f][1,4]oxazepin-12-one Z6

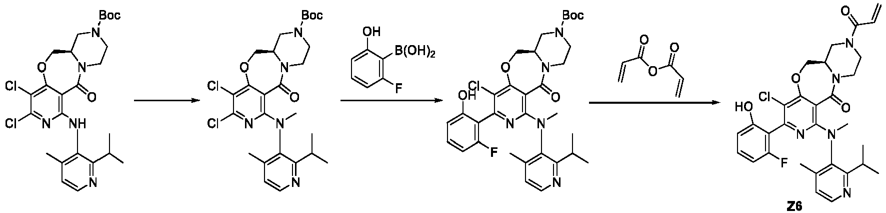

Step 1: 60% sodium hydride (42 mg, 1.05 mmol) and THF (5 mL) were added to a 100 mL flask. At 0°C, *tert*-butyl (*R*)-3,4-dichloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (200 mg, 0.35 mmol) and iodomethane (176 mg, 1.24 mmol) were added thereto in turn. The reaction was carried out in an oil bath at 50°C for 3 hours. The completion of reaction was detected by LC-MS. The reaction solution was poured into 30 mL of icy saturated aqueous NH₄Cl solution. The mixture was extracted 3 times with 40 mL of ethyl acetate. The organic phase was dried and concentrated to obtain *tert*-butyl (*R*)-3,4-dichloro-1-((2-isopropyl-4-methylpyridin-3 -yl)(methyl)amino)-12-oxo-6a,7,9,10-tetrahydro-12*H*-pyrazino[2,1 - *c*]pyrido[3,4-*f*][1,4]oxazepine-8(6*H*)-carboxylate (200 mg, 95%) as a yellow solid. ES-API: [M+H]⁺=550.2.

Step 2: *tert*-Butyl (*R*)-3,4-dichloro-1-((2-isopropyl-4-methylpyridin-3-yl)(methyl)amino)-12-oxo-6a,7,9,10-tetrahydro-12*H*-pyrazino[2,1-*c*]pyrido[3,4-*f*][1,4]oxazepine-8(6*H*)-carboxylate (200 mg, 0.36 mmol), 2-fluoro-6-hydroxyphenylboronic acid (198 mg, 1.27 mmol), Sphos (15 mg, 0.036 mmol), Sphos-Pd-G2 (26 mg, 0.036 mmol), potassium phosphate (269 mg, 1.27 mmol), 8 mL of dioxane and 2 mL of water were added to a 100 mL reaction flask. The reaction was carried out in an oil bath at 70°C for 2 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-3-(2-fluoro-6-hydroxyphenyl)-1-((2-isopropyl-4-methylpyridin-3-yl)(methyl)amino)-12-oxo-6a,7,9,10-tetrahydro-12*H*-pyrazino[2,1-*c*]pyrido[3,4-*f*][1,4]oxazepine-8(6*H*)-carboxylate (180 mg, 79%) as a yellow solid. ES-API: [M+H]⁺=626.2.

Step 3: *tert*-Butyl (6a*R*)-4-chloro-3-(2-fluoro-6-hydroxyphenyl)-1-((2-isopropyl-4-methylpyridin-3-yl)(methyl)amino)-12-oxo-6a,7,9,10-tetrahydro-12*H*-pyrazino[2,1-*c*]pyrido[3,4-*f*][1,4]oxazepine-8(6*H*)-carboxylate (180 mg, 0.28 mmol), methanol (1 mL) and 4 M hydrogen chloride/dioxane solution (2.5 mL) were added to a flask. After stirring at room temperature for 1 hour, the reaction solution was concentrated to obtain a yellow solid. The yellow solid was dissolved in 5 mL of dichloromethane and triethylamine (87 mg, 0.86 mmol). At 0°C, a solution of acrylic anhydride in dichloromethane (20 mg, 0.24 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-((2-isopropyl-4-methylpyridin-3 -yl)(methyl)amino)-6,6a,7,8,9,10-hexahydro-12/7-pyrazino [2,1-*c*]pyrido[3,4-*f*][1,4]oxazepin-12-one (Z6, 23 mg, 14%) as a white solid. ¹HNMR (500MHz, DMSO-*d*₆) 10.01-9.89 (m, 1H), 8.27-8.25 (m, 1H), 7.20-7.12 (m, 2H), 6.78-6.60 (m, 3H), 6.20 (d, *J* = 17Hz, 1H), 5.75-5.74 (m, 1H), 4.70-3.82 (m, 9H), 3.21-3.02 (m, 4H), 2.07-1.79 (m, 3H), 1.42-0.87 (m, 6H); ES-API: [M+H]⁺=580.2.

### Embodiment 6 Synthesis of (6aS)-8-acryloyl-4-chloro-3-(2-fluoro-6-hydroxyphenyl)-1-((2-isopropyl-4-methylpyridin-3-yl)amino)-6,6a,7,8,9,10-hexahydro-12H-pyrazino[2,1-c]pyrido[3,4-f][1,4]oxazepin-12-one Z7

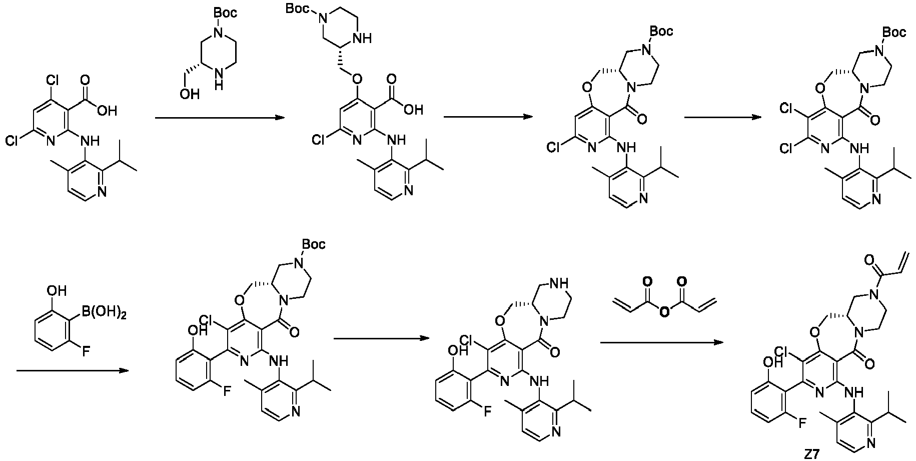

Step 1: Taking tert-Butyl (*S*)-3-(hydroxymethyl)piperazine-1-carboxylate (640mg, 2.94 mmol) and 4,6-dichloro-2-((2-isopropyl-4-methylpyridin-3-yl)amino)nicotinic acid (1 g, 2.94 mmol) as raw materials, the reaction was carried out with reference to step 2 of Embodiment 4 to obtain (*S*)-4-((4-(*tert-*butoxycarbonyl)piperazin-2-yl)methoxy)-6-chloro-2-((2-isopropyl-4-methylpyridin-3-yl)amino)nicotinic acid (993 mg, 65%) as a yellow solid. ES-API: [M+H]⁺=520.1.

Step 2: Taking (*S*)-4-((4-(tert-butoxycarbonyl)piperazin-2-yl)methoxy)-6-chloro-2-((2-isopropyl-4-methylpyridin-3-yl)amino)nicotinic acid (500 mg, 0.95 mmol) as a raw material, the reaction was carried out with reference to step 3 of Embodiment 4 to obtain tert-butyl (*S*)-3-chloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (300 mg, 63%). ES-API: [M+H]⁺=502.2.

Step 3: Taking *tert*-butyl (*S*)-3-chloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (300 mg, 0.60 mmol) as a raw material, the reaction was carried out with reference to step 4 of Embodiment 4 to obtain *tert*-butyl (*S*)-3-chloro-4-fluoro-1-((2-isopropyl-4-methylpyridin-3-yl)amino)-12-oxo-6a,7,9,10-tetrahydro-12*H*-pyrazino[2,1-*c*]pyrido[3,4-*f*][1.4]oxazcpinc-8(6*H*)-carboxylatc (296 mg, 92%) as a yellow solid. ES-API: [M+H]⁺=536.2.

Step 4: Taking tert-butyl (*S*)-3,4-dichloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (296 mg, 0.55 mmol) as a raw material, the reaction was carried out with reference to step 5 of Embodiment 4 to obtain tert-butyl (6a*S*)-4-chloro-3-(2-fluoro-6-hydroxyphenyl)-1-((2-isopropyl-4-methylpyridin-3-yl)amino)-12-oxo-6a,7,9,10-tetrahydro-12*H*-pyrazino[2,1-c]pyrido[3,4-*f*][1,4]oxazepine-8(6*H*)-carboxylate (239 mg, 71%) as a yellow solid. ES-API: [M+H]⁺=612.2.

Step 5: Taking *tert*-butyl (6a*S*)-4-chloro-3-(2-fluoro-6-hydroxyphenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (239 mg, 0.39 mmol) as a raw material, the reaction was carried out with reference to step 6 of Embodiment 4 to obtain (6a*S*)-4-chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (305 mg) as a yellow solid. The crude product was directly used in the next step. ES-API: [M+H]⁺=512.2.

Step 6: Taking (6a*S*)-4-chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (305 mg, 0.39 mmol) as a raw material, the reaction was carried out with reference to step 7 of Embodiment 4 to obtain (6a*S*)-8-acryloyl-4-chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (Z7, 68 mg, 31%). ES-API: [M+H]⁺=566.2. ¹H NMR (500 MHz, DMSO-*d*₆) δ 9.85 (s, 1H), 8.84 (s, 1H), 8.13 (d, *J* = 4.8 Hz, 1H), 7.12 (dd, *J* = 15.3, 8.2 Hz, 1H), 6.99 (d, *J* = 4.3 Hz, 1H), 6.84-6.49 (m, 3H), 6.12 (t, *J* = 14.8 Hz, 1H), 5.69 (dd, *J* = 19.0, 11.8 Hz, 1H), 4.51-4.30 (m, 2H), 4.24 (s, 1H), 4.05-3.77 (m, 4H), 3.72-3.56 (m, 2H), 3.30-3.21 (m, 1 H), 2.07 (s, 3H), 1.10 (s, 3H), 0.98 (s, 3H).

### Embodiment 7 Synthesis of (R)-8-acryloyl-4-chloro-3-(2-fluorophenyl)-1-((2-isopropyl-4-methylpyridin-3-yl)amino)-6,6a,7,8,9,10-hexahydro-12H-pyrazino[2,1-c]pyrido[3,4-f][1,4]oxazepin-12-one Z8

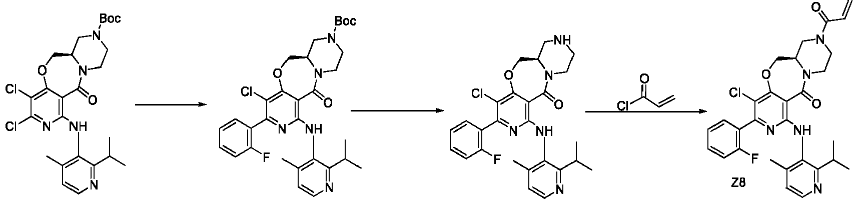

Step 1: *tert*-Butyl (*R*)-3,4-dichloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (190 mg, 0.35 mmol), 2-fluorophenylboronic acid (235.2 mg, 1.68 mmol), tetrakis(triphenylphosphine)palladium (40 mg, 0.035 mmol), potassium carbonate (231.8 mg, 1.68 mmol), 5 mL of dioxane and 1 mL of water were added to a 100 mL reaction flask. The reaction was carried out in an oil bath at 90°C for 5 hours. Then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (*R*)-4-chloro-3-(2-fluorophenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (170 mg, 80%) as a yellow solid. ES-API: [M+H]⁺=596.2.

Step 2: *tert*-Butyl (*R*)-4-chloro-3-(2-fluorophenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (170 mg, 0.28 mmol), trifluoroacetic acid (2 mL) and 2 mL of dichloromethane were added to a 50 mL flask. The mixture was stirred at room temperature for 1 hour, and the completion of reaction was detected by LC-MS. The reaction solution was concentrated to obtain (*R*)-4-chloro-3-(2-fluorophenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (crude product, 150 mg) as a yellow solid. ES-API: [M+H]⁺=496.2.

Step 3: (*R*)-4-Chloro-3-(2-fluorophenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (130 mg), 3 mL of dichloromethane and triethylamine (166 mg, 1.65 mmol) were added to a 50 mL flask. At 0°C, a solution of acryloyl chloride in dichloromethane (20 mg, 0.22 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (*R*)-8-acryloyl-4-chloro-3-(2-fluorophenyl)-1-((2-isopropyl-4-methylpyridin-3-yl)amino)-6,6a,7,8,9,10-hexahydro-12*H-*pyrazino[2,1-*c*]pyrido[3,4-*f*][1,4]oxazepin-12-one (Z8, 80 mg, 52%) as a white solid. ES-API: [M+H]⁺=550.2. ¹H NMR (500 MHz, DMSO-*d*₆) δ 8.91 (s, 1H), 8.21 (d, *J* = 4.9 Hz, 1H), 7.45 (d, *J* = 6.1 Hz, 1H), 7.30-7.20 (m, 3H), 7.06 (d, *J* = 4.7 Hz, 1H), 6.85-6.65 (m, 1H), 6.27-6.09 (m, 1H), 5.70-5.60 (m, 1H), 4.55-4.34 (m, 3H), 4.04-3.67 (m, 6H), 3.35-3.25 (m, 1H), 2.15 (s, 3H), 1.15 (brs, 3H), 1.04 (d, *J* = 6.2 Hz, 3H).

### Embodiment 8 Synthesis of (6aR)-8-acryloyl-4-fluoro-3-(2-fluoro-6-hydroxyphenyl)-1-((2-isopropyl-4-methylpyridin-3-yl)amino)-6,6a,7,8,9,10-hexahydro-12H-pyrazino[2,1-c]pyrido[3,4-f][1,4]oxazepin-12-one Z9

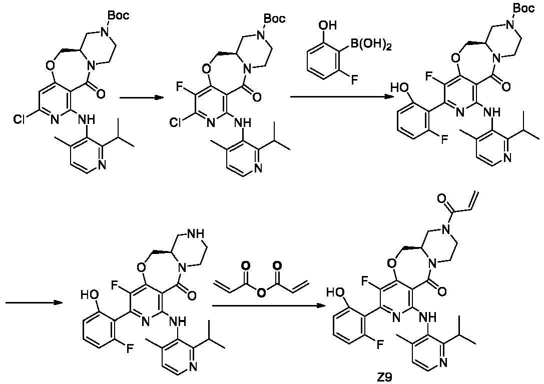

Step 1: *tert*-Butyl (*R*)-3-chloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (706 mg, 1.4 mmol), 1-chloromethyl-4-fluoro-1,4-diazabicyclo[2.2.2]octane bis(tetrafluoroborate) (991 mg, 2.8 mmol) and acetonitrile (10 mL) were added to a flask. The reaction was stirred at 50°C for 2 hours. The reaction solution was added with 50 mL of water and extracted with ethyl acetate. The organic phase was dried, concentrated and then purified by flash silica gel column (MeOH/DCM: 0-3%) to obtain *tert*-butyl (*R*)-3-chloro-4-fluoro-1-((2-isopropyl-4-methylpyridin-3-yl)amino)-12-oxo-6a,7,9,10-tetrahydro-12*H-*pyrazino[2,1-*c*]pyrido[3,4-*f*][1.4]oxazcpinc-8(6*H*)-carboxylate (416 mg, 50%) as a yellow solid. ES-API: [M+H]⁺=520.2.

Step 2: *tert*-Butyl (*R*)-3-chloro-4-fluoro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (416 mg, 0.7 mmol), 2-fluoro-phenylboronic acid (327 mg, 2.1 mmol), SPhos-Pd-G2 (50 mg, 0.07 mmol), SPhos (29 mg, 0.07 mmol), potassium phosphate (445 mg, 2.1 mmol), 18 mL of dioxane and 2 mL of water were added to a 100 mL reaction flask. The reaction was stirred at 80°C for 2.5 hours under nitrogen protection, and the reaction was stopped. The reaction solution was added with 20 mL of water, extracted three times with 20 mL of ethyl acetate. The organic phase was dried, concentrated and then purified by flash silica gel column (EtOAc/PE: 0-100%) to obtain *tert*-butyl (6a*R*)-4-fluoro-3-(2-fluoro-6-hydroxyphenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (304 mg, 73%) as a yellow solid. ES-API: [M+H]⁺=596.3.

Step 3: *tert*-Butyl (6a*R*)-4-fluoro-3-(2-fluoro-6-hydroxyphenyl)-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (304 mg, 0.51 mmol), 3 mL of methanol and 6 mL of hydrogen chloride/dioxane solution (4 M) were added to a flask. The mixture was stirred at room temperature for 1 hour, and the completion of reaction was detected by LC-MS. The reaction solution was concentrated to obtain (6a*R*)-4-fluoro-3-(2-fluoro-6-hydroxyphenyl)-1-((2-isopropyl-4-methylpyridin-3-yl)amino)-6,6a,7,8,9,10-hexahydro-12*H-*pyrazino[2,1-*c*]pyrido[3,4-*f*][1,4]oxazepin-12-one (310 mg) as a yellow solid. The crude product was directly used in the next step. ES-API: [M+H]⁺=496.3.

Step 4: (6a*R*)-4-Fluoro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (310 mg, 0.62 mmol), 3 mL of dichloromethane and triethylamine (627 mg, 6.2 mmol) were added to a flask. At 0°C, a solution of acrylic anhydride in dichloromethane (50 mg, 0.4 mmol, 0.5 mL) was added dropwise thereto. The reaction was carried out at 0°C for 10 minutes. 40 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-fluoro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one (Z9, 143 mg, 42%). ES-API: [M+H]⁺=550.2. ¹H NMR (500 MHz, DMSO-*d*₆) δ 10.04 (s, 1H), 8.90 (s, 1H), 8.21 (d, *J* = 4.8 Hz, 1H), 7.22 (dd, *J* = 15.4, 8.0 Hz, 1H), 7.07 (d, *J* = 4.0 Hz, 1H), 6.84-6.62 (m, 3H), 6.19 (t, *J* = 16.0 Hz, 1H), 5.80-5.69 (m, 1H), 4.58-4.39 (m, 3H), 4.14-3.56 (m, 4H), 2.15 (s, 3H), 1.13-1.10 (m, 3H), 1.13-1.10 (m, 3H), 1.05 (d, *J* = 6.5 Hz, 3H).

### Embodiment 9 Synthesis of (6aR)-8-acryloyl-4-chloro-3-(2-fluoro-6-hydroxyphenyl)-1-morpholino-6,6a,7,8,9,10-hexahydro-12H-pyrazino[2,1-c]pyrido[3,4-f][1,4]oxazepin-12-one Z10

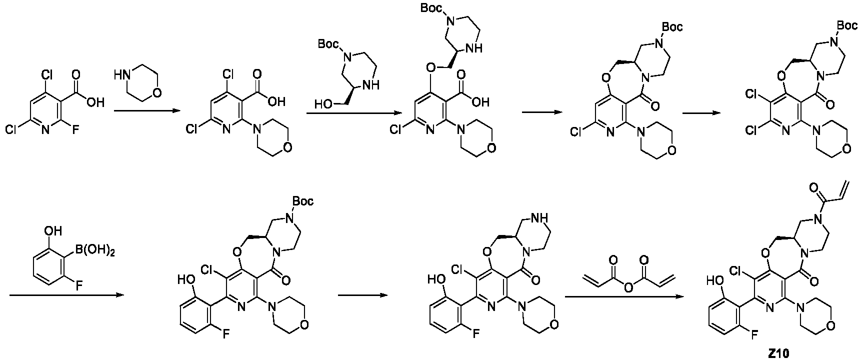

Step 1: 4,6-Dichloro-2-fluoronicotinic acid (500 mg, 2.39 mmol), morpholine (208 mg, 2.39 mmol) and potassium carbonate (989 mg, 7.17 mmol) in dichloromethane (5 mL) were added to a 100 mL flask at room temperature. After the reaction was completed, the reaction solution was poured into ice-water, and the aqueous phase was extracted with ethyl acetate to remove impurities. The pH of the aqueous phase was adjusted to 3 with 6 M hydrochloric acid, and the aqueous phase was extracted with ethyl acetate (20 mL×2). The organic phase was dried and concentrated to obtain 4,6-dichloro-2-morpholinonicotinic acid (600 mg, 90%). ES-API: [M+H]⁺=277.1.

Step 2: 60% sodium hydride (259 mg, 6.48 mmol), *tert*-butyl (*R*)-3-(hydroxymethyl)piperazine-1-carboxylate (466 mg, 2.16 mmol) and THF (8 mL) were added to a 100 mL flask. A THF suspension (4 mL) of 4,6-dichloro-2-morpholinonicotinic acid (600 mg, 2.16 mmol) was added dropwise thereto at 0°C. The reaction was carried out in an ice bath at 0°C for 20 minutes. The completion of reaction was detected by LC-MS. 30 mL of saturated aqueous NH₄Cl solution was poured into the reaction solution, and the pH was adjusted to 5-6, and the mixture was extracted 4 times with 40 mL of ethyl acetate. The organic phase was dried and then concentrated to obtain (*R*)-4-((4-(*tert*-butoxycarbonyl)piperazin-2-yl)methoxy)-6-chloro-2-morpholinonicotinic acid (crude product, 910 mg) as a yellow solid. ES-API: [M+H]⁺=457.3.

Step 3: (*R*)-4-((4-(*tert*-Butoxycarbonyl)piperazin-2-yl)methoxy)-6-chloro-2-morpholinonicotinic acid (910 mg), diisopropylethylamine (4 mL) and dichloromethane (10 mL) were added to a 100 mL flask. Propylphosphonic anhydride solution (4 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of aqueous hydrochloric acid solution (1 M), and 30 mL of sodium bicarbonate, respectively. The organic phase was dried and concentrated to obtain *tert*-butyl (*R*)-3-chloro-1-morpholino-12-oxo-6a.7.9.10-tetrahydro-12*H*-pyrazino[2,1-*c*]pyrido[3,4-*f*][1,4]oxazepine-8(6*H*)-carboxylate (crude product, 725 mg) as a yellow solid. ES-API: [M+H]⁺=439.3.

Step 4: *tert*-Butyl (*R*)-3-chloro-1-morpholino-12-oxo-6a,7,9,10-tetrahydro-12*H*-pyrazino[2,1-*c*]pyrido[3,4-*f*][1,4]oxazepine-8(6*H*)-carboxylate (crude product, 725 mg), *N*-chlorosuccinimide (442.6 mg, 3.3 mmol) and acetonitrile (10 mL) were added to a 100 mL flask. The reaction was stirred at 80 °C for 2 hours. Aqueous sodium thiosulfate solution was added to the reaction solution and the mixture was extracted with ethyl acetate. The organic phase was dried, concentrated and then purified by flash silica gel column (EtOAc/PE: 0-50%) to obtain *tert*-butyl (*R*)-3,4-dichloro-1-morpholino-12-oxo-6a,7,9,10-tetrahydro-12*H*-pyrazino[2,1-*c*]pyrido[3,4-*f*][1,4]oxazepine-8(6*H*)-carboxylate (550 mg, 3-step yield of 70%). ES-API: [M+H]⁺=473.2.

Step 5: *tert*-Butyl (*R*)-3,4-dichloro-1-morpholino-12-oxo-6a,7,9,10-tetrahydro-12*H*-pyrazino[2,1-*c*]pyrido[3,4-*f*][1,4]oxazepine-8(6*H*)-carboxylate (264.8 mg, 0.56 mmol), 2-fluoro-6-hydroxyphenylboronic acid (262 mg, 1.68 mmol), SPhos (23 mg, 0.056 mmol), SPhos-Pd-G2 (40 mg, 0.056 mmol), potassium phosphate (356 mg, 1.68 mmol), 10 mL of dioxane and 2 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-3-(2-fluoro-6-hydroxyphenyl)-1-morpholino-12-oxo-6a,7,9,10-tetrahydro-12*H*-pyrazino[2,1-*c*]pyrido[3,4-*f*][1,4]oxazepine-8(6*H*)-carboxylate (142 mg, yield of 46%) as a yellow solid. ES-API: [M+H]⁺=549.2.

Step 6: *tert*-Butyl (6a*R*)-4-chloro-3-(2-fluoro-6-hydroxyphenyl)-1-morpholino-12-oxo-6a,7,9,10-tetrahydro-12*H*-pyrazino[2,1-*c*]pyrido[3,4-*f*][1,4]oxazepine-8(6*H*)-carboxylate (142 mg, 0.25 mmol), 3 mL of dichloromethane and trifluoroacetic acid (2 mL) were added to a 50 mL flask. The mixture was stirred at room temperature for 1 hour, and the completion of reaction was detected by LC-MS. The reaction solution was concentrated to obtain (6a*R*)-4-chloro-3-(2-fluoro-6-hydroxyphenyl)-1-morpholino-6,6a,7,8,9,10-hexahydro-12*H*-pyrazino[2,1-*c*]pyrido[3,4-*f*][1,4]oxazepin-12-one (130 mg, crude product) as a yellow oil. ES-API: [M+H]⁺=449.2.

Step 7: (6a*R*)-4-Chloro-3-(2-fluoro-6-hydroxyphenyl)-1-morpholino-6,6a,7,8,9,10-hexahydro-12*H*-pyrazino[2,1-*c*]pyrido[3,4-*f*[1,4]oxazepin-12-one (130 mg), 5 mL of dichloromethane and triethylamine (166 mg, 1.65 mmol) were added to a 50 mL flask. At 0°C, a solution of acrylic anhydride in dichloromethane (30 mg, 0.24 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-morpholino-6,6a,7, 8,9,10-hexahydro-12H-pyrazino [2,1-*c*]pyrido [3,4-*f*][1,4]oxazepin-12-one (Z10, 3 mg, 2-step yield of 2.4%) as a white solid. ¹H NMR (400 MHz, DMSO-*d*₆) δ 9.52 (brs, 1H), 7.25 (q, *J* = 7.9 Hz, 1H), 7.08-6.61 (m, 3H), 6.17 (dd, *J* = 16.7, 1.8 Hz, 1H), 5.75 (d, *J* = 9.7 Hz, 1H), 4.54-3.87 (m, 6H), 3.70-3.52 (m, 6H), 3.51-3.29 (m, 3H), 3.37-3.11 (m, 2H). ES-API: [M+H]⁺=503.2.

### Embodiment 10 Preparation of (6aR)-8-acryloyl-4-chloro-3-(2-fluoro-6-hydroxyphenyl)-1-(pyrrolidin-1-yl)-6,6a,7,8,9,10-hexahydro-12H-pyrazino[2,1-c]pyrido[3,4-f][1,4]oxazepin-12-one Z11

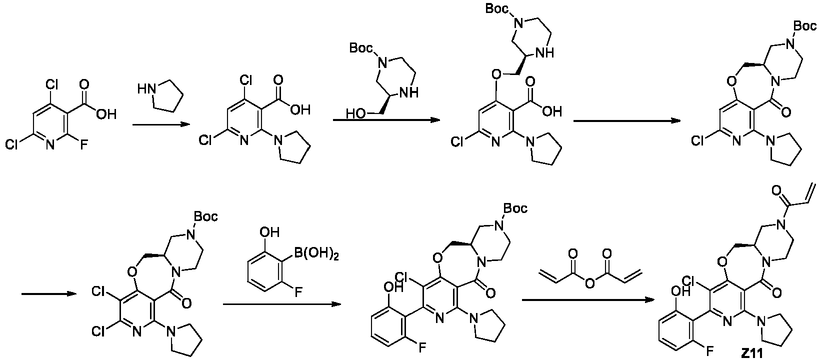

Step 1: 4,6-Dichloro-2-fluoronicotinic acid (500 mg, 2.39 mmol), pyrrolidine (169.7 mg, 2.39 mmol) and potassium carbonate (989 mg, 7.17 mmol) in dichloromethane (5 mL) were added to a 100 mL flask at room temperature. After the reaction was completed, the reaction solution was poured into ice-water, and the aqueous phase was extracted with ethyl acetate to remove impurities. The pH of the aqueous phase was adjusted to 3 with 6 M hydrochloric acid, and the aqueous phase was extracted with ethyl acetate (20 mL×2), dried and concentrated to obtain 4,6-dichloro-2-(pyrrolidin-1-yl)nicotinic acid (563 mg, 90%). ES-API: [M+H]⁺=261.1.

Step 2: 60% sodium hydride (259 mg, 6.48 mmol), *tert*-butyl (R)-3-(hydroxymethyl)piperazine-1-carboxylate (466 mg, 2.16 mmol) and THF (8 mL) were added to a 100 mL flask. A THF suspension (4 mL) of 4,6-dichloro-2-(pyrrolidin-1-yl)nicotinic acid (563 mg, 2.16 mmol) was added dropwise thereto at 0°C. The reaction was carried out in an ice bath at 0°C for 20 minutes. The completion of reaction was detected by LC-MS. 30 mL of saturated aqueous NH₄Cl solution was poured into the reaction solution, the pH was adjusted to 5-6, and the mixture was extracted 4 times with 40 mL of ethyl acetate. The organic phase was dried and then concentrated to obtain (*R*)-4-((4-(*tert*-butoxycarbonyl)piperazin-2-yl)methoxy)-6-chloro-2-(pyrrolidin-1-yl)nicotinic acid (crude product, 910 mg) as a yellow solid. ES-API: [M+H]⁺=441.3.

Step 3: (*R*)-4-((4-(*tert*-butoxycarbonyl)piperazin-2-yl)methoxy)-6-chloro-2-(pyrrolidin-1-yl)nicotinic acid (910 mg), diisopropylethylamine (4 mL) and dichloromethane (10 mL) were added to a 100 mL flask. Propylphosphonic anhydride solution (4 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of aqueous hydrochloric acid solution (1 M), and 30 mL of sodium bicarbonate, respectively. The organic phase was dried and concentrated to obtain *tert*-butyl (*R*)-3-chloro-12-oxo-1-(pyrrolidin-1-yl)-6a,7,9,10-tetrahydro-12*H*-pyrazino[2,1-*c*]pyrido[3,4-*f*][1,4]oxazepine-8(6*H*)-carboxylate (crude product, 725 mg) as a yellow solid. ES-API: [M+H]⁺=423.3.

Step 4: *tert*-Butyl (*R*)-3-chloro-12-oxo-1-(pyrrolidin-1-yl)-6a,7,9,10-tetrahydro-12H-pyrazino[2,1-*c*]pyrido[3,4-*f*][1,4]oxazepine-8(6*H*)-carboxylate (crude product, 725 mg), *N-*chlorosuccinimide (442.6 mg, 3.3 mmol) and acetonitrile (10 mL) were added to a 100 mL flask. The reaction was stirred at 80 °C for 2 hours. Aqueous sodium thiosulfate solution was added to the reaction solution and the mixture was extracted with ethyl acetate. The organic phase was dried, concentrated and then purified by flash silica gel column (EtOAc/PE: 0-50%) to obtain *tert*-butyl (*R*)-3,4-dichloro-12-oxo-1-(pyrrolidin-1-yl)-6a,7,9,10-tetrahydro-12*H*-pyrazino[2,1-*c*]pyrido[3,4-*f*][1,4]oxazepine-8(6*H*)-carboxylate (550 mg, 72%). ES-API: [M+H]⁺=457.2.

Step 5: *tert*-Butyl (*R*)-3,4-dichloro-12-oxo-1-(pyrrolidin-1-yl)-6a,7,9,10-tetrahydro-12H-pyrazino[2,1-*c*]pyrido[3,4-*f*][1,4]oxazepine-8(6*H*)-carboxylate (255.9 mg, 0.56 mmol), 2-fluoro-6-hydroxyphenylboronic acid (262 mg, 1.68 mmol), SPhos (23 mg, 0.056 mmol), SPhos-Pd-G2 (40 mg, 0.056 mmol), potassium phosphate (356 mg, 1.68 mmol), 5 mL of dioxane and 1 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-3-(2-fluoro-6-hydroxyphenyl)-12-oxo-1-(pyrrolidin-1-yl)-6a,7,9,10-tetrahydro-12*H*-pyrazino[2.1-*c*]pyrido[3,4-*f*][1,4]oxazcpinc-8(6*H*)-carboxylate (142 mg, 47%) as a yellow solid. ES-API: [M+H]⁺=533.2.

Step 6: *tert*-Butyl (6a*R*)-4-chloro-3-(2-fluoro-6-hydroxyphenyl)-12-oxo-1-(pyrrolidin-1-yl)-6a,7,9,10-tetrahydro-12*H*-pyrazino[2,1-*c*]pyrido[3,4-*f*][1,4]oxazepine-8(6*H*)-carboxylate (133 mg, 0.25 mmol), 3 mL of dichloromethane and trifluoroacetic acid (2 mL) were added to a 50 mL flask. The mixture was stirred at room temperature for 1 hour, and the completion of reaction was detected by LC-MS. The reaction solution was concentrated to obtain a crude product (130 mg) as a yellow oil, and then 5 mL of dichloromethane and triethylamine (166 mg, 1.65 mmol) were added. At 0°C, a solution of acrylic anhydride in dichloromethane (30 mg, 0.24 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3 -(2-fluoro-6-hydroxyphenyl)-1-(pyrrolidin-1-yl)-6,6a,7,8,9,10-hexahydro-12*H*-pyrazino [2,1-*c*]pyrido[3,4-*f*][1,4]oxazepin-12-one (Z11, 16 mg, 13.2%) as a white solid. ES-API: [M+H]⁺=487.2.

### Embodiment 12 Synthesis of (6aR)-8-acryloyl-4-chloro-3-(2-fluoro-6-hydroxyphenyl)-1-(((R)-1-methylpyrrolidin-2-yl)methoxy)-6,6a,7,8,9,10-hexahydro-12H-pyrazino[2,1-c]pyrido[3,4-f][1,4]oxazepin-12-one Z13

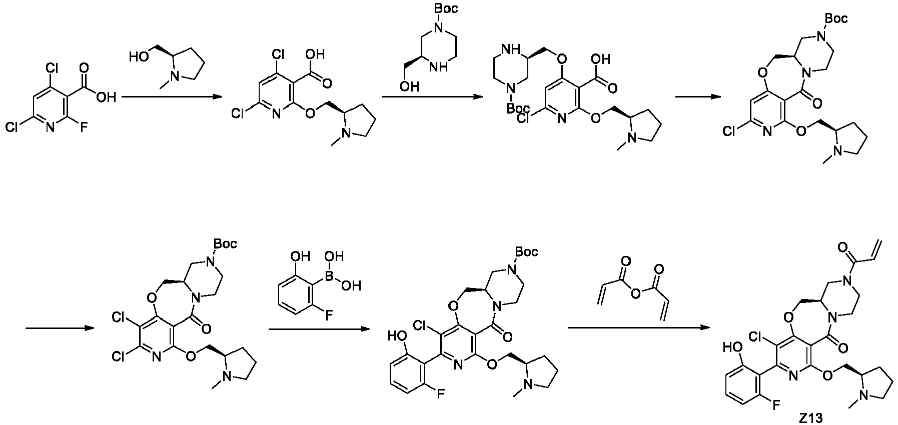

Step 1: (*R*)-(1-Methylpyrrolidin-2-yl)methanol (328 mg, 2.85 mmol) and 7 mL of THF were added to a 100 mL flask. After the system was cooled to 0°C, sodium bis(trimethylsilyl)amide (3.6 mL, 2 M solution in THF, 7.12 mmol) was added dropwise to the reaction solution. After the dropwise addition, the reaction was carried out at 0°C for 10 minutes. Then, a solution of 4,6-dichloro-2-fluoronicotinic acid (600 mg, 2.85 mmol) in THF (3 mL) was added dropwise to the reaction solution. After the dropwise addition, the reaction was carried out at 0°C for 20 minutes. After the reaction was completed, the reaction solution was directly used in the next step without post-treatment. ES-API: [M+H]⁺=305.0.

Step 2: 60% sodium hydride (342 mg, 8.55 mmol), *tert*-butyl (*R*)-3-(hydroxymethyl)piperazine-1-carboxylate (739 mg, 3.42 mmol) and THF (5 mL) were added to a 100 mL flask. At 0°C, the reaction solution of step 1 was added dropwise thereto. The reaction was carried out in an oil bath at 60°C for 1 hour. The completion of reaction was detected by LC-MS. The reaction solution was poured into ice-water. The pH of the reaction solution was adjusted to 7 with 3 M hydrochloric acid. The above solution was concentrated to dryness to obtain a yellow solid. DCM/MeOH (v/v 10:1, 20 mL) was added to the solid, stirred at room temperature for 10 minutes, filtered, and the filtrate was dried and concentrated to obtain 4-(((*R*)-4-(*tert*-butoxycarbonyl)piperazin-2-yl)methoxy)-6-chloro-2-(((*R*)-1-1-methylpyrrolidin-2-yl)methoxy)nicotinic acid (1.3 g, crude product) as a yellow solid. ES-API: [M+H]⁺=485.2.

Step 3: 4-(((*R*)-4-(*tert*-Butoxycarbonyl)piperazin-2-yl)methoxy)-6-chloro-2-(((R)-1-methylpyrrolidin-2-yl)methoxy)nicotinic acid (1.3 g, crude product), *N*,*N*-diisopropylethylamine (3 mL) and dichloromethane (10 mL) were added to a 100 mL flask. Propylphosphonic anhydride solution (3 mL, 50%w/w ethyl acetate solution) was added dropwise thereto. The mixture was reacted at room temperature for 1 hour. The completion of reaction was detected by LC-MS. 50 mL of dichloromethane was added to the reaction, then washed with 30 mL of water, and 30 mL of saturated aqueous sodium bicarbonate solution, respectively. The organic phase was dried, concentrated and then purified by flash silica gel column (MeOH/DCM: 0-10%) to obtain *tert*-butyl (*R*)-3-chloro-1-(((R)-1-methylpyrrolidin-2-yl)methoxy)-12-oxo-6a,7,9,10-tetrahydro-12*H*-pyrazino[2,1-*f*]pyrido[3,4-*f*][1,4]oxazepine-8(6*H*)-carboxylate (700 mg, 52%) as a yellow solid. ES-API: [M+H]⁺=467.3.

Step 4: *tert*-Butyl (*R*)-3-chloro-1-(((*R*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (700 mg, 1.5 mmol), N-chlorosuccinimide (442 mg, 3.3 mmol) and acetonitrile (10 mL) were added to a 100 mL flask. The reaction was stirred at 80 °C for 2 hours. Aqueous sodium thiosulfate solution was added to the reaction solution and the mixture was extracted with ethyl acetate. The organic phase was dried, concentrated and then purified by flash silica gel column (MeOH/DCM: 0-5%) to obtain *tert*-butyl (*R*)-3,4-dichloro-1-(((*R*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (390 mg, 51%). ES-API: [M+H]⁺=501.2.

Step 5: *tert*-Butyl (*R*)-3,4-dichloro-1-(((*R*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (390 mg, 0.78 mmol), 2-fluoro-6-hydroxyphenylboronic acid (365 mg, 2.34 mmol), Sphos (32 mg, 0.078 mmol), Sphos-Pd-G2 (56 mg, 0.078 mmol), potassium phosphate (496 mg, 2.34 mmol), 10 mL of dioxane and 2 mL of water were added to a 100 mL reaction flask. The reaction was stirred in an oil bath at 70°C for 2 hours, then the reaction solution was added with 20 mL of water, extracted three times with 20 mL of ethyl acetate. The organic phase was dried, concentrated and then purified by flash silica gel column (MeOH/DCM: 0-5%) to obtain *tert*-butyl (6a*R*)-4-chloro-3-(2-fluoro-6-hydroxyphenyl)-1-(((*R*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (330 mg, yield of 73%) as a yellow solid. ES-API: [M+H]⁺=577.3.

Step 6: *tert-*Butyl (6a*R*)-4-chloro-3-(2-fluoro-6-hydroxyphenyl)-1-(((*R*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (330 mg, 0.57 mmol), methanol (2 mL) and 4 M hydrogen chloride/dioxane solution (5 mL) were added to a flask. After stirring at room temperature for 1 hour, the reaction solution was concentrated to obtain a yellow solid. The yellow solid was dissolved in 5 mL of dichloromethane and triethylamine (288 mg, 2.85 mmol). At 0°C, a solution of acrylic anhydride in dichloromethane (57 mg, 0.45 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-3-(2-fluoro-6-hydroxyphenyl)-1-(((*R*)-1-methylpyrrolidin-2-yl)methoxy)-6,6a,7,8,9,10-hexahydro-12*H*-pyrazino[2,1-*c*]pyrido[3,4-*f*][1,4]oxazepin-12-one (Z13, 32 mg, yield of 10%) as a white solid. ¹H NMR (500 MHz, CDCl₃) 7.31-7.26 (m, 1H), 6.84-6.83 (m, 1H), 6.70-6.69 (m, 1H), 6.54-6.41 (m, 2H), 5.83-5.81 (m, 1H), 4.42-4.12 (m, 7H), 3.92-3.91 (m, 2H), 3.70-3.60 (m, 2H), 3.12-2.90 (m, 2H), 2.50-2.38 (m, 5H), 2.03-2.02 (m, 1H), 1.84-1.83 (m, 2H); ES-API: [M+H]⁺=531.2.

### Embodiment 13 Synthesis of (6aR)-8-acryloyl-4-chloro-3-(2-fluoro-6-hydroxyphenyl)-1-(((S)-1-methylpyrrolidin-2-yl)methoxy)-6,6a,7,8,9,10-hexahydro-12H-pyrazino[2,1-c]pyrido[3,4-f][1,4]oxazepin-12-one Z14

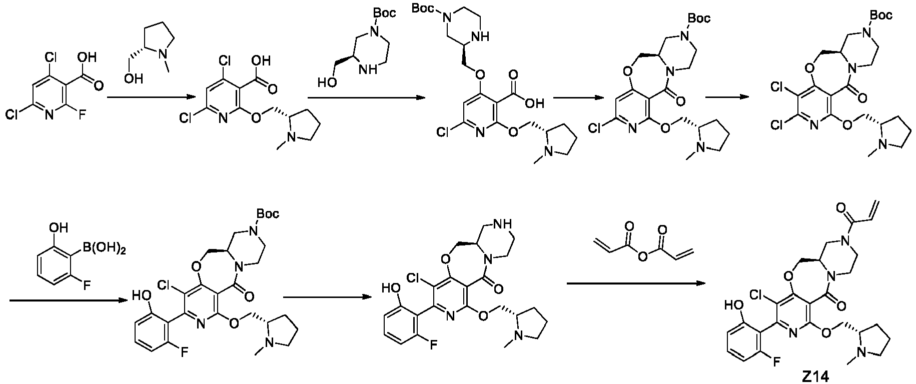

Step 1: A solution of (*S*)-(1-methylpyrrolidin-2-yl)methanol (543 mg, 4.71 mmol) in THF (9 mL) was cooled to 0°C, and a solution of 2 M sodium bis(trimethylsilyl)amino in THF (4.29 mL, 8.58 mmol) was slowly added thereto and stirred for 10 minutes. 4,6-Dichloro-2-fluoronicotinic acid (900 mg, 4.29 mmol) was then added to the mixture and stirring was continued for 10 minutes. After the reaction was completed, the reaction was quenched with 1M dilute hydrochloric acid, and concentrated under vacuum to obtain (*S*)-4,6-dichloro-2-((1-methylpyrrolidin-2-yl)methoxy)nicotinic acid (1.31 g, crude product) as a yellow solid. ES-API: [M+H]⁺=305.0.

Step 2: *tert*-Butyl (*R*)-3-(hydroxymethyl)piperazine-1-carboxylate (1.86 g, 8.59 mmol) was added to a turbid solution of sodium hydride (860 mg, 21.46 mmol) in THF (10 mL) under an ice bath, and stirred for 10 minutes. Then the crude product of (*S*)-4,6-dichloro-2-((1-methylpyrrolidin-2-yl)methoxy)nicotinic acid (1.31 g, crude product) obtained above was added thereto, heated to 60°C and stirred for 1 hour. After the reaction was completed, the reaction solution was quenched with 10 mL of saturated ammonium chloride solution, and concentrated to obtain 4-(((*R*)-4-(*tert*-butoxycarbonyl)piperazin-2-yl)methoxy)-6-chloro-2-(((*S*)-1-methylpyrrolidin-2-yl)methoxy)nicotinic acid (2.08 g, crude product). ES-API: [M+H]⁺=485.2.

Step 3: 4-(((*R*)-4-(*tert*-Butoxycarbonyl)piperazin-2-yl)methoxy)-6-chloro-2-(((*S*)-1-methylpyrrolidin-2-yl)methoxy)nicotinic acid (2.08 g, crude product) was dissolved in dichloromethane (20 mL), and *N,N*-diisopropylethylamine (7.28 mL) was added thereto, and then a solution of 1-propylphosphonic anhydride in ethyl acetate (7.28 mL) was added thereto. The mixture was stirred at room temperature for 10 minutes. The reaction solution was poured into 50 mL of dichloromethane, and washed three times with 50 mL of sodium bicarbonate solution. The organic phase was dried, concentrated and then purified by flash silica gel column (MeOH/DCM: 0-10%) to obtain *tert*-butyl (*R*)-3-chloro-1-(((*S*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (900 mg, 3-step yield of 45%). ES-API: [M+H]⁺=467.2.

Step 4: A solution of *tert*-butyl (*R*)-3-chloro-1-(((*S*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (500 mg, 1.07 mmol) in acetonitrile (5 mL) was added with *N*-chlorosuccinimide (286 mg, 2.14 mmol) and stirred at 80°C for 4 hours. The reaction solution was concentrated and purified by flash silica gel column (MeOH/DCM: 0-10%) to obtain *tert*-butyl (*R*)-3,4-dichloro-1-(((*S*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (120 mg, 22%). ES-API: [M+H]⁺=501.3.

Step 5: Under nitrogen protection, a mixed solution of *tert*-butyl (*R*)-3,4-dichloro-1-(((*S*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (120 mg, 0.24 mmol), 2-fluoro-6-hydroxyphenylboronic acid (112 mg, 0.72 mmol), tetrakis(triphenylphosphine)palladium (28 mg, 0.02 mmol) and potassium carbonate (66 mg, 0.48 mmol) in 1,4-dioxane (2 mL) and water (0.4 mL) was reacted at 110°C for 30 minutes under microwave irradiation. The reaction solution was added to 30 mL of water and extracted three times with 30 mL of ethyl acetate. The organic phases were combined, dried and concentrated to obtain *tert*-butyl (6a*R*)-4-chloro-3-(2-fluoro-6-hydroxyphenyl)-1-(((*S*)-1-methylpyrrolidin-2-yl)methoxy)-12-oxo-6a,7,9,10-tetrahydro-12*H*-pyrazino[2,1-*c*]pyrido[3,4-*f*][1,4]oxazepine-8(6*H*)-carboxylate (200 mg, crude product). ES-API: [M+H]⁺=577.3.

Step 6: Under an ice bath, trifluoroacetic acid (1 mL) was added to a solution of *tert*-butyl (6a*R*)-4-chloro-3-(2-fluoro-6-hydroxyphenyl)-1-(((*S*)-1-methylpyrrolidin-2-yl)methoxy)-12-oxo-6a,7,9,10-tetrahydro-12H-pyrazino[2,1-*c*]pyrido[3,4-*f*][1,4]oxazepine-8(6*H*)-carboxylate (200 mg, crude product) in dichloromethane (4 mL), warmed to room temperature and stirred for 1 hour. The reaction solution was concentrated to obtain (6a*R*)-4-chloro-3-(2-fluoro-6-hydroxyphenyl)-1-(((*S*)-1-methylpyrrolidin-2-yl)methoxy)-6,6a,7,8,9,10-hexahydro-12*H*-pyrazino[2,1-*c*]pyrido[3,4-*f*][1,4]oxazepin-12-one (200 mg, crude product) as a yellow oil. ES-API: [M+H]⁺=477.3.

Step 7: Under an ice bath, *N,N*-diisopropylethylamine (500 mg) was added to a solution of the above crude product (6a*R*)-4-chloro-3-(2-fluoro-6-hydroxyphenyl)-1-(((*S*)-1-methylpyrrolidin-2-yl)methoxy)-6,6a,7,8,9,10-hexahydro-12*H*-pyrazino[2,1-*c*]pyrido[3,4-*f*][1,4]oxazepin-12-one (200 mg) in dichloromethane (4 mL), and then acrylic anhydride (25 mg) was added thereto and stirred for 10 minutes. The reaction solution was concentrated and redissolved in a mixed solution of THF (4 mL) and water (1 mL), and lithium hydroxide monohydrate (300 mg) was added thereto, and the mixture was stirred for 1 hour. The reaction solution was added with 5 mL of water, and the pH was adjusted to 7 with 1 M hydrochloric acid. The mixture was extracted with 10 mL of dichloromethane, concentrated and subjected to preparative HPLC to obtain (6a*R*)-8-acryloyl-4-chloro-3-(2-fluoro-6-hydroxyphenyl)-1-(((*S*)-1-methylpyrrolidin-2-yl)methoxy)-6,6a,7,8,9,10-hexahydro-12*H*-pyrazino[2,1-*c*]pyrido[3,4-*f*][1,4]oxazepin-12-one (Z14, 12.7 mg, 3-step yield of 10%) as a yellow solid. ES-API: [M+H]⁺=531.2.

### Embodiment 14 Synthesis of (6aR)-8-acryloyl-4-chloro-1-((4,6-diisopropylpyrimidin-5-yl)amino)-3-(2-fluoro-6-hydroxyphenyl)-6,6a,7,8,9,10-hexahydro-12H-pyrazino[2,1-c]pyrido[3,4-f][1,4]oxazepin-12-one Z15

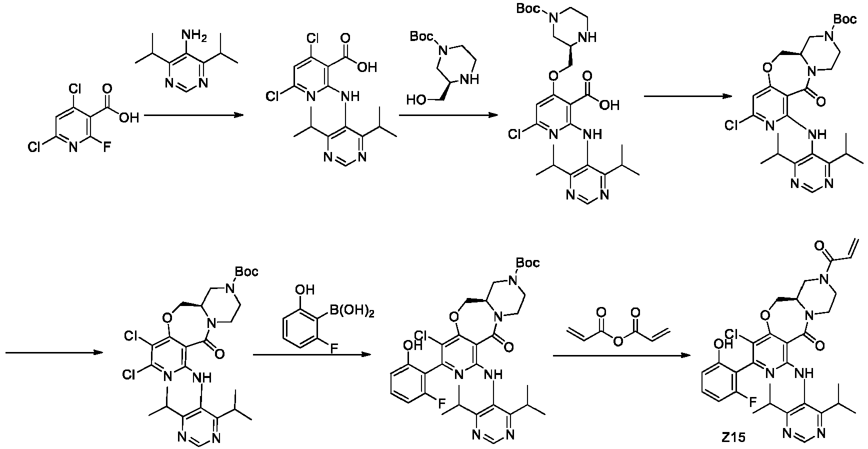

Step 1: 4,6-Diisopropylpyrimidin-5-amine (2.56 g, 14.28 mmol) and 40 mL of THF were added to a 100 mL flask. After the system was cooled to 0°C, sodium bis(trimethylsilyl)amide (21.42 mL, 2 M solution in THF, 42.84 mmol) was added dropwise to the reaction solution. After the dropwise addition, the reaction was carried out at 0°C for 10 minutes. Then, a solution of 4,6-dichloro-2-fluoronicotinic acid (3 g, 14.28 mmol) in THF (20 mL) was added dropwise to the reaction solution. After the dropwise addition, the reaction was carried out at 0°C for 20 minutes. Then the reaction solution was poured into ice-water, and the aqueous phase was extracted with ethyl acetate to remove impurities. The pH of the aqueous phase was adjusted to 3 with 6 M hydrochloric acid, and the aqueous phase was extracted with ethyl acetate, and the organic phase was dried, and evaporated to dryness by rotary evaporation to obtain *tert*-butyl (*R*)-3-(hydroxymethyl)piperazine-1-carboxylate (2.3 g, yield of 43.8%). ES-API: [M+H]⁺=369.0.

Step 2: 60% sodium hydride (1.03 g, 25.8 mmol), *tert*-butyl (*R*)-3-(hydroxymethyl)piperazine-1-carboxylate (2.78 g, 12.9 mmol) and THF (30 mL) were added to a 100 mL flask. A solution of 4,6-dichloro-2-(((4,6-diisopropylpyrimidin-5-yl)amino)nicotinic acid (1.6 g, 4.3 mmol) in THF (10 mL) was added dropwise thereto at 0°C. The reaction was carried out in an oil bath at 60°C for 8 hours. The reaction solution was poured into 100 mL of saturated aqueous NH₄Cl solution. The mixture was extracted 2 times with 80 mL of ethyl acetate. The organic phase was dried and then concentrated to obtain (*R*)-4-((4-(*tert*-butoxycarbonyl)piperazin-2-yl)methoxy)-6-chloro-2-((4,6-diisopropylpyrimidin-5-yl)amino)nicotinic acid (2.6 g, crude product). ES-API: [M+H]⁺=549.2.

Step 3: (*R*)-4-((4-(*tert*-Butoxycarbonyl)piperazin-2-yl)methoxy)-6-chloro-2-((4,6-diisopropylpyrimidin-5-yl)amino)nicotinic acid (1.5 g, 2.8 mmol), diisopropylethylamine (8 mL) and dichloromethane (30 mL) were added to a 100 mL flask. Propylphosphonic anhydride solution (8 mL, 50%w/w ethyl acetate solution) was added dropwise thereto. The mixture was reacted at room temperature for 1 hour. The completion of reaction was detected by LC-MS. 60 mL of dichloromethane was added to the reaction, then washed with 60 mL of water, 30 mL of aqueous hydrochloric acid solution (1 M), and 60 mL of sodium bicarbonate, respectively. The organic phase was dried, concentrated and then purified by column chromatography (PE/EtOAc=1/1, R_{f}=0.5) to obtain *tert*-butyl (*R*)-3-chloro-1-((4,6-diisopropylpyrimidin-5-yl)amino)-12-oxo-6a,7,9,10-tetrahydro-6*H*-pyrazino[2,1-*c*]pyrido[3,4-*f*][1,4]oxazepine-8(6*H*)-carboxylate (1 g, yield of 67.5%) as a product. ES-API: [M+H]⁺=531.2.

Step 4: *tert*-Butyl (*R*)-3-chloro-1-((4,6-diisopropylpyrimidin-5-yl)amino)-12-oxo-6a,7,9,10-tetrahydro-6*H*-pyrazino[2,1-*c*]pyrido[3,4-*f*][1,4]oxazepine-8(6*H*)-carboxylate (900 mg, 0.1.69 mmol), *N-*chlorosuccinimide (451 mg, 3.38 mmol) and acetonitrile (10 mL) were added to a 100 mL flask. The reaction was stirred at 80 °C for 1 hour. The reaction solution was extracted with ethyl acetate/water. The organic phase was dried and concentrated. The crude product was purified by flash silica gel column (EtOAc/PE: 0-30%, R_{f}=0.4) to obtain *tert*-butyl (*R*)-3,4-dichloro-1-((4,6-diisopropylpyrimidin-5-yl)amino)-12-oxo-6a,7,9,10-tetrahydro-6*H*-pyrazino[2,1-*c*]pyrido[3,4-*f*][1,4]oxazepine-8(6*H*)-carboxylate (480 mg, yield of 51%). ¹H NMR (400 MHz, CDCl₃) δ 9.05 (s, 1H), 4.42 (d, *J* = 4.2 Hz, 2H), 4.17-4.03 (m, 3H), 3.94-3.61 (m, 5H), 3.30-3.10 (m, 2H), 1.50 (s, 9H), 1.28 (d, *J* = 6.7 Hz, 6H), 1.17 (d, *J* = 6.7 Hz, 6H). ES-API: [M+H]⁺=565.2.

Step 5: *tert*-Butyl (*R*)-3,4-dichloro-1-((4,6-diisopropylpyrimidin-5-yl)amino)-12-oxo-6a,7,9,10-tetrahydro-12*H*-pyrazino[2,1-*c*]pyrido[3,4-*f*][1,4]oxazepine-8(6*H*)-carboxylate (200 mg, 0.35 mmol), 2-fluoro-6-hydroxyphenylboronic acid (262 mg, 1.68 mmol), tetrakis(triphenylphosphine)palladium (40 mg, 0.035 mmol), potassium carbonate (356 mg, 1.68 mmol), 5 mL of dioxane and 1 mL of water were added to a 100 mL reaction flask. The reaction was carried out in an oil bath at 90°C for 2 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-1-((4,6-diisopropylpyrimidin-5-yl)amino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (180 mg, 80%) as a yellow solid. ES-API: [M+H]⁺=641.2.

Step 6: *tert*-Butyl (6a*R*)-4-chloro-1-((4,6-diisopropylpyrimidin-5-yl)amino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (180 mg, 0.28 mmol), trifluoroacetic acid (2 mL) and 2 mL of dichloromethane were added to a 50 mL flask. The mixture was stirred at room temperature for 1 hour, and the completion of reaction was detected by LC-MS. The reaction solution was concentrated to obtain a crude product (160 mg), and then 3 mL of dichloromethane and triethylamine (166 mg, 1.65 mmol) were added. At 0°C, a solution of acrylic anhydride in dichloromethane (28 mg, 0.22 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-1-((4,6-diisopropylpyrimidin-5 -yl)amino)-3-(2-fluoro-6-hydroxyphenyl)-6,6a,7, 8,9,10-hexahydro-12*H*-pyrazino[2,1-*c*]pyrido[3,4-*f*][1,4]oxazepin-12-one (Z15, 25 mg, 15%) as a white solid. ¹H NMR (400 MHz, DMSO-*d*₆) δ 9.95 (s, 1H), 8.85 (s, 1H), 7.16 (dd, *J* = 15.4, 8.2 Hz, 1H), 6.81-6.56 (m, 3H), 6.23-6.14 (m, 1H), 5.75 (t, *J* = 11.9 Hz, 1H), 4.57-4.31 (m, 3H), 4.08-3.66 (m, 6H), 3.30-3.16 (m, 2H), 1.17-1.00 (m, 12H). ES-API: [M+H]⁺=595.2.

### Embodiment 15 Synthesis of (6aR)-8-acryloyl-4-chloro-1-((3-chloro-1-isopropyl-4-methyl-1H-pyrazol-5-yl)amino)-3-(2-fluoro-6-hydroxyphenyl)-6,6a,7,8,9,10-hexahydro-12H-pyrazino[2,1-c]pyrido[3,4-f][1,4]oxazepin-12-one Z16

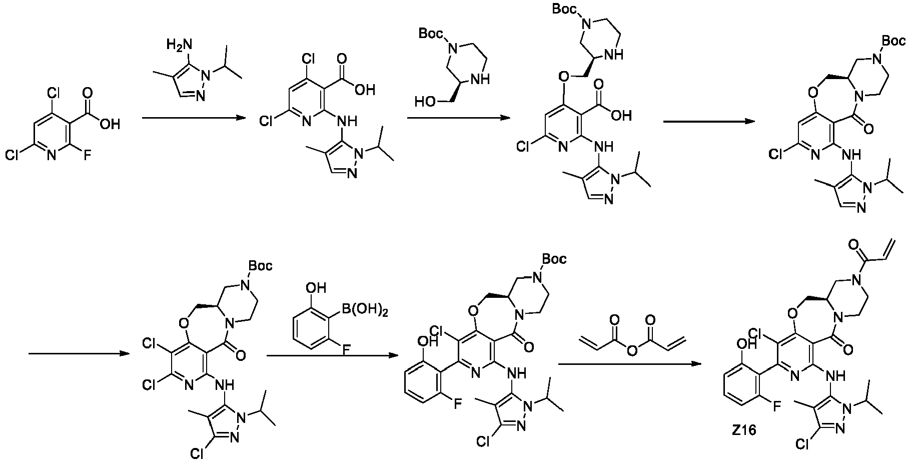

Step 1: 1-Isopropyl-4-methyl-5-aminopyrazole (662 mg, 4.76 mmol) and 20 mL of THF were added to a 100 mL flask. After the system was cooled to 0°C, sodium bis(trimethylsilyl)amide (7.1 mL, 2 M solution in THF, 14.2 mmol) was added dropwise to the reaction solution. After the dropwise addition, the reaction was carried out at 0°C for 10 minutes. Then, a solution of 4,6-dichloro-2-fluoronicotinic acid (1 g, 4.76 mmol) in THF (6 mL) was added dropwise to the reaction solution. After the dropwise addition, the reaction was carried out at 0°C for 20 minutes, then the reaction solution was poured into ice-water, and the aqueous phase was extracted with ethyl acetate to remove impurities. The pH of the aqueous phase was adjusted to 3 with 6 M hydrochloric acid, and the aqueous phase was extracted with ethyl acetate, and then the organic phase was dried, and evaporated to dryness by rotary evaporation to obtain 4,6-dichloro-2-((1-isopropyl-4-methyl-1*H*-pyrazol-5-yl)amino)nicotinic acid (1.1 g, yield of 70%). ES-API: [M+H]⁺=329.0.

Step 2: 60% sodium hydride (468 mg, 11.7 mmol), *tert*-butyl (*R*)-3-(hydroxymethyl)piperazine-1-carboxylate (867 mg, 4.0 mmol) and THF (15 mL) were added to a 100 mL flask. A solution of 4,6-dichloro-2-((1-isopropyl-4-methyl-1*H*-pyrazol-5-yl)amino)nicotinic acid (1.1 g, 3.34 mmol) in THF (10 mL) was added dropwise thereto at 0°C. The reaction was carried out in an oil bath at 60°C for 8 hours. The reaction solution was poured into 100 mL of saturated aqueous NH₄Cl solution. The mixture was extracted 2 times with 80 mL of ethyl acetate. The organic phase was dried and then concentrated to obtain (*R*)-4-((4-(*tert*-butoxycarbonyl)piperazin-2-yl)methoxy)-6-chloro-2-((1-isopropyl-4-methyl-1*H*-pyrazol-5-yl)amino)nicotinic acid (2.1 g, crude product). ES-API: [M+H]⁺=509.2.

Step 3: (*R*)-4-((4-(*tert*-Butoxycarbonyl)piperazin-2-yl)methoxy)-6-chloro-2-((1-isopropyl-4-methyl-1*H*-pyrazol-5-yl)amino)nicotinic acid (2.1 g, 4.12 mmol), diisopropylethylamine (11 mL) and dichloromethane (40 mL) were added to a 100 mL flask. Propylphosphonic anhydride solution (11 mL, 50%w/w ethyl acetate solution) was added dropwise thereto. The mixture was reacted at room temperature for 1 hour. The completion of reaction was detected by LC-MS. 60 mL of dichloromethane was added to the reaction, then washed with 60 mL of water, 30 mL of aqueous hydrochloric acid solution (1 M), and 60 mL of sodium bicarbonate, respectively. The organic phase was dried, concentrated and then purified by column chromatography (PE/EtOAc=1/1, R_{f}=0.4) to obtain *tert*-butyl (*R*)-3-chloro-1-((1-isopropyl-4-methyl-1*H*-pyrazol-5-yl)amino)-12-oxo-6a,7,9,10-tetrahydro-12*H*-pyrazino[2,1-*c*]pyrido[3,4-*f*][1,4]oxazepine-8(6*H*)-carboxylate (650 mg, yield of 32%) as a product. ES-API: [M+H]⁺=491.0.

Step 4: *tert*-Butyl (*R*)-3-chloro-1-((1-isopropyl-4-methyl-1*H*-pyrazol-5-yl)amino)-12-oxo-6a,7,9,10-tetrahydro-12*H*-pyrazino[2,1-*c*]pyrido[3,4-*f*][1,4]oxazepine-8(6*H*)-carboxylate (200 mg, 0.41 mmol), *N*-chlorosuccinimide (162 mg, 1.2 mmol) and acetonitrile (2 mL) were added to a 100 mL flask. The reaction was stirred at 80°C for 1 hour. The reaction solution was extracted with ethyl acetate/water. The organic phase was dried and concentrated. The crude product was purified by flash silica gel column (EtOAc/PE: 0-50%) to obtain *tert*-butyl (*R*)-3,4-dichloro-1-((3-chloro-1-isopropyl-4-methyl-1*H*-pyrazol-5-yl)amino)-12-oxo-6a,7,9,10-tetrahydro-12*H*-pyrazino[2,1-*c*]pyrido[3,4-*f*][1,4]oxazepine-8(6*H*)-carboxylate (120 mg, yield of 52%). ¹H NMR (400 MHz, CDCl₃) δ 8.97 (s, 1H), 4.54-4.44 (m, 1H), 4.40 (dd, *J=* 11.1, 4.1 Hz, 1H), 4.30 (p, *J* = 6.6 Hz, 1H), 4.09-3.99 (m, 2H), 3.94-3.59 (m, 5H), 1.87 (s, 3H), 1.49 (s, 9H), 1.45 (d, *J* = 6.6 Hz, 3H), 1.41 (d, *J* = 6.6 Hz, 3H). ES-API: [M+H]⁺=559.1.

Step 5: *tert*-Butyl (*R*)-3,4-dichloro-1-((3-chloro-1-isopropyl-4-methyl-1*H*-pyrazol-5-yl)amino)-12-oxo-6a,7,9,10-tetrahydro-12*H*-pyrazino[2,1-*c*]pyrido[3,4-*f*][1,4]oxazepine-8(6*H*)-carboxylate (195 mg, 0.35 mmol), 2-fluoro-6-hydroxyphenylboronic acid (262 mg, 1.68 mmol), tetrakis(triphenylphosphine)palladium (40 mg, 0.035 mmol), potassium carbonate (356 mg, 1.68 mmol), 5 mL of dioxane and 1 mL of water were added to a 100 mL reaction flask. The reaction was carried out in an oil bath at 90°C for 2 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-1-((3-chloro-1-isopropyl-4-methyl-1*H*-pyrazol-5-yl)amino)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (178 mg, 80%) as a yellow solid. ES-API: [M+H]⁺=635.2.

Step 6: *tert-*Butyl (6a*R*)-4-chloro-1-((3-chloro-1-isopropyl-4-methyl-1*H*- pyrazol-5-yl)amino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (177.8 mg, 0.28 mmol), trifluoroacetic acid (2 mL) and 2 mL of dichloromethane were added to a 50 mL flask. The mixture was stirred at room temperature for 1 hour, and the completion of reaction was detected by LC-MS. The reaction solution was concentrated to obtain a crude product (150 mg). 3mL of dichloromethane and triethylamine (166 mg, 1.65 mmol) were added to the crude product. At 0°C, a solution of acrylic anhydride in dichloromethane (28 mg, 0.22 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-1-((3-chloro-1-isopropyl-4-methyl-1*H*-pyrazol-5-yl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z16, 50 mg, 30%) as a white solid. ¹H NMR (400 MHz, DMSO-*d*₆) δ 10.05-9.95 (m, 1H), 8.82 (d, *J* = 15.1 Hz, 1H), 7.23 (dd, *J =* 15.3, 8.3 Hz, 1H), 6.79-6.64 (m, 3H), 6.22-6.14 (m, 1H), 5.78-5.70 (m, 1H), 4.55-4.36 (m, 4H), 3.99-3.68 (m, 6H), 1.74 (s, 3H), 1.25 (d, *J* = 6.6 Hz, 3H), 1.21 (d, *J* = 6.6 Hz, 3H). ES-API: [M+H]⁺=589.2.

### Embodiment 16 Synthesis of (6aR)-8-acryloyl-4-chloro-3-(2-fluoro-6-hydroxyphenyl)-1-methoxy-6,6a,7,8,9,10-hexahydro-12H-pyrazino[2,1-c]pyrazino[3,4-f][1,4]oxazepin-12-one Z17

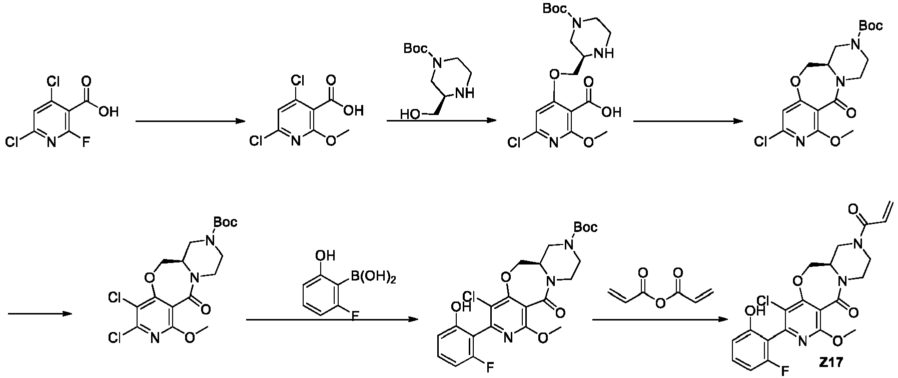

Step 1: 4,6-Dichloro-2-fluoronicotinic acid (500 mg, 2.39 mmol), sodium methoxide (387.1 mg, 7.17 mmol) and methanol (5 mL) were added to a 100 mL flask, and stirred at room temperature. After the reaction was completed, the reaction solution was poured into ice-water, and the aqueous phase was extracted with ethyl acetate to remove impurities. The pH of the aqueous phase was adjusted to 3 with 6 M hydrochloric acid, and the aqueous phase was extracted with ethyl acetate (20 mL×2), dried and concentrated to obtain 4,6-dichloro-2-methoxynicotinic acid (481 mg, 90%). ES-API: [M+H]⁺=222.1.

Step 2: 60% sodium hydride (259 mg, 6.48 mmol), *tert*-butyl (*R*)-3-(hydroxymethyl)piperazine-1-carboxylate (466 mg, 2.16 mmol) and THF (8 mL) were added to a 100 mL flask. A THF suspension (4 mL) of 4,6-dichloro-2-methoxynicotinic acid (481 mg, 2.16 mmol) was added dropwise thereto at 0°C. The reaction was carried out in an ice bath at 0°C for 20 minutes. The completion of reaction was detected by LC-MS. 30 mL of saturated aqueous NH₄Cl solution was poured into the reaction solution, and the pH was adjusted to 5-6, and the mixture was extracted 4 times with 40 mL of ethyl acetate. The organic phase was dried and then concentrated to obtain (*R*)-4-((4-(*tert*-butoxycarbonyl)piperazin-2-yl)methoxy)-6-chloro-2-methoxynicotinic acid (823 mg, crude product) as a yellow solid. ES-API: [M+H]⁺=402.3.

Step 3: (*R*)-4-((4-(*tert*-Butoxycarbonyl)piperazin-2-yl)methoxy)-6-chloro-2-methoxynicotinic acid (823 mg, crude product), diisopropylethylamine (4 mL) and dichloromethane (10 mL) were added to a 100 mL flask. Propylphosphonic anhydride solution (4 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of aqueous hydrochloric acid solution (1 M), and 30 mL of sodium bicarbonate, respectively. The organic phase was dried and concentrated to obtain *tert*-butyl (*R*)-3-chloro-1-methoxy-12-oxo-6a,7,9,10-tetrahydro-12*H*-pyrazino[2,1-*c*]pyrido[3,4-*f*][1,4]oxazepine-8(6*H*)-carboxylate (765 mg, crude product) as a yellow solid. S-API: [M+H]⁺=384.3.

Step 4: *tert*-Butyl (*R*)-3-chloro-1-methoxy-12-oxo-6a,7,9,10-tetrahydro-12*H*-pyrazino[2,1-*c*]pyrido[3,4-*f*][1,4]oxazepine-8(6*H*)-carboxylate (765 mg, crude product), N-chlorosuccinimide (442.6 mg, 3.3 mmol) and acetonitrile (10 mL) were added to a 100 mL flask. The reaction was stirred at 80°C for 2 hours. Aqueous sodium thiosulfate solution was added to the reaction solution and the mixture was extracted with ethyl acetate. The organic phase was dried, concentrated and then purified by flash silica gel column (EtOAc/PE: 0-50%) to obtain *tert*-butyl (*R*)-3,4-dichloro-1-methoxy-12-oxo-6a,7,9,10-tetrahydro-12*H*-pyrazino[2,1-*c*]pyrido[3,4-*f*][1,4]oxazepine-8(6*H*)-carboxylate (550 mg, 61%). ES-API: [M+H]⁺=418.2.

Step 5: *tert*-Butyl (*R*)-3,4-dichloro-1-methoxy-12-oxo-6a,7,9,10-tetrahydro-12*H*-pyrazino[2,1-*c*]pyrido[3,4-*f*][1,4]oxazepine-8(6*H*)-carboxylate (234 mg, 0.56 mmol), 2-fluoro-6-hydroxyphenylboronic acid (262 mg, 1.68 mmol), SPhos (23 mg, 0.056 mmol), SPhos-Pd-G2 (40 mg, 0.056 mmol), potassium phosphate (356 mg, 1.68 mmol), 5 mL of dioxane and 1 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried, concentrated and then purified by flash silica gel column (EtOAc/PE: 0-60%) to obtain *tert*-butyl (6a*R*)-4-chloro-3-(2-fluoro-6-hydroxyphenyl)-1-methoxy-12-oxo-6a,7,9,10-tetrahydro-12*H*-pyrazino[2,1-*c*]pyrido[3,4-*f*][1,4]oxazepine-8(6*H*)-carboxylate (123 mg, 44%) as a yellow solid. ES-API: [M+H]⁺=494.2.

Step 6: The solid *tert*-butyl (6a*R*)-4-chloro-3-(2-fluoro-6-hydroxyphenyl)-1-methoxy-12-oxo-6a,7,9,10-tetrahydro-12*H*-pyrazino[2,1-*c*]pyrido[3,4-*f*][1,4]oxazepine-8(6*H*)-carboxylate (123 mg, 0.25 mmol), 3 mL of dichloromethane and trifluoroacetic acid (2 mL) were added to a 50 mL flask. The mixture was stirred at room temperature for 1 hour, and the completion of reaction was detected by LC-MS. The reaction solution was concentrated to obtain a crude product (130 mg) as a yellow oil, and then 5 mL of dichloromethane and triethylamine (166 mg, 1.65 mmol) were added. At 0°C, a solution of acrylic anhydride in dichloromethane (30 mg, 0.24 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-methoxy-6,6a,7,8,9,10-hexahydro-12*H*-pyrazino[2,1-*c*]pyrido[3,4-*f*][1,4]oxazepin-12-one (Z17, 5 mg, 4.5%) as a white solid. ES-API: [M+H]⁺=448.2.

### Embodiment 17 Synthesis of (6aR)-8-acryloyl-4-chloro-3-(2-fluoro-6-hydroxyphenyl)-1-(2-isopropylphenoxy)-6,6a,7,8,9,10-hexahydro-12H-pyrazino[2,1-c]pyrido[3,4-f][1,4]oxazepin-12-one Z18

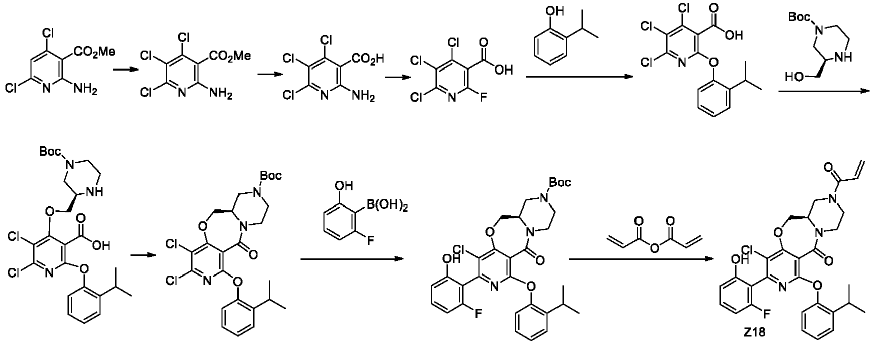

Step 1: Methyl 2-amino-4,6-dichloronicotinate (5.0 g, 22.6 mmol) and *N*-chlorosuccinimide (4.6 g) were dissolved in acetonitrile (150 mL) in a 500 mL flask at room temperature. The reaction was stirred at 80°C for 3 hours. After the reaction was completed, the reaction solution was concentrated to about 50 mL, added with water, extracted with ethyl acetate (100 mL×2), dried and concentrated to obtain a crude product. The crude product was recrystallized with dichloromethane to obtain methyl 2-amino-4,5,6-trichloronicotinate (4.0 g, 70%) as a pale-yellow solid. ES-API: [M+H]⁺=254.9.

Step 2: Methyl 2-amino-4,5,6-trichloronicotinate (1.0 g, 3.93 mmol) was dissolved in 10 mL of THF and 5 mL of water, and sodium hydroxide (310 mg, 7.87 mmol) was added thereto, reacted at room temperature for 4 hours. The pH of the reaction solution was adjusted to 5 with 3.0 M dilute hydrochloric acid, and the reaction solution was extracted with ethyl acetate (40 mL×2). The organic phase was washed with 25 mL of saturated brine, dried and concentrated to obtain 2-amino-4,5,6-trichloroacetic acid (0.94 g, yield of 99%) as a yellow solid. ES-API: [M+H]⁺=241.0.

Step 3: 2-Amino-4,5,6-trichloroacetic acid (400 mg, 1.67 mmol) was dissolved in 4 mL of pyridine hydrofluoride, and sodium nitrite (240 mg, 3.48 mmol) was added thereto in batches at 0°C, and the reaction was stirred at 0°C for 1 hour. The reaction solution was quenched by pouring into an ice-water mixture and extracted twice with 25 mL of ethyl acetate. The organic phase was washed twice with 25 mL of water and twice with 25 mL of saturated brine in turn and dried and concentrated to obtain 4,5,6-trichloro-2-fluoronicotinic acid (400 mg, crude product) as a brown solid. ES-API: [M+H]⁺=244.0. The above crude product was directly used in the next reaction step.

Step 4: 2-Isopropylphenol (164 mg, 1.20 mmol) was dissolved in 3 mL of *N,N*-dimethylformamide. After the system was cooled to 0°C, sodium hydride (60wt%, 145 mg, 3.62 mmol) was added thereto and reacted at 0°C for 15 minutes. Then, a solution of 4,5,6-trichloro-2-fluoronicotinic acid (400 mg, crude product) obtained in the previous step in *N,N-*dimethylformamide (3 mL) was added dropwise to the reaction solution. After the dropwise addition, the reaction was carried out at room temperature for 4 hours. The reaction solution was poured into 20 mL of ice-water. After the mixture was extracted once with ethyl acetate (20 mL) to remove impurities, the pH of the aqueous phase was adjusted to 3 with 3.0 M dilute hydrochloric acid, and the aqueous phase was extracted with ethyl acetate (20 mL×2). The organic phase was washed with 20 mL of water and saturated brine in turn, dried and concentrated to obtain 4,5,6-trichloro-2-(2-isopropylphenoxy)nicotinic acid (390 mg, crude product), which was directly used in the next reaction step. ES-API: [M+H]⁺=360.0.

Step 5: *tert*-Butyl (*R*)-3-(hydroxymethyl)piperazine-1-carboxylate (179 mg, 0.83 mmol) was dissolved in 1.5 mL of THF, and sodium hydride (60wt%, 96 mg, 2.40 mmol) was added thereto at 0°C. The reaction was stirred at 0°C for 15 minutes. 1.5 mL of a solution of 4,5,6-trichloro-2-(2-isopropylphenoxy)nicotinic acid (390 mg, crude product) obtained in step 4 in THF was added dropwise to the above suspension. The reaction was slowly heated to 60°C after the dropwise addition, and stirred at this temperature for 2 hours. The reaction solution was quenched with 2 mL of saturated ammonium chloride solution and extracted with 20 mL of ethyl acetate. The organic phase was washed with 10 mL of saturated brine, dried and concentrated to obtain (R)-4-((4-(*tert*-butoxycarbonyl)piperazin-2-yl)methoxy)-5,6-dichloro-2-(2-isopropylphenoxy)nicotinic acid (450 mg, crude product), which was directly used in the next reaction step. ES-API: [M+H]⁺=540.3.

Step 6: (*R*)-4-((4-(*tert*-Butoxycarbonyl)piperazin-2-yl)methoxy)-5,6-dichloro-2-(2-isopropylphenoxy)nicotinic acid (450 mg, crude product) obtained in the previous step was dissolved in 15 mL of dichloromethane, and then 0.85 mL of diisopropylethylamine, 1.75 mL of propylphosphonic anhydride 50% w/w ethyl acetate solution were added thereto in turn under an ice bath, reacted at room temperature for 1 hour. 30 mL of dichloromethane was added to the reaction, and the organic phase was washed twice with 20 mL of water, washed with 20 mL of saturated brine in turn, dried and concentrated. The crude product was purified by flash silica gel column (EtOAc/PE: 0-40%) to obtain *tert*-butyl (*R*)-3,4-dichloro-1-(2-isopropylphenoxy)-12-oxo-6a,7,9,10-tetrahydro-12*H*-pyrazino[2,1-*c*]pyrido[3,4-*f*][1,4]oxazepine-8(6*H*)-carboxylate (96 mg, a four-step yield of 11%) as a white solid. ES-API: [M+H]⁺=522.2.

Step 7: *tert*-Butyl (*R*)-3,4-dichloro-1-(2-isopropylphenoxy)-12-oxo-6a,7,9,10-tetrahydro-12*H-*pyrazino[2,1-*c*]pyrido[3,4-*f*][1,4]oxazepine-8(6*H*)-carboxylate (96 mg, 0.18 mmol), (2-fluoro-6-hydroxyphenyl)boronic acid (118 mg, 0.75 mmol), tetrakis(triphenylphosphine)palladium (29 mg, 0.025 mmol), potassium carbonate (121 mg, 0.88 mmol), 0.3 mL of water and 1.5 mL of dioxane were added to a 25 mL flask. The reaction was stirred at 90°C for 2 hours under nitrogen protection. The reaction solution was added with 3 mL of water, extracted with ethyl acetate (10×2 mL), and the organic phase was washed with 10 mL of saturated brine, dried and concentrated, and the crude product was purified by flash silica gel column (EtOAc/PE: 0-60%) to obtain *tert*-butyl (6a*R*)-4-chloro-1-((2-isopropylphenoxy)oxy)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (65 mg, 60%) as a yellow solid. ES-API: [M+H]⁺=598.1.

Step 8: *tert*-Butyl (6a*R*)-4-chloro-1-((2-isopropylphenoxy)oxy)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (65 mg, 0.109 mmol) was dissolved in 0.75 mL of dichloromethane, and 0.25 mL of trifluoroacetic acid was added thereto. After stirring at room temperature for 2 hours, the reaction solution was concentrated. The resulting crude product was dissolved in 2 mL of dichloromethane, and triethylamine (147 mL, 1.09 mmol) was added thereto. The reaction was cooled to 0°C, and a solution of acrylic anhydride (12.6 mg, 0.10 mmol) in dichloromethane (0.3 mL) was added to the reaction solution. The reaction was stirred at 0°C for 15 minutes. The reaction solution was added with 30 mL of dichloromethane, washed with 15 mL of water, 15 mL of saturated aqueous NaHCO₃ solution, and 15 mL of saturated brine in turn, dried, concentrated, and purified by preparative HPLC to obtain (6a*R*)-8-acryloyl-4-chloro-3-(2-fluoro-6-hydroxyphenyl)-1-(2-isopropylphenoxy)-6,6a,7,8,9,10-hexahydro-12*H*-pyrazino[2,1-*c*]pyrido[3,4-*f*][1,4]oxazepin-12-one (Z18, 14.73 mg, 2-step yield of 24%) as a white solid. ¹H NMR (500 MHz, DMSO-*d*₆) δ 7.30 (s, 1H), 7.22 (q, *J* = 7.8 Hz, 1H), 7.19-7.09 (m, 2H), 7.03-6.97 (m, 1H), 6.83-6.62 (m, 3H), 6.19 (d, *J* = 17.7 Hz, 1H), 5.85-5.66 (m, 1H), 4.63-4.50 (m, 1H), 4.49-4.32 (m, 2H), 4.17-3.98 (m, 2H), 3.95-3.85 (m, 1H), 3.80-3.56 (m, 2H), 3.44-3.36 (m, 1H), 3.25-3.09 (m, 1H), 1.14 (d, *J* = 6.3 Hz, 3H), 1.09-0.99 (m, 3H). ES-API: [M+H]⁺=552.1.

### Embodiment 18 Synthesis of 1-((6aR)-4-chloro-3-(2-fluoro-6-hydroxyphenyl)-1-((2-isopropyl-4-methylpyridin-3-yl)amino)-6a,7,9,10-tetrahydro-12H-pyrazino[2,1-c]pyrido[3,4-f][1,4]oxazepin-8(6H)-yl)prop-2-en-1-one Z19

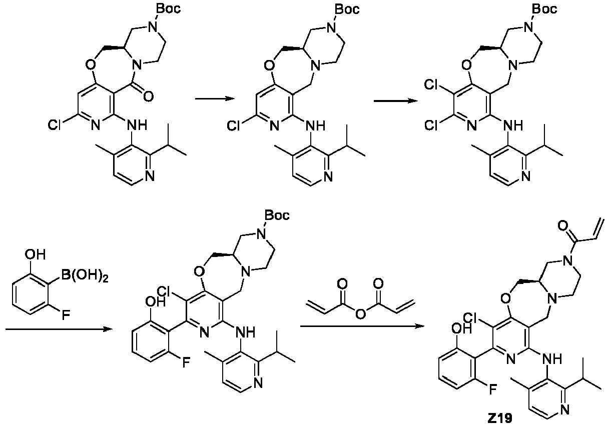

Step 1: Lithium aluminum hydride (1.8 mL, 1.752 mmol, 1 M in THF) was slowly added to a solution of *tert*-butyl (*R*)-3-chloro-1-((2-isopropyl-4-methylpyridin-3-yl)amino)-12-oxo-6a,7,9,10-tetrahydro-12*H*-pyrazino[2,1-*c*]pyrido[3,4-*f*][1,4]oxazepine-8(6*H*)-carboxylate (220 mg, 0.439 mmol) in THF (20 mL) under nitrogen protection at room temperature, and the reaction mixture was heated to 65 °C and stirred for 10 minutes. The completion of reaction was detected by LC-MS. The reaction solution was cooled to room temperature, sodium sulfate decahydrate (170 mg, 0.527 mmol) was added and stirred for 10 minutes. The mixture was filtered, and the filtrate was concentrated to dryness and the crude product was purified by flash silica gel column (MeOH/DCM: 0-100%) to obtain *tert*-butyl (*R*)-3-chloro-1-((2-isopropyl-4-methylpyridin-3-yl)amino)-6a,7,9,10-tetrahydro-12*H*-pyrazino[2,1-*c*]pyrido[3,4-*f*][1,4]oxazepine-8(6*H*)-carboxylate (122 mg, 57%) as an off-white solid. ES-API: [M+H]⁺=488.4.

Step 2: *N*-Chlorosuccinimide (67 mg, 0.501 mmol) was slowly added to a solution of *tert*-butyl (*R*)-3-chloro-1-((2-isopropyl-4-methylpyridin-3-yl)amino)-6a,7,9,10-tetrahydro-12*H*-pyrazino[2,1-*c*]pyrido[3,4-*f*][1,4]oxazepine-8(6*H*)-carboxylate (122 mg, 0.251 mmol) in acetonitrile (15 mL). The reaction mixture was heated to 80°C and stirred for 2.5 hours. The completion of reaction was detected by LC-MS. The reaction solution was cooled to 0°C, added with 20 mL of saturated sodium thiosulfate solution to quench the reaction, and then extracted with ethyl acetate (20 mL*3), and the organic phase was washed with saturated sodium bicarbonate (15 mL) solution, concentrated to dryness. The crude product was purified by flash silica gel column (MeOH/DCM: 0-100%) to obtain *tert*-butyl (*R*)-3,4-dichloro-1-((2-isopropyl-4-methylpyridin-3-yl)amino)-6a,7,9,10-tetrahydro-12*H*-pyrazino[2,1-*c*]pyrido[3,4-*f*][1,4]oxazepine-8(6*H*)-carboxylate (134 mg, 99%) as an off-white solid. ES-API: [M+H]⁺=522.3.

Step 3: Under nitrogen protection, *tert*-butyl (*R*)-3,4-dichloro-1-((2-isopropyl-4-methylpyridin-3-yl)amino)-6a,7,9,10-tetrahydro-12*H*-pyrazino[2,1-*c*]pyrido[3,4-*f*][1,4]oxazepine-8(6*H*)-carboxylate (134 mg, 0.256 mmol), 2-fluoro-6-hydroxyphenylboronic acid (120 mg, 0.769 mmol), potassium phosphate (163 mg, 0.769 mmol), 2-dicyclohexylphosphino-2',6'-dimethoxy-biphenyl (11 mg, 0.0256 mmol), chloro(2-dicyclohexylphosphino-2',6'-dimethoxy-1,1'-biphenyl)(2'-amino-1,1'-biphenyl-2-yl)palladium(II) (18 mg, 0.0256 mmol) were dissolved in 5 mL of dioxane and 1 mL of water. The reaction mixture was heated to 72°C and stirred for 2 hours. The completion of reaction was detected by LC-MS. The reaction solution was cooled to room temperature, concentrated to dryness, and the crude product was purified by flash silica gel column (MeOH/DCM: 0-100%) to obtain *tert*-butyl (6a*R*)-4-chloro-3-(2-fluoro-6-hydroxyphenyl)-1-((2-isopropyl-4-methylpyridin-3-yl)amino)-6a,7,9,10-tetrahydro-12*H*-pyrazino[2,1-*c*]pyrido[3,4-*f*][1,4]oxazepine-8(6*H*)-carboxylate (97 mg, yield of 63%) as a yellow solid. ES-API: [M+H]⁺=598.3.

Step 4: Dioxane hydrochloride solution (3 mL, 4 M) was slowly added to a solution of *tert*-butyl (6a*R*)-4-chloro-3 -(2-fluoro-6-hydroxyphenyl)-1-((2-isopropyl-4-methylpyridin-3-yl)amino)-6a,7,9,10-tetrahydro-12*H*-pyrazino[2,1-*c*]pyrido[3,4-*f*][1,4]oxazepine-8(6*H*)-carboxylate (97 mg, 0.162 mmol) in methanol (3 mL) and stirred at 28°C for 0.5 hours. The completion of reaction was detected by LC-MS. The reaction solution was concentrated to obtain an off-white solid. The solid was dissolved in 5 mL of dichloromethane and triethylamine (1 mL). At 0°C, a solution of acrylic anhydride in dichloromethane (12 mg, 0.097 mmol) was added dropwise thereto. The reaction was stirred at 0°C for 5 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain 1-((6a*R*)-4-chloro-3-(2-fluoro-6-hydroxyphenyl)-1-((2-isopropyl-4-methylpyridin-3-yl)amino)-6a,7,9,10-tetrahydro-12*H*-pyrazino[2,1-*c*]pyrido[3,4-*f*][1,4]oxazepin-8(6*H*)-yl)prop-2-en-1-one (Z19, 32.27 mg, 36%) as a white solid. ¹H NMR (500 MHz, CDCl₃) 8.19 (d, *J*= 5.0 Hz, 1 *H*)*,* 7.68 (s, 1 H), 7.14-7.09 (q, 1 H), 7.04 (d, J= 5.0 Hz, 1 H), 6.90-6.82 (m, 1 H), 6.62 *(d, J=* 8.0 Hz, 1 H), 6.56 (t, J1 = 8.5 Hz, J2 = 8.5 Hz, 1 H), 6.17-6.13 (m, 1 H), 5.74-5.70 (m, 1 H), 4.57-4.52 (m, 1 H), 4.30-4.28 (m, 1 H), 4.13-3.92 (m, 4 H), 3.25-3.16 (m, 2 H), 3.00-2.86 (m, 2 H), 2.65-2.64 (m, 2 H), 2.08 (s, 3 H), 1.15 (s, 3 H), 0.96 (s, 3 H). ES-API: [M+H]⁺=552.2.

### Embodiment 19 Synthesis of (6aR)-8-acryloyl-4-chloro-1-((4,6-diisopropylpyrimidin-5-yl)(methyl)amino)-3-(2-fluoro-6-hydroxyphenyl)-6,6a,7,8,9,10-hexahydro-12H-pyrazino[2,1-c]pyrido[3,4-f][1,4]oxazepin-12-one Z20

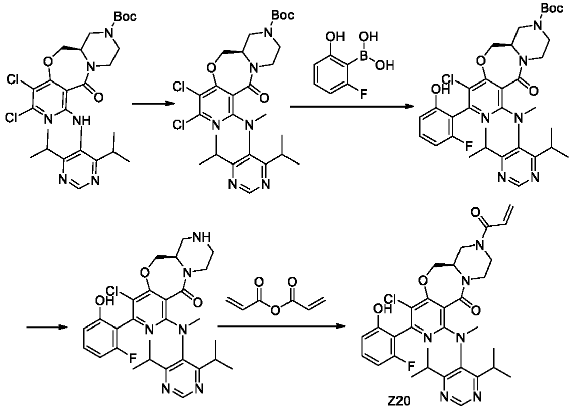

Step 1: *tert*-Butyl (*R*)-3,4-dichloro-1-((4,6-diisopropylpyrimidin-5-yl)amino)-12-oxo-6a,7,9,10-tetrahydro-12*H*-pyrazino[2,1-*c*]pyrido[3,4-*f*][1,4]oxazepine-8(6*H*)-carboxylate (150 mg, 0.26 mmol) was dissolved in THF (5 mL), and cooled to 0°C under an ice bath, stirred for 5 minutes, and then sodium hydride (52 mg, 1.3 mmol, 60%) was added thereto in batches. After the mixture was stirred at 0°C for 10 minutes, iodomethane (369.2 mg, 2.6 mmol) was slowly added dropwise thereto, stirred for 2 minutes and then the ice bath was removed. The mixture was heated to 50°C under an oil bath, and stirred at this temperature for half an hour. After the completion of reaction was detected by LC-MS, the reaction was quenched by adding 10 mL of water under an ice bath, extracted three times with 50 mL of ethyl acetate, and the organic phase was washed once with 20 mL of saturated brine, dried over sodium sulfate, and evaporated to dryness by rotary evaporation to obtain *tert*-butyl (*R*)-3,4-dichloro-1-((4,6-diisopropylpyrimidin-5-yl)(methyl)amino)-12-oxo-6a,7,9,10-tetrahydro-12*H*-pyrazino[2,1-*c*]pyrido[3,4-*f*][1,4]oxazepine-8(6*H*)-carboxylate as a crude product, which was directly used in the next step. ES-API: [M+H]⁺= 579.2.

Step 2: *tert*-Butyl (*R*)-3,4-dichloro-1-((4,6-diisopropylpyrimidin-5-yl)(methyl)amino)-12-oxo-6a,7,9,10-tetrahydro-12*H*-pyrazino[2,1-*c*]pyrido[3,4-*f*][1,4]oxazepine-8(6*H*)-carboxylate (200 mg, 0.34 mmol), 2-fluoro-phenylboronic acid (185 mg, 1.19 mmol), Pd(PPh₃)₄ (40 mg, 0.034 mmol), sodium carbonate (108 mg, 1.02 mmol), 8 mL of dioxane and 2 mL of water were added to a 100 mL reaction flask. The reaction was stirred at 80°C for 2.5 hours under nitrogen protection, and the reaction was stopped. The reaction was added with 20 mL of water, extracted three times with 20 mL of ethyl acetate. The organic phase was dried, concentrated and then purified by flash silica gel column (EtOAc/PE: 0-100%) to obtain *tert*-butyl (6a*R*)-4-chloro-1-((4,6-diisopropylpyrimidin-5-yl)(methyl)amino)-3-(2-fluoro-6-hydroxyphenyl)-12-oxo-6a,7,9,10-tetrahydro-12*H*-pyrazino[2,1-*c*]pyrado[3,4-*f*][1,4]oxazepine-8(6*H*)-carboxylate (173 mg, 78%) as a yellow solid. ES-API: [M+H]⁺= 655.3.

Step 3: *tert*-Butyl (6a*R*)-4-chloro-1-((4,6-diisopropylpyrimidin-5-yl)(methyl)amino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (173 mg, 0.26 mmol), 1 mL of methanol and 3 mL of hydrogen chloride/dioxane solution (4 M) were added to a flask. The mixture was stirred at room temperature for 1 hour, and the completion of reaction was detected by LC-MS. The reaction solution was concentrated to obtain compound (6a*R*)-4-chloro-1-((4,6-diisopropylpyrimidin-5-yl)(methyl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (167 mg) as a yellow solid. The crude product was directly used in the next step. ES-API: [M+H]⁺=555.2.

Step 4: (6a*R*)-4-Chloro-1-((4,6-diisopropylpyrimidin-5-yl)(methyl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (167 mg, 0.30 mmol), 3 mL of dichloromethane and triethylamine (303 mg, 3 mmol) were added to a flask. At 0°C, a solution of acrylic anhydride in dichloromethane (24 mg, 0.195 mmol, 0.5 mL) was added dropwise thereto. The reaction was carried out at 0°C for 10 minutes. 40 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-1-((4,6-diisopropylpyrimidin-5-yl)(methyl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z20, 58 mg, 32%). ES-API: [M+H]⁺=609.2. HNMR (500MHz, DMSO-*d*₆) δ 9.87 (d, *J* = 7.8 Hz, 1H), 8.92 (s, 1H), 7.14 (s, 1H), 6.80 (s, 1H), 6.72-6.49 (m, 2H), 6.19 (d, *J* = 17.0 Hz, 1H), 5.76 (d, J = 10.0 Hz, 1H), 4.77 (s, 1H), 4.55-4.25 (m, 2H), 4.24-4.02 (m, 2H), 4.01-3.76 (m, 1H), 3.74-3.43 (m, 3H), 3.33-3.01 (m, 1H)3.08 (s, 4H), 1.18 (d, *J* = 6.0 Hz, 6H), 1.04 (s, 3H), 0.88(s, 3H).

### Embodiment 20 Synthesis of (6aR)-8-acryloyl-4-chloro-1-(3,3-dimethylpyrrolidin-1-yl)-3-(2-fluoro-6-hydroxyphenyl)-6,6a,7,8,9,10-hexahydro-12H-pyrazino[2,1-c]pyrido[3,4-f][1,4]oxazepin-12-one Z16

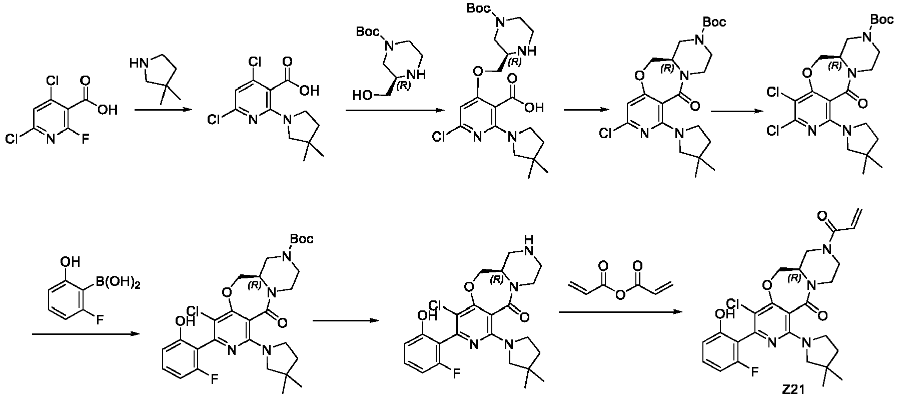

Step 1: 4,6-Dichloro-2-fluoronicotinic acid (1.58 g, 7.52 mmol), *N,N*-diisopropylethylamine (2.4 g, 18.8 mmol) and 3,3-dimethylpyrrolidine hydrochloride (850 mg, 6.27 mmol) were added to acetonitrile (10 mL), heated to 70°C and reacted for 3 hours. The completion of the reaction was detected by thin-layer chromatography plate. The mixture was added with ethyl acetate (50 mL), washed with water and brine, and then dried and evaporated to dryness by rotary evaporation to obtain 4,6-dichloro-2-(3,3-dimethylpyrrolidin-1-yl)nicotinic acid (1.7 g, yield of 78%). ES-API: [M+H]⁺= 289.1.

Step 2: *tert*-Butyl (*R*)-3-(hydroxymethyl)piperazine-1-carboxylate (3.4 g, 15.56 mmol) was dissolved in THF (30 mL), and sodium hydride (1.7 g, 41.49 mmol) was added thereto at a cooled temperature of 0°C. After holding the temperature and reacting for half an hour, a solution of the raw material 4,6-dichloro-2-(3,3-dimethylpyrrolidin-1-yl)nicotinic acid (1.5 g, 5.19 mmol) in THF (10 mL) was added dropwise thereto. After the addition, the mixture was heated to 65°C and reacted overnight. The reaction solution was quenched with ammonium chloride solution, extracted with ethyl acetate, dried and the solvent was evaporated to dryness under reduced pressure to obtain (*R*)-4-((4-(*tert-*butoxycarbonyl)piperazin-2-yl)methoxy)-6-chloro-2-(3,3-dimethylpyrrolidin-1-yl)nicotinic acid (1.2 g, yield of 49%) as a crude product, which was directly used in the next step. ES-API: [M+H]⁺= 469.1.

Step 3: (*R*)-4-((4-(*tert*-Butoxycarbonyl)piperazin-2-yl)methoxy)-6-chloro-2-(3,3-dimethylpyrrolidin-1-yl)nicotinic acid (1.2 g, 2.56 mmol) and *N,N*-diisopropylethylamine (5 mL) were added to dichloromethane (120 mL), and 1-propylphosphonic anhydride (50% ethyl acetate solution, 5 mL) was added slowly dropwise thereto at room temperature. After the addition, the reaction was carried out at room temperature for 1 hour. The reaction solution was quenched by ammonium chloride solution, extracted with dichloromethane, and the organic phase was washed with sodium bicarbonate solution, dried and evaporated to dryness by rotary evaporation, and then purified by column chromatography (PE:EtOAc=3:1) to obtain *tert*-butyl (*R*)-3-chloro-1-(3,3-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (400 mg, yield of 34%) as a yellow foamy solid. ES-API: [M+H]⁺=451.2.

Step 4: *tert*-Butyl (*R*)-3-chloro-1-(3,3-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (270 mg, 0.55 mmol) was dissolved in acetonitrile (3 mL), and 1-chloropyrrolidin-2,5-dione (88 mg, 0.66 mmol) was added thereto. The mixture was heated to 35°C and reacted for 3 hours. The reaction solution was cooled and then added with ethyl acetate (10 mL), washed with water, brine, dried and evaporated to dryness by rotary evaporation, and then purified by column chromatography (PE:EtOAc=3:1) to obtain *tert*-butyl (*R*)-3,4-dichloro-1-(3,3-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (200 mg, yield of 49%) as a yellow foamy solid. ES-API: [M+H]⁺=485.2.

Step 5: *tert-*Butyl (*R*)-3,4-dichloro-1-(3,3-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (205 mg, 0.42 mmol), (2-fluoro-6-hydroxyphenyl)boronic acid (99 mg, 0.63 mmol), sodium carbonate (134 mg, 4.95 mmol), and tetrakis(triphenylphosphine)palladium (20 mg) were added to dioxane (2 mL) and water (0.2 mL) in turn, and the reaction was carried out at 105°C for 16 hours under nitrogen protection. The mixture was cooled to room temperature, and then poured into ethyl acetate (20 mL), washed once with brine and purified by silica gel column (EtOAc:PE=10%-30%) to obtain *tert*-butyl (6a*R*)-4-chloro-1-(3,3-dimethylpyrrolidin-1-yl)-3-(2-fluoro-6-hydroxyphenyl)-12-oxo-6a,7,9,10-tetrahydro-6*H*-pyrazino[2,1-*c*]pyrido[3,4-*f*][1,4]oxazepine-8(12*H*)-carboxylate (30 mg, 12%) as a yellow foamy solid. ES-API: [M+H]⁺=561.2.

Step 6: *tert*-Butyl (6a*R*)-4-chloro-1-(3,3-dimethylpyrrolidin-1-yl)-3-(2-fluoro-6-hydroxyphenyl)-12-oxo-6a,7,9,10-tetrahydro-6*H*-pyrazino[2,1-*c*]pyrido[3,4-*f*][1,4]oxazepine-8(12*H*)-carboxylate (30 mg, 0.053 mmol) was dissolved in dichloromethane (1 mL), added with trifluoroacetic acid (1 mL) and reacted at room temperature for 1 hour, evaporated to dryness by rotary evaporation to obtain (6a*R*)-4-chloro-1-(3,3-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (25 mg, yield of 100%) as a yellow oily crude product. ES-API: [M+H]⁺=461.2.

Step 7: (6a*R*)-4-Chloro-1-(3,3-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (24 mg, 0.053 mmol) and triethylamine (16 mg, 0.16 mmol) were dissolved in dichloromethane (0.5 mL), cooled to 0°C, and then acrylic anhydride (6.7 mg, 0.053 mmol) was added thereto, held at 0°C and reacted for 1 hour. The reaction solution was added with water (10 mL), extracted with dichloromethane (10 mL × 3), dried and then evaporated to dryness by rotary evaporation, and purified by chromatography plate (DCM:MeOH=20:1) to obtain (6a*R*)-8-acryloyl-4-chloro-1-(3,3-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z21, 20 mg, yield of 60%) as a white solid. ¹H NMR (400 MHz, CDCl₃) δ 7.29 (s, 1H), 6.83 (d, *J* = 8.2 Hz, 1H), 6.69 (t, *J* = 8.9 Hz, 1H), 6.57 (s, 1H), 6.42 (d, *J* = 16.1 Hz, 1H), 5.83 (d, *J* = 9.5 Hz, 1H), 4.40 (s, 3H), 4.23 (s, 2H), 4.03 (s, 1H), 3.76 (s, 2H), 3.60-3.50 (m, 2H), 3.40-3.20 (m, 2H), 2.99 (s, 1H), 1.25 (s, 2H), 1.18 (s, 3H), 1.07 (s, 3H). ES-API: [M+H]⁺=515.2.

### Embodiment 21 Synthesis of (6aR)-8-acryloyl-4-chloro-1-(2,2-dimethylpyrrolidin-1-yl)-3-(2-fluoro-6-hydroxyphenyl)-6,6a,7,8,9,10-hexahydro-12H-pyrazino[2,1-c]pyrido[3,4-f][1,4]oxazepin-12-one Z22

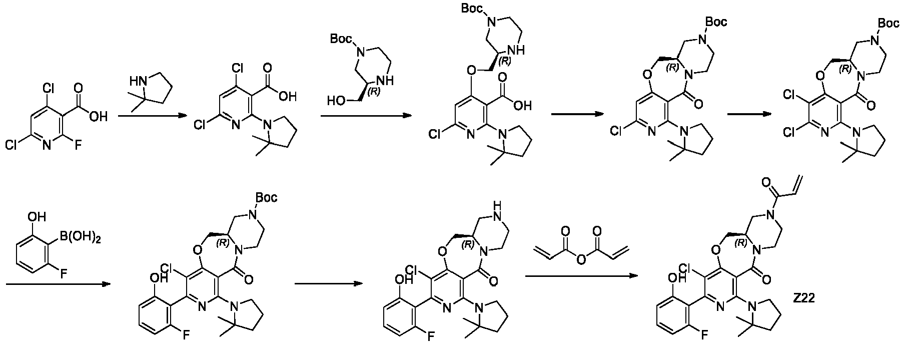

Step 1: 4,6-Dichloro-2-fluoronicotinic acid (2 g, 9.56 mmol), potassium carbonate (3.3 g, 23.89 mmol) and 2,2-dimethylpyrrolidine hydrochloride (1.08 g, 7.96 mmol) were added to DMSO (30 mL), heated to 80°C and reacted for 18 hours. The completion of the reaction was detected by thin-layer chromatography plate. The mixture was added with ethyl acetate (50 mL), washed with water and brine, and then dried and evaporated to dryness by rotary evaporation to obtain 4,6-dichloro-2-(2,2-dimethylpyrrolidin-1-yl)nicotinic acid (2.2 g, yield of 75%). ES-API: [M+H]⁺=289.0.

Step 2: *tert*-Butyl (*R*)-3-(hydroxymethyl)piperazine-1-carboxylate (4.5 g, 20.75 mmol) was dissolved in THF (40 mL), and sodium hydride (2.2 g, 55.33 mmol) was added thereto at a cooled temperature of 0°C. After holding the temperature and reacting for half an hour, the raw material 4,6-dichloro-2-(2,2-dimethylpyrrolidin-1-yl)nicotinic acid (2 g, 6.92 mmol) dissolved in THF (40 mL) was added thereto. After the addition, the mixture was heated to 65°C and reacted overnight. The reaction was quenched with ammonium chloride solution, extracted with ethyl acetate, dried and evaporated to dryness under reduced pressure to obtain (*R*)-4-((4-(*tert*-butoxycarbonyl)piperazin-2-yl)methoxy)-6-chloro-2-(2,2-dimethylpyrrolidin-1-yl)nicotinic acid (2 g, yield of 49%) as a crude product, which was directly used in the next step. ES-API: [M+H]⁺= 469.2.

Step 3: (*R*)-4-((4-(*tert-*Butoxycarbonyl)piperazin-2-yl)methoxy)-6-chloro-2-(2,2-dimethylpyrrolidin-1-yl)nicotinic acid (2 g, 4.26 mmol) and *N,N*-diisopropylethylamine (8 mL) were added to dichloromethane (200 mL), and 1-propylphosphonic anhydride (50% ethyl acetate solution, 8 mL) was added slowly dropwise thereto at room temperature. After the addition, the reaction was carried out at room temperature for 1 hour. The reaction solution was washed with ammonium chloride solution, washed with sodium bicarbonate solution, dried and evaporated to dryness by rotary evaporation, and then purified by column chromatography (PE:EtOAc=3:1) to obtain *tert*-butyl (*R*)-3-chloro-1-(2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (400 mg, yield of 21%) as a yellow foamy solid. ES-API: [M+H]⁺=451.2.

Step 4: *tert*-Butyl (*R*)-3-chloro-1-(2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (420 mg, 0.93 mmol) was dissolved in acetonitrile (5 mL), and 1-chloropyrrolidin-2,5-dione (137 mg, 1.02 mmol) was added thereto. The mixture was heated to 35°C and reacted for 0.5 hours. The reaction solution was cooled and then added with ethyl acetate (10 mL), washed with water and then brine, dried and evaporated to dryness by rotary evaporation, and then purified by column chromatography (PE:EtOAc=3:1) to obtain *tert*-butyl (*R*)-3,4-dichloro-1-(2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (405 mg, yield of 80%) as a yellow foamy solid. ES-API: [M+H]⁺=485.2.

Step 5: *tert-*Butyl (*R*)-3,4-dichloro-1-(2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (403 mg, 0.83 mmol), (2-fluoro-6-hydroxyphenyl)boronic acid (194 mg, 1.25 mmol), sodium carbonate (264 mg, 2.49 mmol), and tetrakis(triphenylphosphine)palladium (40 mg) were added to dioxane (4 mL) and water (0.4 mL) in turn, and the reaction was heated to 105°C and carried out for 18 hours under nitrogen protection. The mixture was cooled to room temperature, and then poured into ethyl acetate (20 mL), washed once with brine, dried and purified by silica gel column (EtOAc:PE=10%-30%) to obtain *tert*-butyl (6a*R*)-4-chloro-1-(2,2-dimethylpyrrolidin-1-yl)-3-(2-fluoro-6-hydroxyphenyl)-12-oxo-6a,7,9,10-tetrahydro-6*H*-pyrazino[2,1-*c*]pyrido[3,4-*f*][1,4]oxazepine-8(12*H*)-carboxylate (200 mg, 43%) as a yellow foamy solid. ES-API: [M+H]⁺=561.2.

Step 6: *tert*-Butyl (6a*R*)-4-chloro-1-(2,2-dimethylpyrrolidin-1-yl)-3-(2-fluoro-6-hydroxyphenyl)-12-oxo-6a,7,9,10-tetrahydro-6*H*-pyrazino[2,1-*c*]pyrido[3,4-*f*][1,4]oxazepine-8(12*H*)-carboxylate (220 mg, 0.48 mmol) was dissolved in dichloromethane (2 mL), added with trifluoroacetic acid (2 mL) and reacted at room temperature for 1 hour, evaporated to dryness by rotary evaporation to obtain (6a*R*)-4-chloro-1-(2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (220 mg, yield of 100%) as a yellow oily crude product. ES-API: [M+H]⁺=461.2.

Step 7: (6a*R*)-4-Chloro-1-(2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (221 mg, 0.48 mmol) and triethylamine (146 mg, 1.44 mmol) were dissolved in dichloromethane (2 mL), cooled to 0°C, and then acrylic anhydride (60 mg, 0.48 mmol) was added dropwise thereto, held at 0°C and reacted for 1 hour. The reaction solution was added with water (10 mL), extracted with dichloromethane (10 mL × 3), dried and then evaporated to dryness by rotary evaporation, and purified by chromatography plate (DCM:MeOH=20:1) to obtain (6a*R*)-8-acryloyl-4-chloro-1-(2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (*Z*22, 40 mg, yield of 32%) as a white solid. ¹H NMR (400 MHz, CDCl₃) δ 7.30-7.25 (m, 1H), 6.82 (d, *J* = 8.3 Hz, 1H), 6.70 (t, *J* = 8.9 Hz, 1H), 6.65-6.48 (m, 1H), 6.45-6.35 (m, 1H), 5.81 (d, *J* = 10.3 Hz, 1H), 4.55-4.35 (m, 3H), 4.20-4.10 (m 2H), 4.06-3.98 (m, 1H), 3.80-3.30 (m, 4H), 2.93-2.78 (s, 1H), 2.0-1.75 (m, 4H), 1.64-1.40 (m, 6H). ES-API: [M+H]⁺=515.2.

### Embodiment 22 Synthesis of (6aR)-8-acryloyl-4-chloro-3-(2-fluoro-6-hydroxyphenyl)-1-((S)-2-methylmorpholino)-6,6a,7,8,9,10-hexahydro-12H-pyrazino[2,1-c]pyrido[3,4-f][1,4]oxazepin-12-one Z23

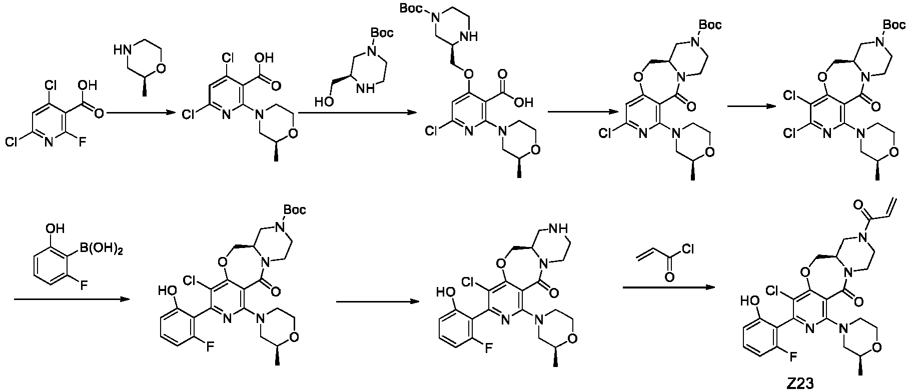

Step 1: 4,6-Dichloro-2-fluoronicotinic acid (500 mg, 2.38 mmol), (S)-2-methylmorpholine (361 mg, 3.57 mmol), and a solution of *N,N-*diisopropylethylamine (920 mg, 7.14 mmol) in acetonitrile (10 mL) were stirred at 70°C for 30 minutes. The reaction solution was poured into 30 mL of ethyl acetate, washed with 30 mL of 1 M dilute hydrochloric acid solution, and the resulting organic phase was dried and concentrated to obtain (*S*)-4,6-dichloro-2-(2-methylmorpholino)nicotinic acid (750 mg, crude product) as a yellow oil. ES-API: [M+H]⁺=291.1.

Step 2: *tert*-Butyl (*R*)-3-(hydroxymethyl)piperazine-1-carboxylate (1.11 g, 5.15 mmol) was added to a turbid solution of sodium hydride (515 mg, 12.88 mmol) in THF (10 mL) under an ice bath, and stirred for 10 minutes. Then the above (*S*)-4,6-dichloro-2-(2-methylmorpholino)nicotinic acid (750 mg, crude product) was added thereto, heated to 60°C and stirred for 1 hour. After the reaction was completed, the reaction solution was quenched with 10 mL of saturated ammonium chloride solution, extracted with 10 mL of ethyl acetate for three times, and the organic phases were combined, dried and concentrated to obtain 4-(((*R*)-4-(*tert-*butoxycarbonyl)piperazin-2-yl)methoxy)-6-chloro-2-((*S*)-2-methylmorpholino)nicotinic acid (1.2 g, crude product). ES-API: [M+H]⁺=471.3.

Step 3: 4-(((*R*)-4-(*tert-*Butoxycarbonyl)piperazin-2-yl)methoxy)-6-chloro-2-((*S*)-2-methylmorpholino)nicotinic acid (1.2 g, crude product) obtained above was dissolved in dichloromethane (20 mL), and *N,N*-diisopropylethylamine (4.2 mL) was added thereto, and then a solution of 1-propylphosphonic anhydride in ethyl acetate (4.2 mL) was added thereto. The mixture was stirred at room temperature for 10 minutes. The reaction solution was poured into 50 mL of dichloromethane, and washed three times with 50 mL of sodium bicarbonate solution. The organic phase was dried, concentrated and then purified by flash silica gel column (EtOAc/PE: 0-70%) to obtain *tert*-butyl (*R*)-3-chloro-1-((*S*)-2-methylmorpholino)-12-oxo-6a,7,9,10-tetrahydro-12*H*-pyrazino[2,1-*c*]pyrido[3,4-*f*][1,4]oxapezine-8(6*H*)-carboxylate (530 mg, 3-step yield of 49%). ES-API: [M+H]⁺=453.3.

Step 4: A solution of *tert*-butyl (*R*)-3-chloro-1-((*S*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (480 mg, 1.06 mmol) in acetonitrile (5 mL) was added with *N*-chlorosuccinimide (212 mg, 1.59 mmol) and stirred at 55°C overnight. The reaction solution was concentrated and purified by flash silica gel column (EtOAc/PE: 0-70%) to obtain *tert*-butyl (*R*)-3,4-dichloro-1-((*S*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (350 mg, 67%). ES-API: [M+H]⁺=487.1.

Step 5: Under nitrogen protection, a mixed solution of *tert*-butyl (*R*)-3,4-dichloro-1-((*S*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (350 mg, 0.72 mmol), 2-fluoro-6-hydroxyphenylboronic acid (336 mg, 2.15 mmol), tetrakis(triphenylphosphine)palladium (83 mg, 0.07 mmol) and potassium carbonate (298 mg, 2.15 mmol) in 1,4-dioxane (5 mL) and water (1 mL) was reacted at 110°C for 30 minutes under microwave irradiation. The reaction solution was added to 30 mL of water and extracted three times with 30 mL of ethyl acetate. The organic phases were combined, dried, concentrated and purified by flash silica gel column (EtOAc/PE: 0-80%) to obtain *tert*-butyl (6a*R*)-4-chloro-3-(2-fluoro-6-hydroxyphenyl)-1-((*S*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (330 mg, 82%). ES-API: [M+H]⁺=563.2.

Step 6: Under an ice bath, trifluoroacetic acid (2 mL) was added to a solution of *tert*-butyl (6a*R*)-4-chloro-3-(2-fluoro-6-hydroxyphenyl)-1-((*S*)-2-methylmorpholino)-12-oxo-6a,7,9,10-tetrahydro-12*H-*pyrazino[2,1-*c*]pyrido[3,4-*f*][1,4]oxazepine-8(6*H*)-carboxylate (330 mg) in dichloromethane (8 mL), warmed to room temperature and stirred for 1 hour. The reaction solution was concentrated to obtain a crude product of (6a*R*)-4-chloro-3-(2-fluoro-6-hydroxyphenyl)-1-((*S*)-2-methylmorpholino)-6,6a,7,8,9,10-hexahydro-12*H*-pyrazino[2,1-*c*]pyrido[3,4-*f*][1,4]oxazepin-12-one as a yellow oil, which was directly used in the next reaction step. ES-API: [M+H]⁺=463.1.

Step 7: Under an ice bath, saturated potassium carbonate solution (2 mL) was added to a solution of the above crude product of (6a*R*)-4-chloro-3-(2-fluoro-6-hydroxyphenyl)-1-((*S*)-2-methylmorpholino)-6,6a,7,8,9,10-hexahydro-12*H*-pyrazino[2,1-*c*]pyrido[3,4-*f*][1,4]oxazepin-12-one in THF (4 mL), and then acryloyl chloride (55 mg) was added thereto and stirred for 10 minutes. The reaction solution was dissolved in 10 mL of water, extracted with 10 mL of ethyl acetate, and the organic phase was dried, concentrated and then purified by preparative HPLC to obtain an isomer of (6a*R*)-8-acryloyl-4-chloro-3-(2-fluoro-6-hydroxyphenyl)-1-((*S*)-2-methylmorpholino)-6,6a,7,8,9,10-hexahydro-12*H*-pyrazino[2,1-*c*]pyrido[3,4-*f*][1,4]oxazepin-12-one (Z23, 155 mg, 2-step yield of 51%) as a yellow solid. ¹H NMR (500 MHz, DMSO-*d*₆) δ 9.99 (s, 1H), 7.29-7.20 (m, 1H), 6.82-6.65 (m, 3H), 6.17 (d, *J* = 16.5 Hz, 1H), 5.81-5.69 (m, 1H), 4.51-4.28 (m, 2H), 4.28-4.14 (m, 1H), 4.14-3.86 (m, 4H), 3.85-3.63 (m, 2H), 3.61-3.51 (m, 2H), 3.51-3.39 (m, 3H), 2.95 (t, *J* = 11.5 Hz, 1H), 2.66-2.55 (m, 1H), 1.06 (d, *J* = 6.1 Hz, 3H). ES-API: [M+H]⁺=517.1.

### Embodiment 23 Synthesis of (6aR)-8-acryloyl-4-chloro-3-(2-fluoro-6-hydroxyphenyl)-1-((R)-2-methylmorpholino)-6,6a,7,8,9,10-hexahydro-12H-pyrazino[2,1-c]pyrido[3,4-f][1,4]oxazepin-12-one Z24

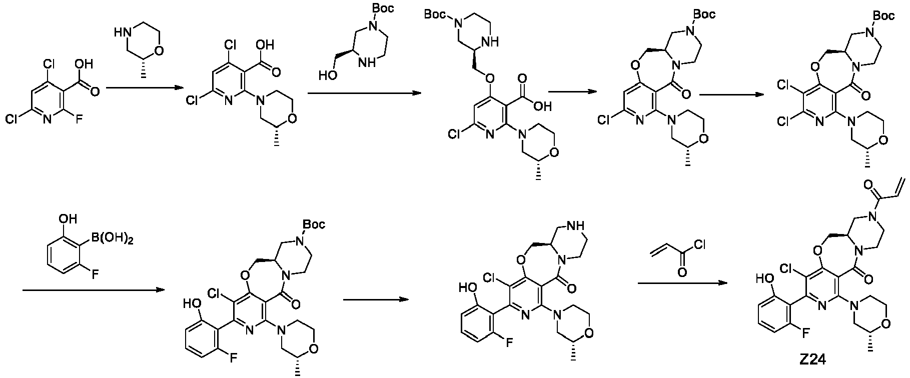

Step 1: 4,6-Dichloro-2-fluoronicotinic acid (500 mg, 2.38 mmol), (R)-2-methylmorpholine (361 mg, 3.57 mmol), and a solution of *N,N-*diisopropylethylamine (920 mg, 7.14 mmol) in acetonitrile (10 mL) were stirred at 70°C for 30 minutes. The reaction solution was poured into 30 mL of ethyl acetate, washed with 30 mL of 1 M dilute hydrochloric acid solution, and the resulting organic phase was dried and concentrated to obtain (*R*)-4,6-dichloro-2-(2-methylmorpholino)nicotinic acid (750 mg, crude product) as a yellow oil. ES-API: [M+H]⁺=291.1.

Step 2: *tert*-Butyl (*R*)-3-(hydroxymethyl)piperazine-1-carboxylate (1.11 g, 5.15 mmol) was added to a turbid solution of sodium hydride (515 mg, 12.88 mmol) in THF (10 mL) under an ice bath, and stirred for 10 minutes. Then the above (*R*)-4,6-dichloro-2-(2-methylmorpholino)nicotinic acid (750 mg, crude product) was added thereto, heated to 60°C and stirred for 1 hour. After the reaction was completed, the reaction solution was quenched with 10 mL of saturated ammonium chloride solution, extracted with 10 mL of ethyl acetate for three times, and the organic phases were combined, dried and concentrated to obtain a crude product of 4-(((*R*)-4-(*tert*-butoxycarbonyl)piperazin-2-yl)methoxy)-6-chloro-2-((*R*)-2-methylmorpholino)nicotinic acid (1.2 g). ES-API: [M+H]⁺=471.3.

Step 3: The crude product of 4-(((*R*)-4-(*tert*-butoxycarbonyl)piperazin-2-yl)methoxy)-6-chloro-2-((*R*)-2-methylmorpholino)nicotinic acid (1.2 g) obtained above was dissolved in dichloromethane (20 mL), and *N,N-*diisopropylethylamine (4.2 mL) was added thereto, and then a solution of 1-propylphosphonic anhydride in ethyl acetate (4.2 mL) was added thereto. The mixture was stirred at room temperature for 10 minutes. The reaction solution was poured into 50 mL of dichloromethane, and washed three times with 50 mL of sodium bicarbonate solution. The organic phase was dried, concentrated and then purified by flash silica gel column (EtOAc/PE: 0-70%) to obtain *tert*-butyl (*R*)-3-chloro-1-((*R*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (120 mg, 3-step yield of 11%). ES-API: [M+H]⁺=453.3.

Step 4: A solution of *tert*-butyl (*R*)-3-chloro-1-((*R*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (120 mg, 0.26 mmol) in acetonitrile (5 mL) was added with *N*-chlorosuccinimide (53 mg, 0.40 mmol) and stirred at 55°C overnight. The reaction solution was concentrated and purified by flash silica gel column (EtOAc/PE: 0-70%) to obtain *tert-*butyl (*R*)-3,4-dichloro-1-((*R*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (120 mg, 93%). ES-API: [M+H]⁺=487.1.

Step 5: Under nitrogen protection, a mixed solution of *tert*-butyl (*R*)-3,4-dichloro-1-((*R*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (120 mg, 0.72 mmol), 2-fluoro-6-hydroxyphenylboronic acid (115 mg, 0.74 mmol), tetrakis(triphenylphosphine)palladium (28 mg, 0.02 mmol) and potassium carbonate (102 mg, 0.74 mmol) in 1,4-dioxane (2 mL) and water (0.4 mL) was reacted at 110°C for 30 minutes under microwave irradiation. The reaction solution was added to 30 mL of water and extracted three times with 30 mL of ethyl acetate. The organic phases were combined, dried, concentrated and purified by flash silica gel column (EtOAc/PE: 0-80%) to obtain *tert*-butyl (6a*R*)-4-chloro-3-(2-fluoro-6-hydroxyphenyl)-1-((*R*)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (130 mg, 94%). ES-API: [M+H]⁺=563.2.

Step 6: Under an ice bath, trifluoroacetic acid (2 mL) was added to a solution of *tert-*butyl (6a*R*)-4-chloro-3-(2-fluoro-6-hydroxyphenyl)-1-((*R*)-2-methylmorpholino)-12-oxo-6a,7,9,10-tetrahydro-12*H-*pyrazino[2,1-*c*]pyrido[3,4-*f*][1,4]oxazepine-8(6*H*)-carboxylate (130 mg) in dichloromethane (8 mL), warmed to room temperature and stirred for 1 hour. The reaction solution was concentrated to obtain a crude product of (6a*R*)-4-chloro-3-(2-fluoro-6-hydroxyphenyl)-1-((R)-2-methylmorpholino)-6,6a,7,8,9,10-hexahydro-12*H*-pyrazino[2,1-*c*]pyrido[3,4-*f*][1,4]oxazepin-12-one as a yellow oil, which was directly used in the next reaction step. ES-API: [M+H]⁺=463.1.

Step 7: Under an ice bath, saturated potassium carbonate solution (2 mL) was added to a solution of the above crude product of (6a*R*)-4-chloro-3-(2-fluoro-6-hydroxyphenyl)-1-((*R*)-2-methylmorpholino)-6,6a,7,8,9,10-hexahydro-12*H*-pyrazino[2,1-*c*]pyrido[3,4-*f*][1,4]oxazepin-12-one in THF (4 mL), and then acryloyl chloride (21 mg) was added thereto and stirred for 10 minutes. The reaction solution was dissolved in 10 mL of water, extracted with 10 mL of ethyl acetate, and the organic phase was dried, concentrated and then purified by preparative HPLC to obtain (6a*R*)- 8-acryloyl-4-chloro-3-(2-fluoro-6-hydroxyphenyl)-1-((*R*)-2-methylmorpholino)-6,6a,7,8,9,10-hexahydro-12*H*-pyrazino[2,1-*c*]pyrido[3,4-*f*][1,4]oxazepin-12-one (Z24, 36 mg, 30%) as a yellow solid. ¹H NMR (500 MHz, DMSO-*d*₆) δ 9.99 (s, 1H), 7.31-7.21 (m, 1H), 6.81-6.65 (m, 3H), 6.17 (d, *J* = 17.0 Hz, 1H), 5.79-5.68 (m, 1H), 4.53-4.27 (m, 2H), 4.27-3.94 (m, 4H), 3.91-3.73 (m, 3H), 3.66-3.45 (m, 5H), 2.97-2.87 (m, 1H), 2.68-2.56 (m, 1H), 1.12-1.01 (m, 3H). ES-API: [M+H]⁺=517.2.

### Embodiment 24 Synthesis of (6aR)-8-acryloyl-4-chloro-3-(2-fluoro-6-hydroxyphenyl)-1-(methyl(tetrahydro-2H-pyran-4-yl)amino)-6,6a,7,8,9,10-hexahydro-12H-pyrazino[2,1-c]pyrido[3,4-f][1,4]oxazepin-12-one Z25

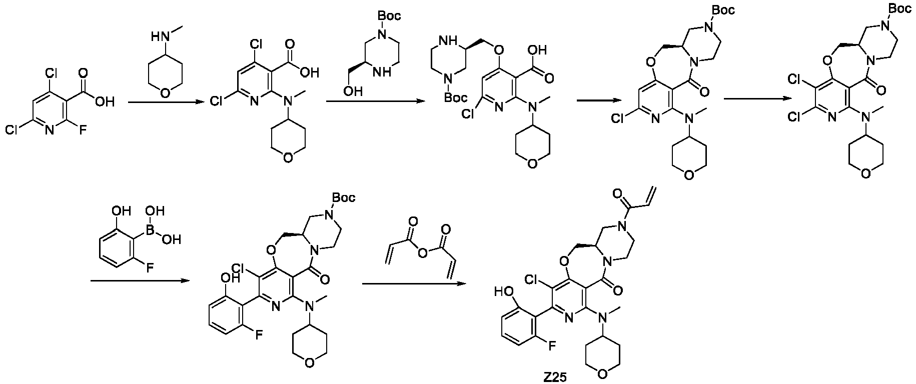

Step 1: 4,6-Dichloro-2-fluoronicotinic acid (600 mg, 2.86 mmol), *N*-methyltetrahydro-2*H*-pyran-4-amine (329 mg, 2.86 mmol), *N,N*-diisopropylethylamine (738 mg, 5.72 mmol) and 10 mL of acetonitrile were added to a 100 mL flask. The reaction was stirred at 85°C for 4 hours. The reaction was quenched with water and extracted with dichloromethane. The organic phase was dried and concentrated to obtain 4,6-dichloro-2-(methyl(tetrahydro-2*H*-pyran-4-yl)amino)nicotinic acid (800 mg, crude product) as a yellow solid. ES-API: [M+H]⁺=305.0.

Step 2: 60% sodium hydride (524 mg, 13.1 mmol), *tert*-butyl (*R*)-3-(hydroxymethyl)piperazine-1-carboxylate (850 mg, 3.93 mmol) and THF (10 mL) were added to a 100 mL flask. A solution of 4,6-dichloro-2-(methyl(tetrahydro-2*H*-pyran-4-yl)amino)nicotinic acid (800 mg, crude product) in THF was added dropwise thereto at 0°C. The reaction was carried out in an oil bath at 60°C for 1 hour. The completion of reaction was detected by LC-MS. The reaction solution was poured into ice-water. The pH of the reaction solution was adjusted to 7 with 2 M hydrochloric acid. The mixture was extracted three times with DCM/MeOH (v/v 10:1). The organic phase was dried and concentrated to obtain (*R*)-4-((4-(tert-butoxycarbonyl)piperazin-2-yl)methoxy)-6-chloro-2-(methyl(tetrahydro-2*H*-pyran-4-yl)amino)nicotinic acid (1 g, 78%) as a yellow solid. ES-API: [M+H]⁺=485.1.

Step 3: (*R*)-4-((4-(*tert*-Butoxycarbonyl)piperazin-2-yl)methoxy)-6-chloro-2-(methyl(tetrahydro-2*H*-pyran-4-yl)amino)nicotinic acid (1 g, 2.06 mmol), *N,N*-diisopropylethylamine (3.5 mL) and dichloromethane (20 mL) were added to a 100 mL flask. Propylphosphonic anhydride solution (3.5 mL, 50%w/w ethyl acetate solution) was added dropwise thereto. The mixture was reacted at room temperature for 1 hour. The completion of reaction was detected by LC-MS. 50 mL of dichloromethane was added to the reaction, then washed with 30 mL of water, and 30 mL of saturated aqueous sodium bicarbonate solution, respectively. The organic phase was dried, concentrated and then purified by flash silica gel column (EtOAc/PE: 10-100%) to obtain *tert*-butyl (*R*)-3-chloro-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (530 mg, 55%) as a yellow solid. ES-API: [M+H]⁺=467.2.

Step 4: *tert*-Butyl (*R*)-3-chloro-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (500 mg, 1.07 mmol), *N-*chlorosuccinimide (286 mg, 2.14 mmol) and acetonitrile (15 mL) were added to a 100 mL flask. The reaction was stirred at 70°C for 3 hours. Aqueous sodium thiosulfate solution was added to the reaction solution and the mixture was extracted with ethyl acetate. The organic phase was dried, concentrated and then purified by flash silica gel column (EtOAc/PE: 10-65%) to obtain *tert*-butyl (*R*)-3,4-dichloro-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (130 mg, 24%). ES-API: [M+H]⁺=501.3.

Step 5: *tert*-Butyl (*R*)-3,4-dichloro-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (130 mg, 0.26 mmol), 2-fluoro-6-hydroxyphenylboronic acid (121 mg, 0.78 mmol), Pd(PPh₃)₄ (30 mg, 0.026 mmol), sodium carbonate (83 mg, 0.78 mmol), 6 mL of dioxane and 1.5 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried, concentrated and then purified by flash silica gel column (EtOAc/PE: 10-70%) to obtain *tert*-butyl (6a*R*)-4-chloro-3-(2-fluoro-6-hydroxyphenyl)-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (130 mg, yield of 86%) as a yellow solid. ES-API: [M+H]⁺=577.2.

Step 6: *tert*-Butyl (6a*R*)-4-chloro-3-(2-fluoro-6-hydroxyphenyl)-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (130 mg, 0.22 mmol), methanol (2 mL) and 4 M hydrogen chloride/dioxane solution (5 mL) were added to a flask. After stirring at room temperature for 1 hour, the reaction solution was concentrated to obtain a yellow solid. The yellow solid was dissolved in 5 mL of dichloromethane and triethylamine (111 mg, 1.1 mmol). At 0°C, a solution of acrylic anhydride in dichloromethane (22 mg, 0.18 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-3-(2-fluoro-6-hydroxyphenyl)-1-(methyl(tetrahydro-2*H*-pyran-4-yl)amino)-6,6a,7,8,9,10-hexahydro-12*H*-pyrazino[2,1-*c*]pyrido[3,4-*f*][1,4]oxazepin-12-one (Z25, 35 mg, yield of 30%) as a white solid. HNMR (500 MHz, CDCl₃) 7.32-7.26 (m, 1H), 6.84-6.83 (m, 1H), 6.71-6.69 (m, 1H), 6.57-6.41 (m, 1H), 6.44-6.41 (m, 1H), 5.83 (d, J=10.5 Hz, 1H), 4.50-4.12 (m, 9H), 3.51-3.44 (m, 3H), 3.42-3.39 (m, 2H), 2.80 (s, 3H), 2.03-1.90 (m, 2H), 1.72-1.65 (m, 2H). ES-API: [M+H]⁺=531.2.

### Embodiment 25 Synthesis of (6aR)-8-acryloyl-4-chloro-1-(1,1-dioxidothiomorpholino)-3-(2-fluoro-6-hydroxyphenyl)-6,6a,7,8,9,10-hexahydro-12H-pyrazino[2,1-c]pyrido[3,4-f][1,4]oxazepin-12-one Z26

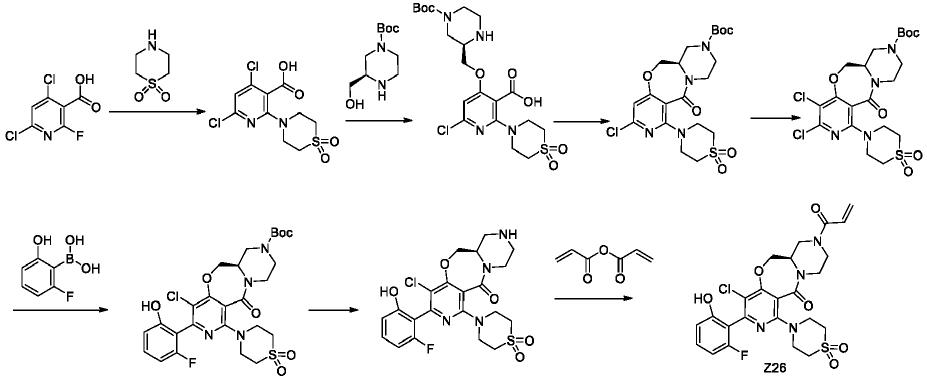

Step 1: 4,6-Dichloro-2-fluoronicotinic acid (1.0 g, 4.8 mmol) and 50 mL of acetonitrile were added to a flask. Diisopropylethylamine (1.2 g, 9.6 mmol) and thiomorpholine-1,1-dioxide (1.3 g, 9.6 mmol) were then added to the reaction solution. The reaction was stirred at 90°C overnight. After the reaction was completed, the pH of the organic phase was adjusted to 5 with 1M hydrochloric acid, and then washed with saturated brine, and extracted with ethyl acetate. The organic phase was dried over anhydrous sodium sulfate, filtered, and the filtrate was concentrated to dryness to obtain 4,6-dichloro-2-(1,1-dioxidothiomorpholino)nicotinic acid (1.2 g, crude product) as a yellow solid, and the crude product was directly subjected to the next reaction step. ES-API: [M+H]⁺=325.0.

Step 2: *tert-*Butyl (*R*)-3-(hydroxymethyl)piperazine-1-carboxylate (466 mg, 3.7 mmol) was added to a suspension of 60% sodium hydride (592 mg, 14.8 mmol) in THF (50 mL) at 0°C. Then, a solution of 4,6-dichloro-2-(1,1-dioxidothiomorpholino)nicotinic acid (1.2 g, 3.7 mmol) in THF (20 mL) was added dropwise thereto. The reaction was carried out at 0°C for 30 minutes. The completion of reaction was detected by LC-MS. The reaction solution was poured into 50 mL of ice-water. The pH of the reaction solution was adjusted to 5 with 2M dilute hydrochloric acid solution, and the reaction solution was extracted with ethyl acetate for three times. The organic phase was dried, concentrated and then purified by flash silica gel column (EtOAc/PE: 20-100%) to obtain (*R*)-4-((4-(*tert*-butoxycarbonyl)piperazin-2-yl)methoxy)-6-chloro-2-(1,1-dioxidothiomorpholino)nicotinic acid (932 mg, 50%) as a yellow solid. ES-API: [M+H]⁺=505.1.

Step 3: (*R*)-4-((4-(*tert*-Butoxycarbonyl)piperazin-2-yl)methoxy)-6-chloro-2-(1,1-dioxidothiomorpholino)nicotinic acid (932 mg, 1.85 mmol), diisopropylethylamine (10 mL) and dichloromethane (30 mL) were added to a flask. Propylphosphonic anhydride solution (10 mL, 50%w/w ethyl acetate solution) was added dropwise thereto. The reaction was carried out at room temperature for 30 minutes. The completion of reaction was detected by LC-MS. 50 mL of dichloromethane was added to the reaction, and the organic phase was washed with 30 mL of hydrochloric acid (1 M) and 100 mL of saturated aqueous sodium bicarbonate solution in turn. The organic phase was dried, concentrated and then purified by flash silica gel column (MeOH/DCM: 0-3%) to obtain *tert*-butyl (*R*)-3-chloro-1-(1,1-dioxidothio)-12-oxo-6a,7,9,10-tetrahydro-12*H*-pyrazino[2,1-*c*]pyrido[3,4-*f*][1,4]oxazepine-8(6*H*)-carboxylate (583 mg, 65%). ES-API: [M+H]⁺=487.1.

Step 4: *tert-*Butyl (*R*)-3-chloro-1-(1,1-dioxidothio)-12-oxo-6a,7,9,10-tetrahydro-12*H-*pyrazino[2,1-*c*]pyrido[3,4-*f*][1,4]oxazepine-8(6*H*)-carboxylate (583 mg, 1.2 mmol), *N*-chlorosuccinimide (321 mg, 2.4 mmol) and acetonitrile (50 mL) were added to a flask. The reaction was stirred at 70°C for 1 hour. The reaction solution was added with 50 mL of water and extracted with ethyl acetate. The organic phase was dried, concentrated and then purified by flash silica gel column (MeOH/DCM: 0-3%) to obtain *tert-*butyl (*R*)-3,4-dichloro-1-(1,1-dioxidothiomorpholino)-12-oxo-6a,7,9,10-tetrahydro-12*H*-pyrazino[2,1-*c*]pyrido[3,4-*f*][1,4]oxazepine-8(6*H*)-carboxylate (380 mg, 61%) as a yellow solid. ES-API: [M+H]⁺=521.1.

Step 5: *tert*-Butyl (*R*)-3,4-dichloro-1-(1,1-dioxidothiomorpholino)-12-oxo-6a,7,9,10-tetrahydro-12*H*-pyrazino[2,1-*c*]pyrido[3,4-*f*][1,4]oxazepine-8(6*H*)-carboxylate (380 mg, 0.73 mmol), 2-fluoro-phenylboronic acid (327 mg, 2.1 mmol), tetrakis(triphenylphosphine)palladium (84 mg, 0.073 mmol), sodium carbonate (223 mg, 2.1 mmol), 15 mL of dioxane and 3 mL of water were added to a 100 mL reaction flask. The reaction was stirred at 80°C for 2.5 hours under nitrogen protection, and the reaction was stopped. The reaction was added with 20 mL of water, extracted three times with 20 mL of ethyl acetate. The organic phase was dried, concentrated and then purified by flash silica gel column (EtOAc/PE: 0-100%) to obtain *tert*-butyl (6a*R*)-4-chloro-1-(1,1-dioxidothiomorpholino)-3-(2-fluoro-6-hydroxyphenyl)-12-oxo-6a,7,9,10-tetrahydro-12*H*-pyrazinol[2,1-*c*]pyrido[3,4-*f*][1,4]oxazepine-8(6*H*)-carboxylate (356 mg, 82%) as a yellow solid. ES-API: [M+H]⁺=597.1.

Step 6: *tert*-Butyl (6a*R*)-4-chloro-1-(1,1-dioxidothiomorpholino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (356 mg, 0.60 mmol), 3 mL of methanol and 6 mL of hydrogen chloride/dioxane solution (4 M) were added to a flask. The mixture was stirred at room temperature for 1 hour, and the completion of reaction was detected by LC-MS. The reaction solution was concentrated to obtain (6a*R*)-4-chloro-1-(1,1-dioxidothiomorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (390 mg) as a yellow solid. The crude product was directly used in the next step. ES-API: [M+H]⁺=497.1.

Step 7: (6a*R*)-4-Chloro-1-(1,1-dioxidothiomorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (390 mg, 0.60 mmol), 3 mL of dichloromethane and triethylamine (627 mg, 6.0 mmol) were added to a flask. At 0°C, a solution of acrylic anhydride in dichloromethane (50 mg, 0.4 mmol, 0.5 mL) was added dropwise thereto. The reaction was carried out at 0°C for 10 minutes. 40 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-1-(1,1-dioxidothiomorpholino)-3-(2-fluoro-6-hydroxyphenyl)-6,6a,7,8,9,10-hexahydro-12*H-*pyrazino[2,1-*c*]pyrido[3,4-*f*][1,4]oxazepin-12-one (*Z*26, 83 mg, 25%). ES-API: [M+H]⁺=551.1. ¹H NMR (500 MHz, DMSO-*d*₆) δ 10.05 (d, *J* = 9.5 Hz, 1H), 7.40-7.07 (m, 1H), 6.82-6.64 (m, 3H), 6.18 (d, *J* = 16.5 Hz, 1H), 5.80-5.68 (m, 1H), 4.63-4.48 (m, 1H), 4.46-4.32 (m, 1H), 4.28-4.14 (m, 1H), 4.13-3.93(m, 3H), 3.87-3.68 (m, 4H), 3.60-3.42 (m, 3H), 3.24-3.09 (m, 4H).

### Embodiment 26 Synthesis of (6aR)-8-acryloyl-4-chloro-3-(2-fluoro-6-hydroxyphenyl)-1-((R)-3-methylmorpholino)-6,6a,7,8,9,10-hexahydro-12H-pyrazino[2,1-c]pyrido[3,4-f][1,4]oxazepin-12-one Z27

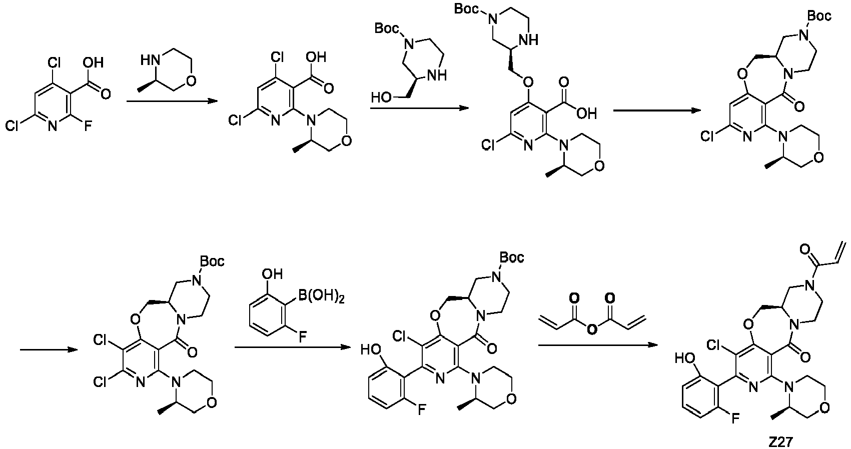

Step 1: (*R*)-3-Methylmorpholine (606 mg, 6.00 mmol), 4,6-dichloro-2-fluoronicotinic acid (630 mg, 3.00 mmol), DIEA (1.16 g, 9.00 mmol) and 20 mL of acetonitrile were added to a 100 mL flask. The reaction was stirred at 80 °C for 3 hours. After the reaction was completed, the reaction solution was evaporated to dryness by rotary evaporation and subjected to column chromatography to obtain (*R*)-4,6-dichloro-2-(3-methylmorpholino)nicotinic acid (1 g, 61%). ES-API: [M+H]⁺=291.1.

Step 2: 60% sodium hydride (1.06 g, 26.5 mmol), *tert*-butyl (*R*)-3-(hydroxymethyl)piperazine-1-carboxylate (1.14 g, 5.27 mmol) and THF (8 mL) were added to a 100 mL flask. A THF suspension (4 mL) of 4,6-dichloro-2-((2-isopropyl-4-methylpyridin-3-yl)amino)nicotinic acid (1 g, 3.70 mmol) was added dropwise thereto at 0°C. The reaction was stirred in an oil bath at 80°C for 2 hours. The completion of reaction was detected by LC-MS. 30 mL of saturated aqueous NH₄Cl solution was poured into the reaction solution. The mixture was extracted 4 times with 40 mL of ethyl acetate. The organic phase was dried, and then concentrated and subjected to column chromatography to obtain 4-(((*R*)-4-(*tert-*butoxycarbonyl)piperazin-2-yl)methoxy)-6-chloro-2-((*R*)-3-methylmorpholino)nicotinic acid (720 mg, 41%) as a yellow solid. ES-API: [M+H]⁺=470.8.

Step 3: 4-(((*R*)-4-(*tert*-Butoxycarbonyl)piperazin-2-yl)methoxy)-6-chloro-2-((*R*)-3-methylmorpholino)nicotinic acid (700 mg, 1.49 mmol), diisopropylethylamine (4 mL) and dichloromethane (15 mL) were added to a 100 mL flask. Propylphosphonic anhydride solution (4 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of aqueous hydrochloric acid solution (1 M), and 30 mL of sodium bicarbonate, respectively. The organic phase was dried, and then concentrated and subjected to column chromatography to obtain *tert*-butyl (*R*)-3-chloro-1-((*R*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (360 mg, 53%) as a yellow solid. ES-API: [M+H]⁺=453.3.

Step 4: *tert*-Butyl (*R*)-3-chloro-1-((*R*)-3-methylmorpholino)-12-oxo-6a,7,9,10-tetrahydro-12*H-*pyrazino[2,1-*c*]pyrido[3,4-*f*][1,4]oxazepine-8(6*H*)-carboxylate (300 mg, 0.66 mmol), *N*-chlorosuccinimide (177 mg, 1.16 mmol) and acetonitrile (20 mL) were added to a 100 mL flask. The reaction was stirred at 75°C for 30 minutes. Aqueous sodium thiosulfate solution was added to the reaction solution and the mixture was extracted with ethyl acetate. The organic phase was dried, concentrated and then purified by flash silica gel column (EtOAc/PE: 0-50%) to obtain *tert*-butyl (*R*)-3,4-dichloro-1-((*R*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (210 mg, 65%). ES-API: [M+H]⁺=487.1.

Step 5: *tert*-Butyl (*R*)-3,4-dichloro-1-((*R*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (210 mg, 0.43 mmol), 2-fluoro-6-hydroxyphenylboronic acid (280 mg, 1.72 mmol), Pd(PPh₃)₄ (56 mg, 0.04 mmol), sodium carbonate (140 mg, 1.32 mmol), 10 mL of dioxane and 2 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried, concentrated and then purified by flash silica gel column to obtain *tert*-butyl (6a*R*)-4-chloro-3-(2-fluoro-6-hydroxyphenyl)-1-((*R*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (220 mg, 90%) as a yellow solid. ES-API: [M+H]⁺=563.2.

Step 6: *tert-*Butyl (6a*R*)-4-chloro-3-(2-fluoro-6-hydroxyphenyl)-1-((*R*)-3-methylmorpholino)-12-oxo-6a,7,9,10-tetrahydro-12*H*-pyrazino[2,1-*c*]pyrido[3,4*-f*][1,4]oxazepine-8(6*H*)-carboxylate (220 mg, 0.39 mmol), 2 mL of trifluoroacetic acid and 4 mL of dichloromethane were added to a 50 mL flask. The mixture was stirred at room temperature for 1 hour, and the completion of reaction was detected by LC-MS. The reaction solution was concentrated to obtain a crude product of an intermediate, which was dissolved in 8 mL of dichloromethane. Triethylamine (131 mg, 1.3 mmol) and acrylic anhydride (49 mg, 0.38 mmol) were added thereto under an ice-water bath, and the reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-((*R*)-3-methylmorpholino)-6,6a,7,8,9,10-hexahydro-12*H*-pyrazino[2,1-*c*]pyrido[3,4-*f*][1,4]oxazepin-12-one (*Z*27, 45 mg, 22%) as a white solid. ¹H NMR (500 MHz, DMSO-*d*₆) δ 9.98 (s, 1H), 7.24 (s, 1H), 6.74 (d, *J* = 8.4 Hz, 3H), 6.17 (d, *J* = 17.0 Hz, 1H), 5.75 (d, *J* = 9.7 Hz, 1H), 4.40-4.30 (m, 3H), 4.20-3.76 (m, 5H), 3.66-3.53 (m, 3H), 3.35 (s, 2H), 3.30 (s, 2H), 3.01 (d, *J* = 10.9 Hz, 1H), 1.09 (d, *J* = 6.7 Hz, 3H). ES-API: [M+H]⁺=517.1.

### Embodiment 27 Synthesis of (6aR)-8-acryloyl-4-chloro-3-(2-fluoro-6-hydroxyphenyl)-1-(((R)-1-phenylethyl)amino)-6,6a,7,8,9,10-hexahydro-12H-pyrazino[2,1-c]pyrido[3,4-f][1,4]oxazepin-12-one Z28

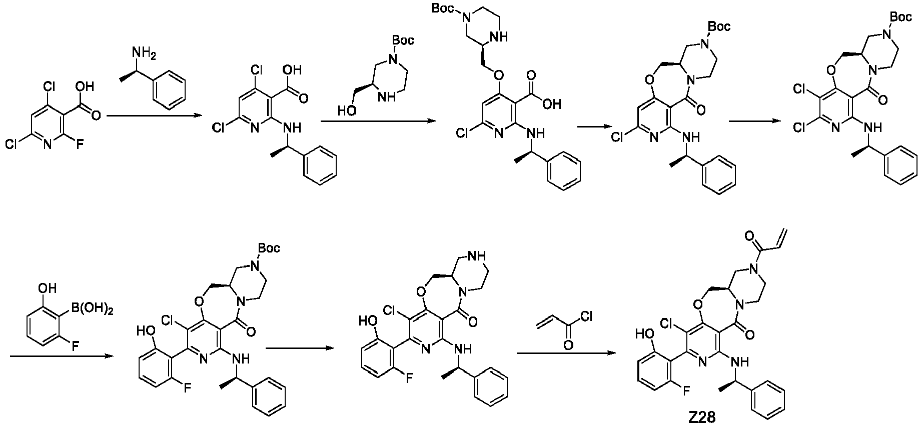

Step 1: 4,6-Dichloro-2-fluoronicotinic acid (500 mg, 2.38 mmol), (*R*)-(+)-α-phenylethylamine (346 mg, 2.86 mmol), and a solution of *N,N*-diisopropylethylamine (920 mg, 7.14 mmol) in acetonitrile (10 mL) were stirred at 70°C for 30 minutes. The reaction solution was poured into 30 mL of ethyl acetate, washed with 30 mL of 1 M dilute hydrochloric acid solution, and the organic phase was dried and concentrated to obtain a crude product of (*R*)-4,6-dichloro-2-((1-phenylethyl)amino)nicotinic acid (750 mg) as a yellow oil. ES-API: [M+H]⁺=311.0.

Step 2: *tert*-Butyl (*R*)-3-(hydroxymethyl)piperazine-1-carboxylate (1.04 g, 4.82 mmol) was added to a turbid solution of sodium hydride (482 mg, 12.05 mmol) in THF (10 mL) under an ice bath, and stirred for 10 minutes. Then the above crude product of (*R*)-4,6-dichloro-2-((1-phenylethyl)amino)nicotinic acid (750 mg, 2.41 mmol) was added thereto, heated to 60°C and stirred for 1 hour. After the reaction was completed, the reaction solution was quenched with 10 mL of saturated ammonium chloride solution, extracted with 10 mL of ethyl acetate for three times, and the organic phases were combined, dried and concentrated to obtain 4-(((*R*)-4-(*tert*-butoxycarbonyl)piperazin-2-yl)methoxy)-6-chloro-2-(((R-1-1-phenylethyl)amino)nicotinic acid (1.2 g, crude product). ES-API: [M+H]⁺=491.2.

Step 3: 4-(((*R*)-4-(*tert*-Butoxycarbonyl)piperazin-2-yl)methoxy)-6-chloro-2-(((*R*-1-1-phenylethyl)amino)nicotinic acid (1.2 g, crude product) obtained above was dissolved in dichloromethane (20 mL), and *N,N*-diisopropylethylamine (4.2 mL) was added thereto, and then a solution of 1-propylphosphonic anhydride in ethyl acetate (4.2 mL) was added thereto. The mixture was stirred at room temperature for 10 minutes. The reaction solution was poured into 50 mL of dichloromethane, and washed three times with 50 mL of sodium bicarbonate solution. The organic phase was dried, concentrated and then purified by flash silica gel column (EtOAc/PE: 0-70%) to obtain *tert*-butyl (*R*)-3-chloro-12-oxo-1-(((*R*)-1-phenylethyl)amino)-6a,7,9,10-tetrahydro-12*H*-pyrazino[2,1-*c*]pyrido[3,4-*f*][1,4]oxazepine-8(6*H*)-carboxylate (620 mg, 3-step yield of 55%). ES-API: [M+H]⁺=473.2.

Step 4: At -55°C, a solution of *tert*-butyl (*R*)-3-chloro-12-oxo-1-(((*R*)-1-phenylethyl)amino)-6a,7,9,10-tetrahydro-12*H*-pyrazino[2,1-*c*]pyrido[3,4-*f*][1,4]oxazepine-8(6*H*)-carboxylate (600 mg, 1.27 mmol) in dichloromethane (20 mL) was added with sulfonyl chloride (342 mg, 2.54 mmol) and stirred for 10 minutes. The reaction solution was quenched with 10 mL of methanol, heated to room temperature, concentrated, and redissolved in 10 mL of dichloromethane, added with 1 mL of triethylamine and 1 mL of di-*tert*-butyl dicarbonate, stirred for 10 minutes, concentrated and purified by flash silica gel column (EtOAc/PE: 0-70%) to obtain *tert*-butyl (*R*)-3,4-dichloro-12-oxo-1-(((*R*)-1-phenylethyl)amino)-6a,7,9,10-tetrahydro-12*H*-pyrazino[2,1-*c*]pyrido[3,4-*f*][1,4]oxazepine-8(6*H*)-carboxylate (430 mg, 67%). ES-API: [M+H]⁺=507.1.

Step 5: Under nitrogen protection, a mixed solution of *tert*-butyl (*R*)-3,4-dichloro-12-oxo-1-(((*R*)-1-phenylethyl)amino)-6a,7,9,10-tetrahydro-12*H*-pyrazino[2,1-*c*]pyrido[3,4-*f*][1,4]oxazepine-8(6*H*)-carboxylate (430 mg, 0.85 mmol), 2-fluoro-6-hydroxyphenylboronic acid (400 mg, 2.54 mmol), tetrakis(triphenylphosphine)palladium (98 mg, 0.08 mmol) and potassium carbonate (351 mg, 2.54 mmol) in 1,4-dioxane (8 mL) and water (2 mL) was reacted at 110°C for 30 minutes under microwave irradiation. The reaction solution was added to 30 mL of water and extracted three times with 30 mL of ethyl acetate. The organic phases were combined, dried, concentrated and purified by flash silica gel column (EtOAc/PE: 0-80%) to obtain *tert*-butyl (6a*R*)-4-chloro-3-(2-fluoro-6-hydroxyphenyl)-12-oxo-1-(((R)-1-phenylethyl)amino)-6a,7,9,10-tetrahydro-12*H*-pyrazino[2,1-*c*]pyrido[3,4-*f*][1,4]oxazepine-8(6*H*)-carboxylate (400 mg, 81%). ES-API: [M+H]⁺=583.2.

Step 6: Under an ice bath, trifluoroacetic acid (2 mL) was added to a solution of *tert*-butyl (6a*R*)-4-chloro-3-(2-fluoro-6-hydroxyphenyl)-12-oxo-1-(((*R*)-1-phenylethyl)amino)-6a,7,9,10-tetrahydro-12*H-*pyrazino[2,1-*c*]pyrido[3,4-*f*][1,4]oxazepine-8(6*H*)-carboxylate (400 mg) in dichloromethane (8 mL), warmed to room temperature and stirred for 1 hour. The reaction solution was concentrated to obtain a crude product of (6a*R*)-4-chloro-3-(2-fluoro-6-hydroxyphenyl)-1-(((*R*)-1-phenylethyl)amino)-6,6a,7,8,9,10-hexahydro-12*H*-pyrazino[2,1-*c*]pyrido[3,4-*f*][1,4]oxazepin-12-one as a yellow oil, which was directly used in the next reaction step. ES-API: [M+H]⁺=483.1.

Step 7: Under an ice bath, saturated potassium carbonate solution (2 mL) was added to a solution of the above crude product of (6a*R*)-4-chloro-3-(2-fluoro-6-hydroxyphenyl)-1-(((R)-1-phenylethyl)amino)-6,6a,7,8,9,10-hexahydro-12*H*-pyrazino[2,1-*c*]pyrido[3,4-*f*][1,4]oxazepin-12-one in THF (4 mL), and then acryloyl chloride (62 mg) was added thereto and stirred for 10 minutes. The reaction solution was dissolved in 10 mL of water, extracted with 10 mL of ethyl acetate, and the organic phase was dried, concentrated and then purified by preparative HPLC to obtain (6a*R*)-8-acryloyl-4-chloro-3-(2-fluoro-6-hydroxyphenyl)-1-(((*R*)-1-phenylethyl)amino)-6,6a,7,8,9,10-hexahydro-12*H*-pyrazino[2,1-*c*]pyrido[3,4-*f*][1,4]oxazepin-12-one (Z28, 134 mg, 36%) as a yellow solid. ¹H NMR (500 MHz, DMSO-*d*₆) δ 10.0-9.80 (m, 1H), 7.69-7.48 (m, 1H), 7.38-7.29 (m, 4H), 7.28-7.20 (m, 2H), 6.80-6.63 (m, 3H), 6.22-6.09 (m, 1H), 5.81-5.66 (m, 1H), 5.19-5.05 (m, 1H), 4.48-4.26 (m, 2H), 4.12 (s, 1H), 4.04-3.54 (m, 6H), 1.46 (d, *J* = 6.5 Hz, 3H). ES-API: [M+H]⁺=537.1.

### Embodiment 28 Synthesis of (6aR)-8-acryloyl-4-chloro-3-(2-fluoro-6-hydroxyphenyl)-1-(((S)-1-phenylethyl)amino)-6,6a,7,8,9,10-hexahydro-12H-pyrazino[2,1-c]pyrido[3,4-f][1,4]oxazepin-12-one Z29

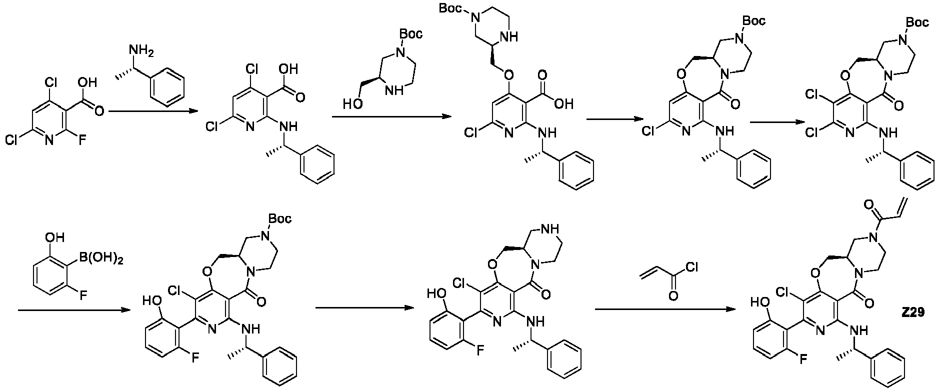

Step 1: 4,6-Dichloro-2-fluoronicotinic acid (500 mg, 2.38 mmol), (*S*)-(-)-α-phenylethylamine (346 mg, 2.86 mmol), and a solution of *N,N-*diisopropylethylamine (920 mg, 7.14 mmol) in acetonitrile (10 mL) were stirred at 70°C for 30 minutes. The reaction solution was poured into 30 mL of ethyl acetate, washed with 30 mL of 1 M dilute hydrochloric acid solution, and the organic phase was dried and concentrated to obtain (*S*)-4,6-dichloro-2-((1-phenylethyl)amino)nicotinic acid (750 mg, crude product) as a yellow oil. ES-API: [M+H]⁺=311.0.

Step 2: *tert*-Butyl (*S*)-3-(hydroxymethyl)piperazine-1-carboxylate (1.04 g, 4.82 mmol) was added to a turbid solution of sodium hydride (482 mg, 12.05 mmol) in THF (10 mL) under an ice bath, and stirred for 10 minutes. Then the above (*S*)-4,6-dichloro-2-((1-phenylethyl)amino)nicotinic acid (750 mg, crude product) was added thereto, heated to 60°C and stirred for 1 hour. After the reaction was completed, the reaction solution was quenched with 10 mL of saturated ammonium chloride solution, extracted with 10 mL of ethyl acetate for three times, and the organic phases were combined, dried and concentrated to obtain 4-(((*R*)-4-(*tert*-butoxycarbonyl)piperazin-2-yl)methoxy)-6-chloro-2-(((*S*-1-1-phenylethyl)amino)nicotinic acid (1.2 g, crude product). ES-API: [M+H]⁺=491.2.

Step 3: 4-(((*R*)-4-(*tert-*Butoxycarbonyl)piperazin-2-yl)methoxy)-6-chloro-2-(((*S*-1-1-phenylethyl)amino)nicotinic acid (1.2 g, crude product) obtained above was dissolved in dichloromethane (20 mL), and *N,N*-diisopropylethylamine (4.2 mL) was added thereto, and then a solution of 1-propylphosphonic anhydride in ethyl acetate (4.2 mL) was added thereto. The mixture was stirred at room temperature for 10 minutes. The reaction solution was poured into 50 mL of dichloromethane, and washed three times with 50 mL of sodium bicarbonate solution. The organic phase was dried, concentrated and then purified by flash silica gel column (EtOAc/PE: 0-70%) to obtain *tert*-butyl (*R*)-3-chloro-12-oxo-1-(((*S*)-1-phenylethyl)amino)-6a,7,9,10-tetrahydro-12*H*-pyrazino[2,1-*c*]pyrido[3,4-*f*][1,4]oxazepine-8(6*H*)-carboxylate (620 mg, 3-step yield of 55%). ES-API: [M+H]⁺=473.2.

Step 4: At -55°C, a solution of *tert*-butyl (*R*)-3-chloro-12-oxo-1-(((*S*)-1-phenylethyl)amino)-6a,7,9,10-tetrahydro-12*H*-pyrazino[2,1-*c*]pyrido[3,4-*f*][1,4]oxazepine-8(6*H*)-carboxylate (600 mg, 1.27 mmol) in dichloromethane (20 mL) was added with sulfonyl chloride (342 mg, 2.54 mmol) and stirred for 10 minutes. The reaction solution was quenched with 10 mL of methanol, heated to room temperature, concentrated, and redissolved in 10 mL of dichloromethane, added with 1 mL of triethylamine and 1 mL of di-*tert*-butyl dicarbonate, stirred for 10 minutes, concentrated and purified by flash silica gel column (EtOAc/PE: 0-70%) to obtain *tert*-butyl (*R*)-3,4-dichloro-12-oxo-1-(((*S*)-1-phenylethyl)amino)-6a,7,9,10-tetrahydro-12*H*-pyrazino[2,1-*c*]pyrido[3,4-*f*][1,4]oxazepine-8(6*H*)-carboxylate (430 mg, 67%). ES-API: [M+H]⁺=507.1.

Step 5: Under nitrogen protection, a mixed solution of *tert*-butyl (*R*)-3,4-dichloro-12-oxo-1-(((*S*)-1-phenylethyl)amino)-6a,7,9,10-tetrahydro-12*H*-pyrazino[2,1-*c*]pyrido[3,4-*f*][1,4]oxazepine-8(6*H*)-carboxylate (430 mg, 0.85 mmol), 2-fluoro-6-hydroxyphenylboronic acid (400 mg, 2.54 mmol), tetrakis(triphenylphosphine)palladium (98 mg, 0.08 mmol) and potassium carbonate (351 mg, 2.54 mmol) in 1,4-dioxane (8 mL) and water (2 mL) was reacted at 110°C for 30 minutes under microwave irradiation. The reaction solution was added to 30 mL of water and extracted three times with 30 mL of ethyl acetate. The organic phases were combined, dried, concentrated and purified by flash silica gel column (EtOAc/PE: 0-80%) to obtain *tert*-butyl (6a*R*)-4-chloro-3-(2-fluoro-6-hydroxyphenyl)-12-oxo-1-(((*S*-1-phenylethyl)amino)-6a,7,9,10-tetrahydro-12*H*-pyrazino[2,1-*c*]pyrido[3,4-*f*][1,4]oxazepine-8(6*H*)-carboxylate (450 mg, 91%). ES-API: [M+H]⁺=583.2.

Step 6: Under an ice bath, trifluoroacetic acid (2 mL) was added to a solution of *tert*-butyl (6a*R*)-4-chloro-3-(2-fluoro-6-hydroxyphenyl)-12-oxo-1-(((*S*)-1-phenylethyl)amino)-6a,7,9,10-tetrahydro-12*H-*pyrazino[2,1-*c*]pyrido[3,4-*f*][1,4]oxazepine-8(6*H*)-carboxylate (450 mg) in dichloromethane (8 mL), warmed to room temperature and stirred for 1 hour. The reaction solution was concentrated to obtain a crude product of (6a*R*)-4-chloro-3-(2-fluoro-6-hydroxyphenyl)-1-(((*S*)-1-phenylethyl)amino)-6,6a,7,8,9,10-hexahydro-12*H*-pyrazino[2,1-*c*]pyrido[3,4-*f*][1,4]oxazepin-12-one as a yellow oil, which was directly used in the next reaction step. ES-API: [M+H]⁺=483.1.

Step 7: Under an ice bath, saturated potassium carbonate solution (2 mL) was added to a solution of the above crude product of (6a*R*)-4-chloro-3-(2-fluoro-6-hydroxyphenyl)-1-(((*S*)-1-phenylethyl)amino)-6,6a,7,8,9,10-hexahydro-12*H*-pyrazino[2,1-*c*]pyrido[3,4-*f*][1,4]oxazepin-12-one in THF (4 mL), and then acryloyl chloride (62 mg) was added thereto and stirred for 10 minutes. The reaction solution was dissolved in 10 mL of water, extracted with 10 mL of ethyl acetate, and the organic phase was dried, concentrated and then purified by preparative HPLC to obtain (6a*R*)-8-acryloyl-4-chloro-3-(2-fluoro-6-hydroxyphenyl)-1-(((*S*)-1-phenylethyl)amino)-6,6a,7,8,9,10-hexahydro-12*H*-pyrazino[2,1-*c*]pyrido[3,4-*f*][1,4]oxazepin-12-one (Z29, 255 mg, 62%) as a yellow solid. ¹H NMR (500 MHz, DMSO-*d*₆) δ 9.90 (s, 1H), 7.69-7.48 (m, 1H), 7.38-7.29 (m, 4H), 7.28-7.20 (m, 2H), 6.84-6.57 (m, 3H), 6.22-6.09 (m, 1H), 5.81-5.66 (m, 1H), 5.19-5.05 (m, 1H), 4.48-4.26 (m, 2H), 4.17 (s, 1H), 4.07-3.54 (m, 6H), 1.43 (d, *J* = 6.8 Hz, 3H). ES-API: [M+H]⁺=537.1.

### Embodiment 29 Synthesis of (6aR)-8-acryloyl-4-chloro-3-(2-fluoro-6-hydroxyphenyl)-1-(4-methylpiperazin-1-yl)-6,6a,7,8,9,10-hexahydro-12H-pyrazino[2,1-c]pyrido[3,4-f][1,4]oxazepin-12-one Z30

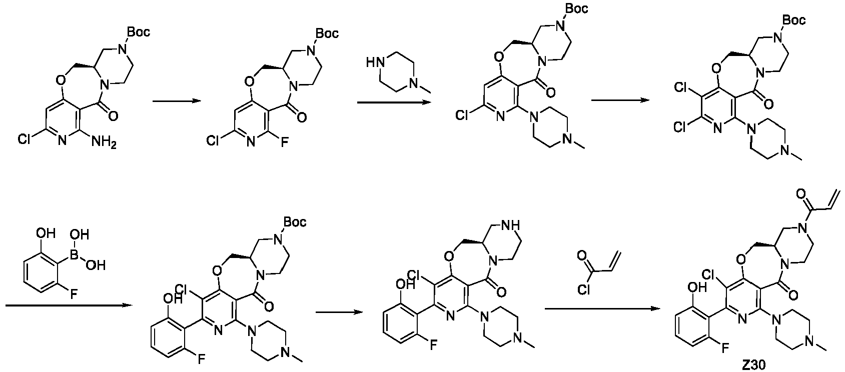

Step 1: *tert*-Butyl (*R*)-1-amino-3-chloro-12-oxo-6a,7,9,10-tetrahydro-6*H*-pyrazino[2,1-*c*]pyrido[3,4-*f*][1,4]oxazepine-8(12*H*)-carboxylate (500 mg, 1.36 mmol) and a solution of 70% hydrofluoric acid in pyridine (1 mL) were added to a 100 mL round bottom plastic flask. Sodium nitrite (257 mg, 4.06 mmol) was added thereto under an ice-water bath and stirred for 30 minutes, and the reaction was completed. A mixed solution of aqueous NaOH solution (2 g, 20 mL) and di-*tert*-butyl dicarbonate (877 mg, 4.06 mmol) in THF (20 mL) was prepared, and the above reaction solution was slowly added dropwise to the above mixed solution under an ice-water bath, and stirred for 1 hour after the addition, and extracted with ethyl acetate. The mixture was washed with saturated ammonium chloride solution, 1M aqueous hydrochloric acid solution, saturated sodium bicarbonate and saturated brine in turn. The organic phase was dried, concentrated and then purified by flash silica gel column (EtOAc/PE: 0-30%) to obtain *tert-*butyl (*R*)-3-chloro-1-fluoro-12-oxo-6a,7,9,10-tetrahydro-6*H*-pyrazino[2,1-*c*]pyrido[3,4-*f*][1,4]oxazepine-8(12*H*)-carboxylate (210 mg, 42%). ES-API: [M+H]⁺=371.1.

Step 2: *tert*-Butyl (*R*)-3-chloro-1-fluoro-12-oxo-6a,7,9,10-tetrahydro-6*H*-pyrazino[2,1-*c*]pyrido[3,4-*f*][1,4]oxazepine-8(12*H*)-carboxylate (210 mg, 0.566 mmol), N-methylpiperazine (170 mg, 1.70 mmol) and acetonitrile (10 mL) were added to a 100 mL flask. The reaction was stirred at 60°C for 30 minutes. The reaction solution was extracted with ethyl acetate, washed with aqueous ammonium chloride solution and saturated brine in turn, and the organic phase was dried and concentrated to obtain *tert-*butyl (*R*)-3-chloro-1-(4-methylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (220 mg, crude product). ES-API: [M+H]⁺=452.2.

Step 3: *tert*-Butyl (*R*)-3-chloro-1-(4-methylpiperazin-1-yl)-12-oxo-6a,7,9,10-tetrahydro-6H-pyrazino[2,1-*c*]pyrido[3,4-*f*][1,4]oxazepine-8(12*H*)-carboxylate (220 mg, crude product), *N-*chlorosuccinimide (118 mg, 0.89 mmol) and acetonitrile (10 mL) were added to a 100 mL flask. The reaction was stirred at 80°C for 20 minutes. The reaction solution was added with aqueous sodium thiosulfate solution, extracted with ethyl acetate, washed with sodium bicarbonate and saturated brine in turn, and the organic phase was dried and concentrated to obtain *tert*-butyl (*R*)-3,4-dichloro-1-(4-methylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (250 mg, crude product). ES-API: [M+H]⁺=486.2.

Step 4: *tert*-Butyl (*R*)-3,4-dichloro-1-(4-methylpiperazin-1-yl)-12-oxo-6a,7,9,10-tetrahydro-6*H-*pyrazino[2,1-*c*]pyrido[3,4-*f*][1,4]oxazepine-8(12*H*)-carboxylate (250 mg, crude product), 2-fluoro-6-hydroxyphenylboronic acid (321 mg, 2.06 mmol), Sphos (34 mg, 0.082 mmol), Sphos-Pd-G2 (59 mg, 0.082 mmol), potassium carbonate (170 mg, 1.23 mmol), 10 mL of dioxane and 2 mL of water were added to a 100 mL reaction flask. The reaction was stirred in an oil bath at 90°C for 1 hour, and the reaction was stopped. The reaction solution was added with 20 mL of water, extracted three times with 20 mL of ethyl acetate, and the organic phase was dried, concentrated, and purified by a flash silica gel column (EtOAc/PE: 0-60%) to obtain a target product (150 mg, 3-step yield of 47%) as a yellow solid. ES-API: [M+H]⁺=562.2.

Step 5: *tert*-Butyl (6a*R*)-4-chloro-3-(2-fluoro-6-hydroxyphenyl)-1-(4-methylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (150 mg, 0.267 mmol), 1 mL of trifluoroacetic acid and 4 mL of dichloromethane were added to a 50 mL flask. The mixture was stirred at room temperature for 1 hour, and the completion of reaction was detected by LC-MS. The reaction solution was concentrated to obtain a crude product of (6aR)-4-chloro-3-(2-fluoro-6-hydroxyphenyl)-1-(4-methylpiperazin-1-yl)-7,8,9,10-tetrahydro-6*H*-pyrazino[2,1-*c*]pyrido[3,4-*f*][1,4]oxazepin-12-one (122 mg, crude product) as a yellow solid. ES-API: [M+H]⁺=462.2.

Step 6: (6a*R*)-4-Chloro-3-(2-fluoro-6-hydroxyphenyl)-1-(4-methylpiperazin-1-yl)-7,8,9,10-tetrahydro-6*H*-pyrazino[2,1*-*c]pyrido[3,4-*f*][1,4]oxazepin-12-one (122 mg, crude product), 5 mL of dichloromethane and diisopropylethylamine (171 mg, 1.36 mmol) were added to a 50 mL flask. At 0°C, a solution of acryloyl chloride in dichloromethane (21 mg, 0.267 mmol, 1 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-(4-methylpiperazin-1-yl)-6,6a,7,8,9,10-hexahydro-12*H*-pyrazino[2,1-*c*]pyrido[3,4-*f*][1,4]oxazepin-12-one (Z30, 35 mg, 2-step yield of 25.5%) as a white solid. ¹H NMR (400 MHz, DMSO-*d*₆) δ 10.02 (d, *J* = 3.7 Hz, 1H), 7.25 (q, *J* = 7.9 Hz, 1H), 6.73 (tt, *J* = 18.4, 8.7 Hz, 3H), 6.17 (d, *J* = 16.7 Hz, 1H), 5.76 (d, *J* = 9.9 Hz, 1H), 4.52-4.29 (m, 2H), 4.33-4.10 (m, 2H), 4.08-3.77 (m, 3H), 3.66 (s, 1H), 3.47 (s, 4H), 3.25 (d, *J* = 4.8 Hz, 2H), 2.67 (s, 3H), 2.34 (s, 3H). ES-API: [M+H]⁺=516.2.

### Embodiment 30 Synthesis of (6aR)-8-acryloyl-4-chloro-1-(4-(dimethylamino)piperidin-1-yl)-3-(2-fluoro-6-hydroxyphenyl)-6,6a,7,8,9,10-hexahydro-12H-pyrazino[2,1-c]pyrido[3,4-f][1,4]oxazepin-12-one Z31

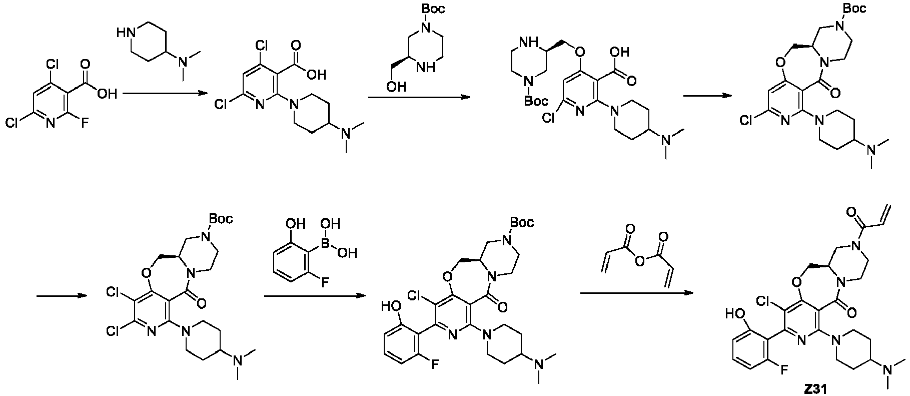

Step 1: 4,6-Dichloro-2-fluoronicotinic acid (600 mg, 2.86 mmol), *N,N*-dimethylpiperidin-4-amine (366 mg, 2.86 mmol), *N,N*-diisopropylethylamine (738 mg, 5.72 mmol) and 10 mL of acetonitrile were added to a 100 mL flask. The reaction was stirred at 70°C for 1 hour. The reaction solution was concentrated to obtain 4,6-dichloro-2-(4-(dimethylamino)piperidin-1-yl)nicotinic acid (900 mg, 98%) as a yellow solid. ES-API: [M+H]⁺=318.1.

Step 2: 60% sodium hydride (572 mg, 14.3 mmol), *tert*-butyl (*R*)-3-(hydroxymethyl)piperazine-1-carboxylate (927 mg, 4.29 mmol) and THF (10 mL) were added to a 100 mL flask. A solution of 4,6-dichloro-2-(4-(dimethylamino)piperidin-1-yl)nicotinic acid (900 mg, crude product) in THF was added dropwise thereto at 0°C. The reaction was stirred in an oil bath at 85°C for 1 hour. The completion of reaction was detected by LC-MS. The reaction solution was poured into ice-water. The pH of the reaction solution was adjusted to 7 with 2 M hydrochloric acid. The reaction solution was concentrated to dryness. The crude product was purified by flash silica gel column (MeOH/DCM: 10-100%) to obtain (*R*)-4-((4-(*tert-*butoxycarbonyl)piperazin-2-yl)methoxy)-6-chloro-2-(4-(dimethylamino)piperidin-1-yl)nicotinic acid (730 mg, 51%) as a yellow solid. ES-API: [M+H]⁺=498.2.

Step 3: (*R*)-4-((4-(*tert*-Butoxycarbonyl)piperazin-2-yl)methoxy)-6-chloro-2-(4-(dimethylamino)piperidin-1-yl)nicotinic acid (700 mg, 1.40 mmol), HATU (1.06 g, 2.80 mmol), *N,N-*diisopropylethylamine (542 mg, 4.20 mmol) and dichloromethane (10 mL) were added to a 100 mL flask. The mixture was reacted at room temperature for 1 hour. The completion of reaction was detected by LC-MS. 50 mL of dichloromethane was added to the reaction, then washed with 30 mL of water, and 30 mL of saturated aqueous sodium bicarbonate solution, respectively. The organic phase was dried, concentrated and then purified by flash silica gel column (MeOH/DCM: 0-10%) to obtain *tert*-butyl (*R*)-3-chloro-1-(4-(dimethylamino)piperidin-1-yl)-12-oxo-6a,7,9,10-tetrahydro-12*H*-pyrazino[2,1-*c*]pyrido[3,4-*f*][1,4]oxazepine-8(6*H*)-carboxylate (540 mg, 80%) as a yellow solid. ES-API: [M+H]⁺=480.3.

Step 4: *tert*-Butyl (*R*)-3-chloro-1-(4-(dimethylamino)piperidin-1-yl)-12-oxo-6a,7,9,10-tetrahydro-12*H*-pyrazino[2,1-*c*]pyrido[3,4-*f*][1,4]oxazepine-8(6*H*)-carboxylate (500 mg, 1.04 mmol), *N-*chlorosuccinimide (222 mg, 1.67 mmol), *N,N*-dimethylformamide (8 mL) and acetonitrile (10 mL) were added to a 100 mL flask. The reaction solution was stirred at 50°C for 2.5 hours. Aqueous sodium thiosulfate solution was added to the reaction solution and extracted with ethyl acetate. The organic phase was dried, concentrated and then purified by flash silica gel column (MeOH/DCM: 0-20%) to obtain *tert-*butyl (*R*)-3,4-dichloro-1-(methyl(tetrahydro-2*H*-pyran-4-yl)amino)-12-oxo-6a,7,9,10-tetrahydro-12*H-*pyrazino[2,1-*c*]pyrido[3,4-*f*][1,4]oxazepine-8(6*H*)-carboxylate (130 mg, 90%). ES-API: [M+H]⁺=514.1.

Step 5: *tert*-Butyl (*R*)-3,4-dichloro-1-(methyl(tetrahydro-2*H*-pyran-4-yl)amino)-12-oxo-6a,7,9,10-tetrahydro-12*H*-pyrazino[2,1-*c*]pyrido[3,4-*f*][1,4]oxazepine-8(6*H*)-carboxylate (480 mg, 0.93 mmol), 2-fluoro-6-hydroxyphenylboronic acid (437 mg, 2.80 mmol), Pd(PPh₃)₄ (86 mg, 0.074 mmol), sodium carbonate (297 mg, 2.80 mmol), 10 mL of dioxane and 2 mL of water were added to a 100 mL reaction flask. The reaction was carried out in an oil bath at 110°C for 2 hours, then the reaction solution was added with 20 mL of water, extracted three times with with DCM/MeOH (10:1, v/v). The organic phase was dried, concentrated and then purified by flash silica gel column (MeOH/DCM: 0-80%) to obtain *tert*-butyl (6a*R*)-4-chloro-1-(4-(dimethylamino)piperidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (300 mg, yield of 54%) as a yellow solid. ES-API: [M+H]⁺=590.2.

Step 6: *tert-*Butyl (6a*R*)-4-chloro-1-(4-(dimethylamino)piperidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (300 mg, 0.51 mmol), trifluoroacetic acid (3 mL) and dichloromethane (2 mL) were added to a flask. After stirring at room temperature for 1 hour, the reaction solution was concentrated to obtain a yellow solid. The yellow solid was dissolved in 5 mL of dichloromethane and triethylamine (154 mg, 1.53 mmol). At 0°C, a solution of acrylic anhydride in dichloromethane (65 mg, 0.51 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-1-(4-(dimethylamino)piperidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z31, 23 mg, 8%) as a yellow solid. ¹H NMR (400 MHz, CDCl₃) 7.32-7.26 (m, 1H), 6.84-6.83 (m, 1H), 6.70-6.69 (m, 1H), 6.57-6.41 (m, 1H), 6.44-6.41 (m, 1H), 5.83 (d, J=9.6 Hz, 1H), 4.40-4.07 (m, 7H), 3.84-3.55 (m, 4H), 3.00-3.94 (m, 2H), 2.61-2.60 (m, 1H), 2.40 (s, 6H), 2.04-1.99 (m, 2H), 1.70-1.55 (m, 2H). ES-API: [M+H]⁺=544.2.

### Embodiment 31 Synthesis of (6aR)-8-acryloyl-4-chloro-3-(2-fluoro-6-hydroxyphenyl)-1-((S)-3-methylmorpholino)-6,6a,7,8,9,10-hexahydro-12H-pyrazino[2,1-c]pyrido[3,4-f][1,4]oxazepin-12-one Z32

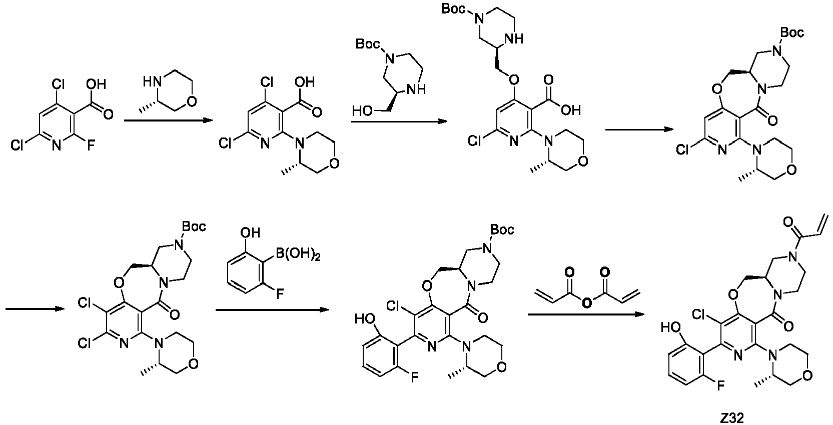

Step 1: (*S*)-3-Methylmorpholine (606 mg, 6.00 mmol), 4,6-dichloro-2-fluoronicotinic acid (630 mg, 3.00 mmol), DIEA (1.16 g, 9.00 mmol) and 20 mL of acetonitrile were added to a 100 mL flask. The reaction was stirred at 80 °C for 3 hours. After the reaction was completed, the reaction solution was evaporated to dryness by rotary evaporation and subjected to column chromatography to obtain (R)-4,6-dichloro-2-(3-methylmorpholino)nicotinic acid (1.1 g, 67%). ES-API: [M+H]⁺=291.1.

Step 2: 60% sodium hydride (1.16 g, 28.5 mmol), *tert*-butyl (*R*)-3-(hydroxymethyl)piperazine-1-carboxylate (1.24 g, 5.7 mmol) and THF (8 mL) were added to a 100 mL flask. A THF suspension (4 mL) of 4,6-dichloro-2-((2-isopropyl-4-methylpyridin-3-yl)amino)nicotinic acid (1.1 g, 4.07 mmol) was added dropwise thereto at 0°C. The reaction was stirred in an oil bath at 80°C for 2 hours. The completion of reaction was detected by LC-MS. 30 mL of saturated aqueous NH₄Cl solution was poured into the reaction solution. The mixture was extracted 4 times with 40 mL of ethyl acetate. The organic phase was dried, and then concentrated and subjected to column chromatography to obtain 4-(((*R*)-4-(*tert-*butoxycarbonyl)piperazin-2-yl)methoxy)-6-chloro-2-((S)-3-methylmorpholino)nicotinic acid (800 mg, 41%) as a yellow solid. ES-API: [M+H]⁺=470.8.

Step 3: 4-(((*R*)-4-(*tert*-Butoxycarbonyl)piperazin-2-yl)methoxy)-6-chloro-2-((*S*)-3-methylmorpholino)nicotinic acid (800 mg, 1.70 mmol), diisopropylethylamine (4 mL) and dichloromethane (15 mL) were added to a 100 mL flask. Propylphosphonic anhydride solution (4 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of aqueous hydrochloric acid solution (1 M), and 30 mL of sodium bicarbonate, respectively. The organic phase was dried, and then concentrated and subjected to column chromatography to obtain *tert*-butyl (*R*)-3-chloro-1-((*S*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (600 mg, 81%) as a yellow solid. ES-API: [M+H]⁺=453.3.

Step 4: *tert*-Butyl (*R*)-3-chloro-1-((*S*)-3-methylmorpholino)-12-oxo-6a,7,9,10-tetrahydro-12*H-*pyrazino[2,1-*c*]pyrido[3,4-*f*][1,4]oxazepine-8(6*H*)-carboxylate (280 mg, 0.62 mmol), N-chlorosuccinimide (166 mg, 1.08 mmol) and acetonitrile (20 mL) were added to a 100 mL flask. The reaction was stirred at 75°C for 30 minutes. Aqueous sodium thiosulfate solution was added to the reaction solution and extracted with ethyl acetate. The organic phase was dried, concentrated and then purified by flash silica gel column (EtOAc/PE: 0-50%) to obtain *tert*-butyl (*R*)-3,4-dichloro-1-((*S*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (300 mg, 98%). ES-API: [M+H]⁺=487.1.

Step 5: *tert*-Butyl (*R*)-3,4-dichloro-1-((*S*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (300 mg, 0.61 mmol), 2-fluoro-6-hydroxyphenylboronic acid (400 mg, 2.56 mmol), Pd(PPh₃)₄ (80 mg, 0.07 mmol), sodium carbonate (200 mg, 1.89 mmol), 10 mL of dioxane and 2 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried, concentrated and then purified by flash silica gel column to obtain *tert*-butyl (6a*R*)-4-chloro-3-(2-fluoro-6-hydroxyphenyl)-1-((*S*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (110 mg, 31%) as a yellow solid. ES-API: [M+H]⁺=563.2.

Step 6: *tert*-Butyl (6a*R*)-4-chloro-3-(2-fluoro-6-hydroxyphenyl)-1-((*S*)-3-methylmorpholino)-12-oxo-6a,7,9,10-tetrahydro-12*H*-pyrazino[2,1-*c*]pyrido[3,4-*f*][1,4]oxazepine-8(6*H*)-carboxylate (110 mg, 0.19 mmol), 2 mL of trifluoroacetic acid and 4 mL of dichloromethane were added to a 50 mL flask. The mixture was stirred at room temperature for 1 hour, and the completion of reaction was detected by LC-MS. The reaction solution was concentrated to obtain a crude product of an intermediate, which was dissolved in 8 mL of dichloromethane. Triethylamine (66 mg, 0.65 mmol) and acrylic anhydride (24 mg, 0.19 mmol) were added thereto under an ice-water bath, and the reaction was carried out at 0°C for 10 minutes. 20 mL of saturated aqueous sodium bicarbonate solution was added to the reaction solution, and the mixture was extracted three times with 10 mL of dichloromethane. The organic phase was dried, concentrated and then purified by preparative HPLC to obtain (6a*R*)-8-acryloyl-4-chloro-3-(2-fluoro-6-hydroxyphenyl)-1-((*S*)-3-methylmorpholino)-6,6a,7,8,9,10-hexahydro-12*H*-pyrazino[2,1-*c*]pyrido[3,4-*f*][1,4]oxazepin-12-one (Z32, 52 mg, 53%) as a white solid. ¹H NMR (400 MHz, DMSO-*d*₆) δ 10.05-9.95 (m, 1H), 7.25 (d, *J* = 8.0 Hz, 1H), 6.87-6.62 (m, 3H), 6.17 (d, *J* = 16.7 Hz, 1H), 5.74 (s, 1H), 4.10-4.00 (m, 7H), 3.83-3.41 (m, 7H), 3.29 (d, *J* = 11.2 Hz, 2H), 1.25 (t, *J* = 5.7 Hz, 3H). ES-API: [M+H]⁺=517.1.

### Embodiment 32 Synthesis of N-((6aR)-8-acryloyl-4-chloro-3-(2-fluoro-6-hydroxyphenyl)-12-oxo-6,6a,7,8,9,10-hexahydro-12H-pyrazino[2,1-c]pyrido[3,4-f][1,4]oxazepin-1-yl)-3,3-dimethylbutanamide Z33

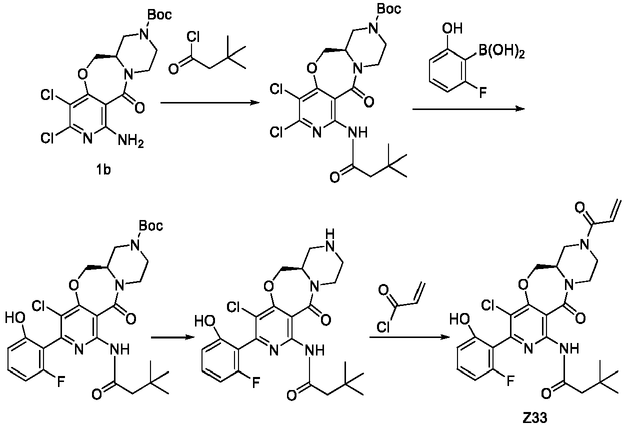

Step 1: *tert*-Butyl (*R*)-1-amino-3,4-dichloro-12-oxo-6a,7,9,10-tetrahydro-12H-pyrazino[2,1-*c*]pyrido[3,4-*f*][1,4]oxazepine-8(6*H*)-carboxylate (350 mg, 0.87 mmol) was dissolved in 8 mL of pyridine, and 3,3-dimethylbutanoyl chloride (233 mg, 1.74 mmol) was added thereto. The reaction solution was stirred at 70°C for 3 hours. The cooled reaction solution was poured into 30 mL of ice-water, and the reaction solution was concentrated, and extracted with 60 mL of ethyl acetate. The organic phase was washed twice with 40 mL of 1.0 M dilute brine, 30 mL of saturated sodium bicarbonate, 30 mL of saturated brine in turn, dried and concentrated, and the crude product was purified by flash silica gel column (EtOAc/PE: 0-40%) to obtain *tert*-butyl (*R*)-3,4-dichloro-1-(3,3-dimethylbutanamido)-12-oxo-6a,7,9,10-tetrahydro-12*H*-pyrazino[2,1-*c*]pyrido[3,4-*f*][1,4]oxazepine-8(6*H*)-carboxylate (320 mg, 73%) as a white solid. ES-API: [M+H]⁺=501.2.

Step 2: *tert*-Butyl (*R*)-3,4-dichloro-1-(3,3-dimethylbutanamido)-12-oxo-6a,7,9,10-tetrahydro-12*H*-pyrazino[2,1-*c*]pyrido[3,4-*f*][1,4]oxazepine-8(6*H*)-carboxylate (300 mg, 0.60 mmol), (2-fluoro-6-hydroxyphenyl)boronic acid (281 mg, 1.80 mmol), tetrakis(triphenylphosphine)palladium (69 mg, 0.06 mmol), sodium carbonate (191 mg, 1.80 mmol), 3 mL of water and 15 mL of dioxane were added to a 100 mL flask. The reaction solution was stirred at 110°C for 2 hours under nitrogen protection. The reaction solution was added with 30 mL of water, extracted with 60 mL of ethyl acetate, and the organic phase was washed with 30 mL of saturated brine, dried and concentrated, and the crude product was purified by flash silica gel column (EtOAc/PE: 0-50%) to obtain *tert*-butyl (6a*R*)-4-chloro-1-(3,3-dimethylbutanamido)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (340 mg, 98%) as a white solid. ES-API: [M+H]⁺=577.2.

Step 3: *tert*-Butyl (6a*R*)-4-chloro-1-(3,3-dimethylbutanamido)-3-(2-fluoro-6-hydroxyphenyl)-12-oxo-6a,7,9,10-tetrahydro-12*H*-pyrazino[2,1-*c*]pyrido[3,4-*f*[1,4]oxazepine-8(6*H*)-carboxylate (340 mg, 0.59 mmol) was dissolved in 4 mL of dichloromethane, and 1 mL of trifluoroacetic acid was added thereto. After stirring at room temperature for 2 hours, the reaction solution was concentrated to obtain *N*-((6a*R*)-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-3,3-dimethylbutanamide (450 mg, crude product), which was directly used in the next reaction step. ES-API: [M+H]⁺=477.1.

Step 4: *N*-((6a*R*)-4-Chloro-3-(2-fluoro-6-hydroxyphenyl)-12-oxo-6,6a,7,8,9,10-hexahydro-12*H-*pyrazino[2,1-*c*]pyrido[3,4-*f*][1,4]oxazepin-1-yl)-3,3-dimethylbutanamide (450 mg, crude product) was dissolved in 10 mL of dichloromethane, and *N,N*-diisopropylethylamine (387 mg, 3.0 mmol) was added thereto. Acryloyl chloride (50 mg, 0.55 mmol) was added dropwise thereto at 0°C, and the reaction solution was stirred at 0°C for 15 minutes. The reaction solution was added with 30 mL of dichloromethane, washed with 15 mL of water, 15 mL of saturated aqueous NaHCO₃ solution, and 15 mL of saturated brine in turn, dried, concentrated, and purified by preparative HPLC to obtain *N*-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-3,3-dimethylbutanamide (Z33, 240 mg, 2-step yield of 77%) as a white solid. ¹H NMR (400 MHz, DMSO-*d*₆) δ 10.70 (s, 1H), 10.13 (s, 1H), 7.33-7.25 (m, 1H), 6.84-6.58 (m, 3H), 6.22-6.08 (m, 1H), 5.80-5.65 (m, 1H), 4.46-4.26 (m, 3H), 4.12-3.55 (m, 6H), 2.28 (d, *J* = 12.4 Hz, 1H), 2.06-1.93 (m, 1H), 1.00 (s, 9H). ES-API: [M+H]⁺=531.1.

### Embodiment 33 Synthesis of (6aR)-8-acryloyl-4-chloro-3-(2-fluoro-6-hydroxyphenyl)-1-(2-azaspiro[4.4]nonan-2-yl)-6,6a,7,8,9,10-hexahydro-12H-pyrazino[2,1-c]pyrido[3,4-f][1,4]oxazepin-12-one Z34

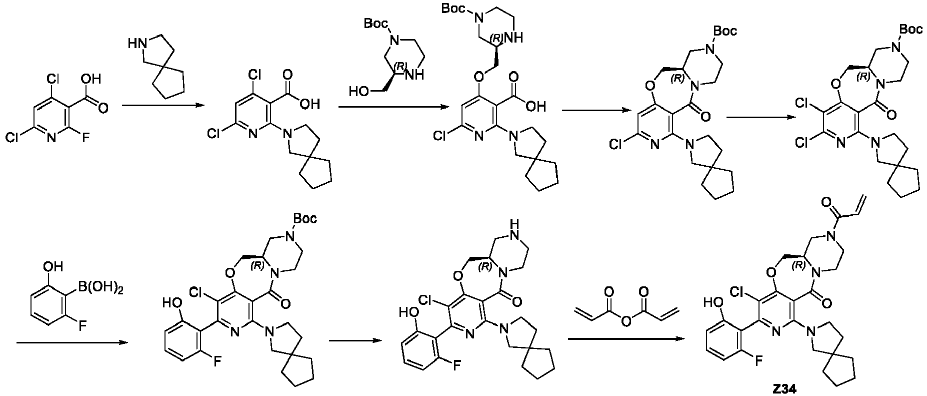

Step 1: 4,6-Dichloro-2-fluoronicotinic acid (0.9 g, 4.31 mmol) was dissolved in 9 mL of acetonitrile, and *N,N*-diisopropylethylamine (1.67 g, 12.93 mmol) and 2-azaspiro[4.4]nonane (449 mg, 3.59 mmol) were added thereto. The reaction solution was stirred at room temperature for 2 hours. The reaction solution was added with water, and the pH was adjusted to 5-6 with dilute hydrochloric acid, extracted with ethyl acetate, and the organic phase was washed with brine, dried over anhydrous sodium sulfate and concentrated to obtain a crude product of 4,6-dichloro-2-(2-azaspiro[4.4]nonan-2-yl)nicotinic acid (900 mg, 79.8%). ES-API: [M+H]⁺=315.1.

Step 2: Sodium hydride (1.15 g, 28.7 mmol) was suspended in 3 mL of THF and cooled to 0°C. A solution of *tert*-butyl (*R*)-3-(hydroxymethyl)piperazine-1-carboxylate (1.24 g, 5.73 mmol) in THF (3 mL) was added dropwise thereto, and after the addition, the mixture was stirred and reacted for 30 minutes while maintaining the temperature. A solution of 4,6-dichloro-2-(2-azaspiro[4.4]nonan-2-yl)nicotinic acid (0.9 g, 2.87 mmol) in THF (3 mL) was added dropwise thereto, and after the addition, the mixture was stirred and reacted at 70°C for 4 hours. The reaction solution was quenched with saturated ammonium chloride solution, extracted with ethyl acetate, and the organic phase was washed with brine, dried over anhydrous sodium sulfate, and concentrated to obtain (*R*)-4-((4-(*tert*-butoxycarbonyl)piperazin-2-yl)methoxy)-6-chloro-2-(2-azaspiro[4.4]nonan-2-yl)nicotinic acid (1.44 g, crude product). The crude product was directly used in the next reaction step. ES-API: [M+H]⁺=495.2.

Step 3: (*R*)-4-((4-(*tert*-Butoxycarbonyl)piperazin-2-yl)methoxy)-6-chloro-2-(2-azaspiro[4.4]nonan-2-yl)nicotinic acid (1.44 g, crude product) was dissolved in 150 mL of dichloromethane, and 1.5 mL of *N,N*-diisopropylethylamine and HATU (1.33 g, 3.5 mmol) were added thereto. After the addition, the reaction solution was stirred at room temperature for 1 hour. The reaction solution was washed with saturated sodium bicarbonate solution, and the organic phase was washed with brine, dried over anhydrous sodium sulfate, and concentrated to obtain a crude product of *tert-*butyl (*R*)-3-chloro-12-oxo-1-(2-azaspiro[4.4]nonan-2-yl)-6a,7,9,10-tetrahydro-6*H*-pyrazino[2,1-*c*]pyrido[3,4-*f*][1,4]oxazepine-8(12*H-*carboxylate (1.29 g, 2-step yield of 93.1%). ES-API: [M+H]⁺=477.2.

Step 4: *tert*-Butyl (*R*)-3-chloro-12-oxo-1-(2-azaspiro[4.4]nonan-2-yl)-6a,7,9,10-tetrahydro-6H-pyrazino[2,1-*c*]pyrido[3,4-*f*][1,4]oxazepine-8(12*H*)-carboxylate (1.29 g, 2.71 mmol) was dissolved in 15 mL of acetonitrile, and *N*-chlorosuccinimide (325.6 mg, 2.44 mmol) was added thereto. The reaction solution was stirred and reacted at 50°C for 3 hours. The reaction solution was added with water, extracted with ethyl acetate, and the organic phase was washed with brine, dried over anhydrous sodium sulfate, concentrated, and the crude product was purified by flash silica gel column (PE/EtOAc=1/1) to obtain *tert-*butyl (*R*)-3,4-dichloro-12-oxo-1-(2-azaspiro[4.4]nonan-2-yl)-6a,7,9,10-tetrahydro-6*H*-pyrazino[2,1-*c*]pyrido[3,4-*f*][1,4]oxazepine-8(12*H*)-carboxylate (730 mg, 52.8%). ES-API: [M+H]⁺=511.2.

Step 5: *tert*-Butyl (*R*)-3,4-dichloro-12-oxo-1-(2-azaspiro[4.4]nonan-2-yl)-6a,7,9,10-tetrahydro-6*H*-pyrazino[2,1-*c*]pyrido[3,4-*f*][1,4]oxazepine-8(12*H*)-carboxylate (400 mg, 0.784 mmol) was suspended in dioxane/water (4 mL/1 mL), and sodium carbonate (415 mg, 3.92 mmol), (2-fluoro-6-hydroxyphenyl)boronic acid (367 mg, 2.353 mmol) and tetrakis(triphenylphosphine)palladium (90.6 mg, 0.0784 mmol) were added thereto. The reaction solution was stirred at 100°C for 16 hours under nitrogen protection. The reaction solution was added with water, extracted with ethyl acetate. The organic phase was washed with brine, dried over anhydrous sodium sulfate, concentrated, and the crude product was purified by flash silica gel column chromatography (PE/EtOAc=1/1) to obtain *tert*-butyl (6a*R*)-4-chloro-3-(2-fluoro-6-hydroxyphenyl)-12-oxo-1-(2-azaspiro[4.4]nonan-2-yl)-6a,7,9,10-tetrahydro-6*H*-pyrazino[2,1-*c*]pyrido[3,4-*f*][1,4]oxazepine-8(12*H*)-carboxylate (200 mg, 43.5%). ES-API: [M+H]⁺=587.2.

Step 6: *tert*-Butyl (6a*R*)-4-chloro-3-(2-fluoro-6-hydroxyphenyl)-12-oxo-1-(2-azaspiro[4.4]nonan-2-yl)-6a,7,9,10-tetrahydro-6*H*-pyrazino[2,1-*c*]pyrido[3,4-*f*][1,4]oxazepine-8(12*H*)-carboxylate (200 mg, 0.34 mmol) was dissolved in 2 mL of dichloromethane, and 2 mL of trifluoroacetic acid was added thereto. The reaction was carried out at room temperature for 0.5 hours, then the reaction solution was concentrated and redissolved in dichloromethane, and concentrated to obtain (6a*R*)-4-chloro-3-(2-fluoro-6-hydroxyphenyl)-1-(2-azaspiro[4.4]nonan-2-yl)-6,6a,7,8,9,10-hexahydro-12*H*-pyrazino[2,1-*c*]pyrido[3,4-*f*][1,4]oxazepin-12-one (250 mg, crude product). The crude product was directly used in the next reaction step. ES-API: [M+H]⁺=487.2.

Step 7: (6a*R*)-4-Chloro-3-(2-fluoro-6-hydroxyphenyl)-1-(2-azaspiro[4.4]nonan-2-yl)-6,6a,7,8,9,10-hexahydro-12*H*-pyrazino[2,1-*c*]pyrido[3,4-*f*][1,4]oxazepin-12-one (250 mg, crude product) was dissolved in 3 mL of dichloromethane, cooled with ice-water, added with 1 mL of triethylamine, then slowly added with acrylic anhydride (38.6 mg, 0.306 mmol), reacted at 10°C for 10 minutes, then added with water, extracted with dichloromethane. The organic phase was washed with brine, dried over anhydrous sodium sulfate, concentrated, and the crude product was purified by preparative HPLC to obtain (6a*R*)-8-acryloyl-4-chloro-3-(2-fluoro-6-hydroxyphenyl)-1-(2-azaspiro[4.4]nonan-2-yl)-6,6a,7,8,9,10-hexahydro-12*H*-pyrazino[2,1-*c*]pyrido[3,4-*f*][1,4]oxazepin-12-one (Z34, 44.9 mg, 2-step yield of 10.6%). ¹H NMR (400 MHz, CDCl₃) δ7.29 (s, 1H), 6.86 (d, *J* = 8.2 Hz, 1H), 6.69 (t, *J* = 8.9 Hz, 1H), 6.56 (s, 1H), 6.41 (d, *J* = 16.1 Hz, 1H), 5.82 (d, *J* = 9.9 Hz, 1H), 4.43 (s, 3H), 4.28 (s, 1H), 4.20 (d, *J* = 13.1 Hz, 1H), 4.03 (s, 1H), 3.72 (s, 3H), 3.50 (s, 2H), 3.27 (s, 1H), 3.11 (s, 1H), 1.85 (s, 2H), 1.65 (s, 6H), 1.53 (s, 2H). ES-API: [M+H]⁺=541.2.

### Embodiment 34 Synthesis of (6aR)-8-acryloyl-4-chloro-3-(2-fluoro-6-hydroxyphenyl)-1-(6-azaspiro[3.4]octan-6-yl)-6,6a,7,8,9,10-hexahydro-12H-pyrazino[2,1-c]pyrido[3,4-f][1,4]oxazepin-12-one Z35

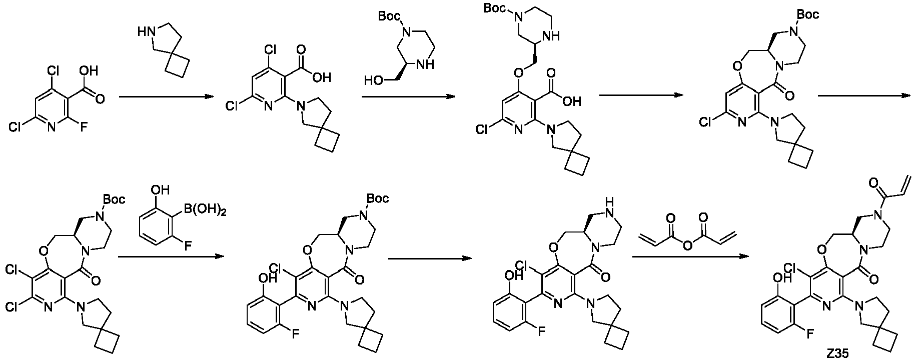

Step 1: 4,6-Dichloro-2-fluoronicotinic acid (1.3 g, 6.2 mmol) was dissolved in 10 mL of acetonitrile, and *N,N*-diisopropylethylamine (800 mg, 12.4 mmol) and 6-azaspiro[3.4]octane (480 mg, 4.34 mmol) were added thereto. The reaction solution was stirred at room temperature for 2 hours, and the completion of reaction was monitored by LCMS. The reaction solution was quenched with water, extracted with ethyl acetate (20 mL*3), and the organic layer was washed with ammonium chloride solution (50 mL*2), dried, filtered and concentrated to obtain 4,6-dichloro-2-(6-azaspiro[3.4]*n*-octyl-6-yl)nicotinic acid (1.44 g, crude product), and the crude product was directly used in the next reaction step. ES-API: [M+H]⁺=301.0.

Step 2: *tert-*Butyl (*R*)-3-(hydroxymethyl)piperazine-1-carboxylate (3.10 g, 14.4 mmol) was suspended in 30 mL of THF, and sodium hydride (1.91 g, 47.8 mmol) was added thereto at 0°C. The mixture was stirred at temperature and reacted for 60 minutes. A solution of 4,6-dichloro-2-(6-azaspiro[3.4]*n*-octan-6-yl)nicotinic acid (1.44 g, crude product) in THF (5 mL) was added dropwise thereto, and after the addition, the mixture was stirred and reacted at 60°C for 16 hours. The reaction solution was quenched with saturated ammonium chloride solution, extracted with ethyl acetate, and the organic phase was concentrated to obtain (*R*)-4-((4-(*tert*-butoxycarbonyl)piperazin-2-yl)methoxy)-6-chloro-2-(6-azaspiro[3.4]octan-6-yl)nicotinic acid (2.7 g, crude product). The crude product was directly used in the next reaction step. ES-API: [M+H]⁺=481.2.

Step 3: (*R*)-4-((4-(*tert*-Butoxycarbonyl)piperazin-2-yl)methoxy)-6-chloro-2-(6-azaspiro[3.4]octan-6-yl)nicotinic acid (2.45 g, crude product) was dissolved in 200 mL of dichloromethane, and 12 mL of *N,N*-diisopropylethylamine was added thereto. T₃P (12 mL, 50 wt% ethyl acetate solution) was added dropwise thereto. After the addition, the reaction solution was stirred at room temperature for 18 hours. The reaction solution was washed with water, and the organic phase was concentrated, and the crude product was purified by flash silica gel column (PE/EtOAc=4/1) to obtain *tert*-butyl (*R*)-3-chloro-12-oxo-1-(6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (630 mg, 3-step yield of 26.7%) as a yellow solid. ES-API: [M+H]⁺=463.2.

Step 4: *tert*-Butyl (*R*)-3-chloro-12-oxo-1-(6-azaspiro[3.4]octan-6-yl)-6a,7,9,10-tetrahydro-6*H-*pyrazino[2,1-*c*]pyrido[3,4-*f*][1,4]oxazepine-8(12*H*)-carboxylate (254 mg, 0.55 mmol) was dissolved in 8 mL of acetonitrile, and *N*-chlorosuccinimide (80 mg, 0.61 mmol) was added thereto. The reaction solution was stirred and reacted at 70°C for 2 hours. The reaction solution was directly purified by flash silica gel column (PE/EtOAc=3/1) to obtain *tert*-butyl (*R*)-3,4-dichloro-12-oxo-1-(6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (260 mg, 95.6%) as a white solid. ES-API: [M+H]⁺=497.2.

Step 5: *tert*-Butyl (*R*)-3,4-dichloro-12-oxo-1-(6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (182 mg, 0.37 mmol) was suspended in dioxane/water (5 mL/1 mL), and sodium carbonate (118 mg, 1.11 mmol), (2-fluoro-6-hydroxyphenyl)boronic acid (86 mg, 0.55 mmol) and tetrakis(triphenylphosphine)palladium (42 mg, 0.037 mmol) were added thereto. The reaction solution was stirred at 130°C for 4 hours under nitrogen protection. The reaction solution was directly mixed with silica gel and purified by flash silica gel column chromatography (PE/EtOAc=3/2) to obtain *tert*-butyl (6a*R*)-4-chloro-3-(2-fluoro-6-hydroxyphenyl)-12-oxo-1-(6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (176 mg, yield of 83.0%) as a yellow solid. ES-API: [M+H]⁺=573.3.

Step 6: *tert*-Butyl (6a*R*)-4-chloro-3-(2-fluoro-6-hydroxyphenyl)-12-oxo-1-(6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (176 mg, 0.308 mmol) was dissolved in 3 mL of dichloromethane, and 3 mL of trifluoroacetic acid was added thereto. The reaction solution was stirred at room temperature for 1 hour. The reaction solution was concentrated and redissolved in dichloromethane, and concentrated to obtain (6a*R*)-4-chloro-3-(2-fluoro-6-hydroxyphenyl)-1-(6-azaspiro[3.4]octan-6-yl)-7,8,9,10-hexahydro-6*H*-pyrazino[2,1-*c*]pyrido[3,4-*f*][1,4]-12(6a*H*)-one (650 mg, crude product), which was directly used in the next reaction step. ES-API: [M+H]⁺=473.2.

Step 7: (6a*R*)-4-Chloro-3-(2-fluoro-6-hydroxyphenyl)-1-(6-azaspiro[3.4]octan-6-yl)-7,8,9,10-hexahydro-6*H*-pyrazino[2,1-*c*]pyrido[3,4-*f*][1,4]-12(6a*H*)-one (650 mg, 0.308 mmol, crude product) and triethylamine (310 mg, 3.08 mmol) were dissolved in 5 mL of dichloromethane, and acrylic anhydride (35 mg, 0.277 mmol) was added thereto at 0°C. The reaction solution was stirred at room temperature for 1 hour. The reaction solution was washed with water and concentrated, and the crude product was purified by preparative HPLC to obtain (6a*R*)-8-acryloyl-4-chloro-3-(2-fluoro-6-hydroxyphenyl)-1-(6-azaspiro[3.4]octan-6-yl)-6,6a,7,8,9,10-hexahydro-12*H*-pyrazino[2,1-*c*]pyrido[3,4-*f*][1,4]oxazepin-12(6a*H*)-one (Z35, 28.4 mg, 2-step yield of 17.6%) as a white solid. ¹H NMR (400 MHz, CDCl₃) δ 9.70 (s, 1H), 7.29 (d, *J* = 8.0 Hz, 1H), 6.83 (d, *J* = 8.2 Hz, 1H), 6.69 (t, *J=* 9.1 Hz, 1H), 6.57 (s, 1H), 6.42 (d, *J* = 16.4 Hz, 1H), 5.83 (d, *J=* 10.4 Hz, 1H), 4.41 (s, 3H), 4.25-4.15 (m, 2H), 4.03 (s, 1H), 3.76 (s, 2H), 3.57 (td, *J=* 11.1, 6.2 Hz, 2H), 3.46 *(d, J=* 10.8 Hz, 1H), 3.21 (s, 2H), 2.26-2.02 (m, 2H), 2.02-1.94 (m, 2H), 1.92 (s, 2H), 1.85 (td, *J* = 11.8, 8.2 Hz, 2H). ES-API: [M+H]⁺=527.2.

### Embodiment 35 Synthesis of (6aR)-8-acryloyl-4-chloro-3-(2-fluoro-6-hydroxyphenyl)-1-(5-oxa-8-azaspiro[3.5]nonan-8-yl)-6,6a,7,8,9,10-hexahydro-12H-pyrazino[2,1-c]pyrido[3,4-f][1,4]oxazepin-12-one Z36

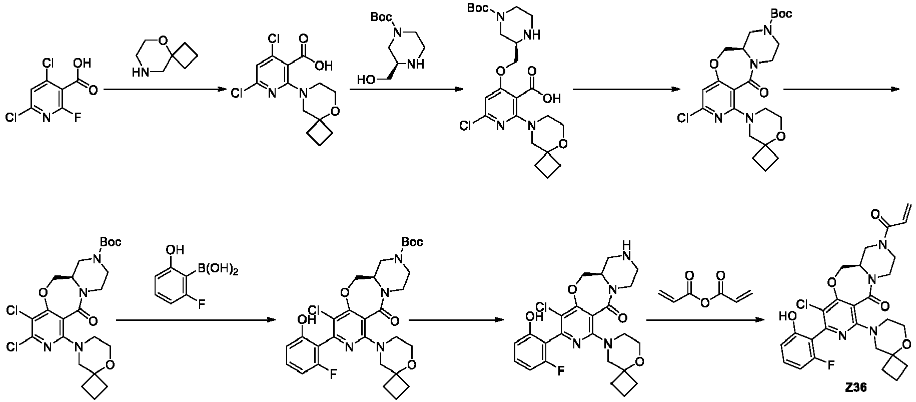

Step 1: 4,6-Dichloro-2-fluoronicotinic acid (893 mg, 4.25 mmol) was dissolved in 10 mL of acetonitrile, and *N,N-*diisopropylethylamine (1.37 g, 10.62 mmol) and 5-oxa-8-azaspiro[3.5]nonane (450 mg, 3.54 mmol) were added thereto. The reaction solution was stirred at room temperature overnight. The reaction solution was added with water, dilute hydrochloric acid to adjust pH to 5-6 and extracted with ethyl acetate. The organic phase was concentrated to obtain 4,6-dichloro-2-(5-oxa-8-azaspiro[3.5]nonan-8-yl)nicotinic acid (1.4 g, crude product), which was directly used in the next reaction step. ES-API: [M+H]⁺=317.0.

Step 2: Sodium hydride (755 mg, 31.5 mmol) was suspended in 10 mL of THF and cooled to 0°C. A solution of *tert*-butyl (*R*)-3-(hydroxymethyl)piperazine-1-carboxylate (1.02 g, 4.75 mmol) in THF (5 mL) was added dropwise thereto, and after the addition, the mixture was stirred and reacted for 30 minutes while maintaining the temperature. A solution of 4,6-dichloro-2-(5-oxa-8-azaspiro[3.5]nonan-8-yl)nicotinic acid (1.1 g, crude product) in THF (5 mL) was added dropwise thereto, and after the addition, the mixture was stirred and reacted at 60°C for 5 hours. The reaction solution was quenched with saturated ammonium chloride solution, extracted with ethyl acetate, and the organic phase was concentrated to obtain (*R*)-4-((4-(*tert*-butoxycarbonyl)piperazin-2-yl)methoxy)-6-chloro-2-(5-oxa-8-azaspiro[3.5]nonan-8-yl)nicotinic acid (2.2 g, crude product). The crude product was directly used in the next reaction step. ES-API: [M+H]⁺=497.2.

Step 3: The crude product of (*R*)-4-((4-(*tert*-butoxycarbonyl)piperazin-2-yl)methoxy)-6-chloro-2-(5-oxa-8-azaspiro[3.5]nonan-8-yl)nicotinic acid (2.2 g, crude product) was dissolved in 200 mL of dichloromethane, and 5 mL of *N,N*-diisopropylethylamine was added thereto. T₃P (5 mL, 50wt% ethyl acetate solution) was added dropwise thereto. After the addition, the reaction solution was stirred at room temperature for 1 hour. The reaction solution was washed with saturated sodium bicarbonate solution, and the organic phase was concentrated, and the crude product was purified by flash silica gel column (PE/EtOAc=1/1) to obtain *tert*-butyl (*R*)-3-chloro-12-oxo-1-(5-oxa-8-azaspiro[3.5]nonan-8-yl)-6a,7,9,10-tetrahydro-6*H*-pyrazino[2,1-*c*]pyrido[3,4-*f*][1,4]oxazepine-8(6*H*)-carboxylate (800 mg, 36%) as a pale yellow solid. ES-API: [M+H]⁺=479.1.

Step 4: *tert*-Butyl (*R*)-chloro-12-oxo-1-(5-oxa-8-azaspiro[3.5]nonan-8-yl)-6a,7,9,10-tetrahydro-6*H*-pyrazino[2,1-*c*]pyrido[3,4-*f*][1,4]oxazepine-8(12*H*)-carboxylate (800 mg, 1.67 mmol) was dissolved in 50 mL of acetonitrile, and *N*-chlorosuccinimide (223 mg, 1.67 mmol) was added thereto. The reaction solution was stirred at 40°C overnight. The reaction solution was washed with brine, extracted with ethyl acetate, and the organic phase was concentrated, and the crude product was purified by flash silica gel column (PE/EtOAc=1/1) to obtain *tert*-butyl (*R*)-3,4-dichloro-12-oxo-1-(5-oxa-8-azaspiro[3.5]nonan-8-yl)-6a,7,9,10-tetrahydro-6*H*-pyrazino[2,1-*c*]pyrido[3,4-*f*][1,4]oxazepine-8(6*H*)-carboxylate (520 mg, 60%) as a pale yellow solid. ES-API: [M+H]⁺=513.2.

Step 5: *tert-*Butyl (*R*)-3,4-dichloro-12-oxo-1-(5-oxa-8-azaspiro[3.5]nonan-8-yl)-6a,7,9,10-tetrahydro-6*H*-pyrazino[2,1-*c*]pyrido[3,4-*f*][1,4]oxazepine-8(6*H*)-carboxylate (500 mg, 0.98 mmol) was suspended in dioxane/water (6 mL/1 mL), and sodium carbonate (311 mg, 2.93 mmol), (2-fluoro-6-hydroxyphenyl)boronic acid (457 mg, 2.93 mmol) and tetrakis(triphenylphosphine)palladium (109 mg, 0.098 mmol) were added thereto. The reaction solution was stirred at 110°C for 5 hours under nitrogen protection. The reaction solution was extracted with ethyl acetate, washed with brine, and the organic phase was concentrated, and the crude product was purified by flash silica gel column chromatography (PE/EtOAc=1/1) to obtain *tert-*butyl (6a*R*)-4-chloro-3-(2-fluoro-6-hydroxyphenyl)-12-oxo-1-(5-oxa-8-azaspiro[3.5]nonan-8-yl)-6a,7,9,10-tetrahydro-6*H*-pyrazino[2,1-*c*]pyrido[3,4-*f*][1,4]oxazepine-8(6*H*)-carboxylate (475 mg, 80%) as a pale yellow solid. ES-API: [M+H]⁺=589.3.

Step 6: *tert-*Butyl (6a*R*)-4-chloro-3-(2-fluoro-6-hydroxyphenyl)-12-oxo-1-(5-oxa-8-azaspiro[3.5]nonan-8-yl)-6a,7,9,10-tetrahydro-6*H*-pyrazino[2,1-c]pyrido[3,4-*f*][1,4]oxazepine-8(6*H*)-carboxylate (475 mg, 0.81 mmol) was dissolved in 5 mL of dichloromethane, and 3 mL of trifluoroacetic acid was added thereto. The reaction solution was stirred at room temperature for 1 hour. The reaction solution was concentrated and redissolved with dichloromethane, and concentrated to obtain (6a*R*)-4-chloro-3-(2-fluoro-6-hydroxyphenyl)-1-(5-oxa-8-azaspiro[3.5]nonan-8-yl)-6,6a,7,8,9,10-hexahydro-12*H-*pyrazino[2,1*-c*]pyrido[3,4-*f*][1,4]oxazepin-12-one (500 mg, crude product). The crude product was directly used in the next reaction step. ES-API: [M+H]⁺=489.2.

Step 7: (6a*R*)-4-Chloro-3-(2-fluoro-6-hydroxyphenyl)-1-(5-oxa-8-azaspiro[3.5]nonan-8-yl)-6,6a,7,8,9,10-hexahydro-12*H*-pyrazino[2,1-*c*]pyrido[3,4-*f*][1,4]oxazepin-12-one (394 mg, 0.806 mmol) was dissolved in 2 mL of dichloromethane, and 1 mL of triethylamine was added thereto, and acrylic anhydride (86 mg, 0.68 mmol) was added dropwise thereto at 0°C. The reaction solution was stirred at room temperature for 1 hour. The reaction solution was washed with brine, extracted with dichloromethane, and the organic phase was concentrated, and the crude product was subjected to preparative HPLC to obtain (6a*R*)-8-acryloyl-4-chloro-3-(2-fluoro-6-hydroxyphenyl)-1-(5-oxa-8-azaspiro[3.5]nonan-8-yl)-6,6a,7,8,9,10-hexahydro-12*H*-pyrazino[2,1-*c*]pyrido[3,4-*f*][1,4]oxazepin-12-one (Z36, 70.2 mg, 16%) as a white solid. ¹H NMR (400 MHz, CDCl₃) δ 7.33-7.28 (m, 1H), 6.87 (d, *J* = 8.3 Hz, 1H), 6.71 (t, *J* = 9.0 Hz, 1H), 6.56 (s, 1H), 6.42 (d, *J* = 16.1 Hz, 1H), 5.83 (d, *J* = 10.2 Hz, 1H), 4.58-4.22 (m, 4H), 4.18-4.00 (m, 2H), 3.75-3.55 (m, 8H), 3.30 (s, 1H), 2.05 (d, *J* = 33.4 Hz, 4H), 1.86 (s, 1H), 1.58 (d, *J* = 9.9 Hz, 1H). ES-API: [M+H]⁺=543.2.

### Embodiment 36 Synthesis of (6aR)-8-acryloyl-4-chloro-1-(2,2-dimethylmorpholino)-3-(2-fluoro-6-hydroxyphenyl)-6,6a,7,8,9,10-hexahydro-12H-pyrazino[2,1-c]pyrido[3,4-f][1,4]oxazepin-12-one Z37

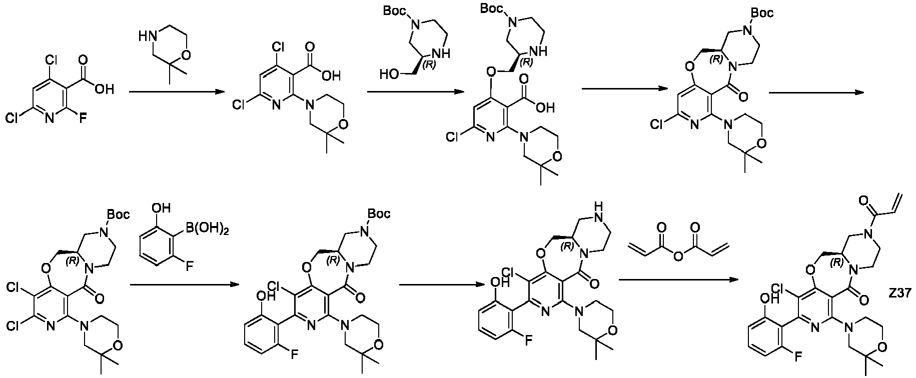

Step 1: 4,6-Dichloro-2-fluoronicotinic acid (2.6 g, 12.4 mmol) was dissolved in 60 mL of acetonitrile, and *N,N*-diisopropylethylamine (3.2 g, 24.8 mmol) and 5-oxa-8-azaspiro[3.5]nonane (1 g, 8.7 mmol) were added thereto. The reaction solution was stirred at room temperature for 2 hours. The reaction solution was added with water, dilute hydrochloric acid to adjust pH to 5-6 and extracted with ethyl acetate. The organic phase was concentrated to obtain 4,6-dichloro-2-(2,2-dimethylmorpholino)nicotinic acid (3.2 g, brown viscous liquid, crude product), and the crude product was directly used in the next reaction step. ES-API: [M+H]⁺=305.0.

Step 2: Sodium hydride (2.6 g, 65.8 mmol) was suspended in 30 mL of THF and cooled to 0°C. A solution of *tert-*butyl (*R*)-3-(hydroxymethyl)piperazine-1-carboxylate (4.3 g, 19.7 mmol) in THF (10 mL) was added dropwise thereto, and after the addition, the mixture was stirred and reacted for 30 minutes while maintaining the temperature. A solution of 4,6-dichloro-2-(2,2-dimethylmorpholino)nicotinic acid (2 g, 6.58 mmol) in THF (10 mL) was added dropwise thereto, and after the addition, the mixture was stirred and reacted at 70°C for 16 hours. The reaction solution was quenched with saturated ammonium chloride solution, extracted with ethyl acetate, and the organic phase was concentrated to obtain (*R*)-4-((4*-*(*tert-*butoxycarbonyl)piperazin-2-yl)methoxy)-6-chloro-2-(2,2-dimethylmorpholino)nicotinic acid (4.4 g, brown viscous oil, crude product). The crude product was directly used in the next reaction step. ES-API: [M+H]⁺=485.2.

Step 3: (*R*)-4-((4-(*tert*-Butoxycarbonyl)piperazin-2-yl)methoxy)-6-chloro-2-(2,2-dimethylmorpholino)nicotinic acid (4.4 g, crude product) was dissolved in 100 mL of dichloromethane, and 10 mL of *N,N*-diisopropylethylamine was added thereto. T₃P (10 mL, 50wt% ethyl acetate solution) was added dropwise thereto. After the addition, the reaction solution was stirred and reacted at room temperature for 1 hour. The reaction solution was washed with saturated sodium bicarbonate solution, and the organic phase was concentrated, and the crude product was purified by flash silica gel column (PE/EtOAc=1/1) to obtain *tert*-butyl (*R*)-3-chloro-1-(2,2-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (300 mg, 3-step yield of 7.5%, a pale yellow solid). ES-API: [M+H]⁺=467.2.

Step 4: *tert*-Butyl (*R*)-3-chloro-1-(2,2-dimethylmorpholino)-12-oxo-6a,7,9,10-tetrahydro-12*H-*pyrazino[2,1-*c*]pyrido[3,4-*f*][1,4]oxazepine-8(6*H*)-carboxylate (280 mg, 0.6 mmol) was dissolved in 6 mL of acetonitrile, and *N-*chlorosuccinimide (80 mg, 0.6 mmol) was added thereto. The reaction solution was stirred at 30°C overnight. The reaction solution was washed with brine, extracted with ethyl acetate, and the organic phase was concentrated, and the crude product was purified by flash silica gel column (PE/EtOAc=1/1) to obtain *tert*-butyl (*R*)-3,4-dichloro-1-(2,2-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (220 mg, 76%, a pale yellow solid). ES-API: [M+H]⁺=501.1.

Step 5: *tert*-Butyl (*R*)-3,4-dichloro-1-(2,2-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (200 mg, 0.4 mmol) was suspended in dioxane/water (4 mL/1 mL), and sodium carbonate (130 mg, 1.2 mmol), (2-fluoro-6-hydroxyphenyl)boronic acid (187 mg, 1.2 mmol) and tetrakis(triphenylphosphine)palladium (46 mg, 0.04 mmol) were added thereto. The reaction solution was stirred at 110°C for 16 hours under nitrogen protection. The reaction solution was extracted with ethyl acetate, washed with brine, and the organic phase was concentrated, and the crude product was purified by flash silica gel column chromatography (PE/EtOAc=1/1) to obtain *tert*-butyl (6a*R*)-4-chloro-1-(2,2-dimethylmorpholino)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (210 mg, yield of 83%, a pale yellow solid). ES-API: [M+H]⁺=577.3.

Step 6: *tert*-Butyl (6a*R*)-4-chloro-1-(2,2-dimethylmorpholino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (210 mg, 0.36 mmol) was dissolved in 5 mL of dichloromethane, and 3 mL of trifluoroacetic acid was added thereto. The reaction solution was stirred at room temperature for 1 hour. The reaction solution was concentrated and redissolved with dichloromethane and concentrated to obtain a crude product of (6a*R*)-4-chloro-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (160 mg, yield of 100%). The crude product was directly used in the next reaction step. ES-API: [M+H]⁺=477.2.

Step 7: (6a*R*)-4-Chloro-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (160 mg, 0.34 mmol) was dissolved in 2 mL of dichloromethane, and 2 mL of triethylamine was added thereto, and then acrylic anhydride (33 mg, 0.27 mmol) was slowly added thereto. The reaction solution was stirred at room temperature for 10 minutes. The reaction solution was washed with brine, extracted with dichloromethane, and the organic phase was concentrated, and the crude product was purified by preparative HPLC to obtain (6a*R*)-8-acryloyl-4-chloro-1-(2,2-dimethylmorpholino)-3-(2-fluoro-6-hydroxyphenyl)-6,6a,7,8,9,10-hexahydro-12*H*-pyrazino[2,1-c]pyrido[3,4-*f*][1,4]oxazepin-12-one (Z37, 15 mg, yield of 8.4%, white solid). ¹H NMR (400 MHz, CDCl₃) δ 7.30 (d, *J* = 8.4 Hz, 1H), 6.83 (d, *J* = 8.2 Hz, 1H), 6.69 (t, *J* = 9.0Hz, 1H), 6.56 (s, 1H), 6.41 (d, *J* = 16.4 Hz, 1H), 5.82 (d, *J* = 10.1 Hz, 1H), 4.45-4.23 (m, 4H), 4.19-3.97 (m, 2H), 3.93-3.70 (m, 4H), 3.63 (s, 1H), 3.48-3.33 (m, 3H), 3.10 (s, 1H), 1.28 (d, *J* = 8.7 Hz, 6H). ES-API: [M+H]⁺=531.2.

### Embodiment 37 Synthesis of (6aR)-8-acryloyl-1-((1R,5S)-3-oxa-8-azabicyclo[3.2.1]octan-8-yl)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z38

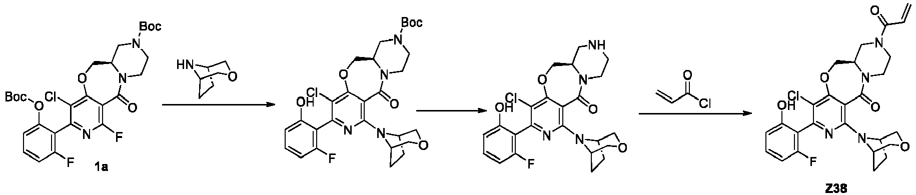

Step 1: (6a*R*)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (70 mg, 0.120 mmol), 3-oxa-8-azabicyclo[3.2.1]octane (41 mg, 0.361 mmol) and acetonitrile (10 mL) were added to a 100 mL flask. The reaction was stirred at 60°C for 1 hour. The mixture was extracted with ethyl acetate, washed with aqueous ammonium chloride solution and saturated brine in turn, and the organic phase was dried and concentrated, and purified by silica gel column to obtain (6a*R*)-8-*tert*-butoxycarbonyl-1-((1*R*,5*S*)-3-oxa-8-azabicyclo[3.2.1]octan-8-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (60 mg, 87%). ES-API: [M+H]⁺=575.2.

Step 2: (6a*R*)-8-*tert*-Butoxycarbonyl-1-((1*R*,5*S*)-3-oxa-8-azabicyclo[3.2.1]octan-8-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (60 mg, 0.104 mmol), 1 mL of trifluoroacetic acid and 4 mL of dichloromethane were added to a 50 mL flask. The mixture was stirred at room temperature for 1 hour, and the completion of reaction was detected by LC-MS. The reaction solution was concentrated to obtain a crude product of (6a*R*)-1-((1*R*,5*S*)-3-oxa-8-azabicyclo[3.2.1]octan-8-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (48 mg, crude product) as a yellow solid. ES-API: [M+H]⁺=475.2.

Step 3: (6a*R*)-1-((1*R*,5*S*)-3-Oxa-8-azabicyclo[3.2.1]octan-8-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (48 mg, crude product), 10 mL of dichloromethane and diisopropylethylamine (67 mg, 0.52 mmol) were added to a 50 mL flask. At 0°C, a solution of acryloyl chloride in dichloromethane (9.4 mg, 0.104 mmol, 1 mL) was added dropwise thereto. The reaction was carried out at 0°C for 10 minutes. The mixture was washed with 20 mL of water, and the organic phase was dried, concentrated and purified by preparative HPLC to obtain (6a*R*)-8-acryloyl-1-((1*R*,5*S*)-3-oxa-8-azabicyclo[3.2.1]octan-8-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z38, 14 mg, 2-step yield of 25%) as a white solid. ES-API: [M+H]⁺=529.2. ¹H NMR (500 MHz, DMSO-*d*₆) δ 7.25 (q, *J=* 7.9 Hz, 1H), 6.87-6.62 (m, 3H), 6.22-6.13 (m, 1H), 5.75 (d, *J* = 10.5 Hz, 1H), 4.38-4.28 (m, 4H), 4.16-3.98 (m, 4H), 3.88-3.77 (m, 2H), 3.49-3.43 (m, 5H), 2.00-1.69 (m, 4H).

### Embodiment 38 Synthesis of (6aR)-8-acryloyl-1-(2-azabicyclo[3.1.0]hexan-2-yl)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z39

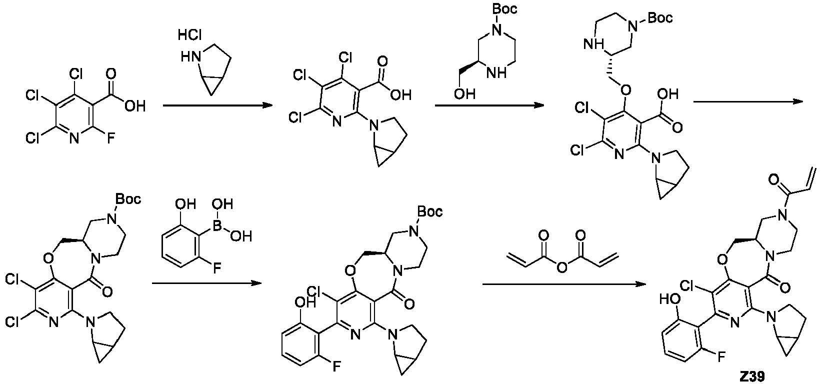

Step 1: 4,5,6-Trichloro-2-fluoronicotinic acid (286 mg, 1.17 mmol), 2-azabicyclo[3.1.0]hexane hydrochloride (140 mg, 1.17 mmol), *N,N*-diisopropylethylamine (604 mg, 4.68 mmol) and 6 mL of acetonitrile were added to a 100 mL flask. The reaction was stirred at 70°C for 1 hour. The reaction was quenched with 1 M hydrochloric acid and extracted with ethyl acetate. The organic phase was washed with saturated brine, dried and concentrated to obtain 2-(2-azabicyclo[3.1.0]hexan-2-yl)-4,5,6-trichloronicotinic acid (359 mg, crude product) as a yellow solid. ES-API: [M+H]⁺=307.0.

Step 2: 60% sodium hydride (187 mg, 4.68 mmol), *tert*-butyl (*R*)-3-(hydroxymethyl)piperazine-1-carboxylate (303 mg, 1.40 mmol) and THF (10 mL) were added to a 100 mL flask at 0°C. A solution of 2-(2-azabicyclo[3.1.0]hexan-2-yl)-4,5,6-trichloronicotinic acid (359 mg, crude product) in THF was added dropwise thereto. The reaction was stirred in an oil bath at 70°C for 30 minutes. The completion of reaction was detected by LC-MS. The reaction solution was poured into ice-water. The pH of the reaction solution was adjusted to 7 with 1 M hydrochloric acid. The reaction solution was concentrated to dryness to obtain 2-(2-azabicyclo[3.1.0]hexan-2-yl)-4-(((*R*)-4-(*tert*-butoxycarbonyl)piperazin-2-yl)methoxy)-5,6-dichloronicotinic acid (565 mg, crude product) as a yellow solid. ES-API: [M+H]⁺=487.1.

Step 3: 2-(2-Azabicyclo[3.1.0]hexan-2-yl)-4-(((R)-4-(*tert-*butoxycarbonyl)piperazin-2-yl)methoxy)-5,6-dichloronicotinic acid (565 mg, crude product), *N,N*-diisopropylethylamine (3 mL) and dichloromethane (10 mL) were added to a 100 mL flask. T₃P (3 mL, 50%w/w ethyl acetate solution) was added dropwise thereto. The mixture was reacted at room temperature for 1 hour. The completion of reaction was detected by LC-MS. 50 mL of dichloromethane was added to the reaction, then washed with 30 mL of water, and 30 mL of saturated aqueous sodium bicarbonate solution, respectively. The organic phase was dried, concentrated and then purified by flash silica gel column (EtOAc/PE: 0-100%) to obtain *tert*-butyl (6a*R*)-1-(2-azabicyclo[3.1.0]hexan-2-yl)-3,4-dichloro-12-oxo-6a,7,9,10-tetrahydro-12H-pyrazino[2,1-*c*]pyrido[3,4-*f*][1,4]oxazepine-8(6*H*)-carboxylate (350 mg, 3-step yield of 64%,) as a yellow solid. ES-API: [M+H]⁺=469.1.

Step 4: *tert*-Butyl (6a*R*)-1-(2-azabicyclo[3.1.0]hexan-2-yl)-3,4-dichloro-12-oxo-6a,7,9,10-tetrahydro-12*H*-pyrazino[2,1-*c*]pyrido[3,4-*f*][1,4]oxazepine-8(6*H*)-carboxylate (300 mg, 0.64 mmol), 2-fluoro-6-hydroxyphenylboronic acid (299 mg, 1.92 mmol), Pd(PPh₃)₄ (74 mg, 0.064 mmol), sodium carbonate (204 mg, 1.92 mmol), 10 mL of dioxane and 2 mL of water were added to a 100 mL reaction flask. The reaction was carried out in an oil bath at 110°C for 2 hours, then the reaction solution was added with 20 mL of water, extracted with ethyl acetate. The organic phase was dried and concentrated to obtain *tert-*butyl (6a*R*)-1-(2-azabicyclo[3.1.0]hexan-2-yl)-4-chloro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (348 mg, crude product) as a yellow solid. ES-API: [M+H]⁺=545.2.

Step 5: *tert*-Butyl (6a*R*)-1-(2-azabicyclo[3.1.0]hexan-2-yl)-4-chloro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (348 mg, crude product), trifluoroacetic acid (2 mL) and dichloromethane (0.5 mL) were added to a 100 mL reaction flask. After stirring at room temperature for 1 hour, the reaction solution was concentrated, added with water, and washed once with ethyl acetate. The pH of the aqueous phase was adjusted to 9 with saturated sodium bicarbonate. The aqueous phase was extracted with DCM/MeOH (10:1) system. The organic phase was dried and concentrated to obtain a yellow solid. The yellow solid was dissolved in 5 mL of dichloromethane and triethylamine (194 mg, 1.92 mmol). At 0°C, a solution of acrylic anhydride in dichloromethane (74 mg, 0.57 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-1-(2-azabicyclo[3 .1. 0]hexan-2-yl)-4-chloro-3-(2-fluoro-6-hydroxyphenyl)-6,6a, 7,8,9,1 0-hexahydro-12*H-*pyrazino[2,1-*c*]pyrido[3,4-*f*][1,4]oxazepin-12-one (Z39, 100.3 mg, 31%) as a yellow solid. ¹HNMR (500 MHz, CDCl₃) 7.47-7.26 (m, 1H), 6.84-6.83 (m, 1H), 6.70-6.69 (m, 1H), 6.58-6.56 (m, 1H), 6.43-6.41 (m, 1H), 5.83 (d, J=9.5 Hz, 1H), 4.40-4.37 (m, 2H), 4.25-4.20 (m, 2H), 4.05-4.02 (m, 2H), 3.84-3.42 (m, 4H), 3.25-2.65 (m, 1H), 2.42-2.07 (m, 1H), 2.04-1.65 (m, 3H), 0.88-0.65 (m, 1H), 0.51-0.41 (m, 1H). ES-API: [M+H]⁺=499.1.

### Embodiment 39 Synthesis of (6aR)-8-acryloyl-1-((1-methylpiperidin-4-yl)methoxy)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z40

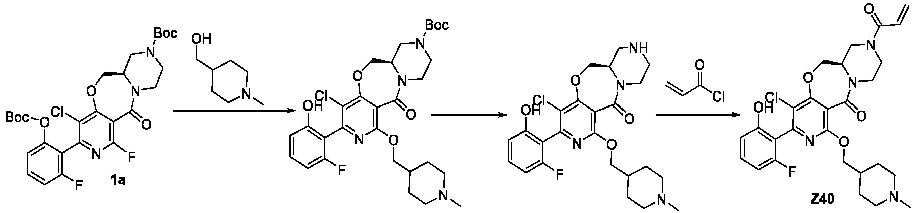

Step 1: (1-Methylpiperidin-4-yl)methanol (37 mg, 0.29 mmol), NaH (11.6 mg, 0.29 mmol) and THF (10 mL) were added to a 100 mL flask, stirred in an ice-water bath for 10 minutes. (6a*R*)*-*8-*tert-*Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (56 mg, 0.096 mmol) was added to the above reaction and stirred at room temperature for 1 hour. The mixture was quenched with aqueous ammonium chloride solution, extracted with ethyl acetate, washed with saturated brine, and the organic phase was dried and concentrated, and purified by silica gel column to obtain (6a*R*)-8-*tert*-butoxycarbonyl-1-((1-methylpiperidin-4-yl)methoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, crude product). ES-API: [M+H]⁺=591.2.

Step 2: (6a*R*)-8-*tert-*Butoxycarbonyl-1-((1-methylpiperidin-4-yl)methoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (60 mg, 0.104 mmol), 1 mL of trifluoroacetic acid and 4 mL of dichloromethane were added to a 50 mL flask. The mixture was stirred at room temperature for 1 hour, and the completion of reaction was detected by LC-MS. The reaction solution was concentrated to obtain a crude product of (6a*R*)-1-((1-methylpiperidin-4-yl)methoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (35 mg, crude product) as a yellow solid. ES-API: [M+H]⁺=491.2.

Step 3: (6a*R*)-1-((1-Methylpiperidin-4-yl)methoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (35 mg, crude product), 10 mL of dichloromethane and diisopropylethylamine (67 mg, 0.52 mmol) were added to a 50 mL flask. At 0°C, a solution of acryloyl chloride in dichloromethane (9.4 mg, 0.104 mmol, 1 mL) was added dropwise thereto. The reaction was carried out at 0°C for 10 minutes. The mixture was washed with 20 mL of water, and the organic phase was dried, concentrated and purified by preparative HPLC to obtain (6a*R*)-8-acryloyl-1-((1-methylpiperidin-4-yl)methoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z40, 6 mg, 3-step yield of 11.4%) as a white solid. ¹H NMR (500 MHz, DMSO-*d*₆) δ 8.22 (s, 1H), 7.29 (q, *J* = 8.3 Hz, 1H), 6.81-6.71 (m, 3H), 6.17 (d, *J* = 17.9 Hz, 1H), 5.75 (d, *J* = 14.3 Hz, 1H), 4.54-4.21 (m, 4H), 4.14-3.97 (m, 4H), 3.67 (d, *J* = 57.3 Hz, 4H), 2.81 (d, *J* = 11.3 Hz, 2H), 2.20 (s, 3H), 1.93 (t, *J* = 11.9 Hz, 2H), 1.78-1.64 (m, 3H), 1.34-1.27 (m, 1H).

### Embodiment 40 Synthesis of (6aR)-8-acryloyl-1-(bicyclo[1.1.1]pentan-1-ylamino)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z41

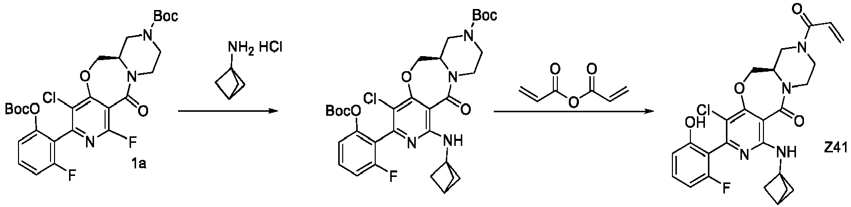

Step 1: *tert*-Butyl (6a*R*)-1-fluoro-4-chloro-3-((2-*tert*-butoxycarbonyloxy)-6-fluorophenyl)-12-oxo-6a,7,9,10-tetrahydro-12*H*-pyrazino[2,1-*c*]pyrido[3,4-*f*][1,4]oxazepine-8(6*H*)-carboxylate (50 mg, 0.086 mmol), bicyclo[1.1.1]pentan-1-amine hydrochloride (30 mg, 0.25 mmol), *N,N*-diisopropylethylamine (0.1 mL) and acetonitrile (0.5 mL) were added to a 10 mL reaction flask, and the reaction was stirred at 80°C for 1 hour. The reaction solution was extracted with ethyl acetate, washed with aqueous ammonium chloride solution and saturated brine in turn, and the organic phase was dried and concentrated to obtain *tert-*butyl (6aR)-1-(bicyclo[1.1.1]pentan-1-ylamino)-3-((2-tert-butoxycarbonyloxy)-6-fluorophenyl)-4-chloro-12-oxo-6a,7,9,10-tetrahydro-12*H*-pyrazino[2,1-*e*]pyrido[3,4-*f*][1,4]oxazepine-8(6*H*)-carboxylate (50 mg, crude product). ES-API: [M+H]⁺=645.3.

Step 2: *tert*-Butyl (6aR)-1-(bicyclo[1.1.1]pentan-1-ylamino)-3-((2-*tert*-butoxycarbonyloxy)-6-fluorophenyl)-4-chloro-12-oxo-6a,7,9,10-tetrahydro-12*H*-pyrazino[2,1-*c*]pyrido[3,4-*f*][1,4]oxazepine-8(6*H*)-carboxylate (50 mg, crude product) was dissolved in 1 mL of dichloromethane, and 1 mL of trifluoroacetic acid was added thereto. After stirring at room temperature for 2 hours, the reaction solution was concentrated. The resulting crude product was dissolved in 1.5 mL of dichloromethane, and triethylamine (110 mL, 0.79 mmol) was added thereto. The reaction was cooled to 0°C, and a solution of acrylic anhydride (8.8 mg, 0.07 mmol) in dichloromethane (0.3 mL) was added to the reaction solution. The reaction was stirred at 0°C for 15 minutes. The reaction solution was added with 30 mL of dichloromethane, washed with 15 mL of water, 15 mL of saturated aqueous NaHCO₃ solution, and 15 mL of saturated brine in turn, dried, concentrated, and purified by preparative HPLC to obtain (6aR)-8-acryloyl-1-(bicyclo[1.1.1]pentan-1-ylamino)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z41, 10.22 mg, 2-step yield of 24%) as a white solid. ¹H NMR (500 MHz, DMSO-*d*₆) *δ* 10.01-9.81 (m, 1H), 7.70-7.43 (m, 1H), 7.30-7.18 (m, 1H), 6.82-6.63 (m, 3H), 6.23-6.10 (m, 1H), 5.85-5.64 (m, 1H), 4.52-4.25 (m, 2H), 4.14 (s, 1H), 3.99-3.53 (m, 6H), 2.38 (s, 1H), 2.05-1.97 (m, 6H). ES-API: [M+H]⁺=499.1.

### Embodiment 41 Synthesis of (6aR)-8-acryloyl-4-chloro-1-((2,2-dimethyl-2,3-dihydrobenzofuran-7-yl)oxy)-3-(2-fluoro-6-hydroxyphenyl)-6,6a,7,8,9,10-hexahydro-12H-pyrazino[2,1-c]pyrido[3,4-f][1,4]oxazepin-12-one Z42

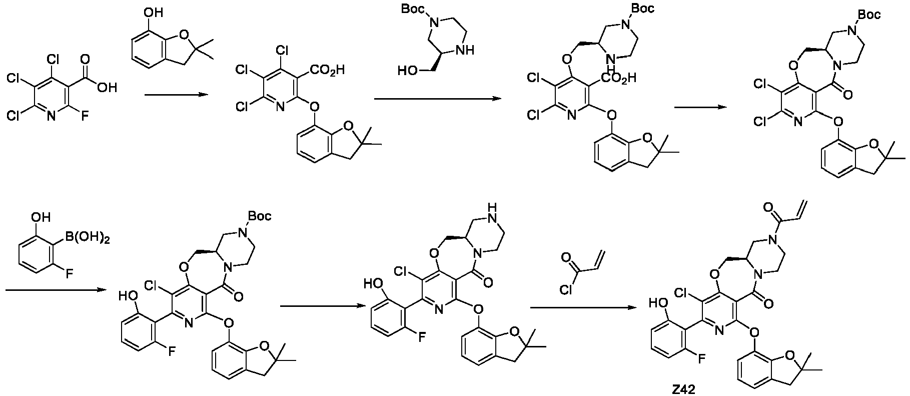

Step 1: 2,2-Dimethyl-2,3-dihydrobenzofuran-7-ol (403 mg, 2.46 mmol) was dissolved in 20 mL of *N,N*-dimethylformamide. After the system was cooled to 0°C, NaH (295 mg, 7.38 mmol) was added thereto and reacted at 0°C for 15 minutes. Then, a solution 4,5,6-trichloro-2-fluoronicotinic acid (600 mg, 2.46 mmol) in *N,N*-dimethylformamide (5 mL) was added dropwise to the reaction solution. After the dropwise addition, the reaction was carried out at room temperature for 4 hours. The reaction solution was poured into 60 mL of ice-water, and the pH of the reaction solution was adjusted to 4 with 3.0 M dilute hydrochloric acid, and the reaction solution was extracted twice with 50 mL of ethyl acetate. The organic phase was washed 4 times with 40 mL of saturated brine, dried and concentrated to obtain 4,5,6-trichloro-2-((2,2-dimethyl-2,3-dihydrobenzofuran-7-yl) oxy)nicotinic acid (1.05 g, crude product), which was directly used in the next reaction step. ES-API: [M+H]⁺=388.0.

Step 2: (*R*)-1-Boc-3-hydroxymethylpiperazine (476 mg, 2.20 mmol) was dissolved in 20 mL of THF, and NaH (264 mg, 6.60 mmol) was added thereto at 0°C, and the reaction was stirred at 0°C for 15 minutes. A solution of 4,5,6-trichloro-2-((2,2-dimethyl-2,3-dihydrobenzofuran-7-yl)oxy)nicotinic acid (950 mg, crude product) in 20 mL of THF was added dropwise to the above suspension. After the dropwise addition, the reaction was slowly heated to room temperature, and stirred at room temperature for 2 hours. The reaction solution was quenched with 20 mL of saturated ammonium chloride solution and extracted with 60 mL of ethyl acetate. The organic phase was washed with 30 mL of saturated brine, dried and concentrated to obtain (*R*)-4-((4-(*tert*-butoxycarbonyl)piperazin-2-yl)methoxy)-5,6-dichloro-2-((2,2-dimethyl-2,3-dihydrobenzofuran-7-yl)oxy)nicotinic acid (1.3 g, crude product), which was directly used in the next reaction step. ES-API: [M+H]⁺=568.2.

Step 3: (*R*)-4-((4-(tert-Butoxycarbonyl)piperazin-2-yl)methoxy)-5,6-dichloro-2-(2,2-dimethyl-2,3-dihydrobenzofuran-7-yl)oxy)nicotinic acid (1.3 g, crude product) was dissolved in 20 mL of dichloromethane, and 4 mL of diisopropylethylamine, 4 mL of propylphosphonic anhydride 50% w/w ethyl acetate solution were added thereto in turn, reacted at room temperature for 1 hour. 30 mL of dichloromethane was added to the reaction, and the organic phase was washed twice with 20 mL of water, washed with 20 mL of saturated brine in turn, dried and concentrated. The crude product was purified by flash silica gel column (EtOAc/PE: 0-40%) to obtain tert-butyl (*R*)-3,4-dichloro-1-(2,2-dimethyl-2,3-dihydrobenzofuran-7-yl)oxy)-12-oxo-6a,7,9,10-tetrahydro-12*H-*pyrazino[2,1-*c*]pyrido[3,4-*f*][1,4]oxazepine-8(6*H*)-carboxylate (320 mg, 3-step yield of 24%) as a white solid. ES-API: [M+H]⁺=550.2.

Step 4: *tert*-Butyl (*R*)-3,4-dichloro-1-(2,2-dimethyl-2,3-dihydrobenzofuran-7-yl)oxy)-12-oxo-6a,7,9,10-tetrahydro-12*H*-pyrazino[2,1-*c*]pyrido[3,4-*f*][1,4]oxazepine-8(6*H*)-carboxylate (300 mg, 0.55 mmol), (2-fluoro-6-hydroxyphenyl)boronic acid (257 mg, 1.65 mmol), tetrakis(triphenylphosphine)palladium (63 mg, 0.055 mmol), sodium carbonate (175 mg, 1.65 mmol), 3 mL of water and 15 mL of dioxane were added to a 100 mL flask. The reaction was stirred at 120°C for 2 hours under nitrogen protection. The reaction solution was added with 30 mL of water, extracted with 60 mL of ethyl acetate, and the organic phase was washed with 30 mL of saturated brine, dried and concentrated, and the crude product was purified by flash silica gel column (EtOAc/PE: 0-40%) to obtain *tert*-butyl (6a*R*)-4-chloro-1-((2,2-dimethyl-2,3-dihydrobenzofuran-7-yl)oxy)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (280 mg, 82%) as a white solid. ES-API: [M+H]⁺=626.2.

Step 5: *tert*-Butyl (6a*R*)-4-chloro-1-((2,2-dimethyl-2,3-dihydrobenzofuran-7-yl)oxy)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (280 mg, 0.45 mmol) was dissolved in 4 mL of dichloromethane, and 1 mL of trifluoroacetic acid was added thereto. After stirring at room temperature for 2 hours, the reaction solution was concentrated to obtain (6a*R*)-4-chloro-1-((2,2-dimethyl-2,3-dihydrobenzofuran-7-yl)oxy)-3-(2-fluoro-6-hydroxyphenyl)-6a,6,7,8,9,10-hexahydro-12*H*-pyrazino[3,4-*c*]pyrido[3,4-*f*][1,4]oxazepin-12-one (390 mg, crude product), which was directly used in the next reaction step. ES-API: [M+H]⁺=526.2.

Step 6: (6a*R*)-4-Chloro-1-((2,2-dimethyl-2,3-dihydrobenzofuran-7-yl)oxy)-3-(2-fluoro-6-hydroxyphenyl)-6a,6,7,8,9,10-hexahydro-12*H*-pyrazino[3,4-*c*]pyrido[3,4-*f*][1,4]oxazepin-12-one (390 mg, crude product) was dissolved in 10 mL of dichloromethane, and *N,N*-diisopropylethylamine (348 mg, 2.70 mmol) was added thereto. Acryloyl chloride (36 mg, 0.40 mmol) was added dropwise thereto at 0°C, and the reaction solution was stirred at 0°C for 15 minutes. The reaction solution was added with 30 mL of dichloromethane, washed with 15 mL of water, 15 mL of saturated aqueous NaHCO₃ solution, and 15 mL of saturated brine in turn, dried, concentrated, and purified by preparative HPLC to obtain (6aR)-8-acryloyl-4-chloro-1-((2,2-dimethyl-2,3-dihydrobenzofuran-7-yl)oxy)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z42, 145 mg, 2-step yield of 56%) as a white solid. ¹H NMR (400 MHz, DMSO-*d*₆) δ 9.39 (s, 1H), 7.27-7.18 (m, 1H), 6.95 (d, *J* = 7.2 Hz, 1H), 6.85 (t, *J* = 7.8 Hz, 1H), 6.80-6.59 (m, 4H), 6.18 (d, *J* = 16.8 Hz, 1H), 5.80-5.69 (m, 1H), 4.63-4.48 (m, 1H), 4.45-4.25 (m, 2H), 4.17-4.86 (m, 3H), 3.81-3.37 (m, 3H), 2.97 (s, 2H), 1.38-1.20 (m, 6H). ES-API: [M+H]⁺=580.0.

### Embodiment 42 Synthesis of (6aR)-8-acryloyl-4-chloro-3-(2-fluoro-6-hydroxyphenyl)-1-((1-methylpiperidin-3-yl)oxy)-6,6a,7,8,9,10-hexahydro-12H-pyrazino[2,1-c]pyrido[3,4-f][1,4]oxazepin-12-one Z43

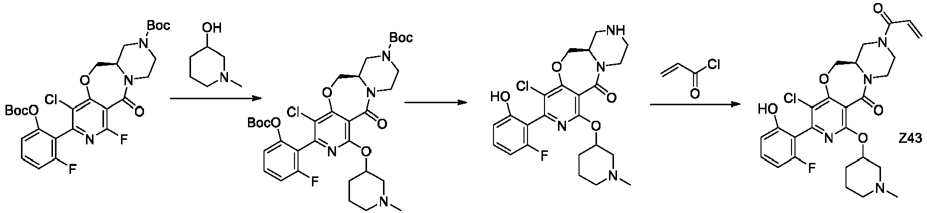

Step 1: 1-Methylpiperidin-3-ol (12 mg, 0.10 mmol) was added to a mixed solution of sodium hydride (10 mg, 0.26 mmol) in THF (3 mL) under an ice bath, and stirred for 10 minutes. Then tert-butyl (6aR)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (30 mg, 0.05 mmol) was added thereto and continued stirring for 10 minutes. The reaction solution was quenched with 10 mL of saturated ammonium chloride solution, extracted three times with 10 mL of ethyl acetate, and the organic phases were combined, dried and concentrated to obtain a crude product of *tert*-butyl (6aR)-4-chloro-3-(2-((*tert-*butoxycarbonyl)oxy)-6-fluorophenyl)-1-((1-methylpiperidin-3-yl)oxy)-12-oxo-6a,7,9,10-tetrahydro-12*H-*pyrazino[2,1-*c*]pyrido[3,4-*f*][1,4]oxazepine-8(6*H*)-carboxylate (30 mg). ES-API: [M+H]⁺=677.2.

Step 2: Under an ice bath, a solution of *tert*-butyl (6aR)-4-chloro-3-(2-((*tert*-butoxycarbonyl)oxy)-6-fluorophenyl)-1-((1-methylpiperidin-3-yl)oxy)-12-oxo-6a,7,9,10-tetrahydro-12*H*-pyrazino[2,1-*c*]pyrido[3,4-*f*][1,4]oxazepine-8(6*H*)-carboxylate (30 mg) in dichloromethane (2 mL) was added with trifluoroacetic acid (0.5 mL). The mixture was stirred at room temperature for 1 hour. After the reaction was completed, the mixture was concentrated to obtain a crude product of (6a*R*)-4-chloro-3-(2-fluoro-6-hydroxyphenyl)-1-((1-methylpiperidin-3 -yl)oxy)-6,6a,7,8,9,10-hexahydro-12*H*-pyrazino[2,1-*c*]pyrido[3,4-*f*] [1,4]oxazepin-12-one, which was directly used in the next reaction step. ES-API: [M+H]⁺=477.1.

Step 3: Under an ice bath, the crude product obtained above was dissolved in THF (2 mL), then saturated potassium carbonate solution (1 mL) was added thereto, and then acryloyl chloride (4.6 mg, 0.05 mmol) was added dropwise thereto, and the mixture was stirred for 10 minutes. The reaction solution was extracted with 2 mL of ethyl acetate, dried and concentrated, and purified by preparative HPLC to obtain (6a*R*)-8-acryloyl-4-chloro-3-(2-fluoro-6-hydroxyphenyl)-1-((1-methylpiperidin-3-yl)oxy)-6,6a,7,8,9,10-hexahydro-12*H*-pyrazino[2,1-*c*]pyrido[3,4-*f*][1,4]oxazepin-12-one (Z43, 3 mg, 3-step yield of 11%) as a white solid. ES-API: [M+H]⁺=531.1.

### Embodiment 43 Synthesis of (6aR)-8-acryloyl-4-chloro-3-(2-fluoro-6-hydroxyphenyl)-1-(5-azaspiro[2.4]heptan-5-yl)-6,6a,7,8,9,10-hexahydro-12H-pyrazino[2,1-c]pyrido[3,4-f][1,4]oxazepin-12-one Z44

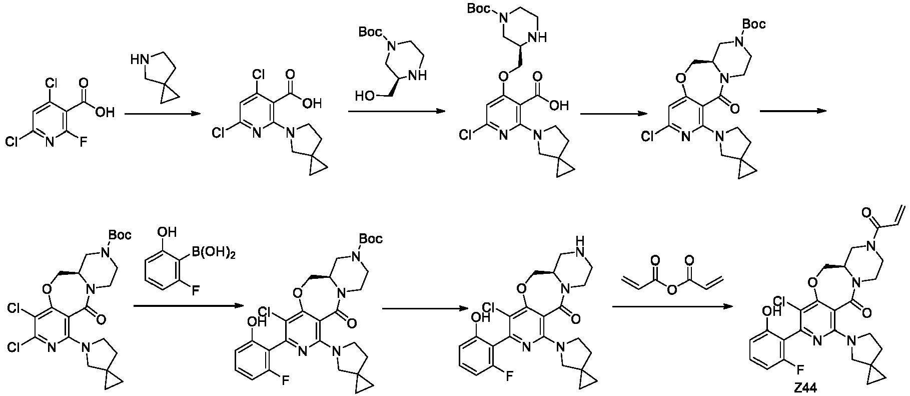

Step 1: 4,6-Dichloro-2-fluoronicotinic acid (1 g, 4.94 mmol) was dissolved in acetonitrile (15 mL), then 5-azaspiro[2.4]heptane (480 mg, 4.94 mmol) and *N,N*-diisopropylethylamine (1.3 g, 9.9 mmol) were added thereto and reacted for 2 hours at room temperature. After the reaction was completed, the reaction was quenched by adding water. The pH of the mixture was adjusted to 3 with 1 N HCl, and the mixture was extracted with ethyl acetate, dried and evaporated to dryness to obtain 4,6-dichloro-2-(5-azaspiro[2.4]heptan-5-yl)nicotinic acid (1.3 g, crude). ES-API: [M+H]⁺=287.0.

Step 2: *tert*-Butyl (*R*)-3-(hydroxymethyl)piperazine-1-carboxylate (2.8 g, 13.1 mmol) was dissolved in THF (15 mL), cooled to 0 to 5°C, and sodium hydride (1.4 g, 34.8 mmol) was added thereto. After stirring under an ice bath for 10 minutes, a solution of 4,6-dichloro-2-(5-azaspiro[2.4]heptan-5-yl)nicotinic acid (1.25 g, 4.36 mmol) in THF (5 mL) was then added thereto. The reaction solution was heated to 60°C and reacted for 6 hours. After the reaction was completed, the reaction was quenched by adding saturated ammonium chloride. The pH of the mixture was adjusted to 3 with 1 N HCl, and the mixture was extracted with ethyl acetate, dried and evaporated to dryness to obtain (*R*)-4-((4-(*tert-*butoxycarbonyl)piperazin-2-yl)methoxy)-6-chloro-2-(5-azaspiro[2.4]heptan-5-yl)nicotinic acid (3 g, crude product). ES-API: [M+H]⁺=467.1.

Step 3: (*R*)-4-((4-(*tert*-Butoxycarbonyl)piperazin-2-yl)methoxy)-6-chloro-2-(5-azaspiro[2.4]heptan-5-yl)nicotinic acid (2.2 g, 4.71 mmol) was dissolved in dichloromethane (240 mL), and HATU (2.1 g, 5.65 mmol) and *N,N*-diisopropylethylamine (1.8 g, 14.1 mmol) were added thereto. The reaction solution was reacted at room temperature for 2 hours. After the reaction was completed, water and dichloromethane were added for extraction. The organic phase was dried, concentrated and purified by column chromatography (PE/EtOAc=2/1, R_{f}=0.5) to obtain a product (560 mg, 27%, yellow solid). ES-API: [M+H]⁺=449.2.

Step 4: *tert*-Butyl (R)-3-chloro-12-oxo-1-(5-azaspiro[2.4]heptan-5-yl)-6a,7,9,10-tetrahydro-12H-pyrazino[2,1-*c*]pyrido[3,4-*f*][1,4]oxazepine-8(6*H*)-carboxylate (450 mg, 1.0 mmol) was dissolved in 5 mL of acetonitrile, and N-chlorosuccinimide (133 mg, 1.0 mmol) was added thereto. The reaction solution was heated to 35°C and reacted for 4 hours. After the reaction was completed, water and ethyl acetate were added for extraction. The organic phase was dried, concentrated and purified by column chromatography (PE/EtOAc=2/1, R_{f}=0.5) to obtain a product (330 mg, 68%, white solid). ES-API: [M+H]⁺=483.2.

Step 5: *tert*-Butyl (R)-3,4-dichloro-12-oxo-1-(5-azaspiro[2.4]heptan-5-yl)-6a,7,9,10-tetrahydro-12*H*-pyrazino[2,1-*c*]pyrido[3,4-*f*][1,4]oxazepine-8(6*H*)-carboxylate (200 mg, 0.41 mmol) was dissolved in 1,4-dioxane/water (3 mL/0.6 mL), and then (2-fluoro-6-hydroxyphenyl)boronic acid (129 mg, 0.83 mmol), tetrakis(triphenylphosphine)palladium (49 mg, 0.04 mmol) and sodium carbonate (132 mg, 1.24 mmol) were added thereto. The reaction solution was heated to 105°C and reacted for 7 hours. After the reaction was completed, brine and ethyl acetate were added for extraction. The organic phase was dried, concentrated and purified by column chromatography (PE/EtOAc=1/1, R_{f}=0.4) to obtain a product (260 mg, crude product, yellow oil). ES-API: [M+H]⁺=559.2.

Step 6: *tert*-Butyl (6aR)-4-chloro-3-(2-fluoro-6-hydroxyphenyl)-12-oxo-1-(5-azaspiro[2.4]heptan-5-yl)-6a,7,9,10-tetrahydro-12*H*-pyrazino[2,1-*c*]pyrido[3,4-*f*][1,4]oxazepine-8(6*H*)-carboxylate (260 mg, 0.46 mmol) was dissolved in dichloromethane (1 mL) and trifluoroacetic acid (1 mL) was added thereto. The reaction solution was reacted at room temperature for 1 hour. After TLC monitoring that the reaction was completed, the reaction solution was evaporated to dryness, dissolved in dichloromethane and evaporated to dryness to obtain a crude product, which was directly used in the next reaction step.

Step 7: (6a*R*)-4-Chloro-3-(2-fluoro-6-hydroxyphenyl)-1-(5-azaspiro[2.4]heptan-5-yl)-7,8,9,10-hexahydro-12*H*-pyrazino[2,1-*c*]pyrido[3,4-*f*][1,4]oxazepin-12-one (crude product, 0.46 mmol) was dissolved in dichloromethane (2 mL) and cooled to 0 to 5°C, and triethylamine (2 mL) and acrylic anhydride (47 mg, 0.37 mmol) were added dropwise thereto. The reaction solution was reacted in an ice bath for 0.5 hours. After the reaction was completed, the mixture was extracted with dichloromethane and water, and the organic phase was dried, evaporated to dryness by rotary evaporation, and purified by preparative HPLC to obtain (6a*R*)-8-acryloyl-4-chloro-3-(2-fluoro-6-hydroxyphenyl)-1-(5-azaspiro[2.4]heptan-5-yl)-6,6a,7,8,9,10-hexahydro-12*H*-pyrazino[2,1-c]pyrido[3,4*f*][1,4]oxazepin-12-one (Z44, 32.4 mg, 14%, white solid). ES-API: [M+H]⁺=513.1. ¹H NMR (400 MHz, CDCl₃) δ 7.29-7.25 (m, 1H), 6.81 (d, *J=* 8.3 Hz, 1H), 6.68 (t, *J* = 9.1 Hz, 1H), 6.62-6.50 (m, 1H), 6.40 (d, *J* = 16.5 Hz, 1H), 5.81 (d, *J* = 10.3 Hz, 1H), 4.40 (s, 3H), 4.28-3.95 (m, 3H), 3.87-3.61 (m, 4H), 3.53 (d, *J* = 56.5 Hz, 1H), 3.35-3.23 (m, 1H), 2.82 (d, *J* = 10.5 Hz, 1H), 2.20 (q, *J=* 10.4 Hz, 1H), 1.55-1.47 (m, 1H), 0.73-0.63 (m, 2H), 0.58-0.50 (m, 2H).

### Embodiment 44 Synthesis of (6aR)-8-acryloyl-4-chloro-3-(2-fluoro-6-hydroxyphenyl)-1-(2-oxa-6-azaspiro[3.4]octan-6-yl)-6,6a,7,8,9,10-hexahydro-12H-pyrazino[2,1-c]pyrido[3,4-f][1,4]oxazepin-12(6aH)-one Z45

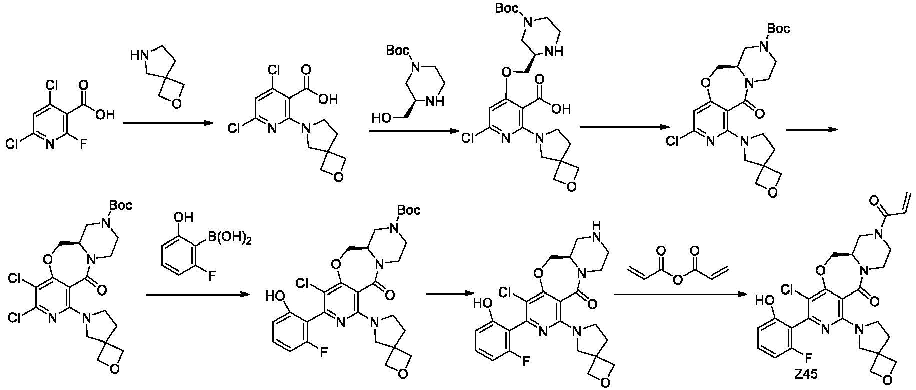

Step 1: 4,6-Dichloro-2-fluoronicotinic acid (1.06 g, 5.07 mmol) was dissolved in 25 mL of acetonitrile, and *N,N*-diisopropylethylamine (1.63 g, 12.66 mmol) and 2-oxa-6-azaspiro[3.4]octane (477 mg, 4.22 mmol) were added thereto. The reaction solution was stirred at room temperature overnight. The pH of the reaction solution was adjusted to 6-7 with dilute hydrochloric acid, and the reaction solution was extracted with ethyl acetate. The organic phase was concentrated to obtain 4,6-dichloro-2-(2-oxa-6-azaspiro[3.4]octan-6-yl)nicotinic acid (1.6 g, crude product, a brown liquid), which was directly used in the next reaction step. ES-API: [M+H]⁺=303.0.

Step 2: Sodium hydride (871 mg, 36.3 mmol) was suspended in 10 mL of THF, and a solution of *tert*-butyl (*R*)-3-(hydroxymethyl)piperazine-1-carboxylate (1.17 g, 5.45 mmol) in THF was added thereto at 0°C. The mixture was stirred and reacted for 30 minutes while maintaining the temperature. A solution of 4,6-dichloro-2-(2-oxa-6-azaspiro[3.4]octan-6-yl)nicotinic acid (1.1 g, 3.63 mmol) in THF (5 mL) was added dropwise thereto. The reaction solution was stirred and reacted at 60°C overnight. The reaction solution was quenched with saturated ammonium chloride solution, extracted with ethyl acetate, and the organic phase was concentrated, and the crude product was purified by flash silica gel column to obtain (*R*)-4-((4-(*tert*-butoxycarbonyl)piperazin-2-yl)methoxy)-6-chloro-2-(2-oxa-6-azaspiro[3.4]octan-6-yl)nicotinic acid (800 mg, 46%, a brown solid). ES-API: [M+H]⁺=483.1.

Step 3: (*R*)-4-((4-(*tert*-Butoxycarbonyl)piperazin-2-yl)methoxy)-6-chloro-2-(2-oxa-6-azaspiro[3.4]octan-6-yl)nicotinic acid (650 mg, 1.34 mmol) was dissolved in 50 mL of dichloromethane, and *N,N*-diisopropylethylamine (518 mg, 4.02 mmol) and HATU (508 mg, 1.6 mmol) were added thereto. The mixture was stirred and reacted overnight at room temperature. The reaction solution was washed with brine, extracted with dichloromethane, and the organic phase was concentrated, and the crude product was purified by flash silica gel column (PE/EtOAc=2/1, R_{f}=0.6) to obtain *tert*-butyl (R)-3-chloro-12-oxo-1-(2-oxa-6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (200 mg, 20%, a pale yellow solid). ES-API: [M+H]⁺=465.2.

Step 4: *tert*-Butyl (*R*)-3-chloro-12-oxo-1-(2-oxa-6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (160 mg, 0.34 mmol) was dissolved in 10 mL of acetonitrile, and *N*-chlorosuccinimide (46 mg, 0.34 mmol) was added thereto. The mixture was stirred and reacted at 50°C for 5 hours. The reaction solution was extracted with dichloromethane, and the organic phase was concentrated, and the crude product was purified by flash silica gel column (PE/EtOAc=1/1, R_{f}=0.3) to obtain *tert*-butyl (R)-3,4-dichloro-12-oxo-1-(2-oxa-6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (130 mg, 67%, a pale yellow solid). ES-API: [M+H]⁺=499.1.

Step 5: *tert*-Butyl (R)-3,4-dichloro-12-oxo-1-(2-oxa-6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (80 mg, 0.16 mmol) was dissolved in dioxane/water (1 mL/0.2 mL), and sodium carbonate (50 mg, 0.48 mmol), (2-fluoro-6-hydroxyphenyl)boronic acid (75 mg, 0.48 mmol) and tetrakis(triphenylphosphine)palladium (18 mg, 0.02 mmol) were added thereto. The mixture was stirred and reacted at 100°C overnight under nitrogen protection. The reaction solution was extracted with ethyl acetate, washed with brine, and the organic phase was concentrated, and the crude product was purified by chromatography plate (PE/EtOAc=1/1, R_{f}=0.2) to obtain *tert*-butyl (6a*R*)-4-chloro-3-(2-fluoro-6-hydroxyphenyl)-12-oxo-1-(2-oxa-6-azaspiro[3.4]octan-6-yl)-6a,7,9,10-tetrahydro-6*H*-pyrazino[2,1-*c*]pyrido[3,4-*f*][1,4]oxazepine-8(12*H*)-carboxylate (80 mg, 53%, a pale yellow solid). ES-API: [M+H]⁺=575.2.

Step 6: *tert*-Butyl (6a*R*)-4-chloro-3-(2-fluoro-6-hydroxyphenyl)-12-oxo-1-(2-oxa-6-azaspiro[3.4]octan-6-yl)-6a,7,9,10-tetrahydro-6*H*-pyrazino[2,1-*c*]pyrido[3,4-*f*][1,4]oxazepine-8(12*H-*carboxylate (80 mg, 0.14 mmol) was dissolved in 1 mL of dichloromethane, and 1 mL of trifluoroacetic acid was added thereto. The mixture was stirred and reacted at room temperature for 1 hour. The reaction solution was concentrated and redissolved by adding dichloromethane, and concentrated to obtain (6a*R*)-4-chloro-3-(2-fluoro-6-hydroxyphenyl)-1-(2-oxa-6-azaspiro[3.4]octan-6-yl)-6,6a,7,8,9,10-hexahydro-12*H-*pyrazino[2,1-*c*]pyrido[3,4-*f*][1,4]oxazepin-12-one (100 mg, crude product). The crude product was directly used in the next reaction step. ES-API: [M+H]⁺=475.1.

Step 7: (6a*R*)-4-Chloro-3-(2-fluoro-6-hydroxyphenyl)-1-(2-oxa-6-azaspiro[3.4]octan-6-yl)-6,6a,7,8,9,10-hexahydro-12*H*-pyrazino[2,1-*c*]pyrido[3,4-*f*][1,4]oxazepin-12-one (35 mg, 0.074 mmol) was dissolved in 0.5 mL of dichloromethane, and 0.5 mL of triethylamine was added thereto. Acrylic anhydride (7.9 mg, 0.063 mmol) was added thereto. The mixture was stirred and reacted at room temperature for 1 hour. The reaction solution was extracted with dichloromethane, washed with brine, and the organic phase was concentrated, and the crude product was purified by chromatography plate (DCM/MeOH=20/1, R_{f}=0.4) to obtain (6a*R*)-8-acryloyl-4-chloro-3-(2-fluoro-6-hydroxyphenyl)-1-(2-oxa-6-azaspiro[3.4]octan-6-yl)-6,6a,7,8,9,10-hexahydro-12*H*-pyrazino[2,1-*c*]pyrido[3,4-*f*][1,4]oxazepin-12-one (Z45, 7.5 mg, 10%) as a white solid. ES-API: [M+H]⁺=529.2. ¹H NMR (400 MHz, CDCl₃) δ 7.29 (d, *J* = 8.3 Hz, 1H), 6.82 (d, *J* = 8.2 Hz, 1H), 6.69 (t, *J* = 9.0 Hz, 1H), 6.56 (s, 1H), 6.41 (d, *J* = 16.3 Hz, 1H), 5.82 (d, *J* = 10.0 Hz, 1H), 4.81-4.52 (m, 4H), 4.51-3.93 (m, 6H), 3.89-3.39 (m, 6H), 3.31 (s, 1H), 2.41 (s, 1H), 2.17-1.92 (m, 2H).

### Embodiment 45 Synthesis of (6aR)-8-acryloyl-4-chloro-3-(2-fluoro-6-hydroxyphenyl)-1-(((1R,5S,6s)-3-methyl-3-azabicyclo[3.1.0]hexan-6-yl)amino)-6,6a,7,8,9,10-hexahydro-12H-pyrazino[2,1-c]pyrido[3,4f][1,4]oxazepin-12-one Z46

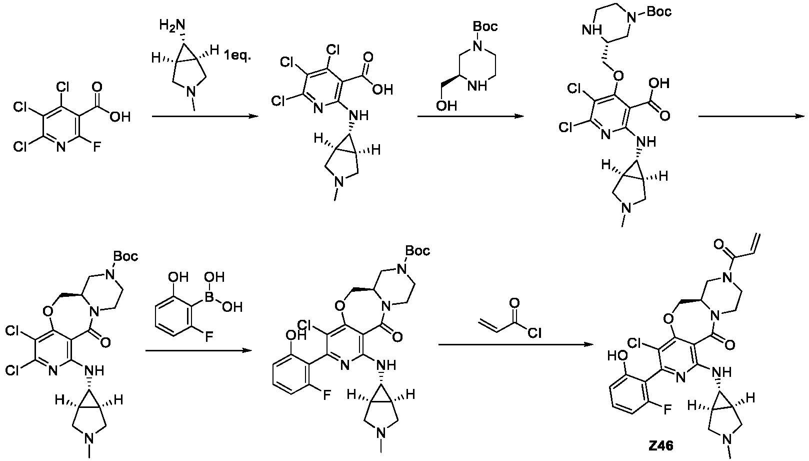

Step 1: 4,5,6-Trichloro-2-fluoronicotinic acid (283 mg, 1.16 mmol), (1R,5S,6s)-3-methyl-3-azabicyclo[3.1.0]hexan-6-amine (130 mg, 1.16 mmol), *N,N*-diisopropylethylamine (449 mg, 3.48 mmol) and 10 mL of acetonitrile were added to a 100 mL flask. The reaction was stirred at 75°C for 4 hours. The reaction was directly concentrated. The crude product was purified by flash silica gel column (MeOH/DCM: 10-100%) to obtain 4,5,6-trichloro-2-(((1*R*,5*S*,6*s*)-3-methyl-3-azabicyclo[3.1.0]hexan-6-yl)amino)nicotinic acid (250 mg, 64%) as a yellow solid. ES-API: [M+H]⁺=336.0.

Step 2: 60% sodium hydride (118 mg, 2.96 mmol), *tert*-butyl (*R*)-3-(hydroxymethyl)piperazine-1-carboxylate (240 mg, 1.11 mmol), THF (8 mL) and DMF (1 mL) were added to a 100 mL flask at 0°C. A solution of 4,5,6-trichloro-2-(((1*R*,5*S*,6*s*)-3-methyl-3-azabicyclo[3.1.0]hexan-6-yl)amino)nicotinic acid (250 mg, 0.74 mmol) in THF was added dropwise thereto. The reaction was carried out in an oil bath at 60°C for 30 minutes. The completion of reaction was detected by LC-MS. The reaction solution was poured into ice-water. The pH of the reaction solution was adjusted to 8 with 1 M hydrochloric acid. The reaction solution was purified by reverse phase column (acetonitrile/water: 0-30%) to obtain 4-(((*R*)-4-(*tert-*butoxycarbonyl)piperazin-2-yl)methoxy)-5,6-dichloro-2-(((1*R*,5*S*,6*s*)-3-methyl-3-azabicyclo[3.1.0]hexan-6-yl)amino)nicotinic acid (380 mg, crude product) as a yellow oil. ES-API: [M+H]⁺=516.1.

Step 3: 4-(((*R*)-4-(tert-Butoxycarbonyl)piperazin-2-yl)methoxy)-5,6-dichloro-2-(((1R,SS,6s)-3-methyl-3-azabicyclo[3.1.0]hexan-6-yl)amino)nicotinic acid (380 mg, crude product), *N,N-*diisopropylethylamine (3 mL) and dichloromethane (10 mL) were added to a 100 mL flask. T₃P (3 mL, 50%w/w ethyl acetate solution) was added dropwise thereto. The mixture was reacted at room temperature for 1 hour. The completion of reaction was detected by LC-MS. 50 mL of dichloromethane was added to the reaction, then washed with 30 mL of water, and 30 mL of saturated aqueous sodium bicarbonate, respectively. The organic phase was dried, concentrated and then purified by flash silica gel column (MeOH/DCM: 10-50%) to obtain *tert*-butyl (R)-3,4-dichloro-1-(((1R,SS,6s)-3-methyl-3-azabicyclo [3 .1. 0]hexan-6-yl)amino)-12-oxo-6a,7,9,10-tetrahydro-12*H*-pyrazino[2,1-*c*]pyrido[3,4-*f*][1,4]oxazepine-8(6*H*)-carboxylate (90 mg, 2-step yield of 24%,) as a yellow oil. ES-API: [M+H]⁺=498.1.

Step 4: *tert*-Butyl (*R*)-3,4-dichloro-1-(((1*R*,5*S*,6*s*)-3-methyl-3-azabicyclo[3.1.0]hexan-6-yl)amino)-12-oxo-6a,7,9,10-tetrahydro-12*H*-pyrazino[2,1-*c*]pyrido[3,4-*f*][1,4]oxazepine-8(6*H*)-carboxylate (90 mg, 0.18 mmol), 2-fluoro-6-hydroxyphenylboronic acid (112 mg, 0.72 mmol), Pd(PPh₃)₄ (21 mg, 0.018 mmol), sodium carbonate (57 mg, 0.54 mmol), 3 mL of dioxane and 0.6 mL of water were added to a 100 mL reaction flask. The reaction was stirred for 30 minutes at 110°C under microwave irradiation, and the reaction was stopped. The reaction solution was added with 20 mL of water, extracted with ethyl acetate, and the organic phase was dried and concentrated to obtain *tert*-butyl (6a*R*)-4-chloro-3-(2-fluoro-6-hydroxyphenyl)-1-(((1R,SS,6s)-3-methyl-3-azabicyclo[3.1.0]hexan-6-yl)amino)-12-oxo-6a,7,9,10-tetrahydro-12*H*-pyrazino[2,1-*c*]pyrido[3,4-*f*][1,4]oxazepine-8(6*H*)-carboxylate (103 mg, crude product) as a yellow solid. ES-API: [M+H]⁺=574.2.

Step 5: *tert*-Butyl (6aR)-4-chloro-3-(2-fluoro-6-hydroxyphenyl)-1-(((1R,SS,6s)-3-methyl-3-azabicyclo [3 .1. 0]hexan-6-yl)amino)-12-oxo-6a,7,9,10-tetrahydro-12*H*-pyrazino[2,1-*c*]pyrido[3,4-*f*][1,4]oxazepine-8(6*H*)-carboxylate (103 mg, crude product), trifluoroacetic acid (1 mL) and dichloromethane (0.5 mL) were added to a 100 mL reaction flask. After stirring at room temperature for 1 hour, the reaction solution was concentrated to obtain a yellow solid. The yellow solid was dissolved in 3 mL of THF and a solution of sodium carbonate (191 mg, 1.8 mmol) in water (1 mL). Acryloyl chloride (13 mg, 0.14 mmol) was added to the reaction solution. The reaction solution was stirred at room temperature for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-3-(2-fluoro-6-hydroxyphenyl)-1-(((1*R*,5*S*,6*s*)-3-methyl-3-azabicyclo[3.1.0]hexan-6-yl)amino)-6,6a,7,8,9,10-hexahydro-12*H*-pyrazino[2,1-*c*]pyrido[3,4-*f*][1,4]oxazepin-12-one (Z46, 8 mg, 2-step yield of 8%) as a white solid. ES-API: [M+H]⁺=528.1.

### Embodiment 46-1 Synthesis of (6aR)-8-acryloyl-4-chloro-3-(2-fluoro-6-hydroxyphenyl)-1-(5-methylhexahydropyrrolo[3,4-b]pyrrol-1(2H)-yl)-6,6a,7,8,9,10-hexahydro-12H-pyrazino[2,1-c]pyrido[3,4-f][1,4]oxazepin-12-one Z47

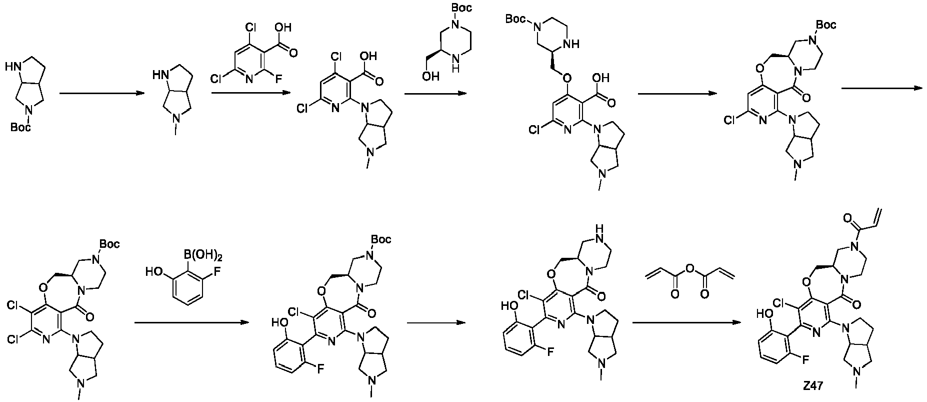

Step 1: *tert*-Butyl hexahydropyrrolo[3,4-*b*]pyrrole-5(1*H*)-carboxylate (2.0 g, 9.4 mmol) and anhydrous tetrahydrofuran (30 mL) were added to a round-bottom flask, and then lithium aluminum hydride (893 mg, 23.5 mmol) was slowly added thereto under an ice-water bath, and the reaction was stirred at 80°C overnight. After the reaction was completed, the temperature of the reaction was restored to room temperature, then water (0.9 mL) and sodium hydroxide solution (2.5 mL, 15%w/w aqueous solution) were slowly added dropwise thereto under an ice-water bath, and then water (0.9 mL) was added thereto. After stirring at 23°C for 30 minutes, the mixture was filtered, and the filter cake was washed with tetrahydrofuran, and the filtrate was evaporated to dryness by rotary evaporation to obtain a product of 5-methyloctahydropyrrolo[3,4-*b*]pyrrole (1.6 g, crude product) as a colorless oily liquid.

Step 2: 4,6-Dichloro-2-fluoronicotinic acid (1.0 g, 4.8 mmol) and 50 mL of acetonitrile were added to a round bottom flask. Diisopropylethylamine (1.2 g, 9.6 mmol) and 5-methyloctahydropyrrolo[3,4-*b*]pyrrole (1.2 g, 9.6 mmol) were then added to the reaction solution. The reaction was stirred at 90°C for 2 hours. After the reaction was completed, the organic phase was concentrated to dryness to obtain a crude product of 4,6-dichloro-2-(5-methylhexahydropyrrolo[3,4-*b*]pyrrol-1(2*H*)-yl)nicotinic acid (1.2 g, crude product) as a yellow solid, and the crude product were directly used in the next reaction step. ES-API: [M+H]⁺=317.2.

Step 3: tert-Butyl (*R*)-3-(hydroxymethyl)piperazine-1-carboxylate (466 mg, 3.7 mmol) was added to a suspension of 60% sodium hydride (592 mg, 14.8 mmol) in tetrahydrofuran (50 mL) at 0°C. Then, a solution of 4,6-dichloro-2-(5-methylhexahydropyrrolo[3,4-*b*]pyrrol-1(2*H*)-yl)nicotinic acid (1.2 g, 3.80 mmol) in tetrahydrofuran (20 mL) was added dropwise thereto. The reaction was stirred at 0°C for 30 minutes. The completion of reaction was detected by LC-MS. The reaction solution was concentrated, and the crude product was purified by flash silica gel column (methanol/dichloromethane: 20-100%) to obtain 4-(((*R*)-4-(*tert*-butoxycarbonyl)piperazin-2-yl)methoxy)-6-chloro-2-(5-methylhexahydropyrrolo[3,4-*b*]pyrrol-1(2*H*)-yl)nicotinic acid (1.2 g, 65%) as a yellow solid. ES-API: [M+H]⁺=497.1.

Step 4: 4-(((R)-4-(*tert*-Butoxycarbonyl)piperazin-2-yl)methoxy)-6-chloro-2-(5-methylhexahydropyrrolo[3,4-*b*]pyrrol-1(2*H*)-yl)nicotinic acid (700 mg, 1.4 mmol), diisopropylethylamine (10 mL) and dichloromethane (30 mL) were added to a round bottom flask. Propylphosphonic anhydride solution (10 mL, 50%w/w ethyl acetate solution) was added dropwise thereto. The reaction was stirred at room temperature for 30 minutes. The completion of reaction was detected by LC-MS. 50 mL of dichloromethane was added to the reaction, and the organic phase was washed with 30 mL of hydrochloric acid (1 M) and 100 mL of saturated aqueous sodium bicarbonate solution in turn. The organic phase was dried and concentrated, and the crude product was purified by flash silica gel column (methanol/dichloromethane: 0-3%) to obtain *tert*-butyl (6a*R*)-3-chloro-1-(5-methylhexahydropyrrolo[3,4-*b*]pyrrol-1(2*H*)-yl)-12-oxo-6a,7,9,10-tetrahydro-12*H*-pyrazino[2,1-*c*]pyrido[3,4-*f*][1,4]oxazepine-8(6*H*)-carboxylate (387 mg, 58%). ES-API: [M+H]⁺=478.1.

Step 5: *tert*-Butyl (6a*R*)-3-chloro-1-(5-methylhexahydropyrrolo[3,4-*b*]pyrrol-1(2*H*)-yl)-12-oxo-6a,7,9,10-tetrahydro-12*H*-pyrazino[2,1-*c*]pyrazino[3,4-*f*][1,4]oxazepine-8(6*H*)-carboxylate (387 mg, 0.81 mmol), *N*-chlorosuccinimide (130 mg, 0.97 mmol) and acetonitrile (30 mL) were added to a round bottom flask. The reaction was stirred at 70°C for 1 hour. The reaction solution was added with 50 mL of water and extracted with ethyl acetate. The organic phase was dried and concentrated, and the crude product was purified by flash silica gel column (methanol/dichloromethane: 0-3%) to obtain a product of *tert*-butyl (6a*R*)-3,4-dichloro-1 -(5 -methylhexahydropyrrolo [3,4-*b*]pyrrol-1(2*H*)-yl)-12-oxo-6a,7,9,10-tetrahydro-12*H-*pyrazino[2,1-*c*]pyrido[3,4-*f*][1,4]oxazepine-8(6*H*)-carboxylate (351 mg, 85%) as a pale yellow solid. ES-API: [M+H]⁺=512.1.

Step 6: *tert*-Butyl (6a*R*)-3,4-dichloro-1-(5-methylhexahydropyrrolo[3,4-*b*]pyrrol-1(2*H*)-yl)-12-oxo-6a,7,9,10-tetrahydro-12*H*-pyrazino[2,1-*c*]pyrido[3,4-*f*][1,4]oxazepine-8(6*H*)-carboxylate (351 mg, 0.69 mmol), (2-fluoro-6-hydroxyphenyl)boronic acid (328 mg, 2.1 mmol), tetrakis(triphenylphosphine)palladium (80 mg, 0.069 mmol), sodium carbonate (223 mg, 2.1 mmol), 15 mL of dioxane and 3 mL of water were added to a 100 mL reaction flask. The reaction was stirred at 110°C for 2.5 hours under nitrogen protection, and the reaction was stopped. The reaction solution was added with 20 mL of water, extracted 3 times with 20 mL of ethyl acetate. The organic phase was dried and concentrated, and the crude product was purified by flash silica gel column (ethyl acetate/petroleum ether: 0-100%) to obtain a product of *tert*-butyl (6a*R*)-4-chloro-3-(2-fluoro-6-hydroxyphenyl)-1-(5-methylhexahydropyrrolo[3,4-*b*]pyrrol-1(2*H*)-yl)-12-oxo-6a,7,9,10-tetrahydro-12*H*-pyrazino[2,1-*c*]pyrido[3,4-*f*][1,4]oxazepine-8(6*H*)-carboxylate (372 mg, 92%) as a yellow solid. ES-API: [M+H]⁺= 588.2

Step 7: *tert*-Butyl (6aR)-4-chloro-3-(2-fluoro-6-hydroxyphenyl)-1-(5-methylhexahydropyrrolo[3,4-*b*]pyrrol-1(2*H*)-yl)-12-oxo-6a,7,9,10-tetrahydro-12*H*-pyrazino[2,1-*c*]pyrido[3,4-*f*][1,4]oxazepine-8(6*H*)-carboxylate (372 mg, 0.63 mmol), 3 mL of methanol and 6 mL of hydrogen chloride/dioxane solution (4 M) were added to a round bottom flask. The mixture was stirred at room temperature for 1 hour, and the completion of reaction was detected by LC-MS. The reaction solution was concentrated to obtain (6a*R*)-4-chloro-3-(2-fluoro-6-hydroxyphenyl)-1-(5-methylhexahydropyrrolo[3,4-*b*]pyrrol-1(2*H*)*-*yl)-6,6a,7,8,9,10-hexahydro-12*H*-pyrazino[2,1-*c*]pyrido[3,4-*f*][1,4]oxazepin-12-one (391 mg) as a yellow solid. The crude product was directly used in the next step. ES-API: [M+H]⁺=488.2.

Step 8: (6aR)-4-Chloro-3-(2-fluoro-6-hydroxyphenyl)-1-(5-methylhexahydropyrrolo[3,4-*b*]pyrrol-1(2*H*)-yl)-6,6a,7,8,9,10-hexahydro-12*H*-pyrazino[2,1-*c*]pyrido[3,4-*f*][1,4]oxazepin-12-one (391 mg, 0.80 mmol), 10 mL of dichloromethane and triethylamine (627 mg, 6.0 mmol) were added to a round bottom flask. The reaction was cooled to 0°C, and a solution of acrylic anhydride in dichloromethane (80 mg, 0.64 mmol, 2 mL) was added dropwise to the reaction solution. The reaction was stirred at 0°C for 10 minutes. 40 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-3-(2-fluoro-6-hydroxyphenyl)-1-(5-methylhexahydropyrrolo[3,4-*b*]pyrrol-1(2*H*)-yl)-6,6a,7,8,9,10-hexahydro-12*H*-pyrazino[2,1-*c*]pyrido[3,4-*f*][1,4]oxazepin-12-one (Z47, 65 mg, 15%). ES-API: [M+H]⁺=542.2. ¹H NMR (500 MHz, DMSO-*d*₆) δ 9.96 (d, *J=* 21.3 Hz, 1H), 7.24 (dd, *J* = 15.7, 7.9 Hz, 1H), 6.87-6.60 (m, 3H), 6.17 (d, *J* = 18.1 Hz, 1H), 5.74 (s, 1H), 4.64-4.26 (m, 3H), 4.21-3.93 (m, 4H), 3.80-3.58 (m, 1H), 3.53-3.44 (m, 3H), 3.22-3.01 (m, 1H), 2.80 (d, *J* = 40.6 Hz, 2H), 2.45-2.30 (m, 2H), 2.16 (d, *J=* 16.5 Hz, 3H), 2.01-1.96 (m, 1H), 1.91-1.86 (m, 1H), 1.81-1.69 (m, 1H).

### Embodiment 46-2 Synthesis of (6aR)-8-acryloyl-1-((R)-2-methylpyrrolidin-1-yl)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z48

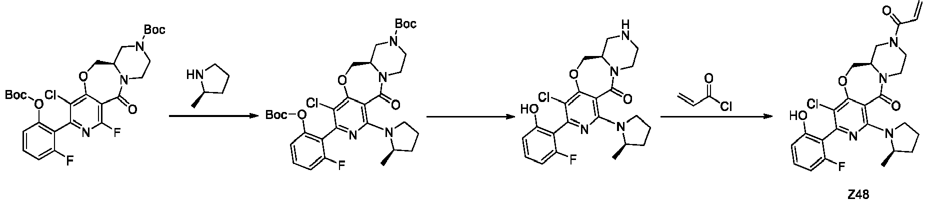

Step 1: (*R*)-2-Methylpyrrolidine (14.6 mg, 0.172 mmol), *N,N*-diisopropylethylamine (33 mg, 0.258 mmol) and acetonitrile (10 mL) were added to a 100 mL round bottom flask. (6*aR)*-8-*tert-*Butoxycarbonyl-3-(2-((*tert*-butoxycarbonyl)oxy)-6-fluorophenyl)-4-chloro-1-fluoro-6,6a,7,8,9,10-hexahydro-12H-pyrazino[2,1-c]pyrido[3,4-*f*][1,4]oxazepin-12-one (intermediate 1a, 50 mg, 0.086 mmol) was added to the above reaction and stirred at 60°C for 1 hour. The mixture was extracted with ethyl acetate, washed with saturated brine, and the organic phase was dried, concentrated, and purified by silica gel column (petroleum ether:ethyl acetate = 20:80) to obtain (6aR)-8-*tert*-butoxycarbonyl-1-((R)-2-methylpyrrolidin-1-yl)-4-chloro-3-(2-fluoro-6-hydroxyphenyl)-6,6a,7, 8,9,10-hexahydro-12H-pyrazino [2,1-*c*]pyrido [3,4-*f*][1,4]oxazepin-12-one (55 mg, crude product). ES-API: [M+H]⁺=647.2.

Step 2: (6aR)-8-*tert*-Butoxycarbonyl-1-((R)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (55 mg, 0.085 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6aR)-1-((R)-2-methylpyrrolidin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (38 mg, crude product) as a yellow solid. ES-API: [M+H]⁺=447.2.

Step 3: (6aR)-1-((R)-2-Methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (38 mg, crude product), 10 mL of dichloromethane and *N,N*-diisopropylethylamine (55 mg, 0.425 mmol) were added to a 50 mL round bottom flask. The reaction was cooled to 0°C, and a solution of acryloyl chloride in dichloromethane (7 mg, 0.0766 mmol) was added dropwise to the reaction solution. The reaction was stirred at 0°C for 10 minutes. The mixture was washed with 20 mL of water, and the organic phase was dried, then concentrated and the crude product was purified by preparative HPLC (alkali method) to obtain (6aR)-8-acryloyl-1-((R)-2-methylpyrrolidin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z48, 20 mg, 3-step yield of 46%) as a white solid. ES-API: [M+H]⁺= 501.1. ¹H NMR (400 MHz, DMSO-*d*₆) δ 9.93 (s, 1H), 7.27-7.21 (m, 1H), 6.76-6.67 (m, 3H), 6.17 (d, *J* = 16.7 Hz, 1H), 5.75 (d, *J =* 10.2 Hz, 1H), 4.49 (s, 1H), 4.30 (s, 1H), 4.19 - 3.99 (m, 4H), 3.88-3.83 (m, 1H), 3.64 - 3.36 (m, 3H), 3.30-3.25 (m, 1H), 3.05 - 2.90 (m, 1H), 2.07 (s, 1H), 1.88 (s, 1H), 1.67 (d, *J* = 10.2 Hz, 1H), 1.58 1.42 (m, 1H), 1.14 (d, J= 5.9 Hz, 3H).

### Embodiment 46-3 Synthesis of (6aR)-8-acryloyl-1-((S)-2-methylpyrrolidin-1-yl)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z49

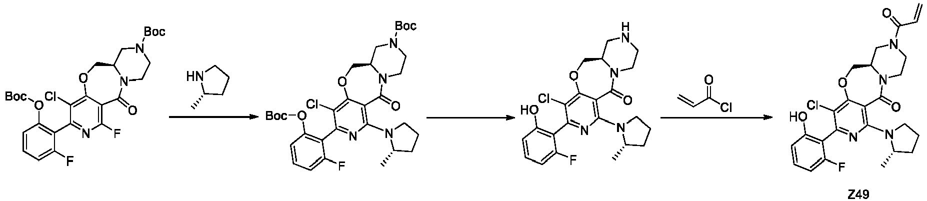

Step 1: (6a*R*)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12H-pyrazino[2,1-c]pyrido[3,4 f][1,4]oxazepin-12-one (50 mg, 0.086 mmol), (S)-2-methylpyrrolidine (14.6 mg, 0.172 mmol), *N,N*-diisopropylethylamine (33 mg, 0.258 mmol) and acetonitrile (10 mL) were added to a 100 mL round bottom flask, stirred at 60°C for 1 hour. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-((*S*)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (45 mg, crude product). ES-API: [M+H]⁺=647.2.

Step 2: (6a*R*)-8-*tert*-Butoxycarbonyl-1-((*S*)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (45 mg, 0.070 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-((*S*)-2-methylpyrrolidin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (31 mg, crude product) as a yellow solid. ES-API: [M+H]⁺=447.2.

Step 3: (6a*R*)-1-((*S*)-2-Methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (31 mg, 0.0695), 10 mL of dichloromethane and *N,N*-diisopropylethylamine (45 mg, 0.347 mmol) were added to a 50 mL round bottom flask. The reaction was cooled to 0°C, and a solution of acryloyl chloride in dichloromethane (5.7 mg, 0.0625 mmol) was added dropwise to the reaction solution. The reaction was stirred at 0°C for 10 minutes. The mixture was washed with 20 mL of water, and the organic phase was dried, then concentrated and the crude product was purified by preparative HPLC (alkali method) to obtain (6a*R*)-8-acryloyl-1-((*S*)-2-methylpyrrolidin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z49, 25 mg, 3-step yield of 58%) as a white solid. ES-API: [M+H]⁺= 501.1. ¹H NMR (400 MHz, DMSO-*d*₆) δ 9.97 (s, 1H), 7.26-7.21 (m, 1H), 6.88 - 6.63 (m, 3H), 6.17 (dd, *J* = 16.6, 2.3 Hz, 1H), 5.74 (d, *J*= 10.3 Hz, 1H), 4.47 (s, 1H), 4.37 - 3.67 (m, 7H), 3.52-3.45 (m, 3H), 2.95 (t, *J* = 9.2 Hz, 1H), 2.08 - 1.82 (m, 3H), 1.61 (m, 1H), 1.18 (d, *J* = 7.1 Hz, 3H).

### Embodiment 46-4 Synthesis of (6aR)-8-acryloyl-4-chloro-1-((S)-2,4-dimethylpiperazin-1-yl)-3-(2-fluoro-6-hydroxyphenyl)-6,6a,7,8,9,10-hexahydro-12H-pyrazino[2,1-c]pyrido[3.4-f][1,4]oxazepin-12-one Z51

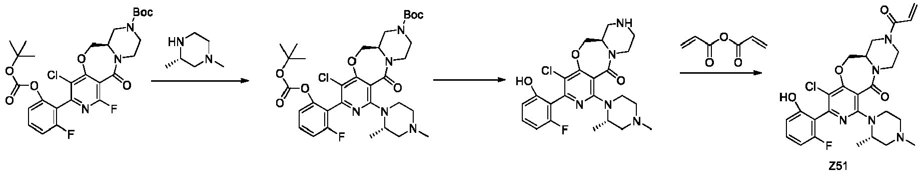

Step 1: *tert*-Butyl (6aR)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol) and 3 mL of acetonitrile were added to a round bottom flask. Diisopropylethylamine (44 mg, 0.35 mmol) and (*S*)-1,3-dimethylpiperazine (24 mg, 0.21 mmol) were then added to the reaction solution. The reaction was stirred at 80°C overnight. After the reaction was completed, the organic phase was concentrated to dryness to obtain a crude product of *tert*-butyl (6aR)-3-(2-((*tert*-butoxycarbonyl)oxy)-6-fluorophenyl)-4-chloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (52 g, crude product) as a yellow solid, and the crude product was directly used in the next reaction step. ES-API: [M+H]⁺=676.2.

Step 2: *tert*-Butyl (6a*R*)-3-(2-((*tert*-butoxycarbonyl)oxy)-6-fluorophenyl)-4-chloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (52 mg, 0.08 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-1-((*S*)-2,4-dimethylpiperazin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one (61 mg) as a brown oily liquid. The crude product was directly used in the next step. ES-API: [M+H]⁺=476.2.

Step 3: (6a*R*)-4-Chloro-1-((*S*)-2,4-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (61 mg, 0.13 mmol), 10 mL of dichloromethane and triethylamine (131 mg, 1.3 mmol) were added to a round bottom flask. The reaction was cooled to 0°C, and a solution of acrylic anhydride in dichloromethane (12 mg, 0.10 mmol, 1 mL) was added to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-1-((*S*)-2,4-dimethylpiperazin-1-yl)-3-(2-fluoro-6-hydroxyphenyl)-6,6a,7, 8,9,10-hexahydro-12*H*-pyrazino [2,1-*c*]pyrido[3,4-*f*][1,4]oxazepin-12-one (Z51, 10 mg, 15%). ES-API: [M+H]⁺=530.1. ¹H NMR (500 MHz, DMSO-*d*₆) δ 10.05 (d, *J* = 24.2 Hz, 1H), 9.58 (s, 1H), 7.36 - 7.17 (m, 1H), 6.90 - 6.61 (m, 3H), 6.18 (d, *J* = 17.2 Hz, 1H), 5.80-5.68 (m, 1H), 4.56-4.33 (m, 2H), 4.30-4.12 (m, 2H), 4.01-3.89 (m, 3H), 3.85-3.63 (m, 1H), 3.61-3.49 (m, 2H), 3.47-3.35 (m, 3H), 3.12-3.06 (m, 1H), 3.02-2.91 (m, 1H), 2.83 (s, 3H), 1.32 (d, *J* = 7.0 Hz, 3H).

### Embodiment 46-5 Synthesis of (6aR)-8-acryloyl-4-chloro-1-((S)-3-ethylmorpholino)-3-(2-fluoro-6-hydroxyphenyl)-6,6a,7,8,9,10-hexahydro-12H-pyrazino[2,1-c]pyrido[3,4f][1,4]oxazepin-12-one Z52

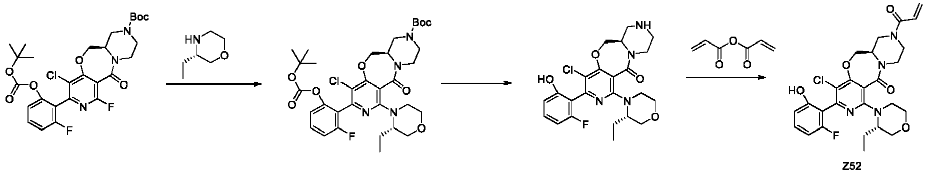

Step 1: *tert*-Butyl (6aR)-3-(2-((tert-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol) and 3 mL of acetonitrile were added to a round bottom flask. Diisopropylethylamine (44 mg, 0.35 mmol) and (*S*)-3-ethylmorpholine (24 mg, 0.21 mmol) were then added to the reaction solution. The reaction was stirred at 80°C overnight. After the reaction was completed, the organic phase was concentrated to dryness to obtain a crude product of *tert*-butyl (6aR)-3-(2-((*tert*-butoxycarbonyl)oxy)-6-fluorophenyl)-4-chloro-1-((*S*)-3-ethylmorpholino)-12-oxo-6a,7,9,10-tetrahydro-12/7-pyrazino [2,1-*c*]pyrido[3,4-*f*][1,4]oxazepine-8(6*H*)-carboxylate (55 g, crude product) as a yellow solid, and the crude product was directly used in the next reaction step. ES-API: [M+H]⁺=677.1.

Step 2: *tert*-Butyl (6a*R*)-3-(2-((*tert*-butoxycarbonyl)oxy)-6-fluorophenyl)-4-chloro-1-((*S*)-3-ethylmorpholino)-12-oxo-6a,7,9,10-tetrahydro-12*H*-pyrazino[2,1-*c*]pyrido[3,4-*f*][1,4]oxazepine-8(6*H*)-carboxylate (55 mg, 0.08 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-1-((*S*)-3-ethylmorpholino)-3-(2-fluoro-6-hydroxyphenyl)-6,6a,7,8,9,10-hexahydro-12H-pyrazino [2,1-*c*]pyrido[3,4-*f*][1,4]oxazepin-12-one (58 mg) as a brown oily liquid. The crude product was directly used in the next step. ES-API: [M+H]⁺=477.2.

Step 3: (6a*R*)-4-ehloro-1-((*S*)-3-ethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (58 mg, 0.12 mmol), 10 mL of dichloromethane and triethylamine (130 mg, 1.2 mmol) were added to a round bottom flask. The reaction was cooled to 0°C, and a solution of acrylic anhydride in dichloromethane (12 mg, 0.10 mmol, 1 mL) was added dropwise to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-1-((*S*)-3-ethylmorpholino)-3-(2-fluoro-6-hydroxyphenyl)-6,6a,7,8,9,10-hexahydro-12H-pyrazino [2,1-c]pyrido [3,4-*f*][1,4]oxazepin-12-one (Z52, 3 mg, 4.7%). ES-API: [M+H]⁺=531.1.

### Embodiment 46-6 Synthesis of (6aR)-8-acryloyl-4-chloro-3-(2-fluoro-6-hydroxyphenyl)-1-thiomorpholino-6,6a,7,8,9,10-hexahydro-12H-pyrazino[2,1-c]pyrido[3,4-f][1,4]oxazepin-12-one Z264

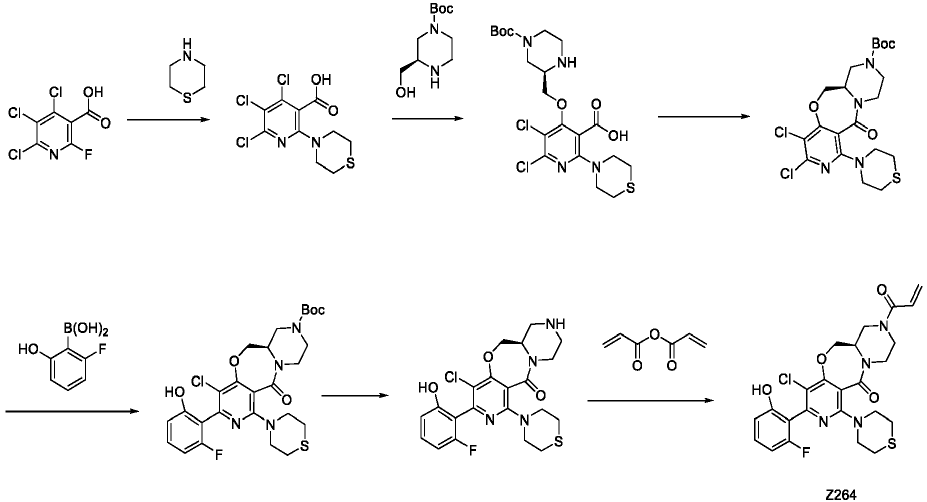

Step 1: 4,5,6-Trichloro-2-fluoronicotinic acid (580 mg, 2.4 mmol) and 50 mL of acetonitrile were added to a round bottom flask. Diisopropylethylamine (1.2 g, 9.6 mmol) and thiomorpholine (494 mg, 4.8 mmol) were then added to the reaction solution. The reaction was stirred at 90°C overnight. After the reaction was completed, the organic phase was adjusted to pH=5 with 1 M hydrochloric acid, washed with saturated brine, and extracted with ethyl acetate. The organic phase was dried over anhydrous sodium sulfate, filtered, and the filtrate was concentrated to dryness to obtain a crude product of 4,5,6-trichloro-2-thiomorpholinonicotinic acid (0.61 g, crude product) as a yellow solid, and the crude product was directly subjected to the next reaction step. ES-API: [M+H]⁺=328.0.

Step 2: *tert*-Butyl (R)-3-(hydroxymethyl)piperazine-1-carboxylate (466 mg, 3.7 mmol) was added to a suspension of 60% sodium hydride (592 mg, 14.8 mmol) in tetrahydrofuran (50 mL) at 0°C. Then, a solution of 4,5,6-trichloro-2-thiomorpholinonicotinic acid (610 g, 1.8 mmol) in tetrahydrofuran (20 mL) was added dropwise thereto. The reaction was stirred at 0°C for 30 minutes. The completion of reaction was detected by LC-MS. The reaction solution was poured into 50 mL of ice-water. The pH of the reaction solution was adjusted to 5 with 2 M dilute hydrochloric acid solution, and the reaction solution was extracted with ethyl acetate for three times. The organic phase was dried and concentrated, and the crude product was purified by flash silica gel column (ethyl acetate/petroleum ether: 20-100%) to obtain (*R*)-4-((4-(*tert-*butoxycarbonyl)piperazin-2-yl)methoxy)-5,6-dichloro-2-thiomorpholinonicotinic acid (643 mg, yield of 71%) as a yellow solid. ES-API: [M+H]⁺=508.1.

Step 3: (*R*)-4-((4-(*tert*-Butoxycarbonyl)piperazin-2-yl)methoxy)-5,6-dichloro-2-thiomorpholinonicotinic acid (643 mg, 1.27 mmol), diisopropylethylamine (10 mL) and dichloromethane (30 mL) were added to around bottom flask. Propylphosphonic anhydride solution (10 mL, 50%w/w ethyl acetate solution) was added dropwise thereto. The reaction was stirred at room temperature for 30 minutes. The completion of reaction was detected by LC-MS. 50 mL of dichloromethane was added to the reaction, and the organic phase was washed with 30 mL of hydrochloric acid (1 M) and 100 mL of saturated aqueous sodium bicarbonate solution in turn. The organic phase was dried and concentrated, and the crude product was purified by flash silica gel column (methanol/dichloromethane: 0-3%) to obtain *tert*-butyl (*R*)-3,4-dichloro-12-oxo-1-thiomorpholino-6a,7,9,10-tetrahydro-12*H*-pyrazino[2,1-*c*]pyrido[3,4-*f*][1,4]oxazepine-8(6*H*)-carboxylate (316 mg, 51%). ES-API: [M+H]⁺=490.1.

Step 4: *tert*-Butyl (*R*)-3,4-dichloro-12-oxo-1-thiomorpholino-6a,7,9,10-tetrahydro-12*H-*pyrazino[2,1-*c*]pyrido[3,4-*f*][1,4]oxazepine-8(6*H*)-carboxylate (316 mg, 0.65 mmol), 2-fluoro-phenylboronic acid (327 mg, 2.1 mmol), tetrakis(triphenylphosphine)palladium (84 mg, 0.073 mmol), sodium carbonate (223 mg, 2.1 mmol), 15 mL of dioxane and 3 mL of water were added to a 100 mL reaction flask. The reaction was stirred at 80°C for 2.5 hours under nitrogen protection, and the reaction was stopped. The reaction solution was added with 20 mL of water, extracted 3 times with 20 mL of ethyl acetate. The organic phase was dried and concentrated, and the crude product was purified by flash silica gel column (ethyl acetate/petroleum ether: 0-100%) to obtain a product of *tert*-butyl (6aR)-4-chloro-3-(2-fluoro-6-hydroxyphenyl)-12-oxo-1-thiomorpholino-6a,7,9,10-tetrahydro-12*H*-pyrazino[2,1-*c*]pyrido[3,4-*f*][1,4]oxazepine-8(6*H*)-carboxylate (326 mg, 89%) as a yellow solid. ES-API: [M+H]⁺= 566.0

Step 5: *tert*-Butyl (6aR)-4-chloro-3-(2-fluoro-6-hydroxyphenyl)-12-oxo-1-thiomorpholino-6a,7,9,10-tetrahydro-12*H*-pyrazino[2,1-*c*]pyrido[3,4-*f*][1,4]oxazepine-8(6*H*)-carboxylate (326 mg, 0.60 mmol), 3 mL of methanol and 6 mL of hydrogen chloride/dioxane solution (4 M) were added to a round bottom flask. The mixture was stirred at room temperature for 1 hour, and the completion of reaction was detected by LC-MS. The reaction solution was concentrated to obtain (6a*R*)-4-chloro-3-(2-fluoro-6-hydroxyphenyl)-1-thiomorpholino-6,6a,7,8,9,10-hexahydro-12*H*-pyrazino [2,1-*c*]pyrido [3,4-*f*][1,4]oxazepin-12-hydrochloride (380 mg) as a yellow solid. The crude product was directly used in the next step. ES-API: [M+H]⁺=465.0.

Step 6: (6a*R*)-4-Chloro-3-(2-fluoro-6-hydroxyphenyl)-1-thiomorphofino-6,6a,7,8,9,10-hexahydro-12*H*-pyrazino[2,1-*c*]pyrido[3,4-*f*][1,4]oxazepine-12-hydrochloride (380 mg, 0.81 mmol), 3 mL of dichloromethane and triethylamine (627 mg, 6.0 mmol) were added to a round bottom flask. The reaction was cooled to 0°C, and a solution of acrylic anhydride in dichloromethane (50 mg, 0.4 mmol, 0.5 mL) was added to the reaction solution. The reaction was stirred at 0°C for 10 minutes. 40 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6aR)-8-acryloyl-4-chloro-3-(2-fluoro-6-hydroxyphenyl)-1-thiomorpholino-6,6a,7,8,9,10-hexahydro-12*H*-pyrazino [2,1-c]pyrido [3,4-*f*][1,4]oxazepin-12-one (Z264, 67 mg, 16%). ES-API: [M+H]⁺=519.1. ¹H NMR (500 MHz, DMSO-*d*₆) δ 10.00 (s, 1H), 7.25 (dd, *J* = 15.6, 7.8 Hz, 1H), 6.82-6.45 (m, 3H), 6.17 (d, *J* = 16.7 Hz, 1H), 5.75 (d, *J* = 10.0 Hz, 1H), 4.53-4.29 (m, 2H), 4.25-4.11 (m, 1H), 4.07-3.88 (m, 2H), 3.86-3.40 (m, 7H), 3.30 (s, 1H), 2.75-2.65 (m, 2H), 2.61-2.52 (m, 2H).

### Embodiment 46-7 Synthesis of (6aR)-8-acryloyl-4-chloro-3-(2-fluoro-6-hydroxyphenyl)-1-(5-methylhexahydropyrrolo[3,4-c]pyrrol-2(1H)-yl)-6,6a,7,8,9,10-hexahydro-12H-pyrazino[2,1-c]pyrido[3,4-f][1,4]oxazepin-12-one Z60

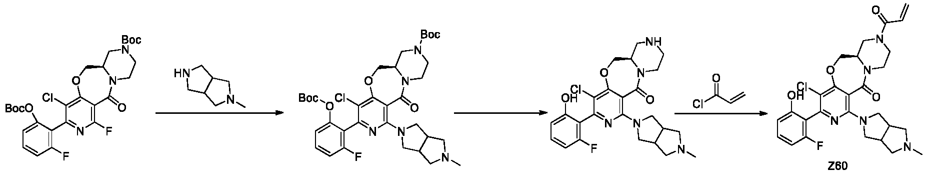

Step 1: A solution of *tert*-butyl (6aR)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (50 mg, 0.085 mmol), 2-methyloctahydropyrrolo[3,4-c]pyrrole (16.2 mg, 0.12 mmol) and *N*-ethyl-*N-*isopropylpropan-2-amine (33.2 mg, 0.25 mmol) in acetonitrile (3 mL) was added to a 100 mL round bottom flask at room temperature. After the reaction was completed, the mixture was directly concentrated to obtain a crude product of *tert*-butyl (6aR)-3-(2-((*tert*-butoxycarbonyl)oxy)-6-fluorophenyl)-4-chloro-1-(5-methylhexahydropyrrolo[3,4-*c*]pyrrol-2(1*H*)-yl)-12-oxo-6a,7,9,10-tetrahydro-12*H*-pyrazino[2,1-*c*]pyrido[3,4-*f*][1,4]oxazepine-8(6*H*)-carboxylate (99 mg). ES-API: [M+H]⁺=688.1.

Step 2: A solid of *tert*-butyl (6aR)-3-(2-((*tert*-butoxycarbonyl)oxy)-6-fluorophenyl)-4-chloro-1-(5-methylhexahydropyrrolo[3,4-*c*]pyrrol-2(1*H*)-yl)-12-oxo-6a,7,9,10-tetrahydro-12*H*-pyrazino[2,1-*c*]pyrido[3,4-*f*][1,4]oxazepine-8(6*H*)-carboxylate (99 mg), dichloromethane (3 mL) and trifluoroacetic acid (2 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-3-(2-fluoro-6-hydroxyphenyl)-1-(5-methylhexahydropyrrolo[3,4-*c*]pyrrol-2(1*H*)-yl)-6,6a,7,8,9,10-hexahydro-12*H*-pyrazino[2,1-*c*]pyrido[3,4-*f*][1,4]oxazepin-12-one (130 mg). ES-API: [M+H]⁺=488.2

Step 3: (6a*R*)-4-Chloro-3-(2-fluoro-6-hydroxyphenyl)-1-(5-methylhexahydropyrrolo[3,4-*c*]pyrrol-2(1*H*)-yl)-6,6a,7,8,9,10-hexahydro-12*H*-pyrazino[2,1-*c*]pyrido[3,41][1,4]oxazepin-12-one (130 mg), dichloromethane (5 mL) and *N*-ethyl-*N*-isopropylpropan-2-amine (25.8 mg, 0.21 mmol) were added to a 100 mL eggplant-shaped flask. The reaction was cooled to 0°C, and a solution of acryloyl chloride in dichloromethane (6.9 mg, 0.077 mmol, 0.5 mL) was added dropwise to the reaction solution. The reaction was stirred at 0°C for 10 minutes. 20 mL of saturated aqueous sodium bicarbonate solution was added to the reaction solution, and the mixture was extracted three times with 10 mL of dichloromethane. The organic phase was dried and concentrated, and the crude product was purified by preparative HPLC to obtain (6a*R*)-8-acryloyl-4-chloro-3-(2-fluoro-6-hydroxyphenyl)-1-(5-methylhexahydropyrrolo[3,4-*c*]pyrrol-2(1*H*)-yl)-6,6a,7,8,9,10-hexahydro-12*H*-pyrazino[2,1-*c*]pyrido[3,4-*f*][1,4]oxazepin-12-one (Z60, 4 mg, P: 75%, Y: 8.6%) as a white solid. ES-API: [M+H]⁺=542.2.

### Embodiment 46-8 Synthesis of (6aR)-8-acryloyl-4-chloro-1-((1R,5S)-8-oxa-3-azabicyclo[3.2.1]octan-3-yl)-3-(2-fluoro-6-hydroxyphenyl)-6,6a,7,8,9,10-hexahydro-12H-pyrazino[2,1-c]pyrido[3,4-f][1,4]oxazepin-12-one Z62

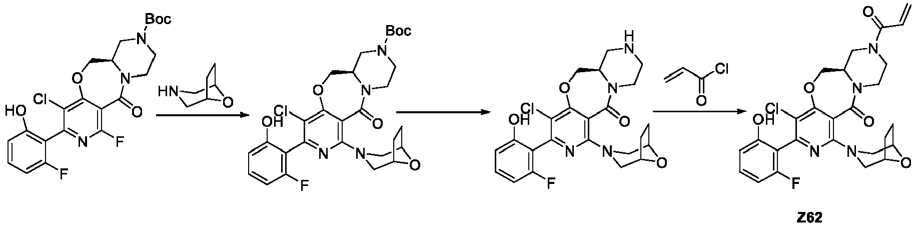

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (90 mg, 0.18 mmol), (1*R*,5*S*)-8-oxa-3-azabicyclo[3.2.1]octane (24 mg, 0.21 mmol), *N,N*-diisopropylethylamine (60 mg, 0.53 mmol) and acetonitrile (5 mL) were added to a 50 mL round bottom flask, reacted at 60°C for 1 hour. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain a solid of *tert*-butyl (6a*R*)-1-((1*R*,5*S*)-8-oxa-3-azabicyclo[3.2.1]octan-3-yl)-4-chloro-3-(2-fluoro-6-hydroxyphenyl)-12-oxo-6a,7,9,10-tetrahydro-12*H*-pyrazino[2,1-*c*]pyrido [3,4-*f*][1,4]oxazepine-8(6*H*)-carboxylate (21 mg, yield of 20%). ES-API: [M+H]⁺=575.2.

Step 2: Trifluoroacetic acid (2 mL) was added to a solution of tert-butyl (6a*R*)-1-((1*R*,5*S*)-8-oxa-3-azabicyclo[3.2.1]octan-3-yl)-4-chloro-3-(2-fluoro-6-hydroxyphenyl)-12-oxo-6a,7,9,10-tetrahydro-12*H-*pyrazino[2,1-*c*]pyrido[3,4-f][1,4]oxazepine-8(6*H*)-carboxylate (21 mg, 0.03 mmol) in dichloromethane (5 mL), reacted at room temperature for 1 hour. The reaction solution was directly concentrated to obtain a crude product of (6a*R*)-1-((1*R*,5*S*)-8-oxa-3-azabicyclo[3.2.1]octan-3-yl)-4-chloro-3-(2-fluoro-6-hydroxyphenyl)-6,7-6a,7,8,9,10-hexahydro-12*H*-pyrazino[2,1-*c*]pyrido[3,4-*f*][1,4]oxazepin-12-one (16 mg) as an oil. ES-API: [M+H]⁺=475.2.

Step 3: The above crude product of (6a*R*)-1-((1*R*,5*S*)-8-oxa-3-azabicyclo[3.2.1]octan-3-yl)-4-chloro-3-(2-fluoro-6-hydroxyphenyl)-6,7-6a,7,8,9,10-hexahydro-12H-pyrazino [2,1-c]pyrido [3,4-*f*][1,4]oxazepin-12-one (16 mg, 0.03 mmol) as an oil was dissolved in dichloromethane (2 mL), and *N,N-*diisopropylethylamine (13 mg, 0.1 mmol) was added thereto. The reaction solution was cooled to 0°C, and acryloyl chloride (3 mg, 0.03 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-((1*R*,5*S*)-8-oxa-3-azabicyclo[3.2.1]octan-3-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z62, 10 mg, yield of 63%). ES-API: [M+H]⁺=529.1.

### Embodiment 46-9 Synthesis of (6aR)-8-acryloyl-4-chloro-1-(4-oxa-7-azaspiro[2.5]octan-7-yl)-3-(2-fluoro-6-hydroxyphenyl)-6,6a,7,8,9,10-hexahydro-12H-pyrazino[2,1-c]pyrido[3,4-f][1,4]oxazepin-12-one Z63

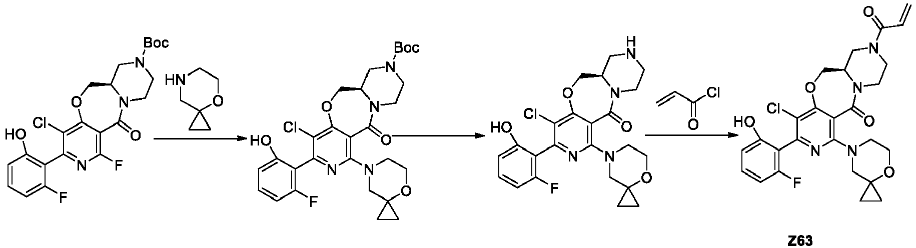

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (43 mg, 0.09 mmol), 4-oxa-7-azaspiro[2.5]octane (24 mg, 0.21 mmol), *N,N*-diisopropylethylamine (60 mg, 0.53 mmol) and acetonitrile (5 mL) were added to a 50 mL round bottom flask, reacted at 60°C for 1 hour. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain a solid of *tert*-butyl (6aR)-4-chloro-3-(2-fluoro-6-hydroxyphenyl)-12-oxo-1-(4-oxa-7-azaspiro[2.5]octyl-7-yl)-6a,7,9,10-tetrahydro-12*H*-pyrazino[2,1-*c*]pyrido[3,4-*f*][1,4]oxazepine-8(6*H*)-carboxylate (50 mg, yield of 96%). ES-API: [M+H]⁺=575.2.

Step 2: Trifluoroacetic acid (2 mL) was added to a solution of *tert*-butyl (6a*R*)-4-chloro-3-(2-fluoro-6-hydroxyphenyl)-12-oxo-1-(4-oxa-7-azaspiro[2.5]octyl-7-yl)-6a,7,9,10-tetrahydro-12*H-*pyrazino[2,1-*c*]pyrido[3,4-*f*][1,4]oxazepine-8(6*H*)-carboxylate (50 mg, 0.08 mmol) in dichloromethane (5 mL), reacted at room temperature for 1 hour. The reaction solution was directly concentrated to obtain a crude product of (6a*R*)-4-chloro-3-(2-fluoro-6-hydroxyphenyl)-1-(4-oxa-7-azaspiro[2.5]octan-7-yl)-6,6a,7,8,9,10-hexahydro-12*H*-pyrazino[2,1-*c*]pyrido[3,4-*f*][1,4]oxazepin-12-one (46 mg) as an oil. ES-API: [M+H]⁺=475.2.

Step 3: The above crude product of (6a*R*)-4-chloro-3-(2-fluoro-6-hydroxyphenyl)-1-(4-oxa-7-azaspiro[2.5]octan-7-yl)-6,6a,7,8,9,10-hexahydro-12*H*-pyrazino[2,1-*c*]pyrido[3,4-*f*][1,4]oxazepin-12-one (46 mg, 0.09 mmol) as an oil was dissolved in dichloromethane (2 mL), and *N,N-*diisopropylethylamine (40 mg, 0.3 mmol) was added thereto. The reaction solution was cooled to 0°C, and acryloyl chloride (8 mg, 0.09 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6aR)-8-acryloyl-4-chloro-1-(4-oxa-7-azaspiro[2.5]octan-7-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z63, 18 mg, yield of 38%). ES-API: [M+H]⁺=529.1. ¹H NMR (400 MHz, DMSO-*d*₆) δ 10.10 - 9.95 (m, 1H), 7.29 - 7.19 (m, 1H), 6.88 - 6.54 (m, 3H), 6.23 - 6.10 (m, 1H), 5.82 - 5.67 (m, 1H), 4.48 - 3.44 (m, 12H), 3.32 - 3.00 (m, 3H), 0.85 - 0.36 (m, 4H).

### Embodiment 46-10 Synthesis of (6aR)-8-acryloyl-1-((S)-2-methylazetidin-1-yl)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z68

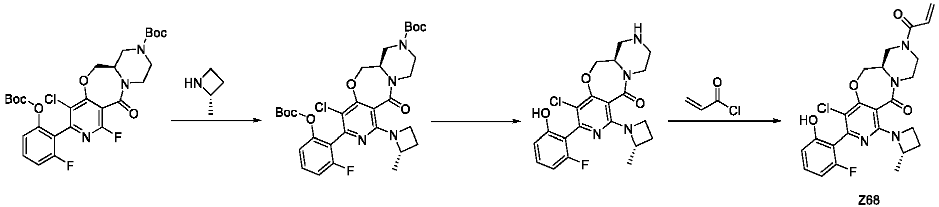

Step 1: (6a*R*)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12H-pyrazino[2,1-c]pyrido[3,4 f][1,4]oxazepin-12-one (50 mg, 0.086 mmol), (*S*)-2-methylazetidine (12.2 mg, 0.172 mmol), *N,N*-diisopropylethylamine (33 mg, 0.258 mmol) and acetonitrile (10 mL) were added to a 100 mL round bottom flask, stirred at 60°C for 1 hour. The mixture was extracted with ethyl acetate, washed with saturated brine, and the organic phase was dried, concentrated, and purified by silica gel column (petroleum ether: ethyl acetate = 20:80) to obtain (6a*R*)-8-*tert-*butoxycarbonyl-1-((*S*)-2-methylazetidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (38 mg, yield of 84%). ES-API: [M+H]⁺=633.2.

Step 2: (6a*R*)-9-*tert*-Butoxycarbonyl-1-((*S*)-2-methylazetidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (38 mg, 0.071 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-((*S*)-2-methylazetidin-1-yl)-4-chloro-3-(2-fluoro-6-hydroxyphenyl)-6,6a,7,8,9,10-hexahydro-12*H*-pyrazino[2,1-e]pyrido[3,41] [1,4]oxazepin-12-one (31 mg, crude product) as a yellow solid. ES-API: [M+H]⁺=433.2.

Step 3: (6a*R*)-1-((*S*)-2-Methylazetidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (31 mg, 0.071), 10 mL of dichloromethane and *N,N*-diisopropylethylamine (27 mg, 0.213 mmol) were added to a 50 mL round bottom flask. The reaction was cooled to 0°C, and a solution of acryloyl chloride in dichloromethane (5.7 mg, 0.064 mmol) was added dropwise to the reaction solution. The reaction was stirred at 0°C for 10 minutes. The mixture was washed with 20 mL of water, and the organic phase was dried, then concentrated and the crude product was purified by preparative HPLC (alkali method) to obtain (6a*R*)-8-acryloyl-1-((*S*)-2-methylazetidin-1-yl)-4-chloro-3-(2-fluoro-6-hydroxyphenyl)-6,6a,7,8,9,10-hexahydro-12*H*-pyrazino[2,1-*c*]pyrido[3,4*f*][1,4]oxazepin-12-one (Z68, 8 mg, 3-step yield of 19%) as a white solid. ES-API: [M+H]⁺= 487.1. ¹H NMR (500 MHz, DMSO-*d*₆) δ 9.98-9.94 (m, 1H), 7.26-7.21 (m, 1H), 6.75-6.70 (m, 3H), 6.17 (d, *J =* 16.7 Hz, 1H), 5.80 - 5.68 (m, 1H), 4.46 - 4.12 (m, 4H), 4.01 (td, *J =* 9.3, 5.3 Hz, 3H), 3.92 - 3.69 (m, 2H), 3.65 - 3.45 (m, 2H), 3.30 (s, 1H), 2.47 (d, *J* = 9.0 Hz, 1H), 1.74-1.70 (m, 1H), 1.41 (d, *J* = 6.0 Hz, 3H).

### Embodiment 46-11 Synthesis of (6aR)-8-acryloyl-1-(tert-butylamino)-4-chloro-3-(2-fluoro-6-hydroxyphenyl)-6,6a,7,8,9,1[2,1-c]pyrido[3,4-f][1,4]oxazepin-12-one Z69

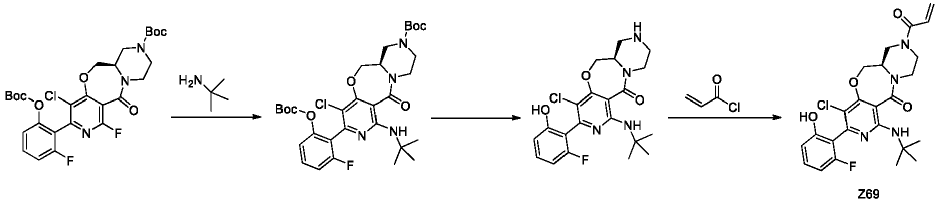

Step 1: (6aR)-8-tert-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-c]pyrido[3,4 f][1,4]oxazepin-12-one (300 mg, 0.516 mmol), *tert*-butylamino (113 mg, 1.549 mmol), *N,N*-diisopropylethylamine (200 mg, 1.549 mmol) and acetonitrile (10 mL) were added to a 100 mL round bottom flask, stirred at 120°C for 1.5 hours. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-(*tert*-butylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (230 mg, yield of 83%). ES-API: [M+H]⁺=535.2.

Step 2: (6aR)-8-tert-Butoxycarbonyl-1-(*tert*-butylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, 0.056 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6aR)-1-(*tert*-butylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (24 mg, crude product) as a yellow solid. ES-API: [M+H]⁺=435.2.

Step 3: (6a*R*)-1-(*tert*-Butylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (24 mg, 0.056), 10 mL of dichloromethane and *N,N*-diisopropylethylamine (22 mg, 0.168 mmol) were added to a 50 mL round bottom flask. The reaction was cooled to 0°C, and a solution of acryloyl chloride in dichloromethane (4.56 mg, 0.0504 mmol) was added dropwise to the reaction solution. The reaction was stirred at 0°C for 10 minutes. The mixture was washed with 20 mL of water, and the organic phase was dried, then concentrated and the crude product was purified by preparative HPLC (alkali method) to obtain (6a*R*)-8-acryloyl-1-(*tert-*butylamino)-4-chloro-3-(2-fluoro-6-hydroxyphenyl)-6,6a,7,8,9,10-hexahydro-12*H*-pyrazino[2,1-*c*]pyrido[3,4-*f*] [1,4]oxazepin-12-one (Z69, 12 mg, 3-step yield of 28%) as a white solid. ES-API: [M+H]⁺= 489.1. ¹H NMR (500 MHz, DMSO-*d*₆) δ 10.01 - 9.83 (m, 1H), 7.29 - 7.11 (m, 2H), 6.82 - 6.64 (m, 3H), 6.16 (t, *J=* 17.7 Hz, 1H), 5.75-5.65 (m, 1H), 4.42 - 4.24 (m, 2H), 4.12 (s, 1H), 3.91 (m, 5H), 3.68 - 3.61 (m, 1H), 1.34 (s, 9H).

### Embodiment 46-12 Synthesis of (6aR)-8-acryloyl-4-chloro-1-(isopropylamino)-3-(2-fluoro-6-hydroxyphenyl)-6,6a,7,8,9,10-hexahydro-12H-pyrazino[2,1-c]pyrido[3,4-f][1,4]oxazepin-12-one Z75

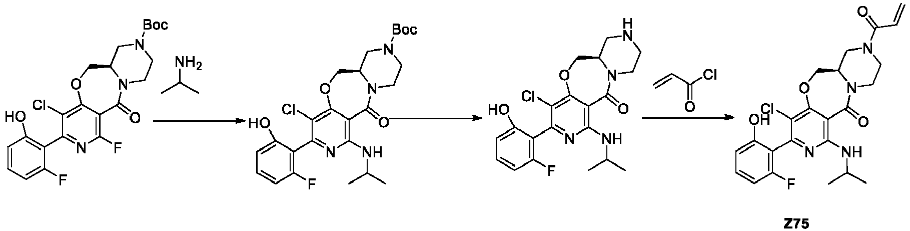

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (48 mg, 0.1 mmol), dimethylamine hydrochloride (24 mg, 0.30 mmol), *N,N*-diisopropylethylamine (39 mg, 0.3 mmol) and acetonitrile (5 mL) were added to a 50 mL round bottom flask, reacted at 80°C for 2 hours. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain a solid of *tert*-butyl (6aR)-4-chloro-3-(2-fluoro-6-hydroxyphenyl)-1-(isopropylamino)-12-oxo-6a,7,9,10-tetrahydro-12*H*-pyrazino[2,1-*c*]pyrido[3,4-*f*][1,4]oxazepine-8(6*H*)-carboxylate (45 mg, yield of 86%). ES-API: [M+H]⁺=521.2.

Step 2: Trifluoroacetic acid (2 mL) was added to a solution of *tert*-butyl (6a*R*)-4-chloro-3-(2-fluoro-6-hydroxyphenyl)-1-(isopropylamino)-12-oxo-6a,7,9,10-tetrahydro-12/7-pyrazino [2,1-c]pyrido [3,4-*f*][1,4]oxazepine-8(6*H*)-carboxylate (45 mg, 0.08 mmol) in dichloromethane (5 mL), reacted at room temperature for 1 hour. The reaction solution was directly concentrated to obtain a crude product of (6a*R*)-4-chloro-3-(2-fluoro-6-hydroxyphenyl)-1-(isopropylamino)-6,6a,7,8,9,10-hexahydro-12H-pyrazino [2,1-*c*]pyrido[3,4-*f*][1,4]oxazepin-12-one (40 mg) as an oil. ES-API: [M+H]⁺=475.2.

Step 3: The above crude product of (6a*R*)-4-chloro-3-(2-fluoro-6-hydroxyphenyl)-1-(isopropylamino)-6,6a,7,8,9,10-hexahydro-12*H*-pyrazino[2,1-*c*]pyrido[3,4-*f*][1,4]oxazepin-12-one (40 mg, 0.08 mmol) as an oil was dissolved in dichloromethane (2 mL), and *N,N*-diisopropylethylamine (40 mg, 0.3 mmol) was added thereto. The reaction solution was cooled to 0°C, and acryloyl chloride (7 mg, 0.08 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(isopropylamino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z75, 21 mg, yield of 55%). ES-API: [M+H]⁺=475.1. ¹H NMR (500 MHz, DMSO-*d*₆) δ 9.89 (d, *J* = 47.1 Hz, 1H), 7.25 (dd, *J=* 15.3, 8.1 Hz, 1H), 7.03 (s, 1H), 6.83 - 6.63 (m, 3H), 6.17 (t, *J* = 17.7 Hz, 1H), 5.73 (dd, *J* = 26.1, 11.0 Hz, 1H), 4.35 (dd, *J =* 51.5, 8.9 Hz, 2H), 4.14 (s, 1H), 4.05 - 3.52 (m, 7H), 1.14 (dd, *J* = 9.5, 6.6 Hz, 6H).

### Embodiment 46-13 Synthesis of (6aR)-8-acryloyl-4-chloro-1-(2-oxa-5-azabicyclo[4.1.0]heptan-5-yl)-3-(2-fluoro-6-hydroxyphenyl)-6,6a,7,8,9,10-hexahydro-12H-pyrazino[2,1-c]pyrido[3,4-f][1,4]oxazepin-12-one Z76

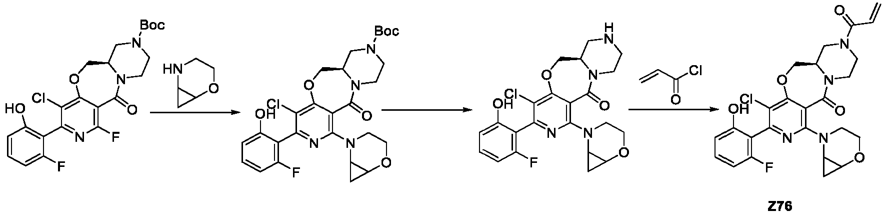

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (40 mg, 0.08 mmol), 2-oxa-5-azabicyclo[4.1.0]heptane hydrochloride (80 mg, 0.53 mmol), *N,N*-diisopropylethylamine (72 mg, 0.56 mmol) and acetonitrile (5 mL) were added to a 50 mL round bottom flask, reacted at 80°C for 5 hour. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain a solid of *tert*-butyl (6aR)-1-(2-oxa-5-azabicyclo[4.1.0]heptan-5-yl)-4-chloro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (35 mg, yield of 78%). ES-API: [M+H]⁺=561.1.

Step 2: Trifluoroacetic acid (2 mL) was added to a solution of tert-butyl (6a*R*)-1-(2-oxa-5-azabicyclo[4.1.0]heptan-5-yl)-4-chloro-3-(2-fluoro-6-hydroxyphenyl)-12-oxo-6a,7,9,10-tetrahydro-12*H-*pyrazino[2,1-*c*]pyrido[3,4-f][1,4]oxazepine-8(6*H*)-carboxylate (35 mg, 0.06 mmol) in dichloromethane (5 mL), reacted at room temperature for 1 hour. The reaction solution was directly concentrated to obtain a crude product of (6a*R*)-1-(2-oxa-5-azabicyclo[4.1.0]heptan-5-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (33 mg) as an oil. ES-API: [M+H]⁺=461.1.

Step 3: The above crude product of (6a*R*)-1-(2-oxa-5-azabicyclo[4.1.0]heptan-5-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (33 mg, 0.07 mmol) as an oil was dissolved in dichloromethane (2 mL), and *N,N*-diisopropylethylamine (26 mg, 0.2 mmol) was added thereto. The reaction solution was cooled to 0°C, and acryloyl chloride (6 mg, 0.07 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2-oxa-5-azabicyclo[4.1.0]heptan-5-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z76, 18 mg, yield of 50%). ES-API: [M+H]⁺=515.2.

### Embodiment 46-14 Synthesis of (6aR)-8-acryloyl-1-((2-(dimethylamino)ethoxy)-4-chloro-3-(2-fluoro-6-hydroxyphenyl)-6,6a,7,8,9,10-hexahydro-12H-pyrazino[2,1-c]pyrido[3,4-f][1,4]oxazepin-12-one Z12

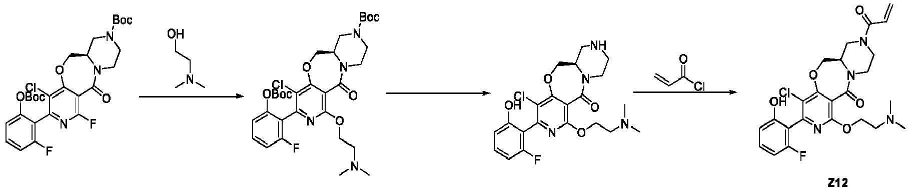

Step 1: 2-(Dimethylamino)ethanol (23 mg, 0.258 mmol), 60% NaH (17 mg, 0.43 mmol) and tetrahydrofuran (10 mL) were added to a 100 mL round bottom flask, stirred in an ice-water bath for 10 minutes. (6aR)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (intermediate 1a, 50 mg, 0.086 mmol) was added to the above reaction and stirred at room temperature for 1 hour. The mixture was quenched with aqueous ammonium chloride solution, extracted with ethyl acetate, washed with saturated brine, and the organic phase was dried and concentrated, and purified by silica gel column to obtain (6a*R*)-8-*tert*-butoxycarbonyl-1-((2-(dimethylamino)ethoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, yield of 50%). ES-API: [M+H]⁺=551.0.

Step 2: (6a*R*)-8-*tert*-Butoxycarbonyl-1-((2-(dimethylamino)ethoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, 0.084 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-((2-(dimethylamino)ethoxy)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (25 mg, crude product) as a yellow solid. ES-API: [M+H]⁺=451.0.

Step 3: (6a*R*)-1-((2-(Dimethylamino)ethoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (25 mg, crude product), 10 mL of dichloromethane and *N,N*-diisopropylethylamine (21 mg, 0.163 mmol) were added to a 50 mL round bottom flask. The reaction was cooled to 0°C, and a solution of acryloyl chloride in dichloromethane (4.4 mg, 0.049 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:H₂O=50:50(0.1% NH₄HCO₃), flow rate: 15 mL/min, column temperature: 25°C) to obtain a target product of (6a*R*)-8-acryloyl-1-((2-(dimethylamino)ethoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z12, 4.5 mg, 3-step yield of 8%) as a white solid. ES-API: [M+H]⁺= 505.2. ¹H NMR (500 MHz, DMSO-*d*₆) δ 10.10 (s, 1H), 7.33 - 7.25 (m, 1H), 6.83 - 6.77 (m, 1H), 6.77 - 6.69 (m, 2H), 6.17 (d, *J* = 16.6 Hz, 1H), 5.73 (dd, *J* = 19.3, 10.5 Hz, 1H), 4.53 - 4.43 (m, 1H), 4.31-4.24 (m, 4H), 4.11 - 3.97 (m, 2H), 3.88 (d, *J* = 17.3 Hz, 2H), 3.68-3.64 (m, 2H), 2.60-2.54 (m, 2H), 2.16 (s, 6H).

### Embodiment 47-1 Synthesis of (6aR)-8-acryloyl-4-chloro-3-(2-fluoro-6-hydroxyphenyl)-1-((1-methylpiperidin-4-yl)oxy)-6,6a,7,8,9,10-hexahydro-12H-pyrazino[2,1-c]pyrido[3,4-f][1,4]oxazepin-12-one Z61

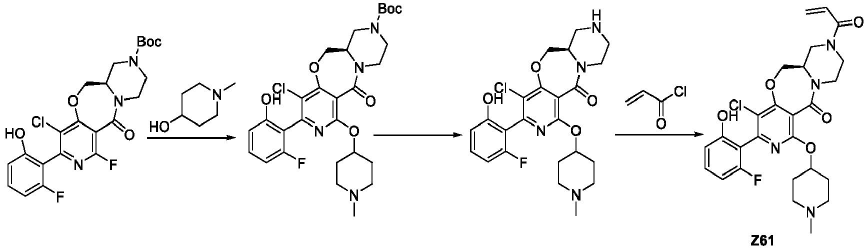

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (91 mg, 0.18 mmol), 1-methylpiperidin-4-ol (24 mg, 0.21 mmol), potassium *tert*-butoxide (60 mg, 0.54 mmol) and acetonitrile (5 mL) were added to a 50 mL round bottom flask, reacted at room temperature for 16 hours. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain a solid of *tert*-butyl (6aR)-4-chloro-3-(2-fluoro-6-hydroxyphenyl)-1-((1-methylpiperidin-4-yl)oxy)-12-oxo-6a,7,9,10-tetrahydro-12*H*-pyrazino[2,1-*c*]pyrido[3,4-*f*][1,4]oxazepine-8(6*H*)-carboxylate (61 mg, yield of 56%). ES-API: [M+H]⁺=577.2.

Step 2: Trifluoroacetic acid (2 mL) was added to a solution of *tert*-butyl (6a*R*)-4-chloro-3-(2-fluoro-6-hydroxyphenyl)-1-((1-methylpiperidin-4-yl)oxy)-12-oxo-6a,7,9,10-tetrahydro-12*H*-pyrazino[2,1-*c*]pyrido[3,4-*f*][1,4]oxazepine-8(6*H*)-carboxylate (61 mg, 0.11 mmol) in dichloromethane (5 mL), reacted at room temperature for 1 hour. The reaction solution was directly concentrated to obtain a crude product of (6a*R*)-4-chloro-3-(2-fluoro-6-hydroxyphenyl)-1-((1-methylpiperidin-4-yl)oxy)-6,6a,7,8,9,10-hexahydro-12*H*-pyrazino[2,1-*c*]pyrido[3,4-*f*][1,4]oxazepin-12-one (55 mg) as an oil. ES-API: [M+H]⁺=477.2.

Step 3: The above crude product of (6a*R*)-4-chloro-3-(2-fluoro-6-hydroxyphenyl)-1-((1-methylpiperidin-4-yl)oxy)-6,6a,7,8,9,10-hexahydro-12*H*-pyrazino[2,1-*c*]pyrido[3,4-*f*][1,4]oxazepin-12-one (55 mg, 0.12 mmol) as an oil was dissolved in dichloromethane (2 mL), and *N,N-*diisopropylethylamine (46 mg, 0.36 mmol) was added thereto. The reaction solution was cooled to 0°C, and acryloyl chloride (9 mg, 0.10 mmol) was added dropwise thereto. After stirring for 5 minutes, the mixture was added with methanol (2 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-((1-methylpiperidin-4-yl)oxy)-6,6a,7,8,9,10-hexahydro-12*H*-pyrazino[2,1-c]pyrido[3,4*f*][1,4]oxazepin-12-one (Z61, 30 mg, yield of 47%). ES-API: [M+H]⁺=531.1. ¹H NMR (400 MHz, DMSO-*d*₆) δ 10.11 (s, 1H), 7.28 (dd, *J* = 15.8, 7.7 Hz, 1H), 7.01 - 6.58 (m, 3H), 6.17 (d, *J* = 16.4 Hz, 1H), 5.75 (d, *J* = 11.7 Hz, 1H), 4.92 (s, 1H), 4.67 - 3.33 (m, 10H), 2.52 (s, 2H), 2.19 - 1.55 (m, 8H).

### Embodiment 47-2 Synthesis of (6aR)-8-acryloyl-4-chloro-3-(2-fluoro-6-hydroxyphenyl)-1-(((R)-1-methylpyrrolidin-3-yl)oxy)-6,6a,7,8,9,10-hexahydro-12H-pyrazino[2,1-c]pyrido[3,4-f][1,4]oxazepin-12-one Z64

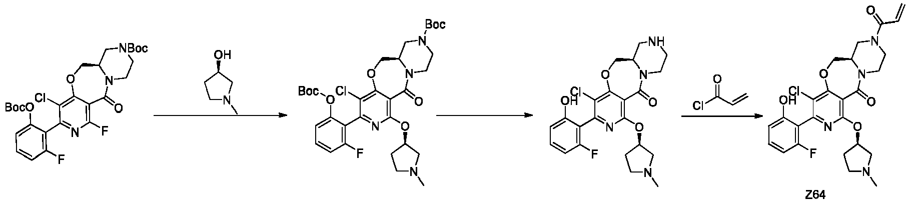

Step 1: 60% sodium hydride (17 mg, 0.43 mmol), (*R*)-1-methylpyrrolidin-3-ol (8.6 mg, 0.086 mmol) and tetrahydrofuran (2 mL) were added to a 100 mL three-necked round bottom flask. The reaction was cooled to 0°C, and a solution of *tert*-butyl (6aR)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (50 mg, 0.086 mmol) in tetrahydrofuran (4 mL) was added dropwise thereto. The reaction was stirred in an ice bath at 0°C for 20 minutes and the completion of reaction was detected by LC-MS. 30 mL of saturated aqueous NH₄Cl solution was poured into the reaction solution, and the pH was adjusted to 5-6, and the mixture was extracted 4 times with 40 mL of ethyl acetate. The organic phase was dried and concentrated to obtain a crude product of *tert*-butyl (6aR)-3-(2-((*tert*-butoxycarbonyl)oxy)-6-fluorophenyl)-4-chloro-1-(((*R*)-1-methylpyrrolidin-3-yl)oxy)-12-oxo-6a,7,9,10-tetrahydro-12*H*-pyrazino [2,1-*c*]pyrido[3,4-*f*][1,4]oxazepine-8(6*H*)-carboxylate (53 mg) as a yellow solid. ES-API: [M+H]⁺=663.3.

Step 2: The solid of *tert*-butyl (6aR)-3-(2-((*tert*-butoxycarbonyl)oxy)-6-fluorophenyl)-4-chloro-1-(((*R*)-1-methylpyrrolidin-3-yl)oxy)-12-oxo-6a,7,9,10-tetrahydro-12*H*-pyrazino[2,1-*c*]pyrido[3,4-*f*][1,4]oxazepine-8(6*H*)-carboxylate (53 mg), dichloromethane (3 mL) and trifluoroacetic acid (1 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-3-(2-fluoro-6-hydroxyphenyl)-1-(((*R*)-1-methylpyrrolidin-3-yl)oxy)-6,6a,7,8,9,10-hexahydro-12*H*-pyrazino[2,1-*c*]pyrido[3,4-*f*][1,4]oxazepin-12-one (80 mg). ES-API: [M+H]⁺=463.9.

Step 3: (6a*R*)-4-Chloro-3-(2-fluoro-6-hydroxyphenyl)-1-(((*R*)-1-methylpyrrolidin-3-yl)oxy)-6,6a,7,8,9,10-hexahydro-12*H*-pyrazino[2,1-*c*]pyrido[3,4-*f*][1,4]oxazepin-12-one (80 mg), dichloromethane (5 mL) and *N*-ethyl-*N*-isopropylpropan-2-amine (25.8 mg, 0.21 mmol) were added to a 100 mL eggplant-shaped flask. The reaction was cooled to 0°C, and a solution of acryloyl chloride in dichloromethane (6.9 mg, 0.077 mmol, 0.5 mL) was added dropwise to the reaction solution, and the mixture was stirred at 0°C for 10 minutes. 20 mL of saturated aqueous sodium bicarbonate solution was added to the reaction solution, and the mixture was extracted three times with 10 mL of dichloromethane. The organic phase was dried and concentrated, and the crude product was purified by basic preparative HPLC to obtain (6aR)-8-acryloyl-4-chloro-3-(2-fluoro-6-hydroxyphenyl)-1-(((*R*)-1-methylpyrrolidin-3-yl)oxy)-6,6a,7,8,9,10-hexahydro-12*H*-pyrazino[2,1-*c*]pyrido[3,4-*f*][1,4]oxazepin-12-one (Z64, 5 mg, P: 99%, Y: 11%) as a white solid. ES-API: [M+H]⁺=517.2.

### Embodiment 47-3 Synthesis of (6aR)-8-acryloyl-4-chloro-3-(2-fluoro-6-hydroxyphenyl)-1-(((S)-1-methylpyrrolidin-3-yl)oxy)-6,6a,7,8,9,10-hexahydro-12H-pyrazino[2,1-c]pyrido[3,4-f][1,4]oxazepin-12-one Z65

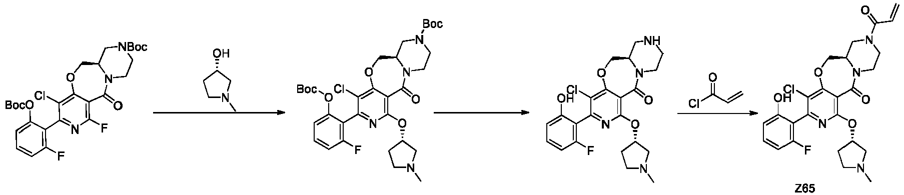

Step 1: 60% sodium hydride (17 mg, 0.43 mmol), (*S*)-1-methylpyrrolidin-3-ol (8.6 mg, 0.086 mmol) and tetrahydrofuran (2 mL) were added to a 100 mL three-necked round bottom flask. The reaction was cooled to 0°C, and a solution of *tert*-butyl (6aR)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*H*][1,4]oxazepine-8(6*H*)-carboxylate (50 mg, 0.086 mmol) in tetrahydrofuran (4 mL) was added dropwise thereto. The reaction was stirred in an ice bath at 0°C for 20 minutes and the completion of reaction was detected by LC-MS. 30 mL of saturated aqueous NH₄Cl solution was poured into the reaction solution, and the pH was adjusted to 5-6, and the mixture was extracted 4 times with 40 mL of ethyl acetate. The organic phase was dried and concentrated to obtain a crude product of *tert*-butyl (6aR)-3-(2-((*tert*-butoxycarbonyl)oxy)-6-fluorophenyl)-4-chloro-1-(((*S*)-1-methylpyrrolidin-3 -yl)oxy)-12-oxo-6a,7,9,10-tetrahydro-12*H*-pyrazino[2,1-*c*]pyrido[3,4-*f*][1,4]oxazepine-8(6*H*)-carboxylate (53 mg) as a yellow solid. ES-API: [M+H]⁺=663.3.

Step 2: The solid of *tert*-butyl (6aR)-3-(2-((*tert*-butoxycarbonyl)oxy)-6-fluorophenyl)-4-chloro-1-(((*S*)-1-methylpyrrolidin-3-yl)oxy)-12-oxo-6a,7,9,10-tetrahydro-12*H*-pyrazino[2,1-*c*]pyrido[3,4-*f*][1,4]oxazepine-8(6*H*)-carboxylate (53 mg), dichloromethane (3 mL) and trifluoroacetic acid (1 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-3-(2-fluoro-6-hydroxyphenyl)-1-(((*S*)-1-methylpyrrolidin-3-yl)oxy)-6,6a,7,8,9,10-hexahydro-12*H*-pyrazino[2,1-*c*]pyrido[3,4-*f*][1,4]oxazepin-12-one (80 mg). ES-API: [M+H]⁺=463.9.

Step 3: (6a*R*)-4-Chloro-3-(2-fluoro-6-hydroxyphenyl)-1-(((*S*)-1-methylpyrrolidin-3-yl)oxy)-6,6a,7,8,9,10-hexahydro-12*H*-pyrazino[2,1-*c*]pyrido[3,4-*f*][1,4]oxazepin-12-one (80 mg), dichloromethane (5 mL) and *N*-ethyl-*N*-isopropylpropan-2-amine (25.8 mg, 0.21 mmol) were added to a 100 mL eggplant-shaped flask. The reaction was cooled to 0°C, and a solution of acryloyl chloride in dichloromethane (6.9 mg, 0.077 mmol, 0.5 mL) was added dropwise to the reaction solution, and the mixture was stirred at 0°C for 10 minutes. 20 mL of saturated aqueous sodium bicarbonate solution was added to the reaction solution, and the mixture was extracted three times with 10 mL of dichloromethane. The organic phase was dried and concentrated, and the crude product was purified by basic preparative HPLC to obtain (6a*R*)-8-acryloyl-4-chloro-3-(2-fluoro-6-hydroxyphenyl)-1-(((*S*)-1-methylpyrrolidin-3-yl)oxy)-6,6a,7,8,9,10-hexahydro-12*H*-pyrazino[2,1-*c*]pyrido[3,4-*f*][1,4]oxazepin-12-one (Z65, 6 mg, P: 99%, Y: 13.4%) as a white solid. ES-API: [M+H]⁺=517.2.

### Embodiment 48 Synthesis of 8-acryloyl-4-chloro-3-(2-fluoro-6-hydroxyphenyl)-1-(4-hydroxy-2,2-dimethylpyrrolidin-1-yl)-6,6a,7,8,9,10-hexahydro-12H-pyrazino[2,1-c]pyrido[3,4-f][1,4]oxazepin-12-one and isomer compounds thereof (Z50, Z81, Z82)

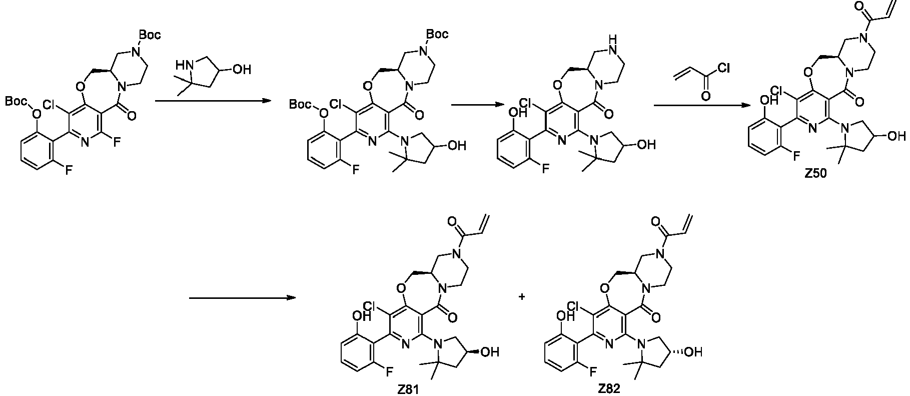

Step 1: 5,5-Dimethylpyrrolidin-3-ol (59 mg, 0.51 mmol) and potassium carbonate (214 mmol, 1.55 mmol) were added to a solution of *tert*-butyl (6a*R*)-1-fluoro-4-chloro-3-((2-((*tert*-butoxycarbonyloxy)-6-fluorophenyl)-12-oxo-6a,7,9,10-tetrahydro-12*H*-pyrazino[2,1-*c*]pyrido[3,4-*f*][1,4]oxazepine-8(6*H*)-carboxylate (300 mg, 0.51 mmol) in acetonitrile (5 mL), stirred at 80°C for 2 hours. The reaction solution was filtered and dissolved in 20 mL of ethyl acetate, washed with 20 mL of water, and the organic phase was dried and concentrated to obtain *tert*-butyl (6aR)-4-chloro-3-((2-*tert*-butoxycarbonyloxy)-6-fluorophenyl)-1-(4-hydroxy-2,2-dimethylpyrrolidin-1-yl)-12-oxo-6a,7,9,10-tetrahydro-12*H*-pyrazino [2,1-c]pyrido [3,4-*f*][1,4]oxazepine-8(6*H*)-carboxylate (250 mg, crude product) as a white solid. ES-API: [M+H]⁺=677.2.

Step 2: Under an ice bath, a solution of *tert*-butyl (6aR)-4-chloro-3-((2-*tert*-butoxycarbonyloxy)-6-fluorophenyl)-1-(4-hydroxy-2,2-dimethylpyrrolidin-1-yl)-12-oxo-6a,7,9,10-tetrahydro-12H-pyrazino[2,1-*c*]pyrido[3,4-*f*][1,4]oxazepine-8(6*H*)-carboxylate (250 mg, 0.37 mmol) in dichloromethane (4 mL) was added with trifluoroacetic acid (1 mL). The mixture was stirred at room temperature for 1 hour. After the reaction was completed, the mixture was concentrated to obtain a crude product of (6aR)-4-chloro-3-(2-fluoro-6-hydroxyphenyl)-1-(4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one, which was directly used in the next reaction step. ES-API: [M+H]⁺=477.1.

Step 3: Under an ice bath, the crude product obtained above was dissolved in dichloromethane (2 mL), and *N,N*-diisopropylethylamine (240 mg, 1.85 mmol) was added thereto. After the reaction solution was clarified, acryloyl chloride (33 mg, 0.37 mmol) was added dropwise thereto and stirred for 10 minutes. The reaction solution was concentrated and purified by preparative HPLC to obtain (6aR)-8-acryloyl-4-chloro-3 -(2-fluoro-6-hydroxyphenyl)-1-(4-hydroxy-2,2-dimethylpyrrolidin-1-yl)-6, 6 a, 7, 8,9,10-hexahydro-12*H*-pyrazino[2,1-*c*]pyrido[3,4-*f*][1,4]oxazepin-12-one (Z50, 85 mg, 43%) as a white solid. ES-API: [M+H]⁺=531.1.

Step 4: The Z50 obtained from the above steps was split by chiral chromatographic column (separation column: IE column 250 mm*4.6 mm*5 µm; mobile phase: ethanol:*n*-hexane = 40:60; flow rate: 2 mL/min; column temperature 30°C) to obtain two isomer compounds.

One isomer with a structure arbitrarily designated as (6aR)-8-acryloyl-4-chloro-3-(2-fluoro-6-hydroxyphenyl)-1-((*S*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z81, 26.66 mg, peak 1, retention time of 7.31 min, purity of 100%, de value: 100%). ES-API: [M+H]⁺=531.1. ¹H NMR (500 MHz, DMSO-*d*₆) δ 10.03-9.93 (m, 1H), 7.30-7.17 (m, 1H), 6.86-6.61 (m, 3H), 6.17 (d, *J=* 17.0 Hz, 1H), 5.78-5.70 (m, 1H), 4.60-4.45 (m, 1H), 4.39-3.69 (m, 10H), 3.26 (t, *J* = 9.2 Hz, 1H), 3.09-3.04 (m, 1H), 2.01-1.95 (m, 1H), 1.71 (t, *J* = 11.0 Hz, 1H), 1.48-1.41 (m, 6H).

Another isomer with a structure arbitrarily designated as (6a*R*)-8-acryloyl-4-chloro-3-(2-fluoro-6-hydroxyphenyl)-1-((*R*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z82, 50.61 mg, peak 2, retention time of 10.08 min, purity of 100%, de value: 100%). ES-API: [M+H]⁺=531.1. ¹H NMR (500 MHz, DMSO-*d*₆) δ 9.97 (brs, 1H), 7.31-7.18 (m, 1H), 6.85-6.64 (m, 3H), 6.17 (d, *J* = 16.6 Hz, 1H), 5.81-5.71 (m, 1H), 4.57-3.85 (m, 7H), 3.69-3.61 (m, 1H), 3.60-3.14 (m, 3H), 2.79 (d, *J* = 11.3 Hz, 1H), 1.92 (dd, *J* = 13.1, 4.1 Hz, 1H), 1.86 (d, *J* = 13.3 Hz, 1H), 1.70-1.64 (m, 3H), 1.42-1.37 (m, 3H).

The configuration of the hydroxyl on pyrrolidine in Z81 and Z82 was arbitrarily specified.

### Embodiment 49 Synthesis of (6aR)-8-acryloyl-4-chloro-3-(2-fluoro-6-hydroxyphenyl)-1-phenyl-6,6a,7,8,9,10-hexahydro-12H-pyrazino[2,1-c]pyrido[3,4-f]oxazepin-12-one Z53

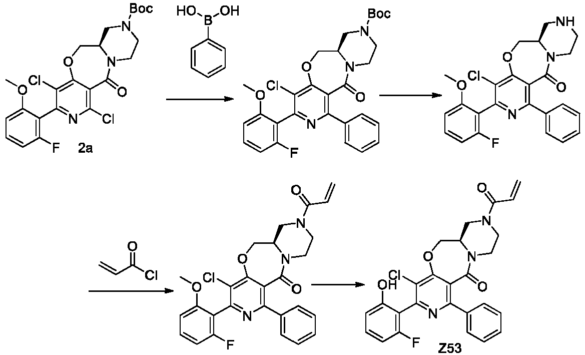

Step 1: *tert*-Butyl (6a*R*)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (240 mg, 0.4687 mmol), phenylboronic acid (240 mg, 1.967 mmol), tetrakis(triphenylphosphine)palladium (54 mg, 0.047 mmol) and sodium carbonate (250 mg, 2.343 mmol) were added to a mixed solution of dioxane (15 mL) and water (4 mL), reacted at 100°C for 1 hour under nitrogen protection. After the reaction was completed, the reaction solution was cooled to room temperature, added with ethyl acetate (200 mL), washed with saturated brine (2*80 mL). The organic phase was dried over anhydrous sodium sulfate, filtered, and the filtrate was evaporated to dryness by rotary evaporation under reduced pressure. The crude product was purified by flash silica gel column (EtOAc/PE: 0-100%) to obtain tert-butyl (6aR)-4-chloro-3-(2-fluoro-6-methoxyphenyl)-12-oxo-1-phenyl-6a,7,9,10-tetrahydro-12*H*-pyrazino[2,1-*c*]pyrido[3,4-*f*][1,4]oxazepine-8(6*H*)-carboxylate (233 mg, yield of 89.7%). ES-API: [M+H]⁺=554.2.

Step 2: Trifluoroacetic acid (2 mL) was added to a solution of *tert*-butyl (6a*R*)-4-chloro-3-(2-fluoro-6-methoxyphenyl)-12-oxo-1-phenyl-6a,7,9,10-tetrahydro-12*H*-pyrazino[2,1-*c*]pyrido[3,4-*f*][1,4]oxazepine-8(6*H*)-carboxylate (233 mg, 0.4212 mmol) in dichloromethane (8 mL). After stirring at room temperature for 2 hours, the reaction solution was concentrated to obtain (6a*R*)-4-chloro-3-(2-fluoro-6-methoxyphenyl)-1-phenyl-6,6a,7,8,9,10-hexahydro-12*H*-pyrazino[2,1-*c*]pyrido[3,4-*f*][1,4]oxazepin-12-one (300 mg, crude product), which was directly used in the next reaction step. ES-API: [M+H]⁺=454.1.

Step 3: (6a*R*)-4-Chloro-3-(2-fluoro-6-methoxyphenyl)-1-phenyl-6,6a,7,8,9,10-hexahydro-12H-pyrazino[2,1-*c*]pyrido[3,4-*f*][1,4]oxazepin-12-one (300 mg, crude product) obtained in step 8 was dissolved in dichloromethane (15 mL), and triethylamine (3.0 mL, 21.62 mmol) was added thereto. Acryloyl chloride (46 mg, 0.5083 mmol) was added dropwise to the reaction solution at 0 to 5°C. The reaction was stirred at 0°C for 15 minutes. The reaction solution was added with 80 mL of dichloromethane, washed with 100 mL of saturated aqueous NaHCO₃ solution and 80 mL of saturated brine, and the organic phase was dried over anhydrous sodium sulfate, concentrated, and purified by flash silica gel column (EtOAc/PE: 0-60%) to obtain (6a*R*)-8-acryloyl-4-chloro-3-(2-fluoro-6-methoxyphenyl)-1-phenyl-6,6a,7,8,9,10-hexahydro-12*H-*pyrazino[2,1-*c*]pyrido[3,4-*f*]oxazepin-12-one (233 mg, crude product). ES-API: [M+H]⁺=508.1.

Step 4: Under an ice-water bath, (6a*R*)-8-acryloyl-4-chloro-3-(2-fluoro-6-methoxyphenyl)-1-phenyl-6,6a,7,8,9,10-hexahydro-12*H*-pyrazino[2,1-*c*]pyrido[3,4-*f*]oxazepin-12-one (233 mg, crude product) obtained in step 3 was added to dichloromethane (10.0 mL), and then boron tribromide (20.0 mL, 1 M, 20.0 mmol) was added thereto, heated to room temperature and reacted overnight. Under an ice-water bath, the above reaction solution was added dropwise to saturated sodium bicarbonate solution, extracted twice with dichloromethane (80 mL), dried, concentrated, and the crude product was purified by preparation to obtain (6a*R*)-8-acryloyl-4-chloro-3-(2-fluoro-6-hydroxyphenyl))-1-phenyl-6,6a,7,8,9,10-hexahydro-12*H-*pyrazino[2,1-*c*]pyrido[3,4-*f*]oxazepin-12-one (Z53, 54 mg, yield of 26%). ES-API: [M+H]⁺=494.1. ¹H NMR (500 MHz, DMSO-*d*₆) δ 10.12 (d, *J* = 48.2 Hz, 1H), 7.59 (d, *J* = 3.8 Hz, 2H), 7.49-7.37 (m, 3H), 7.32 (dd, *J* = 15.4, 8.1 Hz, 1H), 6.80 (dd, *J* = 18.5, 9.3 Hz, 3H), 6.19 (d, *J* = 16.2 Hz, 1H), 5.76 (s, 1H), 4.56 (d, *J* = 59.2 Hz, 2H), 4.37 (d, *J* = 37.4 Hz, 1H), 4.08 (t, *J* = 49.4 Hz, 4H), 3.56 (d, *J* = 44.7 Hz, 2H).

### Embodiment 50-1 Synthesis of Z54

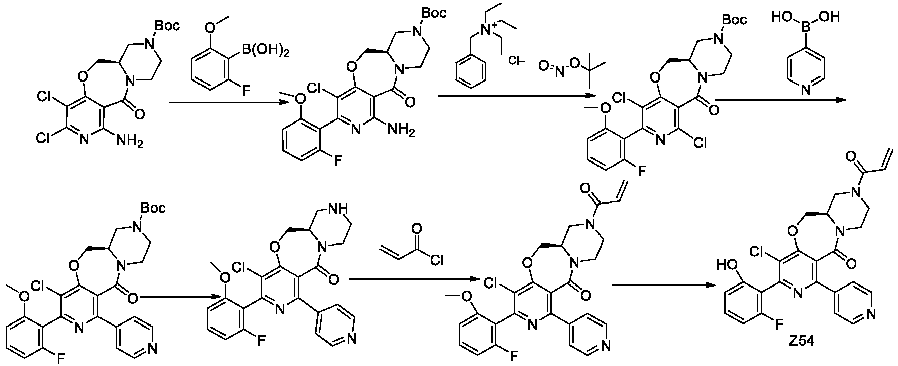

Step 1: (*R*)-1-Amino-3,4-dichloro-12-oxo-6a,7,9,10-tetrahydro-12*H*-pyrazino [2,1-*c*]pyrido [3,4-*f*][1,4]oxazepine-8(6*H*)-carboxylate (intermediate 2a, 4.06 g, 10.099 mmol), (2-fluoro-6-methoxyphenyl)boronic acid (6.01 g, 35.34 mmol), tetrakis(triphenylphosphine)palladium (1.15 g, 0.9956 mmol), sodium carbonate (5.0 g, 47.17 mmol), dioxane (100 mL) and water (20 mL) were added to a microwave tube. After the system was replaced with nitrogen for 4 times, the mixture was reacted at 100°C for 5 hours under nitrogen protection. After the reaction was completed, the reaction solution was cooled to room temperature, added with ethyl acetate (200 mL), washed twice with saturated brine (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-100%) to obtain a target product of *tert*-butyl (6a*R*)-1-amino-4-chloro-3-(2-fluoro-6-methoxyphenyl)-12-oxo-6a,7,9,10-tetrahydro-12*H*-pyrazino[2,1-*c*]pyrido [3,4-*f*][1,4]oxazepine-8(6*H*)-carboxylate (4.05 g, Y: 81.5%). ES-API: [M+H]⁺=493.1.

Step 2: *tert*-Butyl (6a*R*)-1-amino-4-chloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (4.05 g, 8.230 mmol) was added to dichloromethane (80 mL) at room temperature. After the dissolution, benzyltriethylammonium chloride (18.74 g, 82.30 mmol) was added thereto, stirred at room temperature for 5 minutes, and then *tert-*butyl nitrite (3.82 g, 37.03 mmol) was added thereto, stirred at room temperature overnight. After the reaction was completed, dichloromethane (100 mL) was added to the system, and washed twice with saturated aqueous sodium bicarbonate solution (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-50%) to obtain a target product of *tert*-butyl (6aR)-1,4-dichloro-3-(2-fluoro-6-methoxyphenyl)-12-oxo-6a,7,9,10-tetrahydro-12*H*-pyrazino[2,1-c]pyrido[3,4*f*][1,4]oxazepine-8(6*H*)-carboxylate (3.17 g, Y: 75%). ES-API: [M+H]⁺=512.2.

Step 3: *tert*-Butyl (6aR)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.3906 mmol), pyridine-4-boronic acid (154 mg, 1.255 mmol), tetrakis(triphenylphosphine)palladium (68 mg, 0.05859 mmol) and sodium carbonate (331 mg, 3 mmol) were added to a mixed solution of dioxane (20 mL) and water (5.0 mL). After the system was replaced with nitrogen for 4 times, the mixture was reacted at 100°C for 1 hour under nitrogen protection. After the reaction was completed, the reaction solution was cooled to room temperature, added with ethyl acetate (200 mL), washed twice with saturated brine (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-100%) to obtain a target product of *tert*-butyl (6a*R*)-4-chloro-3-(2-fluoro-6-methoxyphenyl)-12-oxo-1-(pyridin-4-yl)-6a,7,9,10-tetrahydro-12*H*-pyrazino [2,1-c]pyrido [3,4-*f*][1,4]oxazepine-8(6*H*)-carboxylate (236 mg, crude product). ES-API: [M+H]⁺=555.1.

Step 4: Trifluoroacetic acid (2 mL) was added to a solution of *tert*-butyl (6a*R*)-4-chloro-3-(2-fluoro-6-methoxyphenyl)-12-oxo-1-(pyridin-4-yl)-6a,7,9,10-tetrahydro-12*H*-pyrazino [2,1-c]pyrido [3,4-*f*][1,4]oxazepine-8(6*H*)-carboxylate (236 mg, crude product) in dichloromethane (8 mL). After stirring at room temperature for 2 hours, the reaction solution was concentrated under reduced pressure to obtain a crude target product of (6a*R*)-4-chloro-3-(2-fluoro-6-methoxyphenyl)-1-(pyridin-4-yl)-6,6a,7,8,9,10-hexahydro-12*H*-pyrazino[2,1-*c*]pyrido[3,4-*f*][1,4]oxazepin-12-one (243 mg, crude product), which was directly used in the next reaction step. ES-API: [M+H]⁺=455.0.

Step 5: (6aR)-4-Chloro-3-(2-fluoro-6-methoxyphenyl)-1-(pyridin-4-yl)-6,6a,7,8,9,10-hexahydro-12*H*-pyrazino[2,1-*c*]pyrido[3,4-*f*][1,4]oxazepin-12-one (243 mg, crude product) obtained in step 8 was dissolved in dichloromethane (10 mL), and triethylamine (3.0 mL, 21.62 mmol) was added thereto. The reaction solution was cooled to 0 to 5°C, and acryloyl chloride (47 mg, 0.5193 mmol) was added dropwise to the reaction solution. The reaction was stirred at 0°C for 15 minutes. The reaction solution was added with 80 mL of dichloromethane, washed with 100 mL of saturated aqueous sodium bicarbonate solution and 80 mL of saturated brine, and the organic phase was dried over anhydrous sodium sulfate, then concentrated, and the crude product was purified by flash silica gel column (ethyl acetate/petroleum ether: 0-60%) to obtain a target product of (6a*R*)-8-acryloyl-4-chloro-3-(2-fluoro-6-methoxyphenyl)-1-(pyridin-4-yl)-6,6a,7,8,9,10-hexahydro-12*H*-pyrazino[2,1-*c*]pyrido[3,4-*f*]oxazepin-12-one (136 mg, 2-step yield of 68%). ES-API: [M+H]⁺=509.2.

Step 6: Under an ice-water bath, (6a*R*)-8-acryloyl-4-chloro-3-(2-fluoro-6-methoxyphenyl)-1-(pyridin-4-yl)-6,6a,7,8,9,10-hexahydro-12*H*-pyrazino[2,1-*c*]pyrido[3,4-*f*]oxazepin-12-one (136 mg, 0.2676 mmol) obtained in step 9 was added to dry dichloromethane (10.0 mL), and then boron tribromide (20.0 mL, 1 M, 20.0 mmol) was added thereto, heated to room temperature and reacted overnight. Under an ice-water bath, the above reaction solution was added dropwise to saturated sodium bicarbonate solution, extracted twice with dichloromethane (80 mL), dried, concentrated, and the crude product was purified by preparation to obtain a target product of (6a*R*)-8-acryloyl-4-chloro-3-(2-fluoro-6-hydroxyphenyl))-1-(pyridin-4-yl)-6,6a,7,8,9,10-hexahydro-12*H*-pyrazino[2,1-*c*]pyrido[3,4-*f*]oxazepin-12-one (Z54, 17 mg, Y: 12.8%). ES-API: [M+H]⁺=495.1. ¹H NMR (500 MHz, DMSO-*d*₆) δ 10.16 (d, *J* = 43.7 Hz, 1H), 8.63 (d, *J* = 6.0 Hz, 2H), 7.58 (d, *J* = 4.3 Hz, 2H), 7.33 (dd, *J* = 15.6, 7.8 Hz, 1H), 6.79 (dt, *J* = 21.2, 8.9 Hz, 3H), 6.19 (d, *J* = 16.6 Hz, 1H), 5.76 (s, 1H), 4.71 - 4.51 (m, 2H), 4.42 (d, *J* = 33.9 Hz, 1H), 4.23 - 3.84 (m, 4H), 3.65 (s, 2H).

### Embodiment 50-2 Synthesis of Z55

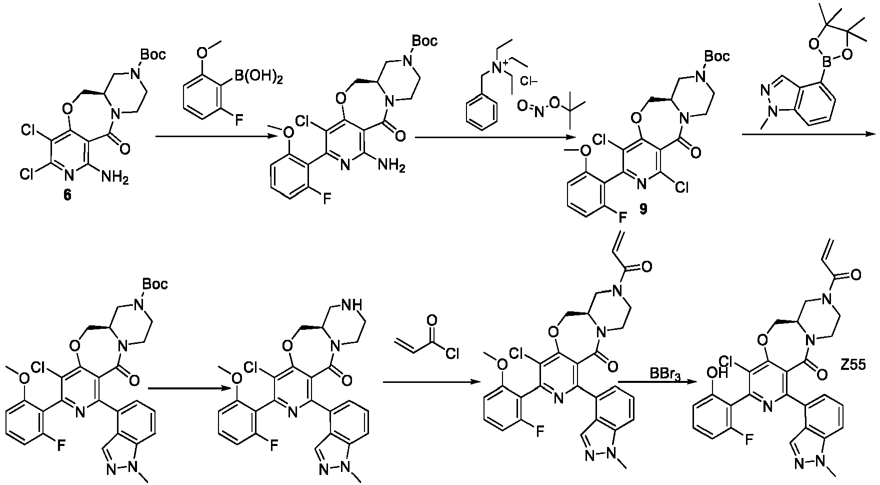

Step 1: (*R*)-1 -Amino-3,4-dichloro-12-oxo-6a,7,9,10-tetrahydro-12*H*-pyrazino [2,1-*c*]pyrido [3,4-*f*][1,4]oxazepine-8(6*H*)-carboxylate (4.06 g, 10.099 mmol), (2-fluoro-6-methoxyphenyl)boronic acid (6.01 g, 35.34 mmol), tetrakis(triphenylphosphine)palladium (1.15 g, 0.9956 mmol), sodium carbonate (5.0 g, 47.17 mmol), dioxane (100 mL) and water (20 mL) were added to a microwave tube. After the system was replaced with nitrogen for 4 times, the mixture was reacted at 100°C for 5 hours under nitrogen protection. After the reaction was completed, the reaction solution was cooled to room temperature, added with ethyl acetate (200 mL), washed twice with saturated brine (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-100%) to obtain a target product of *tert*-butyl (6aR)-1-amino-4-chloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (4.05 g, Y: 81.5%). ES-API: [M+H]⁺=493.1.

Step 2: *tert*-Butyl (6aR)-1-amino-4-chloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (4.05 g, 8.230 mmol) was added to dichloromethane (80 mL) at room temperature. After the dissolution, benzyltriethylammonium chloride (18.74 g, 82.30 mmol) was added thereto, stirred at room temperature for 5 minutes, and then *tert-*butyl nitrite (3.82 g, 37.03 mmol) was added thereto, stirred at room temperature overnight. After the reaction was completed, dichloromethane (100 mL) was added to the system, and washed twice with saturated aqueous sodium bicarbonate solution (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-50%) to obtain a target product of *tert*-butyl (6a*R*)-1,4-dichloro-3-(2-fluoro-6-methoxyphenyl)-12-oxo-6a,7,9,10-tetrahydro-12*H*-pyrazino[2,1-c]pyrido[3,4*f*][1,4]oxazepine-8(6*H*)-carboxylate (3.17 g, Y: 75%). ES-API: [M+H]⁺=512.2.

Step 3: *tert*-Butyl (6a*R*)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (150 mg, 0.2929 mmol), 1-methyl-4-(4,4,5,5-tetramethyl-1,3,2-dioxaboran-2-yl)-1*H*-indazole (302 mg, 1.1698 mmol), tetrakis(triphenylphosphine)palladium (35 mg, 0.030 mmol) and sodium carbonate (155 mg, 1.462 mmol) were added to a mixed solution of dioxane (15 mL) and water (4 mL). After the system was replaced with nitrogen for 4 times, the mixture was reacted at 100°C for 1 hour under nitrogen protection. After the reaction was completed, the reaction solution was cooled to room temperature, added with ethyl acetate (200 mL), washed twice with saturated brine (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-100%) to obtain a target product of *tert*-butyl (6a*R*)-4-chloro-3-(2-fluoro-6-methoxyphenyl)-1-(1-methyl-1*H*-indazol-4-yl)-12-oxo-6a,7,9,10-tetrahydro-12*H*-pyrazino[2,1-*c*]pyrido[3,4-*f*][1,4]oxazepine-8(6*H*)-carboxylate (230 mg, crude product). ES-API: [M+H]⁺=608.2.

Step 4: Trifluoroacetic acid (2 mL) was added to a solution of *tert*-butyl (6a*R*)-4-chloro-3-(2-fluoro-6-methoxyphenyl)-1-(1-methyl-1*H*-indazol-4-yl)-12-oxo-6a,7,9,10-tetrahydro-12*H*-pyrazino[2,1-*c*]pyrido[3,4-*f*][1,4]oxazepine-8(6*H*)-carboxylate (230 mg, crude product) in dichloromethane (8 mL). After stirring at room temperature for 2 hours, the reaction solution was concentrated under reduced pressure to obtain a crude target product of (6a*R*)-4-chloro-3-(2-fluoro-6-methoxyphenyl)-1-(1-metliyl-1*H*-indazol-4-yl)-6,6a,7,8,9,10-hexahydro-12*H*-pyrazino[2,1-*c*]pyrido[3,4-*f*][1,4]oxazepin-12-one (257 mg, crude product), which was directly used in the next reaction step. ES-API: [M+H]⁺=508.1.

Step 5: (6a*R*)-4-Chloro-3-(2-fluoro-6-methoxyphenyl)-1-(1-methyl-1*H*-indazol-4-yl)-6,6a,7,8,9,10-hexahydro-12*H*-pyrazino[2,1-*c*]pyrido[3,4-*f*][1,4]oxazepin-12-one (257 mg, crude product) obtained in step 8 was dissolved in dichloromethane (10 mL), and triethylamine (3.0 mL, 21.62 mmol) was added thereto. The reaction solution was cooled to 0 to 5°C, and acryloyl chloride (42 mg, 0.4641 mmol) was added dropwise to the reaction solution. The reaction was stirred at 0°C for 15 minutes. The reaction solution was added with 80 mL of dichloromethane, washed with 100 mL of saturated aqueous NaHCO₃ solution and 80 mL of saturated brine, and the organic phase was dried over anhydrous sodium sulfate, then concentrated, and the crude product was purified by flash silica gel column (ethyl acetate/petroleum ether: 0-60%) to obtain a target product of (6a*R*)-8-acryloyl-4-chloro-3-(2-fluoro-6-methoxyphenyl)-1-(1-methyl-1*H*-indazol-4-yl)-6,6a,7,8,9,10-hexahydro-12*H*-pyrazino[2,1-*c*]pyrido[3,4-*f*]oxazepin-12-one (180 mg, 2-step yield of 91%). ES-API: [M+H]⁺=562.2.

Step 6: Under an ice-water bath, (6a*R*)-8-acryloyl-4-chloro-3-(2-fluoro-6-methoxyphenyl)-1-(1-methyl-1*H*-indazol-4-yl)-6,6a,7,8,9,10-hexahydro-12*H*-pyrazino[2,1-*c*]pyrido[3,4-*f*]oxazepin-12-one (180 mg, 0.3208 mmol) obtained in step 9 was added to dry dichloromethane (10.0 mL), and then boron tribromide (20.0 mL, 1 M, 20.0 mmol) was added thereto, heated to room temperature and reacted overnight. Under an ice-water bath, the above reaction solution was added dropwise to saturated sodium bicarbonate solution, extracted twice with dichloromethane (80 mL), dried, concentrated, and the crude product was purified by preparation to obtain a target product of (6a*R*)-8-acryloyl-4-chloro-3-(2-fluoro-6-hydroxyphenyl)-1-(2-fluoro-6-methoxyphenyl)-6,6a,7, 8,9,10-hexahydro-12*H*-pyrazino [2,1-*c*]pyrido [3,4-*f*]oxazepin-12-one (Z55, 7 mg, Y: 4%). ES-API: [M+H]⁺=548.2. ¹H NMR (500 MHz, DMSO-*d*₆) δ 10.23 (d, *J* = 98.6 Hz, 1H), 8.02 (d, *J* = 71.9 Hz, 1H), 7.69 (d, *J =* 8.4 Hz, 1H), 7.42 (t, *J* = 7.7 Hz, 1H), 7.33 (dd, *J* = 15.3, 8.2 Hz, 1H), 7.25 (d, *J* = 6.6 Hz, 1H), 6.93 - 6.72 (m, 3H), 6.20 (d, *J* = 15.9 Hz, 1H), 5.77 (s, 1H), 4.74 (s, 1H), 4.56 (s, 1H), 4.41 (d, *J* = 32.1 Hz, 1H), 4.12 (d, *J* = 50.2 Hz, 6H), 3.54 (d, *J* = 52.9 Hz, 2H), 3.30 (s, 1H).

### Embodiment 50-3 Synthesis of Z67

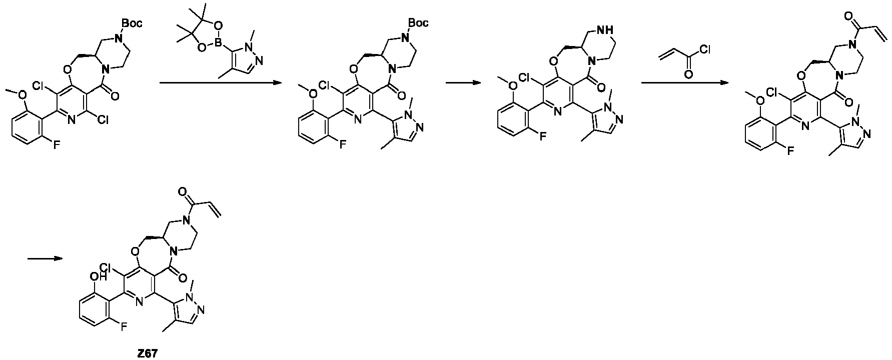

Step 1: Under nitrogen protection, a mixture of compound *tert*-butyl (6aR)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.39 mmol), 1,4-dimethyl-5-(4,4,5,5-tetramethyl-1,3,2-dioxaboran-2-yl)-1*H-*pyrazole (260 mg, 1.17 mmol), tetrakis(triphenylphosphine)palladium (45 mg, 0.04 mmol) and potassium carbonate (162 mg, 1.17 mmol) in 1,4-dioxane (4 mL) and water (1 mL) was reacted at 110°C for 30 minutes under microwave irradiation. The reaction solution was poured into water (10 mL), extracted with ethyl acetate (10 mL * 3), and the organic phases were combined, washed with saturated brine (10 mL), dried and concentrated, and then purified by flash silica gel column (ethyl acetate:petroleum ether: 0-100%) to obtain *tert*-butyl (6a*R*)-4-chloro-1-(1,4-dimethyl-1*H*-pyrazol-5-yl)-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg). ES-API: [M+H]⁺=572.2.

Step 2: Under an ice bath, a solution of *tert*-butyl (6a*R*)-4-chloro-1-(1,4-dimethyl-1*H*-pyrazol-5-yl)-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.35 mmol) in dichloromethane (4 mL) was added with trifluoroacetic acid (1 mL), and the reaction solution was stirred at room temperature for 1 hour. The reaction solution was concentrated under reduced pressure to obtain a crude product of (6a*R*)-4-chloro-1-(1,4-dimethyl-1*H*-pyrazol-5-yl)-3-(2-fluoro-6-methoxyphenyl)-6,6a,7,8,9,10-hexahydro-12*H-*pyrazino[2,1-*c*]pyrido[3,4-*f*][1,4]oxazepin-12-one (165 mg). ES-API: [M+H]⁺=472.1.

Step 3: The above crude product of (6a*R*)-4-chloro-1-(1,4-dimethyl-1*H*-pyrazol-5-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (165 mg, 0.35 mmol) was dissolved in dichloromethane (2 mL), and *N,N*-diisopropylethylamine (225 mg, 1.75 mmol) was added thereto. The reaction solution was cooled to 0°C, and acryloyl chloride (32 mg, 0.35 mmol) was added dropwise thereto. After stirring for 10 minutes, the mixture was concentrated and the crude product was purified by flash silica gel column (ethyl acetate:petroleum ether: 0-100%) to obtain (6a*R*)-8-acryloyl-4-chloro-1-(1,4-dimethyl-1*H*-pyrazol-5-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (150 mg) as a white solid. ES-API: [M+H]⁺=501.2.

Step 4: Under an ice bath, a solution of boron tribromide in dichloromethane (2 M, 2 mL, 4 mmol) was added dropwise to a solution of (6a*R*)-8-acryloyl-4-chloro-1-(1,4-dimethyl-1*H*-pyrazol-5-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (150 mg, 0.29 mmol) in dichloromethane (2 mL), stirred at room temperature for 2 hours. After the reaction was completed, the mixture was quenched with saturated sodium bicarbonate solution (20 mL) under an ice bath, extracted with dichloromethane (20 mL), and the organic phase was dried, concentrated and purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(1,4-dimethyl-1*H*-pyrazol-5-yl)-3 -(2-fluoro-6-hydroxyphenyl)-6,6a,7, 8,9,10-hexahydro-12H-pyrazino [2,1-c]pyrido [3,4-*f*][1,4]oxazepin-12-one (Z67, 39 mg, yield: 27%). ES-API: [M+H]⁺ = 512.1. ¹H NMR (500 MHz, DMSO-*d*₆) δ 7.32 (dd, *J=* 15.5, 7.5 Hz, 1H), 7.24 (s, 1H), 6.85 - 6.70 (m, 3H), 6.23 - 6.11 (m, 1H), 5.80 - 5.66 (m, 1H), 4.66 - 4.38 (m, 3H), 4.15 - 3.77 (m, 4H), 3.71 - 3.63 (m, 4H), 3.59 - 3.46 (m, 1H), 1.90 - 1.79 (m, 3H).

### Embodiment 50-4 Synthesis of Z74

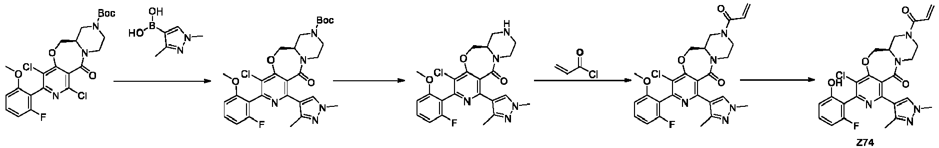

Step 1: *tert*-Butyl (6a*R*)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.39 mmol), (1,3-dimethyl-1*H*-pyrazol-4-yl)boronic acid (219 mg, 1.56 mmol), tetrakis(triphenylphosphine)palladium (45 mg, 0.039 mmol), sodium carbonate (124 mg, 1.17 mmol), 5 mL of dioxane and 1 mL of water were added to a 100 mL reaction flask. The reaction was stirred in an oil bath at 110°C for 2 hours, and the reaction was stopped. The reaction was added with 20 mL of water, extracted three times with 20 mL of ethyl acetate. The organic phase was dried and concentrated, and the crude product was purified by flash silica gel column (ethyl acetate/petroleum ether: 0-60%) to obtain *tert*-butyl (6a*R*)-4-chloro-1-(1,3-dimethyl-1*H*-pyrazol-4-yl)-3-(2-fluoro-6-methoxyphenyl)-12-oxo-6a,7,9,10-tetrahydro-12*H*-pyrazino[2,1-*c*]pyrido [3,4-*f*][1,4]oxazepine-8(6*H*)-carboxylate (190 mg, 85%) as a yellow solid. ES-API: [M+H]⁺=572.2.

Step 2: The solid *tert*-butyl (6a*R*)-4-chloro-1-(1,3-dimethyl-1*H*-pyrazol-4-yl)-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (190 mg, 0.33 mmol), dichloromethane (3 mL) and trifluoroacetic acid (2 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-1-(1,3-dimethyl-1*H*-pyrazol-4-yl)-3-(2-fluoro-6-methoxyphenyl)-6,6a,7,8,9,10-hexahydro-12*H*-pyrazino[2,1-c]pyrido[3,4-*f*][1,4]oxazepin-12-one (210 mg). ES-API: [M+H]⁺=472.9.

Step 3: (6a*R*)-4-Chloro-1-(1,3-dimethyl-1*H*-pyrazol-4-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (210 mg), dichloromethane (5 mL) and N-ethyl-N-isopropylpropan-2-amine (128.5 mg, 0.99 mmol) were added to a 100 mL eggplant-shaped flask. The reaction was cooled to 0°C, and a solution of acrylic anhydride in dichloromethane (24.3 mg, 0.27 mmol, 0.5 mL) was added to the reaction solution. The reaction was stirred at 0°C for 10 minutes, then 20 mL of saturated aqueous sodium bicarbonate solution was added to the reaction solution, and the mixture was extracted three times with 10 mL of dichloromethane. The organic phase was dried and concentrated, and the crude product was purified by an automatic column chromatography machine (40 g, mobile phase gradient: methanol/dichloromethane = 6%-8%) to obtain a yellow solid of (6a*R*)-8-acryloyl-4-chloro-1-(1,3-dimethyl-1*H*-pyrazol-4-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (150 mg, 86.7%). ES-API: [M+H]⁺= 526.2.

Step 4: (6a*R*)-8-Acryloyl-4-chloro-1-(1,3-dimethyl-1*H*-pyrazol-4-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (150 mg, 0.28 mmol) was dissolved in dichloromethane (1.5 mL). The reaction was cooled to 0°C, and a 1 mol/L solution of boron tribromide in tetrahydrofuran (5 mL) was added dropwise thereto. The reaction was stirred at room temperature for 5 hours, and the reaction solution was poured into 40 mL of saturated NaHCO₃ ice-water solution, extracted twice with 25 mL of dichloromethane. The organic phase was dried and concentrated, and the crude product was purified by basic preparative HPLC to obtain a pale yellow solid of (6a*R*)-8-acryloyl-4-chloro-1-(1,3-dimethyl-1*H*-pyrazol-4-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z74, 42 mg, P: 99% Y: 29.3%). ES-API: [M+H]⁺=512.2. ¹HNMR (500 MHz, DMSO-*d*₆) *δ* 10.16-9.98 (m, 1H), 7.84 (s, 1H), 7.29 (dd, *J* = 15.0, 8.0 Hz, 1H), 6.86 - 6.69 (m, 3H), 6.18 (d, *J* = 15.0 Hz, 1H), 5.80-5.67 (m, 1H), 4.50 - 3.99 (m, 6H), 3.76 (s, 3H), 3.64-3.35 (m, 3H), 2.15 (s, 3H).

### Embodiment 50-5 Synthesis of Z77

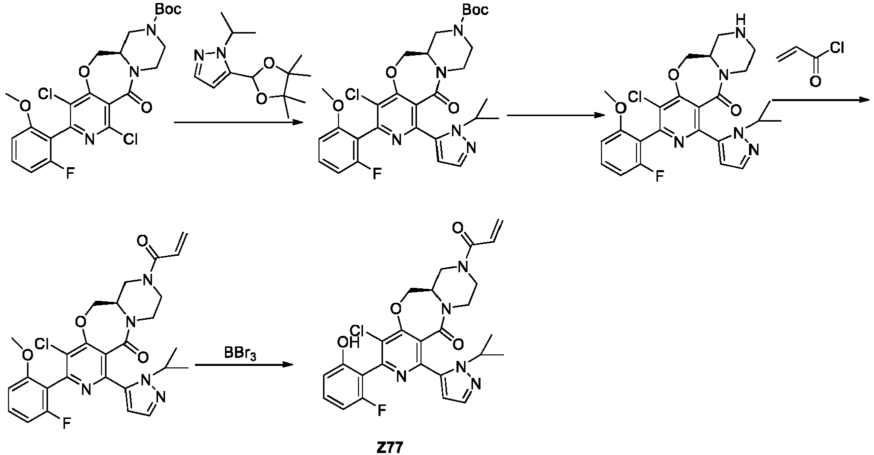

Step 1: *tert*-Butyl (6aR)-1,4-dichloro-3-(2-fluoro-6-methoxyphenyl)-12-oxo-6a,7,9,10-tetrahydro-12*H*-pyrazino[2,1-*c*]-pyrido[3,4-*f*][1,4]oxazepine-8(6*H*)-carboxylate (256 mg, 0.5 mmol), 1-isopropyl-5-(4,4,5,5-tetramethyl-1,3-dioxolan-2-yl)-1*H*-pyrazole (354 mg, 1.5 mmol), tetrakis(triphenylphosphine)palladium (57 mg, 0.05 mmol), potassium carbonate (206 mg, 1.5 mmol), dioxane (10 mL) and water (1 mL) were added to a 50 mL round bottom flask, reacted at 110°C for 2 hours under nitrogen protection. The reaction solution was added with ethyl acetate (30 mL), washed with water (30 mL*3), and the organic phase was dried and concentrated, and the residue was purified by flash silica gel column (ethyl acetate:petroleum ether: 0-90%) to obtain a solid of tert-butyl (6a*R*)-4-chloro-3-(2-fluoro-6-methoxyphenyl)-1-(1-isopropyl-1*H*-pyrazol-5-yl)-12-oxo-6a,7,9,10-tetrahydro-12*H-*pyrazino[2,1-*c*]pyrido[3,4-*f*][1,4]oxazepine-8(6*H*)-carboxylate (270 mg, yield of 90%). ES-API: [M+H]⁺=586.2.

Step 2: Trifluoroacetic acid (2 mL) was added to a solution of *tert*-butyl (6a*R*)-4-chloro-3-(2-fluoro-6-methoxyphenyl)-1-(1-isopropyl-1*H*-pyrazol-5-yl)-12-oxo-6a,7,9,10-tetrahydro-12*H*-pyrazino[2,1-*c*]pyrido[3,4-*f*][1,4]oxazepine-8(6*H*)-carboxylate (270 mg, 0.46 mmol) in dichloromethane (5 mL), reacted at room temperature for 1 hour. The reaction solution was directly concentrated to obtain a crude product of (6a*R*)-4-chloro-3-(2-fluoro-6-methoxyphenyl)-1-(1-isopropyl-1*H*-pyrazol-5-yl)-6,6a,7,8,9,10-hexahydro-12*H*-pyrazino[2,1-*c*]pyrido[3,4-*f*][1,4]oxazepin-12-one (220 mg) as an oil. ES-API: [M+H]⁺=486.2.

Step 3: The above crude procuct of (6a*R*)-4-chloro-3-(2-fluoro-6-methoxyphenyl)-1-(1-isopropyl-1*H*-pyrazol-5-yl)-6,6a,7,8,9,10-hexahydro-12*H*-pyrazino[2,1-*c*]pyrido[3,4-*f*][1,4]oxazepin-12-one (220 mg, 0.45 mmol) as an oil was dissolved in dichloromethane (10 mL), and *N,N*-diisopropylethylamine (170 mg, 1.3 mmol) was added thereto. The reaction solution was cooled to 0°C, and acryloyl chloride (40 mg, 0.45 mmol) was added dropwise thereto. After stirring for 5 minutes, the mixture was added with methanol (1 mL) to quench and then concentrated, and the crude product was purified by flash silica gel column (methanol:dichloromethane: 0-10%) to obtain (6a*R*)-8-acryloyl-4-chloro-3-(2-fluoro-6-methoxyphenyl)-1-(1-isopropyl-1*H*-pyrazol-5-yl)-6,6a,7,8,9,10-hexahydro-12*H*-pyrazino[2,1-*c*]pyrido[3,4-*f*][1,4]oxazepin-12-one (120 mg, yield of 50%). ES-API: [M+H]⁺=540.2.

Step 4: The above product (6a*R*)-8-acryloyl-4-chloro-3-(2-fluoro-6-methoxyphenyl)-1-(1-isopropyl-1*H*-pyrazol-5-yl)-6,6a,7,8,9,10-hexahydro-12*H*-pyrazino[2,1-*c*]pyrido[3,4-*f*][1,4]oxazepin-12-one (120 mg, 0.22 mmol) was dissolved in dichloromethane (3 mL), and the reaction solution was cooled to 0°C, and a 1 M solution of boron tribromide in dichloromethane (3 mL) was added thereto. After stirring for 2 hours, the reaction solution was added with dichloromethane (30 mL), washed with water (30 mL*3). The organic phase was dried and concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(1-isopropyl-1*H-*pyrazol-5-yl)-6,6a,7,8,9,10-hexahydro-12*H*-pyrazino[2,1-*c*]pyrido[3,4-*f*][1,4]oxazepin-12-one (Z77, 75 mg, yield of 31%). ES-API: [M+H]⁺=526.2. ¹H NMR (500 MHz, DMSO-*d*₆) δ 10.17 (s, 1H), 7.45 (d, J= 1.7 Hz, 1H), 7.32 (dd, *J* = 15.5, 7.9 Hz, 1H), 6.90 - 6.70 (m, 3H), 6.40 (s, 1H), 6.19 (d, *J* = 16.5 Hz, 1H), 5.75 (s, 1H), 4.84 - 3.81 (m, 8H), 3.59 (s, 2H), 1.34 (dd, J= 49.1, 6.4 Hz, 6H).

### Embodiment 50-6 Synthesis of Z79

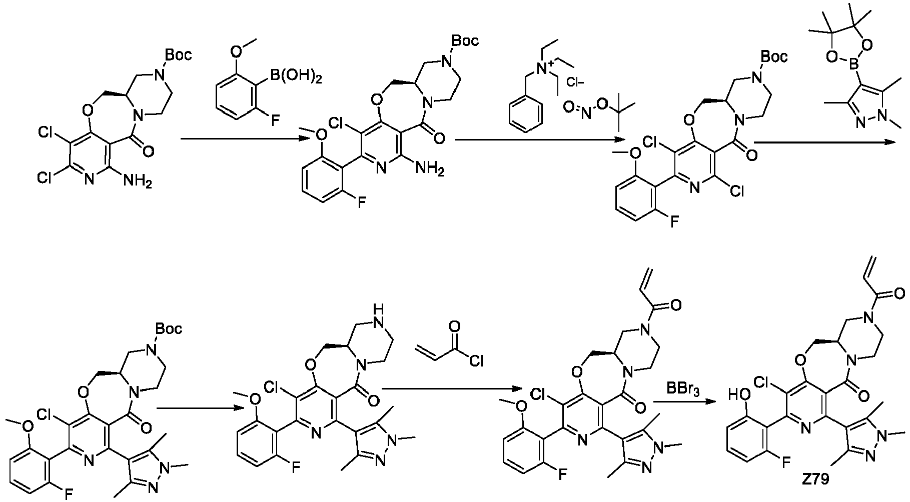

Step 1: (*R*)-1 -Amino-3,4-dichloro-12-oxo-6a,7,9,10-tetrahydro-12*H*-pyrazino [2,1-*c*]pyrido [3,4-*f*][1,4]oxazepine-8(6*H*)-carboxylate (4.06 g, 10.099 mmol), (2-fluoro-6-methoxyphenyl)boronic acid (6.01 g, 35.34 mmol), tetrakis(triphenylphosphine)palladium (1.15 g, 0.9956 mmol), sodium carbonate (5.0 g, 47.17 mmol), dioxane (100 mL) and water (20 mL) were added to a microwave tube. After the system was replaced with nitrogen for 4 times, the mixture was reacted at 100°C for 5 hours under nitrogen protection. After the reaction was completed, the reaction solution was cooled to room temperature, added with ethyl acetate (200 mL), washed twice with saturated brine (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-100%) to obtain a target product of *tert*-butyl (6aR)-1-amino-4-chloro-3-(2-fluoro-6-methoxyphenyl)-12-oxo-6a,7,9,10-tetrahydro-12*H*-pyrazino[2,1*-c*]pyrido[3,4-*f*][1,4]oxazepine-8(6*H*)-carboxylate (4.05 g, Y: 81.5%). ES-API: [M+H]⁺=493.1.

Step 2: *tert*-Butyl (6a*R*)-1-amino-4-chloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (4.05 g, 8.230 mmol) was added to dichloromethane (80 mL) at room temperature. After the dissolution benzyltriethylammonium chloride (18.74 g, 82.30 mmol) was added thereto, stirred at room temperature for 5 minutes, and then *tert-*butyl nitrite (3.82 g, 37.03 mmol) was added thereto, stirred at room temperature overnight. After the reaction was completed, dichloromethane (100 mL) was added to the system, and washed twice with saturated aqueous sodium bicarbonate solution (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-50%) to obtain a target product of *tert*-butyl (6aR)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (3.17 g, Y: 75%). ES-API: [M+H]⁺=512.2.

Step 3: *tert*-Butyl (6aR)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.4687 mmol), 1,3,5-trimethyl-4-(4,4,5,5-tetramethyl-1,3,2-dioxaborolan-2-yl)-1*H*-pyrazole (300 mg, 1.270 mmol), tetrakis(triphenylphosphine)palladium (45 mg, 0.03906 mmol) and sodium carbonate (210 mg, 1.981 mmol) were added to a mixed solution of dioxane (15 mL) and water (4 mL). After the system was replaced with nitrogen for 4 times, the mixture was reacted at 100°C for 1 hour under nitrogen protection.

After the reaction was completed, the reaction solution was cooled to room temperature, added with ethyl acetate (200 mL), washed twice with saturated brine (2*80 mL). The organic phase was dried over anhydrous sodium sulfate, filtered, and the filtrate was evaporated to dryness by rotary evaporation under reduced pressure. The crude product was purified by flash silica gel column (ethyl acetate/petroleum ether: 0-100%) to obtain a target product of *tert*-butyl (6a*R*)-4-chloro-3-(2-fluoro-6-methoxyphenyl)-12-oxo-1-(1,3,5-trimethyl-1*H*-pyrazol-4-yl)-6a,7,9,10-tetrahydro-12*H*-pyrazino[2,1-*c*]pyrido[3,4-*f*][1,4]oxazepine-8(6*H*)-carboxylate (680 mg, crude product). ES-API: [M+H]⁺=586.1.

Step 4: Trifluoroacetic acid (3 mL) was added to a solution of *tert*-butyl (6a*R*)-4-chloro-3-(2-fluoro-6-methoxyphenyl)-12-oxo-1-(1,3,5-trimethyl-1*H*-pyrazol-4-yl)-6a,7,9,10-tetrahydro-12*H-*pyrazino[2,1-*c*]pyrido[3,4-*f*][1,4]oxazepine-8(6*H*)-carboxylate (680 mg, crude product) in dichloromethane (12 mL). After stirring at room temperature for 2 hours, the reaction solution was concentrated under reduced pressure to obtain a crude target product of (6aR)-4-chloro-3-(2-fluoro-6-methoxyphenyl)-1-(1,3,5-trimethyl-1*H*-pyrazol-4-yl)-6,6a,7,8,9,10-hexahydro-12*H*-pyrazino[2,1-*c*]pyrido[3,4-*f*][1,4]oxazepin-12-one (720 mg, crude product), which was directly used in the next reaction step. ES-API: [M+H]⁺=486.1.

Step 5: (6a*R*)-4-Chloro-3-(2-fluoro-6-methoxyphenyl)-1-(1,3,5-trimethyl-1*H*-pyrazol-4-yl)-6,6a,7,8,9,10-hexahydro-12*H*-pyrazino[2,1-*c*]pyrido[3,4-*f*][1,4]oxazepin-12-one (720 mg, crude product) obtained in step 8 was dissolved in dichloromethane (15 mL), and triethylamine (3.0 mL, 21.62 mmol) was added thereto. The reaction solution was cooled to 0 to 5°C, and acryloyl chloride (70 mg, 0.7735 mmol) was added dropwise to the reaction solution. The reaction was stirred at 0°C for 15 minutes. The reaction solution was added with 80 mL of dichloromethane, washed with 100 mL of saturated aqueous NaHCO₃ solution and 80 mL of saturated brine, and the organic phase was dried over anhydrous sodium sulfate, then concentrated, and the crude product was purified by flash silica gel column (ethyl acetate/petroleum ether: 0-60%) to obtain a target product of (6a*R*)-8-acryloyl-4-chloro-3-(2-fluoro-6-methoxyphenyl)-1-(1,3,5-trimethyl-1*H*-pyrazol-4-yl)-6,6a,7,8,9,10-hexahydro-12*H*-pyrazino[2,1-*c*]pyrido[3,4-*f*]oxazepin-12-one (230 mg, crude product). ES-API: [M+H]⁺=540.1.

Step 6: Under an ice-water bath, (6a*R*)-8-acryloyl-4-chloro-3-(2-fluoro-6-methoxyphenyl)-1-(1,3,5-trimethyl-1*H*-pyrazol-4-yl)-6,6a,7,8,9,10-hexahydro-12*H*-pyrazino[2,1-*c*]pyrido[3,4-*f*]oxazepin-12-one (230 mg, crude product) obtained in step 9 was added to dry dichloromethane (10.0 mL), and then boron tribromide (20.0 mL, 1 M, 20.0 mmol) was added thereto, heated to room temperature and reacted overnight. Under an ice-water bath, the above reaction solution was added dropwise to saturated sodium bicarbonate solution, extracted twice with dichloromethane (80 mL), dried, concentrated, and the crude product was purified by preparation to obtain a target product of (6aR)-8-acryloyl-4-chloro-3-(2-fluoro-6-hydroxyphenyl)-1-(1,3,5-trimethyl-1*H*-pyrazol-4-yl)-6,6a,7,8,9,10-hexahydro-12*H*-pyrazino[2,1-*c*]pyrido[3,4-*f*]oxazepin-12-one (Z79, 50 mg, 4-step yield of 12.8%). ES-API: [M+H]⁺=526.2. ¹H NMR (500 MHz, DMSO-d₆) δ 10.10 (s, 1H), 7.30 (dd, J= 15.4, 8.1 Hz, 1H), 6.78 (dd, J= 23.5, 8.7 Hz, 3H), 6.19 (d, *J=* 14.9 Hz, 1H), 5.82 - 5.64 (m, 1H), 4.50 (s, 2H), 4.34 (d, *J=* 19.4 Hz, 1H), 4.15 - 3.96 (m, 2H), 3.94 - 3.50 (m, 7H), 2.15 (s, 3H), 2.01 (s, 3H).

### Embodiment 51 Synthesis of (6aR)-8-acryloyl-4-chloro-3-(2-fluoro-6-hydroxyphenyl)-1-(4-azaspiro[2.4]heptan-4-yl)-6,6a,7,8,9,10-hexahydro-12H-pyrazino[2,1-c]pyrido[3,4-f][1,4]oxazepin-12-one Z56

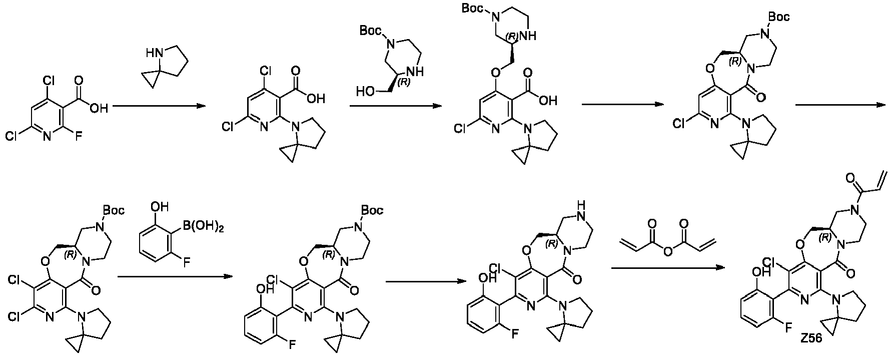

Step 1: 4,6-Dichloro-2-fluoronicotinic acid (886 mg, 4.22 mmol), potassium carbonate (1.5 g, 1.05 mmol) and 4-azaspiro[2.4]heptane (1/2 oxalate) (0.5 g, 3.52 mmol) were added to DMSO (20 mL), reacted at 65°C for 3 hours. The completion of the reaction was detected by thin-layer chromatography plate. The mixture was added with ethyl acetate (50 mL), washed with water and brine, and then dried and evaporated to dryness by rotary evaporation to obtain 4,6-dichloro-2-(4-azaspiro[2.4]heptan-4-yl)nicotinic acid (720 mg, yield of 60%).

Step 2: *tert*-Butyl (R)-3-(hydroxymethyl)piperazine-1-carboxylate (1.4 g, 6.27 mmol) was dissolved in THF (20 mL), and sodium hydride (836 mg, 20.9 mmol) was added thereto at a cooled temperature of 0°C. After holding the temperature and reacting for half an hour, a solution of 4,6-dichloro-2-(4-azaspiro[2.4]heptan-4-yl)nicotinic acid (600 mg, 2.09 mmol) dissolved in THF (10 mL) was added dropwise thereto. After the addition, the mixture was heated to 65°C and reacted overnight. The reaction solution was quenched with ammonium chloride solution, extracted with ethyl acetate to obtain (R)-4-((4-(*tert*-butoxycarbonyl)piperazin-2-yl)methoxy)-6-chloro-2-(4-azaspiro[2.4]heptan-4-yl)nicotinic acid (1 g, yield of 85%) as a crude product, which was directly used in the next step. ES-API: [M+H]⁺= 467.2.

Step 3: (R)-4-((4-(*tert*-Butoxycarbonyl)piperazin-2-yl)methoxy)-6-chloro-2-(4-azaspiro[2.4]heptan-4-yl)nicotinic acid (1 g, 2.14 mmol) and *N,N*-diisopropylethylamine (552 mg, 4.28 mmol) were dissolved in dichloromethane (100 mL), and HATU (974 mg, 2.57 mmol) was added at room temperature. After the addition, the mixture was reacted at room temperature for 1 hour. The reaction solution was washed with ammonium chloride solution, washed with sodium bicarbonate solution, dried and evaporated to dryness by rotary evaporation, and then purified by column chromatography (PE:EtOAc=3:1, R_{f}=0.4) to obtain *tert*-butyl (*R*)-3-chloro-12-oxo-1-(4-azaspiro[2.4]heptan-4-yl)-6a,7,9,10-tetrahydro-6H-pyrazino[2,1-*c*]pyrido[3,4-*f*][1,4]oxazepine-8(12*H*)-carboxylate (600 mg, yield of 62%) as a yellow foamy solid.

Step 4: *tert*-Butyl (R)-3-chloro-12-oxo-1-(4-azaspiro[2.4]heptan-4-yl)-6a,7,9,10-tetrahydro-6*H-*pyrazino[2,1-*c*]pyrido[3,4-*f*][1,4]oxazepine-8(12*H*)-carboxylate (220 mg, 0.49 mmol) was dissolved in acetonitrile (1.5 mL), and 1-chloropyrrolidin-2,5-dione (65 mg, 0.49 mmol) was added thereto. The mixture was heated to 35°C and reacted for 4 hours. The reaction solution was cooled and then added with ethyl acetate (10 mL), washed with water, brine, dried and evaporated to dryness by rotary evaporation, and then purified by column chromatography (PE:EtOAc=4:1, R_{f}=0.4) to obtain *tert*-butyl (*R*)-3,4-dichloro-12-oxo-1-(4-azaspiro[2.4]heptan-4-yl)-6a,7,9,10-tetrahydro-6*H*-pyrazino[2,1-*c*]pyrido[3,4-*f*][1,4]oxazepine-8(12*H*)-carboxylate (237 mg, yield of 100%) as a yellow foamy solid. ES-API: [M+H]⁺= 483.1.

Step 5: *tert*-Butyl (R)-3,4-dichloro-12-oxo-1-(4-azaspiro[2.4]heptan-4-yl)-6a,7,9,10-tetrahydro-6*H*-pyrazino[2,1-*c*]pyrido[3,4-*f*][1,4]oxazepine-8(12*H*)-carboxylate (237 mg, 0.49 mmol), (2-fluoro-6-hydroxyphenyl)boronic acid (115 mg, 0.74 mmol), sodium carbonate (104 mg, 0.98 mmol), and tetrakis(triphenylphosphine)palladium (25 mg) were added to dioxane (2 mL) and water (0.2 mL) in turn, and the reaction was carried out at 105°C for 18 hours under nitrogen protection. The mixture was cooled to room temperature, and then poured into ethyl acetate (20 mL), washed once with brine and then dried. The organic phase was distilled under reduced pressure to obtain a crude product, which was purified by silicagel column (EtOAc:PE=10%-30%) to obtain *tert*-butyl (6aR)-4-chloro-3-(2-fluoro-6-hydroxyphenyl)-12-oxo-1-(4-azaspiro[2.4]heptan-4-yl)-6a,7,9,10-tetrahydro-6*H*-pyrazino[2,1-*c*]pyrido[3,4-*f*][1,4]oxazepine-8(12*H*)-carboxylate (75 mg, yield of 27%) as a yellow foamy solid. ES-API: [M+H]⁺= 559.2.

Step 6: *tert*-Butyl (6aR)-4-chloro-3-(2-fluoro-6-hydroxyphenyl)-12-oxo-1-(4-azaspiro[2.4]heptan-4-yl)-6a,7,9,10-tetrahydro-6*H*-pyrazino[2,1-*c*]pyrido[3,4-*f*][1,4]oxazepine-8(12*H*)-carboxylate (75 mg, 0.13 mmol) was dissolved in dichloromethane (0.5 mL), added with trifluoroacetic acid (0.5 mL) and reacted at room temperature for 1 hour, evaporated to dryness by rotary evaporation to obtain (6a*R*)-4-chloro-3-(2-fluoro-6-hydroxyphenyl)-1-(4-azaspiro[2.4]heptan-4-yl)-7,8,9,10-tetrahydro-6*H-*pyrazino[2,1-*c*]pyrido[3,4-*f*][1,4]oxazepin-12-one (60 mg, yield of 100%) as a yellow oily crude product.

Step 7: (6aR)-4-Chloro-3-(2-fluoro-6-hydroxyphenyl)-1-(4-azaspiro[2.4]heptan-4-yl)-7,8,9,10-tetrahydro-6*H*-pyrazino[2,1-*c*]pyrido[3,4-*f*][1,4]oxazepin-12-one (60 mg, 0.13 mmol) and triethylamine (26 mg, 0.26 mmol) were dissolved in dichloromethane (1 mL), cooled to 0°C, and then acrylic anhydride (16 mg, 0.13 mmol) was added thereto, held the temperature at 0°C and reacted for 1 hour. The reaction solution was added with water (10 mL), extracted with dichloromethane (10 mL x 3), dried and then evaporated to dryness by rotary evaporation, and purified by chromatography plate (PE/EtOAc=1/1) to obtain (6a*R*)-8-acryloyl-4-chloro-3-(2-chioro-6-hydroxyphenyl)-1-(4-azaspiro[2.4]heptan-4-yl)-6,6a,7,8,9,10-hexahydro-12*H*-pyrazino[2,1-*c*]pyrido[3,4-*f*][1,4]oxazepin-12-one (Z56, 6 mg, yield of 10%) as a white solid. ES-API: [M+H]⁺=513.1. ¹H NMR (400 MHz, CDCl₃) δ 7.30 (s, 1H), 6.83 (d, *J* = 8.3 Hz, 1H), 6.69 (t, *J=* 9.0 Hz, 1H), 6.57 (s, 1H), 6.41 (d, *J=* 16.4 Hz, 1H), 5.83 (d, *J=* 10.3 Hz, 1H), 4.77 (s, 1H), 4.36 (m, 3H), 4.27-4.09 (m, 2H), 3.91 (m, 2H), 3.74-3.28 (m, 3H), 3.05-2.93 (m, 1H), 2.02 (dq, *J* = 14.0, 7.5, 6.5 Hz, 2H), 1.77 (m, 3H), 1.26 (s, 1H), 0.90 (d, *J=* 10.0 Hz, 1H), 0.49 (dt, *J* = 10.5, 5.4 Hz, 1H).

### Embodiment 52 Synthesis of (6aR)-8-acryloyl-4-chloro-3-(2-fluoro-6-hydroxyphenyl)-1-(7-methyl-4,7-diazaspiro[2.5]octan-4-yl)-6,6a,7,8,9,10-hexahydro-12H-pyrazino[2,1-c]pyrido[3,4-f][1,4]oxazepin-12-one Z57

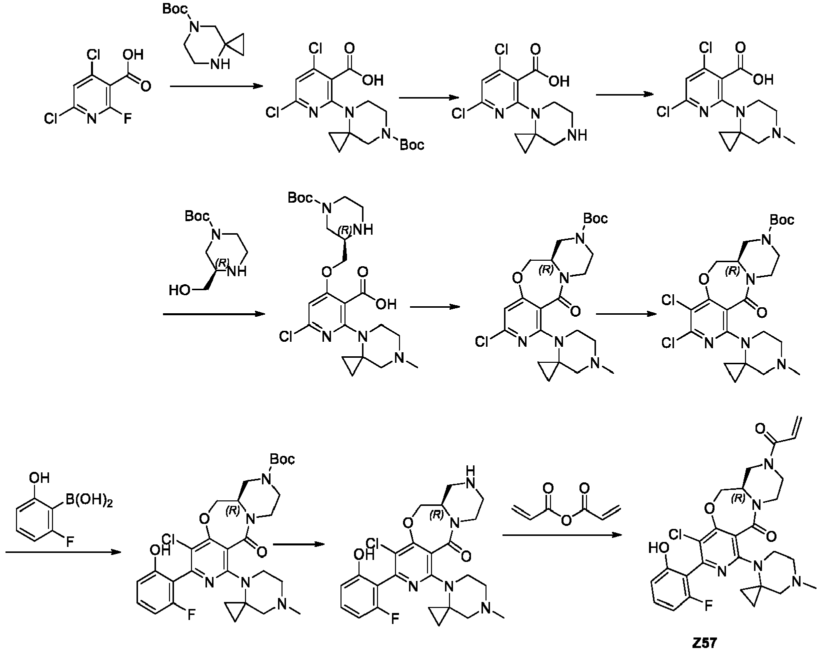

Step 1: 4,6-Dichloro-2-fluoronicotinic acid (0.8 g, 3.82 mmol) was dissolved in 12 mL of dimethyl sulfoxide, and then potassium carbonate (1.59 g, 11.46 mmol) and *tert*-butyl 4,7-diazaspiro[2.5]octane-7-carboxylate (812 mg, 3.82 mmol) were added thereto. The reaction solution was heated to 80°C and reacted for 6 hours. The reaction solution was added with water and dilute hydrochloric acid to adjust pH to 5-6, extracted with ethyl acetate, and the organic phase was washed with brine, dried over anhydrous sodium sulfate and concentrated to obtain a crude product of 2-(7-(*tert*-butoxycarbonyl)-4,7-diazaspiro[2.5]octan-4-yl)-4,6-dichloronicotinic acid (560 mg, 38.2%). ES-API: [M+H]⁺=402.1.

Step 2: 2-(7-(*tert*-Butoxycarbonyl)-4,7-diazaspiro[2.5]octan-4-yl)-4,6-dichloronicotinic acid (560 mg, 1.4 mmol) was dissolved in 5 M hydrochloric acid/dioxane solution, reacted at room temperature for 1 hour, and the solvent was evaporated to dryness by rotary evaporation to obtain a crude product of 4,6-dichloro-2-(4,7-diazaspiro[2.5]octan-4-yl)nicotinic acid (500 mg, 100%). ES-API: [M+H]⁺=302.1.

Step 3: 4,6-Dichloro-2-(4,7-diazaspiro[2.5]octan-4-yl)nicotinic acid (500 mg, 1.4 mmol) was dissolved in dichloromethane/acetic acid (9 mL/1 mL). 37% formaldehyde solution (1130 mg, 14 mmol) was added thereto, reacted at room temperature for 10 minutes. Sodium triacetoxyborohydride (890 mg, 4.2 mmol) was added thereto, reacted at room temperature for 1 hour, added with water to quench, and a product could not be extracted. The water was directly concentrated to remove, and the product was obtained by washing with dichloromethane/isopropanol=3/1, dried over anhydrous sodium sulfate, and concentrated to obtain a crude product of 4,6-dichloro-2-(7-methyl-4,7-diazaspiro[2.5]octan-4-yl)nicotinic acid (250 mg, 100%). ES-API: [M+H]⁺=316.1.

Step 4: Sodium hydride (318 mg, 7.94 mmol) was suspended in 1 mL of THF and cooled to 0°C. A solution of *tert*-butyl (R)-3-(hydroxymethyl)piperazine-1-carboxylate (343 mg, 1.587 mmol) in THF (2 mL) was added dropwise thereto, and after the addition, the mixture was stirred and reacted for 30 minutes while maintaining 0°C. A solution of 4,6-dichloro-2-(7-methyl-4,7-diazaspiro[2.5]octan-4-yl)nicotinic acid (250 mg, 0.794 mmol) in THF (2 mL) was added dropwise thereto, and after the addition, the mixture was stirred and reacted at 70°C for 4 hours. The reaction solution was quenched with saturated ammonium chloride solution, the product could not be extracted. The water was directly concentrated to remove, and the product was obtained by washing with dichloromethane/isopropanol=3/1, dried over anhydrous sodium sulfate, and concentrated to obtain a crude product of (*R*)-4-((4-(*tert*-butoxycarbonyl)piperazin-2-yl)methoxy)-6-chloro-2-(7-methyl-4,7-diazaspiro[2.5]octan-4-yl)nicotinic acid (0.5 g, 100%). ES-API: [M+H]⁺=496.2.

Step 5: (R)-4-((4-(*tert*-Butoxycarbonyl)piperazin-2-yl)methoxy)-6-chloro-2-(7-methyl-4,7-diazaspiro[2.5]octan-4-yl)nicotinic acid (0.5 g, 1.01 mmol) was dissolved in 10 mL of dichloromethane, and 1 mL of *N,N*-diisopropylethylamine and HATU (461 mg, 1.21 mmol) were added thereto. After the addition, the reaction solution was stirred at room temperature for 1 hour. The reaction solution was washed with saturated sodium bicarbonate solution, and the organic phase was washed with brine, dried over anhydrous sodium sulfate, concentrated, and purified by flash column chromatography (DCM/MeOH=10:1+0.5% ammonia water) to obtain *tert*-butyl (*R*)-3-chloro-1-(7-methyl-4,7-diazaspiro[2.5]octan-4-yl)-12-oxo-6a,7,9,10-tetrahydro-6*H*-pyrazino[2,1-*c*]pyrido[3,4-*f*][1,4]oxazepine-8(6*H*)-carboxylate (70 mg, 14.5%). ES-API: [M+H]⁺=478.2.

Step 6: *tert*-Butyl (*R*)-3-chloro-1-(7-methyl-4,7-diazaspiro[2.5]octan-4-yl)-12-oxo-6a,7,9,10-tetrahydro-6*H*-pyrazino[2,1-*c*]pyrido[3,4-*f*][1,4]oxazepine-8(6*H*)-carboxylate (70 mg, 0.147 mmol) was dissolved in 1.5 mL of acetonitrile, and *N*-chlorosuccinimide (19.6 mg, 0.147 mmol) was added thereto. The reaction solution was stirred and reacted at 50°C for 3 hours. After the reaction was completed, the reaction solution was quenched by adding water, extracted with ethyl acetate, and the organic phase was washed with saturated brine, dried over anhydrous sodium sulfate, concentrated to obtain *tert*-butyl (*R*)-3,4-dichloro-1-(7-methyl-4,7-diazaspiro[2.5]octan-4-yl)-12-oxo-6a,7,9,10-tetrahydro-12*H*-pyrazino[2,1-*c*]pyrido[3,4-*f*][1,4]oxazepine-8(6*H*)-carboxylate (70 mg, 93.2%). ES-API: [M+H]⁺=512.2.

Step 7: *tert*-Butyl (*R*)-3,4-dichloro-1-(7-methyl-4,7-diazaspiro[2.5]octan-4-yl)-12-oxo-6a,7,9,10-tetrahydro-12*H*-pyrazino[2,1-*c*]pyrido[3,4-*f*][1,4]oxazepine-8(6*H*)-carboxylate (70 mg, 0.137 mmol), (2-fluoro-6-hydroxyphenyl)boronic acid (64 mg, 0.412 mmol), sodium carbonate (73 mg, 0.685 mmol) and tetrakis(triphenylphosphine)palladium (16 mg, 0.0137 mmol) were dissolved in 20 mL of 1,4-dioxane and 4 mL of water. The reaction solution was stirred at 100°C for 16 hours under nitrogen protection. After the reaction solution was cooled, the reaction solution was added with 10 mL of water, extracted with ethyl acetate, and the organic phases were combined and then washed with brine, dried over anhydrous sodium sulfate, concentrated and purified by preparative thin-layer chromatography plate (DCM/MeOH=8/1, R_{f}=0.5) to obtain *tert*-butyl (6aR)-4-chloro-3-(2-fluoro-6-hydroxyphenyl)-1-(7-methyl-4,7-diazaspiro[2.5]octan-4-yl)-12-oxo-6a,7,9,10-tetrahydro-6*H*-pyrazino[2,1-*c*]pyrido[3,4-*f*][1,4]oxazepine-8(6*H*)-carboxylate (35 mg, 43.5%). ES-API: [M+H]⁺=588.2.

Step 8: *tert*-Butyl (6aR)-4-chloro-3-(2-fluoro-6-hydroxyphenyl)-1-(7-methyl-4,7-diazaspiro[2.5]octan-4-yl)-12-oxo-6a,7,9,10-tetrahydro-6*H*-pyrazino[2,1-*c*]pyrido[3,4-*f*][1,4]oxazepine-8(6*H*)-carboxylate (35 mg, 0.06 mmol) was dissolved in 1 mL of dichloromethane, and 1 mL of trifluoroacetic acid was added thereto. The reaction was carried out at room temperature for 0.5 hours, then the reaction solution was concentrated and redissolved in dichloromethane, and concentrated to obtain a crude product of (6aR)-4-chloro-3-(2-fluoro-6-hydroxyphenyl)-1-(7-methyl-4,7-diazaspiro[2.5]octan-4-yl)-7,8,9,10-tetrahydro-6*H*-pyrazino[2,1-*c*]pyrido[3,4-*f*][1,4]oxazepin-12-one (40 mg, 100%). The crude product was directly used in the next reaction step. ES-API: [M+H]⁺=488.2.

Step 9: (6aR)-4-Chloro-3-(2-fluoro-6-hydroxyphenyl)-1-(7-methyl-4,7-diazaspiro[2.5]octan-4-yl)-7,8,9,10-tetrahydro-6*H*-pyrazino[2,1-*c*]pyrido[3,4-*f*][1,4]oxazepin-12-one (40 mg, 0.06 mmol) was dissolved in 1 mL of dichloromethane, cooled with ice-water, added with 1 mL of triethylamine, and then acrylic anhydride (6 mg, 0.048 mmol) was slowly added dropwise thereto, reacted at 10°C for 10 minutes, then added with water to quench, extracted with dichloromethane. The organic phases were combined and washed with brine, dried over anhydrous sodium sulfate, concentrated, and purified by preparative thin-layer chromatography plate (DCM/MeOH=8/1, R_{f}=0.4) to obtain (6aR)-8-acryloyl-4-chloro-3-(2-fluoro-6-hydroxyphenyl)-1-(7-methyl-4,7-diazaspiro[2.5]octan-4-yl)-6,6a,7,8,9,10-hexahydro-12*H*-pyrazino[2,1-c]pyrido[3,4*f*][1,4]oxazepin-12-one (Z57, 3.1 mg, 9.55%) ES-API: [M+H]⁺=542.2. ¹H NMR (400 MHz, CDCl₃) δ 7.31 (d, *J* = 8.2 Hz, 1H), 6.84 (d, *J* = 8.3 Hz, 1H), 6.71 (t, *J* = 9.0 Hz, 1H), 6.56 (s, 1H), 6.43 (d, *J* = 16.5 Hz, 1H), 5.84 (d, *J* = 10.3 Hz, 1H), 4.51-3.96 (m, 5H), 3.88-3.48 (m, 4H), 2.58 (s, 3H), 2.39 (s, 1H),2.09-1.98 (m, 2H), 1.38-1.27 (m, 4H), 1.06 (s, 1H), 0.98(s, 1H), 0.90 (s, 2H).

### Embodiment 53 Synthesis of (6aR)-8-acryloyl-4-chloro-3-(2-fluoro-6-hydroxyphenyl)-1-(7-oxa-1-azaspiro[4.4]nonan-1-yl)-6,6a,7,8,9,10-hexahydro-12H-pyrazino[2,1-c]pyrido[3,4-f][1,4]oxazepin-12-one Z58

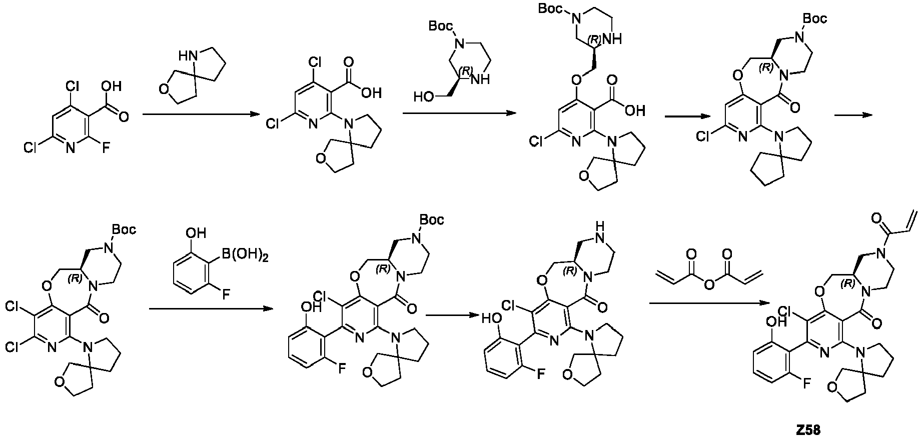

Step 1: 4,6-Dichloro-2-fluoronicotinic acid (662 mg, 3.15 mmol) was dissolved in 10 mL of dimethyl sulfoxide, and then potassium carbonate (1.44 g, 10.48 mmol) and 7-oxa-1-azaspiro[4.4]nonane hydrochloride (430 mg, 2.62 mmol) were added thereto. The reaction was carried out at 80°C for 2 hours. The reaction solution was added with water and dilute hydrochloric acid to adjust pH to 5-6, extracted with ethyl acetate, and the organic phase was washed with brine, dried over anhydrous sodium sulfate and concentrated to obtain 4,6-dichloro-2-(7-oxa-1-azaspiro[4.4]nonan-1-yl)nicotinic acid (1 g, crude product). ES-API: [M+H]⁺=317.0.

Step 2: Sodium hydride (340 mg, 8.5 mmol) was suspended in 15 mL of THF and cooled to 0°C. A solution of *tert*-butyl (*R*)-3-(hydroxymethyl)piperazine-1-carboxylate (551 mg, 2.55 mmol) in THF (3 mL) was added dropwise thereto, and after the addition, the mixture was stirred and reacted for 30 minutes while maintaining the temperature. A solution of 4,6-dichloro-2-(7-oxa-1-azaspiro[4.4]nonan-1-yl)nicotinic acid (270 mg, 0.85 mmol) in THF (3 mL) was added dropwise thereto, and after the addition, the mixture was stirred and reacted at 60°C for 8 hours. The reaction solution was quenched with saturated ammonium chloride solution, extracted with ethyl acetate, and the organic phase was washed with brine, dried over anhydrous sodium sulfate, and concentrated to obtain 4-(((*R*)-4-(*tert*-butoxycarbonyl)piperazin-2-yl)methoxy)-6-chloro-2-(7-oxa-1-azaspiro[4.4]nonan-1-yl)nicotinic acid (300 mg, crude product). The crude product was directly used in the next reaction step. ES-API: [M+H]⁺=497.2.

Step 3: 4-(((*R*)-4-(*tert*-Butoxycarbonyl)piperazin-2-yl)methoxy)-6-chloro-2-(7-oxa-1-azaspiro[4.4]nonan-1-yl)nicotinic acid (300 mg, crude product) was dissolved in 10 mL of dichloromethane, and 232 mg of *N*,*N*-diisopropylethylamine and HATU (228 mg, 0.6 mmol) were added thereto. After the addition, the reaction solution was stirred at room temperature for 1 hour. The reaction solution was washed with saturated sodium bicarbonate solution, and the organic phase was washed with brine, dried over anhydrous sodium sulfate, concentrated, and the crude product was purified by flash silica gel column chromatography (PE/EtOAc=1/1, R_{f}=0.5) to obtain *tert*-butyl (*R*)-3-chloro-12-oxo-1-(7-oxa-1-azaspiro[4.4]nonan-1-yl)-6a,7,9,10-tetrahydro-6*H*-pyrazino[2,1-*c*]pyrido[3,4-*f*][1,4]oxazepine-8(12*H*)-carboxylate (100 mg, 34.9%). ES-API: [M+H]⁺=477.2.

Step 4: *tert*-Butyl (*R*)-3-chloro-12-oxo-1-(7-oxa-1-azaspiro[4.4]nonan-1-yl)-6a,7,9,10-tetrahydro-6*H*-pyrazino[2,1-*c*]pyrido[3,4-*f*][1,4]oxazepine-8(12*H*)-carboxylate (100 mg, 0.21 mmol) was dissolved in 5 mL of acetonitrile, and N-chlorosuccinimide (26.5 mg, 0.21 mmol) was added thereto. The reaction solution was stirred and reacted at 35°C for 16 hours. The reaction solution was added with water, extracted with ethyl acetate, and the organic phase was washed with brine, dried over anhydrous sodium sulfate, concentrated, and the crude product was purified by flash silica gel column (PE/EtOAc=1/1, R_{f}=0.4) to obtain *tert*-butyl (*R*)-3,4-dichloro-12-oxo-1-(7-oxa-1-azaspiro[4.4]nonan-1-yl)-6a,7,9,10-tetrahydro-6*H-*pyrazino[2,1-*c*]pyrido[3,4-*f*][1,4]oxazepine-8(12*H*)-carboxylate (36 mg, 35.2%). ES-API: [M+H]⁺=513.2.

Step 5: *tert*-Butyl (R)-3,4-dichloro-12-oxo-1-(7-oxa-1-azaspiro[4.4]nonan-1-yl)-6a,7,9,10-tetrahydro-6*H*-pyrazino[2,1-*c*]pyrido[3,41][1,4]oxazepine-8(12*H*)-carboxylate (36 mg, 0.784 mmol) was suspended in dioxane/water (0.5 mL/0.1 mL), and sodium carbonate (21 mg, 0.198 mmol), (2-fluoro-6-hydroxyphenyl)boronic acid (31 mg, 0.198 mmol) and tetrakis(triphenylphosphine)palladium (8 mg, 0.0066 mmol) were added thereto. The reaction solution was stirred at 100°C for 16 hours under nitrogen protection. The reaction solution was added with water, extracted with ethyl acetate. The organic phase was washed with brine, dried over anhydrous sodium sulfate, concentrated, and the crude product was purified by flash silica gel column chromatography (PE/EtOAc=1/1, R_{f}=0.5) to obtain *tert*-butyl (6a*R*)-4-chloro-3-(2-fluoro-6-hydroxyphenyl)-12-oxo-1-(7-oxa-1-azaspiro[4.4]nonan-1-yl)-6a,7,9,10-tetrahydro-6*H*-pyrazino[2,1-*c*]pyrido[3,4-*f*][1,4]oxazepine-8(12*H*)-carboxylate (16 mg, 42.1%). ES-API: [M+H]⁺=589.2.

Step 6: *tert*-Butyl (6a*R*)-4-chloro-3-(2-fluoro-6-hydroxyphenyl)-12-oxo-1-(7-oxa-1-azaspiro[4.4]nonan-1-yl)-6a,7,9,10-tetrahydro-6*H*-pyrazino[2,1-c]pyrido[3,4-*f*][1,4]oxazepine-8(12*H*)-carboxylate (16 mg, 0.044 mmol) was dissolved in 2 mL of dichloromethane, and 0.5 mL of trifluoroacetic acid was added thereto. The reaction was carried out at room temperature for 0.5 hours, then the reaction solution was concentrated and redissolved in dichloromethane, and concentrated to obtain (6a*R*)-4-chloro-3-(2-fluoro-6-hydroxyphenyl)-1-(7-oxa-1-azaspiro[4.4]nonan-1-yl)-6,6a,7,8,9,10-hexahydro-12*H-*pyrazino[2,1-*c*]pyrido[3,4-*f*][1,4]oxazepin-12-one (44 mg, crude product). The crude product was directly used in the next reaction step. ES-API: [M+H]⁺=489.0.

Step 7: (6a*R*)-4-Chloro-3-(2-fluoro-6-hydroxyphenyl)-1-(7-oxa-1-azaspiro[4.4]nonan-1-yl)-7,8,9,10-tetrahydro-6*H*-pyrazino[2,1-*c*]pyrido[3,4-*f*][1,4]oxazepin-12-one (40 mg, crude product) was dissolved in 0.5 mL of dichloromethane, cooled with ice-water, added with 0.2 mL of triethylamine, then slowly added with acrylic anhydride (5 mg, 0.04 mmol), reacted at 10°C for 10 minutes, then added with water, extracted with dichloromethane. The organic phase was washed with brine, dried over anhydrous sodium sulfate, concentrated, and the crude product was purified by chromatography plate (DCM/MeOH=10:1, R_{f}=0.3) to obtain (6a*R*)-8-acryloyl-4-chloro-3-(2-fluoro-6-hydroxyphenyl)-1-(7-oxa-1-azaspiro[4.4]nonan-1-yl)-6,6a,7,8,9,10-hexahydro-12*H*-pyrazino[2,1-*c*]pyrido[3,4-*f*][1,4]oxazepin-12-one (Z58, 1.2 mg, 5.2%). ES-API: [M+H]⁺=543.2. ¹H NMR (400 MHz, CDCl₃) δ 7.32 (d, *J* = 5.8 Hz, 1H), 6.91 (d, *J* = 8.1 Hz, 1H), 6.70 (t, *J* = 8.8 Hz, 1H), 6.56 (s, 1H), 6.41 (d, *J* = 15.3 Hz, 1H), 5.81 (s, 1H), 4.68-4.10 (m, 6H), 4.00-3.60 (m, 6H), 3.27 (s, 2H), 2.76 (s, 1H), 2.23 (d, *J* = 7.6 Hz, 3H), 2.04 (s, 3H).

### Embodiment 54 Synthesis of (6aR)-8-acryloyl-4-chloro-3-(2-fluoro-6-hydroxyphenyl)-1-(2-oxa-7-azaspiro[4.4]nonan-7-yl)-6,6a,7,8,9,10-hexahydro-12H-pyrazino[2,1-c]pyrido[3,4-f][1,4]oxazepin-12-(6aH)-one Z59

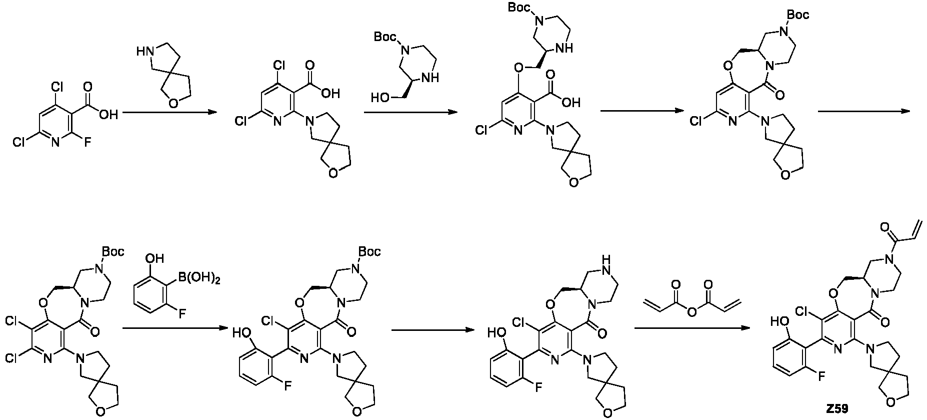

Step 1: 4,6-Dichloro-2-fluoronicotinic acid (909 mg, 4.33 mmol) was dissolved in acetonitrile (15 mL), then 2-oxa-7-azaspiro[4.4]nonane (440 mg, 3.46 mmol) and *N*,*N*-diisopropylethylamine (1.1 g, 8.66 mmol) were added thereto and reacted for 2 hours at room temperature. After the reaction was completed, the reaction was quenched by adding water. The pH of the mixture was adjusted to 3 with 1 N HCl, and the mixture was extracted with ethyl acetate, and the organic phase was dried and concentrated to obtain a product (1 g, crude product). ES-API: [M+H]⁺=317.0.

Step 2: *tert*-Butyl (*R*)-3-(hydroxymethyl)piperazine-1-carboxylate (818 mg, 3.78 mmol) was dissolved in THF (20 mL), cooled to 0 to 5°C, and sodium hydride (757 mg, 18.9 mmol) was added thereto. After stirring under an ice bath for 10 minutes, a solution of 4,6-dichloro-2-(2-oxa-7-azaspiro[4.4]nonan-7-yl)nicotinic acid (1 g, 3.15 mmol) in THF (10 mL) was then added thereto. The reaction solution was heated to 60°C and reacted overnight. After the reaction was completed, the reaction was quenched by adding saturated ammonium chloride. The pH of the mixture was adjusted to 3 with 1 N HCl, and the mixture was extracted with ethyl acetate, dried and evaporated to dryness to obtain a product (1.7 g, crude product). ES-API: [M+H]⁺=497.2.

Step 3: 4-(((*R*)-4-(*tert*-Butoxycarbonyl)piperazin-2-yl)methoxy)-6-chloro-2-(2-oxa-7-azaspiro[4.4]nonan-7-yl)nicotinic acid (1.5 g, 3.02 mmol) was dissolved in dichloromethane (240 mL), and HATU (1.4 g, 3.62 mmol) and *N*,*N*-diisopropylethylamine (1.17 g, 9.05 mmol) were added thereto. The reaction solution was reacted at room temperature for 2 hours. After the reaction was completed, water and dichloromethane were added for extraction. The organic phase was dried, concentrated and purified by column chromatography (PE/EtOAc=2/1) to obtain a product (800 mg, 55%, yellow oil). ES-API: [M+H]⁺=479.1.

Step 4: *tert*-Butyl (6a*R*)-3-chloro-12-oxo-1-(2-oxa-7-azaspiro[4.4]nonan-7-yl)-6a,7,9,10-tetrahydro-6*H*-pyrazino[2,1-*c*]pyrido[3,41][1,4]oxazepine-8(12*H*)-carboxylate (300 mg, 0.63 mmol) was dissolved in acetonitrile (6 mL), and *N*-chlorosuccinimide (83 mg, 0.63 mmol) was added thereto. The reaction was carried out at 50°C overnight, and then water and ethyl acetate were added for extraction. The organic phase was dried, concentrated and purified by column chromatography (PE/EtOAc=1/5) to obtain a product (260 mg, 80%, yellow oil). (R_{f}=0.7, ethyl acetate=100%). ES-API: [M+H]⁺=513.2.

Step 5: *tert*-Butyl (6a*R*)-3,4-dichloro-12-oxo-1-(2-oxa-7-azaspiro[4.4]nonan-7-yl)-6a,7,9,10-tetrahydro-6*H*-pyrazino[2,1-*c*]pyrido[3,41][1,4]oxazepine-8(12*H*)-carboxylate (260 mg, 0.51 mmol) was dissolved in 1,4-dioxane/water (3 mL/0.6 mL), and (2-fluoro-6-hydroxyphenyl)boronic acid (158 mg, 1.0 mmol), tetrakis(triphenylphosphine)palladium (58 mg, 0.05 mmol) and sodium carbonate (161 mg, 1.52 mmol) were added thereto. The reaction was carried out at 100°C overnight, and then brine and ethyl acetate were added thereto for extraction. The organic phase was dried, concentrated, and purified by column chromatography (ethyl acetate=100%) to obtain a product (230 mg, 77%, yellow solid). ES-API: [M+H]⁺=589.2.

Step 6: *tert*-Butyl (6a*R*)-4-chloro-3-(2-fluoro-6-hydroxyphenyl)-12-oxo-1-(2-oxa-7-azaspiro[4.4]nonan-7-yl)-6a,7,9,10-tetrahydro-6*H*-pyrazino[2,1-*c*]pyrido[3,4-*f*][1,4]oxazepine-8(12*H*)-carboxylate (230 mg, 0.39 mmol) was dissolved in dichloromethane (1 mL), and trifluoroacetic acid (1 mL) was added thereto. The reaction solution was reacted at room temperature for 1 hour. After TLC monitoring that the reaction was completed, the reaction solution was concentrated to obtain a crude product, which was directly used in the next reaction step.

Step 7: (6a*R*)-4-Chloro-3-(2-fluoro-6-hydroxyphenyl)-1-(2-oxa-7-azaspiro[4.4]nonan-7-yl)-6a,7,9,10-hexahydro-6*H*-pyrazino[2,1-*c*]pyrido[3,4-*f*][1,4]oxazepin-12(6a*H*)-one (crude product, 0.39 mmol) was dissolved in dichloromethane (2 mL) and cooled to 0 to 5°C, and triethylamine (2 mL) and acrylic anhydride (29 mg, 0.23 mmol) were added dropwise thereto in turn. The reaction solution was reacted in an ice bath for 0.5 hours. After the reaction was completed, the mixture was extracted with dichloromethane and water, and the organic phase was dried, evaporated to dryness by rotary evaporation, and purified by preparative HPLC to obtain (6a*R*)-8-acryloyl-4-chloro-3-(2-fluoro-6-hydroxyphenyl)-1-(2-oxa-7-azaspiro[4.4]nonan-7-yl)-6,6a,7,8,9,10-hexahydro-12*H*-pyrazino[2,1-*c*]pyrido[3,4-*f*][1,4]oxazepin-12(6a*H*)-one (Z59, 27 mg, 13%) as a white solid. ES-API: [M+H]⁺=543.1. ¹H NMR (500 MHz, DMSO-*d*₆) δ 9.94 (s, 1H), 7.24 (q, *J* = 7.8 Hz, 1H), 6.80-6.66 (m, 3H), 6.17 (d, *J* = 16.6 Hz, 1H), 5.77-5.70 (m, 1H), 4.51 (s, 1H), 4.40-4.27 (m, 1H), 4.25-3.91 (m, 4H), 3.86-3.64 (m, 3H), 3.63-3.40 (m, 6H), 3.20-3.11 (m, 2H), 2.00-1.83 (m, 3H), 1.78 (t, *J* = 6.9 Hz, 1H).

### Embodiment 55 Synthesis of 2-(6aR)-8-acryloyl-4-chloro-1-(2,2-dimethylpyrrolidin-1-yl)-6,6a,7,8,9,10-hexahydro-12H-pyrazino[2,1-c]pyrido[3,4-f][1,4]oxazepin-3-yl)-3-fluorophenol Z66

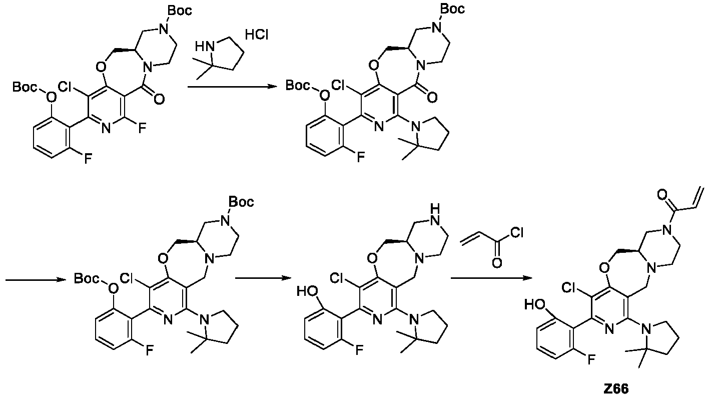

Step 1: 2,2-Dimethylpyrrolidine hydrochloride (25 mg, 0.18 mmol) and potassium carbonate (36 mmol, 0.26 mmol) were added to a solution of *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(*6H*)-carboxylate (50 mg, 0.09 mmol) in acetonitrile (2 mL), stirred at 80°C for 2 hours. The reaction solution was concentrated and purified by flash silica gel column (EtOAc/PE: 0-100%) to obtain tert-butyl (6a*R*)-3-(2-((*tert*-butoxycarbonyl)oxy)-6-fluorophenyl)-4-chloro-1-(2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (30 mg, 53%) as a pale yellow oil. ES-API: [M+H]⁺=661.2.

Step 2: Under an ice bath, a solution of *tert*-butyl (6a*R*)-3-(2-((*tert*-butoxycarbonyl)oxy)-6-fluorophenyl)-4-chloro-1-(2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (30 mg, 0.05 mmol) in THF (2 mL) was added with 1 M borane THF complex (450 mL), heated to 70°C and stirred overnight. The reaction solution was cooled to room temperature, quenched with 3 mL of methanol, added with 10 mL of water, extracted with 10 mL of dichloromethane, dried and concentrated to obtain *tert*-butyl (6a*R*)-3-(2-((*tert*-butoxycarbonyl)oxy)-6-fluorophenyl)-4-chloro-1-(2,2-dimethylpyrrolidin-1-yl)-6a,7,9,10-tetrahydro-12*H*-pyrazino[2,1-*c*]pyrido[3,4-*f*][1,4]oxazepine-8(6*H*)-carboxylate (30 mg, crude product). ES-API: [M+H]⁺=647.2.

Step 3: Under an ice bath, a solution of *tert*-butyl (6a*R*)-3-(2-((*tert*-butoxycarbonyl)oxy)-6-fluorophenyl)-4-chloro-1-(2,2-dimethylpyrrolidin-1-yl)-6a,7,9,10-tetrahydro-12*H*-pyrazino[2,1-*c*]pyrido[3,4-*f*][1,4]oxazepine-8(6*H*)-carboxylate (30 mg, 0.05 mmol) in dichloromethane (2 mL) was added with trifluoroacetic acid (0.5 mL). The mixture was stirred at room temperature for 1 hour. After the reaction was completed, the mixture was concentrated to obtain a crude product of 2-((6a*R*)-4-chloro-1-(2,2-dimethylpyrrolidin-1-yl)-6,6a,7,8,9,10-hexahydro-12*H*-pyrazino[2,1-*c*]pyrido[3,4-*f*][1,4]oxazepin-3-yl)-3-fluorophenol, which was directly used in the next reaction step. ES-API: [M+H]⁺=447.1.

Step 4: Under an ice bath, the crude product obtained above was dissolved in dichloromethane (2 mL), and *N*,*N*-diisopropylethylamine (30 mg, 0.23 mmol) was added thereto. After the reaction solution was clarified, acryloyl chloride (3.5 mg, 0.04 mmol) was added dropwise thereto and stirred for 10 minutes. The reaction solution was concentrated and purified by preparative HPLC to obtain 2-((6a*R*)-8-acryloyl-4-chloro-1-(2,2-dimethylpyrrolidin-1-yl)-6,6a,7,8,9,10-hexahydro-12*H*-pyrazino[2,1-*c*]pyrido[3,4-*f*][1,4]oxazepin-3-yl)-3-fluorophenol (Z66, 15 mg, 3-step yield of 64%) as a white solid. ES-API: [M+H]⁺=501.2.

### Embodiment 55-1 Synthesis of Z476

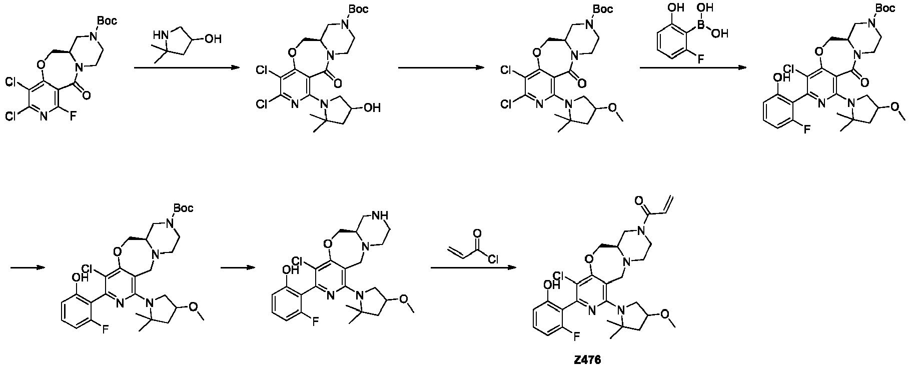

Step 1: tert-Butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (300 mg, 0.74 mmol), 5,5-dimethylpyrrolidin-3-ol (102 mg, 0.89 mmol), potassium carbonate (307 mg, 2.22 mmol) and acetonitrile (5 mL) were added to a 100 mL flask, reacted at 90°C for 2 hours. The reaction solution was extracted with ethyl acetate, washed with aqueous ammonium chloride solution and saturated brine in turn, and the organic phase was dried and concentrated, and purified by silica gel column to obtain *tert*-butyl (6a*R*)-3,4-dichloro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (250 mg, yield of 67%) as a white solid. ES-API: [M+H]⁺=501.2.

Step 2: *tert*-Butyl (6a*R*)-3,4-dichloro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (250 mg, 0.50 mmol), iodomethane (354 mg, 2.49 mmol), potassium *tert*-butoxide (279 mg, 2.49 mmol) and 5 mL of tetrahydrofuran were added to a 50 mL flask. The reaction solution was stirred at room temperature for 3 hours, extracted with ethyl acetate, washed with saturated brine, and the organic phase was dried and concentrated, and purified by silica gel column to obtain *tert*-butyl (6a*R*)-3,4-dichloro-1-(4-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, yield of 78%) as a white solid. ES-API: [M+H]⁺=515.2.

Step 3: *tert*-Butyl (6a*R*)-3,4-dichloro-1-(4-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.39 mmol), 2-fluoro-6-hydroxyphenylboronic acid (182 mg, 1.16 mmol), SPhos-Pd-G2 (28 mg, 0.04 mmol), potassium carbonate (161 mg, 1.16 mmol), 4 mL of dioxane and 1 mL of water were added to a 50 mL flask. Under nitrogen protection, the reaction was carried out at 80°C for 2 hours. The reaction solution was added with 50 mL of ethyl acetate, washed with 30 mL of saturated brine for 3 times, dried and concentrated, and the crude product was purified by flash silica gel column to obtain *tert*-butyl (6a*R*)-4-chloro-3-(2-fluoro-6-hydroxyphenyl)-1-(4-methoxy-2,2-dimethylpyrrolidin-1-yl)-12-oxo-6a,7,9,10-tetrahydro-12*H-*pyrazino[2,1-*c*]pyrido[3,4-*c*][1,4-*f*]oxazepine-8(6*H*)-carboxylate (150 mg, yield of 65%). ES-API: [M+H]⁺=591.3.

Step 4: *tert*-Butyl (6a*R*)-4-chloro-3-(2-fluoro-6-hydroxyphenyl)-1-(4-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg, 0.25 mmol), 2 mL of tetrahydrofuran and 2.5 mL of 1 M borane tetrahydrofuran complex were added to a 50 mL flask. The reaction solution was reacted at 70°C for 2 hours, quenched with methanol, extracted with ethyl acetate, washed with saturated brine, and the organic phase was dried and concentrated, and the crude product was purified by flash silica gel column to obtain *tert*-butyl (6a*R*)-4-chloro-3-(2-fluoro-6-hydroxyphenyl)-1-(4-methoxy-2,2-dimethylpyrrolidin-1-yl)-6a,7,9,10-tetrahydro-12*H*-pyrazino[2,1-*c*]pyrido[3,4-*f*][1,4-*f*]oxazepine-8(6*H*)-carboxylate (120 mg, yield of 82%). ES-API: [M+H]⁺=577.3.

Step 5: *tert*-Butyl (6a*R*)-4-chloro-3-(2-fluoro-6-hydroxyphenyl)-1-(4-methoxy-2,2-dimethylpyrrolidin-1-yl)-6a,7,9,10-tetrahydro-12*H*-pyrazino[2,1-*c*]pyrido[3,4-*f*][1,4]oxazepine-8(6*H*)-carboxylate (120 mg, 0.21 mmol), 0.5 mL of trifluoroacetic acid and 2 mL of dichloromethane were added to a 50 mL flask. The mixture was stirred at room temperature for 1 hour, and the completion of reaction was detected by LC-MS. The reaction solution was concentrated to obtain a crude product of 2-((6a*R*)-4-chloro-1-(4-methoxy-2,2-dimethylpyrrolidin-1-yl)-6,6a,7,8,9,10-hexahydro-12*H*-pyrazino[2,1-*c*]pyrido[3,4-*f*][1,4-*f*]oxazepin-3-yl)-3-fluorophenol trifluoroacetate (99 mg, theoretical value) as a yellow solid. ES-API: [M+H]⁺=477.3.

Step 6: 2-((6a*R*)-4-Chloro-1-(4-methoxy-2,2-dimethylpyrrolidin-1-yl)-6,6a,7,8,9,10-hexahydro-12*H*-pyrazino[2,1-*c*]pyrido[3,4-*f*][1,4]oxazepin-3-yl)-3-fluorophenol trifluoroacetate (99 mg, 0.21 mmol), 4 mL of dichloromethane and diisopropylethylamine (136 mg, 1.05 mmol) were added to a 50 mL flask. At 0°C, a solution of acryloyl chloride in dichloromethane (19 mg, 0.21 mmol, 1 mL) was added dropwise thereto. The reaction was carried out at 0°C for 10 minutes. The reaction mixture was washed with 20 mL of water, and the organic phase was dried and concentrated, and the crude product was purified by preparative HPLC (CH₃CN:H₂O, NH₄HCO₃) to obtain 1-((6a*R*)-4-chloro-3-(2-fluoro-6-hydroxyphenyl)-1-(4-methoxy-2,2-dimethylpyrrolidin-1-yl)-6a,7,9,10-tetrahydro-12*H*-pyrazino[2,1-*c*]pyrido[3,4-*f*][1,4]oxazepin-8(6*H*)-yl)prop-2-en-1-one (Z476, 32 mg, 2-step yield: 29%) as a white solid. ES-API: [M+H]⁺=531.3. ¹H NMR (400 MHz, DMSO-*d*₆) δ 9.89 (d, *J* = 19.6 Hz, 1H), 7.25 - 7.16 (m, 1H), 6.91 - 6.79 (m, 1H), 6.76 - 6.72 (m, 1H), 6.71 - 6.63 (m, 1H), 6.15 (d, *J* = 16.4 Hz, 1H), 5.71 (t, *J* = 9.6 Hz, 1H), 4.67 - 4.54 (m, 1H), 4.47 - 4.32 (m, 1H), 4.24 - 3.94 (m, 3H), 3.91 - 3.38 (m, 4H), 3.35 - 3.32 (m, 2H), 3.28 - 3.18 (m, 3H), 3.12 - 2.73 (m, 2H), 2.47 - 2.30 (m, 1H), 2.10 - 1.94 (m, 1H), 1.84 - 1.70 (m, 1H), 1.43 - 1.23 (m, 6H).

### Embodiment 56 Synthesis of (6aR)-8-acryloyl-4-chloro-1-(3,3-dimethylmorpholino)-3-(2-fluoro-6-hydroxyphenyl)-6,6a,7,8,9,10-hexahydro-12H-pyrazino[2,1-c]pyrido[3,4f][1,4]oxazepin-12-one Z70

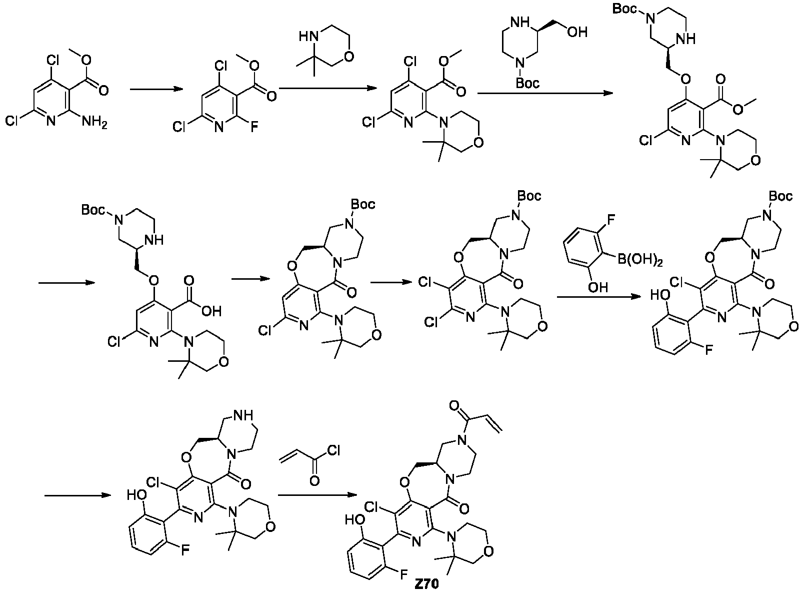

Step 1: Under an ice bath, methyl 2-amino-4,6-dichloronicotinate (4 g, 18.1 mmol) was dissolved in pyridine hydrofluoride solution (4 mL), and sodium nitrite (125 mg, 1.81 mmol) was slowly added thereto in batches and stirred for 10 minutes. The mixture was then quenched with ammonia water (30 mL), extracted with 50 mL of ethyl acetate, concentrated and then purified by flash silica gel column (EtOAc/PE: 0-10%) to obtain methyl 4,6-dichloro-2-fluoronicotinate as a white solid (2.5 g, 62%). ES-API: [M+H]⁺=224.1.

Step 2: 3,3-Dimethylmorpholine (515 mg, 4.46 mmol)) and sodium bicarbonate (1.13 g, 13.39 mmol) were added to a solution of compound methyl 4,6-dichloro-2-fluoronicotinate (1 g, 4.46 mmol) in acetonitrile (10 mL), and the mixture was sealed in the tube and stirred at 90°C overnight. After the reaction was completed, the reaction mixture was filtered, concentrated and purified by flash silica gel column (EtOAc/PE: 0-10%) to obtain methyl 4,6-dichloro-2-(3,3-dimethylmorpholino)nicotinate (400 mg, 28%) as a white solid. ES-API: [M+H]⁺=319.0.

Step 3: Under an ice bath, *tert*-butyl (*R*)-3-(hydroxymethyl)piperazine-1-carboxylate (400 mg, 1.25 mmol) was slowly added to a turbid solution of sodium hydride (250 mg, 6.27 mmol) in THF (5 mL) and stirred for 10 minutes. Then, methyl 4,6-dichloro-2-(3,3-dimethylmorpholino)nicotinate (400 mg, 1.25 mmol) was added thereto, heated to 60° C and stirred for 1 hour. Under an ice bath, the mixture was quenched with 20 mL of saturated ammonium chloride solution, extracted three times with 30 mL of ethyl acetate, and the organic phases were combined, dried and concentrated to obtain *tert*-butyl (*R*)-3-(((6-chloro-2-(3,3-dimethylmorpholino)-3-(methoxycarbonyl)pyridin-4-yl)oxy)methyl)piperazine-1-carboxylate (500 mg, 80%) as a pale yellow oil. ES-API: [M+H]⁺=499.1.

Step 4: *tert*-Butyl (*R*)-3-(((6-chloro-2-(3,3-dimethylmorpholino)-3-(methoxycarbonyl)pyridin-4-yl)oxy)methyl)piperazine-1-carboxylate (500 mg, 1 mmol) was dissolved in methanol (4 mL) and water (1 mL), and sodium hydroxide (300 mg, 7.50 mmol) was added thereto, stirred at 70°C overnight. The mixture was cooled and concentrated to remove methanol, dissolved with 5 mL of water, and the pH of the mixture was adjusted to 6 with 1 M dilute hydrochloric acid. The mixture was extracted three times with a mixed solvent of 10 mL of dichloromethane/isopropanol (3:1), dried and concentrated to obtain (*R*)-4-((4-(tert-butoxycarbonyl)piperazin-2-yl)methoxy)-6-chloro-2-(3,3-dimethylmorpholino)nicotinic acid (350 mg, 72%) as a white solid. ES-API: [M+H]⁺=485.2.

Step 5: *N,N*-Diisopropylethylamine (1 mL) and 1-propylphosphoric anhydride (1 mL) were added to a solution of compound (*R*)-4-((4-(*tert*-butoxycarbonyl)piperazin-2-yl)methoxy)-6-chloro-2-(3,3-dimethylmorpholino)nicotinic acid (350 mg, 0.72 mmol) in dichloromethane (3.5 mL) in turn, and stirred at room temperature for 10 minutes. The reaction solution was quenched with 20 mL of saturated sodium bicarbonate solution, and extracted three times with 20 mL of ethyl acetate. The organic phases were combined, dried, concentrated and purified by flash silica gel column (EtOAc/PE: 0-80%) to obtain *tert-*butyl (*R*)-3-chloro-1-(3,3-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (250 mg, 74%) as a white solid. ES-API: [M+H]⁺=467.2.

Step 6: *tert*-Butyl (*R*)-3-chloro-1-(3,3-dimethylmorpholino)-12-oxo-6a,7,9,10-tetrahydro-12*H-*pyrazino[2,1-*c*]pyrido[3,4-*c*][1,4-*f*]oxazepine-8(6*H*)-carboxylate (250 mg, 0.54 mmol) in dichloromethane (5 mL) was cooled to -40°C, and sulfonyl chloride (144 mg, 1.07 mmol) was added dropwise therreto and stirred for 5 minutes. Then the mixture was quenched with 10 mL of ammonia water, and after returning to room temperature, extracted three times with 10 mL of ethyl acetate, and the organic phases were combined, dried and concentrated to obtain *tert*-butyl (*R*)-3,4-dichloro-1-(3,3-dimethylmorpholino)-12-oxo-6a,7,9,10-tetrahydro-12*H*-pyrazino[2,1-*c*]pyrldo[3,4-*f*][1,4]oxazepine-8(6*H*)-carboxylate (250 mg, 93%) as a white solid. ES-API: [M+H]⁺=501.1.

Step 7: Under nitrogen protection, a mixed solution of *tert*-butyl (*R*)-3,4-dichloro-1-(3,3-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (250 mg, 0.50 mmol), 2-fluoro-6-hydroxyphenylboronic acid (233 mg, 1.50 mmol), tetrakis(triphenylphosphine)palladium (58 mg, 0.05 mmol) and potassium carbonate (207 mg, 1.50 mmol) in dioxane (4 mL) and water (1 mL) was reacted at 100°C for half an hour under microwave irradiation. The reaction solution was poured into 20 mL of water, extracted three times with 20 mL of ethyl acetate, and the organic phase was dried and concentrated, and purified by flash silica gel column (EtOAc/PE: 0-100%) to obtain *tert*-butyl (6a*R*)-4-chloro-1-(3,3-dimethylmorpholino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (250 mg, 83%) as a white solid. ES-API: [M+H]⁺=577.2.

Step 8: Under an ice bath, trifluoroacetic acid (1 mL) was added to a solution of *tert*-Butyl (6a*R*)-4-chloro-1-(3,3-dimethylmorpholino)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (250 mg, 0.42 mmol) in dichloromethane (4 mL). The mixture was stirred at room temperature for 1 hour. After the reaction was completed, the mixture was concentrated to obtain a crude product of (6a*R*)-4-chloro-1-(3,3-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one, which was directly used in the next reaction step. ES-API: [M+H]⁺=477.3.

Step 9: Under an ice bath, the crude product obtained above was dissolved in dichloromethane (5 mL), and *N*,*N*-diisopropylethylamine (270 mg, 2.08 mmol) was added thereto. After the reaction solution was clarified, acryloyl chloride (37 mg, 0.42 mmol) was added dropwise thereto and stirred for 10 minutes. The reaction solution was concentrated and purified by flash silica gel column (EtOAc/PE: 0-100%) to obtain (6a*R*)-8-acryloyl-4-chloro-1-(3,3-dimethylmorpholino)-3-(2-fluoro-6-hydroxyphenyl)-6,6a,7,8,9,10-hexahydro-12*H*-pyrazino[2,1-*c*]pyrido[3,4--*f*][1,4]oxazepin-12-one (Z70, 190 mg, 2-step yield of 86%) as a white solid. ES-API: [M+H]⁺=531.1. ¹H NMR (500 MHz, DMSO-*d*₆) δ 10.14-9.93 (m, 1H), 7.33-7.20 (m, 1H), 6.84-6.64 (m, 3H), 6.16 (d, *J* = 16.6 Hz, 1H), 5.81-5.64 (m, 1H), 4.43-3.43 (m, 10H), 3.32-3.25 (m, 3H), 3.22-3.15 (m, 1H), 3.04 (d, *J* = 13.2 Hz, 1H), 1.40-1.26 (m, 3H), 1.26-1.17 (m, 3H).

### Embodiment 57 Synthesis of 1-((6aR)-1-(tert-butylamino)-4-chloro-3-(2-fluoro-6-hydroxyphenyl)-6a,7,9,10-tetrahydro-12H-pyrazino[2,1-c]pyrido[3,4-f][1,4]oxazepin-8(6H)-yl)prop-2-en-1-one Z71 and 1-((6aR)-1-(tert-butylamino)-3-(2-fluoro-6-hydroxyphenyl)-6a,7,9,10-tetrahydro-12H-pyrazino[2,1-c]pyrido[3,4-f][1,4]oxazepin-8(6H)-yl)prop-2-en-1-one Z72

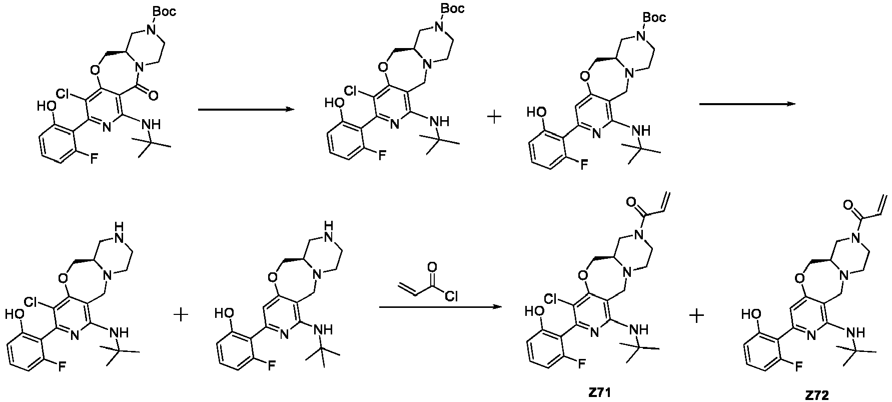

Step 1: (6a*R*)-8-*tert*-Butoxycarbonyl-1-(tert-butylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepan-12-one (50 mg, 0.094 mmol) and THF (2 mL) were added to a 100 mL flask, and borane THF solution (1 M, 4 mL) was added thereto under an ice-water bath. After the addition, the mixture was stirred at 70°C for 3 hours. The mxiture was cooled in an ice-water bath, quenched by the dropwise addition of methanol, extracted with ethyl acetate, washed with saturated brine, and the organic phase was dried and concentrated, and purified by silica gel column to obtain a mixture (30 mg, crude product) of *tert*-butyl (6a*R*)-1-(tert-butylamino)-4-chloro-3-(2-fluoro-6-hydroxyphenyl)-6a,7,9,10-tetrahydro-6*H*-pyrazino[2,1-*c*]pyrido[3,4-*f*][1,4]oxazepine-8(12*H*)-carboxylate and *tert*-butyl (6a*R*)-1-(*tert*-butylamino)-3-(2-fluoro-6-hydroxyphenyl)-6a,7,9,10-tetrahydro-6*H-*pyrazino[2,1-*c*]pyrido[3,4-*f*][1,4]oxazepine-8(12*H*)-carboxylate. ES-API: [M+H]⁺=521.2 (component one); ES-API: [M+H]⁺=487.2 (component two).

Step 2: The above mixture (30 mg), 1 mL of trifluoroacetic acid and 4 mL of dichloromethane were added to a 50 mL flask. The mixture was stirred at room temperature for 1 hour, and the completion of reaction was detected by LC-MS. The reaction solution was concentrated to obtain a mixture of the two (26 mg, crude product) as a yellow solid. ES-API: [M+H]⁺=421.2 (component one); ES-API: [M+H]⁺=387.2 (component two).

Step 3: The above mixture (26 mg), 10 mL of dichloromethane and diisopropylethylamine (42 mg, 0.325 mmol) were added to a 50 mL flask. At 0°C, a solution of acryloyl chloride in dichloromethane (5 mg, 0.058 mmol, 1 mL) was added dropwise thereto. The reaction was carried out at 0°C for 10 minutes. The reaction mixture was washed with 20 mL of water, and the organic phase was dried, concentrated and purified by preparative HPLC to obtain Z71 and Z72.

Z71: retention time of 8.41 min, 1-((6a*R*)-1-(*tert*-butylamino)-4-chloro-3-(2-fluoro-6-hydroxyphenyl)-6a,7,9,10-tetrahydro-12*H*-pyrazino[2,1-*c*]pyrido[3,4-*f*][1,4]oxazepin-8(6*H*)-yl)prop-2-en-1-one (2.4 mg, 3-step yield of 6%). A white solid. ES-API: [M+H]⁺=475.1.

Z72: retention time of 8.57 min, 1-((6a*R*)-1-(1-(*tert*-butylamino)-3-(2-fluoro-6-hydroxyphenyl)-6a,7,9,10-tetrahydro-12H-pyrazino[2,1-*c*]pyrido[3,4-*f*][1,4]oxazepin-8(6*H*)-yl)prop-2-en-1-one (0.96 mg, 3-step yield of 2.5%). A white solid. ES-API: [M+H]⁺=441.1.

### Embodiment 58 Synthesis of (6aR)-8-acryloyl-4-chloro-1-(2,2-dimethyl-5-oxopyrrolidin-1-yl)-3-(2-fluoro-6-hydroxyphenyl)-6,6a,7,8,9,10-hexahydro-12H-pyrazino[2,1-c]pyrido[3,4-f][1,4]oxazepin-12-one Z73

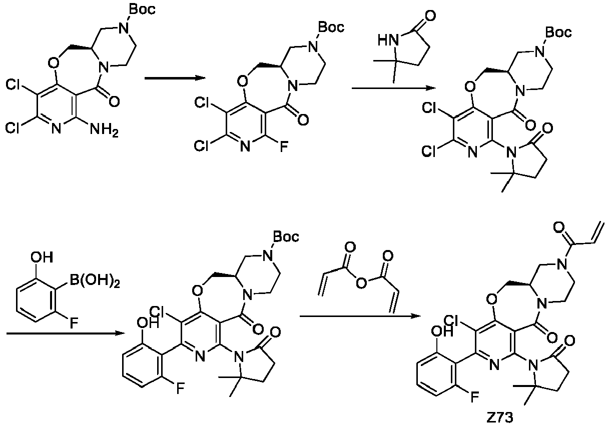

Step 1: *tert*-Butyl (*R*)-1-amino-3,4-dichloro-12-oxo-6a,7,9,10-tetrahydro-12*H*-pyrazino[2,1-c]pyrido[3,4-*f*][1,4]oxazepine-8(6*H*)-carboxylate (1 g, 2.48 mmol) and a solution of 70% pyridine hydrofluoride were added to a 100 mL plastic flask. The mixture was cooled to 0°C, and sodium nitrite (206 mg, 2.98 mmol) was slowly added thereto, stirred for 5 minutes. The reaction solution was poured into iced 2 M aqueous sodium hydroxide solution (40 mL) and THF (30 mL). BoczO (3.2 g, 14.8 mmol) was added thereto. The reaction was stirred at 0°C for 20 minutes. The reaction mixture was extracted with ethyl acetate. The organic phase was washed with 1 M hydrochloric acid and saturated brine, respectively, dried and concentrated. The crude product was purified by flash silica gel column (EtOAc/PE: 10-50%) to obtain *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (600 mg, 59%). ES-API: [M+H]⁺=406.1.

Step 2: *tert*-Butyl (*R*)-3,4-dichloro-1-fluoro-12-oxo-6a,7,9,10-tetrahydro-12*H*-pyrazino[2,1-c]pyrido[3,4-*f*][1,4]oxazepine-8(6*H*)-carboxylate (400 mg, 0.98 mmol), 5,5-dimethylpyrrolidin-2-one (1.1 g, 9.8 mmol), cesium carbonate (964 mg, 2.94 mmol) and 5 mL of toluene were added to a 100 mL flask. The reaction solution was stirred at 110°C for 2 hours. The reaction solution was poured into ice-water, and extracted with ethyl acetate. The organic phase was dried, concentrated and then purified by flash silica gel column (EtOAc/PE: 10-100%) to obtain *tert*-butyl (*R*)-3,4-dichloro-1-(2,2-dimethyl-5-oxopyrrolidin-1-yl)-12-oxo-6a,7,9,10-tetrahydro-12*H*-pyrazino[2,1-*c*]pyrido[3,4*-f*][1,4]oxazepine-8(6*H*)-carboxylate (300 mg, crude product) as a yellow oil. ES-API: [M+H]⁺=443.0.

Step 3: *tert*-Butyl (*R*)-3,4-dichloro-1-(2,2-dimethyl-5-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (300 mg, crude product), 2-fluoro-6-hydroxyphenylboronic acid (281 mg, 1.8 mmol), Pd(PPh₃)₄ (69 mg, 0.006 mmol), sodium carbonate (191 mg, 1.8 mmol), 6 mL of dioxane and 2 mL of water were added to a 100 mL reaction flask. The reaction was stirred for 30 minutes at 110°C under microwave irradiation, and the reaction was stopped. The reaction was added with 20 mL of water, extracted with ethyl acetate, and the organic phase was dried, and then purified by flash silica gel column (EtOAc/PE: 0-100%) to obtain *tert*-butyl (6a*R*)-4-chloro-1-(2,2-dimethyl-5-oxopyrrolidin-1-yl)-3-(2-fluoro-6-hydroxyphenyl)-12-oxo-6a,7,9,10-tetrahydro-12*H-*pyrazino[2.1-*c*]pyrido[3.4-*f*][1.4|oxazepine-8(6H)-carboxylate (120 mg, crude product) as a yellow oil. ES-API: [M+H]⁺=575.2.

Step 4: *tert*-Butyl (6a*R*)-4-chloro-1-(2,2-dimethyl-5-oxopyrrolidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (120 mg, crude product), trifluoroacetic acid (1 mL) and dichloromethane (0.5 mL) were added to a 100 mL reaction flask. After stirring at room temperature for 1 hour, the reaction solution was concentrated to obtain a yellow solid. The yellow solid was dissolved in 3 mL of dichloromethane and triethylamine (85 mg, 0.84 mmol). The reaction was cooled to 0°C and acrylic anhydride (21 mg, 0.17 mmol) was added to the reaction solution. The reaction was carried out at 0°C for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-1-(2,2-dimethyl-5-oxopyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z73, 10.45 mg, 3-step yield of 2%) as a white solid. ES-API: [M+H]⁺=529.1.

### Embodiment 59 Synthesis of (6aR)-8-acryloyl-4-chloro-3-(2-fluoro-6-hydroxyphenyl)-1-(2-methyl-5-oxopyrrolidin-1-yl)-6,6a,7,8,9,10-hexahydro12H-pyrazino[2,1-c]pyrido[3,4-f][1,4]oxazepin-12-one Z78

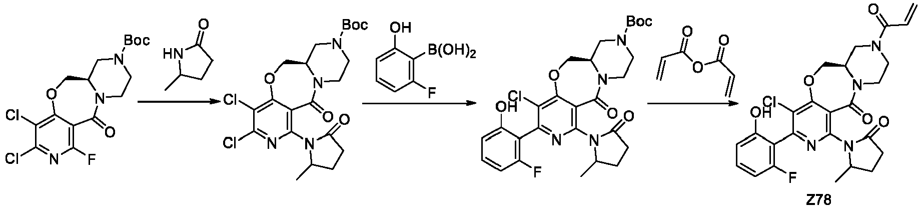

Step 1: *tert*-Butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (300 mg, 0.74 mmol), 5-methylpyrrolidin-2-one (733 mg, 7.4 mmol), cesium carbonate (728 mg, 2.22 mmol) and 5 mL of toluene were added to a 100 mL flask. The reaction solution was stirred at 110°C for 2 hours. The reaction solution was poured into ice-water and extracted with *tert*-butyl methyl ether. The organic phase was dried and concentrated to obtain tert-butyl (6a*R*)-3,4-dichloro-1-(2-methyl-5 -oxopyrrolidin-1-yl)-12-oxo-6a,7,9,10-tetrahydro-12*H*-pyrazino [2,1-*c*]pyrido[3,4-*f*][1,4]oxazepine-8(6*H*)-carboxylate (350 mg, crude product) as a yellow oil. ES-API: [M+H]⁺=485.1.

Step 2: *tert*-Butyl (6a*R*)-3,4-dichloro-1-(2-methyl-5-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (350 mg, crude product), 2-fluoro-6-hydroxyphenylboronic acid (338 mg, 2.16 mmol), Pd(PPh₃)₄ (83 mg, 0.072 mmol), sodium carbonate (229 mg, 2.16 mmol), 8 mL of dioxane and 2 mL of water were added to a 100 mL reaction flask. The reaction was stirred for 30 minutes at 110°C under microwave irradiation, and the reaction was stopped. The reaction solution was added with 20 mL of water, extracted with ethyl acetate, and the organic phase was dried and concentrated, and purified by flash silica gel column (EtOAc/PE: 0-100%) to obtain tert-butyl (6a*R*)-4-chloro-3-(2-fluoro-6-hydroxyphenyl)-1-(2-methyl-5-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (300 mg, crude product) as a yellow oil. ES-API: [M+H]⁺=561.1.

Step 3: The above crude product (300 mg, crude product), trifluoroacetic acid (2 mL) and dichloromethane (0.5 mL)were added to a 100 mL reaction flask. After stirring at room temperature for 1 hour, the reaction solution was concentrated to obtain a yellow solid. The yellow solid was dissolved in 3 mL of dichloromethane and triethylamine (85 mg, 0.84 mmol). The reaction was cooled to 0°C and acrylic anhydride (21 mg, 0.17 mmol) was added to the reaction solution. The reaction was carried out at 0°C for 10 minutes. 20 mL of saturated aqueous sodium bicarbonate solution was added to the reaction solution, and the mixture was extracted three times with 20 mL of dichloromethane. The organic phase was dried, concentrated and then purified by preparative HPLC to obtain (6a*R*)-8-acryloyl-4-chloro-3-(2-fluoro-6-hydroxyphenyl)-1-(2-methyl-5-oxopyrrolidin-1-yl)-6,6a,7,8,9,10-hexahydro-12*H*-pyrazino[2,1-*c*]pyrido[3,4-*f*][1,4]oxazepin-12-one (Z78, 65 mg, 3-step yield of 23%) as a white solid. ¹H NMR (500 MHz, CDCl₃) 7.34-7.26 (m, 1H), 6.86-6.83 (m, 1H), 6.74-6.70 (m, 1H), 6.60-6.55 (m, 1H), 6.44-6.40 (m, 1H), 5.82-5.80 (m, 1H), 4.43-4.38 (m, 1H), 4.32-3.72 (m, 9H), 2.57-2.48 (m, 1H), 2.41-2.35 (m, 2H), 1.82-1.76 (m, 1H), 1.56-1.35 (m, 3H). ES-API: [M+H]⁺=515.0.

### Embodiment 60 Synthesis of (R)-9-acryloyl-2-(2-fluoro-6-hydroxyphenyl)-4-((S)-3-methylmorpholino)-7,8,9,10,10a,11-hexahydro-5H-pyrazino[2,1-c]pyrimido[5,4-f][1,4]oxazepin-5-one Z80

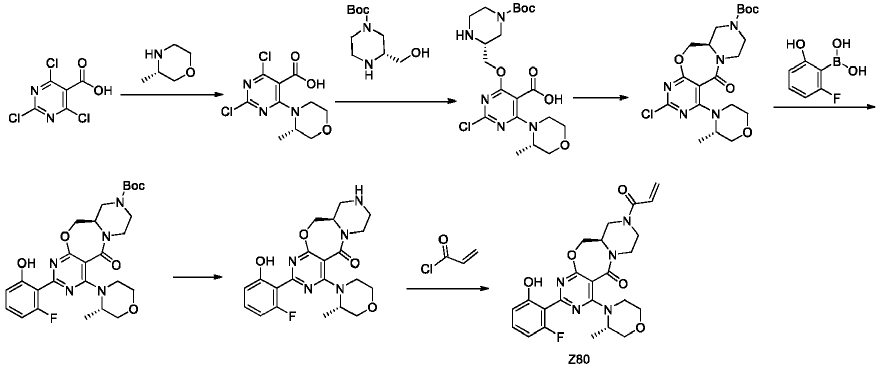

Step 1: 2,4,6-Trichloropyrimidine-5-carboxylic acid (100 mg, 0.44 mmol) was dissolved in 4 mL of THF, and triethylamine (133 mg, 1.32 mmol) and (S)-3-methylmorpholine (49 mg, 0.48 mmol) were added in turn at 0°C, reacted at room temperature for 16 hours. The pH of the reaction solution was adjusted to 3 with 3.0 M dilute hydrochloric acid, and the reaction solution was added with 40 mL of ethyl acetate. The organic phase was washed with 15 mL of saturated brine, dried and concentrated to obtain (S)-2,4-dichloro-6-(3-methylmorpholino)pyrimidine-5-carboxylic acid (125 mg, yield of 97%) as a pale yellow solid. ES-API: [M+H]⁺=292.0.

Step 2: (*R*)-1-Boc-3-hydroxymethylpiperazine (93 mg, 0.43 mmol) was dissolved in 5 mL of THF, NaH (52 mg, 1.29 mmol) was added thereto at 0°C, and the reaction was stirred at 0°C for 15 minutes. A solution of (S)-2,4-dichloro-6-(3-methylmorpholino)pyrimidine-5-carboxylic acid (125 mg, 0.43 mmol) in 1 mL of THF was added dropwise to the above suspension. After the dropwise addition, the reaction was slowly heated to room temperature, and stirred at room temperature for 3 hours. The reaction solution was cooled to 0°C, and the pH of the reaction solution was adjusted to 4 with 3.0 M dilute hydrochloric acid, evaporated to dryness by rotary evaporation and then added with 20 mL of dichloromethane, filtered, and the filtrate was concentrated to obtain (4-(((*R*)-4-(*tert-*butoxycarbonyl)piperazin-2-yl)methoxy)-2-chloro-6-((*S*)-3-methylmorpholino)pyrimidine-5-carboxylic acid (220 mg, crude product), which was directly used in the next reaction step. ES-API: [M+H]⁺=472.2.

Step 3: (4-(((*R*)-4-(*tert*-Butoxycarbonyl)piperazin-2-yl)methoxy)-2-chloro-6-((*S*)-3-methylmorpholino)pyrimidine-5-carboxylic acid (220 mg, crude product) was dissolved in 5 mL of dichloromethane, and 1 mL of triethylamine and 1 mL of propylphosphonic anhydride 50%w/w ethyl acetate solution were added thereto in turn, reacted at room temperature for 2 hours. 30 mL of dichloromethane was added to the reaction, and the organic phase was washed twice with 15 mL of water, washed with 15 mL of saturated brine in turn, dried and concentrated. The crude product was purified by flash silica gel column (EtOAc/PE: 0-100%) to obtain *tert*-butyl (*R*)-2-chloro-4-((*S*)-3-methylmorpholino)-5-oxo-7,8,10a,11-tetrahydro-5*H*-pyrazino[2,1-*c*]pyrimido[5,4-*f*][1,4]oxazepine-9(10*H*)-carboxylate (105 mg, 2-step yield of 54%) as a white solid. ES-API: [M+H]⁺=454.1.

Step 4: *tert*-Butyl (*R*)-2-chloro-4-((*S*)-3-methylmorpholino)-5-oxo-7,8,10a,11-tetrahydro-5*H-*pyrazino[2,1-*c*]pyrimido[5,4-*f*][1,4]oxazepine-9(10*H*)-carboxylate (75 mg, 0.16 mmol), (2-fluoro -6-hydroxyphenyl)boronic acid (100 mg, 0.64 mmol), sphos-Pd-G2 (12 mg, 0.016 mmol), sphos (7 mg, 0.016 mmol), sodium carbonate (51 mg, 0.48 mmol), 2 mL water and 9 mL dioxane were added to a 50 mL flask. The reaction was stirred at 90°C for 2 hours under nitrogen protection. The reaction solution was added with 10 mL of water, extracted with 30 mL of ethyl acetate, and the organic phase was washed with 10 mL of saturated brine, dried and concentrated, and the crude product was purified by flash silica gel column (EtOAc/PE: 0-60%) to obtain *tert*-butyl (*R*)-2-(2-fluoro-6-hydroxyphenyl)-4-((*S*)-3-methylmorpholino)-5-oxo-7,8,10a,11-tetrahydro-5*H*-pyrazino[2,1-c]pyrimido[5,4-*f*][1,4]oxazepine-9(10*H*)-carboxylate (85 mg, 97%) as a white solid. ES-API: [M+H]⁺=530.2.

Step 5: *tert*-Butyl (*R*)-2-(2-fluoro-6-hydroxyphenyl)-4-((*S*)-3-methylmorpholino)-5-oxo-7,8,10a,11-tetrahydro-5*H*-pyrazino[2,1-*c*]pyrimido[5,4-*f*][1,4]oxazepine-9(10*H*)-carboxylate (85 mg, 0.16 mmol) was dissolved in 3 mL of dichloromethane, and 1 mL of trifluoroacetic acid was added thereto. After stirring at room temperature for 1 hour, the reaction solution was concentrated to obtain (R)-2-(2-fluoro-6-hydroxyphenyl)-4-((5)-3-methylmorpholino)-7,8,9,10,10a,11-hexahydro-5*H*-pyrazino[2,1-*c*]pyrimido[5,4-*f*][1,4]oxazepin-5-one (120 mg, crude product), which was directly used in the next reaction step. ES-API: [M+H]⁺=430.1.

Step 6: (*R*)-2-(2-Fluoro-6-hydroxyphenyl)-4-((*S*)-3-methylmorpholino)-7,8,9,10,10a,11-hexahydro-5*H*-pyrazino[2,1-*c*]pyrimido[5,4-*f*][1,4]oxazepin-5-one (120 mg, crude product) was dissolved in 5 mL of dichloromethane, and triethylamine (81 mg, 0.80 mmol) was added thereto. Acryloyl chloride (13 mg, 0.14 mmol) was added dropwise thereto at 0°C, and the reaction solution was stirred at 0°C for 15 minutes. The reaction solution was added with 30 mL of dichloromethane, washed with 15 mL of water, 15 mL of saturated aqueous NaHCO₃ solution, and 15 mL of saturated brine in turn, dried, concentrated, and purified by preparative HPLC to obtain (*R*)-9-acryloyl-2-(2-fluoro-6-hydroxyphenyl)-4-((*S*)-3-methylmorpholino)-7,8,9,10,10a,11-hexahydro-5*H*-pyrazino[2,1-*c*]pyrimido[5,4-*f*][1,4]oxazepin-5-one (Z80, 42 mg, 2-step yield of 54%) as a white solid. ¹H NMR (500 MHz, DMSO-*d*₆) δ 12.71 (s, 1H), 7.33 (dd, *J* = 14.5, 8.5 Hz, 1H), 6.86-6.65 (m, 3H), 6.17 (d, *J* = 16.5 Hz, 1H), 5.75 (d, *J* = 9.5 Hz, 1H), 4.58-4.25 (m, 4H), 4.04-3.45 (m, 11H), 3.40 (t, *J* = 12.0 Hz, 1H), 1.34 (d, *J* = 6.5 Hz, 3H). ES-API: [M+H]⁺=484.1.

### Embodiment 61 Synthesis of (6aR)-8-acryloyl-4-chloro-3-(2-fluoro-6-hydroxyphenyl)-1-(8-oxa-5-azaspiro[3.5]nonan-5-yl)-6, 6a,7,8,9,10-hexahydro-12H-pyrazino[2,1-c]pyrido[3,4-f][1,4]oxazepin-12-one Z90

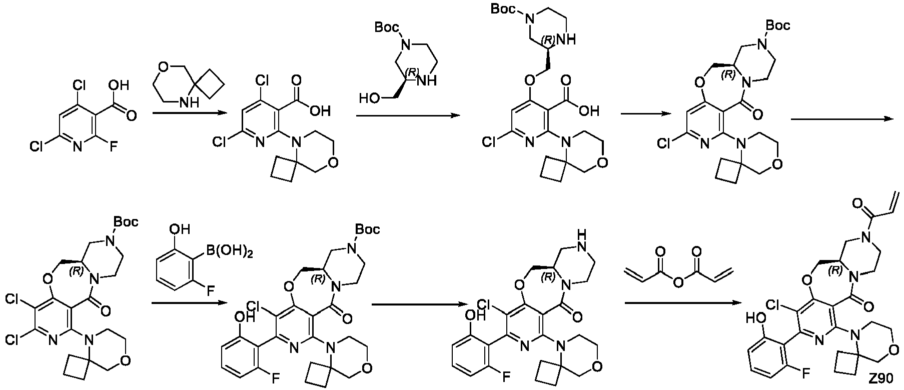

Step 1: 4,6-Dichloro-2-fluoronicotinic acid (288 mg, 1.378 mmol) was dissolved in 3 mL of acetonitrile, and then potassium carbonate (570 mg, 4.134 mmol) and 8-oxa-5-azaspiro[3.5]nonane (175 mg, 1.378 mmol) were added thereto. The reaction was stirred and reacted at 80°C for 10 hours. The reaction solution was added with water and dilute hydrochloric acid to adjust pH to 5-6, extracted with ethyl acetate, and the organic phase was washed with brine, dried over anhydrous sodium sulfate and concentrated to obtain a crude product of 4,6-dichloro-2-(8-oxa-5-azaspiro[3.5]nonan-5-yl)nicotinic acid (180 mg, yield: 41.4%). ES-API: [M+H]⁺=317.0.

Step 2: Sodium hydride (228 mg, 5.7 mmol) was suspended in 1 mL of THF and cooled to 0°C. A solution of *tert*-butyl (*R*)-3-(hydroxymethyl)piperazine-1-carboxylate (246 mg, 1.14 mmol) in THF (1 mL) was added dropwise thereto, and after the addition, the mixture was stirred and reacted for 30 minutes while maintaining the temperature. A solution of 4,6-dichloro-2-(8-oxa-5-azaspiro[3.5]nonan-5-yl)nicotinic acid (180 mg, 0.57 mmol) in THF (1 mL) was added dropwise thereto, and after the addition, the mixture was stirred and reacted at 70°C for 4 hours. The reaction solution was quenched with saturated ammonium chloride solution, extracted with ethyl acetate, and the organic phase was washed with brine, dried over anhydrous sodium sulfate, and concentrated to obtain (*R*)-4-((4-(*tert*-butoxycarbonyl)piperazin-2-yl)methoxy)-6-chloro-2-(8-oxa-5-azaspiro[3.5]nonan-5-yl)nicotinic acid (350 mg, yield: 100%). The crude product was directly used in the next reaction step. ES-API: [M+H]⁺=497.3.

Step 3: (*R*)-4-((4-(*tert*-Butoxycarbonyl)piperazin-2-yl)methoxy)-6-chloro-2-(8-oxa-5-azaspiro[3.5]nonan-5-yl)nicotinic acid (350 mg, 0.706 mmol) was dissolved in 3 mL of dichloromethane, and 0.5 mL of *N*,*N*-diisopropylethylamine and HATU (322 mg, 0.847 mmol) were added thereto. After the addition, the reaction solution was stirred at room temperature for 1 hour. The reaction solution was washed with saturated sodium bicarbonate solution, and the organic phase was washed with brine, dried over anhydrous sodium sulfate, and concentrated to obtain a crude product of *tert*-butyl (*R*)-3-chloro-12-oxo-1-(8-oxa-5-azaspiro[3.5]nonan-5-yl)-6a,7,9,10-tetrahydro-12*H*-pyrazino[2,1-*c*]pyrido[3,4-*f*][1,4]oxazepine-8(6*H*)-carboxylate (87 mg, yield: 31.9%). ES-API: [M+H]⁺=479.2.

Step 4: *tert*-Butyl (*R*)-3-chloro-12-oxo-1-(8-oxa-5-azaspiro[3.5]nonan-5-yl)-6a,7,9,10-tetrahydro-12*H*-pyrazino[2,1-*c*]pyrido[3,4-*f*][1,4]oxazepine-8(6*H*)-carboxylate (87 mg, 0.182 mmol) was dissolved in 1 mL of acetonitrile, and *N*-chlorosuccinimide (24.3 mg, 0.182 mmol) was added thereto. The reaction solution was stirred and reacted at 80°C for 10 hours. The reaction solution was added with water, extracted with ethyl acetate, and the organic phase was washed with brine, dried over anhydrous sodium sulfate, and concentrated to obtain *tert*-butyl (*R*)-3.4-chloro-12-oxo-1-(8-oxa-5-azaspiro[3.5]nonan-5-yl)-6a,7,9,10-tetrahydro-12*H*-pyrazino[2,1-*c*]pyrido[3,4-*f*][1,4]oxazepine-8(6*H*)-carboxylate (80 mg, yield: 85.8%). ES-API: [M+H]⁺=513.2.

Step 5: *tert*-Butyl (*R*)-3,4-chloro-12-oxo-1-(8-oxa-5-azaspiro[3.5]nonan-5-yl)-6a,7,9,10-tetrahydro-12*H*-pyrazino[2,1-*c*]pyrido[3,4-*f*][1,4]oxazepine-8(6*H*)-carboxylate (80 mg, 0.156 mmol) was suspended in dioxane/water (1 mL/0.25 mL), and sodium carbonate (83 mg, 0.78 mmol), (2-fluoro-6-hydroxyphenyl)boronic acid (73 mg, 0.468 mmol) and tetrakis(triphenylphosphine)palladium (18 mg, 0.0156 mmol) were added thereto. The reaction solution was stirred at 100°C for 4 hours under nitrogen protection. The reaction solution was added with water, extracted with ethyl acetate. The organic phase was washed with brine, dried over anhydrous sodium sulfate, concentrated and purified by thin-layer chromatography (PE/EtOAc=1/1, R_{f}=0.4) to obtain *tert*-butyl (6a*R*)-4-chloro-3-(2-fluoro-6-hydroxyphenyl)-12-oxo-1-(8-oxa-5-azaspiro[3.5]nonan-5-yl)-6a,7,9,10-tetrahydro-12*H*-pyrazino[2,1-*c*]pyrido[3,4-*f*][1,4]oxazepine-8(6*H*)-carboxylate (65 mg, yield: 70.9%). ES-API: [M+H]⁺=589.2

Step 6: *tert*-Butyl (6a*R*)-4-chloro-3-(2-fluoro-6-hydroxyphenyl)-12-oxo-1-(8-oxa-5-azaspiro[3.5]nonan-5-yl)-6a,7,9,10-tetrahydro-12*H*-pyrazino[2,1-*c*]pyrido[3,4-*f*][1,4]oxazepine-8(6*H*)-carboxylate (65 mg, 0.11 mmol) was dissolved in 1 mL of dichloromethane, and 1 mL of trifluoroacetic acid was added thereto. The reaction was carried out at room temperature for 0.5 hours, then the reaction solution was concentrated and redissolved in dichloromethane, and concentrated to obtain a crude product of (6a*R*)-4-chloro-3-(2-fluoro-6-hydroxyphenyl)-1-(8-oxa-5-azaspiro[3.5]nonan-5-yl)-6,6a,7,8,9,10-hexahydro-12*H*-pyrazino[2,1-*c*]pyrido[3,4-*f*][1,4]oxazepin-12-one (70 mg,yield: 100%). The crude product was directly used in the next reaction step. ES-API: [M+H]⁺=485.2.

Step 7: (6a*R*)-4-Chloro-3-(2-fluoro-6-hydroxyphenyl)-1-(8-oxa-5-azaspiro[3.5]nonan-5-yl)-6,6a,7,8,9,10-hexahydro-12*H*-pyrazino[2,1-*c*]pyrido[3,4-*f*][1,4]oxazepin-12-one (70 mg, 0.11 mmol) was dissolved in 1 mL of dichloromethane, cooled with ice-water, added with 1 mL of triethylamine, and then slowly added with acrylic anhydride (11.1 mg, 0.088 mmol), reacted at 10°C for 10 minutes, then added with water, extracted with dichloromethane. The organic phase was washed with brine, dried over anhydrous sodium sulfate, concentrated, and purified by thin-layer chromatography (DCM/MeOH=10/1, R_{f}=0.5) to obtain (6a*R*)-8-acryloyl-4-chloro-3-(2-fluoro-6-hydroxyphenyl)-1-(8-oxa-5-azaspiro[3.5]nonan-5-yl)-6,6a,7,8,9,10-hexahydro-12*H*-pyrazino[2,1-*c*]pyrido[3,4-*f*][1,4]oxazepin-12-one (Z90, 8.1 mg, yield: 13.6%). ¹H NMR (400 MHz, CDCl₃) δ7.28 (s, 1H), 6.82 (d, *J* = 8.2 Hz, 1H), 6.69 (t, *J* = 9.0 Hz, 1H), 6.54 (s, 1H), 6.41 (d, *J* = 16.3 Hz, 1H), 5.83 (d, *J* = 10.0 Hz, 1H), 4.40 (s, 1H), 4.26 (s, 3H), 4.07 (s, 1H), 3.88 (d, *J* = 11.1 Hz, 3H), 3.71 (s, 3H), 3.60-3.32 (m, 3H), 3.10 (s, 1H), 2.87 (s, 1H), 2.43-2.37 (m, 2H), 2.24-2.14 (m, 1H), 2.03 (s, 1H), 1.41 (s, 1H), 1.30 (s, 1H). ES-API: [M+H]⁺=543.2.

### Embodiment 62 Synthesis of (6aR)-8-acryloyl-1-((2S,6R)-2,6-dimethylmorpholino)-4-chloro-3-(2-fluoro-6-hydroxyphenyl)-6,6a,7,8,9,10-hexahydro-12H-pyrazino[2,1-c]pyrido[3.4-f][1,4]oxazepin-12-one Z247

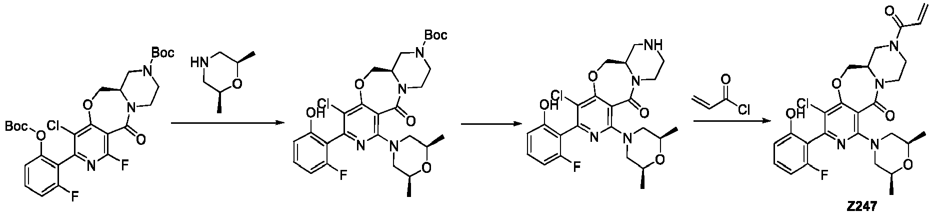

Step 1: (6a*R*)-8-*tert*-Butoxycarbonyl-3-(2-((tert-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (70 mg, 0.120 mmol), (2*S*,6*R*)-2,6-dimethylmorpholine (14 mg, 0.120 mmol), diisopropylethylamine (46 mg, 0.36 mmol) and acetonitrile (5 mL) were added to a 100 mL flask. The reaction solution was stirred at 80°C for 1 hour. The mixture was extracted with ethyl acetate, washed with aqueous ammonium chloride solution and saturated brine in turn, and the organic phase was dried and concentrated, and purified by silica gel column to obtain (6a*R*)-8-*tert*-butoxycarbonyl-1-((2*S*,6*R*)-2,6-dimethylmoipholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (69 mg, yield of 100%). ES-API: [M+H]⁺=577.2.

Step 2: (6a*R*)-8-*tert*-Butoxycarbonyl-1-((2S,6R)-2,6-dimethylmorpholino)-4-chloro-3-(2-fluoro-6-hydroxyphenyl)-6,6a, 7,8,9,10-hexahydro-12*H*-pyrazino[2,1-*c*]pyrido[3,4-*f*][1,4]oxazepin-12-one (69 mg, 0.12 mmol), 1 mL of trifluoroacetic acid and 2 mL of dichloromethane were added to a 50 mL flask. The mixture was stirred at room temperature for 1 hour, and the completion of reaction was detected by LC-MS. The reaction solution was concentrated to obtain a crude product of (6a*R*)-1-((2*S*,6*R*)-2,6-dimethylmorpholino)-4-chloro-3-(2-fluoro-6-hydroxyphenyl)-6,6a,7,8,9,10-hexahydro-12*H*-pyrazino [2,1-*c*]pyrido[3,4-*f*][1,4]oxazepin-12-one trifluoroacetate (71 mg, crude product) as a yellow solid. ES-API: [M+H]⁺=477.2.

Step 3: (6a*R*)-1-((2*S*,6*R*)-2,6-Dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one trifluoroacetate (71 mg, crude product), 10 mL of dichloromethane and diisopropylethylamine (46 mg, 0.36 mmol) were added to a 50 mL flask. At 0°C, a solution of acryloyl chloride in dichloromethane (10.9 mg, 0.12 mmol, 1 mL) was added dropwise thereto. The reaction was carried out at 0°C for 10 minutes. The mixture was washed with 20 mL of water, and the organic phase was dried and concentrated, and the crude product was purified by preparative HPLC to obtain (6a*R*)-8-acryloyl-1-((2*S*,6*R*)-2,6-dimethylmorphofino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z247, 25 mg, 3-step yield of 39%) as a white solid. ¹H NMR (500 MHz, DMSO-*d*₆) δ 7.25 (q, *J* = 7.8 Hz, 1H), 6.81-6.65 (m, 3H), 6.17 (d, *J* = 16.1 Hz, 1H), 5.74 (t, *J* = 13.9 Hz, 1H), 4.49-4.30 (m, 2H), 4.27-3.99 (m, 3H), 3.86 (d, *J* = 12.8 Hz, 2H), 3.68-3.49 (m, 4H), 3.42 (d, *J* = 14.3 Hz, 2H), 2.58-2.51 (m, 2H), 1.06 (d, *J* = 6.2 Hz, 6H). ES-API: [M+H]⁺=531.1.

### Embodiment 62-1 Synthesis of Z248-1

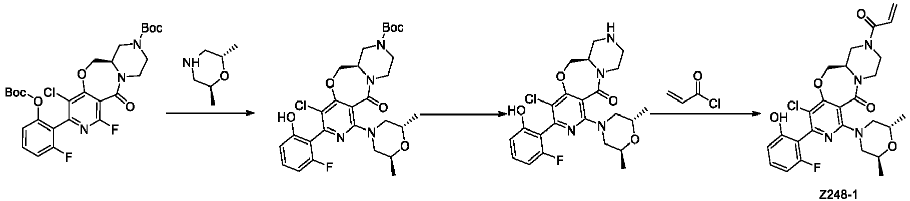

Step 1: (6a*R*)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (50 mg, 0.086 mmol), (2*S*,6*S*)-2,6-dimethylmorpholine (30 mg, 0.258 mmol), *N*,*N*-diisopropylethylamine (33 mg, 0.258 mmol) and acetonitrile (10 mL) were added to a 100 mL round bottom flask, stirred at 80°C for 1.5 hours. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-((2*S*,6*S*)-2,6-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (49.5 mg, yield of 100%). ES-API: [M+H]⁺=577.2.

Step 2: (6a*R*)-8-*tert*-Butoxycarbonyl-1-((2*S*,6*S*)-2,6-dimethylmoipholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (49.5 mg, 0.086 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-((2*S*,6*S*)-2,6-dimethylmorpholino)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (41 mg, crude product) as a yellow solid. ES-API: [M+H]⁺=477.2.

Step 3: (6a*R*)-1-((2*S*,6*S*)-2,6-Dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (41 mg, 0.086 mmol), 10 mL of dichloromethane and *N*,*N*-diisopropylethylamine (33 mg, 0.258 mmol) were added to a 50 mL round bottom flask. The reaction was cooled to 0°C, and a solution of acryloyl chloride in dichloromethane (7 mg, 0.077 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:HzO=50:50 (0.1% NH₄HCO₃), flow rate: 15 mL/min, column temperature: 25°C) to obtain a target product of (6a*R*)-8-acryloyl-1-((2*S*,6*S*)-2,6-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z248-1, 8 mg, 3-step yield of 40%) as a white solid. ES-API: [M+H]⁺= 531.1. ¹H NMR (500 MHz, DMSO-*d*₆) δ 10.02-9.99 (m, 1H), 7.25 (q, *J* = 8.1 Hz, 1H), 6.83 - 6.65 (m, 3H), 6.17 (d, *J* = 16.7 Hz, 1H), 5.75 (d, *J* = 12.2 Hz, 1H), 4.36 (s, 2H), 4.28 - 4.04 (m, 2H), 4.03 - 3.95 (m, 3H), 3.88 (s, 1H), 3.71 - 3.53 (m, 2H), 3.44 (s, 3H), 2.98 (dd, *J* = 13.5, 6.0 Hz, 2H), 1.06 (d, *J* = 6.3 Hz, 6H).

### Embodiment 62-2 Synthesis of Z153

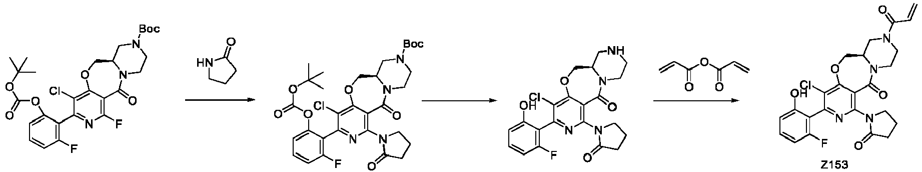

Step 1: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol) and 3 mL of acetonitrile were added to a round bottom flask. Diisopropylethylamine (44 mg, 0.35 mmol) and pyrrolidin-2-one (19 mg, 0.21 mmol) were then added to the reaction solution. The reaction was stirred at 80°C overnight. After the reaction was completed, the organic phase was concentrated to dryness to obtain a crude product of *tert*-butyl (6a*R*)-3-(2-((*tert*-butoxycarbonyl)oxy)-6-fluorophenyl)-4-chloro-12-oxo-1-(2-oxopyrrolidin-1-yl)-6a,7,9,10-tetrahydro-12*H*-pyrazino[2,1-*c*]pyrido[3,4-*f*][1,4]oxazepine-8(6*H*)-carboxylate (55 mg, crude product) as a yellow solid, and the crude product was directly used in the next reaction step. ES-API: [M+H]⁺=648.0.

Step 2: *tert*-Butyl (6a*R*)-3-(2-((*tert*-butoxycarbonyl)oxy)-6-fluorophenyl)-4-chloro-12-oxo-1-(2-oxopyrrolidin-1-yl)-6a,7,9,10-tetrahydro-12*H*-pyrazino[2,1-*c*]pyrido[3,4-*f*][1,4]oxazepine-8(6*H*)-carboxylate (55 mg, 0.08 mmol), 3 mL of dichloromethane and 1.5 mL of trifluoroacetic acid were stirred at room temperature for 1 hour, and the completion of reaction was detected by LC-MS. The reaction solution was concentrated to obtain (6a*R*)-4-chloro-3-(2-fluoro-6-hydroxyphenyl)-1-(2-oxopyrrolidin-1-yl)-6,6a,7,8,9,10-hexahydro-12*H*-pyrazino[2,1-*c*]pyrido[3,4-*f*][1,4]oxazepin-12-one (58 mg) as a brown oily liquid. The crude product was directly used in the next step. ES-API: [M+H]⁺=447.0.

Step 3: (6a*R*)-4-Chloro-3-(2-fluoro-6-hydroxyphenyl)-1-(2-oxopyrrolidin-1-yl)-6,6a,7,8,9,10-hexahydro-12*H*-pyrazino[2,1-*c*]pyrido[3,4--*f*][1,4]oxazepin-12-one (58 mg, 0.10 mmol), 10 mL of dichloromethane and triethylamine (130 mg, 1.2 mmol) were added to a round bottom flask. The reaction was cooled to 0°C, and a solution of acrylic anhydride in dichloromethane (12 mg, 0.10 mmol, 1 mL) was added to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-3-(2-fluoro-6-hydroxyphenyl)-1-(2-oxopyrrolidin-1-yl)-6,6a,7,8,9,10-hexahydro-12*H*-pyrazino[2,1-*c*]pyrido[3,4-*f*][1,4]oxazepin-12-one (Z153, 2.5 mg, 5.0%). ES-API: [M+H]⁺=501.1.

### Embodiment 62-3 Synthesis of Z263

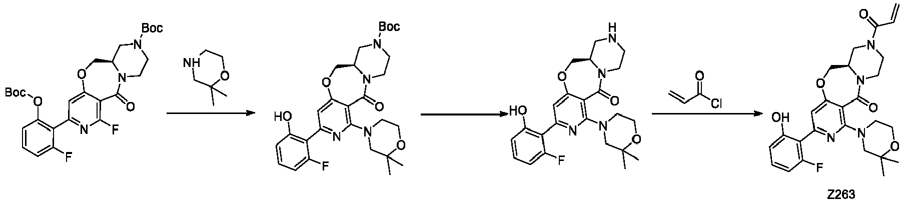

Step 1: (6a*R*)-8-*tert*-Butoxycarbonyl-3-(2-((tert-butoxycarbonyl)oxy)-6-fluorophenyl)-1-fluoro-6,6a,7,8,9,10-hexahydro-12*H*-pyrazino[2,1-*c*]pyrido[3,4-*f*][1,4]oxazepin-12-one (22 mg, 0.039 mmol), 2,2-dimethylmorpholine (13.4 mg, 0.117 mmol), *N*,*N*-diisopropylethylamine (15 mg, 0.117 mmol) and acetonitrile (10 mL) were added to a 100 mLround bottom flask, stirred at 80°C for 1.5 hours. The mixture was extracted with ethyl acetate, washed with saturated brine, and the organic phase was dried, concentrated, and purified by silica gel column (petroleum ether: ethyl acetate = 20:80) to obtain (6a*R*)-8-*tert-*butoxycarbonyl-1-(2,2-dimethylmorpholino)-3-(2-fluoro-6-hydroxyphenyl)-6,6a,7,8,9,10-hexahydro-12*H-*pyrazino[2,1-*c*]pyrido[3,4-*f*][1,4]oxazepin-12-one (25 mg, crude product). ES-API: [M+H]⁺=543.3.

Step 2: (6a*R*)-8-*tert*-Butoxycarbonyl-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (25 mg, crude product), 2 mL of trifluoroacetic acid and 5 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(2,2-dimethylmorpholino)-3-(2-fluoro-6-hydroxyphenyl)-6,6a,7,8,9,10-hexahydro-12*H*-pyrazino[2,1-*c*]pyrido[3.4-*f*][1,4]oxazepin-12-one (30 mg, crude product) as a yellow solid. ES-API: [M+H]⁺=443.3.

Step 3: (6a*R*)-1-(2,2-Dimethylmorpholino)-3-(2-fluoro-6-hydroxyphenyl)-6,6a,7,8,9,10-hexahydro-12*H*-pyrazino[2,1-*c*]pyrido[3,4--*f*][1,4]oxazepin-12-one (30 mg, crude product), 10 mL of dichloromethane and *N*,*N*-diisopropylethylamine (15 mg, 0.117 mmol) were added to a 50 mL round bottom flask. The reaction was cooled to 0°C, and a solution of acryloyl chloride in dichloromethane (3.2 mg, 0.035 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:HzO=50:50 (0.1% NH₄HCO₃), flow rate: 15 mL/min, column temperature: 25°C) to obtain a target product of (6a*R*)-8-acryloyl-1-(2,2-dimethylmorphofino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z263, 12 mg, 3-step yield of 50%) as a white solid. ES-API: [M+H]⁺= 597.2. ¹H NMR (500 MHz, DMSO-*d*₆) δ 13.14-13.08 (m, 1H), 7.29 (td, *J* = 8.2, 6.4 Hz, 1H), 6.87 (s, 1H), 6.83 - 6.63 (m, 3H), 6.17 (d, *J* = 16.6 Hz, 1H), 5.74 (t, *J* = 12.8 Hz, 1H), 4.41 (s, 1H), 4.28 (q, *J* = 11.9, 11.4 Hz, 1H), 4.18-4.16(m, 1H), 4.08-3.99 (m, 2H), 3.86 (s, 1H), 3.76 (td, *J* = 7.5, 6.8, 3.5 Hz, 1H), 3.67 - 3.50 (m, 3H), 3.28 (s, 2H), 3.22 - 3.08 (m, 2H), 1.25 - 1.12 (m, 6H)

### Embodiment 62-4 Synthesis of Z93

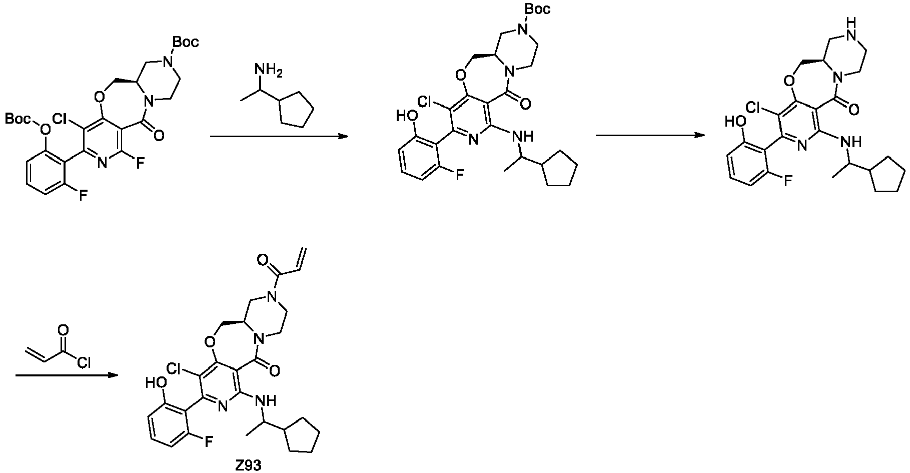

Step 1: (6a*R*)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (50 mg, 0.086 mmol), 1-cyclopentylethylamine (30 mg, 0.258 mmol), *N*,*N*-diisopropylethylamine (33 mg, 0.258 mmol) and acetonitrile (10 mL) were added to a 100 mL round bottom flask, stirred at 80°C for 1.5 hours. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-(((*R*)-1-cyclopentylethyl)amino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (49 mg, yield of 98%). ES-API: [M+H]⁺=575.2.

Step 2: (6a*R*)-8-*tert*-Butoxycarbonyl-1-(((*R*)-1-cyclopentylethyl)amino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (49 mg, 0.086 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(((*R*)-1-cyclopentylethyl)amino)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (41 mg, crude product) as a yellow solid. ES-API: [M+H]⁺=475.2.

Step 3: (6a*R*)-1-(((*R*)-1-Cyclopentylethyl)amino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (41 mg, 0.086), 10 mL of dichloromethane and *N*,*N-*diisopropylethylamine (33 mg, 0.258 mmol) were added to a 50 mL round bottom flask. The reaction was cooled to 0°C, and a solution of acryloyl chloride in dichloromethane (7 mg, 0.077 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:H₂O=50:50 (0.1% NH₄HCO₃), flow rate: 15 mL/min, column temperature: 25°C) to obtain a target product of (6a*R*)-8-acryloyl-1-(((*R*)-1-cyclopentylethyl)amino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z93, 20 mg, 3-step yield of 44%). ES-API: [M+H]⁺=529.2.

### Embodiment 62-5 Synthesis of Z259

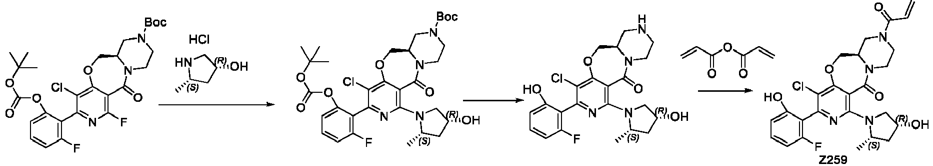

Step 1: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol) and 3 mL of acetonitrile were added to a round bottom flask. Diisopropylethylamine (44 mg, 0.35 mmol) and (3*R*,5*S*)-5-methylpyrrolidin-3-ol hydrochloride (29 mg, 0.21 mmol) were then added to the reaction solution. The reaction was stirred at 80°C overnight. After the reaction was completed, the organic phase was concentrated to dryness to obtain a crude product of *tert*-butyl (6a*R*)-3-(2-((*tert-*butoxycarbonyl)oxy)-6-fluorophenyl)-4-chloro-1-((2*S*,4*R*)-4-hydroxy-2-methylpyrrolidin-1-yl)-12-oxo-6a,7,9,10-tetrahydro-12*H*-pyrazino[2,1-*c*]pyrazino[3,4-*f*][1,4]oxazepine-8(6*H*)-carboxylate (65 mg, crude product) as a yellow solid, and the crude product was directly used in the next reaction step. ES-API: [M+H]⁺=663.1.

Step 2: (6a*R*)-3-(2-((*tert*-Butoxycarbonyl)oxy)-6-fluorophenyl)-4-chloro-1-((2*S*,4*R*)-4-hydroxy-2-methylpyrrolidin-1-yl)-12-oxo-6a,7,9,10-tetrahydro-12*H*-pyrazino[2,1-*c*]pyrido[3.4-*f*][1,4]oxazepine-8(6*H*)-carboxylate (68 mg, 0.1 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-3-(2-fluoro-6-hydroxyphenyl)-1-((2*S*,4*S*)-4-hydroxy-2-methylpyrrolidin-1-yl)-6,6a,7,8,9,10-hexahydro-12*H-*pyrazino[2,1-*c*]pyrido[3,4-*f*][1,4]oxazepin-12-one (76 mg) as a brown oily liquid. The crude product was directly used in the next step. ES-API: [M+H]⁺=463.0.

Step 3: (6a*R*)-4-Chloro-3-(2-fluoro-6-hydroxyphenyl)-1-((2*S*,4*R*)-4-hydroxy-2-methylpyrrolidin-1-yl)-6,6a,7,8,9,10-hexahydro-12*H*-pyrazino[2,1-*c*]pyrido[3,4-*f*][1,4]oxazepin-12-one (76 mg, 0.17 mmol), 10 mL of dichloromethane and triethylamine (130 mg, 1.2 mmol) were added to around bottom flask. The reaction was cooled to 0°C, and a solution of acrylic anhydride in dichloromethane (12 mg, 0.10 mmol, 1 mL) was added to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-3-(2-fluoro-6-hydroxyphenyl)-1-((2*S*,4*R*)-4-hydroxy-2-methylpyrrolidin-1-yl)-6,6a,7,8,9,10-hexahydro-12*H-*pyrazino[2,1-*c*]pyrido[3,4-*f*][1,4]oxazepin-12-one (Z259, 21 mg, 24%). ES-API: [M+H]⁺=517.1. ¹H NMR (500 MHz, DMSO-*d*₆) δ 9.95 (s, 1H), δ 7.23 (dd, *J* = 15.3, 8.2 Hz, 1H), 6.91 - 6.58 (m, 3H), 6.17 (d, *J* = 17.0 Hz, 1H), 6.05 (s, 1H), 5.74 (d, *J* = 9.0 Hz, 1H), 5.15-4.72 (m, 1H), 4.46 (s, 1H), 4.39 (s, 2H), 4.24 - 4.17 (m, 2H), 4.14 (s, 4H), 3.72 (d, *J* = 6.7 Hz, 2H), 2.85 (d, *J* = 11.5 Hz, 1H), 2.13-2.02 (m, 1H), 1.65 (d, *J* = 12.8 Hz, 1H), 1.40 (d, *J* = 6.0 Hz, 3H).

### Embodiment 62-6 Synthesis of Z260

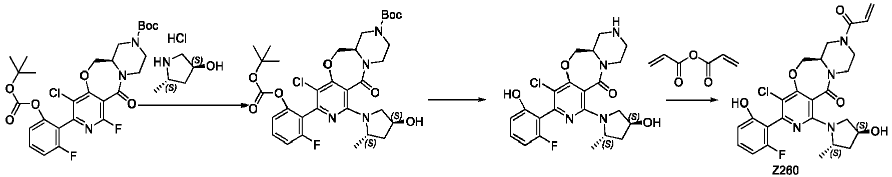

Step 1: *tert*-Butyl (6a*R*)-3-(2-((tert-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol) and 3 mL of acetonitrile were added to a round bottom flask. Diisopropylethylamine (44 mg, 0.35 mmol) and (3*S*,5*S*)-5-methylpyrrolidin-3-ol hydrochloride (29 mg, 0.21 mmol) were then added to the reaction solution. The reaction was stirred at 80°C overnight. After the reaction was completed, the organic phase was concentrated to dryness to obtain a crude product of *tert*-butyl (6a*R*)-3-(2-((*tert-*butoxycarbonyl)oxy)-6-fluorophenyl)-4-chloro-1-((2*S*,4*S*)-4-hydroxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (68 mg, crude product) as a yellow solid, and the crude product was directly used in the next reaction step. ES-API: [M+H]⁺=663.1.

Step 2: (6a*R*)-3-(2-((*tert*-Butoxycarbonyl)oxy)-6-fluorophenyl)-4-chloro-1-((2*S*,4*S*)-4-hydroxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (68 mg, 0.1 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-3-(2-fluoro-6-hydroxyphenyl)-1-((2*S*,4*S*)-4-hydroxy-2-methylpyrrolidin-1-yl)-6,6a,7,8,9,10-hexahydro-12*H-*pyrazino[2,1-*c*]pyrido[3,4-*f*][1,4]oxazepin-12-one (76 mg) as a brown oily liquid. The crude product was directly used in the next step. ES-API: [M+H]⁺=463.0.

Step 3: (6a*R*)-4-Chloro-3-(2-fluoro-6-hydroxyphenyl)-1-((2*S*,4*S*)-4-hydroxy-2-methylpyrrolidin-1-yl)-6,6a,7,8,9,10-hexahydro-12*H*-pyrazino[2,1-*c*]pyrido[3,4-*f*][1,4]oxazepin-12-one (76 mg, 0.17 mmol), 10 mL of dichloromethane and triethylamine (130 mg, 1.2 mmol) were added to around bottom flask. The reaction was cooled to 0°C, and a solution of acrylic anhydride in dichloromethane (12 mg, 0.10 mmol, 1 mL) was added to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-3-(2-fluoro-6-hydroxyphenyl)-l-((2*S*,4*S*)-4-hydroxy-2-methylpyrrolidin-1-yl)-6,6a,7,8,9,10-hexahydro-12*H-*pyrazino[2,1-*c*]pyrido[3,4-*f*][1,4]oxazepin-12-one (Z260, 21 mg, 24%). ES-API: [M+H]⁺=517.2. ¹H NMR (500 MHz, DMSO-*d*₆) δ 9.92 (s, 1H), δ 7.23 (dd, *J* = 15.2, 8.2 Hz, 1H), 6.92 - 6.60 (m, 3H), 6.28 - 6.11 (m, 1H), 5.74 (s, 1H), 5.14 (s, 1H), 4.54-4.39 (m, 2H), 4.35-4.25 (s, 1H), 4.24-4.14 (m, 2H), 4.15 - 3.41 (m, 6H), 3.24-3.14 (m, 2H), 1.87-1.78 (m, 2H), 1.18-1.12 (m, 3H).

### Embodiment 62-7 Synthesis of Z248-2

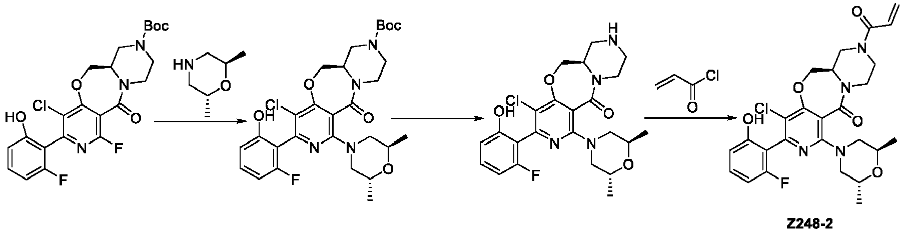

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (580 mg, 1.0 mmol), (2*R*,6*R*)-2,6-dimethylmorpholine (128 mg, 1.0 mmol), *N*,*N-*diisopropylethylamine (390 mg, 3.0 mmol) and acetonitrile (15 mL) were added to a 50 mL round bottom flask, reacted at 60°C for 1 hour. The reaction solution was added with ethyl acetate (30 mL), washed with water (30 mL*3), and the organic phase was dried and concentrated, and the residue was purified by flash silica gel column (ethyl acetate:petroleum ether: 0-90%) to obtain *tert*-butyl (6a*R*)-4-chloro-1-((2*R*,6*R*)-2,6-dimethylmorpholino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (50 mg, yield of 9%). ES-API: [M+H]⁺=577.2.

Step 2: Trifluoroacetic acid (2 mL) was added to a solution of *tert*-butyl (6a*R*)-4-chloro-1-((2*R*,6*R*)-2,6-dimethylmorpholino)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (50 mg, 0.09 mmol) in dichloromethane (5 mL), reacted at room temperature for 1 hour. The reaction solution was directly concentrated to obtain a crude product of (6a*R*)-4-chloro-1-((2*R*,6*R*)-2,6-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg). ES-API: [M+H]⁺=477.2.

Step 3: The above crude procuct of (6a*R*)-4-chloro-1-((2*R*,6*R*)-2,6-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, 0.06 mmol) was dissolved in dichloromethane (2 mL), and *N*,*N*-diisopropylethylamine (23 mg, 0.18 mmol) was added thereto. The reaction solution was cooled to 0°C, and acryloyl chloride (4 mg, 0.05 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-((2*R*,6*R*)-2,6-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one. (Z248-2, 16 mg, yield: 50%). ES-API: [M+H]⁺=531.1. ¹H NMR (500 MHz, DMSO-*d*₆) δ 9.98 (s, 1H), 7.25 (d, *J* = 7.9 Hz, 1H), 6.83-6.60 (m, 3H), 6.17 (d, *J* = 16.9 Hz, 1H), 5.75 (d, *J* = 10.7 Hz, 1H), 4.60-3.50 (m, 11H), 3.17 (s, 4H), 1.10 (s, 6H).

### Embodiment 62-8 Synthesis of Z246

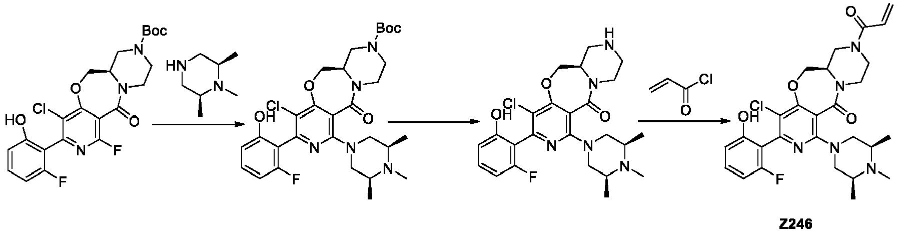

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (24 mg, 0.05 mmol), (2*S*,6*R*)-1,2,6-trimethylpiperazine (13 mg, 0.1 mmol), *N*,*N*-diisopropylethylamine (26 mg, 0.2 mmol) and acetonitrile (2 mL) were added to a 50 mL round bottom flask, reacted at 80°C for 2 hours. The reaction solution was added with ethyl acetate (10 mL), washed with water (10 mL*3), and the organic phase was dried and concentrated, and the residue was purified by flash silica gel column (ethyl acetate :petroleum ether: 0-90%) to obtain *tert*-butyl (6a*R*)-4-chloro-3-(2-fluoro-6-hydroxyphenyl)-12-oxo-1-((3*S*,5*R*)-3,4,5-trimethylpiperazin-1-yl)-6a,7,9,10-tetrahydro-12*H*-pyrazino[2,1-*c*]pyrido[3,4-*f*][1,4]oxazepine-8(6*H*)-carboxylate (30 mg, yield of 87%). ES-API: [M+H]⁺=690.3.

Step 2: Trifluoroacetic acid (1 mL) was added to a solution of *tert*-butyl (6a*R*)-4-chloro-3-(2-fluoro-6-hydroxyphenyl)-12-oxo-1-((3*S*,5*R*)-3,4,5-trimethylpiperazin-1-yl)-6a,7,9,10-tetrahydro-12*H-*pyrazino[2,1-*c*]pyrido[3,4-*f*][1,4]oxazepine-8(6*H*)-carboxylate (30 mg, 0.04 mmol) in dichloromethane (2 mL), reacted at room temperature for 1 hour. The reaction solution was directly concentrated to obtain a crude procuct of (6a*R*)-4-chloro-3-(2-fluoro-6-hydroxyphenyl)-1-((3*S*,S*R*)-3,4,5-trimethylpiperazin-1-yl)-6,6a,7,8,9,10-hexahydro-12*H*-pyrazino[2,1-*c*]pyrido[3,4-*f*][1,4]oxazepin-12-one (25 mg). ES-API: [M+H]⁺=590.3.

Step 3: The above crude procuct of (6a*R*)-4-chloro-1-((2*R*,6*R*)-2,6-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (25 mg, 0.04 mmol) was dissolved in dichloromethane (2 mL), and *N*,*N*-diisopropylethylamine (16 mg, 0.12 mmol) was added thereto. The reaction solution was cooled to 0°C, and acryloyl chloride (3 mg, 0.04 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6aR)-8-acryloyl-4-chloro-3-(2-fluoro-6-hydroxyphenyl)-1-((3S,SR)-3,4,5-trimethylpiperazin-1-yl)-6,6a,7,8,9,10-hexahydro-12*H*-pyrazino[2,1-c]pyrido[3,4-*f*][1,4]oxazepin-12-one (Z246, 12 mg, yield: 55%). ES-API: [M+H]⁺=544.2. 1H NMR (500 MHz, DMSO-*d*₆) δ 9.98 (d, *J* = 8.9 Hz, 1H), 7.29-7.20 (m, 1H), 6.84 - 6.64 (m, 3H), 6.16 (s, 1H), 5.74 (s, 1H), 4.54 - 3.41 (m, 12H), 2.64 (s, 2H), 2.16 (s, 4H), 1.07 (d, *J* = 79.7 Hz, 6H).

### Embodiment 62-9 Synthesis of Z308

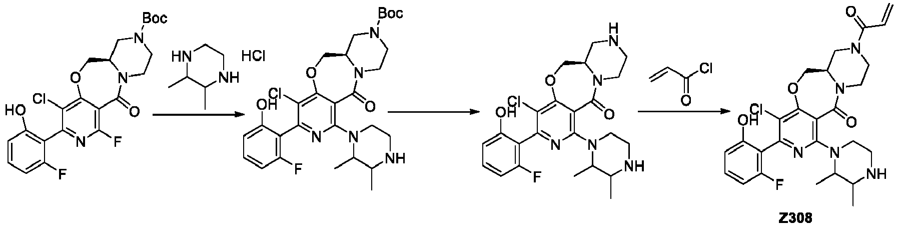

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (48 mg, 0.1 mmol), 2,3-dimethylpiperazine hydrochloride (15 mg, 0.1 mmol), *N*,*N-*diisopropylethylamine (40 mg, 0.3 mmol) and acetonitrile (2 mL) were added to a 50 mL round bottom flask, reacted at 80°C for 2 hours. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain *tert*-butyl (6a*R*)-4-chloro-1-(2,3-dimethylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (41 mg, yield of 61%). ES-API: [M+H]⁺=676.3.

Step 2: Trifluoroacetic acid (1 mL) was added to a solution of tert-butyl (6a*R*)-4-chloro-1-(2,3-dimethylpiperazin-1-yl)-3-(2-fluoro-6-hydroxyphenyl)-12-oxo-6a,7,9,10-tetrahydro-12*H*-pyrazino [2,1-*c*]pyrido[3,4-*f*][1,4]oxazepine-8(6*H*)-carboxylate (41 mg, 0.06 mmol) in dichloromethane (2 mL), reacted at room temperature for 1 hour. The reaction solution was directly concentrated to obtain a crude product of (6a*R*)-4-chloro-1-(2,3-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (35 mg). ES-API: [M+H]⁺=576.3.

Step 3: The above crude procuct of (6a*R*)-4-chloro-1-(2,3-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (35 mg, 0.04 mmol) was dissolved in dichloromethane (2 mL), and *N*,*N-*diisopropylethylamine (16 mg, 0.12 mmol) was added thereto. The reaction solution was cooled to 0°C, and acryloyl chloride (3 mg, 0.04 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,3-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z308, 3.2 mg, yield: 15%). ES-API: [M+H]⁺=530.2.

### Embodiment 62-10 Synthesis of Z308b

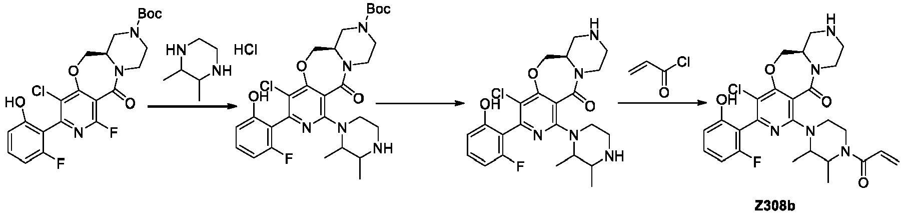

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (48 mg, 0.1 mmol), 2,3-dimethylpiperazine hydrochloride (15 mg, 0.1 mmol), *N*,*N-*diisopropylethylamine (40 mg, 0.3 mmol) and acetonitrile (2 mL) were added to a 50 mL round bottom flask, reacted at 80°C for 2 hours. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain *tert*-butyl (6a*R*)-4-chloro-1-(2,3-dimethylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (41 mg, yield of 61%). ES-API: [M+H]⁺=676.3.

Step 2: Trifluoroacetic acid (1 mL) was added to a solution of *tert*-butyl (6a*R*)-4-chloro-1-(2,3-dimethylpiperazin-1-yl)-3-(2-fluoro-6-hydroxyphenyl)-12-oxo-6a,7,9,10-tetrahydro-12*H*-pyrazino [2,1-*c*]pyrido[3,4-*f*][1,4]oxazepine-8(6*H*)-carboxylate (41 mg, 0.06 mmol) in dichloromethane (2 mL), reacted at room temperature for 1 hour. The reaction solution was directly concentrated to obtain a crude product of (6a*R*)-4-chloro-1-(2,3-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (35 mg). ES-API: [M+H]⁺=576.3.

Step 3: The above crude procuct of (6a*R*)-4-chloro-1-(2,3-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (35 mg, 0.04 mmol) was dissolved in dichloromethane (2 mL), and *N*,*N-*diisopropylethylamine (16 mg, 0.12 mmol) was added thereto. The reaction solution was cooled to 0°C, and acryloyl chloride (3 mg, 0.04 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-1-(4-acryloyl-2,3-dimethylpiperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z308b, 1.8 mg, yield: 8%). ES-API: [M+H]⁺=530.2.

### Embodiment 62-11 Synthesis of Z235

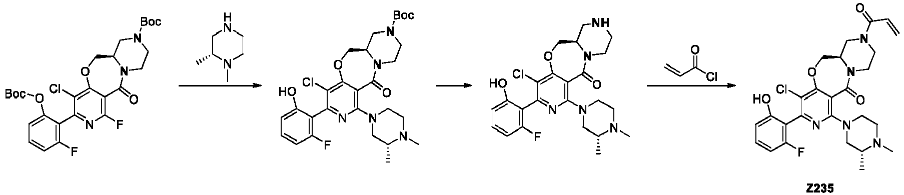

Step 1: A solution of compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (50 mg, 0.086 mmol), (*R*)-1,2-dimethylpiperazine (20 mg, 0.172 mmol) and potassium carbonate (36 mg, 0.258 mmol) in acetonitrile (1 mL) was stirred at 90°C for 2 hours. The reaction solution was filtered, concentrated, and then purified by flash silica gel column (ethyl acetate:petroleum ether: 0-80%) to obtain *tert*-butyl (6a*R*)-4-chloro-1-((*R*)-3,4-dimethylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (40 mg). ES-API: [M+H]⁺=576.2.

Step 2: Under an ice bath, a solution of *tert*-butyl (6a*R*)-4-chloro-1-((*R*)-3,4-dimethylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol) in dichloromethane (2 mL) was added with trifluoroacetic acid (0.5 mL), and the reaction solution was stirred at room temperature for 1 hour. The reaction solution was concentrated under reduced pressure to obtain a crude product of (6a*R*)-4-chloro-1-((*R*)-3,4-dimethylpiperazin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one (33 mg). ES-API: [M+H]⁺=476.2.

Step 3: The above crude procuct of (6a*R*)-4-chloro-1-((*R*)-3,4-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (33 mg, 0.07 mmol) was dissolved in dichloromethane (2 mL), and *N*,*N*-diisopropylethylamine (45 mg, 0.35 mmol) was added thereto. The reaction solution was cooled to 0°C, and acryloyl chloride (6 mg, 0.07 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-((R)-3,4-dimethylpiperazin-1-yl)-3-(2-fluoro-6-hydroxyphenyl)-6,6a,7, 8,9,10-hexahydro-12*H*-pyrazino [2,1-*c*]pyrido[3,4-*f*][1,4]oxazepin-12-one (Z235, 19 mg, yield: 42%). ES-API: [M+H]⁺ = 530.2. ¹H NMR (500 MHz, DMSO-*d*₆) δ 10.01 - 9.95 (m, 1H), 7.30 - 7.21 (m, 1H), 6.83 - 6.66 (m, 3H), 6.24 - 6.12 (m, 1H), 5.81 - 5.69 (m, 1H), 4.49 - 4.28 (m, 2H), 4.26 - 3.73 (m, 6H), 3.69 - 3.46 (m, 2H), 3.44 - 3.29 (m, 2H), 2.94 (t, *J* = 11.6 Hz, 1H), 2.74 - 2.60 (m, 2H), 2.18 (s, 3H), 1.97 (s, 1H), 0.94 (s, 3H).

### Embodiment 62-12 Synthesis of Z239

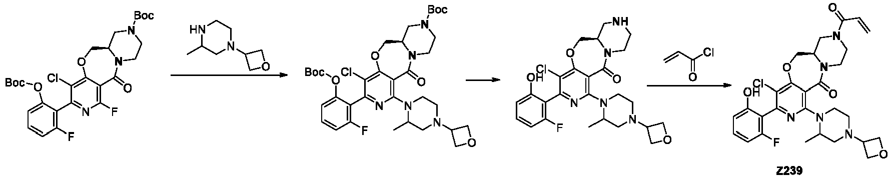

Step 1: A solution of compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H-*carboxylate (50 mg, 0.086 mmol), 3-methyl-1-(oxetan-3-yl)piperazine (27 mg, 0.172 mmol) and potassium carbonate (36 mg, 0.258 mmol) in acetonitrile (1 mL) was stirred at 90°C for 2 hours. The reaction solution was filtered, concentrated, and then purified by flash silica gel column (ethyl acetate:petroleum ether: 0-80%) to obtain *tert*-butyl (6a*R*)-3-(2-((*tert*-butoxycarbonyl)oxy)-6-fluorophenyl)-4-chloro-1-(2-methyl-4-(oxetan-3-yl)piperazin-1-yl)-12-oxo-6a,7,9,10-tetrahydro-12*H*-pyrazino[2,1-*c*]pyrido[3,4-*f*][1,4]oxazepine-8(6*H*)-carboxylate (40 mg). ES-API: [M+H]⁺=718.3.

Step 2: Under an ice bath, a solution of *tert*-butyl (6a*R*)-3-(2-((*tert*-butoxycarbonyl)oxy)-6-fluorophenyl)-4-chloro-1-(2-methyl-4-(oxetan-3-yl)piperazin-1-yl)-12-oxo-6a,7,9,10-tetrahydro-12*H-*pyrazino[2,1-*c*]pyrido[3,4-*f*][1,4]oxazepine-8(6*H*)-carboxylate (40 mg, 0.056 mmol) in dichloromethane (2 mL) was added with trifluoroacetic acid (0.5 mL), and the reaction solution was stirred at room temperature for 1 hour. The mixture was concentrated under reduced pressure to obtain a crude product of (6a*R*)-4-chloro-3-(2-fluoro-6-hydroxyphenyl)-1-(2-methyl-4-(oxetan-3-yl)piperazin-1-yl)-6,6a,7,8,9,10-hexahydro-12*H*-pyrazino[2,1-*c*]pyrido[3,4-*f*][1,4]oxazepin-12-one (29 mg). ES-API: [M+H]⁺=518.2.

Step 3: The above crude procuct of (6a*R*)-4-chloro-3-(2-fluoro-6-hydroxyphenyl)-1-(2-methyl-4-(oxetan-3-yl)piperazin-1-yl)-6,6a,7,8,9,10-hexahydro-12*H*-pyrazino[2,1-*c*]pyrido[3,4-*f*][1,4]oxazepin-12-one (29 mg, 0.056 mmol) was dissolved in dichloromethane (2 mL), and *N*,*N*-diisopropylethylamine (36 mg, 0.28 mmol) was added thereto. The reaction solution was cooled to 0°C, and acryloyl chloride (5 mg, 0.056 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(2-methyl-4-(oxetan-3 -yl)piperazin-1 -yl)-6,6a,7,8,9,10-hexahydro-12*H*-pyrazino [2,1-*c*]pyrido[3,4-*f*][1,4]oxazepin-12-one (Z239, 16.5 mg, yield: 50%). ES-API: [M+H]⁺ = 572.2. ¹H NMR (500 MHz, DMSO-*d*₆) δ 10.05 - 9.92 (m, 1H), 7.30 - 7.20 (m, 1H), 6.83 - 6.64 (m, 3H), 6.17 (d, *J* = 17.0 Hz, 1H), 5.81 - 5.69 (m, 1H), 4.65 - 4.50 (m, 3H), 4.49 - 4.30 (m, 4H), 4.25 - 3.95 (m, 4H), 3.92 - 3.71 (m, 1H), 3.69 - 3.39 (m, 3H), 3.26 - 3.06 (m, 2H), 2.74 - 2.56 (m, 2H), 2.04 - 1.73 (m, 2H), 1.29 - 1.09 (m, 3H).

### Embodiment 62-13 Synthesis of Z110

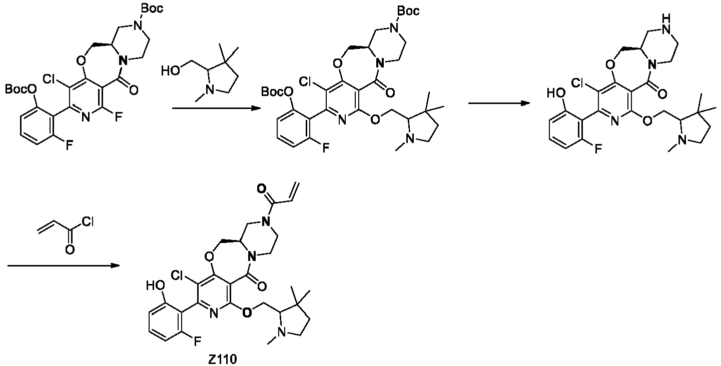

Step 1: Sodium hydride (20 mg, 60wt%, 0.52 mmol) was added to (1,3,3-trimethyltetrahydropyrrol-2-yl)methanol (37 mg, 0.26 mmol) dissolved in 1 mL of dry tetrahydrofuran under an ice bath. After stirring for 10 minutes, a solution of *tert*-butyl (6a*R*)-3-(2-((*tert*-butoxycarbonyl)oxy)-6-fluorophenyl-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.172 mmol) in dry tetrahydrofuran (1 mL) was slowly added dropwise thereto. The reaction was carried out at 0°C for 1 hour. After the reaction was completed, saturated ammonium chloride solution was added thereto to quench the reaction. The reaction solution was extracted twice with dichloromethane/isopropanol (4: 1) and dried over anhydrous sodium sulfate. The organic phase was concentrated to obtain *tert*-butyl (6a*R*)-3-(2-((*tert*-butoxycarbonyl)oxy)-6-fluorophenyl)-4-chloro-12-oxo-1-((1,3,3 -trimethyltetrahydropyrrol-2-yl)methoxy)-6a,7,9,10-tetrahydro-12*H-*pyrazino[2,1-*c*]pyrido[3,4-*c*][1,4-*f*]oxazepine-8(6*H*)-carboxylate (crude product), which was directly used in the next reaction step. ES-API: [M+H]⁺=705.1.

Step 2: *tert*-Butyl (6a*R*)-3-(2-((*tert*-butoxycarbonyl)oxy)-6-fluorophenyl)-4-chloro-12-oxo-1-((1,3,3-trimethyltetrahydropyrrol-2-yl)methoxy)-6a,7,9,10-tetrahydro-12*H*-pyrazino[2,1-*c*]pyrido[3,4-*f*][1,4]oxazepine-8(6*H*)-carboxylate (crude product), 0.75 mL of dichloromethane and 0.25 mL of trifluoroacetic acid were added to a round bottom flask. The mixture was stirred at room temperature for 2 hours, and the completion of reaction was detected by LC-MS. The reaction solution was concentrated to obtain (6a*R*)-4-chloro-1-((1,3,3-trimethyltetrahydropyrrol-2-yl)methoxy)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (crude product) as a brown oily liquid. The crude product was directly used in the next step. ES-API: [M+H]⁺=505.2.

Step 3: (6a*R*)-4-Chloro-1-((1,3,3-trimethyltetrahydropyrrol-2-yl)methoxy)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (crude product), 2 mL of dichloromethane and triethylamine (0.24 mL, 1.72 mmol) were added to a round bottom flask. The reaction was cooled to 0°C, and a solution of acrylic anhydride in dichloromethane (12 mg, 0.13 mmol, 1 mL) was added to the reaction solution. The reaction was stirred at 0°C for 20 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-1-((1,3,3-trimethyltetrahydropyrrol-2-yl)methoxy)-3-(2-fluoro-6-hydroxyphenyl)-6,6a,7,8,9,10-hexahydro-12*H-*pyrazino [2,1-*c*]pyrido [3,4-*f*][1,4]oxazepin-12-one (Z110, 7 mg, 3-step yield of 7.3%). ¹H NMR (500 MHz, DMSO-*d*₆) δ 10.24-9.93 (m, 1H), 7.35-7.25 (m, 1H), 6.85-6.67 (m, 3H), 6.22-6.12 (m, 1H), 5.78-5.68 (m, 1H), 4.56-4.41 (m, 1H), 4.41-4.08 (m, 5H), 4.07-3.78 (m, 2H), 3.78-3.52 (m,3H), 3.00-2.87 (m, 1H), 2.38-2.17 (m, 5H), 1.57-1.41 (m, 2H), 1.00 (s, 3H), 0.94-0.86 (m, 3H). ES-API: [M+H]⁺=559.2.

### Embodiment 62-14 Synthesis of Z261

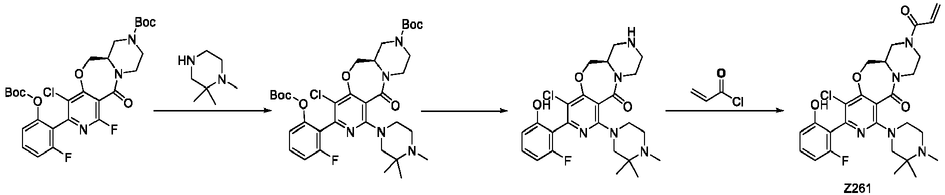

Step 1: A solution of *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (intermediate 1a, 50 mg, 0.085 mmol), 1,2,2-trimethylpiperazine (15.3 mg, 0.12 mmol) and *N*-ethyl-*N-*isopropylpropan-2-amine (33.2 mg, 0.25 mmol) in acetonitrile (3 mL) was added to a 100 mL round bottom flask at room temperature. After the reaction was completed, the mixture was directly concentrated to obtain a crude product of *tert*-butyl (6a*R*)-3-(2-((*tert*-butoxycarbonyl)oxy)-6-fluorophenyl)-4-chloro-12-oxo-1-(3,3,4-trimethylpiperazin-1-yl)-6a,7,9,10-tetrahydro-12*H*-pyrazino[2,1-*c*]pyrido [3,4-*f*][1,4]oxazepine-8(6*H*)-carboxylate (72 mg). ES-API: [M+H]⁺=690.3.

Step 2: A solid of *tert*-butyl (6a*R*)-3-(2-((*tert*-butoxycarbonyl)oxy)-6-fluorophenyl)-4-chloro-12-oxo-1-(3,3,4-trimethylpiperazin-1-yl)-6a,7,9,10-tetrahydro-12*H*-pyrazino [2,1-*c*]pyrido [3,4-*f*][1,4]oxazepine-8(6*H*)-carboxylate (72 mg, 0.10 mmol), dichloromethane (3 mL) and trifluoroacetic acid (2 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-3-(2-fluoro-6-hydroxyphenyl)-1-(3,3,4-trimethylpiperazin-1-yl)-6,6a,7,8,9,10-hexahydro-12*H*-pyrazino[2,1-*c*]pyrido[3,4*f*][1,4]oxazepin-12-one (70 mg). ES-API: [M+H]⁺=490.2.

Step 3: The crude product of (6a*R*)-4-chloro-3-(2-fluoro-6-hydroxyphenyl)-1-(3,3,4-trimethylpiperazin-1-yl)-6,6a,7,8,9,10-hexahydro-12*H*-pyrazino[2,1-*c*]pyrido[3,4-*f*][1,4]oxazepin-12-one (70 mg), dichloromethane (5 mL) and *N*-ethyl-*N*-isopropylpropan-2-amine (128.5 mg, 0.99 mmol) were added to a 100 mL eggplant-shaped flask. The reaction was cooled to 0°C, and a solution of acrylic anhydride in dichloromethane (24.3 mg, 0.27 mmol, 0.5 mL) was added dropwise to the reaction solution, stirred at 0°C for 10 minutes, and then the mixture was added with 20 mL of saturated aqueous sodium bicarbonate, extracted three times with 10 mL of dichloromethane. The organic phase was dried, concentrated, and purified by basic preparative HPLC (column: Ultimate XB-C18, 50*250 mm, 10 µm; mobile phase system: A: purified water (0.1% NH₄HCO₃); B: pure acetonitrile (0.1% NH₄HCO₃); flow rate : 80 mL/min; gradient: B/A=20%-100%; column temperature: room temperature) to obtain a yellow solid of (6a*R*)-8-acryloyl-4-chloro-3-(2-fluoro-6-hydroxyphenyl)-1-(3,3,4-trimethylpiperazin-1-yl)-6,6a,7,8,9,10-hexahydro-12*H*-pyrazino[2,1-*c*]pyrido[3,4--*f*][1,4]oxazepin-12-one (Z261, 3.7 mg, 8.0%). ¹H NMR (500 MHz, CDCl₃) δ 7.30 - 7.19 (m, 1H), 6.82 (d, *J* = 8.3 Hz, 1H), 6.68 (t, *J* = 9.0 Hz, 1H), 6.63 - 6.44 (m, 1H), 6.40 (d, *J* = 16.4 Hz, 1H), 5.81 (d, *J* = 10.0 Hz, 1H), 4.46 - 3.93 (m, 6H), 3.85 - 3.48 (m, 4H), 3.41 - 3.12 (m, 3H), 2.69 (dd, *J* = 9.7, 6.2 Hz, 1H), 2.56 - 2.48 (m, 1H), 2.25 (s, 3H), 1.08 (s, 3H), 1.01 (s, 3H). ES-API: [M+H]⁺=544.2.

### Embodiment 62-15 Synthesis of Z257

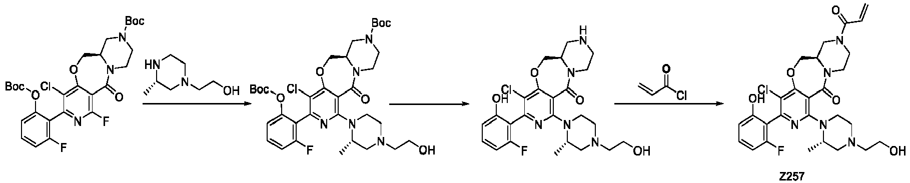

Step 1: A solution of *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.17 mmol), (*S*)-2-(3-methylpiperazin-1-yl)ethan-1-ol (36 mg, 0.25 mmol) and *N*-ethyl-*N-*isopropylpropan-2-amine (65.8 mg, 0.51 mmol) in acetonitrile (3 mL) were added to a 100 mL round bottom flask at room temperature. After the reaction was completed, the mixture was directly concentrated to obtain a crude product of *tert*-butyl (6a*R*)-3-(2-((*tert*-butoxycarbonyl)oxy)-6-fluorophenyl)-4-chloro-1-((*S*)-4-(2-hydroxyethyl)-2-methylpiperazin-1 -yl)-12 -oxo-6a,7,9,10-tetrahydro-12*H*-pyrazino[2,1-*c*]pyrido [3,4-*f*][1,4]oxazepine-8(6*H*)-carboxylate (120 mg). ES-API: [M+H]⁺=706.3.

Step 2: A solid of *tert*-butyl (6a*R*)-3-(2-((*tert*-butoxycarbonyl)oxy)-6-fluorophenyl)-4-chloro-1-((*S*)-4-(2-hydroxyethyl)-2-methylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg), dichloromethane (3 mL) and trifluoroacetic acid (2 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-3-(2-fluoro-6-hydroxyphenyl)-1-((*S*)-4-(2-hydroxyethyl)-2-methylpiperazin-1-yl)-6,6a,7,8,9,10-hexahydro-12*H*-pyrazino[2,1-*c*]pyrido[3,4-*f*][1,4]oxazepin-12-one (100 mg). ES-API: [M+H]⁺=506.2.

Step 3: A crude product of (6a*R*)-4-chloro-3-(2-fluoro-6-hydroxyphenyl)-1-((*S*)-4-(2-hydroxyethyl)-2-methylpiperazin-1 -yl)-6,6a,7,8,9,10-hexahydro-12*H*-pyrazino[2,1-*c*]pyrido [3,4-*f*][1,4]oxazepin-12-one (100 mg), dichloromethane (5 mL) and *N*-ethyl-*N*-isopropylpropan-2-amine (128.5 mg, 0.99 mmol) were added to a 100 mL eggplant-shaped flask. The reaction was cooled to 0°C, and a solution of acryloyl chloride in dichloromethane (17.1 mg, 0.19 mmol, 0.5 mL) was added dropwise to the reaction solution, stirred at 0°C for 10 minutes, and then the mixture was added with 20 mL of saturated aqueous sodium bicarbonate solution, extracted three times with 10 mL of dichloromethane. The organic phase was dried and concentrated, and the crude product was purified by basic preparative HPLC to obtain (6a*R*)-8-acryloyl-4-chloro-3-(2-fluoro-6-hydroxyphenyl)-1-((*S*)-4-(2-hydroxyethyl)-2-methylpiperazin-1-yl)-6,6a,7,8,9,10-hexahydro-12*H*-pyrazino[2,1-*c*]pyrido[3,4-*f*][1,4]oxazepin-12-one (Z257, 15 mg, 15.7%) as a yellow solid. ES-API: [M+H]⁺=560.2. ¹HNMR (500MHz, DMSO-*d*₆) *δ* 10.04-9.90(m,1H), 7.26-7.22(m,1H), 6.78-6.68 (m,3H), 6.20-6.15(m,1H), 5.80-5.70(m,1H), 4.50-4.25(m,3H), 4.20-3.82 (m,5H), 3.70-3.40(m,5H), 3.33-3.3.25 (m,1H), 3.22-3.13(m,1H), 2.80-2.55(m,2H), 2.40-2.27(m, 2H), 2.20-2.02(s,2H), 1.30-1.18(m,3H).

### Embodiment 62-16 Synthesis of Z308a

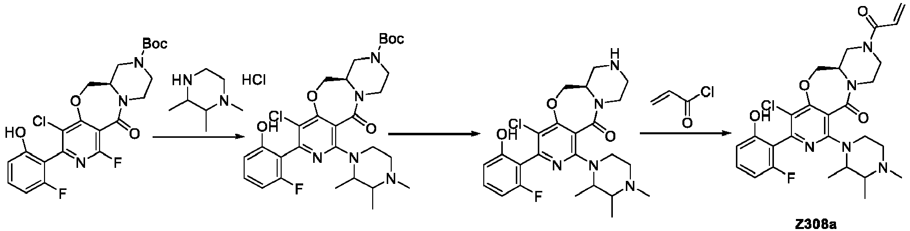

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (48 mg, 0.1 mmol), 1,2,3-trimethylpiperazine hydrochloride (15 mg, 0.1 mmol), *N*,*N-*diisopropylethylamine (40 mg, 0.3 mmol) and acetonitrile (2 mL) were added to a 50 mL round bottom flask, reacted at 80°C for 2 hours. The reaction solution was added with ethyl acetate (10 mL), washed with water (10 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-12-oxo-1-(2,3,4-trimethylpiperazin-1-yl)-6a,7,9,10-tetrahydro-12*H*-pyrazino[2,1-*c*]pyrido[3,4-*f*][1,4]oxazepine-8(6*H*)-carboxylate (30 mg, yield of 50%). ES-API: [M+H]⁺=590.2.

Step 2: Trifluoroacetic acid (1 mL) was added to a solution of *tert*-butyl (6a*R*)-4-chloro-3-(2-fluoro-6-hydroxyphenyl)-12-oxo-1-(2,3,4-trimethylpiperazin-1-yl)-6a,7,9,10-tetrahydro-12*H*-pyrazino[2,1-*c*]pyrido[3,4-*f*][1,4]oxazepine-8(6*H*)-carboxylate (30 mg, 0.05 mmol) in dichloromethane (2 mL), reacted at room temperature for 1 hour. The reaction solution was directly concentrated to obtain a crude procuct of (6a*R*)-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one (24 mg). ES-API: [M+H]⁺=490.2.

Step 3: The above crude product of (6a*R*)-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one (24 mg, 0.05 mmol) was dissolved in dichloromethane (2 mL), and *N*,*N*-diisopropylethylamine (20 mg, 0.15 mmol) was added thereto. The reaction solution was cooled to 0°C, and acryloyl chloride (3 mg, 0.04 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one (Z308a, 2 mg, yield: 7%). ES-API: [M+H]⁺=544.2.

### Embodiment 62-17 Synthesis of Z308c

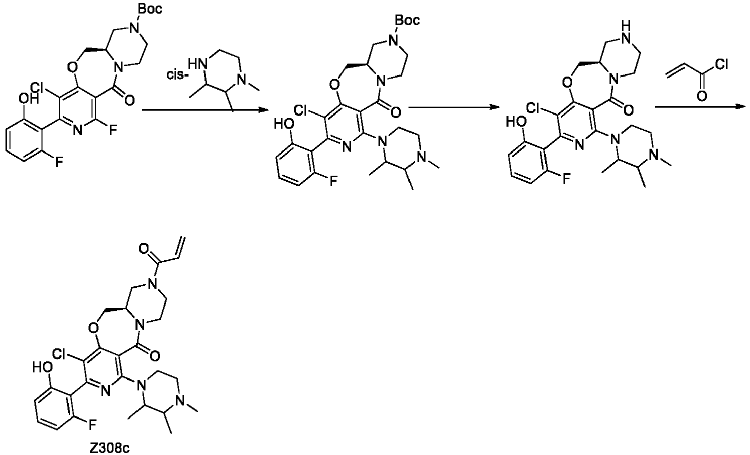

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (330 mg, 0.68 mmol), *cis-*1,2,3-trimethylpiperazine (110 mg, 0.68 mmol), *N*,*N-*diisopropylethylamine (260 mg, 1.87 mmol) and acetonitrile (10 mL) were added to a 50 mL round bottom flask, reacted at 85°C for 16 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-12-oxo-1-(*cis*-2,3,4-trimethylpiperazin-1-yl)-6a,7,9,10-tetrahydro-12*H*-pyrazino[2,1-*c*]pyrido[3,4-*f*][1,4]oxazepine-8(6*H*)-carboxylate (120 mg, yield of 30%). ES-API: [M+H]⁺=590.2.

Step 2: Trifluoroacetic acid (2 mL) was added to a solution of *tert*-butyl (6a*R*)-4-chloro-3-(2-fluoro-6-hydroxyphenyl)-12-oxo-1-(*cis*-2,3,4-trimethylpiperazin-1-yl)-6a,7,9,10-tetrahydro-12*H-*pyrazino[2,1-*c*]pyrido[3,4-*f*][1,4]oxazepine-8(6*H*)-carboxylate (120 mg, 0.20 mmol) in dichloromethane (5 mL), reacted at room temperature for 1 hour. The reaction solution was directly concentrated to obtain a crude procuct of (6aR)-4-chloro-3-(2-fluoro-6-hydroxyphenyl)-1-(*cis*-2,3,4-trimethylpiperazin-1-yl)-6,6a,7,8,9,10-hexahydro-12*H*-pyrazino[2,1-*c*]pyrido[3,4-*f*][1,4]oxazepin-12-one (100 mg). ES-API: [M+H]⁺=490.2.

Step 3: The above crude product of (6a*R*)-4-chloro-3-(2-fluoro-6-hydroxyphenyl)-1-(cis-2,3,4-trimethylpiperazin-1-yl)-6,6a,7,8,9,10-hexahydro-12*H*-pyrazino[2,1-*c*]pyrido[3,4-*f*][1,4]oxazepin-12-one (100 mg, 0.20 mmol) was dissolved in dichloromethane (5 mL), and *N*,*N*-diisopropylethylamine (77 mg, 0.6 mmol) was added thereto. The reaction solution was cooled to 0°C, and acryloyl chloride (16 mg, 0.18 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one (Z308c, 80 mg, yield: 73%). ES-API: [M+H]⁺=544.2.

### Embodiment 62-18 Synthesis of Z254

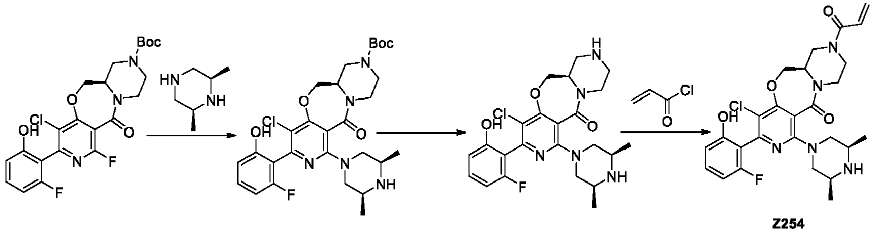

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (96 mg, 0.2 mmol), (2S,6R)-2,6-dimethylpiperazine (23 mg, 0.2 mmol), *N*,*N*-diisopropylethylamine (80 mg, 0.6 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-1-((3*S*,5*R*)-3,5-dimethylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (60 mg, yield of 50%). ES-API: [M+H]⁺=576.2.

Step 2: Trifluoroacetic acid (1 mL) was added to a solution of *tert*-butyl (6a*R*)-4-chloro-1-((3*S*,5*R*)-3,5-dimethylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (60 mg, 0.1 mmol) in dichloromethane (2 mL), reacted at room temperature for 1 hour. The reaction solution was directly concentrated to obtain a crude product of (6a*R*)-4-chloro-1-((3*S*,S*R*)-3,5-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (50 mg). ES-API: [M+H]⁺=476.2.

Step 3: The above (6a*R*)-4-chloro-1-((3*S*,S*R*)-3,5-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (50 mg, 0.1 mmol) was dissolved in dichloromethane (2 mL), and *N*,*N*-diisopropylethylamine (40 mg, 0.3 mmol) was added thereto. The reaction solution was cooled to 0°C, and acryloyl chloride (6 mg, 0.08 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-((3*S*,5*R*)-3,5-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z254, 22.7 mg, yield: 43%). ES-API: [M+H]⁺=530.2.

### Embodiment 62-19 Synthesis of Z99

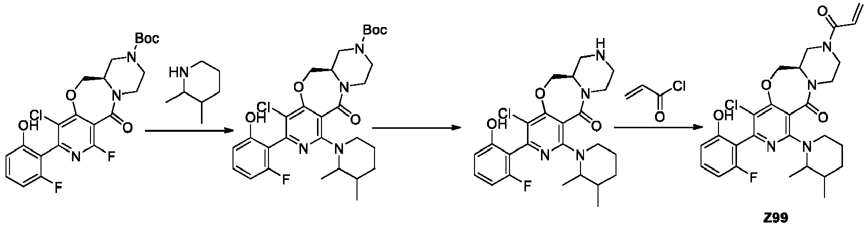

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (75 mg, 0.15 mmol), 2,3-dimethylpiperidine (23 mg, 0.2 mmol), *N*,*N*-diisopropylethylamine (58 mg, 0.45 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-1-(2,3-dimethylpiperidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (80 mg, yield of 92%). ES-API: [M+H]⁺=575.2.

Step 2: Trifluoroacetic acid (1 mL) was added to a solution of *tert*-butyl (6a*R*)-4-chloro-1-(2,3-dimethylpiperidin-1-yl)-3-(2-fluoro-6-hydroxyphenyl)-12-oxo-6a,7,9,10-tetrahydro-12*H*-pyrazino [2,1-*c*]pyrido[3,4-*f*][1,4]oxazepine-8(6*H*)-carboxylate (80 mg, 0.14 mmol) in dichloromethane (2 mL), reacted at room temperature for 1 hour. The reaction solution was directly concentrated to obtain a crude product of (6a*R*)-4-chloro-1-(2,3-dimethylpiperidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (60 mg). ES-API: [M+H]⁺=475.2.

Step 3: The above crude procuct of (6a*R*)-4-chloro-1-(2,3-dimethylpiperidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (60 mg, 0.13 mmol) was dissolved in dichloromethane (2 mL), and *N*,*N-*diisopropylethylamine (51 mg, 0.39 mmol) was added thereto. The reaction solution was cooled to 0°C, and acryloyl chloride (9 mg, 0.1 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,3-dimethylpiperidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z99, 22.7 mg, yield: 33%). ES-API: [M+H]⁺=529.2.

### Embodiment 62-20 Synthesis of Z102a

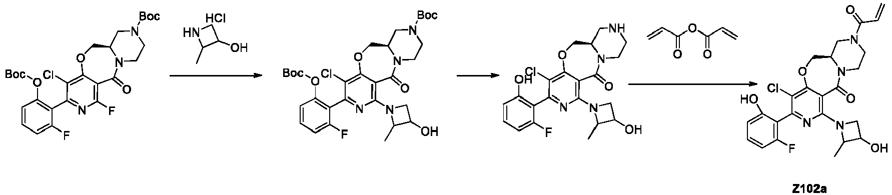

Step 1: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (300 mg, 0.515 mmol), 2-methylazetidin-3-ol hydrochloride (65 mg, 0.515 mmol) and potassium carbonate (250 mg, 1.8 mmol) were dissolved in acetonitrile (4 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert*-butoxycarbonyl)oxy)-6-fluorophenyl)-4-chloro-1-(3-hydroxy-2-methylazetidin-1-yl)-12-oxo-6a,7,9,10-tetrahydro-12*H-*pyrazino[2,1-*c*]pyrido[3,4-*f*][1,4]oxazepine-8(6*H*)-carboxylate (340 mg, yield: 100%). ES-API: [M+H]⁺=649.1.

Step 2: *tert*-Butyl (6a*R*)-3-(2-((*tert*-butoxycarbonyl)oxy)-6-fluorophenyl)-4-chloro-1-(3-hydroxy-2-methylazetidin-1-yl)-12-oxo-6a,7,9,10-tetrahydro-12*H*-pyrazino[2,1-*c*]pyrido[3,4-*f*][1,4]oxazepine-8(6*H*)-carboxylate (340 mg, 0.525 mmol) was dissolved in dichloromethane (4 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-3-(2-fluoro-6-hydroxyphenyl)-1-(3-hydroxy-2-methylazetidin-1-yl)-6,6a,7,8,9,10-hexahydro-12*H*-pyrazino[2,1-*c*]pyrido[3,4-*f*][1,4]oxazepin-12-one (300 mg, 100%). ES-API: [M+H]⁺=449.

Step 3: (6a*R*)-4-Chloro-3-(2-fluoro-6-hydroxyphenyl)-1-(3-hydroxy-2-methylazetidin-1-yl)-6,6a,7,8,9,10-hexahydro-12*H*-pyrazino[2,1-*c*]pyrido[3,4-*f*][1,4]oxazepin-12-one (300 mg, 0.46 mmol) was dissolved in dichloromethane (4 mL), then cooled to 0°C in an ice-water bath, and triethylamine (2 mL) was added dropwise thereto. The pH value was basic, and acrylic anhydride (67 mg, 0.54 mmol) was added dropwise thereto, stirred for ten minutes. After the reaction was completed, the mixture was extracted with ethyl acetate, washed with water, washed with brine, dried over sodium sulfate, evaporated to dryness by rotary evaporation and then purified by preparative chromatographic column: Ultimate XB-C18, 50*250 mm, 10 µm, mobile phase: acetonitrile/water= 10/90-90/10, 40 min) to obtain (6a*R*)-8-acryloyl-4-chloro-3-(2-fluoro-6-hydroxyphenyl)-1-(3-hydroxy-2-methylazetidin-1-yl)-6,6a,7,8,9,10-hexahydro-12*H-*pyrazino[2,1-*c*]pyrido[3,4-*f*][1,4]oxazepin-12-one (Z102a, 74 mg, yield: 21.9%). ¹H NMR (400 MHz, CDCl₃) δ 7.32 - 7.27 (m, 1H), 6.82 (d, *J* = 8.3 Hz, 1H), 6.70 (t, *J* = 9.1 Hz, 1H), 6.59 - 6.36 (m, 2H), 5.82 (d, *J* = 10.3 Hz, 1H), 4.74 - 4.47 (m, 2H), 4.26 (s, 4H), 4.12 (s, 2H), 4.08 3.92 (m, 2H), 3.87 - 3.71 (m, 2H), 3.60 (s, 1H), 1.50 -1.43 (m, 2H), 1.32 (d, *J* = 5.4 Hz, 1H). ES-API: [M+H]⁺=503.1

### Embodiment 62-21 Synthesis of Z236

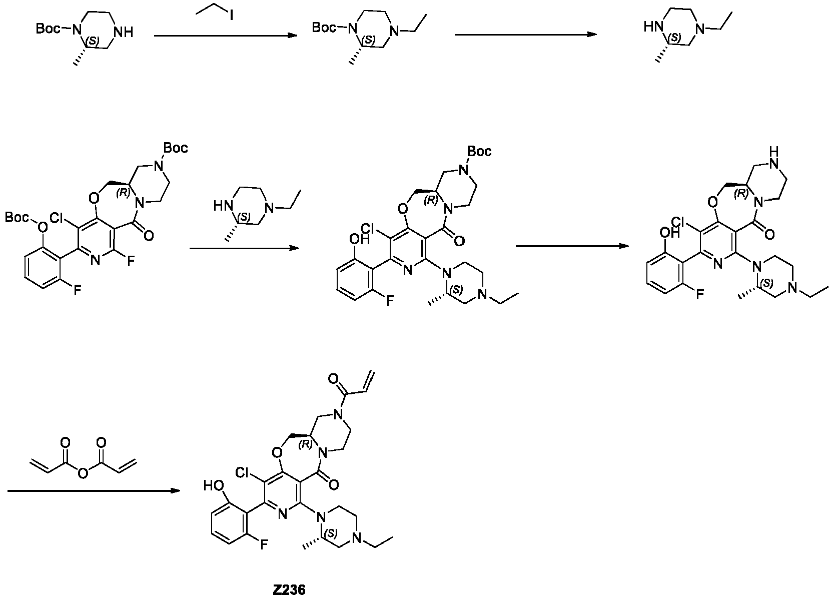

Step 1: tert-Butyl (*S*)-2-methylpiperazine-1-carboxylate (2.07 g, 0.28 mmol), iodoethane (1.93 g, 12.4 mmol) and potassium carbonate (2.85 g, 20.67 mmol) were added to acetonitrile (30 mL), heated to 60°C and reacted for 3 hours. (Thin-layer chromatography plate, 10% MeOH in DCM, R_{f}=0.7). After the reaction was completed, the mixture was filtered and evaporated to dryness by rotary evaporation to obtain a product (2.2 g, yield of 100%) ¹H NMR (400 MHz, CDCl₃) δ 4.12 (p, *J* = 6.2 Hz, 1H), 3.72 (d, *J* = 13.2 Hz, 1H), 3.00 (td, *J* = 12.9, 3.3 Hz, 1H), 2.70 (d, *J* = 11.2 Hz, 1H), 2.64 - 2.54 (m, 1H), 2.27 (ddt, *J* = 31.7, 12.4, 6.4 Hz, 2H), 1.97 (dd, *J* = 11.3, 4.0 Hz, 1H), 1.83 (td, *J* = 11.8, 3.4 Hz, 1H), 1.41 - 1.35 (m, 9H), 1.15 (d, *J* = 6.7 Hz, 3H), 1.01 - 0.94 (m, 3H).

Step 2: *tert*-Butyl (*S*)-4-ethyl-2-methylpiperazine-1-carboxylate (68 mg, 0.28 mmol) was dissolved in dichloromethane (0.5 mL), and trifluoroacetic acid (0.5 mL) was added thereto, reacted at room temperature for 1 hour and then evaporated to dryness by rotary evaporation, and the crude product was directly used in the next step.

Step 3: Compound tert-butyl (6a*R*)-3-(2-((*tert*-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepin-12(6a*H*)-one-carboxylate (150 mg, 0.26 mmol), (*S*)-1-ethyl-3-methylpiperazine (36 mg, 0.28 mmol) and potassium carbonate (72 mg, 0.52 mmol) were dissolved in acetonitrile (2 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-4-chloro-1-((*S*)-4-ethyl-2-methylpiperazin-1-yl)-3-(2-fluoro-6-hydroxyphenyl)-12-oxo-6a,7,9,10-tetrahydro-6*H*-pyrazino[2,1-*c*]pyrido[3,4-*f*][1,4]oxazepine-8(12*H*)-carboxylate (180 mg, yield: 100%). ES-API: [M+H]⁺= 690.3.

Step 4: *tert*-Butyl (6a*R*)-4-chloro-1-((*S*)-4-ethyl-2-methylpiperazin-1-yl)-3-(2-fluoro-6-hydroxyphenyl)-12-oxo-6a,7,9,10-tetrahydro-6*H*-pyrazino[2,1-*c*]pyrido[3,4-*f*][1,4]oxazepine-12(6a*H*)-one-carboxylate (178 mg, 0.26 mmol) was dissolved in dichloromethane (1 mL), then trifluoroacetic acid (1 mL) was added dropwise thereto, stirred at room temperature for 1 hour. After the reaction was completed, the mixture was evaporated to dryness by rotary evaporation to obtain a crude product of (6a*R*)-4-chloro-1-((*S*)-4-ethyl-2-methylpiperazin-1-yl)-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (127 mg, 100%). (thin-layer chromatography plate, R_{f}=0.2, 15% MeOH in DCM).

Step 5: (6a*R*)-4-Chloro-1-((*S*)-4-ethyl-2-methylpiperazin-1-yl)-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (127 mg, 0.26 mmol) was dissolved in dichloromethane (2 mL), then cooled to 0°C in an ice-water bath. Triethylamine (79 mg, 0.78 mmol) was added dropwise thereto, and acrylic anhydride (26 mg, 0.21 mmol) was added dropwise thereto, stirred for ten minutes. After the reaction was completed, the mixture was extracted with ethyl acetate, washed with water, washed with brine, dried over sodium sulfate, evaporated to dryness by rotary evaporation and then purified by preparative chromatographic column: Ultimate XB-C18, 50*250 mm, 10 µm, mobile phase: acetonitrile/water= 10/90-90/10, 40 min) to obtain 30 mg of crude product. The crude product was purified (dichloromethane/methanol=10/1, R_{f}=0.4) to obtain (6a*R*)-8-acryloyl-4-chloro-3-(2-fluoro-6-hydroxyphenyl)-1-(3-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (4.5 mg, yield: 5%). ¹HNMR (400 MHz, CDCl₃) δ 7.29 (t, *J* = 8.1 Hz, 1H), 6.82 (d, *J* = 8.3 Hz, 1H), 6.69 (t, *J* = 9.0 Hz, 1H), 6.56 (m, 1H), 6.41 (d, *J* = 16.4 Hz, 1H), 5.83 (d, *J* = 10.3 Hz, 1H), 4.37 (s, 4H), 4.21 (s, 1H), 4.18 - 3.96 (m, 2H), 3.77 (s, 1H), 3.70 - 3.31 (m, 4H), 2.95 (s, 1H), 2.79 (s, 1H), 2.47 (m, 4H), 1.47 (d, *J* = 6.7 Hz, 3H), 1.17 (t, 3H). ES-API: [M+H]⁺= 544.3.

### Embodiment 62-22 Synthesis of Z221

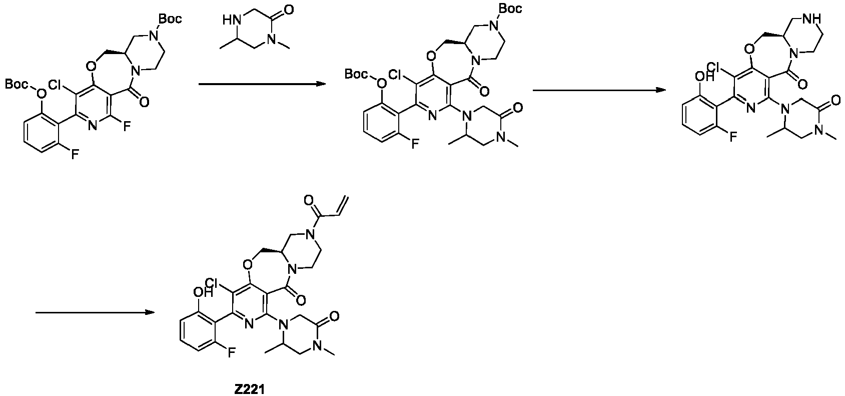

Step 1: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1*-c*]pyrido[3,4-*f*][1,4]oxazepine-8(6*H*)-carboxylate (100 mg, 0.17 mmol) was dissolved in 2 mL of acetonitrile, and 1,5-dimethylpiperazin-2-one (50 mg, 0.37 mmol) and potassium carbonate (76 mg, 0.55 mmol) were added thereto, and the reaction solution was stirred at 80°C for 2 hours. After the reaction was completed, the reaction solution was cooled to room temperature, extracted with ethyl acetate, washed with brine, and the organic phase was concentrated under reduced pressure to obtain a crude product. The crude product was purified by flash silica gel column (PE/EtOAc=1/1, R_{f}=0.2) to obtain *tert*-butyl (6a*R*)-3-(2-((*tert*-butoxycarbonyl)oxy)-6-fluorophenyl)-4-chloro-1-(2,4-dimethyl-5-oxopiperazin-1-yl)-12-oxo-6a,7,9,10-tetrahydro-6*H*-pyrazino[2,1-*c*]pyrido[3,4-*f*][1,4]oxazepine-8(12*H*)-carboxylate (95 mg, 80%, a pale yellow solid). ES-API: [M+H-Bo*c*]⁺= 590.3.

Step 2: *tert*-butyl (6a*R*)-3-(2-((tert-butoxycarbonyl)oxy)-6-fluorophenyl)-4-chloro-1-(2,4-dimethyl-5-oxopiperazin-1-yl)-12-oxo-6a,7,9,10-tetrahydro-6*H*-pyrazino[2,1-*c*]pyrido[3,4-*f*][1,4]oxazepine-8(12*H*)-carboxylate (95 mg, 0.13 mmol) was dissolved in 2 mL of dichloromethane, and 2 mL of trifluoroacetic acid was added thereto, and the reaction solution was stirred at room temperature for 2 hours. The reaction solution was directly concentrated under reduced pressure, redissolved in dichloromethane, and concentrated to obtain a crude product of (6a*R*)-4-chloro-1-(2,4-dimethyl-5-oxopiperazin-1-yl)-3 -(2-fluoro-6-hydroxyphenyl)-7, 8,9,10-tetrahydro-6*H*-pyrazino [2,1-c] pyrido [3,4-*f*][1,4]oxazepin-12(6a*H*)-one (63 mg, crude product). ES-API: [M+H]⁺= 490.2.

Step 3: (6a*R*)-4-Chloro-1-(2,4-dimethyl-5-oxopiperazin-1-yl)-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (63 mg, 0.13 mmol) was dissolved in 3 mL of dichloromethane, and then 1 mL of triethylamine was added thereto, and acrylic anhydride (13 mg, 0.10 mmol) was added dropwise thereto at 0°C. The reaction solution was stirred for 10 minutes. The reaction solution was washed with brine, extracted with dichloromethane, and the organic phase was concentrated under reduced pressure, and the crude product was purified by preparative HPLC (preparative separation conditions: chromatographic column information: Ultimate XB-C18, 50*250 mm, 10 µm, mobile phase system: A: purified water B : pure acetonitrile, flow rate: 80 mL/min, gradient: within 50 minutes, B/A=20%-90%, wavelength: 214 nm, column temperature: room temperature) to obtain (6a*R*)-8-acryloyl-4-chloro-1-(2,4-dimethyl-5-oxopiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (15 mg, 17%, white solid). ¹H NMR (400 MHz, CDCl₃) δ 7.30 (td, *J* = 7.5, 6.4, 3.5 Hz, 1H), 6.85 - 6.77 (m, 1H), 6.71 (td, *J* = 10.3, 9.9, 2.8 Hz, 1H), 6.56 (s, 1H), 6.41 (d, *J* = 16.5 Hz, 1H), 5.83 (d, *J* = 10.4 Hz, 1H), 4.61 - 3.98 (m, 7H), 4.00 - 3.41 (m, 7H), 2.99 (d, *J* = 4.2 Hz, 3H), 1.31 (d, *J* = 6.5 Hz, 3H). ES-API: [M+H]⁺= 544.2.

### Embodiment 62-23 Synthesis of Z249

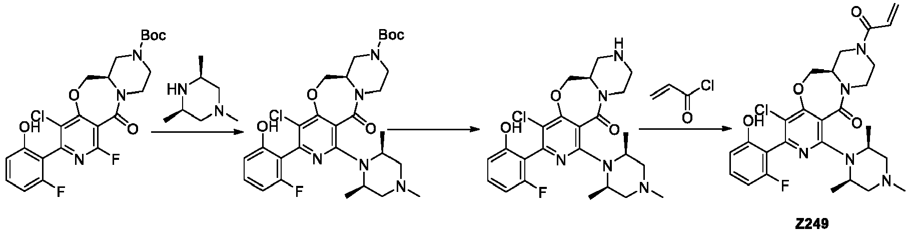

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (75 mg, 0.15 mmol), (3*R*,5*S*)-1,3,5-trimethylpiperazine (25 mg, 0.2 mmol), *N*,*N*-diisopropylethylamine (58 mg, 0.45 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-12-oxo-1-((2*R*,6*S*)-2,4,6-trimethylpiperazin-1-yl)-6a,7,9,10-tetrahydro-12*H*-pyrazino[2,1-*c*]pyrido[3,4-*f*][1,4]oxazepine-8(6*H*)-carboxylate (70 mg, yield of 79%). ES-API: [M+H]⁺=590.2.

Step 2: Trifluoroacetic acid (1 mL) was added to a solution of *tert*-butyl (6a*R*)-4-chloro-3-(2-fluoro-6-hydroxyphenyl)-12-oxo-1-((2*R*,6*S*)-2,4,6-trimethylpiperazin-1-yl)-6a,7,9,10-tetrahydro-12*H-*pyrazino[2,1-*c*]pyrido[3,4-*f*][1,4]oxazepine-8(6*H*)-carboxylate (70 mg, 0.12 mmol) in dichloromethane (2 mL), reacted at room temperature for 1 hour. The reaction solution was directly concentrated to obtain a crude procuct of (6a*R*)-4-chloro-3-(2-fluoro-6-hydroxyphenyl)-1-((2*R*,6*S*)-2,4,6-trimethylpiperazin-1-yl)-6,6a,7,8,9,10-hexahydro-12*H*-pyrazino[2,1-*c*]pyrido[3,4-*f*][1,4]oxazepin-12-one (60 mg). ES-API: [M+H]⁺=490.2.

Step 3: The above crude product of (6a*R*)-4-chloro-3-(2-fluoro-6-hydroxyphenyl)-1-((2*R*,6*S*)-2,4,6-trimethylpiperazin-1-yl)-6,6a,7,8,9,10-hexahydro-12*H*-pyrazino[2,1-*c*]pyrido[3,4-*f*][1,4]oxazepin-12-one (60 mg, 0.12 mmol) was dissolved in dichloromethane (2 mL), and *N*,*N-*diisopropylethylamine (51 mg, 0.39 mmol) was added thereto. The reaction solution was cooled to 0°C, and acryloyl chloride (9 mg, 0.1 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-((2*R*,6*S*)-2,4,6-trimethylpiperazin-1-yl)-6,6a,7,8,9,10-hexahydro-12*H*-pyrazino[2,1-*c*]pyrido[3,4-*f*][1,4]oxazepin-12-one (Z249, 1.7 mg, yield: 3%). ES-API: [M+H]⁺=544.2.

### Embodiment 62-24 Synthesis of Z252

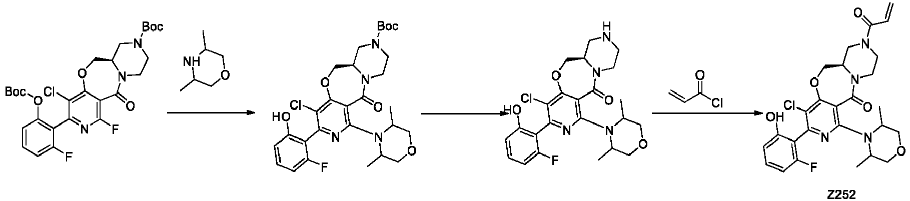

Step 1: (6a*R*)-8-*tert*-Butoxycarbonyl-3-(2-((tert-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (100 mg, 0.172 mmol), 3,5-dimethylmorpholine (119 mg, 1.03 mmol), *N*,*N*-diisopropylethylamine (66 mg, 0.516 mmol) and 1,4-dioxane (10 mL) were added to a 100 mL round bottom flask, stirred at 140°C for 2 hours under microwave irradiation. The mixture was extracted with ethyl acetate, washed with saturated brine, and the organic phase was dried, concentrated, and purified by silica gel column (petroleum ether: ethyl acetate = 20:80) to obtain (6a*R*)-8-*tert*-butoxycarbonyl-1-(3,5-dimethylmorphofino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (20 mg, yield of 20%). ES-API: [M+H]⁺=577.2.

Step 2: (6a*R*)-8-*tert*-Butoxycarbonyl-1-(3,5-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (20 mg, 0.035 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(3,5-dimethylmorpholino)-4-chloro-3-(2-fluoro-6-hydroxyphenyl)-6,6a,7,8,9,10-hexahydro-12*H*-pyrazino [2,1-*c*]pyrido[3,4-*f*][1,4]oxazepin-12-one (16 mg, crude product) as a yellow solid. ES-API: [M+H]⁺=477.2.

Step 3: (6a*R*)-1-(3,5-Dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (16 mg, crude product), 10 mL of dichloromethane and *N*,*N-*diisopropylethylamine (13 mg, 0.105 mmol) were added to a 50 mL round bottom flask. The reaction was cooled to 0°C, and a solution of acryloyl chloride in dichloromethane (3 mg, 0.035 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:H₂O=50:50 (0.1% NH₄HCO₃), flow rate: 15 mL/min, column temperature: 25°C) to obtain a target product of (6a*R*)-8-acryloyl-1-(3,5-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z252, 5 mg, 3-step yield of 20%) as a white solid. ES-API: [M+H]⁺= 531.1.

### Embodiment 62-25 Synthesis of Z252-1

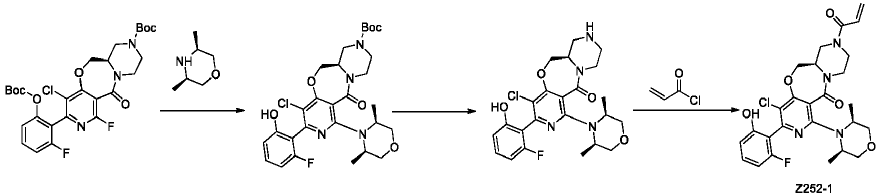

Step 1: (6a*R*)-8-*tert*-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (100 mg, 0.172 mmol), (3*R*,5*S*)-dimethylmorpholine (119 mg, 1.03 mmol), *N*,*N-*diisopropylethylamine (66 mg, 0.516 mmol) and 1,4-dioxane (10 mL) were added to a 100 mL round bottom flask, stirred at 140°Cfor 2 hours under microwave irradiation. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-(3*R*,5*S*-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (20 mg, yield of 20%). ES-API: [M+H]⁺=577.2.

Step 2: (6a*R*)-8-*tert*-Butoxycarbonyl-1-(3*R*,5*S*-dimethylmorphofino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (20 mg, 0.035 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(3*R*,5*S-*dimethylmorpholino)-4-chloro-3-(2-fluoro-6-hydroxyphenyl)-6,6a,7,8,9,10-hexahydro-12*H*-pyrazino [2,1-*c*]pyrido[3,4-*f*][1,4]oxazepin-12-one (16 mg, crude product) as a yellow solid. ES-API: [M+H]⁺=477.2.

Step 3: (6a*R*)-1-(3*R*,5*S*-Dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (16 mg, crude product), 10 mL of dichloromethane and *N*,*N*-diisopropylethylamine (13 mg, 0.105 mmol) were added to a 50 mL round bottom flask. The reaction was cooled to 0°C, and a solution of acryloyl chloride in dichloromethane (3 mg, 0.035 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:HzO=50:50 (0.1% NH₄HCO₃), flow rate: 15 mL/min, column temperature: 25°C) to obtain a target product of (6a*R*)-8-acryloyl-1-(3*R*,5*S*-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z252-1, 2 mg, 3-step yield of 10%) as a white solid. ES-API: [M+H]⁺= 531.1.

### Embodiment 62-26 Synthesis of Z244

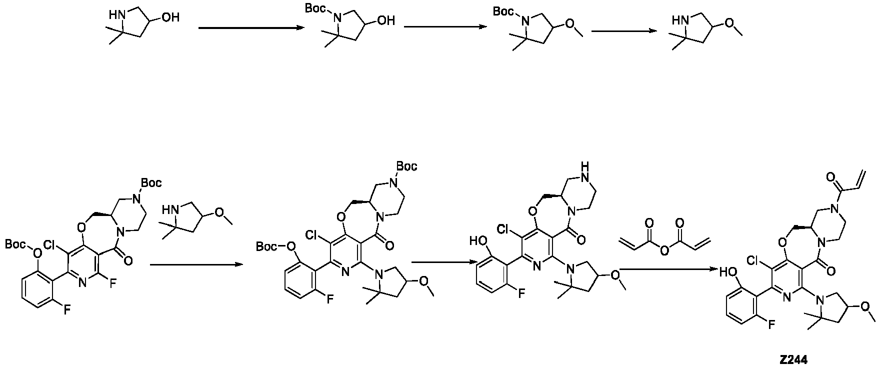

Step 1: 5,5-Dimethylpyrrolidin-3-ol (200 mg, 1.74 mmol) and triethylamine (2 mL) were dissolved in dichloromethane (4 mL), stirred at room temperature for 10 minutes, and then di*-tert*-butyl dicarbonate (400 mg, 1.83 mmol) was added dropwise to the solution, and the reaction was carried out at room temperature for three hours. After the reaction was completed, the mixture was extracted with dichloromethane, washed with water, and dried over sodium sulfate to obtain a product of *tert*-butyl 4-hydroxy-2,2-dimethylpyrrolidine-1-carboxylate (300 mg, Y: 76.68%), ES-API: [M+H]+= 216.1.

Step 2: *tert*-Butyl 4-hydroxy-2,2-dimethylpyrrolidine-1-carboxylate (300 mg, 1.39 mmol) and sodium hydride (30 mg, 1.25 mmol) were dissolved in anhydrous tetrahydrofuran (7 mL) at 0°C, and then iodomethane (240 mg, 1.69 mmol) was added dropwise to the solution, and the reaction was stirred for 1 hour. After the reaction was completed, the reaction mixture was quenched with sodium bicarbonate solution, extracted with ethyl acetate, washed with brine, and dried over sodium sulfate to obtain *tert*-butyl 4-methoxy-2,2-dimethylpyrrolidine-1-carboxylate (300 mg, Y: 98%), ES-API: [M+H]⁺= 230.1.

Step 3: *tert*-Butyl 4-methoxy-2,2-dimethylpyrrolidine-1-carboxylate (300 mg, 1.31 mmol) was dissolved in dichloromethane (3 mL), and then trifluoroacetic acid (3 mL) was added dropwise to the solution, and the reaction was stirred at room temperature for 2 hours. After the reaction was completed, the solvent was evaporated to dryness by rotary evaporation to obtain a product of 4-methoxy-2,2-dimethylpyrrolidine (160 mg, Y: 94.68%), ES-API: [M+H]⁺= 130.1.

Step 4: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (700 mg, 1.2 mmol) and 4-methoxy-2,2-dimethylpyrrolidine (160 mg, 1.24 mmol) were dissolved in acetonitrile (5 mL), and then potassium carbonate (500 mg, 3.6 mmol) was added thereto. The reaction was heated to 80°C and stirred for 16 hours, then quenched by adding water, extracted with ethyl acetate, washed with brine, dried over sodium sulfate, and subjected to thin-layer chromatography (petroleum ether/ethyl acetate=3/1) to obtain a product of (6a*R*)-3-(2-((*tert*-butoxycarbonyl)oxy)-6-fluorophenyl)-4-chloro-1-(4-methoxy-2,2-dimethylpyrrolidin-1-yl)-12-oxo-6a,7,9,10-tetrahydro-6*H*-pyrazino[2,1-*c*]pyrido[3,4-*f*][1,4]oxazepin-12-one (410 mg, Y: 49.5%), ES-API: [M+H]⁺= 691.2.

Step 5: (6a*R*)-3-(2-((*tert*-Butoxycarbonyl)oxy)-6-fluorophenyl)-4-chloro-1-(4-methoxy-2,2-dimethylpyrrolidin-1-yl)-12-oxo-6a,7,9,10-tetrahydro-6*H*-pyrazino[2,1-*c*]pyrido[3,4-*f*][1,4]oxazepin-12-one (410 mg, 0.594 mmol) was dissolved in dichloromethane (2 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-3-(2-fluoro-6-hydroxyphenyl)-1-(4-methoxy-2,2-dimethylpyrrolidin-1-yl)-12-oxo-6a,7,9,10-tetrahydro-6*H-*pyrazino[2,1-*c*]pyrido[3,4-*f*][1,4]oxazepin-12-one (400 mg, yield: 100%), ES-API: [M+H]⁺= 491.2.

Step 6: (6a*R*)-4-Chloro-3-(2-fluoro-6-hydroxyphenyl)-1-(4-methoxy-2,2-dimethylpyrrolidin-1-yl)-12-oxo-6a,7,9,10-tetrahydro-6*H*-pyrazino[2,1-*c*]pyrido[3,4-*f*][1,4]oxazepin-12-one (400 mg, crude, 0.594 mmol) was dissolved in dichloromethane (4 mL), then cooled to 0°C in an ice-water bath, and triethylamine (1 mL) was added dropwise thereto. The pH value was basic, and acrylic anhydride (52 mg, 0.416 mmol) was added dropwise thereto, stirred for 10 minutes. After the reaction was completed, the mixture was washed with water, washed with brine, dried over sodium sulfate, evaporated to dryness by rotary evaporation, and purified by thin-layer chromatography (ethyl acetate=100%) to obtain (6a*R*)-8-acryloyl-4-chloro-3 -(2-fluoro-6-hydroxyphenyl)-1-(4-methoxy-2,2-dimethylpyrrolidin-1-yl)-12-oxo-6,6a,7,8,9,10-hexahydro-12*H*-pyrazino[2,1-*c*]pyrido[3,4-*f*][1,4]oxazepin-12-one (Z244, 90 mg, yield: 27.9%). ¹H NMR (400 MHz, CDCl₃) δ 12.03 (s, 1H), 6.75 (s, 3H), 6.52 (s, 1H), 6.36 (d, *J* = 17.6 Hz, 2H), 5.84 - 5.71 (m, 1H), 4.38 (s, 3H), 4.14 (d, *J* = 13.4 Hz, 2H), 3.77 (s, 1H), 3.70 (s, 2H), 3.59 (s, 1H), 3.32 (s, 1H), 3.26 (s, 1H), 3.08 (s, 3H), 1.47 (s, 2H), 1.31 (d, *J* = 7.6 Hz, 6H). ES-API: [M+H]⁺=545.19

### Embodiment 62-27 Synthesis of Z243

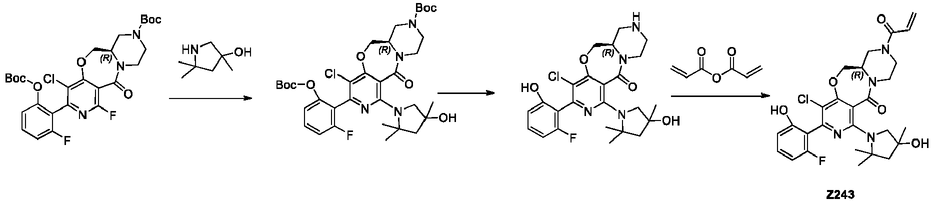

Step 1: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (300 mg, 0.516 mmol), 3,5,5-trimethylpyrrolidin-3-ol (500 mg, 0.774 mmol, crude product) and potassium carbonate (214 mg, 1.548 mmol) were dissolved in acetonitrile (3 mL), reacted at 80°C for 4 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert-*butoxycarbonyl)oxy)-6-fluorophenyl)-4-chloro-1-(4-hydroxy-2,2,4-trimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (250 mg, yield: 70.2%), ES-API: [M+H]⁺= 691.2.

Step 6: *tert*-Butyl (6a*R*)-3-(2-((*tert*-butoxycarbonyl)oxy)-6-fluorophenyl)-4-chloro-1-(4-hydroxy-2,2,4-trimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (250 mg, 0.362 mmol) was dissolved in dichloromethane (2 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-3-(2-fluoro-6-hydroxyphenyl)-1-(4-hydroxy-2,2,4-trimethylpyrrolidin-1-yl)-7,8,9,10-tetrahydro-6*H*-pyrazino[2,1-c]pyrido[3,4-*f*][1,4]oxazepin-12-one (300 mg, 100%), ES-API: [M+H]⁺= 491.2.

Step 7: (6a*R*)-4-Chloro-3-(2-fluoro-6-hydroxyphenyl)-1-(4-hydroxy-2,2,4-trimethylpyrrolidin-1-yl)-7,8,9,10-tetrahydro-6*H*-pyrazino[2,1-c]pyrido[3,4-*f*][1,4]oxazepin-12-one (300 mg, crude, 0.362 mmol) was dissolved in dichloromethane (3 mL), then cooled to 0°C in an ice-water bath, and triethylamine (1 mL) was added dropwise thereto. The pH value was basic, and acrylic anhydride (27 mg, 0.217 mmol) was added dropwise thereto, stirred for 10 minutes. After the reaction was completed, the reaction solution was added with water, washed with brine, dried over sodium sulfate, evaporated to dryness by rotary evaporation, and purified by thin-layer chromatography (ethyl acetate=100%) to obtain (6a*R*)-8-acryloyl-4-chloro-3-(2-fluoro-6-hydroxyphenyl)-1-(4-hydroxy-2,2,4-trimethylpyrrolidin-1-yl)-6,6a,7,8,9,10-hexahydro-12*H-*pyrazino[2,1-*c*]pyrido[3,4-*f*][1,4]oxazepin-12-one (Z243, 40 mg, yield: 20.3%). ¹H NMR (400 MHz, CDCl₃) δ7.34 - 7.29 (m, 1H),6.83 (d, *J=* 8.2 Hz, 1H), 6.71 (t, *J=* 8.9 Hz, 1H), 6.56 (s,1H), 6.42 (d, *J=* 15.6 Hz, 1H), 5.83 (s, 1H), 4.58 - 4.11(m, 6H), 4.09 - 3.44 (m, 6H), 1.86 (m, 2H), 1.42 (s, 6H), 1.25 (s, 3H). ES-API: [M+H]⁺= 545.2.

### Embodiment 62-28 Synthesis of Z309

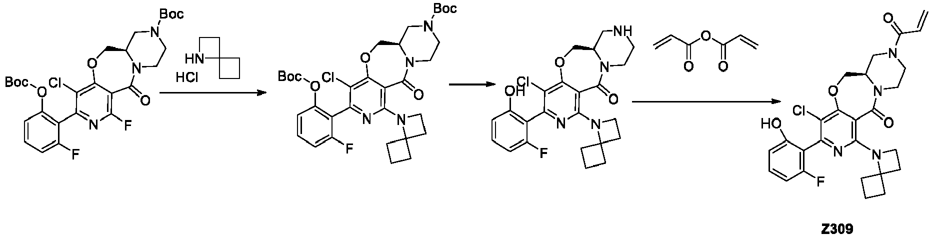

Step 1: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.172 mmol), 1-azaspiro[3.3]heptane hydrochloride (25 mg, 0.189 mmol) and potassium carbonate (71 mg, 0.515 mmol) were dissolved in acetonitrile (4 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert*-butoxycarbonyl)oxy)-6-fluorophenyl)-4-chloro-12-oxo-1-(1-azaspiro[3.3]heptan-1-yl)-6a,7,9,10-tetrahydro-12*H*-pyrazino[2,1-*c*]pyrido[3,4-*f*][1,4]oxazepine-8(6*H*)-carboxylate (120 mg, yield: 100%), ES-API: [M+H]⁺= 659.1.

Step 2: *tert*-Butyl (6a*R*)-3-(2-((*tert*-butoxycarbonyl)oxy)-6-fluorophenyl)-4-chloro-12-oxo-1-(1-azaspiro[3.3]heptan-1-yl)-6a,7,9,10-tetrahydro-12*H*-pyrazino[2,1-*c*]pyrido[3,4-*f*][1,4]oxazepine-8(6H)-carboxylate (120 mg, 0.182 mmol) was dissolved in dichloromethane (4 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-3-(2-fluoro-6-hydroxyphenyl)-1-(1-azaspiro[3.3]heptan-1-yl)-6,6a,7,8,9,10-hexahydro-12*H-*pyrazino[2,1-*c*]pyrido[3,4-*f*][1,4]oxazepin-12-one (80 mg, 100%), ES-API: [M+H]⁺= 459.

Step 3: (6a*R*)-4-Chloro-3-(2-fluoro-6-hydroxyphenyl)-1-(1-azaspiro[3.3]heptan-1-yl)-6,6a,7,8,9,10-hexahydro-12*H*-pyrazino[2,1-*c*]pyrido[3,4-*f*][1,4]oxazepin-12-one (80 mg, 0.171 mmol) was dissolved in dichloromethane (4 mL), then cooled to 0°C in an ice-water bath, and triethylamine (2 mL) was added dropwise thereto. The pH value was basic, and acrylic anhydride (17 mg, 0.137 mmol) was added dropwise thereto, stirred for ten minutes. After the reaction was completed, the mixture was extracted with ethyl acetate, washed with water, washed with brine, dried over sodium sulfate, evaporated to dryness by rotary evaporation and then prepared (preparative separation conditions: ELITE P270, chromatographic column information: Ultimate XB-C18, 50*250 mm, 10 µm, mobile phase system: A: purified water B: pure acetonitrile, flow rate: 80 mL/min, gradient: within 40 minutes, B/A=20%-90%, wavelength: 214 nm, column temperature: room temperature) to obtain (6a*R*)-8-acryloyl-4-chloro-3-(2-fluoro-6-hydroxyphenyl)-1-(1-azaspiro[3.3]heptan-1-yl)-6,6a,7,8,9,10-hexahydro-12*H*-pyrazino[2,1-*c*]pyrido[3,4-*f*][1,4]oxazepin-12-one (Z309, 2.2 mg, yield: 2.5%). ¹H NMR (400 MHz, CDCl₃) δ 6.86 (d, *J* = 8.2 Hz, 1H), 6.72 (t, *J* = 8.9 Hz, 1H), 6.50 (d, *J* = 18.4 Hz, 2H), 5.83 (s, 1H), 5.21 (s, 1H), 4.43 (s, 2H), 4.13 (s, 3H), 4.02 (s, 1H), 3.88 (s, 5H), 2.70 (t, *J*= 8.1 Hz, 4H), 1.30 (d, *J*= 21.4 Hz, 4H). ES-API: [M+H]⁺=513.1.

### Embodiment 62-29 Synthesis of Z257a

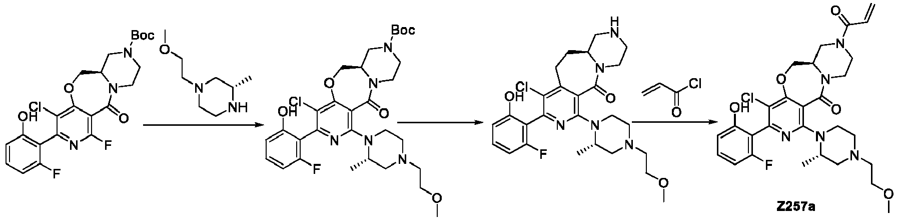

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (75 mg, 0.15 mmol), (S)-1-(2-methoxyethyl)-3-methylpiperazine (30 mg, 0.2 mmol), N,N-diisopropylethylamine (58 mg, 0.45 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-1-((*S*)-4-(2-methoxyethyl)-2-methylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (81 mg, yield of 75%). ES-API: [M+H]⁺=620.3.

Step 2: Trifluoroacetic acid (1 mL) was added to a solution of *tert*-butyl (6a*R*)-4-chloro-3-(2-fluoro-6-hydroxyphenyl)-1-((*S*)-4-(2-methoxyethyl)-2-methylpiperazin-1-yl)-12-oxo-6a,7,9,10-tetrahydro-12H-pyrazino[2,1-*c*]pyrido[3,4-*f*][1,4]oxazepine-8(6*H*)-carboxylate (81 mg, 0.13 mmol) in dichloromethane (2 mL), reacted at room temperature for 1 hour. The reaction solution was directly concentrated to obtain a crude procuct of *tert*-butyl (6a*R*)-4-chloro-3-(2-fluoro-6-hydroxyphenyl)-1-((*S*)-4-(2-methoxyethyl)-2-methylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (60 mg). ES-API: [M+H]⁺=520.3.

Step 3: The above crude product of *tert*-butyl (6a*R*)-4-chloro-3-(2-fluoro-6-hydroxyphenyl)-1-((*S*)-4-(2-methoxyethyl)-2-methylpiperazin-1-yl)-12-oxo-6a,7,9,10-tetrahydro-12*H*-pyrazino[2,1-*c*]pyrido[3,4-f][1,4]oxazepine-8(6H)-carboxylate (60 mg, 0.12 mmol) was dissolved in dichloromethane (5 mL), and *N,N*-diisopropylethylamine (46 mg, 0.36 mmol) was added thereto. The reaction solution was cooled to 0°C, and acryloyl chloride (10 mg, 0.11 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-((*S*)-4-(2-methoxyethyl)-2-methylpiperazin-1-yl)-6,6a,7,8,9,10-hexahydro-12*H-*pyrazino[2,1-*c*]pyrido[3,4-*f*][1,4]oxazepin-12-one (Z257a, 10.3 mg, yield: 15%). ES-API: [M+H]⁺=574.2.

### Embodiment 62-30 Synthesis of Z310

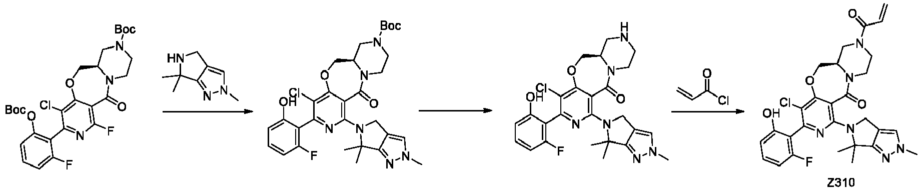

Step 1: (6a*R*)-8-tert-Butoxycarbonyl-3-(2-((tert-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (50 mg,, 0.1086 mmol), 2,6,6-trimethyl-2,4,5,6-tetrahydropyrrolo[3,4-*c*]pyrazole (13 mg, 0.086 mmol), *N,N-*diisopropylethylamine (33 mg, 0.258 mmol) and acetonitrile (8 mL) were added to a 100 mL round bottom flask, stirred at 115°C for 2 hours under microwave irradiation. The mixture was extracted with ethyl acetate, washed with saturated brine, and the organic phase was dried, concentrated, and purified by silica gel column (petroleum ether: ethyl acetate = 0:100) to obtain (6a*R*)-8-*tert*-butoxycarbonyl-1-(2,6,6-trimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (20 mg, yield of 38%). ES-API: [M+H]⁺=613.2.

Step 2: (6a*R*)-8-tert-Butoxycarbonyl-1-(2,6,6-trimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (20 mg, 0.032 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(2,6,6-trimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (16 mg, crude product) as a yellow solid. ES-API: [M+H]⁺=513.2.

Step 3: (6a*R*)-1-(2,6,6-Trimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (16 mg, crude product), 10 mL of dichloromethane and *N*,*N*-diisopropylethylamine (15 mg, 0.117 mmol) were added to a 50 mL round bottom flask. The reaction was cooled to 0°C, and a solution of acryloyl chloride in dichloromethane (3.5 mg, 0.039 mmol) was added dropwise to the reaction solution. The reaction was stirred at 0°C for 10 minutes. The mixture was washed with 5 mL of water, and the organic phase was dried and concentrated, and the crude product was purified by preparative HPLC (column: C18 19*150 mm 5 µm, mobile phase: ACN:H₂O=50:50 (0.1% NH₄HCO₃), flow rate: 15 mL/min, column temperature: 25°C) to obtain a target product of (6a*R*)-8-acryloyl-1-(2,6,6-trimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z310, 4 mg, 3-step yield of 12%) as a white solid. ES-API: [M+H]⁺=567.2.

### Embodiment 62-31 Synthesis of Z279-1

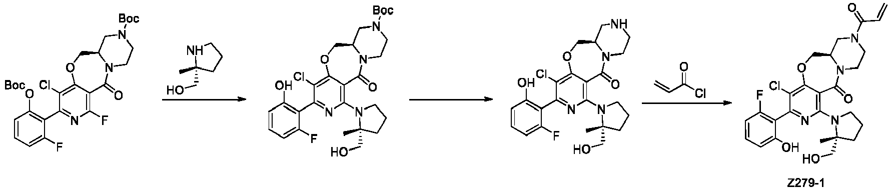

Step 1: (6a*R*)-8-*tert*-Butoxycarbonyl-3-(2-((tert-butoxycarbonyl)oxy)-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (66 mg, 0.113 mmol), (R)-2-(hydroxymethyl)-2-methylpyrrolidine (51 mg, 0.34 mmol), *N*,*N*-diisopropylethylamine (33 mg, 0.258 mmol) and acetonitrile (10 mL) were added to a 100 mL round bottom flask, stirred at 80°C for 1.5 hours. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-((*R*)-2-(hydroxymethyl)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (48 mg, yield of 73%). ES-API: [M+H]⁺=577.2.

Step 2: (6a*R*)-8-*tert*-Butoxycarbonyl-1-((*R*)-2-(hydroxymethyl)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (48 mg, 0.083 mmol), 1 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-((*R*)-2-(hydroxymethyl)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (40 mg, crude product) as a yellow solid. ES-API: [M+H]⁺=477.2.

Step 3: (6a*R*)-1-((*R*)-2-(Hydroxymethyl)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (40 mg, 0.083mmol), 10 mL of dichloromethane and N,N-diisopropylethylamine (32 mg, 0.0249 mmol) were added to a 50 mL round bottom flask. The reaction was cooled to 0°C, and a solution of acryloyl chloride in dichloromethane (7.5 mg, 0.083 mmol) was added dropwise to the reaction solution. The reaction was stirred at 0°C for 10 minutes. The mixture was washed with 10 mL of water, and the organic phase was dried and concentrated, and the crude product was purified by preparative HPLC (column: C18 19*150 mm 5 µm, mobile phase: ACN:HzO=50:50 (0.1% NH₄HCO₃), flow rate: 15 mL/min, column temperature: 25°C) to obtain a target product of (6a*R*)-8-acryloyl-1-((*R*)-2-(hydroxymethyl)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z279-1, 19 mg, 3-step yield of 48%) as a white solid. ES-API: [M+H]⁺= 531.1. ¹H NMR (500 MHz, DMSO-*d*₆) δ 9.99 (dd, *J* = 4.7, 1.7 Hz, 1H), 7.23 (q, *J* = 7.5 Hz, 1H), 6.82 - 6.65 (m, 3H), 6.17 (d, *J* = 16.8 Hz, 1H), 5.74 (s, 1H), 4.80-4.75 (m, 1H), 4.51 (s, 1H), 4.27 (s, 1H), 4.19 -3.92 (m, 5H), 3.49 (s, 3H), 3.38 (d, *J=* 14.1 Hz, 2H), 2.94 (s, 1H), 2.21 (s, 1H), 1.84 (s, 1H), 1.75 (s, 1H), 1.60 - 1.52 (m, 1H), 1.36 (d, *J* = 6.5 Hz, 3H).

### Embodiment 62-32 Synthesis of Z279-2

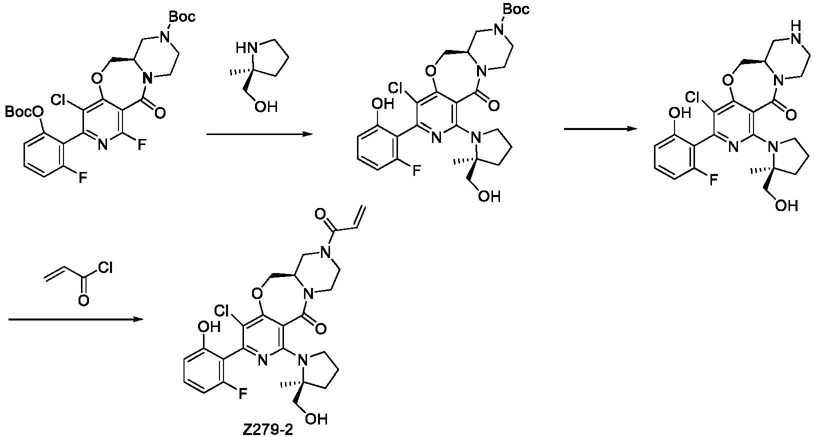

Step 1: *tert*-Butyl (6a*R*)-3-(2-((tert-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (60 mg, 0.103 mmol) and 3 mL of acetonitrile were added to a round bottom flask. Potassium carbonate (80 mg, 0.58 mmol) and (S)-(2-methyltetrahydropyrrol-2yl)methanol (45 mg, 0.30 mmol) were then added to the reaction solution. The reaction was stirred at 80°C overnight. After completion of the reaction, the reaction solution was diluted with ethyl acetate, washed with saturated brine, and dried over anhydrous sodium sulfate. The organic phase was concentrated to dryness and purified by column chromatography (ethyl acetate/*n*-hexane: 0-100%) to obtain *tert*-butyl (6a*R*)-3-(2-((tert-butoxycarbonyl)oxy)-6-fluorophenyl)-4-chloro-1-((*S*)-(2-hydroxymethyl-2-methyltetrahydropyrrol-1-yl)-12-oxo-6a,7,9,10-tetrahydro-12*H*-pyrazino[2,1-*c*]pyrido[3,4-*f*][1,4]oxazepine-8(6*H*)-carboxylate (32 mg, yield of 54%). ES-API: [M+H]⁺=577.1.

Step 2: *tert*-Butyl (6a*R*)-3-(2-((*tert*-butoxycarbonyl)oxy)-6-fluorophenyl)-4-chloro-1-((*S*)-(2-hydroxymethyl-2-methyltetrahydropyrrol-1-yl)-12-oxo-6a,7,9,10-tetrahydro-12*H*-pyrazino[2,1-*c*]pyrido[3,4-*f*][1,4]oxazepine-8(6*H*)-carboxylate (32 mg, 0.056 mmol), 2 mL of dichloromethane and 0.5 mL of trifluoroacetic acid were added to around bottom flask. The mixture was stirred at room temperature for 3 hours, and the completion of reaction was detected by LC-MS. The reaction solution was concentrated to obtain (6a*R*)-4-chloro-1-((*S*)-(2-hydroxymethyl-2-methyltetrahydropyrrol-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (crude product) as a brown oily liquid. The crude product was directly used in the next step. ES-API: [M+H]⁺=477.2.

Step 3: (6a*R*)-4-Chloro-1-((*S*)-(2-hydroxymethyl-2-methyltetrahydropyrrol-1-yl)-3-(2-fluoro-6-hydroxyphenyl)-6,6a,7,8,9,10-hexahydro-12*H*-pyrazino[2,1-*c*]pyrido [3,4-*f*][1,4]oxazepin-12-one (crude product), 10 mL of dichloromethane and triethylamine (0.093 mL, 0.67 mmol) were added to a round bottom flask. The reaction was cooled to 0°C, and a solution of acrylic anhydride in dichloromethane (3.65 mg, 0.04 mmol, 1 mL) was added to the reaction solution. The reaction was stirred at 0°C for 10 minutes. 10 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-1-((*S*)-(2-hydroxymethyl-2-methyltetrahydropyrrol-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z279-2, 6.5 mg, 2-step yield of 22%). ¹H NMR (500 MHz, DMSO-d₆) δ 10.00 (s, 1H), 7.31-7.18 (m, 1H), 6.86-6.62 (m, 3H), 6.17 (d, *J =* 17.0 Hz, 1H), 5.82-5.66 (m, 1H), 4.80-4.68 (m, 1H), 4.53 (s, 1H), 4.36-3.87 (m, 5H), 3.79-3.70 (m, 1H), 3.60-3.50 (m, 2H), 3.50-3.37 (m, 2H), 3.19 (s, 1H), 2.96 (s, 1H), 1.99-1.90 (m, 1H), 1.83 (s, 2H), 1.64 (d, *J* = 11.0 Hz, 1H), 1.42 (d, *J* = 10.0 Hz, 3H). ES-API: [M+H]⁺=531.1.

### Embodiment 62-33 Synthesis of Z277-1

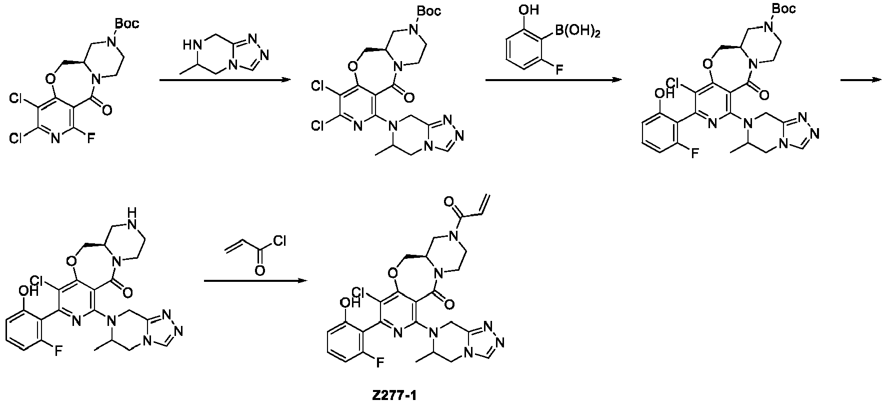

Step 1: A solution of compound *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.25 mmol), 6-methyl-5,6,7,8-tetrahydro-[1,2,4]triazolo[4,3-*a*]pyrazine (80 mg, 0.58 mmol) and *N*,*N*-diisopropylethylamine (100 mg, 0.74 mmol) in 1,4-dioxane (1 mL) was stirred at 140°C for 2 hours under microwave irradiation. The reaction solution was filtered and concentrated, purified by flash silica gel column (methanol: dichloromethane: 0-10%) to obtain *tert*-butyl (6a*R*)-3,4-dichloro-1-(6-methyl-5,6-dihydro-[1,2,4]triazolo[4,3-*a*]pyrazin-7(8*H*)-yl)-12-oxo-6a,7,9,10-tetrahydro-12*H*-pyrazino[2,1-c]pyrido[3,4-*f*][1,4]oxazepine-8(6*H*)-carboxylate (50 mg). ES-API: [M+H]⁺=524.0.

Step 2: Under nitrogen protection, a mixed solution of compound *tert*-butyl (6a*R*)-3,4-dichloro-1-(6-methyl-5,6-dihydro-[1,2,4]triazolo[4,3-*a*]pyrazin-7(8*H*)-yl)-12-oxo-6a,7,9,10-tetrahydro-12*H-*pyrazino[2,1-*c*]pyrido[3,4-*f*][1,4]oxazepine-8(6*H*)-carboxylate (50 mg, 0.095 mmol), 2-fluoro-6-hydroxyphenylboronic acid (45 mg, 0.284 mmol), tetrakis(triphenylphosphine)palladium (11 mg, 0.010 mmol) and potassium carbonate (40 mg, 0.286 mmol) in 1,4-dioxane (1 mL) and water (0.2 mL) was stirred at 110°C for 2 hours. The reaction solution was poured into water (10 mL) and ethyl acetate (10 mL * 3), and the organic phases were combined, filtered, dried and purified by flash silica gel column (ethyl acetate:petroleum ether: 0-100%) to obtain *tert*-butyl (6a*R*)-4-chloro-3-(2-fluoro-6-hydroxyphenyl)-1-(6-methyl-5,6-dihydro-[1,2,4]triazolo[4,3-*a*]pyrazin-7(8*H*)-yl)-12-oxo-6a,7,9,10-tetrahydro-12*H-*pyrazino[2,1-*c*]pyrido[3,4-*f*][1,4]oxazepine-8(6*H*)-carboxylate (30 mg). ES-API: [M+H]⁺=600.1.

Step 3: Under an ice bath, a solution of *tert*-butyl (6a*R*)-4-chloro-3-(2-fluoro-6-hydroxyphenyl)-1-(6-methyl-5,6-dihydro-[1,2,4]triazolo[4,3-*a*]pyrazin-7(8*H*)-yl)-12-oxo-6a,7,9,10-tetrahydro-12*H-*pyrazino[2,1-*c*]pyrido[3,4-*f*][1,4]oxazepine-8(6*H*)-carboxylate (30 mg, 0.05 mmol) in dichloromethane (2 mL) was added with trifluoroacetic acid (0.5 mL), and the reaction solution was stirred at room temperature for 1 hour. The mixture was concentrated under reduced pressure to obtain a crude product of (6aR)-4-chloro-3-(2-fluoro-6-hydroxyphenyl)-1-(6-methyl-5,6-dihydro-[1,2,4]tnazolo[4,3-*a*]pyrazin-7(8*H*)-yl)-6,6a,7,8,9,10-hexahydro-12*H*-pyrazino[2,1-*c*]pyrido[3,4-*f*][1,4]oxazepin-12-one (25 mg). ES-API: [M+H]⁺=500.0.

Step 3: The above crude procuct of (6a*R*)-4-chloro-3-(2-fluoro-6-hydroxyphenyl)-1-(6-methyl-5,6-dihydro-[1,2,4]triazolo[4,3-*a*]pyrazin-7(8*H*)-yl)-6,6a,7,8,9,10-hexahydro-12*H*-pyrazino[2,1-*c*]pyrido[3,4-*f*][1,4]oxazepin-12-one (25 mg, 0.05 mmol) was dissolved in dichloromethane (2 mL), and *N*,*N*-diisopropylethylamine (32 mg, 0.25 mmol) was added thereto. The reaction solution was cooled to 0°C, and acryloyl chloride (5 mg, 0.05 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(6-methyl-5,6-dihydro-[1,2,4]triazolo[4,3-*a*]pyrazin-7(8*H*)-yl)-6,6a,7,8,9,10-hexahydro-12*H*-pyrazino[2,1-*c*]pyrido[3,4-f][1,4]oxazepin-12-one (Z277-1, 7 mg, yield: 25%). ES-API: [M+H]⁺ = 554.1. ¹H NMR (500 MHz, DMSO-*d*₆) δ 10.13 - 9.97 (m, 1H), 8.55 - 8.42 (m, 1H), 7.36 - 7.21 (m, 1H), 6.86 - 6.66 (m, 3H), 6.24 - 6.13 (m, 1H), 5.84 - 5.69(m, 1H), 5.09 - 4.92 (m, 1H), 4.79 - 4.53 (m, 2H), 4.52 - 4.47 (m, 1H), 4.47 - 4.34 (m, 2H), 4.32 - 4.19 (m, 1H), 4.18 - 4.01 (m, 4H), 4.01 - 3.70 (m, 1H), 3.69 - 3.43 (m, 2H), 1.17 -1.00 (m, 3H).

### Embodiment 62-34 Synthesis of Z311-1

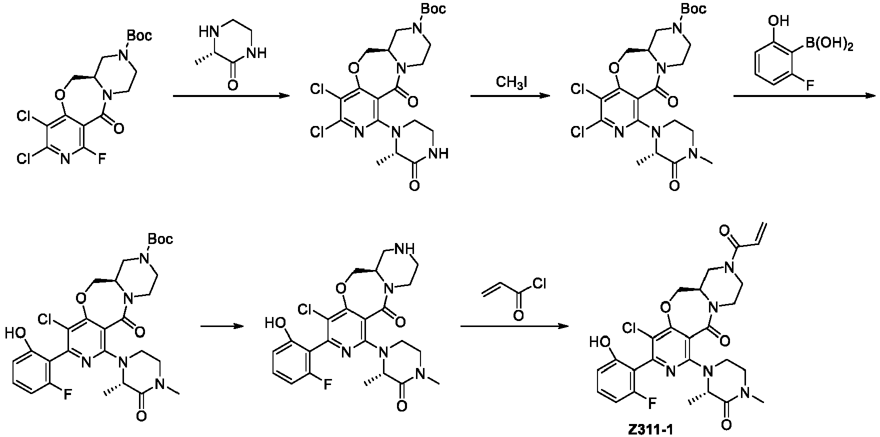

Step 1: A solution of compound *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.25 mmol), (S)-3-methylpiperazin-2-one (56 mg, 0.49 mmol) and *N*,*N*-diisopropylethylamine (100 mg, 0.74 mmol) in 1,4-dioxane (2 mL) was stirred at 140°C for 2 hours under microwave irradiation. The reaction solution was filtered and concentrated, purified by flash silica gel column (methanol:dichloromethane: 0-10%) to obtain tert-butyl (6a*R*)-3,4-dichloro-1-((*S*)-2-mefliyl-3-oxopiperazin-1-yl)-12-oxo-6a,7,9,10-tetrahydro-12*H-*pyrazino[2,1-*c*]pyrido[3,4-*f*][1,4]oxazepine-8(6*H*)-carboxylate (80 mg). ES-API: [M+H]⁺=500.0.

Step 2: A mixed solution of compound *tert*-butyl (6a*R*)-3,4-dichloro-1-((S)-2-methyl-3-oxopiperazin-1-yl)-12-oxo-6a,7,9,10-tetrahydro-12*H*-pyrazino[2,1-*c*]pyrido[3,4-*f*][1,4]oxazepine-8(6*H*)-carboxylate (80 mg, 0.16 mmol), iodomethane (340 mg, 2.40 mmol) and potassium carbonate (66 mg, 0.48 mmol) in acetonitrile (1 mL) were stirred overnight at 50°C in a sealed tube. After the reaction was completed, the mixture was filtered and concentrated to obtain a white solid of *tert*-butyl (*R*)-3,4-dichloro-1-((*S*)-2,4-dimethyl-3 -oxopiperazin-1-yl)-12-oxo-6a,7,9,10-tetrahydro-12*H*-pyrazino[2,1-*c*]pyrido[3,4-*f*][1,4]oxazepine-8(6*H*)-carboxylate (80 mg). ES-API: [M+H]⁺=514.2.

Step 3: Under nitrogen protection, a mixed solution of compound *tert*-butyl (*R*)-3,4-dichloro-1-((*S*)-2,4-dimethyl-3-oxopiperazin-1-yl)-12-oxo-6a,7,9,10-tetrahydro-12H-pyrazino[2,1-c]pyrido[3,4-*f*][1,4]oxazepine-8(6*H*)-carboxylate (80 mg, 0.16 mmol), 2-fluoro-6-hydroxyphenylboronic acid (73 mg, 0.47 mmol), tetrakis(triphenylphosphine)palladium (18 mg, 0.02 mmol) and potassium carbonate (64 mg, 0.47 mmol) in 1,4-dioxane (2 mL) and water (0.4 mL) was stirred at 110°C for 30 minutes. The reaction solution was poured into water (10 mL) and ethyl acetate (10 mL * 3), and the organic phases were combined, filtered, dried and purified by flash silica gel column (methanol:dichloromethane: 0-10%) to obtain *tert-*butyl (6a*R*)-4-chloro-3-(2-fluoro-6-hydroxyphenyl)-1-((*S*)-2,4-dimethyl-3-oxopiperazin-1-yl)-12-oxo-6a,7,9,10-tetrahydro-12*H*-pyrazino[2,1-*c*]pyrido[3,4-*f*][1,4]oxazepine-8(6*H*)-carboxylate (50 mg). ES-API: [M+H]⁺=590.2.

Step 4: Under an ice bath, a solution of *tert*-butyl (6a*R*)-4-chloro-3-(2-fluoro-6-hydroxyphenyl)-1-((*S*)-2,4-dimethyl-3-oxopiperazin-1-yl)-12-oxo-6a,7,9,10-tetrahydro-12*H*-pyrazino[2,1-*c*]pyrido[3,4-*f*][1,4]oxazepine-8(6*H*)-carboxylate (50 mg, 0.085 mmol) in dichloromethane (2 mL) was added with trifluoroacetic acid (0.5 mL), and the reaction solution was stirred at room temperature for 1 hour. The reaction solution was concentrated under reduced pressure to obtain a crude product of (6aR)-4-chloro-1-((S)-2,4-dimethyl-3-oxopiperazin-1-yl)-3-(2-fluoro-6-hydroxyphenyl)-6,6a,7,8,9,10-hexahydro-12H-pyrazino[2,1-*c*]pyrido[3,4-*f*][1,4]oxazepin-12-one (42 mg). ES-API: [M+H]⁺=490.1.

Step 5: The above crude procuct of (6a*R*)-4-chloro-1-((*S*)-2,4-dimethyl-3-oxopiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (42 mg, 0.085 mmol) was dissolved in dichloromethane (2 mL), and N ,N-diisopropylethylamine (55 mg, 0.428 mmol) was added thereto. The reaction solution was cooled to 0°C, and acryloyl chloride (8 mg, 0.085 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-((*S*)-2,4-dimethyl-3-oxopiperazin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z311-1, 28 mg, yield: 60%). ES-API: [M+H]⁺= 544.1. ¹H NMR (400 MHz, DMSO-d₆) δ 10.07 - 9.99 (m, 1H), 7.26 (dd, *J*= 15.5, 7.5 Hz, 1H), 6.84 - 6.63 (m, 3H), 6.17 (d, *J*= 16.5 Hz, 1H), 5.79 - 5.67 (m, 1H), 4.59 - 3.75 (m, 8H), 3.73 - 3.43 (m, 5H), 3.15 (d, *J*= 11.2 Hz, 1H), 2.84 (s, 3H), 1.40 (t, *J*= 7.5 Hz, 3H).

### Embodiment 62-35 Synthesis of Z241

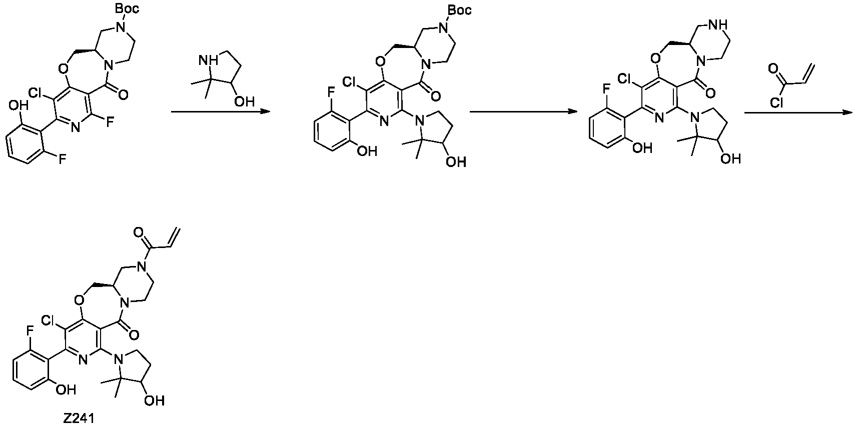

Step 1: (6a*R*)-8-*tert*-Butoxycarbonyl-3-(2-hydroxy-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (250 mg, 0.52 mmol), 3-hydroxy-2,2-dimethylpyrrolidine (120 mg, 1.04 mmol), *N*,*N*-diisopropylethylamine (201 mg, 1.56 mmol) and acetonitrile (10 mL) were added to a 100 mL round bottom flask, stirred at 80°C for 18 hours. The mixture was extracted with ethyl acetate, washed with saturated brine, and the organic phase was dried, concentrated, and purified by silica gel column (petroleum ether:ethyl acetate = 10:90) to obtain (6a*R*)-8-*tert*-butoxycarbonyl-1-(3-hydroxy-2,2-dimethylpyrrolidin-1-yl)-4-chloro-3 -(2-fluoro-6-hydroxyphenyl)-6,6a,7,8,9,10-hexahydro-12*H*-pyrazino[2,1-*c*]pyrido[3,4-*f*][1,4]oxazepin-12-one (237 mg, yield of 79%). ES-API: [M+H]⁺=577.2.

Step 2: (6a*R*)-8-*tert*-Butoxycarbonyl-1-(3-hydroxy-2,2-dimethylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (237 mg, 0.411 mmol), 5 mL of trifluoroacetic acid and 20 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(3-hydroxy-2,2-dimethylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one(196 mg, crude product) as a yellow solid. ES-API: [M+H]⁺=477.2.

Step 3: (6a*R*)-1-(3-Hydroxy-2,2-dimethylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (196 mg, 0.411 mmol), 10 mL of dichloromethane and *N*,*N*-diisopropylethylamine (159 mg, 1.233 mmol) were added to a 50 mL round bottom flask. The reaction was cooled to 0°C, and a solution of acryloyl chloride in dichloromethane (33 mg, 0.37 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:HzO=50:50 (0.1% NH₄HCO₃), flow rate: 15 mL/min, column temperature: 25°C) to obtain a target product of (6a*R*)-8-acryloyl-1-(3-hydroxy-2,2-dimethylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z241, 150 mg, 3-step yield of 54%) as a white solid. ES-API: [M+H]⁺= 531.1. ¹H NMR (400 MHz, CDCl₃) δ 7.35 (s, 1H), 6.85 (d, *J =* 8.1 Hz, 1H), 6.71 (t, *J =* 8.7 Hz, 1H),6.56 (s, 1H), 6.41 (d, *J =* 15.1 Hz, 1H), 5.82 (s, 1H), 5.34 (s, 1H), 4.38-4.21 (m, 5H), 3.95 (m, 3H), 3.76-3.66 (m, 3H), 2.22 (t, *J*= 7.7 Hz, 1H), 2.01 (s, 2H), 1.25 (s, 6H).

### Embodiment 62-36 Synthesis of Z276-1

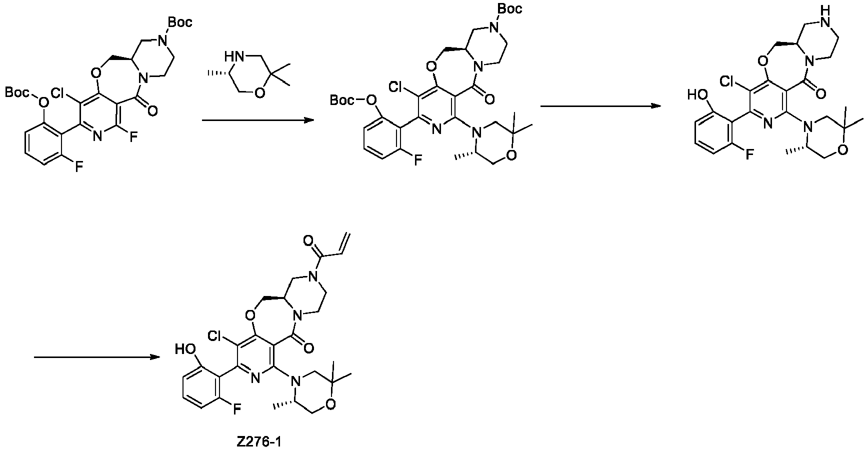

Step 1: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.172 mmol), (*S*)-2,2,5-trimethylmorpholine (20 mg, 0.172 mmol) and potassium carbonate (62 mg, 0.516 mmol) were dissolved in acetonitrile (2 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert*-butoxycarbonyl)oxy)-6-fluorophenyl)-4-chloro-12-oxo-1-((*S*)-2,2,5-trimethylmorpholino)-6a,7,9,10-tetrahydro-6*H*-pyrazino[2,1-*c*]pyrido[3,4-*f*][1,4]oxazepine-8(12*H*)-carboxylate(105 mg, yield of 89%, white solid). ES-API: [M+H]⁺= 691.3.

Step 2: tert-Butyl (6a*R*)-*tert*-Butyl 3-(2-((tert-butoxycarbonyl)oxy)-6-fluorophenyl)-4-chloro-12-oxo-1-((*S*)-2,2,5-trimethylmorpholino)-6a,7,9,10-tetrahydro-6*H*-pyrazino[2,1-*c*]pyrido[3,4-*f*][1,4]oxazepine-8(12*H*)-carboxylate (105 mg, 0.15 mmol) was dissolved in 2 mL of dichloromethane, then trifluoroacetic acid (2 mL) was added thereto. The reaction solution was stirred at room temperature for 2 hours, and the reaction was monitored to be complete, diluted with dichloromethane, washed with water, washed with saturated sodium bicarbonate and brine once respectively, dried over anhydrous sodium sulfate, and the filtrate was evaporated to dryness by rotary evaporation to obtain compound (6a*R*)-4-chloro-3-(2-fluoro-6-hydroxyphenyl)-1-((*S*)-2,2,5-trimethylmorpholino)-7,8,9,10-tetrahydro-6*H*-pyrazino[2,1-*c*]pyrido[3,4-*f*][1,4]oxazepin-12(6a*H*)-one (74 mg, yield: 100%, white solid). ES-API: [M+H]⁺=491.3

Step 3: (6a*R*)-4-Chloro-3-(2-fluoro-6-hydroxyphenyl)-1-((*S*)-2,2,5-trimethylmorpholino)-7,8,9,10-tetrahydro-6*H*-pyrazino[2,1-*c*]pyrido[3,4-*f*][1,4]oxazepin-12(6a*H*)-one (74 mg, 0.15 mmol) was dissolved in dichloromethane (3 mL), then cooled to 0°C in an ice-water bath, and triethylamine (45 mg, 0.45 mmol) was added dropwise thereto. The pH value was basic, and acrylic anhydride (15 mg, 0.12 mmol) was added dropwise thereto, stirred for 10 minutes. After the reaction was completed, the mixture was diluted with dichloromethane, washed with water, dried over sodium sulfate, evaporated to dryness by rotary evaporation and then purified (dichloromethane/methanol=10/l, R_{f}=0.5) to obtain (6aR)-8-acryloyl-4-chloro-3-(2-fluoro-6-hydroxyphenyl)-1-((*S*)-2,2,5-trimethylmorpholino)-6,6a,7,8,9,10-hexahydro-12*H-*pyrazino[2,1-*c*]pyrido[3,4-*f*][1,4]oxazepin-12-one (Z276-1, 12 mg, yield: 15%, white solid). ¹H NMR (400 MHz, CDCl₃) δ 7.30 (t, *J =* 7.5 Hz, 2H), 6.87 (s, 1H), 6.70 (t, *J =* 9.0 Hz, 1H), 6.41 (d, *J =* 16.0 Hz, 1H), 5.82 (d, *J =* 10.2 Hz, 1H), 4.37 (s, 2H), 4.25 (s, 2H), 4.07 (s, 1H), 3.93 (d, *J =* 11.2 Hz, 3H), 3.82 (s, 1H), 3.60 (s, 5H), 1.38 (d, *J*= 6.3 Hz, 3H), 1.32 (s, 6H). ES-API: [M+H]⁺=545.2

### Embodiment 62-37 Synthesis of Z312-1

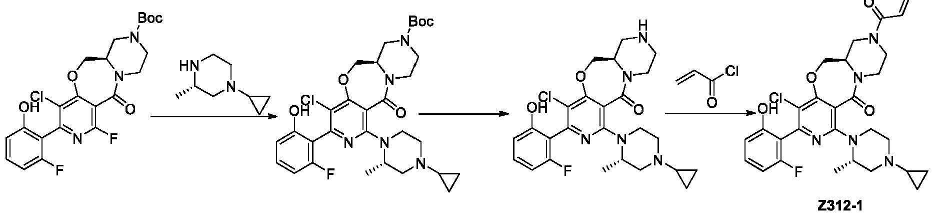

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (130 mg, 0.27 mmol), (S)-1-cyclopropyl-3-methylpiperazine (70 mg, 0.5 mmol), N,N-diisopropylethylamine (102 mg, 0.8 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-1-((*S*)-4-cyclopropyl-2-methylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (82 mg, yield of 50%). ES-API: [M+H]⁺=602.3.

Step 2: Trifluoroacetic acid (1 mL) was added to a solution of tert-butyl (6a*R*)-4-chloro-1-((*S*)-4-cyclopropyl-2-methylpiperazin-1-yl)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (82 mg, 0.14 mmol) in dichloromethane (2 mL), reacted at room temperature for 1 hour. The reaction solution was directly concentrated to obtain a crude product of (6a*R*)-4-chloro-1-((*S*)-4-cyclopropyl-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (68 mg). ES-API: [M+H]⁺=502.3.

Step 3: The above crude procuct of (6a*R*)-4-chloro-1-((*S*)-4-cyclopropyl-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (68 mg, 0.11 mmol) was dissolved in dichloromethane (5 mL), and N,N-diisopropylethylamine (42 mg, 0.33 mmol) was added thereto. The reaction solution was cooled to 0°C, and acryloyl chloride (9 mg, 0.10 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-((*S*)-4-cyclopropyl-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z312-1, 16 mg, yield: 26%). ES-API: [M+H]⁺=556.2.

### Embodiment 62-38 Synthesis of Z313-1

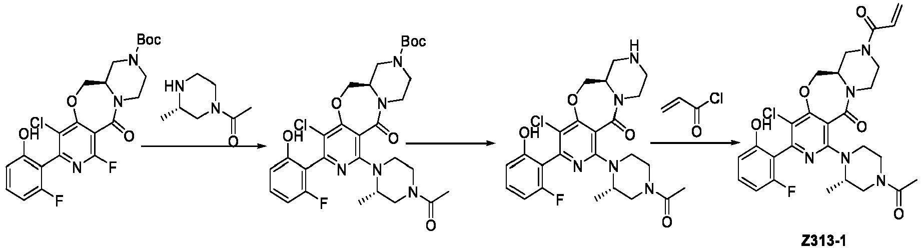

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (24 mg, 0.05 mmol), (S)-1-(3-methylpiperazin-1-yl)ethan-1-one (70 mg, 0.5 mmol), *N*,*N*-diisopropylethylamine (102 mg, 0.8 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-1-((*S*)-4-acetyl-2-methylpiperazin-1-yl)-4-chloro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (20 mg, yield of 66%). ES-API: [M+H]⁺=604.3.

Step 2: Trifluoroacetic acid (1 mL) was added to a solution of tert-butyl (6a*R*)-1-((*S*)-4-acetyl-2-methylpiperazin-1-yl)-4-chloro-3-(2-fluoro-6-hydroxyphenyl)-12-oxo-6a,7,9,10-tetrahydro-12H-pyrazino[2,1-*c*]pyrido[3,4-*f*][1,4]oxazepine-8(6*H*)-carboxylate (20 mg, 0.03 mmol) in dichloromethane (2 mL), reacted at room temperature for 1 hour. The reaction solution was directly concentrated to obtain a crude product of (6a*R*)-1-((*S*)-4-acetyl-2-methylpiperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (16 mg). ES-API: [M+H]⁺=504.3.

Step 3: The above crude procuct of (6a*R*)-4-chloro-1-((*S*)-4-cyclopropyl-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (16 mg, 0.03 mmol) was dissolved in dichloromethane (5 mL), and N,N-diisopropylethylamine (13 mg, 0.1 mmol) was added thereto. The reaction solution was cooled to 0°C, and acryloyl chloride (2 mg, 0.02 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-1-((*S*)-4-acetyl-2-methylpiperazin-1-yl)-8-acryloyl-4-chloro-3-(2-fluoro-6-hydroxyphenyl)-6,6a,7,8,9,10-hexahydro-12*H*-pyrazino[2,1-c]pyrido[3,4-f][1,4]oxazepin-12-one (Z313-1, 8.5 mg, yield: 50%). ES-API: [M+H]⁺=558.2.

### Embodiment 62-39 Synthesis of Z240

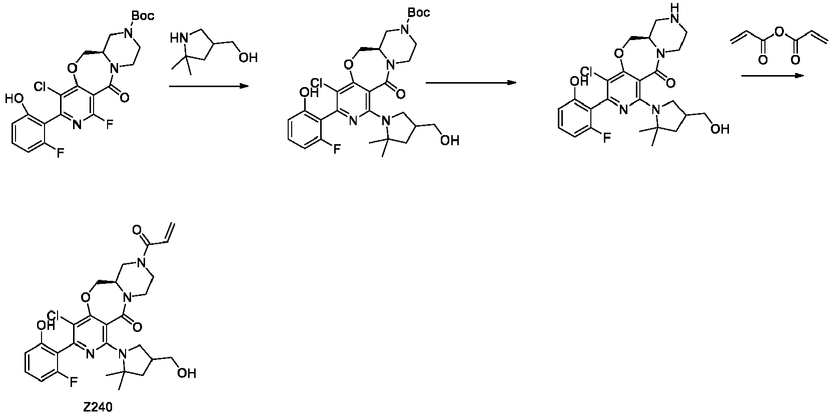

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (50 mg, 0.1 mmol) and 5 mL of acetonitrile were added to a round bottom flask. Sodium bicarbonate (29 mg, 0.35 mmol) and (5,5-dimethylpyrrolidin-3-yl)methanol (27 mg, 0.21 mmol) were then added to the reaction solution. The reaction was stirred at 80°C overnight. After the reaction was completed, the mixture was filtered to obtain a filtrate, and the filtrate was concentrated to dryness to obtain a crude product of *tert*-butyl (6a*R*)-4-chloro-3-(2-fluoro-6-hydroxyphenyl)-1-(4-(hydroxymethyl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (70 mg, crude product) as a yellow solid, and the crude product was directly used in the next reaction step. ES-API: [M+H]⁺=591.1.

Step 2: *tert*-Butyl (6a*R*)-4-chloro-3-(2-fluoro-6-hydroxyphenyl)-1-(4-(hydroxymethyl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (70 mg, 0.12 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-3-(2-fluoro-6-hydroxyphenyl)-1-(4-(hydroxymethyl)-2,2-dimethylpyrrolidin-1-yl)-6,6a,7,8,9,10-hexahydro-12*H*-pyrazino[2,1-*c*]pyrido[3,4-*f*][1,4]oxazepin-12-one (79 mg) as a brown oily liquid. The crude product was directly used in the next step. ES-API: [M+H]⁺=491.0.

Step 3: (6a*R*)-4-Chloro-3-(2-fluoro-6-hydroxyphenyl)-1-(4-(hydroxymethyl)-2,2-dimethylpyrrolidin-1-yl)-6,6a,7,8,9,10-hexahydro-12*H*-pyrazino[2,1-*c*]pyrido[3,4-*f*][1,4]oxazepin-12-one (79 mg, 0.17 mmol), 10 mL of dichloromethane and triethylamine (130 mg, 1.2 mmol) were added to around bottom flask. The reaction was cooled to 0°C, and a solution of acrylic anhydride in dichloromethane (12 mg, 0.10 mmol, 1 mL) was added dropwise to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-chloro-3-(2-fluoro-6-hydroxyphenyl)-1-(4-(hydroxymethyl)-2,2-dimethylpyrrolidin-1-yl)-6,6a,7,8,9,10-hexahydro-12*H*-pyrazino[2,1-*c*]pyrido[3,4-*f*][1,4]oxazepin-12-one (Z240, 64, 69%). ES-API: [M+H]⁺=545.1.

### Embodiment 62-40 Synthesis of Z315

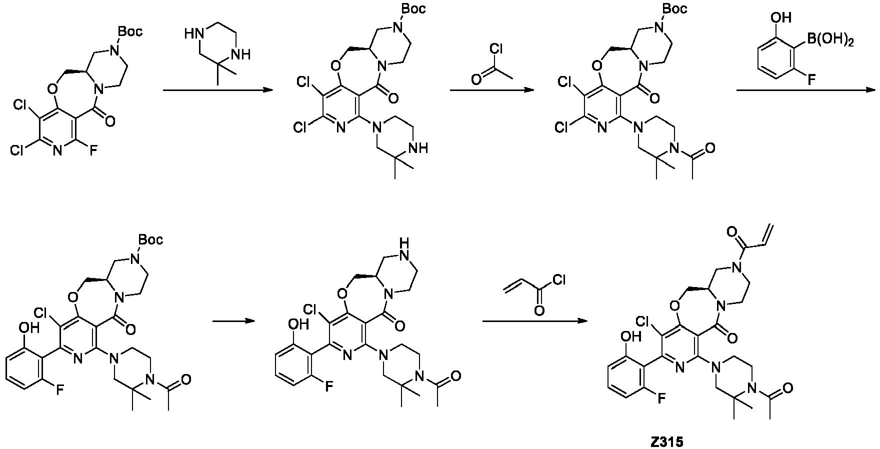

Step 1: A solution of compound *tert*-butyl (*R*)-3,4-dichloro-1-fluoro-12-oxo-6a,7,9,10-tetrahydro-12*H*-pyrazino[2,1-c]pyrido[3,4-f][1,4]oxazepine-8(6H)-carboxylate (100 mg, 0.25 mmol), 2,2-dimethylpiperazine (56 mg, 0.49 mmol) and *N*,*N*-diisopropylethylamine (100 mg, 0.74 mmol) in acetonitrile (2 mL) was stirred at 85°C for 3 hours. The reaction solution was filtered and concentrated to obtain a crude product of *tert*-butyl (6a*R*)-3,4-dichloro-1-(3,3-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg). ES-API: [M+H]⁺=500.2.

Step 2: Under an ice bath, a solution of compound *tert*-butyl (6a*R*)-3,4-dichloro-1-(3,3-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.40 mmol) in dichloromethane (4 mL) was added with *N,N-*diisopropylethylamine (155 mg, 1.20 mmol) and acetyl chloride (63 mg, 0.80 mmol) in turn, and stirred for 10 minutes. After the reaction was completed, the mixture was filtered, concentrated and purified by flash silica gel column (ethyl acetate:petroleum ether: 0-80%) to obtain a white solid of *tert*-butyl (*R*)-1-(4-acetyl-3,3-dimethylpiperazin-1-yl)-3,4-dichloro-12-oxo-6a,7,9,10-tetrahydro-12*H*-pyrazino[2,1-*c*]pyrido [3,4-f][1,4]oxazepine-8(6*H*)-carboxylate (120 mg). ES-API: [M+H]⁺=542.2.

Step 3: Under nitrogen protection, a mixed solution of compound tert-butyl (*R*)-1-(4-acetyl-3,3-dimethylpiperazin-1 -yl)-3,4-dichloro-12-oxo-6a,7,9,10-tetrahydro-12*H*-pyrazino[2,1-*c*]pyrido[3,4-*f*][1,4]oxazepine-8(6H)-carboxylate (120 mg, 0.22 mmol), 2-fluoro-6-hydroxyphenylboronic acid (103 mg, 0.66 mmol), tetrakis(triphenylphosphine)palladium (26 mg, 0.02 mmol) and potassium carbonate (92 mg, 0.66 mmol) in 1,4-dioxane (2 mL) and water (0.4 mL) was stirred at 110°C for 30 minutes. The reaction solution was poured into water (10 mL) and ethyl acetate (10 mL * 3), and the organic phases were combined, filtered, dried and purified by flash silica gel column (ethyl acetate:petroleum ether: 0-100%) to obtain *tert-*butyl (6a*R*)-1-(4-acetyl-3,3-dimethylpiperazin-1-yl)-4-chloro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (130 mg). ES-API: [M+H]⁺=618.1.

Step 4: Under an ice bath, a solution of *tert*-butyl (6a*R*)-1-(4-acetyl-3,3-dimethylpiperazin-1-yl)-4-chloro-3-(2-fluoro-6-hydroxyphenyl)-12-oxo-6a,7,9,10-tetrahydro-12*H*-pyrazino[2,1-*c*]pyrido [3,4-*f*][1,4]oxazepine-8(6*H*)-carboxylate (130 mg, 0.21 mmol) in dichloromethane (4 mL) was added with trifluoroacetic acid (1 mL), and the reaction solution was stirred at room temperature for 1 hour. The reaction solution was concentrated under reduced pressure to obtain a crude product of (6a*R*)-1-(4-acetyl-3,3-dimethylpiperazin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (109 mg). ES-API: [M+H]⁺=518.2.

Step 5: The above crude procuct of (6a*R*)-1-(4-acetyl-3,3-dimethylpiperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (109 mg, 0.21 mmol) was dissolved in dichloromethane (2 mL), and *N*,*N*-diisopropylethylamine (136 mg, 1.05 mmol) was added thereto. The reaction solution was cooled to 0°C, and acryloyl chloride (19 mg, 0.21 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-1-(4-acetyl-3,3-dimethylpiperazin -1-yl)-8-acryloyl-4-chloro-3 -(2-fluoro-6-hydroxyphenyl)-6,6a, 7,8,9,1 0-hexahydro-12*H-*pyrazino[2,1-*c*]pyrido[3,4-*f*][1,4]oxazepin-12-one (Z315, 57 mg, yield: 47%). ES-API: [M+H]⁺ =572.1. ¹H NMR (500 MHz, DMSO-6) δ 10.09 - 9.85 (m, 1H), 7.25 (dd, *J*= 15.5, 8.0 Hz, 1H), 6.85 - 6.65 (m, 3H), 6.17 (d, *J =* 16.5 Hz, 1H), 5.81 - 5.68 (m, 1H), 4.58 - 3.73 (m, 7H), 3.72 - 3.41 (m, 6H), 3.28 - 3.08 (m, 2H), 1.99 (s, 3H), 1.39 (s, 3H), 1.31 (d, *J*= 10.5 Hz, 3H).

### Embodiment 62-41 Synthesis of a mixture of Z291-2 and Z292-2

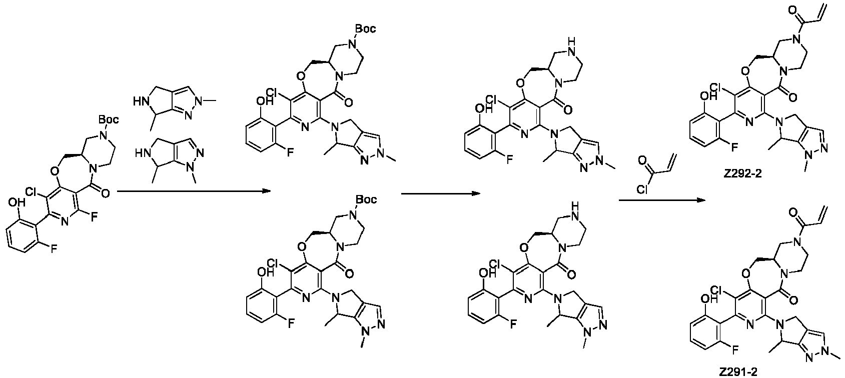

Step 1: (6a*R*)-8-*tert*-Butoxycarbonyl-3-(2-hydroxy-6-fluorophenyl)-4-chloro-1-fluoro-6,6a,7,8,9,10-hexahydro-12*H*-pyrazino[2,1-*c*]pyrido[3,4-*f*|[1,4]oxazepin-12-one (550 mg, 1.14 mmol), a mixture of 2,6-dimethyl-2,4,5,6-tetrahydropyrrolo[3,4-c]pyrazole and 1,6-dimethyl-1,4,5,6-tetrahydropyrrolo[3,4-c]pyrazole (188 mg, 1.37 mmol), *N*,*N*-diisopropylethylamine (441 mg, 3.42 mmol) and acetonitrile (10 mL) were added to a 100 mL round bottom flask, stirred at 100°C for 0.5 hours under microwave irradiation. The mixture was extracted with ethyl acetate, washed with saturated brine, and the organic phase was dried, concentrated, and purified by silica gel column (petroleum ether: ethyl acetate=10:90) to obtain a mixture (560 mg, yield of 82%) of (6a*R*)-8-*tert*-butoxycarbonyl-1,6-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H*-pyrazino [2,1-*c*]pyrido[3,4-*f*][1,4]oxazepin-12-one and (6a*R*)-8-tert-butoxycarbonyl-2,6-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino [2,1-*c*]pyrido[3,4-*f*][1,4]oxazepin-12-one. ES-API: [M+H]⁺=599.2.

Step 2: The above mixture (560 mg, 0.936 mmol), 5mL of trifluoroacetic acid and 20mL dichloromethane were stirred at room temperature for 1 hour, and the completion of reaction was detected by LC-MS. The reaction solution was concentrated to obtain a mixture (466 mg, crude product) of (6a*R*)-1-(1,6-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*|[1,4]oxazepin-12-one and (6a*R*)-1-(2,6-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one. ES-API: [M+H]⁺=499.2.

Step 3: The above mixture (466 mg, crude product), 10 mL of dichloromethane and *N,N-*diisopropylethylamine (362 mg, 2.8 mmol) were mixed. The reaction was cooled to 0°C, and a solution of acryloyl chloride in dichloromethane (85 mg, 0.936 mmol) was added dropwise to the reaction solution. The reaction was stirred at 0°C for 10 minutes. The mixture was washed with 20 mL of water, and the organic phase was dried and concentrated to obtain a crude product of a mixture (mixture of Z291-2 and Z292-2) of (6a*R*)-8-acryloyl-1-(1,6-dimethylpyrrolo[3,4-*c*]pyrazol-5(4*H*)-yl)-4-chloro-3-(2-fluoro-6-hydroxyphenyl)-6,6a,7,8,9,10-hexahydro-12*H*-pyrazino[2,1-c]pyrido [3,4-*f*][1,4]oxazepin-12-one and (6a*R*)-8-acryloyl-1-(2,6-dimethylpyrrolo[3,4-*c*]pyrazol-5(1*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one. ES-API: [M+H]⁺=553.1.

### Embodiment 62-42 Synthesis of Z316-1

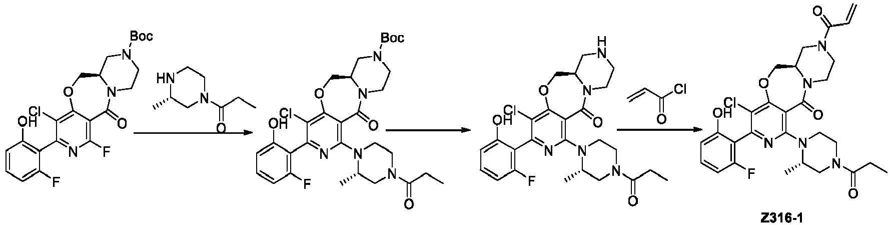

Step 1: te*r*t-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.41 mmol), (S)-1-(3-methylpiperazin-1-yl)propan-1-one (780 mg, 5 mmol), *N*,*N*-diisopropylethylamine (102 mg, 0.8 mmol) and acetonitrile (5 mL) were added to a 50 mL round bottom flask, reacted at 80°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-1-((*S*)-4-propionyl-2-methylpiperazin-1-yl)-4-chloro-3-(2-fluoro-6-hydroxyphenyl)-12-oxo-6a,7,9,10-tetrahydro-12*H*-pyrazino[2,1-c]pyrido[3,4-*f*][1,4]oxazepine-8(6*H*)-carboxylate (150 mg, yield of 60%). ES-API: [M+H]⁺=618.2.

Step 2: Trifluoroacetic acid (2 mL) was added to a solution of tert-butyl (6aR)-1-((S)-4-propionyl-2-methylpiperazin-1-yl)-4-chloro-3-(2-fluoro-6-hydroxyphenyl)-12-oxo-6a,7,9,10-tetrahydro-12H-pyrazino[2,1-*c*]pyrido[3,4-*f*][1,4]oxazepine-8(6*H*)-carboxylate (150 mg, 0.24 mmol) in dichloromethane (5 mL), reacted at room temperature for 1 hour. The reaction solution was directly concentrated to obtain a crude product of (6a*R*)-1-((*S*)-4-propionyl-2-methylpiperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (120 mg). ES-API: [M+H]⁺=518.2.

Step 3: The above crude procuct of (6a*R*)-4-chloro-1-((*S*)-4-cyclopropyl-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (120 mg, 0.23 mmol) was dissolved in dichloromethane (5 mL), and *N*,*N*-diisopropylethylamine (90 mg, 0.7 mmol) was added thereto. The reaction solution was cooled to 0°C, and acryloyl chloride (18 mg, 0.20 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-((*S*)-2-methyl-4-propionylpiperazin-1-yl)-6,6a,7,8,9,10-hexahydro-12*H*-pyrazino[2,1-*c*]pyrido[3,4-*f*][1,4]oxazepin-12-one (Z316-1, 79 mg, yield: 59%). ES-API: [M+H]⁺=572.2. ¹H NMR (500 MHz, DMSO-*d*₆) δ 9.99 (d, *J=* 24.9 Hz, 1H), 7.25 (dd, *J* = 15.6, 7.8 Hz, 1H), 6.85-6.61 (m, 3H), 6.18 (d, *J*= 16.7 Hz, 1H), 5.76 (d, *J*= 10.8 Hz, 1H), 4.55 - 3.33 (m, 14H), 3.20-2.75 (m, 2H), 2.43 - 2.18 (m, 2H), 1.2-0.91 (m, 6H).

### Embodiment 62-43 Synthesis of Z317-1

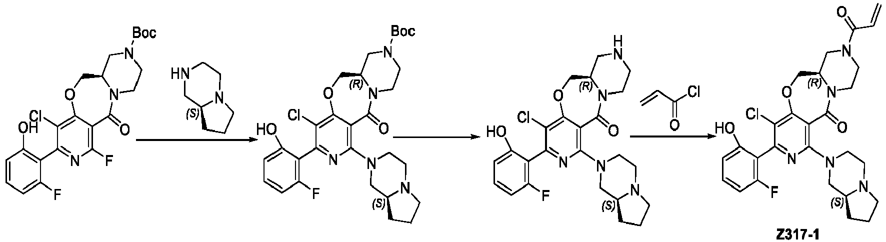

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (100 mg, 0.21 mmol), (S)-octahydropyrrolo[1,2-*a*]pyrazine (105 mg, 0.8 mmol), *N*,*N*-diisopropylethylamine (80 mg, 0.62 mmol) and acetonitrile (5 mL) were added to a 50 mL round bottom flask, reacted at 90°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-1-((*S*)-hexahydropyrrolo[1,2-*a*]pyrazin-2(1*H*)-yl)-12-oxo-6a,7,9,10-tetrahydro-12*H*-pyrazino[2,1-*c*]pyrido[3,4-*f*][1,4]oxazepine-8(6*H*)-carboxylate (80 mg, yield of 81%). ES-API: [M+H]⁺=588.2.

Step 2: Trifluoroacetic acid (2 mL) was added to a solution of tert-butyl (6aR)-4-chloro-3-(2-fluoro-6-hydroxyphenyl)-1-((*S*)-hexahydropyrrolo[1,2-*a*]pyrazin-2(1*H*)-yl)-12-oxo-6a,7,9,10-tetrahydro-12*H*-pyrazino[2,1-*c*]pyrido[3,4-*f*][1,4]oxazepine-8(6*H*)-carboxylate (80 mg, 0.14 mmol) in dichloromethane (5 mL), reacted at room temperature for 1 hour. The reaction solution was directly concentrated to obtain a crude procuct of (6a*R*)-4-chloro-3-(2-fluoro-6-hydroxyphenyl)-1-((*S*)-hexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (61 mg). ES-API: [M+H]⁺=488.2.

Step 3: The above crude product of (6aR)-4-chloro-3-(2-fluoro-6-hydroxyphenyl)-1-((S)-hexahydropyrrolo[1,2-a]pyrazin-2(1H)-yl)-6,6a,7,8,9,10-hexahydro-12H-pyrazino[2,1-c]pyrido[3,4-f][1,4]oxazepin-12-one (61 mg, 0.13 mmol) was dissolved in dichloromethane (5 mL), and *N,N-*diisopropylethylamine (51 mg, 0.4 mmol) was added thereto. The reaction solution was cooled to 0°C, and acryloyl chloride (11 mg, 0.12 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-((*S*)-hexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (Z317-1, 37 mg, yield: 53%). ES-API: [M+H]⁺=542.2. ¹H NMR (500 MHz, DMSO-d₆) δ 9.98 (d, *J*= 5.5 Hz, 1H), 7.25 (d, *J*= 7.8 Hz, 1H), 6.84 - 6.60 (m, 3H), 6.17 (d, *J*= 16.7 Hz, 1H), 5.75 (d, *J*= 10.9 Hz, 1H), 4.50-3.30 (s, 12H), 3.08 - 2.85 (m, 3H), 2.59 (dd, *J*= 25.6, 14.4 Hz, 1H), 2.02 (d, *J=* 8.5 Hz, 3H), 1.68 (dd, *J*= 19.3, 9.8 Hz, 3H).

### Embodiment 62-44 Synthesis of Z256b

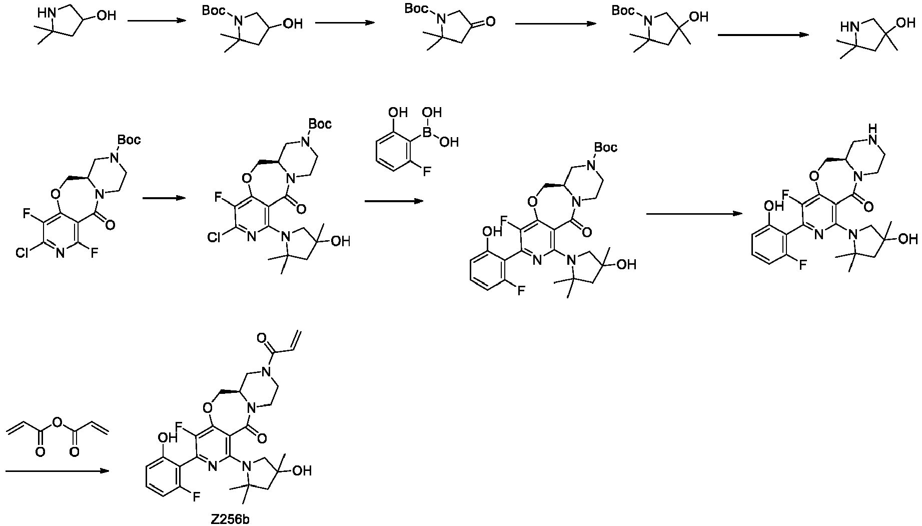

Step 1: 5,5-Dimethylpyrrolidin-3-ol (600mg, 5.2 mmol), di-tert-butyl dicarbonate (2.27g, 10.4 mmol), sodium carbonate (1.65 g, 15.6 mmol), tetrahydrofuran (10 mL) and water (10 mL) were added to a round bottom flask. The reaction was stirred at room temperature overnight. The reaction solution was added with 20 mL of water and extracted with ethyl acetate. The organic phase was dried and concentrated, and the crude product was purified by flash silica gel column (ethyl acetate/petroleum ether: 0-50%) to obtain a product of tert-butyl 4-hydroxy-2,2-dimethylpyrrolidine-1-carboxylate (900 mg, 81%) as a white solid.

Step 2: tert-Butyl 4-hydroxy-2,2-dimethylpyrrolidine-1-carboxylate (500 mg, 2.33 mmol) and dichloromethane (20 mL) were added to a round bottom flask, and Dess-Martin periodinane (1.97 g, 4.66 mmol) was added thereto under an ice-water bath. The reaction was stirred at room temperature overnight. The reaction solution was added with 20 mL of saturated sodium thiosulfate solution, extracted three times with ethyl acetate, and the organic phase was washed once with saturated sodium bicarbonate solution. The organic phase was dried and concentrated, and the crude product was purified by flash silica gel column (ethyl acetate/petroleum ether: 0-30%) to obtain a product of *tert*-butyl 2,2-dimethyl-4-oxopyrrolidine-1-carboxylate (311 mg, 63%) as a white solid.

Step 3: *tert*-Butyl 2,2-dimethyl-4-oxopyrrolidine-1-carboxylate (311 mg, 1.46 mmol) and anhydrous tetrahydrofuran (20 mL) were added to a round bottom flask, and methylmagnesium bromide (1.46 mL, 4.38 mmol, 3 M) was added thereto under an ice-water bath under nitrogen protection. The reaction was stirred at room temperature overnight. 10 mL of saturated ammonium chloride solution was added dropwise to the reaction solution under an ice-water bath and extracted three times with ethyl acetate. The organic phase was dried and concentrated, and the crude product was purified by flash silica gel column (ethyl acetate/petroleum ether: 0-50%) to obtain a product of *tert*-butyl 4-hydroxy-2,2,4-trimethylpyrrolidine-1-carboxylate (330 mg, 98%) as a white solid.

Step 4: *tert*-Butyl 4-hydroxy-2,2,4-trimethylpyrrolidine-1-carboxylate (330 mg, 1.44 mmol), 3 mL of dichloromethane and 3 mL of trifluoroacetic acid were added to a round bottom flask. The mixture was stirred at room temperature for 1 hour. The reaction solution was concentrated to obtain 3,5,5-trimethylpyrrolidin-3-ol (423 mg, crude product), and the crude product was directly used in the next reaction step.

Step 5: *tert*-Butyl (*R*)-3-chloro-1,4-difluoro-12-oxo-6a,7,9,10-tetrahydro-12*H*-pyrazino[2,1-*c*]pyrido[3,4-*f*][1,4]oxazepine-8(6*H*)-carboxylate (50 mg, 0.1 mmol) and 5 mL of acetonitrile were added to a round bottom flask. Sodium bicarbonate (29 mg, 0.35 mmol) and 3,5,5-trimethylpyrrolidin-3-ol (27 mg, 0.21 mmol) were then added to the reaction solution. The reaction was stirred at 80°C overnight. After the reaction was completed, the mixture was filtered to obtain a filtrate, and the filtrate was concentrated to dryness to obtain a crude product of tert-butyl (6a*R*)-3-chloro-4-fluoro-1-(4-hydroxy-2,2,4-trimethylpyrrolidin-1-yl)-12-oxo-6a,7,9,10-tetrahydro-12*H*-pyrazino[2,1-*c*]pyrido[3,4-*f*][1,4]oxazepine-8(6H)-carboxylate (72 mg, crude product) as a yellow solid, and the crude product was directly used in the next reaction step. ES-API: [M+H]⁺=499.1.

Step 6: Under nitrogen protection, *tert*-butyl (6a*R*)-3-chloro-4-fluoro-1-(4-hydroxy-2,2,4-trimethylpyrrolidin-1-yl)-12-oxo-6a,7,9,10-tetrahydro-12*H*-pyrazino[2,1-*c*]pyrido[3,4-*f*][1,4]oxazepine-8(6H)-carboxylate (72 mg, 0.14 mmol), (2-fluoro-6-hydroxyphenyl)boronic acid (66 mg, 0.42 mmol), 2-dicyclohexylphosphino-2',6'-dimethoxy-biphenyl (11 mg, 0.026 mmol) and chloro(2-dicyclohexylphosphoryl-2',6'-dimethoxy-1,1'-biphenyl)(2'-amino-1,1'-biphenyl-2-yl)palladium II (19 mg, 0.026 mmol) and potassium carbonate (58 mg, 0.42 mmol) were added to around bottom flask, dissolved in 8 mL of dioxane and 2 mL of water. The reaction mixture was reacted at 110°C for 1.5 hours under microwave irradiation. The completion of reaction was detected by LC-MS, and the reaction solution was concentrated to dryness under reduced pressure, and the crude product was separated by column chromatography (petroleum ether:ethyl acetate=5:1) to obtain a product of *tert*-butyl (6a*R*)-4-fluoro-3-(2-fluoro-6-hydroxyphenyl)-1-(4-hydroxy-2,2,4-trimethylpyrrolidin-1-yl)-12-oxo-6a,7,9,10-tetrahydro-12H-pyrazino[2,1-*c*]pyrido[3,4-*f*][1,4]oxazepine-8(6*H*)-carboxylate (65 mg, 81%). ES-API: [M+H]⁺=575.1.

Step 7: *tert*-Butyl (6a*R*)-4-fluoro-3-(2-fluoro-6-hydroxyphenyl)-1-(4-hydroxy-2,2,4-trimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (65 mg, 0.11 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-fluoro-3-(2-fluoro-6-hydroxyphenyl)-1-(4-hydroxy-2,2,4-trimethylpyrrolidin-1-yl)-6,6a,7,8,9,10-hexahydro-12*H-*pyrazino[2,1-*c*]pyrido[3,4-*f*][1,4]oxazepin-12-one (79 mg) as a brown oily liquid. The crude product was directly used in the next step. ES-API: [M+H]⁺=475.0.

Step 8: (6a*R*)-4-Fluoro-3-(2-fluoro-6-hydroxyphenyl)-1-(4-hydroxy-2,2,4-trimethylpyrrolidin-1-yl)-6,6a,7,8,9,10-hexahydro-12H-pyrazino[2,1-*c*]pyrido[3,4*f*][1,4]oxazepin-12-one (79 mg, 0.17 mmol), 10 mL of dichloromethane and triethylamine (130 mg, 1.2 mmol) were added to around bottom flask. The reaction was cooled to 0°C, and a solution of acrylic anhydride in dichloromethane (12 mg, 0.10 mmol, 1 mL) was added dropwise to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-4-fluoro-3-(2-fluoro-6-hydroxyphenyl)-1-4-hydroxy-2,2,4-trimethylpyrrolidin-1-yl)-6,6a,7,8,9,10-hexahydro-12*H*-pyrazino [2,1-*c*]pyrido[3,4-f][1,4]oxazepin-12-one (Z256b, 68 mg, 76%). ES-API: [M+H]⁺=529.2.

### Embodiment 62-45 Synthesis of Z318-1

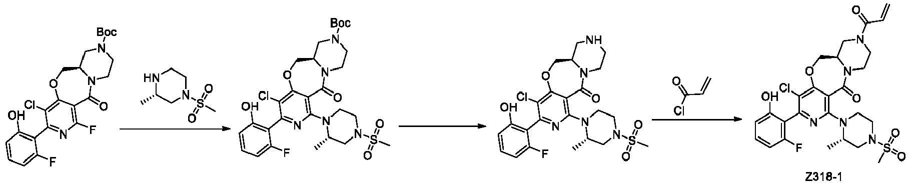

Step 1: (6a*R*)-8-*tert*-Butoxycarbonyl-3-(2-hydroxy-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (190 mg, 0.395 mmol), (S)-2-methyl-4-(methylsulfonyl)piperazine (140 mg, 0.79 mmol), *N*,*N*-diisopropylethylamine (100 mg, 1.185 mmol) and acetonitrile (10 mL) were added to a 100 mL round bottom flask, stirred at 100°C for 2 hours under microwave irradiation. The mixture was extracted with ethyl acetate, washed with saturated brine, and the organic phase was dried, concentrated, and purified by silica gel column (petroleum ether:ethyl acetate = 10:90) to obtain (6a*R*)-8-*tert*-butoxycarbonyl-1-((*S*)-2-methyl-4-(methylsulfonyl)piperazin-1-yl)-4-chloro-3-(2-fluoro-6-hydroxyphenyl)-6,6a,7,8,9,10-hexahydro-12*H*-pyrazino [2,1-c]pyrido [3,4-*f*][1,4]oxazepin-12-one (90 mg, yield of 35%). ES-API: [M+H]⁺=640.2.

Step 2: (6a*R*)-8-*tert*-Butoxycarbonyl-1-((*S*)-2-methyl-4-(methylsulfonyl)piperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (90 mg, 0.141 mmol), 3 mL of trifluoroacetic acid and 5 mL of dichloromethane were added to a 50 mL round bottom flask. The mixture was stirred at room temperature for 1 hour, and the completion of reaction was detected by LC-MS. The reaction solution was concentrated to obtain a crude product of (6aR)-1-((*S*)-2-methyl-4-(methylsulfonyl)piperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (76 mg, crude product). ES-API: [M+H]⁺=540.2.

Step 3: (6a*R*)-1-((*S*)-2-Methyl-4-(methylsulfonyl)piperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (76 mg, crude product), 10 mL of dichloromethane and *N*,*N*-diisopropylethylamine (55 mg, 0.423 mmol) were added to a 50 mL round bottom flask. The reaction was cooled to 0°C, and a solution of acryloyl chloride in dichloromethane (13 mg, 0.141 mmol) was added dropwise to the reaction solution. The reaction was stirred at 0°C for 10 minutes. The mixture was washed with 10 mL of water, and the organic phase was dried, then concentrated, and purified by preparative HPLC (column: C18 19*150 mm 5 µm, mobile phase: ACN:HzO=50:50 (0.1% NH₄HCO₃), flow rate: 15 mL/min, column temperature: 25°C) to obtain a target product of (6a*R*)-8-acryloyl-1-((*S*)-2-methyl-4-(methylsulfonyl)piperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z318-1, 30 mg, 3-step yield of 50%). ES-API: [M+H]⁺= 594.1. ¹H NMR (500 MHz, DMSO-d₆) δ 9.97 (s, 1H), 7.25 (q, *J*= 7.7 Hz, 1H), 6.79-6.67 (m, 3H), 6.17 (d, *J*= 16.7 Hz, 1H), 5.75 (d, *J*= 10.8 Hz, 1H), 4.50-4.30 (m, 2H), 4.23-4.20 (m, 2H), 4.10-4.05 (m, 3H), 3.81 (s, 1H), 3.66-3.60 (m, 3H), 3.45 (d, *J*= 11.3 Hz, 1H), 3.29-3.23 (m, 3H), 2.96-2.77 (m, 5H), 1.24 (t, *J*= 7.1 Hz, 3H). ES-API: [M+H]⁺=594.1

### Embodiment 62-46 Synthesis of Z319-1

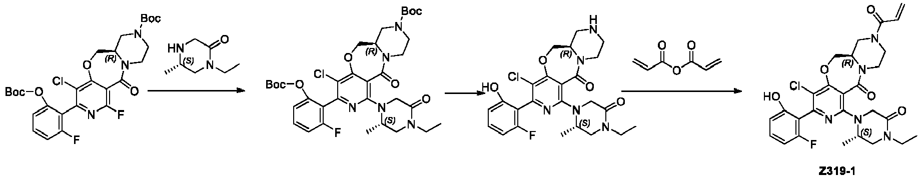

Step 1: Compound *tert*-butyl (6a*R*)-3-(2-((tert-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepin-12-one (200 mg, 0.34 mmol), (S)-1-ethyl-5-methylpiperazin-2-one (61 mg, 0.34 mmol) and potassium carbonate (141 mg, 1.02 mmol) were dissolved in acetonitrile (6 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of (6a*R*)-*tert*-butyl 3-(2-((*tert*-butoxycarbonyl)oxy)-6-fluorophenyl)-4-chloro-1-((*S*)-4-ethyl-2-methyl-5-oxopiperazine-12-oxo-6a,7,9,10-tetrahydro-12H-pyrazino[2,1-*c*]pyrido[3,4-*f*][1,4]oxazepin-12-one (100 mg, 41.8%) as a yellow solid. ES-API: [M+H]⁺= 704.4.

Step 2: (6aR)-tert-Butyl 3-(2-((tert-butoxycarbonyl)oxy)-6-fluorophenyl)-4-chloro-1-((*S*)-4-ethyl-2-methyl-5-oxopiperazine-12-oxo-6a,7,9,10-tetrahydro-12*H*-pyrazino[2,1-c]pyrido[3,4-f][1,4]oxazepin-12-one (100 mg, 0.14 mmol) was dissolved in 2 mL of dichloromethane, and trifluoroacetic acid (2 mL) was added thereto, and the reaction solution was stirred at room temperature for 2 hours. After the reaction was monitored to be complete, the mixture was diluted with dichloromethane, washed with water, washed with saturated sodium bicarbonate and brine once respectively, dried over anhydrous sodium sulfate, and the filtrate was evaporated to dryness by rotary evaporation to obtain compound (6a*R*)-4-chloro-1 -((S)-4-ethyl-2-methyl-5 -oxopiperazin-1-yl)-3-(2-fluoro-6-hydroxyphenyl)-7,8,9,10-tetrahydro-6H-pyrazino[2,1-c]pyrido[3,4-f][1,4]oxal-12(6aH)-one (100 mg, yield: 100%).

Step 3: (6a*R*)-4-Chloro-1-((*S*)-4-ethyl-2-methyl-5-oxopiperazin-1-yl)-3-(2-fluoro-6-hydroxyphenyl)-7,8,9,10-tetrahydro-6*H*-pyrazino[2,1-*c*]pyrido[3,4-*f*][1,4]oxal-12(6a*H*)-one (100 mg, 0.14 mmol) was dissolved in dichloromethane (3 mL), then cooled to 0°C in an ice-water bath, and triethylamine (2 mL) was added dropwise thereto. The pH value was basic, and acrylic anhydride (11 mg, 0.084 mmol) was added dropwise thereto, stirred for ten minutes. After the reaction was completed, the mixture was diluted with dichloromethane, washed with water, dried over sodium sulfate, evaporated to dryness by rotary evaporation and the crude product was prepared and purified (Ultimate XB-C18, 50*250 mm, 10 µm, mobile phase system: A: purified water B: pure acetonitrile, flow rate: 80 mL/min, gradient: within 50 minutes, B/A=20%-90%, wavelength: 214 nm, column temperature: room temperature) to obtain a product of (6a*R*)-8-acryloyl-4-chloro-1-((*S*)-4-ethyl-2-methyl-5-oxopiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z319-1, 14.1 mg, yield: 17.9%, white solid). ¹H NMR (400 MHz, CDCl₃) δ 8.69 (s, 1H), 7.30 (d, *J*= 8.4 Hz, 1H), 6.83 (d, *J=* 8.2 Hz, 1H), 6.71 (t, *J*= 9.1 Hz, 1H), 6.56 (d, *J*= 12.9 Hz, 1H), 6.40 (d, *J*= 16.5 Hz, 1H), 5.81 (d, *J*= 10.2 Hz, 1H), 4.79 (s, 1H), 4.30 (dd, *J =* 33.0, 15.3 Hz, 5H), 4.04 (d, *J*= 60.2 Hz, 2H), 3.90 - 3.35 (m, 7H), 3.05 (d, *J*= 12.5 Hz, 1H), 1.37 - 1.27 (m, 3H), 1.17 (d, *J*= 7.2 Hz, 3H). ES-API: [M+H]⁺= 558.2.

### Embodiment 62-47 Synthesis of Z317-2

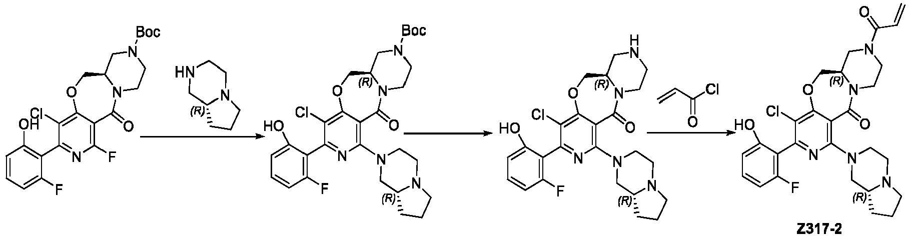

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (100 mg, 0.21 mmol), (R)-octahydropyrrolo[1,2-*a*]pyrazine (105 mg, 0.8 mmol), *N*,*N*-diisopropylethylamine (80 mg, 0.62 mmol) and acetonitrile (5 mL) were added to a 50 mL round bottom flask, reacted at 90°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-1-((R)-hexahydropyrrolo[1,2-*a*]pyrazin-2(1*H*)-yl)-12-oxo-6a,7,9,10-tetrahydro-12*H*-pyrazino[2,1-*c*]pyrido[3,4-*f*][1,4]oxazepine-8(6*H*)-carboxylate (77 mg, yield of 62%). ES-API: [M+H]⁺=588.2.

Step 2: Trifluoroacetic acid (2 mL) was added to a solution of *tert*-butyl (6a*R*)-4-chloro-3-(2-fluoro-6-hydroxyphenyl)-1-((*R*)-hexahydropyrrolo[1,2-*a*]pyrazin-2(1*H*)-yl)-12-oxo-6a,7,9,10-tetrahydro-12*H*-pyrazino[2,1-*c*]pyrido[3,4-*f*][1,4]oxazepine-8(6*H*)-carboxylate (77mg, 0.13 mmol) in dichloromethane (5 mL), reacted at room temperature for 1 hour. The reaction solution was directly concentrated to obtain a crude procuct of (6a*R*)-4-chloro-3-(2-fluoro-6-hydroxyphenyl)-1-((*R*)-hexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (66 mg). ES-API: [M+H]⁺=488.2.

Step 3: The above crude product of (6a*R*)-4-chloro-3-(2-fluoro-6-hydroxyphenyl)-1-((*R*)-hexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (66 mg, 0.14 mmol) was dissolved in dichloromethane (5 mL), and *N,N-*diisopropylethylamine (64 mg, 0.5 mmol) was added thereto. The reaction solution was cooled to 0°C, and acryloyl chloride (12 mg, 0.13 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-((*R*)-hexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (Z317-2, 40 mg, yield: 53%). ES-API: [M+H]⁺=542.2. ¹H NMR (500 MHz, DMSO-d₆) δ 9.98 (s, 1H), 7.25 (d, *J=* 7.5 Hz, 1H), 6.85 - 6.62 (m, 3H), 6.17 (d, *J =* 16.8 Hz, 1H), 5.75 (d, *J =* 11.2 Hz, 1H), 4.51 - 3.33 (m, 14H), 2.93 (dd, *J*= 26.3, 11.6 Hz, 3H), 2.71-2.55 (m, 1H), 2.24 - 1.52 (m, 6H).

### Embodiment 62-48 Synthesis of Z320-1

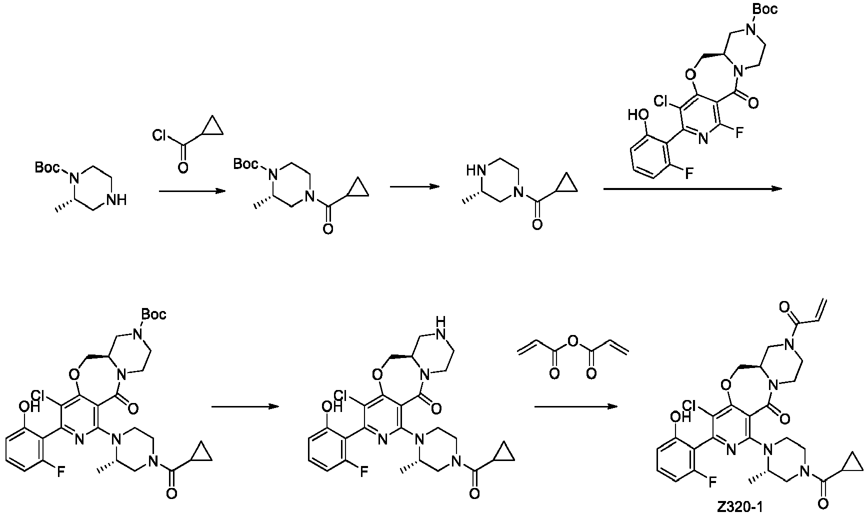

Step 1: *tert*-Butyl (*S*)-2-methylpiperazine-1-carboxylate (500 mg, 2.5 mmol), 20 mL of dichloromethane and diisopropylethylamine (1 mL) were added to a round bottom flask. The reaction was cooled to 0°C, and a solution of cyclopropanecarbonyl chloride in dichloromethane (390 mg, 3.75 mmol, 5 mL) was added dropwise to the reaction solution. The reaction was stirred at 0°C for 10 minutes. 40 mL of saturated aqueous sodium bicarbonate solution was added to the reaction solution, and the mixture was extracted three times with 20 mL of dichloromethane. The organic phase was dried and concentrated to obtain a crude product of *tert*-butyl (*S*)-4-(cyclopropanecarbonyl)-2-methylpiperazine-1-carboxylate (1.01 g, crude product), which was directly used in the next reaction step. ES-API: [M+H]⁺=213.2.

Step 2: *tert*-Butyl (*S*)-4-(cyclopropanecarbonyl)-2-methylpiperazine-1-carboxylate (1.01 g, crude product), 6 mL of dichloromethane and 6 mL of trifluoroacetic acid were added to a round bottom flask. The mixture was stirred at room temperature for 1 hour, and the completion of reaction was detected by LC-MS. The reaction solution was concentrated to obtain (*S*)-cyclopropyl(3-methylpiperazin-1-yl)methanone (1.31 g, crude product), and the crude product was directly used in the next reaction step.

Step 3: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (100 mg, 0.2 mmol) and 5 mL of acetonitrile were added to a round bottom flask. Sodium bicarbonate (60 mg, 0.7 mmol) and (S)-cyclopropyl(3-methylpiperazin-1-yl)methanone (70 mg, 0.42 mmol) were then added to the reaction solution. The reaction was stirred at 80°C overnight. After the reaction was completed, the mixture was filtered to obtain a filtrate, and the filtrate was concentrated to dryness to obtain a crude product of *tert*-butyl (6a*R*)-4-chloro-1-((*S*)-4-(cyclopropanecarbonyl)-2-methylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (151 mg, crude product) as a yellow solid, and the crude product was directly used in the next reaction step. ES-API: [M+H]⁺=630.1.

Step 4: *tert*-Butyl (6a*R*)-4-chloro-1-((*S*)-4-(cyclopropanecarbonyl)-2-methylpiperazin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (151 mg, 0.24 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-1-((*S*)-4-(cyclopropanecarbonyl)-2-methylpiperazin-1-yl)-3-(2-fluoro-6-hydroxyphenyl)-6,6a,7,8,9,10-hexahydro-12*H*-pyrazino[2.1-*c*]pyrido[3.4-*f*][1,4]oxazepin-12-one (181 mg) as a brown oily liquid. The crude product was directly used in the next step. ES-API: [M+H]⁺=530.0.

Step 5: (6a*R*)-4-Chloro-1-((*S*)-4-(cyclopropanecarbonyl)-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (181 mg, 0.34 mmol), 10 mL of dichloromethane and triethylamine (303 mg, 3.0 mmol) were added to around bottom flask. The reaction was cooled to 0°C, and a solution of acrylic anhydride in dichloromethane (38 mg, 0.3 mmol, 1 mL) was added to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain compound (6a*R*)-8-acryloyl-4-chloro-1-((*S-*4-(cyclopropanecarbonyl)-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z320-1, 90 mg, 44%). ES-API: [M+H]⁺=584.2. ¹H NMR (500 MHz, DMSO-d₆) δ 9.99 (d, *J=* 26.2 Hz, 1H), 7.25 (dd, *J=* 15.6, 7.7 Hz, 1H), 6.73 (ddd, *J*= 24.6, 17.0, 8.3 Hz, 3H), 6.18 (d, *J*= 16.6 Hz, 1H), 5.75 (d, *J*= 10.3 Hz, 1H), 4.47-4.32 (m, 2H), 4.28 - 3.92 (m, 6H), 3.86-3.79 (m, 1H), 3.73-3.37 (m, 4H), 3.29-3.26 (m, 1H), 3.19-3.08 (m, 1H), 2.89 (s, 1H), 1.97-1.90 (m, 1H), 1.18-1.10 (m, 3H), 0.74 (d, *J=* 14.2 Hz, 4H).

### Embodiment 62-49 Synthesis of Z317a

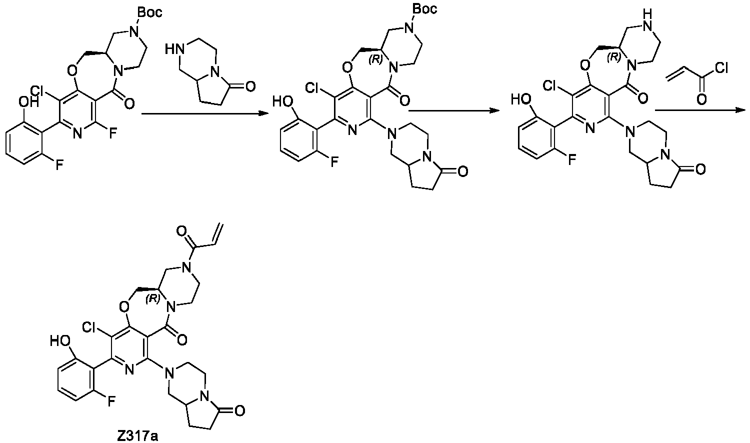

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (130 mg, 0.27 mmol), hexahydropyrrolo[1,2-*a*]pyrazin-6(2*H*)-one (116 mg, 1.0 mmol), N,N-diisopropylethylamine (103 mg, 0.80 mmol) and acetonitrile (5 mL) were added to a 50 mL round bottom flask, reacted at 90°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-12-oxo-1-(6-oxohexahydropyrrolo[1,2-*a*]pyrazin-2(1*H*)-yl)-6a,7,9,10-tetrahydro-12*H*-pyrazino[2,1-*c*]pyrido[3,4-*f*][1,4]oxazepine-8(6*H*)-carboxylate (85 mg, yield of 52%). ES-API: [M+H]⁺=602.2.

Step 2: Trifluoroacetic acid (2 mL) was added to a solution of *tert*-butyl (6a*R*)-4-chloro-3-(2-fluoro-6-hydroxyphenyl)-12-oxo-1-(6-oxohexahydropyrrolo[1,2-*a*]pyrazin-2(1*H*)-yl)-6a,7,9,10-tetrahydro-12*H*-pyrazino[2,1-*c*]pyrido[3,4*f*][1,4]oxazepine-8(6*H*)-carboxylate (85 mg, 0.14 mmol) in dichloromethane (5 mL), reacted at room temperature for 1 hour. The reaction solution was directly concentrated to obtain a crude procuct of (6a*R*)-4-chloro-3-(2-fluoro-6-hydroxyphenyl)-1-(6-oxohexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (70 mg). ES-API: [M+H]⁺=502.2.

Step 3: The above crude product of (6a*R*)-4-chloro-3-(2-fluoro-6-hydroxyphenyl)-1-((*R*)-hexahydropyrrolo[1,2-*a*]pyrazin-2(1*H*)-yl)-6,6a,7,8,9,10-hexahydro-12*H*-pyrazino[2,1-*c*]pyrido[3,4-f][1,4]oxazepin-12-one (70 mg, 0.14 mmol) was dissolved in dichloromethane (5 mL), and *N,N-*diisopropylethylamine (64 mg, 0.5 mmol) was added thereto. The reaction solution was cooled to 0°C, and acryloyl chloride (12 mg, 0.13 mmol) was added dropwise thereto. After stirring for 5 minutes, the mixture was added with methanol (1 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(6-oxohexahydropyrrolo[1,2-a]pyrazin-2(1*H*)-yl)-6,6a,7,8,9,10-hexahydro-12*H*-pyrazino[2,1-c]pyrido[3,4-f][1,4]oxazepin-12-one (Z317a, 40 mg, yield: 51%). ES-API: [M+H]⁺=556.2.

### Embodiment 62-50 Synthesis of Z321

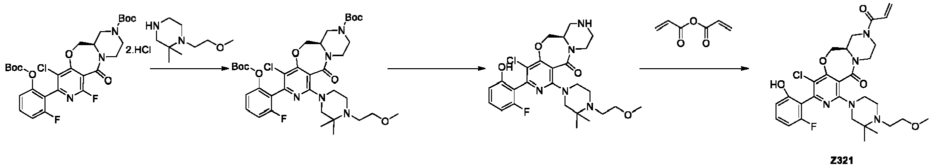

Step 1: Compound tert-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (200 mg, 0.34 mmol), 1-(2-methoxyethyl)-2,2-dimethylpiperazine hydrochloride (92 mg, 0.37 mmol) and potassium carbonate (234 mg, 0.1.7 mmol) were dissolved in acetonitrile (2 mL), reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert-*butoxycarbonyl)oxy)-6-fluorophenyl)-4-chloro-1-(4-(2-methoxyethyl)-3,3-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (250 mg, crude product). ES-API: [M+H]⁺= 734.3.

Step 2: *tert*-Butyl (6a*R*)-3-(2-((tert-butoxycarbonyl)oxy)-6-fluorophenyl)-4-chloro-1-(4-(2-methoxyethyl)-3,3 -dimethylpiperazin-1-yl)-12-oxo-6a,7,9,10-tetrahydro-12*H*-pyrazino[2,1-*c*]pyrido [3,4-*f*] [1,4]oxazepine-8(6*H*)-carboxylate (250 mg, crude product) was dissolved in dichloromethane (4 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-3-(2-fluoro-6-hydroxyphenyl)-1-(4-(2-methoxyethyl)-3,3-dimethylpiperazin-1-yl)-6,6a,7,8,9,10-hexahydro-12*H*-pyrazino[2,1-*c*]pyrido[3,4-*f*][1,4]oxazepin-12-one (180 mg, crude product). ES-API: [M+H]⁺= 534.3.

Step 3: (6a*R*)-4-Chloro-3-(2-fluoro-6-hydroxyphenyl)-1-(4-(2-methoxyethyl)-3,3-dimethylpiperazin-1-yl)-6,6a,7,8,9,10-hexahydro-12*H*-pyrazino[2,1-*c*]pyrido[3,4-*f*][1,4]oxazepin-12-one (180 mg, 0.34 mmol) was dissolved in dichloromethane (4 mL), then cooled to 0°C in an ice-water bath, and triethylamine (2 mL) was added dropwise thereto. The pH value was basic, and acrylic anhydride (34 mg, 0.27 mmol) was added dropwise thereto, stirred for 10 minutes. After the reaction was completed, the mixture was extracted with ethyl acetate, washed with water, washed with brine, dried over sodium sulfate, evaporated to dryness by rotary evaporation and purified twice with chromatography plate (dichloromethane/methanol=10/l, R_{f}=0.3) to obtain (6a*R*)-8-acryloyl-4-chloro-3-(2-fluoro-6-hydroxyphenyl)-1-(4-(2-methoxyethyl)-3,3-dimethylpiperazin-1-yl)-6,6a,7,8,9,10-hexahydro-12H-pyrazino[2,1-c]pyrido[3,4-f][1,4]oxazepin-12-one (18.3 mg, yield: 9.2%). ¹H NMR (400 MHz, CDCl₃) δ 7.30 (t, *J*= 7.3 Hz, 1H), 6.84 (d, *J*= 8.3 Hz, 1H), 6.70 (t, *J*= 9.1 Hz, 1H), 6.56 (s, 1H), 6.42 (d, *J*= 16.4 Hz, 1H), 5.83 (d, *J=* 10.3 Hz, 1H), 4.38 (d, *J=* 28.5 Hz, 3H), 4.20 (dd, *J=* 41.4, 11.9 Hz, 3H), 4.01 (s, 1H), 3.78 (s, 2H), 3.52 (s, 6H), 3.37 (s, 3H), 2.69 (s, 2H), 1.60 (s, 6H). ES-API: [M+H]⁺= 588.2.

### Embodiment 62-51 Synthesis of Z322

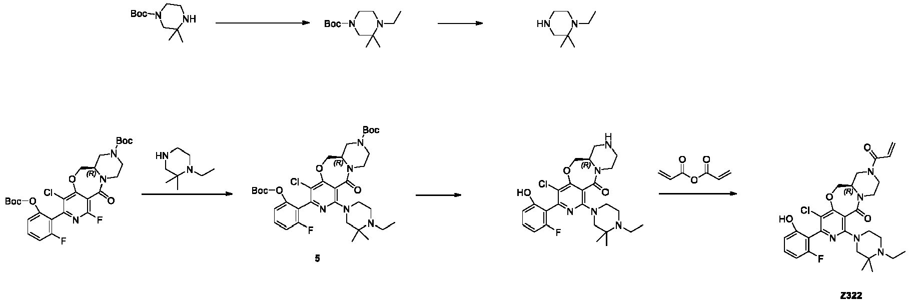

Step 1: *tert*-Butyl 3,3-dimethylpiperazine-1-carboxylate (200 mg,0.935 mmol) was dissolved in acetonitrile (2 mL), then potassium carbonate (258 mg,1.87 mmol) and iodoethane (219 mg,1.4 mmol) were added thereto, reacted at 60°C for 3 hours. The reaction solution was cooled, added with water and ethyl acetate, and the organic phase was washed with brine, dried and concentrated to obtain a crude product of tert-butyl 4-ethyl-3,3-dimethylpiperazine-1-carboxylate (250 mg, yield: 100%). ES-API: [M-55]⁺= 187.2

Step 2: *tert*-Butyl 4-ethyl-3,3-dimethylpiperazine-1-carboxylate (250 mg, 1.03 mmol) was dissolved in dichloromethane (2 mL), and trifluoroacetic acid (2 mL) was added thereto, reacted for 0.5 hours. After the reaction was completed, the mixture was evaporated to dryness by rotary evaporation to obtain a crude product of 1-ethyl-2,2-dimethylpiperazine (200 mg, yield: 100%). ES-API: [M+H]⁺=143.2

Step 3: Compound tert-butyl (6a*R*)-3-(2-((*tert*-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepin-12-one (150 mg, 0.258 mmol), 1-ethyl-2,2-dimethylpiperazine (200 mg, crude, 0.516 mmol) and potassium carbonate (107 mg, 0.774 mmol) were dissolved in acetonitrile (2 mL), reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of *tert*-butyl (6aR)-3-(2-((tert-butoxycarbonyl)oxy)-6-fluorophenyl)-4-chloro-1-(4-ethyl-3,3-dimethylpiperazin-1-yl)-12-oxo-6a,7,9,10-tetrahydro-6H-pyrazino[2,1-c]pyrido[3,4-f][1,4]oxazepin-12-one (300 mg, yield: 100%). ES-API: [M+H]⁺= 704.3.

Step 4: (6a*R*)-3-(2-((tert-Butoxycarbonyl)oxy)-6-fluorophenyl)-4-chloro-1-(4-ethyl-3,3-dimethylpiperazin-1-yl)-12-oxo-6a,7,9,10-tetrahydro-6*H*-pyrazino[2,1-c]pyrido[3,4-f][1,4]oxazepin-12-one (300 mg, crude product, 0.427 mmol) was dissolved in dichloromethane (3 mL), and then trifluoroacetic acid (3 mL) was added dropwise thereto, stirred at room temperature for 0.5 hours. After the reaction was completed, the mixture was evaporated to dryness by rotary evaporation to obtain a crude product of (6aR)-4-chloro-3-(2-fluoro-6-hydroxyphenyl)-1-(4-ethyl-3,3-dimethylpiperazin-1-yl)-7,8,9,10-tetrahydro-6*H-*pyrazino[2,1-*c*]pyrido[3,4-f][1,4]oxazepin-12-one (250 mg,100%). ES-API: [M+H]⁺=504.2

Step 5: (6aR)-4-Chloro-3-(2-fluoro-6-hydroxyphenyl)-1-(4-ethyl-3,3-dimethylpiperazin-1-yl)-7,8,9,10-tetrahydro-6*H*-pyrazino[2,1-*c*]pyrido[3,4-*f*][1,4]oxazepin-12-one(250 mg, crude, 0.258 mmol) was dissolved in dichloromethane (3 mL), then cooled to 0°C in an ice-water bath, and triethylamine (1 mL) was added dropwise thereto. The pH value was basic, and acrylic anhydride (19 mg, 0.155 mmol) was added dropwise thereto, stirred for 10 minutes. After the reaction was completed, the reaction solution was added with water, washed with brine, dried over sodium sulfate, evaporated to dryness by rotary evaporation, and purified by thin-layer chromatography (dichloromethane/methanol=10/1) to obtain (6a*R*)-8-acryloyl-4-chloro-3-(2-fluoro-6-hydroxyphenyl)-1-(4-ethyl-3,3-dimethylpiperazin-1-yl)-6,6a,7,8,9,10-hexahydro-12*H*-pyrazino[2,1-*c*]pyrido[3,4-*f*][1,4]oxazepin-12-one (Z322,11.5 mg, yield: 8.0%). ¹HNMR (400 MHz, DMSO-d₆) δ 10.04 (s, 1H), 7.23 (q, *J*= 7.9 Hz, 1H), 6.86 - 6.61 (m, 3H), 6.16 (d, *J*= 16.7 Hz, 1H), 5.72 (s, 1H),4.38 (s, 2H), 4.22 (d, *J*= 15.9 Hz, 1H), 4.13 -3.96 (m, 2H), 3.86 (s, 1H), 3.65-3.55 (m, 4H),3.35-3.45 (m, 4H), 3.30-3.20 (m,3H),1.22-1.14 (m, 9H). ES-API: [M+H]⁺=558.2

### Embodiment 62-52 Synthesis of Z324

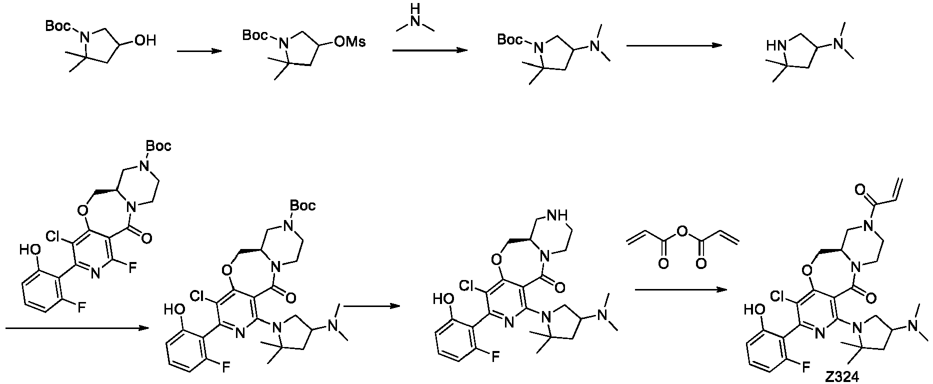

Step 1: *tert*-Butyl 4-hydroxy-2,2-dimethylpyrrolidine-1-carboxylate (500 mg, 2.32 mmol), methanesulfonyl chloride (793 mg, 6.96 mmol), 4-dimethylaminopyridine (849 mg, 6.96 mmol), pyridine (550 mg, 6.96 mmol) and dichloromethane (50 mL) were added to a round bottom flask. The reaction was stirred at room temperature overnight. 20 mL of saturated sodium bicarbonate solution was added to the reaction solution, and the mixture was extracted with dichloromethane. The organic phase was dried and concentrated, and the crude product was purified by flash silica gel column (ethyl acetate/petroleum ether: 0-50%) to obtain a product of *tert*-butyl 2,2-dimethyl-4-((methylsulfonyl)oxy)pyrrolidine-1-carboxylate (512 mg, 75%) as a colorless oil.

Step 2: *tert*-Butyl 2,2-dimethyl-4-((methylsulfonyl)oxy)pyrrolidine-1-carboxylate (512 mg, 1.74 mmol) and a solution of dimethylamine in tetrahydrofuran (6 mL, 2 M) were added to a round bottom flask, stirred at 105°C for 20 hours. The reaction solution was concentrated, and the crude product was purified by flash silica gel column (ethyl acetate/petroleum ether: 0-70%) to obtain *tert*-butyl 4-(dimethylamino)-2,2-dimethylpyrrolidine-1-carboxylate (236 mg, 56%) as a yellow solid. The crude product was directly used in the next step. ES-API: [M+H]⁺=648.2.

Step 3: *tert*-Butyl 4-(dimethylamino)-2,2-dimethylpyrrolidine-1-carboxylate (236 mg, 0.98 mmol), 3 mL of dichloromethane and 2 mL of trifluoroacetic acid were added to a round bottom flask. The mixture was stirred at room temperature for 1 hour, added with saturated sodium bicarbonate solution (3 mL) to quench the reaction, extracted three times with dichloromethane, and the organic phases were combined, dried over anhydrous sodium sulfate and concentrated to obtain N,N,5,5-tetramethylpyrrolidin-3-amine (151 mg, crude product) as a yellow solid, and the crude product was directly used in the next step.

Step 4: *tert*-Butyl (6a*R*)-4-chloro-1-fluoro-3-(2-fluoro-6-hydroxyphenyl)-12-oxo-6a,7,9,10-tetrahydro-12H*-*pyrazino[2,1-*c*]pyrido[3,4-*f*][1,4]oxazepine-8(6*H*)-carboxylate (50 mg, 0.1 mmol) and 5 mL of acetonitrile were added to a round bottom flask. Sodium bicarbonate (29 mg, 0.35 mmol) and *N,N,5,5-*tetramethylpyrrolidin-3-amine (30 mg, 0.21 mmol) were then added to the reaction solution. The reaction was stirred at 80°C overnight. After the reaction was completed, the mixture was filtered to obtain a filtrate, and the filtrate was concentrated to dryness to obtain a crude product of *tert*-butyl (6a*R*)-4-chloro-1-(4-(dimethylamino)-2,2-dimethylpyrrolidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (71 mg, crude product) as a yellow solid, and the crude product was directly used in the next reaction step. ES-API: [M+H]⁺=603.1.

Step 5: *tert*-Butyl (6a*R*)-4-chloro-1-(4-(dimethylamino)-2,2-dimethylpyrrolidin-1-yl)-3-(2-fluoro-6-hydroxyphenyl)-12-oxo-6a,7,9,10-tetrahydro-12H-pyrazino[2,1-c]pyrido[3,4-f][1,4]oxazepine-8(6*H*)-carboxylate (71 mg, 0.12 mmol), 3 mL of dichloromethane and 1.5 mL of trifluoroacetic acid were added to a round bottom flask. The mixture was stirred at room temperature for 1 hour, and the completion of reaction was detected by LC-MS. The reaction solution was concentrated to obtain (6a*R*)-4-chloro-1-(4-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (73 mg) as a brown oily liquid. The crude product was directly used in the next step. ES-API: [M+H]⁺=503.0.

Step 6: (6a*R*)-4-Chloro-1-(4-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (73 mg, 0.15 mmol), 10 mL of dichloromethane and triethylamine (151 mg, 1.5 mmol) were added to around bottom flask. The reaction was cooled to 0°C, and a solution of acrylic anhydride in dichloromethane (12 mg, 0.10 mmol, 1 mL) was added to the reaction solution. The reaction was stirred at 0°C for 10 minutes. 20 mL of saturated aqueous sodium bicarbonate solution was added to the reaction solution, and the mixture was extracted three times with 20 mL of dichloromethane. The organic phase was dried and then concentrated to obtain a crude product of (6a*R*)-8-acryloyl-4-chloro-1-(4-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z324).

### Embodiment 62-53 Synthesis of Z326

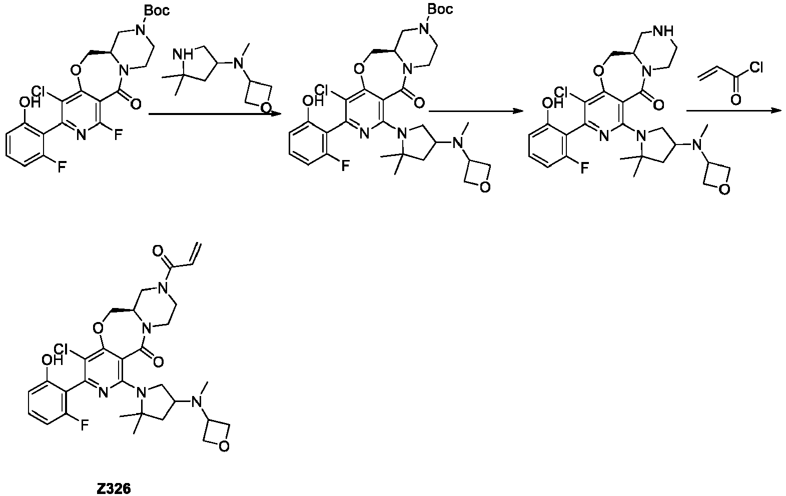

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (670 mg, 1.4 mmol), N,5,5-trimethyl-*N*(oxetan-3-yl)pyrrolidin-3-amine (250 mg, 1.4 mmol), N,N-diisopropylethylamine (540 mg, 4.2 mmol) and acetonitrile (10 mL) were added to a 50 mL round bottom flask, reacted at 85°C for 2 hours. The reaction solution was added with ethyl acetate (20 mL), washed with water (20 mL*3), and the organic phase was dried and concentrated, and the residue was purified by flash silica gel column (ethyl acetate:petroleum ether: 0-90%) to obtain tert-butyl (6a*R*)-4-chloro-1-(2,2-dimethyl-4-(methyl(oxetan-3-yl)amino)pyrrolidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (180 mg, yield of 20%). ES-API: [M+H]⁺=646.3.

Step 2: Trifluoroacetic acid (2 mL) was added to a solution of *tert*-butyl (6a*R*)-4-chloro-1-(2,2-dimethyl-4-(methyl(oxetan-3 -yl)amino)pyrrolidin-1-yl)-3 -(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (180 mg, 0.28 mmol) in dichloromethane (5 mL), reacted at room temperature for 1 hour. The reaction solution was directly concentrated to obtain a crude product of (6a*R*)-4-chloro-1-(2,2-dimethyl-4-(methyl(oxetan-3-yl)amino)pyrrolidin-1-yl)-3-(2-fluoro-6-hydroxyphenyl)-6,6a,7,8,9,10-hexahydro-12*H*-pyrazino [2,1-*c*]pyrido[3,4*f*][1,4]oxazepin-12-one (134 mg). ES-API: [M+H]⁺=546.2.

Step 3: The above crude procuct of (6a*R*)-4-chloro-1-(2,2-dimethyl-4-(methyl(oxetan-3-yl)amino)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (134 mg, 0.24 mmol) was dissolved in dichloromethane (5 mL), and *N*,*N*-diisopropylethylamine (93 mg, 0.72 mmol) was added thereto. The reaction solution was cooled to 0°C, and acryloyl chloride (18 mg, 0.20 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,2-dimethyl-4-(methyl(oxetan-3-yl)amino)pyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z326, 82 mg, yield: 68%). ES-API: [M+H]⁺=600.2.

### Embodiment 62-54 Synthesis of Z330-1

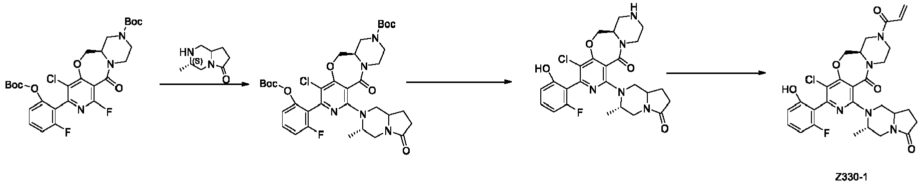

Step 1: Compound tert-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (200 mg, 0.344 mmol) was dissolved in acetonitrile (4 mL), and (3*S*)-3-methylhexahydropyrrolo[1,2-*a*]pyrazin-6(2*H*)-one (88 mg, 0.413 mmol) and potassium carbonate (142 mg,1.032 mmol) were added to the reaction solution, and the reaction solution was reacted at 80°C for 2 hours. After the reaction was completed, the reaction system was cooled to room temperature, quenched with water (10 mL), extracted with ethyl acetate (10 mL*3), washed once with brine (10 mL), dried over anhydrous sodium sulfate, and the filtrate was evaporated to dryness by rotary evaporation to obtain a crude product of compound tert-butyl (6a*R*)-4-chloro-3-(2-fluoro-6-hydroxyphenyl)-1-((3*S*)-3-methyl-6-oxohexahydropyrrolo[1,2-*a*]pyrazin-2(1*H*)-yl)-7,8,9,10-tetrahydro-6*H*-pyrazino[2,1-*c*]pyrido[3,4-*f*][1,4]oxazepine-12(6a*H*)-carboxylate (250 mg, yield: 100%, yellow solid). ES-API: [M+H]⁺=716.39

Step 2: *tert*-Butyl (6a*R*)-4-chloro-3-(2-fluoro-6-hydroxyphenyl)-1-((3*S*)-3-methyl-6-oxohexahydropyrrolo[1,2-*a*]pyrazin-2(1*H*)-yl)-7,8,9,10-tetrahydro-6*H*-pyrazino[2,1-*c*]pyrido[3,4-*f*][1,4]oxazepine-12(6a*H*)-carboxylate (250 mg, 0.349 mmol) was dissolved in 6 mL of dichloromethane, and then trifluoroacetic acid (3 mL) was added thereto. The reaction solution was stirred at room temperature for 2 hours, and the reaction was monitored to be complete, diluted with dichloromethane (20 mL), washed once with water (10 mL), saturated sodium bicarbonate (10 mL) and brine (10 mL) respectively, dried over anhydrous sodium sulfate, and the filtrate was evaporated to dryness by rotary evaporation to obtain a crude product of compound (6a*R*)-4-chloro-3-(2-fluoro-6-hydroxyphenyl)-1-((3*S*)-3-methyl-6-oxohexahydropyrrolo[1,2-*a*]pyrazin-2(1*H*)-yl)-7,8,9,10-tetrahydro-6*H*-pyrazino[2,1-*c*]pyrido[3,4-*f*][1,4]oxazepin-12(6a*H*)-one (167 mg, yield: 93%, yellow solid). ES-API: [M+H]⁺=516.27

Step 3: (6a*R*)-4-Chloro-3-(2-fluoro-6-hydroxyphenyl)-1-((3S)-3-methyl-6-oxohexahydropyrrolo[1,2-*a*]pyrazin-2(1*H*)-yl)-7,8,9,10-tetrahydro-6*H*-pyrazino[2,1-*c*]pyrido[3,4-*f*][1,4]oxazepin-12(6a*H*)-one (167 mg, 0.324 mmol) was dissolved in dichloromethane (5 mL), then cooled to 0°C in an ice-water bath, and triethylamine (98 mg, 0.972 mmol) was added dropwise thereto. The pH value was basic, and acrylic anhydride (33 mg, 0.259 mmol) was added dropwise thereto, stirred for 10 minutes. After the reaction was completed, the mixture was diluted with dichloromethane (20 mL), washed with water (10 mL), dried over sodium sulfate, evaporated to dryness by rotary evaporation and then purified (dichloromethane/methanol=10/l, R_{f}=0.3) to obtain (6a*R*)-8-acryloyl-4-chloro-3-(2-fluoro-6-hydroxyphenyl)-1-((3*S*)-3-methyl-6-oxohexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one (Z330-1, 5.8 mg, yield: 3%, white solid). ¹H NMR (400 MHz, CDCl₃) δ 7.31 (s, 1H), 6.85 (s, 1H), 6.69 (s, 1H), 6.53 (s, 1H), 6.44 (s, 1H),5.84 (s, 1H), 4.43 (s, 1H), 4.29 (s, 3H), 4.17 (s, 1H), 4.03 (s, 3H), 3.75 (s, 2H), 3.66 (s, 2H), 3.15 (d, *J* =12.7 Hz, 1H), 2.41 (s, 2H), 2.21(s, 1H), 1.34 (s, 3H), 1.16 (s, 3H). ES-API: [M+H]⁺=570.2

### Embodiment 62-55 Synthesis of Z332

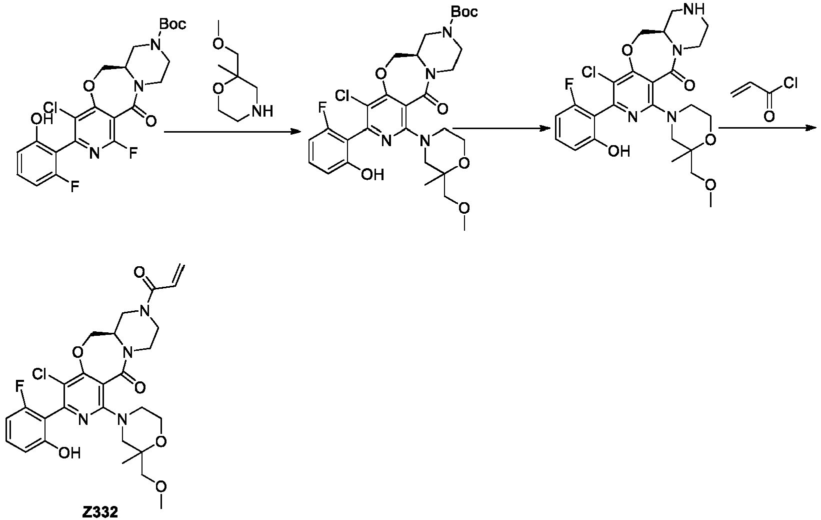

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (480 mg, 1.0 mmol), 2-(methoxymethyl)-2-methylmorpholine (290 mg, 2.0 mmol), N,N-diisopropylethylamine (390 mg, 3 mmol) and acetonitrile (10 mL) were added to a 50 mL round bottom flask, reacted at 85°C for 16 hour. The reaction solution was added with ethyl acetate (30 mL), washed with water (30 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-1-(2-(methoxymethyl)-2-methylmorpholino)-12-oxo-6a,7,9,10-tetrahydro-12*H*-pyrazino[2,1-*c*]pyrido[3,4-*f*][1,4]oxazepine-8(6*H*)-carboxylate (200 mg, yield of 33%). ES-API: [M+H]⁺=607.2.

Step 2: Trifluoroacetic acid (2 mL) was added to a solution of tert-butyl (6a*R*)-4-chloro-3-(2-fluoro-6-hydroxyphenyl)-12-oxo-1-(6-oxohexahydropyrrolo[1,2-*a*]pyrazin-2(1*H*)-yl)-6a,7,9,10-tetrahydro-12*H*-pyrazino[2,1-*c*]pyrido[3,4-*f*][1,4]oxazepine-8(6*H*)-carboxylate (200 mg, 0.33 mmol) in dichloromethane (5 mL), reacted at room temperature for 1 hour. The reaction solution was directly concentrated to obtain a crude procuct of (6a*R*)-4-chloro-3-(2-fluoro-6-hydroxyphenyl)-1-(2-(methoxymethyl)-2-methylmorpholino)-6,6a,7,8,9,10-hexahydro-12*H*-pyrazino[2,1-*c*]pyrido[3,4-f][1,4]oxazepin-12-one (150 mg). ES-API: [M+H]⁺=507.2.

Step 3: The above crude product of (6a*R*)-4-chloro-3-(2-fluoro-6-hydroxyphenyl)-1-(2-(methoxymethyl)-2-methylmorpholino)-6,6a,7,8,9,10-hexahydro-12*H*-pyrazino[2,1-*c*]pyrido[3,4-*f*][1,4]oxazepin-12-one (150 mg, 0.30 mmol) was dissolved in dichloromethane (5 mL), and *N,N-*diisopropylethylamine (115 mg, 0.9 mmol) was added thereto. The reaction solution was cooled to 0°C, and acryloyl chloride (22 mg, 0.25 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(2-(methoxymethyl)-2-methylmorpholino)-6,6a,7, 8,9,10-hexahydro-12*H*-pyrazino[2,1-c]pyrido[3,4-*f*][1,4]oxazepin-12-one (Z332, 110 mg, yield: 67%). ES-API: [M+H]⁺=561.2.

### Embodiment 62-56 Synthesis of Z333

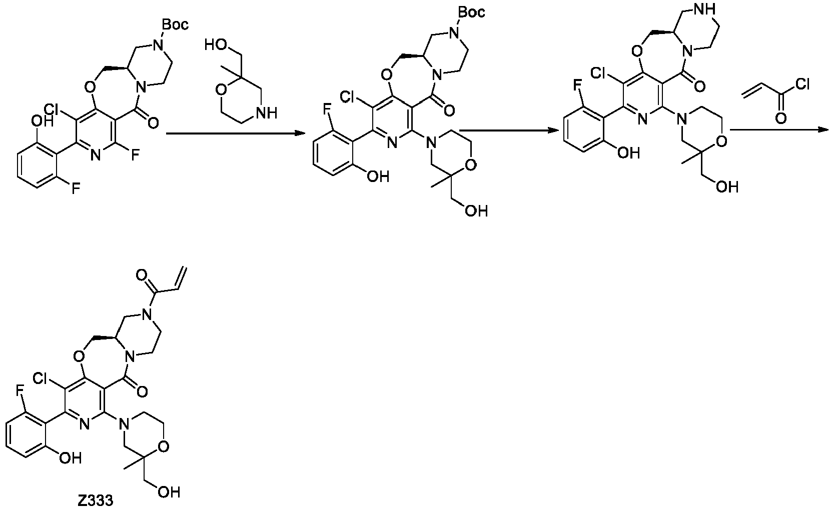

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (480 mg, 1.0 mmol), (2-methylmorpholin-2-yl)methanol (260 mg, 2.0 mmol), *N*,*N*-diisopropylethylamine (387 mg, 3.0 mmol) and acetonitrile (10 mL) were added to a 50 mL round bottom flask, reacted at 85°C for 16 hours. The reaction solution was added with ethyl acetate (30 mL), washed with water (30 mL*3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-1-(2-(hydroxymethyl)-2-methylmorpholino)-12-oxo-6a,7,9,10-tetrahydro-12H-pyrazino[2,1-*c*]pyrido[3,4-*f*][1,4]oxazepine-8(6H)-carboxylate (200 mg, yield of 34%). ES-API: [M+H]⁺=593.2.

Step 2: Trifluoroacetic acid (2 mL) was added to a solution of *tert*-butyl (6a*R*)-4-chloro-3-(2-fluoro-6-hydroxyphenyl)-1-(2-(hydroxymethyl)-2-methylmorpholino)-12-oxo-6a,7,9,10-tetrahydro-12*H-*pyrazino[2,1-*c*]pyrido[3,4-*f*][1,4]oxazepine-8(6*H*)-carboxylate (200 mg, 0.34 mmol) in dichloromethane (5 mL), reacted at room temperature for 1 hour. The reaction solution was directly concentrated to obtain a crude procuct of (6a*R*)-4-chloro-3-(2-fluoro-6-hydroxyphenyl)-1-(2-(hydroxymethyl)-2-methylmorpholino)-6,6a,7,8,9,10-hexahydro-12*H*-pyrazino[2,1-*c*]pyrido[3,4*f*][1,4]oxazepin-12-one (150 mg). ES-API: [M+H]⁺=493.2.

Step 3: The above crude product of (6a*R*)-4-chloro-3-(2-fluoro-6-hydroxyphenyl)-1-(2-(hydroxymethyl)-2-methylmorpholino)-6,6a,7,8,9,10-hexahydro-12*H*-pyrazino [2,1-*c*]pyrido [3,4-*f*][1,4]oxazepin-12-one (150 mg, 0.30 mmol) was dissolved in dichloromethane (5 mL), and *N,N-*diisopropylethylamine (115 mg, 0.9 mmol) was added thereto. The reaction solution was cooled to 0°C, and acryloyl chloride (22 mg, 0.25 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluoro-6-hydroxyphenyl)-1-(2-(hydroxymethyl)-2-methylmorpholino)-6,6a,7,8,9,10-hexahydro-12*H*-pyrazino[2,1-*c*]pyrido[3,4-*f*][1,4]oxazepin-12-one (Z333, 120 mg, yield: 73%). ES-API: [M+H]⁺=547.2.

### Embodiment 62-57 Synthesis of Z334

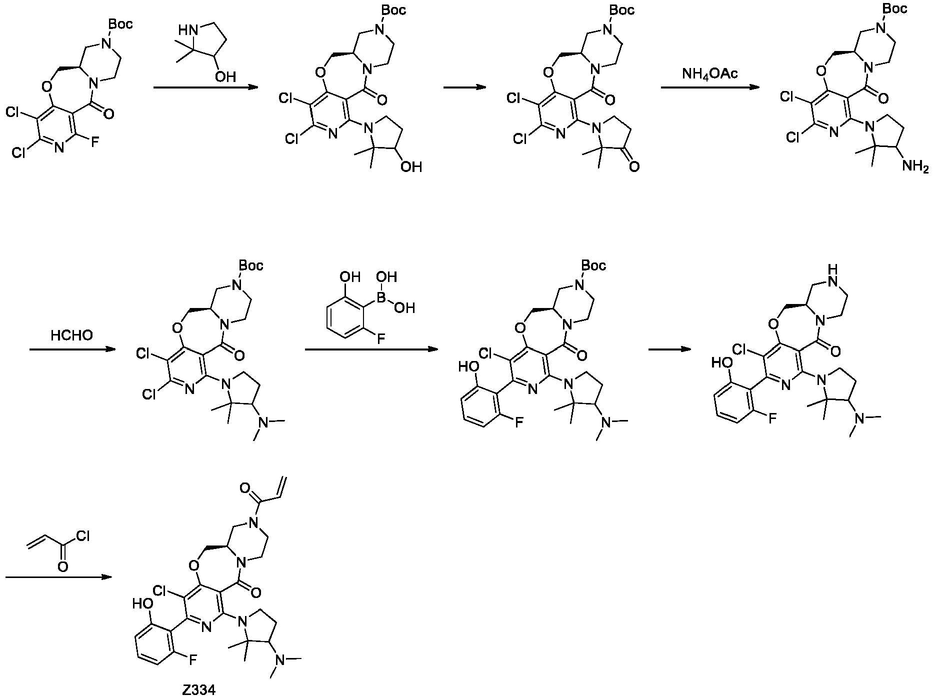

Step 1: A solution of compound (*R*)-3,4-dichloro-1-fluoro-12-oxo-6a,7,9,10-tetrahydro-12*H-*pyrazino[2,1-*c*]pyrido[3,4-*f*][1,4]oxazepine-8(6*H*)-carboxylate (1 g, 2.46 mmol), 2,2-dimethylpyrrolidin-3-ol (300 mg, 2.60 mmol) and potassium carbonate (1.02 g, 7.38 mmol) in acetonitrile (20 mL) was stirred at 90°C for 2 hours. The reaction solution was filtered, concentrated, and then purified by flash silica gel column (ethyl acetate: petroleum ether: 0-80%) to obtain *tert*-butyl (6aR)-3,4-dichloro-1-(3-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6H)-carboxylate (1.20 g). ES-API: [M+H]⁺=501.1.

Step 2: A solution of compound *tert*-butyl (6a*R*)-3,4-dichloro-1-(3-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6H)-carboxylate (1.20 g, 2.39 mmol) and Dess-Martin reagent (3.05 g, 7.18 mmol) in dichloromethane (50 mL) was stirred at room temperature overnight. The reaction solution was quenched with saturated sodium thiosulfate solution (30 mL) and saturated sodium bicarbonate solution (30 mL), extracted with dichloromethane (30 mL * 3), and the organic phases were combined, dried and concentrated, and then purified by flash silica gel column (ethyl acetate: petroleum ether: 0-80%) to obtain tert-butyl (*R*)-3,4-dichloro-1-(2,2-dimethyl-3-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (1 g). ES-API: [M+H]⁺=499.1.

Step 3: A mixture of tert-butyl (*R*)-3,4-dichloro-1-(2,2-dimethyl-3-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (300 mg, 0.60 mmol) and a solution of ammonium acetate (1.85 g, 24 mmol) in ethanol were reacted at 130°C for 30 minutes under microwave irradiation. The mixture was cooled to room temperature, sodium cyanoborohydride (76 mg, 1.20 mmol) was added thereto and reacted at 80°C for 1 hour. The reaction solution was concentrated, dissolved in ethyl acetate (10 mL), washed with water (10 mL*3), and the organic phase was dried and concentrated to obtain a crude product of *tert*-butyl (6a*R*)-1-(3-amino-2,2-dimethylpyrrolidin-1-yl)-3,4-dichloro-12-oxo-6a,7,9,10-tetrahydro-12*H*-pyrazino[2,1-*c*]pyrido [3,4-*f*][1,4]oxazepine-8(6*H*)-carboxylate (300 mg). ES-API: [M+H]⁺=500.2.

Step 4: The crude product of tert-butyl (6a*R*)-1-(3-amino-2,2-dimethylpyrrolidin-1-yl)-3,4-dichloro-12-oxo-6a,7,9,10-tetrahydro-12*H*-pyrazino[2,1-*c*]pyrido[3,4-*f*][1,4]oxazepine-8(6*H*)-carboxylate (300 mg, 0.60 mmol) obtained above was dissolved in in methanol (5 mL), and then 37% aqueous formaldehyde solution (1 mL) and sodium cyanoborohydride (75 mg, 1.20 mmol) were added thereto in turn, and stirred at room temperature for 1 hour. The reaction solution was dissolved in dichloromethane (30 mL), washed with water (10 mL*3), and the organic phase was dried and concentrated to obtain a crude product of *tert*-butyl (6a*R*)-3,4-dichloro-1-(3-(dimethylamino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (280 mg). ES-API: [M+H]⁺=528.1.

Step 5: Under nitrogen protection, a mixture of *tert*-butyl (6a*R*)-3,4-dichloro-1-(3-(dimethylamino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (280 mg, 0.53 mmol), 2-fluoro-6-hydroxyphenylboronic acid (250 mg, 1.59 mmol), tetrakis(triphenylphosphine)palladium (61 mg, 0.05 mmol) and potassium carbonate (220 mg, 1.59 mmol) in 1,4-dioxane (3 mL) and water (0.7 mL) were reacted at 100°C for 1 hour under microwave irradiation. The reaction solution was dissolved in ethyl acetate (30 mL) and washed with saturated brine (10 mL*3). The organic phase was dried and concentrated, and purified by flash silica gel column (methanol:dichloromethane: 0-10%) to obtain *tert*-butyl (6a*R*)-4-chloro-1-(3-(dimethylamino)-2,2-dimethylpyrrolidin-1-yl)-3 -(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (200 mg). ES-API: [M+H]⁺=604.2.

Step 6: Under an ice bath, a solution of *tert*-butyl (6a*R*)-4-chloro-1-(3-(dimethylamino)-2,2-dimethylpyrrolidin-1-yl)-3-(2-fluoro-6-hydroxyphenyl)-12-oxo-6a,7,9,10-tetrahydro-12*H*-pyrazino[2,1-c]pyrido[3,4-*f*][1,4]oxazepine-8(6*H*)-carboxylate (200 mg, 0.33 mmol) in dichloromethane (2 mL) was added with trifluoroacetic acid (0.5 mL), and the reaction solution was stirred at room temperature for 1 hour. The reaction solution was concentrated under reduced pressure to obtain a crude product of (6a*R*)-4-chloro-1-(3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (167 mg). ES-API: [M+H]⁺=504.3.

Step 7: The above crude procuct of (6a*R*)-4-chloro-1-(3-(dimethylamino)-2,2-dimethylpyrrolidin-1-yl)-3-(2-fluoro-6-hydroxyphenyl)-6,6a,7,8,9,10-hexahydro-12*H*-pyrazino 2,1-c]pyrido[3,4-*f*][1,4]oxazepin-12-one (167 mg, 0.33 mmol) was dissolved in dichloromethane (2 mL), and *N,N-*diisopropylethylamine (214 mg, 1.66 mmol) was added thereto. The reaction solution was cooled to 0°C, and acryloyl chloride (30 mg, 0.33 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z334, 29 mg, yield: 16%). ES-API: [M+H]⁺ = 559.2. ¹H NMR (500 MHz, DMSO-d₆) δ 10.0 - 9.96 (m, 1H), 7.27 - 7.18 (m, 1H), 6.83 - 6.65 (m, 3H), 6.17 (d, *J* = 17.0 Hz, 1H), 5.77 - 5.70 (m, 1H), 4.59 - 4.46 (m, 1H), 4.35 - 3.87 (m, 6H), 3.75 - 3.17 (m, 3H), 2.93 - 2.81 (m, 1H), 2.48 - 2.40 (m, 1H), 2.26 (s, 6H), 2.03 - 1.93 (m, 1H), 1.82 - 1.68 (m, 1H), 1.53 - 1.45 (m, 3H), 1.41 - 1.33 (m, 3H).

### Embodiment 62-58 Synthesis of Z337

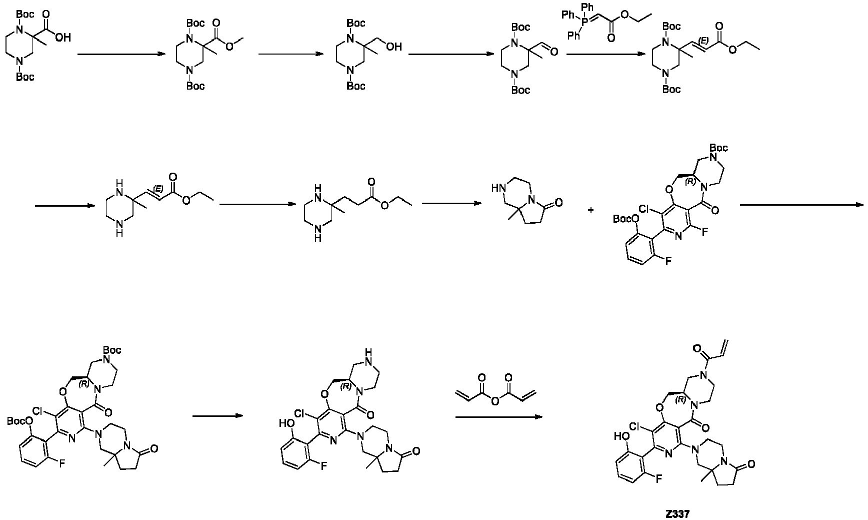

Step 1: 1,4-bis(*tert*-Butoxycarbonyl)-2-methylpiperazine-2-carboxylic acid (9 g, 26.16 mmol) was dissolved in 90 mL of tetrahydrofuran, cooled to -78°C, and lithium bis(trimethylsilyl)amide (1 M, 52 mL) was added dropwise thereto, reacted at -78°C for 2 hours, and iodomethane (5.57 g, 39.24 mmol) was added dropwise thereto, naturally heated to room temperature and reacted for 16 hours. The reaction was quenched with saturated ammonium chloride, extracted with ethyl acetate, and the organic phase was washed with saturated brine, dried over sodium sulfate and concentrated under reduced pressure to obtain a crude product of 1,4-di-tert-butyl 2-methyl 2-methylpiperazine-1,2,4-tricarboxylate (10 g, crude product). ES-API: [M-200]⁺=159.2.

Step 2: 1,4-Di-*tert*-butyl 2-methyl 2-methylpiperazine-1,2,4-tricarboxylate (10 g, crude product) was dissolved in 100 mL of tetrahydrofuran, cooled to -30°C, and lithium aluminum hydride (1.55 g, 41.9 mmol) was added thereto and reacted for 1 hour. The reaction solution was quenched with 1.5 mL of water, 1.5 mL of 15% sodium hydroxide, and 4.5 mL of water in turn, filtered, and concentrated under reduced pressure to obtain di-tert-butyl 2-(hydroxymethyl)-2-methylpiperazine-1,4-dicarboxylate (6.2 g, 66.8%). ES-API: [M-200]⁺=131.2.

Step 3: Di-*tert*-butyl 2-(hydroxymethyl)-2-methylpiperazine-1,4-dicarboxylate (6.2 g, 18.8 mmol) was dissolved in 60 mL of dichloromethane, and Dess-Martin periodinane (23.9 g, 56.4 mmol) was added thereto and reacted for 16 hours. The reaction solution was quenched with aqueous sodium sulfite solution, washed with saturated sodium bicarbonate, washed with brine, dried over sodium sulfate, and concentrated under reduced pressure to obtain a crude product of di-*tert*-butyl 2-formyl-2-methylpiperazine-1,4-dicarboxylate (6 g, crude product). ES-API: [M-200]⁺=129.2.

Step 4: Di-*tert*-butyl 2-formyl-2-methylpiperazine-1,4-dicarboxylate (6 g, 18.3 mmol) and ethyl 2-(triphenylphosphoranylidene)acetate (15.9 g, 45.7 mmol) were dissolved in 60 mL of toluene, heated to 80°C and reacted for 16 hours. The solvent was evaporated to dryness by rotary evaporation, and the residue was purified by column chromatography (petroleum ether/ethyl acetate=5/1, R_{f}=0.4) to obtain di-tert-butyl 2-(3-ethoxy-3-oxoprop-1-en-1-yl)-2-methyl-1,4-dicarboxylate (1.8 g, 24.7%). ES-API: [M-200]⁺=199.2.

Step 5: Di-*tert*-butyl 2-(3-ethoxy-3-oxoprop-1-en-1-yl)-2-methyl-1,4-dicarboxylate (1.8 g, 4.52 mmol) was dissolved in 20 mL of 4 M hydrochloric acid/1,4-dioxane, reacted for 1 hour, and the solvent was evaporated to dryness by rotary evaporation obtain a crude product of ethyl (2-methylpiperazin-2-yl)acrylate (1 g, crude product), which was directly used in the next step. ES-API: [M+H]⁺=199.2.

Step 6: Ethyl (2-methylpiperazin-2-yl)acrylate (1 g, crude product) was dissolved in 20 mL of ethanol, and then 360 mg of 10% palladium on carbon and 360 mg of palladium hydroxide were added thereto, reacted at room temperature under the pressure of 3 kilograms of hydrogen for 16 hours. The reaction solution was filtered, and the solvent was evaporated to dryness by rotary evaporation to obtain a crude product of ethyl 3-(2-methylpiperazin-2-yl)propanoate (1.2 g, crude product). ES-API: [M+H] 4=201.2.

Step 7: Ethyl 3-(2-methylpiperazin-2-yl)propionate (1.2 g, crude product) was dissolved in 15 mL of ethanol, heated to 85°C and reacted for 16 hours, and the solvent was evaporated to dryness by rotary evaporation to obtain a crude product of 8a-methylhexahydropyrrolo[1,2-*a*]pyrazin-6(2*H*)-one (500 mg, 54.1%). ES-API: [M+H]+=155.1.

Step 8: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepin-12-one (150 mg, 0.258 mmol), 8a-methylhexahydropyrrolo[1,2-*a*]pyrazin-6(2*H*)-one (200 mg, crude, 0.516 mmol) and potassium carbonate (107 mg, 0.774 mmol) were dissolved in acetonitrile (2 mL), reacted at 80°C for 4 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of *tert*-butyl (6a*R)*-3-(2-((*tert-*butoxycarbonyl)oxy)-6-fluorophenyl)-4-chloro-1-(8a-methylhexahydropyrrolo[1,2-*a*]pyrazin-6(2*H*)-one-1-yl)-12-oxo-6a,7,9,10-tetrahydro-6*H*-pyrazino[2,1-*c*]pyrido[3,4-*f*][1,4]oxazepin-12-one (120 mg, 65.2%). ES-API: [M+H]⁺= 716.2.

Step 9: *tert*-Butyl (6a*R*)-4-chloro-3-(2-fluoro-6-hydroxyphenyl)-12-oxo-1-(8a-methylhexahydropyrrolo[1,2-*a*]pyrazin-6(2*H*)-one-1-yl)-6a,7,9,10-tetrahydro-6*H*-pyrazino[2,1-c]pyrido[3,4-*f*][1,4]oxazepine-8(6*H*)-carboxylate (120 mg, 0.168 mmol) was dissolved in 5 mL of dichloromethane, and 3 mL of trifluoroacetic acid was added thereto. The reaction solution was stirred at room temperature for 1 hour. The reaction solution was directly concentrated under reduced pressure, redissolved in dichloromethane, and concentrated to obtain a crude procuct of (6a*R*)-4-chloro-3-(2-fluoro-6-hydroxyphenyl)-1-(8a-methylhexahydropyrrolo[1,2-*a*]pyrazin-6(2*H*)-one-1-yl)-7,8,9,10-tetrahydro-6*H-*pyrazino[2,1-*c*]pyrido[3,4-*f*][1,4]oxazepin-12(6a*H*)-one (100 mg, crude product). The crude product was directly used in the next reaction step. ES-API: [M+H]⁺=516.2.

Step 10: (6a*R*)-4-ehloro-3-(2-fluoro-6-hydroxyphenyl)-1-(8a-methylhexahydropyrrolo[1,2-*a*]pyrazin-6(2*H*)-one-1-yl)-7,8,9,10-tetrahydro-6*H*-pyrazino[2,1-*c*]pyrido[3,4-*f*][1,4]oxazepin-12(6a*H*)-one (100 mg, crude product) (100 mg, 0.168 mmol) was dissolved in 2 mL of dichloromethane, and then 1 mL of triethylamine was added thereto, and acrylic anhydride (15 mg, 0.118 mmol) was added dropwise thereto at 0°C, stirred and reacted for 10 minutes. The reaction solution was washed with brine, extracted with dichloromethane, and the organic phase was concentrated under reduced pressure, and the crude product was purified by preparative HPLC (preparative separation conditions: chromatographic column information: Ultimate XB-C18, 50*250 mm, 10 µm, mobile phase system: A: purified water B : pure acetonitrile, flow rate: 80 mL/min, gradient: within 40 minutes, B/A=20%-90%, wavelength: 214 nm, column temperature: room temperature) to obtain (6a*R*)-8-acryloyl-4-chloro-3-(2-fluoro-6-hydroxyphenyl)-1-(8a-methylhexahydropyrrolo[1,2-*a*]pyrazin-6(2*H*)-one-1-yl)-6,6a,7,8,9,10-hexahydro-12*H*-pyrazino[2,1-*c*]pyrido[3,4-*f*][1,4]oxazepin-12-one (Z337, 7.8 mg, 8.16%) as a white solid. ¹HNMR(400 MHz, CDCl₃) δ7.30 (d, *J*= 7.2 Hz, 1H), 6.84 (d, *J*= 8.2 Hz, 1H), 6.75-6.71 (m, 1H), 6.60-6.56 (m, 1H), 6.43 (d, *J*= 14.9 Hz, 1H), 5.90-5.84 (m, 1H), 4.50-3.52 (m, 10H), 3.41 - 2.68 (m,3H), 2.47 (d, *J=* 30.6 Hz, 2H), 2.03-1.99 (m, 2H), 1.95-1.86 (m, 2H), 1.5-1.42 (m, 3H). ES-API: [M+H]⁺=570.2.

### Embodiment 62-59 Synthesis of Z338-1

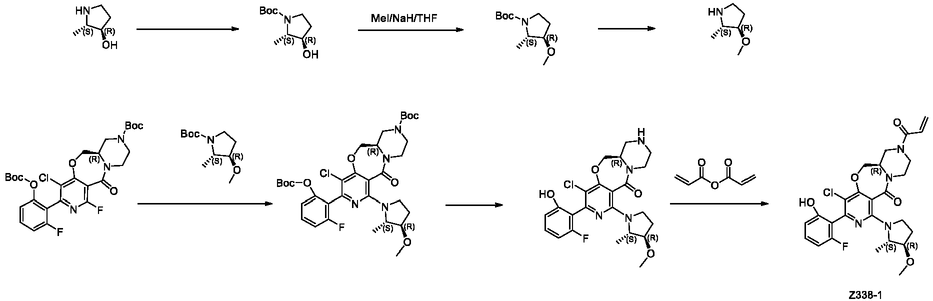

Step 1: (2S,3R)-2-Methylpyrrolidin-3-ol (200 mg, 1.98 mmol) was dissolved in 2 mL of dichloromethane, and triethylamine (300 mg, 2.97 mmol) and di-*tert*-butyl dicarbonate (518 mg, 2.38 mmol) were added thereto, reacted at room temperature for 1 hour. The reaction solution was washed with 0.5 N dilute hydrochloric acid, saturated sodium bicarbonate, and brine in turn, and the organic phase was dried over sodium sulfate and evaporated to dryness by rotary evaporation to obtain a crude product of *tert*-butyl (2*S*,3*R*)-3-hydroxy-2-methylpyrrolidine-1-carboxylate (500 mg, crude product). ES-API: [M-55]⁺= 146.1

Step 2: *tert*-Butyl (2*S*,3R*)*-3-hydroxy-2-methylpyrrolidine-1-carboxylate (500 mg, crude product) was dissolved in tetrahydrofuran (5 mL), then sodium hydride (299 mg, 7.47 mmol) was added thereto at 0°C, reacted for 0.5 hours, and iodomethane (530 mg, 3.73 mmol) was added thereto, reacted at room temperature for 16 hours. The mixture was quenched with water, extracted with ethyl acetate, washed with brine, dried over sodium sulfate and evaporated to dryness by rotary evaporation to obtain a crude product of *tert*-butyl (2*S*,3*R*)-3-methoxy-2-methylpyrrolidine-1-carboxylate (600 mg, crude product), ES-API: [M-55]⁺= 160.1.

Step 3: *tert*-Butyl (2*S*,3*R*)-3-methoxy-2-methylpyrrolidine-1-carboxylate (600 mg, crude product) was dissolved in dichloromethane (3 mL), and then trifluoroacetic acid (3 mL) was added dropwise thereto, stirred at room temperature for 0.5 hours. After the reaction was completed, the mixture was evaporated to dryness by rotary evaporation to obtain a crude product of (2*S*,3*R*)-3-methoxy-2-methylpyrrolidine (500 mg, crude product), ES-API: [M+H]⁺= 116.1.

Step 4: Compound *tert*-butyl (6a*R*)-3-(2-((tert-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepin-12-one (150 mg, 0.258 mmol), (2S,3R)-3-methoxy-2-methylpyrrolidine (500 mg, crude product), and potassium carbonate (107 mg, 0.774 mmol) were dissolved in acetonitrile (2 mL), reacted at 80°C for 4 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of *tert*-butyl (6a*R*)-3-(2-((*tert*-butoxycarbonyl)oxy)-6-fluorophenyl)-4-chloro-1-(2*S*,3*R*)-3-methoxy-2-methylpyrrolidine-12-oxo-6a,7,9,10-tetrahydro-6*H-*pyrazino[2,1-*c*]pyrido[3,4-*f*][1,4]oxazepin-12-one (110 mg, 63.1%). ES-API: [M+H]⁺= 677.2.

Step 5: *tert*-Butyl (6a*R*)-3-(2-((*tert*-butoxycarbonyl)oxy)-6-fluorophenyl)-4-chloro-1-(2*S*,3*R*)-3-methoxy-2-methylpyrrolidine-12-oxo-6a,7,9,10-tetrahydro-6*H*-pyrazino[2,1-*c*]pyrido[3,4-*f*][1,4]oxazepin-12-one (110 mg, 0.162 mmol) was dissolved in dichloromethane (2 mL), and then trifluoroacetic acid (2 mL) was added dropwise thereto, stirred at room temperature for 0.5 hours. Then the mixture was evaporated to dryness by rotary evaporation to obtain a crude product of (6a*R*)-4-chloro-3-(2-fluoro-6-hydroxyphenyl)-1-((2*S*,3*R*)-3-methoxy-2-methylpyrrolidin-1-yl)-7,8,9,10-tetrahydro-6*H*-pyrazino[2,1-*c*]pyrido[3,4-*f*][1,4]oxazepin-12-one (100 mg, crude product). ES-API: [M+H]⁺= 477.2.

Step 6: (6a*R*)-4-Chloro-3-(2-fluoro-6-hydroxyphenyl)-1-((2*S*,3*R*)-3-methoxy-2-methylpyrrolidin-1-yl)-7,8,9,10-tetrahydro-6*H*-pyrazino[2,1-*c*]pyrido[3,4-*f*][1,4]oxazepin-12-one (100 mg, crude product) was dissolved in dichloromethane (2 mL), then cooled to 0°C in an ice-water bath, and triethylamine (1 mL) was added dropwise thereto. The pH value was basic, and acrylic anhydride (14 mg, 0.113 mmol) was added dropwise thereto, stirred for 10 minutes. After the reaction was completed, the reaction solution was added with water, washed with brine, dried over sodium sulfate, evaporated to dryness by rotary evaporation, and the crude product was purified by preparative HPLC to obtain (6a*R*)-8-acryloyl-4-chloro-3-(2-fluoro-6-hydroxyphenyl)-1-((2*S*,3*R*)-3-methoxy-2-methylpyrrolidin-1-yl)-6,6a,7,8,9,10-hexahydro-12H-pyrazino[2,1-*c*]pyrido[3,4-*f*][1,4]oxazepin-12-one (Z338-1, 7.6 mg, 8.85%), (preparative separation conditions: chromatographic column information: Ultimate XB-C18, 50*250 mm, 10 µm, mobile phase system: A: purified water B: pure acetonitrile, flow rate: 80 mL/min, gradient: within 40 minutes, B/A=20%-90%, wavelength : 214 nm, column temperature: room temperature). ¹H NMR (400 MHz, CDCl₃) δ 7.28 (s, 1H), 6.85 (d, *J*= 8.0 Hz, 1H), 6.69 (t, *J*= 8.8 Hz, 1H), 6.60-6.56 (m, 1H), 6.39 (d, *J*= 15.9 Hz, 1H), 5.81 (d, *J=* 9.4 Hz, 1H), 4.63 - 3.94 (m, 7H), 3.90-3.86 (b, 1H), 3.70-3.62 (m, 4H), 3.30 (s, 3H), 3.20-3.05 (m, 1H),2.13-2.05 (m, 2H), 1.3-1.26 (m, 3H). ES-API: [M+H]⁺= 531.2.

### Embodiment 62-60 Synthesis of Z339

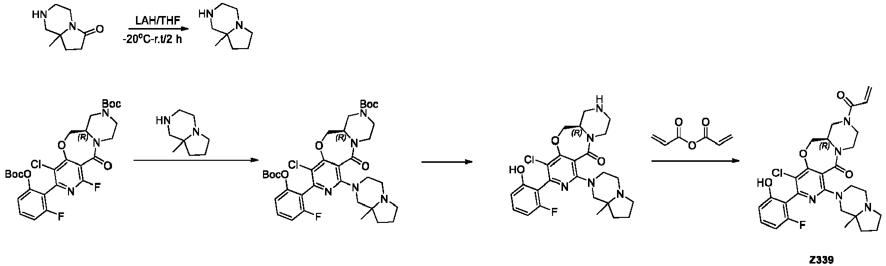

Step 1: 8a-Methylhexahydropyrrolo[1,2-*a*]pyrazin-6(2*H*)-one (200 mg, crude product) was dissolved in 2 mL of tetrahydrofuran, cooled with ice-water, and lithium aluminum hydride (99 mg, 2.6 mmol) was added thereto and reacted at room temperature for 3 hours. The reaction solution was quenched with 0.5 mL of water, 0.5 mL of 15% sodium hydroxide, and 1.5 mL of water in turn, filtered, and concentrated under reduced pressure to obtain 8a-methyloctahydropyrrolo[1,2-*a*]pyrazine (200 mg, crude product). ES-API: [M+H]⁺=141.1.

Step 2: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepin-12-one (150 mg, 0.258 mmol), 8a-methyloctahydropyrrolo[1,2-*a*]pyrazin-6(2*H*)-one (200 mg, crude, 0.516 mmol) and potassium carbonate (107 mg, 0.774 mmol) were dissolved in acetonitrile (2 mL), reacted at 80°C for 4 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of tert-butyl (6a*R*)-3-(2-((tert-butoxycarbonyl)oxy)-6-fluorophenyl)-4-chloro-1-(8a-methyloctahydropyrrolo[1,2-*a*]pyrazin-6(2*H*)-one-1-yl)-12-oxo-6a,7,9,10-tetrahydro-6*H*-pyrazino[2,1-*c*]pyrido[3,4-*f*][1,4]oxazepin-12-one (110 mg, 60.8%). ES-API: [M+H]⁺= 702.3.

Step 3: *tert*-Butyl (6a*R*)-4-chloro-3-(2-fluoro-6-hydroxyphenyl)-12-oxo-1-(8a-methyloctahydropyrrolo [1,2-*a*]pyrazin-6(2*H*)-one-1-yl)-6a,7,9,10-tetrahydro-6*H*-pyrazino[2,1-*c*]pyrido[3,4-*f*][1,4]oxazepine-8(6*H*)-carboxylate (110 mg, 0.157 mmol) was dissolved in 5 mL of dichloromethane, and 3 mL of trifluoroacetic acid was added thereto. The reaction solution was stirred at room temperature for 1 hour. The reaction solution was directly concentrated under reduced pressure, redissolved in dichloromethane, and concentrated to obtain a crude procuct of (6a*R*)-4-chloro-3-(2-fluoro-6-hydroxyphenyl)-1-(8a-methyloctahydropyrrolo[1,2-*a*]pyrazin-6(2*H*-one-1-yl)-7,8,9,10-tetrahydro-6*H-*pyrazino[2,1-*c*]pyrido[3,4-*f*][1,4]oxazepin-12(6a*H*)-one (100 mg, crude product). The crude product was directly used in the next reaction step. ES-API: [M+H]⁺=502.3.

Step 4: (6a*R*)-4-Chloro-3-(2-fluoro-6-hydroxyphenyl)-1-(8a-methyloctahydropyrrolo[1,2-*a*]pyrazin-6(2*H*)-one-1-yl)-7,8,9,10-tetrahydro-6*H*-pyrazino[2,1-*c*]pyrido[3,4-*f*][1,4]oxazepin-12(6a*H*)-one (100 mg, 0.157 mmol) was dissolved in 2 mL of dichloromethane, and then 1 mL of triethylamine was added thereto, and acrylic anhydride (12 mg, 0.0942 mmol) was added dropwise thereto at 0°C, stirred and reacted for 10 minutes. The reaction solution was washed with brine, extracted with dichloromethane, and the organic phase was concentrated under reduced pressure, and the crude product was purified by preparative HPLC (preparative separation conditions: chromatographic column information: Ultimate XB-C18, 50*250 mm, 10 µm, mobile phase system: A: purified water B : pure acetonitrile, flow rate: 80 mL/min, gradient: within 40 minutes, B/A=20%-90%, wavelength: 214 nm, column temperature: room temperature) to obtain (6a*R*)-8-acryloyl-4-chloro-3-(2-fluoro-6-hydroxyphenyl)-1-(8a-methyloctahydropyrrolo[1,2-*a*]pyrazin-6(2*H*)-one-1-yl)-6,6a,7,8,9,10-hexahydro-12*H*-pyrazino[2,1-*c*]pyrido[3,4*f*][1,4]oxazepin-12-one (Z339, 3.5 mg, 4.02%) as a white solid. ¹H NMR (400 MHz, CDCl₃) δ 7.31-7.29 (m,1H), 6.83 (d, *J=* 8.4 Hz, 1H), 6.69 (t, *J*= 9.2 Hz, 1H),6.67-6.55 (m, 1H), 6.41 (d, *J*= 16.2 Hz, 1H), 5.82 (d, *J*= 10.2 Hz, 1H), 4.51 - 4.20 (m, 4H), 4.13 (t, *J*= 16.9 Hz, 1H), 3.96 (s, 1H), 3.90-3.80 (m, 1H), 3.62 (t, *J*= 18.8 Hz, 3H), 3.5-3.47 (m, 1H), 3.30 - 3.18 (m, 2H), 3.1-3.09 (m, 2H), 2.94-2.84(m,2H),2.02 - 1.96 (m, 2H), 1.95-1.91 (m, 2H), 1.45-1.40(m, 3H). ES-API: [M+H]⁺=556.2.

### Embodiment 62-61 Synthesis of Z275

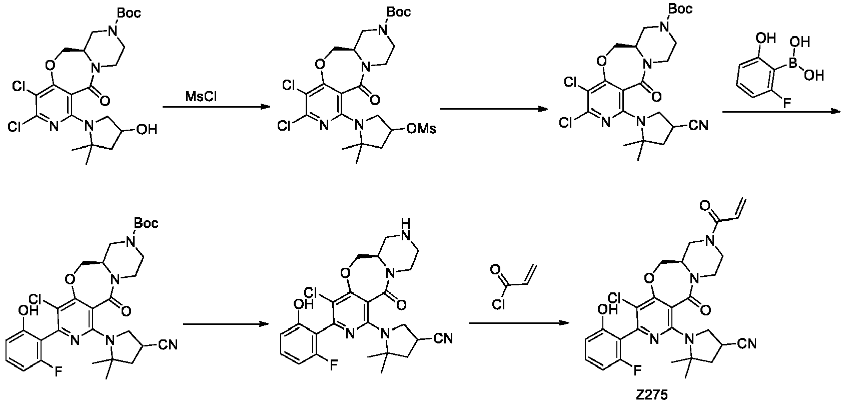

Step 1: (6a*R*)-8-*tert*-Butoxycarbonyl-1-(4-hydroxy-2,2-dimethylpyrrolidinyl)-4-chloro-3-chloro-6,6a,7,8,9,10-hexahydro-12*H*-pyrazino[2,1-*c*]pyrido[3,4-*f*][1,4]oxazepin-12-one (1.23 g, 2.46 mmol), *N,N-*diisopropylethylamine (952 g, 7.38 mmol) and 20 mL of dichloromethane were added to a 50 mL round bottom flask. Under ice-water bath, methanesulfonyl chloride (336 mg, 2.95 mmol) was added thereto, heated to room temperature and reacted for 2 hours. The mixture was poured into ice-water, extracted with dichloromethane, washed with saturated brine, and the organic phase was dried, concentrated, and purified by silica gel column (petroleum ether/ethyl acetate = 50:50) to obtain (6a*R*)-8-*tert*-butoxycarbonyl-1-(2,2-dimethyl-4-((methylsulfonyl)oxy)pyrrolidinyl)-4-chloro-3-chloro-6,6a,7,8,9,10-hexahydro-12*H-*pyrazino[2,1-*c*]pyrido[3,4-*f*][1,4]oxazepin-12-one (1.2 g, yield of 84%). ES-API: [M+H]⁺=579.1.

Step 2: (6a*R*)-8-*tert*-Butoxycarbonyl-1-(2,2-dimethyl-4-((methylsulfonyl)oxy)pyrrolidinyl)-4-chloro-3-chloro-6,6a,7,8,9,10-hexahydro-12*H*-pyrazino[2,1-*c*]pyrido[3,4-*f*][1,4]oxazepin-12-one (1.0 g, 1.73 mmol), tetrabutylammonium cyanide (927 mg, 3.46 mmol) and acetonitrile (8 mL) were added to a 5 mL microwave reaction tube. The mixture was reacted at 110°C for 30 minutes under microwave irradiation. The mixture was added with ethyl acetate, washed with water and saturated brine in turn, and dried over anhydrous sodium sulfate. The mixture was concentrated, and then purified by silica gel column (petroleum ether/ethyl acetate = 60:40) to obtain (6a*R*)-8-*tert*-butoxycarbonyl-1-(3-cyano-5,5-dimethylpyrrolidinyl)-4-chloro-3 -chloro-6,6a,7,8,9,10-hexahydro-12/7-pyrazino [2,1 -c]pyrido [3,4-f][1,4]oxazepin-12-one (55 mg, yield of 6%). ES-API: [M+H]⁺=510.1.

Step 3: (6a*R*)-8-*tert*-Butoxycarbonyl-1-(3-cyano-5,5-dimethylpyrrolidinyl)-4-chloro-3-chloro-6,6a,7,8,9,10-hexahydro-12*H*-pyrazino[2,1-*c*]pyrido[3,4-*f*][1,4]oxazepin-12-one (55 mg, 0.108 mmol), (2-fluoro-6-hydroxyphenyl)boronic acid (50 mmol, 0.324 mmol), Sphos Pd G2 (7.8 mg, 0.0108 mmol) and potassium carbonate (45 mg, 0.324 mmol) were added to a mixed solution of 1,4-dioxane (2 mL) and water (0.5 mL), and system was replaced with nitrogen for 3 times, and the reaction was carried out at 90°C for 1 hour under microwave irradiation. The mixture was added with ethyl acetate, washed with water and saturated brine in turn, and dried over anhydrous sodium sulfate. The mixture was concentrated, and then purified by silica gel column (petroleum ether/ethyl acetate = 10:90) to obtain (6a*R*)-8-*tert*-butoxycarbonyl-1-(4-cyano-2,2-dimethylpyrrolidin-1-yl)*)*-4-chloro-3-(2-fluoro-6-hydroxyphenyl)-6,6a,7,8,9,10-hexahydro-12*H*-pyrazino[2,1-c]pyrido[3,4-*f*][1,4]oxazepin-12-one (25 mg, yield of 40%). ES-API: [M+H]⁺=586.0.

Step 4: (6a*R*)-8-*tert*-Butoxycarbonyl-1-(4-cyano-2,2-dimethylpyrrolidin-1-yl))-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (25 mg, 0.043 mmol), 2 mL of trifluoroacetic acid and 4 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(4-cyano-2,2-dimethylpyrrolidin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (20 mg, trifluoroacetate). ES-API: [M+H]⁺=486.1.

Step 5: (6a*R*)-1-(4-Cyano-2,2-dimethylpyrrolidin-1-yl)-4-chloro-3-chloro-6,6a,7,8,9,10-hexahydro-12*H*-pyrazino[2,1-*c*]pyrido[3,4-*f*][1,4]oxazepin-12-one (20 mg, trifluoroacetate), 5 mL of dichloromethane and N,N-diisopropylethylamine (16.6 mg, 0.129 mmol) were added to a 50 mL round bottom flask. The reaction was cooled to 0°C, and a solution of acryloyl chloride in dichloromethane (3.9 mg, 0.043 mmol) was added dropwise to the reaction solution. The reaction was stirred at 0°C for 10 minutes. The mixture was washed with 5 mL of water, and the organic phase was dried, then concentrated and purified by preparative HPLC (alkali method) to obtain (6a*R*)-8-acryloyl-1-(4-cyano-2,2-dimethylpyrrolidin-1-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z275, 4 mg, 2-step yield of 20%). ¹HNMR (500 MHz, DMSO-*d*₆) δ 10.09-10.01 (m, 1H), 7.24 (q, *J*= 7.9 Hz, 1H), 6.82 - 6.66 (m, 3H), 6.17 (d, *J*= 16.7 Hz, 1H), 5.74 (s, 1H), 4.55-4.42 (m, 1H), 4.31-3.95 (m, 6H), 3.78 (s, 1H), 3.58 (t, *J*= 9.9 Hz, 2H), 3.43 (s, 2H), 2.25 (dd, *J*= 11.8, 5.7 Hz, 1H), 2.07 (t, *J*= 12.3 Hz, 1H), 1.54 - 1.39 (m, 6H). ES-API: [M+H]⁺=540.2

### Embodiment 62-62 Synthesis of Z344

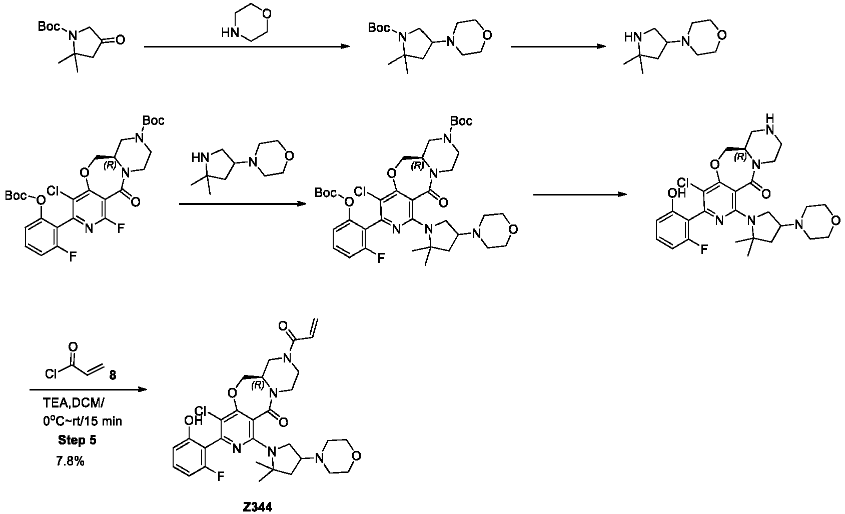

Step 1: *tert*-Butyl 2,2-dimethyl-4-oxopyrrolidine-1-carboxylate (200 mg, 0.94 mmol, 1.0 eq.), morpholine (123 mg, 1.41 mmol) and 0.1 mL of acetic acid were dissolved in dichloromethane, then sodium triacetoxyborohydride (298 mg, 1.41 mmol) was slowly added thereto. The reaction solution was stirred at room temperature for 16 hours. After the reaction, the reaction solution was added with dichloromethane, then added with saturated sodium bicarbonate solution, brine and water in turn to wash. The organic phase was dried over anhydrous sodium sulfate, concentrated, and the crude product was separated by column chromatography (ethyl acetate/petroleum ether=30%-100%, R_{f}=0.4) to obtain *tert*-butyl 2,2-dimethyl-4-morpholinopyrrolidine-1-carboxylate (160 mg, yield of 59.9%) as a colorless oily liquid. ES-API: [M+H]⁺=285.2

Step 2: *tert*-Butyl 2,2-dimethyl-4-morpholinopyrrolidine-1-carboxylate (160 mg, 0.56 mmol) was dissolved in 5 mL of dichloromethane, and trifluoroacetic acid (2 mL) was slowly added dropwise thereto, and the reaction solution was stirred at room temperature for 2 hours. After the reaction was completed, the reaction solution was concentrated to obtain 140 mg of a crude product of 4-(5,5-dimethylpyrrolidin-3-yl)morpholine, which was directly used in the next reaction step. (Crude product). ES-API: [M+H]⁺=185.2

Step 3: Compound *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepin-12-one (200 mg, 0.34 mmol), (4-(5,5-dimethylpyrrolidin-3-yl)morpholine (63 mg, 0.34 mmol) and potassium carbonate (140 mg, 1.02 mmol) were dissolved in acetonitrile (8 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was concentrated. The crude product was chromatographed by column chromatography to obtain (6a*R*)-*tert*-butyl 3-(2-((*tert-*butoxycarbonyl)oxy)-6-fluorophenyl)-4-chloro-1-((4-(5,5-dimethylpyrrolidinyl-3-yl)morpholino-12-oxo-6a,7,9,10-tetrahydro-12*H*-pyrazino[2,1-*c*]pyrido[3,4-*f*][1,4]oxazepin-12-one (134 mg, 52.3%) as a yellow solid. ES-API: [M+H]⁺= 746.4.

Step 4: (6a*R*)-*tert*-Butyl 3-(2-((*tert*-butoxycarbonyl)oxy)-6-fluorophenyl)-4-chloro-1-((4-(5,5-dimethylpyrrolidinyl-3-yl)morpholino-12-oxo-6a,7,9,10-tetrahydro-12*H*-pyrazino[2,1-*c*]pyrido[3,4-*f*][1,4]oxazepin-12-one (134 mg, 0.18 mmol) was dissolved in 5 mL of dichloromethane, and then trifluoroacetic acid (1 mL) was added thereto. The reaction solution was stirred at room temperature for 2 hours. After the reaction was monitored to be complete, the mixture was diluted with dichloromethane, washed with water, washed with saturated sodium bicarbonate and brine once respectively, dried over anhydrous sodium sulfate, and the filtrate was evaporated to dryness by rotary evaporation to obtain compound (6a*R*)-4-chloro-1-((2,2-dimethyl-4-morpholinopyrrolidin-1-yl))-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (140 mg, crude product). ES-API: [M+H]⁺= 546.4.

Step 5: (6a*R*)-4-Chloro-1-((2,2-dimethyl-4-morpholinopyrrolidin-1-yl))-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (140 mg, 0.18 mmol) was dissolved in dichloromethane (5 mL), then cooled to 0°C in an ice-water bath, and triethylamine (91 mg) was added dropwise thereto. The pH value was basic, and acrylic anhydride (18 mg, 0.14 mmol) was added dropwise thereto, stirred for 10 minutes. After the reaction was completed, the mixture was diluted with dichloromethane, washed with water, dried over sodium sulfate, evaporated to dryness by rotary evaporation and the crude product was purified by chromatography plate to obtain a product of (6a*R*)-8-acryloyl-4-chloro-1-((2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z344, 8.4 mg, yield: 7.8%, white solid). ¹HNMR (400 MHz, CDCl₃) δ 7.29 (d, *J* = 8.0 Hz, 1H), 6.83 (d, *J* = 8.3 Hz, 1H), 6.71 (t, *J* = 8.9 Hz, 1H), 6.55 (s, 1H), 6.39 (d, *J* = 16.4 Hz, 1H), 5.81 (d, *J* = 10.3 Hz, 1H), 4.51-3.47 (m, 16H), 2.63 (s, 2H), 2.20-1.99 (m, 2H), 1.85-1.75 (m,2H), 1.46 (s, 3H), 1.33-1.23 (m, 3H). ES-API: [M+H]⁺=600.3.

### Embodiment 62-63 Synthesis of a mixture of Z345 and Z346

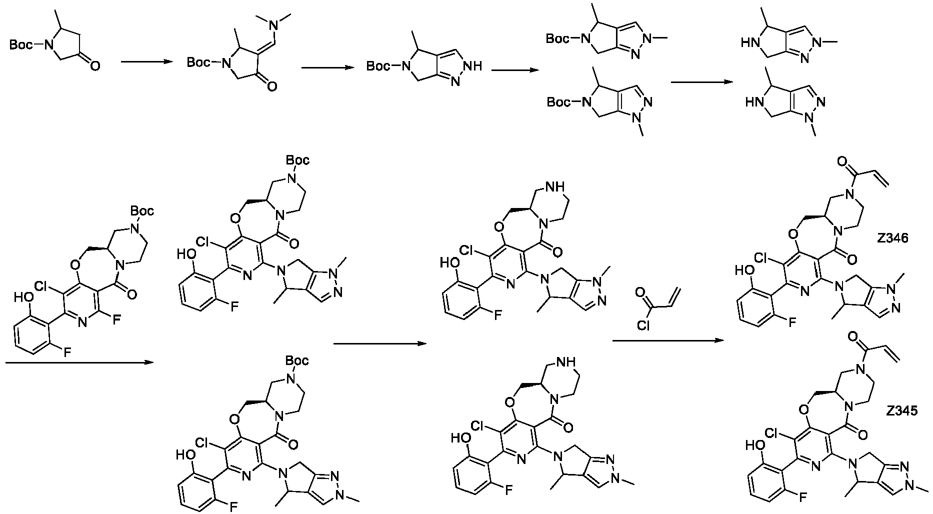

Step 1: *tert*-Butyl 2-methyl-4-oxopyrrolidine-1-carboxylate (1.0 g, 5.02 mmol) and *N,N-*dimethylformamide dimethyl acetal (5 mL) were added to a 25 mL reaction flask, heated to 80°C and reacted for 3 hours. The reaction was completed, and the mixture was concentrated, and purified by silica gel column to obtain a target product of *tert*-butyl 3-((dimethylamino)methylene)-2-methyl-4-oxopyrrolidine-1-carboxylate (700 mg, yield of 55%). ES-API: [M+H]⁺=255.2.

Step 2: *tert*-Butyl 3-((dimethylamino)methylene)-2-methyl-4-oxopyrrolidine-1-carboxylate (700 mg, 2.75 mmol), 80% hydrazine hydrate (0.5 mL) and ethanol (5 mL) were added to a 20 mL microwave reaction flask, heated to 90°C and reacted for 2 hours under microwave irradiation. After the reaction was completed, the mixture was cooled to room temperature, poured into ice-water, extracted with ethyl acetate, washed with water and saturated brine in turn, dried over anhydrous sodium sulfate, filtered, concentrated to obtain a crude target product of *tert*-butyl 4-methyl-4,6-dihydropyrrolo[3,4-c]pyrazole-5(2*H*)-carboxylate (370 mg, yield of 60%). ES-API: [M+H]⁺=224.1.

Step 3: *tert*-Butyl 4-methyl-4,6-dihydropyrrolo[3,4-*c*]pyrazole-5(2*H*)-carboxylate (370 mg, 1.65 mmol) was added to a 50 mL reaction flask, cooled to 0°C in an ice-water bath, and 60% sodium hydride (330 mg, 8.25 mmol) was slowly added thereto. After stirring for 30 minutes, iodomethane (1.17 g, 8.25 mmol) was added thereto, and the mixture was heated to room temperature and reacted for 1 hour. After the reaction was completed, the mixture was poured into ice-water, extracted with ethyl acetate, washed with water and saturated brine in turn, dried over anhydrous sodium sulfate, filtered, concentrated, and purified by silicagel column to obtain a mixture of *tert*-butyl 1,4-dimethyl-4,6-dihydropyrrolo[3,4-c]pyrazole-5(2*H*)-carboxylate and *tert*-butyl 2,4-dimethyl-4,6-dihydropyrrolo[3,4-*c*]pyrazole-5(1*H*)-carboxylate (312 mg, yield of 80%). ES-API: [M+H]⁺=238.1.

Step 4: The above mixture (312 mg, 1.31 mmol) was added to a 25 mL reaction flask, and then 4 M HCl (5 mL, MeOH solution) was added thereto, and reacted at room temperature for 1 hour. After the reaction was completed, the mixture was concentrated to obtain a mixture of 1,4-dimethyl-1,4,5,6-tetrahydropyrrolo[3,4-*c*]pyrazole and 2,4-dimethyl-2,4,5,6-tetrahydropyrrolo[3,4-*c*]pyrazole (161 mg, yield of 90%). [M+H]⁺=138.1.

Step 5: (6a*R*)-8-*tert*-Butoxycarbonyl-3-(2-hydroxy-6-fluorophenyl)-4-chloro-1-fluoro-6,6a,7,8,9,10-hexahydro-12*H*-pyrazino[2,1-*c*]pyrido[3,4-*f*][1,4]oxazepin-12-one (406 mg, 0.844 mmol), a mixture of 2,4-dimethyl-2,4,5,6-tetrahydropyrrolo[3,4-c]pyrazole and 1,4-dimethyl-1,4,5,6-tetrahydropyrrolo[3,4-*c*]pyrazole (115 mg, 0.844 mmol), *N,N*-diisopropylethylamine (327 mg, 2.53 mmol) and acetonitrile (8 mL) were added to a 100 mL round bottom flask, stirred at 90°C for 1 hour under microwave irradiation. The mixture was extracted with ethyl acetate, washed with saturated brine, and the organic phase was dried, concentrated, and purified by silica gel column (petroleum ether: ethyl acetate=10:90) to obtain a mixture of (6a*R*)-8-*tert*-butoxycarbonyl-1-(2,4-dimethylpyrrolo[3,4-c]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one and (6a*R*)-8-*tert*-butoxycarbonyl-1-(1,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (300 mg, yield of 70%). ES-API: [M+H]⁺=599.2.

Step 6: The above mixture (300 mg, 0.5 mmol), 5mL of trifluoroacetic acid and 15mL of dichloromethane were stirred at room temperature for 1 hour, and the completion of reaction was detected by LC-MS. The reaction solution was concentrated to obtain a mixture of (6a*R*)-1-(2,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one and (6a*R*)-1-(1,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (249 mg, yield of 100%). ES-API: [M+H]⁺=499.2.

Step 7: The above mixture (249 mg, crude product), 10 mL of dichloromethane and *N,N-*diisopropylethylamine (193 mg, 1.5 mmol) were mixed. The reaction was cooled to 0°C, and a solution of acryloyl chloride in dichloromethane (45 mg, 0.5 mmol) was added dropwise to the reaction solution. The reaction was stirred at 0°C for 10 minutes. The mixture was washed with 20 mL of water, and the organic phase was dried and concentrated to obtain a crude product of a mixture of (6a*R*)-8-acryloyl-1-(2,4-dimethylpyrrolo[3,4-*c*]pyrazol-5(4*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one and (6a*R*)-8-acryloyl-1-(1,4-dimethylpyrrolo[3,4-*c*]pyrazol-5(1*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (mixture of Z345 and Z346). ES-API: [M+H]⁺=553.2.

### Embodiment 62-64 Synthesis of Z347-1

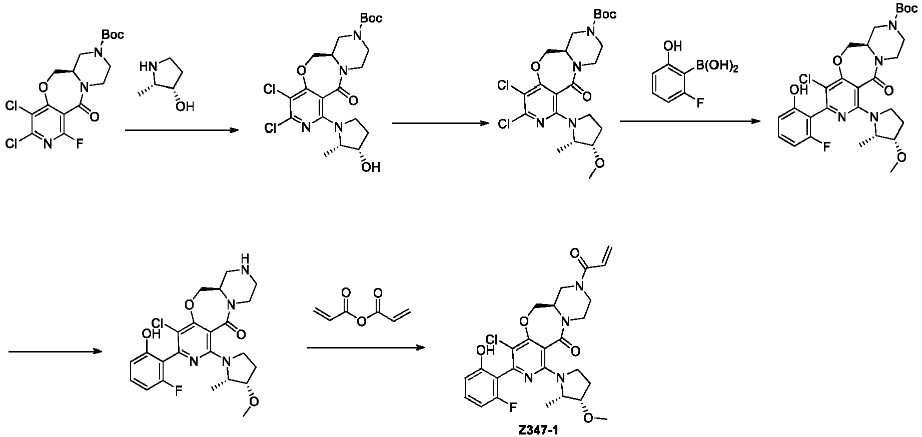

Step 1: (*R*)-*tert*-Butyl 3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (0.88 g, 2.2 mmol), (2*S*,3*S*)-2-methylpyrrolidin-3-ol (0.29 g, 2.9 mmol) and potassium carbonate (0.91 g, 6.6 mmol) were dissolved in acetonitrile (5 mL) and reacted at room temperature for 3 hours. After the reaction was completed, the mixture was added with ethyl acetate for extraction, dried, and concentrated under reduced pressure to obtain a product (0.7 g, crude product). ES-API: [M+H]⁺=487.2

Step 2: (*R*)-*tert*-Butyl 3,4-dichloro-1-((2*S*,3*S*)-3-hydroxy-2-methylpyrrolidin-1-yl)-12-oxo-6a,7,9,10-tetrahydro-6*H*-pyrazino[2,1-*c*]pyrido[3,4*-f*][1,4]oxazepine-8(12*H*-carboxylate (0.7 g, 1.4 mmol) was dissolved in tetrahydrofuran (5 mL), and sodium hydride (84 mg, 2.1 mmol) was added thereto under nitrogen protection at 0°C, and after stirring for 30 minutes, iodomethane (0.40 g, 2.8 mmol) was then added thereto and stirred overnight. After the reaction was completed, the reaction mixture was quenched by adding water, extracted with ethyl acetate, dried, evaporated to dryness, and purified by column chromatography (petroleum ether/ethyl acetate=3/1, R_{f}=0.6) to obtain a product (0.58 g, yield: 86%, white solid). ES-API: [M+H]⁺= 501.2

Step 3: (*R*)-*tert*-Butyl 3,4-dichloro-1-(((2*S*,3*S*)-3-methoxy-2-methylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (0.58 g, 1.2 mmol), (2-fluoro-6-hydroxyphenyl)boronic acid (0.25 g, 1.6 mmol), tetrakis(triphenylphosphine)palladium (138.7 mg, 0.12 mmol) and potassium carbonate (331.2 mg, 2.4 mmol) were mixed in a flask, and after argon protection, a mixed solvent of 1,4-dioxane (5 mL) and water (1 mL) was added thereto, and the reaction solution was reacted at 110°C overnight. After the reaction was completed, the reaction mixture was extracted with water and ethyl acetate, dried, evaporated to dryness, and purified by column chromatography (petroleum ether/ethyl acetate=1/1, R_{f}=0.6) to obtain a product (0.6 g, yield: 83%, white solid). ES-API: [M+H]⁺= 577.3

Step 4: (6a*R*)-*tert*-Butyl 4-chloro-3-(2-fluoro-6-hydroxyphenyl)-1-((2*S*,3*S*)-3-methoxy-2-methylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (0.6 g, 1.0 mmol) was dissolved in dichloromethane (3.0 mL), and TFA (1.0 mL) was added thereto at 0°C and the reaction solution was reacted at room temperature for 2 hours. After the reaction was completed, the mixture was added with saturated NaHCO₃ solution until no bubbles were generated, extracted by adding ethyl acetate, dried and concentrated to obtain a product (0.4 g, crude product). ES-API: [M+H]⁺= 477.2

Step 5: (6a*R*)-4-Chloro-3-(2-fluoro-6-hydroxyphenyl)-1-((2*S*,3*S*)-3-methoxy-2-methylpyrrolidin-1-yl)-7,8,9,10-tetrahydro-6*H*-pyrazino[2,1-c]pyrido[3,4-*f*][1,4]oxal-12(6a*H*)-one (0.4 g, 0.8 mmol) was dissolved in dichloromethane, and triethylamine (1.0 mL) and acrylic anhydride (50.4 mg, 0.4 mmol) were added thereto at 0°C. The reaction was stirred at room temperature for 2 hours. After the reaction was completed, the mixture was added with water and ethyl acetate for extraction, dried, concentrated, prepared and purified to obtain (6a*R*)-8-acryloyl-4-chloro-3-(2-fluoro-6-hydroxyphenyl)-1-((2*S*,3*S*)-3-methoxy-2-methylpyrrolidin-1-yl)-6,6a,7,8,9,10-hexahydro-12*H*-pyrazino[2,1-c]pyrido[3,4-*f*][1,4]oxazepin-12-one (Z347-1, 62.8 mg, 15%, white solid) (preparative separation conditions: chromatographic column information: Ultimate XB-C18,50 ^{∗}250 mm, 10 µm, mobile phase system: A: purified water B: pure acetonitrile, flow rate: 80 mL/min, gradient: within 40 minutes, B%=20%-100%, wavelength: 214 nm, column temperature: room temperature.) ¹H NMR (400 MHz, CDCl₃) δ 8.85-8.65 (brs, 1H), 7.30 - 7.25 (m, 1H), 6.82 (d, *J* = 8.0 Hz, 1H), 6.71 (d, *J* = 9.6 Hz, 1H), 6.56 (s, 1H), 6.44 - 6.38 (m, 1H), 5.88 - 5.75 (m, 1H), 4.53 - 4.34 (m, 4H), 4.27 - 4.09 (m, 3H), 4.09 - 3.87 (m, 2H), 3.77 - 3.60 (m, 3H), 3.38 (s, 3H), 3.08 - 2.96 (m, 1H), 2.29 - 2.18 (m, 1H), 2.11 - 1.97 (m, 1H), 1.18 (d, *J* = 6.1 Hz, 3H). ES-API: [M+H]⁺= 531.2

### Embodiment 62-65 Synthesis of Z360

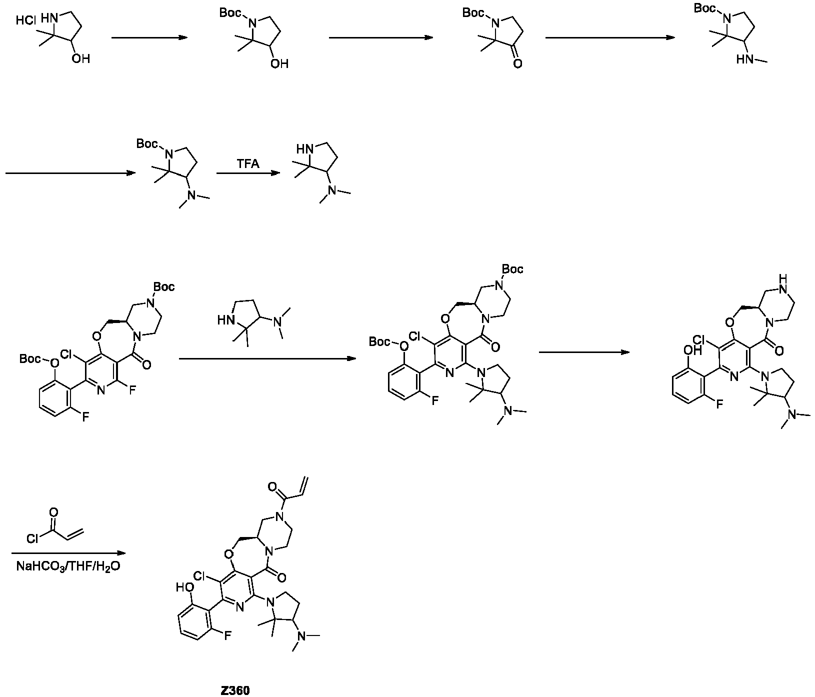

Step 1: 2,2-Dimethylpyrrolidin-3-ol hydrochloride (1 g, 6.6 mmol) was dissolved in dichloromethane (20 mL), and triethylamine (2 g, 19.8 mmol) and di-*tert*-butyl dicarbonate (2.8 g, 13.2 mmol) were added thereto, reacted at room temperature for 2 hours. The reaction solution was extracted with dichloromethane and water, and the organic phase was dried, evaporated to dryness, and purified by column chromatography (petroleum ether/ethyl acetate=5/1) to obtain a product (1.4 g, yield: 95%). (R_{f}=0.6, petroleum ether/ethyl acetate=3/1). ES-API: [M+1-56]⁺=160.2.

Step 2: *tert*-Butyl 3-hydroxy-2,2-dimethylpyrrolidine-1-carboxylate (1.3 g, 6.0 mmol) was dissolved in dichloromethane (30 mL), and Dess-Martin reagent (7.7 g, 18.1 mmol) was added thereto and reacted at room temperature for 2 hours. The reaction solution was quenched with aqueous sodium thiosulfate solution, extracted with dichloromethane, and the organic phase was dried, evaporated to dryness, and purified by column chromatography (petroleum ether/ethyl acetate=20/1, R_{f}=0.5) to obtain a product (1.3 g, yield: 95%).

Step 3: *tert*-Butyl 2,2-dimethyl-3-oxopyrrolidine-1-carboxylate (500 mg, 2.35 mmol) was added to titanium tetraisopropanolate (2 g, 7.0 mmol), and a tetrahydrofuran solution of methylamine (5.9 mL, 11.7 mmol, 2 M) was added thereto, and the tube was sealed and heated to 60°C and reacted for 8 hours, then reacted overnight at room temperature. Ethanol (6 mL) and sodium cyanoborohydride (739 mg, 11.7 mmol) were added to the reaction solution, and the reaction was carried out at room temperature for 2 hours. The reaction solution was quenched with water, extracted with ethyl acetate, and the organic phase was dried, evaporated to dryness, and purified by column chromatography (ethyl acetate) to obtain a product (67 mg, yield of 12%). (R_{f}=0.3, ethyl acetate). ES-API: [M+H]⁺=229.3.

Step 4: *tert*-Butyl 2,2-dimethyl-3-(methylamino)pyrrolidine-1-carboxylate (67 mg, 0.29 mmol) was dissolved in methanol (3 mL), and then 37% aqueous formaldehyde solution (0.6 mL) and sodium cyanoborohydride (56 mg, 0.88 mmol) were added thereto and reacted at room temperature overnight. The reaction solution was quenched with water, extracted with ethyl acetate, and the organic phase was dried and evaporated to dryness to obtain a crude product (70 mg). (R_{f}=0.6, ethyl acetate). ES-API: [M+H]⁺=243.3.

Step 5: *tert*-Butyl 3-(dimethylamino)-2,2-dimethylpyrrolidine-1-carboxylate (86 mg, 0.35 mmol) was dissolved in dichloromethane (2 mL), and then trifluoroacetic acid (1 mL) was added thereto and reacted at room temperature for 2 hours. The reaction solution was directly evaporated to dryness to obtain a crude product, which was directly used in the next reaction step.

Step 6: (6a*R*)-3-(2-((*tert*-Butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepine-8(12*H*)-carboxylate (207 mg, 0.35 mmol) was dissolved in acetonitrile (2 mL), then *N,N*,2,2-tetramethylpyrrolidin-3-amine (crude product, 0.35 mmol) and potassium carbonate (196 mg, 1.42 mmol) were added thereto, heated to 80°C and reacted overnight. The mixture was cooled to room temperature, and di-*tert*-butyl dicarbonate (309 mg, 1.42 mmol) was added thereto and stirred for 1 hour at room temperature. After the reaction was completed, the reaction mixture was quenched by adding water, extracted with ethyl acetate, dried, evaporated to dryness by rotary evaporation, and purified by column chromatography (dichloromethane/methanol=10/1, R_{f}=0.4) to obtain a product (36 mg, yield: 14%). ES-API: [M+H]⁺=704.4.

Step 7: (6a*R*)-*tert*-Butyl 3-(2-((*tert*-butoxycarbonyl)oxy)-6-fluorophenyl)-4-chloro-1-(3-(dimethylamino)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (36 mg, 0.05 mmol) was dissolved in dichloromethane (1 mL), and then trifluoroacetic acid (1 mL) was added thereto. The reaction solution was reacted at room temperature for 2 hours. After TLC monitoring that the reaction was completed, the reaction solution was evaporated to dryness, dissolved in dichloromethane and evaporated to dryness to obtain a crude product, which was directly used in the next reaction step. ES-API: [M+H]⁺=504.3.

Step 8: (6a*R*)-4-Chloro-1-(3-(dimethylamino)-2,2-dimethylpyrrolidin-1-yl)-3-(2-fluoro-6-hydroxyphenyl)-7,8,9,10-tetrahydro-6*H*-pyrazino[2,1-*c*]pyrido [3,4-*f*][1,4]oxazepin-12(6a*H*)-one (crude product, 0.05 mmol) was dissolved in tetrahydrofuran/water (1 mL/0.5 mL), cooled to 0 to 5°C, and sodium bicarbonate (43 mg, 0.5 mmol) and potassium phosphate (5 mg, 0.025 mmol) were added thereto. A solution of acryloyl chloride (5 mg, 0.05 mmol) in tetrahydrofuran was added dropwise thereto under an ice bath and stirred for 0.5 hours. After the reaction was completed, the mixture was extracted with ethyl acetate and water, and the organic phase was dried and evaporated to dryness by rotary evaporation to obtain a crude product of (6a*R*)-8-acryloyl-4-chloro-1-(3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z360).

### Embodiment 62-66 Synthesis of Z452

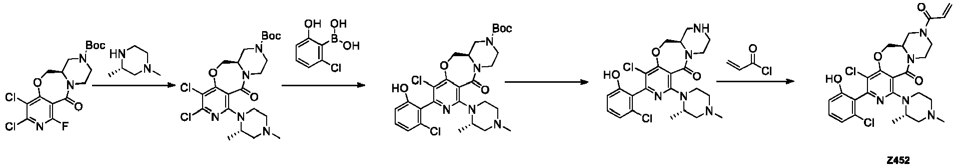

Step 1: *tert*-Butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (1.5 g, 3.7 mmol), (*S*)-1,3-dimethylpiperazine (422 mg, 3.7 mmol) and potassium carbonate (1.5 g, 11.1 mmol) were dissolved in acetonitrile (10 mL) and reacted at room temperature overnight. The completion of reaction was confirmed by LC-MS, and the mixture was quenched with water, extracted with ethyl acetate, washed with brine, dried over sodium sulfate, and then subjected to thin-layer chromatographic column chromatography (methanol/dichloromethane=1/10) to obtain a product of *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (1.1 g, Y: 59.52%). ES-API: [M+H]⁺= 501.42.

Step 2: Under nitrogen protection, *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (100 mg, 0.2 mmol), (2-chloro-6-hydroxyphenyl)boronic acid (100 mg, 0.58 mmol), potassium carbonate (83 mg,0.6 mmol) and Pd(PPh₃)₄ (20 mg) were dissolved in dioxane (2 mL) and water (0.2 mL), then heated to 100°C and reacted overnight. Then after the reaction was completed, the mixture was quenched with water, extracted with ethyl acetate, washed with brine, dried over sodium sulfate, and then subjected to thin-layer chromatographic column chromatography (methanol/dichloromethane=1/10) to obtain a product of *tert-*butyl (6a*R*)-4-chloro-3-(2-chloro-6-hydroxyphenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (70 mg, Y: 59.10%). ES-API: [M+H]⁺= 592.52.

Step 3: *tert*-Butyl (6a*R*)-4-chloro-3-(2-chloro-6-hydroxyphenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (70 mg, 0.12 mmol) was dissolved in dichloromethane (2 mL), then trifluoroacetic acid (2 mL) was added thereto, reacted at room temperature for 1 hour. After the reaction was completed, the mixture was evaporated to dryness by rotary evaporation to obtain a product of (6a*R*)-4-chloro-3-(2-chloro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (70 mg, Y: 100%). ES-API: [M+H]⁺= 492.15.

Step 4: (6a*R*)-4-Chloro-3-(2-chloro-6-hydroxyphenyl)-1-((*S*)-2,4-dimethylpiperazin-1-yl)-6,6a,7,8,9,10-hexahydro-12*H*-pyrazino[2,1-*c*]pyrido[3,4*-f*][1,4]oxazepin-12-one (70 mg, 0.14 mmol) was dissolved in tetrahydrofuran (5 mL), then cooled to 0°C. Saturated sodium bicarbonate (2 mL) was added dropwise to the reaction solution to adjust to pH to 8-9, then acryloyl chloride (19 mg, 0.21 mmol) was slowly added dropwise to the solution, stirred for 10 minutes. The completion of reaction was confirmed by LC-MS, then the reaction mixture was quenched by adding saturated sodium bicarbonate, extracted with ethyl acetate, washed with brine, dried over sodium sulfate, and then prepared and purified to obtain a product of (6a*R*)-8-acryloyl-4-chloro-3-(2-chloro-6-hydroxyphenyl)-1-((*S*)-2,4-dimethylpiperazin-1-yl)-6,6a,7,8,9,10-hexahydro-12*H*-pyrazino[2,1-*c*]pyrido[3,4*-f*][1,4]oxazepin-12-one (Z452, 20 mg, Y: 32.38%). (Preparative separation conditions: chromatographic column information: Ultimate XB-C18, 50*250 mm, 10 µm, mobile phase system: A: purified water B: pure acetonitrile, flow rate: 80 mL/min, gradient: within 50 minutes, B/A= 20%-90%, wavelength: 214 nm, column temperature: room temperature) ¹H NMR (400 MHz, CDCl₃) δ 7.26 - 7.19 (m, 2H), 7.05 - 7.01 (m, 1H), 6.92 (ddd, *J* = 19.5, 8.3, 1.1 Hz, 1H), 6.65 - 6.34 (m, 2H), 5.82 (d, *J* = 10.4 Hz, 1H), 4.38 (s, 3H), 4.26 (d, *J* = 17.6 Hz, 3H), 4.17 - 4.08 (m, 1H), 3.78 (s, 1H), 3.59 (d, *J* = 14.2 Hz, 2H), 3.50 (d, *J* = 13.4 Hz, 1H), 3.39 (d, *J* = 11.7 Hz, 1H), 2.73 (t, *J* = 11.5 Hz, 1H), 2.59 (d, *J* = 10.7 Hz, 1H), 2.27 (s, 2H), 2.25 (s, 3H), 1.40 - 1.34 (m, 3H). ES-API: [M+H]⁺= 546.2.

### Embodiment 62-67 Synthesis of Z462

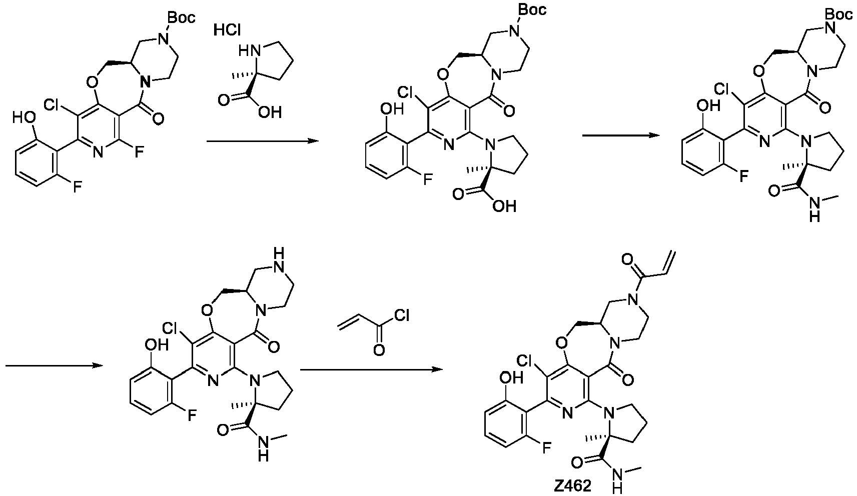

Step 1: *tert*-Butyl (6aR)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.42 mmol) and 2 mL of acetonitrile were added to around bottom flask. Potassium carbonate (171 mg, 1.24 mmol) and (S)-2-methyltetrahydropyrrole-2-carboxylate (138 mg, 0.84 mmol) were then added to the reaction solution. The reaction was stirred at 80°C overnight. After the reaction was completed, the mixture was quenched with water, and the aqueous phase was extracted twice with ethyl acetate, and the organic phase was concentrated to dryness to obtain a crude product of (2*S*)-1-((6a*R*)-8-(*tert*-butoxycarbonyl)-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-2-methyltetrahydropyrrole-2-carboxylic acid (crude product) as a yellow solid, and the crude product was directly used in the next reaction step. ES-API: [M+H]⁺=591.1.

Step 2: (2*S*)-1-((6a*R*)-8-(*tert*-Butoxycarbonyl)-4-chloro-3-(2-fluoro-6-hydroxyphenyl)-12-oxo-6,6a,7,8,9,10-hexahydro-12*H*-pyrazino[2,1-*c*]pyrido[3.4-*f*][1,4]oxazepin-1-yl)-2-methyltetrahydropyrrole-2-carboxylic acid (crude product from the previous step), methylamine hydrochloride (56 mg, 0.82 mmol), diisopropylethylamine (0.460 mL, 2.80 mmol) and DMF (4 mL) were added to a round bottom flask, and HATU (304 mg, 0.80 mmol) was added thereto under stirring, reacted for 1 hour and then quenched with water. The reaction solution was extracted twice with ethyl acetate, and the organic phase was washed once with 1 M HCl solution, saturated sodium bicarbonate solution, and saturated brine, and dried over anhydrous sodium sulfate. The organic phase was concentrated to dryness to obtain a crude product of *tert*-butyl (6a*R*)-4-chloro-3-(2-fluoro-6-hydroxyphenyl)-1-((*S*)-2-methyl-2-(methylcarbamoyl)tetrahydropyrrol-1-yl)-12-oxo-6a,7,9,10-tetrahydro-12*H*-pyrazino[2,1-*c*]pyrido[3,4-*f*][1,4]oxazepine-8(6*H*)-carboxylate (250 mg, crude product), and the crude product was directly used in the next reaction step. ES-API: [M+H]⁺=604.2.

Step 3: *tert*-Butyl (6a*R*)-4-chloro-3-(2-fluoro-6-hydroxyphenyl)-1-((*S*)-2-methyl-2 (methylcarbamoyl)tetrahydropyrrol-1-yl)-12-oxo-6a,7,9,10-tetrahydro-1*2H*-pyrazino[2,1-*c*]pyrido[3,4-*f*][1,4]oxazepine-8(6*H*)-carboxylate (65 mg, crude product) was dissolved in 1 mL of dichloromethane and 1 mL of trifluoroacetic acid. The mixture was stirred at room temperature for 1 hour, and the completion of reaction was detected by LC-MS. The reaction solution was concentrated to obtain (2*S*)-1-((6a*R*)-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide (70 mg, crude product). The crude product was directly used in the next step. ES-API: [M+H]⁺=504.2.

Step 4: (2*S*)-1-((6aR)-4-Chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide (70 mg, crude product), potassium phosphate (11 mg, 0.05 mmol), tetrahydrofuran (2 mL) and water (1 mL) were added to a round bottom flask. The reaction was cooled to 0°C, and acryloyl chloride (4.5 mg, 0.05 mmol) was added dropwise to the reaction solution. The reaction was stirred at 0°C for 10 minutes. 20 mL of saturated aqueous sodium bicarbonate solution was added to the reaction solution, and the mixture was extracted twice with 20 mL of dichloromethane. The organic phase was dried and concentrated, and the crude product was purified by preparative HPLC to obtain (2*S*)-1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide (Z462, 5.57 mg, 10%). ¹H NMR (400 MHz, DMSO-*d*₆) δ 9.81 (s, 1H), 7.22 (dd, *J* = 15.3, 8.3 Hz, 1H), 6.97 - 6.59 (m, 4H), 6.18 (d, *J* = 16.8 Hz, 1H), 5.85 - 5.68 (m, 1H), 4.65 - 4.50 (m, 1H), 4.44 - 3.45 (m, 9H), 3.11 (s, 1H), 2.44 - 2.38 (m, 3H), 2.05 - 1.79 (m, 4H), 1.59 (s, 3H). ES-API: [M+H]⁺=558.2.

### Embodiment 62-68 Synthesis of Z369

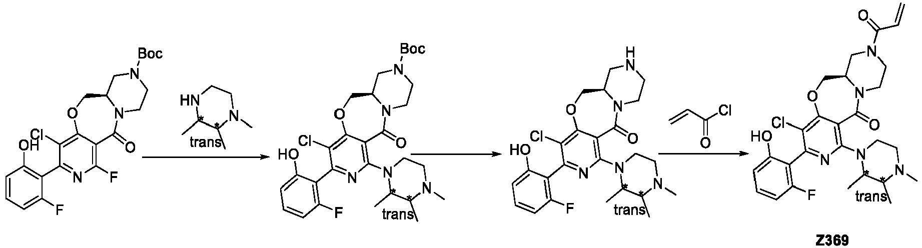

Step 1: *tert*-Butyl (6a*R*)-4-chloro-1-fluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (240 mg, 0.5 mmol), 1,2,3-trimethylpiperazine (255 mg, 2.0 mmol), *N,N-*diisopropylethylamine (193 mg, 1.5 mmol) and acetonitrile (5 mL) were added to a 50 mL round bottom flask, reacted at 90°C for 16 hours. The reaction solution was added with ethyl acetate (20 mL), washed with water (20 mL^{∗}3), and the organic phase was dried and concentrated, and the residue was purified by flash silica gel column (ethyl acetate:petroleum ether: 0-90%) to obtain *tert*-butyl (6a*R*)-4-chloro-3-(2-fluoro-6-hydroxyphenyl)-12-oxo-1-(2,3,4-trimethylpiperazin-1-yl)-6a,7,9,10-tetrahydro-12*H*-pyrazino[2,1-*c*]pyrido[3,4-*f*][1,4]oxazepine-8(6*H*)-carboxylate (77 mg, yield of 62%). ES-API: [M+H]⁺=590.3.

Step 2: Trifluoroacetic acid (2 mL) was added to a solution of *tert*-butyl (6a*R*)-4-chloro-3-(2-fluoro-6-hydroxyphenyl)-12-oxo-1-(2,3,4-trimethylpiperazin-1 -yl)-6a,7,9,10-tetrahydro-12*H*-pyrazino [2,1-*c*]pyrido[3,4-*f*][1,4]oxazepine-8(6*H*)-carboxylate (77 mg, 0.13 mmol) in dichloromethane (5 mL), reacted at room temperature for 1 hour. The reaction solution was directly concentrated to obtain a crude procuct of (6a*R*)-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one (61 mg). ES-API: [M+H]⁺=490.2.

Step 3: The above crude product of (6a*R*)-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one (61 mg, 0.12 mmol) was dissolved in dichloromethane (5 mL), and *N,N*-diisopropylethylamine (46 mg, 0.36 mmol) was added thereto. The reaction solution was cooled to 0°C, and acryloyl chloride (9 mg, 0.10 mmol) was added dropwise thereto. After stirring for 5 minutes, the mixture was added with methanol (1 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3)-(2-fluoro-6-hydroxyphenyl)-1-(2,3,4-trimethylpiperazin-1-yl-6,6a,7,8,9,10-hexahydro-12*H*-pyrazino[2,1-*c*]pyrido[3,4-*f*][1,4]oxazepin-12-one (Z369, 20 mg, yield: 28%). ES-API: [M+H]⁺=544.2.

### Embodiment 62-69 Synthesis of Z448

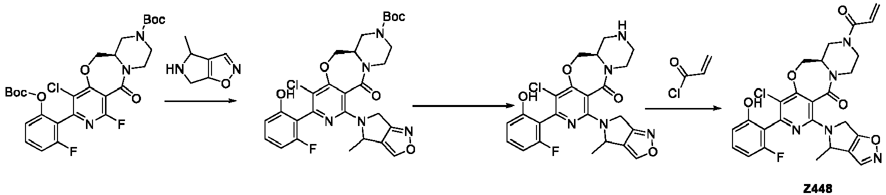

Step 1: (6aR)-8-tert-Butoxycarbonyl-3-(2-((*tert*-butoxycarbonyl)oxy)-6-fluorophenyl)-4-chloro-1-fluoro-6,6a,7,8,9,10-hexahydro-12*H*-pyrazino[2,1-*c*]pyrido[3,4- f][1,4]oxazepin-12-one (101 mg, 0.175 mmol), 4-methyl-5,6-dihydro-4*H*-pyrrolo[3,4-*d*]isoxazole hydrochloride (28 mg, 0.175 mmol), DIPEA (112 mg, 0.875 mmol) and acetonitrile (5 mL) were added to a 100 mL round bottom flask. The reaction was stirred at 90 °C for 1 hour. The mixture was extracted with ethyl acetate, washed with aqueous ammonium chloride solution and saturated brine in turn, and the organic phase was dried and concentrated, and purified by silica gel column to obtain (6a*R*)-8-*tert*-butoxycarbonyl-1-(4-methyl-4*H*-pyrrolo[3,4-*c*]isoxazol-5(6*H*)-yl)-4-chloro-3-(2-fluoro-6-hydroxyphenyl)-6,6a,7, 8,9,10-hexahydro-12*H*-pyrazino [2,1-*c*]pyrido [3,4-*f*][1,4]oxazepin-12-one (30 mg, yield of 29%). ES-API: [M+H]⁺=586.1.

Step 2: (6a*R*)-8-*tert*-Butoxycarbonyl-1-(4-methyl-4*H*-pyrrolo[3,4-*c*]isoxazol-5(6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, 0.051 mmol), 1 mL of trifluoroacetic acid and 2 mL of dichloromethane were added to a 50 mL round bottom flask. The mixture was stirred at room temperature for 1 hour, and the completion of reaction was detected by LC-MS. The reaction solution was concentrated to obtain (6a*R*)-4-chloro-3-(2-fluorophenyl)-1-(4-methyl-4*H*-pyrrolo[3,4-*c*]isoxazol-5(6*H*-yl)-6,6a,7,8,9,10-hexahydro-12*H-*pyrazino[2,1-*c*]pyrido[3,4-*f*][1,4]oxazepin-12-one (crude product), which was directly used in the next step.

Step 3: (6a*R*)-4-Chloro-3-(2-fluorophenyl)-1-(4-methyl-4*H*-pyrrolo[3,4-*c*]isoxazol-5(6*H*)-yl)-6,6a,7,8,9,10-hexahydro-12*H*-pyrazino[2,1-*c*]pyrido[3,4-*f*][1,4]oxazepin-12-one (crude product) was dissolved in dichloromethane (5 mL), and triethylamine (32 mg, 0.246 mmol) was added thereto. Acryloyl chloride (7.5 mg, 0.082 mmol) was added dropwise thereto at 0°C. The reaction was stirred at 0°C for 15 minutes. The reaction solution was added with 10 mL of dichloromethane, washed with 10 mL of saturated aqueous NaHCO₃ solution and 10 mL of saturated brine, dried and concentrated. The crude product was purified by HPLC to obtain (6a*R*)-8-acryloyl-4-chloro-3-(2-fluorophenyl)-1-(4-methyl-4H-pyrrolo[3,4-*c*]isoxazol-5(6*H*)-yl)-6,6a,7,8,9,10-hexahydro-12*H*-pyrazino[2,1-*c*]pyrido[3,4-*f*][1,4]oxazepin-12-one (Z448, 0.5 mg, yield of 1.8%). ES-API: [M+H]⁺=540.1.

### Embodiment 63 Synthesis of (6aR)-8-acryloyl-4-chloro-3-(2-fluorophenyl)-1-(4-hydroxy-2,2-dimethylpyrrolidin-1-yl)-6,6a,7,8,9,10-hexahydro-12H-pyrazino[2,1-c]pyrido[3,4-f][1,4]oxazepin-12-one Z325

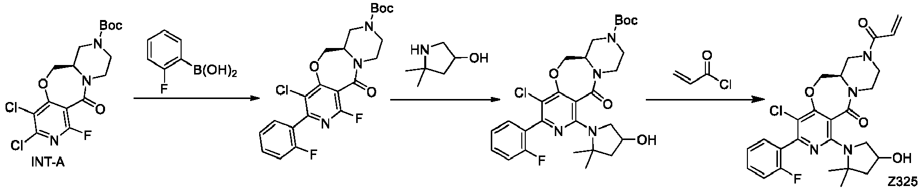

Step 1: *tert*-Butyl (*R*)-3,4-dichloro-1-fluoro-12-oxo-6a,7,9,10-tetrahydro-12*H*-pyrazino[2,1-*c*]pyrido[3,4-*f*][1,4]-8(6*H*)-carboxylate (2 g, 4.93 mmol), (2-fluorophenyl)boronic acid (2.8 g, 20 mmol), tetrakis(triphenylphosphine)palladium (0.58 g, 0.05 mmol), sodium carbonate (1.6 g, 15 mmol), 30 mL of dioxane and 5 mL of water were added to a 50 mL flask. Under nitrogen protection, the reaction was carried out at 100°C for 2 hours. The reaction solution was added with 50 mL of ethyl acetate, washed with 30 mL of saturated brine for 3 times, dried and concentrated, and the crude product was purified by flash silica gel column to obtain *tert*-butyl (6a*R*)-4-chloro-1-fluoro-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (1.8 g, yield of 77%). ES-API: [M+H]⁺=466.1.

Step 2: *tert*-Butyl (6aR)-4-chloro-1-fluoro-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (180 mg, 0.38 mmol) was dissolved in 10 mL of acetonitrile, and DIPEA (200 mg, 1.5 mmol) and 5,5-dimethylpyrrolidin-3-ol (85 mg, 0.74 mmol) were added thereto. The reaction was carried out at 90°C for 16 hours. The reaction solution was added with 30 mL of ethyl acetate, washed with 30 mL of saturated brine for 3 times, dried and concentrated, and the crude product was purified by flash silica gel column to obtain tert-butyl (6a*R*)-4-chloro-3-(2-fluorophenyl)-1-(4-hydroxy-2,2-dimethylpyrrolidin-1-yl)-12-oxo-6a,7,9,10-tetrahydro-12*H*-pyrazino [2,1-*c*]pyrido [3,4-*f*][1,4]oxazepine-8(6*H*)-carboxylate (200 mg, yield of 94%). ES-API: [M+H]⁺=561.3.

Step 3: *tert*-Butyl (6a*R*)-4-chloro-3-(2-fluorophenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.36 mmol) was dissolved in dichloromethane (8 mL), and trifluoroacetic acid (4 mL) was added thereto. After stirring at room temperature for 1 hour, the reaction solution was concentrated to obtain a target intermediate. The intermediate was dissolved in dichloromethane (15 mL), and triethylamine (400 mg, 4.0 mmol) was added thereto. Acryloyl chloride (20 mg, 0.22 mmol) was added dropwise thereto at 0°C. The reaction was stirred at 0°C for 15 minutes. The reaction solution was added with 40 mL of dichloromethane, washed with 50 mL of saturated aqueous NaHCO₃ solution and 40 mL of saturated brine, dried and concentrated. The crude product was purified by flash silica gel column to obtain (6a*R*)-8-acryloyl-4-chloro-3-(2-fluorophenyl)-1-(4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z325, 123 mg,yieldof66%). ES-API: [M+H]⁺=515.1.

### Embodiment 63-1 Synthesis of Z147

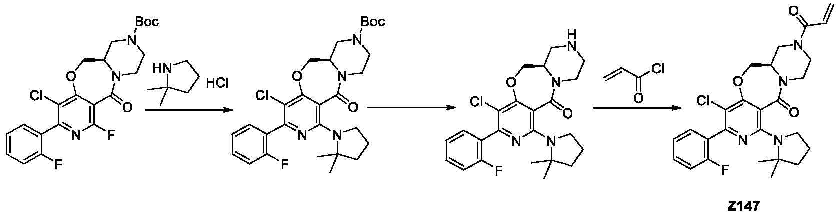

Step 1: *tert*-Butyl (*R*)-4-chloro-1-fluoro-3-(2-fluorophenyl)-12-oxo-6a,7,9,10-tetrahydro-12*H-*pyrazino[2,1-*c*]pyrido[3,4*-f*][1,4]oxazepine-8(6*H*)-carboxylate (466 mg, 1.0 mmol), 2,2-dimethylpyrrolidine hydrochloride (136 mg, 1.0 mmol), DIPEA (516 mg, 4.0 mmol) and acetonitrile (10 mL) were added to a 50 mL round bottom flask, reacted at 90°C for 1 hour. The reaction solution was added with ethyl acetate (30 mL), washed with water (30 mL^{∗}3), and the organic phase was dried and concentrated, and the residue was purified by flash silica gel column (ethyl acetate:petroleum ether: 0-90%) to obtain *tert-*butyl (*R*)-4-chloro-1-(2,2-dimethylpyrrolidin-1-yl)-3-(2-fluorophenyl)-12-oxo-6a,7,9,10-tetrahydro-12*H-*pyrazinol[2,1-*c*]pyrido[3,4-*f*][1,4]oxazepine-8(6*H*)-carboxylate (70 mg, yield of 13%) as a solid. ES-API: [M+H]⁺=545.2.

Step 2: Trifluoroacetic acid (2 mL) was added to a solution of *tert*-butyl (*R*)-4-chloro-1-(2,2-dimethylpyrrolidin-1-yl)-3-(2-fluorophenyl)-12-oxo-6a,7,9,10-tetrahydro-12*H*-pyrazinol [2,1-*c*]pyrido [3,4-*f*][1,4]oxazepine-8(6*H*)-carboxylate (70 mg, 0.12 mmol) in dichloromethane (5 mL), reacted at room temperature for 1 hour. The reaction solution was directly concentrated to obtain a crude product of (*R*)-4-chloro-1-(2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (55 mg) as an oil. ES-API: [M+H]⁺=445.2.

Step 3: The above crude procuct of (*R*)-4-chloro-1-(2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (55mg, 0.13 mmol) was dissolved in dichloromethane (2 mL), and *N,N-*diisopropylethylamine (51 mg, 0.39 mmol) was added thereto. The reaction solution was cooled to 0°C, and acryloyl chloride (9 mg, 0.10 mmol) was added dropwise thereto. After stirring for 5 minutes, the mixture was added with methanol (2 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-4-chloro-1-(2,2-dimethylpyrrolidin-1-yl)-3-(2-fluorophenyl)-6,6a,7,8,9,10-hexahydro-12*H*-pyrazino[2,1-*c*]pyrido[3,4-*f*[1,4]oxazepin-12-one (Z147, 39.1 mg, yield: 60%). ES-API: [M+H]⁺=499.1.

### Embodiment 63-2 Synthesis of Z97a

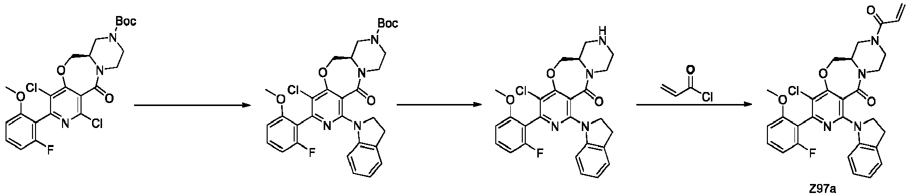

Step 1: Indoline (41 mg, 0.35 mmol) and tetrahydrofuran (5 mL) were added to a 100 mL reaction flask, and then the solution was cooled to 0°C in an ice bath, and sodium hydride (23.4 mg, 0.59 mmol) was slowly added thereto. The mixture was stirred for 0.5 hours, and then *tert*-butyl (6a*R*)-1,4-dichloro-3-(2-fluoro-6-methoxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.39 mmol) was slowly added thereto. The reaction was stirred in an ice bath at 0°C for 2 hours, and the reaction was stopped. The reaction was added with 20 mL of ice-water mixture, stirred for 5 minutes, then extracted three times with 20 mL of ethyl acetate. The organic phase was dried and concentrated, and the crude product was purified by flash silica gel column (ethyl acetate/petroleum ether: 0-60%) to obtain *tert*-butyl (6a*R*)-4-chloro-3-(2-fluoro-6-methoxyphenyl)-1-(indolin-1-yl)-12-oxo-6a,7,9,10-tetrahydro-12*H*-pyrazino[2,1-*c*]pyrido[3,4-*f*][1,4]oxazepine-8(6*H*)-carboxylate (190 mg, 81%) as a yellow solid. ES-API: [M+H]⁺=595.2.

Step 2: The solid *tert*-butyl (6a*R*)-4-chloro-3-(2-fluoro-6-methoxyphenyl)-1-(indolin-1-yl)-12-oxo-6a,7,9,10-tetrahydro-12*H*-pyrazino [2,1-*c*]pyrido [3,4-*f*[1,4]oxazepine-8(6*H*)-carboxylate (190 mg, 0.32 mmol), dichloromethane (3 mL) and trifluoroacetic acid (2 mL) were added to a 50 mL round bottom flask. The mixture was stirred at room temperature for 1 hour, and the completion of reaction was detected by LC-MS. The reaction solution was concentrated to obtain a yellow oily crude product of (6a*R*)-4-chloro-3-(2-fluoro-6-methoxyphenyl)-1-(indolin-1-yl)-12-oxo-6a,7,9,10-tetrahydro-12*H*-pyrazino [2,1-*c*]pyrido[3,4-*f*][1,4]oxazepin-12-one (210 mg). ES-API: [M+H]⁺=495.2.

Step 3: A crude product of (6a*R*)-4-chloro-3-(2-fluoro-6-methoxyphenyl)-1-(indofin-1-yl)-12-oxo-6a,7,9,10-tretahydro-12*H*-pyrazino[2,1-*c*]pyrido[3,4-*f*][1,4]oxazepin-12-one (210 mg), dichloromethane (5 mL) and *N*-ethyl-*N*-isopropylpropan-2-amine (128.5 mg, 0.99 mmol) were added to a 100 mL eggplant-shaped flask. The reaction was cooled to 0°C, and a solution of acrylic anhydride in dichloromethane (24.3 mg, 0.27 mmol, 0.5 mL) was added dropwise to the reaction solution, stirred at 0°C for 10 minutes, and then the mixture was added with 20 mL of saturated aqueous sodium bicarbonate solution, extracted three times with 10 mL of dichloromethane. The organic phase was dried and concentrated, and the crude product was purified by basic preparative HPLC to obtain (6a*R*)-8-acryloyl-4-chloro-3-(2-fluoro-6-methoxyphenyl)-1-(indolin-1-yl)-6,6a,7, 8,9,10-hexahydro-12*H*-pyrazino [2,1-c]pyrido [3,4-*f*][1,4]oxazepin-12-one (Z97a, 3.5 mg, 1.0%) as a yellow solid. ES-API: [M+H]⁺= 549.2.

### Embodiment 63-3 Synthesis of a mixture of Z340 and Z341

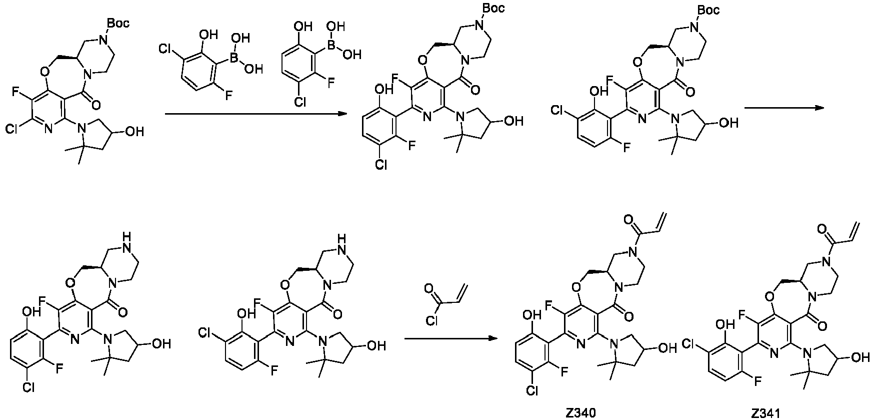

Step 1: (6a*R*)-8-*tert*-Butoxycarbonyl-1-(4-hydroxy-2,2-dimethylpyrrolidinyl)-4-fluoro-3-chloro-6,6a,7,8,9,10-hexahydro-12*H*-pyrazino[2,1-*c*]pyrido[3,4-*f*][1,4]oxazepin-12-one (300 mg, 0.62 mmol), a mixture of (3-chloro-6-fluoro-2-hydroxyphenyl)boronic acid and (3-chloro-2-fluoro-6-hydroxyphenyl)boronic acid (353 mg, 1.86 mmol), Sphos-Pd-G2 (89 mg, 0.124 mmol) and potassium carbonate (257 mg, 1.86 mmol) were added to a mixed solution of 1,4-dioxane (8 mL) and water (2 mL) into a 50 mL round bottom flask, and the system was replaced with nitrogen for 3 times, and the reaction was carried out at 110°C for 1 hour under microwave irradiation. The mixture was added with ethyl acetate, washed with water and saturated brine in turn, and dried over anhydrous sodium sulfate. The mixture was concentrated, and purified by silica gel (petroleum ether/ethyl acetate=10:90) to obtain a mixture of (6a*R*)-8-*tert*-butoxycarbonyl-1-(4-hydroxy-2,2-dimethylpyrrolidin-1-yl)-4-fluoro-3-(3-chloro-6-fluoro-2-hydroxyphenyl)-6,6a,7,8,9,10-hexahydro-12*H*-pyrazino[2,1-*c*]pyrido[3,4-*f*][1,4]oxazepin-12-one and (6a*R*)-8-*tert*-butoxycarbonyl-1-(4-hydroxy-2,2-dimethylpyrrolidin-1-yl)-4-fluoro-3-(3-chloro-2-fluoro-6-hydroxyphenyl)-6,6a,7,8,9,10-hexahydro-12*H*-pyrazino[2,1-*c*]pyrido[3,4-*f*][1,4]oxazepin-12-one (300 mg, yield of 96%). ES-API: [M+H]⁺=595.2.

Step 2: The above mixture (300 mg, 0.505 mmol) was dissolved in dichloromethane (5 mL), and trifluoroacetic acid (3 mL) was added thereto. The mixture was stirred at room temperature for 1 hour, and the completion of reaction was detected by LC-MS. The reaction solution was concentrated to obtain a crude product of a mixture of (6a*R*)-1-(4-hydroxy-2,2-dimethylpyrrolidin-1-yl)-4-fluoro-3-(3-chloro-6-fluoro-2-hydroxyphenyl)-6,6a,7,8,9,10-hexahydro-12*H*-pyrazino[2,1-*c*]pyrido[3,4-*f*][1,4]oxazepin-12-one and (6a*R*)-1-(4-hydroxy-2,2-dimethylpyrrolidin-1-yl)-4-fluoro-3-(3-chloro-2-fluoro-6-hydroxyphenyl)-6,6a,7,8,9,10-hexahydro-12*H*-pyrazino[2,1-*c*]pyrido[3,4-*f*][1,4]oxazepin-12-one (250 mg, trifluoroacetate). ES-API: [M+H]⁺=495.2.

Step 3: The above mixture (250 mg, trifluoroacetate), 10 mL of dichloromethane and *N,N-*diisopropylethylamine (193 mg, 1.5 mmol) were added to a 50 mL round bottom flask. The reaction was cooled to 0°C, and a solution of acryloyl chloride in dichloromethane (45 mg, 0.5 mmol) was added dropwise to the reaction solution. The reaction was stirred at 0°C for 10 minutes. The mixture was washed with 5 mL of water, and the organic phase was dried and concentrated to obtain a crude product of a mixture of (6a*R*)-8-acryloyl-1-(3-hydroxy-5,5-dimethylpyrrolidin-1-yl)-4-fluoro-3-(3-chloro-2-fluoro-6-hydroxyphenyl)-6,6a,7,8,9,10-hexahydro-12*H*-pyrazino[2,1-*c*]pyrido[3,4-*f*][1,4]oxazepin-12-one and (6a*R*)-8-acryloyl-1-(3-hydroxy-5,5-dimethylpyrrolidin-1-yl)-4-fluoro-3-(3-chloro-6-fluoro-6-hydroxyphenyl)-6,6a,7,8,9,10-hexahydro-12*H*-pyrazino[2,1-*c*]pyrido[3,4-*f*][1,4]oxazepin-12-one (mixture of Z340 and Z341). ES-API: [M+H]⁺=549.1.

### Embodiment 63-4 Synthesis of Z463

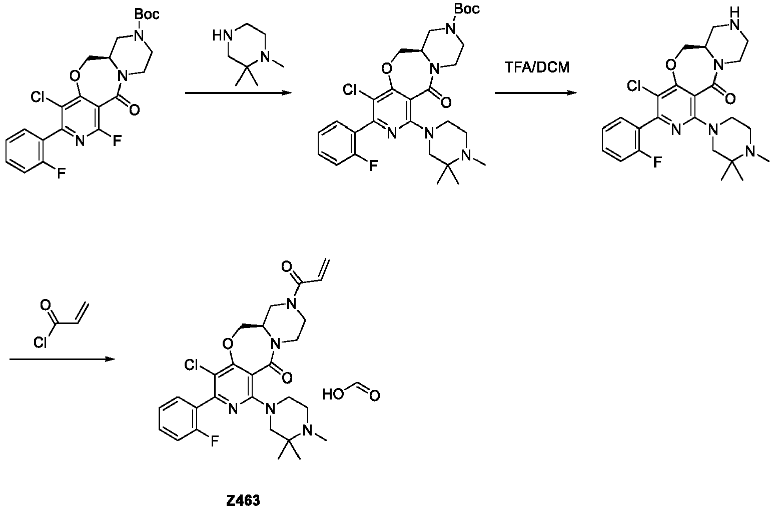

Step 1: *tert*-Butyl (*R*)-4-chloro-1-fluoro-3-(2-fluorophenyl)-12-oxo-6a,7,9,10-tetrahydro-12*H-*pyrazino[2,1-c]pyrido[3,4-*f*][1,4]oxazepine-8(6*H*)-carboxylate (80 mg, 0.172 mmol) was dissolved in acetonitrile (2 mL), and *N,N-*diisopropylethylamine (66 mg, 0.515 mmol) and 1,2,2-trimethylpiperazine (24 mg, 0.189 mmol) were added thereto. The reaction solution was reacted at 90°C for 4 hours. The reaction solution was concentrated, and the crude product was purified by flash silica gel column (4 g, ethyl acetate/petroleum ether=3%-10%) to obtain *tert*-butyl (*R*)-4-chloro-3-(2-fluorophenyl)-12-oxo-1-(3,3,4-trimethylpiperazin-1-yl)-6a,7,9,10-tetrahydro-12*H*-pyrazino[2,1-*c*]pyrido[3,4-*f*][1,4]oxazepine-8(6*H-*carboxylate (90 mg, 0.157 mmol, yield of 81%). ES-API: [M+H]⁺=574.3.

Step 2: *tert*-Butyl (*R*)-4-chloro-3-(2-fluorophenyl)-12-oxo-1-(3,3,4-trimethylpiperazin-1-yl)-6a,7,9,10-tetrahydro-12*H*-pyrazino[2,1-*c*]pyrido[3,4-*f*][1,4]oxazepine-8(6*H*)-carboxylate (90 mg, 0.157 mmol) was dissolved in dichloromethane (1 mL) and trifluoroacetic acid (0.5 mL) was added thereto. The reaction was stirred at room temperature for 1 hour. The reaction solution was concentrated and then redissolved with tetrahydrofuran (2 mL), and the pH of the resulting solution was adjusted to 7 with *N,N-*diisopropylethylamine. A solution of (*R*)-4-chloro-1-fluoro-3-(2-fluorophenyl)-1-(3,3,4-trimethylpiperazin-1-yl)-6,6a,7,8,9,10-hexahydro-12*H*-pyrazinopyrido[3,4-*f*][1,4]oxazepin-12-one (74.3 mg, 0.157 mmol, yield of 100%) was obtained and directly used in the next step.

Step 3: Acryloyl chloride (20 mg, 0.22 mmol) was added to a solution of (*R*)-4-chloro-1-fluoro-3-(2-fluorophenyl)-1-(3,3,4-trimethylpiperazin-1-yl)-6,6a,7,8,9,10-hexahydro-12*H*-pyrazinopyrido[3,4-*f*][1,4]oxazepin-12-one (74.3 mg, 0.157 mmol) from step 2 in tetrahydrofuran. The reaction was stirred at 0°C for 15 minutes. After the reaction solution was concentrated, the resulting residue was subjected to preparative HPLC (column model: Waters XBridge C18, 190^{∗}250 mm, 5 µm; mobile phase system: A: 0.1% aqueous formic acid solution; B: preparative grade acetonitrile; flow rate: 15 mL/min; B%=20%-100%; column temperature: room temperature) to obtain (*R*)-8-acryloyl-4-chloro-3-(2-fluorophenyl)-1-(3,3,4-trimethylpiperazin-1-yl)-6,6a,7,8,9,10-hexahydro-12*H-*pyrazino[2,1-*c*]pyrido[3,4-*f*][1,4]oxazepin-12-one, formate (Z463, 36 mg, 0.157 mmol, 2-step yield of 40%) as a white solid. ES-API: [M+H]⁺=528.2. ¹H NMR (400 MHz, DMSO-*d*₆) δ 8.21 (s, 1H), 7.57 - 7.48 (m, 1H), 7.47 - 7.40 (m, 1H), 7.35 - 7.27 (m, 2H), 6.82 - 6.71 (m, 1H), 6.29 - 6.12 (m, 1H), 5.80 - 5.68 (m, 1H), 4.35 - 4.33 (m, 2H), 4.30 - 4.05 (m, 3H), 4.02 - 3.83 (m, 3H), 3.66 - 3.55 (m, 3H), 3.12 - 2.96 (m, 3H), 2.45 - 2.37 (m, 1H), 2.13 (s, 3H), 0.96 (s, 3H), 0.92 - 0.81 (m, 3H).

### Embodiment 63-5 Synthesis of Z471

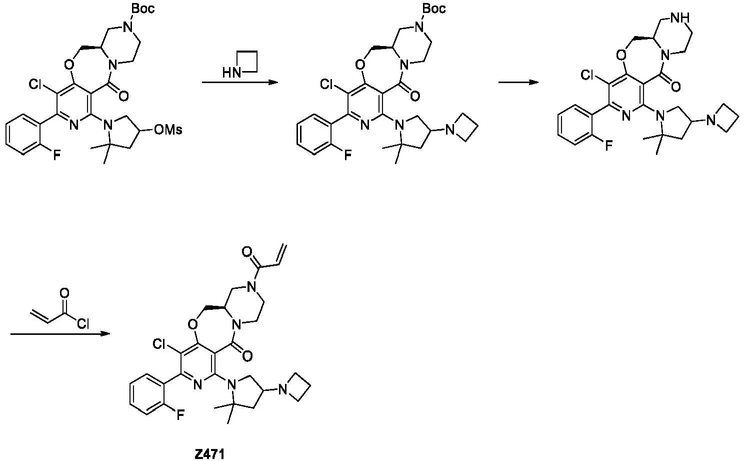

Step 1: A mixture of compound *tert*-butyl (6a*R*)-4-chloro-1-(2,2-dimethyl-4-((methylsulfonyl)oxy)pyrrolidin-1-yl)-3-(2-fluorophenyl)-12-oxo-6a,7,9,10-tetrahydro-12*H*-pyrazino[2,1 - *c*]pyrido[3,4-*f*][1,4]oxazepine-8(6*H*)-carboxylate (300 mg, 0.47 mmol) and azetidine (500 mg, 8.76 mmol) was stirred at 100°C overnight. The reaction solution was dissolved in dichloromethane (20 mL), and washed with water (10 mL^{∗}3). The organic phase was filtered and concentrated, and then purified by flash silica gel column (methanol: dichloromethane: 0-10%) to obtain *tert*-butyl (6a*R*)-1-(4-(azetidin-1-yl)-2,2-dimethylpyrrolidin-1-yl)-4-chloro-3-(2-fluorophenyl)-12-oxo-6a,7,9,10-tetrahydro-12*H*-pyrazino [2,1-c]pyrido[3,4-*f*][1,4]oxazepine-8(6*H*)-carboxylate (80 mg). ES-API: [M+H]⁺=600.3.

Step 2: Under an ice bath, a solution of *tert*-butyl (6a*R*)-1-(4-(azetidin-1-yl)-2,2-dimethylpyrrolidin-1-yl)-4-chloro-3-(2-fluorophenyl)-12-oxo-6a,7,9,10-tetrahydro-12*H*-pyrazino [2,1-*c*]pyrido[3,4-*f*][1,4]oxazepine-8(6*H*)-carboxylate(80 mg, 0.13 mmol) in dichloromethane (2 mL) was added with trifluoroacetic acid (0.5 mL), and the reaction solution was stirred at room temperature for 1 hour. The reaction solution was concentrated under reduced pressure to obtain a crude product of (6a*R*)-1-(4-(azetidin-1-yl)-2,2-dimethylpyrrolidin-1-yl)-4-chloro-3-(2-fluorophenyl)-6,6a,7,8,9,10-hexahydro-12*H-*pyrazino[2,1-*c*]pyrido[3,4-*f*][1,4]oxazepin-12-one (67 mg). ES-API: [M+H]⁺=500.2.

Step 3: The above crude procuct of (6a*R*)-1-(4-(azetidin-1-yl)-2,2-dimethylpyrrolidin-1-yl)-4-chloro-3-(2-fluorophenyl)-6,6a,7,8,9,10-hexahydro-12*H*-pyrazino[2,1-*c*]pyrido[3,4-*f*][1,4]oxazepin-12-one (67 mg, 0.13 mmol) was dissolved in dichloromethane (2 mL), and *N,N*-diisopropylethylamine (84 mg, 0.65 mmol) was added thereto. The reaction solution was cooled to 0°C, and acryloyl chloride (12 mg, 0.13 mmol) was added dropwise thereto. After stirring for 5 minutes, the mixture was concentrated to obtain a crude product of (*R*)-8-acryloyl-1-(4-(azetidin-1-yl)-2,2-dimethylpyrrolidin-1-yl)-4-chloro-3-(2-fluorophenyl)-6,6a,7,8,9,10-hexahydro-12*H*-pyrazino[2,1-*c*]pyrido[3,4-*f*][1,4]oxazepin-12-one (Z471).

### Embodiment 63-6 Synthesis of Z406

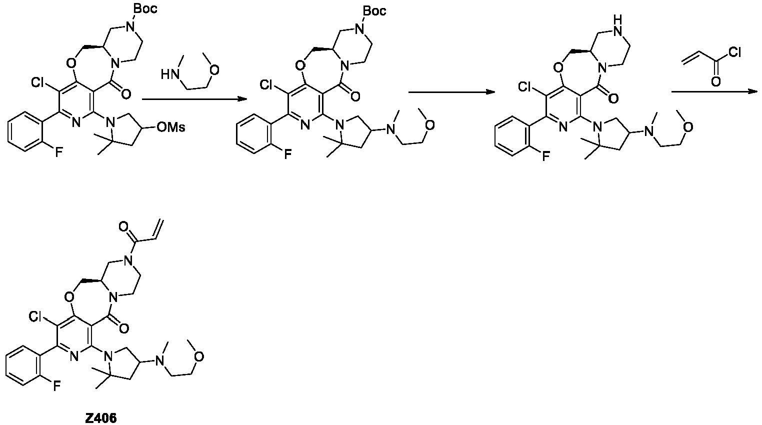

Step 1: 2-Methoxy-*N*-methylethan-1-amine (2 mL) was added to a solution of *tert*-butyl (6a*R*)-4-chloro-1-(2,2-dimethyl-4-((methylsulfonyl)oxy)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (300 mg, 0.47 mmol) in acetonitrile (5 mL), reacted at 100°C for 16 hours. The reaction solution was dissolved in ethyl acetate (20 mL), washed with water (20 mL ^{∗} 3), and the organic phase was dried over anhydrous sodium sulfate, concentrated and purified by flash silicagel column (methanol:dichloromethane: 0-10%) to obtain *tert*-butyl (6aR)-4-chloro-3 -(2-fluorophenyl)-1-(4-((2-methoxyethyl)(methyl)amino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (160 mg, yield of 54%). ES-API: [M+H]⁺=632.3.

Step 2: Trifluoroacetic acid (2 mL) was added to a solution of *tert*-butyl (6a*R*)-4-chloro-3-(2-fluorophenyl)-1-(4-((2-methoxyethyl)(methyl)amino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (160 mg, 0.25 mmol) in dichloromethane (5 mL), reacted at room temperature for 1 hour. The mixture was concentrated under reduced pressure to obtain a crude product of (6a*R*)-4-chloro-3-(2-fluorophenyl)-1-(4-((2-methoxyethyl)(methyl)amino)-2,2-dimethylpyrrolidin-1-yl)-6,6a,7,8,9,10-hexahydro-12*H*-pyrazino [2,1-*c*]pyrido[3,4-*f*][1,4]oxazepin-12-one (160 mg). ES-API: [M+H]⁺=532.3.

Step 3: The above crude product of (6a*R*)-4-chloro-3-(2-fluorophenyl)-1-(4-((2-methoxyethyl)(methyl)amino)-2,2-dimethylpyrrolidin-1-yl)-6,6a,7, 8,9,10-hexahydro-12*H*-pyrazino [2,1-*c*]pyrido[3,4-*f*][1,4]oxazepin-12-one (160 mg, 0.3 mmol) was dissolved in dichloromethane (2 mL), and *N,N-*diisopropylethylamine (116 mg, 0.9 mmol) was added thereto. The reaction solution was cooled to 0°C, and acryloyl chloride (23 mg, 0.25 mmol) was added dropwise thereto. The mixture was added with methanol (1 mL), stirred for 5 minutes and concentrated to obtain a crude product, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-fluorophenyl)-1-(4-((2-methoxyethyl)(methyl)amino)-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z406, 80 mg, yield of 46%). ES-API: [M+H]⁺=586.3.

### Embodiment 63-7 Synthesis of Z465

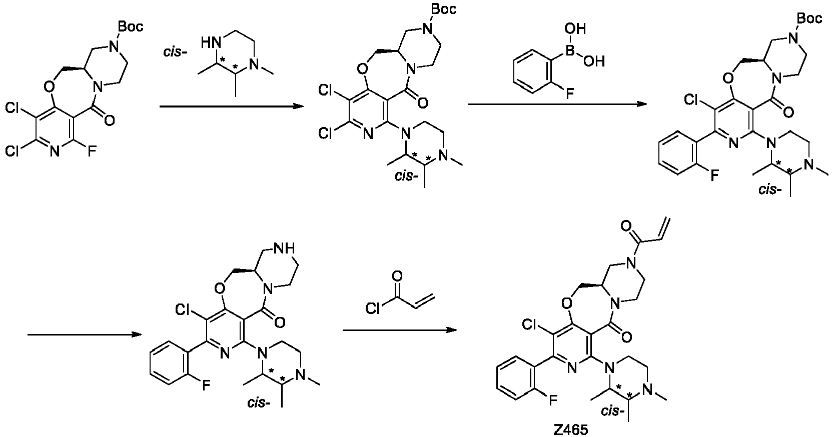

Z465 is a mixture of

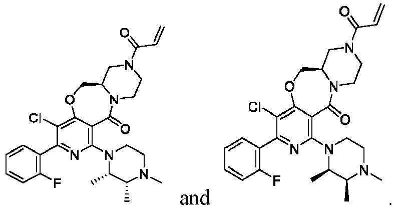

Step 1: Compound *tert*-butyl (6aR)-3-(2-((*tert*-butoxycarbonyl)oxy)-3,4-dichloro-1-fluoro-12-oxo-6a,7,9,10-tetrahydro-12*H*-pyrazino[2,1-*c*]pyrido[3,4-*f*][1,4]oxazepin-12-one (400 mg, 0.988 mmol), *cis*-2,3,4-trimethylpiperazine hydrochloride (190 mg, 1.48 mmol) and potassium carbonate (273 mg, 1.976 mmol) were dissolved in acetonitrile (4 mL), reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert*-butoxycarbonyl)oxy)-3,4-dichloro-1-(*cis*-2,3,4-trimethylpiperazin-1-yl)-12-oxo-6a,7,9,10-tetrahydro-12*H*-pyrazino[2,1-c]pyrido[3,4-*f*][1,4]oxazepin-12-one (500 mg, crude product). ES-API: [M+H]⁺=514.2.

Step 2: *tert*-Butyl (6a*R*)-3-(2-((*tert*-butoxycarbonyl)oxy)-3,4-dichloro-1-(cis-2,3,4-trimethylpiperazin-1-yl)-12-oxo-6a,7,9,10-tetrahydro-12*H*-pyrazino[2,1-*c*]pyrido[3,4-*f*][1,4]oxazepin-12-one (500 mg, crude product), (2-fluorophenyl)boronic acid (205 mg, 1.46 mmol), tetrakis(triphenylphosphine)palladium (71 mg, 0.0975 mmol) and potassium carbonate (269 mg, 1.95 mmol) were dissolved in a mixed solution of dioxane and water (5 mL: mL). The reaction solution was stirred at 110°C for 4 hours, and the reaction was monitored to be complete. The mixture was extracted by adding ethyl acetate, washed once with brine, dried over anhydrous sodium sulfate, and the filtrate was evaporated to dryness by rotary evaporation and purified by flash chromatography (ethyl acetate: petroleum ether: 0-85%) to obtain compound (6a*R*)-*tert*-butyl 4-chloro-3-(2-fluorophenyl)-1-(*cis*-2,3,4-trimethylpiperazin-1-yl)-12-oxo-6a,7,9,10-tetrahydro-12*H*-pyrazino[2,1-*c*]pyrido[3,4-*f*][1,4]oxazepin-12-one (420 mg, yield: 75.2%). ES-API: [M+H]⁺=574.2.

Step 3: Compound (6a*R*)-*tert*-butyl 4-(2-fluorophenyl)-1-(cis-2,3,4-trimethylpiperazin-1-yl)-12-oxo-6a,7,9,10-tetrahydro-12*H*-pyrazino[2,1-*c*]pyrido[3,4-*f*][1,4]oxazepin-12-one (420 mg, 0.73 mmol) was dissolved in dichloromethane (2 mL), then cooled to 0°C in an ice-water bath, and trifluoroacetic acid (2 mL) was added dropwise thereto and stirred for 1 hour. After the reaction was completed, the mixture was added with sodium bicarbonate solution to adjust to basic, extracted with dichloromethane, dried over sodium sulfate, and evaporated to dryness by rotary evaporation to obtain (6a*R*)-4-chloro-3-(2-fluorophenyl)-1-(*cis*-2,3,4-trimethylpiperazin-1-yl)-12-oxo-6a,7,9,10-tetrahydro-12*H*-pyrazino [2,1-c]pyrido[3,4-*f*] [1,4]oxazepin-12-one (500 mg, yield: 100%). ES-API: [M+H]⁺=474.2.

Step 4: (6a*R*)-4-Chloro-3-(2-fluorophenyl)-1-(cis-2,3,4-trimethylpiperazin-1-yl)-12-oxo-6a,7,9,10-tetrahydro-12*H*-pyrazino[2,1-*c*]pyrido[3,4-*f*][1,4]oxazepin-12-one (500 mg, 0.73 mmol) was dissolved in dichloromethane (5 mL), then cooled to 0°C in an ice-water bath, and triethylamine (1 mL) was added dropwise thereto. The pH value was basic, and acrylic anhydride (66 mg, 0.73 mmol) was added dropwise thereto, stirred for 10 minutes. After the reaction was completed, the mixture was extracted with ethyl acetate, washed with water, washed with brine, dried over sodium sulfate, evaporated to dryness by rotary evaporation and then purified by column chromatography (dichloromethane/methanol=10/1, R_{f}=0.5) to obtain (6a*R*)-8-acryloyl-4-chloro-3-(2-fluoro-6-hydroxyphenyl)-1-(*cis*-2,3,4-trimethylpiperazin-1-yl)-12-oxo-6,6a,7,8,9,10-hexahydro-12*H*-pyrazino[2,1-*c*]pyrido[3,4-*f*][1,4]oxazepin-12-one (Z465, 87 mg, yield: 22.6%). ¹H NMR (400 MHz, CDCl₃) δ 7.40 (q, *J* = 7.0 Hz, 2H), 7.21(t, *J* = 7.5 Hz, 1H), 7.17-7.07(m,1H), 6.63-6.50(m,1H), 6.38 (d, *J* = 16.4 Hz, 1H), 5.79 (d, *J* = 10.3 Hz, 1H), 4.43-4.24 (m, 3H), 4.19-4.13 (m, 3H), 4.05-3.94 (m, 1H),3.73-3.72 (m, 1H), 3.66-3.15 (m, 4H), 2.84 (dd, *J* = 31.2 11.2 Hz, 1H), 2.54 (td, *J* = 11.1 3.6 Hz, 1H), 2.47-2.35 (m, 1H), 2.32-2.24 (m, 3H), 1.35-0.97(m, 6H). ES-API: [M+H]⁺= 528.2.

### Embodiment 63-8 Synthesis of Z468

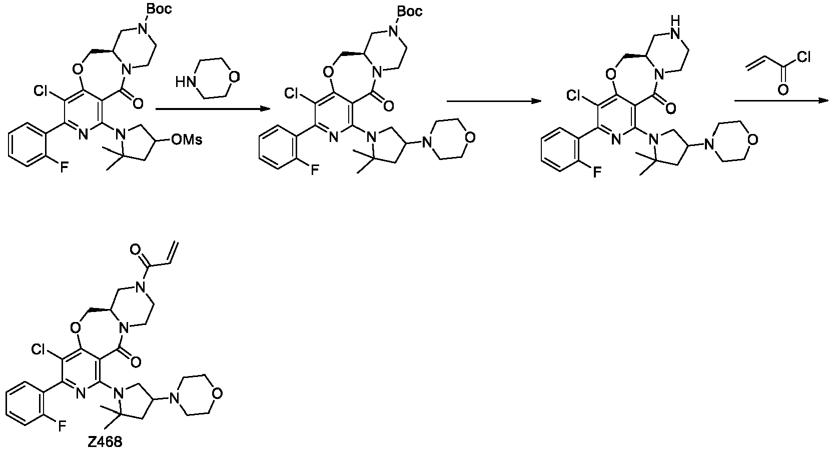

Step 1: Morpholine (2 mL) was added to a solution of *tert*-butyl (6a*R*)-4-chloro-1-(2,2-dimethyl-4-((methylsulfonyl)oxy)pyrrolidin-1-yl)-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (200 mg, 0.31 mmol) in acetonitrile (5 mL), reacted at 100°C for 16 hours. The reaction solution was dissolved in ethyl acetate (20 mL), washed with water (20 mL ^{∗} 3), and the organic phase was dried over anhydrous sodium sulfate, concentrated and purified by flash silica gel column (methanol:dichloromethane: 0-10%) to obtain *tert*-butyl (6a*R*)-4-chloro-1-(2,2-dimethyl-4-morpholinopyrrolidin-1-yl)-3 -(2-fluorophenyl)-12-oxo-6a,7,9,10-tetrahydro-12*H*-pyrazino [2,1-*c*]pyrido[3,4-*f*][1,4]oxazepine-8(6*H*)-carboxylate (130 mg, yield of 67%). ES-API: [M+H]⁺=630.3.

Step 2: Trifluoroacetic acid (2 mL) was added to a solution of *tert*-butyl (6a*R*)-4-chloro-1-(2,2-dimethyl-4-morpholinopyrrolidin-1-yl)-3 -(2-fluorophenyl)-12-oxo-6a,7,9,10-tetrahydro-12/7-pyrazino [2,1-*c*]pyrido[3,4-*f*][1,4]oxazepine-8(6*H*)-carboxylate (130 mg, 0.21 mmol) in dichloromethane (5 mL), and the reaction solution was stirred at room temperature for 1 hour. The reaction solution was concentrated under reduced pressure to obtain a crude product of (6a*R*)-4-chloro-1-(2,2-dimethyl-4-morpholinopyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (130 mg). ES-API: [M+H]⁺=530.3.

Step 3: The above crude product of (6a*R*)-4-chloro-3-(2-fluorophenyl)-1-(4-((2-methoxyethyl)(methyl)amino)-2,2-dimethylpyrrolidin-1-yl)-6,6a,7, 8,9,10-hexahydro-12*H*-pyrazino [2,1-*c*]pyrido[3,4-*f*][1,4]oxazepin-12-one (130 mg, 0.25 mmol) was dissolved in dichloromethane (2 mL), and *N,N*-diisopropylethylamine (97 mg, 0.75 mmol) was added thereto. The reaction solution was cooled to 0°C, and acryloyl chloride (18 mg, 0.2 mmol) was added dropwise thereto. The mixture was added with methanol (1 mL), stirred for 5 minutes and concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,2-dimethyl-4-morpholinopyrrolidin-1-yl)-3-(2-fluorophenyl)-6,6a,7, 8,9,10-hexahydro-12*H*-pyrazino [2,1-c]pyrido [3,4-*f*][1,4]oxazepin-12-one (Z468, 68 mg, yield of 47%). ES-API: [M+H]⁺=584.2.

### Embodiment 63-9 Synthesis of Z470-1

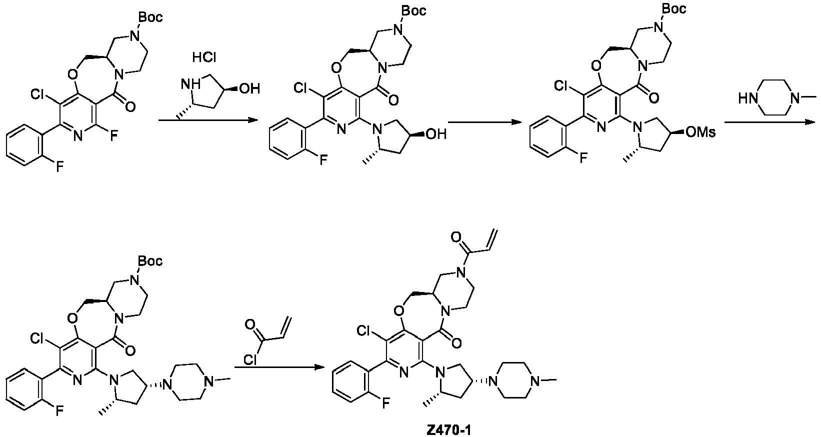

Step 1: *tert*-Butyl (6a*R*)-4-chloro-1-fluoro-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (500 mg, 1.07 mmol) was dissolved in 5 mL of acetonitrile, and K₂CO₃ (443 mg, 3.21 mmol) and (3S,5S)-5-methylpyrrolidin-3-ol hydrochloride (155 mg, 1.18 mmol) were added thereto. The reaction was carried out at 100°C for 16 hours. The reaction solution was added with 30 mL of ethyl acetate, washed with 30 mL of saturated brine for 3 times, dried and concentrated, and the crude product was purified by flash silica gel column to obtain tert-butyl (6a*R*)-4-chloro-3-(2-fluorophenyl)-1-((2*S*,4*S*)-4-hydroxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (450 mg, yield of 82%). ES-API: [M+H]⁺=547.2.

Step 2: *tert*-Butyl (6a*R*)-4-chloro-3-(2-fluorophenyl)-1-((2*S*,4*S*)-4-hydroxy-2-methylpyrrolidin-1-yl)-12-oxo-6a,7,9,10-tetrahydro-12*H-*pyrazino[2,1-*c*]pyrido[3,4-*f*][1,4]oxazepine-8(6*H*)-carboxylate (450 mg, 0.82 mmol) was dissolved in dichloromethane (8 mL), and DIEPA (317 mg, 2.46 mmol) and MsCl (187 mg, 1.64 mmol) were added thereto under an ice-water bath, and the mixture was stirred at room temperature for 1 hour. The mixture was washed with 10 mL of saturated brine for 3 times, dried and concentrated, and the crude product was purified by flash silica gel column to obtain *tert*-butyl (6a*R*)-4-chloro-3-(2-fluorophenyl)-1-((2*S*,4*S*)-2-methyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (480 mg, yield of 93%). ES-API: [M+H]⁺=625.1.

Step 3: *tert*-Butyl (6a*R*)-4-chloro-3-(2-fluorophenyl)-1-((2*S*,4*S*)-2-methyl-4-((methylsulfonyl)oxy)pyrrolidin-1-yl)-12-oxo-6a,7,9,10-tetrahydro-12*H*-pyrazino [2,1-*c*]pyrido[3,4-*f*][1,4]oxazepine-8(6*H*)-carboxylate ( 200 mg,0.32 mmol) was added to *N*-methylpiperazine (8 mL) and stirred at 100°C for 16 hours. The mixture was added with 30 mL of ethyl acetate, washed 3 times with 20 mL of saturated brine, dried and concentrated, and the crude product was purified by flash silica gel column to obtain *tert*-butyl (6a*R*)-4-chloro-3-(2-fluorophenyl)-1-((2*S,*4*R*)-2-methyl-4-(4-methylpiperazin-1-yl)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (125 mg, yield of 62%). ES-API: [M+H]⁺=629.1.

Step 4: *tert*-Butyl (6a*R*)-4-chloro-3-(2-fluorophenyl)-1-((2S,4R)-2-methyl-4-(4-methylpiperazin-1-yl)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (125 mg, 0.20 mmol) was dissolved in dichloromethane (3 mL), and trifluoroacetic acid (2 mL) was added thereto. After stirring at room temperature for 1 hour, the reaction solution was concentrated to obtain a target intermediate. The intermediate was dissolved in dichloromethane (15 mL), and triethylamine (80 mg, 0.62 mmol) was added thereto. Acryloyl chloride (18.7 mg, 0.20 mmol) was added dropwise thereto at 0°C. The reaction was stirred at 0°C for 15 minutes. The reaction solution was added with 40 mL of dichloromethane, washed with 50 mL of saturated aqueous NaHCO₃ solution and 40 mL of saturated brine, dried and concentrated. The crude product was purified by flash silica gel column to obtain (6a*R*)-8-acryloyl-4-chloro-3-(2-fluorophenyl)-1-((2*S*,4*R*)-2-methyl-4-(4-methylpiperazin-1-yl)pyrrolidin-1-yl)-6,6a,7,8,9,10-hexahydro-12*H*-pyrazino[2,1-*c*]pyrido[3,4-*f*][1,4]oxazepin-12-one (52 mg, yield of 41%). ¹H NMR (500 MHz, DMSO-*d*₆) δ 7.52 (m,1H), 7.54-7.49 (td, *J* = 7.6, 1.9 Hz, 1H), 7.48-7.44 (m, 2H), 6.79 (d, *J* = 11.1 Hz, 1H), 6.17 (d, *J* = 16.6 Hz, 1H), 5.74 (s, 1H), 4.48 - 4.23 (m, 3H), 4.15 (q, *J* = 6.6 Hz, 1H), 4.03 - 3.40 (m, 7H), 3.32 (s, 2H), 3.03 (t, *J* = 9.0 Hz, 1H), 2.67 - 2.58 (m, 1H), 2.36-2.22 (m, 7H), 2.14 (s, 3H), 1.57 (s, 1H), 1.30 (d, *J* = 6.1 Hz, 3H). ES-API: [M+H]⁺=583.2.

### Embodiment 63-10 Synthesis of Z470-2

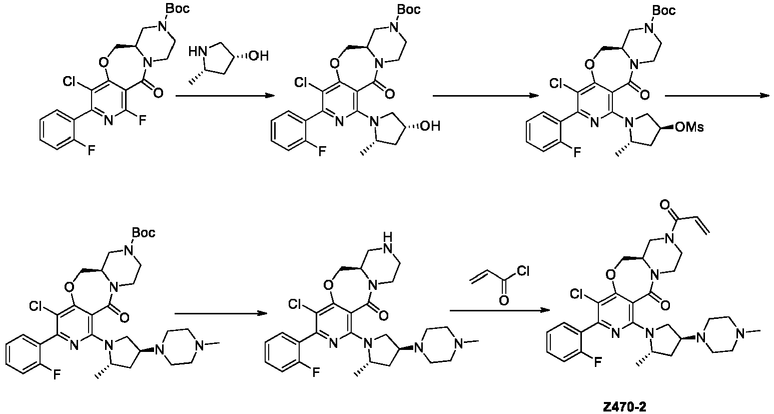

Step 1: (3*R*,5*S*)-5-Methylpyrrolidin-3-ol (110 mg, 1.1 mmol) and potassium carbonate (414 mg, 3.0 mmol) were added to a solution of *tert*-butyl (*R*)-4-chloro-1-fluoro-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (466 mg, 1.0 mmol) in acetonitrile (2 mL), reacted at 100°C for 2 hours. The reaction solution was dissolved in ethyl acetate (20 mL), washed with water (20 mL ^{∗} 3), and the organic phase was dried over anhydrous sodium sulfate, concentrated and purified by flash silica gel column (ethyl acetate:petroleum ether: 0-90%) to obtain a solid of *tert*-butyl (*R*)-4-chloro-3-(2-fluorophenyl)-1-((2*S*,4*R*)-4-hydroxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (482 mg, yield of 88%). ES-API: [M+H]⁺=547.3.

Step 2: Triethylamine (250 mg, 2.5 mmol) and methanesulfonyl chloride (230 mg, 2.0 mmol) were added to a solution of *tert*-butyl (*R*)-4-chloro-3-(2-fluorophenyl)-1-((2*S*,4*R*)-4-hydroxy-2-methylpyrrolidin-1-yl)-12-oxo-6a,7,9,10-tetrahydro-12*H*-pyrazino [2,1-c]pyrido[3,4-*f*][1,4]oxazepine-8(6*H*)-carboxylate (482 mg, 0.88 mmol) in dichloromethane (5 mL), reacted at room temperature for 12 hours. The reaction solution was dissolved in ethyl acetate (20 mL), washed with water (20 mL ^{∗} 3), and the organic phase was dried over anhydrous sodium sulfate, concentrated and purified by flash silica gel column (ethyl acetate:petroleum ether: 0-90%) to obtain a solid of *tert*₋butyl (*R*)-4-chloro-3-(2-fluorophenyl)-1-((2*S*,4*S*)-2-methyl-4-((methylsulfonyl)oxy)pyrrolidin-1-yl)-12-oxo-6a,7,9,10-tetrahydro-12*H*-pyrazino[2,1-c]pyrido[3,4-*f*][1,4]oxazepine-8(6*H*)-carboxylate (270 mg, yield of 49%). ES-API: [M+H]⁺=625.2.

Step 3: *tert*-Butyl (*R*)-4-chloro-3-(2-fluorophenyl)-1-((2S,4S)-2-methyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (270 mg, 0.43 mmol) and *N*-methylpiperazine (2 mL) were added to a 50 mL single-necked flask, reacted at 100°C for 16 hours. The reaction solution was dissolved in ethyl acetate (100 mL), washed with water (50 mL ^{∗} 3), and the organic phase was dried over anhydrous sodium sulfate, concentrated and purified by flash silica gel column (methanol:dichloromethane: 0-10%) to obtain a solid of *tert*-butyl (*R*)-4-chloro-3-(2-fluorophenyl)-1-((2*S*,4*S*)-2-methyl-4-(4-methylpiperazin-1-yl)pyrrolidin-1-yl)-12-oxo-6a,7,9,10-tetrahydro-12*H*-pyrazino[2,1-*c*]pyrido[3,4-*f*[1,4]oxazepine-8(6*H*)-carboxylate (150 mg, yield of 53%). ES-API: [M+H]⁺=629.3.

Step 4: Under an ice bath, trifluoroacetic acid (1 mL) was added to a solution of *tert*-butyl (*R*)-4-chloro-3-(2-fluorophenyl)-1-((2*S*,4*S*)-2-methyl-4-(4-methylpiperazin-1-yl)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg, 0.23 mmol) in dichloromethane (2 mL), and the reaction solution was stirred at room temperature for 1 hour. The mixture was concentrated under reduced pressure to obtain a crude product of (*R*)-4-chloro-3-(2-fluorophenyl)-1-((2*S*,4*S*)-2-methyl-4-(4-methylpiperazin-1-yl)pyrrolidin-1-yl)-6,6a,7,8,9,10-hexahydro-12*H*-pyrazino[2,1-*c*]pyrido[3,4-*f*][1,4]oxazepin-12-one. ES-API: [M+H]⁺=529.3.

Step 5: The above crude product of (*R*)-4-chloro-3-(2-fluorophenyl)-1-((2*S*,4*S*)-2-methyl-4-(4-methylpiperazin-1-yl)pyrrolidin-1-yl)-6,6a,7,8,9,10-hexahydro-12*H*-pyrazino[2,1-c]pyrido [3,4-*f*][1,4]oxazepin-12-one was dissolved in dichloromethane (2 mL), and *N,N*-diisopropylethylamine (120 mg, 0.96 mmol) was added thereto. The reaction solution was cooled to 0°C, and acryloyl chloride (17 mg, 0.19 mmol) was added dropwise thereto. The mixture was stirred for 5 minutes and concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (80 mg). ES-API: [M+H]⁺=583.3. ¹H NMR (500 MHz, DMSO-*d*₆) δ 7.51 (dd, *J* = 13.3, 6.2 Hz, 1H), 7.46-7.38 (m, 1H), 7.31 (dd, *J* = 12.8, 6.0 Hz, 2H), 6.75 (dd, *J* = 16.3, 10.5 Hz, 1H), 6.17 (d, *J* = 16.0 Hz, 1H), 5.79-5.59 (m, 1H), 4.54 - 3.33 (m, 13H), 3.12-2.98 (m, 2H), 2.45-2.20 (m, 6H), 2.15 (s, 3H), 1.89 - 1.68 (m, 2H), 1.26 (d, *J* = 6.2 Hz, 3H).

### Embodiment 63-11 Synthesis of Z407-1

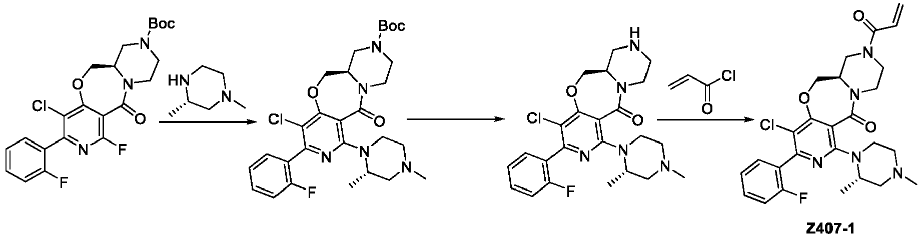

Step 1: *tert*-Butyl (*R*)-4-chloro-1-fluoro-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (233 mg, 0.5 mmol), (5)-1,3-dimethylpiperazine (114 mg, 1.0 mmol), DIPEA (193 mg, 1.5 mmol) and acetonitrile (10 mL) were added to a 50 mL round bottom flask, reacted at 90°C for 1 hour. The reaction solution was added with ethyl acetate (30 mL), washed with water (30 mL^{∗}3), and then the organic phase was dried and concentrated, and the residue was purified by flash silica gel column (ethyl acetate:petroleum ether: 0-90%) to obtain a solid of *tert*-butyl (*R*)-4-chloro-1-((*S*)-2,4-dimethylpiperazin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (200 mg, yield of 71%). ES-API: [M+H]⁺=560.2.

Step 2: Trifluoroacetic acid (2 mL) was added to a solution of *tert*-butyl (*R*)-4-chloro-1-((*S*)-2,4-dimethylpiperazin-1-yl)-3-(2-fluorophenyl)-12-oxo-6a,7,9,10-tetrahydro-12*H*-pyrazinol[2,1-*c*]pyrido[3,4-*f*][1,4]oxazepine-8(6*H*)-carboxylate (210 mg, 0.35 mmol) in dichloromethane (5 mL), reacted at room temperature for 1 hour. The reaction solution was directly concentrated to obtain a crude product of (*R*)-4-chloro-1-((*S*)-2,4-dimethylpiperazin-1-yl)-3-(2-fluorophenyl)-6,6a,7,8,9,10-hexahydro-12*H*-pyrazino[2,1-c]pyrido[3,4-*f*][1,4]oxazepin-12-one (170 mg). ES-API: [M+H]⁺=460.2.

Step 3: The above crude procuct of (*R*)-4-chloro-1-((*S*)-2,4-dimethylpiperazin-1-yl)-3-(2-fluorophenyl)-6,6a,7,8,9,10-hexahydro-12*H*-pyrazino[2,1-*c*]pyrido[3,4-*f*][1,4]oxazepin-12-one (170 mg, 0.37 mmol) was dissolved in dichloromethane (2 mL), and *N*,*N-*diisopropylethylamine (129 mg, 1.0 mmol) was added thereto. The reaction solution was cooled to 0°C, and acryloyl chloride (28 mg, 0.31 mmol) was added dropwise thereto. After stirring for 5 minutes, the mixture was added with methanol (2 mL) to quench and then concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-4-chloro-1-((*S*)-2,4-dimethylpiperazin-1-yl)-3-(2-fluorophenyl)-6,6a,7,8,9,10-hexahydro-12*H*-pyrazino[2,1-*c*]pyrido[3,4-*f*][1,4]oxazepin-12-one (Z407-1, 81 mg, yield: 40%). ES-API: [M+H]⁺=514.2. ¹H NMR (400 MHz, DMSO-*d*₆) δ 7.60-7.25 (m, 4H), 6.75 (d, *J* = 10.0 Hz, 1H), 6.17 (*d, J* = 16.8 Hz, 1H), 5.74 (s, 1H), 4.49 - 3.40 (m, 11H), 3.26-3.15 (m, 1H), 2.73 - 2.53 (m, 2H), 2.25 - 1.83 (m, 5H), 1.28 (d, *J* = 6.6 Hz, 3H)

### Embodiment 63-12 Synthesis of Z356

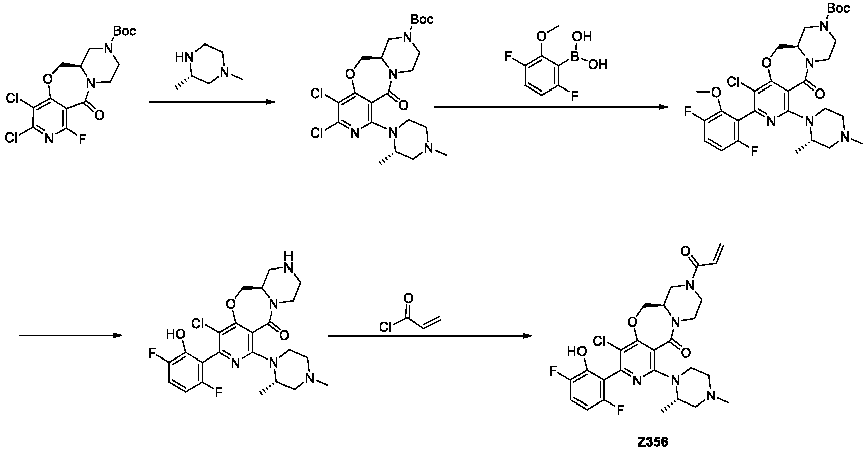

Step 1: *tert*-Butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (500 mg, 1.23 mmol), (*S*)-1,3-dimethylpiperazine (140 mg, 1.23 mmol) and potassium carbonate (500 mg, 3.6 mmol) were dissolved in acetonitrile (5 mL) and reacted at room temperature overnight. The completion of reaction was confirmed by LC-MS, and the mixture was quenched with water, extracted with ethyl acetate, washed with brine, dried over sodium sulfate, and then subjected to thin-layer chromatographic column chromatography (methanol/dichloromethane=1/10, R_{f}=0.4) to obtain a product of *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (500 mg, Y: 81%). ES-API: [M+H]⁺= 501.42.

Step 2: Under nitrogen protection, *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (50 mg, 0.25 mmol), (3,6-difluoro-2-methoxyphenyl)boronic acid (28 mg, 0.37 mmol), sodium carbonate (31 mg, 0.75 mmol) and Pd(dppf)Cl₂ (15 mg) and PdCl₂(PPh₃)₄ (15 mg) were dissolved in dioxane (0.5 mL) and water (0.1 mL), and then reacted at 120°C for 30 minutes under microwave irradiation, repeated seven times. After the reaction was completed, the mixture was quenched with water, extracted with ethyl acetate, washed with brine, dried over sodium sulfate, and then subjected to thin-layer chromatographic column chromatography (methanol/dichloromethane=1/10, R_{f}=0.4) to obtain a product of (6a*R*)-*tert*-butyl 4-chloro-3-(3,6-difluoro-2-methoxyphenyl)-1-((*S*)-2,4-dimethylpiperazin-1-yl)-12-oxo-6a,7,9,10-tetrahydro-6*H-*pyrazino[2,1-*c*]pyrido[3,4-*f*][1,4]oxazepine-8(12*H*)-carboxylate (175 mg, Y: 41.10%). ES-API: [M+H]⁺= 608.08.

Step 3: (6a*R*)-*tert*-Butyl 4-chloro-3-(3,6-difluoro-2-methoxyphenyl)-1-((S)-2,4-dimethylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (175 mg, 0.29 mmol) was dissolved in dichloromethane (2 mL), and boron tribromide (1.4 mL, 1.4 mmol, 1 M dichloromethane solution) was added dropwise thereto under an ice bath. The reaction solution was reacted at room temperature for 2 hours. After the reaction was completed, the mixture was quenched by adding water, neutralized with sodium bicarbonate, extracted with dichloromethane, and the organic phase was dried, and evaporated to dryness to obtain a crude product (200 mg), which was directly used in the next reaction step. ES-API: [M+H]⁺=494.2.

Step 4: (6a*R*)-4-Chloro-3-(3,6-difluoro-2-hydroxyphenyl)-1-((*S*)-2,4-dimethylpiperazin-1-yl)-7,8,9,10-tetrahydro-6*H*-pyrazino[2,1-*c*]pyrido[3,4-*f*][1,4]oxazepin-12(6a*H*)-one (crude product, 0.29 mmol) was dissolved in tetrahydrofuran/water (2 mL/1 mL), cooled to 0 to 5°C, and sodium bicarbonate (244 mg, 2.9 mmol) and potassium phosphate (31 mg, 0.15 mmol) were added thereto. A solution of acryloyl chloride (26 mg, 0.29 mmol) in tetrahydrofuran was added dropwise thereto under an ice bath and stirred for 0.5 hours. After the reaction was completed, the mixture was extracted with ethyl acetate and water, and the organic phase was dried, evaporated to dryness by rotary evaporation and purified by preparative thin-layer chromatographic column (dichloromethane/methanol=10/1, R_{f}=0.3) to obtain (6a*R*)-8-acryloyl-4-chloro-3-(3,6-difluoro-2-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (10.2 mg, yield: 6%, white solid). ¹H NMR (400 MHz, CDCl₃) δ 7.14-7.07 (m, 1H), 6.66-6.48 (m, 2H), 6.42 (d, *J* = 16.3 Hz, 1H), 5.83 (d, *J* = 10.4 Hz, 1H), 4.44-4.30 (m, 4H), 4.27-3.96 (m, 3H), 3.84-3.71 (m, 1H), 3.66-3.46 (m, 4H), 2.91-2.82 (m, 1H), 2.71 (d, *J* = 11.2 Hz, 1H), 2.49-2.32 (m, 5H), 1.46 (d, *J* = 6.7 Hz, 3H). ES-API: [M+H]⁺=548.2.

### Embodiment 63-13 Synthesis of Z454

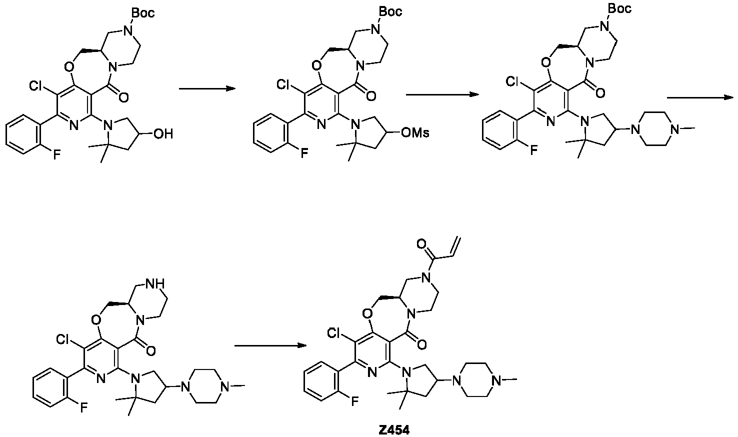

Step 1: Compound *tert*-butyl (6a*R*)-4-chloro-3-(2-fluorophenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (420 mg, 0.75 mmol) was dissolved in 15 mL of dichloromethane, then methanesulfonyl chloride (129 mg, 1.13 mmol) and triethylamine (227 mg, 2.25 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation to obtain a crude product of compound *tert*-butyl (6a*R*)-4-chloro-1-(2,2-dimethyl-4-((methylsulfonyl)oxy)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H-*carboxylate (500 mg, crude product), which was directly used in the next step. ES-API: [M+H]⁺= 639.3.

Step 2: Compound *tert*-butyl (6a*R*)-4-chloro-1-(2,2-dimethyl-4-((methylsulfonyl)oxy)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (500 mg, crude product) and 1-methylpiperazine (2 mL) were mixed, and the reaction solution was heated to 100°C, then stirred for 16 hours. The reaction was monitored to be complete, and then the reaction solution was cooled to room temperature, extracted by adding ethyl acetate, washed once with brine, dried over anhydrous sodium sulfate, and the filtrate was evaporated to dryness by rotary evaporation and purified (dichloromethane/methanol=10/1) to obtain compound *tert*-butyl (6a*R*)-4-chloro-1-(2,2-dimethyl-4-(4-methylpiperazin-1-yl)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (200 mg, two-step yield: 41.5%). ES-API: [M+H]⁺= 643.3.

Step 3: *tert*-Butyl (6a*R*)-4-chloro-1-(2,2-dimethyl-4-(4-methylpiperazin-1-yl)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.31 mmol) was dissolved in dichloromethane (4 mL), then cooled to 0°C in an ice-water bath, and then trifluoroacetic acid (2 mL) was added dropwise thereto, stirred for 1 hour. After the reaction was completed, the mixture was evaporated to dryness by rotary evaporation to obtain (6a*R*)-4-chloro-1-(2,2-dimethyl-4-(4-methylpiperazin-1-yl)pyrrolidin-1-yl)-3-(2-fluorophenyl)-6,6a,7,8,9,10-hexahydro-12*H*-pyrazino[2,1-*c*]pyrido[3,4-*f*[1,4]oxazepin-12-one (168 mg, crude product). ES-API: [M+H]⁺= 543.3.

Step 4: (6a*R*)-4-Chloro-1-(2,2-dimethyl-4-(4-methylpiperazin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (168 mg, 0.31 mmol) was dissolved in tetrahydrofuran (10 mL), then cooled to 0°C in an ice-water bath. Saturated sodium bicarbonate solution was added dropwise thereto to adjust the pH to 8-9. Potassium phosphate (200 mg) was added thereto and stirred for 5 minutes, and then acryloyl chloride (28 mg, 0.31 mmol) was added thereto and stirred for 10 minutes. After the reaction was completed, the mixture was extracted by adding dichloromethane, dried over sodium sulfate, evaporated to dryness by rotary evaporation, and the crude product was purified by preparative thin-layer chromatography plate (dichloromethane/methanol=10/1, R_{f}=0.4) to obtain (6a*R*)-8-acryloyl-4-chloro-1-(2,2-dimethyl-4-(4-methylpiperazin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (103 mg, 2-step yield: 55%). ¹H NMR (400 MHz, CDCl₃) δ 7.40 (q, *J* = 6.9 Hz, 2H), 7.21 (t, *J* = 7.5 Hz, 1H), 7.14 (t, *J* = 9.2 Hz, 1H), 6.55 (s, 1H), 6.40 (d, *J* = 16.6 Hz, 1H), 5.81 (d, *J* = 10.4 Hz, 1H), 4.53 - 3.41 (m, 10H), 3.01 - 2.88 (m, 2H), 2.47 (s, 7H), 2.34 (d, *J* = 16.1 Hz, 3H), 2.08 - 1.82 (m, 3H), 1.64 - 1.48 (m, 6H). ES-API: [M+H]⁺= 597.3.

### Embodiment 63-14 Synthesis of Z456

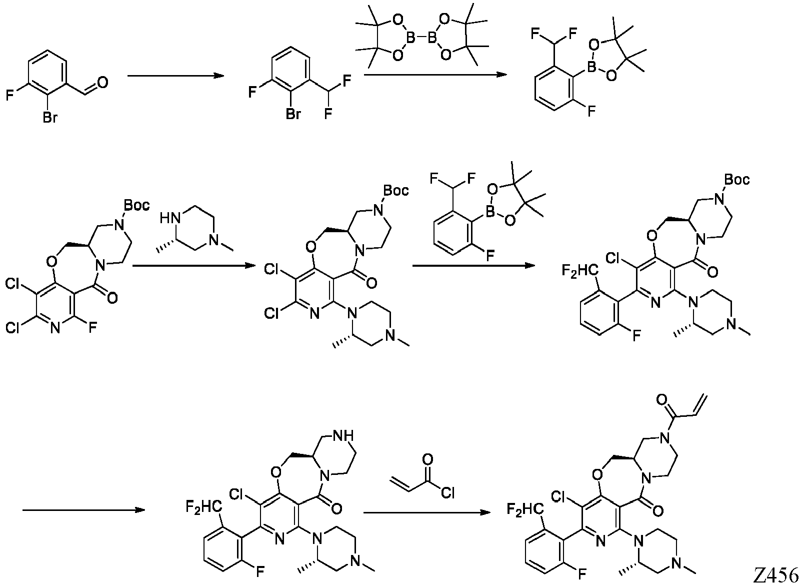

Step 1: Under nitrogen protection, 2-bromo-3-fluorobenzaldehyde (500 mg, 2.48 mmol) was dissolved in dichloromethane (18 mL), cooled to 0°C and then DAST (0.5 mL) was added dropwise thereto, stirred for 1 hour, heated to room temperature, and continued to stir for 1 hour. After the reaction was completed, the mixture was added with saturated sodium bicarbonate solution (15 mL) to quench, extracted with dichloromethane, dried over sodium sulfate, and then subjected to thin-layer chromatographic column chromatography (ethyl acetate/petroleum ether= 1/10, R_{f}=0.8) to obtain a product of 2-bromo-1-(difluoromethyl)-3-fluorobenzene (300 mg, yield: 54.11%). ES-API: [M+H]⁺= 226.01.

Step 2: 2-Bromo-1-(difluoromethyl)-3-fluorobenzene (300 mg, 1.34 mmol), 4,4,4',4',5,5,5',5'-octamethyl-2,2'-bi(1,3,2-dioxaborolane) (408 mg, 1.61 mmol), potassium acetate (394 mg, 4.02 mmol) and Pd(dppf)Cl₂ (50 mg) were dissolved in dimethyl sulfoxide (1 mL) and dioxane (5 mL), then replaced with nitrogen, heated to 100°C, and reacted for 16 hours. After the reaction was completed, the mixture was quenched by adding water, extracted with ethyl acetate, washed with brine, dried over sodium sulfate, and then subjected to thin-layer chromatographic column chromatography (ethyl acetate/petroleum ether=1/3, R_{f}=0.7) to obtain a product of 2-(2-(difluoromethyl)-6-fluorophenyl)-4,4,5,5-tetramethyl-1,3,2-dioxaborolane (150 mg, yield: 41.18%). ES-API: [M+H]⁺= 273.01.

Step 3: *tert*-Butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (1.5 g, 3.7 mmol), (*S*)-1,3-dimethylpiperazine (422 mg, 3.7 mmol) and potassium carbonate (1.5 g, 11.1 mmol) were dissolved in acetonitrile (10 mL) and reacted at room temperature overnight. The completion of reaction was confirmed by LC-MS, and the mixture was quenched with water, extracted with ethyl acetate, washed with brine, dried over sodium sulfate, and then subjected to thin-layer chromatographic column chromatography (methanol/dichloromethane=1/10, R_{f}=0.5) to obtain a product of *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (1.1 g, yield: 59.52%). ES-API: [M+H]⁺= 501.42.

Step 4: *tert*-Butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (100 mg, 0.2 mmol), 2-(2-(difluoromethyl)-6-fluorophenyl)-4,4,5,5-tetramethyl-1,3,2-dioxaborolane (75 mg, 0.28 mmol), potassium carbonate (100 mg, 0.72 mmol) and Pd(PPh₃) (20 mg) were dissolved in dioxane (2 mL) and water (0.2 mL), replaced with nitrogen, then heated to 100°C, stirred for 16 hours. After the reaction was completed, the crude product was purified by preparative thin-layer chromatography plate (methanol/dichloromethane=1/10) to obtain a product of *tert*-butyl (6a*R*)-4-chloro-3-(2-(difluoromethyl)-6-fluorophenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (125 mg, yield=100%). ES-API: [M+ H]⁺= 611.08.

Step 5: *tert*-butyl (6a*R*)-4-chloro-3-(2-(difluoromethyl)-6-fluorophenyl)-1-((S)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (125 mg, 0.21 mmol) was dissolved in dichloromethane (2 mL), and then trifluoroacetic acid (2 mL) was added dropwise thereto, stirred at room temperature for 1 hour. Then after the reaction was completed, the solvent was evaporated to dryness by rotary evaporation to obtain a product of (6a*R*)-4-chloro-3-(2-(difluoromethyl)-6-fluorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (125 mg, yield: 100%). ES-API: [M+H]⁺= 510.96.

Step 6: (6a*R*)-4-Chloro-3-(2-(difluoromethyl)-6-fluorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (125 mg, 0.25 mmol) was dissolved in tetrahydrofuran (5 mL), cooled to 0°C, then sodium bicarbonate (4 mL) and potassium phosphate (0.5 mL) were added dropwise thereto, then acryloyl chloride (22.3 mg, 0.25 mmol) was added dropwise to the reaction solution, stirred at 0°C for 15 minutes. The the mixture was quenched by dropwise addition of sodium bicarbonate, extracted with ethyl acetate, washed with brine, and dried over sodium sulfate to prepare (6a*R*)-8-acryloyl-4-chloro-3-(2-(difluoromethyl)-6-fluorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (10.3 mg, yield: 8.9%). (Preparative separation conditions: chromatographic column information: Ultimate XB-C18, 50*250 mm, 10 µm, mobile phase system: A: purified water B: pure acetonitrile, flow rate: 80 mL/min, gradient: within 50 minutes, B/A= 20%-90%, wavelength: 214 nm, column temperature: room temperature) ¹H NMR (400 MHz, CDCl₃) δ 7.61 - 7.49 (m, 2H), 6.55 (d, *J* = 19.3 Hz, 1H), 6.44 - 6.38 (m, 1H), 5.83 (d, *J* = 10.3 Hz, 1H), 4.22 (dd, *J* = 85.9, 54.9 Hz, 7H), 3.75 (s, 2H), 3.66 - 3.27 (m, 4H), 2.72 (s, 1H), 2.59 (d, *J* = 11.7 Hz, 1H), 2.28 - 2.25 (m, 3H), 2.21 (d, *J* = 10.7 Hz, 1H), 1.34 (dd, *J* = 13.2, 6.7 Hz, 3H), 1.26 (s, 1H). ES-API: [M+H]⁺= 564.2.

### Embodiment 63-15 Synthesis of Z466

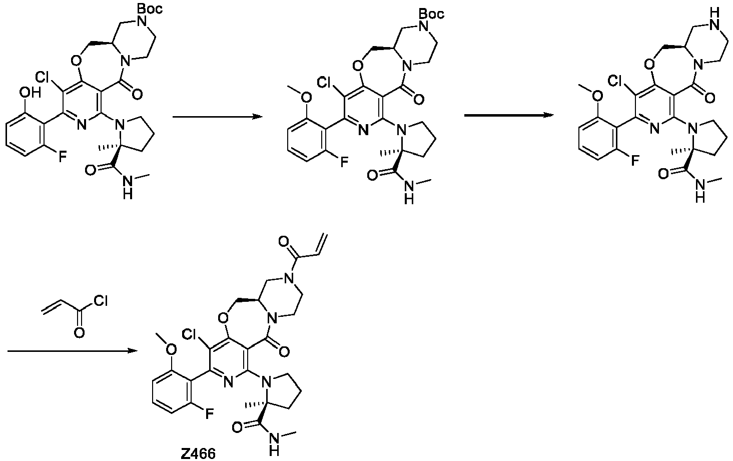

Step 1: *tert-*Butyl (6a*R*)-4-chloro-3-(2-fluoro-6-hydroxyphenyl)-1-((*S*)-2-methyl-2 (methylcarbamoyl)tetrahydropyrrol-1-yl)-12-oxo-6a,7,9,10-tetrahydro-12*H*-pyrazino[2,1-*c*]pyrido[3,4-*f*][1,4]oxazepine-8(6*H*)-carboxylate (65 mg, crude product) was dissolved in acetonitrile (0.5 mL), and potassium carbonate (27 mg, 0.2 mmol) and iodomethane (14.2 mg, 0.1 mmol) were added thereto. The tube was sealed at 60°C and reacted for 4 hours. The reaction system was added with saturated brine, extracted three times with ethyl acetate, and dried over anhydrous sodium sulfate. The organic phase was concentrated to obtain *tert*-butyl (6a*R*)-4-chloro-3-(2-fluoro-6-methoxyphenyl)-1-((*S*)-2-methyl-2-(methylcarbamoyl)tetrahydropyrrol-1-yl)-12-oxo-6a,7,9,10-tetrahydro-12*H*-pyrazino[2,1-*c*]pyrido[3,4-*f*][1,4]oxazepine-8(6*H*)-carboxylate, and the crude product was directly used in the next reaction step. ES-API: [M+H]⁺=618.3.

Step 2: *tert*-Butyl (6a*R*)-4-chloro-3-(2-fluoro-6-methoxyphenyl)-1-((*S*)-2-methyl-2 (methylcarbamoyl)tetrahydropyrrol-1-yl)-12-oxo-6a,7,9,10-tetrahydro-12*H*-pyrazino [2,1-c]pyrido [3,4-*f*][1,4]oxazepine-8(6*H*)-carboxylate (crude product from previous step) was dissolved in 2 mL of dichloromethane and 1 mL of trifluoroacetic acid. The mixture was stirred at room temperature for 1 hour, and the completion of reaction was detected by LC-MS. The reaction solution was concentrated to obtain (2*S*)-1-((6a*R*)-4-chloro-3-(2-fluoro-6-methoxyphenyl)-12-oxo-6,6a,7,8,9,10-hexahydro-12*H*-pyrazino[2,1-c]pyrido[3,4-*f*][1,4]oxazepin-1-yl)-N,2-dimethylpyrrolidine-2-carboxamide (70 mg, crude product). The crude product was directly used in the next step. ES-API: [M+H]⁺=518.3.

Step 3: (2*S*)-1-((6a*R*)-4-Chloro-3-(2-fluoro-6-methoxyphenyl)-12-oxo-6,6a,7,8,9,10-hexahydro-12H-pyrazino[2,1-*c*]pyrido[3,4-*f*][1,4]oxazepin-1-yl)-*N*,2-dimethylpyrrolidine-2-carboxamide (70 mg, crude product), triethylamine (0.042 mL, 0.3 mmol) and dichloromethane (1 mL) were added to a round bottom flask. The reaction was cooled to 0°C, and acryloyl chloride (4.5 mg, 0.05 mmol) was added dropwise to the reaction solution. The reaction was stirred at 0°C for 10 minutes. 20 mL of saturated aqueous sodium bicarbonate solution was added to the reaction solution, and the mixture was extracted twice with 20 mL of dichloromethane. The organic phase was dried and concentrated, and the crude product was purified by preparative HPLC to obtain (2*S*)-1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide (10 mg, 17%). ES-API: [M+H]⁺=572.1.

### Embodiment 63-16 Synthesis of Z457

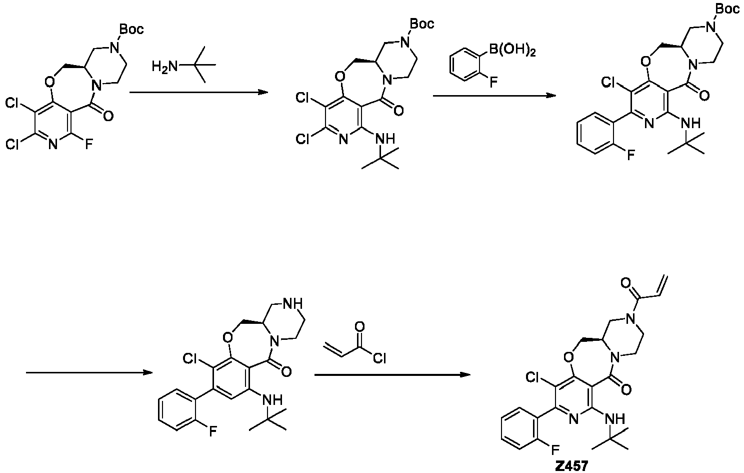

Step 1: Compound (*R*)-*tert*-butyl 3,4-dichloro-1-fluoro-12-oxo-6a,7,9,10-tetrahydro-12*H-*pyrazino[2,1-*c*]pyrido[3,4-*f*][1,4]oxazepine-8(6*H*)-carboxylate (200 mg, 0.492 mmol), 2-methylpropan-2-amine (40 mg, 0.542 mmol) and potassium carbonate (204 mg, 1.476 mmol) were dissolved in acetonitrile (2 mL) and reacted at room temperature for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation and purified by flash chromatography (ethyl acetate:petroleum ether 0-35%) to obtain compound (*R*)*tert*-butyl 1-(*tert*-butylamino)-3,4-dichloro-12-oxo-6a,7,9,10-tetrahydro-6*H*-pyrazino[2,1-*c*]pyrido[3,4-*f*][1,4]oxazepine-8(12*H*)-carboxylate (220 mg, yield: 97%). ES-API: [M+H]⁺= 490.2

Step 2: Compound (*R*)*tert*-butyl 1-(tert-butylamino)-3,4-dichloro-12-oxo-6a,7,9,10-tetrahydro-6*H*-pyrazino[2,1-*c*]pyrido[3,4-*f*][1,4]oxazepine-8(12*H*)-carboxylate (220 mg, 0.480 mmol),(2-fluorophenyl)boronic acid (81 mg, 0.576 mmol), tetrakis(triphenylphosphine)palladium (55 mg, 0.048 mmol) and potassium carbonate (199 mg, 1.44 mmol) were dissolved in a mixed solution of dioxane and water (2.5 mL:0.5 mL), and the reaction solution was stirred at 110°C overnight. The reaction was monitored to be complete, the mixture was extracted with ethyl acetate, washed once with brine, dried over anhydrous sodium sulfate, and the filtrate was evaporated to dryness by rotary evaporation and purified by flash chromatography (ethyl acetate:petroleum ether: 0-40%) to obtain compound (*R*)*tert*-butyl 1-(*tert-*butylamino)-4-chloro-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (221 mg, yield: 89%). ES-API: [M+H]⁺=519.3

Step 3: Compound (*R*)*tert*-butyl 1-(*tert*-butylamino)-4-chloro-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (215 mg, 0.414 mmol) was dissolved in dichloromethane (4 mL), then cooled to 0°C in an ice-water bath, and then trifluoroacetic acid (2 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (*R*)-1-(*tert*-butylamino)-4-chloro-3-(2-fluorophenyl)-7,8,9,10-tetrahydro-6*H*-pyrazino[2,1-*c*]pyridin[3,4-*f*][1,4]oxazepin-12-one (140 mg, yield: 81%)

Step 4: (*R*)-1-(*tert*-Butylamino)-4-chloro-3-(2-fluorophenyl)-7,8,9,10-tetrahydro-6*H-*pyrazino[2,1-*c*]pyridin[3,4-*f*][1,4]oxazepin-12(6a*H*)-one (140 mg, 0.334 mmol) was dissolved in dichloromethane (3 mL), then cooled to 0°C in an ice-water bath, and triethylamine (101 mg, 1.002 mmol) and acryloyl chloride (36 mg, 0.401 mmol) were added dropwise thereto, stirred for 30 minutes. After the reaction was completed, the mixture was added with sodium bicarbonate solution to adjust to alkaline, extracted with dichloromethane, dried over sodium sulfate, and evaporated to dryness by rotary evaporation and purified (dichloromethane/methanol =10/1,R_{f}=0.4) to obtain (*R*)-8-acryloyl-1-(*tert*-butylamino)-4-chloro-3-(2-fluorophenyl)-6,6a,7,8,9,10-hexahydro-12*H*-pyrazino[2,1-*c*]pyrido[3,4-*f*][1,4]oxazepin-12-one (52.3 mg, yield: 33%). ¹H NMR (400 MHz, CDCl₃) δ 7.48 (d, *J* = 7.1 Hz, 2H), 7.24 - 7.13 (m, 2H), 6.46 (t, *J* = 19.7 Hz, 2H), 5.82 (d, *J* = 9.5 Hz, 1H), 4.32 (d, *J* = 6.1 Hz, 2H), 4.17 (s, 2H), 4.06 - 3.95 (m, 1H), 3.86 (s, 2H), 3.72 (d, *J* = 11.5 Hz, 1H), 1.43 (s, 9H). ES-API: [M+H]⁺= 473.2

### Embodiment 63-17 Synthesis of Z461

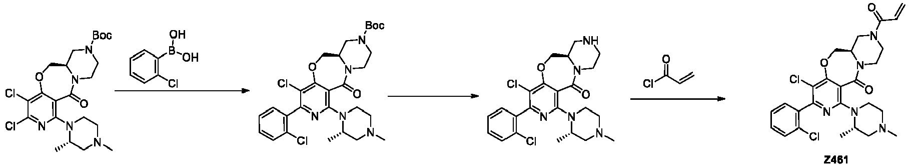

Step 1: Under nitrogen protection, *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (100 mg, 0.2 mmol), (2-chlorophenyl)boronic acid (37.5 mg, 0.24 mmol), potassium carbonate (100 mg,0.72 mmol) and Pd(PPh₃)₄ (20 mg) were dissolved in 1,4-dioxane (2 mL) and water (0.2 mL), then heated to 100°C and reacted overnight. Then after the reaction was completed, the mixture was quenched with water, extracted with ethyl acetate, washed with brine, dried over sodium sulfate, and then subjected to thin-layer chromatographic column chromatography (methanol/dichloromethane=1/10, R_{f}=0.4) to obtain a product of *tert*-butyl (*R*)-4-chloro-3-(2-chlorophenyl)-1-((*S*)-2,4-dimethylpiperazin-1-yl)-12-oxo-6a,7,9,10-tetrahydro-12H-pyrazino[2,1-*c*]pyrido[3,4-*f*][1,4]oxazepine-8(6*H*)-carboxylate (100 mg, Y: 86.78%). ES-API: [M+H]⁺= 576.21.

Step 2: *tert*-Butyl (*R*)-4-chloro-3-(2-chlorophenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*-carboxylate (100 mg, 0.17 mmol) was dissolved in dichloromethane (2 mL), and then trifluoroacetic acid (2 mL) was added thereto, reacted at room temperature for 1 hour. Then after the reaction was completed, the mixture was evaporated to dryness by rotary evaporation to obtain a product of (*R*)-4-chloro-3-(2-chlorophenyl)-1-((S)-2,4-dimethylpiperazin-1-yl)-6,6a,7,8,9,10-hexahydro-12*H*-pyrazino[2,1-*c*]pyrido[3,4-*f*][1,4]oxazepin-12-one (100 mg, yield: 100%). ES-API: [M+H]⁺= 476.15.

Step 3: (*R*)-4-Chloro-3-(2-chlorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (100 mg, 0.21 mmol) was dissolved in tetrahydrofuran (5 mL), then cooled to 0°C. Saturated sodium bicarbonate (2 mL) was added dropwise to the reaction solution to adjust to pH to 8-9, then acryloyl chloride (19 mg, 0.21 mmol) was slowly added dropwise to the solution, stirred for 10 minutes. The completion of reaction was confirmed by LC-MS, then the reaction mixture was quenched by adding saturated sodium bicarbonate, extracted with ethyl acetate, washed with brine, dried over sodium sulfate, and then prepared (preparative separation conditions: chromatographic column information: Ultimate XB-C18, 50^{∗}250 mm, 10 µm, mobile phase system: A: purified water B: pure acetonitrile, flow rate: 40 mL/min, gradient: within 50 minutes, B/A=20%-90%, wavelength: 214 nm, column temperature: room temperature) to obtain a product of (*R*)-8-acryloyl-4-chloro-3-(2-chlorophenyl)-1-((*S*)-2,4-dimethylpiperazin-1-yl)-6,6a,7,8,9,10-hexahydro-12*H*-pyrazino[2,1-c]pyrido[3,4-*f*] [1,4]oxazepin-12-one (13.4 mg, Y: 12.03%). ¹H NMR (400 MHz, CDCl₃) δ 7.46 (d*, J* = 7.5 Hz, 1H), 7.35 (d, *J* = 7.6 Hz, 3H), 6.42 (d, *J* = 16.5 Hz, 1H), 5.83 (d, *J* = 10.4 Hz, 1H), 4.36 (d, *J* = 22.3 Hz, 5H), 4.18 (s, 3H), 3.75 (s, 1H), 3.69 - 3.31 (m, 4H), 2.75 (d, *J* = 10.9 Hz, 1H), 2.61 (d, *J* = 11.1 Hz, 1H), 2.29 (s, 3H), 1.38 (d, *J* = 6.7 Hz, 3H), 1.26 (s, 1H). ES-API: [M+H]⁺= 530.2.

### Embodiment 63-18 Synthesis of Z464

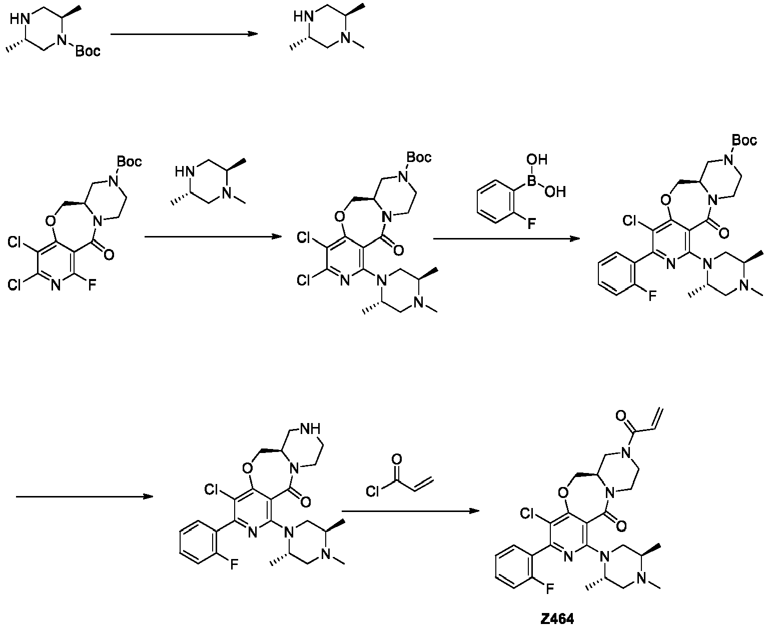

Step 1: *tert*-Butyl (2*R*,5*S*)-2,5-dimethylpiperazine-1-carboxylate (1 g, 4.67 mmol) was dissolved in tetrahydrofuran (40 mL), and lithium aluminum hydride (1.06 g, 28 mmol) was added thereto, refluxed for 16 hours. The reaction solution was added with water (1.06 mL), 15% sodium hydroxide (3.2 mL) and water (1.06 mL) in turn, stirred for 5 minutes, filtered, and the filtrate was directly concentrated under reduced pressure to obtain 460 mg of a crude product of (2R,5S)-2,4,5-trimethylpiperazine, which was directly used in the next step. (Crude product). ES-API: [M+H]⁺= 129.1

Step 2: *tert*-Butyl (6a*R*)-3-(2-((tert-butoxycarbonyl)oxy)3,4-dichloro-1-fluoro-12-oxo-6a,7,9,10-tetrahydro-12*H*-pyrazino[2,1-*c*]pyrido[3,4-*f*][1,4]oxazepin-12-one (400 mg, 0.988 mmol), (2*R*,5*S*)-2,4,5-trimethylpiperazine (128 mg, 1.0 mmol) and potassium carbonate (414 mg, 3.0 mmol) were dissolved in acetonitrile (10 mL), reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (6a*R*)-3-(2-((*tert*-butoxycarbonyl)oxy)-3,4-dichloro-1-((2*R*,5*S*-2,4,5-trimethylpiperazin-1-yl)-12-oxo-6a,7,9,10-tetrahydro-12*H*-pyrazino[2,1-*c*]pyrido[3,4-*f*][1,4]oxazepin-12-one (400 mg, yield of 77.8%). ES-API: [M+H]⁺=514.2

Step 3: *tert*-Butyl (6a*R*)-3-(2-((*tert*-butoxycarbonyl)oxy)-3,4-dichloro-1-((2*R*,5*S*)-2,4,5-trimethylpiperazin-1-yl)-12-oxo-6a,7,9,10-tetrahydro-12*H*-pyrazino[2,1-*c*]pyrido[3,4-*f*][1,4]oxazepin-12-one (400 mg, 0.78 mmol), (2-fluorophenyl)boronic acid (131 mg, 0.94 mmol), tetrakis(triphenylphosphine)palladium (90 mg, 0.078 mmol) and potassium carbonate (215 mg, 1.56 mmol) were dissolved in a mixed solution of dioxane and water (20 mL:4 mL). The reaction solution was stirred at 100°C for 4 hours, and the reaction was monitored to be complete. The mixture was extracted by adding ethyl acetate, washed once with brine, dried over anhydrous sodium sulfate, and the filtrate was evaporated to dryness by rotary evaporation and purified by flash chromatography (ethyl acetate: petroleum ether: 0-85%, R_{f}=0.4) to obtain compound (6a*R*)-*tert*-butyl 4-chloro-3-(2-fluorophenyl)-1-((2*R*,5*S*)-2,4,5-trimethylpiperazin-1-yl)-12-oxo- 6a,7,9,10-tetrahydro-12*H*-pyrazino[2,1-*c*]pyrido[3,4-*f*][1,4]oxazepin-12-one (320 mg, yield: 71.6%). ES-API: [M+H]⁺=574.3

Step 4: Compound (6aR)-*tert*-butyl 4-(2-fluorophenyl)-1-((2*R*,5*S*)-2,4,5-trimethylpiperazin-1-yl)-12-oxo- 6a,7,9,10-tetrahydro-12*H*-pyrazino[2,1-*c*]pyrido[3,4-*f*][1,4]oxazepin-12-one (320 mg, 0.73 mmol) was dissolved in dichloromethane (5 mL), then cooled to 0°C in an ice-water bath, and then trifluoroacetic acid (2 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (6a*R*) 4-chloro-3-(2-fluorophenyl)-1-((2*R*,5*S*)-2,4,5-trimethylpiperazin-1-yl)-12-oxo-6a,7,9,10-tetrahydro-12*H*-pyrazino [2,1 -c]pyrido [3,4-*f*][1,4]oxazepin-12-one (260 mg, yield: 100%) ES-API: [M+H]⁺=474.2

Step 5: (6a*R*)-4-Chloro-3-(2-fluorophenyl)-1-((2*R*,5*S*)-2,4,5-trimethylpiperazin-1-yl)-12-oxo-6a,7,9,10-tetrahydro-12*H*-pyrazino[2,1-*c*]pyrido[3,4-*f*][1,4]oxazepin-12-one (260 mg, 0.55 mmol) was dissolved in dichloromethane (5 mL), then cooled to 0°C in an ice-water bath, and triethylamine (1 mL) was added dropwise thereto. The pH value was basic, and acrylic anhydride (66 mg, 0.73 mmol) was added dropwise thereto, stirred for 10 minutes. After the reaction was completed, the mixture was extracted with ethyl acetate, washed with water, washed with brine, dried over sodium sulfate, evaporated to dryness by rotary evaporation and then purified by column chromatography (dichloromethane/methanol=10/1, R_{f}=0.4) to obtain (6a*R*)-8-acryloyl-4-chloro-3-(2-fluoro-6-hydroxyphenyl)-1-((2*R*,5*S*)-2,4,5-trimethylpiperazin-1-yl)-12-oxo-6,6a,7,8,9,10-hexahydro-12*H*-pyrazino[2,1-*c*]pyrido[3,4-*f*][1,4]oxazepin-12-one (87 mg, yield: 22.6%). ¹H NMR (400 MHz, CDCl₃) δ 7.46 - 7.38 (m, 2H), 7.23 (td, *J* = 7.5, 1.3 Hz, 1H), 7.14 (t, *J* = 9.1 Hz, 1H), 6.57 (s, 1H), 6.41 (d, *J* = 16.4 Hz, 1H), 5.82 (d, *J* = 10.3 Hz, 1H), 4.43 - 4.11 (m, 6H), 3.90 (s, 2H), 3.70 (s, 2H), 3.53 (dd, *J* = 12.3, 3.0 Hz, 1H), 3.30 (dd, *J* = 12.5, 5.8 Hz, 1H), 2.85 - 2.78 (m, 1H), 2.73 (s, 1H), 2.32 (s, 3H), 2.26 (dd, *J* = 11.5, 5.4 Hz, 1H), 1.18 (d, *J* = 6.3 Hz, 3H), 1.07 (d, *J* = 6.4 Hz, 3H). ES-API: [M+H]⁺=528.2

### Embodiment 63-19 Synthesis of Z477

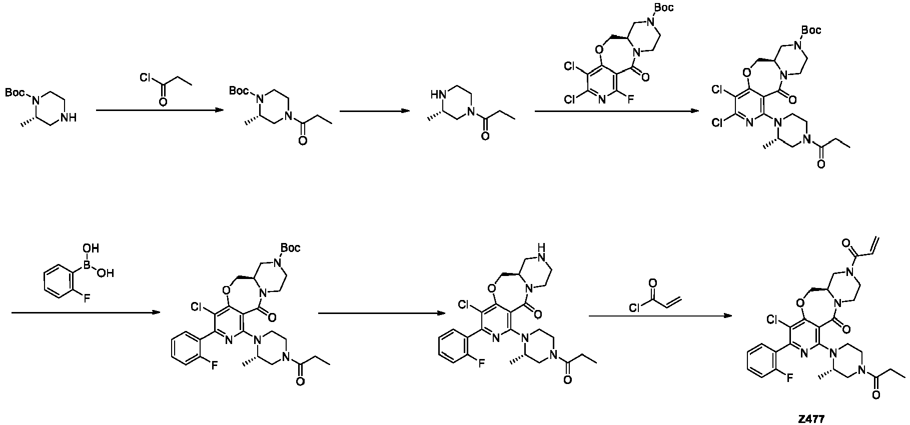

Step 1: *tert*-Butyl (*S*)-2-methylpiperazine-1-carboxylate (1 g, 5 mmol) and propionyl chloride (552 mg, 6 mmol)) were dissolved in dichloromethane (30 mL), and triethylamine (1.0 g, 10 mmol) was added thereto, stirred at 0°C for 1 hour. After the reaction was completed, the mixture was quenched with water, extracted with ethyl acetate, washed with brine, dried over anhydrous sodium sulfate, and the filtrate was rotary evaporated to obtain a crude product of *tert*-butyl (*S*)-2-methyl-4-propionylpiperazine-1-carboxylate (compound 3, brown oil, 1.4 g, yield: 100%). ES-API: [M+H]⁺=257.1

Step 2: *tert*-Butyl (*S*)-2-methyl-4-propionylpiperazine-1-carboxylate (1.4 g, 5.46 mmol) was dissolved in dichloromethane (5 mL), and then trifluoroacetic acid (5 mL) was added thereto, reacted at room temperature for 1 hour. After the reaction was completed, the mixture was directly evaporated to dryness by rotary evaporation to obtain a product of (*S*)-1-(3-methylpiperazin-1-yl)propan-1-one (compound 4, brown oil, 2.1 g, the crude product was mixed with trifluoroacetic acid, yeld: 100%). ES-API: [M+H]⁺=157.1

Step 3: (*R*)-*tert*-Butyl 3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (200 mg, 0.49 mmol) and K₂CO₃ (286 mg, 2.0 mmol) were dissolved in acetonitrile (2 mL), and (*S*)-1-(3-methylpiperazin-1-yl)propan-1-one (154 mg, 0.98 mmol) was added thereto, and the reaction was stirred at 80°C overnight for 16 hours. After the reaction was completed, the mixture was extracted with water and ethyl acetate, washed with brine, dried over anhydrous sodium sulfate, and the filtrate was evaporated to dryness by rotary evaporation and purified by preparative thin-layer chromatography plate (PE/EA=3/1, R_{f}=0.5) to obtain compound (*R*)-*tert*-butyl 3,4-dichloro-1-((*S*)-2-methyl-4-propionylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (compound 6, brown solid, 250 mg, yield: 94.3%). ES-API: [M+H]⁺=542.1

Step 4: (*R*)-*tert*-Butyl 3,4-dichloro-1-((*S*)-2-methyl-4-propionylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (250 mg, 0.46 mmol), (2-fluorophenyl)boronic acid (53.3 mg, 0.55 mmol), tetrakis(triphenylphosphine)palladium (53.3 mg, 0.046 mmol) and potassium carbonate (127.5 mg, 0.92 mmol) were dissolved in 1,4-dioxane (3 mL) and water (0.6 mL), reacted under argon protection at 100°C for 16 hours overnight. After the reaction was completed, the mixture was extracted with water and ethyl acetate, washed with brine, dried over anhydrous sodium sulfate, and the filtrate was evaporated to dryness by rotary evaporation and purified by preparative thin-layer chromatography plate (PE/EA=3/1, R_{f}=0.4) to obtain compound (*R*)-*tert*-butyl 4-chloro-3-(2-fluorophenyl)-1-((*S*)-2-methyl-4-propionylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (yellow solid, 160 mg, yield: 57.9%). ES-API: [M+H]⁺=602.1

Step 5: (*R*)*-tert*-Butyl 4-chloro-3-(2-fluorophenyl)-1-((*S*)-2-methyl-4-propionylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (160 mg, 0.27 mmol) was dissolved in dichloromethane (4 mL), then trifluoroacetic acid (4 mL) was added thereto, stirred at room temperature for 30 minutes. Then the reaction solution was evaporated to dryness by rotary evaporation to obtain a crude product of (*R*)-4-chloro-3-(2-fluorophenyl)-1-((*S*)-2-methyl-4-propionylpiperazin-1-yl)-7,8,9,10-tetrahydro-6*H*-pyrazino[2,1-*c*]pyrido[3,4-*f*][1,4]oxazepin-12(6a*H*)-one, which was directly used in the next step. ES-API: [M+H]⁺=502.1

Step 6: (*R*)-4-Chloro-3-(2-fluorophenyl)-1-((*S*)-2-methyl-4-propionylpiperazin-1-yl)-7,8,9,10-tetrahydro-6*H*-pyrazino[2,1-*c*]pyrido[3,4-*f*][1,4]oxazepin-12(6a*H*)-one (160 mg, 0.32 mmol) was dissolved in dichloromethane (4 mL), and then triethylamine (1.5 mL) and acryloyl chloride (58 mg, 0.64 mmol) were added thereto, and the mixture was stirred under an ice-water bath for 1 hour. After the reaction was completed, the mixture was extracted with water and ethyl acetate, washed with 1 N hydrochloric acid, saturated sodium bicarbonate, brine in turn, dried over anhydrous sodium sulfate, and the filtrate was evaporated to dryness by rotary evaporation and purified by preparative thin-layer chromatography plate (DCM/MeOH=10/1, R_{f}=0.4) to obtain compound (*R*)-8-acryloyl-4-chloro-3-(2-fluorophenyl)-1-((*S*)-2-methyl-4-propionylpiperazin-1-yl)-6,6a,7,8,9,10-hexahydro-12*H*-pyrazino[2,1-*c*]pyrido[3,4-*f*][1,4]oxazepin-12(6a*H*)-one (12 mg, 6.8%). ¹H NMR (400 MHz, CDCl₃) δ 7.41 (s, 2H),7.25 (s, 2H), 6.57 (s, 1H), 6.42 (s, 1H), 5.83 -5.80 (m,1H), 4.35(s,1H),4.18 (s, 1H), 4.02 (d, *J* = 12.1 Hz, 1H),3.71 - 3.41 (m, 10H), 3.18 (d, *J* = 18.7 Hz, 2H), 3.03(s, 1H), 2.32 (d, *J* = 7.8 Hz, 2H), 1.23(s,3H),1.14 (s,3H). ES-API: [M+H]⁺=556.2

### Embodiment 63-20 Synthesis of Z478

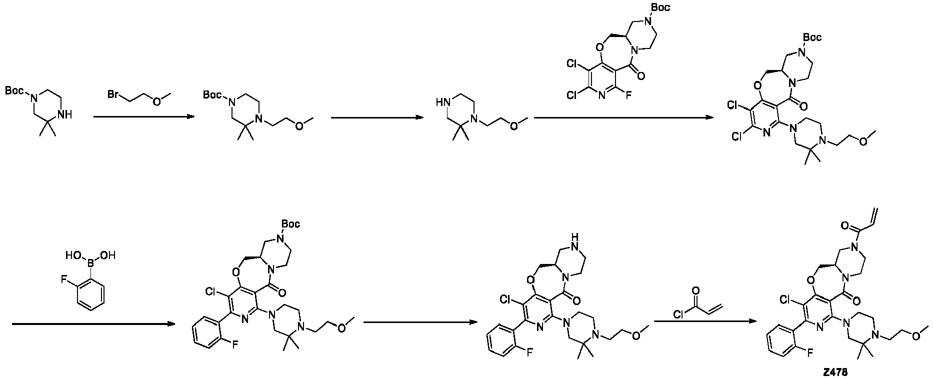

Step 1: *tert*-Butyl 3,3-dimethylpiperazine-1-carboxylate (1 g, 4.67 mmol) and 1-bromo-2-methoxyethane (0.98 g, 7.15 mmol) were dissolved in acetonitrile (20 mL), and potassium carbonate (1.81 g, 13.11 mmol) was added thereto, and the reaction was stirred at room temperature for 16 hours overnight. After the reaction was completed, the mixture was quenched with water, extracted with ethyl acetate, washed with brine, dried over anhydrous sodium sulfate, and the filtrate was evaporated to dryness by rotary evaporation and purified by column to obtain a product of *tert*-butyl 4-(2-methoxyethyl)-3,3-dimethylpiperazine-1-carboxylate (compound 3, colorless oil, 800 mg, yield: 62.9%). ES-API: [M+H]⁺=272.4

Step 2: *tert*-Butyl 4-(2-methoxyethyl)-3,3-dimethylpiperazine-1-carboxylate (800 mg, 2.94 mmol) was dissolved in methanol (20 mL), and trifluoroacetic acid (5 mL) was added thereto, reacted at room temperature for 2 hours. After the reaction was completed, the mixture was directly evaporated to dryness by rotary evaporation to obtain a product of 1-(2-methoxyethyl)-2,2-dimethylpiperazine (compound 4, yellowish oil, 3 g, the crude product was mixed with trifluoroacetic acid). ES-API: [M+H]⁺=172.3

Step 3: *tert*-Butyl 3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (200 mg, 0.49 mmol) and K₂CO₃ (286 mg, 2.0 mmol) were dissolved in acetonitrile (20 mL), and 1-(2-methoxyethyl)-2,2-dimethylpiperazine (168 mg, 0.97 mmol) was added thereto, and the reaction was stirred at 70°C overnight for 16 hours. After the reaction was completed, the mixture was extracted with water and ethyl acetate, washed with brine, dried over anhydrous sodium sulfate, and the filtrate was evaporated to dryness by rotary evaporation and purified by column chromatography (PE/EA=3/1, R_{f}=0.5) to obtain compound *tert*-butyl 3,4-dichloro-1-(4-(2-methoxyethyl)-3,3-dimethylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (compound 6, white solid, 250 mg, yield: 90.9%). ES-API: [M+H]⁺=558.5

Step 4: *tert*-Butyl 3,4-dichloro-1-(4-(2-methoxyethyl)-3,3-dimethylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (190 mg, 0.34 mmol), (2-fluorophenyl)boronic acid (57.2 mg, 041 mmol), tetrakis(triphenylphosphine)palladium (39.5 mg, 0.034 mmol) and potassium carbonate (132 mg, 0.96 mmol) were dissolved in 1,4-dioxane (16 mL) and water (4 mL), reacted under argon protection at 100°C for 16 hours overnight. After the reaction was completed, the mixture was extracted with water and ethyl acetate, washed with brine, dried over anhydrous sodium sulfate, and the filtrate was evaporated to dryness by rotary evaporation and purified by column chromatography (PE/EA=3/1, R_{f}=0.5) to obtain compound *tert*-butyl 4-chloro-3-(2-fluorophenyl)-1-(4-(2-methoxyethyl)-3,3-dimethylpiperazin-1-yl)-12-oxo-6a,7,9,10-tetrahydro-6*H*-pyrazino[2,1-*c*]pyrido [3,4-*f*][1,4]oxazepine-8(12*H*)-carboxylate (white solid, 150 mg, yield: 71.3%). ES-API: [M+H]⁺=618.1

Step 5: *tert*-Butyl 4-chloro-3-(2-fluorophenyl)-1-(4-(2-methoxyethyl)-3,3-dimethylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (150 mg, 0.24 mmol) was dissolved in dichloromethane (3 mL), then trifluoroacetic acid (3 mL) was added thereto, stirred at room temperature for 30 minutes. Then the reaction solution was evaporated to dryness by rotary evaporation to obtain a crude product of 4-chloro-3-(2-fluorophenyl)-1-(4-(2-methoxyethyl)-3,3-dimethylpiperazin-1-yl)-6,6a,7,8,9,10-hexahydro-12*H*-pyrazino[2,1-*c*]pyrido[3,4-*f*][1,4]oxazepin-12-one, which was directly used in the next step.

Step 6: 4-Chloro-3-(2-fluorophenyl)-1-(4-(2-methoxyethyl)-3,3-dimethylpiperazin-1-yl)-6,6a,7,8,9,10-hexahydro-12*H*-pyrazino[2,1-*c*]pyrido[3,4-*f*][1,4]oxazepin-12-one (150 mg, 0.29 mmol) was dissolved in dichloromethane (4 mL), and then triethylamine (1.5 mL) and acryloyl chloride (52.5 mg, 0.58 mmol) were added thereto, and the mixture was stirred under an ice-water bath for 30 minutes. After the reaction was completed, the mixture was extracted with water and ethyl acetate, washed with 1 N hydrochloric acid, saturated sodium bicarbonate, brine in turn, dried over anhydrous sodium sulfate, and the filtrate was evaporated to dryness by rotary evaporation and purified by preparative thin-layer chromatography plate (DCM/MeOH=10/1, R_{f}=0.4) to obtain compound 8-acryloyl-4-chloro-3-(2-fluorophenyl)-1-(4-(2-methoxyethyl)-3,3-dimethylpiperazin-1-yl)-6,6a,7,8,9,10-hexahydro-12*H-*pyrazino[2,1-*c*]pyrido[3,4-*f*][1,4]oxazepin-12-one (27 mg, 16.3%). ¹H NMR (400 MHz, CDCl₃) δ 7.38 (q, *J* = 6.9,6.2 Hz, 2H), 7.21 (s, 1H), 7.16 - 7.06 (m, 1H), 6.56 (t*, J* =14.0 Hz, 1H), 6.39 (d, *J* = 16.4 Hz, 1H), 5.80 (d, *J* = 10.4Hz, 1H), 4.42 - 4.15 (m, 5H), 3.83 - 3.25 (m, 13H), 2.95 -2.26 (m, 4H), 1.24 - 1.08 (m, 6H). ES-API: [M+H]⁺=572.3

### Embodiment 64 Synthesis of (6aS)-8-acryloyl-4-chloro-1-(2,2-dimethylmorpholino)-3-(2-fluoro-6-hydroxyphenyl)-6,6a,7,8,9,10-hexahydro-12H-pyrazino[2,1-c]pyrido[3,4-f][1,4]oxazepin-12-one Z262

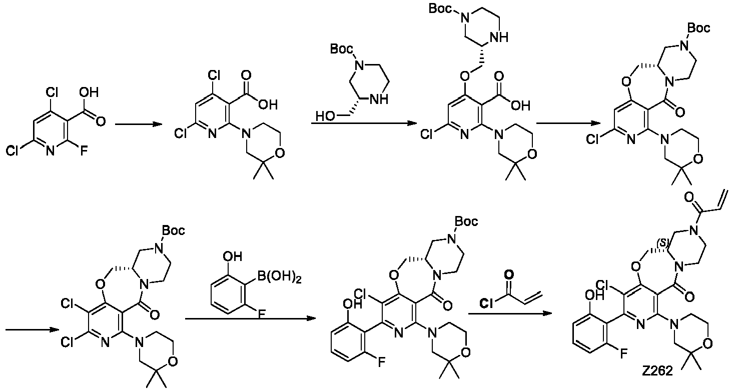

Step 1: 4,6-Dichloro-2-fluoronicotinic acid (500 mg, 2.39 mmol), 2,2-dimethylmorpholine (275 mg, 2.39 mmol) and potassium carbonate (989 mg, 7.17 mmol) dissolved in dichloromethane (5 mL) were added to a 100 mL flask at room temperature. After the reaction was completed, the reaction solution was poured into ice-water, and the aqueous phase was extracted with ethyl acetate to remove impurities. The pH of the aqueous phase was adjusted to 3 with 6 M hydrochloric acid, and the aqueous phase was extracted with ethyl acetate (20 mL×2), dried and concentrated to obtain 4,6-dichloro-2-(2,2-dimethylmorpholino)nicotinic acid (563 mg, 77%). ES-API: [M+H]⁺=305.2.

Step 2: 60% sodium hydride (368 mg, 9.2 mmol), tert-butyl (*S*)-3-(hydroxymethyl)piperazine-1-carboxylate (476 mg, 2.2 mmol) and THF (8 mL) were added to a 100 mL flask. A THF suspension (4 mL) of 4,6-dichloro-2-(2,2-dimethylmorpholino)nicotinic acid (600 mg, 1.84 mmol) was added dropwise thereto at 0°C. The reaction was carried out in an ice bath at 0°C for 20 minutes. The completion of reaction was detected by LC-MS. 30 mL of saturated aqueous NH₄Cl solution was poured into the reaction solution, and the pH was adjusted to 5-6, and the mixture was extracted 4 times with 40 mL of ethyl acetate. The organic phase was dried and then concentrated to obtain (*S*)-4-((4-(*tert*-butoxycarbonyl)piperazin-2-yl)methoxy)-6-chloro-2-(2,2-dimethylmorpholino)nicotinic acid (crude product, 910 mg) as a yellow solid. ES-API: [M+H]⁺=485.2.

Step 3: (*S*)-4-((4-(*tert*-Butoxycarbonyl)piperazin-2-yl)methoxy)-6-chloro-2-(2,2-dimethylmorpholino)nicotinic acid (910 mg), diisopropylethylamine (4 mL) and dichloromethane (10 mL) were added to a 100 mL flask. Propylphosphonic anhydride solution (5 mL, 50%w/w ethyl acetate solution) was added dropwise thereto. The mixture was reacted at room temperature for 1 hour. The completion of reaction was detected by LC-MS. 30 mL of dichloromethane was added to the reaction, then washed with 30 mL of water, 10 mL of aqueous hydrochloric acid solution (1 M), and 30 mL of sodium bicarbonate, respectively. The organic phase was dried and concentrated to obtain *tert*-butyl (*S*)-3-chloro-1-(2,2-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (crude product, 725 mg) as a yellow solid. ES-API: [M+H]⁺=467.3.

Step 4: *tert*-Butyl (*S*)-3-chloro-1-(2,2-dimethylmorpholino)-12-oxo-6a,7,9,10-tetrahydro-12*H-*pyrazino[2,1-*c*]pyrido[3,4-*f*][1,4]oxazepine-8(6*H*)-carboxylate (crude product, 725 mg), *N-*chlorosuccinimide (416 mg, 3.1 mmol) and acetonitrile (10 mL) were added to a 100 mL flask. The reaction was stirred at 80°C for 2 hours. Aqueous sodium thiosulfate solution was added to the reaction solution and extracted with ethyl acetate. The organic phase was dried and concentrated. The crude product was purified by flash silica gel column (EtOAc/PE: 0-50%) to obtain *tert*-butyl (*S*)-3,4-dichloro-1-(2,2-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (550 mg, 45%). ES-API: [M+H]⁺=502.2.

Step 5: *tert*-Butyl (*S*)-3,4-dichloro-1-(2,2-dimethylmorpholino)-12-oxo-6a,7,9,10-tetrahydro-12*H*-pyrazino[2,1-*c*]pyrido[3,4-*f*][1,4]oxazepine-8(6*H*)-carboxylate (281.1 mg, 0.56 mmol), 2-fluoro-6-hydroxyphenylboronic acid (262 mg, 1.68 mmol), SPhos (23 mg, 0.056 mmol), SPhos-Pd-G2 (40 mg, 0.056 mmol), potassium phosphate (356 mg, 1.68 mmol), 4 mL of dioxane and 1 mL of water were added to a 100 mL reaction flask. The reaction was carried out in an oil bath at 110°C for 2 hours, then the reaction solution was added with 20 mL of water, extracted three times with 20 mL of ethyl acetate. The organic phase was dried and concentrated, and the crude product was purified by flash silica gel column (EtOAc/PE: 0-60%) to obtain *tert*-butyl (6a*S*)-4-chloro-1-(2,2-dimethylmorpholino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (142 mg, 43%) as a yellow solid. ES-API: [M+H]⁺=577.2.

Step 6: *tert*-Butyl (6a*S*)-4-chloro-1-(2,2-dimethylmorpholino)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (142 mg, 0.25 mmol), 3 mL of dichloromethane and trifluoroacetic acid (2 mL) were added to a 50 mL flask. The mixture was stirred at room temperature for 1 hour, and the completion of reaction was detected by LC-MS. The reaction solution was concentrated to obtain a crude product (130 mg) as a yellow oil, and then 5 mL of dichloromethane and triethylamine (166 mg, 1.65 mmol) were added. At 0°C, a solution of acrylic anhydride in dichloromethane (30 mg, 0.24 mmol, 0.5 mL) was added dropwise thereto. The reaction was carried out at 0°C for 10 minutes. 20 mL of saturated aqueous sodium bicarbonate solution was added to the reaction solution, and the mixture was extracted three times with 10 mL of dichloromethane. The organic phase was dried and concentrated, and the crude product was purified by preparative HPLC to obtain (6a*S*)-8-acryloyl-4-chloro-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*[1,4]oxazepin-12-one (Z262, 22 mg, 16.7%) as a white solid. ¹H NMR (500 MHz, DMSO-*d*₆) δ 7.25 (dd, *J* = 15.9, 7.7 Hz, 1H), 6.82-6.67 (m, 3H), 6.17 (d, *J* = 17.3 Hz, 1H), 5.79-5.70 (m, 1H), 4.54-3.90 (m, 7H), 3.85-3.40 (m, 6H), 3.20-3.00 (m, 2H), 1.13 (s, 6H). ES-API: [M+H]⁺=531.2.

### Embodiment 65 Synthesis of (6aR)-8-acryloyl-4-chloro-3-(2-fluoro-6-hydroxyphenyl)-1-(4-methyl-4,7-diazaspiro[2.5]octan-7-yl)-6,6a,7,8,9,10-hexahydro-12H-pyrazino[2,1-c]pyrido[3,4-f][1,4]oxazepin-12(6aH)-one Z83

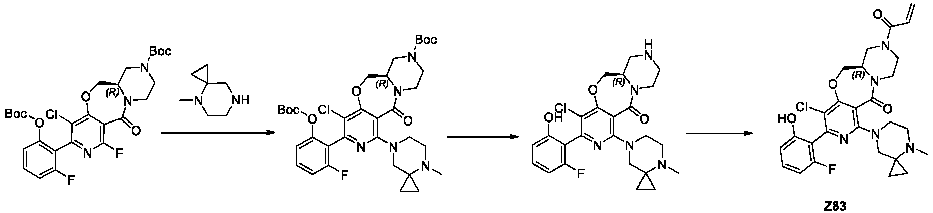

Step 1: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*[1,4]oxazepine-8(6*H*)-carboxylate (100 mg, 0.0.17 mmol), 4-methyl-4,7-diazaspiro[2.5]octane (75 mg, crude product) and potassium carbonate (47 mg, 0.34 mmol) were dissolved in acetonitrile (5 mL), reacted at 80°C for 4 hours. Then the reaction system was cooled to room temperature, quenched with water, extracted with ethyl acetate, washed once with brine, dried over anhydrous sodium sulfate, and the filtrate was evaporated to dryness by rotary evaporation to obtain a crude product of *tert-*butyl (6a*R*)-3-(2-((*tert*-butoxycarbonyl)oxy)-6-fluorophenyl)-4-chloro-1-(4-methyl-4,7-diazaspiro[2.5]octan)-12-oxo-6a,7,9,10-tetrahydro-6*H*-pyrazino[2,1-c]pyrido[3,4-*f*][1,4]oxazepine-8(6*H*)-carboxylate (110 mg, 84.4%). ES-API: [M+H]⁺= 688.3.

Step 2: *tert*-Butyl (6a*R*)-3-(2-((*tert*-butoxycarbonyl)oxy)-6-fluorophenyl)-4-chloro-1-(4-methyl-4,7-diazaspiro[2.5]octan)-12-oxo-6a,7,9,10-tetrahydro-6*H*-pyrazino[2,1-*c*]pyrido[3,4-*f*][1,4]oxazepine-8(6*H*)-carboxylate (110 mg, 0.162 mmol) was dissolved in dichloromethane (4 mL), and then trifluoroacetic acid (2 mL) was added dropwise thereto, stirred at room temperature for 0.5 hours. Then the mixture was evaporated to dryness by rotary evaporation to obtain a crude product of (6a*R*)-4-chloro-3-(2-fluoro-6-hydroxyphenyl)-1-(4-methyl-4,7-diazaspiro[2.5]octan-1-yl)-7,8,9,10-tetrahydro-6*H*-pyrazino[2,1-*c*]pyrido[3,4-*f*][1,4]oxazepin-12-one (100 mg, crude product). ES-API: [M+H]⁺=488.2.

Step 3: (6a*R*)-4-Chloro-3-(2-fluoro-6-hydroxyphenyl)-1-(4-methyl-4,7-diazaspiro[2.5]octan-1-yl)-7,8,9,10-tetrahydro-6*H*-pyrazino[2,1-*c*]pyrido[3,4-*f*][1,4]oxazepin-12-one (100 mg, crude product) was dissolved in dichloromethane (2 mL), then cooled to 0°C in an ice-water bath, and triethylamine (1 mL) was added dropwise thereto. The pH value was basic, and acrylic anhydride (15 mg, 0.12 mmol) was added dropwise thereto, stirred for 10 minutes. After the reaction was completed, the reaction solution was added with water, washed with brine, dried over sodium sulfate, evaporated to dryness by rotary evaporation, and the crude product was purified by preparative HPLC to obtain (6a*R*)-8-acryloyl-4-chloro-3-(2-fluoro-6-hydroxyphenyl)-1-(4-methyl-4,7-diazaspiro[2.5]octan-1-yl)-6,6a,7,8,9,10-hexahydro-12*H*-pyrazino[2,1-*c*]pyrido[3,4-*f*][1,4]oxazepin-12-one (Z83, 11 mg, yield: 10.2%). (Preparation conditions: Ultimate XB-C18, 50*250 mm, 10 µm, mobile phase system: A: purified water B: pure acetonitrile, flow rate: 80 mL/min, gradient: within 50 minutes, B/A= 20%-90%, wavelength: 214 nm, column temperature: room temperature). ¹H NMR (400 MHz, MeOH-*d₄*) δ 7.22 (q, *J* = 7.7 Hz, 1H), 6.84-6.60 (m, 3H), 6.31 (d, *J* = 16.4 Hz, 1H), 5.86-5.79 (m,1H), 5.36-5.30 (m,1H), 4.65-4.40 (m, 5H), 4.38-3.90 (m, 5H), 3.86-3.61 (m, 5H), 2.80 (s, 3H), 1.10-0.98 (m, 2H), 0.91-0.79 (m, 2H). ES-API: [M+H]⁺= 542.2.

### Embodiment 66 Synthesis of (6aR)-8-acryloyl-4-chloro-1-((2S,4S)-4-(dimethylamino)-2-methylpyrrolidin-1-yl)-3-(2-fluoro-6-hydroxyphenyl)-6,6a,7,8,9,10-hexahydro-12H-pyrazino[2,1-c]pyrido[3,4-f][1,4]oxazepin-12-one Z237-2

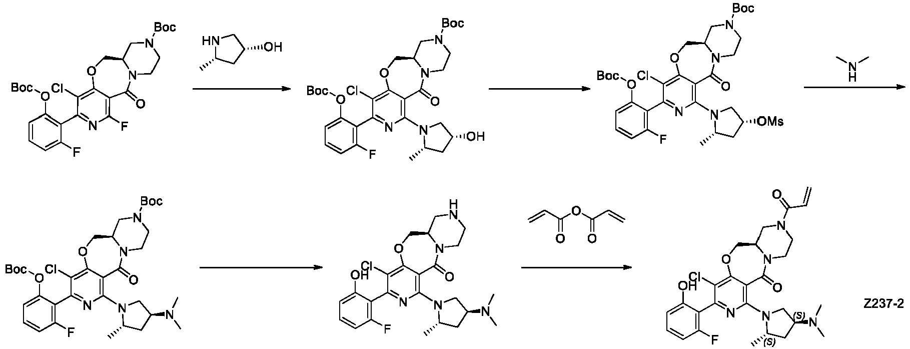

Step 1: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*[1,4]oxazepine-8(6*H*)-carboxylate (320 mg, 0.55 mmol), (3*R*,5*S*)-5-methylpyrrolidin-3-ol (76 mg, 0.55 mmol) and potassium carbonate (230 mg, 1.65 mmol) were dissolved in acetonitrile (4 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain *tert*-butyl (6a*R*)-3-(2-((*tert*-butoxycarbonyl)oxy)-6-fluorophenyl)-4-chloro-1-((2*S*,4*R*)-4-hydroxy-2-methylpyrrolidin-1-yl)-12-oxo-6a,7,9,10-tetrahydro-12*H*-pyrazino[2,1-*c*]pyrido[3,4-*f*[1,4]oxazepine-8(6*H*)-carboxylate (440 mg, yield: 100%). ES-API: [M+H]⁺= 663.1.

Step 2: *tert*-Butyl (6a*R*)-3-(2-((*tert*-butoxycarbonyl)oxy)-6-fluorophenyl)-4-chloro-1-((2S,4R)-4-hydroxy-2-methylpyrrolidin-1-yl)-12-oxo-6a,7,9,10-tetrahydro-12*H*-pyrazino [2,1-c]pyrido [3,4-*f*][1,4]oxazepine-8(6*H*)-carboxylate (400 mg, 0.604 mmol) was dissolved in 4 mL of dichloromethane, then methanesulfonyl chloride (350 mg, 3.02 mmol) and pyridine (240 mg, 3.02 mmol) were added thereto, reacted at room temperature for 2 hours. The reaction was monitored to be complete, and the mixture was extracted with ethyl acetate, washed once with brine, dried over anhydrous sodium sulfate, and the filtrate was evaporated to dryness by rotary evaporation to obtain *tert*-butyl (6a*R*)-3-(2-((*tert*-butoxycarbonyl)oxy)-6-fluorophenyl)-4-chloro-1-((2*S*,4*R*)-2-methyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (900 mg, yield: 100%). ES-API: [M+H]⁺= 741.1.

Step 3: *tert*-Butyl (6a*R*)-3-(2-((*tert*-butoxycarbonyl)oxy)-6-fluorophenyl)-4-chloro-1-((2S,4R)-2-methyl-4-((methylsulfonyl)oxy)pyrrolidin-1-yl)-12-oxo-6a,7,9,10-tetrahydro-12*H*-pyrazino [2,1-*c*]pyrido[3,4-*f*][1,4]oxazepine-8(6*H*)-carboxylate was dissolved in 10 mL of a solution of dimethylamine in THF, and then *N,N*-diisopropylethylamine (600 mg, 5.0 mmol) was added thereto, reacted at 100°C for 48 hours. The reaction was monitored to be complete, and then the reaction solution was cooled to room temperature, then added with 5 mL of di-*tert*-butyl dicarbonate, reacted for 1 hour. The reaction was monitored to be complete, and the mixture was extracted with ethyl acetate, washed once with brine, dried over anhydrous sodium sulfate, and the filtrate was evaporated to dryness by rotary evaporation and purified (dichloromethane/methanol = 20/1) to obtain *tert*-butyl (6a*R*)-3-(2-((*tert*-butoxycarbonyl)oxy)-6-fluorophenyl)-4-chloro-1-((2*S*,4*S*)-4-(dimethylamino)-2-methylpyrrolidinon-1-yl)-12-oxo-6a,7,9,10-tetrahydro-12*H*-pyrazino[2,1-*c*]pyrido[3,4-*f*][1,4]oxazepine-8(6*H*)-carboxylate (130 mg, yield: 20%). ES-API: [M+H]⁺= 690.1.

Step 4: *tert*-Butyl (6a*R*)-3-(2-((*tert*-butoxycarbonyl)oxy)-6-fluorophenyl)-4-chloro-1-((2*S*,4*S*-4-(dimethylamino)-2-methylpyrrolidinon-1-yl)-12-oxo-6a,7,9,10-tetrahydro-12*H*-pyrazino[2,1-*c*]pyrido[3,4-*f*][1,4]oxazepine-8(6*H*)-carboxylate (130 mg, 0.188 mmol) was dissolved in dichloromethane (4 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-1-((2*S*,4*S*-4-(dimethylamino)-2-methylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (90 mg, 100%). ES-API: [M+H]⁺= 490.

Step 5: (6a*R*)-4-Chloro-1-((2*S*,4*S*)-4-(dimethylamino)-2-methylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (90 mg, 0.188 mmol) was dissolved in dichloromethane (4 mL), then cooled to 0°C in an ice-water bath, and triethylamine (2 mL) was added dropwise thereto. The pH value was basic, and acrylic anhydride (19 mg, 0.15 mmol) was added dropwise thereto, stirred for 10 minutes. After the reaction was completed, the mixture was extracted with ethyl acetate, washed with water, washed with brine, dried over sodium sulfate, evaporated to dryness by rotary evaporation, and the crude product was purified by chromatography plate (dichloromethane/methanol=10/ 1, R_{f}=0.5) to obtain (6a*R*)-8-acryloyl-4-chloro-1-((2*S*,4*S*)-4-(dimethylamino)-2-methylpyrrolidin-1-yl)-3-(2-fluoro-6-hydroxyphenyl)-6,6a,7,8,9,10-hexahydro-12*H-*pyrazino[2,1-c]pyrido[3,4-*f*][1,4]oxazepin-12-one (Z237-2, 14.5 mg, yield: 20%). ¹H NMR (400 MHz, CDCl₃) δ7.29 (d, *J* = 8.1 Hz, 1H), 6.82 (d, *J* = 8.3 Hz, 1H), 6.68 (t, *J* = 9.1Hz, 1H), 6.56 (d, *J* = 12.2 Hz, 1H), 6.38 (d, *J* = 16.7 Hz, 1H), 5.81 (d, *J* = 10.5 Hz, 1H), 4.54 (s, 1H), 4.43- 4.21 (m, 3H), 3.99 (d, *J* = 81.1 Hz, 3H), 3.54 (d, *J* = 10.9 Hz, 3H), 3.33 (s, 2H), 2.93 (s, 1H), 2.43 (s,6H), 1.78 (s, 2H), 1.39 (d, *J* = 6.1 Hz, 3H). ES-API: [M+H]⁺= 544.1.

### Embodiment 67 Synthesis of (6aR)-8-acryloyl-4-chloro-1-((2S,4R)-4-(dimethylamino)-2-methylpyrrolidin-1-yl)-3-(2-fluoro-6-hydroxyphenyl)-6,6a,7,8,9,10-hexahydro-12H-pyrazino[2,1-c]pyrido[3,4f][1,4]oxazepin-12-one Z237-1

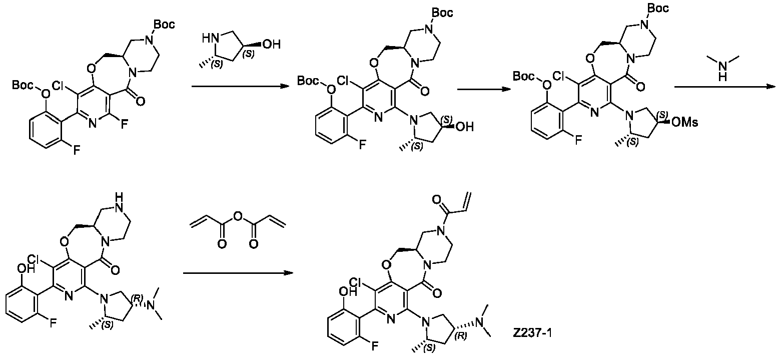

Step 1: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*[1,4]oxazepine-8(6*H*)-carboxylate (100 mg, 0.172 mmol), (3S,5S)-5-methylpyrrolidin-3-ol (24 mg, 0.172 mmol) and potassium carbonate (71 mg, 0.516 mmol) were dissolved in acetonitrile (mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain *tert*-butyl (6a*R*)-3-(2-((*tert*-butoxycarbonyl)oxy)-6-fluorophenyl)-4-chloro-1-((2*S*,4*S*)-4-hydroxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (110 mg, yield: 100%).

Step 2: *tert*-Butyl (6a*R*)-3-(2-((*tert*-butoxycarbonyl)oxy)-6-fluorophenyl)-4-chloro-1-((2*S*,4*S*-4-hydroxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (110 mg, 0.17 mmol) was dissolved in 4 mL of dichloromethane, then methanesulfonyl chloride (38 mg, 0.34 mmol) and pyridine (40 mg, 0.51 mmol) were added thereto, reacted at room temperature for 2 hours. The reaction was monitored to be complete, and the mixture was extracted with ethyl acetate, washed once with brine, dried over anhydrous sodium sulfate, and the filtrate was evaporated to dryness by rotary evaporation to obtain *tert*-butyl (6a*R*)-3-(2-((*tert*-butoxycarbonyl)oxy)-6-fluorophenyl)-4-chloro-1-((2*S*,4*S*)-2-methyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (100 mg, yield: 81%). ES-API: [M+H]⁺= 741.1.

Step 3: *tert*-Butyl (6a*R*)-3-(2-((*tert*-butoxycarbonyl)oxy)-6-fluorophenyl)-4-chloro-1-((2*S*,4*S*-2-methyl-4-((methylsulfonyl)oxy)pyrrolidin-1-yl)-12-oxo-6a,7,9,10-tetrahydro-12*h*-pyrazino[2,1-*c*]pyrido[3,4-*f*][1,4]oxazepine-8(6*H*)-carboxylate (100 mg, 0.135 mmol) was dissolved in 2 mL of a solution of dimethylamine in THF, and then *N,N*-diisopropylethylamine (174 mg, 1.35 mmol) was added thereto, reacted at 100°C for 48 hours. The reaction was monitored to be complete, and the mixture was extracted with ethyl acetate, washed once with brine, dried over anhydrous sodium sulfate, and the filtrate was evaporated to dryness by rotary evaporation and purified (dichloromethane/methanol=20/1, R_{f}=0.6) to obtain compound (6a*R*)-4-chloro-1-((2*S*,4*R*)-4-(dimethylamino)-2-methylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (48 mg, yield: 72%). ES-API: [M+H]⁺= 490.1.

Step 4: (6a*R*)-4-Chloro-1-((2*S*,4*R*)-4-(dimethylamino)-2-methylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (48 mg, 0.098 mmol) was dissolved in dichloromethane (3 mL), and triethylamine (30 mg, 0.294 mmol) was added dropwise thereto at 0°C. The pH value was basic, and acrylic anhydride (9.9 mg, 0.078 mmol) was added dropwise thereto, stirred for 10 minutes. After the reaction was completed, the mixture was extracted with ethyl acetate, washed with water, washed with brine, dried over sodium sulfate, evaporated to dryness by rotary evaporation, and the crude product was purified by chromatography plate (dichloromethane/methanol=10/1, R_{f}=0.5) to obtain (6a*R*)-8-acryloyl-4-chloro-1-((2*S*,4*R*)-4-(dimethylamino)-2-methylpyrrolidin-1-yl)-3-(2-fluoro-6-hydroxyphenyl)-6,6a,7,8,9,10-hexahydro-12*H-*pyrazino[2,1-c]pyrido[3,4-*f*][1,4]oxazepin-12-one (Z237-1, 1.8 mg, yield: 3%). ¹H NMR (400 MHz, CDCl₃) δ 7.34 (d, *J* = 8.1 Hz, 1H), 6.83 (d, *J* = 8.7 Hz, 1H), 6.71 (s, 1H), 6.41 (s, 1H), 6.41 (s, 1H), 5.80 (s, 1H), 4.51-4.29 (m, 4H), 4.25-4.14(m,3H),3.73-3.65(m,2H), 3.55 (d, *J* = 13.8 Hz,3H), 3.26 (s, 1H), 2.54 (s,6H), 1.43 (s, 2H), 1.29 (d, *J* = 6.0 Hz, 3H). ES-API: [M+H]⁺= 544.2.

### Embodiment 67-1 Synthesis of Z358-1

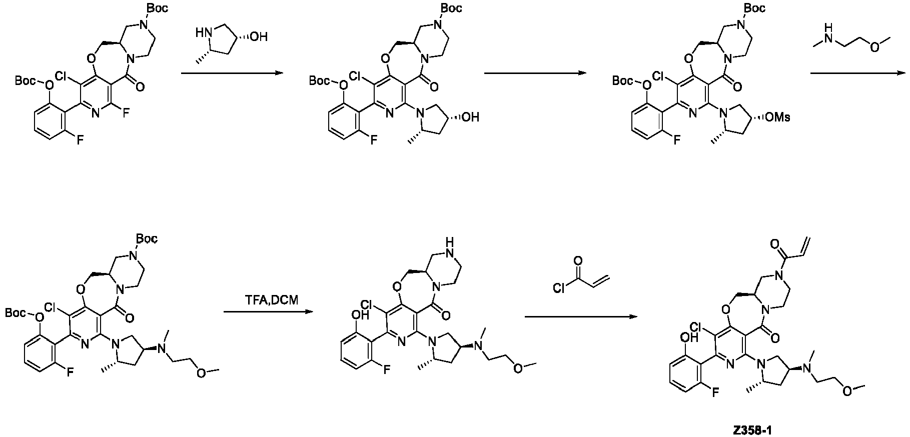

Step 1: (6a*R*)-*tert*-Butyl 3-(2-((*tert*-butoxycarbonyl)oxy)-6-fluorophenyl)-4-chloro-1-fluoro-12-oxo-6a,7,9,10-tetrahydro-6*H*-pyrazino[2,1-*c*]pyrido[3,4-*f*][1,4]oxazepine-8(12*H*)-carboxylate (200 mg, 0.34 mmol) was dissolved in acetonitrile (2 mL), then (3*R*,5*S*)-5-methylpyrrolidin-3-ol (42 mg, 0.41 mmol) and potassium carbonate (95 mg, 0.69 mmol) were added thereto, heated to 80°C and reacted overnight. The mixture was cooled to room temperature, and di-*tert*-butyl dicarbonate (150 mg, 0.69 mmol) was added thereto and stirred for 1 hour at room temperature. After the reaction was completed, the reaction mixture was quenched by adding water, extracted with ethyl acetate, dried, evaporated to dryness by rotary evaporation, and purified by column chromatography (petroleum ether/ethyl acetate=1/2, R_{f}=0.6) to obtain a product (230 mg, yield: 100%). ES-API: [M+H]⁺=663.3.

Step 2: (6a*R*)-*tert*-Butyl 3-(2-((*tert*-butoxycarbonyl)oxy)-6-fluorophenyl)-4-chloro-1-((2S,4R)-4-hydroxy-2-methylpyrrolidin-1-yl)-12-oxo-6a,7,9,10-tetrahydro-6*H*-pyrazino[2,1-*c*]pyrido[3,4-*f*[1,4]oxazepine-8(12*H*)-carboxylate (230 mg, 0.35 mmol) was dissolved in dichloromethane (2 mL), and then triethylamine (105 mg, 1.0 mmol) and methanesulfonyl chloride (59 mg, 0.52 mmol) were added thereto. The reaction solution was reacted at room temperature for 2 hours. After TLC monitoring that the reaction was completed, the mixture was added with water and dichloromethane for extraction, washed once with 0.5 M hydrochloric acid, dried and evaporated to dryness to obtain a product (250 mg, yield: 97%).

Step 3: (6a*R*)-*tert*-Butyl 3-(2-((*tert*-butoxycarbonyl)oxy)-6-fluorophenyl)-4-chloro-1-((2S,4R)-2-methyl-4-((methylsulfonyl)oxy)pyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (200 mg, 0.27 mmol) was dissolved in 2-methoxy-*N-*methylethylamine (552 mg, 6.2 mmol). The reaction solution was heated to 100°C and reacted overnight. After the reaction was completed, the reaction mixture was extracted with water and ethyl acetate, dried, evaporated to dryness, and purified by column chromatography (dichloromethane/methanol=10/1, R_{f}=0.5) to obtain a product (78 mg, yield: 39%). ES-API: [M+1-100]⁺=634.3.

Step 4: (6a*R*)-*tert*-Butyl 3-(2-((*tert*-butoxycarbonyl)oxy)-6-fluorophenyl)-4-chloro-1-((2*S*,4*S*)-4-((2-methoxyethyl)(methyl)amino)-2-methylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (78 mg, 0.12 mmol) was dissolved in dichloromethane (1 mL), and then trifluoroacetic acid (0.5 mL) was added thereto. The reaction solution was reacted at room temperature for 1 hour. After TLC monitoring that the reaction was completed, the reaction solution was evaporated to dryness, dissolved in dichloromethane and evaporated to dryness to obtain a crude product, which was directly used in the next reaction step.

Step 5: (6a*R*)-4-Chloro-3-(2-fluoro-6-hydroxyphenyl)-1-((2*S*,4*S*)-4-((2-methoxyethyl)(methyl)amino)-2-methylpyrrolidin-1-yl)-7, 8,9,10-tetrahydro-6*H*-pyrazino [2,1-*c*]pyrido [3,4-*f*][1,4]oxazepin-12(6a*H*)-one (crude product, 0.12 mmol) was dissolved in tetrahydrofuran (1 mL), cooled to 0 to 5°C, and water (0.5 mL) and sodium bicarbonate (206 mg, 2.4 mmol) were added thereto. A solution of acryloyl chloride (11 mg, 0.12 mmol) in tetrahydrofuran was added dropwise thereto under an ice bath and stirred for 0.5 hours. After the reaction was completed, the mixture was extracted with ethyl acetate and water, and the organic phase was dried, evaporated to dryness by rotary evaporation, and purified by preparative thin-layer chromatography column to obtain (6a*R*)-8-acryloyl-4-chloro-3-(2-fluoro-6-hydroxyphenyl)-1-((2*S*,4*S*)-4-((2-methoxyethyl)(methyl)amino)-2-methylpyrrolidin-1-yl)-6,6a,7,8,9,10-hexahydro-12*H*-pyrazino[2,1-*c*]pyrido[3,4-*f*][1,4]oxazepin-12-one (24.1 mg, yield: 34%, white solid). ¹H NMR (400 MHz, CDCl₃) δ 7.25-7.23(m, 1H), 6.82 (d, *J* = 8.4 Hz, 1H), 6.69 (t, *J* = 9.1 Hz, 1H), 6.56 (brs, 1H), 6.39 (d, *J* = 16.4 Hz, 1H), 5.81 (d, *J* = 10.3 Hz, 1H), 4.47-4.32 (m, 3H), 4.24-4.11 (m, 2H), 3.99 (s, 1H), 3.78-3.42 (m, 7H), 3.36 (s, 3H), 3.20-3.11 (m, 1H), 2.87-2.54 (m, 2H), 2.37 (s, 3H), 2.12-2.00 (m, 1H), 1.83-1.77 (m, 1H), 1.27 (d, *J* = 6.9 Hz, 3H). ES-API: [M+H]⁺=588.3.

### Embodiment 68 Synthesis of (6aR)-8-acryloyl-1-(4-methoxy-2,2-dimethylpyrrolidin-1-yl)-4-fluoro-3-(2-fluoro-6-hydroxyphenyl)-6,6a,7,8,9,10-hexahydro-12H-pyrazino[2,1-c]pyrido[3,4-f][1,4]oxazepin-12-one Z256a

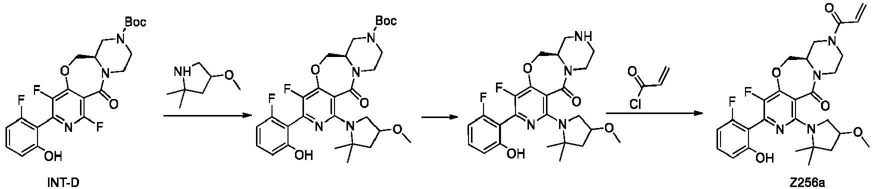

Step 1: (6a*R*)-8-*tert*-Butoxycarbonyl-1,4-difluoro-3-(2-fluoro-6-hydroxyphenyl)-6,6a,7,8,9,10-hexahydro-12*H*-pyrazino[2,1-*c*]pyrido[3,4-*f*[1,4]oxazepin-12-one (540 mg, 1.16 mmol), 4-methoxy-2,2-dimethylpyrrolidine (164 mg, 1.28 mmol), diisopropylethylamine (448 mg, 3.48 mmol) and acetonitrile (10 mL) were added to a 100 mL flask. The reaction was carried out at 80°C for 1 hour. The mixture was extracted with ethyl acetate, washed with aqueous ammonium chloride solution and saturated brine in turn, and the organic phase was dried and concentrated, and purified by silica gel column to obtain (6a*R*)-8-tert-butoxycarbonyl-1-(4-methoxy-2,2-dimethylpyrrolidinyl)-4-fluoro-3-(2-fluoro-6-hydroxyphenyl)-6,6a,7,8,9,10-hexahydro-12*H*-pyrazino[2,1-*c*]pyrido[3,4*-f*][1,4]oxazepin-12-one (600 mg, yield of 90%). ES-API: [M+H]⁺=575.2.

Step 2: (6a*R*)-8-*tert*-Butoxycarbonyl-1-(4-methoxy-2,2-dimethylpyrrolidinyl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (600 mg, 1.04 mmol), 3 mL of trifluoroacetic acid and 5 mL of dichloromethane were added to a 50 mL flask. The mixture was stirred at room temperature for 1 hour, and the completion of reaction was detected by LC-MS. The reaction solution was concentrated to obtain a crude product of (6a*R*)-1-(4-methoxy-2,2-dimethylpyrrolidinyl)-4-fluoro-3-(2-fluoro-6-hydroxyphenyl))-6,6a,7,8,9,10-hexahydro-12*H*-pyrazino [2,1-*c*]pyrido[3,4-*f*][1,4]oxazepin-12-one (496 mg, trifluoroacetate) as a yellow solid. ES-API: [M+H]⁺=475.2.

Step 3: (6a*R*)-1-((2*S*,6*R*)-2,6-Dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one trifluoroacetate (496 mg, crude product), 10 mL of dichloromethane and diisopropylethylamine (402 mg, 3.12 mmol) were added to a 50 mL flask. At 0°C, a solution of acryloyl chloride in dichloromethane (84 mg, 1.04 mmol, 1 mL) was added dropwise thereto. The reaction was carried out at 0°C for 10 minutes. The mixture was washed with 20 mL of water, and the organic phase was dried and concentrated, and the crude product was purified by preparative HPLC to obtain (6a*R*)-8-acryloyl-1-(4-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z256a, 274 mg, 3-step yield: 45%) as a white solid. ES-API: [M+H]⁺=529.2.

### Embodiment 68-1 Synthesis of Z255

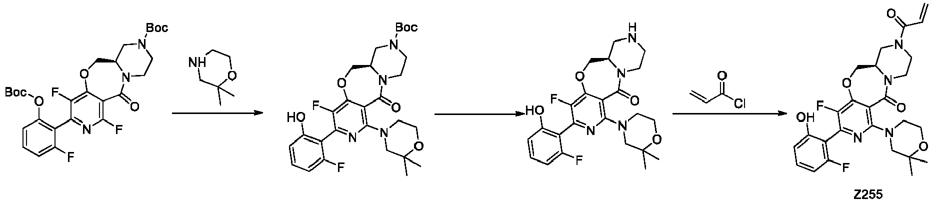

Step 1: (6a*R*)-8-*tert*-Butoxycarbonyl-3-(2-((*tert*-butoxycarbonyl)oxy)-6-fluorophenyl)-4-fluoro-1-fluoro-6,6a,7,8,9,10-hexahydro-12*H*-pyrazino[2,1-*c*]pyrido[3,4-*f*][1,4]oxazepin-12-one (130 mg, 0.23 mmol), 2,2-dimethylmorpholine (79 mg, 0.69 mmol), *N,N*-diisopropylethylamine (148 mg, 1.15 mmol) and acetonitrile (10 mL) were added to a 100 mL round bottom flask, stirred at 80°C for 1.5 hours. The mixture was extracted with ethyl acetate, washed with saturated brine, and the organic phase was dried, concentrated, and purified by silica gel column (petroleum ether: ethyl acetate = 20:80) to obtain (6a*R*)-8-*tert-*butoxycarbonyl-1-(2,2-dimethylmorpholino)-4-fluoro-3 -(2-fluoro-6-hydroxyphenyl)-6,6a,7,8,9,10-hexahydro-12*H*-pyrazino[2,1-c]pyrido[3,4-*f*][1,4]oxazepin-12-one (125 mg, yield of 97%). ES-API: [M+H]⁺=561.3.

Step 2: (6a*R*)-8-*tert*-Butoxycarbonyl-1-(2,2-dimethylmorpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (125 mg, 0.227 mmol), 2 mL of trifluoroacetic acid and 5 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(2,2-dimethylmorpholino)-4-fluoro-3-(2-fluoro-6-hydroxyphenyl)-6,6a,7,8,9,10-hexahydro-12*H*-pyrazino [2,1-*c*]pyrido[3,4-*f*][1,4]oxazepin-12-one (102 mg, crude product) as a yellow solid. ES-API: [M+H]⁺=461.3.

Step 3: (6a*R*)-1-(2,2-Dimethylmorpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (102 mg, 0.227 mmol), 10 mL of dichloromethane and *N,N*-diisopropylethylamine (146 mg, 1.13 mmol) were added to a 50 mL round bottom flask. The reaction was cooled to 0°C, and a solution of acryloyl chloride in dichloromethane (18.4 mg, 0.204 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:H₂O=50:50 (0.1% NH₄HCO₃), flow rate: 15 mL/min, column temperature: 25°C) to obtain a target product of (6a*R*)-8-acryloyl-1-(2,2-dimethylmorpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (30 mg, 3-step yield of 45%) as a white solid. ES-API: [M+H]⁺= 515.2. ¹H NMR (500 MHz, DMSO-*d₆*) δ 10.10 *(d, J* = 1.5 Hz, 1H), 7.28 (td, *J* = 8.3, 6.8 Hz, 1H), 6.80 - 6.70 (m, 3H), 6.17 (d, *J* = 16.9 Hz, 1H), 5.75 (d, *J* = 11.7 Hz, 1H), 4.49 - 4.30 (m, 2H), 4.29 - 4.17 (m, 1H), 4.09 - 4.01 (m, 2H), 3.91-3.86 (m, 1H), 3.74-3.70 (m, 1H), 3.66 - 3.52 (m, 3H), 3.37 (d, *J* = 13.8 Hz, 1H), 3.28 (s, 1H), 3.19 - 2.99 (m, 3H), 1.14 (d, *J* = 6.3 Hz, 6H).

### Embodiment 68-2 Synthesis of Z327

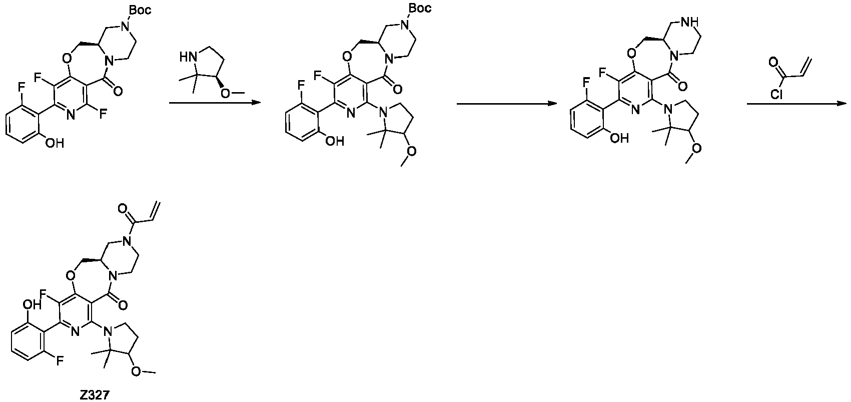

Step 1: (6a*R*)-8-*tert*-Butoxycarbonyl-1,4-difluoro-3-(2-fluoro-6-hydroxyphenyl)-6,6a,7,8, 9,10-hexahydro-12*H*-pyrazino[2,1-*c*]pyrido[3,4-*f*][1,4]oxazepin-12-one (324 mg, 0.696 mmol), 3-methoxy-2,2-dimethylpyrrolidine (96.4 mg, 0.768 mmol), *N,N*-diisopropylethylamine (268 mg, 2.08 mmol) and acetonitrile (10 mL) were added to a 100 mL round bottom flask. The reaction was stirred at 80 °C for 1 hour. The mixture was extracted with ethyl acetate, washed with aqueous ammonium chloride solution and saturated brine in turn, and the organic phase was dried and concentrated, and purified by silica gel column to obtain (6a*R*)-8-*tert*-butoxycarbonyl-1-(3-methoxy-2,2-dimethylpyrrolidinyl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (360 mg, yield of 90%). ES-API: [M+H]⁺=575.2.

Step 2: (6a*R*)-8-*tert*-Butoxycarbonyl-1-(3-methoxy-2,2-dimethylpyrrolidinyl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (360 mg, 0.624 mmol), 3 mL of trifluoroacetic acid and 5 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(3-methoxy-2,2-dimethylpyrrolidinyl)-4-fluoro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (297 mg, trifluoroacetate) as a yellow solid. ES-API: [M+H]⁺=475.2.

Step 3: (6a*R*)-1-(3-Methoxy-2,2-dimethylpyrrolidinyl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (297 mg, trifluoroacetate), 10 mL of dichloromethane and *N,N*-diisopropylethylamine (241 mg, 1.87 mmol) were added to a 50 mL round bottom flask. The reaction was cooled to 0°C, and a solution of acryloyl chloride in dichloromethane (50.4 mg, 0.624 mmol) was added dropwise to the reaction solution. The reaction was stirred at 0°C for 10 minutes. The mixture was washed with 20 mL of water, and the organic phase was dried and then concentrated to obtain a crude product of (6a*R*)-8-acryloyl-1-(3-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4--*f*][1,4]oxazepin-12-one (Z327). ES-API: [M+H]⁺=529.2

### Embodiment 68-3 Synthesis of Z329

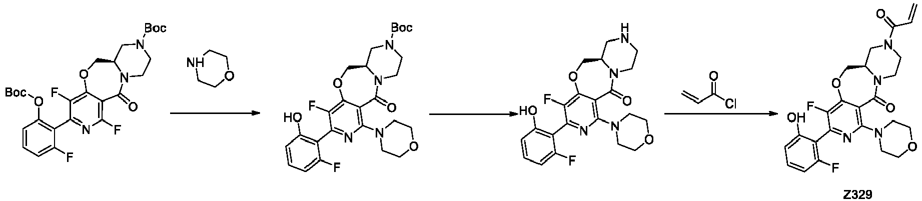

Step 1: (6a*R*)-8-*tert*-Butoxycarbonyl-3-(2-((*tert*-butoxycarbonyl)oxy)-6-fluorophenyl)-4-fluoro-1-fluoro-6,6a,7,8,9,10-hexahydro-12*H*-pyrazino[2,1-*c*]pyrido[3,4-*f*][1,4]oxazepin-12-one (50 mg, 0.086 mmol), morpholine (12 mg, 0.13 mmol), *N,N*-diisopropylethylamine (35 mg, 0.27 mmol) and acetonitrile (10 mL) were added to a 100 mL round bottom flask, stirred at 80°C for 0.5 hours. The mixture was extracted with ethyl acetate, washed with saturated brine, and the organic phase was dried, concentrated, and purified by silica gel column (petroleum ether:ethyl acetate = 20:80) to obtain (6a*R*)-8-*tert*-butoxycarbonyl-1-(morpholino)-4-fluoro-3-(2-fluoro-6-hydroxyphenyl)-6,6a,7,8,9,10-hexahydro-12*H*-pyrazino [2,1-e]pyrido[3,4-*f*][1,4]oxazepin-12-one (33 mg, yield of 80.4%). ES-API: [M+H]⁺=533.2.

Step 2: (6a*R*)-8-*tert*-Butoxycarbonyl-1-(morpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (33 mg, 0.062 mmol), 2 mL of trifluoroacetic acid and 5 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(morpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (28 mg, crude product) as a yellow solid. ES-API: [M+H]⁺=433.2.

Step 3: (6a*R*)-1-(Morpholino)-4-fluoro-3-(2-fluoro-6-hydroxyphenyl)-6,6a,7,8,9,10-hexahydro-12*H*-pyrazinol[2,1-*c*]pyrido[3,4-*f*][1,4]oxazepin-12-one (28 mg, 0.0526), 10 mL of dichloromethane and *N,N*-diisopropylethylamine (20 mg, 0.158 mmol) were added to a 50 mL round bottom flask. The reaction was cooled to 0°C, and a solution of acryloyl chloride in dichloromethane (4.3 mg, 0.047 mmol) was added dropwise to the reaction solution. The reaction was stirred at 0°C for 10 minutes. The mixture was washed with 20 mL of water, and the organic phase was dried and concentrated, and the crude product was purified by preparative HPLC (column: C18 19*150 mm 5 µm, mobile phase: ACN:H₂O=50:50 (0.1% NH₄HCO₃), flow rate: 15 mL/min, column temperature: 25°C) to obtain a target product of (6a*R*)-8-acryloyl-1-(morpholino)-4-fluoro-3-(2-fluoro-6-hydroxyphenyl)-6,6a,7,8,9,10-hexahydro-12*H*-pyrazino [2,1-*c*]pyrido[3,4-*f*][1,4]oxazepin-12-one (12 mg, 3-step yield of 18%) as a white solid. ES-API: [M+H]⁺= 487.1. ¹H NMR (500 MHz, DMSO-*d*₆) δ 10.08 (s, 1H), 7.28 (td, *J* = 8.3, 6.8 Hz, 1H), 7.04-6.60 (m, 3H), 6.17 (d, *J* = 16.7 Hz, 1H), 5.75 (d, *J* = 10.3 Hz, 1H), 4.49 (s, 1H), 4.34 (q, *J* = 13.4, 11.4 Hz, 1H), 4.25-4.12 (m, 2H), 4.03 (d, *J* = 13.9 Hz, 2H), 3.96 - 3.72 (m, 1H), 3.65-3.56 (m, 5H), 3.45 (d, *J* = 13.5 Hz, 1H), 3.41-3.34 (m, 2H), 3.20-3.15 (m, 2H)

### Embodiment 68-4 Synthesis of Z335

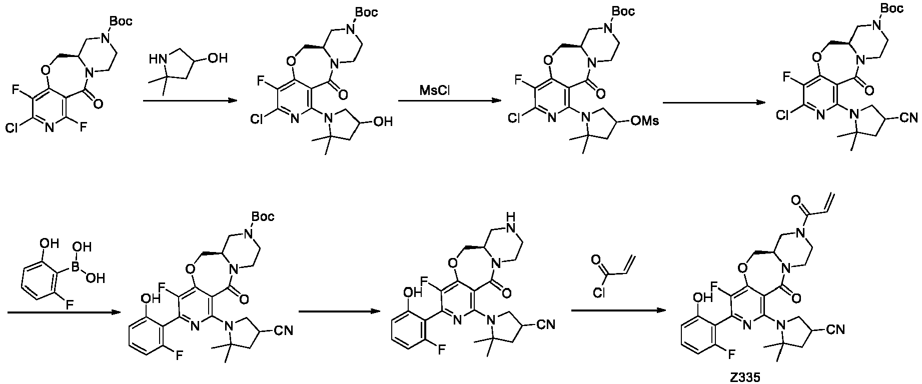

Step 1: (6a*R*)-8-*tert*-Butoxycarbonyl-1,4-difluoro-3-chloro-6,6a,7,8,9,10-hexahydro-12*H-*pyrazino[2,1-*c*]pyrido[3,4-*f*][1,4]oxazepin-12-one (160 mg, 0.411 mmol), 4 -hydroxy-2,2-dimethylpyrrolidine (71 mg, 0.617 mmol), *N,N*-diisopropylethylamine (159 mg, 1.23 mmol) and acetonitrile (10 mL) were added to a 100 mL round bottom flask. The reaction was stirred at 80°C for 1 hour. The mixture was extracted with ethyl acetate, washed with aqueous ammonium chloride solution and saturated brine in turn, and the organic phase was dried, concentrated, and purified by silica gel column (petroleum ether/ethyl acetate = 40:60) to obtain (6a*R*)-8-*tert*-butoxycarbonyl-1-(4-hydroxy-2,2-dimethylpyrrolidin)-4-fluoro-3-chloro-6,6a,7,8,9,10-hexahydro-12*H*-pyrazino[2,1-*c*]pyrido[3,4-*f*][1,4]oxazepin-12-one (150 mg, yield of 76%). ES-API: [M+H]⁺=485.2.

Step 2: (6a*R*)-8-*tert*-Butoxycarbonyl-1-(4-hydroxy-2,2-dimethylpyrrolidin)-4-fluoro-3-chloro-6,6a,7,8,9,10-hexahydro-12*H*-pyrazino[2,1-*c*]pyrido[3,4-*f*][1,4]oxazepin-12-one (150 mg, 0.31 mmol), *N,N*-diisopropylethylamine (120 mg, 0.93 mmol) and 8 mL of dichloromethane were added to a 50 mL round bottom flask. Under ice-water bath conditions, methanesulfonyl chloride (53 mg, 0.465 mmol) was added thereto, heated to room temperature and reacted for 2 hours. The mixture was poured into ice-water, extracted with dichloromethane, washed with saturated brine, and the organic phase was dried, concentrated, and purified by silica gel column (petroleum ether/ethyl acetate = 50:50) to obtain (*6aR*)*-*8*-tert-*butoxycarbonyl-1-(2,2-dimethyl-4-((methylsulfonyl)oxy)pyrrolidinyl)-4-fluoro-3-chloro-6,6a,7,8,9,10-hexahydro-12*H*-pyrazino[2,1-*c*]pyrido[3,4-*f*][1,4]oxazepin-12-one (200 mg, yield of 87%). ES-API: [M+H]⁺=563.1.

Step 2: (6a*R*)-8-*tert*-Butoxycarbonyl-1-(2,2-dimethyl-4-((methylsulfonyl)oxy)pyrrolidinyl)-4-fluoro-3-chloro-6,6a,7,8,9,10-hexahydro-12*H*-pyrazino[2,1-*c*]pyrido[3,4-*f*][1,4]oxazepin-12-one (180 mg, 0.32 mmol), tetrabutylammonium cyanide (258 mg, 0.96 mmol) and acetonitrile (1.5 mL) were added to a 5 mL microwave reaction tube. The mixture was reacted at 100°C for 30 minutes under microwave irradiation. The mixture was added with ethyl acetate, washed with water and saturated brine in turn, and dried over anhydrous sodium sulfate. The mixture was concentrated, and then purified by silica gel (petroleum ether/ethyl acetate = 60:40) to obtain (6a*R*)-8-*tert*-butoxycarbonyl-1-(3-cyano-5,5-dimethylpyrrolidinyl)-4-fluoro-3 -chloro-6,6a,7,8,9,10-hexahydro-12*H*-pyrazino [2,1-*c*]pyrido[3,4--*f*][1,4]oxazepin-12-one (9 mg, yield of 6%). ES-API: [M+H]⁺=494.1.

Step 3: (6a*R*)-8-*tert*-Butoxycarbonyl-1-(3-cyano-5,5-dimethylpyrrolidin)-4-fluoro-3-chloro-6,6a,7,8,9,10-hexahydro-12*H*-pyrazino[2,1-*c*]pyrido[3,4-*f*][1,4]oxazepin-12-one (9 mg, 0.018 mmol), (2-fluoro-6-hydroxyphenyl)boronic acid (8.5 mmol, 0.054 mmol), Sphos-Pd-G2 (2.6 mg, 0.0036 mmol) and potassium carbonate (7.4 mg, 0.054 mmol) were added to a mixed solution of 1,4-dioxane (5 mL) and water (1 mL), and the system was replaced with nitrogen for 3 times, and the reaction was carried out at 90°C for 1 hour under microwave irradiation. The mixture was added with ethyl acetate, washed with water and saturated brine in turn, and dried over anhydrous sodium sulfate. The mixture was concentrated, and then purified by silica gel (petroleum ether/ethyl acetate = 10:90) to obtain (6a*R*)-8-*tert*-butoxycarbonyl-1-(4-cyano-2,2-dimethylpyrrolidin-1-yl))-4-fluoro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (5 mg, yield of 50%). ES-API: [M+H]⁺=570.0.

Step 4: (6a*R*)-8-*tert*-Butoxycarbonyl-1-(4-cyano-2,2-dimethylpyrrolidin-1-yl))-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one (5 mg, 0.0087 mmol), 1 mL of trifluoroacetic acid and 2 mL of dichloromethane were added to a 50 mL round bottom flask. The mixture was stirred at room temperature for 1 hour, and the completion of reaction was detected by LC-MS. The reaction solution was concentrated to obtain a crude product of (6a*R*)-1-(4-cyano-2,2-dimethylpyrrolidin-1-yl)-4-fluoro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (8.2 mg, trifluoroacetate). ES-API: [M+H]⁺=470.1.

Step 5: (6a*R*)-1-(4-Cyano-2,2-dimethylpyrrolidin-1-yl)-4-fluoro-3-chloro-6,6a,7,8,9,10-hexahydro-12*H*-pyrazino[2,1-*c*]pyrido[3,4-*f*][1,4]oxazepin-12-one (8.2 mg, trifluoroacetate), 5 mL of dichloromethane and *N,N*-diisopropylethylamine (6.8 mg, 0.0528 mmol) were added to a 50 mL round bottom flask. The reaction was cooled to 0°C, and a solution of acryloyl chloride in dichloromethane (1.43 mg, 0.0158 mmol) was added dropwise to the reaction solution. The reaction was stirred at 0°C for 10 minutes. The mixture was washed with 5 mL of water, and the organic phase was dried, then concentrated and purified by preparative HPLC (alkali method) to obtain (6a*R*)-8-acryloyl-1-(4-cyano-2,2-dimethylpyrrolidin-1-yl)-4-fluoro-3-(2-fluoro-6-hydroxyphenyl)-6,6a,7,8,9,10-hexahydro-12*H-*pyrazino[2,1-*c*]pyrido[3,4-*f*][1,4]oxazepin-12-one (2.2 mg, 2-step yield of 48%). ES-API: [M+H]⁺=524.2

### Embodiment 68-5 Synthesis of Z359-1

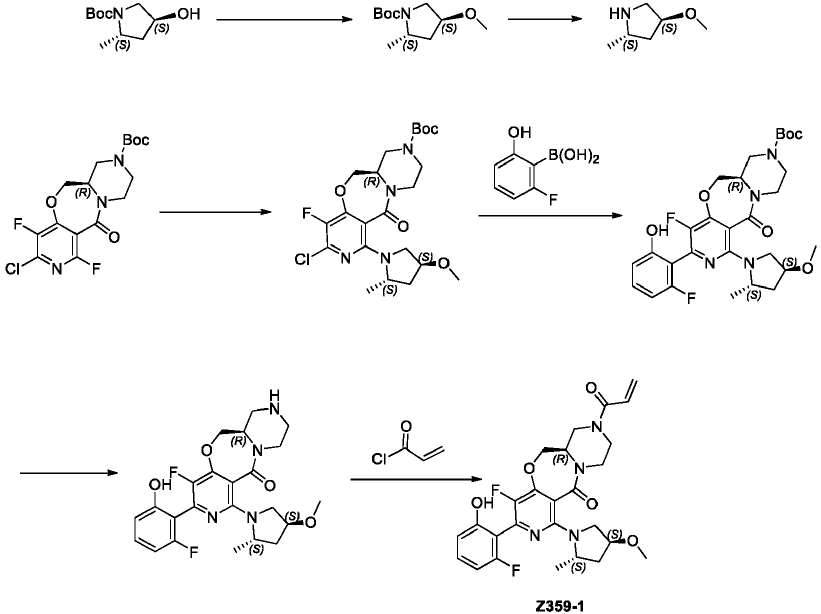

Step 1: *tert*-Butyl (2*S*,4*S*)-4-hydroxy-2-methylpyrrolidine-1-carboxylate (200 mg, 0.995mmol) and iodomethane (424 mg, 2.985 mmol) were dissolved in *N,N*-diformamide (2 mL), then sodium hydride (119 mg, 2.985 mmol) was added thereto at 0°C, reacted at room temperature for 1 hour. The mixture was quenched with water, extracted with ethyl acetate, washed with brine, dried over sodium sulfate and evaporated to dryness by rotary evaporation to obtain a crude product of *tert*-butyl (2*S*,4*S*)-4-methoxy-2-methylpyrrolidine-1-carboxylate (300 mg, crude product). ES-API: [M-55]⁺= 160.1

Step 2: *tert*-Butyl (2*S*,4*S*)-4-methoxy-2-methylpyrrolidine-1-carboxylate (300 mg, crude product) was dissolved in dichloromethane (3 mL), and then trifluoroacetic acid (3 mL) was added dropwise thereto, stirred at room temperature for 0.5 hours. After the reaction was completed, the mixture was evaporated to dryness by rotary evaporation to obtain a crude product of (2*S*,4*S*)-4-methoxy-2-methylpyrrolidine (250 mg, crude product). ES-API: [M+H]⁺= 116.1

Step 3: Compound *tert*-butyl (*R*)-3-chloro-1,4-difluoro-12-oxo-6a,7,9,10-tetrahydro-6*H-*pyrazino[2,1-*c*]pyrido[3,4-*f*][1,4]oxazepin-12-one (260 mg, 0.668 mmol), (2*S*,4*S*)-4-methoxy-2-methylpyrrolidine (250 mg, crude product), and potassium carbonate (277 mg, 2 mmol) were dissolved in acetonitrile (3 mL), reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of *tert*-butyl (*R*)-3-chloro-4-fluoro-1-((2*S*,4*S*)-4-methoxy-2-methylpyrrolidin-1-yl)-12-oxo-6a,7,9,10-tetrahydro-6*H*-pyrazino[2,1-*c*]pyrido[3,4-*f*][1,4]oxazepin-12-one (220 mg, 68.1%). ES-API: [M+H]⁺= 485.2.

Step 4: *tert*-Butyl (*R*)-3-chloro-4-fluoro-1-((2*S*,4*S*)-4-methoxy-2-methylpyrrolidin-1-yl)-12-oxo-6a,7,9,10-tetrahydro-6*H*-pyrazino[2,1-*c*]pyrido[3,4-*f*][1,4]oxazepin-12-one (220 mg, 0.455 mmol), (2-fluoro-6-hydroxyphenyl)boronic acid (142 mg, 0.91 mmol), potassium carbonate (251 mg, 1.82 mmol), and tetrakis(triphenylphosphine)palladium (53 mg, 0.0455 mmol) were dissolved in 1,4-dioxane/water (4/1 mL), heated to 105°C under nitrogen protection and reacted for 16 hours. The mixture was cooled, added with water and ethyl acetate to separate the layers, and the organic phase was washed with brine, dried, concentrated, and purified by column chromatography (petroleum ether/ethyl acetate=2/1) to obtain a product of *tert*-butyl (6a*R*)-3-(2-((*tert*-butoxycarbonyl)oxy)-6-fluorophenyl)-4-fluoro-1-((2*S*,4*S*)-4-methoxy-2-methylpyrrolidin-1-yl)-12-oxo-6a,7,9,10-tetrahydro-6*H*-pyrazino[2,1-*c*]pyrido [3,4-*f*][1,4]oxazepin-12-one (250 mg, 98.1%). ES-API: [M+H]⁺= 561.2

Step 5: *tert*-Butyl (6a*R*)-3-(2-((*tert*-butoxycarbonyl)oxy)-6-fluorophenyl)-4-fluoro-1-(2*S*,4*S*)-4-methoxy-2-methylpyrrolidin-1-yl)-12-oxo-6a,7,9,10-tetrahydro-6*H*-pyrazino[2,1-*c*]pyrido[3,4--*f*][1,4]oxazepin-12-one (250 mg, 0.446 mmol) was dissolved in dichloromethane (2 mL), and then trifluoroacetic acid (2 mL) was added dropwise thereto, stirred at room temperature for 0.5 hours. Then the mixture was evaporated to dryness by rotary evaporation to obtain a crude product of (6a*R*)-4-fluoro-3-(2-fluoro-6-hydroxyphenyl)-1-((2*S*,4*S*)-4-methoxy-2-methylpyrrolidin-1-yl)-7,8,9,10-tetrahydro-6*H-*pyrazino[2,1-*c*]pyrido[3,4-*f*][1,4]oxazepin-12-one (200 mg, crude product). ES-API: [M+H]⁺= 461.2.

Step 6: (6a*R*)-4-Fluoro-3-(2-fluoro-6-hydroxyphenyl)-1-((2*S*,4*S*)-4-methoxy-2-methylpyrrolidin-1-yl)-7,8,9,10-tetrahydro-6*H*-pyrazino[2.1-*c*]pyrido[3,4-*f*][1,4]oxazepin-12-one (200 mg, crude product) was dissolved in tetrahydrofuran (5 mL), then cooled to 0°C in an ice-water bath. Saturated sodium bicarbonate solution was added thereto to adjust the pH value to 8-9. Potassium phosphate (47 mg, 0.223 mmol) was added thereto, and then acryloyl chloride (32 mg, 0.357 mmol) was added dropwise thereto and stirred for 10 minutes. After the reaction was completed, the reaction solution was added with saturated hydrogen carbonate solution, stirred for 10 minutes, extracted with ethyl acetate, and the organic phase was washed with brine, dried over sodium sulfate, evaporated to dryness by rotary evaporation, and the crude product was purified by column chromatography (petroleum ether/ethyl acetate = 1/1, R_{f}= 0.4) to obtain (6a*R*)-8-acryloyl-4-fluoro-3-(2-fluoro-6-hydroxyphenyl)-1-((2*S*,4*S*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one (67 mg, 29.3%). ES-API: [M+H]⁺= 515.2. ¹H NMR (400 MHz, CDCl₃) δ7.24 (d, *J* = 7.9 Hz, 1H), 6.77 (d, *J* = 8.4 Hz, 1H), 6.65 (t, *J* = 9.4 Hz, 1H), 6.54 (d, *J* = 14.4 Hz, 1H), 6.35 (d, *J* = 16.5 Hz, 1H), 5.78 (d, *J* = 10.2 Hz, 1H), 4.46 - 4.19 (m, 4H), 4.14 - 4.04 (m, 2H), 3.93 - 3.54 (m, 5H), 3.41 (s, 2H), 3.30 (d, *J* = 2.2 Hz, 3H), 2.17 (dt, *J* = 13.2, 6.5 Hz, 1H), 1.88 (d, *J* = 7.2 Hz, 1H), 1.30 (d, *J=* 6.2 Hz, 3H).

### Embodiment 68-6 Synthesis of Z474

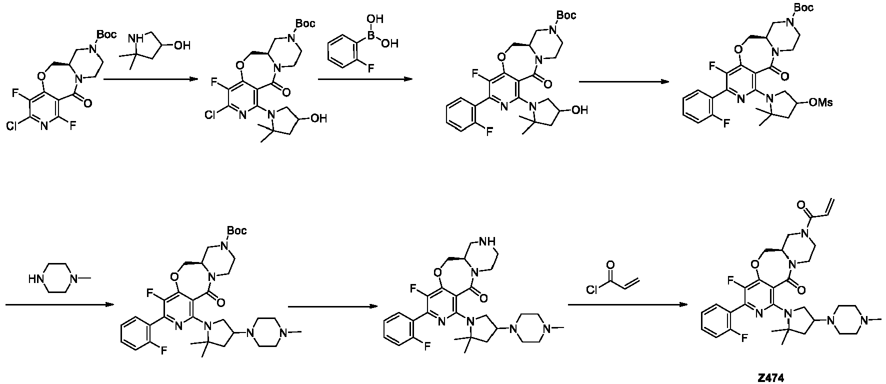

Step 1: Compound (*R*)-*tert*-butyl 3-chloro-1,4-difluoro-12-oxo-6a,7,9,10-tetrahydro-6*H-*pyrazino[2,1-*c*]pyrido[3,4-*f*][1,4]oxazepine-8(12*H*)-carboxylate (200 mg, 0.51 mmol), 5,5-dimethylpyrrolidin-3-ol (196 mg, 1.53 mmol) and potassium carbonate (1942 mg, 14.073 mmol) were dissolved in acetonitrile (2 mL) and reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation and purified by flash chromatography (ethyl acetate:petroleum ether: 0-80%) to obtain compound (6a*R*)-*tert*-butyl 3-chloro-4-fluoro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (165 mg, yield: 66%). ES-API: [M+H]⁺=485.2

Step 2: Compound (6a*R*)-*tert*-butyl 3-chloro-4-fluoro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (165 mg, 0.340 mmol), (2-fluorophenyl)boronic acid (57 mg, 0.408 mmol), tetrakis(triphenylphosphine)palladium (39 mg, 0.034 mmol) and potassium carbonate (141 mg, 1.02 mmol) were dissolved in a mixed solution of dioxane and water (2 mL:0.4 mL), and the reaction solution was stirred at 110°C overnight. The reaction was monitored to be complete, and the mixture was extracted with ethyl acetate, washed once with brine, dried over anhydrous sodium sulfate, and the filtrate was evaporated to dryness by rotary evaporation and purified by flash chromatography (ethyl acetate:petroleum ether: 0-85%) to obtain compound (*6aR*)*-tert-*butyl 4-fluoro-3-(2-fluorophenyl)-1-(4-hydroxy-2,2-dimethylpyrrolidin-1-yl)-12-oxo-6a,7,9,10-tetrahydro-6*H*-pyrazino[2,1*-c*]pyrido[3,4-*f*][1,4]oxazepine-8(12*H*)-carboxylate (198 mg, crude product, yield: 95%). ES-API: [M+H]⁺= 545.3

Step 3: Compound (6a*R*)-*tert*-butyl 4-fluoro-3-(2-fluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (198 mg, 0.364 mmol) was dissolved in 3 mL of dichloromethane, then methanesulfonyl chloride (83 mg, 0.728 mmol) and triethylamine (110 mg, 1.092 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation and purified by thin-layer chromatography plate to obtain compound (6aR)-*tert*-butyl 1-(2,2-dimethyl-4-((methylsulfonyl)oxy)pyrrolidin-1 -yl)-4-fluoro-3 -(2-fluorophenyl)-12-oxo-6a,7,9,10-tetrahydro-6*H-*pyrazino[2,1-*c*]pyrido[3,4-*f*][1,4]oxazepine-8(12*H*)-carboxylate (150 mg, yield: 66%). ES-API: [M+H]⁺= 623.3

Step 4: (6a*R*)-*tert*-Butyl 1-(2,2-dimethyl-4-((methylsulfonyl)oxy)pyrrolidin-1-yl)-4-fluoro-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (150 mg, 0.241 mmol) was dissolved in 1 mL of 1-methylpiperazine, and the reaction solution was heated to 100°C, then stirred for 16 hours. The reaction was monitored to be complete, and then the reaction solution was cooled to room temperature, extracted by adding ethyl acetate, washed once with brine, dried over anhydrous sodium sulfate, and the filtrate was evaporated to dryness by rotary evaporation and purified (dichloromethane/methanol=20/1) to obtain compound (6a*R*)-*tert*-butyl 1-(2,2-dimethyl-4-(4-methylpiperazin-1-yl)pyrrolidin-1-yl)-4-fluoro-3-(2-fluorophenyl)-12-oxo-6a,7,9,10-tetrahydro-6*H-*pyrazino[2,1-*c*]pyrido[3,4-*f*][1,4]oxazepine-8(12*H*)-carboxylate (50 mg, yield: 33%). ES-API: [M+H]⁺=627.4

Step 5: (6a*R*)-*tert*-Butyl 1-(2,2-dimethyl-4-(4-methylpiperazin-1-yl)pyrrolidin-1-yl)-4-fluoro-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (50 mg, 0.08 mmol) was dissolved in dichloromethane (2 mL), then cooled to 0°C in an ice-water bath, and then trifluoroacetic acid (1 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (6a*R*)-1-(2,2-dimethyl-4-(4-methylpiperazin-1-yl)pyrrolidin-1-yl)-4-fluoro-3-(2-fluorophenyl)-7,8,9,10-tetrahydro-6*H*-pyrazino[2,1-*c*]pyridin[3,4-*f*][1,4][1,4]oxazepin-12(6a*H*)-one (45 mg, yield: 95%).

Step 6: (6a*R*)-1-(2,2-Dimethyl-4-(4-methylpiperazin-1-yl)pyrrolidin-1-yl)-4-fluoro-3-(2-fluorophenyl)-7,8,9,10-tetrahydro-6*H*-pyrazino[2,1-*c*]pyridin[3,4-*f*][1,4][1,4]oxazepin-12(6a*H*)-one (42 mg, 0.08 mmol) was dissolved in dichloromethane (2 mL), then cooled to 0°C in an ice-water bath, and triethylamine (25 mg, 0.24 mmol) and acryloyl chloride (8 mg, 0.096 mmol) were added dropwise thereto, stirred for 30 minutes. After the reaction was completed, the mixture was added with sodium bicarbonate solution to adjust to alkaline, extracted with dichloromethane, dried over sodium sulfate, and evaporated to dryness by rotary evaporation and purified (dichloromethane/methanol =10/1, R_{f}=0.5) to obtain (6a*R*)-8-acryloyl-1-(2,2-dimethyl-4-(4-methylpiperazin-1-yl)pyrrolidin-1-yl)-4-fluoro-3 -(2-fluorophenyl)-6,6a,7,8,9,10-hexahydro-12*H*-pyrazino[2,1-*c*]pyrido[3,4-*f*][1,4]oxazepin-12-one (20.6 mg, yield: 45%). ¹H NMR (400 MHz, CDCl₃) δ 7.69 (d, *J* = 7.6 Hz, 4H), 7.42 (dd, *J* = 12.0, 7.3 Hz, 6H), 4.15 *(d, J* = 16.6 Hz, 1H), 3.95 (d, *J* = 16.6 Hz, 1H), 3.81 (d, *J* = 7.7 Hz, 2H), 3.75 - 3.68 (m, 1H),2.62 (d, *J* = 16.8 Hz, 1H), 2.53 - 2.42 (m, 1H), 2.12 (d, *J* = 10.9 Hz, 1H), 2.00 - 1.90 (m, 1H).1.08 (s, 9H). ES-API: [(2M+*H*)/2]⁺=291.1

### Embodiment 69 Synthesis of (6aR)-8-acryloyl-1-(2,2-dimethylmorpholino)-4-fluoro-3-(5-methyl-1H-indazol-4-yl)-6,6a,7,8,9,10-hexahydro-12H-pyrazino[2,1-c]pyrido[3,4-f][1,4]oxazepin-12-one Z238

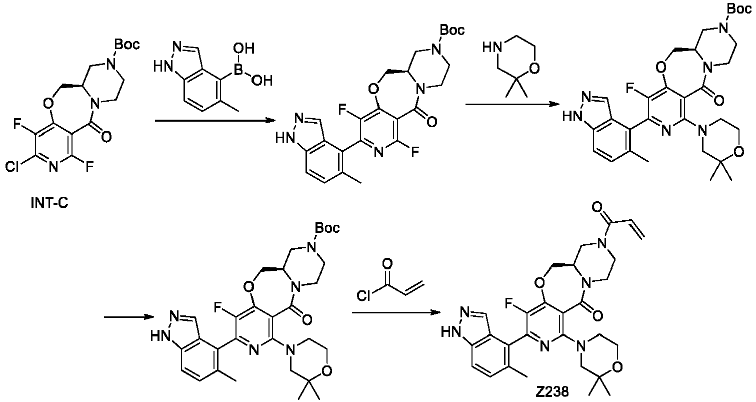

Step 1: *tert*-Butyl (*R*)-3-chloro-1,4-difluoro-12-oxo-6a,7,9,10-tetrahydro-12*H*-pyrazino[2,1-*c*]pyrido[3,4-*f*][1,4]-8-(6*H*)-carboxylate (150 g, 0.39 mmol), (5-methyl-1*H*-indazol-4-yl)boronic acid (206 mg, 1.17 mmol), tetrakis(triphenylphosphine)palladium (45 mg, 0.039 mmol), potassium carbonate (161 mg, 1.17 mmol), 15 mL of dioxane and 3 mL of water were added to a 100 mL reaction flask. The reaction was carried out at 80°C for 2.5 hours under nitrogen protection, then the reaction solution was added with 20 mL of water, extracted three times with 20 mL of ethyl acetate. The organic phase was dried and concentrated, and the crude product was purified by flash silica gel column (EtOAc/PE: 0-100%) to obtain *tert*-butyl (6a*R*)-1,4-difluoro-3-(5-methyl-1*H*-indazol-4-yl)-12-oxo-6a,7,9,10-tetrahydro-12*H*-pyrazino [2,1-*c*]pyrido [3,4-*f*][1,4]oxazepine-8(6*H*)-carboxylate (171 mg, 90%) as a yellow solid. ES-API: [M+H]⁺= 486.1.

Step 2: *tert*-Butyl (6a*R*)-1,4-difluoro-3-(5-methyl-1*H*-indazol-4-yl)-12-oxo-6a,7,9,10-tetrahydro-12*H*-pyrazino[2,1-*c*]pyrido[3,4-*f*][1,4]oxazepine-8(6*H*)-carboxylate (171 mg, 0.35 mmol), 2,2-dimethylmorpholine (80 mg, 0.7 mmol), diisopropylethylamine (130 mg, 1.05 mmol) and acetonitrile (5 mL) were added to a flask. The reaction was stirred at 90°C for 2 hours. The reaction solution was added with 20 mL of water and extracted with ethyl acetate. The organic phase was dried and concentrated, and the crude product was purified by flash silica gel column (EtOAc/PE: 0-50%) to obtain *tert*-butyl (6a*R*)-1-(2,2-dimethylmorpholino)-4-fluoro-3-(5-methyl-1*H*-indazol-4-yl)-12-oxo-6a,7,9,10-tetrahydro-12*H-*pyrazino[2,1-*c*]pyrido[3,4-*f*][1,4]oxazepine-8(6*H*)-carboxylate (191 mg, 94%) as a yellow solid. ES-API: [M+H]⁺=581.2.

Step 3: *tert*-Butyl (6a*R*)-1-(2,2-dimethylmorpholino)-4-fluoro-3-(5-methyl-1*H*-indazol-4-yl)-12-oxo-6a,7,9,10-tetrahydro-12*H*-pyrazino[2,1-*c*]pyrido [3,4-*f*][1,4]oxazepine-8(6*H*)-carboxylate (191 mg, 0.32 mmol), 3 mL of dichloromethane and 2 mL of trifluoroacetic acid were added to a flask. The mixture was stirred at room temperature for 1 hour, and the completion of reaction was detected by LC-MS. The reaction solution was concentrated to obtain *tert*-butyl (6a*R*)-1-(2,2-dimethylmorpholino)-4-fluoro-3-(5-methyl-1*H*-indazol-4-yl)-12-oxo-6a,7,9,10-tetrahydro-12*H*-pyrazino[2,1-*c*]pyrido[3,4-*f*][1,4]oxazepine-8(6*H*)-carboxylate (212 mg, crude product) as a yellow solid, and the crude product was directly used in the next step. ES-API: [M+H]⁺=481.2.

Step 3: *tert*-Butyl (6a*R*)-1-(2,2-dimethylmorpholino)-4-fluoro-3-(5-methyl-1*H*-indazol-4-yl)-12-oxo-6a,7,9,10-tetrahydro-12H-pyrazino[2,1-*c*]pyrido[3,4-*f*][1,4]oxazepine-8(6*H*)-carboxylate (212 mg, 0.44 mmol), 10 mL of dichloromethane and triethylamine (445 mg, 4.4 mmol) were added to a flask. At 0°C, a solution of acryloyl chloride in dichloromethane (36 mg, 0.4 mmol, 1 mL) was added dropwise thereto. The reaction was carried out at 0°C for 10 minutes. 40 mL of saturated aqueous sodium bicarbonate solution was added to the reaction solution, and the mixture was extracted three times with 20 mL of dichloromethane. The organic phase was dried and concentrated, and the crude product was purified by preparative HPLC to obtain (6a*R*)-8-acryloyl-1-(2,2-dimethylmorpholino)-4-fluoro-3-(5-methyl-1*H-*indazol-4-yl)-6,6a,7,8,9,10-hexahydro-12*H*-pyrazino[2,1-*c*]pyrido[3,4-*f*][1,4]oxazepin-12-one (Z238, 60 mg, 22%). ¹HNMR (500 MHz, DMSO-*d₆*) δ 13.09 (s, 1H), 7.84 (s, 1H), 7.54 (d, *J* = 8.5 Hz, 1H), 7.31 (s, 1H), 6.77 (d, *J* = 10.0 Hz, 1H), 6.18 (d, *J* = 15.9 Hz, 1H), 5.76 (d, *J* = 10.7 Hz, 1H), 4.57-4.37 (m, 2H), 4.32-4.21 (m, 1H), 4.18-3.98 (m, 2H), 3.89 (s, 1H), 3.77-3.70 (m, 1H), 3.69-3.50 (m, 3H), 3.45-3.31 (m, 2H), 3.23 (d, *J* = 12.8 Hz, 1H), 3.12-3.06 (m, 2H), 2.38-2.36 (m, 1H), 2.23 (s, 2H), 1.14 (d, *J* = 14.0 Hz, 6H). ES-API: [M+H]⁺=535.2.

### Embodiment 69-1 Synthesis of Z449 and isomers thereof

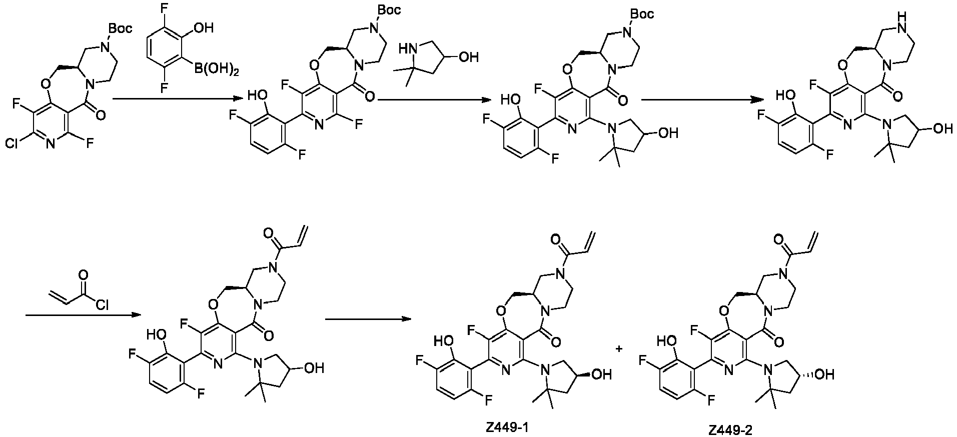

Step 1: *tert*-Butyl (*R*)-3-chloro-1,4-difluoro-12-oxo-6a,7,9,10-tetrahydro-12*H*-pyrazino[2,1-*c*]pyrido[3,4-*f*][1,4]oxazepine-8(6*H*)-carboxylate (200 mg, 0.51 mmol), (3,6-difluoro-2-hydroxyphenyl)boronic acid (400 mg, 2.28 mmol), Sphos-Pd-G2 (36 mg, 0.05 mmol), potassium carbonate (207 mg, 1.5 mmol), 15 mL of dioxane and 2 mL of water were added to a 50 mL flask. Under nitrogen protection, the reaction was carried out at 110°C for 1 hour under microwave irradiation. The reaction solution was added with 50 mL of ethyl acetate, washed with 30 mL of saturated brine for 3 times, dried and concentrated, and the crude product was purified by flash silica gel column (ethyl acetate:petroleum ether: 0-90%) to obtain *tert*-butyl (6a*R*)-3-(3,6-difluoro-2-hydroxyphenyl)-1,4-difluoro-12-oxo-6a,7,9,10-tetrahydro-12*H*-pyrazino[2,1-*c*]pyrido[3,4-*f*][1,4]oxazepine-8(6*H*)-carboxylate (120 mg, yield of 48%). ES-API: [M+H]⁺=484.1.

Step 2: *tert*-Butyl (6a*R*)-3-(3,6-difluoro-2-hydroxyphenyl)-1,4-difluoro-12-oxo-6a,7,9,10-tetrahydro-12*H*-pyrazino[2,1-*c*]pyrido[3,4-*f*][1,4]oxazepine-8(6*H*)-carboxylate (120 mg, 0.25 mmol) was dissolved in 10 mL of acetonitrile, and DIPEA (65 mg, 0.48 mmol) and 5,5-dimethylpyrrolidin-3-ol (86 mg, 0.75 mmol) were added thereto. The reaction was carried out at 95°C for 16 hours. The reaction solution was added with 30 mL of ethyl acetate, washed with 30 mL of saturated brine for 3 times, dried and concentrated, and the crude product was purified by flash silica gel column (methanol:dichloromethane: 0-10%) to obtain *tert*-butyl (6a*R*)-3-(3,6-difluoro-2-hydroxyphenyl)-4-fluoro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (90 mg, yield of 62%). ES-API: [M+H]⁺=579.3.

Step 3: *tert*-Butyl (6a*R*)-3-(3,6-difluoro-2-hydroxyphenyl)-4-fluoro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (90 mg, 0.16 mmol) was dissolved in dichloromethane (5 mL), and trifluoroacetic acid (2 mL) was added thereto. The mixture was stirred at room temperature for 1 hour, and the reaction solution was concentrated to obtain a crude product of (6a*R*)-3-(3,6-difluoro-2-hydroxyphenyl)-4-fluoro-1-(4-hydroxy-2,2-dimethylpyrrolidin-1-yl)-6,6a,7,8,9,10-hexahydro-12*H*-pyrazino[2,1-*c*]pyrido [3,4-*f*][1,4]oxazepin-12-one (75 mg, yield of 48%). ES-API: [M+H]⁺=479.2.

Step 4: (6a*R*)-3-(3,6-Difluoro-2-hydroxyphenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (75 mg, 0.2 mmol) was dissolved in dichloromethane (5 mL), and triethylamine (61 mg, 0.6 mmol) was added thereto. Acryloyl chloride (16 mg, 0.18 mmol) was added dropwise thereto at 0°C. The reaction was stirred at 0°C for 15 minutes. The reaction solution was added with 40 mL of dichloromethane, washed with 50 mL of saturated aqueous NaHCO₃ solution and 40 mL of saturated brine, dried and concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-3-(3,6-difluoro-2-hydroxyphenyl)-4-fluoro-1-(4-hydroxy-2,2-dimethylpyrrolidin-1-yl)-6,6a,7, 8,9,10-hexahydro-12*H-*pyrazino[2,1-*c*]pyrido[3,4-*f*][1,4]oxazepin-12-one (40 mg, yield of 37%). ES-API: [M+H]⁺=533.2.

Step 5: Compound (6a*R*)-8-acryloyl-3-(3,6-difluoro-2-hydroxyphenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-f][1,4]oxazepin-12-one (40 mg, 0.08 mmol) was subjected to chiral separation by SFC (separation column: IE 250 mm^{∗}4.6 mm^{∗}5 µm; mobile phase: *n*-hexane:ethanol=70:30; flow rate: 1 mL/min; column temperature: 30°C). Two isomers were obtained:

One isomer with a structure arbitrarily designated as (6a*R*)-8-acryloyl-3-(3,6-difluoro-2-hydroxyphenyl)-4-fluoro-1-((*S*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z449-1, 11 mg, yield: 28%, retention time: 9.355 min, purity: 99%, ee value: 100%). ES-API: [M+H]⁺=533.1. ¹HNMR (400 MHz, DMSO-*d*₆) δ 10.28 (s, 1H), 7.40-7.20 (m, 1H), 6.90-6.71 (m, 2H), 6.18 (d, *J* = 16.4 Hz, 1H), 5.76 (d, *J* = 9.9 Hz, 1H), 4.85 (s, 1H), 4.56 (s, 1H), 4.51-3.47 (m, 9H), 3.30-2.96 (m, 2H), 1.99 (s, 1H), 1.80-1.61 (m, 1H), 1.48 (d, *J* = 12.7 Hz, 6H).

Another isomer with a structure arbitrarily designated as (6a*R*)-8-acryloyl-3-(3,6-difluoro-2-hydroxyphenyl)-4-fluoro-1-((*R*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z449-2, 18 mg, yield: 45%, retention time: 11.96 min, purity: 99%, ee value: 100%). ES-API: [M+H]⁺=533.1. ¹HNMR (400 MHz, DMSO-*d₆*) δ 10.28 (s, 1H), 7.40-7.20 (m, 1H), 6.90-6.71 (m, 2H), 6.18 (d, *J* = 16.4 Hz, 1H), 5.76 (d, *J* = 9.9 Hz, 1H), 4.85 (s, 1H), 4.56 (s, 1H), 4.41 - 3.39 (m, 9H), 2.96 - 2.71 (m, 2H), 1.98-1.83 (m, 2H), 1.68 (s, 3H), 1.41 (s, 3H).

### Embodiment 69-2 Synthesis of Z451

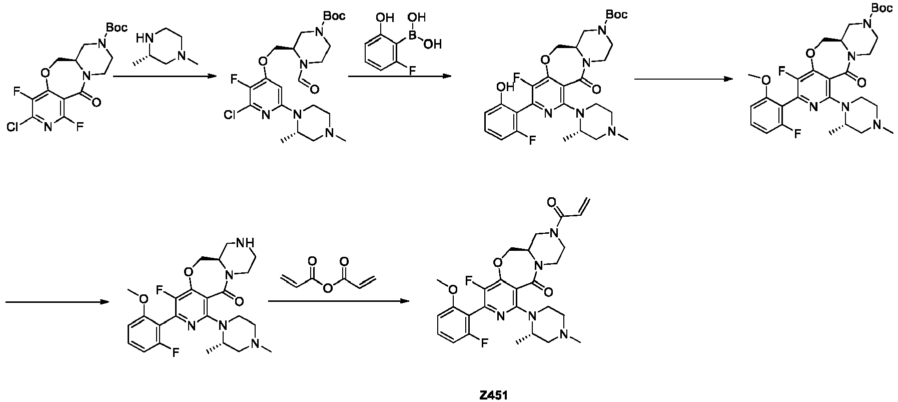

Step 1: *tert*-Butyl (*R*)-3-chloro-1,4-difluoro-12-oxo-6a,7,9,10-tetrahydro-6*H*-pyrazino[2,1-c]pyrido[3,4-*f*][1,4]oxazepine-8(12*H*)-carboxylate (200mg, 0.51 mmol), (*S*)-1,3-dimethylpiperazine (70 mg, 0.614 mmol) and potassium carbonate (227 mg, 1.645 mmol) were dissolved in acetonitrile (3 mL) and reacted at 80°C for 5 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (*R*)-3-chloro-1-((*S*)-2,4-dimethylpiperazin-1-yl)-4-fluoro-12-oxo-6a,7,9,10-tetrahydro-6*H*-pyrazino[2,1-*c*]pyrido[3,4-*f*][1,4]oxazepine-8(12*H*)-carboxylate (320 mg, yield: 100%) as a yellow solid. ES-API: [M+H-100]⁺=484.2.

Step 2: *tert*-Butyl (*R*)-3-chloro-1-(*S*)-2,4-dimethylpiperazin-1-yl)-4-fluoro-12-oxo-6a,7,9,10-tetrahydro-6*H*-pyrazino[2,1-*c*]pyrido[3,4-*f*][1,4]oxazepine-8(12*H*)-carboxylate(320 mg, 0.66 mmol), (2-fluoro-6-hydroxyphenyl)boronic acid (155 mg, 0.99 mmol) and potassium carbonate (182 mg, 1.31 mmol) were dissolved in a mixed solvent (1,4-dioxane:water = 5:1, 6 mL). The reaction solution was bubbled with argon at room temperature for 10 minutes, then added with tetrakis(triphenylphosphine)palladium (76 mg, 0.066 mmol), and the reaction was reacted overnight at 110°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of compound *tert*-butyl (6a*R*)-1-((*S*)-2,4-dimethylpiperazin-1-yl)-4-fluoro-3-(2-fluoro-6-hydroxyphenyl)-12-oxo-6a,7,9,10-tetrahydro-6*H-*pyrazino[2,1-*c*]pyrido[3,4-*f*][1,4]oxazepine-8(12*H*)-carboxylate (230 mg, yield: 67%). ES-API: [M+H]⁺= 560.3.

Step 3: *tert*-Butyl (6a*R*)-1-((*S*)-2,4-dimethylpiperazin-1-yl)-4-fluoro-3-(2-fluoro-6-hydroxyphenyl)-12-oxo-6a,7,9,10-tetrahydro-6*H*-pyrazino[2,1-*c*]pyrido[3,4-*f*][1,4]oxazepine-8(12*H-*carboxylate (150 mg, 0.27 mmol) and potassium hydroxide (18 mg, 0.31 mmol) were dissolved in *N,N-*dimethylformamide (3 mL). The reaction was stirred at room temperature for 20 minutes, then added with iodomethane (47 mg, 0.29 mmol), and the reaction was stirred at room temperature for 2 hours. After the reaction was completed, the reaction system was quenched with water, extracted with ethyl acetate, washed once with brine, dried over anhydrous sodium sulfate, and the filtrate was evaporated to dryness by rotary evaporation to obtain compound (6a*R*)-1-((*S*)-2,4-dimethylpiperazin-1-yl)-4-fluoro-3-(2-fluoro-6-methoxyphenyl)-12-oxo-6a,7,9,10-tetrahydro-6*H*-pyrazino[2,1-*c*]pyrido[3,4-*f*][1,4]oxazepine-8(12*H*)-carboxylate (190 mg, yield: 100%). ES-API: [M+H]⁺= 574.4.

Step 4: (6a*R*)-1-((*S*)-2,4-Dimethylpiperazin-1-yl)-4-fluoro-3-(2-fluoro-6-methoxyphenyl)-12-oxo-6a,7,9,10-tetrahydro-6*H*-pyrazino[2,1-*c*]pyrido[3,4-*f*][1,4]oxazepine-8(12*H*)-carboxylate (190 mg, 0.33 mmol) was dissolved in dichloromethane (2 mL), and then trifluoroacetic acid (2 mL) was added dropwise thereto, stirred at room temperature for 0.5 hours. After the reaction was completed, the mixture was evaporated to dryness by rotary evaporation to obtain a crude product of (6a*R*)-4-chloro-1-(2,4-dimethyl-5-oxopiperazin-1-yl)-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (120 mg, yield: 77%) ES-API: [M+H]⁺:= 474.3.

Step 5: (6a*R*)-4-Chloro-1-(2,4-dimethyl-5-oxopiperazin-1-yl)-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (120 mg, 0.25 mmol) was dissolved in tetrahydrofuran (2 mL), and saturated sodium bicarbonate solution was added dropwise thereto at 0°C to adjust the pH value to 8-9. An aqueous solution (0.5 mL) of potassium phosphate (27 mg, 0.127 mmol) was added thereto. After 5 minutes of reaction, a solution of acryloyl chloride (12 mg, 0.133 mmol) in tetrahydrofuran was added thereto, and the reaction was detected by thin-layer chromatography plate after 5 minutes of reaction. Then a solution of acryloyl chloride (12 mg, 0.133 mmol) in tetrahydrofuran was added thereto until (6a*R*)-4-chloro-1-(2,4-dimethyl-5-oxopiperazin-1-yl)-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one was completely reacted. After the reaction was completed, the mixture was quenched with saturated sodium bicarbonate solution, and the reaction was continued to be stirred at room temperature for 10 minutes, extracted with ethyl acetate, washed once with brine, dried over anhydrous sodium sulfate, and the filtrate was evaporated to dryness by rotary evaporation to obtain a crude product, which was prepared and purified to obtain a product of (6a*R*)-8-acryloyl-1-((*S*)-2,4-dimethylpiperazin-1-yl)-4-fluoro-3-(2-fluoro-6-methoxyphenyl)-6,6a,7,8,9,10-hexahydro-12*H*-pyrazino[2,1-*c*]pyrido[3,4-*f*][1,4]oxazepin-12(6a*H*)-one (15 mg, 12%). (Preparative separation conditions: chromatographic column information: Ultimate XB-C18, 50*250 mm, 10 µm, mobile phase system: A: purified water B: pure acetonitrile, flow rate: 80 mL/min, gradient: within 40 minutes, B/A= 20%-90%, wavelength: 214 nm, column temperature: room temperature). ES-API: [M+H]⁺= 528.2. ¹H NMR (500 MHz, DMSO-*d₆*) δ 7.47 (dd, *J* = 15.4, 8.4 Hz, 1H), 7.02-6.91 (m, 2H), 6.89-6.67 (m, 1H), 6.17 (d, *J=* 16.3 Hz, 1H), 5.85-5.65 (m, 1H), 4.55-4.4 (m, 1H), 4.23 (s, 3H), 4.08-4.02 (m, 4H), 3.91-3.73 (m, 4H), 3.68-3.55 (m, 4H), 3.25-3.15 (m, 1H), 2.59 (d, *J* = 10.0 Hz, 2H), 2.13 (s, 3H), 2.25-1.90 (m, 3H).

### Embodiment 70 Synthesis of Z256, Z256-1 and Z256-2

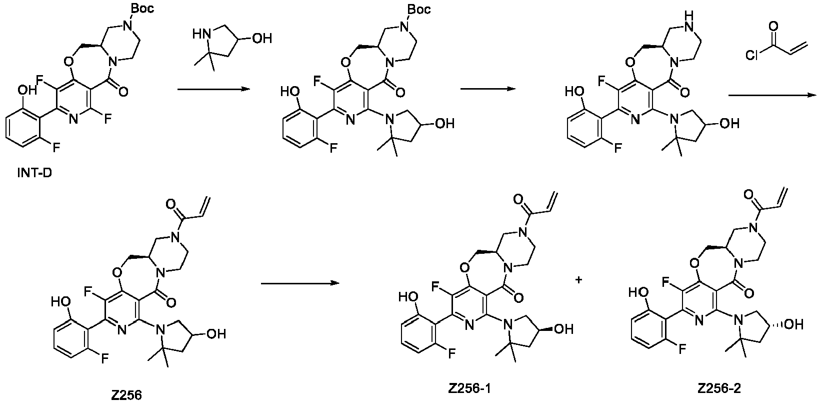

Step 1: *tert*-Butyl (6a*R*)-1,4-difluoro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (250 mg, 0.54 mmol), 5,5-dimethylpyrrolidin-3-ol (186 mg, 1.62 mmol), diisopropylethylamine (210 mg, 1.62 mmol) and acetonitrile (5 mL) were added to a flask. The reaction was stirred at 90°C for 2 hours. The reaction solution was added with 20 mL of water and extracted with ethyl acetate. The organic phase was dried and concentrated, and the crude product was purified by flash column chromatography (EtOAc/PE: 0-50%) to obtain *tert*-butyl (6a*R*)-4-fluoro-3 -(2-fluoro-6-hydroxyphenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (275 mg, 91%) as a yellow solid. ES-API: [M+H]⁺=561.3.

Step 2: *tert*-Butyl (6a*R*)-4-fluoro-3-(2-fluoro-6-hydroxyphenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (275 mg, 0.49 mmol), 3 mL of dichloromethane and 2 mL of trifluoroacetic acid were added to a flask. The mixture was stirred at room temperature for 1 hour, and the completion of reaction was detected by LC-MS. The reaction solution was concentrated to obtain (6a*R*)-4-fluoro-3-(2-fluoro-6-hydroxyphenyl)-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (281 mg, crude product) as a yellow solid, and the crude product was directly used in the next step. ES-API: [M+H]⁺=461.2.

Step 3: (6a*R*)-4-Fluoro-3-(2-fluoro-6-hydroxyphenyl)-1-(4-hydroxy-2,2-dimethylpyrrolidin-1-yl)-6,6a,7,8,9,10-hexahydro-12*H*-pyrazino[2.1-*c*]pyrido[3,4-*f*][1,4]oxazepin-12-one (281 mg, 0.61 mmol), 10 mL of dichloromethane and triethylamine (308 mg, 3.05 mmol) were added to a flask. At 0°C, a solution of acryloyl chloride in dichloromethane (36 mg, 0.4 mmol, 1 mL) was added dropwise thereto. The reaction was carried out at 0°C for 10 minutes. 40 mL of saturated aqueous sodium bicarbonate solution was added to the reaction solution, and the mixture was extracted three times with 20 mL of dichloromethane. The organic phase was dried and then concentrated, and the crude product was purified by basic HPLC (column: Ultimate XB-C18, 50*250 mm, 10 µm; mobile phase system: A: purified water (0.1% NH₄HCO₃); B: pure acetonitrile (0.1% NH₄HCO₃); flow rate : 80 mL/min; gradient: B/A=20%-100%; column temperature: room temperature) to obtain (6a*R*)-8-acryloyl-4-fluoro-3-(2-fluoro-6-hydroxyphenyl)-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (Z256, 86 mg, 27%). ¹H NMR (500 MHz, DMSO-*d*₆) δ 10.07-10.04 (m, 1H), 7.26 (dd, *J* = 15.5, 8.0 Hz, 1H), 6.87-6.68 (m, 3H), 6.17 (d, *J* = 16.5 Hz, 1H), 5.80-5.67 (m, 1H), 5.07-4.77 (m, 1H), 4.58 (bs, 1H), 4.37-3.98 (m, 5H), 3.97-3.69 (m, 1H), 3.66 (dd, *J =* 11.0, 4.0 Hz, 1H), 3.58 -3.11 (m, 3H), 3.10-2.68 (m, 1H), 2.01-1.71 (m, 2H), 1.71-1.38 (m, 6H). ES-API: [M+H]⁺=515.2.

The Z256 (86 mg) obtained in the above step was subjected to chiral separation (mobile phase: *n-*hexane:EtOH=60:40; separation column: IE 250 mm^{∗}4.6 mm^{∗}5 µm; flow rate: 1.0 mL/min; T: 30.0°C) to obtain two isomer compounds (Z256-1 and Z256-2).

One isomer with a structure arbitrarily designated as (6a*R*)-8-acryloyl-4-fluoro-3-(2-fluoro-6-hydroxyphenyl)-1-((*S*)-4-hydroxy-2,2-dimethylpyrrolidin-1-yl)-6,6a,7,8,9,10-hexahydro-12*H-*pyrazino[2,1-c]pyrido[3,4-*f*][1,4]oxazepin-12-one (Z256-1, peak 1, retention time: 7.77 min; 32 mg, purity: 100%, de value: 100%). ¹H NMR (500 MHz, CDCl₃) δ 9.37 (brs, 1H), 7.24-7.20 (m, 1H), 6.75 (d, *J=* 8.3 Hz, 1H), 6.64 (t, *J* = 8.7 Hz, 1H), 6.59-6.45 (m, 1H), 6.35 (d, *J* = 16.0 Hz, 1H), 5.76 (d, *J* = 10.2 Hz, 1H), 4.48-4.24 (m, 4H), 4.24-4.08 (m, 2H), 4.04-3.87 (m, 1H), 3.80-3.33 (m, 4H), 3.23-3.06 (m, 1H), 2.20-2.04 (m, 1H), 1.95 (dd, *J* = 12.3, 8.0 Hz, 1H), 1.51 (s, 6H). ES-API: [M+H]⁺=515.2.

Another isomer with a structure arbitrarily designated as (6a*R*)-8-acryloyl-4-fluoro-3-(2-fluoro-6-hydroxyphenyl)-1-((*R*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z256-2, peak 2, retention time: 10.158 min; 33 mg, purity: 100%, de value: 100%). ¹H NMR (500 MHz, CDCl₃) δ 9.24 (brs, 1H), 7.25-7.20 (m, 1H), 6.76 (d, *J* = 8.1 Hz, 1H), 6.64 (t, *J* = 8.9 Hz, 1H), 6.59-6.46 (m, 1H), 6.35 (d, *J* = 16.2 Hz, 1H), 5.76 (d, *J* = 10.3 Hz, 1H), 4.57-4.26 (m, 4H), 4.22-4.08 (m, 2H), 4.05-3.91 (m, 1H), 3.83 (d, *J* = 8.0 Hz, 1H), 3.71-3.22 (m, 3H), 2.83 (d, *J* = 11.1 Hz, 1H), 2.17-2.06 (m, 1H), 1.95 (d, *J* = 14.6 Hz, 1H), 1.75 (s, 3H), 1.43 (s, 3H). ES-API: [M+H]⁺=515.2.

### Embodiment 71 Synthesis of (R)-8-acryloyl-3-(6-amino-4-methyl-3-(trifluoromethyl)pyridin-2-yl)-4-chloro-1-morpholino-6,6a,7,8,9,10-hexahydro-12H-pyrazino[2,1-c]pyrido[3,4-f][1,4]oxazepin-12-one Z314

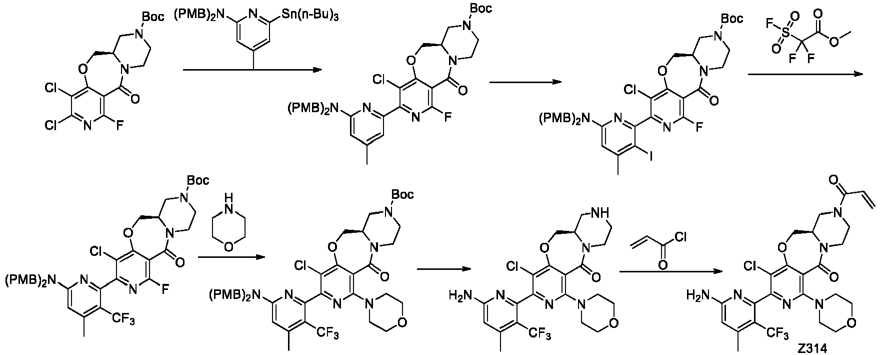

Step 1: Under nitrogen protection, a mixed solution of *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (200 mg, 0.49 mmol), *N,N*-bis(4-methoxybenzyl)-4-methyl-6-(tributylstannyl)pyridin-2-amine (628 mg, 0.98 mmol, prepared by the method disclosed in patent WO2020097537), tetrakis(triphenylphosphine)palladium (114 mg, 0.10 mmol ), cuprous iodide (36 mg, 0.20 mmol) and lithium chloride (104 mg, 2.46 mmol) in 1,4-dioxane (5 mL) was stirred at 110°C for 16 hours, filtered and concentrated, and purified by flash silica gel column (EtOAc/PE: 0-100%) to obtain *tert*-butyl (*R*)-3-(6-(bis(4-methoxybenzyl)amino)-4-methylpyridin-2-yl)-4-chloro-1-fluoro-12-oxo-6a,7,9,10-tetrahydro-12*H*pyrazino[2,1-*c*]pyrido[3,4-*f*][1,4]oxazepine-8(6*H*)-carboxylate (210 mg) as a yellow liquid with a yield of 59%. ES-API: [M+H]⁺=718.2.

Step 2: A solution of *tert*-butyl (*R*)-3-(6-(bis(4-methoxybenzyl)amino)-4-methylpyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (200 mg, 0.28 mmol) in acetonitrile (3 mL) was added with N-iodosuccinimide (313 mg, 1.39 mmol) and *p*-toluenesulfonic acid monohydrate (5 mg, 0.03 mmol). The mixture was stirred at room temperature for 0.5 hours, quenched with saturated sodium thiosulfate solution, extracted with ethyl acetate, concentrated and purified with flash silica gel column (EtOAc/PE: 0-100%) to obtain *tert*-butyl (*R*)-3-(6-(bis(4-methoxybenzyl)amino)-3-iodo-4-methylpyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (170 mg) as a yellow liquid with a yield of 72%. ES-API: [M+H]⁺=844.0.

Step 3: Under nitrogen protection, a solution of *tert*-butyl (*R*)-3-(6-(bis(4-methoxybenzyl)amino)-3-iodo-4-methylpyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (170 mg, 0.20 mmol), methyl 2,2-difluoro-2-(fluorosulfonyl)acetate (770 mg, 4.03 mmol) and cuprous iodide (380 mg, 2.01 mmol) in *N,N-*dimethylformamide (4 mL) was stirred at 90°C for 1.5 hours. The reaction solution was dissolved in 20 mL of ethyl acetate, washed twice with 10 mL of saturated brine and once with 10 mL of water. The organic phase was dried, concentrated and then purified with flash silica gel column (EtOAc/PE: 0-100%) to obtain *tert*-butyl (*R*)-3-(6-(bis(4-methoxybenzyl)amino)-4-methyl-3-(trifluoromethyl)pyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (160 mg) as ayellow solid with ayield of 95%. ES-API: [M+H]⁺=786.1.

Step 4: A solution of *tert*-butyl (*R*)-3-(6-(bis(4-methoxybenzyl)amino)-4-methyl-3-(trifluoromethyl)pyridin-2-yl)-4-chloro-1-fluoro-12-oxo-6a,7,9,10-tetrahydro-12*H*-pyrazino [2,1-*c*]pyrido[3,4*f*][1,4]oxazepine-8(6*H*)-carboxylate (160 mg, 0.20 mmol), morpholine (35 mg, 0.41 mmol) and *N,N*-diisopropylethylamine (79 mg, 0.61 mmol) in acetonitrile (3 mL) was stirred at 80°C for 2 hours. The reaction solution was concentrated and then then purified with flash silica gel column (EtOAc/PE: 0-100%) to obtain *tert*-butyl (*R*)-3-(6-(bis(4-methoxybenzyl)amino)-4-methyl-3-(trifluoromethyl)pyridin-2-yl)-4-chloro-1-morpholino-12-oxo-6a,7,9,10-tetrahydro-12*H*-pyrazino[2,1-*c*]pyrido[3,4-*f*][1,4]oxazepine-8(6*H*)-carboxylate (160 mg) as a yellow solid with a yield of 92%. ES-API: [M+H]⁺=853.2.

Step 5: A solution of *tert*-butyl (*R*)-3-(6-(bis(4-methoxybenzyl)amino)-4-methyl-3-(trifluoromethyl)pyridin-2-yl)-4-chloro-1-morpholino-12-oxo-6a,7,9,10-tetrahydro-12*H*-pyrazino [2,1-*c*]pyrido[3,4-*f*][1,4]oxazepine-8(6*H*)-carboxylate (160 mg, 0.19 mmol) in trifluoroacetic acid (3 mL) was stirred at 50°C for 5 hours, and concentrated to obtain a crude product of (*R*)-3-(6-amino-4-methyl-3-(trifluoromethyl)pyridin-2-yl)-4-chloro-1-morpholino-6,6a,7,8,9,10-hexahydro-12*H*-pyrazino [2,1-*c*]pyrido[3,4-*f*][1,4]oxazepin-12-one (97 mg, theoretical value). ES-API: [M+H]⁺=513.1.

Step 6: The (*R*)-3-(6-amino-4-methyl-3-(trifluoromethyl)pyridin-2-yl)-4-chloro-1-morphofino-6,6a,7,8,9,10-hexahydro-12*H*-pyrazino[2,1-*c*]pyrido[3,4-*f*][1,4]oxazepin-12-one (97 mg, 0.19 mmol) obtained above was dissolved in dichloromethane (2 mL), and *N,N*-diisopropylethylamine (122 mg, 0.95 mmol) was added thereto. The reaction solution was cooled to 0°C, and acryloyl chloride (17 mg, 0.19 mmol) was added dropwise thereto. After stirring for 5 minutes, the mixture was concentrated and the crude product was purified by preparative HPLC to obtain (*R*)-8-acryloyl-3-(6-amino-4-methyl-3-(trifluoromethyl)pyridin-2-yl)-4-chloro-1-morpholino-6,6a,7,8,9,10-hexahydro-12*H*-pyrazino [2,1-*c*]pyrido[3,4-*f*][1,4]oxazepin-12-one (Z314, 28 mg) as a white solid with a yield of 26%. ¹H NMR (500 MHz, DMSO-*d*₆) δ 6.84-6.69 (m, 3H), 6.47-6.41 (m, 1H), 6.17 (d, *J* = 16.5 Hz, 1H), 5.78-5.70 (m, 1H), 4.43-3.83 (m, 7H), 3.69-3.52(m, 6H), 3.50-3.37 (m, 2H), 3.21-3.09 (m, 2H), 2.36-2.28 (m, 3H). ES-API: [M+H]⁺=567.1.

### Embodiment 71-1 Synthesis of Z318

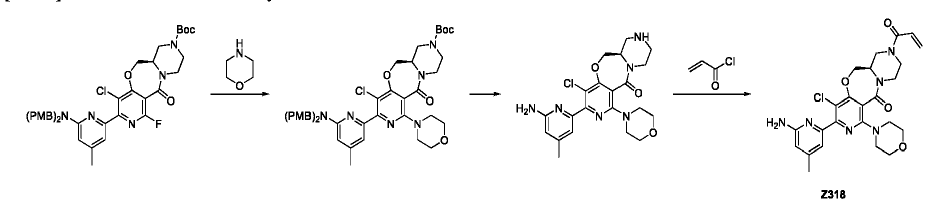

Step 1: A solution of *tert*-butyl (*R*)-3-(6-(bis(4-methoxybenzyl)amino)-4-methylpyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (30 mg, 0.042 mmol), morpholine (8 mg, 0.092 mmol) and *N,N*-diisopropylethylamine (16 mg, 0.126 mmol) in acetonitrile (1 mL) was stirred at 80°C for 2 hours. The reaction solution was concentrated and then then purified with flash silica gel column (ethyl acetate/petroleum ether: 0-100%) to obtain *tert-*butyl (*R*)-3-(6-(bis(4-methoxybenzyl)amino)-4-methylpyridin-2-yl)-4-chloro-1-morpholino-12-oxo-6a,7,9,10-tetrahydro-12*H*-pyrazino[2,1-*c*]pyrido[3,4-*f*][1,4]oxazepine-8(6*H*)-carboxylate (30 mg) as a yellow solid. ES-API: [M+H]⁺=785.2.

Step 2: A solution of *tert*-butyl (*R*)-3-(6-(bis(4-methoxybenzyl)amino)-4-methylpyridin-2-yl)-4-chloro-1-morpholino-12-oxo-6a,7,9,10-tetrahydro-12*H*-pyrazino[2,1-*c*]pyrido[3,4-*f*][1,4]oxazepine-8(6*H-*carboxylate (30 mg, 0.038 mmol) in trifluoroacetic acid (1 mL) was stirred at 50°C for 5 hours, and concentrated to obtain a crude product of (*R*)-3-(6-amino-4-methylpyridin-2-yl)-4-chloro-1-morpholino-6,6a,7,8,9,10-hexahydro-12*H*-pyrazino[2,1-*c*]pyrido[3,4-*f*][1,4]oxazepin-12-one (17 mg, theoretical value). ES-API: [M+H]⁺=445.1.

Step 3: The (*R*)-3-(6-amino-4-methylpyridin-2-yl)-4-chloro-1-morpholino-6,6a,7,8,9,10-hexahydro-12*H*-pyrazino[2,1-*c*]pyrido[3,4-*f*][1,4]oxazepin-12-one (17 mg, 0.038 mmol) obtained above was dissolved in dichloromethane (2 mL), and *N,N*-diisopropylethylamine (25 mg, 0.190 mmol) was added thereto. The reaction solution was cooled to 0°C, and acryloyl chloride (4 mg, 0.038 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-3-(6-amino-4-methylpyridin-2-yl)-4-chloro-1-morpholino-6,6a,7,8,9,10-hexahydro-12*H*-pyrazino[2,1-*c*]pyrido[3,4-*f*][1,4]oxazepin-12-one (4 mg, yield of 21%). ES-API: [M+H]⁺=499.2. ¹HNMR (500 MHz, DMSO-d₆) δ 6.80 - 6.72 (m, 1H), 6.52 (s, 1H), 6.30 (s, 1H), 6.17 (d, *J* = 17.0 Hz, 1H), 5.90 (s, 2H), 5.78 - 5.69 (m, 1H), 4.44 - 3.74 (m, 8H), 3.67 - 3.58 (m, 5H), 3.48 - 3.43 (m, 2H), 3.25 - 3.16 (m, 2H), 2.19 (s, 3H).

### Embodiment 71-2 Synthesis of Z267

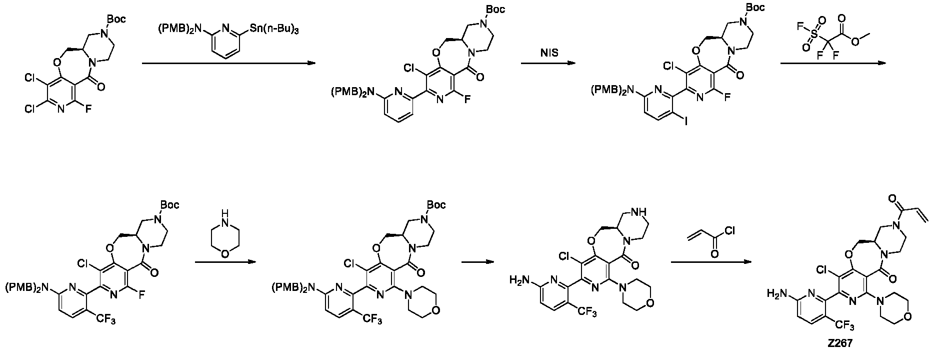

Step 1: Under nitrogen protection, a mixed solution of *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (500 mg, 1.23 mmol), *N,N*-bis(4-methoxybenzyl)-6-(tributylstannyl)pyridin-2-amine (1.53 g, 2.46 mmol, prepared by the method disclosed in patent WO2020097537), tetrakis(triphenylphosphine)palladium (285 mg, 0.25 mmol), cuprous iodide (94 mg, 0.49 mmol) and lithium chloride (260 mg, 6.15 mmol) in 1,4-dioxane (20 mL) were stirred at 110°C for 16 hours, filtered and concentrated, and purified by flash silica gel column (ethyl acetate/petroleum ether: 0-100%) to obtain *tert*-butyl (*R*)-3-(6-(bis(4-methoxybenzyl)amino)pyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (550 mg) as a yellow solid with a yield of 63%. ES-API: [M+H]⁺=704.2.

Step 2: A solution of *tert*-butyl (*R*)-3-(6-(bis(4-methoxybenzyl)amino)pyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg, 0.14 mmol) in acetonitrile (2 mL) was added with *N*-iodosuccinimide (64 mg, 0.28 mmol) and *p-*toluenesulfonic acid monohydrate (3 mg, 0.02 mmol). The mixture was stirred at room temperature for 0.5 hours, quenched with saturated sodium thiosulfate solution (10 mL), extracted with ethyl acetate (10 mL * 3), concentrated and purified with flash silica gel column (ethyl acetate/petroleum ether: 0-100%) to obtain *tert*-butyl (*R*)-3-(6-(bis(4-methoxybenzyl)amino)-3-iodopyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (100 mg) as a yellow liquid with a yield of 85%. ES-API: [M+H]⁺=830.0.

Step 3: Under nitrogen protection, a solution of *tert*-butyl (*R*)-3-(6-(bis(4-methoxybenzyl)amino)-3-iodopyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (200 mg, 0.24 mmol), methyl 2,2-difluoro-2-(fluorosulfonyl)acetate (925 mg, 4.82 mmol) and cuprous iodide (460 mg, 2.41 mmol) in *N,N*-dimethylformamide (5 mL) was stirred at 90°C for 2 hours. The reaction solution was dissolved in ethyl acetate (20 mL) and washed with saturated brine (10 mL^{∗}2) and water (10 mL). The organic phase was dried, concentrated and then purified with flash silica gel column (ethyl acetate/petroleum ether: 0-100%) to obtain *tert*-butyl (*R*)-3-(6-(bis(4-methoxybenzyl)amino)-3-(trifluoromethyl)pyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (120 mg, yield of 63%) as a yellow solid. ES-API: [M+H]⁺=712.2.

Step 4: A solution of *tert*-butyl (*R*)-3-(6-(bis(4-methoxybenzyl)amino)-3-(trifluoromethyl)pyridin-2-yl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (120 mg, 0.16 mmol), morpholine (27 mg, 0.31 mmol) and potassium carbonate (64 mg, 0.47 mmol) in acetonitrile (2 mL) was stirred at 90°C for 5 hours. The reaction solution was concentrated and then then purified with flash silica gel column (ethyl acetate/petroleum ether: 0-100%) to obtain *tert-*butyl (*R*)-3-(6-(bis(4-methoxybenzyl)amino)-3-(trifluoromethyl)pyridin-2-yl)-4-chloro-1-morphofino-12-oxo-6a,7,9,10-tetrahydro-12*H*-pyrazino[2,1-*c*]pyrido[3,4-*f*][1,4]oxazepine-8(6*H*)-carboxylate (100 mg, yield of 77%) as a yellow solid. ES-API: [M+H]⁺=839.2.

Step 5: A solution of *tert*-butyl (*R*)-3-(6-(bis(4-methoxybenzyl)amino)-3-(trifluoromethyl)pyridin-2-yl)-4-chloro-1 -morpholino-12-oxo-6a,7,9,10-tetrahydro-12*H*-pyrazino [2,1-*c*]pyrido[3,4-*f*][1,4]oxazepine-8(6*H*)-carboxylate (100 mg, 0.12 mmol) in trifluoroacetic acid (1 mL) was stirred at 50°C for 5 hours, and concentrated to obtain a crude product of (*R*)-3-(6-amino-3-(trifluoromethyl)pyridin-2-yl)-4-chloro-1-morpholino-6,6a,7,8,9,10-hexahydro-12*H*-pyrazino[2,1-*c*]pyrido[3,4-*f*][1,4]oxazepin-12-one (59 mg, theoretical value). ES-API: [M+H]⁺=499.1.

Step 6: The (*R*)-3-(6-amino-3-(trifluoromethyl)pyridin-2-yl)-4-chloro-1-morpholino-6,6a,7,8,9,10-hexahydro-12*H*-pyrazino[2,1-*c*]pyrido[3,4-*f*][1,4]oxazepin-12-one (59 mg, 0.12 mmol) obtained above was dissolved in dichloromethane (1 mL), and *N,N*-diisopropylethylamine (76 mg, 0.59 mmol) was added thereto. The reaction solution was cooled to 0°C, and acryloyl chloride (11 mg, 0.59 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-3-(6-amino-3-(trifluoromethyl)pyridin-2-yl)-4-chloro-1-morpholino-6,6a,7,8,9,10-hexahydro-12*H*-pyrazino [2,1-*c*]pyrido[3,4-*f*][1,4]oxazepin-12-one (30 mg, yield of 46%) as a white solid. ES-API: [M+H]⁺=553.2. ¹H NMR (500 MHz, DMSO-*d*₆) δ 7.74 (d, *J* = 9.0 Hz, 1H), 6.85 (s, 2H), 6.81 - 6.71 (m, 1H), 6.58 (d, *J* = 9.0 Hz, 1H), 6.17 (d, *J* = 17.0 Hz, 1H), 5.78 - 5.71 (m, 1H), 4.44 - 4.28 (m, 2H), 4.25 - 4.10 (m, 2H), 4.08 - 3.89 (m, 3H), 3.89 - 3.69 (m, 1H), 3.66 - 3.52 (m, 5H), 3.48 - 3.41 (m, 2H), 3.19 - 3.11 (m, 2H).

### Embodiment 71-3 Synthesis of Z328-1

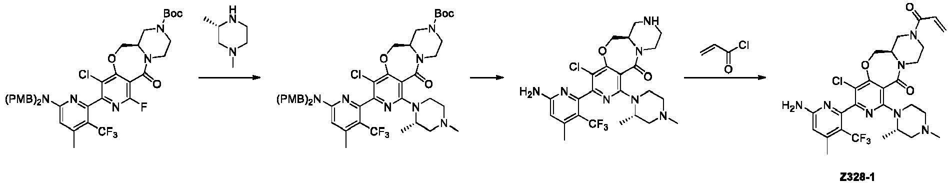

Step 1: A solution of *tert*-butyl (*R*)-3-(6-(bis(4-methoxybenzyl)amino)-4-methyl-3-(trifluoromethyl)pyridin-2-yl)-4-chloro-1-fluoro-12-oxo-6a,7,9,10-tetrahydro-12*H*-pyrazino [2,1-*c*]pyrido[3,4-*f*][1,4]oxazepine-8(6*H*)-carboxylate (120 mg, 0.15 mmol), (*S*)-1,3-dimethylpiperazine (35 mg, 0.30 mmol) and potassium carbonate (64 mg, 0.46 mmol) in acetonitrile (2 mL) was stirred at 90°C for 6 hours. The reaction solution was concentrated and then then purified with flash silica gel column (ethyl acetate/petroleum ether: 0-100%) to obtain *tert*-butyl (*R*)-3-(6-(bis(4-methoxybenzyl)amino)-4-methyl-3-(trifluoromethyl)pyridin-2-yl)-4-chloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg) as a yellow solid. ES-API: [M+H]⁺=772.2.

Step 2: A solution of *tert*-butyl (*R*)-3-(6-(bis(4-methoxybenzyl)amino)-4-methyl-3-(trifluoromethyl)pyridin-2-yl)-4-chloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (80 mg, 0.09 mmol) in trifluoroacetic acid (1 mL) was stirred at 50°C for 5 hours, and concentrated to obtain a crude product of (*R*)-3-(6-amino-4-methyl-3-(trifluoromethyl)pyridin-2-yl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (49 mg, theoretical value). ES-API: [M+H]⁺=540.0.

Step 3: The (*R*)-3-(6-amino-4-methyl-3-(trifluoromethyl)pyridin-2-yl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (49 mg, 0.09 mmol) obtained above was dissolved in dichloromethane (1 mL), and *N,N-*diisopropylethylamine (58 mg, 0.45 mmol) was added thereto. The reaction solution was cooled to 0°C, and acryloyl chloride (8 mg, 0.09 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-3-(6-amino-4-methyl-3-(trifluoromethyl)pyridin-2-yl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (Z328-1, 12 mg, yield of 22%). ES-API: [M+H]⁺=594.3. ¹HNMR (500 MHz, DMSO-*d₆*) δ 6.82 - 6.68 (m, 3H), 6.43 (s, 1H), 6.22 - 6.13 (m, 1H), 5.79 - 5.69 (m, 1H), 4.41 - 4.21 (m, 2H), 4.19 - 3.78 (m, 6H), 3.73 - 3.42 (m, 4H), 3.24 - 3.11 (m, 1H), 2.59 (d, *J* = 10.5 Hz, 1H), 2.33 (d, *J* = 8.5 Hz, 3H), 2.11 (d, *J=* 3.0 Hz, 3H), 2.03 - 1.87 (m, 2H), 1.22 (dd, *J =* 16.0, 6.5 Hz, 3H).

### Embodiment 72 Synthesis of Z241a-2, Z241a-1 and Z241a

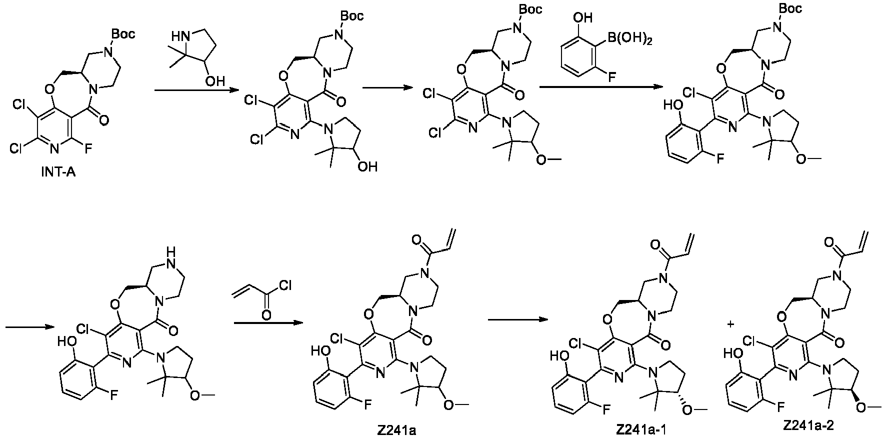

Step 1: A solution of *tert*-butyl (*R*)-3,4-dichloro-1-fluoro-12-oxo-6a,7,9,10-tetrahydro-12*H-*pyrazino[2,1-*c*]pyrido[3,4-*f*][1,4]oxazepine-8(6*H*)-carboxylate (300 mg, 0.74 mmol), 2,2-dimethylpyrrolidin-3-ol (100 mg, 0.88 mmol) and potassium carbonate (300 mg, 2.22 mmol) in acetonitrile (4 mL) was stirred at 85°C for 3 hours, filtered and concentrated to obtain a crude product of *tert*-butyl (6a*R*)-3,4-dichloro-1 -(3 -hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (400 mg) as a yellow solid. ES-API: [M+H]⁺=501.1.

Step 2: A solution of the crude product of *tert*-butyl (6a*R*)-3,4-dichloro-1-(3-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.40 mmol) in acetonitrile (4 mL) was added with sodium hydride (80 mg, 1.99 mmol) and iodomethane (283 mg, 2 mmol) under an ice bath. The mixture was stirred at room temperature for 1 hour, quenched with saturated ammonium chloride solution, extracted with ethyl acetate, concentrated and purified with flash silica gel column (EtOAc/PE: 0-80%) to obtain *tert*-butyl (6a*R*)-3,4-dichloro-1-(3-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (160 mg) as a white solid with a yield of 78%. ES-API: [M+H]⁺=515.2.

Step 3: Under nitrogen protection, a mixed solution of tert-butyl (6a*R*)-3,4-dichloro-1-(3-methoxy-2,2-dimethylpyrrolidin-1-yl)-12-oxo-6a,7,9,10-tetrahydro-12*H*-pyrazino[2,1-*c*]pyrido [3,4-*f*][1,4]oxazepine-8(6*H*)-carboxylate (150 mg, 0.29 mmol), 2-fluoro-6-hydroxyphenylboronic acid (136 mg, 0.87 mmol), tetrakis(triphenylphosphine)palladium (34 mg, 0.03 mmol) and potassium carbonate (121 mg, 0.87 mmol) in 1,4-dioxane (2 mL) and water (0.4 mL) was stirred at 110°C for 2 hours. The reaction solution was dissolved in 20 mL of ethyl acetate, washed twice with 10 mL of saturated brine and once with 10 mL of water. The organic phase was dried, concentrated and then purified with flash silica gel column (EtOAc/PE: 0-100%) to obtain *tert*-butyl (6a*R*)-4-chloro-3-(2-fluoro-6-hydroxyphenyl)-1-(3-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg) as a white solid with a yield of 87%. ES-API: [M+H]⁺=591.1.

Step 4: Under an ice bath, a solution of *tert*-butyl (6a*R*)-4-chloro-3-(2-fluoro-6-hydroxyphenyl)-1-(3 -methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg, 0.25 mmol) in dichloromethane (4 mL) was added with trifluoroacetic acid (1 mL), stirred at room temperature for 1 hour, and concentrated to obtain a crude product of (6a*R*)-4-chloro-3-(2-fluoro-6-hydroxyphenyl)-1-(3-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (125 mg, theoretical value). ES-API: [M+H]⁺=491.2.

Step 5: (6a*R*)-4-Chloro-3-(2-fluoro-6-hydroxyphenyl)-1-(3-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (125 mg, 0.25 mmol) was dissolved in dichloromethane (2 mL), and *N,N*-diisopropylethylamine (165 mg, 1.27 mmol) was added thereto. The reaction solution was cooled to 0°C, and acryloyl chloride (23 mg, 0.25 mmol) was added dropwise thereto. After stirring for 5 minutes, the mixture was concentrated and the crude product was purified by preparative HPLC (ammonium bicarbonate) to obtain (6a*R*)-8-acryloyl-4-chloro-3-(2-fluoro-6-hydroxyphenyl)-1-(3 -methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (Z241a, 81 mg, yield of 58%) as a white solid. ES-API: [M+H]⁺=545.1.

Step 6: The Z241a (81 mg, 0.15 mmol) obtained from the above steps was subjected to chiral separation (separation column: AB 250 mm*4.6 mm*5 µm; mobile phase: ethanol:*n*-hexane = 60:40; flow rate: 1 mL/min; column temperature: 30°C) to obtain two isomer compounds.

One isomer with a structure arbitrarily designated as (6a*R*)-8-acryloyl-4-chloro-3-(2-fluoro-6-hydroxyphenyl)-1-((*S*)-3-methoxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z241a-1, 43 mg, peak 1, retention time: 6.33 min, purity: 100%, de value: 100%). ¹H NMR (500 MHz, CDCl₃) δ 7.32-7.22 (m, 1H), 6.83 (d, *J* = 8.5 Hz, 1H), 6.70 (t, *J* = 8.5 Hz, 1H), 6.63-6.49 (m, 1H), 6.39 (d, *J* = 16.5 Hz, 1H), 5.81 (d, *J* = 10.5 Hz, 1H), 4.54-4.42 (m, 1H), 4.41-4.29 (m, 2H), 4.25-4.11 (m, 2H), 4.08-3.94 (m, 1H), 3.90-3.75 (m, 1H), 3.75-3.62 (m, 1H), 3.62-6.51 (m, 1H), 3.48-3.38 (m, 2H), 3.36 (s, 3H), 2.85-2.71 (m, 1H), 2.09-1.91 (m, 2H), 1.65-1.40 (m, 6H). ES-API: [M+H]⁺=545.1.

Another isomer with a structure arbitrarily designated as (6a*R*)-8-acryloyl-4-chloro-3-(2-fluoro-6-hydroxyphenyl)-1-((*R*)-3-methoxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z241a-2, 33 mg, peak 2, retention time: 8.48 min, purity: 100%, de value: 100%). ¹H NMR (500 MHz, CDCl₃) δ 7.32-7.24 (m, 1H), 6.87-6.79 (m, 1H), 6.71 (t, *J* = 8.0 Hz, 1H), 6.64-6.48 (m, 1H), 6.45-6.35 (m, 1H), 5.82 (d, *J=* 10.5 Hz, 1H), 4.54-4.29 (m, 3H), 4.26-4.12 (m, 2H), 4.10-3.95 (m, 1H), 3.76-3.50 (m, 4H), 3.44 (s, 3H), 3.43-3.35 (m, 1H) 2.92-2.71 (m, 1H), 2.29-2.16 (m, 1H), 1.86-1.71 (m, 1H), 1.70-1.32 (m, 6H). ES-API: [M+H]⁺=545.1.

### Embodiment 73 Synthesis of Z296-1 and Z296-2

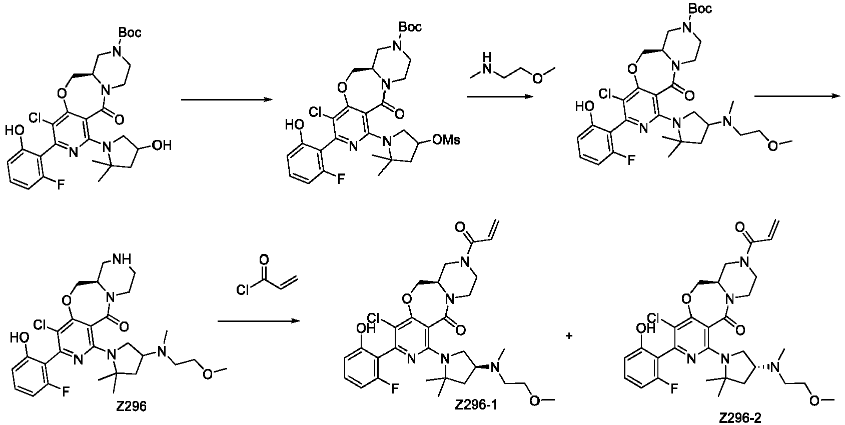

Step 1: *tert*-Butyl (6a*R*)-4-chloro-3-(2-fluoro-6-hydroxyphenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (395 mg, 0.69 mmol), methylsulfonyl chloride (236 mg, 2.07 mmol), 4-dimethylaminopyridine (253 mg, 2.07 mmol), pyridine (164 mg, 2.07 mmol) and dichloromethane (10 mL) were added to a flask. The mixture was reacted overnight at room temperature. The completion of reaction was detected by LC-MS, and then 20 mL of saturated sodium bicarbonate solution was added to the reaction solution, and the mixture was extracted with dichloromethane. The organic phase was dried and concentrated, and the crude product was purified by flash silica gel column (EtOAc/PE: 0-50%) to obtain *tert*-butyl (6a*R*)-4-chloro-1-(2,2-dimethyl-4-((methylsulfonyl)oxy)pyrrolidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (297 mg, 66%) as a yellow solid. ES-API: [M+H]⁺=654.2.

Step 2: *tert*-Butyl (6a*R*)-4-chloro-1-(2,2-dimethyl-4-((methylsulfonyl)oxy)pyrrolidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (297 mg, 0.45 mmol) and 3 mL of 2-methoxy-*N*-methylethan-1-amine were added to a flask. The mixture was stirred at 105°C for 20 hours, and the completion of reaction was detected by LC-MS. The reaction solution was concentrated, and the crude product was purified by flash silica gel column (EtOAc/PE: 0-70%) to obtain *tert*-butyl (6a*R*)-4-chloro-3-(2-fluoro-6-hydroxyphenyl)-1-(4-((2-methoxyethyl)(methyl)amino)-2,2-dimethylpyrrolidin-1-yl)-12-oxo-6,7,9,10-tetrahydro-12*H*-pyrazino[2,1-c]pyrido[3,4-*f*][1,4]oxazepine-8(6*H*)-carboxylate (157 mg, 54%) as a yellow solid, and the crude product was directly used in the next step. ES-API: [M+H]⁺=648.2.

Step 3: *tert*-Butyl (6a*R*)-4-chloro-3-(2-fluoro-6-hydroxyphenyl)-1-(4-((2-methoxyethyl)(methyl)amino)-2,2-dimethylpyrrolidin-1-yl)-12-oxo-6,7,9,10-tetrahydro-12*H*-pyrazino[2,1-*c*]pyrido[3,4-*f*][1,4]oxazepine-8(6*H*)-carboxylate (157 mg, 0.24 mmol), 3 mL of dichloromethane and 2 mL of trifluoroacetic acid were added to a flask. The mixture was stirred at room temperature for 1 hour, and the completion of reaction was detected by LC-MS. The reaction solution was concentrated to obtain a crude product of (6a*R*)-4-chloro-3-(2-fluoro-6-hydroxyphenyl)-1-(4-((2-methoxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one (Z296, 231 mg, crude product) as a yellow solid, and the crude product was directly used in the next step. ES-API: [M+H]⁺=548.2.

Step 4: (6a*R*)-4-Chloro-3-(2-fluoro-6-hydroxyphenyl)-1-(4-((2-methoxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one (231 mg, 0.42 mmol), 10 mL of dichloromethane and triethylamine (425 mg, 4.2 mmol) were added to a flask. At 0°C, a solution of acryloyl chloride in dichloromethane (31 mg, 0.34 mmol, 1 mL) was added dropwise thereto. The reaction was carried out at 0°C for 10 minutes. 20 mL of saturated aqueous sodium bicarbonate solution was added to the reaction solution, and the mixture was extracted three times with 20 mL of dichloromethane. The organic phase was dried and concentrated, and the crude product was purified by preparative HPLC (chromatographic column: Xbridge C18 19*150 mm 5 µm, preparation method: 30-50% acetonitrile: water (0.1% ammonium bicarbonate)) to obtain two isomer compounds.

One isomer with a structure arbitrarily designated as (6a*R*)-8-acryloyl-4-chloro-3-(2-fluoro-6-hydroxyphenyl)-1-((*S*)-4-((2-methoxyethyl)(methyl)amino)-2,2-dimethylpyrrolidon-1-yl)-6,6a,7,8,9,10-hexahydro-12*H*-pyrazino[2,1-*c*]pyrido[3,4-*f*][1,4]oxazepin-12-one (Z296-1, peak 1, retention time: 1.75 min, 60 mg, 24%). ¹H NMR (400 MHz, DMSO-*d*₆) δ 9.98 (d, *J* = 7.6 Hz, 1H), 7.23 (dd, *J* = 15.5, 8.0 Hz, 1H), 6.91-6.61 (m, 3H), 6.19 (s, 1H), 5.73 (s, 1H), 4.63-4.53 (m, 1H), 4.39-4.30 (m, 1H), 4.21-4.10 (m, 1H), 4.10-4.04 (m, 2H), 3.91-3.69 (m, 1H), 3.67-3.51 (m, 1H), 3.47-3.36 (m, 3H), 3.30-3.21 (m, 3H), 3.24 (s, 4H), 3.01 (s, 2H), 2.16 (s, 3H), 1.99 (s, 1H), 1.63 (s, 1H), 1.55-1.38 (m, 6H). ES-API: [M+H]⁺=602.2.

Another isomer with a structure arbitrarily designated as (6a*R*)-8-acryloyl-4-chloro-3-(2-fluoro-6-hydroxyphenyl)-1-((*R*)-4-((2-methoxyethyl)(methyl)amino)-2,2-dimethylpyrrolidon-1-yl)-6,6a,7,8,9,10-hexahydro-12*H*-pyrazino[2,1-*c*]pyrido[3,4-*f*][1,4]oxazepin-12-one (Z296-2, peak 2, retention time: 1.81 min, 21 mg, 8%). ¹H NMR (500 MHz, DMSO-*d*₆) δ 9.95 (d, *J* = 5.6 Hz, 1H), 7.23 (dd, *J* = 15.4, 7.9 Hz, 1H), 6.88-6.58 (m, 3H), 6.16 (d, *J* = 13.8 Hz, 1H), 5.73 (s, 1H), 4.59-4.18 (m, 5H), 3.93-3.49 (m, 4H), 3.39-3.31 (m, 3H), 3.20 (d, *J* = 2.3 Hz, 3H), 2.95-2.85 (m, 2H), 2.62-2.53 (m, 1H), 2.41-2.31 (m, 1H), 2.15 (s, 3H), 1.91 (s, 2H), 1.56 (s, 3H), 1.40 (s, 3H). ES-API: [M+H]⁺=602.2.

### Embodiment 73-1 Synthesis of Z467

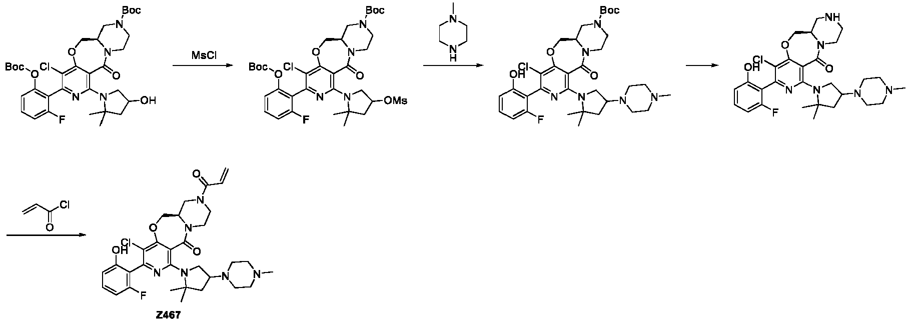

Step 1: Under ice-bath conditions, methanesulfonyl chloride (61 mg, 0.53 mmol) was slowly added dropwise to a solution of *tert*-butyl (6a*R*)-3-(2-((tert-butoxycarbonyl)oxy)-6-fluorophenyl)-4-chloro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (300 mg, 0.44 mmol) and *N,N*-diisopropylethylamine (170 mg, 1.32 mmol) in dichloromethane (10 mL), and the reaction solution was stirred at room temperature for 1 hour. The reaction solution was added with dichloromethane (10 mL), and washed with water (10 mL*3), and the organic phase was dried, concentrated, and then purified by flash silica gel column (ethyl acetate: petroleum ether: 0-70%) to obtain *tert*-butyl (6a*R*)-3-(2-((*tert*-butoxycarbonyl)oxy)-6-fluorophenyl)-4-chloro-1-(2,2-dimethyl-4-((methylsulfonyl)oxy)pyrrolidin-1-yl)-12-oxo-6a,7,9,10-tetrahydro-12*H*-pyrazino [2,1-c]pyrido[3,4-*f*][1,4]oxazepine-8(6*H*)-carboxylate (300 mg). ES-API: [M+H]⁺=755.2.

Step 2: A mixture of compound *tert*-butyl (6a*R*)-3-(2-((*tert*-butoxycarbonyl)oxy)-6-fluorophenyl)-4-chloro-1-(2,2-dimethyl-4-((methylsulfonyl)oxy)pyrrolidin-1-yl)-12-oxo-6a,7,9,10-tetrahydro-12*H-*pyrazino[2,1-*c*]pyrido[3,4-*f*][1,4]oxazepine-8(6*H*)-carboxylate (300 mg, 0.40 mmol) and *N-*methylpiperazine (2.5 mL) was stirred at 100°C overnight. The reaction solution was dissolved in dichloromethane (20 mL), and washed with water (10 mL*3). The organic phase was filtered and concentrated, and then purified by flash silica gel column (methanol: dichloromethane: 0-10%) to obtain *tert*-butyl (6a*R*)-4-chloro-1-(2,2-dimethyl-4-(4-methylpiperazin-1-yl)pyrrolidin-1-yl)-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (180 mg). ES-API: [M+H]⁺=659.2.

Step 3: Under an ice-bath, a solution of *tert*-butyl (6a*R*)-4-chloro-1-(2,2-dimethyl-4-(4-methylpiperazin-1-yl)pyrrolidin-1-yl)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (180 mg, 0.27 mmol) in dichloromethane (4 mL) was added with trifluoroacetic acid (1 mL), and the reaction solution was stirred at room temperature for 1 hour. The reaction solution was concentrated under reduced pressure to obtain a crude product of (6a*R*)-4-chloro-1-(2,2-dimethyl-4-(4-methylpiperazin-1-yl)pyrrolidin-1-yl)-3 -(2-fluoro-6-hydroxyphenyl)-6,6a,7,8,9,10-hexahydro-12*H*-pyrazino[2,1-*c*]pyrido[3,4-*f*][1,4]oxazepin-12-one (153 mg). ES-API: [M+H]⁺=559.3.

Step 4: The above crude procuct of (6a*R*)-4-chloro-1-(2,2-dimethyl-4-(4-methylpiperazin-1-yl)pyrrolidin-1 -yl)-3 -(2-fluoro-6-hydroxyphenyl)-6,6a,7,8,9,10-hexahydro-12*H*-pyrazino [2,1-*c*]pyrido [3,4--*f*][1,4]oxazepin-12-one (153 mg, 0.27 mmol) was dissolved in dichloromethane (4 mL), and *N,N-*diisopropylethylamine (174 mg, 1.35 mmol) was added thereto. The reaction solution was cooled to 0°C, and acryloyl chloride (24 mg, 0.27 mmol) was added dropwise thereto. After stirring for 10 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,2-dimethyl-4-(4-methylpiperazin-1-yl)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z467, 70 mg, yield: 42%). ES-API: [M+H]⁺=613.3.

### Embodiment 73-2 Synthesis of Z297 and isomers thereof

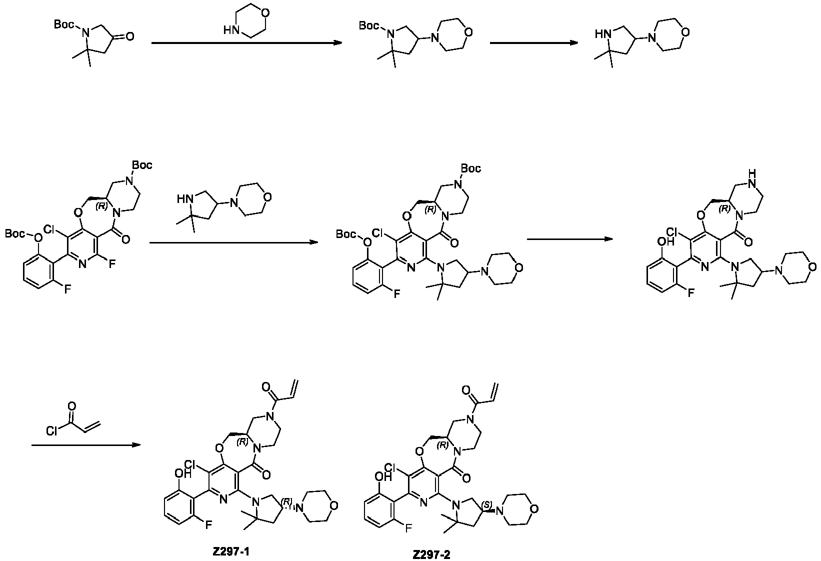

Step 1: *tert*-Butyl 2,2-dimethyl-4-oxopyrrolidine-1-carboxylate (500 mg, 2.32 mmol, 1.0 eq.), morpholine (1.01 g, 11.6 mmol) and 0.2 mLof acetic acid were dissolved in 20 mL of dichloromethane, then sodium triacetoxyborohydride (737 mg, 3.48 mmol) was slowly added thereto. The reaction solution was stirred at room temperature for 16 hours. After the reaction, the reaction solution was added with dichloromethane, then added with saturated sodium bicarbonate solution in turn, washed with brine and water. The organic phase was dried over anhydrous sodium sulfate, concentrated, and the crude product was chromatographed by column chromatography (ethyl acetate/petroleum ether=30%-100%, R_{f}=0.4) to obtain *tert*-butyl 2,2-dimethyl-4-morpholinopyrrolidine-1-carboxylate (240 mg, yield of 36.4%) as a colorless oily liquid. ES-API: [M+H]⁺= 285.2

Step 2: *tert*-Butyl 2,2-dimethyl-4-morpholinopyrrolidine-1-carboxylate (240 mg, 0.84 mmol) was dissolved in 5mL of dichloromethane, and trifluoroacetic acid (2 mL) was slowly added dropwise thereto, and the reaction solution was stirred at room temperature for 2 hours. After the reaction was completed, the reaction solution was concentrated to obtain 160 mg of a crude product of 4-(5,5-dimethylpyrrolidin-3-yl)morpholine, which was directly used in the next reaction step. ES-API: [M+H]⁺= 185.2.

Step 3: Compound *tert*-butyl (6a*R*)-3-(2-((tert-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepin-12-one (300 mg, 0.52 mmol), (4-(5,5-dimethylpyrrolidin-3-yl)morpholine (95 mg, 0.52 mmol) and potassium carbonate (215 mg, 1.56 mmol) were dissolved in acetonitrile (15 mL), reacted at 80°C for 2 hours. After the reaction was completed, the reaction system was cooled to room temperature, quenched with water, extracted with ethyl acetate, washed once with brine, dried over anhydrous sodium sulfate, and the filtrate was concentrated. The crude product was chromatographed by column chromatography (EA:PE=1:1, R_{f}= 0.4) to obtain (6a*R*)-*tert*-butyl 3-(2-((*tert*-butoxycarbonyl)oxy)-6-fluorophenyl)-4-chloro-1-((4-(5,5-dimethylpyrrolidin-3-yl)moipholino-12-oxo-6a,7,9,10-tetrahydro-12*H*-pyrazino[2,1-*c*]pyrido[3,4-*f*][1,4]oxazepin-12-one (120 mg, 31%) as a yellow solid. ES-API: [M+H]⁺= 746.4.

Step 4: (6a*R*)-*tert*-Butyl 3-(2-((*tert*-butoxycarbonyl)oxy)-6-fluorophenyl)-4-chloro-1-((4-(5,5-dimethylpyrrolidin-3 -yl)morpholino-12-oxo-6a,7,9,10-tetrahydro-12*H*-pyrazino [2,1-*c*]pyrido [3,4--*f*][1,4]oxazepin-12-one (120 mg, 0.16 mmol) was dissolved in 5 mL of dichloromethane, and then trifluoroacetic acid (1 mL) was added thereto. The reaction solution was stirred at room temperature for 2 hours. After the reaction was monitored to be complete, the mixture was diluted with dichloromethane, washed with water, washed with saturated sodium bicarbonate and brine once respectively, dried over anhydrous sodium sulfate, and the filtrate was evaporated to dryness by rotary evaporation to obtain compound (6a*R*)-4-chloro-1-((2,2-dimethyl-4-morphofinopyrrolidin-1-yl))-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (120 mg, crude product). ES-API: [M+H]⁺= 546.4.

Step 5: (6a*R*)-4-Chloro-1-((2,2-dimethyl-4-morphofinopyrrolidin-1-yl))-3-(2-fluoro-6-hydroxyphenyl)-7,8,9,10-tetrahydro-6*H*-pyrazino[2,1-*c*]pyrido[3,4-*f*][1,4]oxazepin-12(6a*H*)-one (140 mg, 0.18 mmol) was dissolved in dichloromethane (5 mL), then cooled to 0°C in an ice-water bath. Saturated sodium bicarbonate solution was added thereto to adjust the pH to 8-9. Potassium phosphate (101 mg) was added thereto, and then acryloyl chloride (8.6 mg, 0.096 mmol) was added dropwise thereto and stirred for 10 minutes. After the reaction was completed, the mixture was diluted with dichloromethane, washed with water, dried over sodium sulfate, evaporated to dryness by rotary evaporation, and the crude product was purified by chromatography plate to obtain two isomer compounds:

One isomer (methanol/dichloromethane=1:10, R_{f}=0.6) with a structure arbitrarily designated as (6a*R*)-8-acryloyl-4-chloro-1-((2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z297-1, 17 mg, yield: 17.7%, white solid). ¹H NMR (400 MHz, CDCl₃) δ 7.29 (d, *J* = 8.0 Hz, 1H), 6.83 (d, *J* = 8.3 Hz, 1H), 6.71 (t, *J* = 8.9 Hz, 1H), 6.55 (s, 1H), 6.39 (d, *J* = 16.4 Hz, 1H), 5.81 (d, *J* = 10.3 Hz, 1H), 4.51 - 3.47 (m, 16H), 2.63 (s, 2H), 2.20 - 1.99 (m, 2H), 1.46 (s, 3H), 1.33 - 1.23 (m, 3H). ES-API: [M+H]⁺= 600.3. And
another isomer (methanol/dichloromethane=1:10, R_{f}=0.4) with a structure arbitrarily designated as (6a*R*)-8-acryloyl-4-chloro-1-((2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one (Z297-2, 7 mg, yield: 7.3%, white solid). ¹H NMR (400 MHz, CDCl₃) δ 6.84 (d, *J* =8.3 Hz, 2H), 6.75 - 6.50 (m,1H), 6.39 (d, *J* = 15.9 Hz, 1H),5.80 (d, *J* = 10.6 Hz, 1H), 4.50- 2.89 (m, 20H), 1.51 - 1.45(m, 8H). ES-API: [M+H]⁺= 600.3.

### Embodiment 74 Synthesis of (6aR)-8-acryloyl-4-chloro-1-(1,3-dimethyl-1,2,3,6-tetrahydropyridin-4-yl)-3-(2-fluoro-6-hydroxyphenyl)-6,6a,7,8,9,10-hexahydro-12H-pyrazino[2,1-c]pyrido[3,4-f]oxazepin-12-one Z304

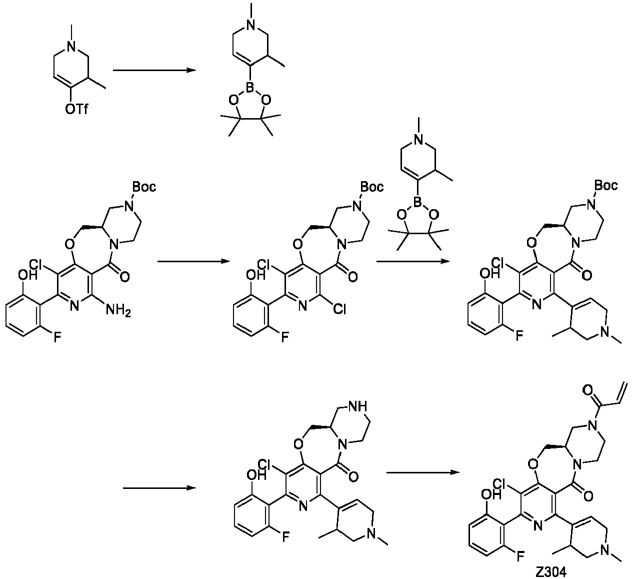

Step 1: Under nitrogen protection, 1,3-dimethyl-1,2,3,6-tetrahydropyridin-4-yl trifluoromethanesulfonate (1.0 g, 3.85 mmol) was dissolved in 1,4-dioxane (35 mL), and then [1,1'-bis(diphenylphosphino)ferrocene]palladium dichloride dichloromethane complex (302 mg, 0.371 mmol), potassium acetate (1.13 g, 11.5 mmol) and bis(pinacolato)diboron (1.08 g, 4.25 mmol) were added to the system. The mixture was heated at 80°C and reacted for 5 hours. The reaction was completed. Ethyl acetate (50 mL) was added to the system. The organic phase was washed with saturated NaHCO₃ solution and saturated brine in turn, and the organic phase was dried over anhydrous sodium sulfate, filtered, and the filtrate was evaporated to dryness by rotary evaporation under reduced pressure to obtain 1,3-dimethyl-4-(4,4,5,5-tetramethyl-1,3,2-dioxaborolan-2-yl)-1,2,3,6-tetrahydropyridine (1.3 g, crude product), which was directly used in step 3.

Step 2: *tert*-Butyl (6a*R*)-1-amino-4-chloro-3-(2-fluoro-6-hydroxyphenyl)-12-oxo-6a, 7,9,10-tetrahydro-12*H*-pyrazino[2,1-*c*]pyrido[3,4-*f*][1,4]oxazepine-8(6*H*)-carboxylate (576 mg, 1.2 mmol) was added to dichloromethane (12 mL) at room temperature. After the dissolution, benzyltriethylammonium chloride (2.76 g, 12.0 mmol) was added thereto, stirred at room temperature for 5 minutes, and then *tert-*butyl nitrite (714 µL, 6.0 mmol) was added thereto, stirred at room temperature overnight. After the reaction was completed, dichloromethane (30 mL) was added to the system, and washed twice with saturated aqueous sodium bicarbonate solution (2*20 mL). The organic phase was dried over anhydrous sodium sulfate, filtered, and the filtrate was evaporated to dryness by rotary evaporation under reduced pressure. The crude product was purified by flash silica gel column (EtOAc/PE: 0-100%) to obtain *tert*-butyl (6a*R*)-1,4-dichloro-3-(2-fluoro-6-hydroxyphenyl)-12-oxo-6a,7,9,10-tetrahydro-12*H*-pyrazino [2,1-*c*]pyrido[3,4-*f*][1,4]oxazepine-8(6*H*)-carboxylate (250 mg, yield of 42%). ES-API: [M+H]⁺=498.1.

Step 3: *tert*-Butyl (6a*R*)-1,4-dichloro-3-(2-fluoro-6-hydroxyphenyl)-12-oxo-6a,7,9,10-tetrahydro-12*H*-pyrazino[2,1-*c*]pyrido[3,4-*f*][1,4]oxazepine-8(6*H*)-carboxylate (200 mg, 0.40 mmol), 1,3-dimethyl-4-(4,4,5,5-tetramethyl-1,3,2-dioxaborolan-2-yl)-1,2,3,6-tetrahydropyridine (400 mg, crude product from step 1), [1,1'-bis(diphenylphosphino)ferrocene]palladium dichloride dichloromethane complex (40 mg, 0.049 mmol) and potassium carbonate (180 mg, 1.38 mmol) were added to a mixed solution of dioxane (6 mL) and water (1.5 mL), reacted at 100°C for 8 hours under nitrogen protection. After the reaction was completed, the reaction solution was cooled to room temperature, added with ethyl acetate (40 mL), washed twice with saturated brine (2^{∗}20 mL). The organic phase was dried over anhydrous sodium sulfate, filtered, and the filtrate was evaporated to dryness by rotary evaporation under reduced pressure. The crude product was purified by HPLC (CH₃CN/water+TFA 0.1%) to obtain *tert*-butyl (6a*R*)-4-chloro-1-(1,3-dimethyl-1,2,3,6-tetrahydropyridin-4-yl)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (70 mg, yield of 30%). ES-API: [M+H]⁺=573.2.

Step 4: Trifluoroacetic acid (1 mL) was added to a solution of *tert*-butyl (6aR)-4-chloro-1-(1,3-dimethyl-1,2,3,6-tetrahydropyridin-4-yl)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (40 mg, 0.070 mmol) in dichloromethane (2 mL). After stirring at room temperature for 2 hours, the reaction solution was concentrated to obtain a crude product of (6a*R*)-4-chloro-1-(1,3-dimethyl-1,2,3,6-tetrahydropyridin-4-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (35 mg, crude product), which was directly used in the next reaction step. ES-API: [M+H]⁺=473.1.

Step 5: The crude product of (6a*R*)-4-chloro-1-(1,3-dimethyl-1,2,3,6-tetrahydropyridin-4-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (35 mg, crude product) obtained in step 4 was dissolved in dichloromethane (5 mL), and triethylamine (97 µL, 0.70 mmol) was added thereto. A solution of acryloyl chloride (7.60 mg, 0.084 mmol) in dichloromethane (1 mL) was added to the reaction solution at 0 to 5°C. The reaction was stirred at 0°C for 5 minutes. The reaction solution was added with 5 mL of dichloromethane, washed with 10 mL of saturated aqueous NaHCO₃ solution and 10 mL of saturated brine, and the organic phase was dried over anhydrous sodium sulfate and then concentrated. The crude product was purified by preparative HPLC to obtain (6a*R*)-8-acryloyl-4-chloro-1-(1,3-dimethyl-1,2,3,6-tetrahydropyridin-4-yl)-3-(2-fluoro-6-hydroxyphenyl)-6,6a,7,8,9,10-hexahydro-12*H*-pyrazino[2,1-*c*]pyrido[3,4-*f*]oxazepin-12-one (Z304, 10.6 mg, 2-step yield of 29%). ES-API: [M+H]⁺=527.1.

### Embodiment 75 Synthesis of (6aR)-8-acryloyl-4-chloro-1-(1,3-dimethylpiperidin-4-yl)-3-(2-fluoro-6-hydroxyphenyl)-6,6a,7,8,9,10-hexahydro-12H-pyrazino[2,1-c]pyrido[3,4-f]oxazepin-12-one Z323

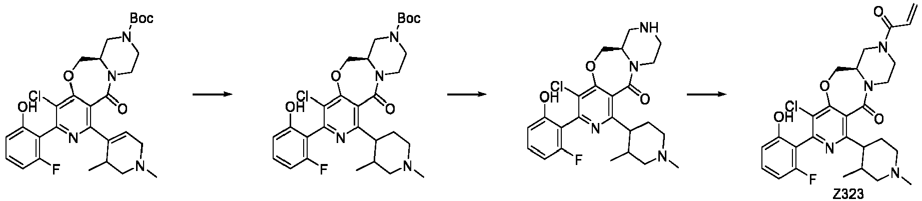

Step 1: Platinum dioxide (40 mg) was added to a solution of *tert*-butyl (6a*R*)-4-chloro-1-(1,3-dimethyl-1,2,3,6-tetrahydropyridin-4-yl)-3-(2-fluoro-6-hydroxyphenyl)-12-oxo-6a,7,9,10-tetrahydro-12*H-*pyrazino[2,1-*c*]pyrido[3,4-*f*][1,4]oxazepine-8(6*H*)-carboxylate (30 mg, 0.052 mmol) in methanol (40 mL). The reaction system was replaced with hydrogen for 3 times and stirred overnight at room temperature. The reaction solution was filtered through diatomite to remove the catalyst. The filtrate was concentrated and subjected to preparative separation to obtain a product of *tert*-butyl (6a*R*)-4-chloro-1-(1,3-dimethylpiperidin-4-yl)-3 -(2-fluoro-6-hydroxyphenyl)-12-oxo-6a,7,9,10-tetrahydro-12*H*-pyrazino [2,1-c]pyrido[3,4-*f*][1,4]oxazepine-8(6*H*)-carboxylate (10 mg, 33%). ES-API: [M+H]⁺=575.2.

Step 2: Trifluoroacetic acid (0.5 mL) was added to a solution of *tert*-butyl (6a*R*)-4-chloro-1-(1,3-dimethylpiperidin-4-yl)-3 -(2-fluoro-6-hydroxyphenyl)-12-oxo-6a,7,9,10-tetrahydro-12*H*-pyrazino [2,1-c]pyrido[3,4-*f*][1,4]oxazepine-8(6*H*)-carboxylate (10 mg, 0.017 mmol) in dichloromethane (2 mL). After stirring at room temperature for 2 hours, the reaction solution was concentrated to obtain a crude product of (6a*R*)-4-chloro-1-(1,3-dimethylpiperidin-4-yl)-3-(2-fluoro-6-methoxyphenyl)-6,6a,7,8,9,10-hexahydro-12*H*-pyrazinol[2,1-*c*]pyrido[3,4-*f*][1,4]oxazepin-12-one (10 mg, crude product), which was directly used in the next reaction step. ES-API: [M+H]⁺=475.2.

Step 3: The crude product of (6a*R*)-4-chloro-1-(1,3-dimethylpiperidin-4-yl)-3-(2-fluoro-6-methoxyphenyl)-6,6a,7,8,9,10-hexahydro-12*H*-pyrazino[2,1-*c*]pyrido[3,4-*f*][1,4]oxazepin-12-one (10 mg, crude product) obtained in step 2 was dissolved in dichloromethane (0.5 mL), and triethylamine (24 µL, 0.17 mmol) was added thereto. A solution of acryloyl chloride (0.77 mg, 0.009 mmol) in dichloromethane (0.1 mL) was added to the reaction solution at 0 to 5°C. The reaction was stirred at 0°C for 5 minutes. The reaction solution was added with 2 mL of dichloromethane, washed with 3 mL of saturated aqueous NaHCO₃ solution and 3 mL of saturated brine, and the organic phase was dried over anhydrous sodium sulfate and then concentrated. The crude product was purified by preparative HPLC to obtain (6a*R*)-8-acryloyl-4-chloro-1-(1,3-dimethylpiperidin-4-yl)-3-(2-fluoro-6-hydroxyphenyl)-6,6a,7,8,9,10-hexahydro-12*H-*pyrazino[2,1-*c*]pyrido[3,4-*f*]oxazepin-12-one (Z323, 2.4 mg, 2-step yield of 26.0%). ES-API: [M+H]⁺=529.2.

### Embodiment 76 Synthesis of (6aR)-8-acryloyl-4-chloro-3-(2-fluoro-6-hydroxyphenyl)-1-((2S,4S)-4-methoxy-2-methylpyrrolidin-1-yl)-6,6a,7,8,9,10-hexahydro-12H-pyrazino[2,1-c]pyrido[3,4--f][1,4]oxazepin-12-one Z260a

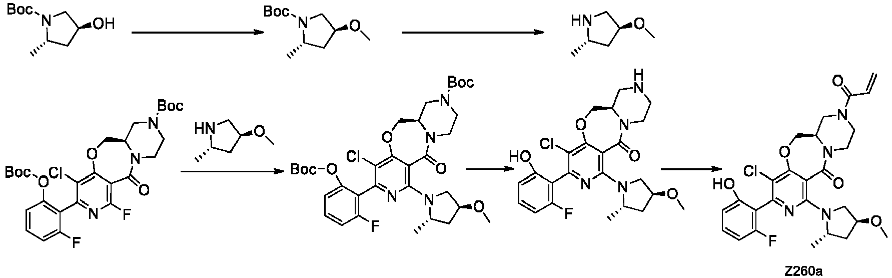

Step 1: After *tert*-butyl (2*S*,4*S*)-4-hydroxy-2-methylpyrrolidine-1-carboxylate (100 mg, 0.5 mmol) was dissolved in THF (1 mL), sodium hydride (24 mg, 1 mmol) was added thereto at 0°C, then iodomethane (105 mg, 0.6 mmol) was added thereto, reacted at 0°C for 2 hours, quenched by adding water, extracted with ethyl acetate, and the organic phase was washed with brine, dried over anhydrous sodium sulfate, evaporated to dryness by rotary evaporation to obtain *tert*-butyl (2*S*,4*S*)-4-methoxy-2-methylpyrrolidine-1-carboxylate (100 mg, yield of 93%). ES-API: [M+H]⁺=216.15.

Step 2: *tert*-Butyl (2*S*,4*S*)-4-methoxy-2-methylpyrrolidine-1-carboxylate (100 mg, 0.465 mmol) was dissolved in dichloromethane (2 mL), and then trifluoroacetic acid (2 mL) was added dropwise thereto, stirred at room temperature for 2 hours. After the reaction was completed, the mixture was evaporated to dryness by rotary evaporation to obtain (2*S*,4*S*)-4-methoxy-2-methylpyrrolidine (53 mg, yield of 100%). ES-API: [M+H]⁺=116.10.

Step 3: (2*S*,4*S*)-4-Methoxy-2-methylpyrrolidine (53 mg, 0.46 mmol) was dissolved in acetonitrile (2 mL), and then potassium carbonate (500 mg, 3.62 mmol) was added thereto to adjust the pH to alkaline, and then *tert*-butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-*c*]pyrido[3,4-*f*][1,4]oxazepine-8(6*H*)-carboxylate (170 mg, 0.29 mmol) was added thereto. The mixture was reacted at 80°C for 16 hours and then quenched by adding water. The mixture was extracted with ethyl acetate, and the organic phases were combined, washed with saturated brine, dried over anhydrous sodium sulfate, and concentrated to obtain a crude product of *tert-*butyl (6a*R*)-3-(2-((*tert*-butoxycarbonyl)oxy)-6-fluorophenyl)-4-chloro-1-((2*S*,4*S*)-4-methoxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (193 mg, yield of 100%). ES-API: [M+H]⁺=677.3.

Step 4: *tert*-Butyl (6a*R*)-3-(2-((*tert*-butoxycarbonyl)oxy)-6-fluorophenyl)-4-chloro-l-((2S,4S)-4-methoxy-2-methylpyrrolidin-1-yl)-12-oxo-6a,7,9,10-tetrahydro-12*H*-pyrazino[2,1-*c*]pyrido [3,4-*f*][1,4]oxazepine-8(6*H*)-carboxylate (193 mg, 0.28 mmol) was dissolved in dichloromethane (2 mL), and then trifluoroacetic acid (2 mL) was added dropwise thereto. The mixture was stirred at room temperature for 2 hours, and then evaporated to dryness by rotary evaporation to obtain (6a*R*)-4-chloro-3-(2-fluoro-6-hydroxyphenyl)-1-((2*S*,4*S*)-4-methoxy-2-methylpyrrolidin-1-yl)-6,6a,7,8,9,10-hexahydro-12*H-*pyrazino[2,1-*c*]pyrido[3,4-*f*][1,4]oxazepin-12-one (138 mg, yield of 100%). ES-API: [M+H]⁺=477.16.

Step 5: (6a*R*)-4-Chloro-3-(2-fluoro-6-hydroxyphenyl)-1-((2*S*,4*S*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one (138 mg, 0.29 mmol) was dissolved in dichloromethane (5 mL), and triethylamine (2 mL) was added dropwise thereto at 0°C to adjust the pH to alkaline, and then acryloyl chloride (20 mg, 0.22 mmol) was added thereto. The mixture was reacted in an ice-water bath for 10 minutes, then quenched with water, extracted with ethyl acetate, and the organic phase was washed with brine, dried over anhydrous sodium sulfate, concentrated, and the crude product was prepared to obtain (6a*R*)-8-acryloyl-4-chloro-3-(2-fluoro-6-hydroxyphenyl)-1-((2*S*,4*S*)-4-methoxy-2-methylpyrrolidin-1-yl)-6,6a,7,8,9,10-hexahydro-12*H*-pyrazino [2,1-*c*]pyrido [3,4--*f*][1,4]oxazepin-12-one (Z260, 53.4 mg, yield of 34.43%). ¹H NMR (400 MHz, CDCl₃) δ 9.39 (brs, 1H), 7.37-7.26 (m, 1H), 6.81 (d, *J* = 8.3 Hz, 1H), 6.68 (t, *J* = 8.9 Hz, 1H), 6.61-6.47 (m, 1H), 6.39 (d, *J* = 16.5 Hz, 1H), 5.81 (d, *J* = 10.5 Hz, 1H), 4.53-4.14 (m, 5H), 4.13-4.06 (m, 1H), 4.05-3.34 (m, , 7H), 3.32 (s, 3H), 2.30-2.13 (m, 1H), 1.92-1.84 (m, *J* = 12.9 Hz, 1H), 1.32 (d, *J* = 6.1 Hz, 3H). ES-API: [M+H]⁺=531.17.

### Embodiment 77 Synthesis of (6aR)-8-acryloyl-4-chloro-3-(2-fluoro-6-hydroxyphenyl)-1-((2S,4R)-4-methoxy-2-methylpyrrolidin-1-yl)-6,6a,7,8,9,10-hexahydro12H-pyrazino[2,1-c]pyrido[3,4--f][1,4]oxazepin-12-one Z259a

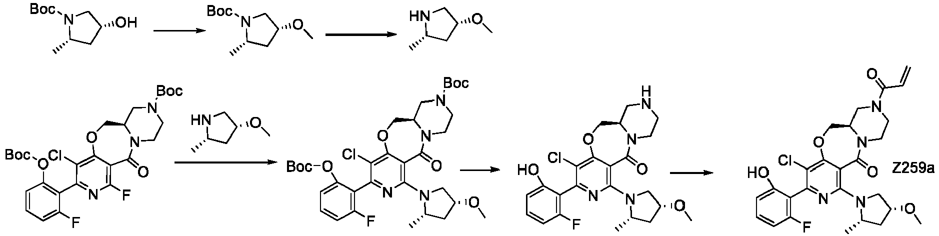

Step 1: *tert*-Butyl (2*S*,4*R*)-4-hydroxy-2-methylpyrrolidine-1-carboxylate (300 mg, 1.5 mmol) was dissolved in 3 mL of THF, and sodium hydride (72 mg, 1.79 mmol) was added thereto at 0°C, and then iodomethane (168µL, 1.8 mmol) was added thereto. The mixture was reacted at 0°C for 2 hours, then quenched by adding water and extracted with ethyl acetate. The organic phase was concentrated to obtain *tert*-butyl (2*S*,4*R*)-4-methoxy-2-methylpyrrolidine-1-carboxylate (280 mg, crude product).

Step 2: *tert*-Butyl (2*S*,4*R*)-4-methoxy-2-methylpyrrolidine-1-carboxylate (280 mg, 1.3 mmol) was dissolved in 2 mL of dichloromethane, and 2 mL of trifluoroacetic acid was added thereto, reacted at room temperature for 2 hours. The reaction solution was concentrated, then redissolved with dichloromethane and concentrated to obtain (2*S*,4*R*)-4-methoxy-2-methylpyrrolidine (150 mg, crude product).

Step 3: *tert*-Butyl (6a*R*)-3-(2-((*tert*-butoxycarbonyl)oxy)-6-fluorophenyl)-4-chloro-1-fluoro-12-oxo-6a,7,9,10-tetrahydro-12*H*-pyrazino[2,1-c]pyrido[3,4-*f*][1,4]oxazepine-8(6*H*)-carboxylate (250 mg, 0.43 mmol) was dissolved in 2 mL of acetonitrile, and (2*S*,4*R*)-4-methoxy-2-methylpyrrolidine (150 mg, 1.3 mmol) and potassium carbonate (190 mg, 1.38 mmol) were added thereto, reacted at 80°C for 2 hours. After the reaction was completed, the reaction solution was cooled to room temperature, extracted with ethyl acetate, washed with brine, and the organic phase was concentrated to obtain a crude product. The crude product was purified by flash silica gel column (PE/EtOAc=1/1, R_{f}=0.6) to obtain *tert*-butyl (6a*R*)-3-(2-((*tert*-butoxycarbonyl)oxy)-6-fluorophenyl)-4-chloro-1-((2*S*,4*R*)-4-methoxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 80%, pale yellow solid). ES-API: [M+H]⁺= 677.57.

Step 4: *tert*-Butyl (6a*R*)-3-(2-((*tert*-butoxycarbonyl)oxy)-6-fluorophenyl)-4-chloro-1-((2*S*,4*R*)-4-methoxy-2-methylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.34 mmol) was dissolved in 2 mL of dichloromethane, and 2 mL of trifluoroacetic acid was added thereto, reacted at room temperature for 2 hours. The reaction solution was concentrated and then redissolved with dichloromethane, and concentrated to obtain (6a*R*)-4-chloro-3-(2-fluoro-6-hydroxyphenyl)-1-((2*S*,4*R*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one (130 mg, crude product, yellow oily liquid). ES-API: [M+H]⁺=477.3.

Step 5: (6a*R*)-4-Chloro-3-(2-fluoro-6-hydroxyphenyl)-1-((2*S*,4*R*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one (100 mg, 0.29 mmol) was dissolved in 3 mL of dichloromethane, and then 1 mL of triethylamine was added, and acrylic anhydride (15 mg, 0.16 mmol) was added dropwise at 0°C. The reaction solution was stirred for 10 minutes, washed with brine, extracted with dichloromethane. The organic phase was concentrated, and the crude product was subjected to preparative HPLC (column: Ultimate XB-C18, 50^{∗}250 mm, 10 µm, mobile phase system: A: purified water B: pure acetonitrile, flow rate: 80 mL/min, gradient: within 50 minutes, B/A=20%-90%, wavelength: 214 nm, column temperature: room temperature) to obtain (6a*R*)-8-acryloyl-4-chloro-3-(2-fluoro-6-hydroxyphenyl)-1-((2*S*,4*R*)-4-methoxy-2-methylpyrrolidin-1-yl)-6,6a,7,8,9,10-hexahydro-12H-pyrazino[2,1-*c*]pyrido[3,4-*f*][1,4]oxazepin-12-one (Z259a, 21 mg, yield: 19%, white solid). ¹H NMR (400 MHz, CDCl₃) δ 8.76 (s, 1H),7.19(d, *J* = 8.0 Hz, 1H) 6.81 (d, *J* = 8.3 Hz, 1H), 6.69 (t, *J* = 9.1 Hz, 1H), 6.75-6.37 (m, 1H), 6.40 (d, *J* = 16.5 Hz, 1H), 5.82 (d, *J* = 10.4 Hz, 1H), 4.51 - 4.25 (m, 4H), 4.25 - 4.20 (m, 1H), 4.12-3.94 (m, 4H), 3.73-3.45 (m, 3H), 3.33 (s, 3H), 3.06 (d, *J* = 12.0 Hz, 1H), 2.09 (dt, *J* = 13.3, 5.6 Hz, 1H), 1.99 (d, *J* = 13.6 Hz, 1H), 1.40 (d, *J* = 6.3 Hz, 3H). ES-API: [M+H]⁺=531.2.

### Embodiment 78 Synthesis of Z336-1 and Z336-2

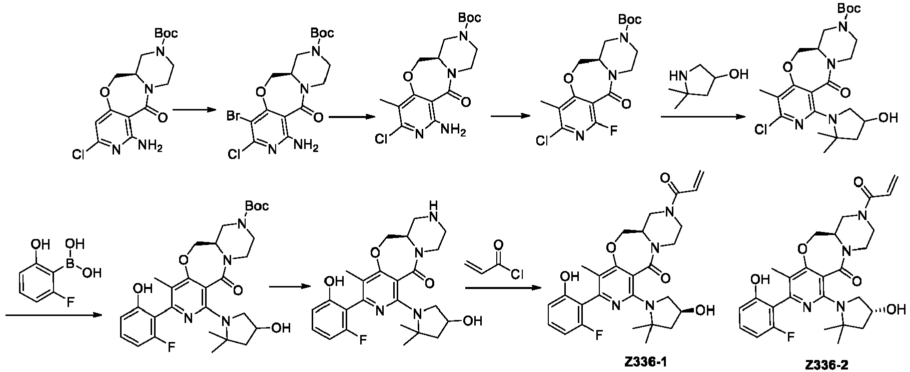

Step 1: (6a*R*)-1-Amino-3-chloro-8-*tert*-butoxycarbonyl-6,6a,7,8,9,10-hexahydro-12*H-*pyrazino[2,1-*c*]pyrido[3,4-*f*][1,4]oxazepin-12-one (1 g, 2.72 mmol), *N*-bromosuccinimide (580 mg, 3.26 mmol) and acetonitrile (30 mL) were added to a 100 mL flask. The reaction was stirred at 80°C for 0.5 hours. Aqueous sodium thiosulfate solution was added to the reaction solution and extracted with ethyl acetate. The organic phase was dried and concentrated. The crude product was purified by flash silica gel column (EtOAc/PE: 0-50%) to obtain (6a*R*)-1-amino-3-chloro-4-bromo-8-tert-butoxycarbonyl-6,6a,7,8,9,10-hexahydro-12*H*-pyrazino[2,1-*c*]pyrido[3,4-*f*][1,4]oxazepin-12-one (1.09 g, yield of 90%). ES-API: [M+H]⁺=447.0.

Step 2: (6a*R*)-1-Amino-3-chloro-4-bromo-8-*tert*-butoxycarbonyl-6,6a,7,8,9,10-hexahydro-12*H-*pyrazino[2,1-*c*]pyrido[3,4-*f*][1,4]oxazepin-12-one (1.09 g, 2.43 mmol), Pd(dppf)Cl₂ (177 mg, 0.243 mmol), trimethylboroxine (2 mL, 7.29 mmol, 50%wt. in THF), potassium carbonate (1 g, 7.29 mmol), 1,4-dioxane (30 mL) and water (3 mL) were added to a 100 mL flask. The reaction was stirred at 80°C for 1 hour. The mixture was extracted with ethyl acetate, and the organic phase was dried and concentrated. The crude product was purified by flash silica gel column (EtOAc/PE: 0-50%) to obtain (6a*R*)-1-amino-3-chloro-4-methyl-8-*tert*-butoxycarbonyl-6,6a,7,8,9,10-hexahydro-12*H*-pyrazino[2,1-*c*]pyrido[3,4-*f*][1,4]oxazepin-12-one (742 mg, yield of 80%). ES-API: [M+H]⁺=383.1.

Step 3: (6a*R*)-1-Amino-3-chloro-4-methyl-8-*tert*-butoxycarbonyl-6,6a,7,8,9,10-hexahydro-12*H-*pyrazino[2,1-*c*]pyrido[3,4-*f*][1,4]oxazepin-12-one (742 mg, 1.94 mmol) and a solution of 70% hydrofluoric acid in pyridine (2 mL) were added to a 100 mL round bottom plastic flask. Sodium nitrite (201 mg, 2.91 mmol) was slowly added thereto under an ice-water bath and stirred for 10 minutes, and the reaction was completed. A mixed solution of NaOH aqueous solution (4 g, 30 mL) and di-*tert*-butyl dicarbonate (838 mg, 3.88 mmol) in THF (30 mL) was prepared, and the above reaction solution was slowly added dropwise to the above mixed solution under an ice-water bath, and stirred for 20 minutes after the dropwise addition, and extracted with ethyl acetate. The mixture was washed with 1 M aqueous hydrochloric acid solution, saturated sodium bicarbonate and saturated brine in turn, and the organic phase was dried and then concentrated. The crude product was purified by flash silica gel column (EtOAc/PE: 0-30%) to obtain (6a*R*)-1-fluoro-3-chloro-4-methyl-8-*tert*-butoxycarbonyl-6,6a,7,8,9,10-hexahydro-12*H*-pyrazino[2,1-*c*]pyrido[3,4-*f*][1,4]oxazepin-12-one (520 mg, yield of 70%). ES-API: [M+H]⁺=386.1.

Step 4: (6a*R*)-1-Fluoro-3-chloro-4-methyl-8-*tert*-butoxycarbonyl-6,6a,7,8,9,10-hexahydro-12*H-*pyrazino[2,1-*c*]pyrido[3,4-*f*][1,4]oxazepin-12-one (520 mg, 1.35 mmol), 5,5-dimethylpyrrolidin-3-ol (170 mg, 1.48 mmol), diisopropylethylamine (522 mg, 4.05 mmol) and acetonitrile (10 mL) were added to a 100 mL flask. The reaction was stirred at 90°C for 2 hours. The mixture was extracted with ethyl acetate, washed with aqueous ammonium chloride solution and saturated brine in turn, and the organic phase was dried and then concentrated to obtain (6a*R*)-8-*tert*-butoxycarbonyl-1-(3-hydroxy-5,5-dimethylpyrrolidin-1-yl)-3-chloro-4-methyl-6,6a,7,8,9,10-hexahydro-12*H*-pyrazino[2,1-*c*]pyrido[3,4-*f*][1,4]oxazepin-12-one (584 mg, yield of 90%). ES-API: [M+H]⁺=481.2.

Step 5: (6a*R*)-8-*tert*-Butoxycarbonyl-1-(3-hydroxy-5,5-dimethylpyrrolidin-1-yl)-3-chloro-4-methyl-6,6a,7,8,9,10-hexahydro-12*H*-pyrazino[2,1-*c*]pyrido[3,4-*f*][1,4]oxazepin-12-one (584 mg, 1.21 mmol), 2-fluoro-6-hydroxyphenylboronic acid (566 mg, 3.63 mmol), Sphos (49 mg, 0.121 mmol), Sphos-Pd-G2 (87 mg, 0.121 mmol), potassium carbonate (501 mg, 3.63 mmol), 10 mL of dioxane and 2 mL of water were added to a 100 mL reaction flask. The reaction was stirred in an oil bath at 90°C for 1 hour, and the reaction was stopped. The reaction solution was added with 20 mL of water, extracted three times with 30 mL of ethyl acetate, and the organic phase was dried and then concentrated, and the crude product was purified by flash silica gel column (EtOAc/PE: 0-60%) to obtain (6a*R*)-8-*tert*-butoxycarbonyl-1-(3-hydroxy-5,5-dimethylpyrrolidin-1-yl)-3-(2-fluoro-6-hydroxyphenyl)-4-methyl-6,6a,7,8,9,10-hexahydro-12*H*-pyrazino[2,1-*c*]pyrido[3,4-*f*][1,4]oxazepin-12-one (538 mg, 3-step yield of 80%) as a yellow solid. ES-API: [M+H]⁺=557.2.

Step 6: (6a*R*)-8-*tert*-Butoxycarbonyl-1-(3-hydroxy-5,5-dimethylpyrrolidin-1-yl)-3-(2-fluoro-6-hydroxyphenyl)-4-methyl-6,6a,7,8,9,10-hexahydro-12*H*-pyrazino[2,1-*c*]pyrido[3,4-*f*][1,4]oxazepin-12-one (538 mg, 0.968 mmol), 3 mL of trifluoroacetic acid and 5 mL of dichloromethane were added to a 50 mL flask. The mixture was stirred at room temperature for 1 hour, and the completion of reaction was detected by LC-MS. The reaction solution was concentrated to obtain a crude product of (6a*R*)-1-(3-hydroxy-5,5-dimethylpyrrolidin-1-yl)-3-(2-fluoro-6-hydroxyphenyl)-4-methyl-6,6a,7,8,9,10-hexahydro-12*H-*pyrazino[2,1-*c*]pyrido[3,4-*f*][1,4]oxazepin-12-one (440 mg, crude product) as a yellow solid. ES-API: [M+H]⁺=457.2.

Step 7: (6a*R*)-1-(3-Hydroxy-5,5-dimethylpyrrolidin-1-yl)-3-(2-fluoro-6-hydroxyphenyl)-4-methyl-6,6a,7,8,9,10-hexahydro-12*H*-pyrazino[2,1-*c*]pyrido[3,4-*f*][1,4]oxazepin-12-one (440 mg, crude product), 5 mL of dichloromethane and diisopropylethylamine (622 mg, 4.82 mmol) were added to a 50 mL flask. At 0°C, a solution of acryloyl chloride in dichloromethane (87 mg, 0.96 mmol) was added dropwise thereto. The reaction was carried out at 0°C for 10 minutes. 20 mL of saturated sodium bicarbonate aqueous solution was added to the reaction solution, and the mixture was extracted three times with 10 mL of dichloromethane. The organic phase was dried and concentrated. The crude product was purified by preparative reversed-phase column HPLC (C18: 4.6 * 150 mm * 3.5 µm; mobile phase: acetonitrile:water : 0.1% TFA, 5%-95% acetonitrile, 10 min, flow rate: 1 mL/min; column temperature: 30°C) to obtain two isomer compounds.

One isomer with a structure arbitrarily designated as (6a*R*)-8-acryloyl-1-((*S*)-3-hydroxy-5,5-dimethylpyrrolidin-1-yl)-4-methyl-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z336-1, peak 1, retention time: 7.415 min, purity: 98%, de value: 100%, 100 mg, two-step yield of 20%). ¹H NMR (500 MHz, DMSO-*d₆*) δ 9.84 (s, 1H), 7.20 (q, *J* = 7.9 Hz, 1H), 6.86-6.64 (m, 3H), 6.17 (d, *J* = 16.7 Hz, 1H), 5.75 (d, *J* = 10.0 Hz, 1H), 4.50-3.86 (m, 8H), 3.70-3.17 (m, 4H), 2.77 (d, *J* = 11.1 Hz, 1H), 1.93-1.82 (m, 2H), 1.82 (s, 3H), 1.67 (d, *J* = 6.1 Hz, 3H), 1.40 (d, *J* = 4.5 Hz, 3H). ES-API: [M+H]⁺=511.2

Another isomer with a structure arbitrarily designated as (6a*R*)-8-acryloyl-1-((*R*)-3-hydroxy-5,5-dimethylpyrrolidin-1-yl)-4-methyl-3-(2-fluoro-6-hydroxyphenyl)-6,6a,7,8,9,10-hexahydro-12*H-*pyrazino[2,1-*c*]pyrido[3,4-*f*][1,4]oxazepin-12-one (Z336-2, peak 2, retention time: 7.966 min, purity: 95%, de value: 100%, 80 mg, two-step yield of 13%). ¹H NMR (500 MHz, DMSO-*d₆*) δ 9.83 (s, 1H), 7.20 (q, *J* = 7.8 Hz, 1H), 6.81-6.65 (m, 3H), 6.20-6.13 (m, 1H), 5.74 (s, 1H), 4.37 (d, *J* = 85.6 Hz, 7H), 3.53-3.12 (m, 5H), 3.03 (q, *J* = 8.1 Hz, 1H), 1.95-1.93 (m, 1H), 1.81 (s, 3H), 1.70 (t, *J* = 11.3 Hz, 1H), 1.51-1.37 (m, 6H). ES-API: [M+H]⁺=511.2.

List of diastereomer preparative separations:

| Number and structure (the following structures are all arbitrarily designated structures) | Name | Starting material, preparation condition, retention time, purity |
|---|---|---|
| 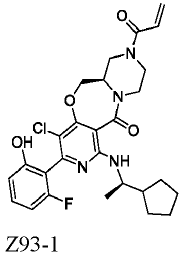 | (6a*R*)-8-Acryloyl-4-chloro-3-(2-fluoro-6-hydroxyphenyl)-1-((*S*)-4-hydroxy-2,2-dimethylpyrrolidin-1 -yl)-6,6a,7,8,9,10-hexahydro-12*H-*pyrazino[2,1-*c*]pyrido[3,4-*f*][1,4]oxazepin-12-one | Chiral separation via Z93. |
| | | Separation column: IE 250 mm*4.6 mm*5 µm; mobile phase: *n-*hexane:ethanol=80:20; |
| | | flow rate: 1 mL/min; column temperature: 30°C. |
| | | Peak 1, retention time: 21.72 min, purity: 100%, de value: 100% |
| 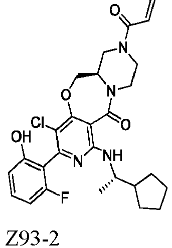 | (6a*R*)-8-Acryloyl-4-chloro-3-(2-fluoro-6-hydroxyphenyl)-1-((*R*)-4-hydroxy-2,2-dimethylpyrrolidin-1 -yl)-6,6a,7,8,9, 10-hexahydro-12H-pyrazino[2,1-*c*]pyrido[3,4-*f*][1,4]oxazepin-12-one | Chiral separation via Z93. |
| | | Separation column: IE 250 mm*4.6 mm*5 µm; mobile phase: *n-*hexane:ethanol=80:20; |
| | | flow rate: 1 mL/min; column temperature: 30°C. |
| | | Peak 2, retention time: 28.46 min, purity: 100%, de value: 100% |
| 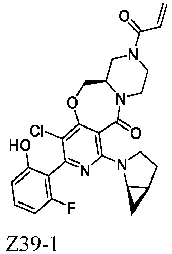 | (6aR)-8-Acryloyl-1-((1R,5S)-2-azabicyclo[3.1.0]hexan-2-yl)-4-chloro-3-(2-fluoro-6-hydroxyphenyl)-6,7-6a,7,8,9,10-hexa-12H-pyrazino[2,1-*c*]pyrido[3,4-*f*][1,4]oxazepin-12-one | Chiral separation via Z39 (76 mg). |
| | | Separation column: IA 150 mm*4.6 mm*5 µm; mobile phase: ethanol:n-hexane=60:40; |
| | | flow rate: 1 mL/min; column temperature: 30°C. |
| | | Peak 1, retention time: 4.178 min, purity: 100%, de value: 98.88%. |
| 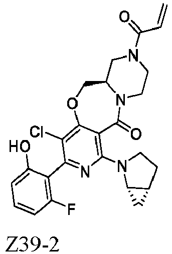 | (6a*R*)-8-Acryloyl-1-((1S,SR)-2-azabicyclo[3.1.0]hexan-2-yl)-4-chloro-3-(2-fluoro-6-hydroxyphenyl)-6,7-6a,7,8,9,10-hexa-12*H*-pyrazino[2,1-*c*]pyrido[3,4-*f*][1,4]oxazepin-12-one | Chiral separation via Z39 (76 mg). |
| | | Separation column: IA 150 mm*4.6 mm*5 µm; mobile phase: ethanol:n-hexane=60:40; |
| | | flow rate: 1 mL/min; column temperature: 30°C. |
| | | Peak 2, retention time: 5.558 min, purity: 98.38%, de value: 96.02%. |
| 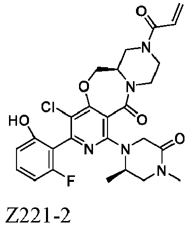 | (6a*R*)-8-Acryloyl-4-chloro-1-((*R*)-2,4-dimethyl-5-oxapiperazin-1-yl)-3-(2-fluoro-6-hydroxyphenyl)-6,6a,7,8,9,10-hexahydro-12*H*-pyrazino[2,1-c]pyrido [3,4-*f*][1,4]oxazepin- 12-one | Chiral separation via Z221. |
| | | Separation column: IA 250 mm*4.6 mm*5 µm; mobile phase: ethanol:n-hexane=40:60; |
| | | flow rate: 1 mL/min; column temperature: 30°C. |
| | | Peak 1, retention time: 4.73 min, purity: 100%, de value: 100% |
| 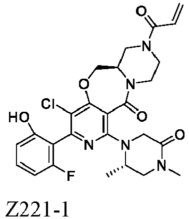 | (6a*R*)-8-Acryloyl-4-chloro-1-((S)-2,4-dimethyl-5-oxapiperazin-1-yl)-3-(2-fluoro-6-hydroxyphenyl)-6,6a,7,8,9,10-hexahydro-12*H*-pyrazino[2,1-c]pyrido[3,4-*f*][1,4]oxazepin-12-one | Chiral separation via Z221. |
| | | Separation column: IA 250 mm*4.6 mm*5 µm; mobile phase: ethanol:n-hexane=40:60; |
| | | flow rate: 1 mL/min; column temperature: 30°C. |
| | | Peak 2, retention time: 6.64 min, purity: 100%, de value: 100% |
| 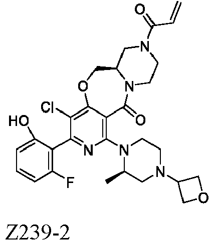 | (6a*R*)-8-Acryloyl-4-chloro-3-(2-fluoro-6-hydroxyphenyl)-1-((*R*)-2-methyl-4-(oxetan-3-yl)piperazin-1-yl)-6,6a,7,8,9,10-hexahydro-12*H*-pyrazino[2,1-*c*]pyrido[3,4-*f*][1,4]oxazepin-12-one | Chiral separation via Z239. |
| | | Separation column: IA 250 mm*4.6 mm*5 µm; mobile phase: ethanol:*n-*hexane=50:50; |
| | | flow rate: 1 mL/min; column temperature: 30°C. |
| | | Peak 1, retention time: 4.33 min, purity: 100%, de value: 100% |
| 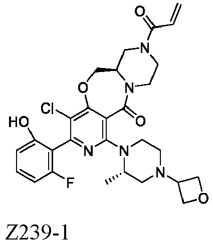 | (6a*R*)-8-Acryloyl-4-chloro-3-(2-fluoro-6-hydroxyphenyl)-1-((*S*)-2-methyl-4-(oxetan-3-yl)piperazin-1-yl)-6,6a,7,8,9,10-hexahydro-12*H*-pyrazino[2,1-c]pyrido[3,4-*f*][1,4]oxazepin-12-one | Chiral separation via Z239. |
| | | Separation column: IA 250 mm*4.6 mm*5 µm; mobile phase: ethanol:*n-*hexane=50:50; |
| | | flow rate: 1 mL/min; column temperature: 30°C. |
| | | Peak 2, retention time: 5.98 min, purity: 100%, de value: 100% |
| 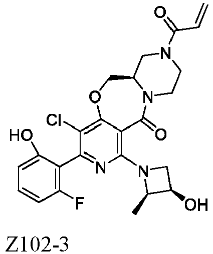 | (6a*R*)-8-Acryloyl-4-chloro-3-(2-fluoro-6-hydroxyphenyl)-1-((2*R*,3*R*)-3-hydroxy-2-methylazetidin-1-yl)-6,6a,7,8,9,10-hexahydro-12*H-*pyrazino[2,1-*c*]pyrido[3,4-*f*][1,4]oxazepin-12-one | Chiral separation via Z102a: |
| | | Separation column: IC 250 mm*4.6 mm*5 µm; mobile phase: ethanol:*n-*hexane=40:60; |
| | | flow rate: 1 mL/min; column temperature: 30°C. |
| | | Peak 1, retention time: 9.517 min, purity: 100%; de value: 100% |
| 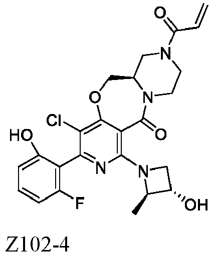 | (6a*R*)-8-Acryloyl-4-chloro-3-(2-fluoro-6-hydroxyphenyl)-1-((2*R*,3*S*)-3-hydroxy-2-methylazetidin-1-yl)-6,6a,7,8,9,10-hexahydro-12*H-*pyrazino[2,1-*c*]pyrido[3,4-*f*][1,4] oxazepin-12-one | Chiral separation via Z102a: |
| | | Separation column: IC 250 mm*4.6 mm*5 µm; mobile phase: ethanol:*n-*hexane=40:60; |
| | | flow rate: 1 mL/min; column temperature: 30°C. |
| | | Peak 2, retention time: 10.914 min, purity: 100%; de value: 97.8% |
| 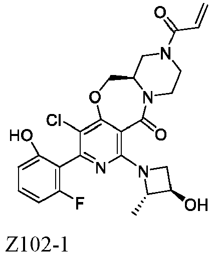 | (6a*R*)-8-Acryloyl-4-chloro-3-(2-fluoro-6-hydroxyphenyl)-1 - ((2*S*,3*R*)-3-hydroxy-2-methylazetidin-1-yl)-6,6a,7,8,9,10-hexahydro-12*H-*pyrazino[2,1-*c*]pyrido[3,4-*f*][1,4]oxazepin-12-one | Chiral separation via Z102a: |
| | | Separation column: IC 250 mm*4.6 mm*5 µm; mobile phase: ethanol:*n-*hexane=40:60; |
| | | flow rate: 1 mL/min; column temperature: 30°C. |
| | | Peak 3, retention time: 14.521 min, purity: 98%; de value: 100% |
| 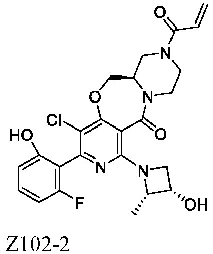 | (6a*R*)-8-Acryloyl-4-chloro-3-(2-fluoro-6-hydroxyphenyl)-1 - ((2*S*,3*S*)-3 -hydroxy -2-methylazetidin-1-yl)-6,6a,7,8,9,10-hexahydro-12*H-*pyrazino[2,1-*c*]pyrido[3,4-*f*][1,4]oxazepin-12-one | Chiral separation via Z102a: |
| | | Separation column: IC 250 mm*4.6 mm*5 µm; mobile phase: ethanol:*n-*hexane=40:60; |
| | | flow rate: 1 mL/min; column temperature: 30°C. |
| | | Peak 4, retention time: 21.336 min, purity: 96.52%; de value: 96.62% |
| 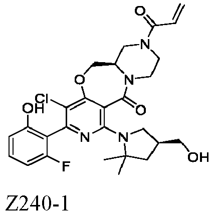 | (6a*R*)-8-Acryloyl-4-chloro-3-(2-fluoro-6-hydroxyphenyl)-1-((*S*)-4-(hydroxymethyl)-2,2-dimethylpyrrolidin-1 -yl)-6,6a,7,8,9, 10-hexahydro-12*H-*pyrazino[2,1-*c*]pyrido[3,4-*f*][1,4]oxazepin-12-one | Chiral separation via Z240: |
| | | Separation column: IC 250 mm*4.6 mm*5 µm; mobile phase: *n*-hexane:ethanol = 50:50; |
| | | flow rate: 1 mL/min; column temperature: 30°C; |
| | | Peak 1, retention time: 7.346 min, purity: 100%; de value: 100%. |
| 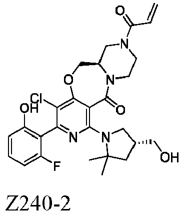 | (6a*R*)-8-Acryloyl-4-chloro-3-(2-fluoro-6-hydroxyphenyl)-1-((*R*)-4-(hydroxymethyl)-2,2-dimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z240: |
| | | Separation column: IC 250 mm*4.6 mm*5 µm; mobile phase: *n*-hexane:ethanol = 50:50; |
| | | flow rate: 1 mL/min; column temperature 30°C; |
| | | Peak 2, retention time: 9.846 min, purity: 100%; de value: 97.68%. |
| 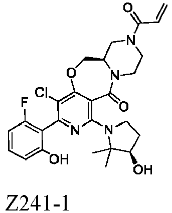 | (6a*R*)-8-Acryloyl-4-chloro-3-(2-fluoro-6-hydroxyphenyl)-1-((*S*)-4-hydroxy-2,2-dimethylpyrrolidin-1 -yl)-6,6a,7,8,9, 10-hexahydro-12*H-*pyrazino[2,1-*c*]pyrido[3,4-*f*][1,4]oxazepin-12-one | Chiral separation via Z241. |
| | | Separation column: IE 250 mm*4.6 mm*5 µm; mobile phase: *n-*hexane:ethanol=70:30; |
| | | flow rate: 1 mL/min; column temperature: 30°C. |
| | | Peak 1, retention time: 10.11 min, purity: 100%, de value: 100% |
| 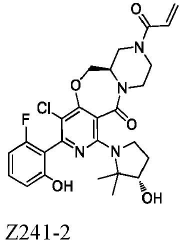 | (6a*R*)-8-Acryloyl-4-chloro-3-(2-fluoro-6-hydroxyphenyl)-1-((*R*)-4-hydroxy-2,2-dimethylpyrrolidin-1 -yl)-6,6a,7,8,9, 10-hexahydro-12*H-*pyrazino[2,1-*c*]pyrido[3,4-*f*][1,4]oxazepin-12-one | Chiral separation via Z241. |
| | | Separation column: IE 250 mm*4.6 mm*5 µm; mobile phase: *n-*hexane:ethanol=70:30; |
| | | flow rate: 1 mL/min; column temperature: 30°C. |
| | | Peak 2, retention time: 12.14 min, purity: 100%, de value: 100% |
| 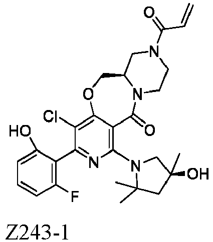 | (6aR)-8-Acryloyl-4-chloro-3-(2-fluoro-6-hydroxyphenyl)-1-((*S*)-4-hydroxy-2,2,4-trimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z243: |
| | | Separation column: IE 250 mm*4.6 mm*5 µm; mobile phase: ethanol:*n-*hexane=50:50; |
| | | flow rate: 1 mL/min; column temperature: 30°C. |
| | | Peak 1, retention time: 5.000 min, purity: 100%; de value: 100% |
| 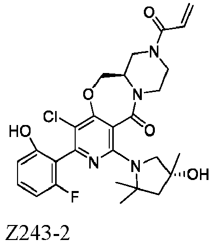 | (6a*R*)-8-Acryloyl-4-chloro-3-(2-fluoro-6-hydroxyphenyl)-1-((*R*)-4-hydroxy-2,2,4-trimethylpyrrolidin-1-yl)-6,6a,7,8,9,10-hexahydro-12*H-*pyrazino[2,1-*c*]pyrido [3,4-*f*][1,4]oxazepin-12-one | Chiral separation via Z243: |
| | | Separation column: IE 250 mm*4.6 mm*5 µm; mobile phase: ethanol:*n-*hexane=50:50; |
| | | flow rate: 1 mL/min; column temperature: 30°C. |
| | | Peak 2, retention time: 6.495 min, purity: 100%; de value: 97.04% |
| 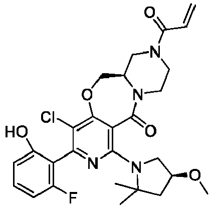 | (6a*R*)-8-Acryloyl-4-chloro-3-(2-fluoro-6-hydroxyphenyl)-1-((*S*)-4-methoxy-2,2-dimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z244: |
| | | Separation column: IA 150 mm*4.6 mm*5 µm; mobile phase: ethanol:*n-*hexane=30:70; |
| | | flow rate: 1 mL/min; column temperature: 30°C. |
| 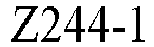 | | Peak 1, retention time: 5.755 min, purity: 99%; de value: 100% |
| 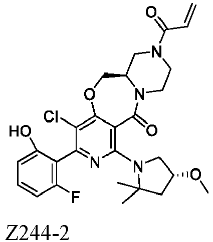 | (6a*R*)-8-Acryloyl-4-chloro-3-(2-fluoro-6-hydroxyphenyl)-1-((*R*)-4-methoxy-2,2-dimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z244: |
| | | Separation column: IA 150 mm*4.6 mm*5 µm; mobile phase: ethanol:*n-*hexane=30:70; |
| | | flow rate: 1 mL/min; column temperature: 30°C. |
| | | Peak 2, retention time: 8.992 min, purity: 99%; de value: 94.66% |
| 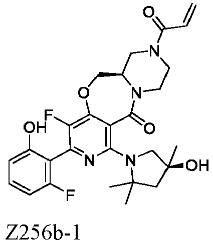 | (6a*R*)-8-Acryloyl-4-fluoro-3-(2-fluoro-6-hydroxyphenyl)-1-((*S*)-4-hydroxy-2,2,4-trimethylpyrrolidin-1-yl)-6,6a,7,8,9,10-hexahydro-12*H-*pyrazino[2,1-*c*]pyrido[3,4-*f*][1,4] oxazepin-12-one | Chiral separation via Z256b: |
| | | Separation column: IE 250 mm*4.6 mm*5 µm; mobile phase: ethanol:*n-*hexane=50:50; |
| | | flow rate: 1 mL/min; column temperature: 30°C. |
| | | Peak 1, retention time: 6.091 min, purity: 100%, de value: 100% |
| 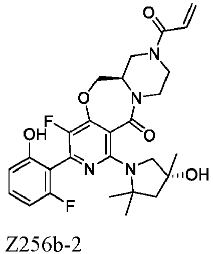 | (6a*R*)-8-Acryloyl-4-fluoro-3-(2-fluoro-6-hydroxyphenyl)-1-((*R*)-4-hydroxy-2,2,4-trimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z256b: |
| | | Separation column: IE 250 mm*4.6 mm*5 µm; mobile phase: ethanol:*n-*hexane=50:50; |
| | | flow rate: 1 mL/min; column temperature: 30°C. |
| | | Peak 2, retention time: 7.024 min, purity: 100%; de value: 100% |
| 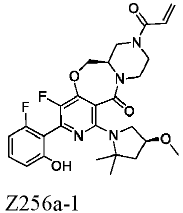 | (6a*R*)-8-Acryloyl-1-((*S*)-4-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one | Chiral separation via Z256a. |
| | | Separation column: IE 250 mm*4.6 mm*5 µm; mobile phase: *n-*hexane:ethanol=50:50; |
| | | flow rate: 1 mL/min; column temperature: 30°C. |
| | | Peak 1, retention time: 8.515 min, purity: 100%, de value: 100% |
| 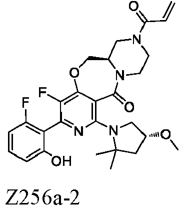 | (6a*R*)-8-Acryloyl-1-((*R*)-4-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-c]pyrido[3,4-*f*][1,4]oxazepin-12-one | Chiral separation via Z256a. |
| | | Separation column: IE 250 mm*4.6 mm*5 µm; mobile phase: *n-*hexane:ethanol=50:50; |
| | | flow rate: 1 mL/min; column temperature: 30°C. |
| | | Peak 2, retention time: 10.719 min, purity: 100%, de value: 100% |
| 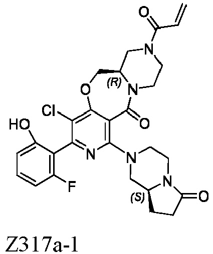 | (6a*R*)-8-Acryloyl-4-chloro-3-(2-fluoro-6-hydroxyphenyl)-1-((*S*)-6-oxohexahydropyrrolo [1,2-a]pyrazin-2(1*H*)-yl)-6,6a,7,8,9,10-hexahydro-12*H-*pyrazino[2,1-*c*]pyrido[3,4-*f*][1,4]oxazepin-12-one | Chiral separation via Z317a. |
| | | Separation column: IB 250 mm*4.6 mm*5 µm; mobile phase: ethanol:*n-*hexane=45:55; |
| | | flow rate: 1 mL/min; column temperature: 30°C. |
| | | Peak 1, retention time: 13.923 min, purity: 100%, de value: 100% |
| 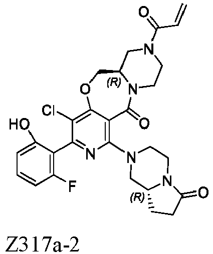 | (6a*R*)-8-Acryloyl-4-chloro-3-(2-fluoro-6-hydroxyphenyl)-1-((*R*)-6-oxohexahydropyrrolo [1,2-*a*]pyrazin-2(1*H*)-yl)-6,6a,7,8,9,10-hexahydro-12*H-*pyrazino[2,1-*c*]pyrido[3,4-*f*][1,4]oxazepin-12-one | Chiral separation via Z317a. |
| | | Separation column: IB 250 mm*4.6 mm*5 µm; mobile phase: ethanol:*n-*hexane=45:55; |
| | | flow rate: 1 mL/min; column temperature: 30°C. |
| | | Peak 2, retention time: 18.626 min, purity: 100%, de value: 100% |
| 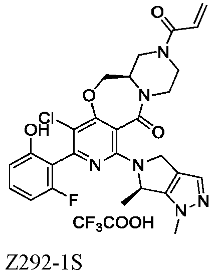 | (6a*R*)-8-Acryloyl-1-((*R*)-1,6-dimethylpyrrolo [3,4-*c*]pyrazol-5(1*H*,4*H*,6*H*-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one trifluoroacetate | Chiral separation via a mixture of Z291-2 and Z292-2. |
| | | Separation column: IE 250 mm*4.6 mm*5 µm; |
| | | mobile phase: *n-*hexane:ethanol:TFA=50:50:2; |
| | | flow rate: 1 mL/min; column temperature: 30°C. |
| | | Peak 1, retention time: 8.15 min, purity: 100%, de value: 100% |
| 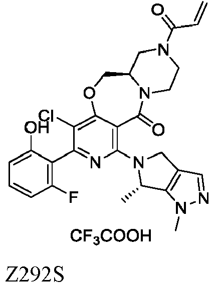 | (6a*R*)-8-Acryloyl-1-((*S*)-1,6-dimethylpyrrolo [3,4-*c*]pyrazol-5(1*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one trifluoroacetate | Chiral separation via a mixture of Z291-2 and Z292-2. |
| | | Separation column: IE 250 mm*4.6 mm*5 µm; |
| | | mobile phase: *n-*hexane:ethanol:TFA=50:50:2; |
| | | flow rate: 1 mL/min; column temperature: 30°C. |
| | | Peak 2, retention time: 15.53min, purity: 100%, de value: 94.8% |
| 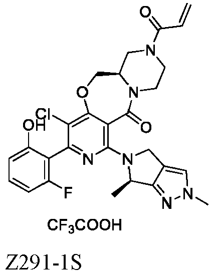 | (6aR)-8-Acryloyl-1-((R)-2,6-dimethylpyrrolo [3,4-c]pyrazol-5(2H,4H,6H)-yl)-4-chloro-3-(2-fluoro-6-hydroxyphenyl)-6,6a,7,8,9,10-hexahydro-12H-pyrazino[2,1-*c*]pyrido[3,4-*f*][1,4]oxazepin-12-one trifluoroacetate | Chiral separation via a mixture of Z291-2 and Z292-2. |
| | | Separation column: IE 250 mm*4.6 mm*5 µm; |
| | | mobile phase: *n-*hexane:ethanol:TFA=50:50:2; |
| | | flow rate: 1 mL/min; column temperature: 30°C. |
| | | Peak 3, retention time: 20.59 min, purity: 100%, de value: 100% |
| 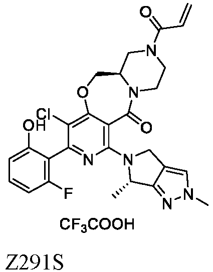 | (6a*R*)-8-Acryloyl-1-((*S*)-2,6-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one trifluoroacetate | Chiral separation via a mixture of Z291-2 and Z292-2. |
| | | Separation column: IE 250 mm*4.6 mm*5 µm; |
| | | mobile phase: *n-*hexane:ethanol:TFA=50:50:2; |
| | | flow rate: 1 mL/min; column temperature: 30°C. |
| | | Peak 4, retention time: 30.66 min, purity: 100%, de value: 98% |
| 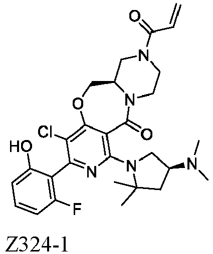 | (6a*R*)-8-Acryloyl-4-chloro-1-((*S*)-4-(dimethylamino)-2,2-dimethylpyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Preparative separation by HPLC via Z324. |
| | | Chromatographic column: Xbridge C18 19*150 mm*5 µm; |
| | | preparation method: acetonitrile:water (0.1% ammonium bicarbonate) |
| | | Peak 1, retention time: 1.58 min |
| 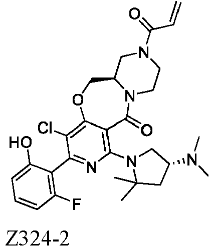 | (6a*R*)-8-Acryloyl-4-chloro-1-((*R*)-4-(dimethylamino)-2,2-dimethylpyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Preparative separation by HPLC via Z324. |
| | | Chromatographic column: Xbridge C18 19*150 mm*5 µm; |
| | | preparation method: acetonitrile:water (0.1% ammonium bicarbonate) |
| | | Peak 2, retention time: 1.62min |
| 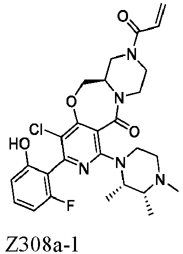 | (6a*R*)-8-Acryloyl-4-chloro-3-(2-fluoro-6-hydroxyphenyl)-1 - ((2*R*,3*S*)-2,3,4-trimethylpiperazin-1 -yl)-6,6a,7,8,9,10-hexahydro-12*H-*pyrazino[2,1-*c*]pyrido[3,4-*f*][1,4]oxazepin-12-one | Chiral separation via Z308c. |
| | | Separation column: IF 250 mm*4.6 mm*5 µm; mobile phase: |
| | | trifluoroacetic acid:ethanol:n-hexane=2:30:70; |
| | | flow rate: 1 mL/min; column temperature: 30°C. |
| | | Peak 1, retention time: 15.513 min, purity: 100%, de value: 100% |
| 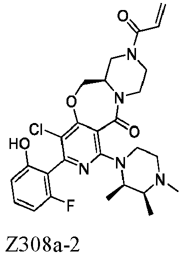 | (6a*R*)-8-Acryloyl-4-chloro-3-(2-fluoro-6-hydroxyphenyl)-1-((2*S*,3*R*)-2,3,4-trimethylpiperazin-1-yl)-6,6a,7,8,9,10-hexahydro-12*H-*pyrazino[2,1-*c*]pyrido[3,4-*f*][1,4]oxazepin-12-one | Chiral separation via Z308c. |
| | | Separation column: IF 250 mm*4.6 mm*5 µm; |
| | | mobile phase: trifluoroacetic acid:ethanol:n-hexane=2:30:70; |
| | | flow rate: 1 mL/min; column temperature: 30°C. |
| | | Peak 2, retention time: 22.608 min, purity: 100%, de value: 100% |
| 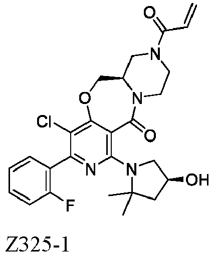 | (*R*)-8-Acryloyl-4-chloro-3-(2-fluorophenyl)-1-((*S*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z325. |
| | | Separation column: IG 250 mm*4.6 mm*5 µm; |
| | | mobile phase: ethanol:n-hexane=40:60; |
| | | flow rate: 1 mL/min; column temperature: 30°C. |
| | | Peak 1, retention time: 7.635 min, purity: 100%, de value: 100% |
| 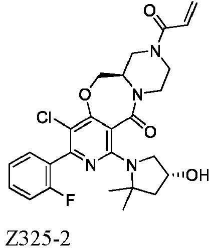 | (*R*)-8-Acryloyl-4-chloro-3-(2-fluorophenyl)-1-((*R*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one | Chiral separation via Z325. |
| | | Separation column: IG 250 mm*4.6 mm*5 µm; |
| | | mobile phase: ethanol:n-hexane=40:60; |
| | | flow rate: 1 mL/min; column temperature: 30°C. |
| | | Peak 2, retention time: 10.783 min, purity: 100%, de value: 100% |
| 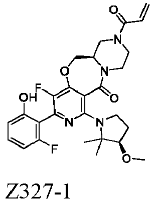 | (6a*R*)-8-Acryloyl-1-((*R*)-3-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-c]pyrido[3,4-*f*][1,4]oxazepin-12-one | Chiral separation via Z327. |
| | | Separation column: IE 250 mm*4.6 mm*5 µm; |
| | | mobile phase: *n-*hexane:ethanol=70:30; |
| | | flow rate: 1 mL/min; column temperature: 30°C. |
| | | Peak 1, retention time: 16.69 min, purity: 100%, de value: 98.2% |
| 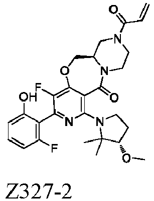 | (6a*R*)-8-Acryloyl-1-((*S*)-3-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one | Chiral separation via Z327. |
| | | Separation column: IE 250 mm*4.6 mm*5 µm; |
| | | mobile phase: *n-*hexane:ethanol=70:30; |
| | | flow rate: 1 mL/min; column temperature: 30°C. |
| | | Peak 2, retention time: 19.48 min, purity: 100%, de value: 99.6% |
| 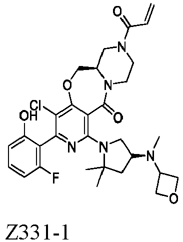 | (6a*R*)-8-Acryloyl-4-chloro-1-((*R*)-2,2-dimethyl-4-(methyl(oxetan-3-yl)amino)pyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via Z326. |
| | | Separation column: IB 250 mm*4.6 mm*5 µm; |
| | | mobile phase: ethanol:n-hexane=70:30; |
| | | flow rate: 1 mL/min; column temperature: 30°C. |
| | | Peak 1, retention time: 6.126 min, purity: 100%, de value: 100% |
| 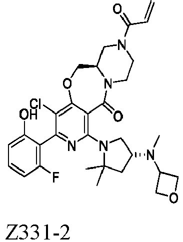 | (6a*R*)-8-Acryloyl-4-chloro-1-((*S*)-2,2-dimethyl-4-(methyl(oxetan-3-yl)amino)pyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via Z326. |
| | | Separation column: IB 250 mm*4.6 mm*5 µm; |
| | | mobile phase: ethanol:n-hexane=70:30; |
| | | flow rate: 1 mL/min; column temperature: 30°C. |
| | | Peak 2, retention time: 12.632 min, purity: 100%, de value: 100% |
| 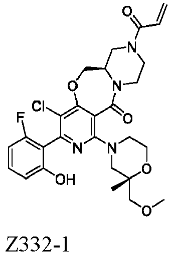 | (6a*R*)-8-Acryloyl-4-chloro-3-(2-fluoro-6-hydroxyphenyl)-1-((*S*)-2-(methoxymethyl)-2-methylmorpholino)-6,6a,7,8,9,10-hexahydro-12*H*-pyrazino[2,1-*c*]pyrido[3,4-*f*][1,4]oxazepin-12-one | Chiral separation via Z332. |
| | | Separation column: IG 250 mm*4.6 mm*5 µm; |
| | | mobile phase: ethanol:n-hexane=40:60; |
| | | flow rate: 1 mL/min; column temperature 30°C. |
| | | Peak 1, retention time: 10.124 min, purity: 100%, de value: 100% |
| 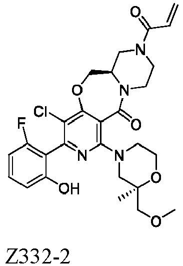 | (6a*R*)-8-Acryloyl-4-chloro-3-(2-fluoro-6-hydroxyphenyl)-1-((*R*)-2-(methoxymethyl)-2-methylmorpholino)-6,6a,7,8,9,10-hexahydro-12*H*-pyrazino[2,1-c]pyrido[3,4-*f*][1,4]oxazepin-12-one | Chiral separation via Z332. |
| | | Separation column: IG 250 mm*4.6 mm*5 µm; |
| | | mobile phase: ethanol:n-hexane=40:60; |
| | | flow rate: 1 mL/min; column temperature: 30°C. |
| | | Peak 2, retention time: 13.474 min, purity: 100%, de value: 100% |
| 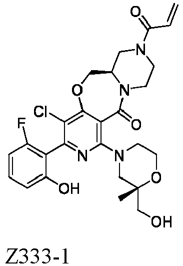 | (6a*R*)-8-Acryloyl-4-chloro-3-(2-fluoro-6-hydroxyphenyl)-1-((*S*)-2-(hydroxymethyl)-2-methylmorpholino)-6,6a,7,8,9,10-hexahydro-12*H*-pyrazino[2,1-*c*]pyrido[3,4-*f*][1,4]oxazepin-12-one | Chiral separation via Z333. |
| | | Separation column: IB 250 mm*4.6 mm*5 µm; |
| | | mobile phase: ethanol:n-hexane=35 :65; |
| | | flow rate: 1 mL/min; |
| | | column temperature: 30°C. |
| | | Peak 1, retention time: 8.078min, purity: 100%, de value: 100% |
| 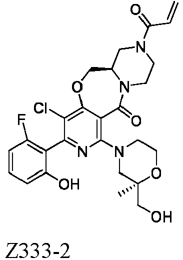 | (6a*R*)-8-Acryloyl-4-chloro-3-(2-fluoro-6-hydroxyphenyl)-1-((*R*)-2-(hydroxymethyl)-2-methylmorpholino)-6,6a,7,8,9,10-hexahydro-12*H*-pyrazino[2,1-*c*]pyrido[3,4-*f*][1,4]oxazepin-12-one | Chiral separation via Z333. |
| | | Separation column: IB 250 mm^{∗}4.6 mm^{∗}5 µm; |
| | | mobile phase: ethanol:n-hexane=35 :65; |
| | | flow rate: 1 mL/min; column temperature: 30°C. |
| | | Peak 2, retention time: 10.226 min, purity: 100%, de value: 100% |
| 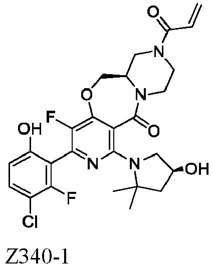 | (6a*R*)-8-Acryloyl-1-((*S*)-3-hydroxy-5,5-dimethylpyrrolidin-1-yl)-4-fluoro-3-(3-chloro-2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z340 and Z341. |
| | | Separation column: IE 250 mm^{∗}4.6 mm^{∗}5 µm; |
| | | mobile phase: *n-*hexane:ethanol=70:30; |
| | | flow rate: 1 mL/min; column temperature: 30°C. |
| | | Peak 1, retention time: 8.177 min, purity: 100%, de value: 100% |
| 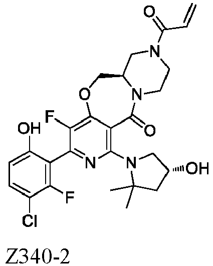 | (*6*a*R*)*-*8*-*Acryloyl-1-((*R*)-3-hydroxy-5,5-dimethylpyrrolidin-1-yl)-4-fluoro-3-(3-chloro-2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z340 and Z341. |
| | | Separation column: IE 250 mm^{∗}4.6 mm^{∗}5 µm; |
| | | mobile phase: *n-*hexane:ethanol=70:30; |
| | | flow rate: 1 mL/min; column temperature: 30°C. |
| | | Peak 2, retention time: 7.365min, purity: 100%, de value: 94.8% |
| 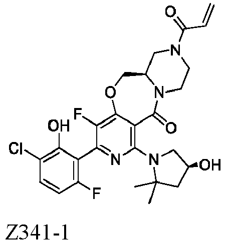 | (6a*R*)-8-Acryloyl-1-((*S*)-3-hydroxy-5,5-dimethylpyrrolidin-1-yl)-4-fluoro-3-(3-chloro-6-fluoro-2-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z340 and Z341. |
| | | Separation column: IE 250 mm^{∗}4.6 mm^{∗}5 µm; |
| | | mobile phase: *n-*hexane:ethanol=70:30; |
| | | flow rate: 1 mL/min; column temperature: 30°C. |
| | | Peak 3, retention time: 20.59 min, |
| | | purity: 89%, de value: 100% |
| 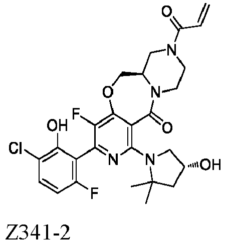 | (6a*R*)-8-Acryloyl-1-((*R*)-3-hydroxy-5,5-dimethylpyrrolidin-1-yl)-4-fluoro-3-(3-chloro-6-fluoro-2-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z340 and Z341. |
| | | Separation column: IE 250 mm^{∗}4.6 mm^{∗}5 µm; |
| | | mobile phase: *n-*hexane:ethanol=70:30; |
| | | flow rate: 1 mL/min; column temperature: 30°C. |
| | | Peak 4, retention time: 10.94 min, purity: 99%, de value: 100% |
| 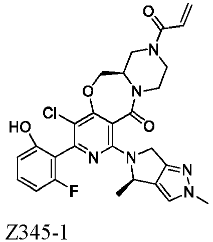 | (6a*R*)-8-Acryloyl-1-((*R*)-2,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z345 and Z346. |
| | | Separation column: IE 250 mm^{∗}4.6 mm^{∗}5 µm; |
| | | mobile phase: *n-*hexane:ethanol=40:60; |
| | | flow rate: 1 mL/min; column temperature: 30°C. |
| | | Peak 1, retention time: 5.822 min, purity: 92%, de value: 100% |
| 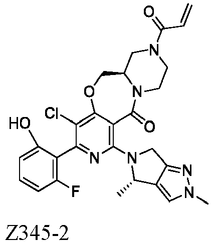 | (6a*R*)-8-Acryloyl-1-((*S*)-2,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z345 and Z346. |
| | | Separation column: IE 250 mm^{∗}4.6 mm^{∗}5 µm; |
| | | mobile phase: *n-*hexane :ethanol=40: 60; |
| | | flow rate: 1 mL/min; column temperature: 30°C. |
| | | Peak 2, retention time: 8.872 min, purity: 92%, de value: 98% |
| 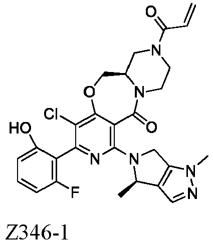 | (*6*a*R*)-8-Acryloyl-1-((*R*)-1,4-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z345 and Z346. |
| | | Separation column: IE 250 mm^{∗}4.6 mm^{∗}5 µm; |
| | | mobile phase: *n-*hexane :ethanol=40: 60; |
| | | flow rate: 1 mL/min; column |
| | | temperature: 30°C. |
| | | Peak 3, retention time: 6.389 min, purity: 98%, de value: 100% |
| 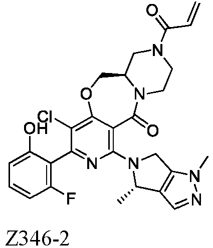 | (6a*R*)-8-Acryloyl-1-((*S*)-1,4-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Chiral separation via a mixture of Z345 and Z346. |
| | | Separation column: IE 250 mm^{∗}4.6 mm^{∗}5 µm; |
| | | mobile phase: *n-*hexane :ethanol=40:60; |
| | | flow rate: 1 mL/min; column temperature: 30°C. |
| | | Peak 4, retention time: 7.017 min, purity: 100%, de value: 97.7% |
| 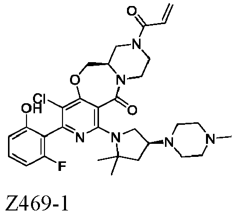 | (6a*R*)-8-Acryloyl-4-chloro-1-((*S*)-2,2-dimethyl-4-(4-methylpiperazin-1-yl)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one | Chiral separation via Z467. |
| | | Separation column: AB 250 mm^{∗}4.6 mm^{∗}5 µm; mobile phase: *n-*hexane:ethanol:diethylamine = 60:40:2; |
| | | flow rate: 1 mL/min; column temperature: 30°C. |
| | | Peak 1, retention time: 7.022 min, purity: 100%, de value: 100% |
| 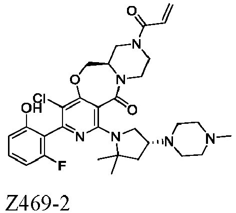 | (6a*R*)-8-Acryloyl-4-chloro-1-((*R*)-2,2-dimethyl-4-(4-methylpiperazin-1-yl)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one | Chiral separation via Z467. |
| | | Separation column: AB 250 mm^{∗}4.6 mm^{∗}5 µm; mobile phase: *n-*hexane:ethanol:diethylamine = 60:40:2; |
| | | flow rate: 1 mL/min; column temperature: 30°C. |
| | | Peak 2, retention time: 8.266 min, purity: 100%, de value: 100% |
| 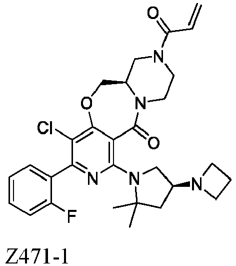 | (*R*)-8-Acryloyl-1-((*S*)-4-(azetidin-1-yl)-2,2-dimethylpyrrolidin-1-yl)-4-chloro-3-(2-fluorophenyl)-6,6a,7,8,9,10-hexahydro-12*H-*pyrazino[2,1-*c*]pyrido[3,4-*f*][1,4]oxazepin-12-one | Prepared via preparative chromatographic column via Z471. |
| | | Separation column: Xbridge C18 19^{∗}150 mm 5µm |
| | | Mobile phase: CH₃CN:H₂O (0.1% NH₄HCO₃); gradient: 45%-70% |
| | | Peak 1, retention time: 6.12 min, purity: 100% |
| 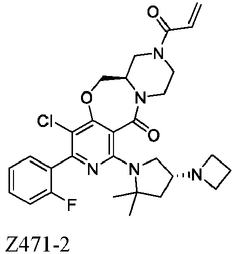 | (*R*)-8-Acryloyl-1-((*R*)-4-(azetidin-1-yl)-2,2-dimethylpyrrolidin-1-yl)-4-chloro-3-(2-fluorophenyl)-6,6a,7,8,9,10-hexahydro-12*H-*pyrazino[2,1-*c*]pyrido[3,4-*f*][1,4]oxazepin-12-one | Prepared via preparative chromatographic column via Z471. |
| | | Separation column: Xbridge C18 19*150 mm 5µm |
| | | Mobile phase: |
| | | CH₃CN:H₂O (0.1% NH₄HCO₃); gradient: 45%-70% |
| | | Peak 2, retention time: 8.63 min, purity: 100% |
| 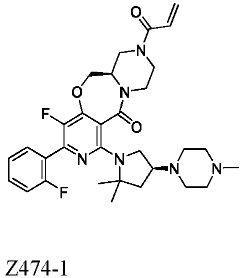 | (*R*)-8-Acryloyl-1-((*S*)-2,2-dimethyl-4-(4-methylpiperazin-1-yl)pyrrolidin-1-yl)-4-fluoro-3-(2-fluorophenyl)-6,6a,7,8,9,10-hexahydro-12*H*-pyrazino[2,1-*c*]pyrido[3,4-*f*][1,4]oxazepin-12-one | Chiral separation via Z474. |
| | | Separation column: IC 250 mm^{∗}4.6 mm^{∗}5 µm; mobile phase: *n-*hexane:ethanol:diethanolamine=60:40 :0.2; |
| | | flow rate: 1 mL/min; column temperature: 30°C. |
| | | Peak 1, retention time: 10.222 min, purity: 97.2%, de value: 100% |
| 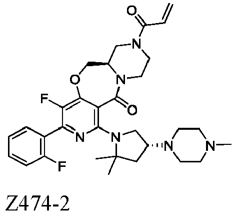 | (*R*)-8-Acryloyl-1-((*R*)-2,2-dimethyl-4-(4-methylpiperazin-1-yl)pyrrolidin-1-yl)-4-fluoro-3-(2-fluorophenyl)-6,6a,7,8,9,10-hexahydro-12*H*-pyrazino[2,1-*c*]pyrido[3,4-*f*][1,4]oxazepin-12-one | Chiral separation via Z474. |
| | | Separation column: IC 250 mm^{∗}4.6 mm^{∗}5 µm; mobile phase: *n-*hexane:ethanol:diethanolamine=60:40 :0.2; |
| | | flow rate: 1 mL/min; column temperature: 30°C. |
| | | Peak 2, retention time: 12.954 min, purity: 97.2%, de value: 95% |
| 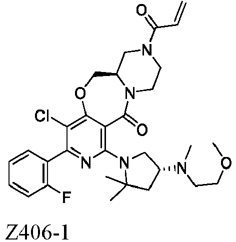 | (*R*)-8-Acryloyl-4-chloro-3-(2-fluorophenyl)-1-((*R*)-4-((2-methoxyethyl)(methyl)amino)-2,2-dimethylpyrrolidin-1-yl)-6,6a,7,8,9,10-hexahydro-12*H-*pyrazino[2,1-*c*]pyrido[3,4-*f*][1,4]oxazepin-12-one | Separated by preparative HPLC via Z406. |
| | | Separation column: IC mobile phase: *n-*hexane:ethanol:diethylamine=60:40:2; |
| | | flow rate: 1 mL/min; column temperature: 30°C. |
| | | Peak 1, retention time: 6.960 min, purity: 100%, de value: 100% |
| 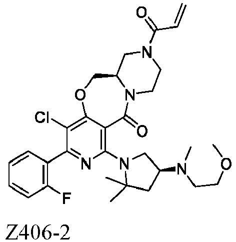 | (*R*)-8-Acryloyl-4-chloro-3-(2-fluorophenyl)-1-((*S*)-4-((2-methoxyethyl)(methyl)amino)-2,2-dimethylpyrrolidin-1-yl)-6,6a,7,8,9,10-hexahydro-12*H-*pyrazino[2,1-*c*]pyrido[3,4-*f*][1,4]oxazepin-12-one | Separated by preparative HPLC via Z406. |
| | | Separation column: IC mobile phase: *n-*hexane:ethanol:diethylamine=60:40:2; |
| | | flow rate: 1 mL/min; column temperature: 30°C. |
| | | Peak 2, retention time: 8.122 min, purity: 100%, de value: 100% |
| 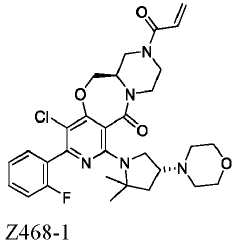 | (*R*)-8-Acryloyl-4-chloro-1-((*R*)-2,2-dimethyl-4-morpholinopyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one | Separated by preparative HPLC via Z468. |
| | | Separation column: IC mobile phase: *n-*hexane:ethanol:diethylamine=60:40:2; |
| | | flow rate: 1 mL/min; column temperature: 30°C. |
| | | Peak 1, retention time: 10.113 min, purity: 100%, de value: 100% |
| 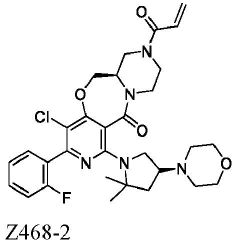 | (*R*)-8-Acryloyl-4-chloro-1-((*S*)-2,2-dimethyl-4-morpholinopyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one | Separated by preparative HPLC via Z468. |
| | | Separation column: IC mobile phase: *n-*hexane:ethanol:diethylamine=60:40:2; |
| | | flow rate: 1 mL/min; column temperature: 30°C. |
| | | Peak 2, retention time: 14.139 min, purity: 100%, de value: 100% |
| 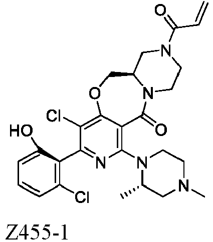 | (3*R*,6a*R*)-8-Acryloyl-4-chloro-3-(2-chloro-6-hydroxyphenyl)-1-((*S*)-2,4-dimethylpiperazin-1-yl)-6,6a,7,8,9,10-hexahydro-12*H-*pyrazino[2,1-*c*]pyrido[3,4-*f*][1,4]oxazepin-12-one | Chiral separation via Z452. |
| | | Separation column: IG 250 mm^{∗}4.6 mm^{∗}5 µm; |
| | | mobile phase: *n-*hexane :ethanol=60:40; |
| | | flow rate: 1 mL/min; column temperature: 30°C. |
| | | peak 1, retention time: 4.521 min, purity: 98%, de value: 80.42% |
| 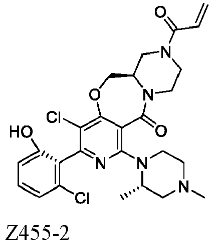 | (3*S*,6a*R*)-8-Acryloyl-4-chloro-3-(2-chloro-6-hydroxyphenyl)-1-((*S*)-2,4-dimethylpiperazin-1-yl)-6,6a,7,8,9,10-hexahydro-12*H-*pyrazino[2,1-*c*]pyrido[3,4-*f*][1,4]oxazepin-12-one | Chiral separation via Z452. |
| | | Separation column: IG 250 mm^{∗}4.6 mm^{∗}5 µm; |
| | | mobile phase: *n-*hexane :ethanol=60:40; |
| | | flow rate: 1 mL/min; column temperature: 30°C. |
| | | Peak 2, retention time: 7.543 min, purity: 98%, de value: 79.34% |
| 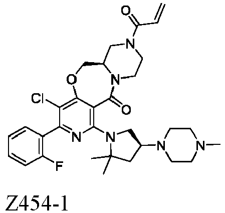 | (*R*)-8-Acryloyl-4-chloro-1-((*S*)-2,2-dimethyl-4-(4-methylpiperazin-1-yl)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one | Chiral separation via Z454. |
| | | Separation column: IC 250 mm^{∗}4.6 mm^{∗}5 µm; |
| | | mobile phase: *n-*hexane:ethanol:diethanolamine=60:40 :0.2; |
| | | flow rate: 1 mL/min; column temperature: 30°C. |
| | | Peak 1, retention time: 7.688 min, purity: 96.33%, de value: 100% |
| 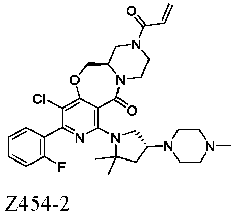 | (*R*)-8-Acryloyl-4-chloro-1-((*R*)-2,2-dimethyl-4-(4-methylpiperazin-1-yl)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one | Chiral separation via Z454. |
| | | Separation column: IC 250 mm^{∗}4.6 mm^{∗}5 µm; |
| | | mobile phase: *n-*hexane:ethanol:diethanolamine=60:40 :0.2; |
| | | flow rate: 1 mL/min; column temperature: 30°C. |
| | | Peak 2, retention time: 10.153 min, purity: 99.38%, de value: 100% |
| 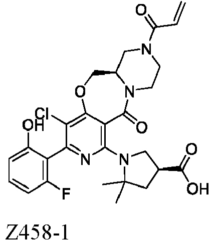 | (3*S*)-1-((6a*R*)-8-Acryloyl-4-chloro-3-(2-fluoro-6-hydroxyphenyl)-12-oxo-6,6a,7,8,9,10-hexahydro-12*H-*pyrazino[2,1-*c*]pyrido[3,4-*f*][1,4]oxazepin-1-yl)-5,5-dimethylpyrrolidine-3-carboxylic acid | Chiral separation via Z458. |
| | | Separation column: IC 250 mm^{∗}4.6 mm^{∗}5 µm; |
| | | mobile phase: *n-*hexane :ethanol=60:40; |
| | | flow rate: 1 mL/min; column temperature: 30°C. |
| | | Peak 1, retention time: 6.838 min, purity: 100%, de value: 100% |
| 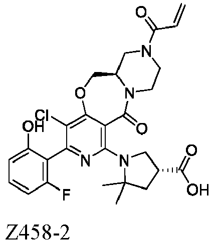 | (3*R*)-1-((6a*R*)-8-Acryloyl-4-chloro-3-(2-fluoro-6-hydroxyphenyl)-12-oxo-6,6a,7,8,9,10-hexahydro-12*H-*pyrazino[2,1-*c*]pyrido[3,4-*f*][1,4]oxazepin-1-yl)-5,5-dimethylpyrrolidine-3-carboxylic acid | Chiral separation via Z458. |
| | | Separation column: IC 250 mm^{∗}4.6 mm^{∗}5 µm; |
| | | mobile phase: *n-*hexane :ethanol=60:40; |
| | | flow rate: 1 mL/min; column temperature: 30°C. |
| | | Peak 2, retention time: 12.443 min, purity: 100%, de value: 100% |
| 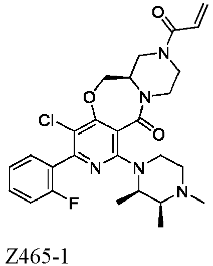 | (*R*)-8-Acryloyl-4-chloro-3-(2-fluorophenyl)-1-((2*R*,3*S*)-2,3,4-trimethylpiperazin-1-yl)-6,6a,7,8,9,10-hexahydro-12*H-*pyrazino[2,1-*c*]pyrido[3,4-*f*][1,4]oxazepin-12-one | Chiral separation via Z465. |
| | | Separation column: IC 250 mm^{∗}4.6 mm^{∗}5 µm; |
| | | mobile phase: *n-*hexane :ethanol:trifluoroacetic acid=60:40:0.2; |
| | | flow rate: 1 mL/min; column temperature: 30°C. |
| | | Peak 1, retention time: 3.815 min, purity: 100%, de value: 100% |
| 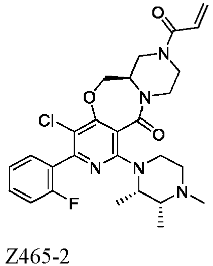 | (*R*)-8-Acryloyl-4-chloro-3-(2-fluorophenyl)-1-((2*S*,3*R*)-2,3,4-trimethylpiperazin-1-yl)-6,6a,7,8,9,10-hexahydro-12*H-*pyrazino[2,1-*c*]pyrido[3,4-*f*][1,4]oxazepin-12-one | Chiral separation via Z465. |
| | | Separation column: IC 250 mm^{∗}4.6 mm^{∗}5 µm; |
| | | mobile phase: *n-*hexane :ethanol:trifluoroacetic acid=60:40:0.2; |
| | | flow rate: 1 mL/min; column temperature: 30°C. |
| | | Peak 2, retention time: 5.834 min, purity: 98%, de value: 100% |
| 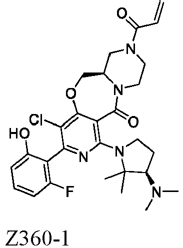 | (6a*R*)-8-Acryloyl-4-chloro-1-((*R*)-3-(dimethylamino)-2,2-dimethylpyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Purified by preparative thin-layer chromatographic column (dichloromethane/methanol= 10/1, R_{f}=0.4) via Z360 |
| | | Peak 1 |
| 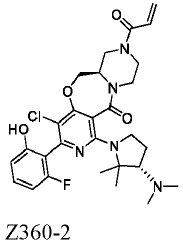 | (6a*R*)-8-Acryloyl-4-chloro-1-((*S*)-3-(dimethylamino)-2,2-dimethylpyrrolidin-1-yl)-3-(2-fluoro-6-hydroxyphenyl)-6,6a,7,8,9,10-hexahydro-12*H-*pyrazino[2,1-*c*]pyrido[3,4-*f*][1,4]oxazepin-12-one | Purified by preparative thin-layer chromatographic column (dichloromethane/methanol= 10/1, R_{f}=0.3) via Z360, peak 2 |
| 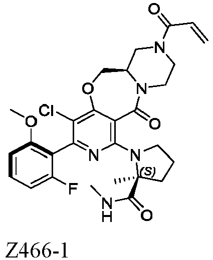 | (2*S*,3*R*)-1-((6a*R*)-8-Acryloyl-4-chloro-3-(2-fluoro-6-methoxyphenyl)-12-oxo-6,6a,7,8,9,10-hexahydro-12*H-*pyrazino[2,1-*c*]pyrido[3,4-*f*][1,4]oxazepin-1-yl)-*N*,2-dimethylpyrrolidine-2-carboxamide | Separated by preparative HPLC via Z466. |
| | | Separation column: Welch Xtimate C18 150 mm^{∗}21.2 mm, 5 µm; |
| | | **1]** mobile phase: acetonitrile:water NH₄HCO₃) = 35%-95%; |
| | | **2]** flow rate: 15 mL/min; peak 1, retention time: 1.44 min, purity: 97%, de value: 100% |
| 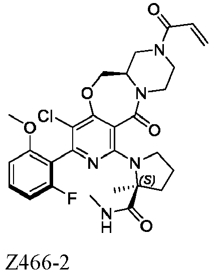 | (2*S*,3*S*)-1-((6a*R*)-8-Acryloyl-4-chloro-3-(2-fluoro-6-methoxyphenyl)-12-oxo-6,6a,7,8,9,10-hexahydro-12*H-*pyrazino[2,1-*c*]pyrido[3,4-*f*][1,4]oxazepin-1-yl)-*N*,2-dimethylpyrrolidine-2-carboxamide | Separated by preparative HPLC via Z466. |
| | | Separation column: Welch Xtimate C18 150 mm^{∗}21.2 mm, 5 µm; |
| | | mobile phase: acetonitrile:water (0.1% NH₄HCO₃) = 35%-95%; |
| | | flow rate: 15 mL/min; |
| | | peak 2, retention time: 1.47 min, purity: 99%, de value: 100% |

NMR and mass spectrometry of chiral resolved compounds:

| No. | NMR, mass spectrometry | Yield |
|---|---|---|
| Z93 | ¹H NMR (500 MHz, DMSO-*d*₆) δ 9.94-9.86 (m, 1*H*), 7.33-7.08 (m, 2H), 6.76-6.68 (m, 3H), 6.16 (t, *J* = 17.0 Hz, 1H), 5.77-5.69 (m, 1H), 4.45-4.27 (m, 2H), 4.12 (s, 1H), 3.98-3.86 (m, 5H), 3.70-3.60 (m 2H), 1.88 (q, *J* = 8.3 Hz, 1H), 1.65 (s, 2H), 1.52-1.46 (m, 4H), 1.24-1.21 (m, 2H), 1.09 (d, *J* = 6.3 Hz, 3H). ES-API: [M+H]⁺ = 529.2 | 6 mg |
| Z93-2 | ES-API: [M+H]⁺ = 529.2 | 2 mg |
| Z39-1 | ¹H NMR (500 MHz, CDCl₃) δ 7.26-7.24 (m, 1H), 6.82-6.80 (m, 1H), 6.68-6.66 (m, 1H), 6.57-6.56 (m, 1H), 6.43-6.40 (m, 1H), 5.84-5.82 (m, 1H), 4.41-4.03 (m, 6H), 3.63-3.49 (m, 5H), 3.26-3.25 (m, 1H), 2.21-2.00 (m, 2H), 1.66-1.65 (m, 1H), 0.67-0.53 (m, 2H). ES-API: [M+H]⁺ = 499.1 | 18 mg |
| Z39-2 | ¹H NMR (500 MHz, CDCl₃) δ 7.47-7.31 (m, 1H), 6.84-6.83 (m, 1H), 6.70-6.68 (m, 1H), 6.57-6.56 (m, 1H), 6.42-6.40 (m, 1H), 5.84-5.82 (m, 1H), 4.40-4.05 (m, 7H), 3.78-3.45 (m, 4H), 2.72-2.71 (m, 1H), 2.41-2.40 (m, 1H), 2.03-2.00 (m, 1H), 1.72-1.71 (m, 1H), 0.88-0.86 (m, 1H), 0.46-0.45 (m, 1H). ES-API: [M+H]⁺ = 499.1 | 20 mg |
| Z221-2 | ¹H NMR (500 MHz, DMSO-*d*₆) δ 10.09-9.92 (m, 1H), 7.30-7.17 (m, 1H), 6.85-6.64 (m, 3H), 6.26-6.07 (m, 1H), 5.83-5.67 (m, 1H), 4.88-3.36 (m, 14H), 2.85 (s, *J* = 10.6 Hz, 3H), 1.14 (t, *J* = 6.8 Hz, 3H). ES-API: [M+H]⁺= 544.2. | 5.2 mg |
| Z221-1 | ¹H NMR (500 MHz, DMSO-*d*₆) δ 10.12-9.82 (m, 1H), 7.25 (dd, *J* = 15.2, 7.5 Hz, 1H), 6.95-6.56 (m, 3H), 6.18 (d, *J* = 17.6 Hz, 1H), 5.75 (s, 1H), 4.70-4.55 (m, 1H), 4.55-4.41 (m, 1H), 4.41-4.29 (m, 1H), 4.29-4.15 (m, 1H), 4.15-4.00 (m, 3H), 3.99-3.42 (m, 7H), 2.84 (s, 3H), 1.12 (t, *J* = 6.7 Hz, 3H). ES-API: [M+H]⁺= 544.2. | 4.0 mg |
| Z239-2 | ¹H NMR (500 MHz, DMSO-*d*₆) δ 9.98 (s, 1H), 7.25 (dd, *J* = 15.5, 8.0 Hz, 1H), 6.85-6.63 (m, 3H), 6.17 (d, *J* = 16.5 Hz, 1H), 5.74 (t, *J* = 10.5 Hz, 1H), 4.65-4.50 (m, 3H), 4.49-4.27 (m, 4H), 4.26-3.95 (m, 4H), 3.94-3.73(m, 1H), 3.68-3.38 (m, 3H), 3.20 (t, *J =* 12.0 Hz, 1H), 3.12 (d, *J* = 10.5 Hz, 1H), 2.67 (d, *J* = 10.0 Hz, 1H), 2.54 (s, 1H), 2.02 (d, *J* = 8.5 Hz, 1H), 1.81 (t, *J* = 10.0 Hz, 1H), 1.14 (d, *J* = 6.0 Hz, 3H). ES-API: [M+H]⁺ = 572.2. | 19 mg |
| Z239-1 | ¹H NMR (500 MHz, DMSO-*d*₆) δ 10.04-9.23 (m, 1H), 7.28-7.21 (m, 1H), 6.80-6.65 (m, 3H), 6.17 (d*, J* = 16.5 Hz, 1H), 5.78-5.69 (m, 1H), 4.56-4.49 (m, 2H), 4.48-4.26 (m, 4H), 4.25-3.72 (m, 6H), 3.71-3.39 (m, 4H), 3.23 (t, *J =* 12.0 Hz, 1H), 2.60 (d, *J =* 9.5 Hz, 1H), 2.46 (d, *J* = 11.0 Hz, 1H), 2.03-1.86 (m, 2H), 1.27 (d, *J* = 6.5 Hz, 3H). ES-API: [M+H]⁺ = 572.2. | 45 mg |
| Z102-3 | ES-API: [M+H]⁺ =503.0 | 7 mg |
| Z102-4 | ES-API: [M+H]⁺ =503.1 | 8.5 mg |
| Z102-1 | ES-API: [M+H]⁺ =503.1 | 38 mg |
| Z102-2 | ES-API: [M+H]⁺ =503.1. | 4 mg |
| Z240-1 | ¹H NMR (500 MHz, DMSO-*d*₆) δ 9.95 (d, *J* = 5.2 Hz, 1H), 7.23 (dd, *J* = 15.6, 7.7 Hz, 1H), 6.85-6.61 (m, 3H), 6.16 (d, *J* = 16.9 Hz, 1H), 5.74 (s, 1H), 4.62-4.52 (m, 2H), 4.47-4.05 (m, 4H), 3.95-3.75 (m, 1H), 3.69-3.55 (m, 4H), 3.22 (t, *J=* 10.1 Hz, 2H), 2.85 (s, 1H), 2.03-1.85 (m, 3H), 1.48 (d, *J* = 7.5 Hz, 3H), 1.37 (d, *J* = 2.5Hz 3H). ES-API: [M+H]⁺ = 545.0. | 31 mg |
| Z240-2 | ¹H NMR (500 MHz, DMSO-*d*₆) δ 9.96 (d, *J* = 7.3 Hz, 1H), 7.23 (dd, *J* = 15.5, 7.5 Hz, 1H), 6.78-6.66 (m, 3H), 6.16 (d, *J* = 16.6 Hz, 1H), 5.73 (s, 1H), 4.52 (s, 2H), 4.34-4.01 (m, 4H), 3.72 (s, 1H), 3.54 (s, 3H), 3.48-3.38 (m, 4H), 2.87 (s, 1H), 1.96 (s, 2H), 1.50 (d, *J* = 3.8 Hz, 3H), 1.29 (d, *J* = 6.9 Hz, 3H). ES-API: [M+H]⁺ = 545.0. | 32 mg |
| Z241-1 | ¹H NMR (500 MHz, DMSO-*d*₆) δ 9.96-9.92 (m, 1H), 7.27-7.19 (m, 1H), 6.81-6.64 (m, 3H), 6.17 (d, *J* = 16.7 Hz, 1H), 5.74 (s, 1H), 4.90 (d, *J* = 3.6 Hz, 1H), 4.51 (s, 1H), 4.25 (s, 1H), 4.16-4.09 (m, 4H), 3.73 (d, *J* = 3.8 Hz, 1H), 3.71-3.63 (m, 1H), 3.52-3.39 (m, 2H), 3.28 (d, *J* = 7.8 Hz, 1H), 2.84 (t, *J* = 8.2 Hz, 1H), 2.07 (d, *J* = 11.5 Hz, 1H), 1.70 (dd, *J* = 12.5, 6.4 Hz, 1H), 1.43-1.34 (m, 6H). ES-API: [M+H]⁺ =531.1 | 70 mg |
| Z241-2 | ¹H NMR (500 MHz, DMSO-*d*₆) δ 9.95 (dd, *J* = 3.4, 1.7 Hz, 1H), 7.27-7.20 (m, 1H), 6.82-6.64 (m, 3H), 6.16 (d, *J* = 17.0 Hz, 1H), 5.73 (s, 1H), 5.17 (s, 1H), 4.52 (s, 1H), 4.31-3.89 (m, 6H), 3.72 (t, *J* = 9.0 Hz, 1H), 3.39 (dd, *J* = 10.7, 6.9 Hz, 2H), 3.17 (s, 1H), 2.82 (d, *J* = 8.5 Hz, 1H), 1.94 (dt, *J* = 12.0, 6.4 Hz, 1H), 1.80-1.70 (m, 1H), 1.41 (d, *J* = 2.9 Hz, 3H), 1.25 (d, *J* = 8.0 Hz, 3H). ES-API: [M+H]⁺ =531.1 | 50 mg |
| Z243-1 | ES-API: [M+H]⁺ =545.1 | 4 mg |
| Z243-2 | ES-API: [M+H]⁺ =545.1 | 10 mg |
| Z244-1 | ES-API: [M+H]⁺ =545.1 | 41 mg |
| Z244-2 | ES-API: [M+H]⁺ =545.2 | 26 mg |
| Z256b-1 | ¹H NMR (500 MHz, DMSO-*d*₆) δ 10.06 (s, 1H), 7.26 (dd, *J* = 15.2, 8.2 Hz, 1H), 6.86-6.64 (m, 3H), 6.17 (d, *J* = 16.3 Hz, 1H), 5.74 (s, 1H), 4.84 (s, 1H), 4.54 (s, 1H), 4.39-3.69 (m, 5H), 3.61-3.42 (m, 3H), 3.25-3.10 (m, 1H), 2.74 (d, *J =* 10.0 Hz, 1H), 2.06 (d, *J* = 12.5 Hz, 1H), 1.82 (d, *J =* 12.3 Hz, 1H), 1.59 (s, 3H), 1.43 (s, 3H), 1.30 (d, *J* = 7.3 Hz, 3H). ES-API: [M+H]⁺ = 529.2. | 33 mg |
| Z256b-2 | ¹H NMR (500 MHz, DMSO-*d*₆) δ 10.05 (s, 1H), 7.26 (dd, *J* = 15.3, 8.2 Hz, 1H), 6.86-6.58 (m, 3H), 6.17 (d, *J* = 17.1 Hz, 1H), 5.75 (d, *J* = 10.4 Hz, 1H), 4.59 (s, 1H), 4.51 (s, 1H), 4.37-3.99 (m, 4H), 3.83-3.71 (m, 1H), 3.63-3.39 (m, 3H), 3.29-3.22 (m, 1H), 2.73 (d*, J* = 10.0 Hz, 1H), 1.90-1.79 (m, 2H), 1.71 (s, 3H), 1.39 (s, 3H), 1.26 (s, 3H). ES-API: [M+H]⁺ = 529.2. | 51 mg |
| Z256a-1 | ¹H NMR (500 MHz, DMSO-*d*₆) δ 10.04 (s, 1H), 7.26 (td, *J* = 8.2, 6.8 Hz, 1H), 6.86-6.66 (m, 3H), 6.17 (d, *J* = 17.7 Hz, 1H), 5.75 (d, *J* = 11.7 Hz, 1H), 4.59 (s, 1H), 4.30-4.25 (m, 1H), 4.17-4.01 (m, 3H), 3.89 (t, *J* = 4.5 Hz, 1H), 3.82 (d, *J* = 15.0 Hz, 1H), 3.68 (dd, *J* = 11.7, 4.5 Hz, 1H), 3.59-3.38 (m, 2H), 3.28 (d, *J* = 8.0 Hz, 1H), 3.18 (s, 3H), 2.86 (d, *J* = 11.6 Hz, 1H), 2.09 (d, *J* = 13.7 Hz, 1H), 1.85 (dd, *J* = 13.8, 4.5 Hz, 1H), 1.60 (s, 3H), 1.43 (s, 3H). ES-API: [M+H]⁺ =529.2 | 85 mg |
| Z256a-2 | ¹H NMR (500 MHz, DMSO-*d*₆) δ 10.06 (d, *J* = 1.6 Hz, 1H), 7.26 (td, *J* = 8.3, 6.8 Hz, 1H), 6.82-6.68 (m, 3H), 6.16 (d, *J* = 16.6 Hz, 1H), 5.74 (d, *J =* 10.2 Hz, 1H), 4.56 (s, 1H), 4.37-4.23 (m, 1H), 4.13-4.10 (m, 5H), 3.86-3.84 (m, 1H), 3.53-3.48 (m, 2H), 3.34 (d, *J* = 9.4 Hz, 1H), 3.25 (s, 3H), 3.15 (t, *J* = 8.6 Hz, 1H), 2.17 (dd, *J* = 11.4, 6.1 Hz, 1H), 1.65 (t, *J* = 11.0 Hz, 1H), 1.48 (d, *J* = 7.8 Hz, 6H). ES-API: [M+H]⁺ =529.2 | 70 mg |
| Z317a-1 | ES-API: [M+H]⁺ = 556.2 | 6 mg |
| Z317a-2 | ES-API: [M+H]⁺ = 556.2 | 7 mg |
| Z292-1S | ¹H NMR (500 MHz, DMSO-*d*₆) δ 10.04-9.94 (m, 1H), 7.30-7.23 (m, 1H), 7.21 (s, 1H), 6.78 (dd, *J* = 8.3, 2.5 Hz, 1H), 6.72 (t, *J* = 8.8 Hz, 1H), 6.17 (dd, *J* = 16.8, 2.3 Hz, 1H), 5.74 (s, 1H), 5.52 (d, *J* = 8.8 Hz, 1H), 4.59 (dd, *J* = 12.1, 2.9 Hz, 2H), 4.35-4.24 (m, 2H), 4.19-3.89 (m, 5H), 3.77 (s, 3H), 3.67-3.39 (m, 3H), 1.42 (t, *J* = 6.3 Hz, 3H). ES-API: [M+H]⁺ =553.2 | 15 mg |
| Z292S | ¹H NMR (500 MHz, DMSO-*d*₆) δ 10.01-9.93 (m, 1H), 7.29-7.21 (m, 1H), 7.21 (s, 1H), 6.80-6.76 (m, 1H), 6.72 (t, *J* = 8.8 Hz, 1H), 6.18-6.14 (m, 1H), 5.74 (s, 1H), 5.52 (d, *J* = 8.8 Hz, 1H), 4.61-4.58 (m, 2H), 4.33-4.21 (m, 2H), 4.19-3.89 (m, 5H), 3.77 (s, 3H), 3.67-3.39 (m, 3H), 1.42-1.39 (m, 3H). ES-API: [M+H]⁺ =553.2 | 48 mg |
| Z291-1S | ¹H NMR (500 MHz, DMSO-*d*₆) δ 10.11-10.01 (m, 1H), 7.34-7.20 (m, 2H), 6.78 (dd, *J* = 25.1, 8.6 Hz, 2H), 6.17 (dd, *J* = 16.7, 2.3 Hz, 1H), 5.75 (d, *J* = 10.5 Hz, 1H), 5.35 (d, *J* = 6.4 Hz, 1H), 4.74 (d, *J* = 11.9 Hz, 1H), 4.48 (s, 1H), 4.42-3.97 (m, 6H), 3.99-3.84 (m, 1H), 3.76 (s, 3H), 3.71 (d, *J* = 13.9 Hz, 1H), 3.61-3.37 (m, 2H), 1.50 (dd, *J* = 18.0, 6.0 Hz, 3H). ES-API: [M+H]⁺ =553.2 | 40 mg |
| Z291S | ¹H NMR (500 MHz, DMSO-*d*₆) δ 10.03 (d, *J* = 10.1 Hz, 1H), 7.49 (s, 1H), 7.27 (td, *J* = 8.3, 6.9 Hz, 1H), 6.85-6.68 (m, 3H), 6.17 (dd, *J* = 16.7, 2.3 Hz, 1H), 5.75 (d, *J* = 10.5 Hz, 1H), 5.10 (d, *J* = 6.9 Hz, 1H), 4.78 (d, *J* = 13.1 Hz, 1H), 4.48 (s, 1H), 4.39-3.96 (m, 6H), 3.82 (s, 3H), 3.82-3.70 (m, 1H), 3.53-3.41 (m, 2H), 1.47 (dd, *J* = 16.4, 6.1 Hz, 3H). ES-API: [M+H]⁺ =553.2 | 80 mg |
| Z324-1 | ES-API: [M+H]⁺ =558.2. | 4.3 mg |
| Z324-2 | ES-API: [M+H]⁺ =558.2. | 4.6 mg |
| Z308a-1 | ¹H NMR (500 MHz, DMSO-*d*₆) δ 10.10 (s, 1H), 7.36-7.19 (m, 1H), 6.87-6.60 (m, 3H), 6.18 (d, *J* = 15.8 Hz, 1H), 5.76 (d, *J* = 10.1 Hz, 1H), 4.56-3.67 (m, 10H), 3.40 (dd, *J* = 28.4, 14.1 Hz, 4H), 3.10 (s, 1H), 2.83 (s, 3H), 1.24 (dd, *J* = 16.9, 10.0 Hz, 6H). ES-API: [M+H]⁺ = 544.2. | 29 mg |
| Z308a-2 | ¹H NMR (500 MHz, DMSO-*d*₆) δ 10.05 (s, 1H), 7.27 (d, *J* = 8.0 Hz, 1H), 6.92-6.63 (m, 3H), 6.18 (d, *J* = 16.5 Hz, 1H), 5.75 (s, 1H), 4.55-3.51 (m, 11H), 3.35-3.21 (m, 3H), 3.05 (s, 1H), 2.84 (s, 3H), 1.26-1.08(m, 6H). ES-API: [M+H]⁺ = 544.2 | 33 mg |
| Z325-1 | ¹H NMR (500 MHz, DMSO-*d*₆) δ 7.59-7.40 (m, 2H), 7.38-7.22 (m, 2H), 6.78 (d, *J* = 8.9 Hz, 1H), 6.18 (d, *J* = 16.4 Hz, 1H), 5.74 (s, 1H), 4.87 (s, 1H), 4.60-3.82 (m, 7H), 3.77-3.40 (m, 3H), 3.20 (s, 1H), 2.81 (d*, J* = 11.2 Hz, 1H), 1.90 (dd, *J* = 23.3, 8.5 Hz, 2H), 1.72 (s, 3H), 1.41 (s, 3H). ES-API: [M+H]⁺ = 515.1 | 60 mg |
| Z325-2 | ¹H NMR (500 MHz, DMSO-*d*₆) δ 7.51 (dd, *J* = 13.9, 6.7 Hz, 1H), 7.44 (t, *J* = 6.8 Hz, 1H), 7.33 (d, *J* = 7.8 Hz, 2H), 6.77 (d, *J* = 9.7 Hz, 1H), 6.17 (d, *J* = 16.8 Hz, 1H), 5.74 (s, 1H), 5.09 (d, *J* = 5.7 Hz, 1H), 4.64-3.83 (m, 7H), 3.77-3.40 (m, 2H), 3.28 (d, *J* = 13.9 Hz, 2H), 3.09 (t, *J* = 8.4 Hz, 1H), 2.00 (dd, *J* = 11.6, 6.0 Hz, 1H), 1.73 (t, *J* = 11.3 Hz, 1H), 1.47 (d, *J* = 10.0 Hz, 6H). ES-API: [M+H]⁺ = 515.1 | 40 mg |
| Z327-1 | ¹H NMR (500 MHz, DMSO-*d*₆) δ 10.07 (s, 1H), 7.30-7.23 (m, 1H), 6.87-6.67 (m, 3H), 6.17 (d, *J* = 16.7 Hz, 1H), 5.75 (d, *J* = 10.4 Hz, 1H), 4.58 (s, 1H), 4.36-4.21 (m, 1H), 4.21-3.84 (m, 4H), 3.75 (s, 1H), 3.47 (dd, *J* = 11.2, 5.9 Hz, 3H), 3.36 (s, 3H), 3.29-3.21(m, 1H) 2.88 (t, *J* = 9.4 Hz, 1H), 2.20 (dt, *J* = 11.9, 6.2 Hz, 1H), 1.67 (q, *J* = 10.6 Hz, 1H), 1.48 (s, 3H), 1.30 (d, *J* = 1.6 Hz, 3H). ES-API: [M+H]⁺=529.2 | 48 mg |
| Z327-2 | ¹H NMR (500 MHz, DMSO-*d*₆) δ 10.05 (s, 1H), 7.26 (td, *J* = 8.3, 6.9 Hz, 1H), 6.84-6.67 (m, 3H), 6.17 (d, *J* = 16.7 Hz, 1H), 5.75 (d, *J* = 10.4 Hz, 1H), 4.56 (s, 1H), 4.36-4.21 (m, 1H), 4.13-4.10 (m, 4H), 3.96-3.68 (m, 1H), 3.53 (q, *J* = 9.6 Hz, 2H), 3.41 (t, *J* = 2.2 Hz, 2H), 3.29 (s, 3H), 3.21 (s, 1H), 2.86-2.76 (m, 1H), 1.97 (d, *J* = 7.5 Hz, 1H), 1.44 (d, *J* = 11.4 Hz, 6H). ES-API: [M+H]⁺ =529.2 | 45 mg |
| Z331-1 | ¹H NMR (500 MHz, DMSO-*d*₆) δ 9.96 (s, 1H), 7.23 (dd, *J* = 15.7, 7.7 Hz, 1H), 6.74 (d*, J* = 8.3 Hz, 3H), 6.16 (d*, J* = 16.7 Hz, 1H), 5.73 (s, 1H), 4.61-3.36 (m, 14H), 3.22 (d, *J* = 52.7 Hz, 1H), 2.86 (dd, *J* = 15.1, 6.3 Hz, 2H), 2.01 (s, 3H), 1.84 (s, 1H), 1.66 (t, *J* = 11.4 Hz, 1H), 1.45 (dd, *J* = 16.4, 4.7 Hz, 6H). ES-API: [M+H]⁺ = 600.2 | 24 mg |
| Z331-2 | ¹H NMR (500 MHz, DMSO-*d*₆) δ 9.94 (d, *J* = 9.4 Hz, 1H), 7.23 (dd, *J* = 15.5, 7.8 Hz, 1H), 6.90-6.62 (m, 3H), 6.17 (d, *J* = 16.8 Hz, 1H), 5.74 (s, 1H), 4.54-3.34 (m, 14H), 3.28-2.71 (m, 3H), 2.03 (s, 3H), 1.93-1.69 (m, 2H), 1.47 (d, *J* = 81.6 Hz, 6H). ES-API: [M+H]⁺ = 600.2 | 41 mg |
| Z332-1 | ¹H NMR (500 MHz, DMSO-*d*₆) δ 9.97 (d, *J* = 20.3 Hz, 1H), 7.25 (dd, *J* = 15.5, 7.7 Hz, 1H), 6.74 (d, *J* = 9.1 Hz, 3H), 6.17 (d, *J* = 16.9 Hz, 1H), 5.74 (s, 1H), 4.44-3.41 (m, 11H), 3.20 (d, *J =* 19.8 Hz, 6H), 1.26 (t, *J* = 15.8 Hz, 3H), 1.10 (s, 3H). ES-API: [M+H]⁺ = 561.2 | 30 mg |
| Z332-2 | ¹H NMR (500 MHz, DMSO-*d*₆) δ 9.99 (s, 1H), 7.25 (d, *J* = 7.9 Hz, 1H), 6.74 (d, *J* = 8.5 Hz, 3H), 6.17 (d, *J* = 16.4 Hz, 1H), 5.75 (d, *J* = 10.6 Hz, 1H), 4.47-3.38 (m, 12H), 3.25 (d, *J* = 6.5 Hz, 5H), 3.11-2.96 (m, 1H), 1.28 (d, *J* = 20.0 Hz, 2H), 1.10 (s, 3H). ES-API: [M+H]⁺ = 561.2 | 57 mg |
| Z333-1 | ¹H NMR (500 MHz, DMSO-*d*₆) δ 9.96 (d, *J* = 18.6 Hz, 1H), 7.25 (dd, *J* = 15.1, 7.5 Hz, 1H), 6.72 (ddd, *J* = 22.5, 15.5, 8.7 Hz, 3H), 6.16 (d, *J* = 16.6 Hz, 1H), 5.73 (s, 1H), 4.69 (s, 1H), 4.54-3.34 (m, 13H), 3.31-3.16 (m, 3H), 3.07 (s, 1H), 1.08 (s, 3H). ES-API: [M+H]⁺ = 547.2 | 38 mg |
| Z333-2 | ¹H NMR (500 MHz, DMSO-*d*₆) δ 9.99 (s, 1H), 7.25 (dd, *J* = 15.4, 7.7 Hz, 1H), 6.80-6.60 (m, 3H), 6.17 (d, *J* = 16.7 Hz, 1H), 5.74 (s, 1H), 4.70-3.41 (m, 13H), 3.34 (d*, J* = 16.2 Hz, 1H), 3.31-3.25 (m, 2H), 3.20 (t, *J* = 12.0 Hz, 1H), 3.10-2.98 (m, 1H), 1.07 (s, 3H). ES-API: [M+H]⁺ = 547.2 | 51 mg |
| Z340-1 | ¹H NMR (500 MHz, DMSO-*d*₆) δ 10.50 (s, 1H), 7.43 (t, *J* = 8.8 Hz, 1H), 6.81 (d, *J* = 8.9 Hz, 2H), 6.17 (d, *J* = 16.6 Hz, 1H), 5.74 (s, 1H), 5.05 (d, *J* = 5.6 Hz, 1H), 4.60 (s, 1H), 4.39-3.84 (m, 7H), 3.53 (d, *J* = 8.7 Hz, 1H), 3.25 (t, *J* = 9.3 Hz, 2H), 3.05 (t*, J* = 8.5 Hz, 1H), 1.98 (dd, *J* = 11.5, 6.3 Hz, 1H), 1.71 (t, *J* = 11.2 Hz, 1H), 1.46 (s, 6H). ES-API: [M+H]⁺ = 549.1 | 12 mg |
| Z340-2 | ¹H NMR (500 MHz, DMSO-*d*₆) δ 10.54 (s, 1H), 7.43 (t, *J* = 8.8 Hz, 1H), 6.85-6.70 (m, 2H), 6.17 (d, *J=* 16.5 Hz, 1H), 5.75 (d, *J* = 7.1 Hz, 1H), 4.84 (s, 1H), 4.56 (s, 1H), 4.38-3.87 (m, 7H), 3.65 (dd, *J* = 11.3, 4.3 Hz, 1H), 3.49 (d, *J* = 17.1 Hz, 2H), 2.76 (d, *J* = 11.1 Hz, 1H), 1.96-1.83 (m, 2H), 1.69 (s, 3H), 1.41 (s, 3H). ES-API: [M+H]⁺ = 549.1 | 15 mg |
| Z341-1 | ¹H NMR (500 MHz, DMSO-*d*₆) δ 8.37 (s, 1H), 7.40 (s, 1H), 6.75 (t, *J* = 38.3 Hz, 2H), 6.17 (d, *J* = 16.6 Hz, 1H), 5.74 (s, 1H), 5.05 (s, 1H), 4.57 (s, 1H), 4.34-3.96 (m, 7H), 3.27 (t, *J* = 9.3 Hz, 2H), 3.03 (t, *J* = 8.5 Hz, 1H), 1.97 (dd, *J* = 11.5,6.2 Hz, 1H), 1.75-1.63 (m, 2H), 1.46 (d, *J* = 2.6 Hz, 6H). ES-API: [M+H]⁺ = 549.1 | 6 mg |
| Z341-2 | ¹H NMR (500 MHz, DMSO-*d*₆) δ 8.32 (s, 1H), 7.48-7.41 (m, 1H), 6.84-6.69 (m, 2H), 6.23-6.12 (m, 1H), 5.79-5.70 (m, 1H), 4.83 (s, 1H), 4.55 (d, *J* = 15.0 Hz, 1H), 4.33-4.01 (m, 7H), 3.66 (dd, *J=* 11.2, 4.4 Hz, 1H), 3.56-3.46 (m, 2H), 2.75 (d, *J* = 11.1 Hz, 1H), 1.92-1.85 (m, 2H), 1.68 (s, 3H), 1.40 (s, 3H). ES-API: [M+H]⁺ = 549.1 | 5 mg |
| Z345-1 | ¹H NMR (500 MHz, DMSO-*d*₆) δ 9.99-9.92 (m, 1H), 7.26 (q, *J* = 7.8 Hz, 1H), 7.20 (s, 1H), 6.79-6.70 (m, 3H), 6.17 (d*, J* = 17.1 Hz, 1H), 5.75 (t, *J* = 12.6 Hz, 1H), 5.30 (s, 1H), 4.71 (d, *J* = 13.9 Hz, 1H), 4.52 (s, 1H), 4.38 (s, 1H), 4.28-4.22 (m, 2H), 4.16-3.87 (m, 3H), 3.81 (s, 1H), 3.75 (s, 3H), 3.68-3.49 (m, 2H), 1.31-1.23 (m, 3H). ES-API: [M+H]⁺ = 553.2 | 40 mg |
| Z345-2 | ¹H NMR (500 MHz, DMSO-*d*₆) δ 10.04 (d, *J* = 10.8 Hz, 1H), 7.26 (q, *J* = 7.9 Hz, 1H), 7.20 (s, 1H), 6.80-6.69 (m, 3H), 6.18 (dd, *J* = 16.7, 2.3 Hz, 1H), 5.75 (s, 1H), 5.15 (s, 1H), 4.85-4.80 (m, 1H), 4.67-4.14 (m, 5H), 4.17-3.88 (m, 3H), 3.77 (s, 3H), 3.70-3.52 (m, 2H), 1.45-1.41(m, 3H). ES-API: [M+H]⁺ = 553.2 | 10 mg |
| Z346-1 | ¹H NMR (500 MHz, DMSO-*d*₆) δ 9.98-9.90 (m, 1H), 7.22 (s, 1H), 7.05-6.94 (m, 1H), 6.82-6.62 (m, 3H), 6.15 (t, *J =* 17.5 Hz, 1H), 5.75-5.70 (m, 1H), 4.77-4.69 (m, 2H), 4.65 (d, *J =* 15.5 Hz, 1H), 4.51-4.27 (m, 3H), 4.15 (s, 1H), 4.03-3.80 (m, 4H), 3.72 (s, 3H), 3.61 (s, 1H), 1.26 (s, 3H). ES-API: [M+H]⁺ = 553.2 | 7 mg |
| Z346-2 | ¹H NMR (500 MHz, DMSO-*d*₆) δ 10.01-9.85 (m, 1H), 7.28-7.24 (m, 1H), 7.21 (s, 1H), 6.82-6.63 (m, 3H), 6.19-6.12 (m, 1H), 5.76-5.70 (m, 1H), 4.83-4.71 (m, 2H), 4.68-4.49 (m, 2H), 4.47-4.27 (m, 2H), 4.15 (d, *J* = 15.7 Hz, 1H), 4.02-3.80 (m, 4H), 3.70 (s, 3H), 3.65-3.57 (m, 1H), 1.26 (s, 3H). ES-API: [M+H]⁺ = 553.2 | 7 mg |
| Z469-1 | ¹H NMR (400 MHz, DMSO-*d*₆) δ 9.99 (s, 1H), 7.31 - 7.18 (m, 1H), 6.83 - 6.63 (m, 3H), 6.24 - 6.09 (m, 1H), 5.83 - 5.65 (m, 1H), 4.64 - 3.36 (m, 12H), 3.02 - 2.92 (m, 1H), 2.84 - 2.72 (m, 1H), 2.41 - 2.19 (m, 6H), 2.14 (s, 3H), 2.03 - 1.94 (m, 1H), 1.67 - 1.57 (m, 1H), 1.52 - 1.39 (m, 6H). ES-API: [M+H]⁺=613.3 | 35 mg |
| Z469-2 | ¹H NMR (400 MHz, DMSO-*d*₆) δ 10.00 (s, 1H), 7.30 - 7.18 (m, 1H), 6.83 - 6.62 (m, 3H), 6.25 - 6.09 (m, 1H), 5.81 - 5.66 (m, 1H), 4.62 - 3.34 (m, 12H), 2.99 - 2.84 (m, 1H), 2.69 - 2.57 (m, 1H), 2.38 - 2.15 (m, 6H), 2.11 (s, 3H), 2.05 - 1.95 (m, 1H), 1.92 - 1.84 (m, 1H), 1.55 (s, 3H), 1.40 (s, 3H). | 20 mg |
| | ES-API: [M+H]⁺= 613.3 | |
| Z471-1 | ES-API: [M+H]⁺= 554.2 | 1 mg |
| Z471-2 | ¹H NMR (400 MHz, DMSO-*d*₆) δ 7.56 - 7.39 (m, 2H), 7.36 - 7.29 (m, 2H), 6.83 - 6.71 (m, 1H), 6.21 - 6.07 (m, 1H), 5.80 - 5.66 (m, 1H), 4.55 - 4.26 (m, 3H), 4.23 - 3.76 (m, 5H), 3.57 - 3.41 (m, 3H), 3.08 - 2.88 (m, 4H), 2.85 - 2.73 (m, 1H), 1.91 - 1.74 (m, 4H), 1.71 (s, 3H), 1.38 (s, 3H). ES-API: [M+H]⁺= 554.2 | 2 mg |
| Z474-1 | ES-API: [M+H]⁺=581.3; ¹H NMR (500 MHz, DMSO-*d*₆) δ 7.60 - 7.50 (m, 2H), 7.41 - 7.31 (m, 2H), 6.81 - 6.66 (m, 1H), 6.23 - 6.10 (m, 1H), 5.81 - 5.67 (m, 1H), 4.59 (s, 1H), 4.44 - 4.28 (m, 1H), 4.27 - 4.15 (m, 2H), 4.13 - 3.96 (m, 1H), 3.95 - 3.85 (m, 1H), 3.83 - 3.33 (m, 6H), 3.01 - 2.89 (m, 1H), 2.86 - 2.70 (m, 1H), 2.46 - 2.19 (m, 6H), 2.15 (s, 3H), 2.01 (dd, *J* = 11.5, 5.5 Hz, 1H), 1.64 (t, *J* = 11.5 Hz, 1H), 1.55 (s, 3H), 1.49 (s, 3H). | 6 mg |
| Z474-2 | ES-API: [M+H]⁺=581.3 | 3 mg |
| Z406-1 | ¹H NMR (400 MHz, DMSO-*d*₆) δ 7.59 - 7.24 (m, 4H), 6.81 - 6.67 (m, 1H), 6.17 (d, *J* = 16.3 Hz, 1H), 5.75 (d, *J* = 10.9 Hz, 1H), 4.54 (s, 1H), 4.40 - 3.36 (m, 10H), 3.33 - 3.29 (m, 1H), 3.24 (s, 3H), 3.02 (s, 2H), 2.56 (d, *J =* 5.6 Hz, 1H), 2.44 (d, *J* = 13.8 Hz, 1H), 2.16 (s, 3H), 2.04 - 1.93 (m, 1H), 1.69-1.57 (m, 1H), 1.48 (d*, J* = 22.1 Hz, 6H). | 30 mg |
| | ES-API: [M+H]⁺=586.3 | |
| Z406-2 | ¹H NMR (400 MHz, DMSO-*d*₆) δ 7.61 - 7.21 (m, 4H), 6.77 (s, 1H), 6.17 (d, *J* = 16.3 Hz, 1H), 5.75 (d, *J* = 9.5 Hz, 1H), 4.67 - 3.37 (m, 10H), 3.29 - 2.76 (m, 6H), 2.63 - 2.53 (m, 1H), 2.45 (s, 2H), 2.21-1.86 (m, 5H), 1.61 (s, 3H), 1.44 (d, *J* = 14.5 Hz, 3H). | 20 mg |
| | ES-API: [M+H]⁺=586.3 | |
| Z468-1 | ES-API: [M+H]⁺=584.2 | 40 mg |
| Z468-2 | ES-API: [M+H]⁺ = 584.2 | 20 mg |
| Z455-1 | ES-API: [M+H]⁺ =546.1; ¹H NMR (500 MHz, DMSO-*d*₆) δ 9.89 (s, 1H), 7.23 (t, *J =* 8.1 Hz, 1H), 6.94-6.89 (m, 2H), 6.85-6.71 (m, 1H), 6.17 (d, *J =* 17.5 Hz, 1H), 5.79-5.68 (m, 1H), 4.45-4.25 (m, 2H), 4.25-4.15 (m, 2H), 4.11-3.95 (m, 3H), 3.83 (s, 2H), 3.66-3.42 (m, 3H), 3.19 (t, *J* = 12.0 Hz, 2H), 2.66-2.58 (m, 2H), 2.13 (s, 3H), 2.07-1.92 (m, 2H). | 0.8 mg |
| Z455-2 | ES-API: [M+H]⁺ =546.1; ¹H NMR (500 MHz, DMSO-*d*₆) δ 9.97 (s, 1H), 7.22 (t, *J* = 8.0 Hz, 1H), 6.97 (d, *J* = 8.0 Hz, 1H), 6.87 (d, *J* = 8.2 Hz, 1H), 6.75 (s, 1H), 6.19 (d, *J* = 17.5 Hz, 1H), 5.81-5.70 (m, 1H), 4.48-4.27 (m, 2H), 4.28-4.18 (m, 2H), 4.15-3.98 (m, 3H), 3.85 (s, 2H), 3.69-3.46 (m, 3H), 3.21 (t, *J* = 12.0 Hz, 2H), 2.68-2.61 (m, 2H), 2.15 (s, 3H), 2.11-1.96 (m, 2H). | 6.8 mg |
| Z454-1 | ES-API: [M+H]⁺ =597.2; ¹H NMR (500 MHz, CDCl₃) δ 7.44 - 7.36 (m, 2H), 7.21 (t, *J* = 7.5 Hz, 1H), 7.14 (t, *J* = 9.0 Hz, 1H), 6.55 (s, 1H), 6.40 (d, *J* = 16.5 Hz, 1H), 5.81 (d, *J* = 10.0 Hz, 1H), 4.51 - 4.42 (m, 1H), 4.40 - 4.20 (m, 3H), 4.19 - 4.10 (m, 1H), 4.00 (s, 1H), 3.79 - 3.44 (m, 4H), 3.00 - 2.82 (m, 2H), 2.78 - 3.40 (m, 8H), 2.35 (s, 3H), 1.96 (dd, *J =* 11.5, 6.0 Hz, 1H), 1.87 (t, *J* = 11.5 Hz, 1H), 1.56 (s, 6H). | 25 mg |
| Z454-2 | ES-API: [M+H]⁺ =597.2; ¹H NMR (500 MHz, CDCl₃) δ 7.44 - 7.36 (m, 2H), 7.21 (t, *J* = 7.5 Hz, 1H), 7.14 (t, *J* = 9.1 Hz, 1H), 6.56 (s, 1H), 6.40 (d, *J* = 16.5 Hz, 1H), 5.81 (d, *J* = 10.5 Hz, 1H), 4.52 - 3.39 (m, 10H), 3.09 - 2.38 (m, 10H), 2.34 (s, 3H), 2.04 - 1.98 (m, 1H), 1.94 - 1.88 (m, 1H), 1.62 (s, 3H), 1.50 (s, 3H). | 27 mg |
| Z458-1 | ES-API: [M+H]⁺ =559.1; ¹H NMR (500 MHz, DMSO-*d*₆) δ 10.42-10.04 (m, 1H), 7.22-7.19 (m, 1H), 6.79-6.74 (m, 2H), 6.71-6.56 (m, 1H), 6.16 (d, *J* = 17 Hz, 1H), 5.77-5.71 (m, 1H), 4.58-4.45(m, 1H), 4.35-4.23 (m, 1H), 4.17-3.85 (m, 4H), 3.83-3.63 (m, 2H), 3.17-2.95 (m, 4H), 1.98-1.88 (m, 1H), 1.49-1.39 (m, 6H), 1.37-1.34 (m, 2H). | 18 mg |
| Z458-2 | ES-API: [M+H]⁺ =559.1; ¹H NMR (500 MHz, DMSO-*d*₆) δ 10.21-9.99 (m, 1H), 7.35-7.22 (m, 1H), 6.81-6.78 (m, 2H), 6.84-6.65 (m, 1H), 6.19-6.08 (m, 1H), 5.79-5.72 (m, 1H), 4.61-4.48(m, 1H), 4.39-4.28 (m, 1H), 4.21-3.89 (m, 4H), 3.85-3.65 (m, 2H), 3.21-2.98 (m, 4H), 1.99-1.91 (m, 1H), 1.52-1.42 (m, 6H), 1.39-1.37 (m, 2H). | 18 mg |
| Z465-1 | ES-API: [M+H]⁺ =528.2 | 8 mg |
| Z465-2 | ES-API: [M+H]⁺ =528.2 | 33 mg |
| Z360-1 | ¹H NMR (400 MHz, CDCl₃) δ 7.30-7.28(m, 1H),6.83 (d, *J* = 8.4 Hz, 1H), 6.71 (t, *J* = 9.0 Hz, 1H), 6.56 (brs, 1H), 6.41 (d, *J* = 16.6 Hz, 1H), 5.82 (d, *J =* 10.2 Hz, 1H), 4.50-4.30 (m, 3H), 4.22-3.95 (m, 3H), 3.76-3.37 (m, 4H), 2.92-2.74 (m, 1H), 2.49 (s, 6H), 2.15-1.97 (m, 1H), 1.35-1.29 (m, 1H), 1.26 (s, 6H). ES-API: [M+H]⁺=558.2 | 3.3 mg |
| Z360-2 | ¹H NMR (400 MHz, CDCl₃) δ 7.30-7.28(m, 1H), 6.85 (d, *J* = 8.3 Hz, 1H), 6.71 (t, *J* = 9.1 Hz, 1H), 6.57 (brs, 1H), 6.41 (d, *J =* 16.5 Hz, 1H), 5.82 (d, *J* = 10.5 Hz, 1H), 4.48-4.28 (m, 3H), 4.24-3.99 (m, 3H), 3.88-3.80 (m, 1H), 3.74-3.30 (m, 2H), 3.02-2.87 (m, 2H), 2.45 (s, 6H), 2.23-2.15 (m, 2H), 1.47 (d, *J* = 19.5 Hz, 6H), 1.33-1.31 (m, 1H). ES-API: [M+H]⁺=558.2. | 3.7 mg |
| Z466-1 | ES-API: [M+H]⁺=572.2; ¹H NMR (500 MHz, CDCl₃) δ 7.35 - 7.28 (m, 1H), 6.73 (dd, *J =* 19.8, 8.5 Hz, 2H), 6.67 - 6.57 (m, 1H), 6.55 - 6.47 (m, 1H), 6.47 - 6.35 (m, 1H), 5.83 (d, *J =* 10.1 Hz, 1H), 4.58 - 4.29 (m, 3H), 4.26 - 4.15 (m, 2H), 4.07 - 3.98 (m, 1H), 3.80 (s, 3H), 3.78 - 3.71 (m, 2H), 3.68 - 3.37 (m, 2H), 2.94 (s, 1H), 2.65 - 2.60 (m, 3H), 2.08 - 2.00 (m, 4H), 1.77 - 1.72 (m, 3H). | 3.5 mg |
| Z466-2 | ES-API: [M+H]⁺=572.2; ¹H NMR (500 MHz, CDCl₃) δ 7.30 (s, 2H), 6.82 - 6.65 (m, 2H), 6.67 - 6.50 (m, 1H), 6.50 - 6.37 (m, 1H), 5.83 (d, *J =* 11.2 Hz, 1H), 4.57 - 4.32 (m, 3H), 4.29 - 4.01 (m, 4H), 3.89 - 3.73 (m, 3H), 3.70 (s, 3H), 3.02 - 2.91 (m, 1H), 2.66 - 2.56 (m, 3H), 2.22 (t, *J* = 7.8 Hz, 1H), 2.12 - 1.99 (m, 4H), 1.76 (s, 3H). | 1.6 mg |

### Embodiment 79 Synthesis of Z342 and Z343

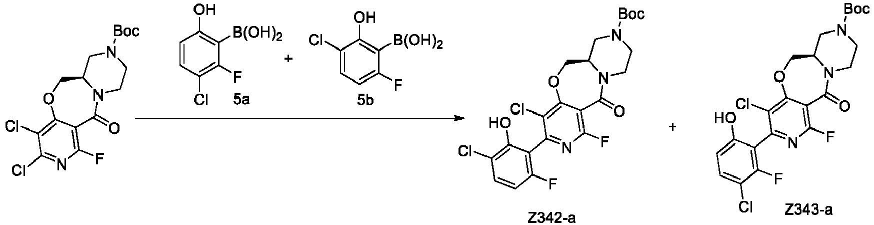

Step 1: *tert*-Butyl (*R*)-3,4-dichloro-1-fluoro-12-oxo-6a,7,9,10-tetrahydro-12*H*-pyrazino[2,1-*c*]pyrido[3,41-*f*][1,4]oxazepine-8(6*H*)-carboxylate (405 mg, 1.0 mmol), a mixture of (3-chloro-6-fluoro-2-hydroxyphenyl)boronic acid and (3-chloro-2-fluoro-6-hydroxyphenyl)boronic acid (prepared in preparation embodiment 9, 750 mg, 3.9 mmol), Sphos-Pd-G2 (72 mg, 0.1 mmol), potassium carbonate (400 mg, 2.9 mmol), 30 mL of dioxane and 5 mL of water were added to a 50 mL flask. Under nitrogen protection, the reaction was carried out at 100°C for 4 hours. The reaction solution was added with 50 mL of ethyl acetate, washed three times with 30 mL of saturated brine, dried and concentrated, and the crude product was purified by flash silica gel column (DCM/MeOH=20:1) to obtain two isomer compounds:

One isomer with a structure arbitrarily designated as *tert*-butyl (6a*R*)-4-chloro-3-(3-chloro-2-fluoro-6-hydroxyphenyl)-1-fluoro-12-oxo-6a,7,9,10-tetrahydro-12*H*-pyrazino[2,1-*c*]pyrido[3,4-*f*][1,4]oxazepine-8(6*H*)-carboxylate (Z343-a, 150 mg, crude product, R_{f}=0.5, DCM:MeOH=20:1), ES-API: [M+H]⁺=516.0.

Another isomer with a structure arbitrarily designated as *tert*-butyl (6a*R*)-4-chloro-3-(3-chloro-6-fluoro-2-hydroxyphenyl)-1-fluoro-12-oxo-6a,7,9,10-tetrahydro-12*H*-pyrazino[2,1-*c*]pyrido[3,4-*f*][1,4]oxazepine-8(6*H*)-carboxylate (Z342-a, 110 mg, crude product, R_{f}=0.4, DCM:MeOH=20:1), ES-API: [M+H]⁺=516.0.

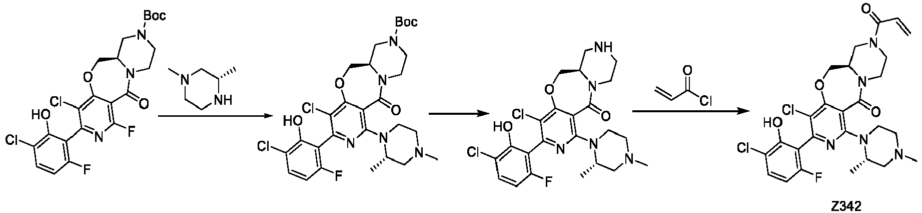

Step 2: Compound *tert*-butyl (6a*R*)-4-chloro-3-(3-chloro-6-fluoro-2-hydroxyphenyl)-1-fluoro-12-oxo-6a,7,9,10-tetrahydro-12*H*-pyrazino[2,1-*c*]pyrido[3,4-*f*][1,4]oxazepine-8(6*H*)-carboxylate (110 mg, 0.21 mmol) was dissolved in 10 mL of acetonitrile, and DIPEA (200 mg, 1.5 mmol) and (*S*)-1,3-dimethylpiperazine (100 mg, 0.84 mmol) were added thereto. The mixture was reacted at 90°C for 4 hours. The reaction solution was added with 30 mL of ethyl acetate, washed with 30 mL of saturated brine for 3 times, dried and concentrated, and the crude product was purified by flash silica gel column to obtain a target product of *tert*-butyl (6a*R*)-4-chloro-3-(3-chloro-6-fluoro-2-hydroxyphenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg). [M+H]⁺=610.1.

Step 3: *tert*-Butyl (6a*R*)-4-chloro-3-(3-chloro-6-fluoro-2-hydroxyphenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg, crude product) was dissolved in dichloromethane (8 mL), and trifluoroacetic acid (4 mL) was added thereto. The mixture was stirred at room temperature for 1 hour, and the reaction solution was concentrated to obtain a crude product of (6a*R*)-4-chloro-3-(3-chloro-6-fluoro-2-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (66 mg). [M+H]⁺=510.1.

Step 4: The crude product of (6aR)-4-chloro-3-(3-chloro-6-fluoro-2-hydroxyphenyl)-1-((S)-2,4-dimethylpiperazin-1-yl)-6,6a,7,8,9,10-hexahydro-12*H*-pyrazino[2,1-*c*]pyrido[3,4-*f*[1,4]oxazepin-12-one (66 mg, 0.13 mmol) was dissolved in dichloromethane (15 mL), and triethylamine (60 mg, 0.6 mmol) was added thereto. The reaction was cooled to 0°C, and acryloyl chloride (10 mg, 0.11 mmol) was added dropwise to the reaction solution. The reaction was stirred at 0°C for 15 minutes. The reaction solution was added with 40 mL of dichloromethane, washed with 50 mL of saturated aqueous NaHCO₃ solution and 40 mL of saturated brine, dried and concentrated. The crude product was purified by preparative liquid phase to obtain a target product of (6a*R*)-8-acryloyl-4-chloro-3-(3-chloro-6-fluoro-2-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (15 mg, yield: 20%). ES-API: [M+H]⁺=564.1. ¹H NMR (500 MHz, DMSO-*d*₆) δ 10.35 (s, 1H), 7.47-7.35 (m, 1H), 6.80 (dd, *J* = 18.1, 9.0 Hz, 2H), 6.17 (d, *J* = 17.0 Hz, 1H), 5.74 (s, 1H), 4.57 - 3.40 (m, 12H), 3.19 (d, *J =* 11.6 Hz, 1H), 2.63 (d, *J* = 9.4 Hz, 1H), 2.29 - 1.76 (m, 5H), 1.25 (d, *J =* 6.6 Hz, 3H). The configuration of this product was assigned according to the configuration of the raw material used in step 2.

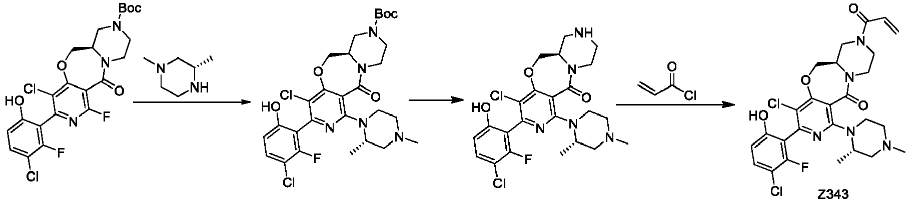

Step 2': Compound *tert*-butyl (6a*R*)-4-chloro-3-(3-chloro-2-fluoro-6-hydroxyphenyl)-1-fluoro-12-oxo-6a,7,9,10-tetrahydro-12*H*-pyrazino[2,1-*c*]pyrido[3,4-*f*][1,4]oxazepine-8(6*H*)-carboxylate (150 mg, 0.29 mmol) was dissolved in 10 mL of acetonitrile, and DIPEA (200 mg, 1.5 mmol) and (*S*)-1,3-dimethylpiperazine (100 mg, 0.84 mmol) were added thereto. The mixture was reacted at 90°C for 4 hours. The reaction solution was added with 30 mL of ethyl acetate, washed with 30 mL of saturated brine for 3 times, dried and concentrated, and the crude product was purified by flash silica gel column to obtain a target product of *tert*-butyl (6a*R*)-4-chloro-3-(3-chloro-2-fluoro-6-hydroxyphenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg). [M+H]⁺=610.1.

Step 3': *tert*-Butyl (6a*R*)-4-chloro-3-(3-chloro-2-fluoro-6-hydroxyphenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg, crude product) was dissolved in dichloromethane (8 mL), and trifluoroacetic acid (4 mL) was added thereto. The mixture was stirred at room temperature for 1 hour, and the reaction solution was concentrated to obtain a crude product of (6a*R*)-4-chloro-3-(3-chloro-2-fluoro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (66 mg). [M+H]⁺=510.1.

Step 4': The crude product of (6a*R*)-4-chloro-3-(3-chloro-2-fluoro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (66 mg, 0.13 mmol) was dissolved in dichloromethane (15 mL), and triethylamine (60 mg, 0.6 mmol) was added thereto. The reaction was cooled to 0°C, and acryloyl chloride (10 mg, 0.11 mmol) was added dropwise to the reaction solution. The reaction was stirred at 0°C for 15 minutes. The reaction solution was added with 40 mL of dichloromethane, washed with 50 mL of saturated aqueous NaHCO₃ solution and 40 mL of saturated brine, dried and concentrated. The crude product was purified by preparative liquid phase to obtain a target product of (6a*R*)-8-acryloyl-4-chloro-3-(3-chloro-2-fluoro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (16 mg, yield: 21%). ES-API: [M+H]⁺=564.1. ¹H NMR (500 MHz, DMSO-*d*₆) δ 10.70 - 9.57 (m, 1H), 7.59 - 7.37 (m, 1H), 6.95 - 6.67 (m, 2H), 6.17 (d, *J* = 16.0 Hz, 1H), 5.74 (s, 1H), 4.51-3.41 (m, 12H), 3.19 (d, *J* = 10.9 Hz, 1H), 2.62 (d, *J* = 10.5 Hz, 1H), 2.20-1.95 (m, 5H), 1.24 (d, *J=* 6.4 Hz, 3H). The configuration of this product was assigned according to the configuration of the raw material used in step 2'.

### Embodiment 80 Synthesis of (6aR)-8-acryloyl-4-chloro-3-(3,6-difluoro-2-hydroxyphenyl)-1-((S)-4-(dimethylamino)-2,2-dimethylpyrrolidin-1-yl)-6,6a,7,8,9,10-hexahydro-12H-pyrazino[2,1-c]pyrido[3,4-f][1,4]oxazepin-12-one (Z475-1) and (6aR)-8-acryloyl-4-chloro-3-(3,6-difluoro-2-hydroxyphenyl)-1-((R)-4-(dimethylamino)-2,2-dimethylpyrrolidin-1-yl)-6,6a,7, 8,9,10-hexahydro-12H-pyrazino [2,1-c]pyrido [3,4-f][1,4]oxazepin-12-one (Z475-2)

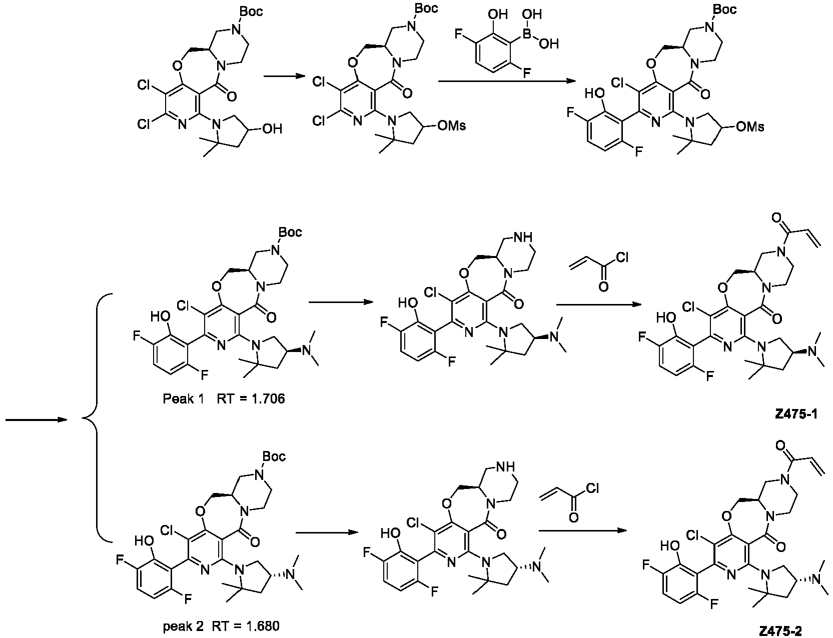

Step 1: *tert*-Butyl (6a*R*)-3,4-dichloro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (1.1 g, 2.2 mmol), diisopropylethylamine (853 mg, 6.6 mmol) and 20 mL of dichloromethane were added to a 100 mL flask. Under an ice bath, methanesulfonyl chloride (302 mg, 2.64 mmol) was slowly added dropwise thereto, stirred at room temperature for 1 hour. The reaction solution was extracted with ethyl acetate, washed with saturated brine, and the organic phase was dried and concentrated, and purified by silica gel column to obtain tert-butyl (6a*R*)-3,4-dichloro-1-(2,2-dimethyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (1.1 g, yield of 86%) as a white solid. ES-API: [M+H]⁺=579.1.

Step 2: *tert*-Butyl (6a*R*)-3,4-dichloro-1-(2,2-dimethyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (750 mg, 1.29 mmol), (3,6-difluoro-2-hydroxyphenyl)boronic acid (675 mg, 3.88 mmol), SPhosPd G2 (93 mg, 0.13 mmol), potassium carbonate (1.92 g, 3.88 mmol), 10 mL of dioxane and 2 mL of water were added to a 50 mL flask. Under nitrogen protection, the reaction was carried out at 80°C for 2 hours. The reaction solution was added with 50 mL of ethyl acetate, washed with 30 mL of saturated brine for 3 times, dried and concentrated, and the crude product was purified by flash silica gel column to obtain *tert*-butyl (6a*R*)-4-chloro-3-(3,6-difluoro-2-hydroxyphenyl)-1-(2,2-dimethyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (460 mg, yield of 53%). ES-API: [M+H]⁺=673.2.

Step 3: *tert*-Butyl (6a*R*)-4-chloro-3-(3,6-difluoro-2-hydroxyphenyl)-1-(2,2-dimethyl-4-((methylsulfonyl)oxy)pyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (400 mg, 0.59 mmol) and 40% aqueous dimethylamine solution (10 mL) were added to a 50 mL sealed tube. The reaction solution was reacted at 100°C for 2 hours. The reaction solution was added with 50 mL of ethyl acetate, washed three times with 30 mL of saturated brine, dried and concentrated, and the crude product was purified by flash silica gel column to obtain two isomers: one isomer with a structure arbitrarily designated as *tert*-butyl (6a*R*)-4-chloro-3-(3,6-difluoro-2-hydroxyphenyl)-1-((*S*)-4-(dimethylamino)-2,2-dimethylpyrrolidin-1-yl)-12-oxo-6a,7,9,10-tetrahydro-12*H*-pyrazino[2,1-*c*]pyrido[3,41-*f*][1,4]oxazepine-8(6*H*)-carboxylate (50 mg, peak 1, retention time: 1.706 min) and another isomer with a structure arbitrarily designated as *tert*-butyl (6a*R*)-4-chloro-3-(3,6-difluoro-2-hydroxyphenyl)-1-((*R*)-4-(dimethylamino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (30 mg, peak 2, retention time: 1.680 min). ES-API: [M+H]⁺=622.3.

Step 4: *tert*-Butyl (6a*R*)-4-chloro-3-(3,6-difluoro-2-hydroxyphenyl)-1-((*S*)-4-(dimethylamino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (peak 1, retention time: 1.706 min) (50 mg, 0.08 mmol), 0.5 mL of trifluoroacetic acid and 2 mL of dichloromethane were added to a 50 mL flask. The mixture was stirred at room temperature for 1 hour, and the completion of reaction was detected by LC-MS. The reaction solution was concentrated to obtain a crude product of (6a*R*)-4-chloro-3-(3,6-difluoro-2-hydroxyphenyl)-1-((*S*)-4-(dimethylamino)-2,2-dimethylpyrrolidin-1-yl)-6,6a,7,8,9,10-hexahydro-12*H*-pyrazino[2,1-*c*]pyrido[3,4-*f*][1,4]oxazepin-12-one trifluoroacetate (42 mg, crude product) as a yellow oil. ES-API: [M+H]⁺=522.2.

Step 5: (6a*R*)-4-Chloro-3-(3,6-difluoro-2-hydroxyphenyl)-1-((*S*)-4-(dimethylamino)-2,2-dimethylpyrrolidin-1-yl)-6,6a,7,8,9,10-hexahydro-12*H*-pyrazino[2,1-*c*]pyrido[3,4-*f*][1,4]oxazepin-12-one trifluoroacetate (42 mg, crude product), 10 mL of dichloromethane and diisopropylethylamine (52 mg, 0.40 mmol) were added to a 50 mL flask. At 0°C, a solution of acryloyl chloride in dichloromethane (7 mg, 0.08 mmol, 1 mL) was added dropwise thereto. The reaction was carried out at 0°C for 10 minutes. The mixture was washed with 20 mL of water, and the organic phase was dried and then concentrated, and the crude product was purified by preparative HPLC to obtain (6aR)-8-acryloyl-4-chloro-3-(3,6-difluoro-2-hydroxyphenyl)-1-((*S*)-4-(dimethylamino)-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z475-1, 10 mg, 2-step yield of 21%) as a white solid. ES-API: [M+H]⁺=576.2. The configuration of this structure was assigned according to the configuration of the raw material used in step 4.

Step 6: *tert*-Butyl (6a*R*)-4-chloro-3-(3,6-difluoro-2-hydroxyphenyl)-1-((*R*)-4-(dimethylamino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (peak 2, retention time: 1.680 min) was used to replace *tert*-butyl (6a*R*)-4-chloro-3-(3,6-difluoro-2-hydroxyphenyl)-1-((*S*)-4-(dimethylamino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (peak 1, retention time: 1.706 min). Referring to step 4 and step 5, (6a*R*)-8-acryloyl-4-chloro-3-(3,6-difluoro-2-hydroxyphenyl)-1-((*R*)-4-(dimethylamino)-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z475-2, 2-step yield of 14%) as a white solid was synthesized. ES-API: [M+H]⁺=576.2. ¹H NMR (400 MHz, DMSO-*d*₆) δ 10.17 (s, 1H), 7.31 - 7.22 (s, 1H), 6.84 - 6.67 (m, 2H), 6.20 - 6.10 (m, 1H), 5.78 - 5.67 (m, 1H), 4.52 - 3.47 (m, 11H), 2.97 - 2.86 (m, 1H), 2.10 (s, 6H), 2.01 - 1.85 (m, 2H), 1.57 (s, 3H), 1.40 (s, 3H). The configuration of this structure was assigned according to the configuration of the raw material used in step 6.

### Embodiment 81 Synthesis of (R)-8-acryloyl-4-chloro-1-((S)-4-(3-fluoroazetidin-1-yl)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12-one (Z480-1) and (R)-8-acryloyl-4-chloro-1-((R)-4-(3-fluoroazetidin-1-yl)-2,2-dimethylpyrrolidin-1-yl)-3-(2-fluorophenyl)-6,6a,7, 8,9,10-hexahydro-12H-pyrazino[2,1-c]pyrido[3,4-f][1,4]oxazepin-12-one (Z480-2)

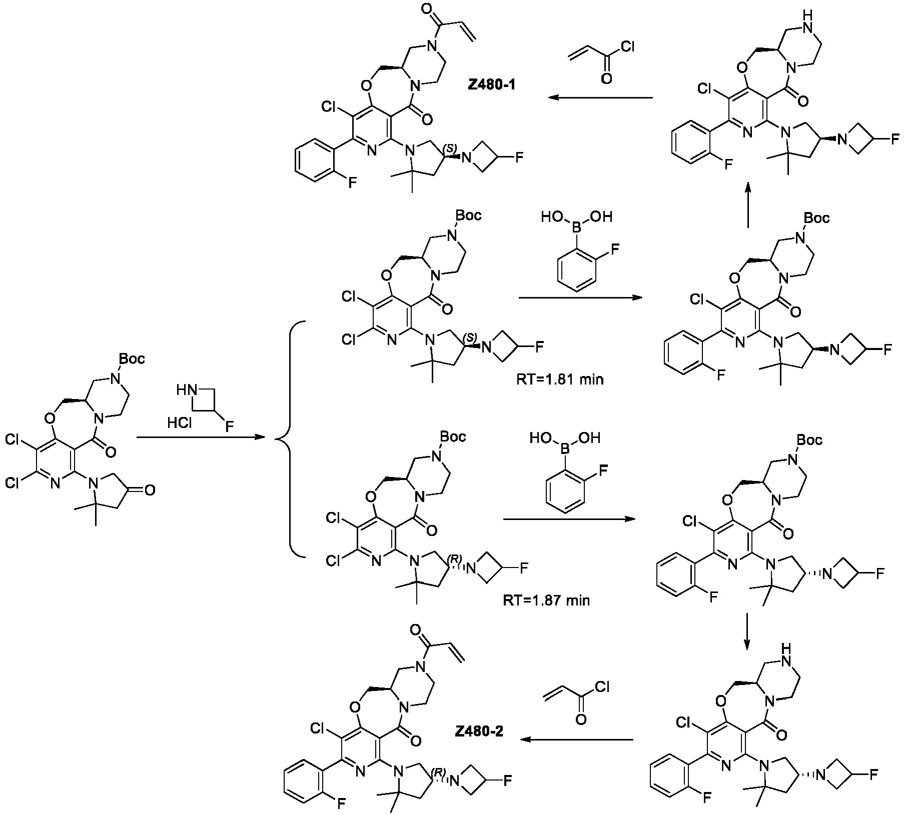

Step 1: *tert*-Butyl (*R*)-3,4-dichloro-1-(2,2-dimethyl-4-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (350 mg, 0.70 mmol), 3-fluoroazetidine hydrochloride (158 mg, 1.40 mmol) and acetonitrile (5 mL) were added to a 100 mL flask, stirred at room temperature for 2 hours. Then sodium cyanoborohydride (88 mg, 1.40 mmol) was added thereto, and the mixture was continued to be stirred for 1 hour. The reaction solution was quenched with saturated ammonium chloride solution, extracted with ethyl acetate, dried and concentrated, and the crude product was purified by flash silica gel column to obtain two isomers: one isomer with a structure arbitrarily designated as *tert*-butyl (*R*)-3,4-dichloro-1-((*S*)-4-(3-fluoroazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (retention time: 1.81 min, 100 mg, yield of 25%). ES-API: [M+H]⁺=558.2; and another isomer with a structure arbitrarily designated as *tert*-butyl (*R*)-3,4-dichloro-1-((*R*)-4-(3-fluoroazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (retention time: 1.87 min, 100 mg, yield of 25%), ES-API: [M+H]⁺=558.2.

Step 2: *tert*-Butyl (*R*)-3,4-dichloro-1-((*S*)-4-(3-fluoroazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate ((retention time: 1.81 min) (100 mg, 0.18 mmol), (2-fluorophenyl)boronic acid (50 mg, 0.36 mmol), tetrakis(triphenylphosphine)palladium (40 mg, 0.03 mmol), potassium carbonate (75 mg, 0.54 mmol), 2 mL of dioxane and 0.4 mL of water were added to a 5 mL microwave reaction tube. Under nitrogen protection, the reaction was carried out at 120°C for 1 hour under microwave irradiation. The reaction solution was added with ethyl acetate, washed with saturated brine for 3 times, dried and concentrated, and the crude product was purified by flash silica gel column to obtain *tert*-butyl (*R*)-4-chloro-1-((*S*)-4-(3-fluoroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (50 mg, yield of 45%). ES-API: [M+H]⁺=618.3.

Step 3: *tert*-Butyl (*R*)-4-chloro-1-((*S*)-4-(3-fluoroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (50 mg, 0.08 mmol), 1 mL of trifluoroacetic acid and 2 mL of dichloromethane were added to a 50 mL flask. The mixture was stirred at room temperature for 1 hour, and the completion of reaction was detected by LC-MS. The reaction solution was concentrated to obtain a crude product of (*R*)-4-chloro-1-((*S*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f]*[1,4]oxazepin-12-one trifluoroacetate (42 mg, crude product) as a yellow oil. ES-API: [M+H]⁺=518.3.

Step 4: (*R*)-4-Chloro-1-((*S*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one trifluoroacetate (42 mg, crude product), 10 mL of dichloromethane and diisopropylethylamine (52 mg, 0.40 mmol) were added to a 50 mL flask. At 0°C, a solution of acryloyl chloride in dichloromethane (7 mg, 0.08 mmol, 1 mL) was added dropwise thereto. The reaction was carried out at 0°C for 10 minutes. The mixture was washed with 20 mL of water, and the organic phase was dried and concentrated, and the crude product was purified by preparative HPLC to obtain (*R*)-8-acryloyl-4-chloro-1-((*S*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z480-1, 18 mg, 2-step yield of 39%) as a white solid. ES-API: [M+H]⁺=572.2. ¹H NMR (500 MHz, DMSO-*d*₆) δ 7.56 - 7.48 (m, 1H), 7.44 (t, *J* = 7.0 Hz, 1H), 7.37 - 7.28 (m, 2H), 6.75 (dd, *J =* 16.5, 10.5 Hz, 1H), 6.22 - 6.10 (m, 1H), 5.79 - 5.68 (m, 1H), 5.29 - 5.05 (m, 1H), 4.50 (s, 1H), 4.43 - 4.26 (m, 1H), 4.22 - 3.76 (m, 4H), 3.68 - 3.44 (m, 5H), 3.24 - 3.02 (m, 4H), 2.92 (s, 1H), 1.93 - 1.85 (m, 1H), 1.58 - 1.52 (m, 1H), 1.51 (s, 3H), 1.44 (s, 3H). The configurations of the product compounds from step 2 to step 4 were assigned according to the configuration of the raw materials used in step 2.

Step 5: Referring to step 2 to step 4, (*R*)-8-acryloyl-4-chloro-1-((*R*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z480-2, 18 mg, 3-step yield of 39%) as a white solid was synthesized from *tert-*butyl (*R*)-3,4-dichloro-1-((*R*)-4-(3-fluoroazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate. ES-API: [M+H]⁺=572.2. ¹H NMR (500 MHz, DMSO-*d*₆) δ 7.54 - 7.48 (m, 1H), 7.47 - 7.42 (m, 1H), 7.35 - 7.29 (m, 2H), 6.77 (dd, *J=* 17, 10.5 Hz, 1H), 6.15 (t, *J=* 19.0 Hz, 1H), 5.79 - 5.65 (m, 1H), 5.08 (d, *J* = 61.0 Hz, 1H), 4.60 - 4.48 (m, 1H), 4.43 - 4.26 (m, 1H), 4.25 - 3.86 (m, 4H), 3.79 (s, 1H), 3.61 - 3.37 (m, 5H), 3.14 - 2.85 (m, 3H), 2.65 - 2.53 (m, 1H), 1.92 - 1.81 (m, 1H), 1.80 - 1.72 (m, 1H), 1.69 (s, 3H), 1.39 (s, 3H). The configuration of the product compound of this step was assigned according to the configuration of the raw material used in this step.

### Embodiment 82 Synthesis of (R)-8-acryloyl-4-chloro-3-(2-fluorophenyl)-1-((S)-4-(3-methoxyazetidin-1-yl)-2,2-dimethylpyrrolidin-1-yl)-6,6a, 7, 8,9,10-hexahydro-12H-pyrazino [2,1-c]pyrido[3,4-f][1,4]oxazepin-12-one (Z479-1) and (R)-8-acryloyl-4-chloro-3-(2-fluorophenyl)-1-((R)-4-(3-methoxyazetidin-1-yl)-2,2-dimethylpyrrolidin-1-yl)-6,6a, 7, 8,9,10-hexahydro-12H-pyrazino [2,1-c]pyrido[3,4-f][1,4]oxazepin-12-one (Z479-2)

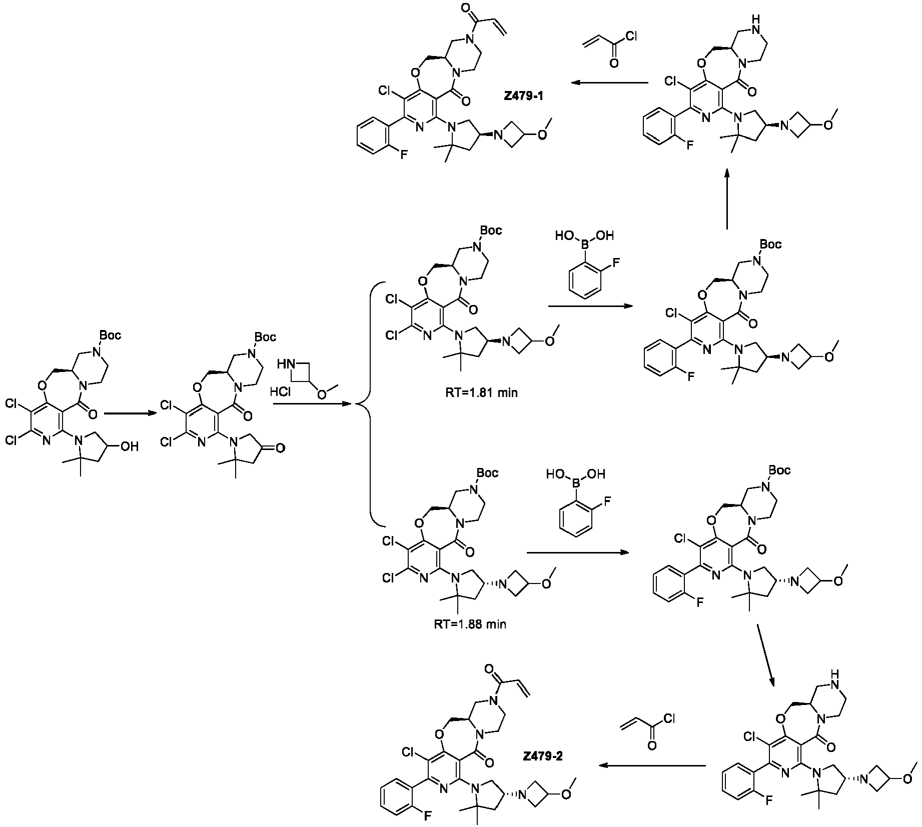

Step 1: *tert*-Butyl (6a*R*)-3,4-dichloro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (1.2 g, 2.4 mmol) and 10 mL of DCM were added to a 100 mL single-necked flask, and a solution of DMP in dichloromethane (2.5 g, 5.8 mmol, 10 mL) was added dropwise thereto at 0°C. The mixture was stirred at room temperature for 1 hour, washed with 20 mL of water. The organic phase was dried, then concentrated, and subjected to column chromatography to obtain *tert*-butyl (*R*)-3,4-dichloro-1-(2,2-dimethyl-4-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (0.8 g, 1.6 mmol, yield of 66.7%), ES-API: [M+H]⁺=499.1.

Step 2: *tert*-Butyl (*R*)-3,4-dichloro-1-(2,2-dimethyl-4-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (400 mg, 0.80 mmol), 3-methoxyazetidine hydrochloride (312 mg, 2.40 mmol) and acetonitrile (5 mL) were added to a 50 mL flask, stirred at room temperature for 2 hours. Then sodium cyanoborohydride (88 mg, 1.40 mmol) was added thereto, and the mixture was continued to be stirred for 1 hour. The reaction solution was quenched with saturated ammonium chloride solution, extracted with ethyl acetate, dried and concentrated, and the crude product was purified by flash silica gel column to obtain two isomers: one isomer with a structure arbitrarily designated as *tert*-butyl (*R*)-3,4-dichloro-1-((*S*)-4-(3-methoxyazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (large polarity, retention time: 1.81 min, 93 mg, yield of 20%) and another isomer with a structure arbitrarily designated as *tert*-butyl (*R*)-3,4-dichloro-1-((*R*)-4-(3-methoxyazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (small polarity, retention time: 1.88 min, 100 mg, yield of 22%). ES-API: [M+H]⁺=570.3.

Step 3: *tert*-Butyl (*R*)-3,4-dichloro-1-((*S*)-4-(3-methoxyazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (large polarity, retention time: 1.81 min) (93 mg, 0.16 mmol), (2-fluorophenyl)boronic acid (50 mg, 0.36 mmol), tetrakis(triphenylphosphine)palladium (40 mg, 0.03 mmol), potassium carbonate (66 mg, 0.48 mmol), 2 mL of dioxane and 0.4 mL of water were added to a 5 mL microwave reaction tube. Under nitrogen protection, the reaction was carried out at 120°C for 1 hour under microwave irradiation. The reaction solution was added with ethyl acetate, washed with saturated brine for 3 times, dried and concentrated, and the crude product was purified by flash silica gel column to obtain *tert*-butyl (*R*)-4-chloro-1-((*S*)-4-(3-methoxyazetidin-1-yl)-2,2-dimethylpyrrolidin-1-yl)-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (88 mg, yield of 87%). ES-API: [M+H]⁺=630.3.

Step 4: *tert*-Butyl (*R*)-4-chloro-1-((*S*)-4-(3-methoxyazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (88 mg, 0.17 mmol), 1 mL of trifluoroacetic acid and 2 mL of dichloromethane were added to a 50 mL flask. The mixture was stirred at room temperature for 1 hour, and the completion of reaction was detected by LC-MS. The reaction solution was concentrated to obtain a crude product of (*R*)-4-chloro-1-((S)-4-(3-methoxyazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one trifluoroacetate (90 mg, crude product) as a yellow oil. ES-API: [M+H]⁺=530.3.

Step 5: (*R*)-4-Chloro-1-((*S*)-4-(3-methoxyazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one trifluoroacetate (90 mg, crude product), 10 mL of dichloromethane and diisopropylethylamine (100 mg, 0.77 mmol) were added to a 50 mL flask. At 0°C, a solution of acryloyl chloride in dichloromethane (15 mg, 0.17 mmol, 1 mL) was added dropwise thereto. The reaction was carried out at 0°C for 10 minutes. The mixture was washed with 20 mL of water, and the organic phase was dried and concentrated, and the crude product was purified by preparative HPLC to obtain (*R*)-8-acryloyl-4-chloro-1-((*S*)-4-(3-methoxyazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z479-1, 33 mg, 2-step yield of 40%) as a white solid. ES-API: [M+H]⁺=584.3. ¹H NMR (500 MHz, DMSO-*d*₆) δ 7.55 - 7.40 (m, 2H), 7.37 - 7.27 (m, 2H), 6.81 - 6.69 (m, 1H), 6.16 (t, *J* = 17.6 Hz, 1H), 5.74 (d, *J* = 10.7 Hz, 1H), 4.51 (s, 1H), 4.42 - 4.26 (m, 1H), 4.24 - 3.38 (m, 10H), 3.30 (s, 1H), 3.17 (d, *J* = 26.6 Hz, 4H), 3.04-2.75 (m, 4H), 1.86 (dd, *J* = 11.5, 5.8 Hz, 1H), 1.51-1.41 (m, 6H). The configurations of the product compounds from step 3 to step 5 were assigned according to the configuration of the raw materials used in step 2.

Step 6: *tert*-Butyl (*R*)-3,4-dichloro-1-((*R*)-4-(3-methoxyazetidin-1-yl)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (small polarity, retention time: 1.88 min) was used to replace *tert*-butyl (*R*)-3,4-dichloro-1-((*S*)-4-(3-methoxyazetidin-1-yl)-2,2-dimethylpyrrolidin-1-yl)-12-oxo-6a,7,9,10-tetrahydro-12*H*-pyrazino[2,1-*c*]pyrido[3,41-*f*][1,4]oxazepine-8(6*H*)-carboxylate (large polarity, retention time: 1.81 min), and (*R*)-8-acryloyl-4-chloro-1-((*R*)-4-(3-methoxyazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z479-2, 4 mg, 2-step yield of 5%) as a white solid was synthesized. ES-API: [M+H]⁺=584.3. The configuration of the product compound of this step was assigned according to the configuration of the raw material used in this step.

### Embodiment 83 Synthesis of 1-((6aR)-8-acryloyl-4-chloro-3-(2-fluoro-6-hydroxyphenyl)-12-oxo-6,6a,7,8,9,10-hexahydro-12H-pyrazino[2,1-c]pyrido[3,4-f][1,4]oxazepin-1-yl)-5,5-dimethylpyrrolidine-3-carboxylic acid (Z458)

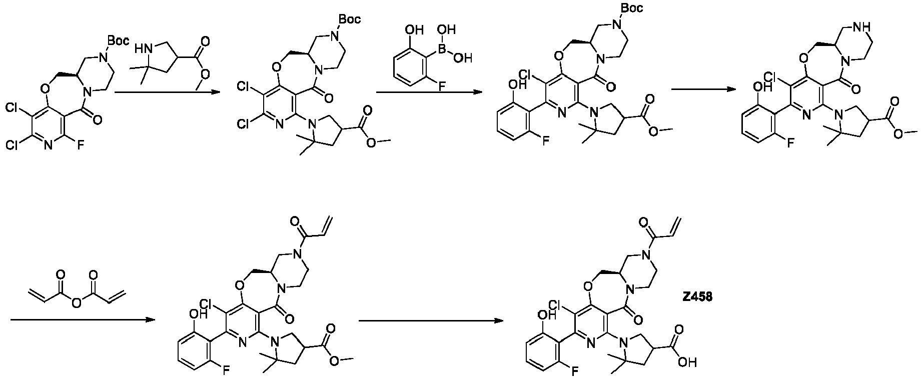

Step 1: *tert*-Butyl (*R*)-3,4-dichloro-1-fluoro-12-oxo-6a,7,9,10-tetrahydro-12*H*-pyrazino[2,1-*c*]pyrido[3,41-*f*][1,4]oxazepine-8(6*H*)-carboxylate (201 mg, 0.5 mmol), potassium carbonate (220 mg, 1.6 mmol), methyl 5,5-dimethylpyrrolidine-3-carboxylate (120 mg, 0.75 mmol) were dissolved in acetonitrile (3 mL) and reacted at 80°C for 5 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain compound *tert*-butyl (*R*)-3,4-dichloro-1,fluoro-(4-methoxycarbonyl)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (220 mg, yield: 81%).

Step 2: *tert*-Butyl (*R*)-3,4-dichloro-1,fluoro-(4-methoxycarbonyl)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (220 mg, 0.405 mmol) , 2-fluoro-6-hydroxyphenylboronic acid (94 mg, 0.607 mmol) and potassium carbonate (112 mg, 0.81 mmol) were dissolved in 1,4-dioxane:water (5:1, 6 mL). The reaction was bubbled with argon at room temperature for 10 minutes, then added with tetrakis(triphenylphosphine)palladium (47 mg, 0.04 mmol), and the reaction was reacted at 110°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation to obtain a crude product of compound *tert*-butyl (6a*R*)-4-chloro-3-(2-fluoro-6-hydroxyphenyl)-1-(4-(methoxycarbonyl)-2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (280 mg, yield: 100%). ES-API: [M+H]⁺= 619.3.

Step 3: *tert*-Butyl (6a*R*)-4-chloro-3-(2-fluoro-6-hydroxyphenyl)-1-(4-(methoxycarbonyl)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (280 mg, 0.453 mmol) was dissolved in dichloromethane (2 mL), and then trifluoroacetic acid (2 mL) was added dropwise thereto, stirred at room temperature for 0.5 hours. After the reaction was completed, the mixture was evaporated to dryness by rotary evaporation to obtain a crude product of methyl 1-((6a*R*)-4-chloro-3-(2-fluoro-6-hydroxyphenyl)-12-oxo-6a,7,8,9,10,12-6*H-*pyrazino[2,1-*c*]pyrido[3,4-*f*][1,4]oxazepin-1-yl)-5,5-dimethylpyrrolidine-3-carboxylate (180 mg, yield: 77%). ES-API: [M+H]⁺= 519.2.

Step 4: Methyl 1-((6a*R*)-4-chloro-3-(2-fluoro-6-hydroxyphenyl)-12-oxo-6a,7,8,9,10,12-6*H-*pyrazino[2,1-*c*]pyrido[3,4-*f*][1,4]oxazepin-1-yl)-5,5-dimethylpyrrolidine-3-carboxylate (180 mg, 0.25 mmol) was dissolved in tetrahydrofuran (2 mL), and saturated sodium bicarbonate solution was added dropwise thereto at 0°C to adjust the pH value to 8-9. An aqueous solution (0.5 mL) of potassium phosphate (27 mg, 0.127 mmol) was added thereto. After 5 minutes of reaction, a solution of acrylic anhydride (22 mg, 0.34 mmol) in tetrahydrofuran was added thereto, and the reaction was detected by thin-layer chromatography plate until the reaction of the raw materials was complete, and then quenched with saturated sodium bicarbonate solution. The reaction was stirred at room temperature for 10 minutes, extracted with ethyl acetate, washed once with brine, dried over anhydrous sodium sulfate, and the filtrate was evaporated to dryness by rotary evaporation to obtain a crude product of methyl 1-((6a*R*)-8-acryloyl-4-chloro-3-(2-fluoro-6-hydroxyphenyl)-12-oxo-6a,7,8,9,10,12-6*H*-pyrazino[2,1-*c*]pyrido[3,4-*f*][1,4]oxazepin-1-yl)-5,5-dimethylpyrrolidine-3-carboxylate (160 mg, yield: 82%). ES-API: [M+H]⁺= 573.2

Step 5: Methyl 1-((6a*R*)-8-acryloyl-4-chloro-3-(2-fluoro-6-hydroxyphenyl)-12-oxo-6a,7,8,9,10,12-6*H*-pyrazino[2,1-*c*]pyrido[3,4-*f*][1,4]oxazepin-1-yl)-5,5-dimethylpyrrolidine-3-carboxylate (160 mg, 0.28 mmol) and lithium hydroxide (47 mg, 1.12 mmol) were dissolved in dichloromethane:tetrahydrofuran (3:1, 4 mL), stirred at room temperature for 1 hour. After the reaction was completed, the mixture was evaporated to dryness by rotary evaporation to obtain a crude product, and the crude product was purified by preparative HPLC to obtain 1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidine-3-carboxylic acid (Z458, 40 mg, yield: 27%). ¹H NMR (400 MHz, MeOH-*d₄*) δ 7.19 (t, *J* = 6.2 Hz, 1H), 6.79 - 6.51 (m, 3H),6.29 (d, J= 16.6 Hz, 1H), 5.81 (d, *J* = 10.3 Hz, 1H), 4.37 - 4.09 (m, 5H), 3.83 -3.43 (m, 5H), 3.12 (d, *J* = 6.4 Hz, 1H), 2.39 - 2.02 (m, 3H), 1.59 - 1.47 (m, 6H). ES-API: [M+H]⁺= 559.2.

### Embodiment 84 Synthesis of (R)-8-acryloyl-4-chloro-1-((S)-2,4-dimethylpiperazin-1-yl)-3-(prop-1-yn-1-yl)-6,6a,7,8,9,10-hexahydro-12H-pyrazino[2,1-c]pyrido[3,4-f][1,4]oxazepin-12-one (Z459)

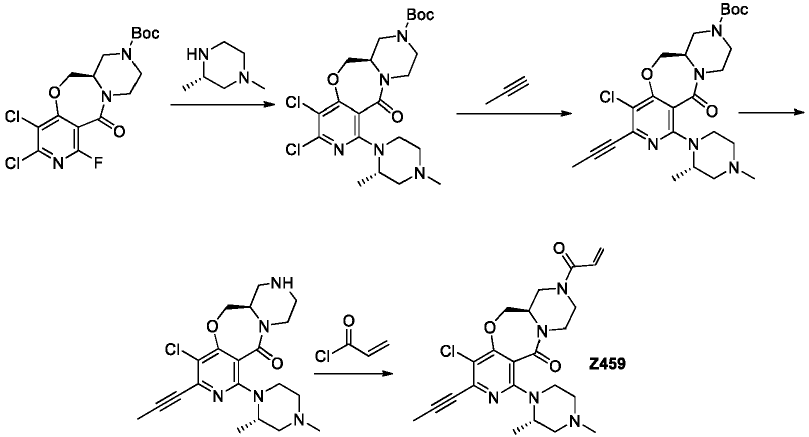

Step 1: *tert*-Butyl (*R*)-3,4-dichloro-1-fluoro-12-oxo-6a,7,9,10-tetrahydro-12*H*-pyrazino[2,1-*c*]pyrido[3,41-*f*][1,4]oxazepine-8(6*H*)-carboxylate (1 g) and (S)-1,3-dimethylpiperazine (285 mg) were dissolved in ACN (7 mL), and then potassium carbonate (1 g) was added thereto, and the reaction was stirred at room temperature for 16 hours. After the reaction was completed, the mixture was quenched with water, extracted with ethyl acetate, washed once with brine, dried over anhydrous sodium sulfate, and the filtrate was evaporated to dryness by rotary evaporation and then purified by column chromatography (PE/EA=3/1, R_{f}=0.4) to obtain *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (yellow solid, 830 mg, yield: 66.4%). ES-API: [M+H]⁺=500.1

Step 2: *tert*-Butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (210 mg) and 1-propyne (1 N THF solution) (4.3 mL) were mixed in a 10 mL microwave tube, and then tetrakis(triphenylphosphine)palladium (200 mg) and diisopropylamine (4.5 mL) were added thereto, and the reaction was reacted at 120°C for 6 hours under microwave irradiation. After the reaction was completed, the mixture was extracted with water and ethyl acetate, washed once with brine, dried over anhydrous sodium sulfate, and the filtrate was evaporated to dryness by rotary evaporation and purified by preparative thin-layer chromatography plate (PE/EA=3/1, R_{f}=0.4) to obtain *tert*-butyl (*R*)-4-chloro-1-((*S*)-2,4-dimethylpiperazin-1-yl)-12-oxo-3-(prop-1-yn-1-yl)-6a,7,9,10-tetrahydro-12*H*-pyrazino[2,1-*c*]pyrido[3,4-*f*][1,4]oxazepine-8(6*H*)-carboxylate (yellow solid, 75 mg, Y=33.3%). ES-API: [M+H]⁺=504.1

Step 3: *tert*-Butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (75 mg) were dissolved in DCM (1 mL), and TFA (1 mL) was added thereto, and the reaction was stirred at room temperature for 0.5 hours. After the reaction was completed, the mixture was extracted with water and ethyl acetate, washed once with brine, dried over anhydrous sodium sulfate, and the filtrate was evaporated to dryness by rotary evaporation to obtain a crude product of (*R*)-4-chloro-1-((*S*)-2,4-dimethylpiperazin-1-yl)-3-(prop-1-yn-1-yl)-6,6a,7,8,9,10-hexahydro-12*H*-pyrazino[2,1-*c*]pyrido[3,4-*f*][1,4]oxazepin-12-one (yellow solid, 36 mg, yield: 59.5%). ES-API: [M+H]⁺=404.1

Step 4: (*R*)-4-Chloro-1-((*S*)-2,4-dimethylpiperazin-1-yl)-3-(prop-1-yn-1-yl)-6,6a,7,8,9,10-hexahydro-12*H*-pyrazino[2,1-*c*]pyrido[3,4-*f*][1,4]oxazepin-12-one (36 mg) was first dissolved in tetrahydrofuran (1 mL) and maintained at 0°C for 5 minutes, then sodium bicarbonate solution was added thereto to maintain the pH at 8-9. Then potassium phosphate solution (9 mg, 0.045 mmol) was added thereto and stirred at 0°C for 5 minutes. Finally, acryloyl chloride (8 mg, 0.09 mmol) was slowly added thereto and stirred at 0°C for 0.5 hours. After the reaction was completed, the mixture was quenched by adding sodium bicarbonate solution, then extracted by adding ethyl acetate, washed once with brine, dried over anhydrous sodium sulfate. The filtrate was evaporated to dryness by rotary evaporation, and then the crude product was purified by preparative thin-layer chromatography plate (PE/EA=2/1, R_{f}=0.5) to obtain (*R*)-8-acryloyl-4-chloro-1-((*S*)-2,4-dimethylpiperazin-1-yl)-3-(prop-1-yn-1-yl)-6,6a,7,8,9,10-hexahydro-12*H*-pyrazino[2,1-*c*]pyrido[3,4-*f*][1,4]oxazepin-12-one (Z459, pale yellow solid, 7.3 mg, yield: 17.7%). ¹H NMR (400 MHz, CDCl₃) δ 6.58-6.51 (m, 1H), 6.38 (s, 1H), 5.80 (s, 1H),4.30(s,1H),4.12 (s, 1H), 4.00 (s, 1H), 3.52(d, *J* = 13.5 Hz, 3H), 3.38 (t, *J* = 12.4 Hz,3H), 2.78 (d, *J* = 11.0 Hz, 2H), 2.65 (d, *J* =11.3 Hz, 2H), 2.14 (s, 6H), 1.40 (s, 3H),1.25 (s, 3H). ES-API: [M+H]⁺=458.1.

### Embodiment 85 Synthesis of (R)-8-acryloyl-4-chloro-1-((S)-4-(dimethylamino)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12-one (Z453-1) and (R)-8-acryloyl-4-chloro-1-((R)-4-(dimethylamino)-2,2-dimethylpyrrolidin-1-yl)-3-(2-fluorophenyl)-6,6a,7,8,9,10-hexahydro-12H-pyrazino [2,1-c]pyrido [3,4-f][1,4]oxazepin-12-one (Z453-2)

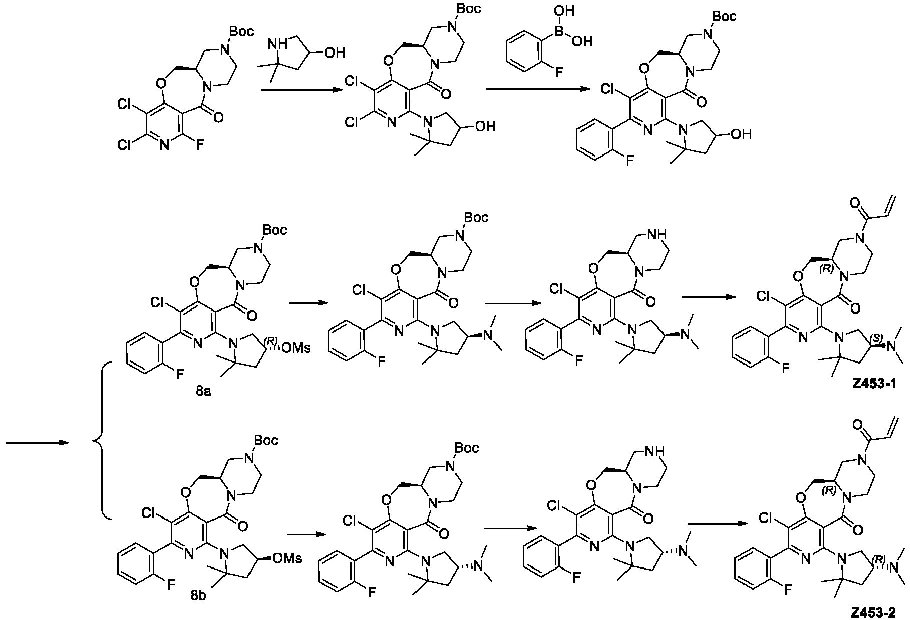

Step 1: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (800 mg, 1.970 mmol), 5,5-dimethylpyrrolidin-3-ol (250 mg, 2.167 mmol) and potassium carbonate (817 mg, 5.91 mmol) were dissolved in acetonitrile (6 mL), reacted at room temperature for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation, and the crude product was purified by flash chromatography (ethyl acetate:petroleum ether: 0-80%) to obtain *tert*-butyl (6a*R*)-3,4-dichloro-1-(4-hydroxy-2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (824 mg, yield: 83%). ES-API: [M+H]⁺= 501.26.

Step 2: *tert*-Butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (820 mg, 1.635 mmol), (2-fluorophenyl)boronic acid (275 mg, 1.962 mmol), tetrakis(triphenylphosphine)palladium (189 mg, 0.164 mmol) and potassium carbonate (451 mg, 3.27 mmol) were dissolved in a mixed solution of dioxane and water (5 mL:1 mL), and the reaction solution was stirred at 110°C overnight. After the reaction was completed, the mixture was extracted with ethyl acetate, washed once with brine, dried over anhydrous sodium sulfate, the filtrate was evaporated to dryness by rotary evaporation, and the crude product was purified by flash chromatography (ethyl acetate:petroleum ether: 0-85%) to obtain *tert*-butyl (6aR)-4-chloro-3-(2-fluorophenyl)-1-(4-hydroxy-2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (600 mg, yield: 65%). ES-API: [M+H]⁺= 561.3.

Step 3: *tert*-Butyl (6a*R*)-4-chloro-3-(2-fluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (600 mg, 1.071 mmol) was dissolved in 6 mL of dichloromethane, then methanesulfonyl chloride (244 mg, 2.142 mmol) and triethylamine (325 mg, 3.213 mmol) were added thereto. The reaction solution was stirred at room temperature for 2 hours. After the reaction was completed, the mixture was quenched with water, extracted with dichloromethane, washed once with brine, dried over anhydrous sodium sulfate, and the filtrate was evaporated to dryness by rotary evaporation. The crude product was prepared by column chromatography to obtain two isomers:

one isomer with a structure arbitrarily designated as *tert*-butyl (*R*)-4-chloro-1-((*R*)-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (8a (PE:EA=1:2, R_{f}=0.5), 460 mg, yield: 67%). ES-API: [M+H]⁺= 639.3.

another isomer with a structure arbitrarily designated as *tert*-butyl (*R*)-4-chloro-1-((*S*)-2,2-dimethyl-4-((methylsulfonyl)oxy)pyrrolidin-1-yl)-3-(2-fluorophenyl)-12-oxo-6a,7,9,10-tetrahydro-6*H-*pyrazino[2,1-*c*]pyrido[3,4-*f*][1,4]oxazepine-8(12*H*)-carboxylate (8b (PE:EA=1:2, R_{f}=0.7), 45 mg, yield: 7%). ES-API: [M+H]⁺= 639.3.

Step 4: *tert*-Butyl (*R*)-4-chloro-1-((*R*)-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (350 mg, 0.548 mmol) was dissolved in 8 mL of aqueous dimethylamine solution (40%wt), and then *N,N*-diisopropylethylamine (354 mg, 2.739 mmol) was added thereto. The reaction solution was heated to 100°C, then stirred for 16 hours to complete the reaction. Then the reaction solution was cooled to room temperature, extracted by adding ethyl acetate, washed once with brine, dried over anhydrous sodium sulfate, and the filtrate was evaporated to dryness by rotary evaporation, and the crude product was purified by column chromatography (dichloromethane/methanol=20/1) to obtain *tert*-butyl (*R*)-4-chloro-1-((*S*)-4-(dimethylamino)-2,2-dimethylpyrrolidin-1-yl)-3-(2-fluorophenyl)-12-oxo-6a,7,9,10-tetrahydro-6*H-*pyrazino[2,1-*c*]pyrido[3,4-*f*][1,4]oxazepine-8(12*H*)-carboxylate (180 mg, yield: 56%). ES-API: [M+H]⁺= 588.3.

Step 5: *tert*-Butyl (*R*)-4-chloro-1-((*S*)-4-(dimethylamino)-2,2-dimethylpyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (180 mg, 0.306 mmol) was dissolved in dichloromethane (4 mL), then cooled to 0°C in an ice-water bath, and then trifluoroacetic acid (2 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (*R*)-4-chloro-1-((*S*)-4-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (170 mg, yield: 95%). ES-API: [M+H]⁺= 488.3.

Step 6: (*R*)-4-Chloro-1-((*S*)-4-(dimethylamino)-2,2-dimethylpyrrolidin-1-yl)-3-(2-fluorophenyl)-7,8,9,10-tetrahydro-6*H*-pyrazino[2,1-*c*]pyrido[3,4-*f*][1,4]oxazepin-12(6a*H*)-one (170 mg, 0.348 mmol) was dissolved in dichloromethane (4 mL), then cooled to 0°C in an ice-water bath, and triethylamine (105 mg, 1.044 mmol) and acryloyl chloride (35 mg, 0.383 mmol) were added dropwise thereto, stirred for 30 minutes. After the reaction was completed, the mixture was added with sodium bicarbonate solution to adjust to alkaline, extracted with dichloromethane, dried over sodium sulfate, evaporated to dryness by rotary evaporation, and the crude product was purified by thin-layer chromatography plate (dichloromethane/methanol=10/1, R_{f}=0.4) to obtain (*R*)-8-acryloyl-4-chloro-1-((*S*)-4-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z453-1, 44.9 mg, yield: 24%). ¹H NMR (400 MHz, CDCl₃) δ 7.40 (t, *J* = 7.0 Hz, 2H), 7.22 (t, *J* = 7.7 Hz, 1H), 7.16 (d, *J* = 9.5 Hz, 1H), 6.57 (s, 1H), 6.40 (d, *J* = 13.1 Hz, 1H), 5.80 (d, *J* = 11.1 Hz, 1H), 4.44 (s, 1H), 4.42 - 4.21 (m, 3H), 4.16 (d, *J* = 10.4 Hz, 1H), 3.97 (s, 1H), 3.80 - 3.49 (m, 4H), 3.11 (d, *J =* 38.8 Hz, 2H), 2.40 (s, 6H), 1.93 (d, *J =* 9.9 Hz, 2H), 1.58 (d, *J =* 18.7 Hz, 6H). ES-API: [M+H]⁺= 542.3.

Step 7: *tert*-Butyl (*R*)-4-chloro-1-((*S*)-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (PE:EA=1:2, R_{f}=0.7) was used to replace *tert*-butyl (*R*)-4-chloro-1-((*R*)-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (PE:EA=1:2, R_{f}=0.5). Referring to step 4, step 5 and step 6, (*R*)-8-acryloyl-4-chloro-1-((*R*)-4-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one was synthesized (Z453-2, 6.9 mg, yield: 18%). ES-API: [M+H]⁺= 542.3. ¹H NMR (500 MHz, DMSO-*d*₆) δ 7.54-7.49 (m, 1H), 7.46-7.42 (m, 1H), 7.37-7.27 (m, 2H), 6.79-6.75 (m, 1H), 6.19-6.12 (m, 1H), 5.78-5.68 (m, 2H), 4.56-4.31 (m, 2H), 4.17-3.84 (m, 3H), 3.71-3.55 (m, 3H),3.11-2.97 (m, 2H), 2.11 (s, 6H), 2.04-1.86 (m, 3H), 1.62 (s, 3H), 1.41 (s, 3H).

The configurations of Z453-1 and Z453-2 were assigned according to the structures of intermediates 8a and 8b.

### Embodiment 86 Synthesis of (R)-8-acryloyl-4-chloro-3-(2,3-difluorophenyl)-1-((S)-4-(dimethylamino)-2,2-dimethylpyrrolidin-1-yl)-6,6a,7,8,9,10-hexahydro-12H-pyrazino[2,1-c]pyrido[3,4-f][1,4]oxazepin-12-one (Z473-1) and (R)-8-acryloyl-4-chloro-3-(2,3-difluorophenyl)-1-((R)-4-(dimethylamino)-2,2-dimethylpyrrolidin-1-yl)-6,6a,7,8,9,10-hexahydro-12H-pyrazino[2,1-c]pyrido[3,4-f][1,4]oxazepin-12-one (Z473-2)

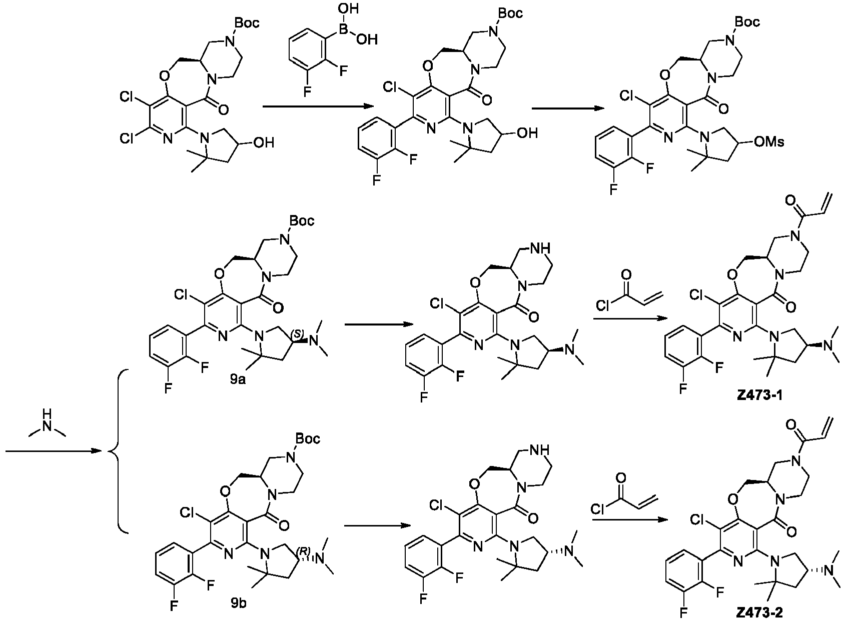

Step 1: *tert*-Butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (100 mg, 0.2 mmol), (2,3-difluorophenyl)boronic acid (38 mg, 0.24 mmol), tetrakis(triphenylphosphine)palladium (23 mg, 0.02 mmol) and potassium carbonate (55 mg, 0.4 mmol) were dissolved in a mixed solution of dioxane and water (5 mL:1 mL), and the reaction solution was stirred at 110°C overnight. After the reaction was completed, the mixture was extracted with ethyl acetate, washed once with brine, dried over anhydrous sodium sulfate, and the filtrate was evaporated to dryness by rotary evaporation, and the crude product was purified by flash chromatography (ethyl acetate:petroleum ether: 0-85%, R_{f}=0.4) to obtain *tert*-butyl (6a*R*)-4-chloro-3-(2,3-difluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (60 mg, yield: 52.1%). ES-API: [M+H]⁺= 579.3.

Step 2: *tert*-Butyl (6a*R*)-4-chloro-3-(2,3-difluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (100 mg, 0.17 mmol) was dissolved in 5 mL of dichloromethane, then methanesulfonyl chloride (25 mg, 0.22 mmol) and triethylamine (34 mg, 0.34 mmol) were added thereto. The reaction solution was stirred at room temperature for 2 hours. After the reaction was completed, the mixture was quenched by adding water, extracted with dichloromethane, washed once with brine, dried over anhydrous sodium sulfate, and the filtrate was evaporated to dryness by rotary evaporation, and concentrated to obtain a crude product of compound *tert*-butyl (*R*)-4-chloro-1-(2,2-dimethyl-4-((methylsulfonyl)oxy)pyrrolidin-1-yl)-3-(2,3-difluorophenyl)-12-oxo-6a,7,9,10-tetrahydro-6*H*-pyrazino[2,1-*c*]pyrido[3,4-*f*][1,4]oxazepine-8(12*H*)-carboxylate (100 mg, crude product). ES-API: [M+H]⁺= 657.3.

Step 3: *tert*-Butyl (*R*)-4-chloro-1-(2,2-dimethyl-4-((methylsulfonyl)oxy)pyrrolidin-1-yl)-3-(2,3-difluorophenyl)-12-oxo-6a,7,9,10-tetrahydro-6*H*-pyrazino[2,1-*c*]pyrido[3,4-*f*][1,4]oxazepine-8(12*H*)-carboxylate (80 mg, 0.12 mmol) was dissolved in 4 mL of aqueous dimethylamine solution (40%wt). The reaction solution was heated to 100°C, and then stirred for 16 hours. After the reaction was completed, the reaction solution was cooled to room temperature, extracted with ethyl acetate, washed once with brine, dried over anhydrous sodium sulfate, and the filtrate was evaporated to dryness by rotary evaporation. The crude product was purified by preparative thin-layer chromatography plate to obtain two isomers:

One isomer with a structure arbitrarily designated as *tert*-butyl (*R*)-4-chloro-3-(2,3-difluorophenyl)-1-((*S*)-4-(dimethylamino)-2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (9a (dichloromethane/methanol=10/1, R_{f}=0.6), 19 mg, yield: 28.3%), ES-API : [M+H]⁺= 606.3.

Another isomer with a structure arbitrarily designated as *tert*-butyl (R)-4-chloro-3-(2,3-difluorophenyl)-1-((*R*)-4-(dimethylamino)-2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (9b (dichloromethane/methanol= 10/1, R_{f}=0.4), 18 mg, yield: 26.8%), ES-API: [M+H]⁺= 606.3.

Step 4: tert-Butyl (*R*)-4-chloro-3-(2,3-difluorophenyl)-1-((*S*)-4-(dimethylamino)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (19 mg, 0.170 mmol) was dissolved in dichloromethane (2 mL), then cooled to 0°C in an ice-water bath, and then trifluoroacetic acid (1 mL) was added dropwise thereto, stirred for 1 hour. After the reaction was completed, the mixture was evaporated to dryness by rotary evaporation to obtain (*R*)-4-chloro-1-((*S*)-4-(dimethylamino)-2,2-dimethylpyrrolidin-1-yl)-3-(2,3-difluorophenyl)-7,8,9,10-tetrahydro-6*H*-pyrazino[2,1-*c*]pyrido[3,4-*f*][1,4]oxazepin-12-one (15 mg, crude product). ES-API: [M+H]⁺= 506.2.

Step 5: (*R*)-4-Chloro-1-((*S*)-4-(dimethylamino)-2,2-dimethylpyrrolidin-1-yl)-3-(2,3-difluorophenyl)-7,8,9,10-tetrahydro-6*H*-pyrazino[2,1-*c*]pyrido[3,4-*f*][1,4]oxazepin-12-one (15 mg, 0.03 mmol) was dissolved in tetrahydrofuran (2 mL), then cooled to 0°C in an ice-water bath. Saturated sodium bicarbonate solution was added thereto to adjust the pH to 8-9, and 15 mg of potassium phosphate was added thereto, stirred for 5 minutes, and then acryloyl chloride (4 mg, 0.045 mmol) was added thereto, stirred for 30 minutes. After the reaction was completed, the mixture was extracted with ethyl acetate, dried over sodium sulfate, evaporated to dryness by rotary evaporation, and the crude product was purified by chromatography plate (dichloromethane/methanol=10/1, R_{f}=0.6) to obtain (*R*)-8-acryloyl-4-chloro-1-((*S*)-4-(dimethylamino)-2,2-dimethylpyrrolidin-1-yl)-3-(2,3-difluorophenyl)-6,6a,7,8,9,10-hexahydro-12*H-*pyrazino[2,1-*c*]pyrido[3,4-*f*][1,4]oxazepin-12-one (Z473-1, 2 mg, yield: 11.9%). ES-API: [M+H]⁺= 560.2.

*tert*-Butyl (*R*)-4-chloro-3-(2,3-difluorophenyl)-1-((*R*)-4-(dimethylamino)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (dichloromethane/methanol=10/1, R_{f}=0.4) was used to replace *tert*-butyl (*R*)-4-chloro-3-(2,3-difluorophenyl)-1-((*S*)-4-(dimethylamino)-2,2-dimethylpyrrolidin-1-yl)-12-oxo-6a,7,9,10-tetrahydro-6*H-*pyrazino[2,1-*c*]pyrido[3,4-*f*][1,4]oxazepine-8(12*H*)-carboxylate (dichloromethane/methanol=10/1, R_{f}=0.6). Referring to step 4 and step 5, (*R*)-8-acryloyl-4-chloro-1-((*R*)-4-(dimethylamino)-2,2-dimethylpyrrolidin-1-yl)-3-(2,3-difluorophenyl)-6,6a,7,8,9,10-hexahydro-12*H*-pyrazino[2,1-*c*]pyrido[3,4-*f*][1,4]oxazepin-12-one was synthesized (Z473-2, 1.5 mg, yield: 8.9%). ES-API: [M+H]⁺= 560.2

The configurations of Z473-1 and Z473-2 were assigned according to the structures of intermediates 9a and 9b.

### Embodiment 87 Synthesis of (R)-8-acryloyl-4-chloro-1-((S)-4-(dimethylamino)-2,2-dimethylpyrrolidin-1-yl)-3-(2-fluoro-3-methylphenyl)-6,6a,7,8,9,10-hexahydro-12H-pyrazino[2,1-c]pyrido[3,4-f][1,4]oxazepin-12-one (Z472-1) and (R)-8-acryloyl-4-chloro-1-((R)-4-(dimethylamino)-2,2-dimethylpyrrolidin-1-yl)-3-(2-fluoro-3-methylphenyl)-6,6a,7,8,9,10-hexahydro-12H-pyrazino[2,1-c]pyrido[3,4-f][1,4]oxazepin-12-one (Z472-2)

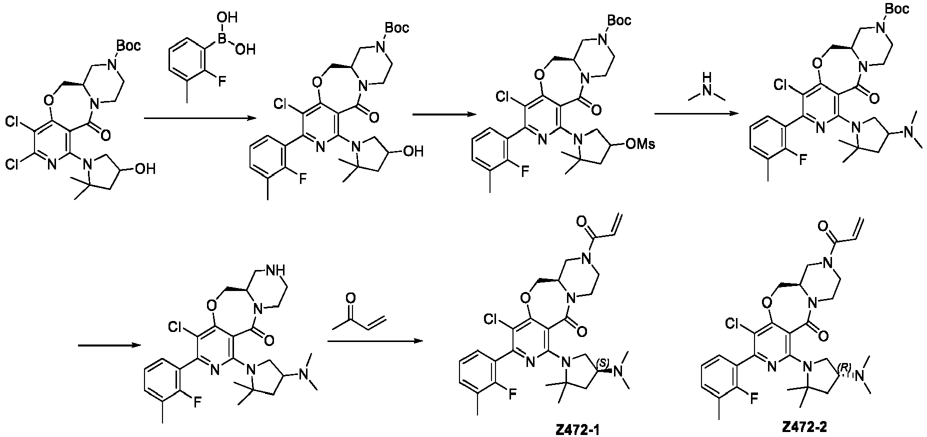

Step 1: *tert*-Butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (200 mg, 0.4 mmol), (2-fluoro-3-methylphenyl)boronic acid (94 mg, 0.6 mmol), tetrakis(triphenylphosphine)palladium (46 mg, 0.04 mmol) and potassium carbonate (110 mg, 0.8 mmol) were dissolved in a mixed solution of dioxane and water (10 mL:2 mL), and the reaction solution was stirred at 100°C overnight. After the reaction was completed, the mixture was extracted with ethyl acetate, washed once with brine, dried over anhydrous sodium sulfate, the filtrate was evaporated to dryness by rotary evaporation, and the crude product was purified by flash chromatography (methanol:dichloromethane: 0-5%, R_{f}=0.6) to obtain *tert*-butyl (6a*R*)-4-chloro-3-(2-fluoro-3-methylphenyl)-1-(4-hydroxy-2,2-dimethylpyrrolidin-1-yl)-12-oxo-6a,7,9,10-tetrahydro-6*H*-pyrazino[2,1-*c*][pyrido[3,4-*f*][1,4]oxazepine-8(12*H*)-carboxylate (123 mg, yield: 52.5%). ES-API: [M+H]⁺= 575.2

Step 2: *tert*-Butyl (6a*R*)-4-chloro-3-(2-fluoro-3-methylphenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (200 mg, 0.34 mmol) was dissolved in 10 mL of dichloromethane, then methanesulfonyl chloride (46 mg, 0.40 mmol) and triethylamine (104 mg, 1.02 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation, and concentrated to obtain a crude product of compound *tert*-butyl (6a*R*)-4-chloro-3-(2-fluoro-3-methylphenyl)-1-(2,2-dimethyl-4-((methylsulfonyl)oxy)pyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (100 mg, crude product). ES-API: [M+H]⁺= 653.2.

Step 3: *tert*-Butyl (6a*R*)-4-chloro-3-(2-fluoro-3-methylphenyl)-1-(2,2-dimethyl-4-((methylsulfonyl)oxy)pyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (100 mg, 0.16 mmol) was dissolved in 4 mL of aqueous dimethylamine solution (40%wt). The reaction solution was heated to 100°C, then stirred for 16 hours. After the reaction was completed, the reaction solution was cooled to room temperature, extracted by adding ethyl acetate, washed once with brine, dried over anhydrous sodium sulfate, and the filtrate was evaporated to dryness by rotary evaporation, and the crude product was purified by column chromatography (dichloromethane/methanol=10/1, R_{f}=0.6) to obtain *tert*-butyl (*R*)-4-chloro-3-(2-fluoro-3-methylphenyl)-1-(4-(dimethylamino)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (40 mg, yield: 44%). ES-API: [M+H]⁺= 602.3

Step 5: *tert*-Butyl (*R*)-4-chloro-3-(2-fluoro-3-methylphenyl)-1-(4-(dimethylamino)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12H)-carboxylate (100 mg, 0.170 mmol) was dissolved in dichloromethane (2 mL), then cooled to 0°C in an ice-water bath, and then trifluoroacetic acid (1 mL) was added dropwise thereto, stirred for 1 hour. After the reaction was completed, the mixture was evaporated to dryness by rotary evaporation to obtain (*R*)-4-chloro-1-(4-(dimethylamino)-2,2-dimethylpyrrolidin-1-yl)-3-(2-fluoro-3-methylphenyl)-7,8,9,10-tetrahydro-6*H*-pyrazino[2,1-*c*]pyrido[3,4-*f*][1,4]oxazepin-12-one (100 mg, crude product). ES-API: [M+H]⁺= 502.2.

Step 6: (*R*)-4-Chloro-1-(4-(dimethylamino)-2,2-dimethylpyrrolidin-1-yl)-3-(2-fluoro-3-methylphenyl)-6,6a,7,8,9,10-hexahydro-6*H*-pyrazino[2,1-*c*]pyrido[3,4-*f*][1,4]oxazepin-12-one (100 mg, 0.045 mmol) was dissolved in tetrahydrofuran (2 mL), then cooled to 0°C in an ice-water bath. Saturated sodium bicarbonate solution was added thereto to adjust the pH to 8-9. 15 mg of potassium phosphate (200 mg) was added thereto and stirred for 5 minutes, and then acryloyl chloride (27 mg, 0.045 mmol) was added thereto and stirred for 30 minutes. After the reaction was completed, the mixture was extracted with ethyl acetate, dried over sodium sulfate, evaporated to dryness by rotary evaporation, and the crude product was purified by chromatographic plate to obtain two isomers:

One isomer with a structure arbitrarily designated as (*R*)-8-acryloyl-4-chloro-1-((*S*)-4-(dimethylamino)-2,2-dimethylpyrrolidin-1-yl)-3-(2-fluoro-3-methylphenyl)-6,6a,7,8,9,10-hexahydro-12*H-*pyrazino[2,1-*c*]pyrido[3,4-*f*][1,4]oxazepin-12-one (Z472-1 (dichloromethane/methanol=10/1, R_{f}=0.6), 19 mg, yield: 17.1%). ¹H NMR (400 MHz, CDCl₃) δ7.21 (dd, *J* = 16.8, 7.5 Hz, 2H), 7.09 (t,*J* = 7.6 Hz, 1H), 6.56 (s, 1H), 6.39 (d,*J* = 16.4 Hz, 1H), 5.80 (d, *J* = 10.4 Hz,1H), 4.46 - 3.88 (m, 7H), 3.64 (d, *J* =59.9 Hz, 3H), 3.10-3.00 (m,2H), 2.43 - 2.30 (m, 9H), 2.03 (m, 2H), 1.65 (s, 3H), 1.49 (s, 3H). ES-API: [M+H]⁺= 556.3.

Another isomer with a structure arbitrarily assigned as (*R*)-8-acryloyl-4-chloro-1-((*R*)-4-(dimethylamino)-2,2-dimethylpyrrolidin-1-yl)-3-(2-fluoro-3-methylphenyl)-6,6a,7,8,9,10-hexahydro-12*H-*pyrazino[2,1-*c*]pyrido[3,4-*f*][1,4]oxazepin-12-one (Z472-2 (dichloromethane/methanol=10/1, R_{f}=0.4), 20 mg, yield: 18%). ¹H NMR (400 MHz, CDCl₃) δ 7.26 - 7.16 (m, 2H), 7.09 (t, *J* = 7.6 Hz, 1H), 6.55 (s, 1H), 6.39 (d, *J* = 16.5 Hz, 1H), 5.80 (d, *J* = 10.4 Hz, 1H), 4.57 - 4.00 (m, 7H), 3.77 - 3.53 (m, 3H), 3.01 (s, 2H), 2.34 (s, 9H), 1.97 - 1.86 (m, 2H), 1.57 (d, *J* = 12.0 Hz, 6H). ES-API: [M+H]⁺= 556.3.

### Embodiment 88 Synthesis of (R)-8-acryloyl-4-fluoro-3-(2-fluorophenyl)-1-((S)-4-hydroxy-2,2-dimethylpyrrolidin-1-yl)-6,6a,7,8,9,10-hexahydro-2H-pyrazino[2,1-c]pyrido[3,4-f][1,4]oxazepin-12-one (Z408-1)

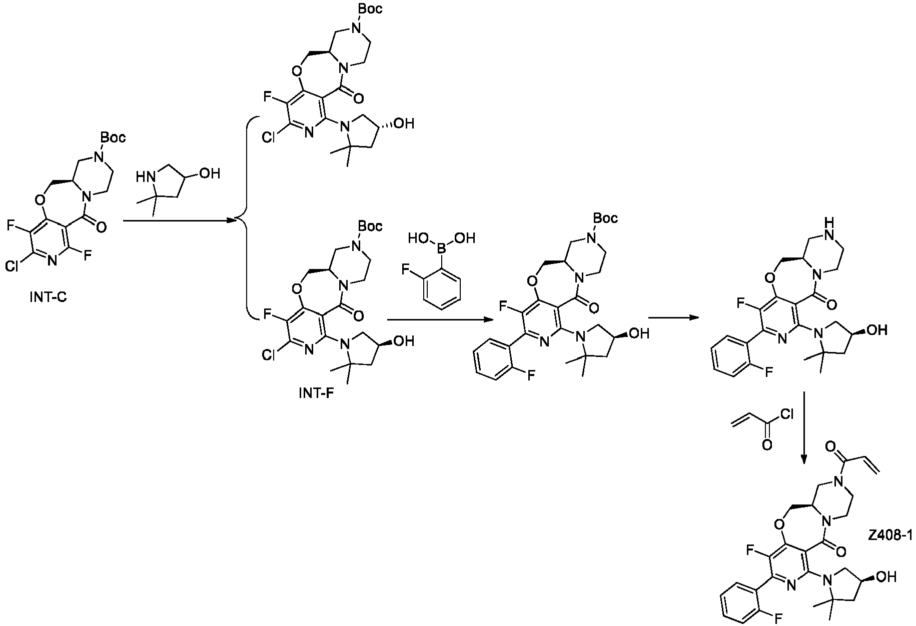

Step 1: *tert*-Butyl (*R*)-3-chloro-1,4-difluoro-12-oxo-6a,7,9,10-tetrahydro-12*H*-pyrazino[2,1-*c*]pyrido[3,41-*f*][1,4]oxazepine-8(6*H*)-carboxylate (3 g, 7.7 mmol), 5,5-dimethylpyrrolidin-3-ol (1.06 g, 9.2 mmol), potassium carbonate (3.1 g, 22.4 mmol) and 15 mL of acetonitrile were added to a 100 mL sealed tube, stirred at 100°C for 3 hours. The mixture was extracted with water and ethyl acetate, and the organic phase was dried, then concentrated and subjected to chiral resolution (IG column, 250 mm ^{∗} 4.6 mm ^{∗} 5 µm; mobile phase: *n*-hexane:ethanol =60:40) to obtain two isomer compounds: one isomer with a structure arbitrarily designated as *tert*-butyl (*R*)-3-chloro-4-fluoro-1-((*R*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (retention time: 4.883 min, 960 mg, yield of 26%) and another isomer with a structure arbitrarily designated as *tert-*butyl (*R*)-3-chloro-4-fluoro-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (INT-F, retention time: 6.687 min, 830 mg, yield of 22%), ES-API: [M+H]⁺=485.2.

Step 2: *tert*-Butyl (*R*)-3-chloro-4-fluoro-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (peak 2, 49 mg, 0.1 mmol), (2-fluorophenyl)boronic acid (56 mg, 0.4 mmol), tetrakis(triphenylphosphine)palladium (12 mg, 0.01 mmol), potassium carbonate (40 mg, 0.29 mmol), 6 mL of dioxane and 1 mL of water were added to a 50 mL single-necked flask. Under nitrogen protection, the reaction was carried out at 100°C for 3 hours. The reaction solution was added with ethyl acetate, washed with saturated brine for 3 times, dried and concentrated, and the crude product was purified by flash silica gel column to obtain *tert*-butyl (*R*)-4-fluoro-3-(2-fluorophenyl)-1-((*S*)-4-hydroxy-2,2-dimethylpyrrolidin-1-yl)-12-oxo-6a,7,9,10-tetrahydro-12*H-*pyrazino[2,1-*c*]pyrido[3,4-*f*][1,4]oxazepine-8(6*H*)-carboxylate (40 mg, yield of 73%). ES-API: [M+H]⁺=545.2.

Step 3: *tert*-Butyl (*R*)-4-fluoro-3-(2-fluorophenyl)-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (40 mg, 0.07 mmol), 1 mL of trifluoroacetic acid and 2 mL of dichloromethane were added to a 50 mL flask. The mixture was stirred at room temperature for 1 hour, and the completion of reaction was detected by LC-MS. The reaction solution was concentrated to obtain a crude product of (*R*)-4-fluoro-3-(2-fluorophenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-/] [1,4]oxazepin-12-one trifluoroacetate (40 mg, crude product) as a yellow oil. ES-API: [M+H]⁺=445.2.

Step 4: The crude product of (*R*)-4-fluoro-3-(2-fluorophenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one trifluoroacetate, 10 mL of dichloromethane and diisopropylethylamine (50 mg, 0.39 mmol) were added to a 50 mL flask. At 0°C, a solution of acryloyl chloride in dichloromethane (6 mg, 0.06 mmol, 1 mL) was added dropwise thereto. The reaction was carried out at 0°C for 10 minutes. The mixture was washed with 20 mL of water, and the organic phase was dried and then concentrated, and the crude product was purified by preparative HPLC to obtain (*R*)-8-acryloyl-4-fluoro-3-(2-fluorophenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (12 mg, 2-step yield of 34%) as a white solid. ES-API: [M+H]⁺=499.2.

The configurations of the product compounds from step 2 to step 4 were assigned according to the configuration of the raw material INT-F used in step 2.

### Embodiment 88-1 Synthesis of Z414-1

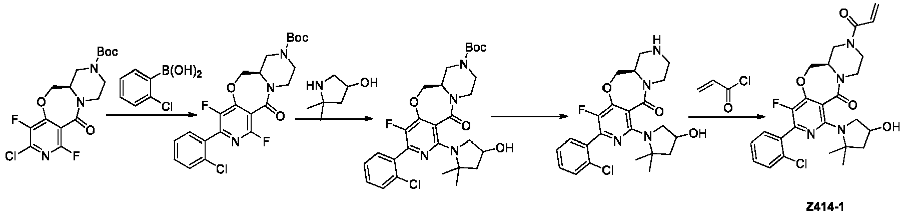

Step 1: (*R*)-3-Chloro-1,4-difluoro-12-oxo-6a,7,9,10-tetrahydro-12*H*-pyrazino[2,1-*c*]pyrido[3,4-*f*][1,4]oxazepine-8-(6*H*)-carboxylate (110 mg, 0.28 mmol), (2-chlorophenyl)boronic acid (140 mg, 0.9 mmol), tetrakis(triphenylphosphine)palladium (35 mg, 0.03 mmol), potassium carbonate (124 mg, 0.9 mmol), 6 mL of dioxane and 1 mL of water were added to a 50 mL single-necked flask. Under nitrogen protection, the reaction was carried out at 100°C for 3 hours. The reaction solution was added with ethyl acetate, washed with saturated brine for 3 times, dried and concentrated, and the crude product was purified by flash silica gel column (ethyl acetate:petroleum ether: 0-90%) to obtain *tert*-butyl (*R*)-1,4-difluoro-3-(2-chlorophenyl)-12-oxo-6a,7,9,10-tetrahydro-12*H*-pyrazino[2,1-*c*]pyrido[3,4-*f*][1,4]oxazepine-8(6*H*)-carboxylate (90 mg, yield of 69%). ES-API: [M+H]⁺=466.1.

Step 2: *tert*-Butyl (*R*)-1,4-difluoro-3-(2-chlorophenyl)-12-oxo-6a,7,9,10-tetrahydro-12*H-*pyrazino[2,1-*c*]pyrido[3,4-*f*][1,4]oxazepine-8(6*H*)-carboxylate (90 mg, 0.19 mmol), 2,2-dimethylpyrrolidine hydrochloride (136 mg, 1.0 mmol), DIPEA (516 mg, 4.0 mmol) and acetonitrile (10 mL) were added to a 50 mL round bottom flask, reacted at 90°C for 1 hour. The reaction solution was added with ethyl acetate (30 mL), washed with water (30 mL*3), and the organic phase was dried and concentrated, and the residue was purified by flash silica gel column (methanol:dichloromethane: 0-10%) to obtain *tert-*butyl (6a*R*)-3-(2-chlorophenyl)-4-fluoro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (70 mg, yield of 66%) as a solid. ES-API: [M+H]⁺=561.2.

Step 3: Trifluoroacetic acid (2 mL) was added to a solution of *tert*-butyl (6a*R*)-3-(2-chlorophenyl)-4-fluoro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (70 mg, 0.13 mmol) in dichloromethane (5 mL), reacted at room temperature for 1 hour. The reaction solution was directly concentrated to obtain a crude product of (6a*R*)-3-(2-chlorophenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (55 mg) as an oil. ES-API: [M+H]⁺=461.2.

Step 4: The above crude product of (6a*R*)-3-(2-chlorophenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (55 mg, 0.12 mmol) was dissolved in dichloromethane (2 mL), and *N,N*-diisopropylethylamine (46 mg, 0.36 mmol) was added thereto. The reaction solution was cooled to 0°C, and acryloyl chloride (9 mg, 0.10 mmol) was added dropwise thereto. After stirring for 5 minutes, the mixture was added with methanol (2 mL) to quench and then concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-3-(2-chlorophenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (22 mg, yield: 36%). ES-API: [M+H]⁺=515.1. ¹H NMR (400 MHz, DMSO-*d*₆) δ 7.68 - 7.29 (m, 4H), 6.78 (d, *J* = 10.4 Hz, 1H), 6.18 (d, *J* = 16.6 Hz, 1H), 5.75 (s, 1H), 5.08 (d, *J* = 5.8 Hz, 1H), 4.59 (s, 1H), 4.42 - 3.40 (m, 8H), 3.31-3.03 (m, 3H), 1.99 (dd, *J* = 11.2, 6.2 Hz, 1H), 1.72 (t, *J* = 11.1 Hz, 1H), 1.46 (t, *J* = 15.8 Hz, 6H)

### Embodiment 88-2 Synthesis of Z450

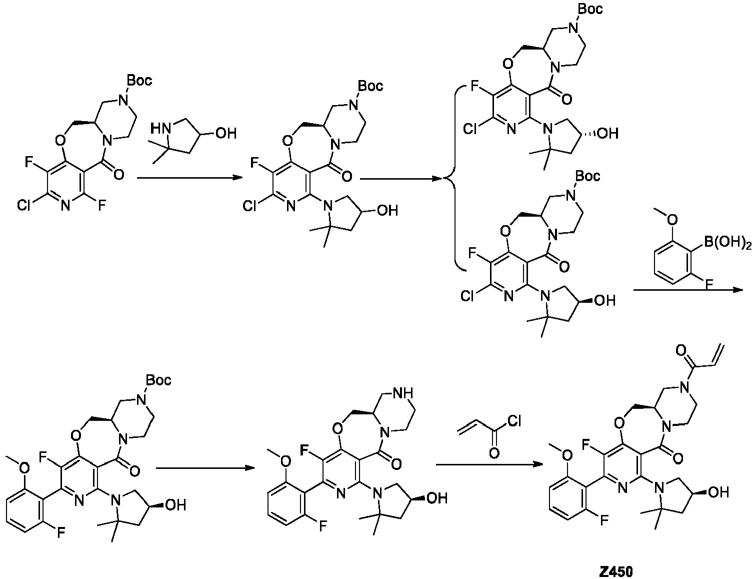

Step 1: 5,5-Dimethylpyrrolidin-3-ol (4.6 g, 40.0 mmol) and potassium carbonate (15.1 g, 110 mmol) were added to a solution of *tert*-butyl (*R*)-3-chloro-1,4-difluoro-12-oxo-6a,7,9,10-tetrahydro-12*H-*pyrazino[2,1-*c*]pyrido[3,4-*f*][1,4]oxazepine-8(6*H*)-carboxylate (14.3 g, 36.7 mmol) in acetonitrile (200 mL), reacted at 100°C for 2.5 hours. The reaction solution was dissolved in ethyl acetate (500 mL), washed with water (300 mL * 3), and the organic phase was dried over anhydrous sodium sulfate, concentrated to obtain *tert*-butyl (6a*R*)-3-chloro-4-fluoro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (16 g, yield of 90%) as a solid. ES-API: [M+H]⁺=485.2.

Step 2: Compound *tert*-butyl (6a*R*)-3-chloro-4-fluoro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (16 g, 33.0 mmol) was subjected to chiral separation by SFC (separation column: IG 250 mm^{∗}4.6 mm^{∗}5 µm; mobile phase: ethanol:*n*-hexane=40:60; flow rate: 1 mL/min; column temperature: 30°C) to obtain two isomer compounds:

One with a structure arbitrarily designated as *tert*-butyl (*R*)-3-chloro-4-fluoro-1-((*R*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (P1, 7.3 g, yield: 46%, retention time: 4.91 min, purity: 99%, ee value: 100%). ES-API: [M+H]⁺=485.2

Aonther one with a structure arbitrarily designated as *tert*-butyl (*R*)-3-chloro-4-fluoro-1-((*S*-4-hydroxy-2,2-dimethylpyrrolidin-1-yl)-12-oxo-6a,7,9,10-tetrahydro-12*H*-pyrazino [2,1-*c*]pyrido [3,4-*f*][1,4]oxazepine-8(6*H*)-carboxylate (P2, 6.9 g, yield: 43%, retention time: 6.747 min, purity: 99%, ee value: 100%). ES-API: [M+H]⁺=485.2

Step 3: *tert*-Butyl (*R*)-3-chloro-4-fluoro-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (240 mg, 0.5 mmol), (2-fluoro-6-methoxyphenyl)boronic acid (240 mg, 1.5 mmol), tetrakis(triphenylphosphine)palladium (60 mg, 0.05 mmol), potassium carbonate (210 mg, 1.5 mmol), dioxane (10 mL) and water (1 mL) were added to a 50 mL single-necked flask. Under nitrogen protection, the reaction was carried out at 100°C for 4 hours. The reaction solution was added with ethyl acetate (30 mL), washed with water (30 mL^{∗}3), and the organic phase was dried and concentrated, and the residue was purified by flash silica gel column (methanol:dichloromethane: 0-10%) to obtain *tert*-butyl (6a*R*)-4-fluoro-3-(2-fluoro-6-methoxyphenyl)-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg, yield of 52%). ES-API: [M+H]⁺=575.5.

Step 4: Trifluoroacetic acid (1 mL) was added to a solution of *tert*-butyl (6a*R*)-4-fluoro-3-(2-fluoro-6-methoxyphenyl)-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (74 mg, 0.13 mmol) in dichloromethane (2 mL), reacted at room temperature for 1 hour. The mixture was concentrated under reduced pressure to obtain a crude product of (6a*R*)-4-fluoro-3-(2-fluoro-6-methoxyphenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (55 mg). ES-API: [M+H]⁺=475.2.

Step 5: The above crude product of (6a*R*)-4-fluoro-3-(2-fluoro-6-methoxyphenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (55 mg, 0.12 mmol) was dissolved in dichloromethane (2 mL), and *N,N-*diisopropylethylamine (46 mg, 0.36 mmol) was added thereto. The reaction solution was cooled to 0°C, and acryloyl chloride (9 mg, 0.10 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-fluoro-3-(2-fluoro-6-methoxyphenyl)-1-(4-hydroxy-2,2-dimethylpyrrolidin-1-yl)-6,6a,7,8,9,10-hexahydro-12*H*-pyrazino[2,1-*c*]pyrido[3,4*f*][1,4]oxazepin-12-one (30 mg, yield: 47%). ES-API: [M+H]⁺=529.2. ¹HNMR (400 MHz, DMSO-*d*₆) δ 7.47 (d, *J* = 7.0 Hz, 2H), 7.06 - 6.87 (m, 2H), 6.87 - 6.67 (m, 3H), 6.17 (d, *J* = 16.8 Hz, 1H), 5.76 (d, *J* = 9.2 Hz, 1H), 5.06 (d, *J* = 5.6 Hz, 1H), 4.60 (s, 1H), 3.76 (d, *J* = 11.8 Hz, 9H), 3.62 - 3.37 (m, 2H), 3.25 (s, 2H), 3.09 - 2.96 (m, 1H), 1.97 (dd, *J* = 11.4, 6.2 Hz, 1H), 1.71 (s, 1H), 1.45 (d, *J* = 3.8 Hz, 3H).

The configurations of the product compounds from step 3 to step 5 were assigned according to the configuration of the raw materials used in step 3.

### Embodiment 88-3 Synthesis of Z481

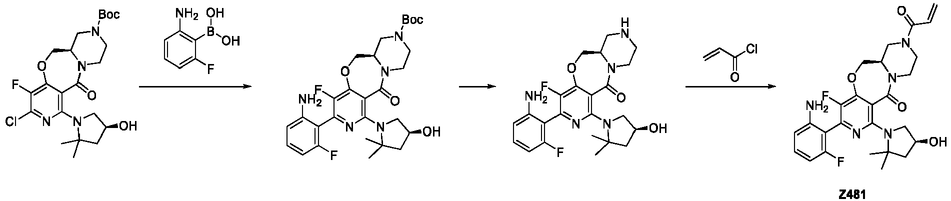

Step 1: Under nitrogen protection, a solution of compound *tert*-butyl (*R*)-3-chloro-4-fluoro-1-((*S*)-4-hydroxy-2,2-dimethylpyrrolidin-1-yl)-12-oxo-6a,7,9,10-tetrahydro-12*H*-pyrazino[2,1-*c*]pyrido [3,4-*f*][1,4]oxazepine-8(6*H*)-carboxylate (100 mg, 0.20 mmol), 2-fluoro-6-aminophenylboronic acid (96 mg, 0.61 mmol), tetrakis(triphenylphosphine)palladium (48 mg, 0.04 mmol) and potassium carbonate (86 mg, 0.61 mmol) in 1,4-dioxane (2 mL) and water (0.4 mL) was stirred at 100°C for 3 hours under microwave irradiation. The reaction solution was filtered and concentrated, purified by flash silica gel column (methanol:dichloromethane: 0-8%) to obtain *tert*-butyl (6a*R*)-3-(2-amino-6-fluorophenyl)-4-fluoro-1-((*S-*4-hydroxy-2,2-dimethylpyrrolidin-1-yl)-12-oxo-6a,7,9,10-tetrahydro-12*H*-pyrazino[2,1-*c*]pyrido [3,4-*f*][1,4]oxazepine-8(6*H*)-carboxylate (100 mg). ES-API: [M+H]⁺=560.3.

Step 2: Under an ice bath, a solution of *tert*-butyl (6a*R*)-3-(2-amino-6-fluorophenyl)-4-fluoro-1-((*S*)-4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg, 0.27 mmol) in dichloromethane (2 mL) was added with trifluoroacetic acid (1 mL), and the reaction solution was stirred at room temperature for 1 hour. The mixture was concentrated under reduced pressure to obtain a crude product of (6a*R*)-3-(2-amino-6-fhiorophenyl)-4-fhioro-1-((*S*)-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (123 mg). ES-API: [M+H]⁺=460.3.

Step 3: The above crude product of (6a*R*)-3-(2-amino-6-fluorophenyl)-4-fluoro-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one (123 mg, 0.27 mmol) was dissolved in dichloromethane (2 mL), and *N*,*N*-diisopropylethylamine(174 mg, 1.35 mmol) was added thereto. The reaction solution was cooled to 0°C, and acryloyl chloride (24 mg, 0.27 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-3-(2-amino-6-fluorophenyl)-4-fluoro-1-((*S*-4-hydroxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (80 mg, yield: 58%). ES-API: [M+H]⁺ = 514.3. ¹H NMR (500 MHz, DMSO-*d*₆) δ 7.11 (dd, *J* = 15.0, 8.0 Hz, 1H), 6.84 - 6.71 (m, 1H), 6.56 (d, *J* = 8.0 Hz, 1H), 6.40 (t, *J* = 9.5 Hz, 1H), 6.17 (d, *J* = 17.0 Hz, 1H), 5.80 - 5.68 (m, 1H), 5.33 (s, 1H), 5.16 (s, 1H), 5.06 (d, *J* = 5.5 Hz, 1H), 4.55 (s, 1H), 4.39 - 4.24 (m, 2H), 4.22 - 3.85 (m, 4H), 3.82 - 3.36 (m, 3H), 3.30 - 3.11 (m, 1H),3.04 (t, *J* = 8.5 Hz, 1H), 1.99 (dd, *J* = 11.2, 6.3 Hz, 1H), 1.73 (t, *J* = 11.0 Hz, 1H), 1.52 (s, 3H), 1.43 (s, 3H).

### Embodiment 89 Synthesis of two isomers (Z637-1 and Z637-2) of 1-(1-((R)-8-acryloyl-4-chloro-3-(2-fluorophenyl)-12-oxo-6,6a,7,8,9,10-hexahydro-12H-pyrazino[2,1-c]pyrido[3,4-f][1,4]oxazepin-1-yl)-5,5-dimethylpyrrolidin-3-yl)azetidine-3-carbonitrile

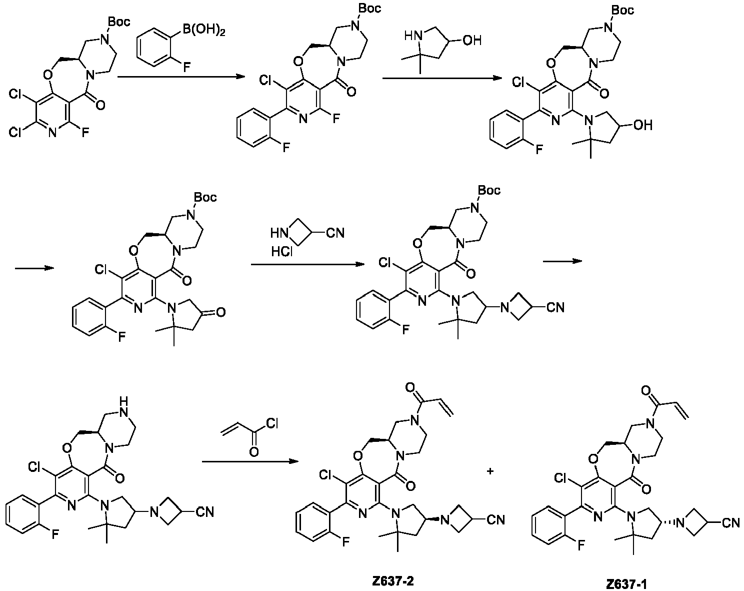

Step 1: Under nitrogen protection, a mixed solution of *tert*-butyl (R)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate(2 g, 4.92 mmol), (2-fluorophenyl)boronic acid (2.07 g, 14.8 mmol), tetrakis(triphenylphosphine)palladium (570 mg, 0.49 mmol) and potassium carbonate (2.04 g, 2.39 mmol) in 1,4-dioxane (20 mL) and water (4 mL) was reacted at 100°C for 3 hours. The reaction solution was dissolved in ethyl acetate (100 mL) and washed with saturated brine (50 mL) and water (50 mL), and the organic phase was dried over anhydrous sodium sulfate, concentrated and purified by flash silica gel column (ethyl acetate:petroleum ether: 0-80%) to obtain *tert*-butyl (*R*)-4-chloro-1-fluoro-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (2 g) with a yield of 87% as a white solid. ES-API: [M+H]⁺=466.1.

Step 2: 5,5-Dimethylpyrrolidin-3-ol (643 mg, 5.58 mmol) and potassium carbonate (1.78 g, 12.87 mmol) were added to a solution of *tert*-butyl (*R*)-4-chloro-1-fluoro-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (2 g, 4.29 mmol) in acetonitrile (20 mL), reacted at 100°C for 2 hours. The reaction solution was filtered, concentrated, then dissolved in ethyl acetate (100 mL), washed with water (50 mL ^{∗} 3), and the organic phase was dried over anhydrous sodium sulfate, concentrated and purified by flash silica gel column (ethyl acetate:petroleum ether: 0-80%) to obtain *tert*-butyl (6a*R*)-4-chloro-3-(2-fluorophenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (1.6 g) with ayield of 66% as a white solid. ES-API: [M+H]⁺=561.1.

Step 3: Under an ice-bath, Dess-Martin reagent (2.42 g, 5.7 mmol) was slowly added to a solution of *tert*-butyl (6a*R*)-4-chloro-3-(2-fluorophenyl)-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (1.6 g, 2.85 mmol) in dichloromethane (20 mL) and stirred at room temperature for 6 hours. The reaction solution was quenched with aqueous sodium thiosulfate solution (20 mL), then saturated sodium bicarbonate solution (20 mL) was added thereto, and extracted with dichloromethane (30 mL^{∗}3). The organic phases were combined, dried, concentrated and purified by flash silica gel column (ethyl acetate:petroleum ether: 0-70%) to obtain *tert*-butyl (*R*)-4-chloro-1-(2,2-dimethyl-4-oxopyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (450 mg) with a yield of 28%. ES-API: [M+H]⁺=559.1.

Step 4: A solution of *tert*-butyl (*R*)-4-chloro-1-(2,2-dimethyl-4-oxopyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (120 mg, 0.21 mmol) in acetonitrile (2 mL) was added with azetidine-3-carbonitrile hydrochloride (51 mg, 0.43 mmol), stirred at room temperature for 1 hour. Then sodium cyanoborohydride (27 mg, 0.43 mmol) was added thereto, and the mixture was continued to be stirred for 2 hours. After the reaction was completed, the mixture was quenched with saturated ammonium chloride solution (20 mL), extracted with ethyl acetate (20 mL ^{∗} 3), and the organic phases were combined, dried and concentrated to obtain a crude product of *tert*-butyl (6aR)-4-chloro-1-(4-(3-cyanoazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (120 mg). ES-API: [M+H]⁺=625.3.

Step 5: Under an ice-bath, a solution of *tert*-butyl (6a*R*)-4-chloro-1-(4-(3-cyanoazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (120 mg, 0.19 mmol) in dichloromethane (2 mL) was added with trifluoroacetic acid (1 mL), and the reaction solution was stirred at room temperature for 1 hour. The reaction solution was concentrated under reduced pressure to obtain a crude product of 1-(1-((*R*)-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)azetidine-3-carbonitrile (101 mg). ES-API: [M+H]⁺=525.2.

Step 6: The above crude procuct of 1-(1-((*R*)-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)azetidine-3-carbonitrile (101 mg, 0.19 mmol) was dissolved in dichloromethane (2 mL), and *N*,*N*-diisopropylethylamine (120 mg, 0.96 mmol) was added thereto. The reaction solution was cooled to 0°C, and acryloyl chloride (17mg, 0.19 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%= 20%-100%; column temperature: room temperature) to obtain two isomer compounds:

One isomer with a structure arbitrarily designated as 1-((*S*)-1-((*R*)-8-acryloyl-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)azetidine-3-carbonitrile (Z637-2, retention time: 7.32 min; 5 mg, yield: 5%). ES-API: [M+H]⁺=579.3. ¹H NMR (500 MHz, DMSO-*d*₆) δ 7.57 - 7.48 (m, 1H), 7.78 - 7.40 (m, 1H), 7.38 - 7.27 (m, 2H), 6.82 - 6.70 (m, 1H), 6.23 - 6.10 (m, 1H), 5.79 - 5.68 (m, 1H), 4.58 - 4.45 (m, 1H), 4.41 - 4.25 (m, 1H), 4.25 - 3.75 (m, 5H), 3.59 (s, 1H), 3.58 - 3.39 (m, 5H), 3.30 - 3.27 (m, 1H), 3.22 - 3.12 (m, 1H), 3.07 (s, 1H), 2.90 (s, 1H), 1.93 - 1.82 (m, 1H), 1.55 - 1.51 (m, 1H), 1.50 (s, 3H), 1.44 (s, 3H).

Another isomer with a structure arbitrarily designated as 1-((*R*)-1-((*R*)-8-acryloyl-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)azetidine-3-carbonitrile (Z637-1, retention time: 8.77 min; 15 mg, yield: 14%). ES-API: [M+H]⁺=579.3. ¹HNMR (500 MHz, DMSO-*d*₆) δ 7.56 - 7.48 (m, 1H), 7.48 - 7.41 (m, 1H), 7.36 - 7.28 (m, 2H), 6.82 - 6.72 (m, 1H), 6.16 (t, *J* = 17.0 Hz, 1H), 5.79 - 5.64 (m, 1H), 4.64 - 4.52 (m, 1H), 4.47 - 4.24 (m, 1H), 3.24 - 3.72 (m, 5H), 3.61 - 3.42 (m, 3H), 3.41 - 3.27 (m, 3H), 3.24 (s, 1H), 3.16 (s, 1H), 2.92 (s, 1H), 2.66 - 2.52 (m, 1H), 1.88 - 1.78 (m, 1H), 1.71 (s, 3H), 1.38 (s, 3H), 1.31 - 1.20 (m, 1H).

### Embodiment 90 Synthesis of (6aR)-8-acryloyl-4-chloro-1-(2,2-dimethyl-4-(methyl(2,2,2-trifluoroethyl)amino)pyrrolidin-1-yl)-3-(2-fluorophenyl)-6,6a,7,8,9,10-hexahydro-12H-pyrazino[2,1-c]pyrido[3,4-f][1,4]oxazepin-12-one and two isomers thereof (Z491a, Z491 and Z493)

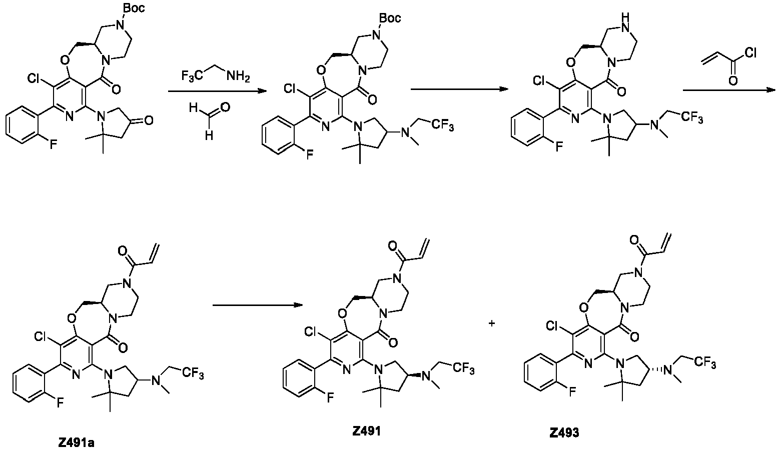

Step 1: A solution of compound *tert*-butyl (*R*)-4-chloro-1-(2,2-dimethyl-4-oxopyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (900 mg, 1.61 mmol) in methanol (20 mL) was added with 2,2,2-trifluoroethylamine (900 mg, 9.1 mmol), acetic acid (0.5 mL) and titanium tetraisopropanolate (2 mL), and stirred at room temperature for 2 hours. Then sodium cyanoborohydride (505.54 mg, 8.05 mmol) was added thereto, stirred for 16 hours, and then aqueous formaldehyde solution (5 mL) was slowly added thereto. The mixture was continued to be stirred for 2 hours. After the reaction was completed, the mixture was quenched with saturated ammonium chloride solution (50 mL), extracted with dichloromethane (50 mL ^{∗} 3), and the organic phases were combined, dried, concentrated and subjected to column chromatography (methanol:dichloromethane: 0-3%) to obtain *tert*-butyl (6a*R*)-4-chloro-1-(2,2-dimethyl-4-(methyl(2,2,2-trifluoroethyl)amino)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (900 mg, yield: 85%). ES-API: [M+H]⁺=656.2.

Step 2: Under an ice-bath, a solution of *tert*-butyl (6a*R*)-4-chloro-1-(2,2-dimethyl-4-(methyl(2,2,2-trifluoroethyl)amino)pyrrolidin-1-yl)-3-(2-fluorophenyl)-12-oxo-6a,7,9,10-tetrahydro-12*H*-pyrazino [2,1-*c*]pyrido[3,4-*f*][1,4]oxazepine-8(6*H*)-carboxylate (900 mg, 1.37 mmol) in dichloromethane (6 mL) was added with trifluoroacetic acid (3 mL), and the reaction solution was stirred at room temperature for 1 hour. The reaction solution was concentrated under reduced pressure to obtain a crude product of (6a*R*)-4-chloro-1-(2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (480 mg, crude product). ES-API: [M+H]⁺=556.3.

Step 3: The above crude procuct of (6a*R*)-4-chloro-1-(2,2-dimethyl-4-(methyl(2,2,2-trifhioroethyl)amino)pyrrolidin-1-yl)-3-(2-fhiorophenyl)-6,6a,7,8,9,10-hexahydro-12*H*-pyrazino[2,1-*c*]pyrido[3,4-*f*][1,4]oxazepin-12-one (480 mg, 0.86 mmol) was dissolved in dichloromethane (10 mL), and *N*,*N*-diisopropylethylamine (360 mg, 2.78 mmol) was added thereto. The reaction solution was cooled to 0°C, and acryloyl chloride (73 mg, 0.80 mmol) was added dropwise thereto. After stirring for 5 minutes, methanol (2 mL) was added thereto, and the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,2-dimethyl-4-(methyl(2,2,2-trifluoroethyl)amino)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one (Z491a, 283 mg, yield: 54%). ES-API: [M+H]⁺=610.3.

Step 4: Compound Z491a was subjected to chiral resolution (column: IC column: 250 mm^{∗}4.6 mm^{∗}5 µm; mobile phase A: *n*-hexane; mobile phase B: ethanol; flow rate: 1 mL/min; B%=30%; column temperature: 30°C) to obtain two isomer compounds:

One isomer with a structure arbitrarily designated as (*R*)-8-acryloyl-4-chloro-1-((*S*)-2,2-dimethyl-4-(methyl(2,2,2-trifluoroethyl)amino)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one (Z491, 200 mg, yield: 70%, retention time: 6.580 min, purity: 99%, ee value: 100%). ES-API: [M+H]⁺=610.3. ¹HNMR (500 MHz, DMSO-*d*₆) δ 7.55-7.49 (m, 1H), 7.47-7.42 (m, 1H), 7.38-7.30 (m, 2H), 6.74 (dd, *J* = 16.7, 10.6 Hz, 1H), 6.20-6.11 (m, 1H), 5.79-5.68 (m, 1H), 4.62 - 3.34 (m, 10H), 3.29 - 2.97 (m, 4H), 2.35 (s, 3H), 2.02 (dd, *J* = 11.3, 5.0 Hz, 1H), 1.72-1.65 (m, 1H), 1.51 (s, 3H), 1.47 (s, 3H).

Another isomer with a structure arbitrarily designated as (*R*)-8-acryloyl-4-chloro-1-((*R*)-2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z493, 25 mg, yield: 9%, retention time: 7.600 min, purity: 99%, ee value: 100%). ES-API: [M+H]⁺=610.3. ¹H NMR (500 MHz, DMSO-*d*₆) δ 7.55-7.49 (m, 1H), 7.48-7.43 (m, 1H), 7.35-7.29 (m, 2H), 6.86-6.68 (m, 1H), 6.16 (d, *J* = 16.6 Hz, 1H), 5.78-5.69 (m, 1H), 4.60-3.40 (m, 10H), 3.28 - 2.86 (m, 4H), 2.35 (s, 3H), 2.03-1.88 (m, 2H), 1.61 (s, 3H), 1.43 (s, 3H).

### Embodiment 91 Synthesis of (6aR)-8-acryloyl-3-(2-ainino-6-fluorophenyl)-4-chloro-1-((S)-2,4-dimcthylpipcrazin-1-yl)-6,6a,7,8,9,10-hexahydro-12H-pyrazino[2,1-c[pyrido|3.4-f][1,4|oxazepin-12-onc (Z503)

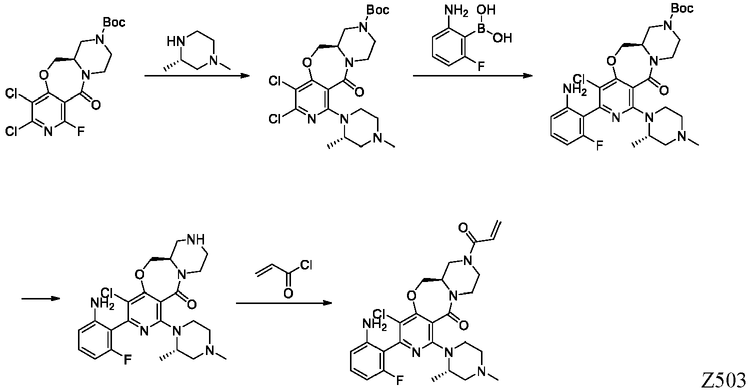

Step 1: (*S*)-1,3-Dimethylpiperazine (169 mg, 1.46 mmol) and potassium carbonate (510 mg, 3.69 mmol) were added to a solution of *tert*-butyl (*R*)-3,4-dichloro-1-fluoro-12-oxo-6a,7,9,10-tetrahydro-12*H-*pyrazino[2,1-*c*]pyrido[3,4-*f*][1,4*f*]oxazepine-8(6*H*)-carboxylate (500 mg, 1.23 mmol) in acetonitrile (5 mL), reacted at 100°C for 2 hours. The reaction solution was filtered, concentrated, then dissolved in ethyl acetate (30 mL), washed with water (15 mL ^{∗} 3), and the organic phase was dried over anhydrous sodium sulfate, concentrated and purified by flash silica gel column (methanol:dichloromethane: 0-5%) to obtain *tert*-butyl (*R*)-3,4-dichloro-1-((*S*)-2,4-dimethylpiperazin-1-yl)-12-oxo-6a,7,9,10-tetrahydro-12*H-*pyrazino[2,1-*c*]pyrido[3,4-*f*][1,4]oxazepine-8(6*H*)-carboxylate (500 mg) with ayield of 81% as a white solid. ES-API: [M+H]⁺=500.2.

Step 2: Under nitrogen protection, a mixed solution of *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (100 mg, 0.20 mmol), (2-amino-6-fluorophenyl)boronic acid (93 mg, 0.60 mmol ), tetrakis(triphenylphosphine)palladium (46 mg, 0.04 mmol) and potassium carbonate (83 mg, 0.60 mmol) in 1,4-dioxane (2 mL) and water (0.4 mL) was reacted at 100°C for 1 hour under microwave irradiation. The reaction solution was dissolved in ethyl acetate (5 mL) and washed with saturated brine (5 mL) and water (5 mL), and the organic phase was dried over anhydrous sodium sulfate, concentrated and purified by flash silica gel column (ethyl acetate:petroleum ether: 0-90%) to obtain *tert*-butyl (6a*R*)-3-(2-amino-6-fluorophenyl)-4-chloro-1-((*S*)-2,4-dimethylpiperazin-1-yl)-12-oxo-6a,7,9,10-tetrahydro-12*H*-pyrazino [2,1-*c*]pyrido[3,4*f*][1,4]oxazepine-8(6*H*-carboxylate(100 mg) with a yield of 87% as a white solid. ES-API: [M+H]⁺=575.2.

Step 3: Under an ice bath, a solution of *tert*-butyl (6a*R*)-3-(2-amino-6-fluorophenyl)-4-chloro-1-((*S*)-2,4-dimethylpiperazin-1-yl)-12-oxo-6a,7,9,10-tetrahydro-12*H*-pyrazino[2,1-*c*]pyrido [3,4-*f*][1,4]oxazepine-8(6*H*)-carboxylate (100 mg, 0.17 mmol) in dichloromethane (2 mL) was added with trifluoroacetic acid (1 mL), and the reaction solution was stirred at room temperature for 1 hour. The mixture was concentrated under reduced pressure to obtain a crude product of (6a*R*)-3-(2-amino-6-fluorophenyl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (82 mg). ES-API: [M+H]⁺=475.2.

Step 4: The above crude product of (6a*R*)-3-(2-amino-6-fluorophenyl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (82 mg) was dissolved in dichloromethane (2 mL), and *N*,*N*-diisopropylethylamine(111 mg, 0.86 mmol) was added thereto. The reaction solution was cooled to 0°C, and acryloyl chloride (16 mg, 0.17 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-3-(2-amino-6-fluorophenyl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (Z503, 32 mg, yield: 35%). ES-API: [M+H]⁺ = 529.2. ¹H NMR (500 MHz, DMSO-*d*₆) δ 7.14 - 7.03 (m, 1H), 6.78 (dd, *J* = 16.5, 10.5 Hz, 1H), 6.54 (dd, *J* = 15.5, 8.0 Hz, 1H), 6.42 - 6.32 (m, 1H), 6.23 - 6.11 (m, 1H), 5.82 - 5.69 (m, 1H), 5.28 (s, 1H), 5.01 (s, 1H), 4.48 - 4.24 (m, 2H), 4.23 - 3.75 (m, 6H), 3.70 - 3.42 (m, 3H), 3.26 - 3.16 (m, 1H), 2.68 - 2.51 (m, 2H), 2.14 (s, 3H), 2.10 - 1.93 (m, 2H), 1.26 (dd, *J* = 16.0, 6.5 Hz, 3H).

### Embodiment 92 Synthesis of (6aR)-8-acryloyl-4-chloro-1-(2,6-dimethyl-2,7-diazaspiro[3.5]nonan-7-yl)-3-(2-fluorophenyl)-6,6a,7,8,9,10-hexahydro-12H-pyrazino[2,1-c]pyrido[3,4-f][1,4]oxazepin-12-one (Z508MD)

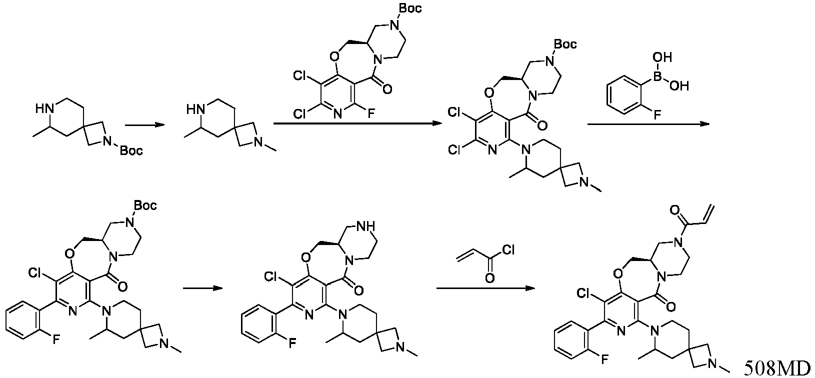

Step 1: A tetrahydrofuran solution of lithium aluminum hydride (1 M, 1 mL) was added to a solution of *tert*-butyl 6-methyl-2,7-diazaspiro[3.5]nonane-2-carboxylate (100 mg, 0.42 mmol) in tetrahydrofuran (1 mL) under an ice-bath and stirred for 2 hours at room temperature. After the reaction was completed, the reaction solution was quenched with water (1 mL), 15% sodium hydroxide solution (1 mL) and water (3 mL) in turn, dried by adding anhydrous sodium sulfate, filtered and concentrated to obtain a crude product of 2,6-dimethyl-2,7-diazaspiro[3.5]nonane (70 mg). ES-API: [M+H]⁺=154.2.

Step 2: 2,6-Dimethyl-2,7-diazaspiro[3.5]nonane (70 mg, 0.45 mmol) and potassium carbonate (182 mg, 1.32 mmol) were added to a solution of *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (180 mg, 0.44 mmol) in acetonitrile (2 mL), reacted at 100°C for 2 hours. The reaction solution was filtered, concentrated, then dissolved in ethyl acetate (30 mL), washed with water (15 mL ^{∗} 3), and the organic phase was dried over anhydrous sodium sulfate, concentrated and purified by flash silica gel column (methanol (1% ammonia water): dichloromethane: 0-50%) to obtain *tert*-butyl (6a*R*)-3,4-dichloro-1-(2,6-dimethyl-2,7-diazaspiro[3.5]nonan-7-yl)-12-oxo-6a,7,9,10-tetrahydro-12*H*-pyrazino[2,1-*c*]pyrido[3,4-*f*][1,4]oxazepine-8(6*H*)-carboxylate (100 mg) with a yield of 42% as a white solid. ES-API: [M+H]⁺=540.3.

Step 3: Under nitrogen protection, a mixed solution of *tert*-butyl (6a*R*)-3,4-dichloro-1-(2,6-dimethyl-2,7-diazaspiro[3.5]nonan-7-yl)-12-oxo-6a,7,9,10-tetrahydro-12*H*-pyrazino[2,1-*c*]pyrido[3,4-*f*][1,4]oxazepine-8(6*H*)-carboxylate (100 mg, 0.18 mmol), (2-fluorophenyl)boronic acid (78 mg, 0.55 mmol), tetrakis(triphenylphosphine)palladium (46 mg, 0.04 mmol) and potassium carbonate (76 mg, 0.55 mmol) in 1,4-dioxane (2 mL) and water (0.4 mL) was reacted at 100°C for 1 hour under microwave irradiation. The reaction solution was dissolved in ethyl acetate (5 mL) and washed with saturated brine (5 mL) and water (5 mL). The organic phase was dried over anhydrous sodium sulfate and concentrated to obtain a crude product of *tert*-butyl (6a*R*)-4-chloro-1-(2,6-dimethyl-2,7-diazaspiro[3.5]nonan-7-yl)-3-(2-fhiorophenyl)-12-oxo-6a,7,9,10-tetrahydro-12*H*-pyrazino[2,1-*c*]pyrido[3,4-*f*][1,4]oxazepine-8(6*H*)-carboxylate (100 mg). ES-API: [M+H]⁺=600.3.

Step 4: Under an ice-bath, a solution of the above crude product of of *tert*-butyl (6a*R*)-4-chloro-1-(2,6-dimethyl-2,7-diazaspiro[3.5]nonan-7-yl)-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (100 mg, 0.17 mmol) in dichloromethane (2 mL) was added with trifluoroacetic acid (1 mL), and the reaction solution was stirred at room temperature for 1 hour. The reaction solution was concentrated under reduced pressure to obtain a crude product of (6a*R*)-4-chloro-1-(2,6-dimethyl-2,7-diazaspiro[3.5]nonan-7-yl)-3-(2-fluorophenyl)-6,6a,7,8,9,10-hexahydro-12*H*-pyrazino[2,1-*c*]pyrido[3,4-*f*][1,4]oxazepin-12-one (82 mg). ES-API: [M+H]⁺=500.2.

Step 5: The above crude procuct of (6a*R*)-4-chloro-1-(2,6-dimethyl-2,7-diazaspiro[3.5]nonan-7-yl)-3-(2-fluorophenyl)-6,6a,7,8,9,10-hexahydro-12*H*-pyrazino[2,1-*c*]pyrido[3,4-*f*][1,4]oxazepin-12-one (82 mg, 0.17 mmol) was dissolved in dichloromethane (2 mL), and *N*,*N*-diisopropylethylamine(111 mg, 0.86 mmol) was added thereto. The reaction solution was cooled to 0°C, and acryloyl chloride (16 mg, 0.17 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-1-(2,6-dimethyl-2,7-diazaspiro[3.5]nonan-7-yl)-3-(2-fluorophenyl)-6,6a,7,8,9,10-hexahydro-12*H*-pyrazino[2,1-*c*]pyrido[3,4-*f*][1,4]oxazepin-12-one (Z508MD, 23 mg, yield: 25%). ES-API: [M+H]⁺=554.06.

### Embodiment 93 Synthesis of (6aR)-8-acryloyl-1-(2,2-dimethyl-4-(4-methylpiperazin-1-yl)pyrrolidin-1-yl)-4-fluoro-3-(2-chloro-6-fluoro-phenyl)-6,6a,7,8,9,10-hexahydro-12H-pyrazino [2,1-c]pyrido[3,4-f][1,4]oxazepin-12-one (Z635) and isomers thereof (Z635-1 and Z635-2)

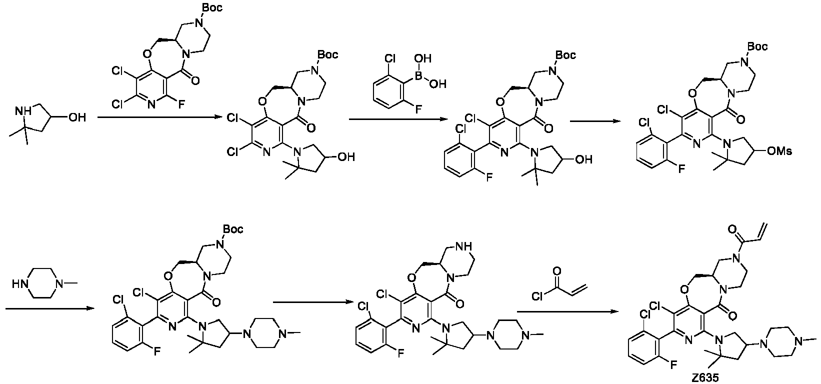

Step 1: Compound *tert*-butyl (6a*R*)-3,4-dichloro-1-fluoro-12-oxo-6a,7,9,10-tetrahydro-6*H-*pyrazino[2,1-*c*]pyrido[3,4-*f*][1,4]oxazepine-8(12*H*)-carboxylate (400 mg , 0.988 mmol), 5,5-dimethylpyrrolidin-3-ol (136 mg, 1.185 mmol) and potassium carbonate (409 mg, 2.964 mmol) were dissolved in acetonitrile (4 mL) and reacted at 80°C for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation and purified by flash chromatography (ethyl acetate:petroleum ether: 0-80%) to obtain compound *tert*-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (410 mg, yield: 83%). ES-API: [M+H]⁺= 501.3

Step 2: Compound *tert*-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (410 mg , 0.818 mmol), (2-chloro-6-fluoro-phenyl)boronic acid (170 mg, 0.982 mmol), tetrakis(triphenylphosphine)palladium (95 mg, 0.082 mmol), potassium carbonate (226 mg, 1.136 mmol) and potassium hydrogen fluoride (128 mg, 1.636 mmol) were dissolved in a mixed solution of tert-butanol and water (5 mL:1mL). The reaction solution was reacted at 120°C for 1 hour under microwave irradiation, and the reaction was monitored to be complete. The mixture was extracted with ethyl acetate, washed once with brine, dried over anhydrous sodium sulfate, and the filtrate was evaporated to dryness by rotary evaporation and purified by flash chromatography (ethyl acetate:petroleum ether: 0-85%) to obtain compound *tert*-butyl (6a*R*)-4-chloro-3-(2-chloro-6-fluoro-phenyl)-1-(4-hydroxy-2,2-dimethylpyrrolidin-1-y1)-12-oxo-6a,7,9,10-tetrahydro-6*H*-pyrazino[2,1-*c*]pyrido[3,4-*f*][1,4]oxazepine-8(12*H*)-carboxylate (270 mg, crude product, yield: 95%). ES-API: [M+H]⁺= 595.3

Step 3: Compound *tert*-butyl (6a*R*)-4-chloro-3-(2-chloro-6-fluoro-phenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (270 mg, 0.454 mmol) was dissolved in 3 mL of dichloromethane, then methanesulfonyl chloride (103 mg, 0.908 mmol) and triethylamine (138 mg, 1.362 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation and purified by thin-layer chromatography plate to obtain compound *tert*-butyl (*R*)-4-chloro-1-((*S*)-2,2-dimethyl-4-((methylsulfonyl)oxy)pyrrolidin-1-yl)-3-(2-chloro-6-fluoro-phenyl)-12-oxo-6a,7,9,10-tetrahydro-6*H-*pyrazino[2,1-*c*]pyrido[3,4-*f*][1,4]oxazepine-8(12*H*)-carboxylate (300 mg, yield: 95%). ES-API: [M+H]⁺= 673.2

Step 4: Compound *tert*-butyl (*R*)-4-chloro-1-((*S*)-2,2-dimethyl-4-((methylsulfonyl)oxy)pyrrolidin-1-yl)-3-(2-chloro-6-fluoro-phenyl)-12-oxo-6a,7,9,10-tetrahydro-6*H-*pyrazino[2,1-*c*]pyrido[3,4-*f*][1,4]oxazepine-8(12*H*)-carboxylate (250 mg, 0.37 mmol) was dissolved in 1.5 mL of 1-methylpiperazine. The reaction solution was heated to 100°C, then stirred for 16 hours. The reaction was monitored to be complete, then the reaction solution was cooled to room temperature, extracted by adding ethyl acetate, washed once with brine, dried over anhydrous sodium sulfate, and the filtrate was evaporated to dryness by rotary evaporation and purified (dichloromethane/methanol=20/1) to obtain compound *tert*-butyl (*R*)-4-chloro-((4-methylpiperazin-1-yl))-2,2-dimethylpyrrolidin-1-yl)-3-(2-chloro-6-fluoro-phenyl)-12-oxo-6a,7,9,10-tetrahydro-6*H*-pyrazino[2,1-*c*]pyrido[3,4-*f*][1,4]oxazepine-8(12*H*)-carboxylate (54 mg, yield: 22%). ES-API: [M+H]⁺= 677.4

Step 5: *tert*-Butyl (*R*)-4-chloro-((4-methylpiperazin-1-yl))-2,2-dimethylpyrrolidin-1-yl)-3-(2-chloro-6-fluoro-phenyl)-12-oxo-6a,7,9,10-tetrahydro-6*H*-pyrazino[2,1-*c*]pyrido[3,4-*f*][1,4]oxazepine-8(12*H*)-carboxylate (65 mg, 0.096 mmol) was dissolved in dichloromethane (1 mL), then cooled to 0°C in an ice-water bath, and then trifluoroacetic acid (0.5 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (*R*)-4-chloro-4-((4-methylpiperazin-1-yl)-2,2-dimethylpyrrolidin-1-yl)-3-(2-chloro-6-fluoro-phenyl)-7,8,9,10-tetrahydro-6*H*-pyrazino[2,1-*c*]pyrido[3,4-*f*][1,4]oxazepin-12-one (60 mg, yield: 95%). ES-API: [M+H]⁺= 577.2

Step 6: (*R*)-4-Chloro-1-4-((4-methylpiperazin-1-yl)-2,2-dimethylpyrrolidin-1-yl)-3-(2-chloro-6-fluoro-pheny1)-7,8,9,10-tetrahydro-6*H*-pyrazino[2,1-*c*]pyrido[3,4-*f*][1,4]oxazepin-12-one (60 mg, 0.104 mmol) was dissolved in dichloromethane (2 mL), then cooled to 0°C in an ice-water bath, and triethylamine (32 mg, 0.312 mmol) and acryloyl chloride (14 mg, 0.156 mmol) were added dropwise thereto, stirred for 30 minutes. After the reaction was completed, the mixture was added with sodium bicarbonate solution to adjust to alkaline, extracted with dichloromethane, dried over sodium sulfate, and evaporated to dryness by rotary evaporation and purified (dichloromethane/methanol =10/1) to obtain (6a*R*)-8-acryloyl-1-(2,2-dimethyl-4-(4-methylpiperazin-1-yl)pyrrolidin-1-yl)-4-fluoro-3-(2-chloro-6-fluoro-phenyl)-6,6a,7, 8,9,10-hexahydro-12*H*-pyrazino[2,1-*c*]pyrido[3,4-*f*][1,4]oxazepin-12-one (Z635, 35 mg, yield: 53%). ES-API: [M+H]⁺= 631.3.

The Z635 obtained in the above steps was subjected to chiral resolution under the following conditions to obtain two isomers:

| No. and structure | Name | Starting material, preparation condition, retention time, purity |
|---|---|---|
| 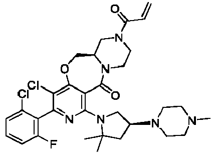 | (6a*R*)-8-Acryloyl-4-chloro-3-(2-chloro-6-fluorophenyl)-1 -((S)-2,2-dimethyl-4-(4-methylpiperazin-1-yl)pyrrolidon-1-yl)-6,6a,7,8,9,10-hexahydro-12*H*-pyrazino [2,1-*c*]pyrido[3,4-*f*][1,4]oxazepin-12-one | Chiral separation via Z635. |
| | | Separation column: IE 250 mm^{∗}4.6 mm^{∗}5 µm; mobile phase: *n-*hexane:ethanol:diethanolamine=70:30:0.2; |
| | | flow rate: 1 mL/min; |
| Peak 1, | | column temperature: 30°C. |
| Z635-1 | | Peak 1, retention time: 19.150 min, purity: 99%, de value: 100%. |
| The structure was arbitrarily designated | | |
| | | ES-API: [M+H]⁺=631.2. |
| 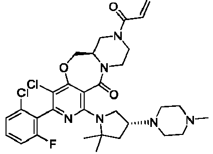 | (6a*R*)-8-Acryloyl-4-chloro-3-(2-chloro-6-fluorophenyl)- 1 -((R)-2,2-dimethyl-4-(4-methylpiperazin-1 - yl)pyrrolidon-1-yl)-6,6a,7,8,9,10-hexahydro-12*H*-pyrazino [2,1-*c*]pyrido[3,4-*f*][1,4]oxazepin-12-one | Chiral separation via Z635. |
| | | Separation column: IE 250 mm^{∗}4.6 mm^{∗}5 µm; mobile phase: *n-*hexane:ethanol:diethanolamine=70:30:0.2; |
| | | flow rate: 1 mL/min; |
| Peak 2, | | column temperature: 30°C. |
| Z635-2 | | Peak 2, retention time: 22.215 min, purity: 99%, de value: 98.54%. |
| The structure was arbitrarily designated | | |
| | | ES-API: [M+H]⁺=631.2. |

### Embodiment 94 Synthesis of (6aR)-8-acryloyl-4-chloro-3-(2-chloro-6-fluorophenyl)-1-((S)-2,4-dimethylpiperazin-1-yl)-6,6a,7,8,9,10-hexahydro-12H-pyrazino[2,1-c]pyrido[3,4-f][1,4]oxazepin-12-one (Z547)

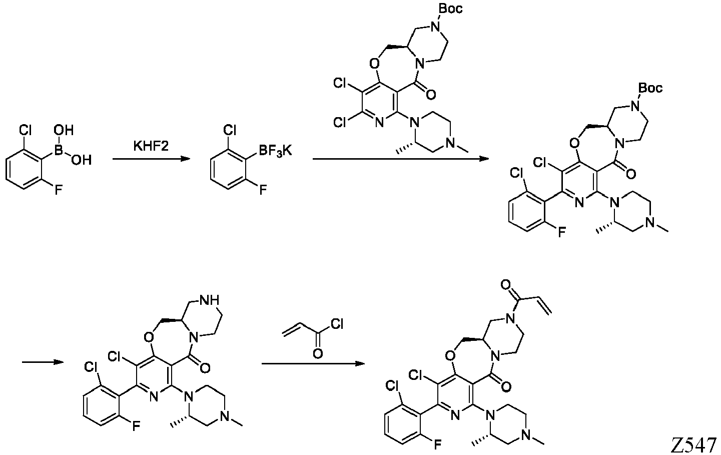

Step 1: A solution of compound (2-chloro-6-fluorophenyl)boronic acid (1.50 g, 8.60 mmol) in methanol (15 mL) was added to a tetrafluoride tube, and a solution of potassium hydrogen fluoride (2.6 g, 33 mmol) in water (10 mL) was added thereto. The reaction solution was stirred at room temperature overnight and concentrated to obtain a crude product of potassium (2-chloro-6-fluorophenyl)trifluoroborate (2 g).

Step 2: Under nitrogen protection, a mixed solution of *tert*-butyl (*R*)-3,4-dichloro-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (100 mg, 0.20 mmol), potassium (2-chloro-6-fluorophenyl)trifluoroborate (140 mg, 0.60 mmol ), tetrakis(triphenylphosphine)palladium (23 mg, 0.02 mmol) and potassium carbonate (83 mg, 0.60 mmol) in 1,4-dioxane (2 mL) and water (0.4 mL) was reacted at 100°C for 1 hour under microwave irradiation. The reaction solution was dissolved in ethyl acetate (5 mL) and washed with saturated brine (5 mL) and water (5 mL), and the organic phase was dried over anhydrous sodium sulfate, concentrated and purified by flash silica gel column (ethyl acetate:petroleum ether: 0-90%) to obtain *tert*-butyl (6a*R*)-4-chloro-3-(2-chloro-6-fhiorophenyl)-1-((*S*)-2,4-dimethylpiperazin-1-yl)-12-oxo-6a,7,9,10-tetrahydro-12*H-*pyrazino[2,1-*c*]pyrido[3,4-*f*][1,4]oxazepine-8(6*H*)-carboxylate (80 mg) with a yield of 56% as a white solid. ES-API: [M+H]⁺=594.3.

Step 3: Under an ice bath, a solution of *tert*-butyl (6a*R*)-4-chloro-3-(2-chloro-6-fluorophenyl)-1-((*S*)-2,4-dimethylpiperazin-1-yl)-12-oxo-6a,7,9,10-tetrahydro-12*H*-pyrazino[2,1-*c*]pyrido[3,4-*f*][1,4]oxazepine-8(6*H*)-carboxylate (80 mg, 0.13 mmol) in dichloromethane (2 mL) was added with trifluoroacetic acid (1 mL), and the reaction solution was stirred at room temperature for 1 hour. The mixture was concentrated under reduced pressure to obtain a crude product of (6a*R*)-4-chloro-3-(2-chloro-6-fluorophenyl)-1-((*S*)-2,4-dimethylpiperazin-1-yl)-6,6a,7,8,9,10-hexahydro-12*H*-pyrazino [2,1-*c*]pyrido[3,4-*f*][1,4]oxazepin-12-one (66 mg). ES-API: [M+H]⁺=494.2.

Step 4: The above crude product of (6a*R*)-4-chloro-3-(2-chloro-6-fluorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one (66 mg) was dissolved in dichloromethane (2 mL), and *N*,*N*-diisopropylethylamine (85 mg, 0.65 mmol) was added thereto. The reaction solution was cooled to 0°C, and acryloyl chloride (12 mg, 0.13 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-4-chloro-3-(2-chloro-6-fluorophenyl)-1-((*S*-2,4-dimethylpiperazin-1-yl)-6,6a,7,8,9,10-hexahydro-12*H*-pyrazino[2,1-*c*]pyrido [3,4-*f*][1,4]oxazepin-12-one (Z547, 30 mg, yield: 41%). ES-API: [M+H]⁺ = 548.2. ¹H NMR (500 MHz, DMSO-*d*₆) δ 7.59 - 7.52 (m, 1H), 7.47 (dd, *J* = 21.0, 8.0 Hz, 1H), 7.42 - 7.32 (m, 1H), 6.83 - 6.69 (m, 1H), 6.17 (d, *J* = 16.6 Hz, 1H), 5.79 - 5.70 (m, 1H), 4.52 - 4.29 (m, 2H), 4.28 - 4.15 (m, 1H), 4.15 - 3.94 (m, 3H), 3.94 - 3.71 (m, 1H), 3.69 - 3.41 (m, 3H), 3.32 - 3.29 (m, 1H), 3.27 - 3.15 (m, 1H), 2.63 (d, *J* = 9.5 Hz, 1H), 2.57 - 2.51 (m, 1H), 2.13 (s, 3H), 2.06 - 1.91 (m, 2H), 1.25 (dd, *J* = 12.5, 6.5 Hz, 3H).

### Embodiment 95 Synthesis of two isomers of (6aR)-8-acryloyl-4-chloro-1-(2,2-dimethyl-4-(3-(trifluoromethyl)azetidin-1-yl)pyrrolidin-1-yl)-3-(2-fluorophenyl)-6,6a,7,8,9,10-hexahydro-12H-pyrazino[2,1-c]pyrido[3,4-f][1,4]oxazepin-12-one (Z608-1 and Z608-2)

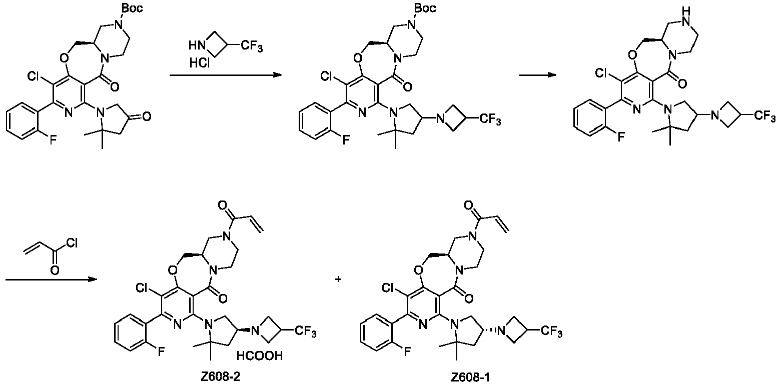

Step 1: Under an ice-bath, a solution of *tert*-butyl (*R*)-4-chloro-1-(2,2-dimethyl-4-oxopyrrolidin-1-yl)-3-(2-fluorophenyl)-12-oxo-6a,7,9,10-tetrahydro-12*H*-pyrazino[2,1-*c*]pyrido[3.4-*f*][1,4]oxazepine-8(6*H*)-carboxylate (120 mg, 0.21 mmol) in acetonitrile (2 mL) was added with 3-(trifluoromethyl)azetidine hydrochloride (70 mg, 0.43 mmol), stirred at room temperature for 1 hour. Then sodium cyanoborohydride (27 mg, 0.43 mmol) was added thereto, and the mixture was continued to be stirred for 2 hours. After the reaction was completed, the mixture was quenched with saturated ammonium chloride solution (20 mL), extracted with ethyl acetate (20 mL ^{∗} 3), and the organic phases were combined, dried and concentrated to obtain a crude product of *tert*-butyl (6a*R*)-4-chloro-1-(2,2-dimethyl-4-(3-(trifluoromethyl)azetidin-1-yl)pyrrolidin-1-yl)-3 -(2-fluorophenyl)-12-oxo-6a,7,9,10-tetrahydro-12*H*-pyrazino[2,1-*c*]pyrido[3,4-*f*][1,4]oxazepine-8(6/*H*)-carboxylate (120 mg). ES-API: [M+H]⁺=668.3.

Step 2: Under an ice-bath, a solution of *tert*-butyl (6a*R*)-4-chloro-1-(2,2-dimethyl-4-(3-(trifhioromethyl)azetidin-1-yl)pyrrolidin-1-yl)-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate (120 mg, 0.18 mmol) in dichloromethane (2 mL) was added with trifluoroacetic acid (1 mL), and the reaction solution was stirred at room temperature for 1 hour. The reaction solution was concentrated under reduced pressure to obtain a crude product of (6a*R*)-4-chloro-1-(2,2-dimethyl-4-(3-(trifluoromethyl)azetidin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (102 mg). ES-API: [M+H]⁺=568.2.

Step 3: The above crude procuct of (6a*R*)-4-chloro-1-(2,2-dimethyl-4-(3-(trifluoromethyl)azetidin-1-yl)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one (102 mg, 0.18 mmol) was dissolved in dichloromethane (2 mL), and *N*,*N*-diisopropylethylamine (116 mg, 0.90 mmol) was added thereto. The reaction solution was cooled to 0°C, and acryloyl chloride (16 mg, 0.18 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%= 20%-100%; column temperature: room temperature) to obtain two isomer compounds:

One isomer with a structure arbitrarily designated as (*R*)-8-acryloyl-4-chloro-1-((*R*)-2,2-dimethyl-4-(3-(trifluoromethyl)azetidin-1-yl)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one (Z608-1, retention time: 8.64 min; 15 mg, yield: 14%). ES-API: [M+H]⁺=622.2. ¹H NMR (500 MHz, DMSO-*d*₆) δ 7.55 - 7.48 (m, 1H), 7.45 (t, *J* = 7.0 Hz, 1H), 7.35 - 7.28 (m, 2H), 6.81 - 6.71 (m, 1H), 6.21 - 6.06 (m, 1H), 5.78 - 5.64 (m, 1H), 4.61 - 4.25 (m, 3H), 4.25-3.71 (m, 6H), 3.62 - 3.39 (m, 4H), 3.28 - 3.18 (m, 1H), 3.15 - 3.01 (m, 2H), 2.91 (s, 1H), 1.89 - 1.80 (m, 1H), 1.79 - 1.72 (m, 1H), 1.72 - 1.66 (m, 3H), 1.46 - 1.35 (m, 3H).

Another isomer (retention time: 6.89 min) was obtained by further preparative HPLC (column: Waters XBridge C18, 190^{∗}250 mm, 5 µm; mobile phase A: 0.1% aqueous formic acid solution; mobile phase B: acetonitrile; flow rate: 15 mL/min; B%=20%-100%; column temperature: room temperature) purification and the structure was arbitrarily designated as (*R*)-8-acryloyl-4-chloro-1-((*S*-2,2-dimethyl-4-(3-(trifluoromethyl)azetidin-1-yl)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one formate (Z608-2, 1.2 mg, yield: 1%). ES-API: [M+H]⁺=622.2.

### Embodiment 96 Synthesis of two isomers of (6aR)-8-acryloyl-4-chloro-1-(4-(3,3-difluoroazetidin-1-yl)-2,2-dimethylpyrrolidin-1-yl)-3-(2-fluorophenyl)-6,6a,7, 8,9,10-hexahydro-12H-pyrazino [2,1-c]pyrido[3,4-f][1,4]oxazepin-12-one (Z525-1 and Z525-2)

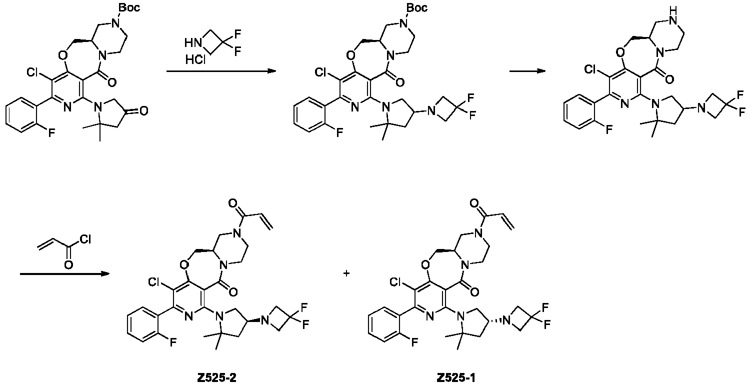

Step 1: A solution of tert-butyl (*R*)-4-chloro-1-(2,2-dimethyl-4-oxopyrrolidin-1-yl)-3-(2-fluoropheny1)-12-oxo-6a,7,9,10-tetrahydro-12*H*-pyrazino[2,1-*c*]pyrido[3,4-*f*][1,4-*f*]oxazepine-8(6*H*)-carboxylate (120 mg, 0.21 mmol) in acetonitrile (2 mL) was added with 3,3-difluoroazetidine hydrochloride (56 mg, 0.43 mmol), stirred at room temperature for 1 hour. Then sodium cyanoborohydride (27 mg, 0.43 mmol) was added thereto, and the mixture was continued to be stirred for 2 hours. After the reaction was completed, the mixture was quenched with saturated ammonium chloride solution (20 mL), extracted with ethyl acetate (20 mL ^{∗} 3), and the organic phases were combined, dried and concentrated to obtain a crude product of *tert*-butyl (6a*R*)-4-chloro-1-(4-(3,3-difluoroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (120 mg). ES-API: [M+H]⁺=636.3.

Step 2: Under an ice-bath, a solution of *tert*-butyl (6a*R*)-4-chloro-1-(4-(3,3-difluoroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (120 mg, 0.19 mmol) in dichloromethane (2 mL) was added with trifluoroacetic acid (1 mL), and the reaction solution was stirred at room temperature for 1 hour. The reaction solution was concentrated under reduced pressure to obtain a crude product of (6a*R*)-4-chloro-1-(4-(3,3-difluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (101 mg). ES-API: [M+H]⁺=536.2.

Step 3: The above crude procuct of (6a*R*)-4-chloro-1-(4-(3,3-difluoroazetidin-1-yl)-2,2-dimethylpyrrolidin-1-yl)-3-(2-fluorophenyl)-6,6a,7, 8,9,10-hexahydro-12*H*-pyrazino [2,1-*c*]pyrido [3,4-*f*][1,4]oxazepin-12-one (101 mg, 0.19 mmol) was dissolved in dichloromethane (2 mL), and *N,N-*diisopropylethylamine (120 mg, 0.96 mmol) was added thereto. The reaction solution was cooled to 0°C, and acryloyl chloride (17 mg, 0.19 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%= 20%-100%; column temperature: room temperature) to obtain two isomer compounds:

One isomer with a structure arbitrarily designated as (*R*)-8-acryloyl-4-chloro-1-((R)-4-(3,3-difluoroazetidin-1-yl)-2,2-dimethylpyrrolidin-1-yl)-3-(2-fluorophenyl)-6,6a,7,8,9,10-hexahydro-12H-pyrazino[2,1-*c*]pyrido[3,4-*f*][1,4]oxazepin-12-one (Z525-1, retention time: 9.00 min; 8 mg, yield: 14%). ES-API: [M+H]⁺=590.2. ¹H NMR (500 MHz, DMSO-*d*₆) δ 7.55 - 7.48 (m, 1H), 7.45 (t, *J* = 7.0 Hz, 1H), 7.35 - 7.28 (m, 2H), 6.81 - 6.71 (m, 1H), 6.21 - 6.06 (m, 1H), 5.78 - 5.64 (m, 1H), 4.61 - 4.25 (m, 3H), 4.25-3.71 (m, 6H), 3.62 - 3.39 (m, 4H), 3.28 - 3.18 (m, 1H), 3.15 - 3.01 (m, 2H), 2.91 (s, 1H), 1.89 - 1.80 (m, 1H), 1.79 - 1.72 (m, 1H), 1.72 - 1.66 (m, 3H), 1.46 - 1.35 (m, 3H).

Another isomer with a structure arbitrarily designated as (*R*)-8-acryloyl-4-chloro-1-((*S*)-4-(3,3-difluoroazetidin-1-yl)-2,2-dimethylpyrrolidin-1-yl)-3-(2-fluorophenyl)-6,6a,7,8,9,10-hexahydro-12H-pyrazino[2,1-*c*]pyrido[3,4-*f*][1,4]oxazepin-12-one (Z525-2, retention time: 6.30 min; 5 mg, yield: 1%). ES-API: [M+H]⁺=590.2. ¹H NMR (500 MHz, DMSO-*d*₆) δ 7.56 - 7.48 (m, 1H), 7.45 (t, *J* = 7.0 Hz, 1H), 7.36 - 7.29 (m, 2H), 6.80 - 6.70 (m, 1H), 6.23 - 6.10 (m, 1H), 5.85 - 5.62 (m, 1H), 4.58 - 3.75 (m, 8H), 3.74 - 3.48 (m, 6H), 3.28 - 3.10 (m, 2H), 3.06 - 2.89 (m, 1H), 1.98 - 1.87 (m, 1H), 1.63 - 1.55 (m, 1H), 1.51 (s, 3H), 1.45 (s, 3H).

### Embodiment 97 Synthesis of two isomers of (6aR)-8-acryloyl-4-chloro-1-(3-(dimethylamino)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12(6aH)-one (Z502-1 and Z502-2)

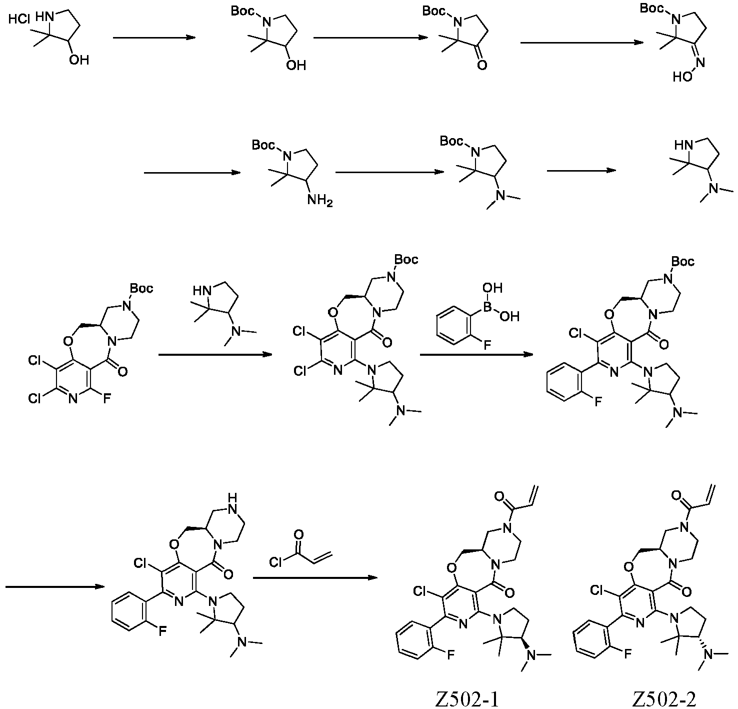

Step 1: 2,2-Dimethylpyrrolidin-3-ol hydrochloride (2 g, 13.2 mmol) was dissolved in dichloromethane (40 mL), and triethylamine (4 g, 39.6 mmol) and di-*tert*-butyl dicarbonate (5.7 g, 26.4 mmol) were added thereto, reacted at room temperature for 2 hours. The reaction solution was extracted with dichloromethane and water, and the organic phase was dried, evaporated to dryness, and purified by column chromatography (petroleum ether/ethyl acetate=5/1) to obtain a product (2.7 g, yield: 96%). (R_{f}=0.6, petroleum ether/ethyl acetate=3/1). MS-API: [M+1-56]⁺=160.2.

Step 2: *tert*-Butyl 3-hydroxy-2,2-dimethylpyrrolidine-1-carboxylate (1 g, 4.65 mmol) was dissolved in dichloromethane (20 mL), and Dess-Martin reagent (5.9 g, 13.9 mmol) was added thereto, reacted at room temperature overnight. The reaction solution was quenched with aqueous sodium thiosulfate solution, extracted with dichloromethane, and the organic phase was dried, evaporated to dryness, and purified by column chromatography (petroleum ether/ethyl acetate=10/1) to obtain a product (950 mg, yield: 96%). (R_{f}=0.5, petroleum ether/ethyl acetate=5/1). MS-API: [M+1-56]⁺=158.1.

Step 3: *tert*-Butyl 2,2-dimethyl-3-oxopyrrolidine-1-carboxylate (850 mg, 4.0 mmol) was dissolved in ethanol/water (8 mL/1.6 mL), and then hydroxylamine hydrochloride (413 mg, 6.0 mmol) and sodium acetate (524 mg, 6.4 mmol) were added thereto, and heated to 80°C and reacted overnight. After the reaction was completed, the mixture was extracted with ethyl acetate and water, dried and evaporated to dryness by rotary evaporation to obtain a product (crude product), which was directly used in the next reaction step. MS-API: [M+H]⁺=229.1.

Step 4: 3-(Hydroxyimino)-2,2-dimethylpyrrolidine-1-carboxylate (0.4 mmol) was dissolved in *n-*propanol (5 mL), heated to 80°C. Sodium (322 mg, 14.0 mmol) was slowly added thereto and then reacted at 80°C for 3 hours. The reaction was quenched with water, extracted with ethyl acetate, dried and evaporated to dryness by rotary evaporation to obtain a product (crude product), which was directly used in the next reaction step. MS-API: [M+1-56]⁺=159.1.

Step 5: *tert*-Butyl 3-amino-2,2-dimethylpyrrolidine-1-carboxylate (4.0 mmol) was dissolved in methanol (27 mL), and then 37% aqueous formaldehyde solution (9 mL) and sodium cyanoborohydride (1.26 g, 20.0 mmol) were added thereto and reacted at room temperature overnight. The reaction solution was quenched with water, extracted with dichloromethane, and the organic phase was dried, evaporated to dryness, and purified by column chromatography (ethyl acetate) to obtain a product (520 mg, yield of 54%). (R_{f}=0.3, ethyl acetate). MS-API: [M+H]⁺=243.2.

Step 6: *tert*-Butyl 3-(dimethylamino)-2,2-dimethylpyrrolidine-1-carboxylate (520 mg, 2.15 mmol) was dissolved in dichloromethane (5 mL), and then trifluoroacetic acid (2 mL) was added thereto and reacted at room temperature for 1 hour. The reaction solution was directly evaporated to dryness to obtain a crude product, which was directly used in the next reaction step. MS-API: [M+H]⁺=143.1.

Step 7: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (698 mg, 1.72 mmol) was dissolved in acetonitrile (10 mL), then *N*,*N*,2,2-tetramethylpyrrolidin-3-amine (crude product, 2.15 mmol) and potassium carbonate (1.5 g, 10.7 mmol) were added thereto, heated to 80°C and reacted for 2 hours. After the reaction was completed, the reaction mixture was quenched by adding water, extracted with dichloromethane, dried, evaporated to dryness by rotary evaporation, and purified by column chromatography (dichloromethane/methanol=10/1) to obtain a product (950 mg, yield: 100%). (R_{f}=0.3, dichloromethane/methanol=10/1). MS-API: [M+H]⁺=528.2.

Step 8: *tert*-Butyl 3,4-dichloro-1-(3-(dimethylamino)-2,2-dimethylpyrrolidin-1-yl)-12-oxo-6a,7,9,10-tetrahydro-6*H*-pyrazino(6aR)-[2,1-*c*]pyrido[3,4-*f*][1,4]oxazepine-8(12*H*-carboxylate (950 mg, 1.8 mmol) was dissolved in dioxane/water (10 mL/2 mL), then (2-fluorophenyl)boronic acid (378 mg, 2.7 mmol), potassium carbonate (745 mg, 5.4 mmol) and 1,1'-bis(diphenylphosphino)ferrocene]palladium dichloride (132 mg, 0.18 mmol) were added thereto, heated to 100°C and reacted overnight. The reaction solution was evaporated to dryness by rotary evaporation and purified by column chromatography (ethyl acetate) to obtain a product (660 mg, crude product, containing raw materials). (R_{f}=0.2, dichloromethane/methanol=10/1). MS-API: [M+H]⁺=588.3

Step 9: *tert*-Butyl (6aR)-4-chloro-1-(3-(dimethylamino)-2,2-dimethylpyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (660 mg, 1.12 mmol) was dissolved in dichloromethane (3 mL), and trifluoroacetic acid (3 mL) was added thereto. The reaction solution was reacted at room temperature for 1 hour. After the reaction was completed, the reaction solution was evaporated to dryness, dissolved in dichloromethane and evaporated to dryness to obtain a crude product, which was directly used in the next reaction step. MS-API: [M+H]⁺=488.3.

Step 10: (6a*R*)-4-Chloro-1-(3-(dimethylamino)-2,2-dimethylpyrrolidin-1-yl)-3-(2-fluorophenyl)-7,8,9,10-tetrahydro-6*H*-pyrazino[2,1-*c*]pyrido[3,4-*f*][1,4]oxazepin-12(6a*H*)-one (crude product, 1.12 mmol) was dissolved in dichloromethane (3 mL), cooled to 0 to 5°C, and triethylamine (2 mL) and acryloyl chloride (101 mg, 1.12 mmol) were added thereto, stirred for 0.5 hours. After the reaction was completed, the mixture was extracted with dichloromethane and water, and the organic phase was dried, evaporated to dryness by rotary evaporation, and purified by preparative thin-layer chromatography column (dichloromethane/methanol=10/1) to obtain a small polar product (Z502-1, R_{f}=0.4, 33.4 mg, yield: 5%, white solid) and a large polar product (Z502-2, R_{f}=0.3, 26.2 mg, yield: 4%, white solid). MS-API: [M+H]⁺=542.2.

Z502-1: ¹H NMR (400 MHz, CDCl₃) δ 7.43-7.37 (m, 2H), 7.24 (t, *J* = 7.5 Hz, 1H), 7.14 (t, *J* = 9.1 Hz, 1H), 6.62-6.49 (m, 1H), 6.40 (d, *J* = 16.5 Hz, 1H), 5.81 (d, *J* = 10.3 Hz, 1H), 4.55-4.26 (m, 3H), 4.24-3.97 (m, 3H), 3.75-3.51 (m, 3H), 3.44-3.30 (m, 1H), 2.73 (t, *J* = 9.0 Hz, 1H), 2.57 (dd, *J* = 12.4, 5.5 Hz, 1H), 2.36 (s, 6H), 2.06-1.98 (m, 1H), 1.90-1.79 (m, 1H), 1.63 (s, 3H), 1.51 (s, 3H).

Z502-2: ¹HNMR (400 MHz, CDCl₃) δ 7.43-7.38 (m, 2H), 7.23-7.20 (m, 1H), 7.15 (t, *J* = 9.5 Hz, 1H), 6.63-6.51 (m, 1H), 6.40 (d, *J* = 16.5 Hz, 1H), 5.81 (d, *J* = 10.2 Hz, 1H), 4.49-4.27 (m, 3H), 4.21-4.13 (m, 2H), 4.07-3.95 (m, 1H), 3.87-3.78 (m, 1H), 3.76-3.39 (m, 2H), 2.88-2.81 (m, 2H), 2.36 (s, 7H), 2.24-2.14 (m, 1H), 2.08-1.98 (m, 1H), 1.69 (s, 3H), 1.49 (s, 3H).

### Embodiment 98 Synthesis of (6aR)-8-acryloyl-3-(2-amino-6-fluorophenyl)-4-chloro-1-(3-methoxy-2,2-dimethylpyrrolidin-1-yl)-6,6a,7,8,9,10-hexahydro-12H-pyrazino[2,1-c]pyrido[3,4-f][1,4]oxazepin-12-one (Z506)

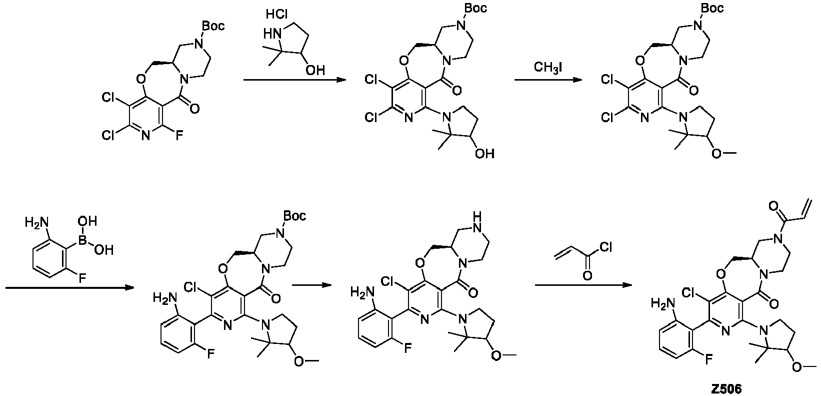

Step 1: 2,2-Dimethylpyrrolidin-3-ol hydrochloride (103 mg, 0.68 mmol) and potassium carbonate (255 mg, 1.85 mmol) were added to a solution of *tert*-butyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*-carboxylate (250 mg, 0.62 mmol) in acetonitrile (3 mL), reacted at 100°C for 2 hours. The reaction solution was filtered, concentrated, then dissolved in ethyl acetate (30 mL), washed with water (15 mL ^{∗} 3), and the organic phase was dried over anhydrous sodium sulfate, concentrated and purified by flash silica gel column (methanol:dichloromethane: 0-5%) to obtain *tert*-butyl (6a*R*)-3,4-dichloro-1-(3-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg) with a yield of 65% as a white solid. ES-API: [M+H]⁺=501.2.

Step 2: Iodomethane (566 mg, 3.99 mmol) and potassium *tert*-butoxide (134 mg, 1.20 mmol) were added to a solution of *tert*-butyl (6a*R*)-3,4-dichloro-1-(3-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.40 mmol) in tetrahydrofuran (10 mL) and stirred at room temperature for 3 hours. The reaction solution was filtered, concentrated, and purified by flash silica gel column (ethyl acetate:petroleum ether: 0-90%) to obtain *tert*-butyl (6a*R*)-3,4-dichloro-1-(3-methoxy-2,2-dimethylpyrrolidin-1-yl)-12-oxo-6a,7,9,10-tetrahydro-12*H*-pyrazino[2,1-*c*]pyrido[3,4-*f*][1,4oxazepine-8(6*H*)-carboxylate (150 mg). ES-API: [M+H]⁺=515.2.

Step 3: Under nitrogen protection, a mixed solution of tert-butyl (6aR)-3,4-dichloro-1-(3-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (150 mg, 0.29 mmol), (2-amino-6-fluorophenyl)boronic acid (135 mg, 0.87 mmol), tetrakis(triphenylphosphine)palladium (35 mg, 0.03 mmol) and potassium carbonate (120 mg, 0.87 mmol) in 1,4-dioxane (2 mL) and water (0.4 mL) was reacted at 100°C for 1 hour under microwave irradiation. The reaction solution was dissolved in ethyl acetate (5 mL) and washed with saturated brine (5 mL) and water (5 mL), and the organic phase was dried over anhydrous sodium sulfate, concentrated and purified by flash silica gel column (ethyl acetate:petroleum ether: 0-90%) to obtain *tert*-butyl (6a*R*)-3-(2-amino-6-fluorophenyl)-4-chloro-1-(3-methoxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (120 mg) as a white solid. ES-API: [M+H]⁺=590.3.

Step 3: Under an ice bath, a solution of *tert*-butyl (6a*R*)-3-(2-amino-6-fluorophenyl)-4-chloro-1-(3-methoxy-2,2-dimethylpyrrolidin-1-yl)-12-oxo-6a,7,9,10-tetrahydro-12*H*-pyrazino[2,1-*c*]pyrido [3,4-*f*][1,4]oxazepine-8(6*H*)-carboxylate (120 mg, 0.20 mmol) in dichloromethane (2 mL) was added with trifluoroacetic acid (1 mL), and the reaction solution was stirred at room temperature for 1 hour. The mixture was concentrated under reduced pressure to obtain a crude product of (6a*R*)-3-(2-amino-6-fluorophenyl)-4-chloro-1-(3-methoxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (100 mg). ES-API: [M+H]⁺=490.3.

Step 4: The above crude product of (6a*R*)-3-(2-amino-6-fluorophenyl)-4-chloro-1-(3-methoxy-2,2-dimethylpyrrolidin-1-yl)-6,6a,7,8,9,10-hexahydro-12*H*-pyrazino[2,1-*c*]pyrido[3,4-*c*][1,4]oxazepin-12-one (100 mg, 0.20 mmol) was dissolved in dichloromethane (2 mL), and *N*,*N* diisopropylethylamine(78 mg, 0.60 mmol) was added thereto. The reaction solution was cooled to 0°C, and acryloyl chloride (18 mg, 0.20 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (6a*R*)-8-acryloyl-3-(2-amino-6-fluorophenyl)-4-chloro-1-(3-methoxy-2,2-dimethylpyrrolidin-1-yl)-6,6a,7,8,9,10-hexahydro-12*H-*pyrazino[2,1-*c*]pyrido[3,4-*f*][1,4]oxazepin-12-one (Z506, 30 mg, yield: 27%). ES-API: [M+H]⁺=544.3.

### Embodiment 99 Synthesis of (R)-8-acryloyl-4-chloro-3-(2-fluorophenyl)-1-((2S,5S)-2,4,5-trimethylpiperazin-1-yl)-6,6a,7,8,9,10-hexahydro-12H-pyrazino[2,1-c]pyrido[3,4-f][1,4]oxazepin-12-one (Z483)

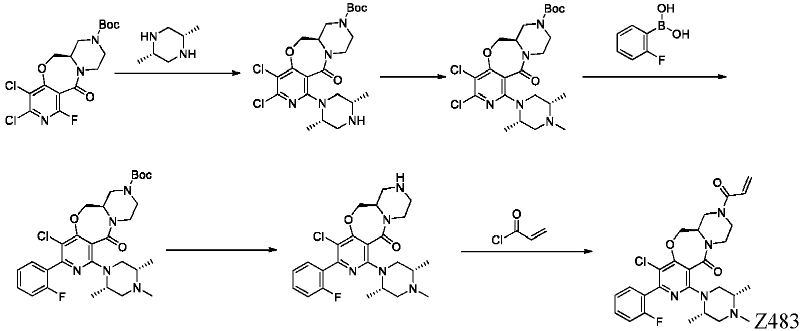

Step 1: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]-8-(12*H*)-carboxylate (300 mg, 0.74 mmol) and (2*S*,5*S*)-2,5-dimethylpiperazine (409 mg, 1.48 mmol) were dissolved in acetonitrile (3 mL), then potassium carbonate (409 mg, 2.96 mmol) was added thereto, and the reaction was stirred at room temperature for 16 hours. After the reaction was completed, the mixture was extracted with ethyl acetate, water, washed with brine, dried over anhydrous sodium sulfate, and the filtrate was evaporated to dryness by rotary evaporation and then and the crude product was purified by column chromatography with dichloromethane and 10% methanol to obtain a product of *tert*-butyl (*R*)-3,4-dichloro-1-((2*S*,5*S*)-2,5-dimethylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (270 mg, yield: 72.9%). ES-API: [M+H]⁺=500.1.

Step 2: *tert*-Butyl (*R*)-3,4-dichloro-1-((2*S*,5*S*)-2,5-dimethylpiperazin-1-yl)-12-oxo-6a,7,9,10-tetrahydro-6*H*-pyrazino[2,1-*c*]pyrido[3,4-*f*][1,4]457xazepane-8(12*H*)-carboxylate (270 mg, 0.54 mmol) was dissolved in methanol (5 mL), then 40% aqueous formaldehyde solution (61 mg, 0.81 mmol) and sodium cyanoborohydride (52 mg, 0.81 mmol) were added thereto and reacted at room temperature for 16 hours. After the reaction was completed, the mixture was quenched with water, extracted with ethyl acetate, washed with brine, dried over anhydrous sodium sulfate, and the filtrate was rotary evaporated and then purified by thin-layer chromatography plate (DCM:MeOH=10:1, R_{f}=0.7) to obtain a product of *tert*-butyl (*R*)-3,4-dichloro-12-oxo-1-((2*S*,5*S*)-2,4,5-trimethylpiperazin-1-yl)-6a,7,9,10-tetrahydro-6*H*-pyrazino[2,1-*c*]pyrido[3,4-*f*][1,4]oxazepine-8(12*H*)-carboxylate (190 mg, yield: 68.9%). ES-API: [M+H]⁺=514.1.

Step 3: *tert*-Butyl (*R*)-3,4-dichloro-12-oxo-1-((2*S*,5*S*)-2,4,5-trimethiylpiperazin-1-yl)-6a,7,9,10-tetrahydro-6*H*-pyrazino[2,1-*c*]pyrido[3,4-*f*][1,4]oxazepine-8(12*H*)-carboxylate (180 mg, 0.35 mmol) and K₂CO₃ (96 mg, 0.70 mmol) were dissolved in dioxane/water (3/0.6 mL), then (2-fluorophenyl)boronic acid (58.9 mg, 0.42 mmol), and 1,1'-bis(diphenylphosphino)ferrocene palladium dichloride (26 mg, 0.035 mmol) were added thereto, and the reaction was stirred at 100°C overnight for 16 hours. After the reaction was completed, the mixture was quenched with water, extracted with ethyl acetate, washed with brine, dried over anhydrous sodium sulfate, and the filtrate was evaporated to dryness by rotary evaporation and then purified by thin-layer chromatography plate (DCM:MeOH=10:1, R_{f}=0.6) to obtain compound *tert*-butyl (*R*)-4-chloro-3-(2-fluorophenyl)-12-oxo-1-((2*S*,5*S*)-2,4,5-trimethylpiperazin-1-yl)-6a,7,9,10-tetrahydro-6/7-pyrazino[2,1-*c*]pyrido[3.4-*f*][1,4|oxazepine-8(1*H*)-carboxylate (150 mg, yield: 90.3%). ES-API: [M+H]⁺=574.1.

Step 4: *tert*-Butyl (*R*)-4-chloro-3-(2-fluorophenyl)-12-oxo-1-((2*S*,5*S*)-2,4,5-trimethylpiperazin-1-yl)-6a,7,9,10-tetrahydro-6*H*-pyrazino[2,1-*c*]pyrido[3,4-*f*][1,4]oxazepine-8(12*H*)-carboxylate (170 mg, 0.29 mmol) was dissolved in dichloromethane (2 mL), then trifluoroacetic acid (2 mL) was added thereto, stirred at room temperature for 30 minutes. Then the reaction solution was evaporated to dryness by rotary evaporation to obtain a crude product of (*R*)-4-chloro-3-(2-fluoropheny1)-1-((2*S*,5*S*)-2,4,5-trimethylpiperazin-1-yl)-7,8,9,10-tetrahydro-6*H*-pyrazino[2,1-*c*]pyrido[3,4-*f*][1,4]oxazepin-12(6a*H*)-one, which was directly used in the next step. ES-API: [M+H]⁺=473.1.

Step 5: (*R*)-4-Chloro-3-(2-fhiorophenyl)-1-((2*S*,5*S*)-2,4,5-trimethylpiperazin-1-yl)-7,8,9,10-tetrahydro-6*H*-pyrazino[2,1-*c*]pyrido[3,4-*f*][1,4]oxazepin-12(6a*H*)-one (170 mg, 0.36 mmol) was dissolved in dichloromethane (4 mL), and then triethylamine (1.5 mL) and acryloyl chloride (65 mg, 0.72 mmol) were added thereto, and the mixture was stirred under an ice-water bath for 0.5 hours. After the reaction was completed, the mixture was extracted with water and ethyl acetate, washed with 1 N hydrochloric acid, saturated sodium bicarbonate, brine in turn, dried over anhydrous sodium sulfate, and the filtrate was evaporated to dryness by rotary evaporation and purified by thin-layer chromatography plate (DCM:MeOH=10:1, R_{f}=0.5) to obtain compound (*R*)-8-aciyloyl-4-chloro-3-(2-fluorophenyl)-1-((2*S*,5*S*)-2,4,5-trimethylpiperazin-1-yl)-6,6a,7,8,9,10-hexahydro-12*H*-pyrazino[2,1-*c*]pyrido[3,4-*f*][1,4]oxazepin-12-one (Z483, 20.4 mg, 10.75%). ¹H NMR (400 MHz, CDCl₃) δ 7.40 (s, 2H), 7.20 (d, *J* =11.0 Hz, 1H), 7.12 (s, 1H),6.53 (d, *J* = 13.6 Hz, 1H), 6.39 (d, *J* = 16.4 Hz, 1H), 5.80 (d, *J* = 10.3 Hz, 1H), 4.38 - 4.24 (m,1H), 4.07(dd, *J* = 61.7, 11.9 Hz,2H), 3.88 - 3.31 (m,8H), 2.98 (t, *J* = 12.4Hz, 2H), 2.61 (d, *J* = 11.4 Hz, 1H), 2.41 (d, *J* = 14.2 Hz, 1H), 2.24(s, 3H), 1.38 (s,3H), 1.05 (s,3H). ES-API: [M+H]⁺=528.2.

### Embodiment 100 Synthesis of (6aR)-8-acryloyl-4-chloro-1-(3,3-difluoro-5',5'-dimethyl-[1,3'-bipyrrolidin]-1'-yl)-3-(2-fluorophenyl)-6,6a,7,8,9,10-hexahydro-12H-pyrazino[2,1-c]pyrido[3,4-f][1,4]oxazepin-12-one (Z532)

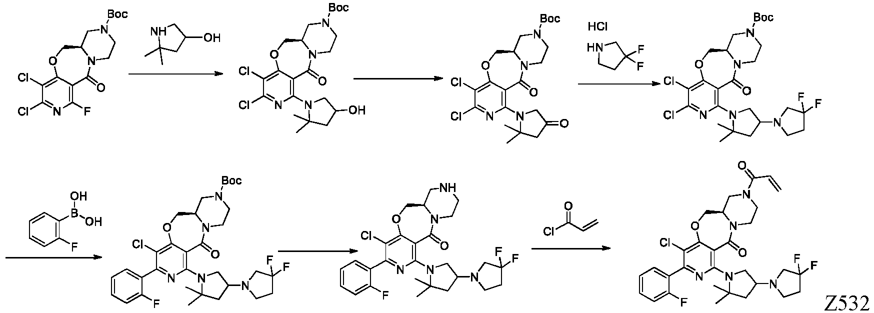

Step 1: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (800 mg, 1.970 mmol), 5,5-dimethylpyrrolidin-3-ol (250 mg, 2.167 mmol) and potassium carbonate (817 mg, 5.91 mmol) were dissolved in acetonitrile (6 mL), reacted at room temperature for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation, and purified by flash chromatography (ethyl acetate:petroleum ether: 0-80%) to obtain compound *tert*-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (800 mg, yield: 85%). ES-API: [M+H]⁺= 501.26

Step 2: Compound *tert*-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (400 mg, 0.8 mmol) and DMP (381 mg, 0.9 mmol) were dissolved in dichloromethane (15 mL). The reaction solution was stirred at room temperature for 4 hours, and the reaction was monitored to be complete. The mixture was extracted with dichloromethane, washed once with brine, dried over anhydrous sodium sulfate, and the filtrate was evaporated to dryness by rotary evaporation and purified by flash chromatography (ethyl acetate:petroleum ether: 20-60%) to obtain compound *tert*-butyl 3,4-dichloro-1-(2,2-dimethyl-4-oxopyrrolidin-1-yl)(R)-12-oxo-6a,7,9,10-tetrahydro-6*H*-pyrazino[2,1-*c*]pyrido[3,4-*f*][1,4]oxazepine-8(12*H*)-carboxylate (380 mg, yield: 95%). ES-API: [M+H]⁺=499.1.

Step 3: Compound *tert*-butyl 3,4-dichloro-1-(2,2-dimethyl-4-oxopyrrolidin-1-yl)(*R*)-12-oxo-6a,7,9,10-tetrahydro-6*H*-pyrazino[2,1-*c*]pyrido[3,4-*f*][1,4]oxazepine-8(12*H*)-carboxylate (380 mg, 0.76 mmol) was dissolved in 15 mL of acetonitrile, then sodium cyanoborohydride (124 mg, 2.28 mmol) was added thereto. The reaction solution was stirred at room temperature for 3 hours, and the reaction was monitored to be complete. The mixture was quenched by adding water, extracted with ethyl acetate, washed once with brine, dried over anhydrous sodium sulfate, and the filtrate was evaporated to dryness by rotary evaporation and purified by flash chromatography (ethyl acetate:petroleum ether: 20-60%) to obtain compound *tert*-butyl (6a*R*)-3,4-dichloro-1 -(3,3-difluoro-5',5'-dimethyl- [1,3'-bipyrrolidin] -1'-yl)-12-oxo-6a,7,9,10-tetrahydro-6*H*-pyrazino[2,1-*c*]pyrido[3,4-*f*[1,4]oxazepine-8(12*H*)-carboxylate (230 mg, yield: 52%). ES-API: [M+H]⁺= 590.1.

Step 4: Compound *tert*-butyl (6a*R*)-3,4-dichloro-1-(3,3-difluoro-5',5'-dimethyl-[1,3'-bipyrrolidin]-1'-yl)-12-oxo-6a,7,9,10-tetrahydro-6*H*-pyrazino[2,1-*c*]pyrido[3,4-*f*][1,4]oxazepine-8(12*H-*carboxylate (230 mg, 0.4 mmol), 2-fluorophenylboronic acid (109 mg, 0.78 mmol), [1,1'-bis(diphenylphosphino)ferrocene]palladium dichloride (10 mg, 0.04 mmol) and potassium carbonate (162 mg, 1.2 mmol) were dissolved in a mixed solution of dioxane and water (5 mL:1mL). The reaction solution was stirred at 100°C overnight, and the reaction was monitored to be complete. The mixture was extracted with ethyl acetate, washed once with brine, dried over anhydrous sodium sulfate, and the filtrate was evaporated to dryness by rotary evaporation and then purified by flash chromatography (ethyl acetate:petroleum ether: 20-60%) to obtain compound *tert*-butyl (6a*R*)-4-chloro-1-(3,3-difluoro-5',5'-dimethyl-[1,3'-bipyrrolidin]-1'-yl)-3-(2-fluorophenyl)-12-oxo-6a,7,9,10-tetrahydro-6*H-*pyrazino[2,1-*c*]pyrido[3,4-*f*][1,4]oxazepine-8(12*H*)-carboxylate (120 mg, yield: 47.4%). ES-API: [M+H]⁺=650.1.

Step 5: *tert*-Butyl (6a*R*)-4-chloro-1-(3,3-difluoro-5',5'-dimethyl-[1,3'-bipyrrolidin]-1'-yl)-3-(2-fluorophenyl)-12-oxo-6a,7,9,10-tetrahydro-6*H*-pyrazino[2,1-*c*]pyrido [3,4-*f*][1,4]oxazepine-8(12*H*)-carboxylate (120 mg, 0.19 mmol) was dissolved in dichloromethane (2 mL), then cooled to 0°C in an ice-water bath, and then trifluoroacetic acid (1.5 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (6a*R*)-4-chloro-1-(3,3-difluoro-5',5'-dimethyl-[1,3'-bipyrrolidin]-1'-yl)-3-(2-fluorophenyl)-7,8,9,10-tetrahydro-6*H*-pyrazino[2,1-*c*]pyrido[3,4-*f*][1,4]oxazepin-12-one (120 mg, crude product). ES-API: [M+H]⁺= 550.1.

Step 6: (6a*R*)-4-Chloro-1-(3,3-difluoro-5',5'-dimethyl-[1,3'-bipyrrolidin]-1'-yl)-3-(2-iluorophenyl)-7,8,9,10-tetrahydro-6*H*-pyrazino[2,1-*c*]pyrido[3,4-*f*][1,4]oxazepin-12-one (120 mg, 0.15 mmol) was dissolved in tetrahydrofuran (5 mL), then cooled to 0°C in an ice-water bath. Aqueous sodium bicarbonate solution was slowly added thereto to adjust the pH value to 8-9. Potassium phosphate (95 mg, 0.45 mmol) was added thereto, stirred at 0°C for 5minutes, and then a solution of acryloyl chloride in tetrahydrofuran (18 mg, 0.18 mmol) was slowly added dropwise thereto and stirred for 10 minutes. After the reaction was completed, the reaction was quenched by adding sodium bicarbonate solution, extracted with ethyl acetate, dried over sodium sulfate, evaporated to dryness by rotary evaporation, and purified by high performance liquid chromatography to obtain (6a*R*)-8-acryloyl-4-chloro-1-(3,3-difluoro-5',5'-dimethyl-[1,3'-bipyrrolidin]-1'-yl)-3-(2-fluorophenyl)-6,6a,7,8,9,10-hexahydro-12*H*-pyrazino[2,1-c]pyrido [3,4-*f*][1,4]oxazepin-12-one (Z532, 10 mg, yield: 8.3%). ¹H NMR (400 MHz, CDCl₃) δ 7.40 (q, *J* = 7.5 Hz, 2H), 7.22 (t, *J* = 7.5 Hz, 1H), 7.15 (t, *J* = 9.1 Hz, 1H), 6.54 (d, *J* = 11.4 Hz, 1H), 6.41 (d, *J* = 16.5 Hz, 1H), 5.82 (d, *J* = 10.3 Hz, 1H), 4.58 - 3.96 (m, 6H), 3.76 - 3.52 (m, 4H), 3.11 - 2.63 (m, 6H), 2.42 - 2.22 (m, 2H), 1.92 (d, *J* = 10.6 Hz, 2H), 1.61 (s, 6H). ES-API: [M+H]⁺= 604.3.

### Embodiment 101 Synthesis of (R)-8-acryloyl-4-chloro-3-(2-fluorophenyl)-1-((2S,3S)-2,3,4-trimethylpiperazin-1-yl)-6,6a,7,8,9,10-hexahydro-12H-pyrazino[2,1-c]pyrido[3,4-f][1,4]oxazepin-12(6aH)-one (Z653)

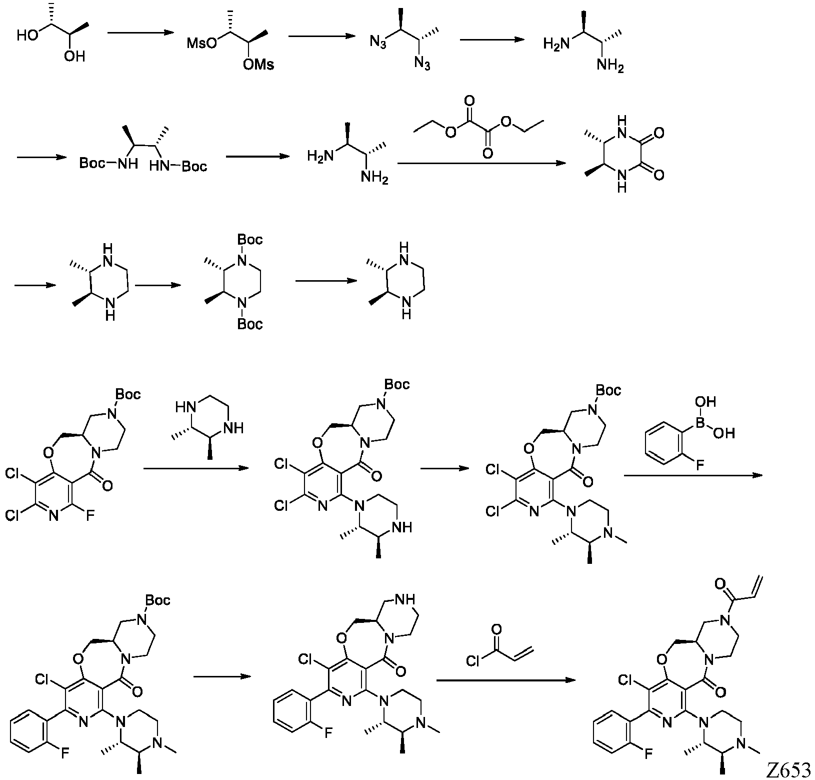

Step 1: (2*R*,3*R*)-Butan-2,3-diol (17 g, 0.18 mol) and triethylamine (114.6 g, 1.14 mol) were dissolved in dichloromethane (1 L), cooled to -60°C, and methylsulfonyl chloride (77.8 g, 0.68 mol) was added dropwise thereto, and the reaction was reacted for 2 hours while maintaining the temperature after the addition was completed. The completion of the reaction was monitored by thin-layer chromatography plate. The saturated sodium bicarbonate solution was added to quench the reaction and the layers were separated. The aqueous phase was extracted once with dichloromethane. The organic phases were combined, dried, filtered, evaporated to dryness by rotary evaporation, slurried with petroleum ether (0.5 L) to obtain (2*R*,3*R*)-butane-2,3-dimethyldimethanesulfonate (46 g, yield of 100%) as a white solid.

Step 2: (2*R*,3*R*)-Butane-2,3-dimethyldimethanesulfonate (37.6 g, 0.15 mol) and sodium azide (49.7 g, 0.76 mol) were added to DMF (0.7 L), heated to 80°C and reacted for 18 hours. The reaction solution was cooled to room temperature, poured into water (1 L), extracted with ethyl acetate (0.5 L ^{∗} 3), and the organic phases were combined, washed with water for three times, washed with brine once, dried and evaporated to dryness by rotary evaporation to obtain (2*S*,3*S*)-2,3-diazidobutane (22 g, yield of 100%), and the crude product was directly used in the next step.

Step 3: (2*S*,3*S*)-2,3-Diazidobutane (21 g, 0.15 mol), palladium on carbon (2 g) were added to methanol (200 mL), and the reaction was carried out for 18 hours after the system was replaced with hydrogen for three times. The completion of reaction was monitored by LC-MS. The mixture was filtered and evaporated to dryness by rotary evaporation to obtain (2*S*,3*S*)-butane-2,3-diamine (13.2 g, yield of 100%), and the crude product was directly used in the next step.

Step 4: (2*S*,3*S*)-Butane-2,3-diamine (0.15 mmol) was dissolved in dichloromethane (200 mL), and triethylamine (76 g, 0.75 mmol) and di-*tert*-butyl dicarbonate (98.1 g, 0.45 mmol) were added thereto, reacted at room temperature overnight. After the reaction was completed, the reaction mixture was extracted with dichloromethane and water, dried, evaporated to dryness by rotary evaporation, and purified by column chromatography (petroleum ether/ethyl acetate=2/1) to obtain a product (2.8 g, yield: 6%). (R_{f}=0.4, petroleum ether/ethyl acetate=2/1)

Step 5: di-*tert*-Butyl ((2*S*,3*S*)-butane-2,3-dicarbamate (1.5 g, 5.2 mmol) was dissolved in hydrochloric acid/dioxane (4 M, 15 mL) and reacted at room temperature for 4 hours. After the reaction was completed, the reaction solution was evaporated to dryness by rotary evaporation. Ethanol solution was added thereto, and potassium carbonate was added thereto and stirred for 0.5 hours, and then filtered, and the filtrate was directly used for the next reaction. MS-API: [M+H]⁺=89.1.

Step 6: Diethyl oxalate (1.1 g, 7.8 mmol) and potassium carbonate (2.2 g, 15.6 mmol) were added to an ethanol solution of (2*S*,3*S*)-butane-2,3-diamine (crude product, 5.2 mmol), heated to 80°C and reacted for 2 hours. After the reaction was completed, the reaction mixture was evaporated to dryness by rotary evaporation, and purified by column chromatography (dichloromethane/methanol=5/1) to obtain a product (440 mg, yield: 60%). (R_{f}=0.4, dichloromethane/methanol=5/1). MS-API: [M+H]⁺=143.1.

Step 7: (5*S*,6*S*)-5,6-Dimethylpiperazin-2,3-dione (440 mg, 3.1 mmol) was dissolved in tetrahydrofuran (15 mL), cool to 0-5°C, and lithium aluminum hydride (589 mg, 15.5 mmol) was added thereto, heated to 70°C and reacted for 2 hours. The reaction solution was quenched with water (0.6 mL)/2 M NaOH (0.6 mL)/water (1.2 mL), filtered, and the filtrate was directly used for the next reaction step. MS-API: [M+H]⁺=115.1.

Step 8: Potassium carbonate (2.1 g, 15.5 mmol) and di-*tert*-butyl dicarbonate (2 g, 9.3 mmol) were added to a solution of (2*S*,3*S*)-2,3-dimethylpiperazine (3.1 mmol) in tetrahydrofuran and reacted overnight at room temperature. The reaction solution was extracted with ethyl acetate and water, dried, evaporated to dryness by rotary evaporation, and purified by column chromatography (petroleum ether/ethyl acetate=3/1) to obtain a product (780 mg, yield: 80%). (R_{f}=0.5, petroleum ether/ethyl acetate=3/1). MS-API: [M+1-156]⁺=159.1

Step 9: di-*tert*-Butyl (2*S*,3*S*)-2,3-dimethylpiperazine-1,4-dicarboxylate (680 mg, 2.16 mmol) was dissolved in dichloromethane (6 mL), and then trifluoroacetic acid (3 mL) was added thereto and stirred at room temperature for 2 hours. The reaction solution was directly evaporated to dryness by rotary evaporation to obtain a crude product, which was directly used in the next reaction step. MS-API: [M+H]⁺=115.1.

Step 10: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (440 mg, 1.08 mmol) was dissolved in acetonitrile (10 mL), then (2*S*,3*S*)-2,3-dimethylpiperazine (crude product, 2.16 mmol) and potassium carbonate (896 mg, 6.5 mmol) were added thereto, heated to 80°C and reacted for 2 hours. The mixture was cooled to room temperature, quenched by adding water, extracted with ethyl acetate, dried, evaporated to dryness by rotary evaporation, and purified by column chromatography (dichloromethane/methanol=5/1) to obtain a product (160 mg, yield: 29%). (R_{f}=0.2, dichloromethane/methanol=10/1). MS-API: [M+H]⁺=500.2

Step 11: *tert*-Butyl (*R*)-3,4-dichloro-1-((2*S*,3*S*)-2,3-dimethylpiperazin-1-yl)-12-oxo-6a,7,9,10-tetrahydro-6*H*-pyrazino[2,1-*c*]pyrido[3,4-*f*][1,4]oxazepine-8(12*H*)-carboxylate (160 mg, 0.32 mmol) was dissolved in methanol (5 mL), then 37% aqueous formaldehyde solution (1 mL) and sodium cyanoborohydride (60 mg, 0.96 mmol) were added thereto and reacted at room temperature overnight. The reaction solution was quenched with water, extracted with dichloromethane, and the organic phase was dried, evaporated to dryness, and purified by column chromatography (dichloromethane/methanol=10/1) to obtain a product (100 mg, yield: 61%). (R_{f}=0.2, dichloromethane/methanol=10/1). MS-API: [M+H]⁺=514.2

Step 12: *tert*-Butyl (*R*)-3,4-dichloro-12-oxo-1-((2*S*,3*S*)-2,3,4-trimethylpiperazin-1-yl)-6a,7,9,10-tetrahydro-6*H*-pyrazino[2,1-*c*]pyrido[3,4-*f*][1,4]oxazepine-8(12*H*)-carboxylate (30 mg, 0.06 mmol) was dissolved in dioxane/water (0.5 mL/0.1 mL), then (2-fluorophenyl)boronic acid (16 mg, 0.12 mmol), potassium carbonate (24 mg, 0.18 mmol) and tetrakis(triphenylphosphine)palladium (7 mg, 0.006 mmol) were heated to 100°C and reacted overnight. The reaction solution was evaporated to dryness by rotary evaporation and purified by preparative thin-layer chromatography column (dichloromethane/methanol=10/1) to obtain a product (17 mg, yield: 51%). (R_{f}=0.2, dichloromethane/methanol=10/1). MS-API: [M+H]⁺= 574.3.

Step 13: *tert*-Butyl (*R*)-4-chloro-3-(2-fluorophenyl)-12-oxo-1-((2*S*,3*S*)-2,3,4-trimethylpiperazin-1-yl)-6a,7,9,10-tetrahydro-6*H*-pyrazino[2,1-*c*]pyrido[3,4-*f*][1,4]oxazepine-8(12*H*)-carboxylate (25 mg, 0.04 mmol) was dissolved in dichloromethane (2 mL), then trifluoroacetic acid (1 mL) was added thereto. The reaction solution was reacted at room temperature for 2 hours. After the reaction was completed, the reaction solution was evaporated to dryness, dissolved in dichloromethane and evaporated to dryness to obtain a crude product, which was directly used in the next reaction step. MS-API: [M+H]⁺=474.2.

Step 14: (*R*)-4-Chloro-3-(2-fluorophenyl)-1-((2*S*,3*S*)-2,3,4-trimethylpiperazin-1-yl)-7,8,9,10-tetrahydro-6*H*-pyrazino[2,1-*c*]pyrido[3,4-*f*][1,4]oxazepin-12(6a*H*)-one (crude product, 0.04 mmol) was dissolved in dichloromethane (2 mL), cooled to 0 to 5°C, and triethylamine (0.2 mL) and acryloyl chloride (4 mg, 0.04 mmol) were added thereto, stirred for 0.5 hours. After the reaction was completed, the mixture was extracted with dichloromethane and water, and the organic phase was dried, evaporated to dryness by rotary evaporation, and purified by preparative thin-layer chromatography column (dichloromethane/methanol=10/1) to obtain a product (Z653, 7.4 mg, yield: 32%, yellow solid). (R_{f}=0.2, dichloromethane/methanol=10/1). MS-API: [M+H]⁺=528.2. ¹H NMR (400 MHz, CDCl₃) δ 7.45-7.37 (m, 2H), 7.23-7.20 (m, 1H), 7.18-7.10 (m, 1H), 6.62-6.51 (m, 1H), 6.41 (d, *J* = 16.3 Hz, 1H), 5.82 (d, *J* = 10.3 Hz, 1H), 4.44-4.29 (m, 4H), 4.22-4.13 (m, 2H), 4.08-3.95 (m, 1H), 3.82-3.67 (m, 2H), 3.66-3.57 (m, 1H), 3.51-3.37 (m, 2H), 3.20-3.08 (m, 1H), 3.05-2.94 (m, 1H), 2.57 (s, 3H), 1.68-1.48 (m, 6H).

### Embodiment 102 Synthesis of two isomers of (6aR)-8-acryloyl-4-fluoro-1-(4-(3-fluoroazetidin-1-yl)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12-one (Z654-1 and Z654-2)

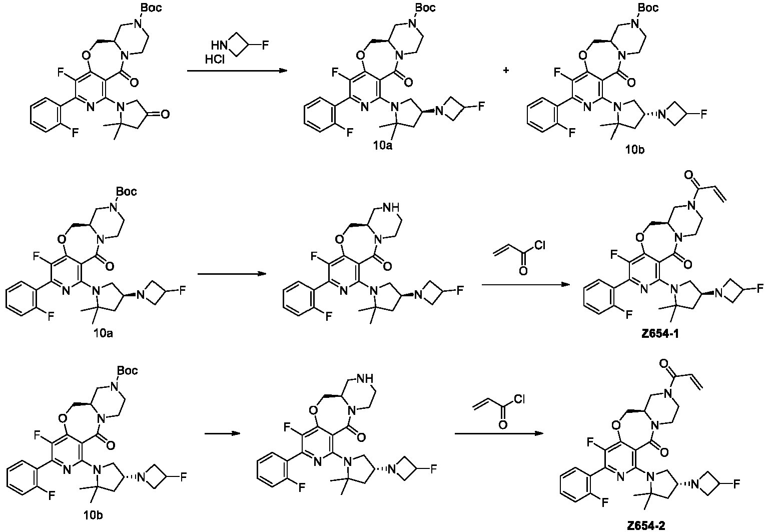

Step 1: A solution of tert-butyl (*R*)-1-(2,2-dimethyl-4-oxopyrrolidin-1-yl)-4-chloro-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (350 mg, 0.65 mmol) in acetonitrile (20 mL) was added with 3-fluoroazetidine hydrochloride (216 mg, 1.94 mmol), stirred at room temperature for 2 hours. Then sodium cyanoborohydride (81 mg, 1.29 mmol) was added thereto, and the mixture was continued to be stirred for 1 hour. After the reaction was completed, the mixture was quenched with saturated ammonium chloride solution (20 mL), extracted with ethyl acetate (20 mL^{∗}3). The organic phases were combined, dried and concentrated, and purified by flash silica gel column, eluted with eluent (ethyl acetate: petroleum ether: 0-100%) to obtain intermediate 10b (with small polarity and arbitrarily designated structure as *tert*-butyl (*R*)-4-fluoro-1-((*R*)-4-(3-fhioroazetidin-1-yl)-2,2-dimethylpyrrolidin-1-yl)-3-(2-fluorophenyl)-12-oxo-6a,7,9,10-tetrahydro-12*H-*pyrazino[2,1-*c*]pyrido[3,4-*f*][1,4]oxazepine-8(6*H*)-carboxylate) (180 mg); and eluted with eluent (methanol:dichloromethane: 0-8%) to obtain intermediate 10a (with large polarity and arbitrarily designated structure as *tert*-butyl (*R*)-4-fluoro-1-((*S*)-4-(3-fhioroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate) (180 mg). ES-API: [M+H]⁺=602.3.

Step 2: Under an ice-bath, a solution of tert-butyl (*R*)-4-fluoro-1-((*S*-4-(3-fluoroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (180 mg, 0.30 mmol) in dichloromethane (2 mL) was added with trifluoroacetic acid (1 mL), and the reaction solution was stirred at room temperature for 1 hour. The reaction solution was concentrated under reduced pressure to obtain a crude product of (*R*)-4-fluoro-1-((*S*)-4-(3 -fhioroazetidin-1 -yl)-2,2-dimethylpyrrolidin-1 -yl)-3-(2-fhiorophenyl)-6,6a,7,8,9,10-hexahydro-12*H*-pyrazino[2,1-*c*]pyrido[3,4-*f*][1,4]oxazepin-12-one (150 mg). ES-API: [M+H]⁺=502.3.

Step 3: The above crude procuct of (*R*)-4-fluoro-1-((*S*-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (150 mg, 0.30 mmol) was dissolved in dichloromethane (2 mL), and *N,N-*diisopropylethylamine (194 mg, 1.50 mmol) was added thereto. The reaction solution was cooled to 0°C, and acryloyl chloride (27 mg, 0.30 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-4-fluoro-1-((*S*-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one(Z654-1,44 mg, yield: 26%). ES-API: [M+H]⁺=556.2. ¹H NMR (500 MHz, DMSO-*d*₆) δ 7.61 - 7.50 (m, 2H), 7.40 - 7.30 (m, 2H), 6.84 - 6.72 (m, 1H), 6.22 - 6.09 (m, 1H), 5.79 - 5.67 (m, 1H), 5.08 (d, *J* = 57.5 Hz, 1H), 4.66 - 4.52 (m, 1H), 4.43 - 4.25 (m, 1H), 4.25 - 4.07 (m, 2H), 4.07 - 3.82 (m, 2H), 3.80 (s, 1H), 3.60 - 3.36 (m, 5H), 3.13 - 2.86 (m, 3H), 2.58 - 2.52 (m, 1H), 1.86 (dd, *J* = 13.5, 5.0 Hz, 1H), 1.81 - 1.74 (m, 1H), 1.73 (s, 3H), 1.43 (s, 3H).

Step 4: Under an ice-bath, a solution of *tert*-butyl (*R*)-4-fluoro-1-((*R*)-4-(3-fluoroazetidin-1-yl)-2,2-dimethylpyrrolidin-1-yl)-3-(2-fluorophenyl)-12-oxo-6a,7,9,10-tetrahydro-12*H*-pyrazino[2,1-*c*]pyrido[3,4*f*][1,4]oxazepine-8(6*H*)-carboxylate (180 mg, 0.30 mmol) in dichloromethane (2 mL) was added with trifluoroacetic acid (1 mL), and the reaction solution was stirred at room temperature for 1 hour. The reaction solution was concentrated under reduced pressure to obtain a crude product of (*R*)-4-fluoro-1-((*R*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (150 mg). ES-API: [M+H]⁺=502.3.

Step 5: The above crude procuct of (*R*)-4-fluoro-1-((*R*)-4-(3-fluoroazetidin-1-yl)-2,2-dimethylpyrrolidin-1-yl)-3-(2-fluorophenyl)-6,6a,7, 8,9,10-hexahydro-12*H*-pyrazino [2,1-c]pyrido [3,4-*f*][1,4]oxazepin-12-one (150 mg, 0.30 mmol) was dissolved in dichloromethane (2 mL), and *N,N-*diisopropylethylamine (194 mg, 1.50 mmol) was added thereto. The reaction solution was cooled to 0°C, and acryloyl chloride (27 mg, 0.30 mmol) was added dropwise thereto. After stirring for 5 minutes, the mixture was concentrated, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=20%-100%; column temperature: room temperature) to obtain (*R*)-8-acryloyl-4-fluoro-1-((*R*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z654-2, 33 mg, yield: 20%). ES-API: [M+H]⁺=556.2. ¹H NMR (500 MHz, DMSO-*d*₆) δ 7.61 - 7.50 (m, 2H), 7.41 -7.30 (m, 2H), 6.75 (dd, *J* = 16.5, 10.5 Hz, 1H), 6.22 - 6.10 (m, 1H), 5.78 - 5.68 (m, 1H), 5.26 - 5.06 (m, 1H), 4.55 (s, 1H), 4.42 - 4.28 (m, 1H), 4.25 - 3.69 (m, 5H), 3.67 - 3.44 (m, 4H), 3.26 - 3.02 (m, 4H), 2.96 - 2.82 (m, 1H), 1.90 (dd, *J* = 11.5, 5.5 Hz, 1H), 1.61 - 1.51 (m, 4H), 1.48 (s, 3H).

The configurations of Z654-1 and Z654-2 were assigned according to the structures of intermediates 10a and 10b.

### Embodiment 103 Synthesis of two isomers of (6aR)-8-acryloyl-4-chloro-1-(4-(dimethylamino)-2,2-dimethylpyrrolidin-1-yl)-3-(phenyl)-6,6a,7,8,9,10-hexahydro-12H-pyrazino[2,1-c]pyrido[3,4-f][1,4]oxazepin-12-one (Z655-1 and Z655-2)

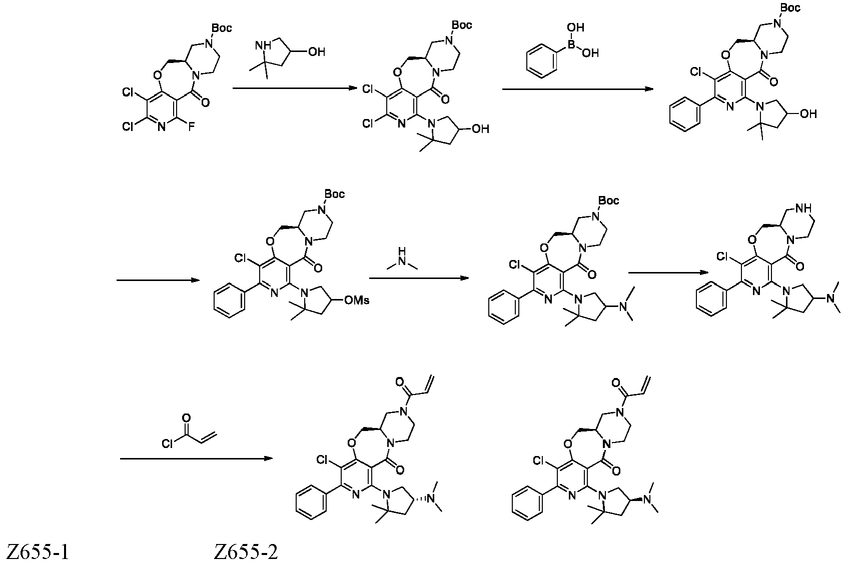

Step 1: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (2640 mg, 6.522 mmol), 5,5-dimethylpyrrolidin-3-ol (700 mg, 7.826 mmol) and potassium carbonate (2700 mg, 19.566 mmol) were dissolved in acetonitrile (26 mL), reacted at room temperature for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation, and purified by flash chromatography (ethyl acetate:petroleum ether: 0-80%) to obtain tert-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (3.2 g, yield: 95%). ES-API: [M+H]⁺= 501.26

Step 2: *tert*-Butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (300 mg , 0.6 mmol), (phenyl)boronic acid (88 mg, 0.72 mmol), [1,1'-bis(diphenylphosphino)ferrocene]palladium dichloride dichloromethane (44 mg, 0.06 mmol) and potassium carbonate (166 mg, 1.2 mmol) were dissolved in a mixed solution of dioxane and water (3 mL:0.6 mL). The reaction solution was stirred at 110°C overnight, and the reaction was monitored to be complete. The mixture was extracted with ethyl acetate, washed once with brine, dried over anhydrous sodium sulfate, and the filtrate was evaporated to dryness by rotary evaporation and purified by flash chromatography (ethyl acetate:petroleum ether: 0-85%) to obtain *tert*-butyl (6a*R*)-4-chloro-3-(2-fluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (290 mg, yield: 89%). ES-API: [M+H]⁺= 543.3

Step 3: *tert-*Butyl (6a*R*)-4-chloro-3-(phenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (290 mg, 0.534 mmol) was dissolved in 4 mL of dichloromethane, then methanesulfonyl chloride (122 mg, 1.068 mmol) and triethylamine (162 mg, 1.602 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation and prepared by column chromatography to obtain *tert*-butyl (*R*)-4-chloro-1-2,2-dimethyl-4-((methylsulfonyl)oxy)pyrrolidin-1-yl)-3-(phenyl)-12-oxo-6a,7,9,10-tetrahydro-6*H*-pyrazino[2,1-*c*]pyrido[3,4-*f*][1,4]oxazepine-8(12*H*)-carboxylate (310 mg, yield: 95%). ES-API: [M+H]⁺= 639.3.

Step 4: *tert*-Butyl (*R*)-4-chloro-1-((*S*)-2,2-dimethyl-4-((methylsulfonyl)oxy)pyrrolidin-1-yl)-3-(phenyl)-12-oxo-6a,7,9,10-tetrahydro-6*H*-pyrazino[2,1-*c*]pyrido[3,4-*f*][1,4]oxazepine-8(12*H*)-carboxylate (310 mg, 0.5 mmol) was dissolved in 10 mL of aqueous dimethylamine solution (40%wt), and then *N,N-*diisopropylethylamine (323 mg, 2.5 mmol) was added thereto. The reaction solution was heated to 100°C, then stirred for 16 hours. The reaction was monitored to be complete, then the reaction solution was cooled to room temperature, extracted by adding ethyl acetate, washed once with brine, dried over anhydrous sodium sulfate, and the filtrate was evaporated to dryness by rotary evaporation and purified (dichloromethane/methanol=20/1) to obtain *tert*-butyl (*R*)-4-chloro-1-4-(dimethylamino)-2,2-dimethylpyrrolidin-1-yl)-3-(phenyl)-12-oxo-6a,7,9,10-tetrahydro-6*H*-pyrazino[2,1-*c*]pyrido[3,4-*f*][1,4]oxazepine-8(12*H*)-carboxylate (68 mg, yield: 24%). ES-API: [M+H]⁺= 570.3

Step 5: *tert*-Butyl (*R*)-4-chloro-1-4-(dimethylamino)-2,2-dimethylpyrrolidin-1-yl)-3-(phenyl)-12-oxo-6a,7,9,10-tetrahydro-6*H*-pyrazino[2,1-*c*]pyrido[3,4-*f*][1,4]oxazepine-8(12*H*)-carboxylate (68 mg, 0.119 mmol) was dissolved in dichloromethane (2 mL), then cooled to 0°C in an ice-water bath, and then trifluoroacetic acid (1 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (*R*)-4-chloro-1-4-(dimethylamino)-2,2-dimethylpyrrolidin-1-yl)-3-(phenyl)-7,8,9,10-tetrahydro-6*H*-pyrazino[2,1-*c*]pyrido[3,4-*f*][1,4]oxazepin-12-one (56 mg, yield: 95%). ES-API: [M+H]⁺= 470.2

Step 6: (*R*)-4-Chloro-1-4-(dimethylamino)-2,2-dimethylpyrrolidin-1-yl)-3-(phenyl)-7,8,9,10-tetrahydro-6*H*-pyrazino[2,1-*c*]pyrido[3,4-*f*][1,4]oxazepin-12-one (56 mg, 0.119 mmol) was dissolved in dichloromethane (1.5 mL), then cooled to 0°C in an ice-water bath. Triethylamine (36 mg, 0.357 mmol) and acryloyl chloride (16 mg, 0.179 mmol) were added dropwise thereto, and stirred for 30 minutes. After the reaction was completed, the mixture was added with sodium bicarbonate solution to adjust to alkaline, extracted with dichloromethane, dried over sodium sulfate, evaporated to dryness by rotary evaporation, and purified by preparative thin-layer chromatography plate (dichloromethane/methanol=10/1) to obtain two isomer compounds:

One isomer compound with a structure arbitrarily designated as (*R*)-8-acryloyl-4-chloro-1-((*S*-4-(dimethylamino)-2,2-dimethylpyrrolidin-1-yl)-3-(phenyl)-6,6a,7, 8,9,10-hexahydro-12*H*-pyrazino [2,1-*c*]pyrido[3,4-*f*][1,4]oxazepin-12-one (Z655-1, R_{f}= 0.5, 2.75 mg, yield: 4%);
another isomer compound with a structure arbitrarily designated as (*R*)-8-acryloyl-4-chloro-1-((*R*)-4-(dimethylamino)-2,2-dimethylpyrrolidin-1-yl)-3-(phenyl)-6,6a,7,8,9,10-hexahydro-12*H-*pyrazino[2,1-*c*]pyrido[3,4-*f*][1,4]oxazepin-12-one (Z655-2, R_{f}=0.4, 2.33 mg, yield: 3.8%). ES-API: [M+H]⁺= 524.3.

### Embodiment 104 Synthesis of (R)-8-acryloyl-4-chloro-3-(2-fluorophenyl)-N-isopropyl-N-methyl-6,6a,7,8,9,10-hexahydro-6H-pyrazino[2,1-c]pyrido[3.4-f][1,4]oxazepine-1-carboxamide (Z656)

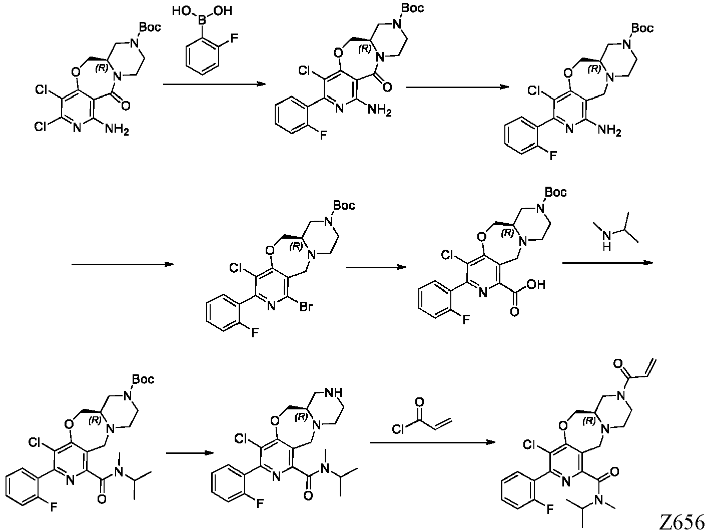

Step 1: *tert*-Butyl (*R*)-1-amino-3,4-dichloro-12-oxo-6a,7,9,10-tetrahydro-6*H*-pyrazino[2,1-*c*]pyrido[3,4-*f*][1,4]oxazepine-8(12*H*)-carboxylate (1.88 g, 4.67 mmol), (2-fluorophenyl)boronic acid (0.98 g, 7 mmol), sodium carbonate (0.98 g, 9.33 mmol), [1,1'-bis(diphenylphosphino)ferrocene]palladium dichloride (0.3 g) were added to dioxane (20 mL) and water (2 mL) in turn, heated to 100°C and reacted for 18 hours. The mixture was cooled, then added with ethyl acetate (100 mL), washed once with brine, dried and evaporated to dryness by rotary evaporation, and purified by column chromatography (ethyl acetate/petroleum ether: 20%-60%) to obtain *tert*-butyl (*R*)-1-amino-4-chloro-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (1.7 g, yield of 70%). ES-API: [M+H]⁺=462.2

Step 2: *tert*-Butyl (*R*)-1-amino-4-chloro-3-(2-fluorophenyl)-12-oxo-6a,7,9,10-tetrahydro-6*H-*pyrazino[2,1-*c*]pyrido[3,4-*f*][1,4]oxazepine-8(12*H*)-carboxylate (1.7 g, 3.67 mmol) was dissolved in tetrahydrofuran (10 mL) and borane (11 mL , 11 mmol, 1 M tetrahydrofuran solution) was added thereto, heated to 70°C and reacted for 3 hours. The mixture was cooled, then quenched with water (50 mL), extracted with ethyl acetate and then purified by column chromatography (ethyl acetate/petroleum ether: 15%-60%) to obtain *tert*-butyl (*R*)-1-amino-4-chloro-3-(2-fluorophenyl)-6a,7,9,10-tetrahydro-6*H-*pyrazino[2,1-*c*]pyrido[3,4-*f*][1,4]oxazepine-8(12*H*)-carboxylate (1.5 g, yield of 90%), which was used in the next step. ES-API: [M+H]⁺=449.2.

Step 3: *tert*-Butyl (*R*)-1-amino-4-chloro-3-(2-fluorophenyl)-6a,7,9,10-tetrahydro-6H-pyrazino[2,1-*c*]pyrido[3,4-*f*][1,4]oxazepine-8(12*H*)-carboxylate (510 mg, 1.14 mmol) was added to water (4 mL), then hydrobromic acid (245 mg, 1.70 mmol) was added thereto, stirred uniformly under an ice bath, and then sodium nitrite (94 mg, 1.36 mmol, dissolved in 1 mL of water) was added dropwise thereto, reacted for 1 hour and then cuprous bromide (245 mg, 1.7 mmol) was added thereto, and stirred in an ice bath for 2 hours. The treatments were done twice in parallel, combined, and the mixture was extracted with ethyl acetate and then purified by column chromatography (ethyl acetate/petroleum ether: 10%-30%) to obtain *tert*-butyl (*R*)-1-bromo-4-chloro-3-(2-fluorophenyl)-6a,7,9,10-tetrahydro-6*H*-pyrazino [2,1-*c*]pyrido [3,4-*f*][1,4]oxazepine-8(12*H*)-carboxylate (0.2 g, yield of 20%). ES-API: [M+H]⁺=512 514

Step 4: *tert*-Butyl (*R*)-1-bromo-4-chloro-3-(2-fluorophenyl)-6a,7,9,10-tetrahydro-6*H-*pyrazino[2,1-*c*]pyrido[3,4-*f*][1,4]oxazepine-8(12*H*)-carboxylate (200 mg, 0.39 mmol) was dissolved in dioxane (2 mL), and palladium acetate (20 mg) was added thereto. After the system was replaced with carbon monoxide for three times, the mixture was heated to 100°C and reacted for 18 hours. The mixture was cooled, the filtered and evaporated to dryness by rotary evaporation for the next step.

Step 5: The crude product of (*R*)-8-(*tert*-butoxycarbonyl)-4-chloro-3-(2-fluorophenyl)-6,6a,7,8,9,10,12-hexahydro-6*H*-pyrazino[2,1-*c*]pyrido[3,4-*f*][1,4]oxazepine-1-carboxylic acid was dissolved in dichloromethane (3 mL), and *N*,*N*-diisopropylethylamine (75 mg, 0.59 mmol) and HATU (221 mg, 0.59 mmol) were added thereto in turn, then reacted at room temperature for 3 hours. The mixture was added with ethyl acetate (50 mL), washed with water and brine, dried and purified by chromatography plate (5% methanol, dichloromethane) to obtain *tert*-butyl (*R*)-4-chloro-3-(2-fluorophenyl)-1-(isopropyl(methyl)carbamoyl)-6a,7,9,10-tetrahydro-6*H*-pyrazino[2,1-*c*]pyrido[3,4-*f*][1,4]oxazepine-8(12*H*)-carboxylate (30 mg, yield of 15%).

Step 6: *tert*-Butyl (*R*)-4-chloro-3-(2-fluorophenyl)-1-(isopropyl(methyl)carbamoyl)-6a,7,9,10-tetrahydro-6*H*-pyrazino[2,1-*c*]pyrido[3,4-*f*][1,4]oxazepine-8(12*H*-carboxylate (30 mg, 0.11 mmol) was dissolved in dichloromethane (0.5 mL), and then trifluoroacetic acid (0.5 mL) was added thereto, stirred at room temperature for 1 hour and then evaporated to dryness by rotary evaporation to obtain (*R*)-4-chloro-3-(2-fluorophenyl)-*N*-isopropyl-*N*-methyl-6a,7,8,9,10,12-hexahydro-6*H*-pyrazino [2,1-*c*]pyrido[3,4-*f*][1,4]oxazepine-1-carboxamide (225 mg, yield of 100%) for the next step.

Step 7: (*R*)-4-Chloro-3-(2-fluorophenyl)-*N*-isopropyl-*N*-methyl-6a,7,8,9,10,12-hexahydro-6*H-*pyrazino[2,1-*c*]pyrido[3,4-*f*][1,4]oxazepine-1-carboxamide (20 mg, 0.046 mmol) was added to dichloromethane (0.5 mL), and triethylamine (7 mg, 0.069 mmol) was added thereto, and acryloyl chloride (4.2 mg, 0.046 mmol) was added thereto after stirring in an ice bath for half an hour. The reaction was quenched by adding water after one hour of reaction, extracted with ethyl acetate and purified by chromatography plate (5% MeOH/DCM) to obtain a product of (*R*)-8-acryloyl-4-chloro-3-(2-fluorophenyl)-*N*-isopropyl-*N*-methyl-6a,7,8,9,10,12-hexahydro-6*H*-pyrazino[2,1-*c*]pyrido[3,4-*f*][1,4]oxazepine-1-carboxamide (Z656, 5 mg, yield of 22%). ES-API: [M+H]⁺= 487.2. ¹H NMR (400 MHz, CDCl₃) δ 7.42 - 7.36 (m, 2H), 7.23 (s, 1H), 7.12 (d, *J* = 9.6 Hz, 1H), 6.51 (d, *J* = 10.7 Hz, 1H), 6.30 (d, *J* = 16.5 Hz, 1H), 5.72 (d, *J* = 10.5 Hz, 1H), 4.47 (d, *J* = 13.0 Hz, 1H), 4.06 (d, *J* = 18.0 Hz, 2H), 3.80 (d, *J* = 19.2 Hz, 2H), 3.39 (d, *J* = 71.2 Hz, 2H), 2.95 (s, 3H), 2.82 (d, *J* = 19.6 Hz, 2H), 2.73 (s, 2H), 2.66 (s, 1H), 1.17 (d, *J* = 3.7 Hz, 6H).

### Embodiment 105 Synthesis of (6aR)-8-acryloyl-4-chloro-1-4-(methylethylamine)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12-one (Z657)

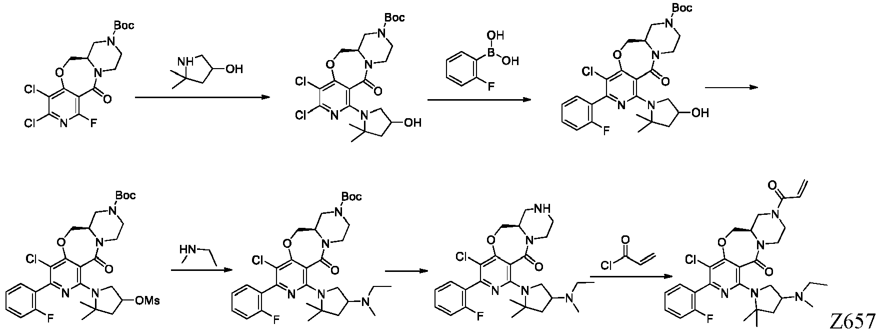

Step 1: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (800 mg, 1.970 mmol), 5,5-dimethylpyrrolidin-3-ol (250 mg, 2.167 mmol) and potassium carbonate (817 mg, 5.91 mmol) were dissolved in acetonitrile (6 mL), reacted at room temperature for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation, and purified by flash chromatography (ethyl acetate:petroleum ether: 0-80%) to obtain tert-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido [3,4-*f*][1,4]oxazepine-8(12*H*)-carboxylate (880 mg, yield: 89%). ES-API: [M+H]⁺= 501.26

Step 2: *tert*-Butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*-carboxylate (880 mg, 1.756 mmol), (2-fluorophenyl)boronic acid (295 mg, 2.108 mmol), [1,1'-bis(diphenylphosphino)ferrocene]palladium dichloride dichloromethane (129 mg, 0.76 mmol) and potassium carbonate (727 mg, 5.268 mmol) were dissolved in a mixed solution of dioxane and water (5 mL:1 mL). The reaction solution was stirred at 110°C overnight, and the reaction was monitored to be complete. The mixture was extracted with ethyl acetate, washed once with brine, dried over anhydrous sodium sulfate, and the filtrate was evaporated to dryness by rotary evaporation and purified by flash chromatography (ethyl acetate:petroleum ether: 0-85%) to obtain (6a*R*)-4-chloro-3-(2-fluorophenyl)-1-(4-hydroxy-2,2-dimethylpyrrolidin-1-yl)-12-oxo-6a,7,9,10-tetrahydro-6*H*-pyrazino[2,1-c]pyrido [3,4-*f*][1,4]oxazepine-8(12*H*)-carboxylate (720 mg, yield: 73%). ES-API: [M+H]⁺= 561.3

Step 3: *tert*-Butyl (6a*R*)-4-chloro-3-(2-fluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (610 mg, 1.087 mmol) was dissolved in 6 mL of dichloromethane, then methanesulfonyl chloride (188 mg, 1.631 mmol) and triethylamine (329 mg, 3.261 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation and purified by thin-layer chromatography plate to obtain tert-butyl (*R*)-4-chloro-1-((*S*)-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (650 mg, yield: 94%). ES-API: [M+H]⁺= 639.3

Step 4: *tert*-Butyl (*R*)-4-chloro-1-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (350 mg, 0.548 mmol) was dissolved in methylethylamine (2.3 g, 39.1 mmol), and then *N,N-*diisopropylethylamine (252 mg, 1.956 mmol) was added thereto. The reaction solution was heated to 100°C, then stirred for 16 hours. The reaction was monitored to be complete, then the reaction solution was cooled to room temperature, extracted by adding ethyl acetate, washed once with brine, dried over anhydrous sodium sulfate, and the filtrate was evaporated to dryness by rotary evaporation and purified (dichloromethane/methanol=20/1) to obtain *tert*-butyl (*R*)-4-chloro-1-4-(methylethylamine)-2,2-dimethylpyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (80 mg, yield: 53%). ES-API: [M+H]⁺= 602.4

Step 5: *tert*-Butyl (*R*)-4-chloro-1-4-(methylethylamine)-2,2-dimethylpyrrolidin-1-yl)-3-(2-fluoropheny1)-12-oxo-6a,7,9,10-tetrahydro-6*H*-pyrazino[2,1-*c*]pyrido[3,4-*f*][1,4]oxazepine-8(12*H*)-carboxylate (80 mg, 0.133 mmol) was dissolved in dichloromethane (2 mL), then cooled to 0°C in an ice-water bath, and then trifluoroacetic acid (1 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (*R*)-4-chloro-1-4-(methylethylamine)-2,2-dimethylpyrrolidin-1-yl)-3-(2-fluorophenyl)-7,8,9,10-tetrahydro-6*H*-pyrazino[2,1-*c*]pyrido[3,4-*f*][1,4]oxazepin-12(6a*H*)-one (68 mg, yield: 95%). ES-API: [M+H]⁺= 502.3

Step 6: (*R*)-4-Chloro-1-(4-(methylethylamine)-2,2-dimethylpyrrolidin-1-yl)-3-(2-fluorophenyl)-7,8,9,10-tetrahydro-6*H*-pyrazino[2,1-*c*]pyrido[3,4-*f*][1,4]oxazepin-12(6a*H*)-one (68 mg, 0.133 mmol) was dissolved in dichloromethane (1.5 mL), then cooled to 0°C in an ice-water bath, and triethylamine (40 mg, 0.399 mmol) and acryloyl chloride (10 mg, 0.201 mmol) were added dropwise thereto, stirred for 30 minutes. After the reaction was completed, the mixture was added with sodium bicarbonate solution to adjust to alkaline, extracted with dichloromethane, dried over sodium sulfate, and evaporated to dryness by rotary evaporation and purified (dichloromethane/methanol=10/1, R_{f}=0.4) to obtain (6a*R*)-8-acryloyl-4-chloro-1-(4-(methylethylamine)-2,2-dimethylpyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one (Z657, 29 mg, yield: 39%). ES-API: [M+H]⁺= 556.3

### Embodiment 106 Synthesis of (6aR)-8-acryloyl-4-chloro-3-(2-fluorophenyl)-1-(4-((2-hydroxyethyl)(methyl)amino)-2,2-dimethylpyrrolidin-1-yl)-6,6a,7,8,9,10-hexahydro-12H-pyrazino[2,1-c]pyrido[3,4-f][1,4]oxazepin-12-one(Z658)

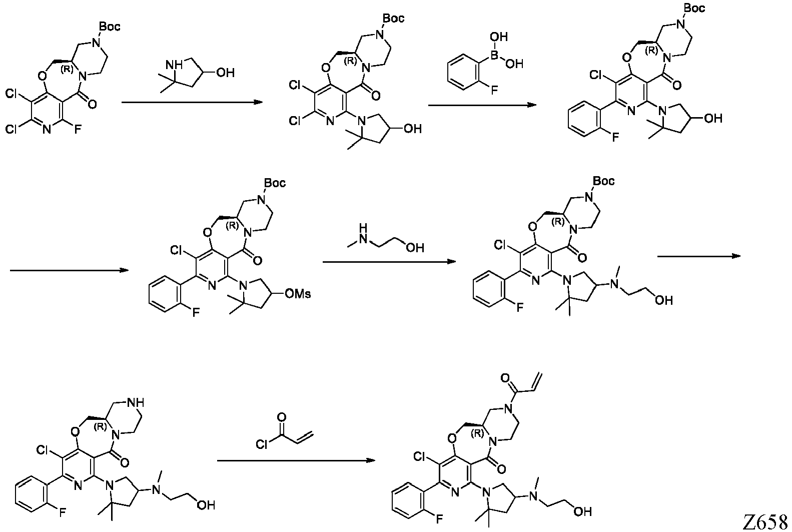

Step 1: *tert*-Butyl (*R*)-3,4-dichloro-1-fluoro-12-oxo-6a,7,9,10-tetrahydro-6*H*-pyrazino[2,1-*c*]pyrido[3,4-*f*][1,4]oxazepine-8(12*H*)-carboxylate (800 mg, 1.970 mmol), 5,5-dimethylpyrrolidin-3-ol (250 mg, 2.167 mmol) and potassium carbonate (817 mg, 5.91 mmol) were dissolved in acetonitrile (6 mL), reacted at room temperature for 16 hours. After the reaction was completed, the reaction system was cooled to room temperature, quenched with water, extracted with ethyl acetate, washed once with brine, dried over anhydrous sodium sulfate, and the filtrate was evaporated to dryness by rotary evaporation, and purified by flash chromatography (ethyl acetate:petroleum ether: 0-80%) to obtain *tert*-butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (880 mg, yield: 89%). ES-API: [M+H]⁺= 501.26.

Step 2: *tert*-Butyl (6a*R*)-3,4-dichloro-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*-carboxylate (300 mg, 0.599 mmol), (2-fluorophenyl)boronic acid (101 mg, 0.718 mmol), [1,1'-bis(diphenylphosphino)ferrocene]palladium dichloride dichloromethane (44 mg, 0.06 mmol) and potassium carbonate (248 mg, 1.797 mmol) were dissolved in a mixed solution of dioxane and water (2.5 mL:0.5 mL). The reaction solution was stirred at 100°C overnight, and the reaction was monitored to be complete. The mixture was extracted with ethyl acetate, washed once with brine, dried over anhydrous sodium sulfate, and the filtrate was evaporated to dryness by rotary evaporation and purified by flash chromatography (ethyl acetate:petroleum ether: 0-85%) to obtain *tert*-butyl (6a*R*)-4-chloro-3-(2-fluorophenyl)-1-(4-hydroxy-2,2-dimethylpyrrolidin-1-yl)-12-oxo-6a,7,9,10-tetrahydro-6*H*-pyrazino[2,1-*c*]pyrido [3,4-*f*][1,4]oxazepine-8(12*H*)-carboxylate (280 mg, yield: 83%). ES-API: [M+H]⁺= 561.3.

Step 3: *tert*-Butyl (6a*R*)-4-chloro-3-(2-fluorophenyl)-1-(4-hydroxy-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (280 mg, 0.499 mmol) was dissolved in 3 mL of dichloromethane, then methanesulfonyl chloride (86 mg, 0.749 mmol) and triethylamine (151 mg, 1.497 mmol) were added thereto. The reaction solution was stirred at room temperature for 2 hours, and the reaction was monitored to be complete. The mixture was quenched by adding water, extracted with dichloromethane, washed once with brine, dried over anhydrous sodium sulfate, and the filtrate was evaporated to dryness by rotary evaporation and purified by thin-layer chromatography plate to obtain tert-butyl (*R*)-4-chloro-1-((*S*)-2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*)-carboxylate (310 mg, yield: 95%). ES-API: [M+H]⁺= 639.3.

Step 4: *tert*-Butyl (6a*R*)-4-chloro-1-(2,2-dimethyl-4-((methylsulfonyl)oxy)pyrrolidin-1-yl)-3-(2-fluorophenyl)-12-oxo-6a,7,9,10-tetrahydro-6*H*-pyrazino[2,1-*c*]pyrido[3,4-*f*][1,4]oxazepine-8(12*H*) carboxylate (260 mg, 0.4 mmol) was dissolved in 2-(methylamino)ethanol (2.5 mL), and then *N,N-*diisopropylethylamine (263 mg, 2.03 mmol) was added thereto. The reaction solution was heated to 100°C, then stirred for 16 hours. The reaction was monitored to be complete, then the reaction solution was cooled to room temperature, extracted by adding ethyl acetate, washed once with brine, dried over anhydrous sodium sulfate, and the filtrate was rotary evaporated, and then purified by thin-layer chromatography plate (DCM:MeOH=10:1, R_{f}=0.8) to obtain *tert*-butyl (6a*R*)-4-chloro-3-(2-fluorophenyl)-1-(4-((2-hydroxyethyl)(methyl)amino)-2,2-dimethylpyrrolidin-1-yl)-12-oxo-6a,7,9,10-tetrahydro-6*H*-pyrazino [2, 1-*c*]pyrido[3,4-*f*][1,4]oxazepine-8(12*H*)-carboxylate (150 mg, yield: 60.7%). ES-API: [M+H]⁺=618.2.

Step 5: *tert*-Butyl (6a*R*)-4-chloro-3-(2-fluorophenyl)-1-(4-((2-hydroxyethyl)(methyl)amino)-2,2-dimethylpyrrolidin-1-yl)-12-oxo-6a,7,9,10-tetrahydro-6*H*-pyrazino[2,1-*c*]pyrido[3,4-*f*][1,4]oxazepine-8(12*H*)-carboxylate (150 mg, 0.24 mmol) was dissolved in dichloromethane (2 mL), then cooled to 0°C in an ice-water bath, and then trifluoroacetic acid (1.5 mL) was added dropwise thereto, stirred for 1 hour. After the reaction was completed, the mixture was added with sodium bicarbonate solution to adjust to alkaline, extracted with dichloromethane, dried over sodium sulfate, evaporated to dryness by rotary evaporation to obtain (6a*R*)-4-chloro-3-(2-fluorophenyl)-1-(4-((2-hydroxyethyl)(methyl)amino)-2,2-dimethylpyrrolidin-1-yl)-7,8,9,10-tetrahydro-6*H*-pyrazino[2,1-*c*]pyrido[3,4-*f*][1,4]oxazepin-12-one (100 mg, yield: 80.6%). ES-API: [M+H]⁺= 518.2.

Step 6: (6a*R*)-4-Chloro-3-(2-fluorophenyl)-1-(4-((2-hydroxyethyl)(methyl)amino)-2,2-dimethylpyrrolidin-1-yl)-7,8,9,10-tetrahydro-6*H*-pyrazino[2,1-*c*]pyrido[3,4-*f*][1,4]oxazepin-12-one (80 mg, 0.15 mmol) was dissolved in tetrahydrofuran (0.8 mL), then cooled to 0°C in an ice-water bath. Aqueous sodium bicarbonate solution was slowly added thereto to adjust the pH value to 8-9. Potassium phosphate (16 mg, 0.075 mmol) and water (0.8 mL) were added thereto, stirred at 0°C for 5 minutes, and then a solution of acryloyl chloride in tetrahydrofuran (9 mg, 0.075 mmol) was added dropwise thereto and stirred for 10 minutes. After the reaction was completed, the reaction was quenched by adding sodium bicarbonate solution, extracted with ethyl acetate, dried over sodium sulfate, evaporated to dryness by rotary evaporation, and purified by silica gel column (dichloromethane/methanol=10/1, R_{f}=0.5) to obtain (6a*R*)-8-acryloyl-4-chloro-3-(2-fluorophenyl)-1-(4-((2-hydroxyethyl)(methyl)amino)-2,2-dimethylpyrrolidin-1-yl)-6,6a,7,8,9,10-hexahydro-12*H*-pyrazino[2,1-*c*]pyrido[3,-*f*][1,4]oxazepin-12-one (Z658, 25.4 mg, yield: 30.2%). ¹H-NMR (400 MHz, CDCl₃) δ 7.43-7.38 (m, 2H), 7.22 (t, *J* = 7.6 Hz, 1H), 7.17-7.12 (m, 1H), 6.59 (d, *J* = 10.2 Hz, 1H), 6.40 (dd, *J* = 16.4, 5.9 Hz, 1H), 5.80 (d, *J* = 11.0 Hz, 1H), 4.48-4.16 (m, 5H), 3.75-3.53 (m, 5H), 3.24-2.95 (m, 2H), 2.71-2.44 (m, 2H), 2.33-2.29 (m, 3H), 2.03-1.77 (m, 4H), 1.70-1.63 (m, 1H), 1.57 (d, *J* = 7.7Hz, 4H), 1.51 (s, 1H). ES-API: [M+H]⁺= 572.2.

The Z658 obtained in the above steps was subjected to chiral resolution under the following conditions to obtain two isomers.

| No. and structure | Name | Starting material, preparation condition, retention time, purity |
|---|---|---|
| 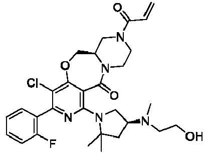 | (*R*)-8-Acryloyl-4-chloro-3-(2-fluorophenyl)-1-((*S*)-4-((2-hydroxyethyl)(methyl)a mino)-2,2-dimethylpyrrolidin-1 - yl)-6,6a,7,8,9,10-hexahydro-12*H-*pyrazino[2,1-*c*]pyrido[3,4-*f*][1,4]oxazepin-12-one | Chiral separation via Z658. |
| | | Separation column: IC 250 mm^{∗}4.6 mm^{∗}5 µm; |
| | | mobile phase: *n-*hexane:ethanol:diethanolamine=70:30:0.2; |
| | | flow rate: 1 mL/min; |
| | | column temperature: 30°C. |
| Peak 1 | | Peak 1, retention time: 9.289 min, purity: 99%, de |
| Z658-1 | | value: 100%. |
| | | ES-API: [M+H]⁺=572.3. |
| 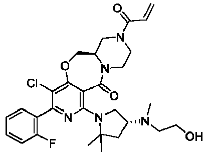 | ((*R*)-8-Acryloyl-4-chloro-3-(2-fluorophenyl)-1-((*R*)-4-((2-hydroxyethyl)(methyl)a mino)-2,2-dimethylpyrrolidin-1 - yl)-6,6a,7,8,9,10-hexahydro-12*H-*pyrazino[2,1-*c*]pyrido[3,4-*f*[1,4]oxazepin-12-one | Chiral separation via Z658. |
| | | Separation column: IC 250 mm^{∗}4.6 mm^{∗}5 µm; |
| | | mobile phase: *n-*hexane:ethanol:diethanolamine=70:30:0.2; |
| | | flow rate: 1 mL/min; |
| Peak 2, | | column temperature: 30°C. |
| Z658-2 | | Peak 2, retention time: 10.873 min, purity: 97%, de value: 100%. |
| | | ES-API: [M+H]⁺=572.2 |

### Embodiment 107 Synthesis of (6aR)-8-acryloyl-4-chloro-1-(4-((2,2-difluoroethyl)(methyl)amino)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12-one and two isomers thereof (Z659, Z659-1 and Z659-2)

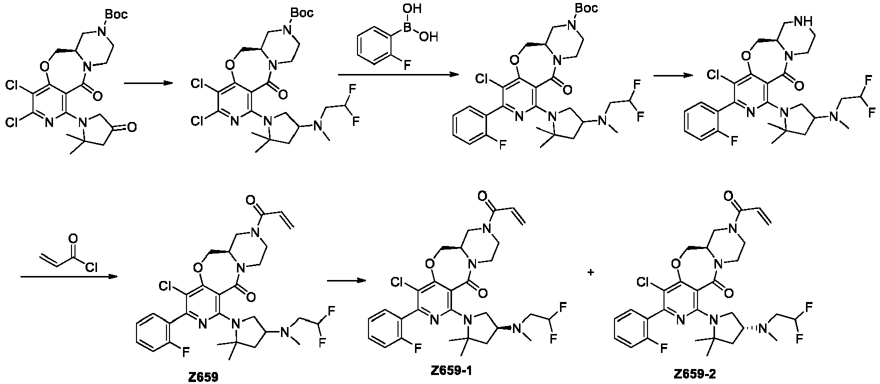

Step 1: *tert*-Butyl (*R*)-3,4-dichloro-1-(2,2-dimethyl-4-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (400 mg, 0.80 mmol), difluoroethylamine hydrochloride (282 mg, 2.40 mmol) and acetonitrile (10 mL) were added to a 100 mL flask, stirred at room temperature for 2 hours. Then sodium cyanoborohydride (650 mg, 8.01 mmol) was added thereto, and the mixture was continued to be stirred overnight. After the raw materials were completely consumed, 37% aqueous formaldehyde solution (650 mg, 8.01 mmol) was added thereto, and the reaction was continued for 1 hour. The reaction solution was quenched with saturated ammonium chloride solution (30 mL), extracted with ethyl acetate (30 mL^{∗}3), and the organic phases were combined, dried and concentrated, and the crude product was purified by flash silica gel column (ethyl acetate:petroleum ether: 0-80%) to obtain *tert*-butyl (6a*R*)-3,4-dichloro-1-(4-((2,2-difluoroethyl)(methyl)amino)-2,2-dimethylpyrrolidin-1-yl)-12-oxo-6a,7,9,10-tetrahydro-12*H-*pyrazino[2,1-*c*]pyrido[3,4-*f*][1,4]oxazepine-8(6*H*)-carboxylate (280 mg) as a white solid. ES-API: [M+H]⁺=578.2.

Step 2: *tert*-Butyl (6a*R*)-3,4-dichloro-1-(4-((2,2-difluoroethyl)(methyl)amino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (280 mg, 0.48 mmol), (2-fluorophenyl)boronic acid (203 mg, 1.45 mmol), tetrakis(triphenylphosphine)palladium (58 mg, 0.05 mmol), potassium carbonate (200 mg, 1.45 mmol), dioxane (2 mL) and water (0.4 mL) were added to a 5 mL microwave reaction tube. Under nitrogen protection, the reaction was carried out at 120°C for 1 hour under microwave irradiation. The reaction solution was poured into ethyl acetate (30 mL), washed with saturated brine (15 mL ^{∗} 3), and the organic phase was dried and concentrated, and the crude product was purified by flash silica gel column (ethyl acetate:petroleum ether: 0-80%) to obtain *tert*-butyl (6a*R*)-4-chloro-1-(4-((2,2-difluoroethyl)(methyl)amino)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (240 mg, yield of 78%). ES-API: [M+H]⁺=638.3.

Step 3: Under an ice-bath, a solution of *tert*-butyl (6a*R*)-4-chloro-1-(4-((2,2-difluoroethyl)(methyl)amino)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (240 mg, 0.33 mmol) in dichloromethane (4 mL) was added with trifluoroacetic acid (2 mL), stirred at room temperature for 1 hour. The reaction solution was concentrated to obtain a crude product of (6a*R*)-4-chloro-1-(4-((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (202 mg) as a yellow oil. ES-API: [M+H]⁺=538.3.

Step 4: The above crude procuct of (6a*R*)-4-chloro-1-(4-((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (190 mg, 0.35 mmol) was dissolved in dichloromethane (5 mL), and *N,N-*diisopropylethylamine (226 mg, 1.75 mmol) was added thereto. The reaction solution was cooled to 0°C, and acryloyl chloride (32 mg, 0.35 mmol) was added dropwise thereto. After stirring for 5 minutes, the mixture was concentrated, and the crude product was purified by preparative HPLC (column: Waters XBridge C18, 190*250 mm, 5 µm; mobile phase A: 0.1% aqueous ammonium bicarbonate solution; mobile phase B: acetonitrile; flow rate: 15 mL/min; B%=50%-70%; column temperature: room temperature) to obtain (6a*R*)-8-acryloyl-4-chloro-1-(4-(((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z659, 160 mg, yield: 76%). ES-API: [M+H]⁺=592.3.

Step 5: (6a*R*)-8-Acryloyl-4-chloro-1-(4-(((2,2-difluoroethyl)(methyl)amino)-2,2-dimethylpyrrolidin-1-yl)-3-(2-fluorophenyl)-6,6a,7, 8,9,10-hexahydro-12*H*-pyrazino [2,1-*c*]pyrido [3,4-*f*][1,4]oxazepin-12-one was subjected to chiral resolution (column type: Chiralpak IC: 10 µm, 20*250 mm, mobile phase: *n*-hexane:ethanol:diethylamine=70:30:0.2, flow rate: 15 mL/min, column temperature: room temperature) to obtain two isomers:

One isomer with a structure arbitrarily designated as (*R*)-8-acryloyl-4-chloro-1-((*S*)-4-((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z659-1, 27 mg, yield: 17%, retention time: 13.95 min, purity: 99%, ee value: 100%). ES-API: [M+H]⁺ = 592.3. ¹H NMR (500 MHz, DMSO-*d*₆) δ 7.54 - 7.48 (m, 1H), 7.48 - 7.41 (m, 1H), 7.37 - 7.28 (m, 2H), 6.85 - 6.72 (m, 1H), 6.23 - 6.11 (m, 1H), 6.11 - 5.89 (m, 1H), 5.79 - 5.66 (m, 1H), 4.68 - 3.32 (m, 10H), 3.08 - 2.90 (m, 2H), 2.89 - 2.76 (m, 1H), 2.75 - 2.59 (m, 1H), 2.32 - 2.21 (m, 3H), 2.06 - 1.88 (m, 2H), 1.70 - 1.49 (m, 3H), 1.48 - 1.36 (m, 3H).

Another isomer with a structure arbitrarily designated as (*R*)-8-acryloyl-4-chloro-1-((*R*)-4-((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z659-2, 122 mg, yield: 77%, retention time: 18.66 min, purity: 99%, ee value: 96%). ES-API: [M+H]⁺ = 592.3. ¹H NMR (500 MHz, DMSO-*d*₆) δ 7.54 - 7.49 (m, 1H), 7.47 - 7.43 (m, 1H), 7.35 - 7.29 (m, 2H), 6.81 - 6.68 (m, 1H), 6.22 - 5.94 (m, 2H), 5.78 - 5.68 (m, 1H), 4.60 - 4.50 (m, 1H), 4.43 - 4.27 (m, 1H), 4.25 - 3.93 (m, 4H), 3.91 - 3.73 (m, 1H), 3.67 - 3.39 (m, 2H), 3.37 - 3.24 (m, 1H), 3.23 - 3.12 (m, 1H), 3.11 - 3.01 (m, 1H), 2.89 - 2.75 (m, 1H), 2.68 (dd, *J* = 30.0, 15.5 Hz, 1H), 2.27 (s, 3H), 2.02 (dd, *J* = 11.5, 5.5 Hz, 1H), 1.67 (t, *J* = 11.5 Hz, 1H), 1.51 (s, 3H), 1.46 (s, 3H).

### Embodiment 108 Synthesis of two isomers of (6aR)-8-acryloyl-4-chloro-1-(4-((2-fluoroethyl)(methyl)amino)-2,2-dimethylpyrrolidin-1-yl)-3-(2-fluorophenyl)-6,6a,7,8,9,10-hexahydro-12H-pyrazino[2,1-c]pyrido[3,4-f][1,4]oxazepin-12-one (Z660-1 and Z660-2)

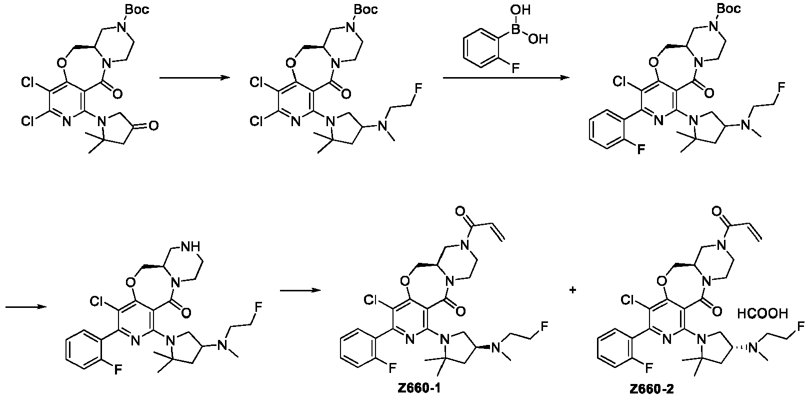

Step 1: *tert*-Butyl (R)-3,4-dichloro-1-(2,2-dimethyl-4-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (400 mg, 0.80 mmol), 2-fluoroethylamine hydrochloride (239 mg, 2.40 mmol) and acetonitrile (10 mL) were added to a 100 mL flask, stirred at room temperature for 2 hours. Then sodium cyanoborohydride (650 mg, 8.01 mmol) was added thereto, and the mixture was continued to be stirred overnight. After the raw materials were completely consumed, 37% aqueous formaldehyde solution (650 mg, 8.01 mmol) was added thereto, and the reaction was continued for 1 hour. The reaction solution was quenched with saturated ammonium chloride solution (30 mL), extracted with ethyl acetate (30 mL^{∗}3), and the organic phases were combined, dried and concentrated, and the crude product was purified by flash silica gel column (ethyl acetate:petroleum ether: 0-80%) to obtain *tert*-butyl (6a*R*)-3,4-dichloro-1-(4-((2-fluoroethyl)(methyl)amino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg) as a white solid. ES-API: [M+H]⁺=560.2.

Step 2: *tert*-Butyl (6a*R*)-3,4-dichloro-1-(4-(((2-fluoroethyl)(methyl)amino)-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (200 mg, 0.36 mmol), (2-fluorophenyl)boronic acid (150 mg, 1.07 mmol), tetrakis(triphenylphosphine)palladium (46 mg, 0.04 mmol), potassium carbonate (150 mg, 1.07 mmol), dioxane (2 mL) and water (0.4 mL) were added to a 5 mL microwave reaction tube. Under nitrogen protection, the reaction was carried out at 120°C for 1 hour under microwave irradiation. The reaction solution was poured into ethyl acetate (30 mL), washed with saturated brine (15 mL ^{∗} 3), and the organic phase was dried and concentrated, and the crude product was purified by flash silica gel column (ethyl acetate: petroleum ether: 0-80%) to obtain *tert*-butyl (6a*R*)-4-chloro-1-(4-(((2-fluoroethyl)(methyl)amino)-2,2-dimethylpyrrolidin-1-yl)-3-(2-fluorophenyl)-12-oxo-6a,7,9,10-tetrahydro-12*H*-pyrazino [2,1-*c*]pyrido[3,4-*f*][1,4]oxazepine-8(6*H*)-carboxylate (180 mg, yield of 78%). ES-API: [M+H]⁺=620.3.

Step 3: Under an ice-bath, a solution of *tert*-butyl (6a*R*)-4-chloro-1-(4-(((2-fluoroethyl)(methyl)amino)-2,2-dimethylpyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (180 mg, 0.33 mmol) in dichloromethane (4 mL) was added with trifluoroacetic acid (2 mL), stirred at room temperature for 1 hour. The reaction solution was concentrated to obtain a crude product of (6a*R*)-4-chloro-1-(4-((2-fluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (151 mg) as a yellow oil. ES-API: [M+H]⁺=520.3.

Step 4: The above crude procuct of (6a*R*)-4-chloro-1-(4-((2-fluoroethyl)(methyl)amino)-2,2-dimethylpyrrolidin-1-yl)-3-(2-fluorophenyl)-6,6a,7,8,9,10-hexahydro-12*H*-pyrazino[2,1-c]pyrido[3,4-*f*][1,4]oxazepin-12-one (145 mg, 0.28 mmol) was dissolved in dichloromethane (5 mL), and *N,N-*diisopropylethylamine (181 mg, 1.40 mmol) was added thereto. The reaction solution was cooled to 0°C, and acryloyl chloride (25 mg, 0.28 mmol) was added dropwise thereto. After stirring for 5 minutes, the mixture was concentrated, and the crude product was purified by preparative HPLC (column: YX C18, 21.2*250 mm, 5 µm; mobile phase A: 0.1% aqueous ammonium bicarbonate solution; mobile phase B: acetonitrile; flow rate: 15 mL/min; B%= 50%-70%; column temperature: room temperature) to obtain two isomer compounds:

One isomer with a structure arbitrarily designated as (*R*)-8-acryloyl-4-chloro-1-((*S*)-4-((2-fluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z660-1, 74 mg, retention time: 8.14 min; yield: 46%, purity 100%). ES-API: [M+H]⁺ = 574.3. ¹H NMR (500 MHz, DMSO-*d*₆) δ 7.54 - 7.49 (m, 1H), 7.45 (t, *J* = 7.5 Hz, 1H), 7.37-7.31 (m, 2H), 6.80 - 6.69 (m, 1H), 6.22 - 6.09 (m, 1H), 5.80 - 5.67 (m, 1H), 4.55 (s, 2H), 4.49 - 4.41 (m, 1H), 4.41 - 4.28 (m, 1H), 4.25 - 3.86 (m, 4H), 3.82 (s, 1H), 3.67 - 3.42 (m, 2H), 3.40 - 3.27 (m, 1H), 3.15 - 2.98 (m, 2H), 2.76 - 2.52 (m, 2H), 2.20 (s, 3H), 2.06 - 1.96 (m, 1H), 1.66 (t, *J* = 11.0 Hz, 1H), 1.52 (s, 3H), 1.46 (s, 3H).

Another isomer (retention time: 9.80 min) was obtained by further preparative HPLC (column: YX C18, 21.2^{∗}250 mm, 5 µm; mobile phase A: 0.1% aqueous formic acid solution; mobile phase B: acetonitrile; flow rate: 15 mL/min; B%=25%-50%; column temperature: room temperature) and the structure was arbitrarily designated as (*R*)-8-acryloyl-4-chloro-1-((*R*)-4-((2-fluoroethyl)(methyl)amino)-2,2-dimethylpyrrolidin-1-yl)-3-(2-fluorophenyl)-6,6a,7, 8,9,10-hexahydro-12*H*-pyrazino[2,1-*c*]pyrido[3,4-*f*][1,4]oxazepin-12-one formate (Z660-2, 3 mg, yield: 2%, purity 99%). ES-API: [M+H]⁺=592.3.

### Embodiment 109 Synthesis of (6aR)-8-acryloyl-4-chloro-1-(2,2-dimethyl-4-(methylamino)pyrrolidin-1-yl)-3-(2-fluorophenyl)-6,6a,7,8,9,10-hexahydro-12H-pyrazino[2,1-c]pyrido[3,4-f][1,4]oxazepin-12-one and isomers thereof (Z661, Z661-1 and Z661-2)

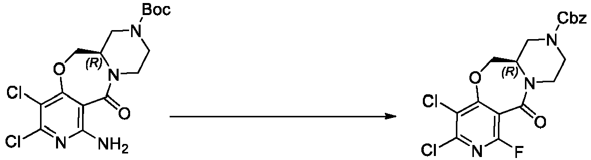

Step 1: *tert*-Butyl (*R*)-3,4-dichloro-1-amino-12-oxo-6a,7,9,10-tetrahydro-12*H*-pyrazino[2,1-c]pyrido[3,4-*f*][1,4]oxazepine-8(6*H*)-carboxylate (2.2 g, 5.47 mmol) was dissolved in pyridine hydrofluoride solution (4.4 mL), and sodium nitrite (0.566 g, 8.2 mmol) was added thereto in batches under ice-water cooling, reacted for 10 minutes to obtain solution 1. Meanwhile, benzoxycarbonyl succinimide (6.8 g, 27.35 mmol) was added to a solution of sodium hydroxide (8.8 g) in tetrahydrofuran (20 mL) and water (15 mL) at 0°C to obtain solution 2. Under stirring at 0°C, solution 1 was slowly added to solution 2, reacted in an ice-water bath for 0.5 hours. Water and ethyl acetate were added to the reaction solution,and the organic phase was washed with brine, dried, concentrated, and purified by column chromatography (petroleum ether/ethyl acetate= 3/1, R_{f}=0.5) to obtain a product of benzyl (*R*)-3,4-dichloro-1-fluoro-12-oxo-6a,7,9,10-tetrahydro-12*H*-pyrazino[2,1-*c*]pyrido[3,4-*f*][1,4]oxazepine-8(6*H*)-carboxylate (0.76 g, 31.7%). ¹H NMR (400 MHz, CDCl₃) δ 7.43-7.30 (m, 5H), 5.17(s, 2H), 4.55 (t, *J* = 11.3 Hz, 1H), 4.42-4.40(m, 1H), 4.14-4.05 (m, 2H), 3.90 - 3.79 (m, 3H), 3.72-3.70 (m, 1H),3.57-3.51 (m, 1H). ES-API: [M+H]⁺=440.1

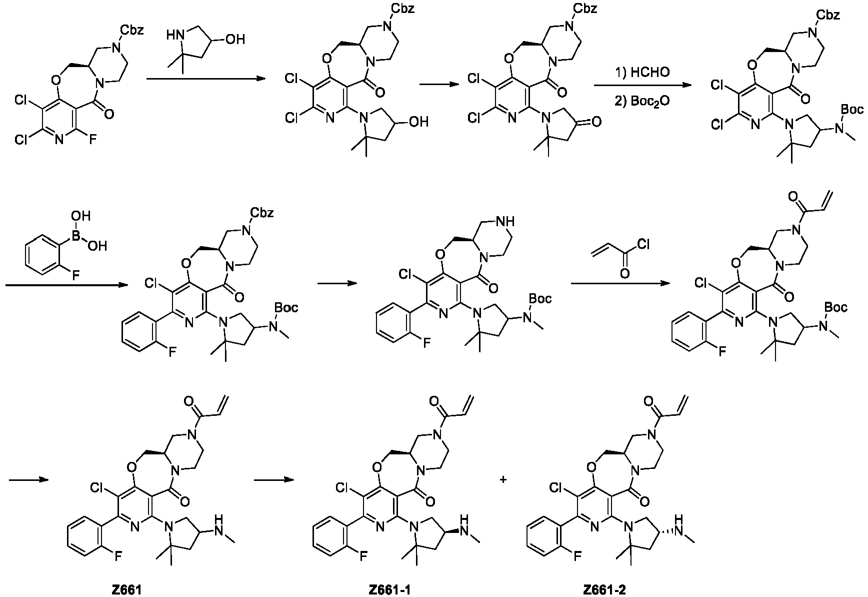

Step 2: 5,5-Dimethylpyrrolidin-3-ol (144 mg, 1.25 mmol) and potassium carbonate (470 mg, 3.41 mmol) were added to a solution of benzyl (*R*)-3,4-dichloro-1-fluoro-12-oxo-6a,7,9,10-tetrahydro-12*H-*pyrazino[2,1-*c*]pyrido[3,4-*f*][1,4]oxazepine-8(6*H*)-carboxylate (500 mg, 1.14 mmol) in acetonitrile (5 mL), reacted at 100°C for 2 hours. The reaction solution was filtered, concentrated, then dissolved in ethyl acetate (30 mL), washed with water (15 mL ^{∗} 3), and the organic phase was dried over anhydrous sodium sulfate, concentrated and purified by flash silica gel column (methanol:dichloromethane: 0-5%) to obtain benzyl (6a*R*)-3,4-dichloro-1-(4-hydroxy-2,2-dimethylpyrrolidin-1-yl)-12-oxo-6a,7,9,10-tetrahydro-12H-pyrazino[2,1-*c*]pyrido[3,4-*f*][1,4]oxazepine-8(6*H*)-carboxylate (380 mg) with ayield of 62% as a white solid. ES-API: [M+H]⁺=535.3.

Step 3: Under an ice-bath, Dess-Martin reagent (330 mg, 0.71 mmol) was slowly added to a solution of benzyl (6a*R*)-3,4-dichloro-1-(4-hydroxy-2,2-dimethylpyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (380 mg, 0.71 mmol) in dichloromethane (5 mL) and stirred at room temperature overnight. The reaction solution was quenched with aqueous sodium thiosulfate solution (20 mL), then saturated sodium bicarbonate solution (20 mL) was added thereto, and extracted with dichloromethane (30 mL^{∗}3). The organic phases were combined, dried, concentrated and purified by flash silica gel column (ethyl acetate:petroleum ether: 0-70%) to obtain benzyl (*R*)-3,4-dichloro-1-(2,2-dimethyl-4-oxopyrrolidin-1-yl)-12-oxo-6a,7,9,10-tetrahydro-12H-pyrazino [2,1-*c*]pyrido[3,4-*f*][1,4]oxazepine-8(6*H*)-carboxylate (330 mg) with a yield of 87%. ES-API: [M+H]⁺=533.2.

Step 4: Benzyl (*R*)-3,4-dichloro-1-(2,2-dimethyl-4-oxopyrrolidin-1-yl)-12-oxo-6a,7,9,10-tetrahydro-12*H*-pyrazino[2,1-*c*]pyrido[3,4-*f*][1,4]oxazepine-8(6*H*)-carboxylate (330 mg, 0.62 mmol) and a solution of methylammonium hydrochloride (125 mg, 1.86 mmol) in acetonitrile (10 mL) were stirred at room temperature for 2 hours. Then sodium cyanoborohydride (117 mg, 1.86 mmol) was added thereto, and the mixture was continued to be stirred overnight. After the raw materials were completely consumed, dicarbonic acid diester (334 mg, 1.53 mmol) was added thereto, and the reaction was continued for 1 hour. The reaction solution was quenched with saturated ammonium chloride solution (30 mL), extracted with ethyl acetate (30 mL^{∗}3), and the organic phases were combined, dried and concentrated, and the crude product was purified by flash silica gel column (ethyl acetate:petroleum ether: 0-80%) to obtain benzyl (6a*R*)-1-(4-((*tert*-butoxycarbonyl)(methyl)amino)-2,2-dimethylpyrrolidin-1-yl)-3,4-dichloro-12-oxo-6a,7,9,10-tetrahydro-12*H*-pyrazino[2,1-*c*]pyrido[3,4-*f*][1,4]oxazepine-8(6*H*)-carboxylate (200 mg) as a white solid. ES-API: [M+H]⁺= 648.3.

Step 5: Benzyl (6a*R*)-1-(4-((*tert*-butoxycarbonyl)(methyl)amino)-2,2-dimethylpyrrolidin-1-yl)-3.4-dichloro-12-oxo-6a,7,9,10-tetrahydro-12*H*-pyrazino[2,1-*c*]pyrido[3,4-*f*][1,4]oxazepine-8(6*H*)-carboxylate (200 mg, 0.31 mmol), (2-fluorophenyl)boronic acid (129 mg, 0.93 mmol), tetrakis(triphenylphosphine)palladium (35 mg, 0.03 mmol), potassium carbonate (128 mg, 0.93 mmol), dioxane (2 mL) and water (0.4 mL) were added to a 5 mL microwave reaction tube. Under nitrogen protection, the reaction was carried out at 120°C for 1 hour under microwave irradiation. The reaction solution was poured into ethyl acetate (30 mL), washed with saturated brine (15 mL ^{∗} 3), and the organic phase was dried and concentrated, and the crude product was purified by flash silica gel column (ethyl acetate: petroleum ether: 0-80%) to obtain benzyl (6a*R*)-1-(4-((*tert*-butoxycarbonyl)(methyl)amino)-2,2-dimethylpyrrolidin-1-yl)-4-chloro-3-(2-fluorophenyl)-12-oxo-6a,7,9,10-tetrahydro-12*H*-pyrazino [2,1-*c*]pyrido[3,4-*f*][1,4|oxazepine-8(6*H*)-carboxylate (160 mg, yield of 73%). ES-API: [M+H]⁺=708.3.

Step 6: A solution of benzyl (6a*R*)-1-(4-((*tert*-butoxycarbonyl)(methyl)amino)-2,2-dimethylpyrrolidin-1-yl)-4-chloro-3-(2-fluorophenyl)-12-oxo-6a,7,9,10-tetrahydro-12*H*-pyrazino [2,1-*c*]pyrido[3,4-*f*][1,4]oxazepine-8(6*H*)-carboxylate (160 mg, 0.23 mmol) in dichloromethane (5 mL) was added with palladium chloride (40 mg, 0.23 mmol), triethylamine (46 mg, 0.45 mmol) and triethylsilane (131 mg, 1.13 mmol) in turn, and stirred at room temperature for 0.5 hours. The reaction solution was quenched with methanol, filtered and concentrated to obtain a white solid of *tert*-butyl (1-((*R*)-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)(methyl)carbamate (130 mg). ES-API: [M+H]⁺=574.3.

Step 7: *tert*-Butyl (1-((*R*)-4-chloro-3-(2-fluorophenyl)-12-oxo-6,6a,7,8,9,10-hexahydro-12H-pyrazino[2,1-*c*]pyrido[3,4-*f*][1,4]oxazepin-1-yl)-5,5 -dimethylpyrrolidin-3 -yl)(methyl)carbamate (120 mg, 0.21 mmol) was dissolved in dichloromethane (5 mL), and *N*,*N* diisopropylethylamine(136 mg, 1.05 mmol) was added thereto. The reaction solution was cooled to 0°C, and acryloyl chloride (38 mg, 0.42 mmol) was added dropwise thereto. After stirring for 5 minutes, the mixture was concentrated and the crude product was purified by flash silica gel column (ethyl acetate/petroleum ether: 0-80%) to obtain *tert*-butyl (1-((R)-8-acryloyl-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)(methyl)carbamate (50 mg, yield: 38%). ES-API: [M+H]⁺=628.3.

Step 8: Under an ice bath, a solution of *tert*-butyl (1-((*R*)-8-acryloyl-4-chloro-3-(2-fluorophenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidin-3-yl)(methyl)carbamate (50 mg, 0.08 mmol) in dichloromethane (1 mL) was added with trifluoroacetic acid (0.5 mL), and the reaction solution was stirred at room temperature for 1 hour. The mixture was concentrated under reduced pressure, and the crude product was purified by preparative HPLC (column: Waters XBridge C18, 190^{∗}250 mm, 5 µm; mobile phase A: 0.1% aqueous ammonium bicarbonate solution; mobile phase B: acetonitrile; flow rate: 15 mL/min; B%=35%-55%; column temperature: room temperature) to obtain (6a*R*)-8-acryloyl-4-chloro-1-(2,2-dimethyl-4-(methylamino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z661, 28 mg) with a yield of 67% as a white solid. ES-API: [M+H]⁺=528.3.

Step 9: (6a*R*)-8-Acryloyl-4-chloro-1-(2,2-dimethyl-4-(methylamino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (26 mg, 0.05 mmol) was subjected to chiral resolution (column type: Chiralpak AB: 10 µm, 20^{∗}250 mm, mobile phase: *n*-hexane: ethanol: diethylamine=5 0:5 0:0.2, flow rate: 15 mL/min, column temperature: room temperature) to obtain two isomers:

One isomer with a structure arbitrarily designated as (*R*)-8-acryloyl-4-chloro-1-((*S*-2,2-dimethyl-4-(methylamino)pyrrolidin-1-yl)-3-(2-fhiorophenyl)-6,6a,7,8,9,10-hexahydro-12*H*-pyrazino[2,1-*c*]pyrido[3,4-*f*][1,4]oxazepin-12-one (Z661-1, 15 mg, yield: 58%, retention time: 8.62 min, purity: 99%, ee value: 100%). ES-API: [M+H]⁺ = 528.3. ¹H NMR (500 MHz, DMSO-*d*₆) δ 7.56 - 7.48 (m, 1H), 7.48 - 7.41 (m, 1H), 7.37 - 7.27 (m, 2H), 6.76 (dd, *J* = 17.0, 10.5 Hz, 1H), 6.17 (d, *J* = 17.0 Hz, 1H), 5.78 - 5.67 (m, 1H), 4.60 - 4.47 (m, 1H), 4.41 - 4.25 (m, 1H), 4.23 - 3.83 (m, 4H), 3.82 - 3.33 (m, 4H), 3.26 - 3.15 (m, 2H), 3.11- 3.04 (m, 1H), 2.25 (s, 3H), 2.11 - 2.01 (m, 1H), 1.58 - 1.52 (m, 1H), 1.51 (s, 3H), 1.46 (s, 3H).

Another isomer with a structure arbitrarily designated as (*R*)-8-acryloyl-4-chloro-1-((R)-2,2-dimethyl-4-(methylamino)pyrrolidin-1-yl)-3-(2-fhiorophenyl)-6,6a,7,8,9,10-hexahydro-12*H*-pyrazino[2,1-*c*]pyrido[3,4-*f*][1,4]oxazepin-12-one (Z661-2, 2 mg, yield: 8%, retention time: 11.51 min, purity: 100%, ee value: 100%). ES-API: [M+H]⁺ = 528.3. ¹H NMR (500 MHz, DMSO-*d*₆) δ 7.55 - 7.48 (m, 1H), 7.48 - 7.41 (m, 1H), 7.36 - 7.27 (m, 2H), 6.83 - 6.70 (m, 1H), 6.17 (d, *J* = 16.0 Hz, 1H), 5.78 - 5.69 (m, 1H), 4.55 - 3.38 (m, 10H), 3.31 - 3.28 (m, 1H), 3.12 (s, 1H), 2.78 (d, *J* = 10.7 Hz, 1H), 2.22 (s, 3H), 1.00 - 1.86 (m, 2H), 1.70 (s, 3H), 1.40 (s, 3H).

### Embodiment 110 Synthesis of two isomers of (6aR)-8-acryloyl-4-chloro-1-(2,2-dimethyl-4-((2,2,2-trifluoroethyl)amino)pyrrolidin-1-yl)-3-(2-fluorophenyl)-6,6a,7,8,9,10-hexahydro-12H-pyrazino[2,1-c]pyrido[3,4-f][1,4]oxazepin-12-one (Z662-1 and Z662-2)

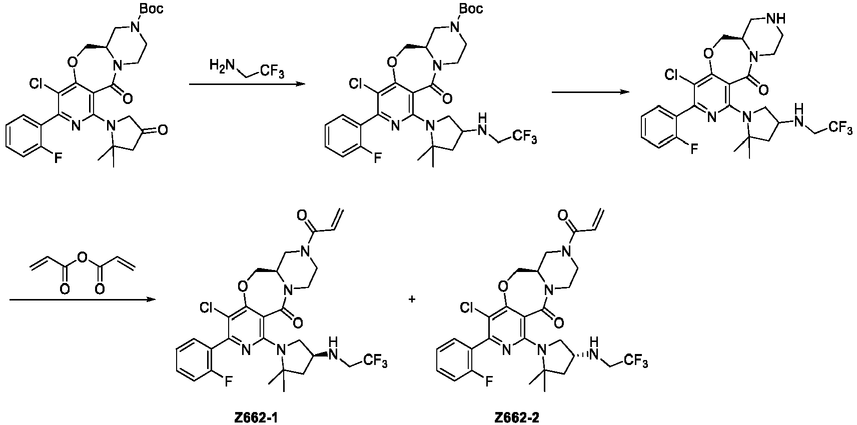

Step 1: A solution of *tert*-butyl (*R*)-4-chloro-1-(2,2-dimethyl-4-oxopyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (500 mg, 0.90 mmol) and trifluoroethylamine (444 mg, 4.48 mmol) in acetonitrile (10 mL) was stirred at room temperature for 2 hours. Then sodium cyanoborohydride (169 mg, 2.69 mmol) was added thereto, and the mixture was continued to be stirred overnight. The reaction solution was quenched with saturated ammonium chloride solution (30 mL), extracted with ethyl acetate (30 mL^{∗}3), and the organic phases were combined, dried and concentrated, and the crude product was purified by flash silica gel column (ethyl acetate:petroleum ether: 0-80%) to obtain *tert*-butyl (6a*R*)-4-chloro-1-(2,2-dimethyl-4-((2,2,2-trifluoroethyl)amino)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (400 mg) as a white solid. ES-API: [M+H]⁺= 642.3.

Step 2: A solution of *tert*-butyl (6a*R*)-4-chloro-1-(2,2-dimethyl-4-((2,2,2-trifluoroethyl)amino)pyrrolidin-1-yl)-3-(2-fluorophenyl)-12-oxo-6a,7,9,10-tetrahydro-12*H*-pyrazino[2,1-*c*]pyrido[3,4-*f*][1,4]oxazepine-8(6*H*)-carboxylate (300 mg, 0.47 mmol) in dichloromethane (4 mL) was added with trifluoroacetic acid (2 mL), and the reaction solution was stirred at room temperature for 1 hour. The reaction solution was concentrated under reduced pressure to obtain a crude product of (6a*R*)-4-chloro-1-(2,2-dimethyl-4-((2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (250 mg). ES-API: [M+H]⁺=574.2.

Step 3: (6a*R*)-4-Chloro-1-(2,2-dimethyl-4-((2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (250 mg, 0.46 mmol) was dissolved in dichloromethane (5 mL), and *N*,*N-*diisopropylethylamine(298 mg, 2.31 mmol) was added thereto. The reaction solution was cooled to -60°C, and acrylic anhydride (58 mg, 0.46 mmol) was added dropwise thereto. After stirring for 5 minutes, the mixture was concentrated, and the crude product was purified by flash silica gel column (ethyl acetate/petroleum ether: 0-80%) and subjected to preparative HPLC (column: YX C18, 21.2^{∗}250 mm, 5 µm; mobile phase A: 0.1% aqueous ammonium bicarbonate solution; mobile phase B: acetonitrile; flow rate: 15 mL/min; B%=50%-70%; column temperature: room temperature) to obtain two isomers.

One isomer with a structure arbitrarily designated as (*R*)-8-acryloyl-4-chloro-1-((*S*-2,2-dimethyl-4-((2,2,2-trifluoroethyl)amino)pyrrolidin-1-yl)-3-(2-fluorophenyl)-6,6a,7,8,9,10-hexahydro-12*H-*pyrazino[2,1-*c*]pyrido[3,4-*f*[1,4]oxazepin-12-one (Z662-1, 30 mg, yield: 11%, retention time: 1.88 min, purity 99%). ES-API: [M+H]⁺ = 596.2. ¹H NMR (500 MHz, DMSO-*d*₆) δ 7.57 - 7.48 (m, 1*H*), 7.48 - 7.41 (m, 1*H*), 7.37 - 7.27 (m, 2*H*), 6.76 (dd, *J* = 16.5, 10.5 Hz, 1*H*), 6.17 (d, *J* = 17.0 Hz, 1H), 5.80 - 5.69 (m, 1H), 4.57 - 4.49 (m, 1H), 4.41 - 4.25 (m, 1H), 4.25 - 3.85 (m, 4H), 3.82 - 3.54 (m, 1H), 3.54 - 3.40 (m, 2H), 3.40 - 3.36 (m, 1H), 3.28 - 3.15 (m, 3H), 3.14 - 3.05 (m, 1H), 2.79 - 2.67 (m, 1H), 2.05 (dd, *J* = 11.5, 5.5 Hz, 1H), 1.62 (t, *J* = 11.5 Hz, 1H), 1.50 (s, 3H), 1.45 (s, 3H).

Another isomer with a structure arbitrarily designated as (*R*)-8-acryloyl-4-chloro-1-((*R*)-2,2-dimethyl-4-((2,2,2-trifluoroethyl)amino)pyrrolidin-1-yl)-3-(2-fluorophenyl)-6,6a,7,8,9,10-hexahydro-12*H-*pyrazino[2,1-*c*]pyrido[3,4-*f*][1,4]oxazepin-12-one (Z662-2, 10 mg, yield: 4%, retention time: 1.98 min, purity: 100%). ES-API: [M+H]⁺ = 596.2. ¹HNMR (500 MHz, DMSO-*d*₆) δ 7.55 - 7.48 (m, 1H), 7.45 (t, *J* = 7.0 Hz, 1H), 7.37 - 7.27 (m, 2H), 6.85 - 6.72 (m, 1H), 6.17 (d, *J* = 16.5 Hz, 1H), 5.80 - 5.69 (m, 1H), 4.47 (s, 1H), 4.39 - 3.85 (m, 6H), 3.82 - 3.40 (m, 4H), 3.29 - 3.15 (m, 3H), 2.83 (d, *J* = 10.5 Hz, 1H), 2.05 - 1.84 (m, 2H), 1.71 (s, 3H), 1.40 (s, 3H).

The compounds in the following embodiments could be synthesized according to the methods described in the previous embodiments by using appropriate starting materials according to their specific structures, or could be synthesized by methods similar to those described in the previous embodiments. The solvent, temperature and other reaction conditions in each step could be the same as or similar to those described in the previous embodiments, or the reaction conditions known in the art could be used. The following mass spectrum was ES-API: [M+H]⁺.

| | Mass spectrum | | Mass spectrum | | Mass spectrum | | Mass spectrum |
|---|---|---|---|---|---|---|---|
| 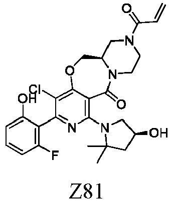 | 531.2 | 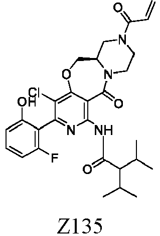 | 559.2 | 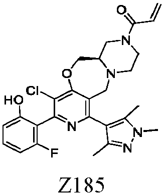 | 512. 2 | 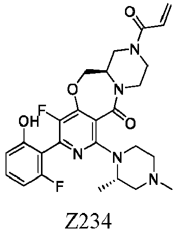 | 514. 2 |
| 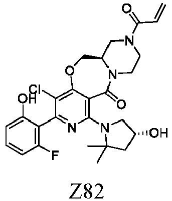 | 531.2 | 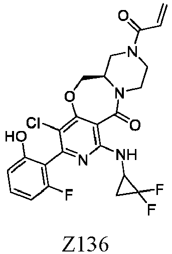 | 509.1 | 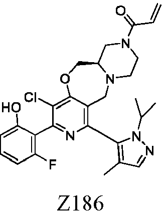 | 526. 2 | 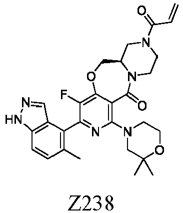 | 535. 2 |
| 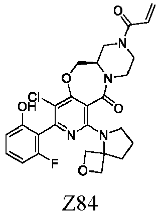 | 529.2 | 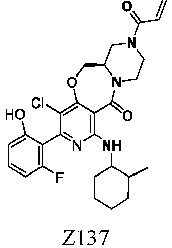 | 529.2 | 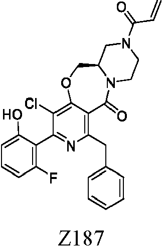 | 508. 1 | 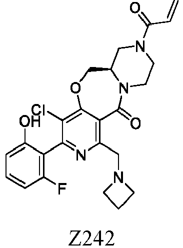 | 487. 2 |
| 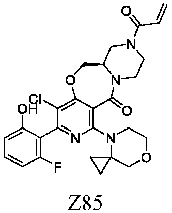 | 529.2 | 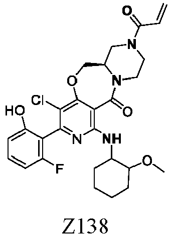 | 545.2 | 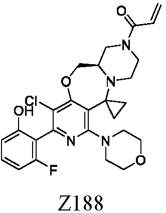 | 515. 2 | 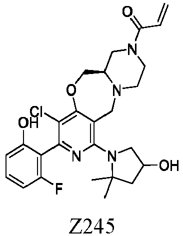 | 517. 2 |
| 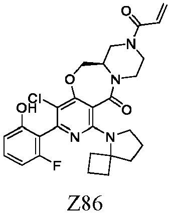 | 527.2 | 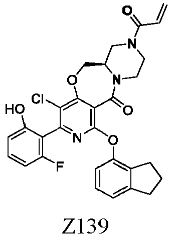 | 550.2 | 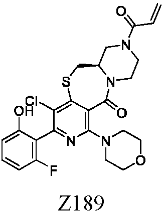 | 519. 1 | 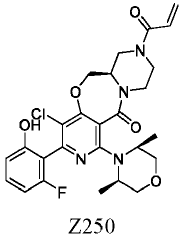 | 531. 2 |
| 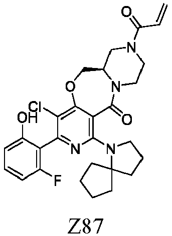 | 541.2 | 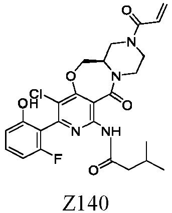 | 517.2 | 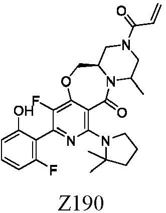 | 513. 2 | 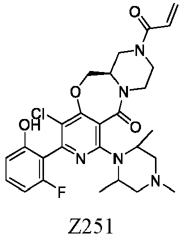 | 544. 2 |
| 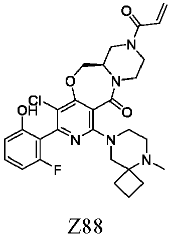 | 556.2 | 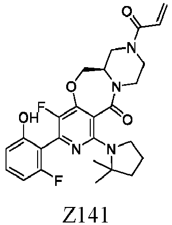 | 499.2 | 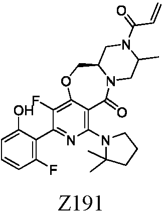 | 512. 2 | 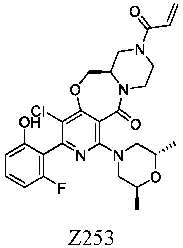 | 531. 2 |
| 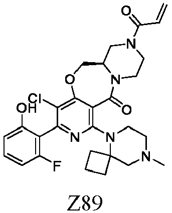 | 556.2 | 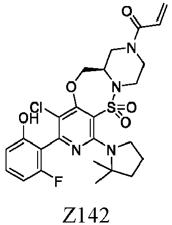 | 551.2 | 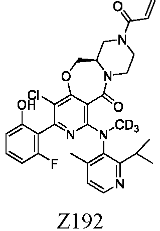 | 583. 2 | 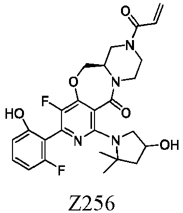 | 515. 2 |
| 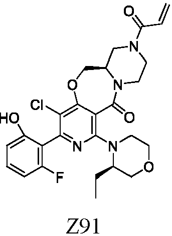 | 531.2 | 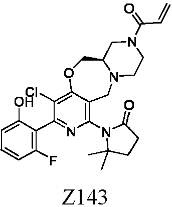 | 515.2 | 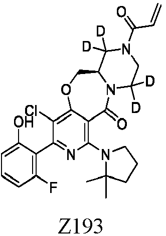 | 519. 2 | 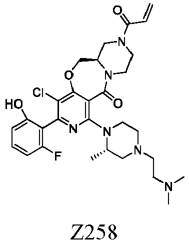 | 587. 3 |
| 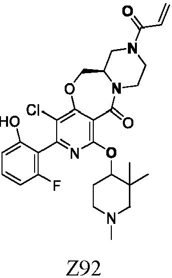 | 559.2 | 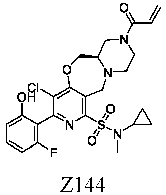 | 537.1 | 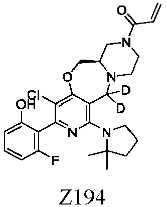 | 503. 2 | 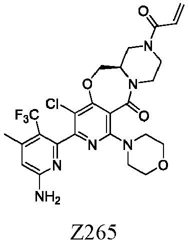 | 567. 2 |
| 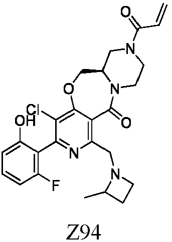 | 501.2 | 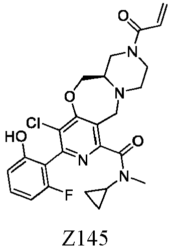 | 501.2 | 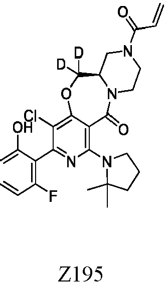 | 517. 2 | 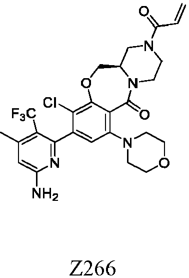 | 566. 2 |
| 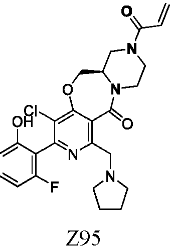 | 501.2 | 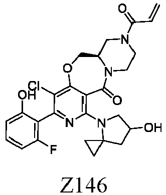 | 529.2 | 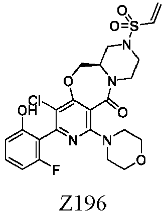 | 539. 1 | 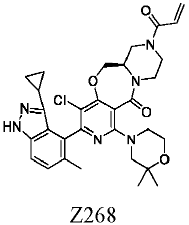 | 591. 2 |
| 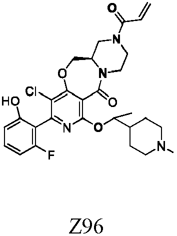 | 559.2 | 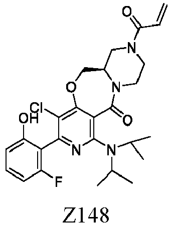 | 517.2 | 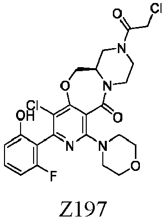 | 525. 1 | 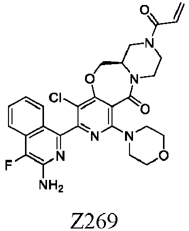 | 553. 2 |
| 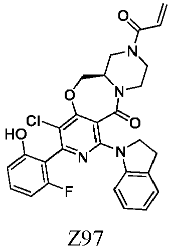 | 535.2 | 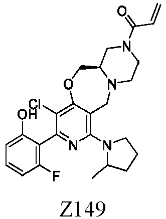 | 487.2 | 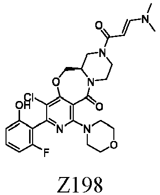 | 546. 2 | 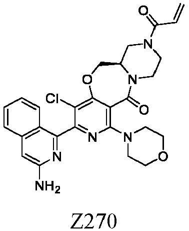 | 535. 2 |
| 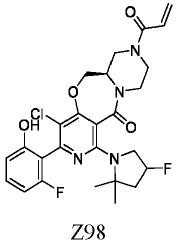 | 533.2 | 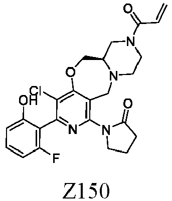 | 487.2 | 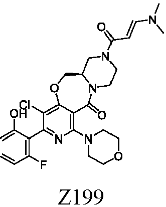 | 546. 2 | 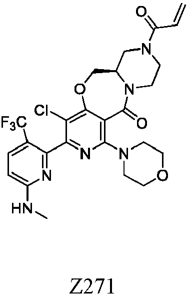 | 567. 2 |
| 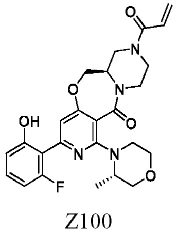 | 483.2 | 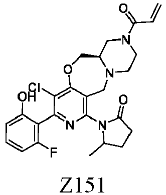 | 501.2 | 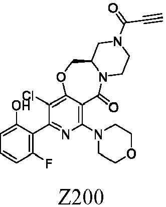 | 501. 1 | 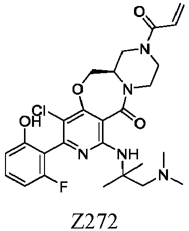 | 532. 2 |
| 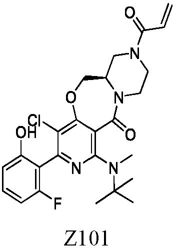 | 503.2 | 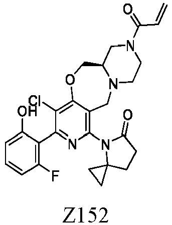 | 513.2 | 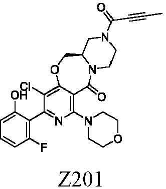 | 515. 1 | 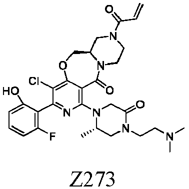 | 601. 2 |
| 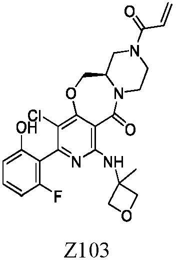 | 503.1 | 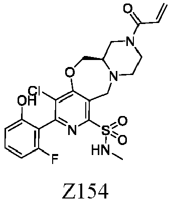 | 497.1 | 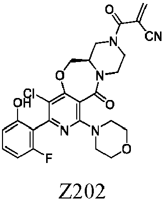 | 528. 1 | 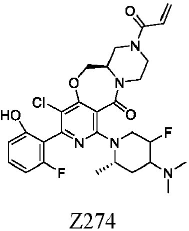 | 576. 2 |
| 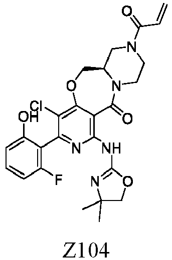 | 530.2 | 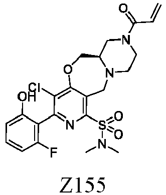 | 511.1 | 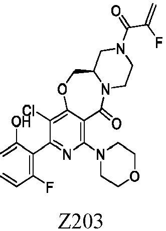 | 521. 1 | 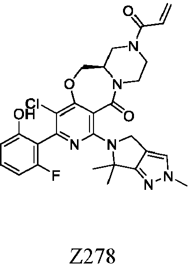 | 567. 2 |
| 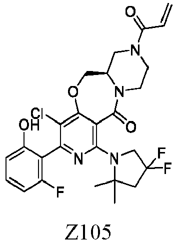 | 551.2 | 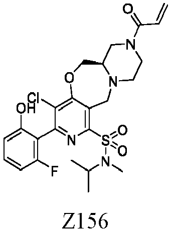 | 539.2 | 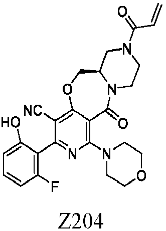 | 494. 2 | 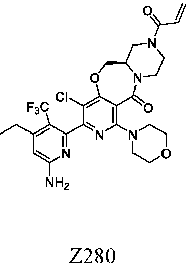 | 581. 2 |
| 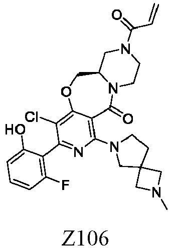 | 542.3 | 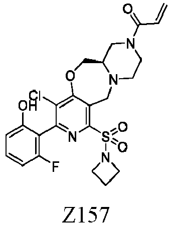 | 523.1 | 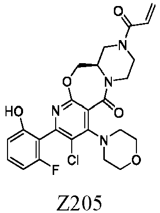 | 503. 1 | 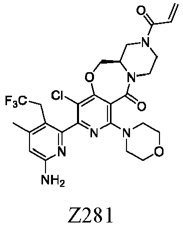 | 581. 2 |
| 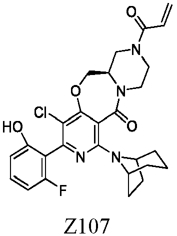 | 527.2 | 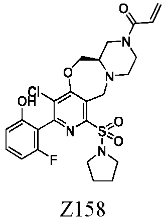 | 537.1 | 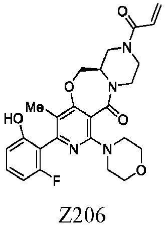 | 483. 2 | 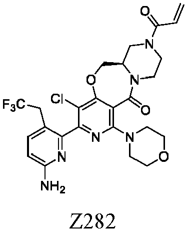 | 567. 2 |
| 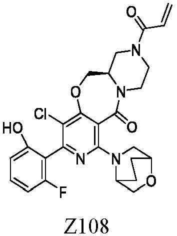 | 529.2 | 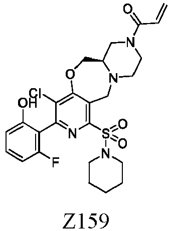 | 551.2 | 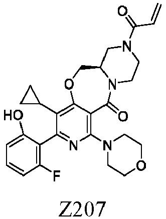 | 509. 2 | 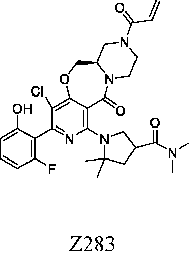 | 586. 2 |
| 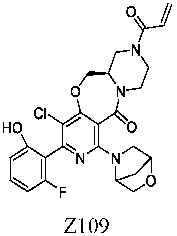 | 515.1 | 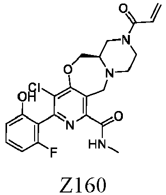 | 461.1 | 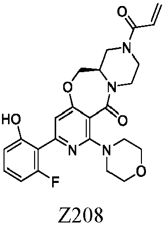 | 469. 2 | 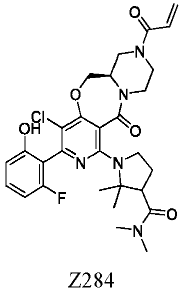 | 586. 2 |
| 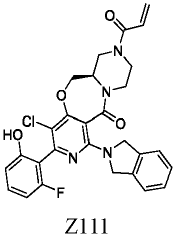 | 535.2 | 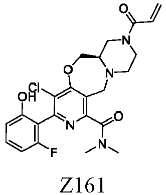 | 475.2 | 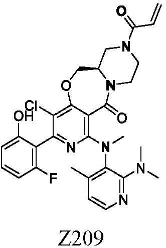 | 581. 2 | 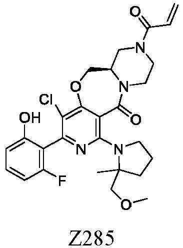 | 545. 2 |
| 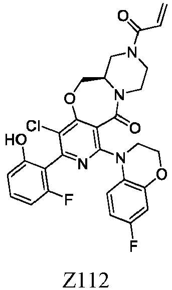 | 569.1 | 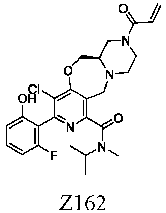 | 503.2 | 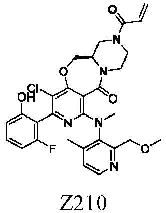 | 582. 2 | 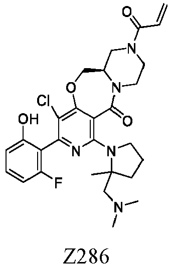 | 558. 2 |
| 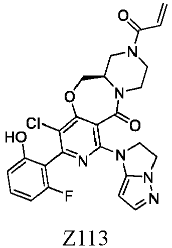 | 525.1 | 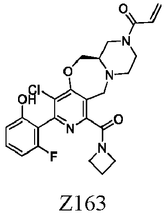 | 487.2 | 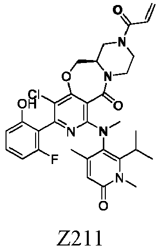 | 610. 2 | 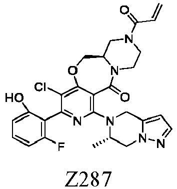 | 553. 2 |
| 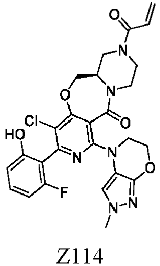 | 555.2 | 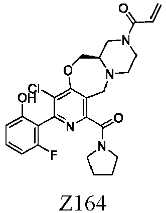 | 501.2 | 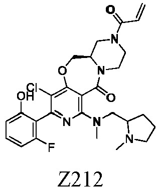 | 544. 2 | 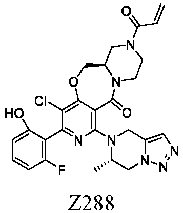 | 554. 2 |
| 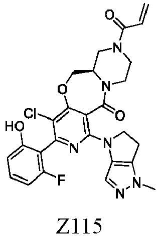 | 539.2 | 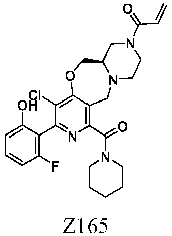 | 515.2 | 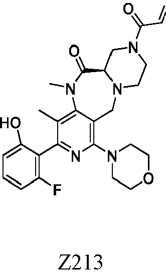 | 496.2 | 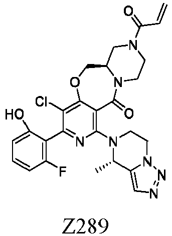 | 554. 2 |
| 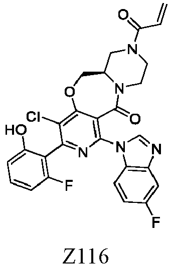 | 552.1 | 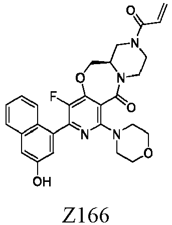 | 519.2 | 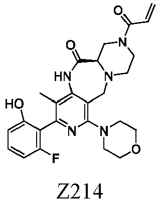 | 482. 2 | 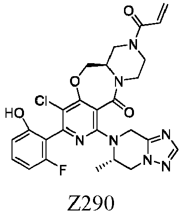 | 554. 2 |
| 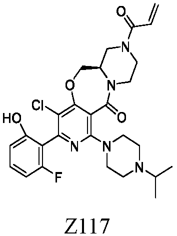 | 544.2 | 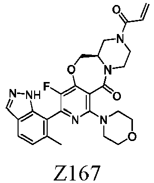 | 507.2 | 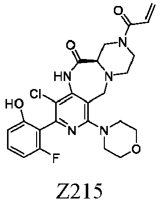 | 502. 2 | 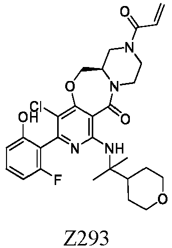 | 559. 2 |
| 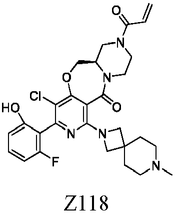 | 556.2 | 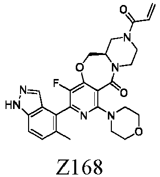 | 507.2 | 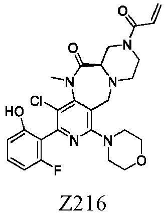 | 516. 2 | 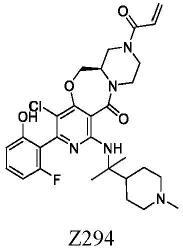 | 572. 2 |
| 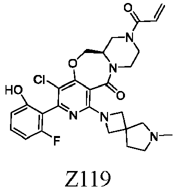 | 542.2 | 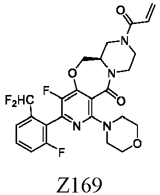 | 521.2 | 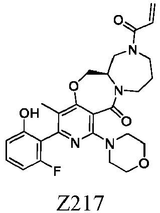 | 497. 2 | 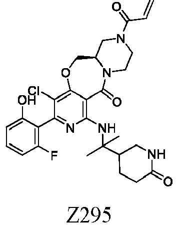 | 572. 2 |
| 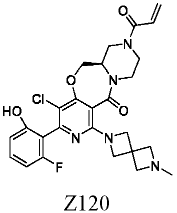 | 528.2 | 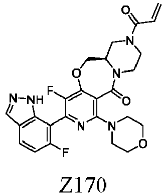 | 511.2 | 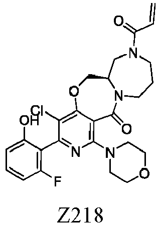 | 517. 2 | 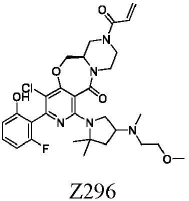 | 602. 3 |
| 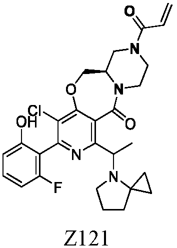 | 541.2 | 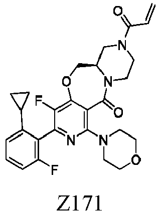 | 511.2 | 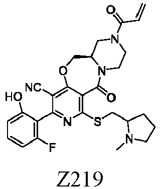 | 538. 2 | 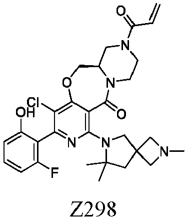 | 570. 2 |
| 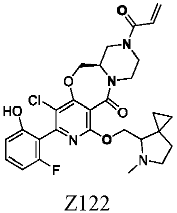 | 557.2 | 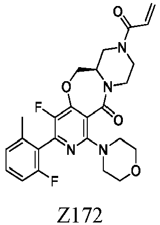 | 485.2 | 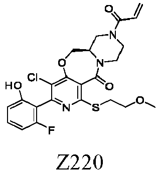 | 508. 10 | 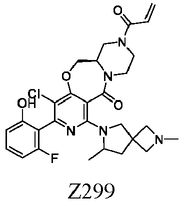 | 556. 2 |
| 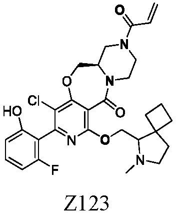 | 571.2 | 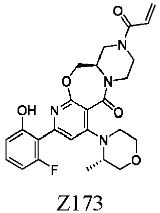 | 483.2 | 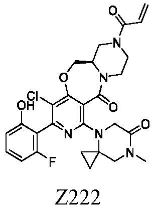 | 556. 2 | 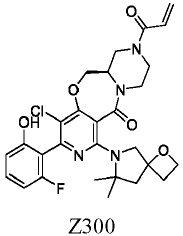 | 557. 2 |
| 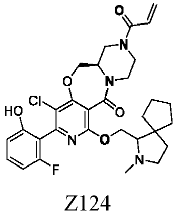 | 585.2 | 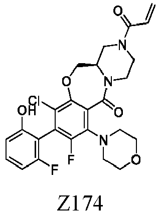 | 520.1 | 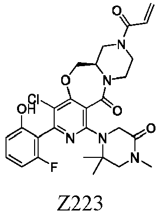 | 558. 2 | 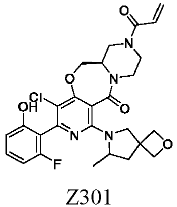 | 543. 2 |
| 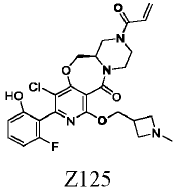 | 517.2 | 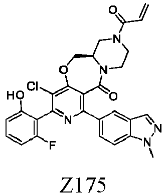 | 548.1 | 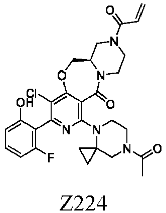 | 570. 2 | 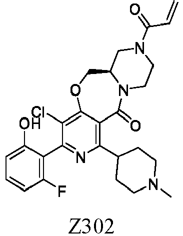 | 515. 2 |
| 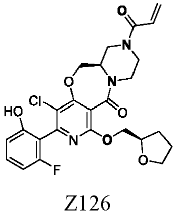 | 518.1 | 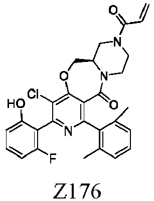 | 522.2 | 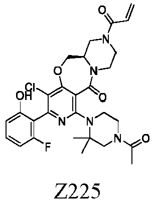 | 572. 2 | 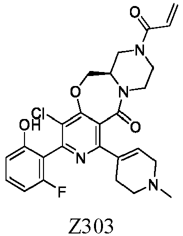 | 513. 2 |
| 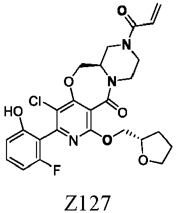 | 518.1 | 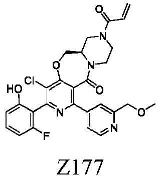 | 539.1 | 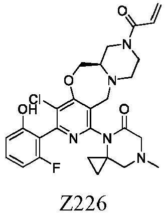 | 542. 2 | 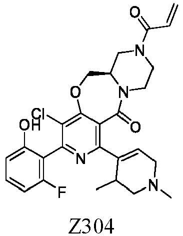 | 527. 2 |
| 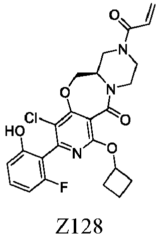 | 488.1 | 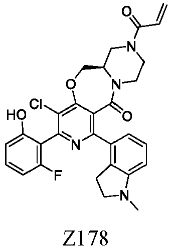 | 549.2 | 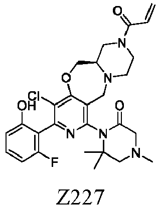 | 544. 2 | 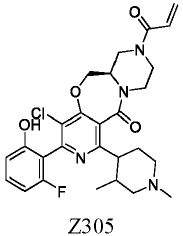 | 529. 2 |
| 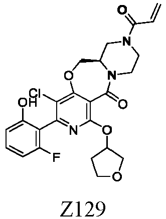 | 504.1 | 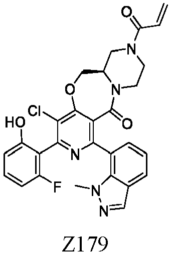 | 548.1 | 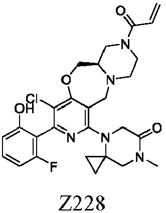 | 542. 2 | 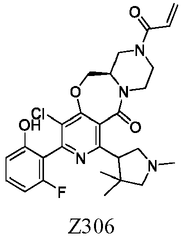 | 529. 2 |
| 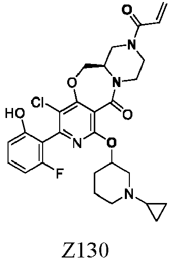 | 557.2 | 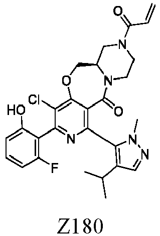 | 540.2 | 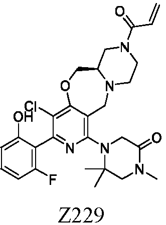 | 544. 2 | 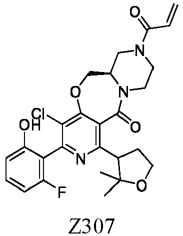 | 516. 2 |
| 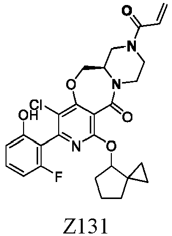 | 528.2 | 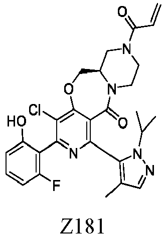 | 540.2 | 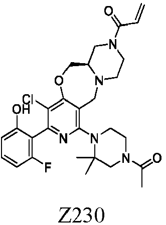 | 558. 2 | 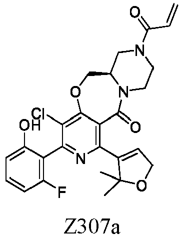 | 514. 2 |
| 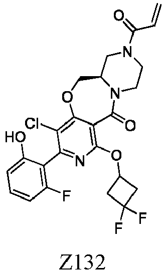 | 524.1 | 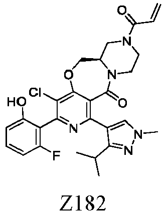 | 540.2 | 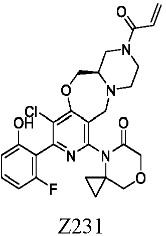 | 529. 2 | | |
| 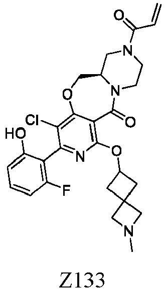 | 543.2 | 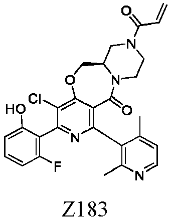 | 523.2 | 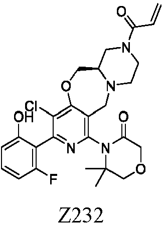 | | | |
| 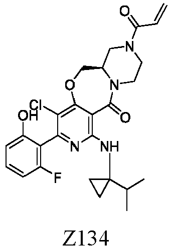 | 515.2 | 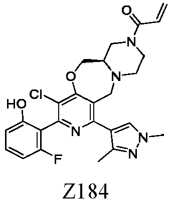 | 498.2 | 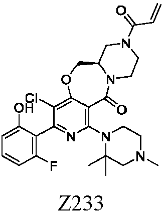 | 544. 2 | | |

| | Mass spectrum | | Mass spectrum | | Mass spectrum | | Mass spectrum |
|---|---|---|---|---|---|---|---|
| 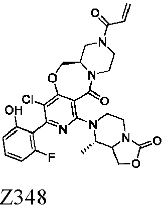 | 572.2 | 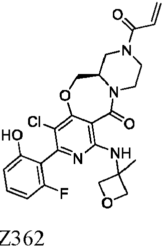 | 503.1 | 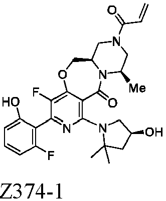 | 529.2 | 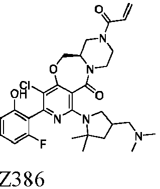 | 572.2 |
| 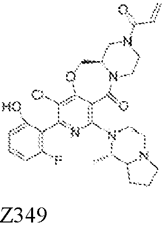 | 556.2 | 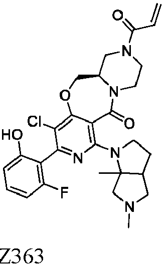 | 556.2 | 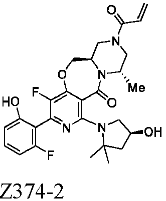 | 529.2 | 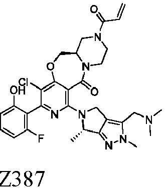 | 610.2 |
| 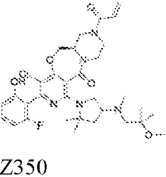 | 630.3 | 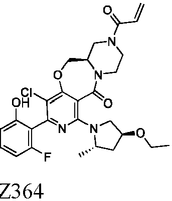 | 545.2 | 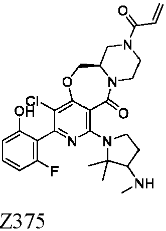 | 544.2 | 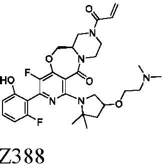 | 586.3 |
| 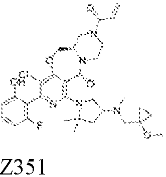 | 628.3 | 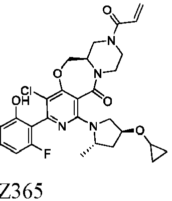 | 557.2 | 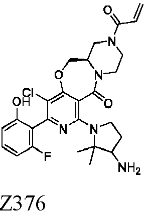 | 530.2 | 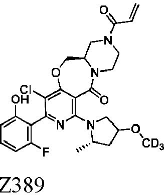 | 534.2 |
| 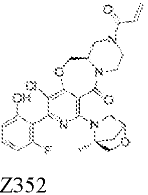 | 529.2 | 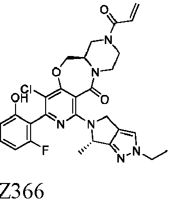 | 567.2 | 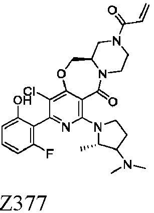 | 544.2 | 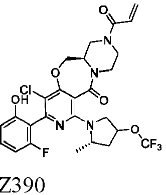 | 585.1 |
| 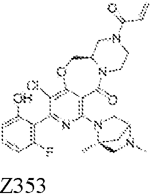 | 542.2 | 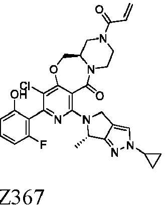 | 579.2 | 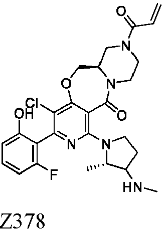 | 530.2 | 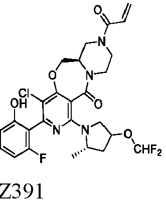 | 567.2 |
| 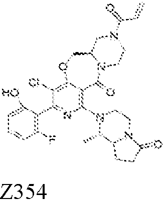 | 570.2 | 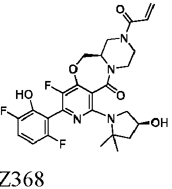 | 533.2 | 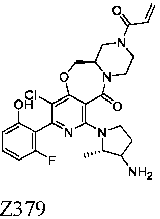 | 516.2 | 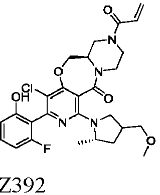 | 545.2 |
| 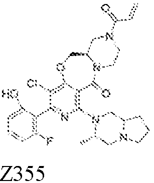 | 556.2 | 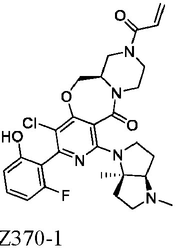 | 556.2 | 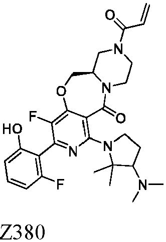 | 542.2 | 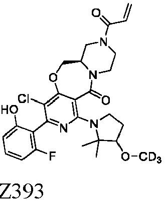 | 548.2 |
| 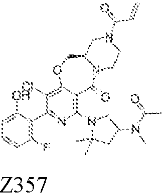 | 586.2 | 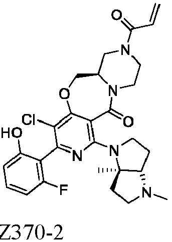 | 556.2 | 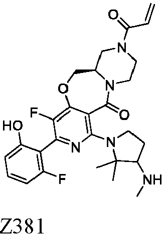 | 528.2 | 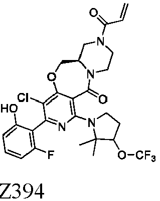 | 599.2 |
| 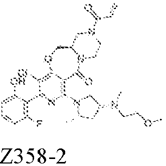 | 588.2 | 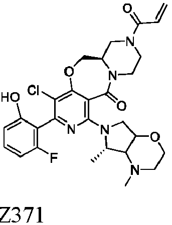 | 572.2 | 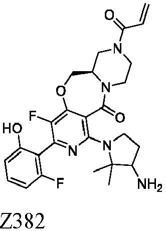 | 514.2 | 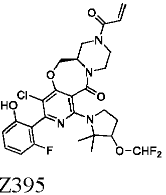 | 581.2 |
| 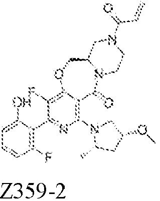 | 515.2 | 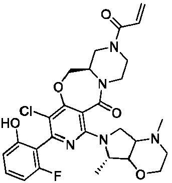 | 572.2 | 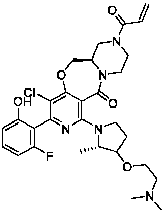 | 588.2 | 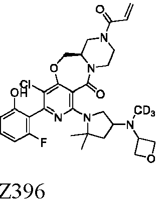 | 603.3 |
| | | 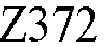 | | 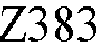 | | | |
| 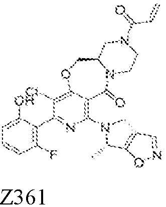 | 540.1 | 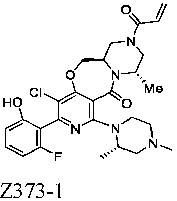 | 544.2 | 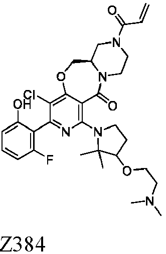 | 602.3 | 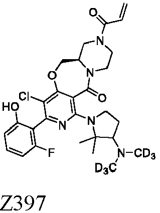 | 564.3 |
| 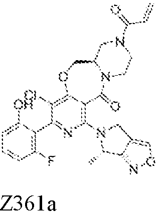 | 540.1 | 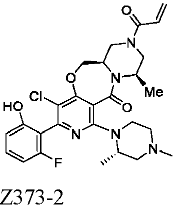 | 544.2 | 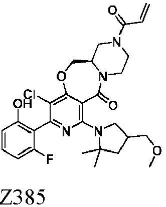 | 559.2 | 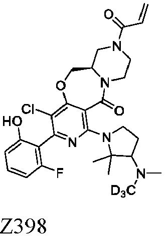 | 561.2 |
| 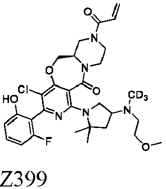 | 605.3 | 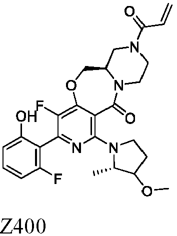 | 515.2 | | | | |

| | Mass spectrum | | Mass spectrum | | Mass spectrum | | Mass spectrum |
|---|---|---|---|---|---|---|---|
| 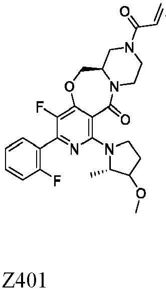 | 499.2 | 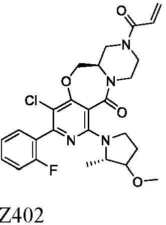 | 515.2 | 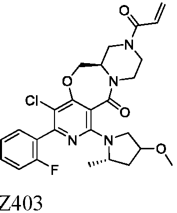 | 515.2 | 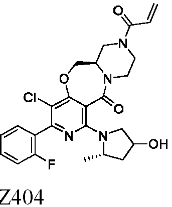 | 501.2 |
| 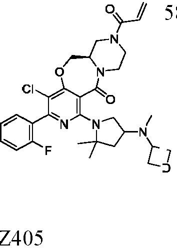 | 584.2 | 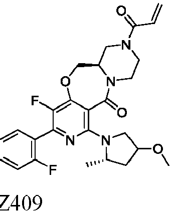 | 499.2 | 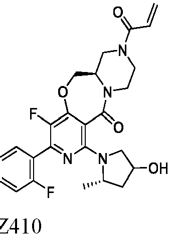 | 485.2 | 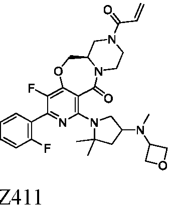 | 568.3 |
| 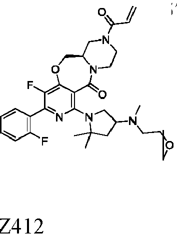 | 570.3 | 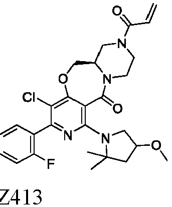 | 529.2 | 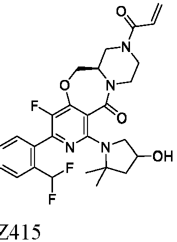 | 531.2 | 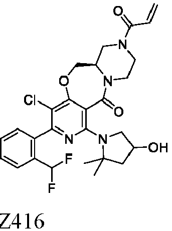 | 547.2 |
| 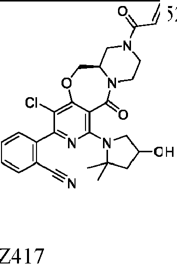 | 522.2 | 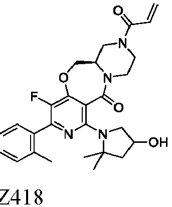 | 495.2 | 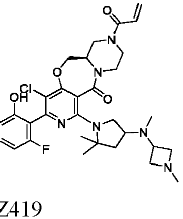 | 613.3 | 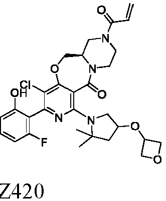 | 587.2 |
| 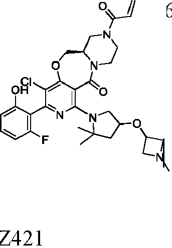 | 600.2 | 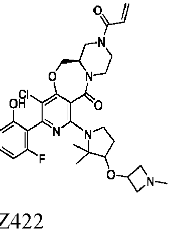 | 600.2 | 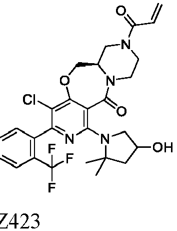 | 565.2 | 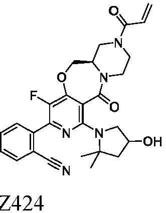 | 506.2 |

| | Mass spectrum | | Mass spectrum | | Mass spectrum |
|---|---|---|---|---|---|
| 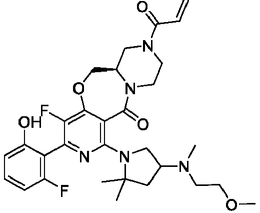 | 586.3 | 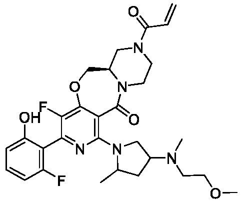 | 572.3 | 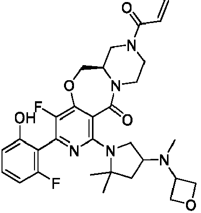 | 584.3 |
| 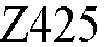 | | 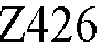 | | 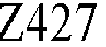 | |
| 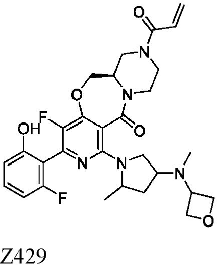 | 570.2 | 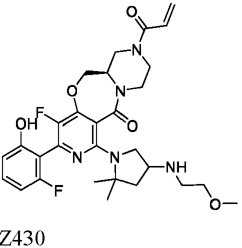 | 572.3 | 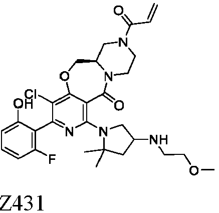 | 588.2 |
| 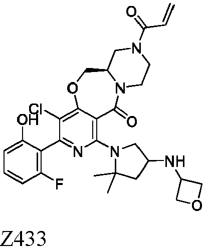 | 586.2 | 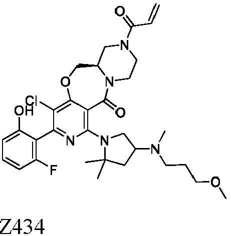 | 616.3 | 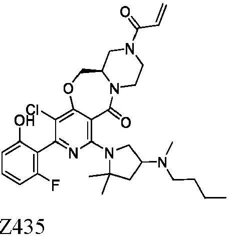 | 599.3 |
| 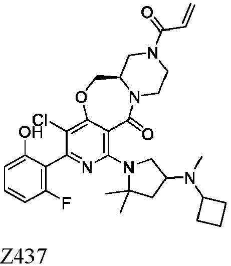 | 598.3 | 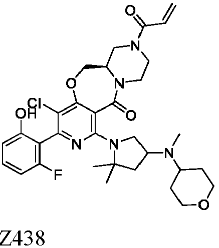 | 628.3 | 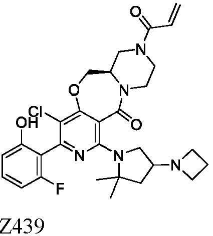 | 570.2 |
| 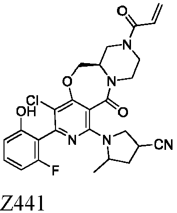 | 526.2 | 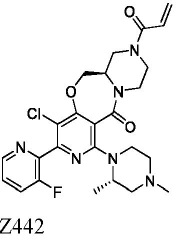 | 515.2 | 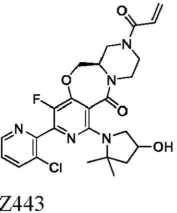 | 516.2 |
| 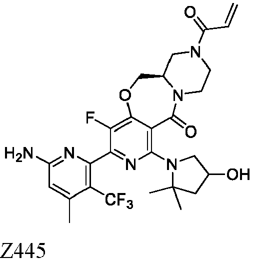 | 579.2 | 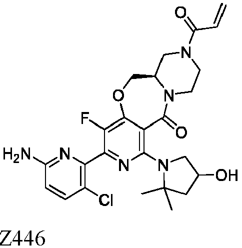 | 531.2 | 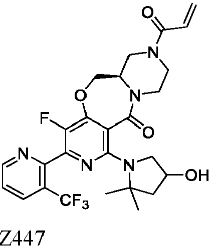 | 550.2 |
| 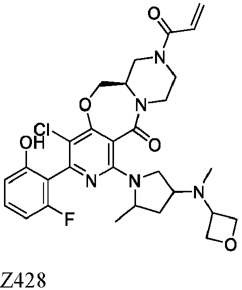 | 586.2 | 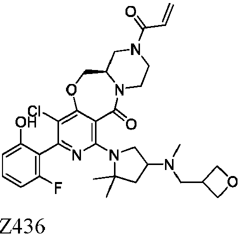 | 614.3 | 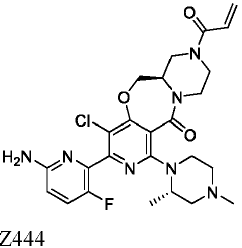 | 530.2 |
| 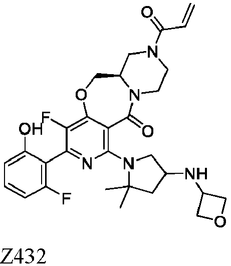 | 570.2 | 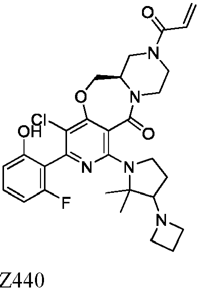 | 570.2 | | |

| | Mass spectrum | | Mass spectrum | | Mass spectrum |
|---|---|---|---|---|---|
| 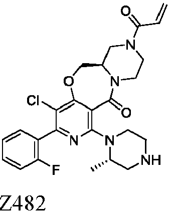 | 500.2 | 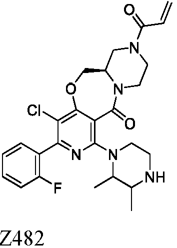 | 514.2 | 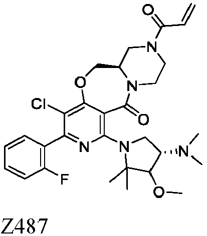 | 572.2 |
| 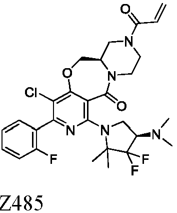 | 578.2 | 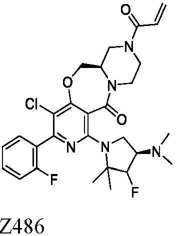 | 560.2 | 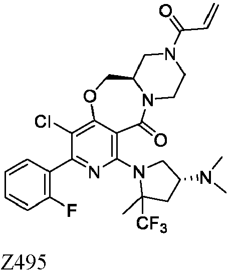 | 596.2 |
| 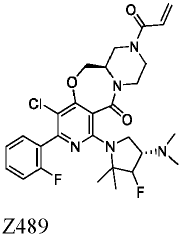 | 560.2 | 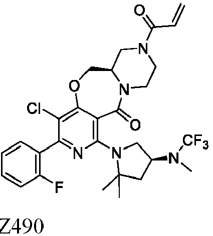 | 596.2 | 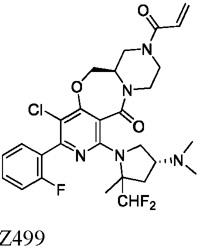 | 578.2 |
| 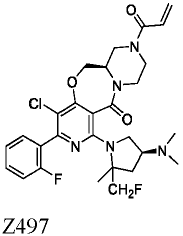 | 560.2 | 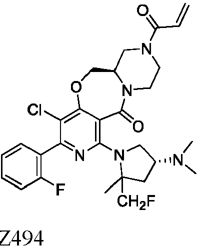 | 560.2 | 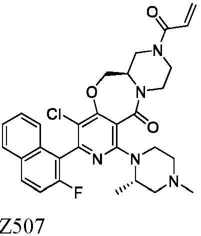 | 564.2 |
| 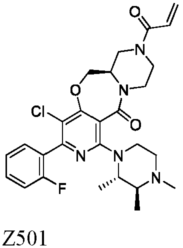 | 528.2 | 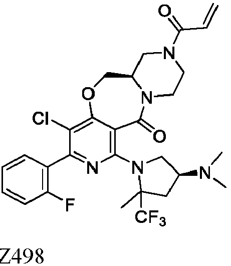 | 596.2 | 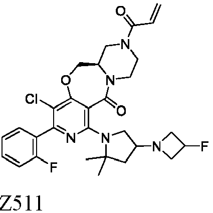 | 572.2 |
| 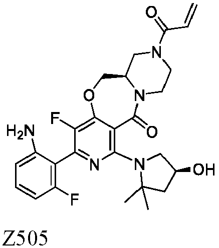 | 514.2 | 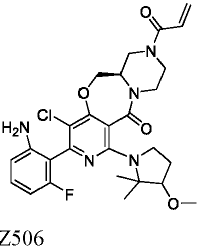 | 544.2 | 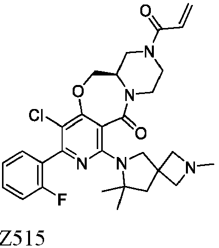 | 554.2 |
| 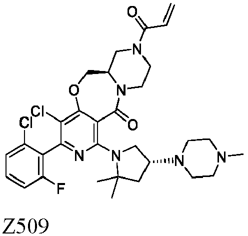 | 631.2 | 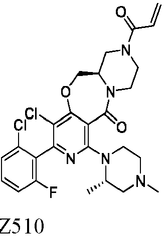 | 548.2 | 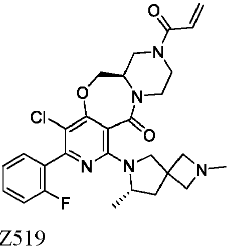 | 540.2 |
| 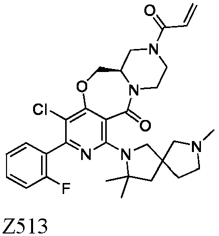 | 568.2 | 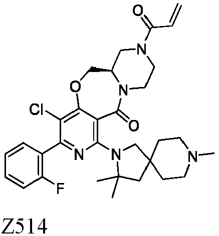 | 582.3 | 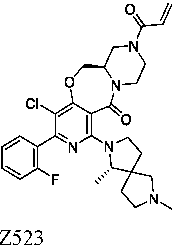 | 554.2 |
| 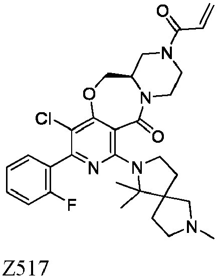 | 568.2 | 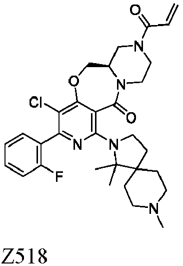 | 582.3 | 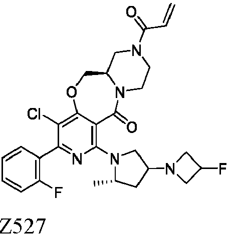 | 558.2 |
| 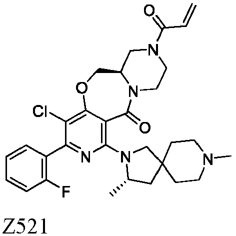 | 568.2 | 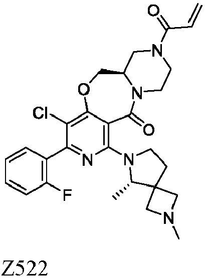 | 540.2 | 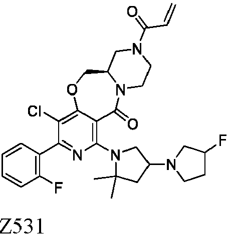 | 586.2 |
| 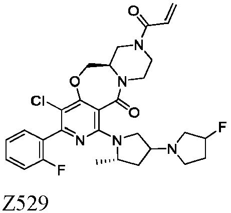 | 572.2 | 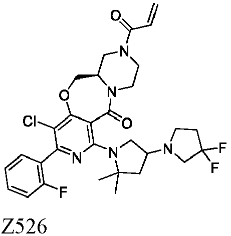 | 604.2 | 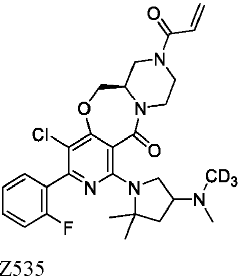 | 545.2 |
| 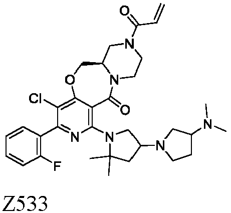 | 611.3 | 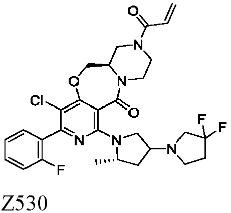 | 590.2 | 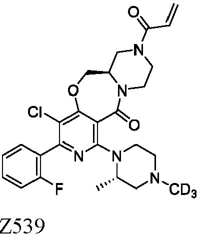 | 517.2 |
| 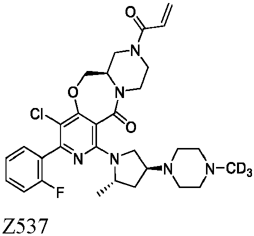 | 586.3 | 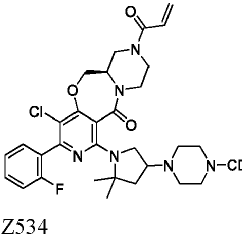 | 600.3 | 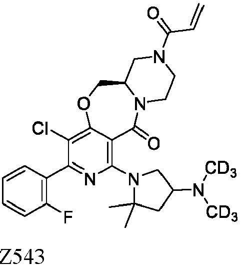 | 548.3 |
| 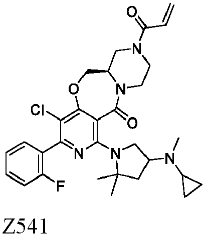 | 568.2 | 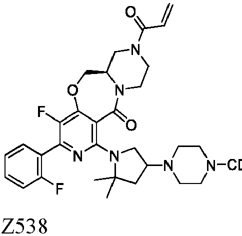 | 584.3 | 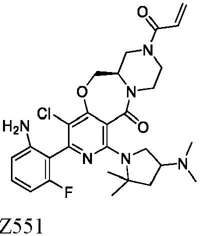 | 557.2 |
| 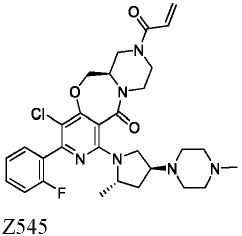 | 583.3 | 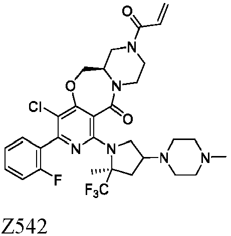 | 651.2 | 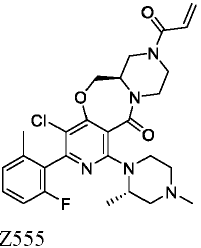 | 528.2 |
| 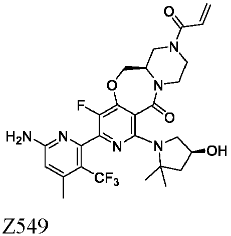 | 579.2 | 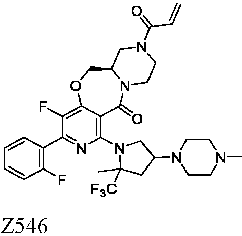 | 635.3 | 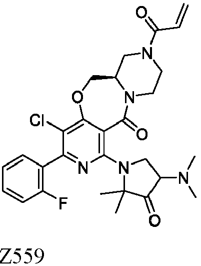 | 556.2 |
| 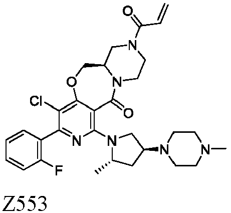 | 583.3 | 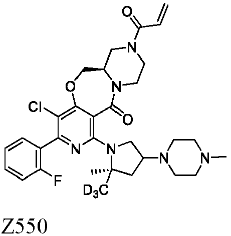 | 600.3 | 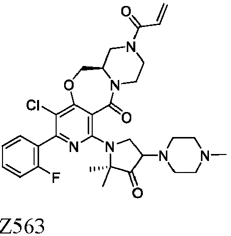 | 611.2 |
| 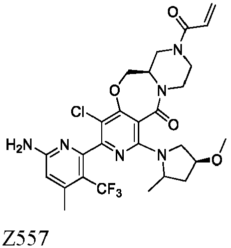 | 595.2 | 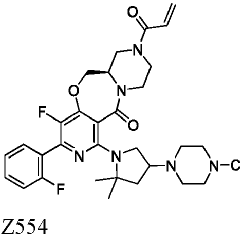 | 635.3 | 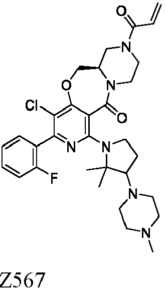 | 597.3 |
| 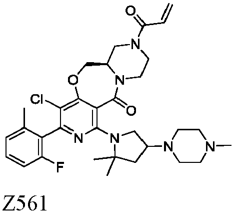 | 611.3 | 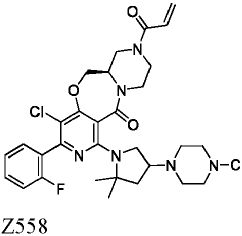 | 651.2 | 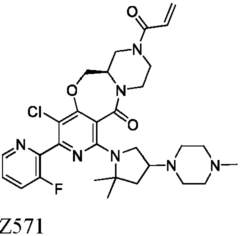 | 598.3 |
| 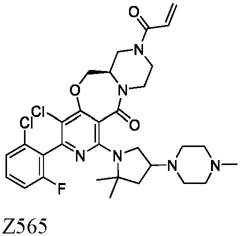 | 631.2 | 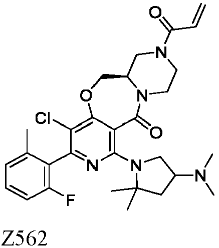 | 556.2 | 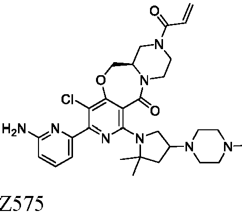 | 595.3 |
| 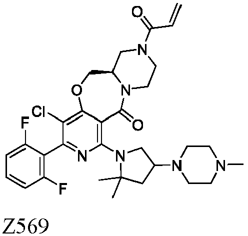 | 615.3 | 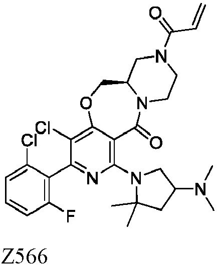 | 576.2 | 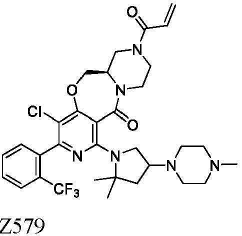 | 647.3 |
| 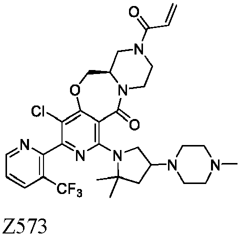 | 648.3 | 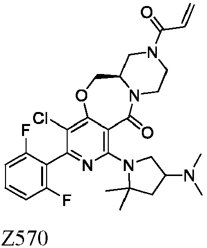 | 560.2 | 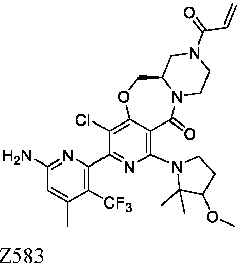 | 609.2 |
| 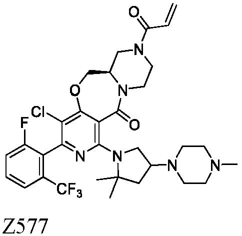 | 665.3 | 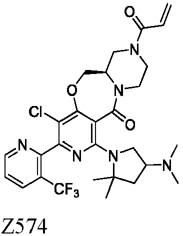 | 593.2 | 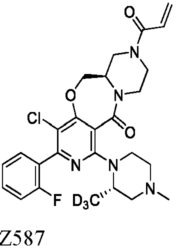 | 517.2 |
| 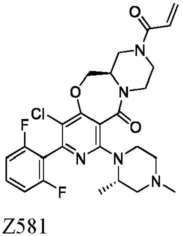 | 532.2 | 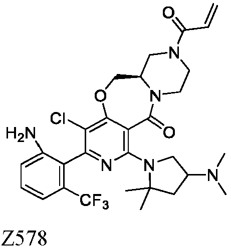 | 607.2 | 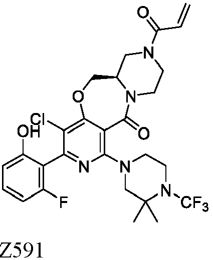 | 598.2 |
| 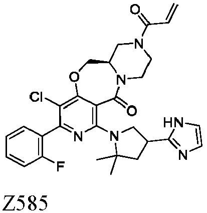 | 565.2 | 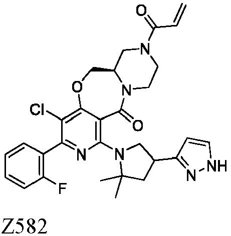 | 565.2 | 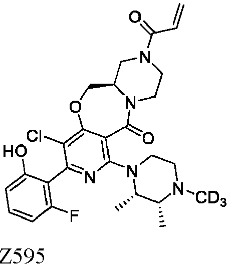 | 547.2 |
| 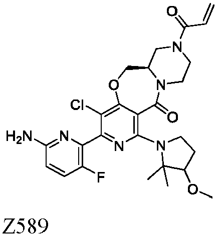 | 545.2 | 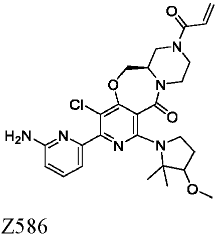 | 527.2 | 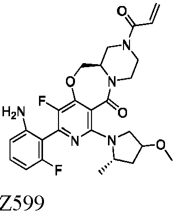 | 514.2 |
| 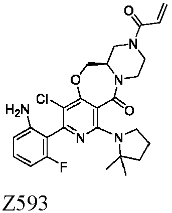 | 514.2 | 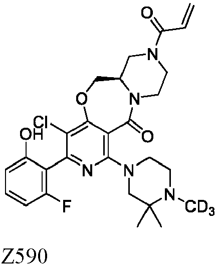 | 547.2 | 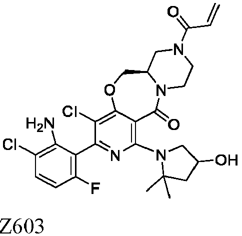 | 564.2 |
| 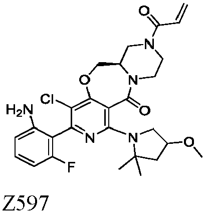 | 544.2 | 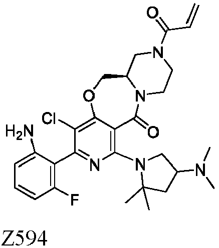 | 557.2 | 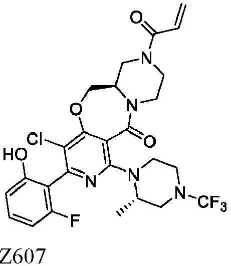 | 584.2 |
| 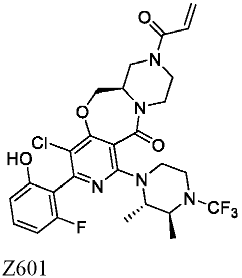 | 598.2 | 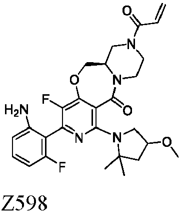 | 528.2 | 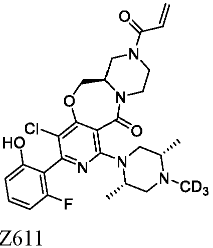 | 547.2 |
| 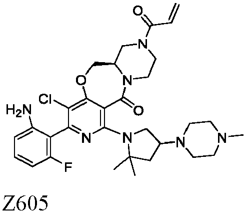 | 612.3 | 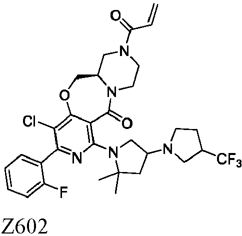 | 636.2 | 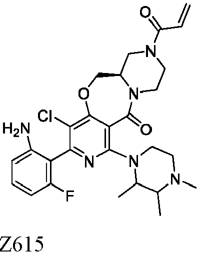 | 543.2 |
| 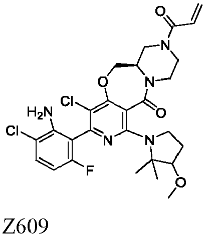 | 578.2 | 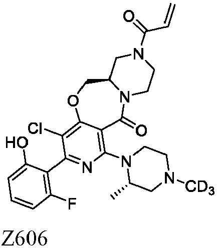 | 533.2 | 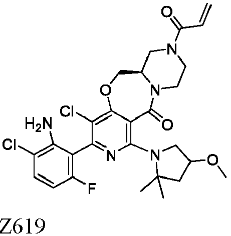 | 578.2 |
| 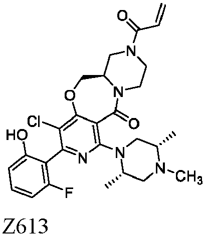 | 544.2 | 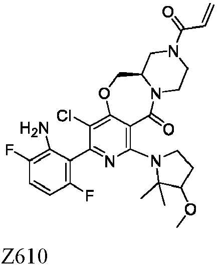 | 562.2 | 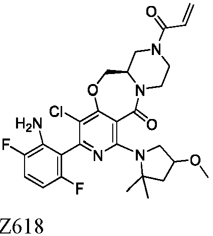 | 562.2 |
| 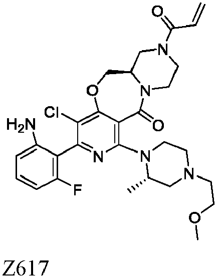 | 573.2 | 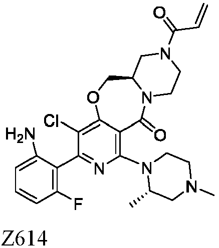 | 529.2 | | |

| | Mass spectrum | | Mass spectrum | | Mass spectrum |
|---|---|---|---|---|---|
| 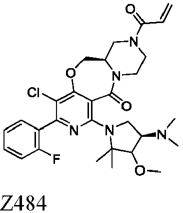 | 572. 2 | 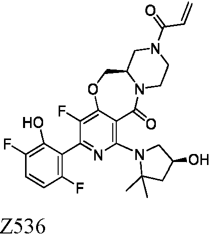 | 533. 2 | 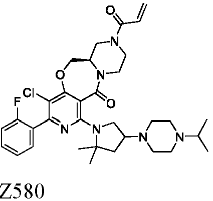 | 625. 3 |
| 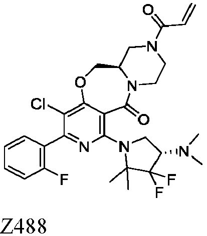 | 578. 2 | 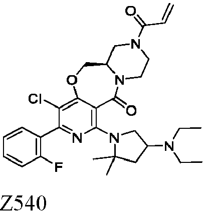 | 570. 3 | 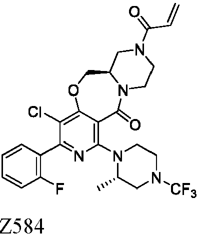 | 568. 2 |
| 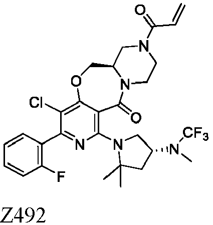 | 596. 2 | 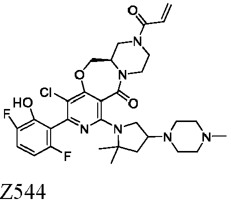 | 631. 3 | 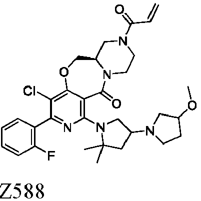 | 598. 3 |
| 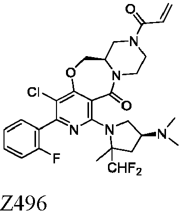 | 578. 2 | 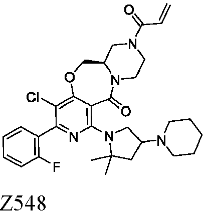 | 582. 3 | 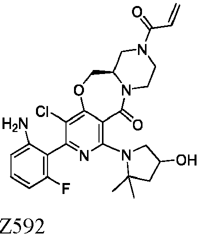 | 530. 2 |
| 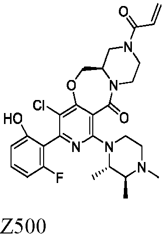 | 544. 2 | 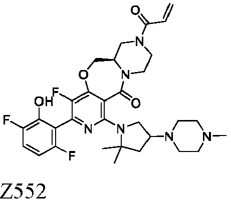 | 615. 3 | 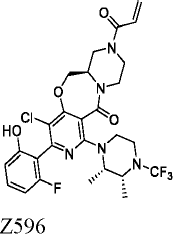 | 598. 2 |
| 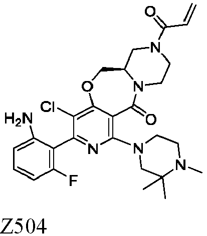 | 543. 2 | 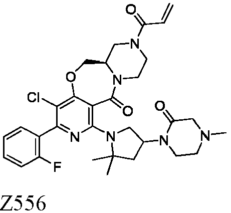 | 611.2 | 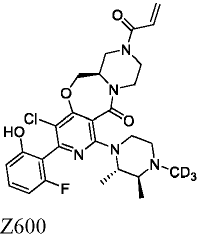 | 547. 2 |
| 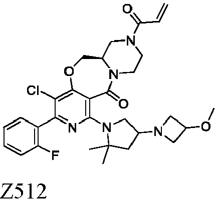 | 584. 2 | 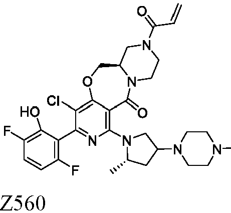 | 617. 2 | 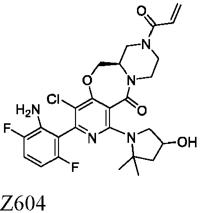 | 548. 2 |
| 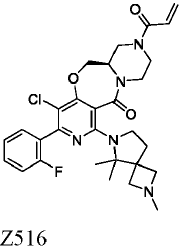 | 554. 2 | 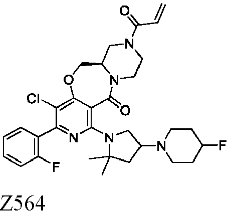 | 600. 2 | 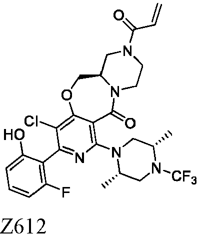 | 598. 2 |
| 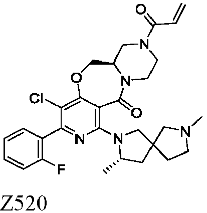 | 554. 2 | 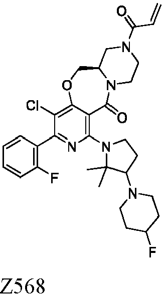 | 600. 2 | 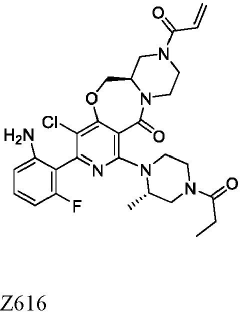 | 571. 2 |
| 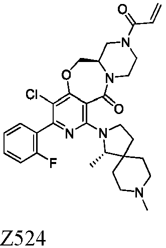 | 568. 2 | 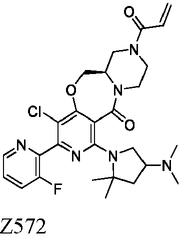 | 543. 2 | 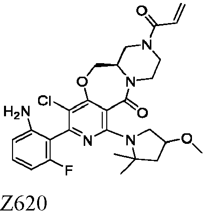 | 544. 2 |
| 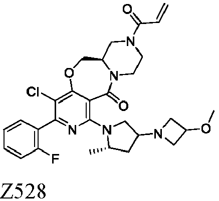 | 570. 2 | 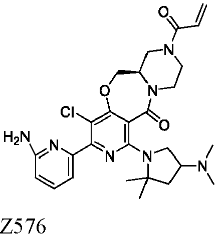 | 540. 2 | 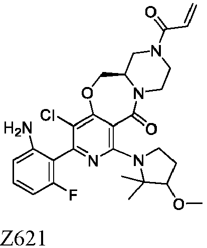 | 544. 2 |
| 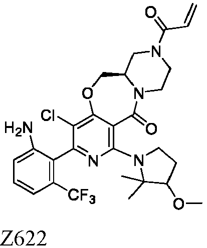 | 594. 2 | 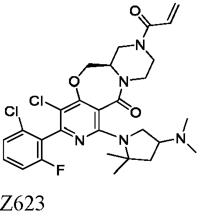 | 576. 2 | 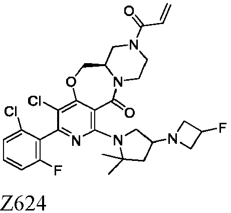 | 606. 2 |
| 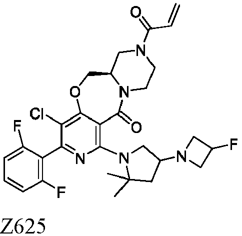 | 590. 2 | 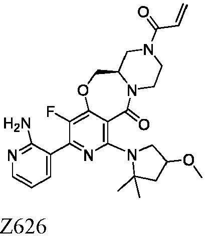 | 511.2 | 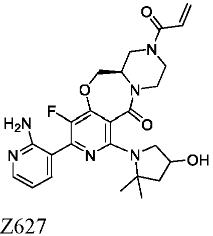 | 497. 2 |
| 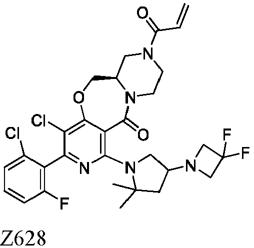 | 624. 2 | 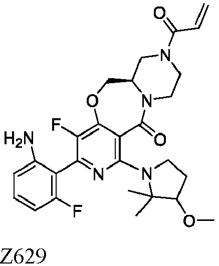 | 528. 2 | 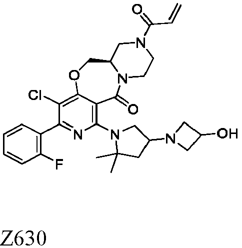 | 570. 2 |
| 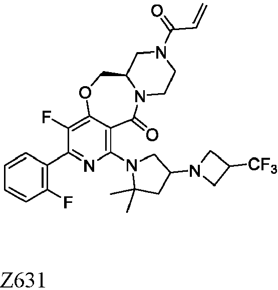 | 606. 2 | 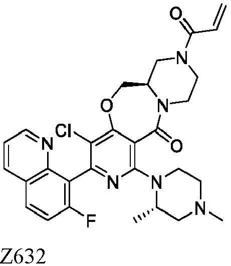 | 565. 2 | 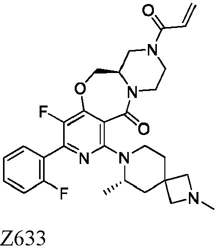 | 538. 3 |
| 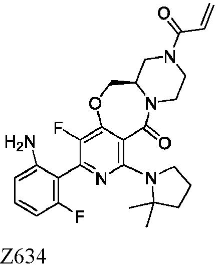 | 498. 2 | 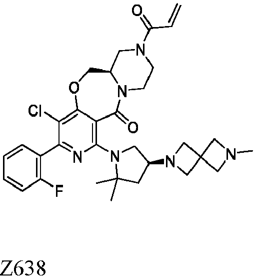 | 609. 2 | 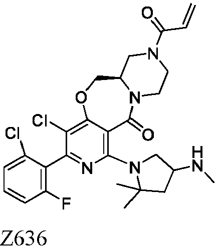 | 562. 2 |
| 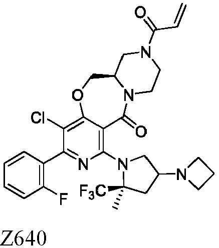 | 608. 2 | 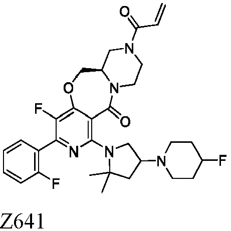 | 584. 3 | 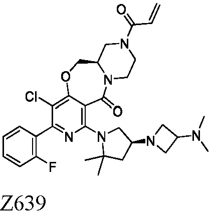 | 597. 3 |
| 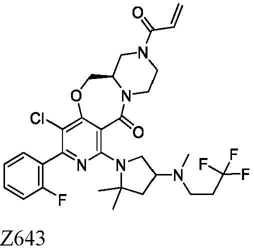 | 624. 2 | 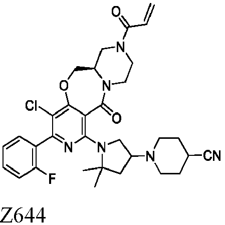 | 607. 3 | 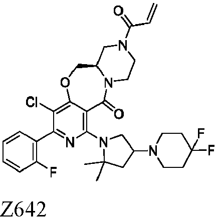 | 618. 2 |
| 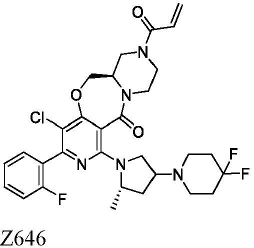 | 604. 2 | 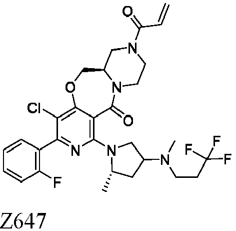 | 610. 2 | 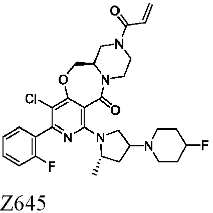 | 586. 2 |
| 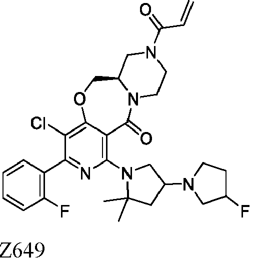 | 586. 2 | 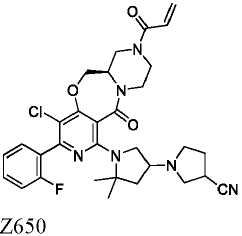 | 593. 2 | 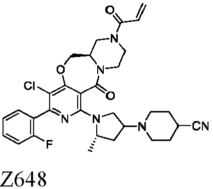 | 593. 2 |
| 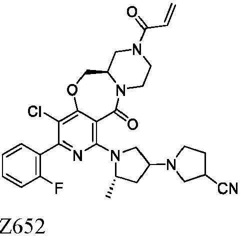 | 579. 2 | 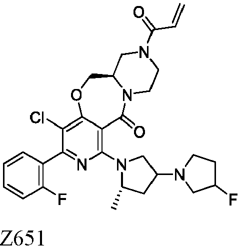 | 572. 2 | | |

### Test embodiment 1 Cell proliferation inhibition experiment

Raw materials and instruments: NCI-H358 purchased from ATCC; article number: CRL-5807; batch number: 70010825; A549 purchased from ATCC; article number: CCL-185; batch number: 70007820; RPMI1640 purchased from Gibco; article number: 11875-093; batch number: 2152748; F-12K purchased from Gibco; article number: 21127-022; batch number: 2185853; FBS purchased from Gibco; article number: 10099-141; batch number: 2109290CP; PS purchased from Gibco; article number: 15140-122; batch number: 2145451; Trypsin-EDTA purchased from Gibco; article number: 25200-072; batch number: 2085552; CellTiter Glo working solution purchased from Promega; article number: G9683; batch number: 398478; DMSO purchased from VWRAMRESCO; article number: 0231-500ML; batch number: 18C0656168; 384-well spheroid plate purchased from Coming; article number: 3830; batch number: 27519032; white opaque 384-well plate purchased from Coming; article number: 3570; batch number: 26819023; microplate reader purchased from PerkinElmer; model: Envision MLD2105-0020; equipment number 1050369.

NCI-H358 is a KRAS G12C mutant human non-small cell lung cancer cell line, cultured in 10% FBS RPMI-1640+1% PS culture medium; and A549 is a KRAS G12S mutant human lung adenocarcinoma cell line, cultured in 10% FBS F-12K+1% PS culture medium. Cells at logarithmic growth stage were taken, digested with trypsin-EDTA, then collected and counted. H358 was adjusted to 1.8 E4 cells/mL with 2% FBS RPMI-1640 culture medium, and A549 was adjusted to 8.9 E3 cells/mL with 2% FBS F-12K culture medium; 800 (45 µL) H358 or 400 (45 µL) A549 were inoculated respectively in a 384-well spheroid plate and cultured overnight to establish a 3D cell model. A 3.16-fold gradient concentration stock solution of 1000X compound was prepared with DMSO, and diluted 100-fold to 10X compound stock solution with 2% FBS culture medium. On the second day after cell inoculation, 5 µL of 10X compound stock solution was added to each cell culture well, with a final concentration of 1X and a DMSO content of 0.1%. DMSO was used as experimental control (control), and 2% FBS culture medium was used as blank control (blank). After 5 days of compound cell culture, 25 µL of CellTiter-Glo working solution was added to each well, mixed well at 400 rpm and incubated for 30 minutes. After standing at room temperature for 30 minutes, 40 µL of the mixed solution was transferred to a white opaque 384-well plate, and the luminescence chemiluminescence value was read with a microplate reader. The cell proliferation inhibition rate IR (%) = (RLU control -RLU compound)/(RLU control -RLU blank) ×100% was calculated. The gradient dilution concentration of the compound and the corresponding cell proliferation inhibition rate were fitted by Prism 6 four-parameter method, and the IC₅₀ value was calculated. The inhibitory activity of the compounds prepared in the above embodiments of the present disclosure on KRAS G12C mutant NCI-H358 cells was 0.0001 µM to 0.5 µM; the inhibitory activity of some compounds on KRAS G12C mutant NCI-H358 cells was 0.001 µM to 0.1 µM or 0.001 µM to 0.05 µM. It can be seen from the results that the exemplary compounds of the present disclosure have high inhibitory activity on KRAS G12C mutant NCI-H358 cells, with IC₅₀ of less than 500 nM, or less than 100 nM or less than 10 nM, while they have low inhibitory activity on A549 cells, with IC₅₀ of more than 1000 nM and obvious selective inhibitory activity.

**Table 1 Inhibitory activity of compounds on H358 and A549 cells**

| No. | H358 IC₅₀ (µM) | A549 IC₅₀ (µM) | No. | H358 IC₅₀ (µM) | A549 IC₅₀ (µM) | No. | H358 IC₅₀ (µM) | A549 IC₅₀ (µM) |
|---|---|---|---|---|---|---|---|---|
| Z5 | 0.031 | 4.677 | Z256-1 | 0.0022 | 12.095 | Z369 | 0.0036 | 27.096 |
| Z6 | 0.007 | >30 | Z256-2 | 0.0270 | - | Z408-1 | 0.0217 | 9.252 |
| Z8 | 0.051 | 5.086 | Z309 | 0.2910 | - | Z414-1 | 0.0190 | 8.886 |
| Z9 | 0.023 | 11.275 | Z257a | 0.0040 | 28.21 | Z407-1 | 0.0188 | 9.155 |
| Z10 | 0.039 | >20 | Z221-2 | 0.0168 | - | Z450 | 0.0131 | 20.487 |
| Z11 | 0.042 | 9.342 | Z221-1 | 0.0027 | >30 | Z451 | 0.1581 | 5.720 |
| Z13 | 0.051 | >5 | Z310 | 0.0106 | - | Z452 | 0.0039 | 8.994 |
| Z15 | 0.010 | 18.995 | Z239-2 | 0.0558 | - | Z453-1 | 0.006 | 3.340 |
| Z16 | 0.037 | >1 | Z239-1 | 0.0150 | - | Z453-2 | 0.0166 | 6.715 |
| Z18 | 0.007 | 8.677 | Z238 | 0.2850 | - | Z454 | 0.002 | 2.127 |
| Z19 | 0.035 | >30 | Z279-1 | 0.1850 | - | Z455-1 | 0.0452 | 4.183 |
| Z20 | 0.004 | 9.020 | Z279-2 | 0.0038 | >30 | Z455-2 | 0.0037 | >10 |
| Z21 | 0.029 | >20 | Z311-1 | 0.0100 | >30 | Z456 | 0.0094 | 9.733 |
| Z22 | 0.002 | >30 | Z83 | 0.0180 | >10 | Z454-1 | 0.0012 | 2.627 |
| Z23 | 0.050 | >5 | Z237-2 | 0.0060 | 5.53 | Z454-2 | 0.0072 | 2.659 |
| Z24 | 0.026 | >5 | Z102-4 | 0.0154 | - | Z457 | 0.0627 | 2.949 |
| Z25 | 0.058 | >20 | Z102-1 | 0.0513 | - | Z458 | 0.3049 | >30 |
| Z26 | 0.479 | >30 | Z241 | 0.0026 | - | Z459 | 0.9063 | |
| Z27 | 0.033 | >5 | Z237-1 | 0.0146 | - | Z458-1 | 0.1894 | >10 |
| Z28 | 0.062 | >5 | Z276-1 | 0.0076 | >30 | Z461 | 0.0240 | |
| Z29 | 0.030 | 2.434 | Z312-1 | 0.0135 | - | Z462 | 0.0124 | 29.138 |
| Z30 | 0.040 | >20 | Z313-1 | 0.0016 | >30 | Z463 | 0.039 | |
| Z31 | 0.176 | >30 | Z296-1 | 0.0004 | 3.34 | Z464 | 0.1441 | |
| Z32 | 0.009 | >5 | Z240 | 0.0024 | - | Z465 | 0.038 | |
| Z33 | 0.113 | >30 | Z314 | 0.0060 | >30 | Z466-1 | 0.049 | |
| Z264 | 0.085 | 9.477 | Z240-1 | 0.0025 | 10.05 | Z466-2 | 0.038 | |
| Z34 | 0.032 | 4.910 | Z240-2 | 0.0019 | >10 | Z467 | 0.0008 | |
| Z35 | 0.021 | >5 | Z241-1 | 0.0028 | >30 | Z465-1 | 0.013 | |
| Z36 | 0.040 | >1 | Z241-2 | 0.0022 | 9.270 | Z406-1 | 0.004 | 2.122 |
| Z37 | 0.009 | >30 | Z243-1 | 0.0006 | 3.097 | Z406-2 | 0.021 | |
| Z38 | 0.055 | >10 | Z243-2 | 0.0111 | 3.440 | Z468-1 | 0.004 | 3.283 |
| Z39 | 0.009 | 3.052 | Z315 | 0.0050 | >30 | Z468-2 | 0.005 | 4.559 |
| Z40 | 0.091 | >30 | Z241a | 0.0011 | 4.943 | Z469-1 | 0.0005 | 5.234 |
| Z41 | 0.022 | 1.221 | Z244-1 | 0.0019 | 2.904 | Z469-2 | 0.002 | 8.458 |
| Z42 | 0.037 | 6.291 | Z244-2 | 0.0005 | 2.080 | Z470-1 | 0.023 | |
| Z43 | 0.039 | >30 | Z304 | 0.1610 | - | Z471-1 | 0.007 | 2.774 |
| Z44 | 0.026 | >5 | Z241a-1 | 0.0014 | 9.550 | Z471-2 | 0.091 | |
| Z45 | 0.046 | >20 | Z241a-2 | 0.0007 | 3.040 | Z470-2 | 0.003 | 9.608 |
| Z46 | 0.460 | >20 | Z316-1 | 0.0013 | >30 | Z472-1 | 0.016 | 1.739 |
| Z47 | 0.047 | >20 | Z317-1 | 0.0541 | - | Z472-2 | 0.008 | 1.647 |
| Z48 | 0.015 | >5 | Z256b-1 | 0.0028 | 10.210 | Z473-1 | 0.022 | |
| Z49 | 0.004 | >5 | Z256b-2 | 0.0447 | - | Z473-2 | 0.012 | |
| Z50 | 0.002 | >5 | Z256a-1 | 0.0034 | 9.160 | Z474 | 0.009 | 6.705 |
| Z51 | 0.004 | >5 | Z256a-2 | 0.0014 | 9.270 | Z475-1 | 0.002 | 5.171 |
| Z52 | 0.036 | >5 | Z318-1 | 0.0057 | 26.260 | Z475-2 | 0.008 | 5.865 |
| Z53 | 0.146 | >30 | Z319-1 | 0.0023 | >30 | Z477 | 0.0081 | |
| Z54 | 0.124 | >30 | Z317-2 | 0.0237 | - | Z478 | 0.0296 | |
| Z55 | 0.134 | >1 | Z320-1 | 0.0013 | 8.919 | Z474-1 | 0.0083 | 7.949 |
| Z56 | 0.017 | >7 | Z317a-1 | 0.1447 | >30 | Z474-2 | 0.0633 | |
| Z57 | 0.134 | >30 | Z317a-2 | 0.1077 | >30 | Z479-1 | 0.0057 | |
| Z58 | 0.145 | >5 | Z296-2 | 0.0024 | 3.787 | Z479-2 | 0.0749 | |
| Z59 | 0.019 | >30 | Z321 | 0.0045 | - | Z480-1 | 0.0034 | 3.979 |
| Z60 | 0.125 | >30 | Z322 | 0.0045 | 10.711 | Z480-2 | 0.1551 | |
| Z61 | 0.033 | >30 | Z292S | 0.0373 | - | Z481 | 0.0032 | |
| Z62 | 0.023 | >10 | Z291-1S | 0.0184 | 9.219 | Z503 | 0.0095 | |
| Z63 | 0.014 | >1 | Z291-2 | 0.0035 | 9.694 | Z635 | 0.0022 | |
| Z64 | 0.287 | >30 | Z260a | 0.0024 | >30 | Z635-1 | 0.0008 | 2.276 |
| Z65 | 0.051 | >30 | Z259a | 0.003 | 9.122 | Z635-2 | 0.0070 | 2.966 |
| Z68 | 0.012 | >5 | Z324-1 | 0.0013 | 2.852 | Z547 | 0.0193 | 8.157 |
| Z69 | 0.009 | >5 | Z324-2 | 0.0044 | 4.654 | Z608-1 | 0.0995 | 2.951 |
| Z70 | 0.025 | >10 | Z308a-1 | 0.0071 | 14.418 | Z637-1 | 0.1253 | 2.973 |
| Z71 | 0.275 | >10 | Z325-2 | 0.0061 | >30 | Z525-1 | 0.2809 | 2.838 |
| Z74 | 0.324 | >30 | Z326 | 0.0007 | 8.844 | Z637-2 | 0.0045 | 2.521 |
| Z75 | 0.029 | >1 | Z327-1 | 0.0040 | 7.930 | Z525-2 | 0.0078 | 5.744 |
| Z76 | 0.022 | >1 | Z327-2 | 0.0084 | 9.508 | Z502-1 | 0.0296 | |
| Z77 | 0.157 | >20 | Z267 | 0.0313 | - | Z502-2 | 0.011 | 6.145 |
| Z81 | 0.001 | >1 | Z328-1 | 0.0022 | 16.096 | Z506 | 0.0008 | 9.187 |
| Z82 | 0.011 | >1 | Z329 | 0.1368 | - | Z491a | 0.002 | 4.032 |
| Z247 | 0.0410 | - | Z331-1 | 0.0005 | 8.332 | Z491 | 0.0014 | 4.533 |
| Z248-1 | 0.2340 | - | Z331-2 | 0.0011 | 9.321 | Z493 | 0.027 | 7.245 |
| Z147 | 0.0220 | - | Z332-1 | 0.016 | - | Z532 | 0.0058 | 3.168 |
| Z255 | 0.0320 | - | Z333-1 | 0.026 | >30 | Z653 | 0.0275 | 9.309 |
| Z263 | 0.0690 | - | Z334 | 0.009 | 8.546 | Z654-2 | 0.015 | 8.578 |
| Z90 | 0.0190 | - | Z335 | 0.011 | >30 | Z655-1 | 0.038 | 3.317 |
| Z93-1 | 0.0530 | - | Z336-1 | 0.0430 | - | Z655-2 | 0.018 | 2.746 |
| Z93-2 | 0.0630 | - | Z336-2 | 0.0050 | - | Z658 | 0.0026 | 5.020 |
| Z259 | 0.0290 | - | Z337 | 0.015 | >30 | Z659 | 0.0018 | 5.455 |
| Z260 | 0.0050 | 27.800 | Z338-1 | 0.006 | 12.345 | Z660-1 | 0.0017 | 3.247 |
| Z248-2 | 0.0160 | - | Z339 | 0.0290 | - | Z660-2 | 0.0108 | 9.084 |
| Z246 | 0.0350 | - | Z340-1 | 0.0052 | 5.507 | Z661 | 0.0040 | 2.658 |
| Z308 | 0.0300 | - | Z340-2 | 0.0267 | 3.171 | Z659-1 | 0.005 | 6.179 |
| Z235 | 0.0210 | 9.480 | Z341-1 | 0.0080 | 11.105 | Z659-2 | 0.001 | 2.656 |
| Z239 | 0.0229 | 13.765 | Z342 | 0.0095 | 3.059 | Z661-1 | 0.006 | 2.901 |
| Z110 | 0.0449 | - | Z343 | 0.0104 | 6.801 | Z658-1 | 0.001 | 2.967 |
| Z261 | 0.0066 | 8.890 | Z275 | 0.0021 | 7.327 | Z658-2 | 0.012 | 4.716 |
| Z257 | 0.0102 | >30 | Z344 | 0.0013 | 3.720 | Z662-1 | 0.003 | 4.087 |
| Z308a | 0.0041 | 7.096 | Z345-1 | 0.0452 | - | | | |
| Z99 | 0.0263 | - | Z345-2 | 0.0118 | 9.401 | | | |
| Z102 | 0.0356 | - | Z347-1 | 0.0021 | 9.547 | | | |
| Z236 | 0.0058 | 9.580 | Z449-1 | 0.007 | >30 | | | |
| Z221 | 0.0030 | 20.110 | Z358-1 | 0.0048 | >10 | | | |
| Z252 | 0.1280 | - | Z356 | 0.020 | 28.338 | | | |
| Z252-1 | 0.1440 | - | Z360-1 | 0.0118 | 2.531 | | | |
| Z256 | 0.0050 | 13.860 | Z359-1 | 0.0083 | 12.218 | | | |
| Z39-2 | 0.0078 | - | Z360-2 | 0.003 | >10 | | | |
| Z244 | 0.0029 | - | Z297-1 | 0.001 | 7.001 | | | |
| Z243 | 0.0022 | - | Z297-2 | 0.001 | 8.704 | | | |

| | | | | | | | | |
|---|---|---|---|---|---|---|---|---|
| - represents not tested. | | | | | | | | |

It can be seen from Table 1 that the exemplary compounds of the present disclosure have high inhibitory activity on KRAS G12C mutant NCI-H358 cells, but have low inhibitory activity on A549 cells, with obvious selective inhibitory activity.

### Test embodiment 2 Cell p-ERK detection experiment

Raw materials and instruments: MIA PaCa-2 purchased from ATCC; article number: CRL-1420; batch number: 63143594; DMEM purchased from Gibco; article number: 11995-065; batch number: 2186878; FBS purchased from Gibco; article number: 10099-141; batch number: 2109290CP; Horse serum purchased from Gibco; article number: 16050-122; batch number: 1818976; Trypsin-EDTA purchased from Gibco; article number: 25200-072; batch number: 2085552; PS purchased from Gibco; article number: 15140-122; batch number: 2145451; DMSO purchased from VWR AMRESCO; article number: 0231-500ML; batch number: 0457C072; 96-well cell culture plate purchased from Coming; article number: 3599; batch number: 22718601; white opaque 96-well plate purchased from Cisbio; article number: 66PL96025; batch number: N°05; Advance ERK phospho-T202/Y204 kit purchased from Cisbio; article number: 64AERPEH; batch number: 11D; microplate reader purchased from PerkinElmer; model: Envision MLD2105-0020; equipment number 1050369.

MIA PaCa2 was a KRAS G12C mutant human pancreatic cancer cell line cultured in 10% FBS+2.5% Horse serum DMEM culture medium. Cells at logarithmic growth stage were taken, digested with trypsin-EDTA, then collected and counted, and 2.5E4 cells were inoculated in a 96-well cell culture plate and cultured overnight. A 3.16-fold gradient concentration stock solution of 1000X compound was prepared with DMSO, and diluted 200-fold to 5X compound stock solution with culture medium. On the second day after cell inoculation, 5X compound stock solution was added to each cell culture well, with a final concentration of 1X and a DMSO content of 0.1%. DMSO was used as experimental control (control). After the compound was added and cultured for two hours, the residual culture medium was removed. 50 µL of cell lysate (from Advance ERK phospho-T202/Y204 kit, the same below) was added to each well, mixed well and incubated for 30 minutes, and then 16 µL of the mixed solution was transferred to a white opaque 96-well plate, and 16 µL of cell lysate was added to the blank wells. After the transfer was completed, 4 µL of p-ERK HTRF antibody (from Advance ERK phospho-T202/Y204 kit) mixture was added to each well, incubated for 4 hours, and the fluorescence value was read with the microplate reader. The inhibition rate of the compound IR (%) = (RLU control -RLU compound)/(RLU control -RLU blank)×100% was calculated. The gradient dilution concentration of the compound and the corresponding cell proliferation inhibition rate were fitted by Prism 8 four-parameter method, and the IC₅₀ value was calculated. The compounds prepared in the above embodiments of the present disclosure have inhibitory activity of 0.0001 µM to 1 µM on the phosphorylated ERK level downstream of the cell channel of KRAS G12C protein mutant; some compounds have inhibitory activity of 0.001 µM to 1 µM or 0.001 µM to 0.5 µM on phosphorylated ERK level downstream of cell channel of KRAS G12C protein mutant. It can be seen from the results that the exemplary compounds of the present disclosure have good inhibitory activity on the level of phosphorylated ERK downstream of the cell channel of KRAS G12C protein mutant, with IC₅₀ lower than 10 µM; or lower than 1000 nM, or lower than 500 nM, or lower than 100 nM. Results for exemplary compounds are shown in Table 2 below.

**Table 2 Inhibitory activity of compounds on p-ERK**

| No. | p-ERK IC₅₀ (µM) | No. | p-ERK IC₅₀ (µM) | No. | p-ERK IC₅₀ (µM) | No. | p-ERK IC₅₀ (µM) |
|---|---|---|---|---|---|---|---|
| Z5 | 0.163 | Z327-1 | 0.066 | Z345-2 | 0.061 | Z479-1 | 0.030 |
| Z6 | 0.128 | Z327-2 | 0.172 | Z347-1 | 0.011 | Z480-1 | 0.013 |
| Z15 | 0.065 | Z328-1 | 0.015 | Z449-1 | 0.151 | Z481 | 0.013 |
| Z18 | 0.073 | Z331-1 | 0.006 | Z358-1 | 0.023 | Z503 | 0.042 |
| Z20 | 0.086 | Z331-2 | 0.007 | Z356 | 0.253 | Z635 | 0.016 |
| Z21 | 0.325 | Z332-1 | 0.130 | Z360-1 | 0.085 | Z635-1 | 0.014 |
| Z22 | 0.026 | Z334 | 0.062 | Z360-2 | 0.025 | Z635-2 | 0.055 |
| Z32 | 0.080 | Z335 | 0.130 | Z359-1 | 0.093 | Z547 | 0.135 |
| Z37 | 0.145 | Z336-2 | 0.063 | Z297-1 | 0.011 | Z608-1 | 1.221 |
| Z39 | 0.140 | Z337 | 0.154 | Z297-2 | 0.018 | Z637-1 | 1.121 |
| Z41 | 0.322 | Z240-1 | 0.019 | Z369 | 0.029 | Z525-1 | 1.276 |
| Z49 | 0.062 | Z240-2 | 0.020 | Z408-1 | 0.305 | Z637-2 | 0.068 |
| Z50 | 0.028 | Z241-1 | 0.031 | Z414-1 | 0.103 | Z525-2 | 0.226 |
| Z51 | 0.073 | Z241-2 | 0.029 | Z407-1 | 0.144 | Z502-1 | 0.196 |
| Z56 | 0.129 | Z243-1 | 0.008 | Z450 | 0.141 | Z608-2 | 0.286 |
| Z63 | 0.154 | Z243-2 | 0.108 | Z451 | 1.475 | Z502-2 | 0.124 |
| Z68 | 0.088 | Z315 | 0.062 | Z452 | 0.034 | Z506 | 0.011 |
| Z69 | 0.079 | Z241a | 0.012 | Z453-1 | 0.116 | Z483 | 0.260 |
| Z81 | 0.013 | Z244-1 | 0.031 | Z453-2 | 0.168 | Z491a | 0.037 |
| Z82 | 0.136 | Z244-2 | 0.005 | Z454 | 0.024 | Z491 | 0.027 |
| Z83 | 0.107 | Z241a-1 | 0.033 | Z455-1 | 0.479 | Z493 | 0.433 |
| Z237-2 | 0.030 | Z241a-2 | 0.009 | Z455-2 | 0.033 | Z532 | 0.085 |
| Z102-4 | 0.134 | Z316-1 | 0.020 | Z456 | 0.077 | Z653 | 0.184 |
| Z241 | 0.022 | Z256b-1 | 0.043 | Z454-1 | 0.013 | Z654-1 | 1.725 |
| Z237-1 | 0.030 | Z256a-1 | 0.042 | Z454-2 | 0.067 | Z654-2 | 0.167 |
| Z276-1 | 0.083 | Z256a-2 | 0.017 | Z457 | 0.397 | Z655-1 | 0.461 |
| Z312-1 | 0.194 | Z318-1 | 0.087 | Z458 | 6.515 | Z655-2 | 0.196 |
| Z313-1 | 0.023 | Z319-1 | 0.016 | Z458-1 | 3.562 | Z657 | 0.093 |
| Z296-1 | 0.008 | Z317-2 | 0.162 | Z462 | 0.165 | Z658 | 0.024 |
| Z240 | 0.022 | Z320-1 | 0.017 | Z463 | 0.271 | Z659 | 0.026 |
| Z314 | 0.062 | Z296-2 | 0.032 | Z464 | 0.523 | Z660-1 | 0.019 |
| Z147 | 0.180 | Z321 | 0.050 | Z467 | 0.011 | Z660-2 | 0.091 |
| Z259 | 0.195 | Z322 | 0.036 | Z465-1 | 0.137 | Z661 | 0.029 |
| Z260 | 0.091 | Z291-1S | 0.089 | Z406-1 | 0.073 | Z659-1 | 0.249 |
| Z235 | 0.138 | Z291-2 | 0.024 | Z468-1 | 0.082 | Z659-2 | 0.022 |
| Z261 | 0.059 | Z260a | 0.022 | Z468-2 | 0.061 | Z661-1 | 0.057 |
| Z257 | 0.104 | Z259a | 0.027 | Z469-1 | 0.008 | Z661-2 | 0.538 |
| Z308a | 0.021 | Z324-1 | 0.015 | Z469-2 | 0.030 | Z658-1 | 0.031 |
| Z236 | 0.025 | Z324-2 | 0.034 | Z470-1 | 0.255 | Z658-2 | 0.180 |
| Z221 | 0.023 | Z308a-1 | 0.071 | Z471-1 | 0.188 | Z662-1 | 0.0326 |
| Z256 | 0.043 | Z325-2 | 0.061 | Z471-2 | 1.079 | Z662-2 | 0.5860 |
| Z39-2 | 0.088 | Z338-1 | 0.046 | Z470-2 | 0.024 | | |
| Z244 | 0.019 | Z339 | 0.143 | Z472-1 | 0.189 | | |
| Z243 | 0.019 | Z340-1 | 0.059 | Z472-2 | 0.108 | | |
| Z256-1 | 0.020 | Z341-1 | 0.106 | Z473-1 | 0.151 | | |
| Z257a | 0.026 | Z342 | 0.084 | Z473-2 | 0.161 | | |
| Z221-2 | 0.173 | Z343 | 0.119 | Z474 | 0.090 | | |
| Z221-1 | 0.019 | Z275 | 0.016 | Z475-1 | 0.035 | | |
| Z310 | 0.104 | Z344 | 0.010 | Z475-2 | 0.107 | | |
| Z279-2 | 0.035 | | | Z477 | 0.061 | | |
| Z311-1 | 0.070 | | | Z474-1 | 0.064 | | |
| Z326 | 0.006 | | | | | | |

### Test embodiment 3 Cell proliferation inhibition experiment

Raw materials and instruments: MIA PaCa-2 purchased from ATCC; article number: CRL-1420; batch number: 63143594; DMEM purchased from Gibco; article number: 11995-065; batch number: 2186878; FBS purchased from Gibco; article number: 10099-141; batch number: 2109290CP; Horse Serum purchased from Gibco; article number: 16050-122; batch number: 1818976; PS purchased from Gibco; article number: 15140-122; batch number: 2145451; Trypsin-EDTA purchased from Gibco; article number: 25200-072; batch number: 2085552; CellTiter Glo working solution purchased from Promega; article number: G9683; batch number: 398478; DMSO purchased from VWR AMRESCO; article number: 0231-500ML; batch number: 18C0656168; 384-well spheroid plate purchased from Coming; article number: 3830; batch number: 27519032; white opaque 384-well plate purchased from Coming; article number: 3570; batch number: 26819023; microplate reader purchased from PerkinElmer; model: Envision MLD2105-0020; equipment number 1050369.

MIA PaCa-2 is a KRAS G12C mutant human pancreatic cancer cell line, cultured in 10% FBS + 2.5% Horse Serum DMEM + 1% PS culture medium; cells at logarithmic growth stage were taken, digested with trypsin-EDTA, then collected and counted, and 200 MIA PaCa-2 were inoculated in a 384-well spheroid plate, respectively, and cultured overnight to establish a 3D cell model. A 3.16-fold gradient concentration stock solution of 1000X compound was prepared with DMSO, and diluted 100-fold to 10X compound stock solution with culture medium. On the second day after cell inoculation, 10X compound stock solution was added to each cell culture well, with a final concentration of 1X and a DMSO content of 0.1%. DMSO was used as experimental control (control), and culture medium was used as blank control (blank). After 5 days of compound cell culture, 30 µL of CellTiter-Glo working solution was added to each well, mixed well and incubated for 30 minutes. After standing at room temperature for 30 minutes, 40 µL of the mixed solution was transferred to a white opaque 384-well plate, and the luminescence chemiluminescence value was read with a microplate reader. The cell proliferation inhibition rate IR (%) = (RLU control -RLU compound)/(RLU control -RLU blank)×100% was calculated. The gradient dilution concentration of the compound and the corresponding cell proliferation inhibition rate were fitted by XLFit four-parameter method, and the IC₅₀ value was calculated. The inhibitory activity of the compounds prepared in the above embodiments of the present disclosure on KRAS G12C mutant MIA PaCa-2 cells was 0.0001 µM to 0.5 µM; the inhibitory activity of some compounds on KRAS G12C mutant MIA PaCa-2 cells was 0.001 µM to 0.1 µM or 0.001 µM to 0.05 µM. It can be seen from the results that the exemplary compounds of the present disclosure have good inhibitory activity on KRAS G12C mutant MIA PaCa-2 cells, with IC₅₀ lower than 1000 nM; or lower than 500 nM, or lower than 100 nM, or lower than 10 nM. Results for exemplary compounds are shown in Table 3 below.

**Table 3 Inhibitory activity of compounds on MIA-PaCa2**

| Compound No. | MIA-PaCa2 IC₅₀ (µM) | Compound No. | MIA-PaCa2 IC₅₀ (µM) | Compound No. | MIA-PaCa2 IC₅₀ (µM) | Compound No. | MIA-PaCa2 IC₅₀ (µM) |
|---|---|---|---|---|---|---|---|
| Z5 | 0.143 | Z308a | 0.0017 | Z341-1 | 0.0240 | Z474-1 | 0.013 |
| Z6 | 0.017 | Z236 | 0.0079 | Z342 | 0.0160 | Z479-1 | 0.015 |
| Z8 | 0.232 | Z221 | 0.0045 | Z343 | 0.0180 | Z480-1 | 0.010 |
| Z9 | 0.095 | Z256 | 0.0108 | Z275 | 0.0050 | Z481 | 0.004 |
| Z10 | 0.067 | Z39-2 | 0.0130 | Z344 | 0.0030 | Z503 | 0.007 |
| Z11 | 0.064 | Z244 | 0.0070 | Z345-2 | 0.0170 | Z635 | 0.006 |
| Z15 | 0.015 | Z243 | 0.0042 | Z347-1 | 0.0040 | Z547 | 0.043 |
| Z19 | 0.063 | Z256-1 | 0.0050 | Z449-1 | 0.0220 | Z608-1 | 0.3305 |
| Z20 | 0.008 | Z257a | 0.0090 | Z358-1 | 0.0072 | Z637-1 | 0.5490 |
| Z21 | 0.065 | Z221-1 | 0.0088 | Z356 | 0.029 | Z525-1 | 0.4777 |
| Z22 | 0.007 | Z310 | 0.0260 | Z360-1 | 0.0196 | Z637-2 | 0.010 |
| Z23 | 0.073 | Z239-1 | 0.0200 | Z360-2 | 0.006 | Z525-2 | 0.014 |
| Z24 | 0.036 | Z279-2 | 0.0049 | Z359-1 | 0.0176 | Z502-2 | 0.0241 |
| Z25 | 0.081 | Z311-1 | 0.0120 | Z297-1 | 0.003 | Z506 | 0.0022 |
| Z27 | 0.041 | Z83 | 0.0374 | Z297-2 | 0.003 | Z491a | 0.010 |
| Z28 | 0.150 | Z237-2 | 0.0077 | Z369 | 0.006 | Z491 | 0.007 |
| Z29 | 0.077 | Z241 | 0.0049 | Z408-1 | 0.0793 | Z493 | 0.112 |
| Z30 | 0.041 | Z276-1 | 0.0149 | Z414-1 | 0.1265 | Z532 | 0.0245 |
| Z32 | 0.011 | Z313-1 | 0.0026 | Z407-1 | 0.039 | Z653 | 0.053 |
| Z264 | 0.098 | Z296-1 | 0.0011 | Z450 | 0.034 | Z654-2 | 0.0502 |
| Z34 | 0.081 | Z314 | 0.0099 | Z451 | 0.3630 | Z655-1 | 0.1132 |
| Z35 | 0.036 | Z240-1 | 0.0037 | Z452 | 0.0112 | Z655-2 | 0.0509 |
| Z36 | 0.057 | Z240-2 | 0.0033 | Z453-1 | 0.014 | Z658 | 0.0112 |
| Z37 | 0.012 | Z241-1 | 0.0047 | Z453-2 | 0.0452 | Z659 | 0.0093 |
| Z38 | 0.088 | Z241-2 | 0.0033 | Z454 | 0.0047 | Z660-1 | 0.0077 |
| Z39 | 0.020 | Z243-1 | 0.0015 | Z455-1 | 0.0869 | Z660-2 | 0.0470 |
| Z40 | 0.057 | Z243-2 | 0.0222 | Z455-2 | 0.0092 | Z661 | 0.0131 |
| Z41 | 0.073 | Z315 | 0.0096 | Z456 | 0.0228 | Z659-1 | 0.016 |
| Z42 | 0.087 | Z241a | 0.0024 | Z454-1 | 0.0035 | Z659-2 | 0.004 |
| Z43 | 0.034 | Z244-1 | 0.0080 | Z454-2 | 0.0168 | Z661-1 | 0.014 |
| Z44 | 0.051 | Z244-2 | 0.0010 | Z457 | 0.5223 | Z661-2 | 0.004 |
| Z45 | 0.060 | Z241a-1 | 0.0043 | Z458 | >1 | Z658-2 | 0.031 |
| Z47 | 0.042 | Z241a-2 | 0.0015 | Z459 | >1 | Z662-1 | 0.011 |
| Z48 | 0.028 | Z316-1 | 0.0022 | Z458-1 | >1 | | |
| Z49 | 0.007 | Z256b-1 | 0.0062 | Z458-2 | >1 | | |
| Z50 | 0.002 | Z256b-2 | 0.1053 | Z461 | 0.033 | | |
| Z51 | 0.007 | Z256a-1 | 0.0116 | Z462 | 0.016 | | |
| Z52 | 0.039 | Z256a-2 | 0.0038 | Z463 | 0.059 | | |
| Z53 | 0.292 | Z318-1 | 0.0109 | Z464 | 0.1792 | | |
| Z54 | 0.169 | Z320-1 | 0.0017 | Z467 | 0.001 | | |
| Z55 | 0.361 | Z296-2 | 0.0080 | Z406-1 | 0.010 | | |
| Z56 | 0.071 | Z321 | 0.0070 | Z468-1 | 0.016 | | |
| Z57 | 0.118 | Z322 | 0.0063 | Z468-2 | 0.012 | | |
| Z58 | 0.237 | Z291-1S | 0.0307 | Z469-1 | 0.0006 | | |
| Z59 | 0.027 | Z291-2 | 0.0070 | Z469-2 | 0.004 | | |
| Z60 | 0.094 | Z260a | 0.0036 | Z470-1 | 0.043 | | |
| Z61 | 0.026 | Z259a | 0.0044 | Z471-1 | 0.042 | | |
| Z62 | 0.029 | Z324-1 | 0.0024 | Z470-2 | 0.004 | | |
| Z63 | 0.023 | Z324-2 | 0.0061 | Z472-1 | 0.074 | | |
| Z64 | 0.268 | Z308a-1 | 0.0078 | Z472-2 | 0.053 | | |
| Z65 | 0.038 | Z325-2 | 0.0155 | Z473-2 | 0.045 | | |
| Z68 | 0.018 | Z326 | 0.0009 | Z474 | 0.017 | | |
| Z69 | 0.028 | Z327-1 | 0.0071 | Z475-1 | 0.005 | | |
| Z70 | 0.052 | Z327-2 | 0.0162 | Z475-2 | 0.015 | | |
| Z71 | 0.467 | Z328-1 | 0.0017 | | | | |
| Z75 | 0.067 | Z331-1 | 0.0011 | | | | |
| Z76 | 0.034 | Z331-2 | 0.0013 | | | | |
| Z77 | 0.365 | Z332-1 | 0.0170 | | | | |
| Z247 | 0.0470 | Z333-1 | 0.0300 | | | | |
| Z260 | 0.0044 | Z334 | 0.0110 | | | | |
| Z308 | 0.0340 | Z335 | 0.0180 | | | | |
| Z235 | 0.0228 | Z337 | 0.0160 | | | | |
| Z239 | 0.0416 | Z338-1 | 0.0090 | | | | |
| Z261 | 0.0106 | Z340-1 | 0.0110 | | | | |
| Z257 | 0.0113 | Z340-2 | 0.0740 | | | | |

### Test embodiment 4 KRAS G12C NEA-HTRF experiment

Raw materials and instruments: KRAS G12C (his-tag) purchased from Reactionbiology; batch number: 3496; SOS1 purchased from Reactionbiology; batch number: 3501; GTP purchased from Sigma; article number: G8877; batch number: SLBV1450; Tris-HCl purchased from Sigma; EDTA purchased from absin; article number: abs42017668; batch number: JE20; NaCl purchased from Sigma; article number: S5886; batch number: SLBW4193; MgCl₂ purchased from Shyuanye; article number: R21455; batch number: L07S9G69806; HEPES purchased from Life Technologies; article number: 15630-080; batch number: 2037042; DTT purchased from Thermo Fisher; article number: R0862; batch number: 00608918; Triton X-100 purchased from Sigma; article number: T8787-100 ML; batch number: SLBW7103; DY647-labeled GDP purchased from Jena Bioscience; article number: NU-840-647P1; batch number: BM019-045; Anti-6HIS-Tb purchased from Cisbio; article number: 61H12TLA; batch number: 13A; Bradford Assay Kit purchased from Thermo Fisher; article number: 23246; batch number: UG287474; white opaque 96-well plate purchased from Cisbio; article number: 66PL96025; batch number: N^{O}06; NAP-5 column purchased from GE Healthcare; article number: 17085301; batch number: 16995164; mircoplate reader purchased from PerkinElmer; model: Envision MLD2105-0020; equipment number 1050369.

The effect of compounds on SOS1-catalyzed displacement of GDP by GTP on KRAS protein was examined using a homogeneous time-resolved fluorescence (HTRF) method. 30 µM 6×his-tagged KRAS G 12C recombinant protein was incubated with 80 µM fluorescent dye DY647-labeled GDP in labeling buffer (1 mM DTT, 7.5 mM EDTA, 25 mM Tris-HCl, 45 mM NaCl) at 20°C in the dark for 2 hours, purified using NAP-5 column, and then protein quantification was performed with Bradford Assay Kit after purification to determine the concentration of KRAS G12C-GDP.

A 3.16-fold gradient concentration stock solution of 1000X compound was prepared with DMSO, and diluted 250-fold to 4× compound stock solution with reaction buffer (40 mM HEPES, 10 mM MgCl₂, 1 mM DTT, 0.002% Triton X-100). KRAS G12C-GDP/Tb working solution (40 nM KRAS G12C-GDP, 1×anti-6his Tb) and SOS1/GTP working solution (0.2 µM SOS1, 200 µM GTP) were prepared using the reaction buffer. A white opaque 96-well plate was taken, and 5 µL of 4× compound stock solution and 10 µL of KRAS G12C-GDP/Tb working solution were added to each well. 5 µL of reaction buffer instead of 4× compound stock solution was used in the control well. After incubation in the dark for 15 minutes at 20°C, 5 µL of SOS1/GTP working solution was added thereto, and the fluorescence value (excitation wavelength: 320 nm, emission wavelength: 615 nm and 665 nm) was read after incubating at 20°C in the dark for 2 hours. Another T0 group was set up with 10 µL reaction buffer + 10 µL KRAS G12C-GDP/Tb working solution, and the fluorescence value was read directly by the microplate reader. The fluorescence signal ratio RLU = (665 nm signal /615 nm signal) × 10⁴; and the compound inhibition rate IR (%) = (RLU_{compound} -RLU_{Control})/(RLU_{T0}-RLU_{control}) × 100% were calculated. The IC₅₀ value was calculated by fitting the gradient dilution concentration of compound and the corresponding inhibition rate with the four-parameter method. The NEA activity of the compounds prepared in the above embodiments of the present disclosure was 0.0001 µM to 1 µM; the inhibitory activity of some compounds on KRAS G12C mutant MIA PaCa-2 cells was 0.001 µM to 0.5 µM or 0.001 µM to 0.1 µM.

| No. | NEA IC₅₀ (µM) | No. | NEA IC₅₀ (µM) | No. | NEA IC₅₀ (µM) | No. | NEA IC₅₀ (µM) |
|---|---|---|---|---|---|---|---|
| Z22 | 0.020 | Z275 | 0.021 | Z369 | 0.0686 | Z503 | 0.1758 |
| Z28 | 0.113 | Z276-1 | 0.113 | Z408-1 | 0.1594 | Z635 | 0.0314 |
| Z32 | 0.142 | Z277-1 | 0.033 | Z414-1 | 0.0866 | Z635-1 | 0.0227 |
| Z37 | 0.167 | Z279-2 | 0.029 | Z407-1 | 0.2621 | Z635-2 | 0.1131 |
| Z39-2 | 0.066 | Z291-1S | 0.076 | Z450 | 0.1469 | Z547 | 0.1391 |
| Z43 | 0.171 | Z291-2 | 0.018 | Z451 | 2.9975 | Z608-1 | 0.6099 |
| Z48 | 0.150 | Z296-1 | 0.007 | Z452 | 0.0469 | Z637-1 | 0.3434 |
| Z49 | 0.066 | Z296-2 | 0.039 | Z453-1 | 0.0436 | Z525-1 | 0.3645 |
| Z50 | 0.021 | Z308a-1 | 0.058 | Z453-2 | 0.1197 | Z637-2 | 0.0238 |
| Z51 | 0.142 | Z308 | 0.167 | Z454 | 0.0198 | Z525-2 | 0.0385 |
| Z56 | 0.085 | Z308a | 0.111 | Z455-1 | 0.6921 | Z502-1 | 0.2796 |
| Z61 | 0.176 | Z310 | 0.036 | Z455-2 | 0.0463 | Z608-2 | 0.0758 |
| Z68 | 0.088 | Z311-1 | 0.034 | Z456 | 0.1467 | Z502-2 | 0.1597 |
| Z69 | 0.079 | Z312-1 | 0.134 | Z454-1 | 0.0093 | Z506 | 0.0183 |
| Z75 | 0.138 | Z313-1 | 0.013 | Z454-2 | 0.0355 | Z483 | 0.3403 |
| Z76 | 0.173 | Z314 | 0.043 | Z457 | 0.1334 | Z491a | 0.0150 |
| Z81 | 0.006 | Z315 | 0.033 | Z458 | 0.0139 | Z491 | 0.015 |
| Z82 | 0.044 | Z316-1 | 0.009 | Z459 | 9.4142 | Z493 | 0.125 |
| Z83 | 0.111 | Z318-1 | 0.037 | Z458-1 | 0.0088 | Z532 | 0.0440 |
| Z93-1 | 0.153 | Z319-1 | 0.009 | Z458-2 | 0.2524 | Z653 | 0.2984 |
| Z102-4 | 0.103 | Z320-1 | 0.009 | Z461 | 0.3309 | Z654-1 | 1.8207 |
| Z102-1 | 0.194 | Z321 | 0.078 | Z462 | 0.1314 | Z654-2 | 0.1677 |
| Z221 | 0.036 | Z322 | 0.062 | Z463 | 0.7597 | Z655-1 | 0.4056 |
| Z221-2 | 0.139 | Z324-1 | 0.016 | Z464 | 1.9319 | Z655-2 | 0.1281 |
| Z221-1 | 0.014 | Z324-2 | 0.061 | Z465 | 0.2747 | Z656 | >10 |
| Z236 | 0.113 | Z325-2 | 0.035 | Z466-1 | 0.7642 | Z657 | 0.0315 |
| Z237-1 | 0.122 | Z326 | 0.008 | Z466-2 | 0.3832 | Z658 | 0.0284 |
| Z237-2 | 0.065 | Z327-1 | 0.026 | Z467 | 0.0077 | Z659 | 0.0194 |
| Z239-1 | 0.124 | Z327-2 | 0.064 | Z465-1 | 0.1442 | Z660-1 | 0.0113 |
| Z240 | 0.010 | Z328-1 | 0.015 | Z465-2 | >10 | Z660-2 | 0.0505 |
| Z240-1 | 0.011 | Z330-1 | 0.226 | Z406-1 | 0.0310 | Z661 | 0.0329 |
| Z240-2 | 0.011 | Z331-1 | 0.005 | Z406-2 | 0.1748 | Z659-1 | 0.0772 |
| Z241 | 0.018 | Z331-2 | 0.007 | Z468-1 | 0.0350 | Z659-2 | 0.0093 |
| Z241-1 | 0.017 | Z332-1 | 0.130 | Z468-2 | 0.0465 | Z661-1 | 0.0335 |
| Z241-2 | 0.011 | Z333-1 | 0.191 | Z469-1 | 0.005 | Z661-2 | 0.5112 |
| Z241a | 0.009 | Z334 | 0.079 | Z469-2 | 0.018 | Z658-1 | 0.0238 |
| Z241a-1 | 0.013 | Z335 | 0.078 | Z470-1 | 0.2585 | Z658-2 | 0.1420 |
| Z241a-2 | 0.005 | Z336-2 | 0.027 | Z471-1 | 0.0730 | Z662-1 | 0.0234 |
| Z243-1 | 0.007 | Z337 | 0.058 | Z471-2 | 0.4611 | Z662-2 | 0.2349 |
| Z243-2 | 0.076 | Z338-1 | 0.039 | Z470-2 | 0.0247 | | |
| Z243 | 0.023 | Z340-1 | 0.027 | Z472-1 | 0.1447 | | |
| Z244 | 0.047 | Z340-2 | 0.098 | Z472-2 | 0.0600 | | |
| Z244-1 | 0.026 | Z341-1 | 0.035 | Z473-1 | 0.1051 | | |
| Z244-2 | 0.005 | Z342 | 0.074 | Z473-2 | 0.0768 | | |
| Z248-2 | 0.169 | Z343 | 0.101 | Z474 | 0.1051 | | |
| Z256 | 0.064 | Z344 | 0.010 | Z475-1 | 0.0225 | | |
| Z256-1 | 0.026 | Z345-2 | 0.041 | Z475-2 | 0.0793 | | |
| Z256a-1 | 0.041 | Z347-1 | 0.012 | Z476 | 8898 | | |
| Z256a-2 | 0.018 | Z449-1 | 0.031 | Z477 | 0.057 | | |
| Z256b-1 | 0.033 | Z449-2 | 0.3885 | Z478 | 0.423 | | |
| Z257 | 0.093 | Z358-1 | 0.0264 | Z474-1 | 0.089 | | |
| Z257a | 0.076 | Z356 | 0.1611 | Z474-2 | 0.593 | | |
| Z259 | 0.113 | Z360-1 | 0.0981 | Z479-1 | 0.0652 | | |
| Z259a | 0.026 | Z360-2 | 0.0369 | Z480-1 | 0.0179 | | |
| Z260 | 0.033 | Z359-1 | 0.0838 | Z480-2 | 0.321 | | |
| Z260a | 0.027 | Z297-1 | 0.0190 | Z481 | 0.042 | | |
| Z261 | 0.150 | Z297-2 | 0.008 | Z479-2 | 0.431 | | |

Although the specific embodiments of the present disclosure have been described in detail, according to all the disclosed teachings, those skilled in the art can make various modifications and substitutions to the details of the technical solutions of the present disclosure, and these changes are all within the protection scope of the present disclosure. The full scope of the present disclosure is given by the appended claims and any equivalents thereof.

## Claims

1. A compound of formula (I), a pharmaceutically acceptable salt thereof, a stereoisomer thereof, a solvate thereof or a prodrug thereof: 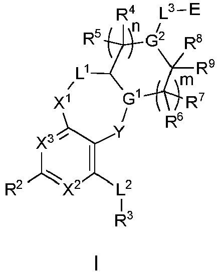 in the formula,
X¹ is O, S, NR^{x1} or CR^{x2}R^{x3}; wherein, R^{x1} is hydrogen or C₁₋₆ alkyl; R^{x2}, R^{x3} are each independently hydrogen, deuterium, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy;
X² is N or CR^{x4}; wherein, R^{x4} is hydrogen, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy;
X³ is N or CR¹; wherein, R¹ is hydrogen, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, C₂₋₆ alkenyl, C₂₋₆ alkynyl, -C₁₋₄ alkyl-hydroxyl, -C₁₋₄ alkyl-cyano, -C₁₋₄ alkyl-C₁₋₆ alkoxy, -C₁₋₄ alkyl-C₃₋₆ cycloalkoxy, -C₁₋₄ alkyl-halo C₁₋₆ alkyl, -C₁₋₄ alkyl-halo C₁₋₆ alkoxy or -C₁₋₄ alkyl-NR^{a}R^{b};
L¹ is a bond, (CR^{L1}R^{L2})ₜ, C(=O), C(=O)C(R^{L1}R^{L2}) or C(R^{L1}R^{L2})C(=O); wherein, R^{L1}, R^{L2} are each independently hydrogen, deuterium, halogen or C₁₋₆ alkyl; t is 1 or 2;
L³ is a bond, NR^{L6} or C₁₋₆ alkyl; wherein, R^{L6} is hydrogen, cyano, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R² is C₆₋₁₄ aryl, 5- or 6-membered monocyclic heteroaryl or 8- to 10-membered bicyclic heteroaryl; wherein, the 5- or 6-membered monocyclic heteroaryl has 1, 2 or 3 heteroatoms selected from N, O and S as ring atoms; the 8- to 10-membered bicyclic heteroaryl has 1, 2, 3, 4 or 5 heteroatoms selected from N, O and S as ring atoms; and the C₆₋₁₄ aryl, the 5- or 6-membered monocyclic heteroaryl or the 8- to 10-membered bicyclic heteroaryl is each independently unsubstituted or substituted by 1, 2, 3 or 4 groups independently selected from Rₛ₁;
or
R² is a structure shown in formula (B): 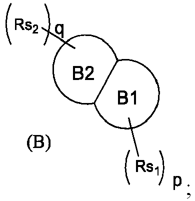
wherein, ring B1 is a benzene ring or 5- or 6-membered monocyclic heteroaryl ring; ring B2 is a fused 5- or 6-membered monocyclic heterocycloalkyl ring or fused 5- or 6-membered monocyclic cycloalkyl ring; wherein, the 5- or 6-membered monocyclic heteroaryl ring or the fused 5- or 6-membered monocyclic heterocycloalkyl ring has 1, 2 or 3 heteroatoms selected from N, O and S as ring atoms;
(Rₛ₁)ₚ means that hydrogen on ring B1 is substituted by p Rₛ₁, p is 0, 1, 2 or 3, each Rₛ₁ is the same or different;
(Rₛ₂)_{q} means that hydrogen on ring B2 is substituted by q Rₛ₂, q is 0, 1, 2 or 3, each Rₛ₂ is the same or different;
Rₛ₁, Rₛ₂ are each independently oxo (=O), halogen, cyano, nitro, hydroxyl, -C₁₋₆ alkyl, -C₁₋₆ alkoxy, - halo C₁₋₆ alkyl, -deuterated C₁₋₆ alkyl, -halo C₁₋₆ alkoxy, -deuterated C₁₋₆ alkoxy, -C₃₋₆ monocyclic cycloalkyl, -NR^{c}R^{d}, -C(O)NR^{c}R^{d}, -SO₂C₁₋₃ alkyl, -SOzhalo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; wherein, the 3- to 6-membered heterocyclyl has 1, 2 or 3 heteroatoms selected from N, O and S as ring atoms;
G¹, G² are each independently N or CH;
1) when G¹ is N,
Y is -C(=O)-, -C(=S)-, -S(=O)z- or -S(=O)-;
L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1; or
2) when G¹ is N,
Y is -C(R^{L7}R^{L8})-; wherein, R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
L² is O, S or NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1; or
3) when G¹ is N,
Y is -C(R^{L7}R^{L8})-; wherein, R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
L² is NH(C=O), S(=O)₂ or C(=O);
R³ is C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; and when R³ is NR^{e}R^{f}, R^{e}, R^{f} are not simultaneously H; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1; or
4) when G¹ is N,
Y is -C(R^{L7}R^{L8})-; wherein, R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl;
L² is a bond or (CR^{L3}R^{L4})ₘ₁; wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy or -C₁₋₄ alkyl-NR^{a}R^{b}; or R^{L3}, R^{L4} combining with the carbon atoms to which they are attached form a C₃₋₆ monocyclic cycloalkyl;
R³ is NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1; or
5) when G¹ is CH,
Y is NR^{L9}, O or S; wherein, R^{L9} is hydrogen, cyano, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
L² is a bond, O, S, CR^{L3}R^{L4}, NR^{L5}, NH(C=O), S(=O)z or C(=O); wherein, R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy or -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1;
the substituents of each group S1 are each independently oxo (=O), halogen, cyano, nitro, hydroxyl, carboxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, C₆₋₁₄ aryl, 5- or 6-membered monocyclic heteroaryl, C₃₋₆ monocyclic cycloalkoxy, -O-3- to 6-membered monocyclic heterocyclyl, -O-5- to 11-membered fused cycloalkyl, -O-5- to 11-membered fused heterocyclyl, -O-6- to 11-membered spirocycloalkyl, -O-7- to 11-membered spiroheterocyclyl, -O-5- to 11-membered bridged cycloalkyl, -O-5- to 11-membered bridged heterocyclyl, -O-C₆₋₁₄ aryl, -O-5- or 6-membered monocyclic heteroaryl, NR^{c}R^{d}, C(O)C₁₋₆ alkyl, C(O)C₂₋₆ alkenyl, C(O)C₃₋₆ monocyclic cycloalkyl, C(O)NR^{e}R^{d}, -SO₂C₁₋₃ alkyl, -SOzhalo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, - C₁₋₄ alkyl-deuterated C₁₋₆ alkoxy, -C₁₋₄ alkyl-3- to 6-membered monocyclic heterocyclyl, -C₁₋₄ alkyl-NR^{c}R^{d}, -O-C₁₋₄ alkyl-NR^{c}R^{d}, -C₁₋₄ alkyl-C(O)NR^{c}R^{d}, -C₁₋₄ alkyl-SO₂C₁₋₃ alkyl, carboxyl, -C₁₋₄ alkyl-carboxyl, C₂₋₆ alkenyl or C₂₋₆ alkynyl; the 3- to 6-membered monocyclic heterocyclyl or 5- or 6-membered monocyclic heteroaryl have 1 or 2 heteroatoms selected from N, O and S as ring atoms; the 3- to 6-membered monocyclic heterocyclyl or 5- or 6-membered monocyclic heteroaryl is optionally substituted by 1 or 2 groups selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl;
m, n are each independently selected from 0, 1, 2 and 3;
in m R⁶, R⁷, R⁶, R⁷ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or one or more pairs of R⁶, R⁷ combining with the carbon atoms to which they are attached form a C₃₋₆ monocyclic cycloalkyl;
in n R⁴, R⁵, R⁴, R⁵ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or one or more pairs of R⁴, R⁵ combining with the carbon atoms to which they are attached form a C₃₋₆ monocyclic cycloalkyl; R⁸, R⁹ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or R⁸, R⁹ combining with the carbon atoms to which they are attached form a C₃₋₆ monocyclic cycloalkyl; or
in n R⁴, R⁵, one R⁴ combining with R⁸ to form (CH₂)ₙ₁; n1 is 1, 2, 3 or 4; R⁹ is hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; R⁵ and the rest R⁴ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy; or one or more of the rest R⁴ and R⁵ combining with the same carbon atom each independently form a C₃₋₆ monocyclic cycloalkyl together with the attached carbon atom; the rest R⁵, R⁴ are each independently hydrogen, deuterium, C₁₋₆ alkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl or -C₁₋₄ alkyl-halo C₁₋₆ alkoxy;
E is an electrophilic addition fragment that is able to form a covalent bond with the cysteine at position 12 of KRAS, HRAS, and NRAS G12C mutant proteins;
the R^{a}, R^{b} are each independently H or C₁₋₆ alkyl or R^{a}, R^{b} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms;
the R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by - CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl.

2. The compound, the pharmaceutically acceptable salt thereof, the stereoisomer thereof, the solvate thereof or the prodrug thereof as claimed in claim 1, wherein, E is 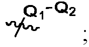 wherein, Q₁ is C(=O) or S(=O)₂; Q₂ is 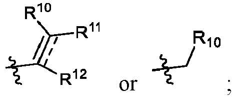 wherein, 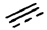 refers to a double bond or triple bond; R¹⁰, R¹¹, R¹² are each independently hydrogen, halogen, cyano, C₁₋₃ alkyl or N(C₁₋₃ alkyl)₂; E is preferably 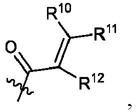 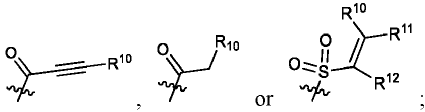 wherein, R¹⁰, R¹¹, R¹² are each independently hydrogen, halogen, cyano, C₁₋₃ alkyl or N(C₁₋₃ alkyl)₂; E is more preferably 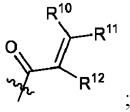 wherein, R¹² is hydrogen, halogen or cyano; R¹⁰, R¹¹ are each independently hydrogen or N(C₁₋₃ alkyl)₂; or E is more preferably 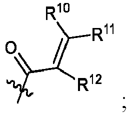 wherein, R¹⁰, R¹¹, R¹² are hydrogen;
and/or, L³ is a bond;
and/or, G¹ and G² are N;
and/or, L¹ is (CR^{L1}R^{L2})ₜ; wherein, R^{L1}, R^{L2} are each independently hydrogen, deuterium, halogen or C₁₋₆ alkyl; t is 1 or 2; L¹ is preferably CH₂ or CD₂; more preferably CH₂;
and/or, X¹ is O, S, NR^{x1} or CR^{x2}R^{x3}; wherein, R^{x1} is hydrogen, C₁₋₃ alkyl or deuterated C₁₋₃ alkyl; R^{x2}, R^{x3} are each independently hydrogen, deuterium, halogen, cyano, C₁₋₃ alkyl, deuterated C₁₋₃ alkyl, C₁₋₃ alkoxy, halo C₁₋₃ alkyl or halo C₁₋₃ alkoxy;
and/or, X² is N or CR^{x4}; wherein, R^{x4} is hydrogen, halogen, cyano, C₁₋₃ alkyl, deuterated C₁₋₃ alkyl, halo C₁₋₃ alkyl or cyclopropyl;
and/or, X³ is N or CR¹; wherein, R¹ is hydrogen, halogen, cyano, C₁₋₆ alkyl, deuterated C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic cycloalkoxy, NR^{a}R^{b}, C₂₋₆ alkenyl, C₂₋₆ alkynyl, -C₁₋₄ alkyl-hydroxyl, -C₁₋₄ alkyl-cyano, -C₁₋₄ alkyl-C₁₋₆ alkoxy, -C₁₋₄ alkyl-C₃₋₆ cycloalkoxy, -C₁₋₄ alkyl-halo C₁₋₆ alkyl, -C₁₋₄ alkyl-halo C₁₋₆ alkoxy or -C₁₋₄ alkyl-NR^{a}R^{b}; R¹ is preferably hydrogen, halogen, cyano, C₁₋₃ alkyl, deuterated C₁₋₃ alkyl, C₁₋₃ alkoxy, halo C₁₋₃ alkyl, halo C₁₋₃ alkoxy or cyclopropyl; more preferably hydrogen or halogen;
and/or, L² is a bond, O, S, (CR^{L3}R^{L4})ₘ₁, NR^{L5}, S(=O)z or C(=O); wherein, R^{L3}, R^{L4} are each independently hydrogen, deuterium, C₁₋₃ alkyl, deuterated C₁₋₃ alkyl, halo C₁₋₃ alkyl or cyclopropyl; or R^{L3}, R^{L4} combining with the carbon atoms to which they are attached form a cyclopropyl; m1 is 1, 2 or 3; R^{L5} is hydrogen, C₁₋₃ alkyl or deuterated C₁₋₃ alkyl;
and/or, m and n are 1;
and/or, R⁴, R⁵, R⁶, R⁷, R⁸, R⁹ are each independently hydrogen or deuterium;
and/or, in R², the C₆₋₁₄ aryl is phenyl, naphthyl, 9- or 10-membered phenyl-heterocyclyl or 9- or 10-membered phenyl-cycloalkyl; preferably phenyl or naphthyl; more preferably phenyl;
and/or, in R², the 5- or 6-membered monocyclic heteroaryl is selected from the following groups: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine and pyrazine;
and/or, in R², the 8- to 10-membered bicyclic heteroaryl is a bicyclic group formed by a monocyclic aryl ring fused with a monocyclic heteroaryl ring, a bicyclic group formed by a monocyclic heteroaryl ring fused with a monocyclic heteroaryl ring, 8- to 10-membered heteroaryl-heterocyclyl or 8- to 10-membered heteroaryl-cycloalkyl; the 8- to 10-membered bicyclic heteroaryl is preferably selected from the following groups: benzo[*d*]isoxazole, 1*H*-indole, isoindole, 1*H*-benzo[*d*]imidazole, benzo[*d*]isothiazole, 1*H-*benzo[*d*][1,2,3]triazole, benzo[*d*]oxazole, benzo[*d*]thiazole, indazole, benzofuran, benzo[*b*]thiophene, quinoline, isoquinoline, quinazoline, quinoxaline, cinnoline, pyrido[3,2-*d*]pyrimidine, pyrido[2,3-*d*]pyrimidine, pyrido[3,4-*d*]pyrimidine, pyrido[4,3-*d*]pyrimidine, 1,8-naphthyridine, 1,7-naphthyridine, 1,6-naphthyridine, 1,5-naphthyridine, pyrazolo[1,5-*a*]pyrimidine, imidazo[1,2-*b*]pyridazine, 1,4,5,6-tetrahydropyrrolo[3,4-*c*]pyrazole, 2,4,5,6-tetrahydropyrrolo[3,4-*c*]pyrazole, 5,6,7,8-tetrahydro-[1,2,4]triazolo[1,5-*a*]pyrazine, 4,5,6,7-tetrahydro-[1,2,3]triazolo[1,5-*a*]pyrazine, 4,5,6,7-tetrahydro-pyrazolo[1,5-*α*]pyrazine, more preferably 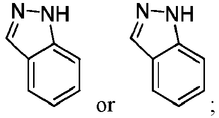
and/or, in R³, the C₆₋₁₄ aryl is phenyl, naphthyl, 9- or 10-membered phenyl-heterocyclyl or 9- or 10-membered phenyl-cycloalkyl;
and/or, in R³, the 5- or 6-membered monocyclic heteroaryl is selected from the following groups: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine and pyrazine;
and/or, in R³, the 8- to 10-membered bicyclic heteroaryl is a bicyclic group formed by a monocyclic aryl ring fused with a monocyclic heteroaryl ring, a bicyclic group formed by a monocyclic heteroaryl ring fused with a monocyclic heteroaryl ring, 8- to 10-membered heteroaryl-heterocyclyl or 8- to 10-membered heteroaryl-cycloalkyl; for example, 8- to 10-membered fused bicyclic heteroaryl formed by 5- or 6-membered monocyclic heteroaryl ring fused with 5- or 6-membered monocyclic heterocyclyl ring, for another example, pyrrolo 5-membered monocyclic heteroaryl ring or pyrrolo 6-membered monocyclic heteroaryl ring; the 8- to 10-membered bicyclic heteroaryl is selected from the following groups: benzo[*d*]isoxazole, 1*H*-indole, isoindole, 1H-benzo[*d*]imidazole, benzo[*d*]isothiazole, *1H-*benzo[*d*][1,2,3]triazole, benzo[*d*]oxazole, benzo[*d*]thiazole, indazole, benzofuran, benzo[*b*]thiophene, quinoline, isoquinoline, quinazoline, quinoxaline, cinnoline, pyrido[3,2-*d*]pyrimidine, pyrido[2,3-*d*]pyrimidine, pyrido[3,4-*d*]pyrimidine, pyrido[4,3-*d*]pyrimidine, 1,8-naphthyridine, 1,7-naphthyridine, 1,6-naphthyridine, 1,5-naphthyridine, pyrazolo[1,5-*a*]pyrimidine, imidazo[1,2-*b*]pyridazine, 1,4,5,6-tetrahydropyrrolo[3,4-c]pyrazole, 2,4,5,6-tetrahydropyrrolo[3,4-*c*]pyrazole, 5,6,7,8-tetrahydro-[1,2,4]triazolo[1,5-*a*]pyrazine, 4,5,6,7-tetrahydro-[1,2,3]triazolo[1,5-*a*]pyrazine, 4,5,6,7-tetrahydro-pyrazolo[1,5-*a*]pyrazine;
and/or, in R³, the C₃₋₆ monocyclic cycloalkyl is selected from the following groups: cyclopropyl, cyclobutyl, cyclopentyl and cyclohexyl;
and/or, in R³, the 3- to 6-membered monocyclic heterocyclyl is selected from the following groups: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3*H*)-one, dihydrofuran-2,5-dione, piperidin-2-one, piperidin-2,6-dione, tetrahydro-2*H*-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxacyclobutadiene, 2,5-dihydro-1*H*-pyrrole, 2,5-dihydrofuran, 2,3-dihydrofuran, 2,3-dihydro-1*H-*pyrrole, 3,4-dihydro-2*H*-pyran, 1,2,3,4-tetrahydropyridine, 3,6-dihydro-2*H*-pyran, 1,2,3,6-tetrahydropyridine, 1,3-oxazinane, hexahydropyrimidine, 1,4-dioxane, tetrahydropyrimidin-2(1*H*)-one, 1,4-dioxan-2-one, 5,6-dihydro-2*H*-pyran-2-one, 5,6-dihydropyrimidin-4(3*H*)-one, 3,4-dihydropyridin-2(1*H*)-one, 5,6-dihydropyridin-2(1*H*)-one, 5,6-dihydropyrimidin-4(1*H*)-one, pyrimidin-4(3*H*)-one, pyrimidin-4(1*H*)-one, 4,5-dihydro-1*H*-imidazole, 2,3-dihydro-1*H*-imidazole, 2,3-dihydrooxazole, 1,3-dioxacyclopentene, 2,3-dihydrothiophene, 2,5-dihydrothiophene, 3,4-dihydro-2*H*-1,4-oxazine, 3,4-dihydro-2*H*-1,4-thiazine 1,1-dioxide, 1,2,3,4-tetrahydropyrazine, 1,3-dihydro-2*H*-pyrrol-2-one, 1,5-dihydro-2*H-*pyrrol-2-one, 1*H*-pyrrol-2,5-dione, furan-2(3*H*)-one, furan-2(5*H*)-one, 1,3-dioxacyclopenten-2-one, oxazol-2(3H)-one, 1,3-dihydro-2H-imidazol-2-one, furan-2,5-dione, 3,6-dihydropyridin-2(1*H*)-one, pyridin-2,6-(1H, 3H)-dione, 5,6-dihydro-2*H*-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine;
and/or, in R³, the 5- to 11-membered fused cycloalkyl is selected from the following groups: fused cycloalkyl formed by 3- to 6-membered monocyclic cycloalkyl ring and 4- to 6-membered monocyclic cycloalkyl ring;
and/or, in R³, the 5- to 11-membered fused cycloalkyl is selected from the following groups: 6-membered fused cycloalkyl formed by 5-membered monocyclic cycloalkyl ring and 3-membered monocyclic cycloalkyl ring, 7-membered fused cycloalkyl formed by 5-membered monocyclic cycloalkyl ring and 4-membered monocyclic cycloalkyl ring, 8-membered fused cycloalkyl formed by 5-membered monocyclic cycloalkyl ring and 5-membered monocyclic cycloalkyl ring, 7-membered fused cycloalkyl formed by 6-membered monocyclic cycloalkyl ring and 3-membered monocyclic cycloalkyl ring, 8-membered fused cycloalkyl formed by 6-membered monocyclic cycloalkyl ring and 4-membered monocyclic cycloalkyl ring, 9-membered fused cycloalkyl formed by 6-membered monocyclic cycloalkyl ring and 5-membered monocyclic cycloalkyl ring, 10-membered fused cycloalkyl formed by 6-membered monocyclic cycloalkyl ring and 6-membered monocyclic cycloalkyl ring;
and/or, in R³, the 5- to 11-membered fused heterocyclyl is selected from the following groups: fused heterocyclyl formed by 3- to 6-membered monocyclic cycloalkyl ring and 4- to 6-membered monocyclic heterocyclyl ring, fused heterocyclyl formed by 3- to 6-membered monocyclic heterocyclyl ring and 4- to 6-membered monocyclic cycloalkyl ring, fused heterocyclyl formed by 3- to 6-membered monocyclic heterocyclyl ring and 4- to 6-membered monocyclic heterocyclyl ring;
and/or, in R³, the 5- to 11-membered fused heterocyclyl is selected from the following groups: 6-membered fused heterocyclyl formed by 5-membered monocyclic heterocyclyl ring and 3-membered monocyclic cycloalkyl ring, 7-membered fused heterocyclyl formed by 5-membered monocyclic heterocyclyl ring and 4-membered monocyclic cycloalkyl ring, 7-membered fused heterocyclyl formed by 5-membered monocyclic heterocyclyl ring and 4-membered monocyclic heterocyclyl ring, 8-membered fused heterocyclyl formed by 5-membered monocyclic cycloalkyl ring and 5-membered monocyclic heterocyclyl ring, 8-membered fused heterocyclyl formed by 5-membered monocyclic heterocyclyl ring and 5-membered monocyclic heterocyclyl ring, 7-membered fused heterocyclyl formed by 6-membered monocyclic heterocyclyl ring and 3-membered monocyclic cycloalkyl ring, 8-membered fused heterocyclyl formed by 6-membered monocyclic heterocyclyl ring and 4-membered monocyclic cycloalkyl ring, 8-membered fused heterocyclyl formed by 6-membered monocyclic heterocyclyl ring and 4-membered monocyclic heterocyclyl ring, 9-membered fused heterocyclyl formed by 6-membered monocyclic heterocyclyl ring and 5-membered monocyclic heterocyclyl ring;
and/or, in R³, the 6- to 11-membered spirocycloalkyl is selected from the following groups: spirocycloalkyl formed by 3- to 6-membered monocyclic cycloalkyl ring and 4- to 6-membered monocyclic cycloalkyl ring;
and/or, in R³, the 6- to 11-membered spirocycloalkyl is selected from the following groups: 7-membered spirocycloalkyl formed by 4-membered monocyclic cycloalkyl ring and 4-membered monocyclic cycloalkyl ring, 7-membered spirocycloalkyl formed by 5-membered monocyclic cycloalkyl ring and 3-membered monocyclic cycloalkyl ring, 8-membered spirocycloalkyl formed by 5-membered monocyclic cycloalkyl ring and 4-membered monocyclic cycloalkyl ring, 9-membered spirocycloalkyl formed by 5-membered monocyclic cycloalkyl ring and 5-membered monocyclic cycloalkyl ring, 8-membered spirocycloalkyl formed by 6-membered monocyclic cycloalkyl ring and 3-membered monocyclic cycloalkyl ring, 9-membered spirocycloalkyl formed by 6-membered monocyclic cycloalkyl ring and 4-membered monocyclic cycloalkyl ring, 10-membered spirocycloalkyl formed by 6-membered monocyclic cycloalkyl ring and 5-membered monocyclic cycloalkyl ring, 11-membered spirocycloalkyl formed by 6-membered monocyclic cycloalkyl ring and 6-membered monocyclic cycloalkyl ring;
and/or, in R³, the 7- to 11-membered spiroheterocyclyl is selected from the following groups: spiroheterocyclyl formed by 3- to 6-membered monocyclic cycloalkyl ring and 4- to 6-membered monocyclic heterocyclyl ring, spiroheterocyclyl formed by 3- to 6-membered monocyclic heterocyclyl ring and 4- to 6-membered monocyclic cycloalkyl ring, spiroheterocyclyl formed by 3- to 6-membered monocyclic heterocyclyl ring and 4- to 6-membered monocyclic heterocyclyl ring;
and/or, in R³, the 7- to 11-membered spiroheterocyclyl is selected from the following groups: 7-membered spiroheterocyclyl formed by 4-membered monocyclic heterocyclyl ring and 4-membered monocyclic heterocyclyl ring, 7-membered spiroheterocyclyl formed by 4-membered monocyclic heterocyclyl ring and 4-membered monocyclic cycloalkyl ring, 7-membered spiroheterocyclyl formed by 5-membered monocyclic heterocyclyl ring and 3-membered monocyclic cycloalkyl ring, 8-membered spiroheterocyclyl formed by 5-membered monocyclic heterocyclyl ring and 4-membered monocyclic cycloalkyl ring, 8-membered spiroheterocyclyl formed by 5-membered monocyclic heterocyclyl ring and 4-membered monocyclic heterocyclyl ring, 9-membered spiroheterocyclyl formed by 5-membered monocyclic heterocyclyl ring and 5-membered monocyclic cycloalkyl ring, 9-membered spiroheterocyclyl formed by 5-membered monocyclic heterocyclyl ring and 5-membered monocyclic heterocyclyl ring, 8-membered spiroheterocyclyl formed by 6-membered monocyclic heterocyclyl ring and 3-membered monocyclic cycloalkyl ring, 9-membered spiroheterocyclyl formed by 6-membered monocyclic heterocyclyl ring and 4-membered monocyclic cycloalkyl ring, 9-membered spiroheterocyclyl formed by 6-membered monocyclic heterocyclyl ring and 4-membered monocyclic heterocyclyl ring;
and/or, Rₛ₁, Rₛ₂ are each independently halogen, cyano, nitro, hydroxyl, -C₁₋₃ alkyl, -C₁₋₃ alkoxy, -halo C₁₋₃ alkyl, -halo C₁₋₃ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₂ alkyl-hydroxyl, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy, -C₁₋₂ alkyl-3- to 6-membered heterocyclyl, -C₁₋₂ alkyl-NR^{c}R^{d}, -C₁₋₂ alkyl-C(O)NR^{c}R^{d}, -C₁₋₂ alkyl-SO₂C₁₋₃ alkyl or C₂₋₄ alkynyl; wherein, the 3- to 6-membered heterocyclyl has 1, 2 or 3 heteroatoms selected from N, O and S as ring atoms;
and/or, the substituents of group S1 each independently comprise oxo (=O), halogen, cyano, nitro, hydroxyl, carboxyl, C₁₋₃ alkyl, C₁₋₃ alkoxy, halo C₁₋₃ alkyl, deuterated C₁₋₃ alkyl, halo C₁₋₃ alkoxy, deuterated C₁₋₃ alkoxy, C₃₋₆ monocyclic cycloalkyl, 3- to 6-membered monocyclic heterocyclyl, C₃₋₆ monocyclic cycloalkoxy, -O-3- to 6-membered monocyclic heterocyclyl, NR^{c}R^{d}, C(O)C₁₋₃ alkyl, C(O)C₂₋₄ alkenyl, C(O)C₃₋₄ monocyclic cycloalkyl, C(O)NR^{c}R^{d}, -SO₂C₁₋₃ alkyl, -SOzhalo C₁₋₃ alkyl, -SO₂NR^{c}R^{d}, -C₁₋₂ alkyl-hydroxyl, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy, -C₁₋₂ alkyl-3- to 6-membered monocyclic heterocyclyl, -C₁₋₂ alkyl-NR^{c}R^{d}, -O-C₁₋₂ alkyl-NR^{c}R^{d}, -C₁₋₂ alkyl-C(O)NR^{c}R^{d}, -C₁₋₂ alkyl-SO₂C₁₋₃ alkyl, carboxyl, -C₁₋₂ alkyl-carboxyl; the R^{c}, R^{d} are each independently H, C₁₋₆ alkyl, deuterated C₁₋₆ alkyl, C₃₋₆ monocyclic cycloalkyl, -C₁₋₄ alkyl-C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms.

3. The compound, the pharmaceutically acceptable salt thereof, the stereoisomer thereof, the solvate thereof or the prodrug thereof as claimed in claim 1 or 2, wherein,
Rₛ₁, Rₛ₂ are each independently fluorine, chlorine, cyano, nitro, NH₂, NHCH₃, hydroxyl, methyl, ethyl, methoxy, halomethyl, haloethyl, halomethoxy, haloethoxy, cyclopropyl;
and/or, the substituents of group S1 are each independently selected from: oxo (=O), COOH, fluorine, hydroxyl, cyano, methyl, ethyl, isopropyl, methoxy, ethoxy, isopropoxy, CHF₂, CH₂F, CF₃, deuterated methyl, deuterated methoxy, OCHF₂, OCH₂F, OCF₃, cyclopropyl, cyclobutyl, cyclopropoxy, cyclobutoxy, oxiranyl, aziridinyl, oxetanyl, azetidinyl, tetrahydrofuranyl, tetrahydropyrrolyl, piperazinyl, *N*-methylpiperazinyl, C(O)CH₃, C(O)CH₂CH₃, C(O)CH₂CH₂CH₃, C(O)CH=CH₂, C(O)-cyclopropane, -SO₂CH₃, - SO₂CH₂CH₃, -SO₂CH₂CH₂CH₃, NH₂, NH(CH)₃, N(CH₃)₂, NH(CH₂CH₃), NH(CH₂CF₃), NH(CH₂CHF₂), NH(CH₂CH₂F), N(CH₂CH₃)₂, N(CH₂CH₃)(CH₃), N(CH₂CF₃)(CH₃), N(CH₂CHF₂)(CH₃), N(CH₂CH₂F)(CH₃), N(CH₂CH₂CH₃)(CH₃), N(CH₂CH₂CH₂CH₃)(CH₃), N(CH₃)(CH₂CH₂OH), N(CH₃)(CH₂CH₂OCH₃), N(CH₃)(CH₂CH₂CH₂OCH₃), N(CH₃)(CH₂CH₂CH₂CH₂OCH₃), N(CH₃)(CH₂C(CH₃)₂OCH₃), N(CH₃)(CH₂C(CH₂)₂OCH₃), N(CD₃)(CH₂C(CH₂)₂OCH₃), NH(CH₂CH₂OH), NH(CH₂CH₂OCH₃), N(cyclobutane)(CH₃), N(azetidine)(CH₃), N(1-methylazetidine)(CH₃), NH(oxetane), N(oxetane)(CH₃), N(oxacycloheptane)(CH₃), N(CH₂-azetidine)(CH₃), N(CH₂(1-methylazetidine))(CH₃), NH(CH₂-oxetane), N(CH₂-oxetane)(CH₃), N(oxetane)(CD₃), N(CD₃)₂, N(CH₃)(CD₃), N(C(O)CH₃)(CH₃), CH₂N(CH₃)₂, CH₂CH₂N(CH₃)₂, -OCH₂CH₂(CH₃)₂, -OCH₂CH₂CH₂(CH₃)₂, -OCH₂CH₂CH₂CH₂(CH₃)₂, -O-oxetane, -O-azetidine, -O-(1-methylazetidine), morpholinyl, CHzOH, CHzCHzOH, CH₂OCH₃, CH₂CH₂OCH₃, -OCH₂CH₂N(CH₃)₂, -CH₂CH₂N(CH₃)₂, -CH₂N(CH₃)₂.

4. The compound, the pharmaceutically acceptable salt thereof, the stereoisomer thereof, the solvate thereof or the prodrug thereof as claimed in any one of claims 1-3, wherein, when G¹ is N, Y is -C(=O)- or - C(=S)-; L² is a bond, O, S, (CR^{L3}R^{L4})ₘ₁, NR^{L5}, NH(C=O), S(=O)z or C(=O); wherein, m1 is 1, 2 or 3; R^{L3}, are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5-to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3-to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1.

5. The compound, the pharmaceutically acceptable salt thereof, the stereoisomer thereof, the solvate thereof or the prodrug thereof as claimed in claim 4, wherein,
when G¹ is N, Y is -C(=O)- or -C(=S)-; L² is O; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is NR^{L5}; wherein, R^{L5} is hydrogen or C₁₋₆ alkyl; R³ is 5-or 6-membered monocyclic heteroaryl, 8- to 10-membered bicyclic heteroaryl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is a bond; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is a bond; R³ is 3- to 6-membered monocyclic heterocyclyl, 5- to 11-membered fused heterocyclyl, 7- to 11-membered spiroheterocyclyl or 5- to 11-membered bridged heterocyclyl; wherein, the 3- to 6-membered monocyclic heterocyclyl has 1, 2 or 3 heteroatoms selected from N, O and S as ring atoms; the 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the 3- to 6-membered monocyclic heterocyclyl, 5-to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is a bond; R³ is 3- to 6-membered monocyclic heterocyclyl, 5- to 11-membered fused heterocyclyl, 7- to 11-membered spiroheterocyclyl or 5- to 11-membered bridged heterocyclyl; wherein, the 3- to 6-membered monocyclic heterocyclyl, 5- to 11-membered fused heterocyclyl, 7- to 11-membered spiroheterocyclyl, 5- to 11-membered bridged heterocyclyl have at least 1 N as a ring atom and R³ is attached to the parent ring through this N atom; the 3- to 6-membered monocyclic heterocyclyl, 5- to 11-membered fused heterocyclyl, 7- to 11-membered spiroheterocyclyl, 5- to 11-membered bridged heterocyclyl also optionally have 1 or 2 heteroatoms selected from N, O and S as ring atoms; the 3- to 6-membered monocyclic heterocyclyl, 5- to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is a bond; R³ is 5-membered monocyclic heterocyclyl or 6-membered monocyclic heterocyclyl; wherein, the 5-membered monocyclic heterocyclyl or 6-membered monocyclic heterocyclyl has at least 1 N as a ring atom and R³ is attached to the parent ring through this N atom; the 5-membered monocyclic heterocyclyl or 6-membered monocyclic heterocyclyl also optionally has 1 or 2 heteroatoms selected from N, O and S as ring atoms; the 5-membered monocyclic heterocyclyl or 6-membered monocyclic heterocyclyl is each independently unsubstituted or substituted by 1, 2 or 3 substituents independently selected from group S1;
or, when G¹ is N, Y is -C(=O)- or -C(=S)-; L² is O or S; R³ is hydrogen, C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is O; R³ is C₆₋₁₄ aryl, 3- to 6-membered monocyclic heterocyclyl or -L⁴-3- to 6-membered monocyclic heterocyclyl; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 3- to 6-membered monocyclic heterocyclyl are each independently unsubstituted or substituted by 1, 2, 3 or 4 substituents independently selected from group S1;
or, when G¹ is N, Y is -C(=O)- or -C(=S)-; L² is (CR^{L1}R^{L4})ₘ₁; wherein, m1 is 1, 2 or 3; R^{L3}, are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, combining with the carbon atoms to which they are attached form a C₃₋₆ monocyclic cycloalkyl; R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7-to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, - L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is hydrogen, C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is NR^{L5}; wherein, R^{L5} is hydrogen, C₁₋₃ alkyl or deuterated C₁₋₃ alkyl; R³ is C₆₋₁₄ aryl, 5- or 6-membered monocyclic heteroaryl, 3- to 6-membered monocyclic heterocyclyl, 5- to 11-membered bridged heterocyclyl or -L⁴-C₆₋₁₄ aryl; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the C₆₋₁₄ aryl, 5- or 6-membered monocyclic heteroaryl, 3- to 6-membered monocyclic heterocyclyl, 5- to 11-membered bridged heterocyclyl are each independently unsubstituted or substituted by 1, 2, 3 or 4 substituents independently selected from group S1;
or, when G¹ is N, Y is -C(=O)- or -C(=S)-; L² is NH(C=O), S(=O)₂ or C(=O); R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
the R^{a}, R^{b} are each independently H or C₁₋₆ alkyl or R^{a}, R^{b} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1.

6. The compound, the pharmaceutically acceptable salt thereof, the stereoisomer thereof, the solvate thereof or the prodrug thereof as claimed in any one of claims 1-5, wherein,
R² is selected from the following structures: 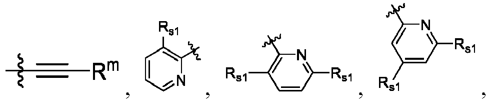 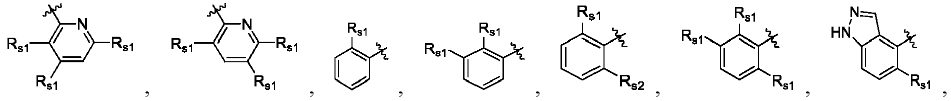 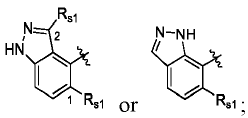 R^{m} is H or C₁₋₆ alkyl, e.g., -CH₃; wherein Rₛ₁ of each position is the same or different; Rₛ₁, Rₛ₂ are the same or different; Rₛ₁, Rₛ₂ are each independently halogen, cyano, nitro, hydroxyl, -C₁₋₆ alkyl, -C₁₋₆ alkoxy, -halo C₁₋₆ alkyl, -deuterated C₁₋₆ alkyl, -halo C₁₋₆ alkoxy, -deuterated C₁₋₆ alkoxy, - C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl;
and/or, R³ is 8- to 10-membered bicyclic heteroaryl, 3- to 6-membered monocyclic heterocyclyl, 5- to 11-membered fused heterocyclyl, 7- to 11-membered spiroheterocyclyl, 5- to 11-membered bridged heterocyclyl or 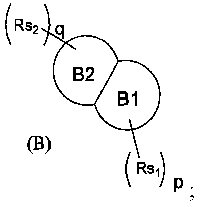 wherein, the 8- to 10-membered bicyclic heteroaryl is 8- to 10-membered heteroaryl-heterocyclyl; the 8- to 10-membered bicyclic heteroaryl is unsubstituted or substituted by 1, 2, 3 or 4 substituents independently selected from group S1; for example, the 8- to 10-membered heteroaryl-heterocyclyl is 8- to 10-membered fused bicyclic heteroaryl formed by 5- or 6-membered monocyclic heteroaryl ring fused with 5- or 6-membered monocyclic heterocyclyl ring; for another example, the 8- to 10-membered heteroaryl-heterocyclyl is pyrrolo 5-membered monocyclic heteroaryl ring or pyrrolo 6-membered monocyclic heteroaryl ring; the 3- to 6-membered monocyclic heterocyclyl has 1, 2 or 3 heteroatoms selected from N, O and S as ring atoms; the 5- to 11-membered fused heterocyclyl, 7- to 11-membered spiroheterocyclyl, 5- to 11-membered bridged heterocyclyl has 1, 2, 3, 4 or 5 heteroatoms selected from N, O and S as ring atoms; the 3- to 6-membered monocyclic heterocyclyl, 5- to 11-membered fused heterocyclyl, 7- to 11-membered spiroheterocyclyl, 5- to 11-membered bridged heterocyclyl are each independently unsubstituted or substituted by 1, 2, 3 or 4 substituents independently selected from group S1; preferably, the 3- to 6-membered monocyclic heterocyclyl, 5- to 11-membered fused heterocyclyl, 7- to 11-membered spiroheterocyclyl, 5- to 11-membered bridged heterocyclyl have at least 1 N as a ring atom and R³ is attached to the parent ring through this N atom;
the 3- to 6-membered monocyclic heterocyclyl is preferably 5-membered monocyclic heterocyclyl or 6-membered monocyclic heterocyclyl; wherein, the 5-membered monocyclic heterocyclyl has at least one N atom as a ring atom and R³ is attached to the parent ring through this N atom; the 5-membered monocyclic heterocyclyl also optionally has 1 heteroatom selected from N, O and S as a ring atom; the 5-membered monocyclic heterocyclyl is unsubstituted or substituted by 1, 2, 3 or 4 substituents independently selected from group S1; preferably, the 5-membered monocyclic heterocyclyl has one N atom as a ring atom and R³ is attached to the parent ring through this N atom; substituted by 1, 2, 3 or 4 substituents independently selected from group S1; more preferably substituted by 2 methyl and another substituent selected from group S1; the 6-membered monocyclic heterocyclyl has at least one N atom as a ring atom and R³ is attached to the parent ring through this N atom; the 6-membered monocyclic heterocyclyl also optionally has 1 heteroatom selected from N, O and S as a ring atom; the 6-membered monocyclic heterocyclyl is unsubstituted or substituted by 1, 2, 3 or 4 substituents independently selected from group S1, preferably substituted by 2 or 3 substituents independently selected from group S1; more preferably substituted by 2 methyl and another substituent selected from group S1;
in a structure shown in formula (B), ring B1 is a benzene ring or 5- or 6-membered monocyclic heteroaryl ring; ring B2 is a fused 5- or 6-membered monocyclic heterocycloalkyl ring or fused 5- or 6-membered monocyclic cycloalkyl ring; wherein, the 5- or 6-membered monocyclic heteroaryl ring or the fused 5- or 6-membered monocyclic heterocycloalkyl ring has 1, 2 or 3 heteroatoms selected from N, O and S as ring atoms; (Rₛ₁)ₚ means that the hydrogen on ring B1 is substituted by p Rₛ₁, p is 0, 1, 2 or 3, each Rₛ₁ is the same or different; (Rₛ₂)_{q} means that the hydrogen on ring B2 is substituted by q Rₛ₂, q is 0, 1, 2 or 3, each Rₛ₂ is the same or different; Rₛ₁, Rₛ₂ are each independently halogen, cyano, nitro, hydroxyl, -C₁₋₆ alkyl, -C₁₋₆ alkoxy, -halo C₁₋₆ alkyl, -halo C₁₋₆ alkoxy, -C₃₋₆ monocyclic cycloalkyl, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; wherein, the 3- to 6-membered heterocyclyl has 1, 2 or 3 heteroatoms selected from N, O and S as ring atoms.

7. The compound, the pharmaceutically acceptable salt thereof, the stereoisomer thereof, the solvate thereof or the prodrug thereof as claimed in claim 6, wherein,
R² is selected from the following structures:
i. 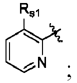 wherein Rₛ₁ is halogen, -C₁₋₆ alkyl, -halo C₁₋₆ alkyl, -deuterated C₁₋₆ alkyl or - NR^{c}R^{d}; preferably, Rₛ₁ is halogen or -halo C₁₋₆ alkyl, e.g., -F, -Cl or -CF₃;
ii. 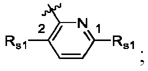 wherein Rₛ₁ at position 1 is -NR^{c}R^{d}; preferably R^{c}, R^{d} are independently H or -C₁₋₆ alkyl; e.g., H or -CH₃; Rₛ₁ at position 2 is halogen, -C₁₋₆ alkyl or -halo C₁₋₆ alkyl, preferably halogen or -halo C₁₋₆ alkyl, e.g., F, Cl, -CF₃ or -CH₂CF₃;
iii. 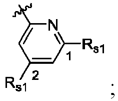 wherein Rₛ₁ at position 1 is -NR^{c}R^{d}; preferably R^{c}, R^{d} are H; Rₛ₁ at position 2 is halogen, -C₁₋₆ alkyl or -halo C₁₋₆ alkyl, preferably -C₁₋₆ alkyl, e.g., -CH₃;
iv. 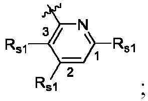 wherein Rₛ₁ at position 1 is -NR^{c}R^{d}; preferably R^{c}, R^{d} are H; Rₛ₁ at position 2 is halogen, -C₁₋₆ alkyl or -halo C₁₋₆ alkyl, preferably -C₁₋₆ alkyl, e.g., -CH₃ or -CH₂CH₃; Rₛ₁ at position 3 is halogen, -C₁₋₆ alkyl or -halo C₁₋₆ alkyl, preferably -halo C₁₋₆ alkyl, e.g., -CF₃ or -CH₂CF₃;
v. 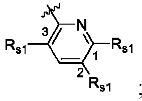 wherein Rₛ₁ at position 1 is -NR^{c}R^{d}; Rₛ₁ at position 2 is halogen, -C₁₋₆ alkyl or -halo C₁₋₆ alkyl; Rₛ₁ at position 3 is halogen, -C₁₋₆ alkyl or -halo C₁₋₆ alkyl;
vi. 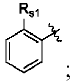 wherein, Rₛ₁ is halogen, cyano, -C₁₋₆ alkyl or -halo C₁₋₆ alkyl, preferably F, Cl, cyano, -CH₃ or -CF₃;
vii. 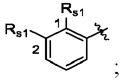 wherein Rₛ₁ at position 1 is halogen, e.g., F; Rₛ₁ at position 2 is halogen, - C₁₋₆ alkyl or -halo C₁₋₆ alkyl, preferably halogen or -C₁₋₆ alkyl, e.g., F or -CH₃;
viii. 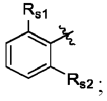 wherein, Rₛ₁ is halogen, cyano, nitro, hydroxyl, -C₁₋₆ alkyl, -C₁₋₆ alkoxy, - halo C₁₋₆ alkyl, -halo C₁₋₆ alkoxy, -C₃₋₆ monocyclic cycloalkyl, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; Rₛ₂ is halogen; preferably, Rₛ₁ is halogen, hydroxyl, -C₁₋₆ alkoxy, -C₁₋₆ alkyl, -halo C₁₋₆ alkyl, -C₃₋₆ monocyclic cycloalkyl or preferably R^{c}, R^{d} are H; Rₛ₂ is halogen or, e.g., F or Cl, or - halo C₁₋₆ alkoxy; more preferably, Rₛ₁ is F, Cl, -OH, -OCH₃, -CH₃, -CF₃, 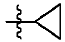 or -NH₂; Rₛ₂ is F, Cl or -CF₃;
ix. 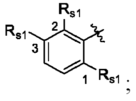 wherein Rₛ₁ at position 1 is halogen and Rₛ₁ at position 2 is hydroxyl, or Rₛ₁ at position 1 is hydroxyl and Rₛ₁ at position 2 is halogen; and Rₛ₁ at position 3 is halogen, cyano, nitro, hydroxyl, -C₁₋₆ alkyl, -C₁₋₆ alkoxy, -halo C₁₋₆ alkyl, -halo C₁₋₆ alkoxy, -C₃₋₆ monocyclic cycloalkyl, -C(O)NR^{c}R^{d}, -SO₂C₁₋₃ alkyl, -SO₂halo C₁₋₃ alkyl, -SO₂NR^{c}R^{d}, - C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl; or Rₛ₁ at position 1 is halogen and Rₛ₁ at position 2 is -NR^{c}R^{d}; or Rₛ₁ at position 1 is -NR^{c}R^{d}; and Rₛ₁ at position 2 is halogen; and Rₛ₁ at position 3 is halogen; preferably, Rₛ₁ at position 1 is F or Cl and Rₛ₁ at position 2 is hydroxyl or - NH₂, or Rₛ₁ at position 1 is hydroxyl or -NH₂ and Rₛ₁ at position 2 is F or Cl; and Rₛ₁ at position 3 is F or Cl;
x. 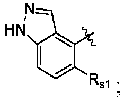 Rₛ₁ is -C₁₋₆ alkyl, e.g., -CH₃;
xi. 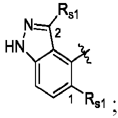 Rₛ₁ at position 1 is -C₁₋₆ alkyl, e.g., -CH₃; Rₛ₁ at position 2 is -C₃₋₆ monocyclic cycloalkyl, e.g., 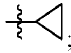
xii. 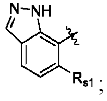 Rₛ₁ is -C₁₋₆ alkyl or halogen, e.g., -CH₃ or F;
or, R³ is selected from the following groups 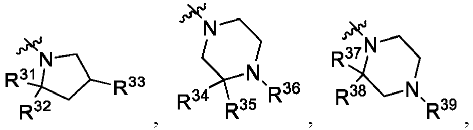 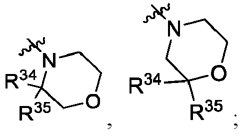 in each formula, R³¹, R³², R³³, each R³⁴, each R³⁵, R³⁶, R³⁷, R³⁸, R³⁹ are each independently selected from group S1;
in R³, the substituents of group S1 are preferably selected from H, C₁₋₆ alkyl, halo C₁₋₆ alkyl, deuterated C₁₋₆ alkyl, hydroxyl, NR^{c}R^{d}; wherein, R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by - CH₂CH₂- to form a cycloalkyl;
in R³, the substituents of group S1 are more preferably selected from H, methyl, hydroxyl, NR^{c}R^{d}; wherein, R^{c}, R^{d} are each independently H, C₁₋₆ alkyl, halo C₁₋₆ alkyl, deuterated C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂- to form a cycloalkyl; the 3- to 6-membered monocyclic heterocyclyl is optionally substituted by 1 or 2 groups selected from the following groups: C₁₋₆ alkyl; or R^{c}, R^{d} combining with the nitrogen atoms to which they are attached form a 3- to 6-membered nitrogen-containing heterocyclyl, 5- to 11-membered fused heterocyclyl, 7- to 11-membered spiroheterocyclyl or 5- to 11-membered bridged heterocyclyl; the 3- to 6-membered nitrogen-containing heterocyclyl has 1 nitrogen atom and optionally 1 or 2 heteroatoms selected from N, O and S as ring atoms; the 5- to 11-membered fused heterocyclyl, 7- to 11-membered spiroheterocyclyl or 5- to 11-membered bridged heterocyclyl has 1 nitrogen atom and optionally 1 or 2 heteroatoms selected from N, O and S as ring atoms; and the 3- to 6-membered nitrogen-containing heterocyclyl, 5- to 11-membered fused heterocyclyl, 7- to 11-membered spiroheterocyclyl or 5- to 11-membered bridged heterocyclyl is optionally substituted by 1 or 2 substituents selected from the following groups: oxo (=O), halogen, carboxyl, cyano, nitro, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, C(O)NR^{c1}R^{d1}, NR^{c1}R^{d1}; R^{c1}, R^{d1} are each independently hydrogen, C₁₋₆ alkyl, deuterated C₁₋₆ alkyl, halo C₁₋₆ alkyl, C₃₋₆ monocyclic cycloalkyl, halo C₃₋₆ monocyclic cycloalkyl, -C₁₋₄ alkyl-C₁₋₆ alkoxy, -C₁₋₄ alkyl-halo C₁₋₆ alkyl, -C₁₋₄ alkyl-deuterated C₁₋₆ alkyl, -C₁₋₄ alkyl-halo C₁₋₆ alkoxy, -C₁₋₄ alkyl-deuterated C₁₋₆ alkoxy, 3- to 6-membered monocyclic heterocyclyl, C₁₋₄ alkyl-3- to 6-membered monocyclic heterocyclyl, C(O)C₁₋₆ alkyl, S(O)₂C₁₋₆ alkyl; wherein, 1 or 2 hydrogen atoms on -C₁₋₄ alkyl- are optionally substituted by C₁₋₆ alkyl or two hydrogen atoms on the same carbon on -C₁₋₄ alkyl- are simultaneously substituted by -CH₂CH₂-to form a cycloalkyl;
in R³, the substituents of group S1 are most preferably selected from H, methyl, hydroxyl, NR^{c}R^{d}; wherein, R^{c}, R^{d} are each independently H, methyl, halomethyl (e.g., trifluoromethyl, difluoromethyl, monofluoromethyl), ethyl, deuterated ethyl, haloethyl (e.g., trifluoroethyl, difluoroethyl, monofluoroethyl); or R^{c}, R^{d} combining with the nitrogen atoms to which they are attached form a piperazinyl, N-methylpiperazinyl, azetidinyl or tetrahydropyrrolyl; and the piperazinyl, N-methylpiperazinyl, azetidinyl or tetrahydropyrrolyl is optionally substituted by 1 or 2 substituents selected from the following groups: methyl, deuterated methyl, halomethyl, ethyl, deuterated ethyl, haloethyl.

8. The compound, the pharmaceutically acceptable salt thereof, the stereoisomer thereof, the solvate thereof or the prodrug thereof as claimed in any one of claims 1-7, wherein,
R² is selected from the following structures: 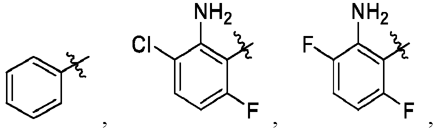 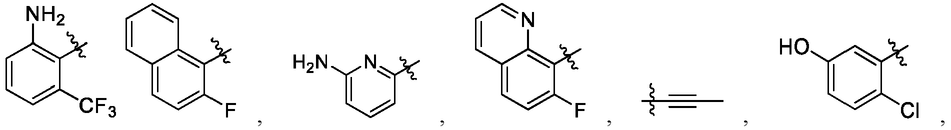 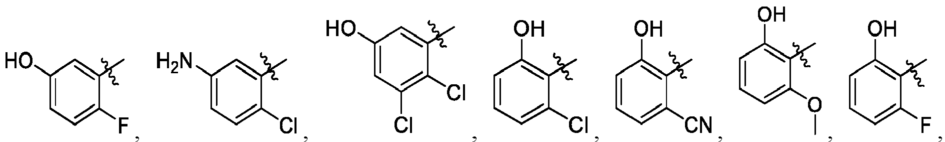 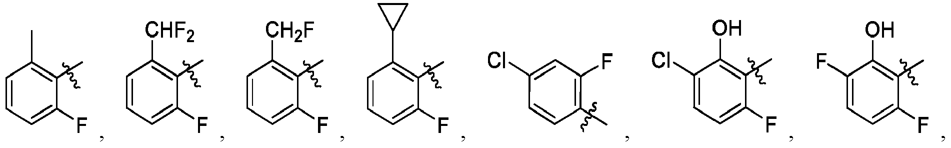 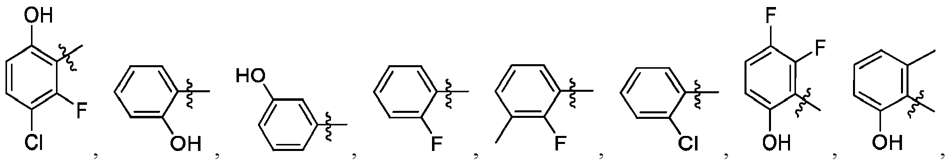 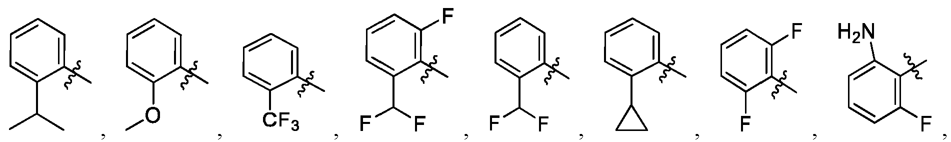 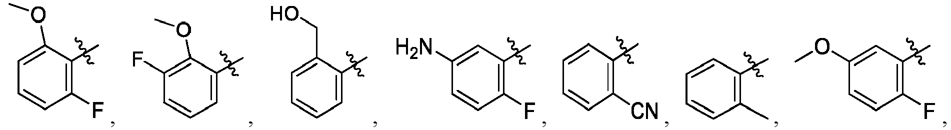 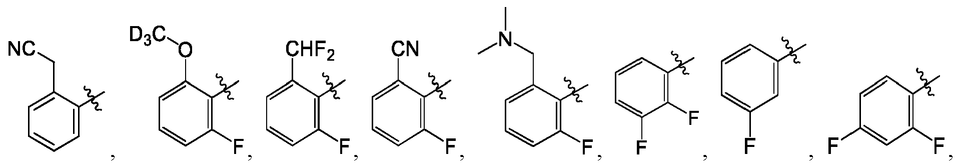 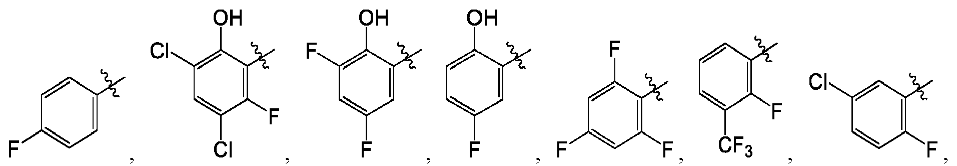 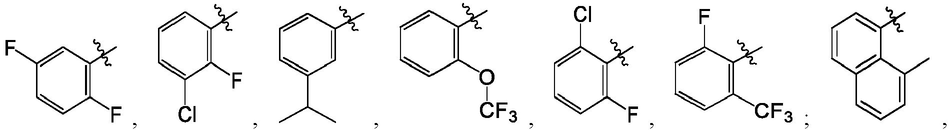 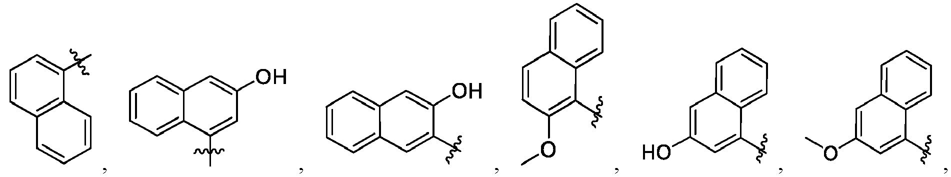 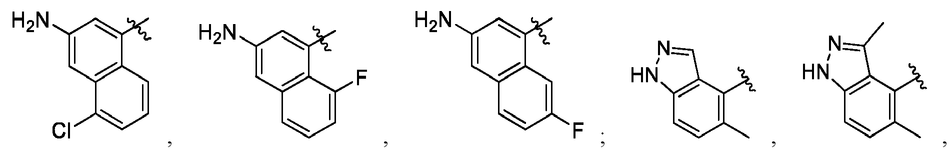 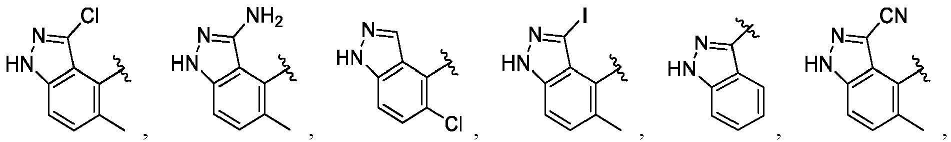 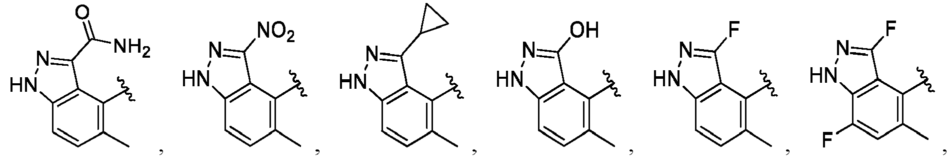 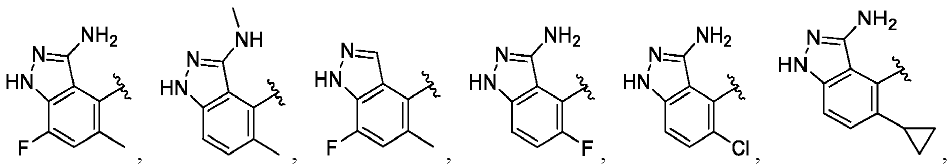 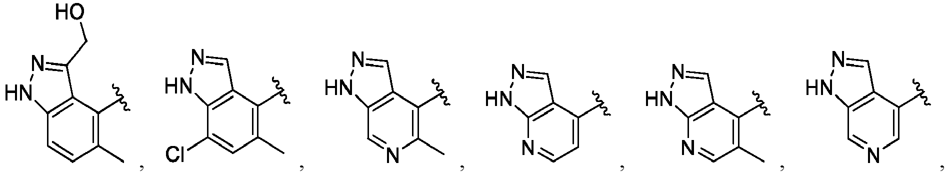 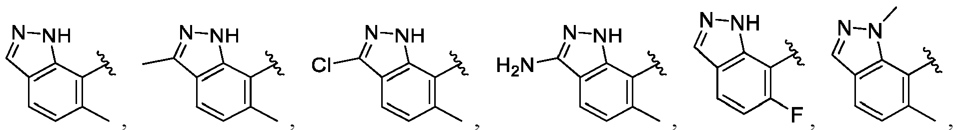 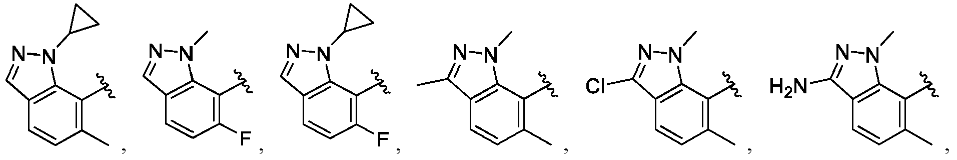 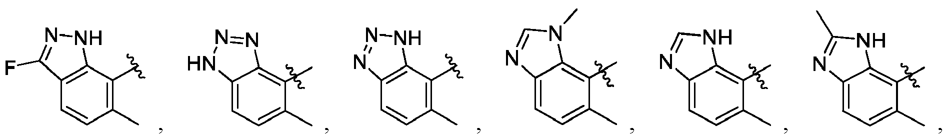 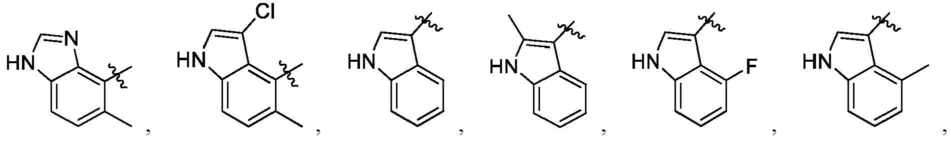 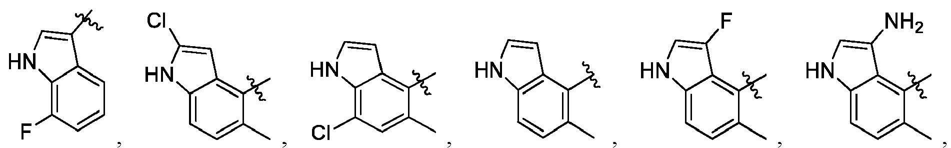 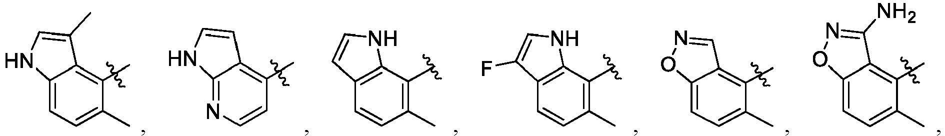 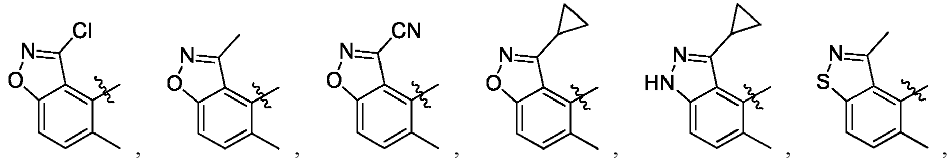 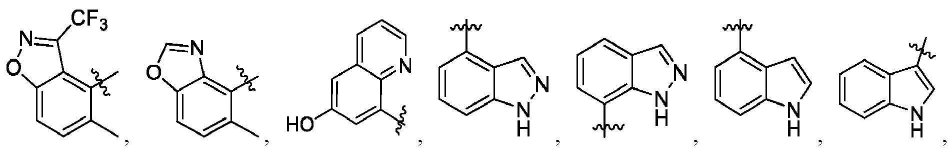 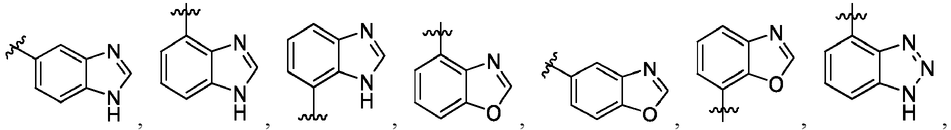 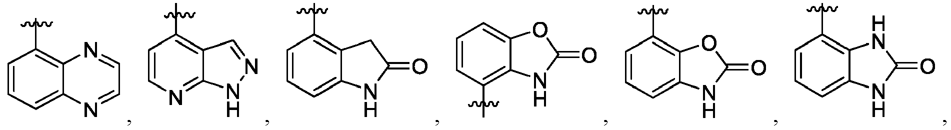 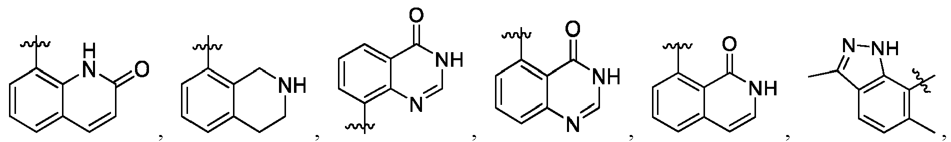 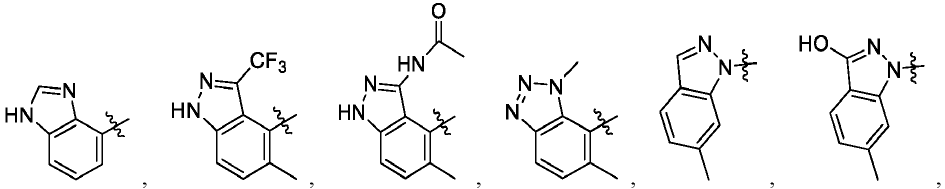 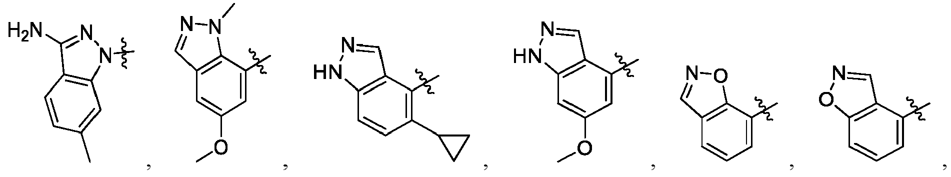 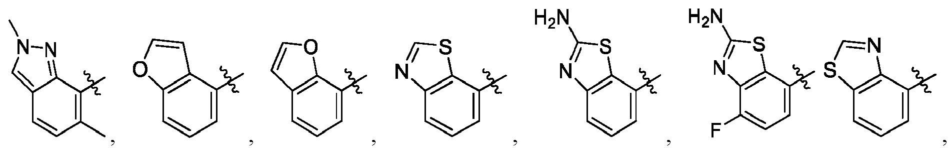 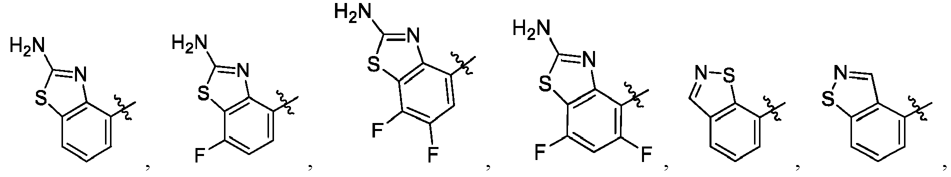 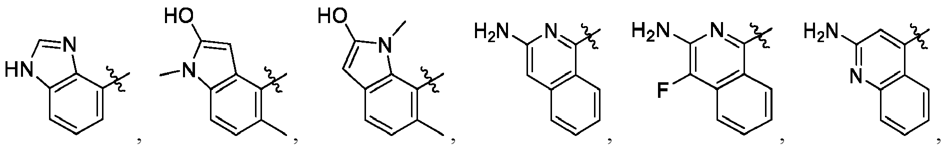 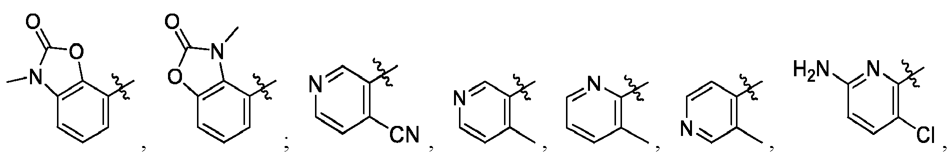 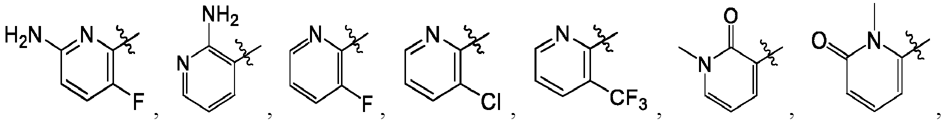 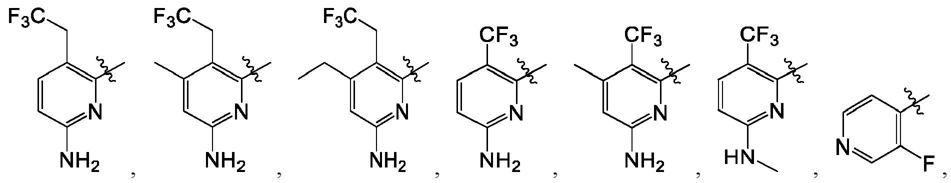 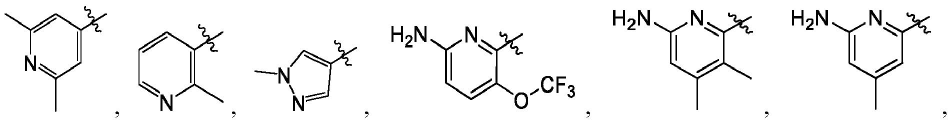 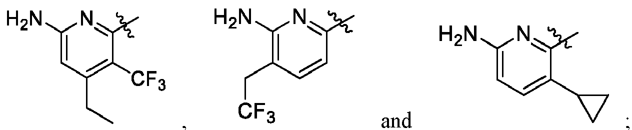 further preferably 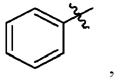 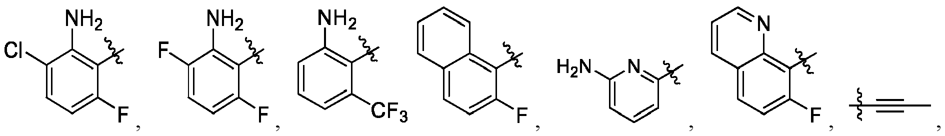 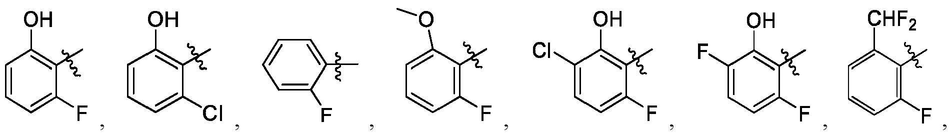 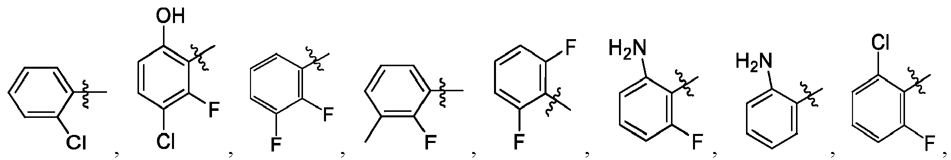 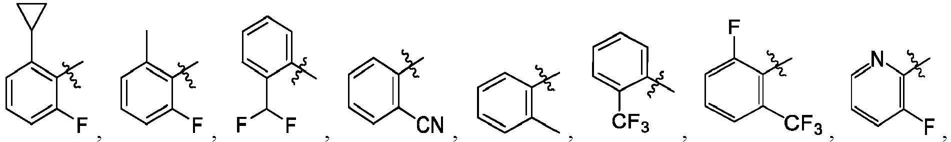 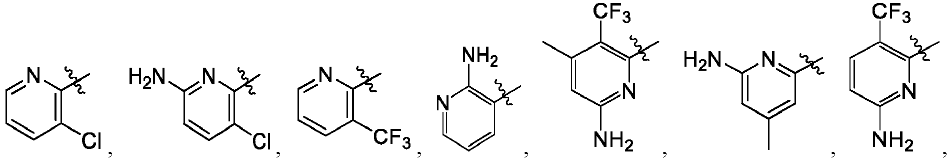 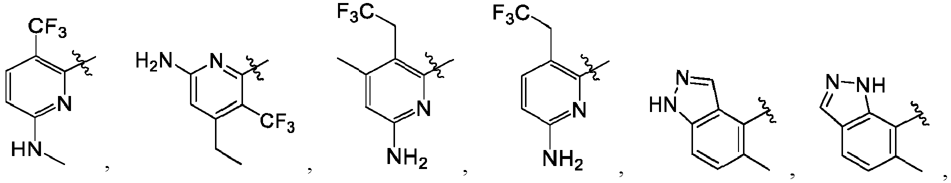 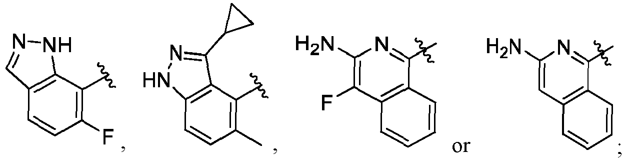
and/or, R³ is selected from the following structures: 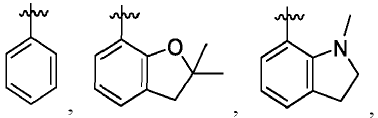 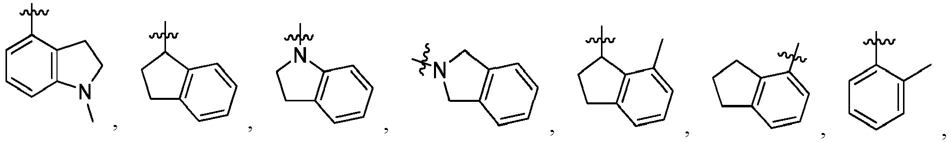 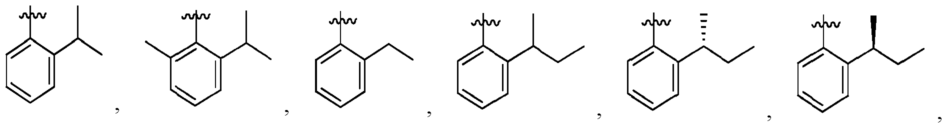 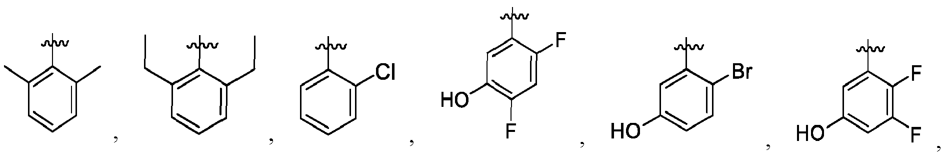 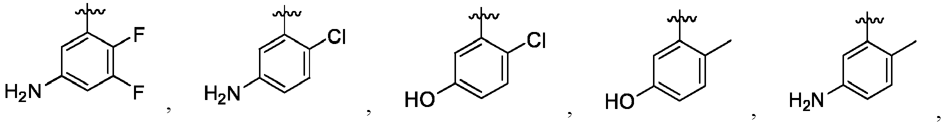 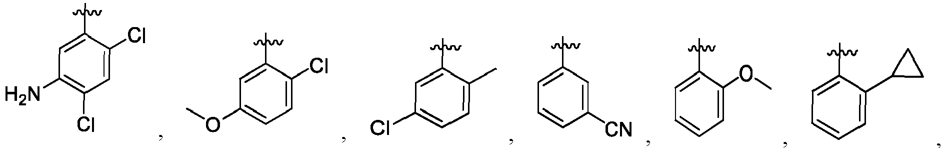 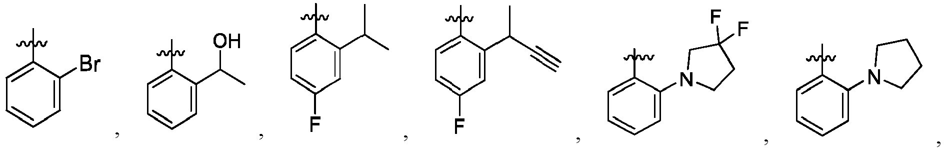 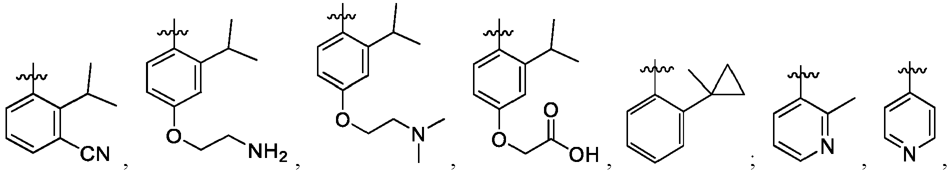 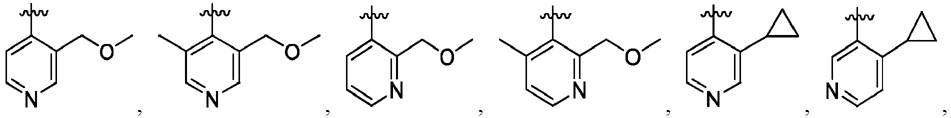 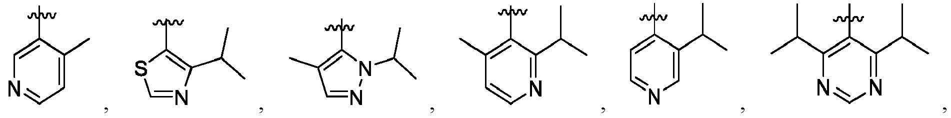 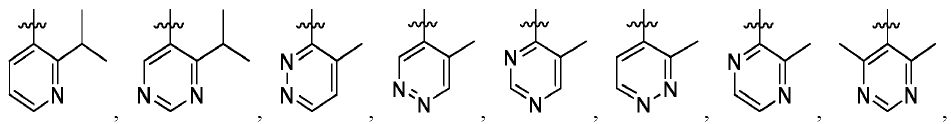 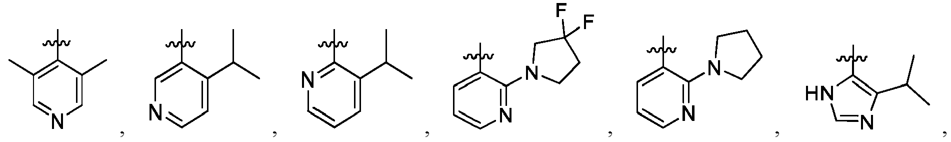 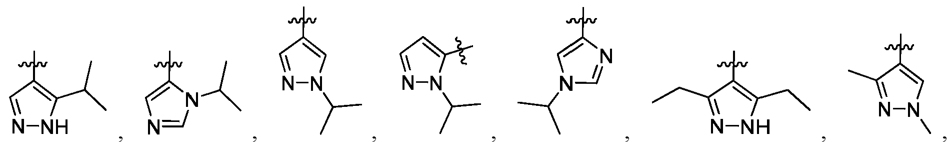 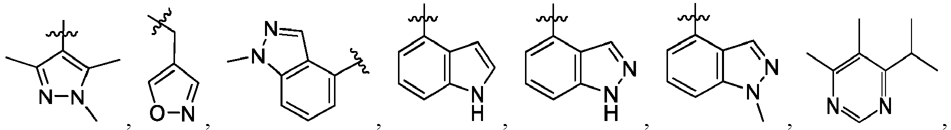 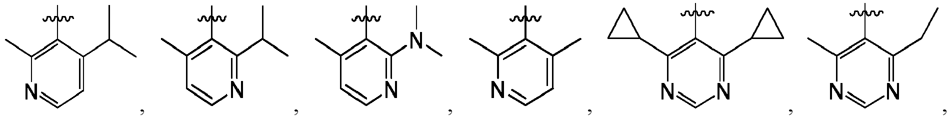 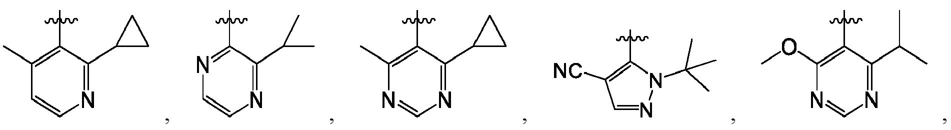 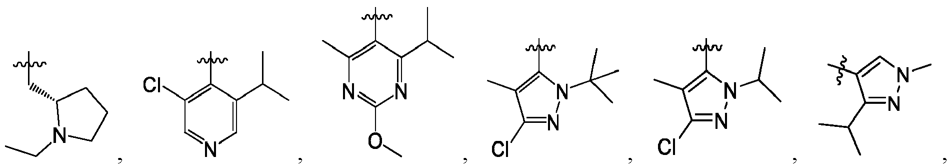 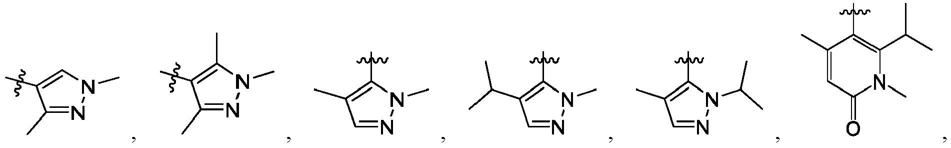 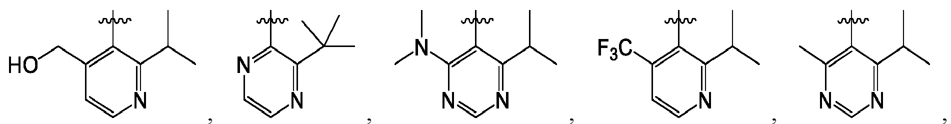 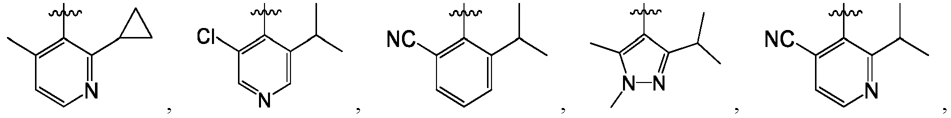 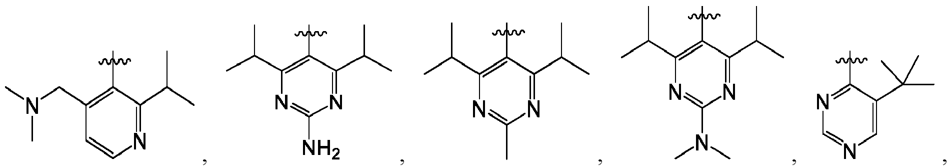 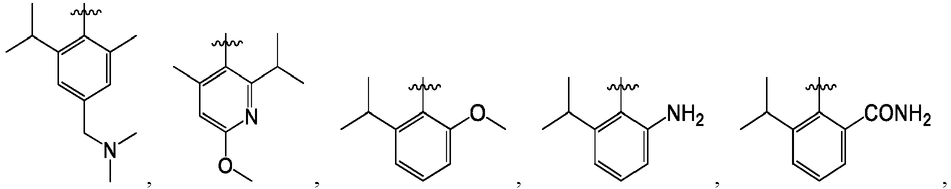 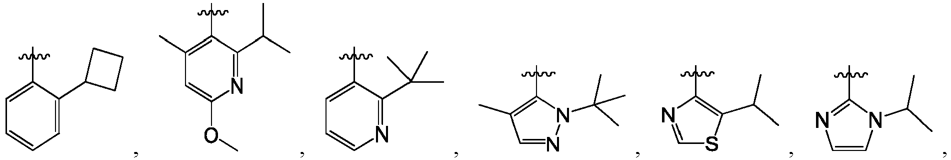 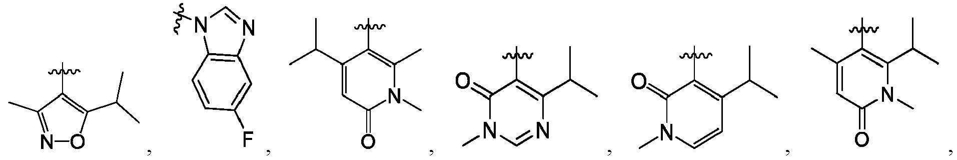 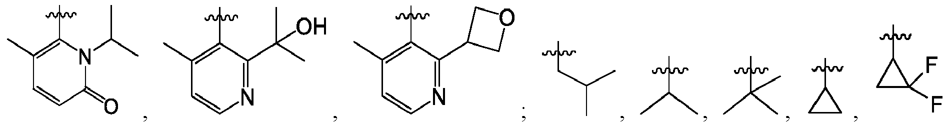 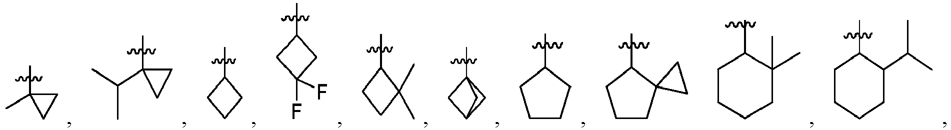 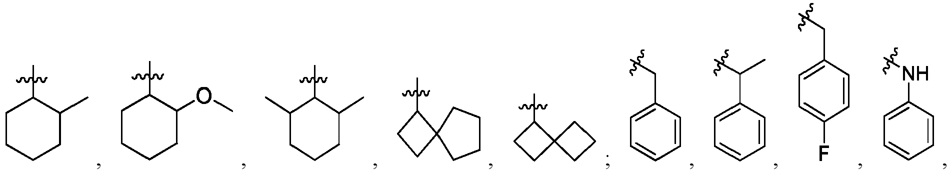 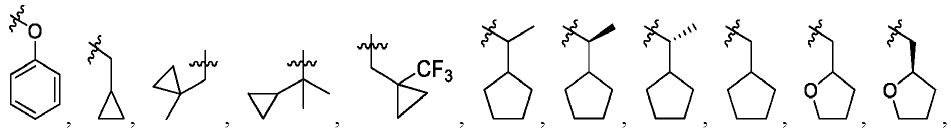 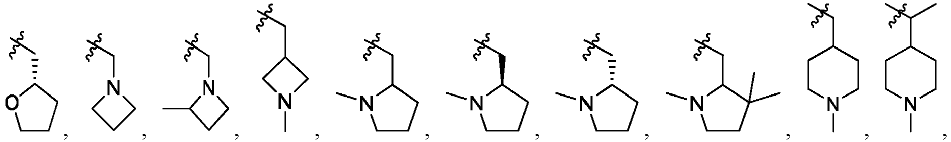 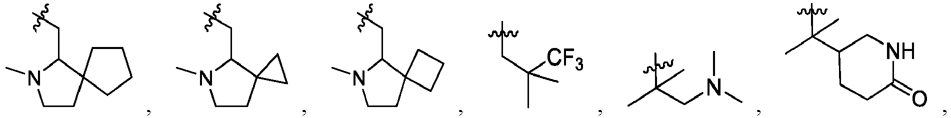 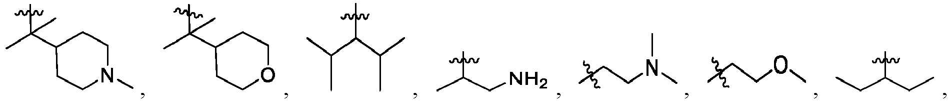 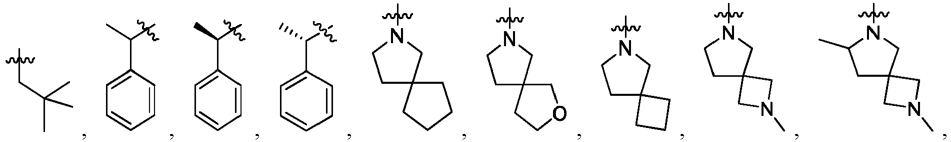 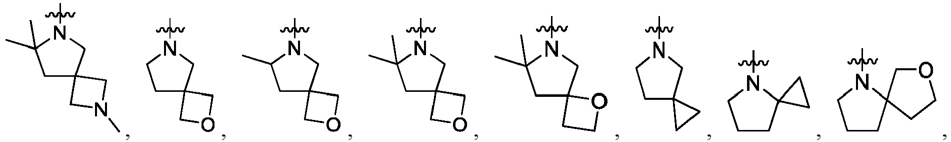 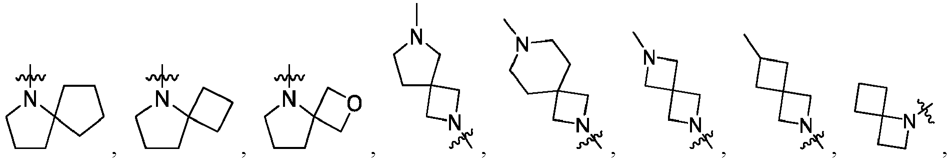 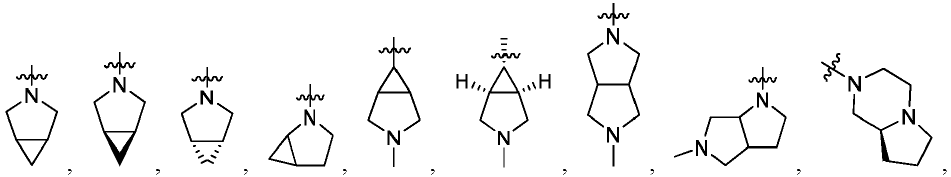 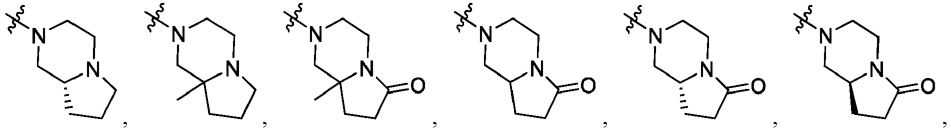 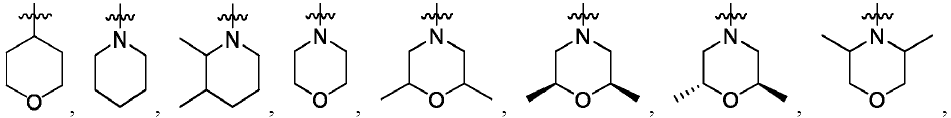 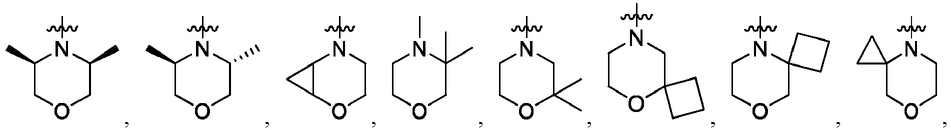 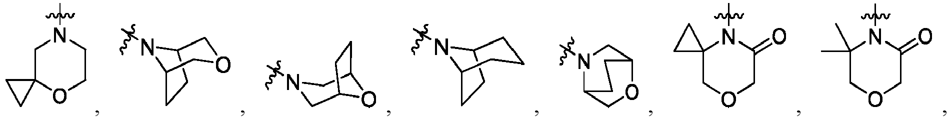 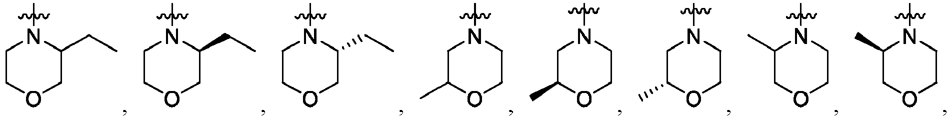 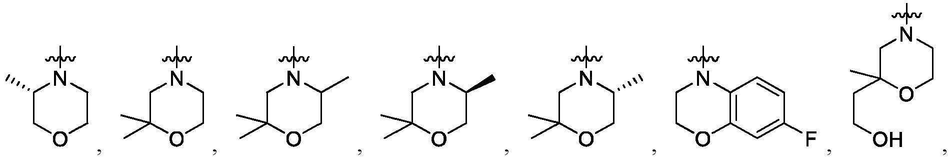 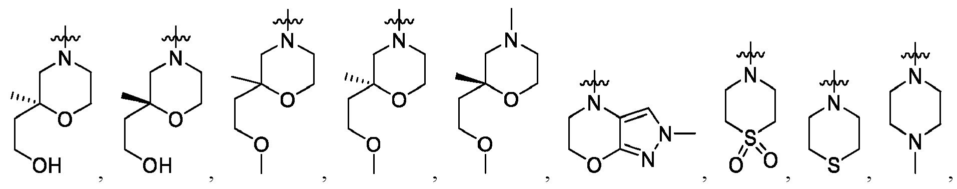 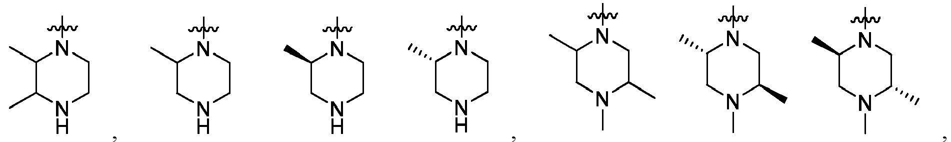 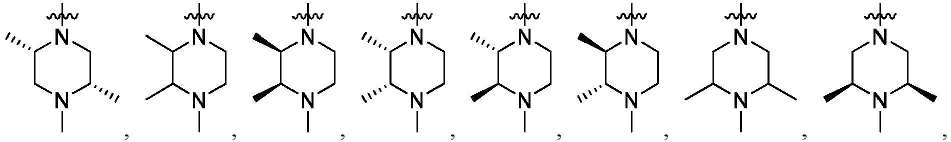 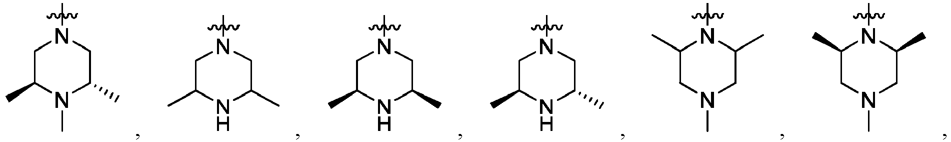 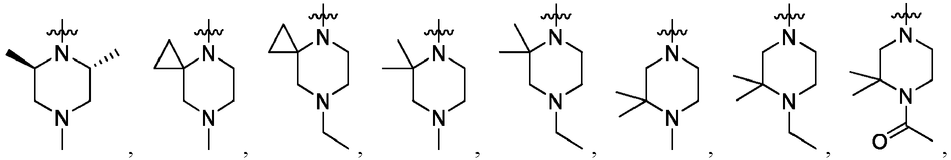 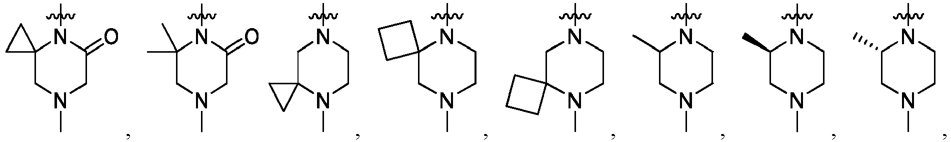 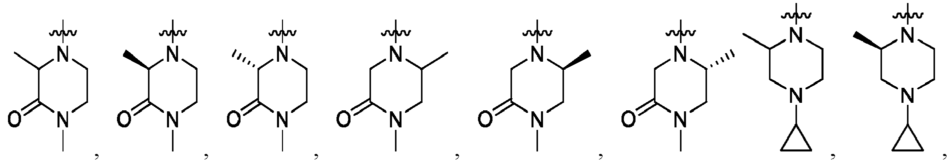 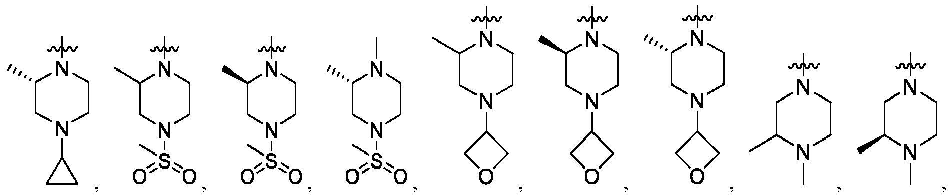 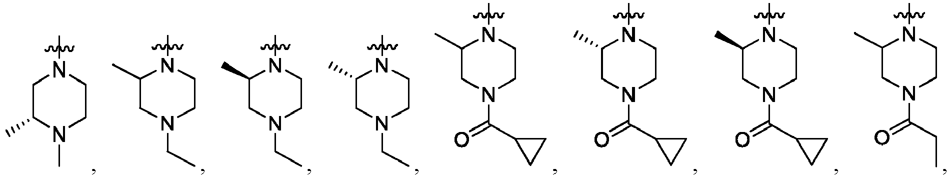 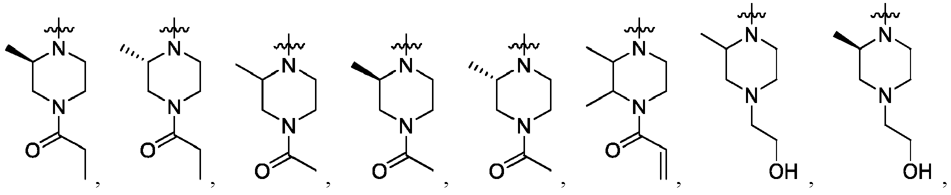 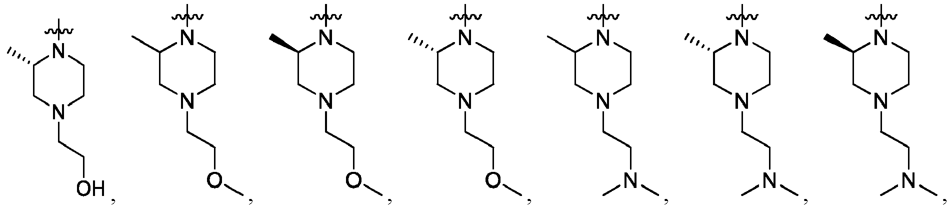 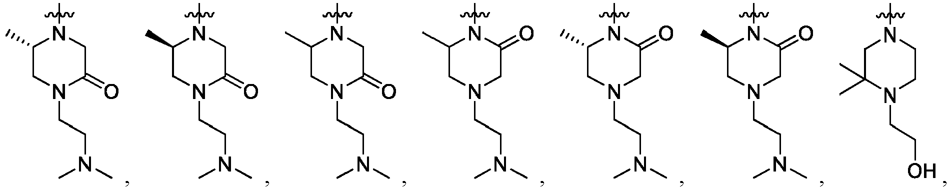 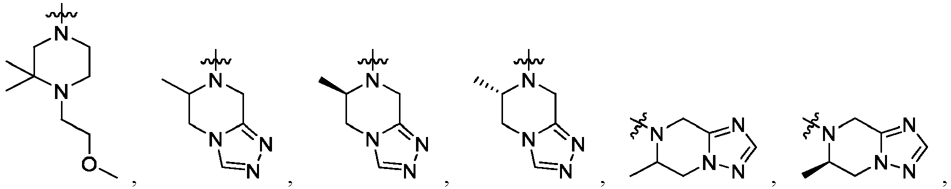 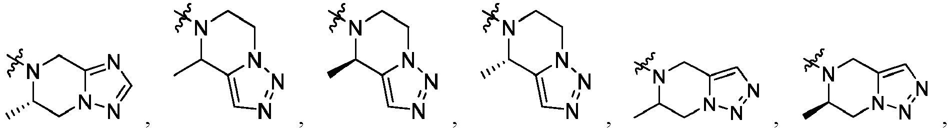 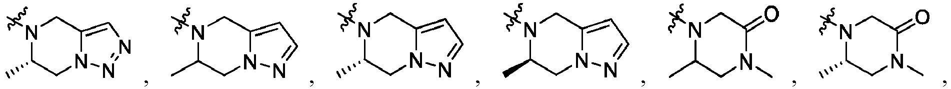 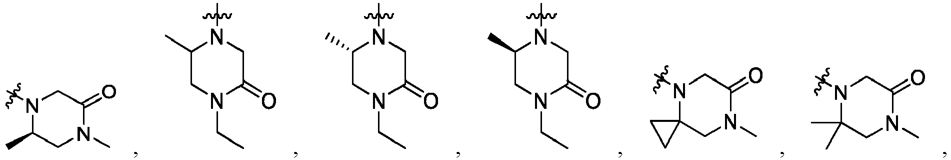 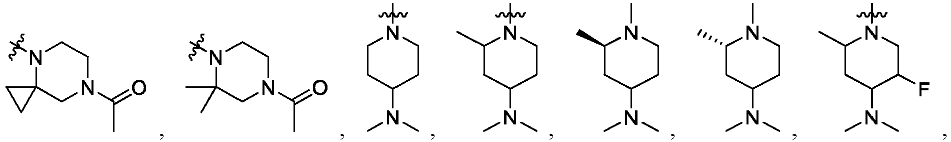 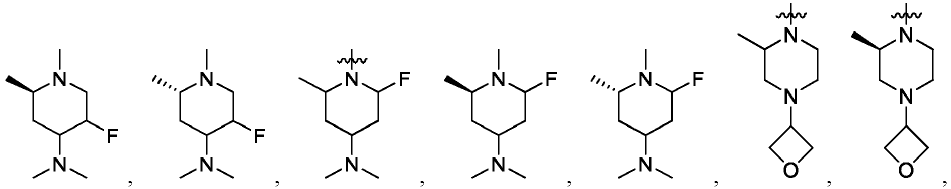 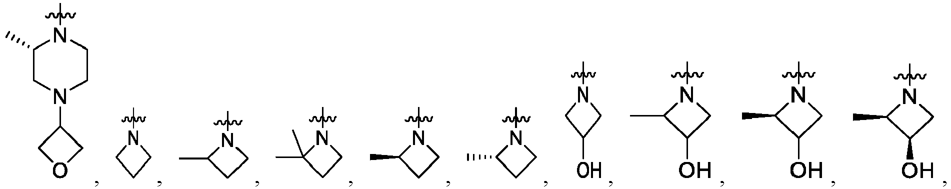 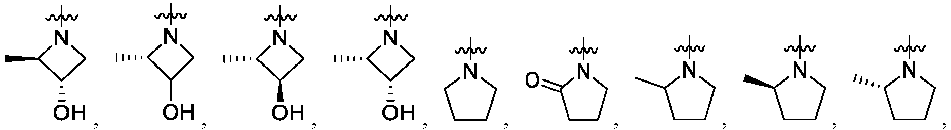 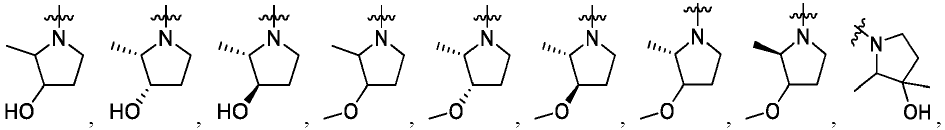 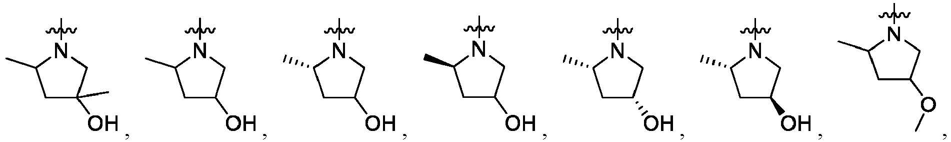 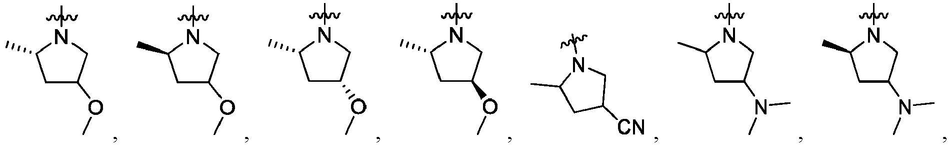 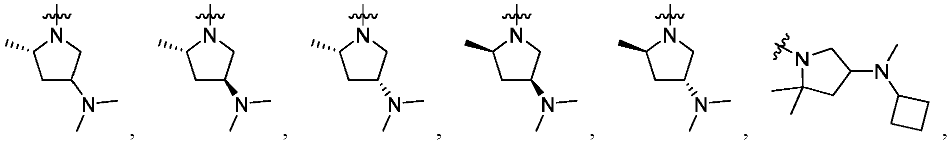 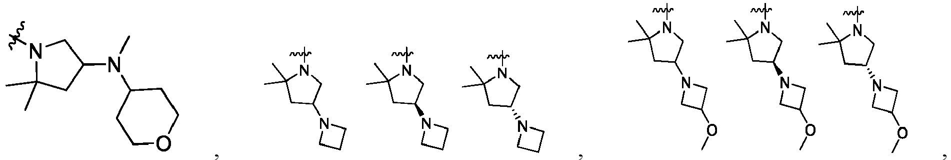 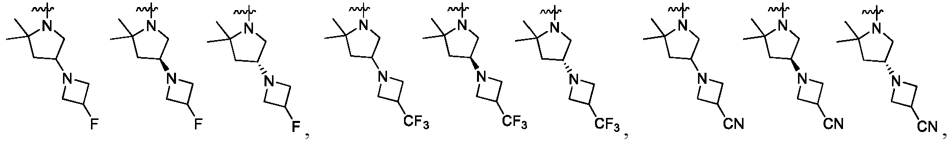 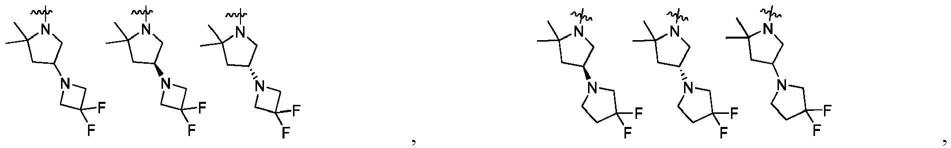 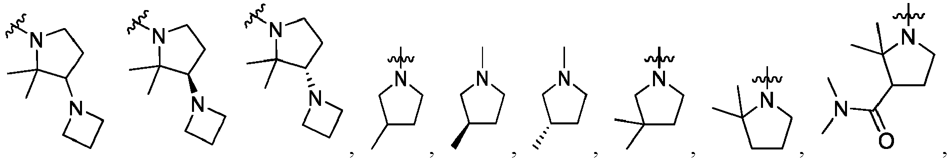 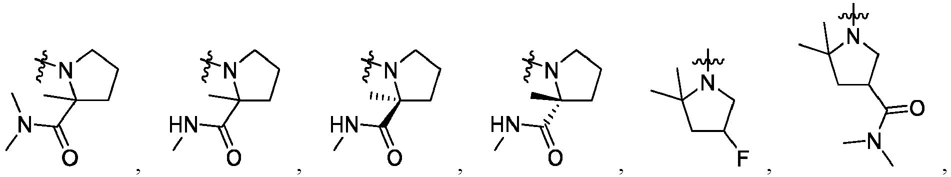 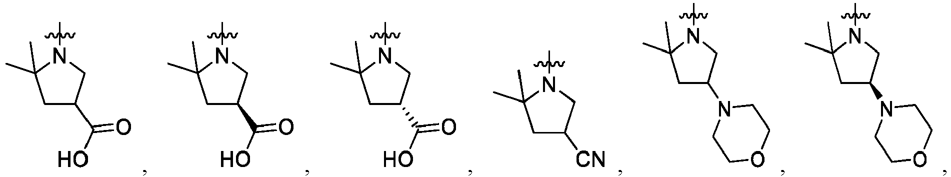 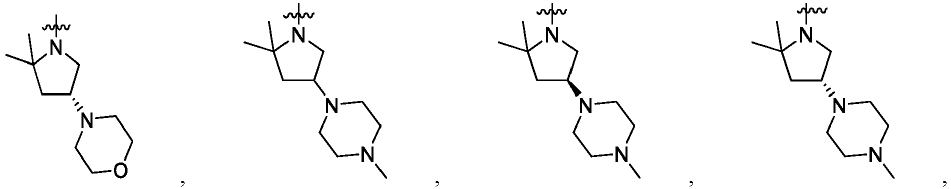 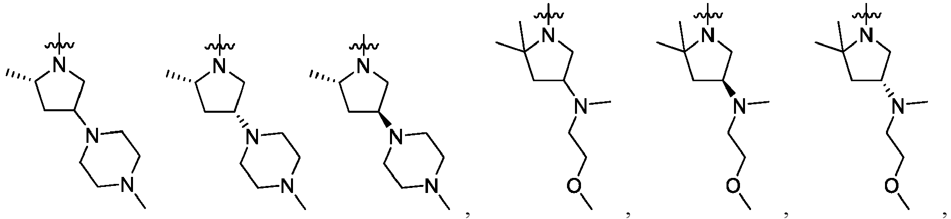 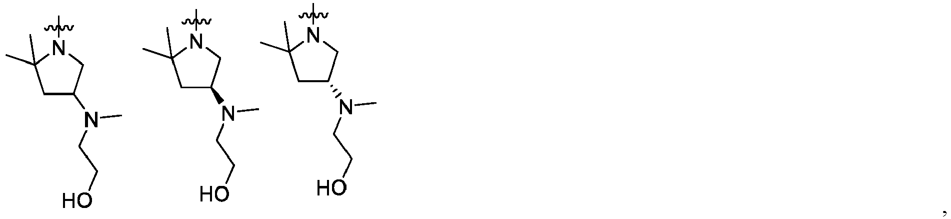 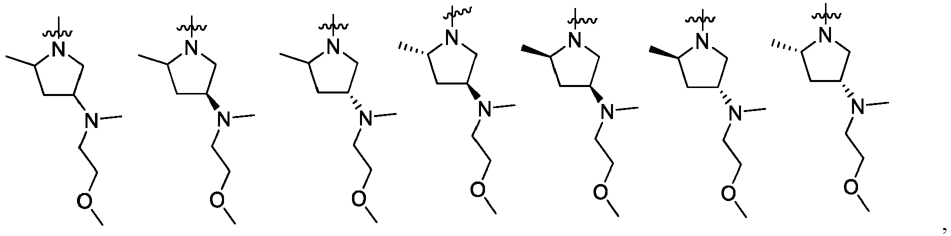 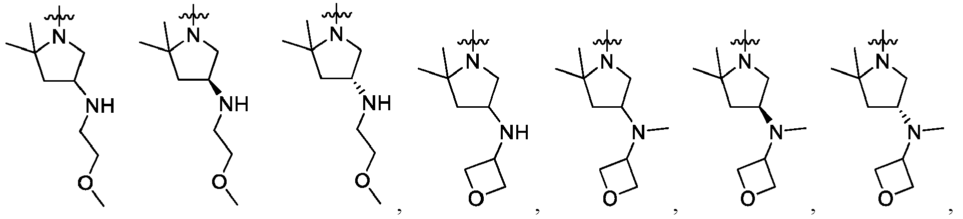 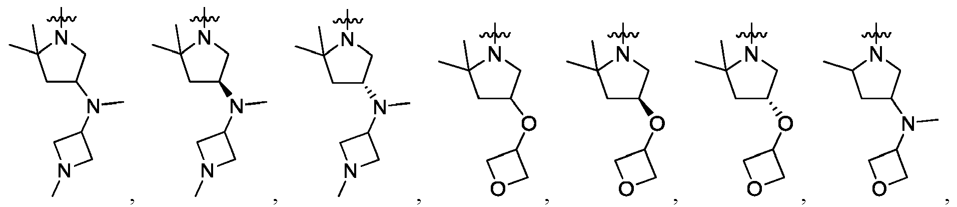 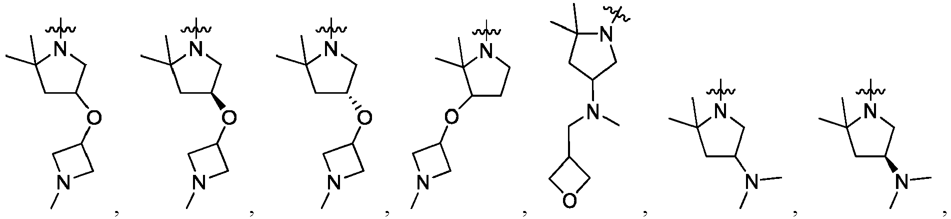 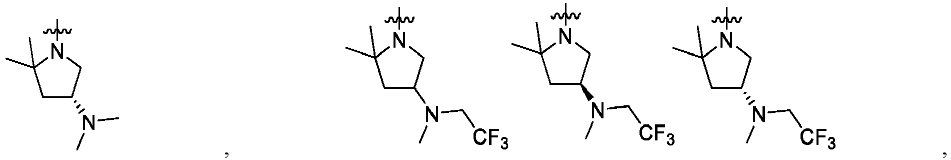 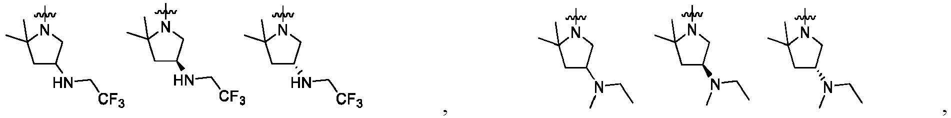 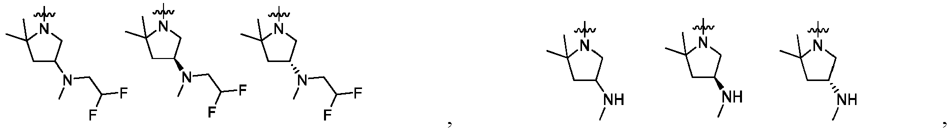 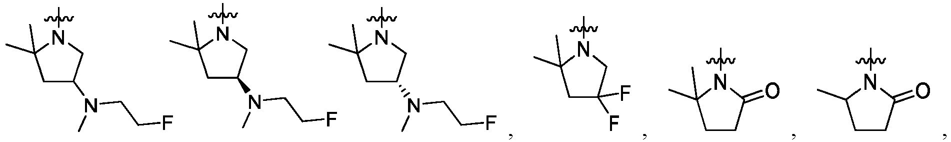 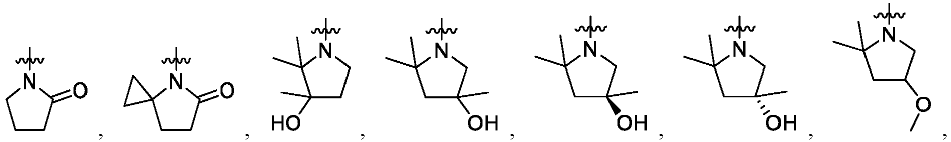 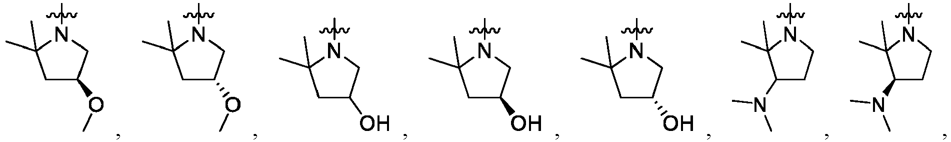 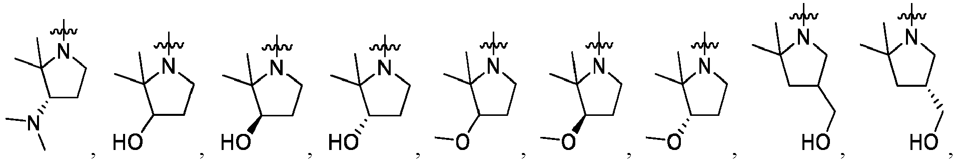 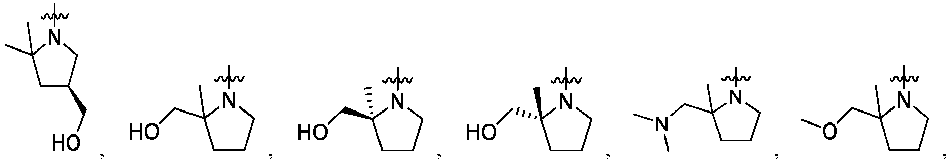 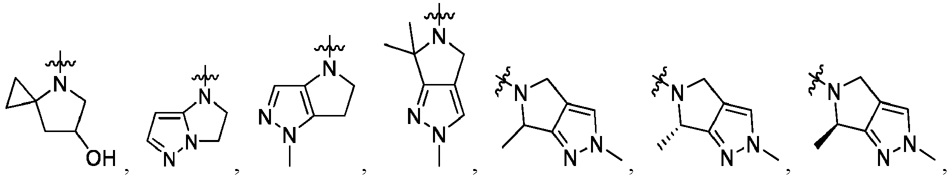 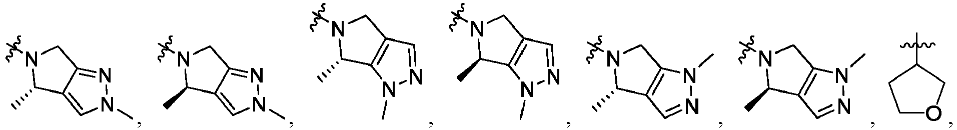 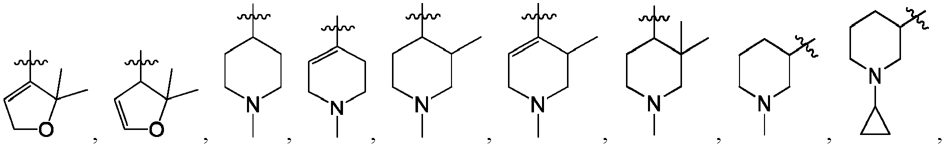 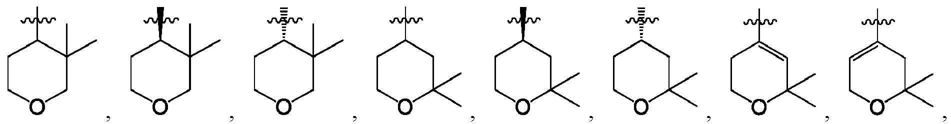 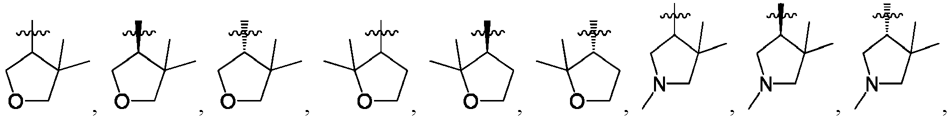 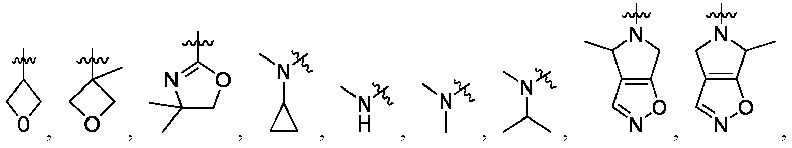 H, NH₂, -CH₃, - C(CH₃)₃, -CH(CH₃)₂,

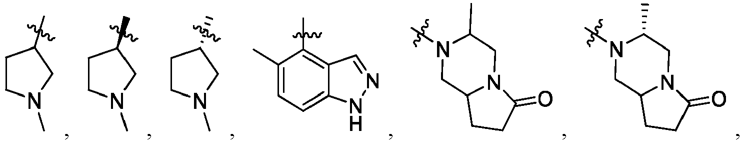

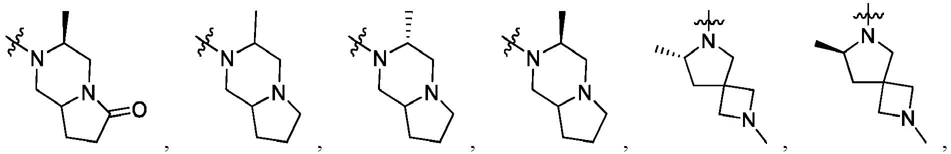

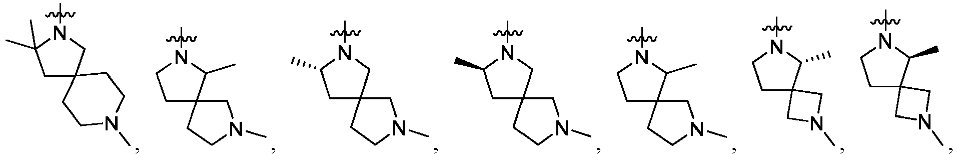

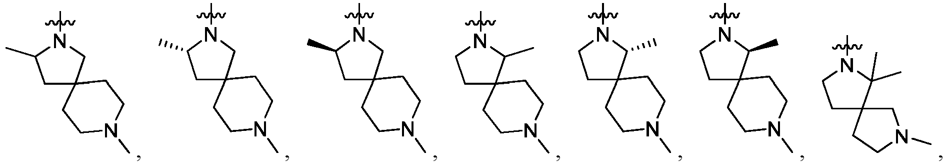

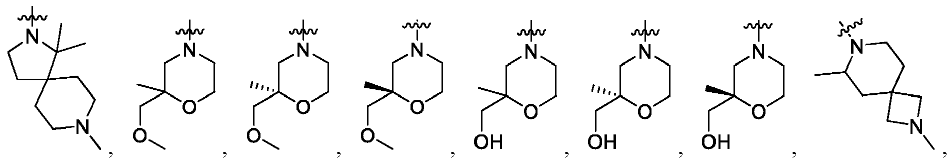

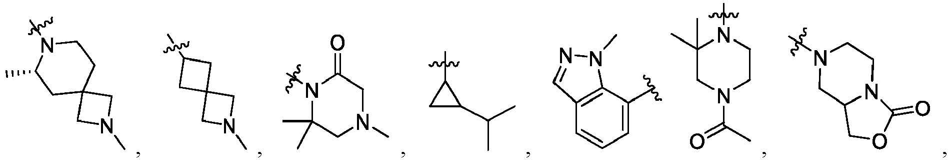

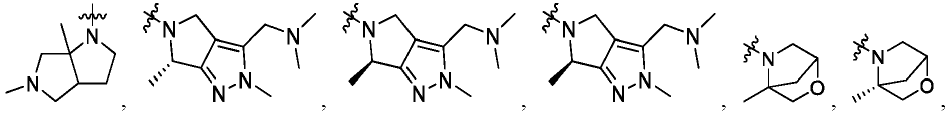

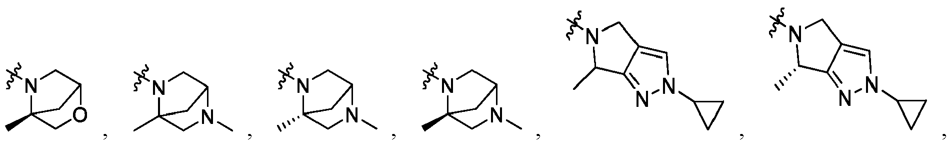

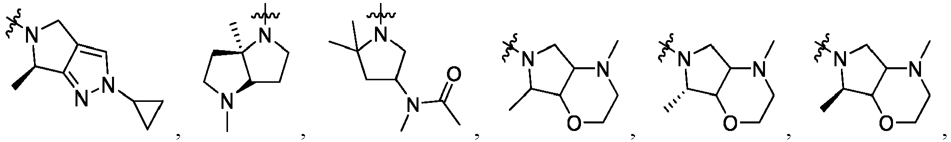

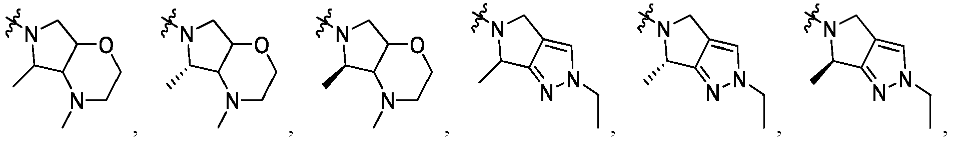

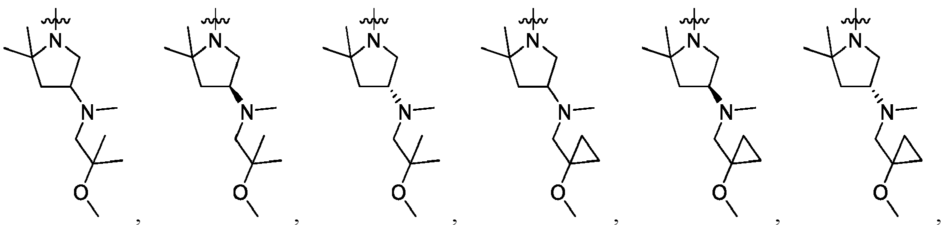

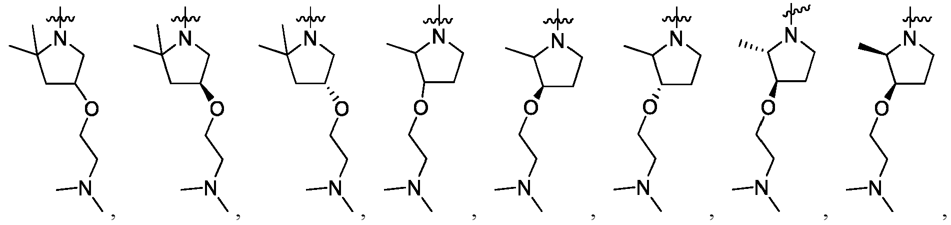

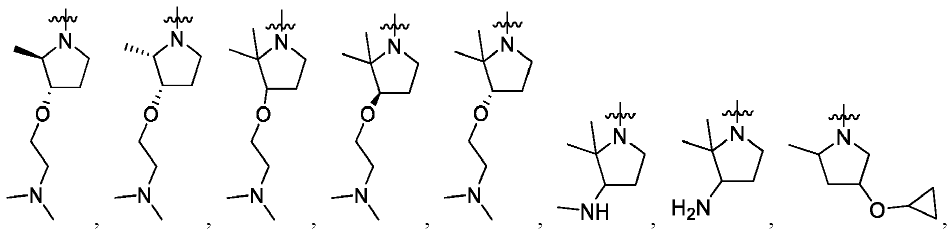

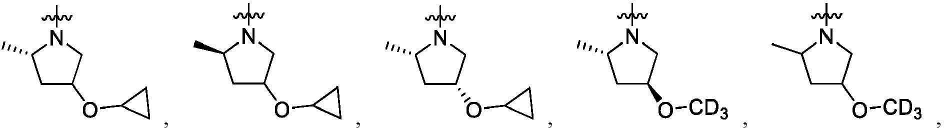

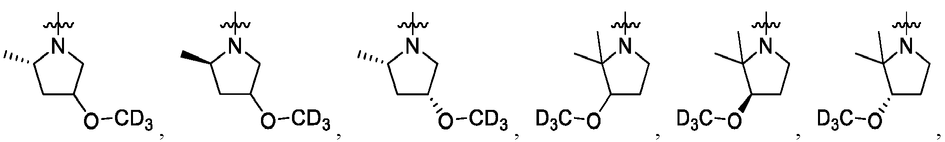

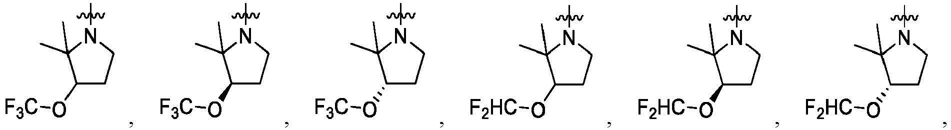

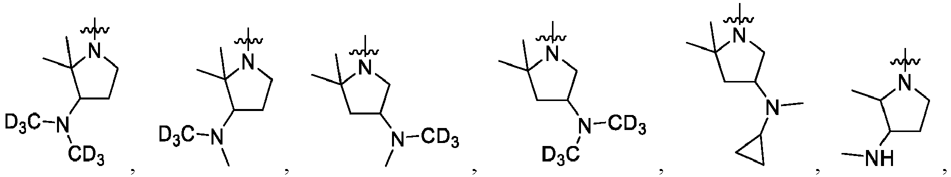

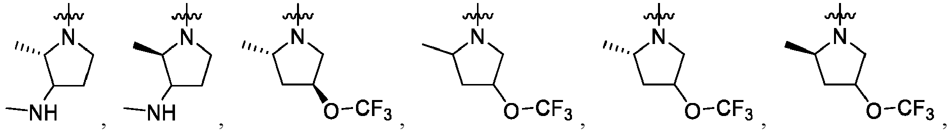

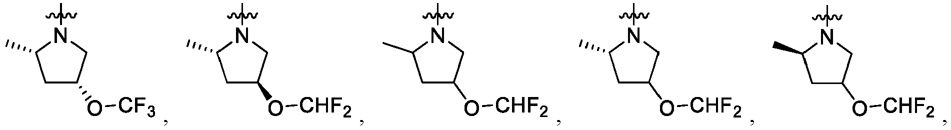

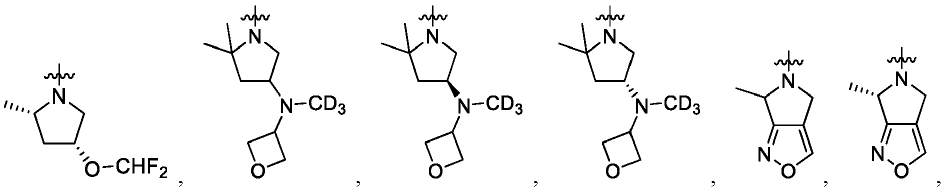

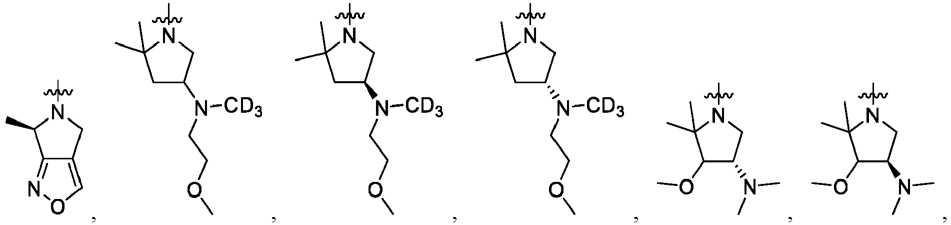

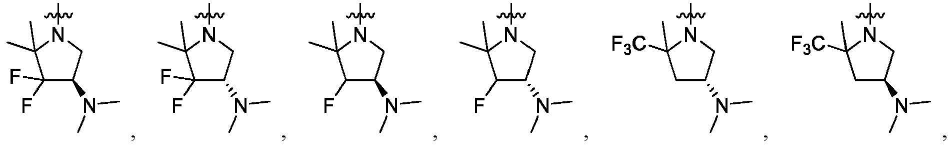

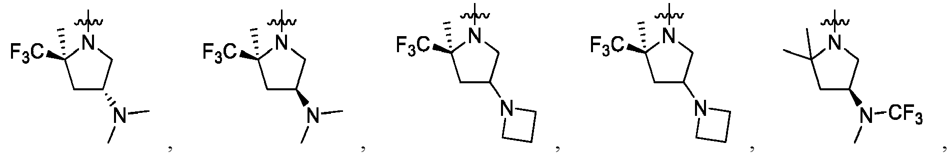

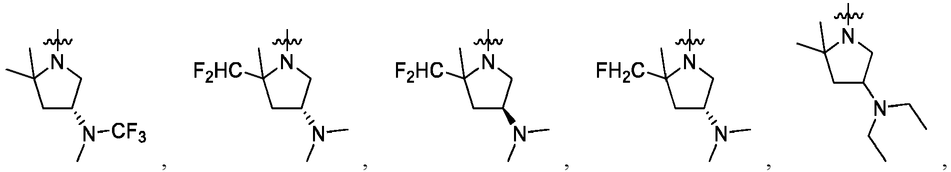

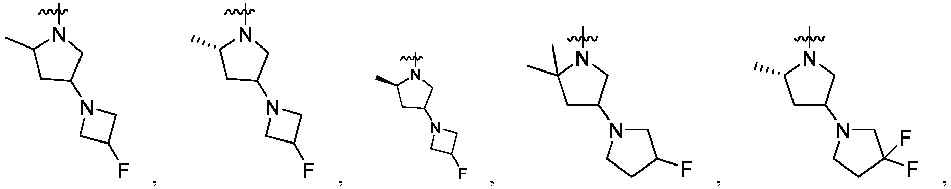

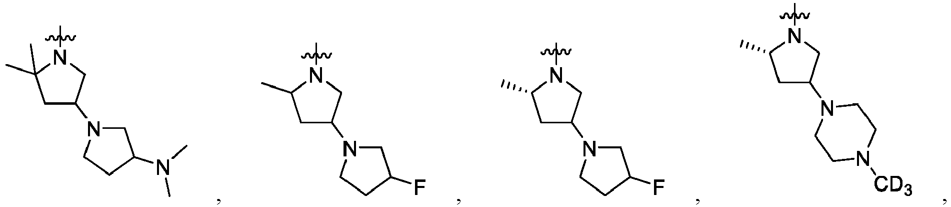

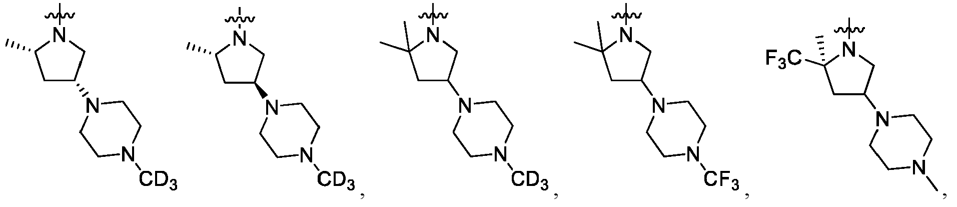

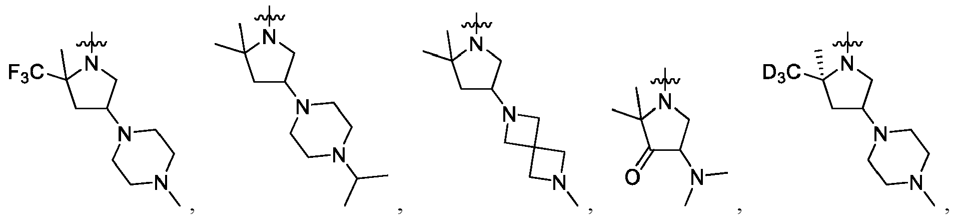

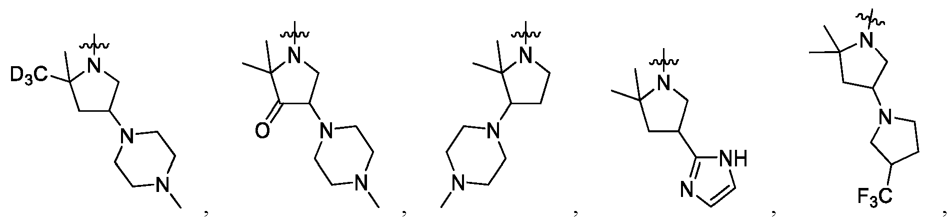

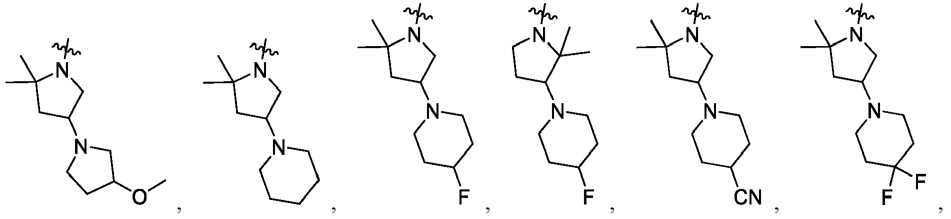

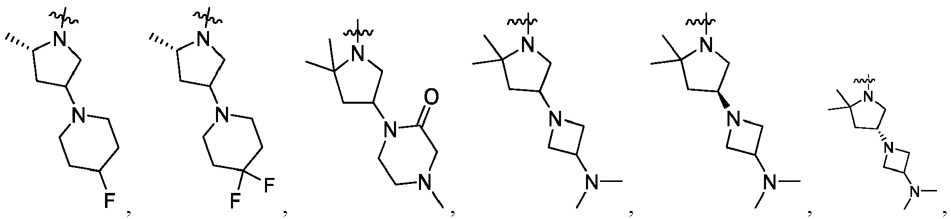

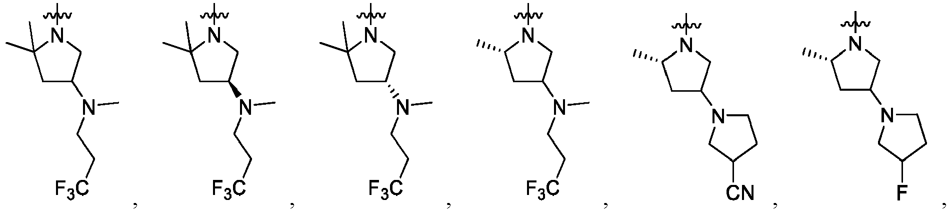

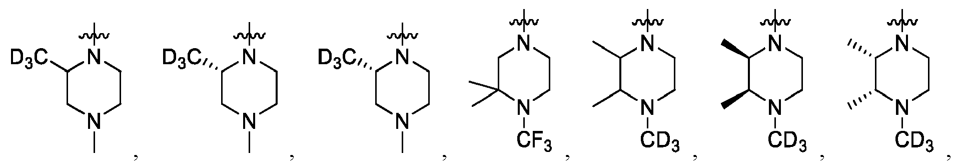

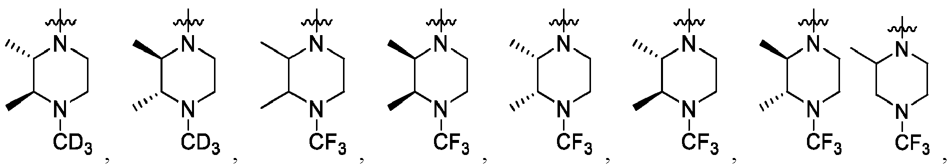

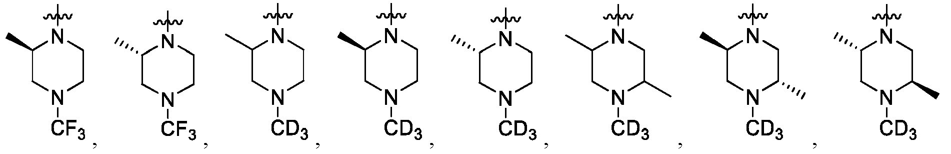

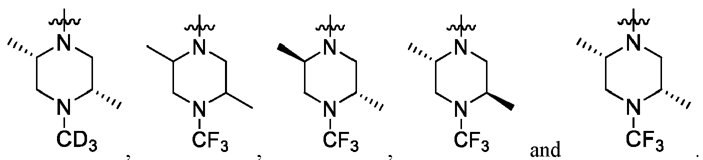

9. The compound, the pharmaceutically acceptable salt thereof, the stereoisomer thereof, the solvate thereof or the prodrug thereof as claimed in any one of claims 1-8, wherein, the compound is shown in formula 1-1, 1-2, II, 11-1, 11-2, III, III-1, III-2, III-1-1, III-1-2, III-1-3, III-1-4, IV, IV-1, IV-2: 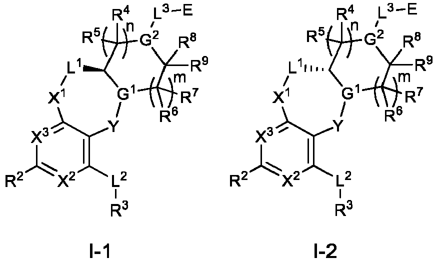 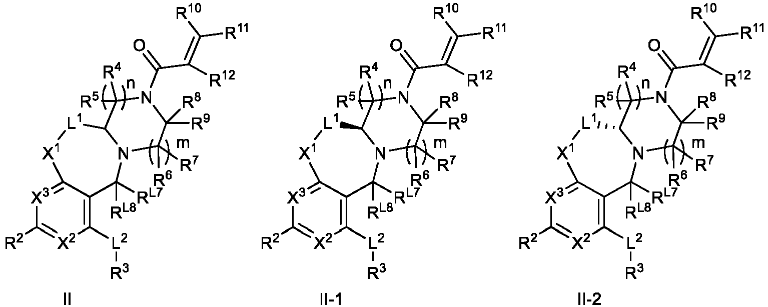 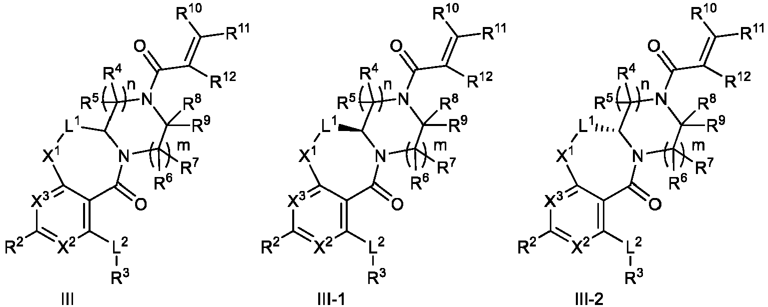 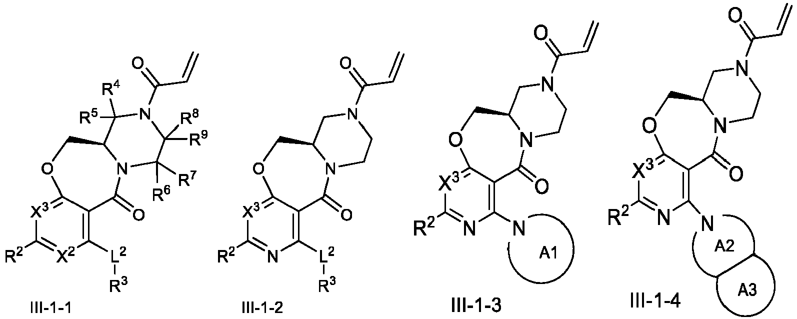 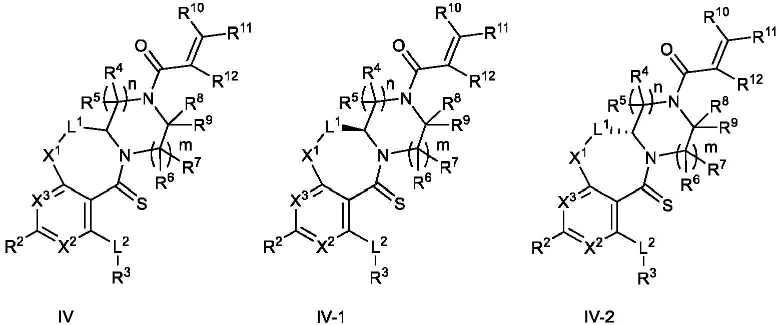
in the each formula, except for the definition of each group below, the definitions of each other letter and group are as defined in any one of claims 1-8; in each formula, R¹⁰, R¹¹, R¹² are each independently hydrogen, halogen, cyano, C₁₋₃ alkyl or N(C₁₋₃ alkyl)₂;
in the compound shown in formula II, 11-1 or 11-2, R^{L7}, R^{L8}, L² and R³ are defined as follows:
R^{L7}, R^{L8} are each independently hydrogen, deuterium, C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; or R^{L7}, R^{L8} combining with the carbon atoms to which they are attached form a C₃₋₆ monocyclic cycloalkyl; L² is O, S or NR^{L5}; wherein, R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b}; R³ is C₁₋₈ alkyl, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1;
or
L² is NH(C=O), S(=O)z or C(=O); R³ is C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1; the R^{e}, R^{f} are each independently H or C₁₋₆ alkyl or R^{e}, R^{f} combining with the nitrogen atoms to which they are attached form a 3- to 6-membered nitrogen-containing heterocyclyl; and when R³ is NR^{e}R^{f}, R^{e}, R^{f} are not simultaneously H; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1;
or
L² is a bond or (CR^{L3}R^{L4})ₘ₁; wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy or -C₁₋₄ alkyl-NR^{a}R^{b}; or R^{L3}, R^{L4} combining with the carbon atoms to which they are attached form a C₃₋₆ monocyclic cycloalkyl; R³ is NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, - L⁴-C₁₋₆ alkoxy, -L⁴-halo C₁₋₆ alkoxy or -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5-to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
in the compound shown in formula III, 111-1, 111-1-1, III-1-2, 111-2, L² and R³ are defined as follows:
L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
in the compound shown in formula III-1-3, X³, R² and 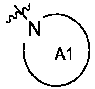 are defined as follows:
R² is selected from the following structures: 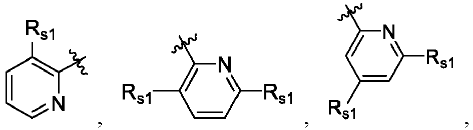 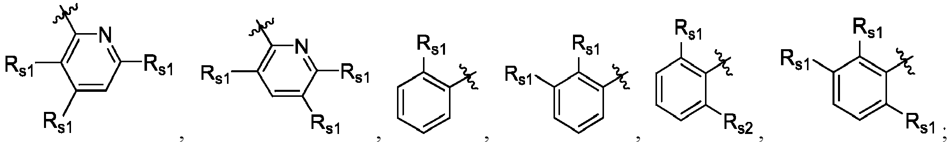 wherein Rₛ₁ of each position is the same or different; Rₛ₁, Rₛ₂ are the same or different; Rₛ₁, Rₛ₂ are each independently halogen, cyano, nitro, hydroxyl, -C₁₋₆ alkyl, -C₁₋₆ alkoxy, -halo C₁₋₆ alkyl, -deuterated C₁₋₆ alkyl, -halo C₁₋₆ alkoxy, -deuterated C₁₋₆ alkoxy, -C₃₋₆ monocyclic cycloalkyl, -NR^{c}R^{d}, -C(O)NR^{c}R^{d}, -SO₂C₁₋₃ alkyl, -SO₂halo C₁₋₃ alkyl, -SO₂NR^{c}R^{d}, -C₁₋₄ alkyl-hydroxyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-3- to 6-membered heterocyclyl, -C₁₋₄ alkyl-NR^{c}R^{d}, -C₁₋₄ alkyl-C(O)NR^{c}R^{d}, -C₁₋₄ alkyl-SO₂C₁₋₃ alkyl or C₂₋₄ alkynyl;
X³ is CR¹; wherein, R¹ is chlorine or fluorine; 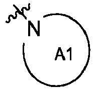 is 3- to 6-membered nitrogen-containing heterocyclyl and the 3- to 6-membered nitrogen-containing heterocyclyl is attached to the rest of the molecule via the nitrogen atom; the 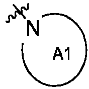 is unsubstituted or substituted by 1, 2, 3 or 4 substituents independently selected from group S1;
in the compound shown in formula III-1-4, X³, R² and 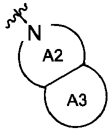 are defined as follows:
R² is 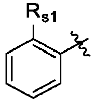 or ; Rₛ₁ is fluorine or chlorine; Rₛ₂ is hydroxyl; X³ is CR¹; wherein, R¹ is chlorine or fluorine; 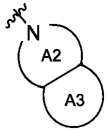 is 8- to 10-membered heteroaryl-heterocyclyl and the 8- to 10-membered heteroaryl-heterocyclyl is attached to the rest of the molecule via the nitrogen atom in ring A2, wherein, ring A2 is 5or 6-membered nitrogen-containing heterocyclyl, ring A3 is 5- or 6-membered monocyclic heteroaryl; the 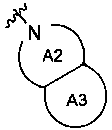 is unsubstituted or substituted by 1, 2, 3 or 4 substituents independently selected from group S1;
in the compound shown in formula IV, IV-1 or IV-2, L² and R³ are defined as follows:
L² is a bond, O, S, (CR^{L3}R^{L4})ₘ₁, NR^{L5}, NH(C=O), S(=O)z or C(=O); wherein, m1 is 1, 2 or 3; R^{L3}, R^{L4} are each independently hydrogen, deuterium, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ monocyclic cycloalkoxy, -C₁₋₄ alkyl-NR^{a}R^{b}; or R^{L3}, R^{L4} combining with the carbon atoms to which they are attached form a C₃₋₆ monocyclic cycloalkyl; R^{L5} is hydrogen, cyano, C₁₋₆ alkyl, deuterated C₁₋₆ alkyl, halo C₁₋₆ alkyl, C₃₋₆ monocyclic cycloalkyl, -C₁₋₄ alkyl-C₁₋₆ alkoxy, -C₁₋₄ alkyl-C₃₋₆ cycloalkoxy or -C₁₋₄ alkyl-NR^{a}R^{b};
R³ is hydrogen, C₁₋₈ alkyl, NR^{e}R^{f}, C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5-to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl, -L⁴-C₆₋₁₄ aryl, -L⁴-5- or 6-membered monocyclic heteroaryl, -L⁴-3- to 6-membered monocyclic heterocyclyl, -L⁴-C₃₋₆ monocyclic cycloalkyl, -L⁴-C₁₋₆ alkoxy, -L⁴-halo C₁₋₆ alkoxy, -L⁴-NR^{e}R^{f}; wherein, -L⁴- is -C₁₋₄ alkyl-; the L⁴ is unsubstituted or wherein 1, 2, 3, or 4 hydrogen atoms are independently substituted by a substituent selected from C₁₋₄ alkyl, halo C₁₋₄ alkyl and deuterated C₁₋₄ alkyl; the 5- or 6-membered monocyclic heteroaryl, 3- to 6-membered monocyclic heterocyclyl have 1, 2 or 3 heteroatoms selected from N, O and S as ring atoms; the 8- to 10-membered bicyclic heteroaryl, 5- to 11-membered fused heterocyclyl, 7- to 11-membered spiroheterocyclyl, 5- to 11-membered bridged heterocyclyl have 1, 2, 3, 4 or 5 heteroatoms selected from N, O and S as ring atoms; the C₆₋₁₄ aryl, 5- or 6-membered monocyclic heteroaryl, 8- to 10-membered bicyclic heteroaryl, C₃₋₆ monocyclic cycloalkyl, 3- to 6-membered monocyclic heterocyclyl, 5- to 11-membered fused cycloalkyl, 5- to 11-membered fused heterocyclyl, 6- to 11-membered spirocycloalkyl, 7- to 11-membered spiroheterocyclyl, 5- to 11-membered bridged cycloalkyl, 5- to 11-membered bridged heterocyclyl are each independently unsubstituted or substituted by 1, 2, 3 or 4 substituents independently selected from group S1;
the R^{a}, R^{b} are each independently H or C₁₋₆ alkyl or R^{a}, R^{b} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms;
the R^{e}, R^{f} are each independently H or C₁₋₆ alkyl or R^{e}, R^{f} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms; the 3- to 6-membered nitrogen-containing heterocyclyl is unsubstituted or substituted by 1, 2, 3 or 4 substituents independently selected from group S1.

10. The compound, the pharmaceutically acceptable salt thereof, the stereoisomer thereof, the solvate thereof or the prodrug thereof as claimed in claim 9, wherein,
in the compound shown in formula III-1-3, 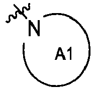 is each independently selected from the following groups: 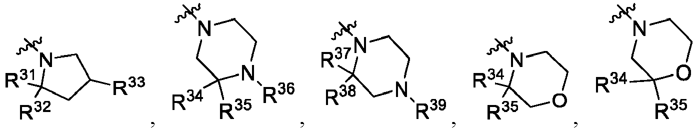 ; in each formula, R³¹, R³², R³³, each R³⁴, each R³⁵, R³⁶, R³⁷, R³⁸, R³⁹ are each independently selected from group S1; preferably selected from the following groups: 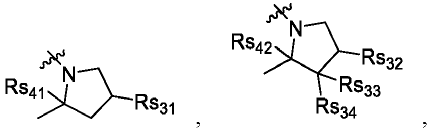 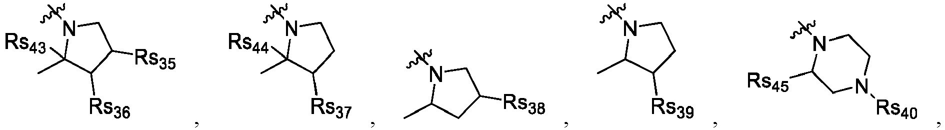 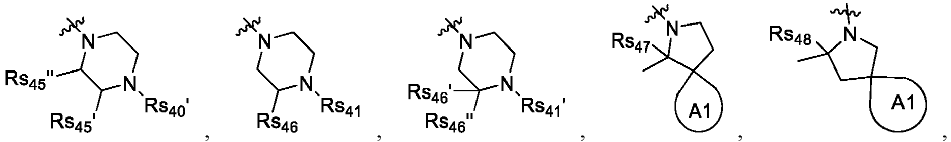 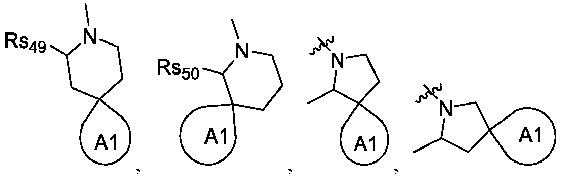 ; in each formula, Rₛ₄₁, Rₛ₄₂, Rₛ₄₃, Rₛ₄₄, Rₛ₄₅, R_{s45'}, R_{s45"}, Rₛ₄₆, R_{s46'}, R_{s46"}, Rₛ₄₇, Rₛ₄₈, Rₛ₄₉, Rₛ₅₀ are each independently selected from the following groups: hydrogen, C₁₋₆ alkyl, halo C₁₋₆ alkyl, deuterated C₁₋₆ alkyl; Rₛ₃₃, Rₛ₃₄, Rₛ₃₆ are each independently selected from the following groups: hydrogen, oxo (=O), halogen, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy; Rₛ₃₁, Rₛ₃₂, Rₛ₃₅, Rₛ₃₇, Rₛ₃₈, Rₛ₃₉, Rₛ₄₀, R_{s40'}, Rₛ₄₁, R_{s41'} are each independently selected from the following groups: hydrogen, halogen, hydroxyl, C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-NR^{c}R^{d}, C(O)C₁₋₆ alkyl, 5- or 6-membered monocyclic heteroaryl, NR^{c}R^{d}; the R^{c}, R^{d} are each independently C₁₋₆ alkyl, halo C₁₋₆ alkyl, deuterated C₁₋₆ alkyl or C₃₋₆ monocyclic cycloalkyl; or R^{c}, R^{d} combining with the nitrogen atoms to which they are attached form a 3- to 6-membered nitrogen-containing heterocyclyl; the 3- to 6-membered nitrogen-containing heterocyclyl has 1 nitrogen atom and optionally 1 or 2 heteroatoms selected from N, O and S as ring atoms and the 3- to 6-membered nitrogen-containing heterocyclyl is optionally substituted by 1 or 2 substituents selected from the following groups: oxo (=O), C₁₋₆ alkyl, C₁₋₆ alkoxy, halo C₁₋₆ alkyl, deuterated C₁₋₆ alkyl, halo C₁₋₆ alkoxy, deuterated C₁₋₆ alkoxy, N(C₁₋₆ alkyl)₂, N(deuterated C₁₋₆ alkyl)₂, N(deuterated C₁₋₆ alkyl)(C₁₋₆ alkyl), halogen; 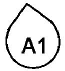 is 4- to 6-membered heterocyclyl, and the 4- to 6-membered heterocyclyl has 1 or 2 heteroatoms selected from N, O and S as ring atoms and is optionally substituted by 1 or 2 substituents selected from the following groups: C₁₋₆ alkyl, halogen; Rₛ₄₁, Rₛ₄₂, Rₛ₄₃, Rₛ₄₄, Rₛ₄₅, R_{s45'}, R_{s45"}, Rₛ₄₆, R_{s46'}, R_{s46"}, Rₛ₄₇, Rₛ₄₈, Rₛ₄₉, Rₛ₅₀ are each independently and preferably selected from the following groups: methyl, deuterated methyl or fluoromethyl;
and/or, in the compound shown in formula III-1-4, 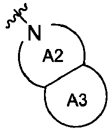
is 8-membered fused bicyclic heteroaryl formed by the 5-membered monocyclic heteroaryl ring fused with one 5-membered monocyclic heterocyclyl ring, or 9-membered fused bicyclic heteroaryl formed by the 5-membered monocyclic heteroaryl ring fused with one 6-membered monocyclic heterocyclyl ring; and 8-membered fused bicyclic heteroaryl formed by the 5-membered monocyclic heteroaryl ring fused with one 5-membered monocyclic heterocyclyl ring is attached to the rest of the molecule via the nitrogen atom in 5-membered monocyclic heterocyclyl ring; 9-membered fused bicyclic heteroaryl formed by the 5-membered monocyclic heteroaryl ring fused with one 6-membered monocyclic heterocyclyl ring is attached to the rest of the molecule via the nitrogen atom in 6-membered monocyclic heterocyclyl ring; and 8-membered fused bicyclic heteroaryl formed by the 5-membered monocyclic heteroaryl ring fused with one 5-membered monocyclic heterocyclyl ring, or 9-membered fused bicyclic heteroaryl formed by the 5-membered monocyclic heteroaryl ring fused with one 6-membered monocyclic heterocyclyl ring are each independently unsubstituted or substituted by 1, 2, 3 or 4 substituents independently selected from group S1;
preferably, 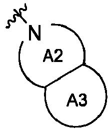 is tetrahydropyrrolopyrazolyl or piperazinotriazole; and the tetrahydropyrrolopyrazolyl is attached to the rest of the molecule via the nitrogen atom in tetrahydropyrrole ring; the piperazinotriazole is attached to the rest of the molecule via the nitrogen atom in piperazine ring; and the tetrahydropyrrolopyrazolyl or piperazinotriazole is unsubstituted or substituted by 1, 2, 3 or 4 substituents independently selected from group S1; for example, the tetrahydropyrrolopyrazolyl is attached to the rest of the molecule via the nitrogen atom in tetrahydropyrrole ring and one hydrogen atom on the ortho carbon atom of the nitrogen atom is substituted by methyl; the piperazinotriazole is attached to the rest of the molecule via the nitrogen atom in piperazine ring and one hydrogen atom on the ortho carbon atom of the nitrogen atom is substituted by methyl; and the hydrogen atoms in the rest positions of the tetrahydropyrrolopyrazolyl or piperazinotriazole are optionally substituted by 1, 2 or 3 substituents independently selected from group S1; for another example, the tetrahydropyrrolopyrazolyl is attached to the rest of the molecule via the nitrogen atom in tetrahydropyrrole ring and two hydrogen atoms on the same ortho carbon atom of the nitrogen atom are both substituted by methyl; the piperazinotriazole is attached to the rest of the molecule via the nitrogen atom in piperazine ring and two hydrogen atoms on the same ortho carbon atom of the nitrogen atom are both substituted by methyl; and the hydrogen atoms in the rest positions of the tetrahydropyrrolopyrazolyl or piperazinotriazole are optionally substituted by 1 or 2 substituents independently selected from group S1.

11. The compound, the pharmaceutically acceptable salt thereof, the stereoisomer thereof, the solvate thereof or the prodrug thereof as claimed in any one of claims 1-10, wherein, the compound is any one of the following compounds:
2-acryloyl-10-chloro-7-((2-isopropylphenyl)amino)-9-(5-methyl-1*H*-indazol-4-yl)-1,2,3,4,12,12a-hexahydro-6*H*-benzo[*f*]pyrazino[2,1-*c*][1,4]oxazepin-6-one
Z1: (12a*R*)-2-acryloyl-10-chloro-7-((2-isopropylphenyl)amino)-9-(5-methyl-1*H*-indazol-4-yl)-1,2,3,4,12,12a-hexahydro-6*H*-benzo[*f*]pyrazino[2,1-*c*][1,4]oxazepin-6-one
Z2: (12a*S*)-2-acryloyl-10-chloro-7-((2-isopropylphenyl)amino)-9-(5-methyl-1*H*-indazol-4-yl)-1,2,3,4,12,12a-hexahydro-6*H*-benzo[*f*]pyrazino[2,1-*c*][1,4]oxazepin-6-one
Z3: 2-acryloyl-10-chloro-9-(5-methyl-1*H*-indazol-4-yl)-1,2,3,4,12,12a-hexahydro-6*H-*benzo [*f*]pyrazino[2,1-*c*][1,4]oxazepin-6-one
Z4: 2-acryloyl-7-amino-10-chloro-9-(5-methyl-1*H*-indazol-4-yl)-1,2,3,4,12,12a-hexahydro-6*H-*benzo [*f*]pyrazino[2,1-*c*][1,4]oxazepin-6-one
Z5: (6a*R*)-8-acryloyl-4-chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one
Z6: (6a*R*)-8-acryloyl-4-chloro-3-(2-fluoro-6-hydroxyphenyl)-1-((2-isopropyl-4-methylpyridin-3-yl)(methyl)amino)-6,6a,7,8,9,10-hexahydro-12*H*-pyrazino[2,1-c]pyrido[3,4-*f*][1,4]oxazepin-12-one
Z7: (6a*S*)-8-acryloyl-4-chloro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one
Z8: (*R*)-8-acryloyl-4-chloro-3-(2-fluorophenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one
Z9: (6a*R*)-8-acryloyl-4-fluoro-3-(2-fluoro-6-hydroxyphenyl)-1-((2-isopropyl-4-methylpyridin-3-yl)amino)-6,6a,7,8,9,10-hexahydro-12*H*-pyrazino[2,1-*c*]pyrido[3,4-*f*][1,4]oxazepin-12-one
Z10: (6a*R*)-8-acryloyl-4-chloro-3-(2-fluoro-6-hydroxyphenyl)-1-morpholino-6,6a,7,8,9,10-hexahydro-12*H*-pyrazino[2,1-*c*]pyrido[3,4-*f*][1,4]oxazepin-12-one
Z11: (6a*R*)-8-acryloyl-4-chloro-3-(2-fluoro-6-hydroxyphenyl)-1-(pyrrolidin-1-yl)-6,6a,7,8,9,10-hexahydro-12*H*-pyrazino[2,1-*c*]pyrido[3,4-*f*][1,4]oxazepin-12-one
Z13: (6a*R*)-8-acryloyl-4-chloro-3-(2-fluoro-6-hydroxyphenyl)-1-(((*R*)-1-methylpyrrolidin-2-yl)methoxy)-6,6a,7,8,9,10-hexahydro-12*H*-pyrazino[2,1-*c*]pyrido[3,4-*f*][1,4]oxazepin-12-one
Z14: (6a*R*)-8-acryloyl-4-chloro-3-(2-fluoro-6-hydroxyphenyl)-1-(((*S*)-1-methylpyrrolidin-2-yl)methoxy)-6,6a,7,8,9,10-hexahydro-12*H*-pyrazino[2,1-*c*]pyrido[3,4-*f*][1,4]oxazepin-12-one
Z15: (6a*R*)-8-acryloyl-4-chloro-1-((4,6-diisopropylpyrimidin-5-yl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z16: (6a*R*)-8-acryloyl-4-chloro-1-((3 -chloro-1-isopropyl-4-methyl-1*H*-pyrazol-5-yl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z17: (6a*R*)-8-acryloyl-4-chloro-3 -(2-fluoro-6-hydroxyphenyl)-1-methoxy-6,6a,7,8,9,10-hexahydro-12*H*-pyrazino[2,-c]pyrido[3,4-*f*][1,4]oxazepin-12-one
Z18: (6a*R*)-8-acryloyl-4-chloro-3-(2-fluoro-6-hydroxyphenyl)-1-(2-isopropylphenoxy)-6,6a,7,8,9,10-hexahydro-12*H*-pyrazino[2,1-*c*]pyrido[3,4-*f*][1,4]oxazepin-12-one
Z19: 1-((6a*R*)-4-chloro-3-(2-fluoro-6-hydroxyphenyl)-1-((2-isopropyl-4-methylpyridin-3-yl)amino)-6a,7,9,10-tetrahydro-12*H*-pyrazino[2,1-*c*]pyrido[3,4-*f*][1,4]oxazepin-8(6*H*-yl)prop-2-en-1-one
Z20: (6a*R*)-8-acryloyl-4-chloro-1-((4,6-diisopropylpyrimidin-5-yl)(methyl)amino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z21: (6a*R*)-8-acryloyl-4-chloro-1-(3,3-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z22: (6a*R*)-8-acryloyl-4-chloro-1-(2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z23: (6a*R*)-8-acryloyl-4-chloro-3-(2-fluoro-6-hydroxyphenyl)-1-((*S*)-2-methylmorpholino)-6,6a,7,8,9,10-hexahydro-12*H*-pyrazino[2,1-*c*]pyrido[3,4-*f*][1,4]oxazepin-12-one
Z24: (6a*R*)-8-acryloyl-4-chloro-3-(2-fluoro-6-hydroxyphenyl)-1-((*R*)-2-methylmorpholino)-6,6a,7,8,9,10-hexahydro-12*H*-pyrazino[2,1-*c*]pyrido[3,4-*f*][1,4]oxazepin-12-one
Z25: (6a*R*)-8-acryloyl-4-chloro-3-(2-fluoro-6-hydroxyphenyl)-1-(methyl(tetrahydro-2*H*-pyran-4-yl)amino)-6,6a,7,8,9,10-hexahydro-12*H*-pyrazino[2,1-*c*]pyrido[3,4-*f*][1,4]oxazepin-12-one
Z26: (6a*R*)-8-acryloyl-4-chloro-1-(1,1-dioxidothiomorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z27: (6a*R*)-8-acryloyl-4-chloro-3-(2-fluoro-6-hydroxyphenyl)-1-((*R*)-3-methylmorpholino)-6,6a,7,8,9,10-hexahydro-12*H*-pyrazino[2,1-*c*]pyrido[3,4-*f*][1,4]oxazepin-12-one
Z28: (6a*R*)-8-acryloyl-4-chloro-3-(2-fluoro-6-hydroxyphenyl)-1-(((*R*)-1-phenylethyl)amino)-6,6a,7,8,9,10-hexahydro-12*H*-pyrazino[2,1-*c*]pyrido[3,4-*f*][1,4]oxazepin-12-one
Z29: (6a*R*)-8-acryloyl-4-chloro-3-(2-fluoro-6-hydroxyphenyl)-1-(((*S*)-1-phenylethyl)amino)-6,6a,7,8,9,10-hexahydro-12*H*-pyrazino[2,1-*c*]pyrido[3,4-*f*][1,4]oxazepin-12-one
Z30: (6a*R*)-8-acryloyl-4-chloro-3-(2-fluoro-6-hydroxyphenyl)-1-(4-methylpiperazin-1-yl)-6,6a,7,8,9,10-hexahydro-12*H*-pyrazino[2,1-*c*]pyrido[3,4-*f*][1,4]oxazepin-12-one
Z31: (6a*R*)-8-acryloyl-4-chloro-1-(4-(dimethylamino)piperidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z32: (6a*R*)-8-acryloyl-4-chloro-3-(2-fluoro-6-hydroxyphenyl)-1-((*S*)-3-methylmorpholino)-6,6a,7,8,9,10-hexahydro-12*H*-pyrazino[2,1-*c*]pyrido[3,4-*f*][1,4]oxazepin-12-one
Z33: *N*-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-3,3-dimethylbutanamide
Z34: (6a*R*)-8-acryloyl-4-chloro-3-(2-fluoro-6-hydroxyphenyl)-1-(2-azaspiro[4.4]nonan-2-yl)-6,6a,7,8,9,10-hexahydro-12*H*-pyrazino[2,1-*c*]pyrido[3,4-*f*][1,4]oxazepin-12-one
Z35: (6a*R*)-8-acryloyl-4-chloro-3-(2-fluoro-6-hydroxyphenyl)-1-(6-azaspiro[3.4]octan-6-yl)-6,6a,7,8,9,10-hexahydro-12*H*-pyrazino[2,1-*c*]pyrido[3,4-*f*][1,4]oxazepin-12-one
Z36: (6a*R*)-8-acryloyl-4-chloro-3-(2-fluoro-6-hydroxyphenyl)-1-(5-oxa-8-azaspiro[3.5]nonan-8-yl)-6,6a,7,8,9,10-hexahydro-12*H*-pyrazino[2,1-*c*]pyrido[3,4-*f*][1,4]oxazepin-12-one
Z37: (6a*R*)-8-acryloyl-4-chloro-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z38: (6a*R*)-8-acryloyl-1-((1*R*,5*S*)-3-oxa-8-azabicyclo[3.2.1]octan-8-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z39: (6a*R*)-8-acryloyl-1-(2-azabicyclo[3.1.0]hexan-2-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z40: (6a*R*)-8-acryloyl-1-((1-methylpiperidin-4-yl)methoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z41: (6a*R*)-8-acryloyl-1-(bicyclo[1.1.1]pentan-1-ylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z42: (6a*R*)-8-acryloyl-4-chloro-1-((2,2-dimethyl-2,3-dihydrobenzofuran-7-yl)oxy)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z43: (6a*R*)-8-acryloyl-4-chloro-3-(2-fluoro-6-hydroxyphenyl)-1-((1-methylpiperidin-3-yl)oxy)-6,6a,7,8,9,10-hexahydro-12*H*-pyrazino[2,1-*c*]pyrido[3,4-*f*][1,4]oxazepin-12-one
Z44: (6a*R*)-8-acryloyl-4-chloro-3-(2-fluoro-6-hydroxyphenyl)-1-(5-azaspiro[2.4]heptan-5-yl)-6,6a,7,8,9,10-hexahydro-12*H*-pyrazino[2,1-*c*]pyrido[3,4-*f*][1,4]oxazepin-12-one
Z45: (6a*R*)-8-acryloyl-4-chloro-3-(2-fluoro-6-hydroxyphenyl)-1-(2-oxa-6-azaspiro[3.4]octan-6-yl)-6,6a,7,8,9,10-hexahydro-12*H*-pyrazino[2,1-*c*]pyrido[3,4-*f*][1,4]oxazepin-12-one
Z46: (6a*R*)-8-acryloyl-4-chloro-3-(2-fluoro-6-hydroxyphenyl)-1-(((1*R*,5*S*,6*s*)-3-methyl-3-azabicyclo [3.1.0]hexan-6-yl)amino)-6,6a,7,8,9,10-hexahydro-12*H*-pyrazino[2,1-*c*]pyrido[3,4-*f*][1,4]oxazepin-12-one
Z47: (6a*R*)-8-acryloyl-4-chloro-3-(2-fluoro-6-hydroxyphenyl)-1-(5-methylhexahydropyrrolo[3,4-*b*]pyrrol-1(2*H*-yl)-6,6a,7,8,9,10-hexahydro-12*H*-pyrazino[2,1-*c*]pyrido[3,4-*f*][1,4]oxazepin-12-one
Z48: (6a*R*)-8-acryloyl-1-((*R*)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z49: (6a*R*)-8-acryloyl-1-((*S*)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z51: (6a*R*)-8-acryloyl-4-chloro-1-((*S*)-2,4-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z52: (6a*R*)-8-acryloyl-4-chloro-1-((*S*)-3-ethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z264: (6a*R*)-8-acryloyl-4-chloro-3-(2-fluoro-6-hydroxyphenyl)-1-thiomorphofino-6,6a,7,8,9,10-hexahydro-12*H*-pyrazino[2,1-*c*]pyrido[3,4-*f*][1,4]oxazepin-12-one
Z60: (6a*R*)-8-acryloyl-4-chloro-3-(2-fluoro-6-hydroxyphenyl)-1-(5-methylhexahydropyrrolo[3,4-*c*]pyrrol-2(1*H*)-yl)-6,6a,7,8,9,10-hexahydro-12*H*-pyrazino[2,1-*c*]pyrido[3,4-*f*][1,4]oxazepin-12-one
Z62: (6a*R*)-8-acryloyl-4-chloro-1-((1*R*,5*S*)-8-oxa-3-azabicyclo[3.2.1]octan-3-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z63: (6a*R*)-8-acryloyl-4-chloro-1-(4-oxa-7-azaspiro[2.5]octan-7-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z68: (6a*R*)-8-acryloyl-1-((*S*)-2-methylazetidin-1-yl)-4-chloro-3 -(2-fluoro-6-hydroxyphenyl)-6,6a,7,8,9,10-hexahydro-12*H*-pyrazino[2,1-*c*]pyrido[3,4-*f*][1,4]oxazepin-12-one
Z69: (6a*R*)-8-acryloyl-1-(*tert*-butylamino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z75: (6a*R*)-8-acryloyl-4-chloro-1-(isopropylamino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z76: (6a*R*)-8-acryloyl-4-chloro-1-(2-oxa-5 -azabicyclo [4.1.0]heptan-5-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z12: (6a*R*)-8-acryloyl-1-((2-(dimethylamino)ethoxy)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z61: (6a*R*)-8-acryloyl-4-chloro-3-(2-fluoro-6-hydroxyphenyl)-1-((1-methylpiperidin-4-yl)oxy)-6,6a,7,8,9,10-hexahydro-12*H*-pyrazino[2,1-*c*]pyrido[3,4-*f*][1,4]oxazepin-12-one
Z64: (6a*R*)-8-acryloyl-4-chloro-3-(2-fluoro-6-hydroxyphenyl)-1-(((*R*)-1-methylpyrrolidin-3-yl)oxy)-6,6a,7,8,9,10-hexahydro-12*H*-pyrazino[2,1-*c*]pyrido[3,4-*f*][1,4]oxazepin-12-one
Z65: (6a*R*)-8-acryloyl-4-chloro-3-(2-fluoro-6-hydroxyphenyl)-1-(((*S*)-1-methylpyrrolidin-3-yl)oxy)-6,6a,7,8,9,10-hexahydro-12*H*-pyrazino[2,1-*c*]pyrido[3,4-*f*][1,4]oxazepin-12-one
Z50: (6a*R*)-8-acryloyl-4-chloro-3-(2-fluoro-6-hydroxyphenyl)-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z81: (6a*R*)-8-acryloyl-4-chloro-3-(2-fluoro-6-hydroxyphenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z82: (6a*R*)-8-acryloyl-4-chloro-3-(2-fluoro-6-hydroxyphenyl)-1-((*R*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z53: (6a*R*)-8-acryloyl-4-chloro-3 -(2-fluoro-6-hydroxyphenyl)-1 -phenyl-6,6a,7,8,9,10-hexahydro-12H-pyrazino [2,1 -*c*]pyrido[3,4-*f*]oxazepin-12-one
Z54: (6a*R*)-8-acryloyl-4-chloro-3-(2-fluoro-6-hydroxyphenyl))-1-(pyridin-4-yl)-6,6a,7,8,9,10-hexahydro-12*H*-pyrazino[2,1-*c*]pyrido[3,4-*f*]oxazepin-12-one
Z55: (6a*R*)-8-acryloyl-4-chloro-3-(2-fluoro-6-hydroxyphenyl)-1-(2-fluoro-6-methoxyphenyl)-6,6a,7,8,9,10-hexahydro-12*H*-pyrazino[2,1-*c*]pyrido[3,4-*f*]oxazepin-12-one
Z67: (6a*R*)-8-acryloyl-4-chloro-1-(1,4-dimethyl-1*H*-pyrazol-5-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z74: (6a*R*)-8-acryloyl-4-chloro-1-(1,3-dimethyl-1*H*-pyrazol-4-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z77: (6a*R*)-8-acryloyl-4-chloro-3-(2-fluoro-6-hydroxyphenyl)-1-(1-isopropyl-1*H*-pyrazol-5-yl)-6,6a,7,8,9,10-hexahydro-12*H*-pyrazino[2,1-*c*]pyrido[3,4-*f*][1,4]oxazepin-12-one
Z79: (6a*R*)-8-acryloyl-4-chloro-3-(2-fluoro-6-hydroxyphenyl)-1-(1,3,5-trimethyl-1*H*-pyrazol-4-yl)-6,6a,7,8,9,10-hexahydro-12*H*-pyrazino[2,1-*c*]pyrido[3,4-*f*]oxazepin-12-one
Z56: (6a*R*)-8-acryloyl-4-chloro-3-(2-fluoro-6-hydroxyphenyl)-1-(4-azaspiro[2.4]heptan-4-yl)-6,6a,7,8,9,10-hexahydro-12*H*-pyrazino[2,1-*c*]pyrido[3,4-*f*][1,4]oxazepin-12-one
Z57: (6a*R*)-8-acryloyl-4-chloro-3-(2-fluoro-6-hydroxyphenyl)-1-(7-methyl-4,7-diazaspiro[2.5]octan-4-yl)-6,6a,7,8,9,10-hexahydro-12*H*-pyrazino[2,1-*c*]pyrido[3,4-*f*][1,4]oxazepin-12-one
Z58: (6a*R*)-8-acryloyl-4-chloro-3-(2-fluoro-6-hydroxyphenyl)-1-(7-oxa-1-azaspiro[4.4]nonan-1-yl)-6,6a,7,8,9,10-hexahydro-12*H*-pyrazino[2,1-*c*]pyrido[3,4-*f*][1,4]oxazepin-12-one
Z59: (6a*R*)-8-acryloyl-4-chloro-3-(2-fluoro-6-hydroxyphenyl)-1-(2-oxa-7-azaspiro[4.4]nonan-7-yl)-6,6a,7,8,9,10-hexahydro-12*H*-pyrazino[2,1-*c*]pyrido[3,4-*f*][1,4]oxazepin-12-one
Z66: 2-((6a*R*)-8-acryloyl-4-chloro-1-(2,2-dimethylpyrrolidin-1-yl)-6,6a,7,8,9,10-hexahydro-12*H-*pyrazino[2,1-*c*]pyrido[3,4-*f*][1,4]oxazepin-3-yl)-3-fluorophenol
Z476: 1-((6a*R*)-4-chloro-3-(2-fluoro-6-hydroxyphenyl)-1-(4-methoxy-2,2-dimethylpyrrolidin-1-yl)-7,8,9,10-tetrahydro-12*H*-pyrazino[2,1-*c*]pyrido[3,4-*f*][1,4]oxazepin-8(6*H*)-yl)prop-2-en-1-one
Z70: (6a*R*)-8-acryloyl-4-chloro-1-(3,3-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z71: 1-((6a*R*)-1-(*tert*-butylamino)-4-chloro-3-(2-fluoro-6-hydroxyphenyl)-6a,7,9,10-tetrahydro-12*H-*pyrazino[2,1-*c*]pyrido[3,4-*f*][1,4]oxazepin-8(6*H*)-yl)prop-2-en-1-one
Z72: 1-((6a*R*)-1-(1-(*tert*-butylamino)-3-(2-fluoro-6-hydroxyphenyl)-6a,7,9,10-tetrahydro-12*H-*pyrazino[2,1-*c*]pyrido[3,4-*f*][1,4]oxazepin-8(6*H*)-yl)prop-2-en-1-one
Z73: (6a*R*)-8-acryloyl-4-chloro-1-(2,2-dimethyl-5-oxopyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z78: (6a*R*)-8-acryloyl-4-chloro-3-(2-fluoro-6-hydroxyphenyl)-1-(2-methyl-5 -oxopyrrolidin-1-yl)-6,6a,7,8,9,10-hexahydro-12*H*-pyrazino[2,1-*c*]pyrido[3,4-*f*][1,4]oxazepin-12-one
Z80: (*R*)-9-acryloyl-2-(2-fluoro-6-hydroxyphenyl)-4-((*S*)-3-methylmoipholino)-7,8,9,10,10a,11-hexahydro-5*H*-pyrazino[2,1-*c*]pyrimido 15,4-*f*][1,4]oxazepin-5-one
Z90: (6a*R*)-8-acryloyl-4-chloro-3-(2-fluoro-6-hydroxyphenyl)-1-(8-oxa-5-azaspiro[3.5]nonan-5-yl)-6,6a,7,8,9,10-hexahydro-12*H*-pyrazino[2,1-*c*]pyrido[3,4-*f*][1,4]oxazepin-12-one
Z247: (6a*R*)-8-acryloyl-1-((2*S*,6*R*)-2,6-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z248-1: (6a*R*)-8-acryloyl-1-((2*S*,6*S*)-2,6-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z153: (6a*R*)-8-acryloyl-4-chloro-3-(2-fluoro-6-hydroxyphenyl)-1-(2-oxopyrrolidin-1-yl)-6,6a,7,8,9,10-hexahydro-12*H*-pyrazino[2,1-*c*]pyrido[3,4-*f*][1,4]oxazepin-12-one
Z263: (6a*R*)-8-acryloyl-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z93: (6a*R*)-8-acryloyl-1-(((*R*)-1-cyclopentylethyl)amino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z259: (6a*R*)-8-acryloyl-4-chloro-3-(2-fluoro-6-hydroxyphenyl)-1-((2S,4R)-4-hydroxy-2-methylpyrrolidin-1-yl)-6,6a,7,8,9,10-hexahydro-12*H*-pyrazino[2,1-*c*]pyrido[3,4-*f*][1,4]oxazepin-12-one
Z260: (6a*R*)-8-acryloyl-4-chloro-3-(2-fluoro-6-hydroxyphenyl)-1-((2*S*,4*S*)-4-hydroxy-2-methylpyrrolidin-1-yl)-6,6a,7,8,9,10-hexahydro-12*H*-pyrazino[2,1-*c*]pyrido[3,4-*f*][1,4]oxazepin-12-one
Z248-2: (6a*R*)-8-acryloyl-4-chloro-1-((2*R*,6*R*)-2,6-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z246: (6a*R*)-8-acryloyl-4-chloro-3-(2-fluoro-6-hydroxyphenyl)-1-((3*S*,5*R*)-3,4,5-trimethylpiperazin-1-yl)-6,6a,7,8,9,10-hexahydro-12*H*-pyrazino[2,1-*c*]pyrido[3,4-*f*][1,4]oxazepin-12-one
Z308: (6a*R*)-8-acryloyl-4-chloro-1-(2,3-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z308b: (6a*R*)-1-(4-acryloyl-2,3-dimethylpiperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z235: (6a*R*)-8-acryloyl-4-chloro-1-((R)-3,4-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z239: (6a*R*)-8-acryloyl-4-chloro-3-(2-fluoro-6-hydroxyphenyl)-1-(2-methyl-4-(oxetan-3-yl)piperazin-1-yl)-6,6a,7,8,9,10-hexahydro-12*H*-pyrazino[2,1-*c*]pyrido[3,4-*f*][1,4]oxazepin-12-one
Z110: (6a*R*)-8-acryloyl-4-chloro-1-((1,3,3-trimethyltetrahydropyrrol-2-yl)methoxy)-3 -(2-fluoro-6-hydroxyphenyl)-6,6a,7,8,9,10-hexahydro-12*H*-pyrazino[2,1-*c*]pyrido[3,4-*f*][1,4]oxazepin-12-one
Z261: (6a*R*)-8-acryloyl-4-chloro-3-(2-fluoro-6-hydroxyphenyl)-1-(3,3,4-trimethylpiperazin-1-yl)-6,6a,7,8,9,10-hexahydro-12*H*-pyrazino[2,1-*c*]pyrido[3,4-*f*][1,4]oxazepin-12-one
Z257: (6a*R*)-8-acryloyl-4-chloro-3-(2-fluoro-6-hydroxyphenyl)-1-((*S*)-4-(2-hydroxyethyl)-2-methylpiperazin-1-yl)-6,6a,7,8,9,10-hexahydro-12*H*-pyrazino[2,1-*c*]pyrido[3,4-*f*][1,4]oxazepin-12-one
Z308a: (6a*R*)-8-acryloyl-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one
Z308c: (6a*R*)-8-acryloyl-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one
Z254: (6a*R*)-8-acryloyl-4-chloro-1-((3*S*,5*R*)-3,5-dimethylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z99: (6a*R*)-8-acryloyl-4-chloro-1-(2,3-dimethylpiperidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z102a: (6a*R*)-8-acryloyl-4-chloro-3-(2-fluoro-6-hydroxyphenyl)-1-(3-hydroxy-2-methylazetidin-1-yl)-6,6a,7,8,9,10-hexahydro-12*H*-pyrazino[2,1-*c*]pyrido[3,4-*f*][1,4]oxazepin-12-one
Z236: (6a*R*)-8-acryloyl-4-chloro-3-(2-fluoro-6-hydroxyphenyl)-1-(3-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z221: (6a*R*)-8-acryloyl-4-chloro-1-(2,4-dimethyl-5-oxopiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z249: (6a*R*)-8-acryloyl-4-chloro-3-(2-fluoro-6-hydroxyphenyl)-1-((2*R*,6*S*)-2,4,6-trimethylpiperazin-1-yl)-6,6a,7,8,9,10-hexahydro-12*H*-pyrazino[2,1-*c*]pyrido[3,4-*f*][1,4]oxazepin-12-one
Z252: (6a*R*)-8-acryloyl-1-(3,5-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z252-1: (6a*R*)-8-acryloyl-1-(3*R*,5*S*-dimethylmorpholino)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z244: (6a*R*)-8-acryloyl-4-chloro-3-(2-fluoro-6-hydroxyphenyl)-1-(4-methoxy-2,2-dimethylpyrrolidin-1-yl)-12-oxo-6,6a,7,8,9,10-hexahydro-12*H*-pyrazino[2,1-*c*]pyrido[3,4-*f*][1,4]oxazepin-12-one
Z243: (6a*R*)-8-acryloyl-4-chloro-3-(2-fluoro-6-hydroxyphenyl)-1-(4-hydroxy-2,2,4-trimethylpyrrolidin-1-yl)-6,6a,7,8,9,10-hexahydro-12*H*-pyrazino[2,1-*c*]pyrido[3,4-*f*][1,4]oxazepin-12-one
Z309: (6a*R*)-8-acryloyl-4-chloro-3-(2-fluoro-6-hydroxyphenyl)-1-(1-azaspiro[3.3]heptan-1-yl)-6,6a,7,8,9,10-hexahydro-12*H*-pyrazino[2,1-*c*]pyrido[3,4-*f*][1,4]oxazepin-12-one
Z257a: (6a*R*)-8-acryloyl-4-chloro-3-(2-fluoro-6-hydroxyphenyl)-1-((*S*)-4-(2-methoxyethyl)-2-methylpiperazin-1-yl)-6,6a,7,8,9,10-hexahydro-12*H*-pyrazino[2,1-*c*]pyrido[3,4-*f*][1,4]oxazepin-12-one
Z310: (6a*R*)-8-acryloyl-1-(2,6,6-trimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z279-1: (6a*R*)-8-acryloyl-1-((R)-2-(hydroxymethyl)-2-methylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z279-2: (6a*R*)-8-acryloyl-4-chloro-1-((*S*)-(2-hydroxymethyl-2-methyltetrahydropyrrol-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z277-1: (6a*R*)-8-acryloyl-4-chloro-3-(2-fluoro-6-hydroxyphenyl)-1-(6-methyl-5,6-dihydro-[1,2,4]triazolo[4,3-*a*]pyrazin-7(8*H*)-yl)-6,6a,7,8,9,10-hexahydro-12*H*-pyrazino[2,1-*c*]pyrido[3,4-*f*][1,4]oxazepin-12-one
Z311-1: (6a*R*)-8-acryloyl-4-chloro-1-((*S*)-2,4-dimethyl-3-oxopiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z241: (6a*R*)-8-acryloyl-1-(3-hydroxy-2,2-dimethylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z276-1: (6a*R*)-8-acryloyl-4-chloro-3-(2-fluoro-6-hydroxyphenyl)-1-((*S*)-2,2,5-trimethylmorpholino)-6,6a,7,8,9,10-hexahydro-12*H*-pyrazino[2,1-*c*]pyrido[3,4-*f*][1,4]oxazepin-12-one
Z312-1: (6a*R*)-8-acryloyl-4-chloro-1-((*S*)-4-cyclopropyl-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z313-1: (6a*R*)-1-((*S*)-4-acetyl-2-methylpiperazin-1-yl)-8-acryloyl-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z240: (6a*R*)-8-acryloyl-4-chloro-3-(2-fluoro-6-hydroxyphenyl)-1-(4-(hydroxymethyl)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z315: (6a*R*)-1-(4-acetyl-3,3-dimethylpiperazin-1-yl)-8-acryloyl-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z291-2: (6a*R*)-8-acryloyl-1-(2,6-dimethylpyrrolo[3,4-*c*]pyrazol-5(4*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z292-2: (6a*R*)-8-acryloyl-1-(1,6-dimethylpyrrolo[3,4-c]pyrazol-5(1*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z316-1: (6a*R*)-8-acryloyl-4-chloro-3-(2-fluoro-6-hydroxyphenyl)-1-((*S*)-2-methyl-4-propionylpiperazin-1-yl)-6,6a,7,8,9,10-hexahydro-12*H*-pyrazino[2,1-*c*]pyrido[3,4-*f*][1,4]oxazepin-12-one
Z317-1: (6a*R*)-8-acryloyl-4-chloro-3 -(2-fluoro-6-hydroxyphenyl)-1-((*S*)-hexahydropyrrolo [1,2-*a*]pyrazin-2(1*H*-yl)-6,6a,7,8,9,10-hexahydro-12*H*-pyrazino[2,1-*c*]pyrido[3,4-*f*][1,4]oxazepin-12-one
Z256b: (6a*R*)-8-acryloyl-4-fluoro-3-(2-fluoro-6-hydroxyphenyl)-1-4-hydroxy-2,2,4-trimethylpyrrolidin-1-yl)-6,6a,7,8,9,10-hexahydro-12*H*-pyrazino[2,1-*c*]pyrido[3,4-*f*][1,4]oxazepin-12-one
Z318-1: (6a*R*)-8-acryloyl-1-((*S*)-2-methyl-4-(methylsulfonyl)piperazin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z319-1: (6a*R*)-8-acryloyl-4-chloro-1-((*S*)-4-ethyl-2-methyl-5-oxopiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z317-2: (6a*R*)-8-acryloyl-4-chloro-3-(2-fluoro-6-hydroxyphenyl)-1-((*R*)-hexahydropyrrolo[1,2-*a*]pyrazin-2(1*H*-yl)-6,6a,7,8,9,10-hexahydro-12*H*-pyrazino[2,1-*c*]pyrido[3,4-*f*][1,4]oxazepin-12-one
Z320-1: (6a*R*)-8-acryloyl-4-chloro-1-((*S*)-4-(cyclopropanecarbonyl)-2-methylpiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z317a: (6a*R*)-8-acryloyl-4-chloro-3-(2-fluoro-6-hydroxyphenyl)-1-(6-oxohexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one
Z321: (6a*R*)-8-acryloyl-4-chloro-3-(2-fluoro-6-hydroxyphenyl)-1-(4-(2-methoxyethyl)-3,3-dimethylpiperazin-1-yl)-6,6a,7,8,9,10-hexahydro-12*H*-pyrazino[2,1-*c*]pyrido[3,4-*f*][1,4]oxazepin-12-one
Z322: (6a*R*)-8-acryloyl-4-chloro-3-(2-fluoro-6-hydroxyphenyl)-1-(4-ethyl-3,3-dimethylpiperazin-1-yl)-6,6a,7,8,9,10-hexahydro-12*H*-pyrazino[2,1-*c*]pyrido[3,4-*f*][1,4]oxazepin-12-one
Z324: (6a*R*)-8-acryloyl-4-chloro-1-(4-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z326: (6a*R*)-8-acryloyl-4-chloro-1-(2,2-dimethyl-4-(methyl(oxetan-3-yl)amino)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z330-1: (6a*R*)-8-acryloyl-4-chloro-3-(2-fluoro-6-hydroxyphenyl)-1-((3*S*)-3-methyl-6-oxohexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one
Z332: (6a*R*)-8-acryloyl-4-chloro-3-(2-fluoro-6-hydroxyphenyl)-1-(2-(methoxymethyl)-2-methylmorpholino)-6,6a,7,8,9,10-hexahydro-12*H*-pyrazino[2,1-*c*]pyrido[3,4-*f*][1,4]oxazepin-12-one
Z333: (6a*R*)-8-acryloyl-4-chloro-3-(2-fluoro-6-hydroxyphenyl)-1-(2-(hydroxymethyl)-2-methylmorpholino)-6,6a,7,8,9,10-hexahydro-12*H*-pyrazino[2,1-*c*]pyrido[3,4-*f*][1,4]oxazepin-12-one
Z334: (6a*R*)-8-acryloyl-4-chloro-1-(3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z337: (6a*R*)-8-acryloyl-4-chloro-3-(2-fluoro-6-hydroxyphenyl)-1-(8a-methylhexahydropyrrolo[1,2-*a*]pyrazin-6(2*H*)-one-1-yl)-6,6a,7,8,9,10-hexahydro-12*H*-pyrazino[2,1-*c*]pyrido[3,4-*f*][1,4]oxazepin-12-one
Z338-1: (6a*R*)-8-acryloyl-4-chloro-3-(2-fluoro-6-hydroxyphenyl)-1-((2S,3R)-3-methoxy-2-methylpyrrolidin-1-yl)-6,6a,7,8,9,10-hexahydro-12*H*-pyrazino[2,1-*c*]pyrido[3,4-*f*][1,4]oxazepin-12-one
Z339: (6a*R*)-8-acryloyl-4-chloro-3-(2-fluoro-6-hydroxyphenyl)-1-(8a-methyloctahydropyrrolo[1,2-*a*]pyrazin-6(2*H*)-one-1-yl)-6,6a,7,8,9,10-hexahydro-12*H*-pyrazino[2,1-*c*]pyrido[3,4-*f*][1,4]oxazepin-12-one
Z275: (6a*R*)-8-acryloyl-1-(4-cyano-2,2-dimethylpyrrolidin-1-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z344: (6a*R*)-8-acryloyl-4-chloro-1-((2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z345: (6a*R*)-8-acryloyl-1-(2,4-dimethylpyrrolo[3,4-*c*]pyrazol-5(4*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z346: (6a*R*)-8-acryloyl-1-(1,4-dimethylpyrrolo[3,4-*c*]pyrazol-5(1*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z347-1: (6a*R*)-8-acryloyl-4-chloro-3-(2-fluoro-6-hydroxyphenyl)-1-((2*S*,3*S*)-3-methoxy-2-methylpyrrolidin-1-yl)-6,6a,7,8,9,10-hexahydro-12*H*-pyrazino[2,1-*c*]pyrido[3,4-*f*][1,4]oxazepin-12-one
Z360: (6a*R*)-8-acryloyl-4-chloro-1-(3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z452: (6a*R*)-8-acryloyl-4-chloro-3-(2-chloro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z462: (2*S*)-1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide
Z36: (6a*R*)-8-acryloyl-4-chloro-3-(2-fluoro-6-hydroxyphenyl)-1-(2,3,4-trimethylpiperazin-1-yl)-6,6a,7,8,9,10-hexahydro-12*H*-pyrazino[2,1-*c*]pyrido[3,4-*f*][1,4]oxazepin-12-one
Z448: (6a*R*)-8-acryloyl-4-chloro-3-(2-fluorophenyl)-1-(4-methyl-4*H*-pyrrolo[3,4-*c*]isoxazol-5(6*H*)-yl)-6,6a,7,8,9,10-hexahydro-12*H*-pyrazino[2,1-*c*]pyrido[3,4-*f*][1,4]oxazepin-12-one
Z325: (6a*R*)-8-acryloyl-4-chloro-3-(2-fluorophenyl)-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z147: (*R*)-8-acryloyl-4-chloro-1-(2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z97a: (6a*R*)-8-acryloyl-4-chloro-3-(2-fluoro-6-methoxyphenyl)-1-(indolin-1-yl)-6,6a,7,8,9,10-hexahydro-12*H*-pyrazino[2,1-*c*]pyrido[3,4-*f*][1,4]oxazepin-12-one
Z340: (6a*R*)-8-acryloyl-1-(3-hydroxy-5,5-dimethylpyrrolidin-1-yl)-4-fluoro-3-(3-chloro-2-fluoro-6-hydroxyphenyl)-6,6a,7,8,9,10-hexahydro-12*H*-pyrazino[2,1-*c*]pyrido[3,4-*f*][1,4]oxazepin-12-one
Z341: (6a*R*)-8-acryloyl-1-(3-hydroxy-5,5-dimethylpyrrolidin-1-yl)-4-fluoro-3-(3-chloro-6-fluoro-6-hydroxyphenyl)-6,6a,7,8,9,10-hexahydro-12*H*-pyrazino[2,1-*c*]pyrido[3,4-*f*][1,4]oxazepin-12-one
Z463: (*R*)-8-acryloyl-4-chloro-3-(2-fluorophenyl)-1-(3,3,4-trimethylpiperazin-1-yl)-6,6a,7,8,9,10-hexahydro-12*H*-pyrazino[2,1-*c*]pyrido[3,4-*f*][1,4]oxazepin-12-one, formate
Z471: (*R*)-8-acryloyl-1-(4-(azetidin-1-yl)-2,2-dimethylpyrrolidin-1-yl)-4-chloro-3-(2-fluorophenyl)-6,6a,7,8,9,10-hexahydro-12*H*-pyrazino[2,1-*c*]pyrido[3,4-*f*][1,4]oxazepin-12-one
Z406: (6a*R*)-8-acryloyl-4-chloro-3-(2-fluorophenyl)-1-(4-((2-methoxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z465: (6a*R*)-8-acryloyl-4-chloro-3-(2-fluoro-6-hydroxyphenyl)-1-(*cis*-2,3,4-trimethylpiperazin-1-yl)-12-oxo-6,6a,7,8,9,10-hexahydro-12*H*-pyrazino[2,1-*c*]pyrido[3,4-*f*][1,4]oxazepin-12-one
Z468: (6a*R*)-8-acryloyl-4-chloro-1-(2,2-dimethyl-4-morpholinopyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z470-1: (6a*R*)-8-acryloyl-4-chloro-3-(2-fluorophenyl)-1-((2S,4R)-2-methyl-4-(4-methylpiperazin-1-yl)pyrrolidin-1-yl)-6,6a,7,8,9,10-hexahydro-12*H*-pyrazino[2,1-*c*]pyrido[3,4-*f*][1,4]oxazepin-12-one
Z470-2: (6a*R*)-8-acryloyl-4-chloro-1-(2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z407-1: (*R*)-8-acryloyl-4-chloro-1-((*S*)-2,4-dimethylpiperazin-1-yl)-3-(2-fluorophenyl)-6,6a,7,8,9,10-hexahydro-12*H*-pyrazino[2,1-*c*]pyrido[3,4-*f*][1,4]oxazepin-12-one
Z356: (6a*R*)-8-acryloyl-4-chloro-3-(3,6-difluoro-2-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z454: (6a*R*)-8-acryloyl-4-chloro-1-(2,2-dimethyl-4-(4-methylpiperazin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z456: (6a*R*)-8-acryloyl-4-chloro-3-(2-(difluoromethyl)-6-fluorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z466: (2*S*)-1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide
Z457: (*R*)-8-acryloyl-1-(*tert*-butylamino)-4-chloro-3-(2-fluorophenyl)-6,6a,7,8,9,10-hexahydro-12*H-*pyrazino[2,1-*c*]pyrido[3,4-*f*][1,4]oxazepin-12-one
Z461: (*R*)-8-acryloyl-4-chloro-3-(2-chlorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z464: (6a*R*)-8-acryloyl-4-chloro-3-(2-fluoro-6-hydroxyphenyl)-1-((2R,5*S*)-2,4,5-trimethylpiperazin-1-yl)-12-oxo-6,6a,7,8,9,10-hexahydro-12*H*-pyrazino[2,1-*c*]pyrido[3,4-*f*][1,4]oxazepin-12-one
Z477: (*R*)-8-acryloyl-4-chloro-3-(2-fluorophenyl)-1-((*S*)-2-methyl-4-propionylpiperazin-1-yl)-6,6a,7,8,9,10-hexahydro-12*H*-pyrazino[2,1-*c*]pyrido[3,4-*f*][1,4]oxazepin-12-one
Z478: 8-acryloyl-4-chloro-3-(2-fluorophenyl)-1-(4-(2-methoxyethyl)-3,3-dimethylpiperazin-1-yl)-6,6a,7,8,9,10-hexahydro-12*H*-pyrazino[2,1-*c*]pyrido[3,4-*f*][1,4]oxazepin-12-one
Z262: (6a*S*)-8-acryloyl-4-chloro-1-(2,2-dimethylmorpholino)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z83: (6a*R*)-8-acryloyl-4-chloro-3-(2-fluoro-6-hydroxyphenyl)-1-(4-methyl-4,7-diazaspiro[2.5]octan-1-yl)-6,6a,7,8,9,10-hexahydro-12*H*-pyrazino[2,1-*c*]pyrido[3,4-*f*][1,4]oxazepin-12-one
Z237-2: (6a*R*)-8-acryloyl-4-chloro-1-((2*S*,4*S*)-4-(dimethylamino)-2-methylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z237-1: synthesis of (6a*R*)-8-acryloyl-4-chloro-1-((2*S*,4*R*)-4-(dimethylamino)-2-methylpyrrolidin-1-yl)-3 -(2-fluoro-6-hydroxyphenyl)-6,6a,7, 8,9,10-hexahydro-12H-pyrazino [2,1-c]pyrido [3,4-*f*][1,4]oxazepin-12-one
Z237-1: (6a*R*)-8-acryloyl-4-chloro-1-((2S,4R)-4-(dimethylamino)-2-methylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z358-1: (6a*R*)-8-acryloyl-4-chloro-3-(2-fluoro-6-hydroxyphenyl)-1-((2*S*,4*S*)-4-((2-methoxyethyl)(methyl)amino)-2-methylpyrrolidin-1-yl)-6,6a,7,8,9,10-hexahydro-12*H*-pyrazino[2,1-c]pyrido[3,4-*f*][1,4]oxazepin-12-one
Z256a: (6a*R*)-8-acryloyl-1-(4-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z255: (6a*R*)-8-acryloyl-1-(2,2-dimethylmorpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z327: (6a*R*)-8-acryloyl-1-(3-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z329: (6a*R*)-8-acryloyl-1-(morpholino)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z335: (6a*R*)-8-acryloyl-1-(4-cyano-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z359-1: (6a*R*)-8-acryloyl-4-fluoro-3-(2-fluoro-6-hydroxyphenyl)-1-((2*S*,4*S*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one
Z474: (6a*R*)-8-acryloyl-1-(2,2-dimethyl-4-(4-methylpiperazin-1-yl)pyrrolidin-1-yl)-4-fluoro-3-(2-fluorophenyl)-6,6a,7,8,9,10-hexahydro-12*H*-pyrazino[2,1-*c*]pyrido[3,4-*f*][1,4]oxazepin-12-one
Z238: (6a*R*)-8-acryloyl-1-(2,2-dimethylmorpholino)-4-fluoro-3-(5-methyl-1H-indazol-4-yl)-6,6a,7,8,9,10-hexahydro-12*H*-pyrazino[2,1-*c*]pyrido[3,4-*f*][1,4]oxazepin-12-one
Z449: (6a*R*)-8-acryloyl-3-(3,6-difluoro-2-hydroxyphenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z449-1: (6a*R*)-8-acryloyl-3-(3,6-difluoro-2-hydroxyphenyl)-4-fluoro-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z449-2: (6a*R*)-8-acryloyl-3-(3,6-difluoro-2-hydroxyphenyl)-4-fluoro-1-((*R*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z451: (6a*R*)-8-acryloyl-1-((*S*)-2,4-dimethylpiperazin-1-yl)-4-fluoro-3-(2-fluoro-6-methoxyphenyl)-6,6a,7,8,9,10-hexahydro-12*H*-pyrazino[2,1-*c*]pyrido[3,4-*f*][1,4]oxazepin-12-one
Z256: (6a*R*)-8-acryloyl-4-fluoro-3-(2-fluoro-6-hydroxyphenyl)-1-(4-hydroxy-2,2-dimethylpyrrolidin-1-yl)-6,6a,7,8,9,10-hexahydro-12*H*-pyrazino[2,1-*c*]pyrido[3,4 f][1,4]oxazepin-12-one
Z256-1: (6a*R*)-8-acryloyl-4-fluoro-3-(2-fluoro-6-hydroxyphenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z256-2: (6aR)-8-acryloyl-4-fluoro-3-(2-fluoro-6-hydroxyphenyl)-1-((*R*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z314: (*R*)-8-acryloyl-3-(6-amino-4-methyl-3-(trifluoromethyl)pyridin-2-yl)-4-chloro-1-morpholino-6,6a,7,8,9,10-hexahydro-12*H*-pyrazino[2,1-*c*]pyrido[3,4-*f*][1,4]oxazepin-12-one
Z318: (*R*)-8-acryloyl-3-(6-amino-4-methylpyridin-2-yl)-4-chloro-1-morpholino-6,6a,7,8,9,10-hexahydro-12*H*-pyrazino[2,1-*c*]pyrido [3,4-*f]*[1,4]oxazepin-12-one
Z267: (*R*)-8-acryloyl-3-(6-amino-3-(trifluoromethyl)pyridin-2-yl)-4-chloro-1-morpholino-6,6a,7,8,9,10-hexahydro-12*H*-pyrazino[2,1-*c*]pyrido[3,4-*f*][1,4]oxazepin-12-one
Z328-1: (*R*)-8-acryloyl-3-(6-amino-4-methyl-3-(trifluoromethyl)pyridin-2-yl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z241a-2: (6a*R*)-8-acryloyl-4-chloro-3-(2-fluoro-6-hydroxyphenyl)-1-(3-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z241a-1: (6a*R*)-8-acryloyl-4-chloro-3-(2-fluoro-6-hydroxyphenyl)-1-((*S*)-3-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z241a-2: (6a*R*)-8-acryloyl-4-chloro-3-(2-fluoro-6-hydroxyphenyl)-1-((*R*)-3-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z296-1: (6a*R*)-8-acryloyl-4-chloro-3-(2-fluoro-6-hydroxyphenyl)-1-((*S*)-4-((2-methoxyethyl)(methyl)amino)-2,2-dimethylpyrrolidon-1-yl)-6,6a,7, 8,9,10-hexahydro-12*H*-pyrazino [2,1-*c*]pyrido[3,4-*f*][1,4]oxazepin-12-one
Z296-2: (6aR)-8-acryloyl-4-chloro-3-(2-fluoro-6-hydroxyphenyl)-1-((*R*)-4-((2-methoxyethyl)(methyl)amino)-2,2-dimethylpyrrolidon-1-yl)-6,6a,7, 8,9,10-hexahydro-12*H*-pyrazino [2,1-*c*]pyrido[3,4-*f*][1,4]oxazepin-12-one
Z467: (6a*R*)-8-acryloyl-4-chloro-1-(2,2-dimethyl-4-(4-methylpiperazin-1-yl)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z297: (6a*R*)-8-acryloyl-4-chloro-1-((2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z297-1: (6a*R*)-8-acryloyl-4-chloro-1-((R)-2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z297-2: (6a*R*)-8-acryloyl-4-chloro-1-((*S*)-2,2-dimethyl-4-morpholinopyrrolidin-1-yl))-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z304: (6a*R*)-8-acryloyl-4-chloro-1-(1,3-dimethyl-1,2,3,6-tetrahydropyridin-4-yl)-3-(2-fluoro-6-hydroxyphenyl)-6,6a,7,8,9,10-hexahydro-12*H*-pyrazino[2,1-*c*]pyrido[3,4-*f*]oxazepin-12-one
Z323: (6a*R*)-8-acryloyl-4-chloro-1-(1,3-dimethylpiperidin-4-yl)-3-(2-fluoro-6-hydroxyphenyl)-6,6a,7,8,9,10-hexahydro-12*H*-pyrazino[2,1-*c*]pyrido[3,4-*f*]oxazepin-12-one
Z260a: (6a*R*)-8-acryloyl-4-chloro-3-(2-fluoro-6-hydroxyphenyl)-1-((2*S*,4*S*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one
Z259a: (6a*R*)-8-acryloyl-4-chloro-3-(2-fluoro-6-hydroxyphenyl)-1-((2*S*,4*R*)-4-methoxy-2-methylpyrrolidin-1-yl)-6,6a,7,8,9,10-hexahydro-12*H*-pyrazino[2,1-*c*]pyrido[3,4-*f*][1,4]oxazepin-12-one
Z336-1: (6a*R*)-8-acryloyl-1-((*S*)-3-hydroxy-5,5-dimethylpyrrolidin-1-yl)-4-methy1-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z33 6-2: (6a*R*)-8 -acryloyl-1-((*R*)-3-hydroxy-5,5-dimethylpyrrolidin-1-yl)-4-methyl-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z93-1: (6a*R*)-8-acryloyl-4-chloro-3-(2-fluoro-6-hydroxyphenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z93-2: (6a*R*)-8-acryloyl-4-chloro-3-(2-fluoro-6-hydroxyphenyl)-1-((R)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z39-1: (6a*R*)-8 -acryloyl-1-((1*R*,5*S*)-2-azabicyclo[3.1.0]hexan-2-yl)-4-chloro-3 -(2-fluoro-6-hydroxyphenyl)-6,7-6a,7,8,9,10-hexa-12*H*-pyrazino[2,1-*c*]pyrido[3,4-*f*][1,4]oxazepin-12-one
Z39-2: (6a*R*)-8 -acryloyl-1-((1*S*,S*R*)-2-azabicyclo [3.1.0]hexan-2-yl)-4-chloro-3 -(2-fluoro-6-hydroxyphenyl)-6,7-6a,7,8,9,10-hexa-12*H*-pyrazino[2,1-*c*]pyrido[3,4-*f*][1,4]oxazepin-12-one
Z221-2: (6a*R*)-8-acryloyl-4-chloro-1-((*R*)-2,4-dimethyl-5-oxapiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z221-1: (6a*R*)-8-acryloyl-4-chloro-1-((*S*)-2,4-dimethyl-5-oxapiperazin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z239-2: (6a*R*)-8-acryloyl-4-chloro-3-(2-fluoro-6-hydroxyphenyl)-1-((R)-2-methyl-4-(oxetan-3-yl)piperazin-1-yl)-6,6a,7,8,9,10-hexahydro-12*H*-pyrazino[2,1-*c*]pyrido[3,4-*f*][1,4]oxazepin-12-one
Z239-1: (6a*R*)-8-acryloyl-4-chloro-3-(2-fluoro-6-hydroxyphenyl)-1-((*S*)-2-methyl-4-(oxetan-3-yl)piperazin-1-yl)-6,6a,7,8,9,10-hexahydro-12*H*-pyrazino[2,1-*c*]pyrido[3,4-*f*][1,4]oxazepin-12-one
Z102-3: (6a*R*)-8-acryloyl-4-chloro-3-(2-fluoro-6-hydroxyphenyl)-1-((2*R*,3*R*)-3-hydroxy-2-methylazetidin-1-yl)-6,6a,7,8,9,10-hexahydro-12*H*-pyrazino[2,1-*c*]pyrido[3,4-*f*][1,4]oxazepin-12-one
Z102-4: (6a*R*)-8-acryloyl-4-chloro-3-(2-fluoro-6-hydroxyphenyl)-1-((2*R*,3*S*)-3-hydroxy-2-methylazetidin-1-yl)-6,6a,7,8,9,10-hexahydro-12*H*-pyrazino[2,1-*c*]pyrido[3,4-*f*][1,4]oxazepin-12-one
Z102-1: (6a*R*)-8-acryloyl-4-chloro-3-(2-fluoro-6-hydroxyphenyl)-1-((2*S*,3*R*)-3-hydroxy-2-methylazetidin-1-yl)-6,6a,7,8,9,10-hexahydro-12*H*-pyrazino[2,1-*c*]pyrido[3,4-*f*][1,4]oxazepin-12-one
Z102-2: (6a*R*)-8-acryloyl-4-chloro-3-(2-fluoro-6-hydroxyphenyl)-1-((2*S*,3*S*)-3 -hydroxy-2-methylazetidin-1-yl)-6,6a,7,8,9,10-hexahydro-12*H*-pyrazino[2,1-*c*]pyrido[3,4-*f*][1,4]oxazepin-12-one
Z240-1: (6a*R*)-8-acryloyl-4-chloro-3-(2-fluoro-6-hydroxyphenyl)-1-((*S*)-4-(hydroxymethyl)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z240-2: (6a*R*)-8-acryloyl-4-chloro-3-(2-fluoro-6-hydroxyphenyl)-1-((*R*)-4-(hydroxymethyl)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z241-1: (6a*R*)-8-acryloyl-4-chloro-3-(2-fluoro-6-hydroxyphenyl)-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z241-2: (6a*R*)-8-acryloyl-4-chloro-3-(2-fluoro-6-hydroxyphenyl)-1-((*R*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z243-1: (6a*R*)-8-acryloyl-4-chloro-3-(2-fluoro-6-hydroxyphenyl)-1-((*S*)-4-hydroxy-2,2,4-trimethylpyrrolidin-1-yl)-6,6a,7,8,9,10-hexahydro-12*H*-pyrazino[2,1-*c*]pyrido[3,4-*f*][1,4]oxazepin-12-one
Z243-2: (6a*R*)-8-acryloyl-4-chloro-3-(2-fluoro-6-hydroxyphenyl)-1-((*R*)-4-hydroxy-2,2,4-trimethylpyrrolidin-1-yl)-6,6a,7,8,9,10-hexahydro-12*H*-pyrazino[2,1-*c*]pyrido[3,4-*f*][1,4]oxazepin-12-one
Z244-1: (6a*R*)-8-acryloyl-4-chloro-3 -(2-fluoro-6-hydroxyphenyl)-1-((*S*)-4-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z244-2: (6a*R*)-8-acryloyl-4-chloro-3 -(2-fluoro-6-hydroxyphenyl)-1-((*R*)-4-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z256b-1: (6a*R*)-8-acryloyl-4-fluoro-3-(2-fluoro-6-hydroxyphenyl)-1-((*S*)-4-hydroxy-2,2,4-trimethylpyrrolidin-1-yl)-6,6a,7,8,9,10-hexahydro-12*H*-pyrazino[2,1-*c*]pyrido[3,4-*f*][1,4]oxazepin-12-one
Z256b-2: (6a*R*)-8-acryloyl-4-fluoro-3-(2-fluoro-6-hydroxyphenyl)-1-((*R*)-4-hydroxy-2,2,4-trimethylpyrrolidin-1-yl)-6,6a,7,8,9,10-hexahydro-12*H*-pyrazino[2,1-*c*]pyrido[3,4-*f*][1,4]oxazepin-12-one
Z256a-1: (6a*R*)-8-acryloyl-1-((*S*)-4-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z256a-2: (6a*R*)-8-acryloyl-1-((*R*)-4-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z317a-1: (6a*R*)-8-acryloyl-4-chloro-3-(2-fluoro-6-hydroxyphenyl)-1-((*S*)-6-oxohexahydropyrrolo[1,2-*a*]pyrazin-2(1*H*-yl)-6,6a,7,8,9,10-hexahydro-12*H*-pyrazino[2,1-*c*]pyrido[3,4-*f*][1,4]oxazepin-12-one
Z317a-2: (6a*R*)-8-acryloyl-4-chloro-3-(2-fluoro-6-hydroxyphenyl)-1-((*R*)-6-oxohexahydropyrrolo[1,2-*a*]pyrazin-2(1*H*)-yl)-6,6a,7,8,9,10-hexahydro-12*H*-pyrazino[2,1-*c*]pyrido[3,4-*f*][1,4]oxazepin-12-one
Z292-1S: (6a*R*)-8-acryloyl-1-((*R*)-1,6-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one trifluoroacetate
Z292S: (6a*R*)-8-acryloyl-1-((*S*)-1,6-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one trifluoroacetate
Z291-1S: (6a*R*)-8-acryloyl-1-((*R*)-2,6-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one trifluoroacetate
Z291S: (6aR)-8-acryloyl-1-((*S*)-2,6-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4*-*f][1,4]oxazepin-12-one trifluoroacetate
Z324-1: (6a*R*)-8-acryloyl-4-chloro-1-((*S*)-4-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4*-*f][1,4]oxazepin-12-one
Z324-2: (6a*R*)-8-acryloyl-4-chloro-1-((*R*)-4-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4*-*f][1,4]oxazepin-12-one
Z308a-1: (6a*R*)-8-acryloyl-4-chloro-3-(2-fluoro-6-hydroxyphenyl)-1-((2*R*,3*S*)-2,3,4-trimethylpiperazin-1-yl)-6,6a,7,8,9,10-hexahydro-12*H*-pyrazino[2,1-*c*]pyrido[3,4*-f*][1,4]oxazepin-12-one
Z308a-2: (6a*R*)-8-acryloyl-4-chloro-3-(2-fluoro-6-hydroxyphenyl)-1-((2*S*,3*R*)-2,3,4-trimethylpiperazin-1-yl)-6,6a,7,8,9,10-hexahydro-12*H*-pyrazino[2,1-*c*]pyrido[3,4*f*][1,4]oxazepin-12-one
Z325-1: (*R*)-8-acryloyl-4-chloro-3-(2-fluorophenyl)-1-((*S*)-4-hydroxy-2,2-dimethylpyrrolidin-1-yl)-6,6a,7,8,9,10-hexahydro-12*H*-pyrazino[2,1-*c*]pyrido[3,4*f*][1,4]oxazepin-12-one
Z325-2: (*R*)-8-acryloyl-4-chloro-3-(2-fluorophenyl)-1-((*R*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z327-1: (6a*R*)-8-acryloyl-1-((*R*)-3-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z327-2: (6a*R*)-8-acryloyl-1-((*S*)-3-methoxy-2,2-dimethylpyrrolidin-1-yl)-4-fluoro-3-(2-fluoro-6-hydroxyphenyl)-6,6a,7,8,9,10-hexahydro-12*H*-pyrazino[2,1-*c*]pyrido[3,4*-f*][1,4]oxazepin-12-one
Z331-1: (6a*R*)-8-acryloyl-4-chloro-1-((*R*)-2,2-dimethyl-4-(methyl(oxetan-3-yl)amino)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z331-2: (6a*R*)-8-acryloyl-4-chloro-1-((*S*)-2,2-dimethyl-4-(methyl(oxetan-3-yl)amino)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z332-1: (6a*R*)-8-acryloyl-4-chloro-3-(2-fluoro-6-hydroxyphenyl)-1-((*S*)-2-(methoxymethyl)-2-methylmorpholino)-6,6a,7,8,9,10-hexahydro-12*H*-pyrazino[2,1-*c*]pyrido[3,4-*f*][1,4]oxazepin-12-one
Z332-2: (6a*R*)-8-acryloyl-4-chloro-3-(2-fluoro-6-hydroxyphenyl)-1-((*R*)-2-(methoxymethyl)-2-methylmorpholino)-6,6a,7,8,9,10-hexahydro-12*H*-pyrazino[2,1-*c*]pyrido[3,4-*f*][1,4]oxazepin-12-one
Z333-1: (6a*R*)-8-acryloyl-4-chloro-3-(2-fluoro-6-hydroxyphenyl)-1-((*S*)-2-(hydroxymethyl)-2-methylmorpholino)-6,6a,7,8,9,10-hexahydro-12*H*-pyrazino[2,1-*c*]pyrido[3,4-*f*][1,4]oxazepin-12-one
Z333-2: (6a*R*)-8-acryloyl-4-chloro-3-(2-fluoro-6-hydroxyphenyl)-1-((*R*)-2-(hydroxymethyl)-2-methylmorpholino)-6,6a,7,8,9,10-hexahydro-12*H*-pyrazino[2,1-*c*]pyrido[3,4-*f*][1,4]oxazepin-12-one
Z340-1: (6a*R*)-8-acryloyl-1-((*S*)-3-hydroxy-5,5-dimethylpyrrolidin-1-yl)-4-fluoro-3-(3-chloro-2-fluoro-6-hydroxyphenyl)-6,6a,7,8,9,10-hexahydro-12*H*-pyrazino[2,1-*c*]pyrido[3,4-*f*][1,4]oxazepin-12-one
Z340-2: (6a*R*)-8-acryloyl-1-((*R*)-3-hydroxy-5,5-dimethylpyrrolidin-1-yl)-4-fluoro-3-(3-chloro-2-fluoro-6-hydroxyphenyl)-6,6a,7,8,9,10-hexahydro-12*H*-pyrazino[2,1-*c*]pyrido[3,4-*f*][1,4]oxazepin-12-one
Z341-1: (6a*R*)-8-acryloyl-1-((*S*)-3-hydroxy-5,5-dimethylpyrrolidin-1-yl)-4-fluoro-3-(3-chloro-6-fluoro-2-hydroxyphenyl)-6,6a,7,8,9,10-hexahydro-12*H*-pyrazino[2,1-*c*]pyrido[3,4-*f*][1,4]oxazepin-12-one
Z341-2: (6a*R*)-8 acryloyl-1-((*R*)-3-hydroxy-5,5-dimethylpyrrolidin-1-yl)-4-fluoro-3-(3-chloro-6-fluoro-2-hydroxyphenyl)-6,6a,7,8,9,10-hexahydro-12*H*-pyrazino[2,1-*c*]pyrido[3,4-*f*][1,4]oxazepin-12-one
Z345-1: (6a*R*)-8-acryloyl-1-((*R*)-2,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z345-2: (6a*R*)-8-acryloyl-1-((*S*)-2,4-dimethylpyrrolo[3,4-*c*]pyrazol-5(2*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z346-1: (6a*R*)-8-acryloyl-1-((*R*)-1,4-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z346-2: (6a*R*)-8-acryloyl-1-((*S*)-1,4-dimethylpyrrolo[3,4-*c*]pyrazol-5(1*H*,4*H*,6*H*)-yl)-4-chloro-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z469-1: (6a*R*)-8-acryloyl-4-chloro-1-((*S*)-2,2-dimethyl-4-(4-methylpiperazin-1-yl)pyrrolidin-l-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z469-2: (6a*R*)-8-acryloyl-4-chloro-1-((*R*)-2,2-dimethyl-4-(4-methylpiperazin-1-yl)pyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z471-1: (*R*)-8-acryloyl-1-((*S*)-4-(azetidin-1-yl)-2,2-dimethylpyrrolidin-1-yl)-4-chloro-3-(2-fluorophenyl)-6,6a,7,8,9,10-hexahydro-12*H*-pyrazino[2,1-*c*]pyrido[3,4*f*][1,4]oxazepin-12-one
Z471-2: (*R*)-8-acryloyl-1-((*R*)-4-(azetidin-1-yl)-2,2-dimethylpyrrolidin-1-yl)-4-chloro-3-(2-fluorophenyl)-6,6a,7,8,9,10-hexahydro-12*H*-pyrazino[2,1-*c*]pyrido[3,4*f*][1,4]oxazepin-12-one
Z474-1: (*R*)-8-acryloyl-1-((*S*)-2,2-dimethyl-4-(4-methylpiperazin-1-yl)pyrrolidin-1-yl)-4-fluoro-3-(2-fluorophenyl)-6,6a,7,8,9,10-hexahydro-12*H*-pyrazino[2,1-*c*]pyrido[3,4*f*][1,4]oxazepin-12-one
Z474-2: (R)-8-acryloyl-1-((R)-2,2-dimethyl-4-(4-methylpiperazin-1-yl)pyrrolidin-1-yl)-4-fluoro-3-(2-fluorophenyl)-6,6a,7,8,9,10-hexahydro-12*H*-pyrazino[2,1-*c*]pyrido[3,4*f*][1,4]oxazepin-12-one
Z406-1: (R)-8-acryloyl-4-chloro-3-(2-fluorophenyl)-1-((R)-4-((2-methoxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z406-2: (*R*)-8-acryloyl-4-chloro-3-(2-fluorophenyl)-1-((*S*)-4-((2-methoxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z468-1: (R)-8-acryloyl-4-chloro-1-((R)-2,2-dimethyl-4-morpholinopyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z468-2: (*R*)-8-acryloyl-4-chloro-1-((*S*)-2,2-dimethyl-4-moipholinopyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z455-1: (3*R*,6a*R*)-8-acryloyl-4-chloro-3-(2-chloro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z455-2: (3*S*,6a*R*)-8-acryloyl-4-chloro-3-(2-chloro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z454-1: (*R*)-8-acryloyl-4-chloro-1-((*S*)-2,2-dimethyl-4-(4-methylpiperazin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z454-2: (*R*)-8-acryloyl-4-chloro-1-((*R*)-2,2-dimethyl-4-(4-methylpiperazin-1-yl)pyrrolidin-1-yl)-3-(2-fhiorophenyl)-6,6a,7,8,9,10-hexahydro-12*H*-pyrazino[2,1-*c*]pyrido[3,4-*f*][1,4]oxazepin-12-one
Z458-1: (3*S*)-1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidine-3-carboxylic acid
Z458-2: (3*R*)-1-((6a*R*)-8-acryloyl-4-chloro-3-(2-fluoro-6-hydroxyphenyl)-12-oxo-6,6a,7,8,9,10-hexahydro-12*H*-pyrazino[2,1-*c*]pyrido[3,4-*f*][1,4]oxazepin-1-yl)-5,5-dimethylpyrrolidine-3-carboxylic acid
Z465-1: (*R*)-8-acryloyl-4-chloro-3-(2-fluorophenyl)-1-((2*R*,3*S*)-2,3,4-trimethylpiperazin-1-yl)-6,6a,7,8,9,10-hexahydro-12*H*-pyrazino[2,1-*c*]pyrido[3,4-*f*][1,4]oxazepin-12-one
Z465-2: (*R*)-8-acryloyl-4-chloro-3-(2-fluorophenyl)-1-((2*S*,3*R*)-2,3,4-trimethylpiperazin-1-yl)-6,6a,7,8,9,10-hexahydro-12*H*-pyrazino[2,1-*c*]pyrido[3,4-*f*][1,4]oxazepin-12-one
Z360-1: (6aR)-8-acryloyl-4-chloro-1-((R)-3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z360-2: (6a*R*)-8-acryloyl-4-chloro-1-((*S*)-3-(dimethylamino)-2,2-dimethylpyrrolidin-1-yl)-3-(2-fluoro-6-hydroxyphenyl)-6,6a,7,8,9,10-hexahydro-12*H*-pyrazino[2,1-*c*]pyrido[3,4-*f*][1,4]oxazepin-12-one
Z466-1: (2S,3R)-1-((6aR)-8-acryloyl-4-chloro-3-(2-fluoro-6-methoxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide
Z466-2: (2*S*,3*S*)-1-((6a*R*)-8-acryloyl-4-chloro-3-(2-fluoro-6-methoxyphenyl)-12-oxo-6,6a,7,8,9,10-hexahydro-12*H*-pyrazino[2,1-*c*]pyrido[3,4-*f*][1,4]oxazepin-1-yl)-*N*,2-dimethylpyrrolidine-2-carboxamide
Z342: (6a*R*)-8-acryloyl-4-chloro-3-(3-chloro-6-fluoro-2-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z343: (6a*R*)-8-acryloyl-4-chloro-3-(3-chloro-2-fluoro-6-hydroxyphenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z475-1: (6a*R*)-8-acryloyl-4-chloro-3-(3,6-difluoro-2-hydroxyphenyl)-1-((*S*)-4-(dimethylamino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z475-2: (6a*R*)-8-acryloyl-4-chloro-3-(3,6-difluoro-2-hydroxyphenyl)-1-((*R*)-4-(dimethylamino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z480-1: (*R*)-8-acryloyl-4-chloro-1-((*S*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z480-2: (*R*)-8-acryloyl-4-chloro-1-((*R*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z479-1: (*R*)-8-acryloyl-4-chloro-3-(2-fluorophenyl)-1-((*S*)-4-(3-methoxyazetidin-1-yl)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z479-2: (*R*)-8-acryloyl-4-chloro-3-(2-fluorophenyl)-1-((*R*)-4-(3-methoxyazetidin-1-yl)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z458: 1-((6aR)-8-acryloyl-4-chloro-3-(2-fluoro-6-hydroxyphenyl)-12-oxo-6,6a,7,8,9,10-hexahydro-12*H*-pyrazino[2,1*-*c]pyrido[3,4-*f*][1,4]oxazepin-1-yl)-5,5-dimethylpyrrolidine-3-carboxylic acid
Z459: (*R*)-8-acryloyl-4-chloro-1-((*S*)-2,4-dimethylpiperazin-1-yl)-3-(prop-1-yn-1-yl)-6,6a,7,8,9,10-hexahydro-12*H*-pyrazino[2,1-*c*]pyrido[3,4-*f*][1,4]oxazepin-12-one
Z453-1: (*R*)-8-acryloyl-4-chloro-1-((*S*)-4-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z453-2: (R)-8-acryloyl-4-chloro-1-((R)-4-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z473-1: (*R*)-8-acryloyl-4-chloro-3-(2,3-difluorophenyl)-1-((*S*)-4-(dimethylamino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z473-2: (*R*)-8-acryloyl-4-chloro-3-(2,3-difluorophenyl)-1-((*R*)-4-(dimethylamino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z472-1: (*R*)-8-acryloyl-4-chloro-1-((*S*)-4-(dimethylamino)-2,2-dimethylpyrrolidin-1-yl)-3-(2-fluoro-3-methylphenyl)-6,6a,7,8,9,10-hexahydro-12*H*-pyrazino[2,1-*c*]pyrido[3,4*-*f][1,4]oxazepin-12-one
Z472-2: (*R*)-8-acryloyl-4-chloro-1-((*R*)-4-(dimethylamino)-2,2-dimethylpyrrolidin-1-yl)-3-(2-fluoro-3-methylphenyl)-6,6a,7,8,9,10-hexahydro-12*H*-pyrazino[2,1-*c*]pyrido[3,4-*f*][1,4]oxazepin-12-one
Z408-1: (*R*)-8-acryloyl-4-fluoro-3-(2-fluorophenyl)-1-((*S*)-4-hydroxy-2,2-dimethylpyrrolidin-1-yl)-6,6a,7,8,9,10-hexahydro-2*H*-pyrazino[2,1-*c*]pyrido[3,4-*f*][1,4]oxazepin-12-one
Z414-1: (6a*R*)-8-acryloyl-3-(2-chlorophenyl)-4-fluoro-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z450: (6a*R*)-8-acryloyl-4-fluoro-3-(2-fluoro-6-methoxyphenyl)-1-(4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z481: (6a*R*)-8-acryloyl-3-(2-amino-6-fluorophenyl)-4-fluoro-1-((*S*)-4-hydroxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z637: 1-(1-((*R*)-8-acryloyl-4-chloro-3-(2-fluorophenyl)-12-oxo-6,6a,7,8,9,10-hexahydro-12*H-*pyrazino[2,1-*c*]pyrido[3,4-*f*][1,4]oxazepin-1-yl)-5,5-dimethylpyrrolidin-3-yl)azetidine-3-carbonitrile
Z637-1: 1-((*R*)-1-((*R*)-8-acryloyl-4-chloro-3-(2-fluorophenyl)-12-oxo-6,6a,7,8,9,10-hexahydro-12*H-*pyrazino[2,1-*c*]pyrido[3,4-*f*][1,4]oxazepin-1-yl)-5,5-dimethylpyrrolidin-3-yl)azetidine-3-carbonitrile
Z637-2: 1-((*S*)-1-((*R*)-8-acryloyl-4-chloro-3-(2-fluorophenyl)-12-oxo-6,6a,7,8,9,10-hexahydro-12*H-*pyrazino[2,1-*c*]pyrido[3,4-*f*][1,4]oxazepin-1-yl)-5,5-dimethylpyrrolidin-3-yl)azetidine-3-carbonitrile
Z491a: (6a*R*)-8-acryloyl-4-chloro-1-(2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z491: (*R*)-8-acryloyl-4-chloro-1-((*S*)-2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4*-*f][1,4]oxazepin-12-one
Z493: (*R*)-8-acryloyl-4-chloro-1-((*R*)-2,2-dimethyl-4-(methyl(2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4*-*f][1,4]oxazepin-12-one
Z503: (6a*R*)-8-acryloyl-3-(2-amino-6-fluorophenyl)-4-chloro-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z508MD: (6a*R*)-8-acryloyl-4-chloro-1-(2,6-dimethyl-2,7-diazaspiro[3.5]nonan-7-yl)-3-(2-fluorophenyl)-6,6a,7,8,9,10-hexahydro-12*H*-pyrazino[2,1-*c*]pyrido[3,4-*f*][1,4]oxazepin-12-one
Z635: (6a*R*)-8-acryloyl-1-(2,2-dimethyl-4-(4-methylpiperazin-1-yl)pyrrolidin-1-yl)-4-fluoro-3-(2-chloro-6-fluoro-phenyl)-6,6a,7,8,9,10-hexahydro-12*H*-pyrazino[2,1-*c*]pyrido[3,4-*f*][1,4]oxazepin-12-one
Z635-1: (6a*R*)-8-acryloyl-4-chloro-3-(2-chloro-6-fluorophenyl)-1-((*S*)-2,2-dimethyl-4-(4-methylpiperazin-1-yl)pyrrolidon-1-yl)-6,6a,7,8,9,10-hexahydro-12*H*-pyrazino[2,1-*c*]pyrido[3,4-*f*][1,4]oxazepin-12-one
Z635-2: (6a*R*)-8-acryloyl-4-chloro-3-(2-chloro-6-fluorophenyl)-1-((*R*)-2,2-dimethyl-4-(4-methylpiperazin-1-yl)pyrrolidon-1-yl)-6,6a,7,8,9,10-hexahydro-12*H*-pyrazino[2,1-*c*]pyrido[3,4-*f*][1,4]oxazepin-12-one
Z547: (6a*R*)-8-acryloyl-4-chloro-3-(2-chloro-6-fluorophenyl)-1-((*S*)-2,4-dimethylpiperazin-1-yl)-6,6a,7,8,9,10-hexahydro-12*H*-pyrazino[2,1-*c*]pyrido[3,4-*f*][1,4]oxazepin-12-one
Z608: (6a*R*)-8-acryloyl-4-chloro-1-(2,2-dimethyl-4-(3-(trifluoromethyl)azetidin-1-yl)pyrrolidin-1-yl)-3-(2-fluorophenyl)-6,6a,7,8,9,10-hexahydro-12*H*-pyrazino[2,1-*c*]pyrido[3,4*-f*][1,4]oxazepin-12-one
Z608-1: (*R*)-8-acryloyl-4-chloro-1-((*R*)-2,2-dimethyl-4-(3-(trifluoromethyl)azetidin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z608-2: (*R*)-8-acryloyl-4-chloro-1-((*S*)-2,2-dimethyl-4-(3-(trifluoromethyl)azetidin-1-yl)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one formate
Z525: (6aR)-8-acryloyl-4-chloro-1-(4-(3,3-difluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z525-1: (*R*)-8-acryloyl-4-chloro-1-((*R*)-4-(3,3-difluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z525-2: (*R*)-8-acryloyl-4-chloro-1-((*S*)-4-(3,3-difluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4*-*f][1,4]oxazepin-12-one \
Z502: (6a*R*)-8-acryloyl-4-chloro-1-(3-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z502-1: (*R*)-8-acryloyl-4-chloro-1-((*R*)-3-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z502-2: (*R*)-8-acryloyl-4-chloro-1-((*S*)-3-(dimethylamino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z506: (6a*R*)-8 acryloyl-3-(2-amino-6-fluorophenyl)-4-chloro-1-(3-methoxy-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z483: (*R*)-8-acryloyl-4-chloro-3-(2-fluorophenyl)-1-((2*S*,5*S*)-2,4,5-trimethylpiperazin-1-yl)-6,6a,7,8,9,10-hexahydro-12*H*-pyrazino[2,1-*c*]pyrido[3,4-*f*][1,4]oxazepin-12-one
Z532: (6a*R*)-8-acryloyl-4-chloro-1-(3,3-difluoro-5',5'-dimethyl-[1,3'-bipyrrolidin]-1'-yl)-3-(2-fluorophenyl)-6,6a,7,8,9,10-hexahydro-12*H*-pyrazino[2,1-*c*]pyrido[3,4-*f*][1,4]oxazepin-12-one
Z653: (*R*)-8-acryloyl-4-chloro-3-(2-fluorophenyl)-1-((2*S*,3*S*)-2,3,4-trimethylpiperazin-1-yl)-6,6a,7,8,9,10-hexahydro-12*H*-pyrazino[2,1-*c*]pyrido[3,4-*f*][1,4]oxazepin-12-one
Z654: (6a*R*)-8-acryloyl-4-fluoro-1-(4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z654-1: (*R*)-8-acryloyl-4-fluoro-1-((*S*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z654-2: (*R*)-8-acryloyl-4-fluoro-1-((*R*)-4-(3-fluoroazetidin-1-yl)-2,2-dimethylpyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z655: (6a*R*)-8-acryloyl-4-chloro-1-(4-(dimethylamino)-2,2-dimethylpyrrolidin-1-yl)-3-(phenyl)-6,6a,7,8,9,10-hexahydro-12*H*-pyrazino[2,1-*c*]pyrido[3,4-*f*][1,4]oxazepin-12-one
Z655-1: (*R*)-8-acryloyl-4-chloro-1-((*S*)-4-(dimethylamino)-2,2-dimethylpyrrolidin-1-yl)-3-(phenyl)-6,6a,7,8,9,10-hexahydro-12*H*-pyrazino[2,1-*c*]pyrido[3,4-*f*][1,4]oxazepin-12-one
Z655-2: (R)-8-acryloyl-4-chloro-1-((R)-4-(dimethylamino)-2,2-dimethylpyrrolidin-1-yl)-3-(phenyl)-6,6a,7,8,9,10-hexahydro-12*H*-pyrazino[2,1-*c*]pyrido[3,4-*f*][1,4]oxazepin-12-one
Z656: (R)-8-acryloyl-4-chloro-3-(2-fluorophenyl)-N-isopropyl-N-methyl-6a,7,8,9,10,12-hexahydro-6*H*-pyrazino[2,1-*c*]pyrido[3,4-*f*][1,4]oxazepine-1-carboxamide
Z657: (6a*R*)-8-acryloyl-4-chloro-1-(4-(methylethylamine)-2,2-dimethylpyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z658: (6aR)-8-acryloyl-4-chloro-3-(2-fluorophenyl)-1-(4-((2-hydroxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z658-1: (*R*)-8-acryloyl-4-chloro-3-(2-fluorophenyl)-1-((*S*)-4-((2-hydroxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z658-2: ((*R*)-8-acryloyl-4-chloro-3-(2-fluorophenyl)-1-((*R*)-4-((2-hydroxyethyl)(methyl)amino)-2,2-dimethylpyrrolidin-1-yl)-6,6a,7,8,9,10-hexahydro-12*H*-pyrazino[2,1-*c*]pyrido[3,4-*f*][1,4]oxazepin-12-one
Z659: (6a*R*)-8-acryloyl-4-chloro-1-(4-(((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z659-1: (*R*)-8-acryloyl-4-chloro-1-((*S*)-4-((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z659-2: (*R*)-8-acryloyl-4-chloro-1-((*R*)-4-((2,2-difluoroethyl)(methyl)amino)-2,2-dimethylpyrrolidin-1-yl)-3-(2-fluorophenyl)-6,6a,7,8,9,10-hexahydro-12*H*-pyrazino[2,1-c]pyrido[3,4-*f*][1,4]oxazepin-12-one
Z660: (6a*R*)-8-acryloyl-4-chloro-1-(4-((2-fluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z660-1: (*R*)-8-acryloyl-4-chloro-1-((*S*)-4-((2-fluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one (Z660-1)
Z660-2: (R)-8-acryloyl-4-chloro-1-((R)-4-((2-fluoroethyl)(methyl)amino)-2,2-dimethylpyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one formate
Z661: (6aR)-8-acryloyl-4-chloro-1-(2,2-dimethyl-4-(methylamino)pyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z661-1: (*R*)-8-acryloyl-4-chloro-1-((*S*)-2,2-dimethyl-4-(methylamino)pyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z661-2: (*R*)-8-acryloyl-4-chloro-1-((*R*)-2,2-dimethyl-4-(methylamino)pyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z662: (6a*R*)-8-acryloyl-4-chloro-1-(2,2-dimethyl-4-((2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4*f*][1,4]oxazepin-12-one
Z662-1: (*R*)-8-acryloyl-4-chloro-1-((*S*)-2,2-dimethyl-4-((2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one
Z662-2: (*R*)-8-acryloyl-4-chloro-1-((*R*)-2,2-dimethyl-4-((2,2,2-trifluoroethyl)amino)pyrrolidin-1-yl)-3-(2-fluorophenyl)-6,6a,7,8,9,10-hexahydro-12*H*-pyrazino[2,1-*c*]pyrido[3,4-*f*][1,4]oxazepin-12-one

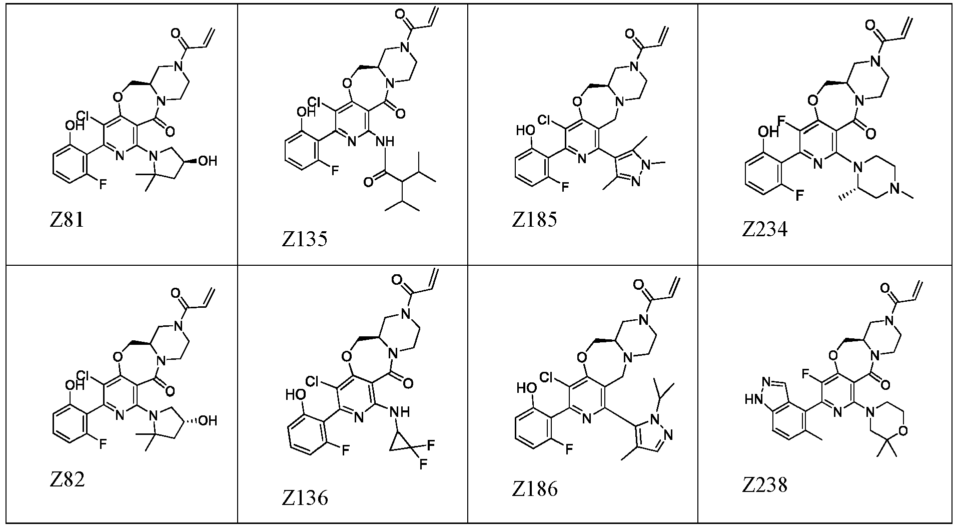

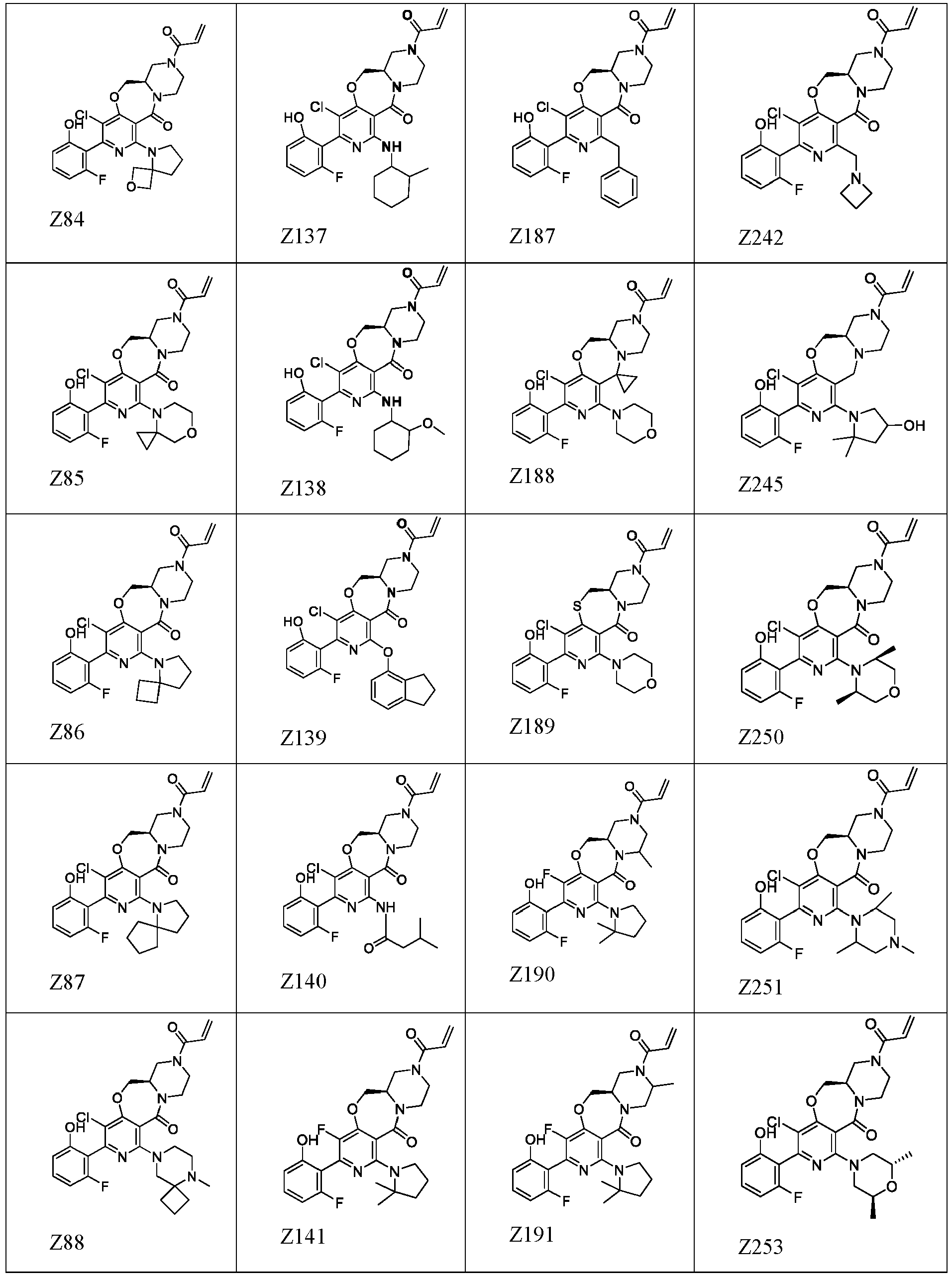

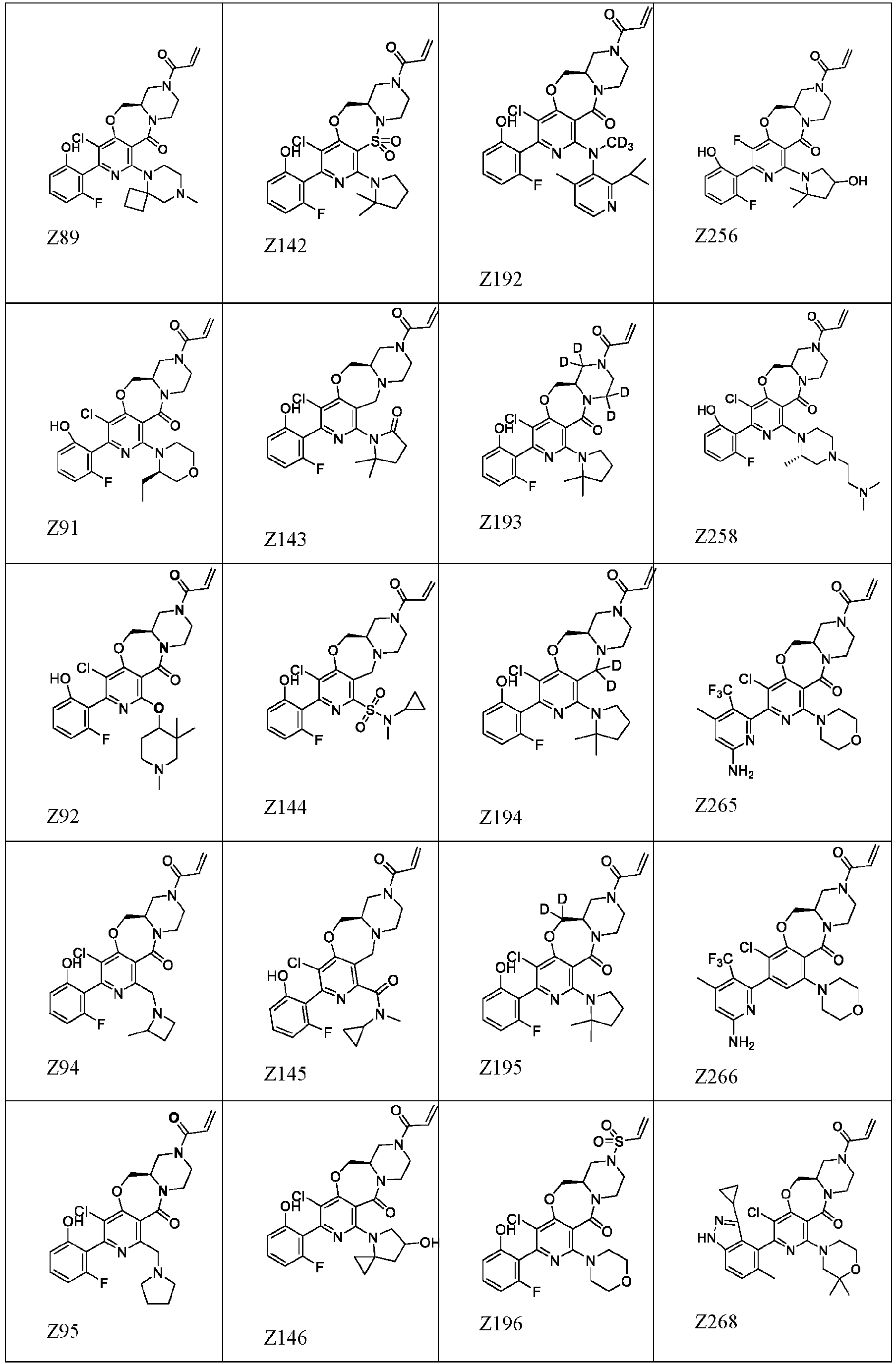

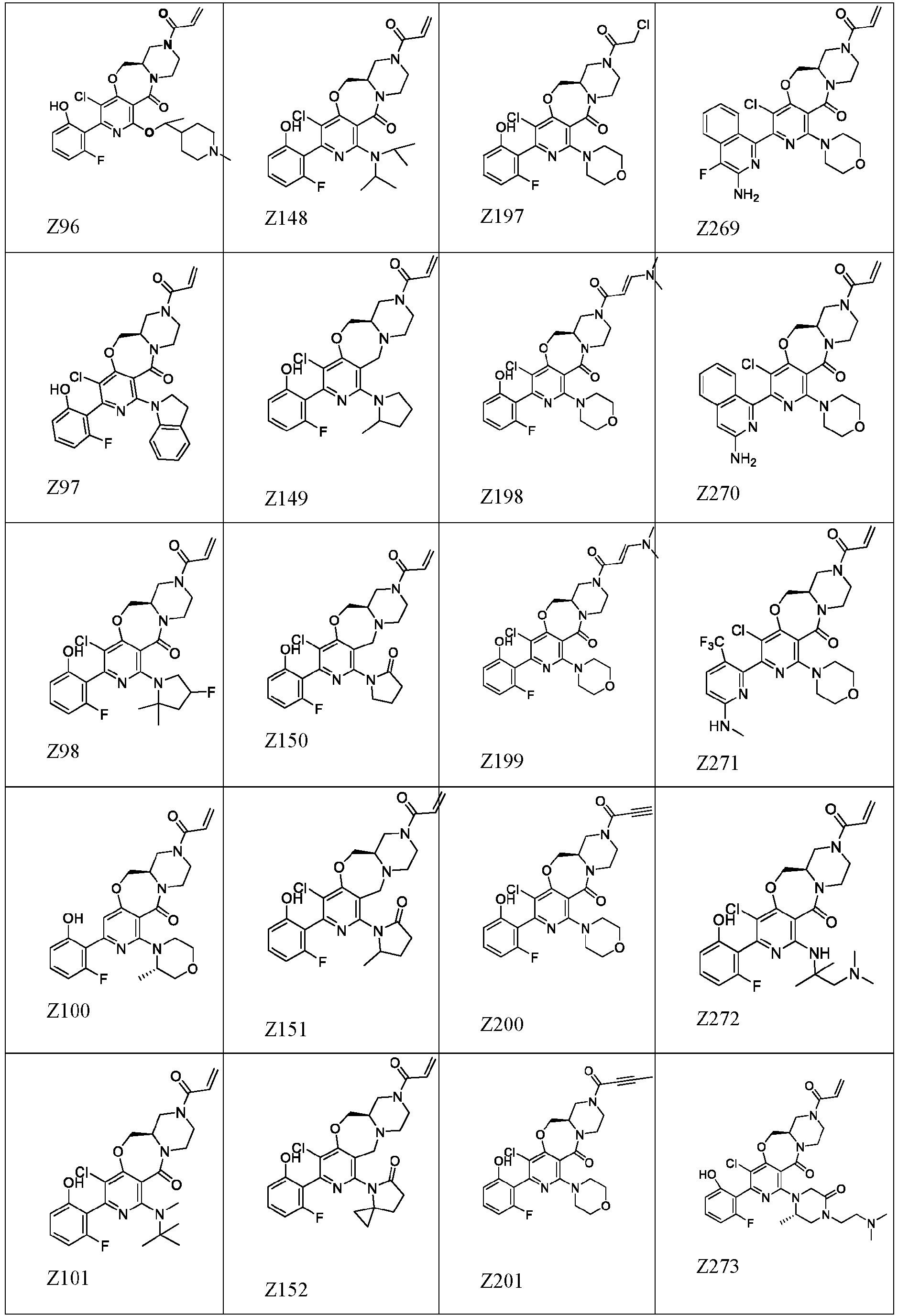

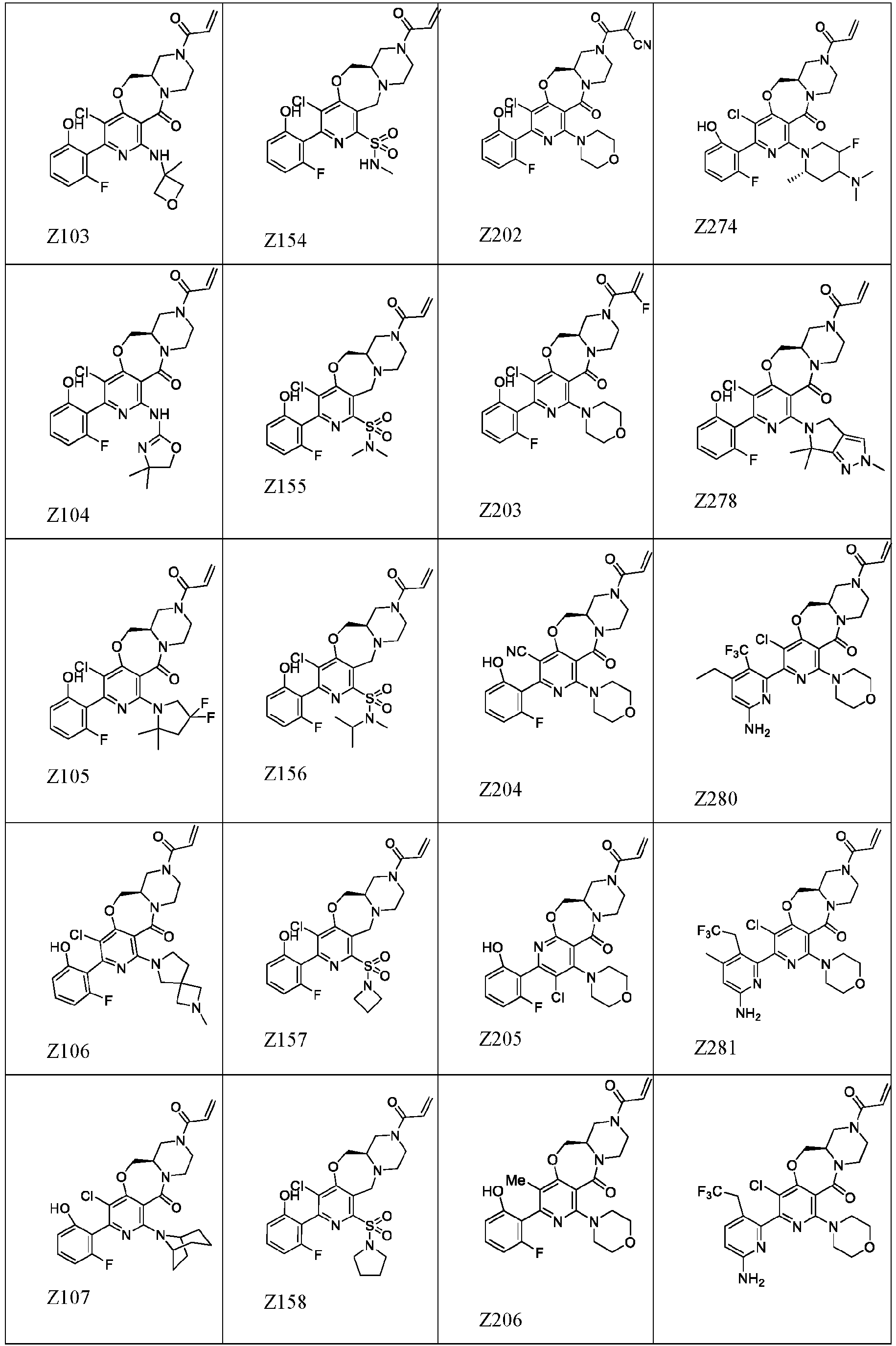

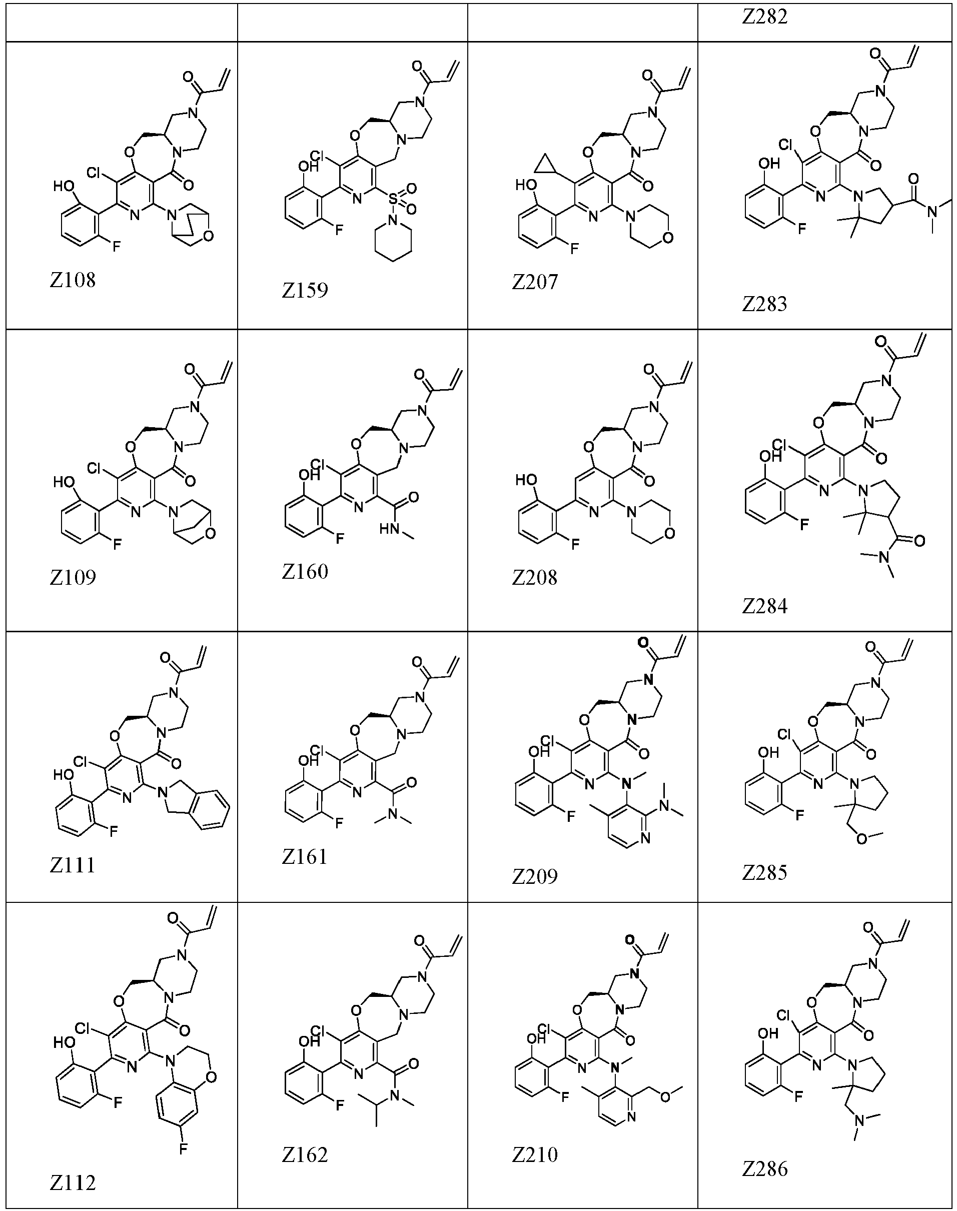

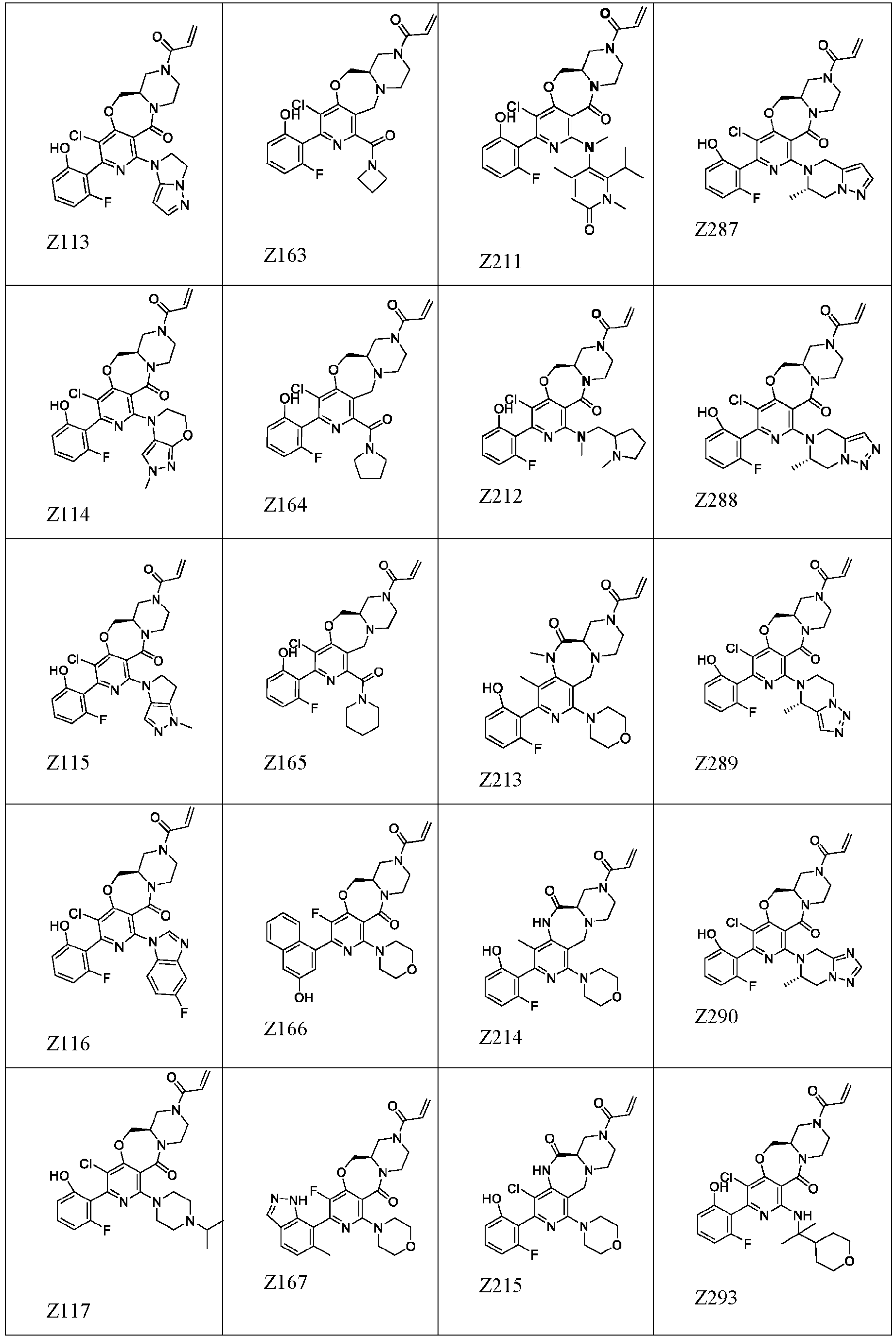

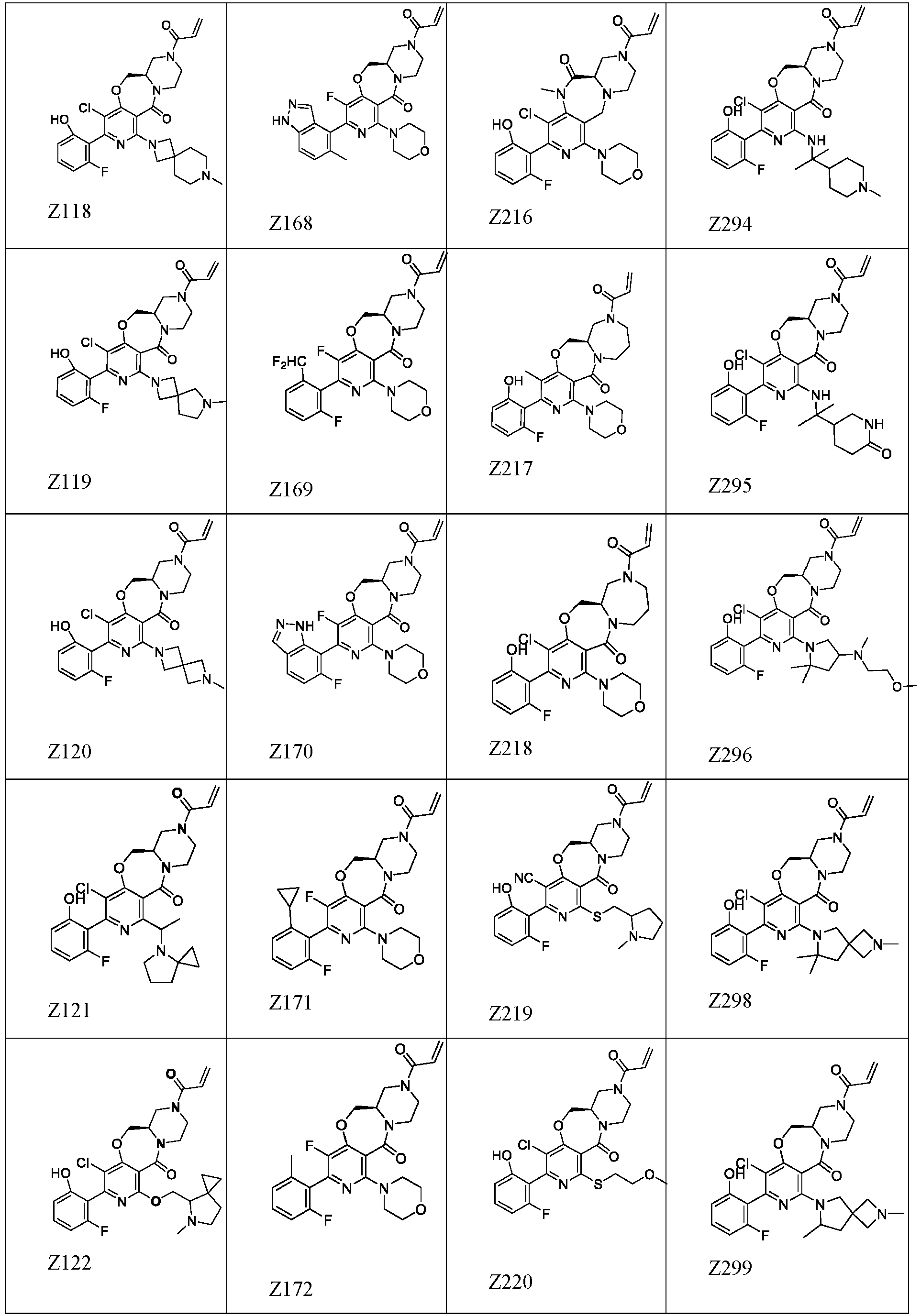

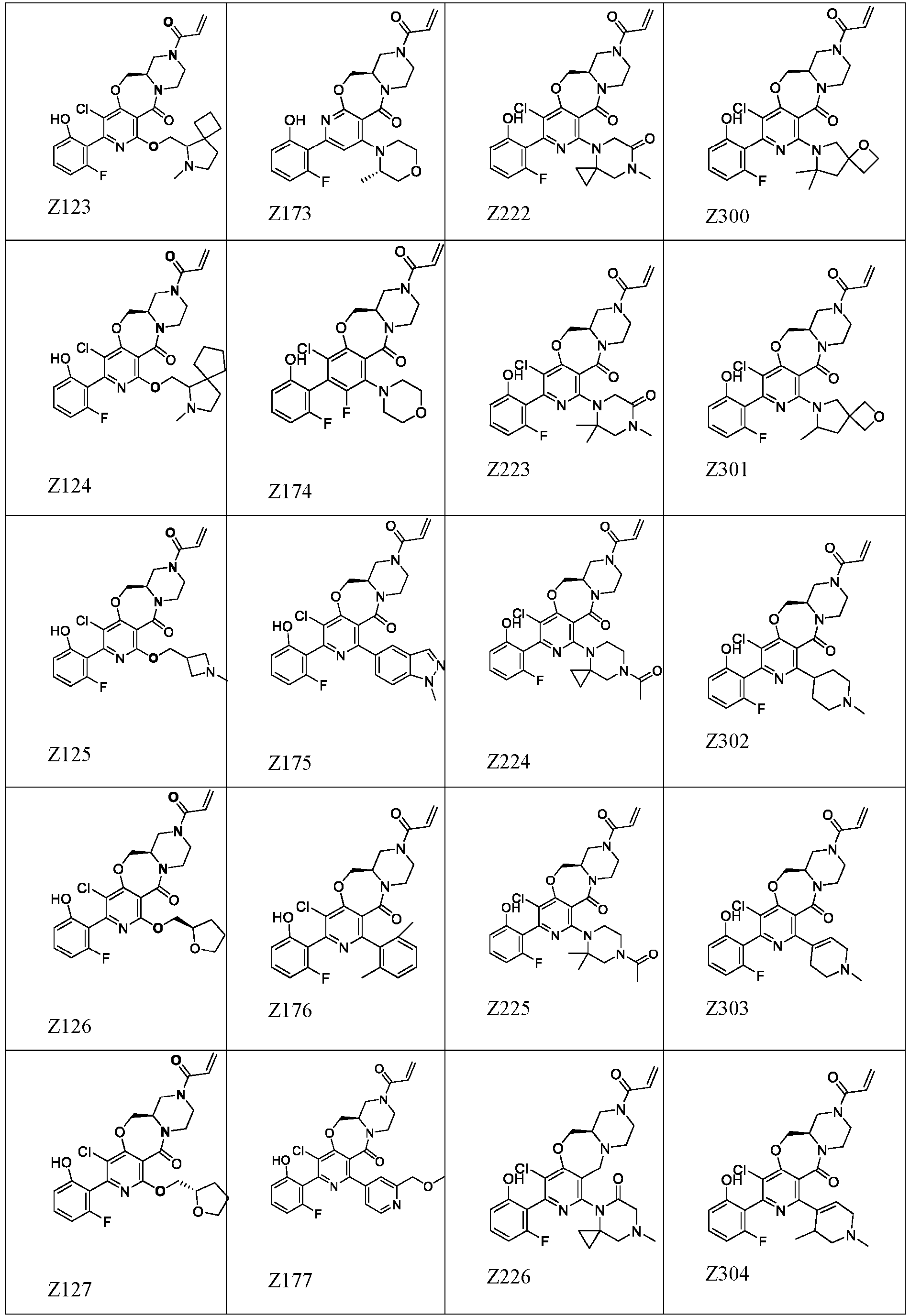

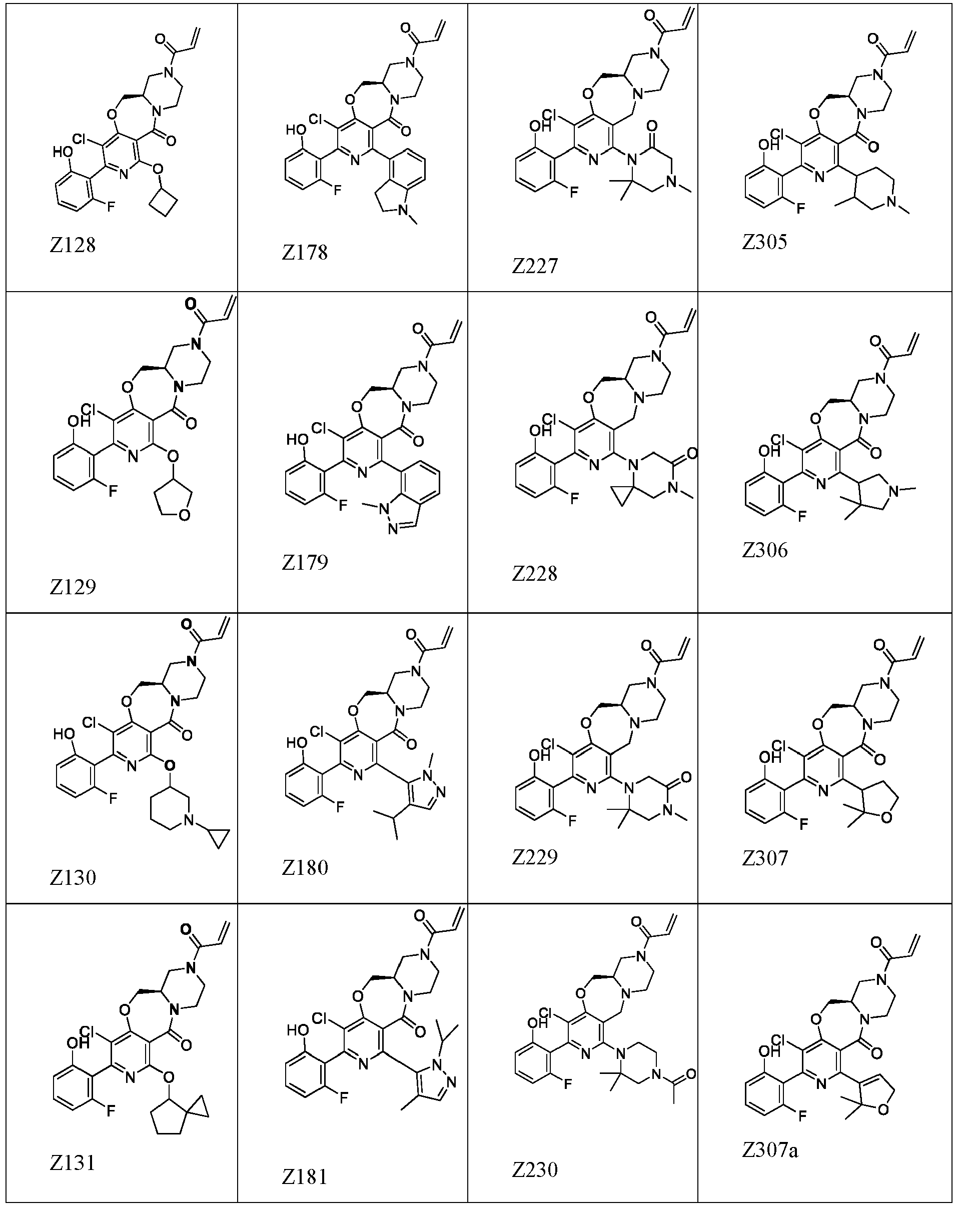

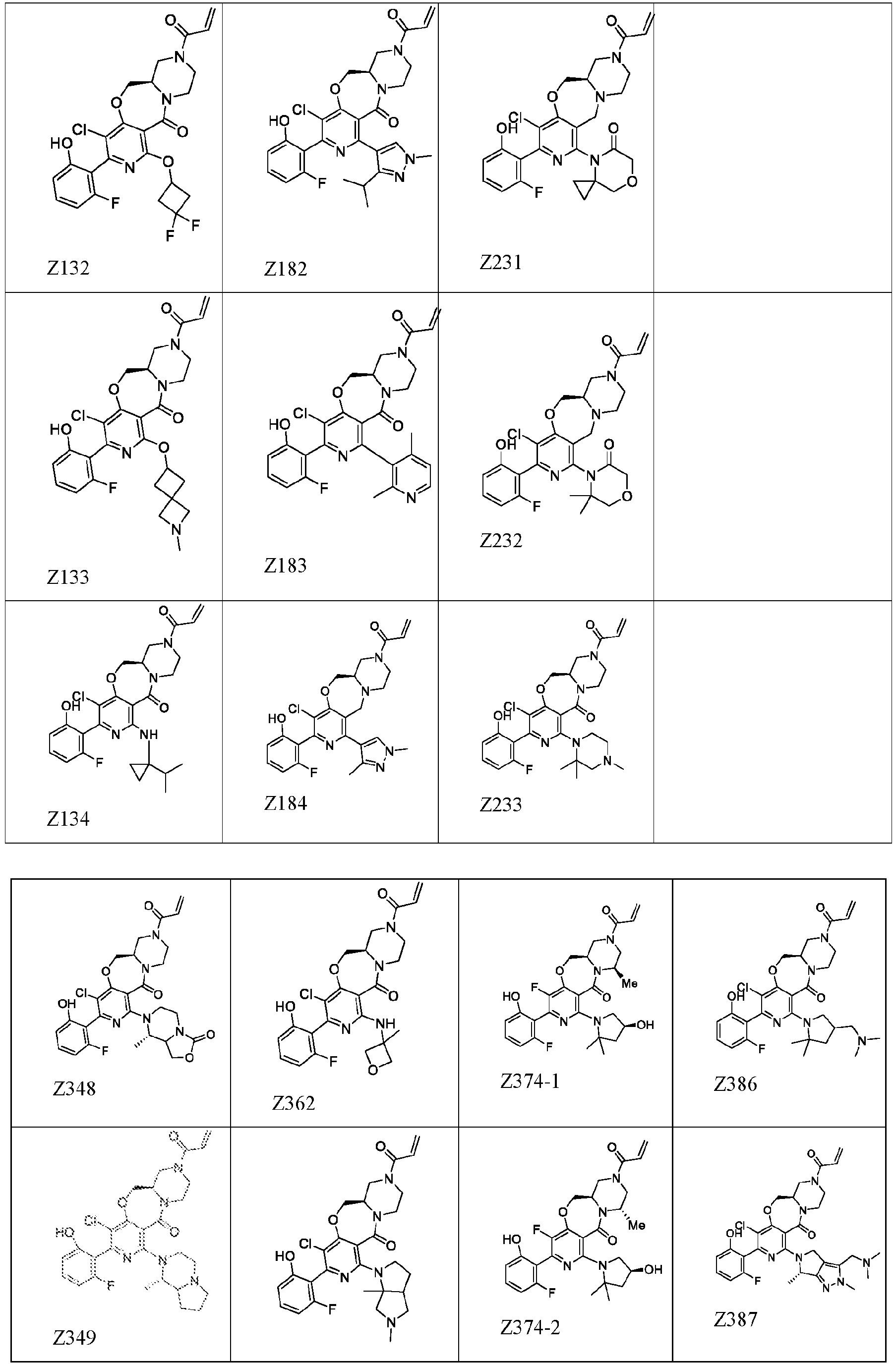

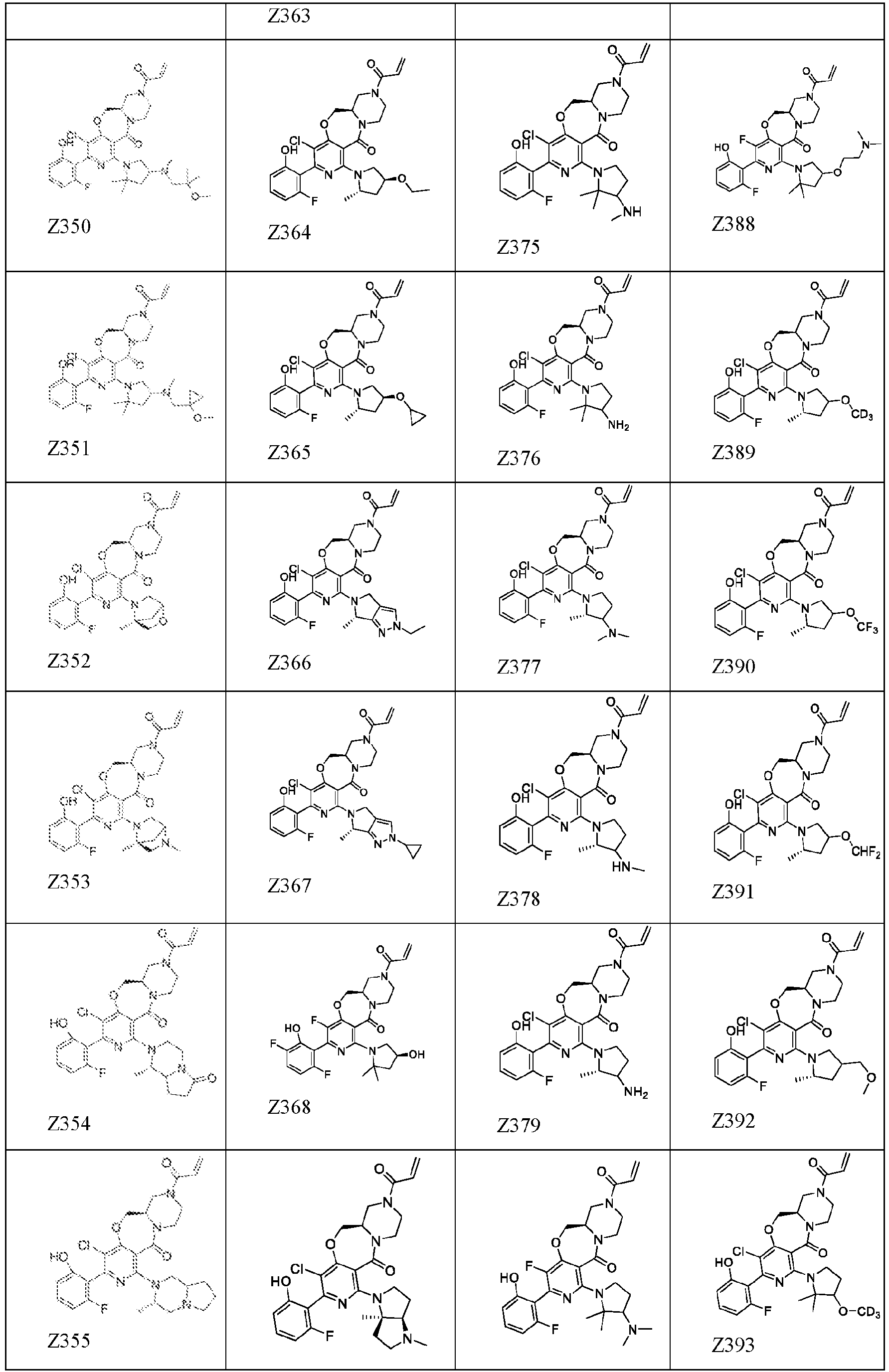

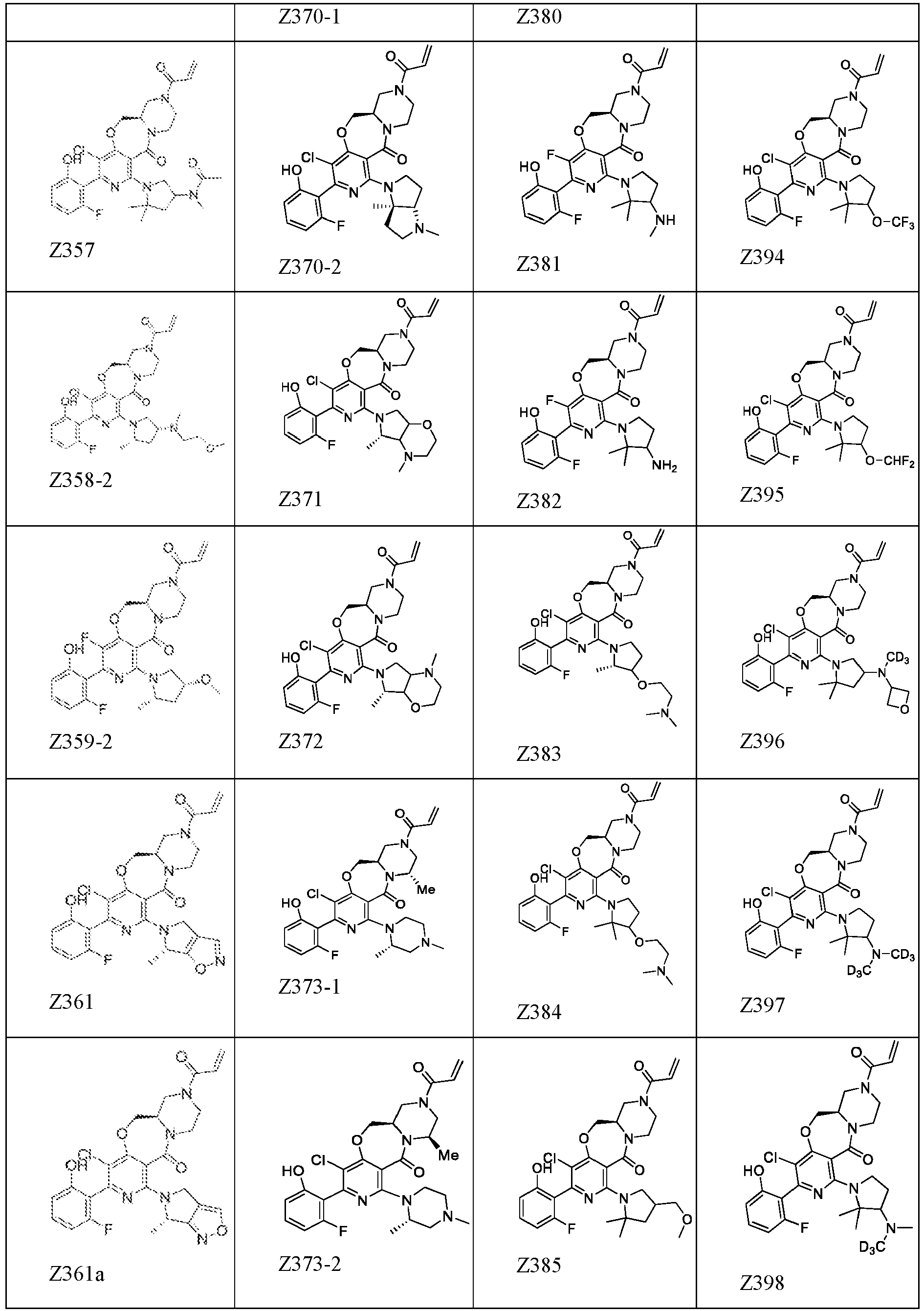

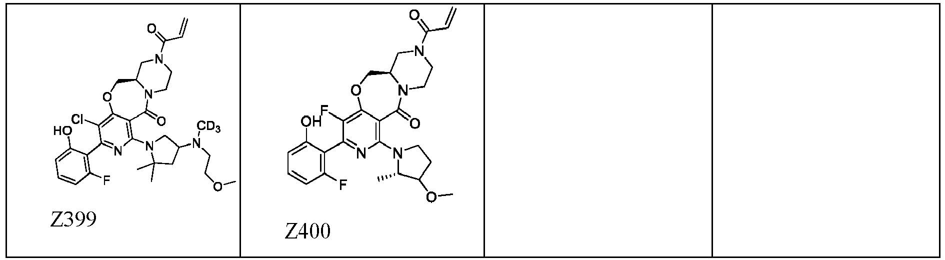

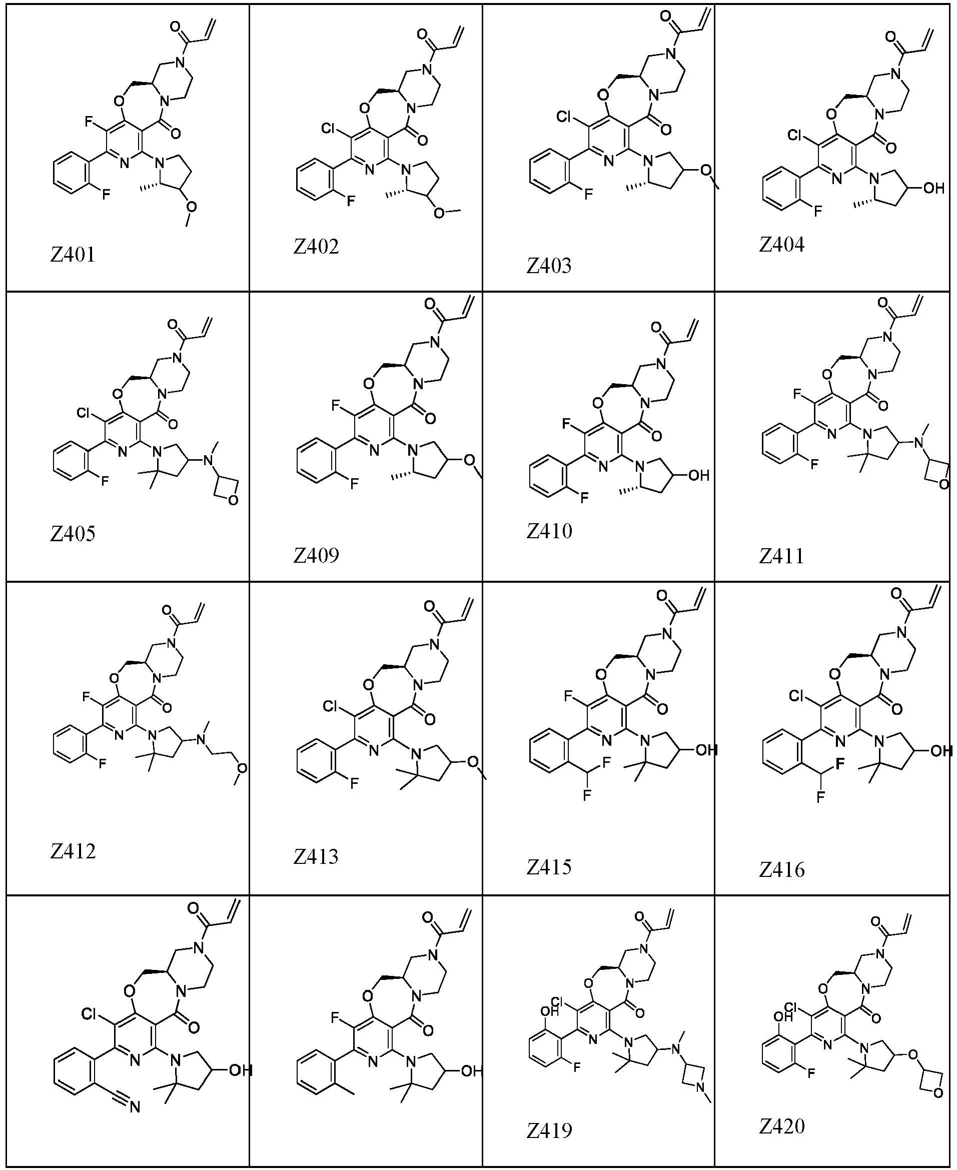

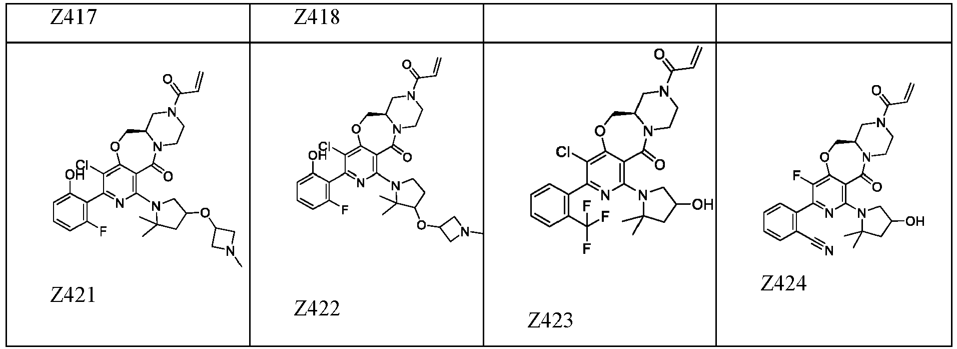

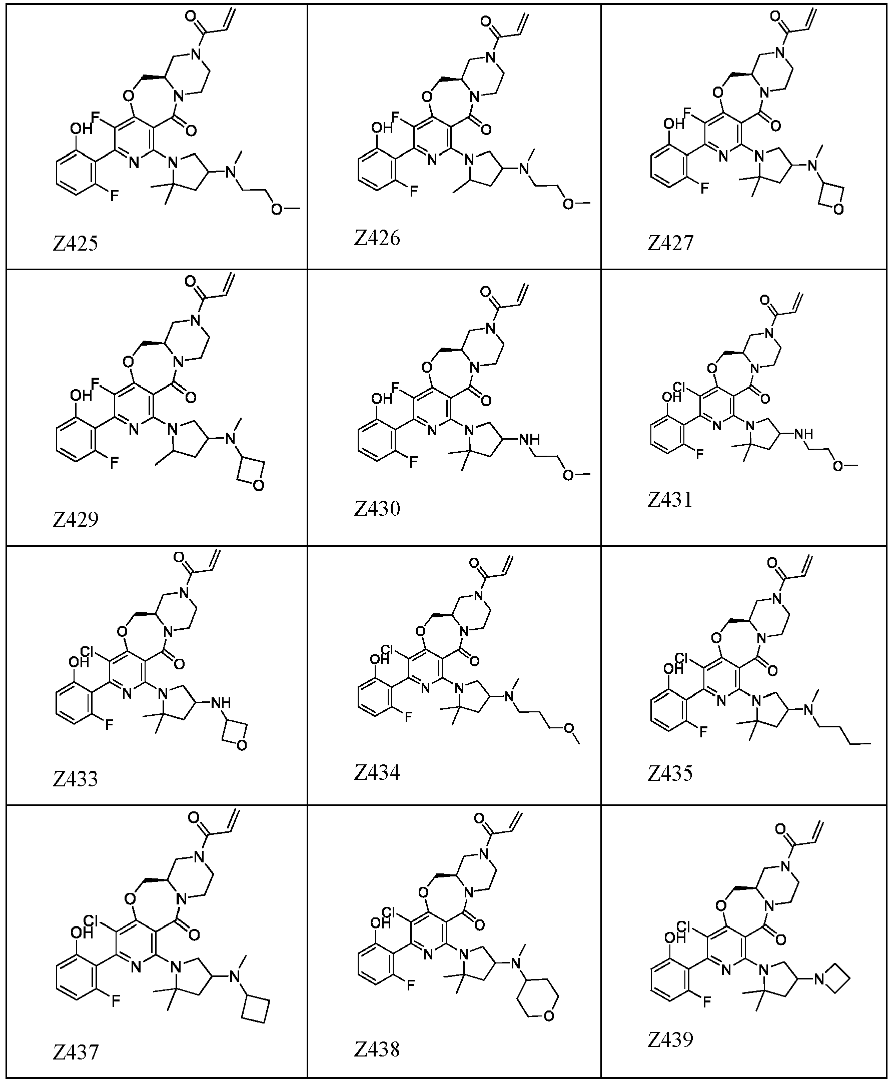

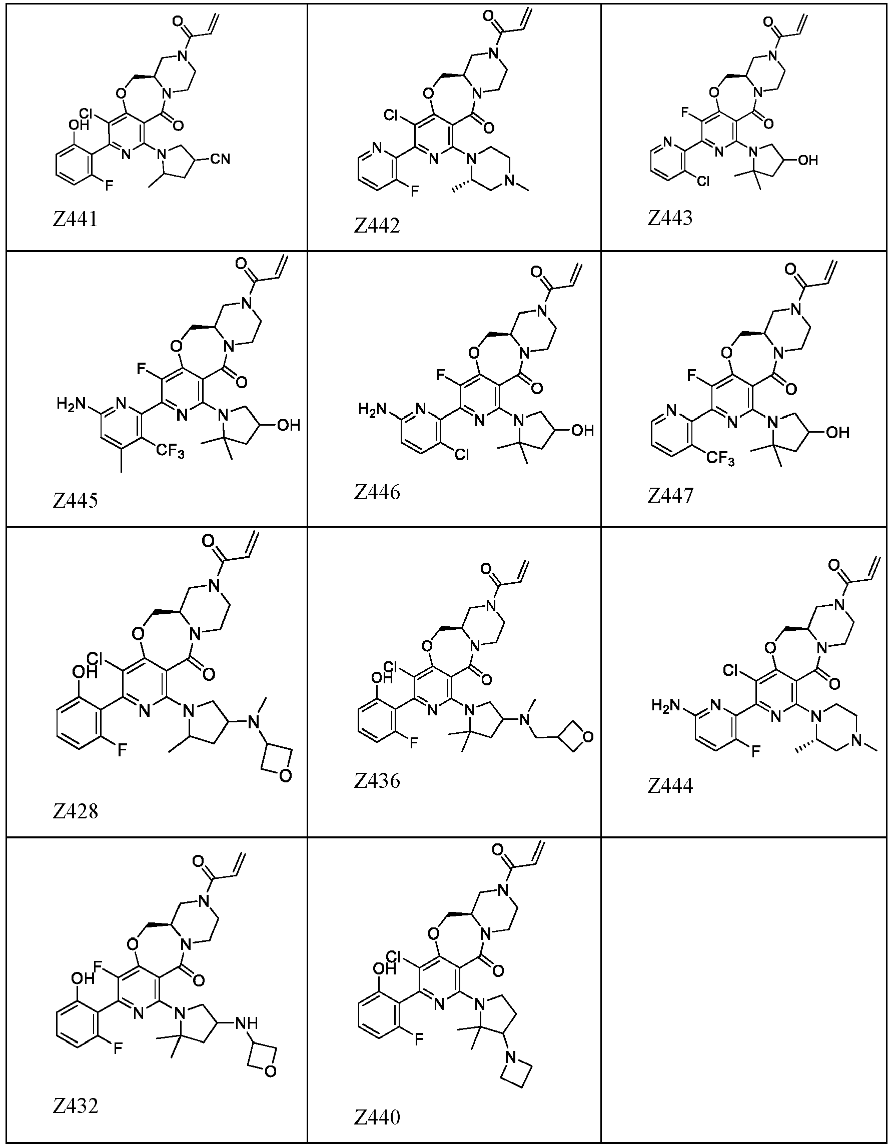

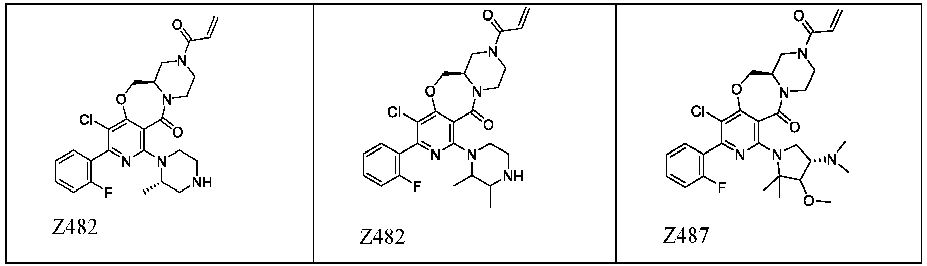

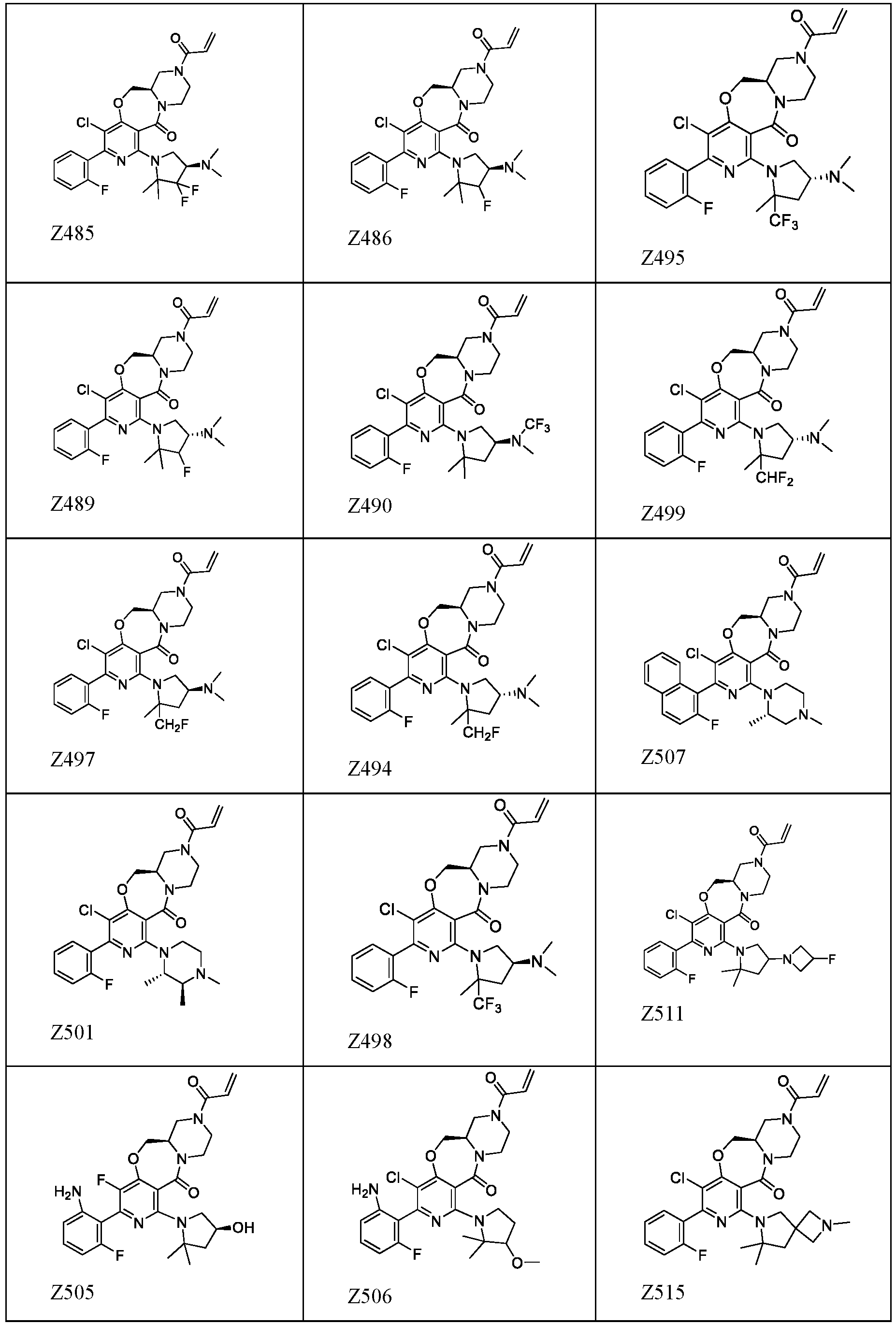

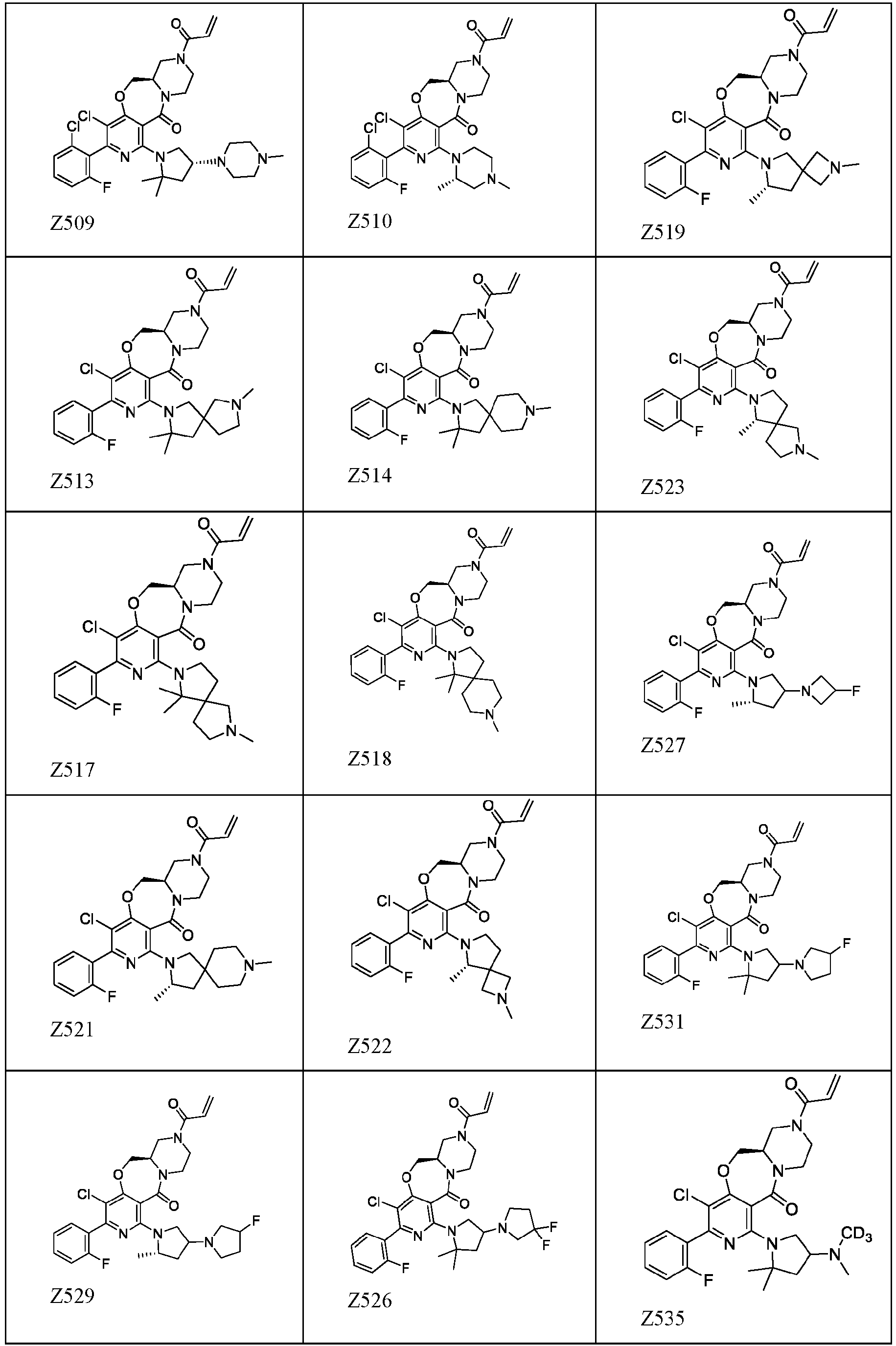

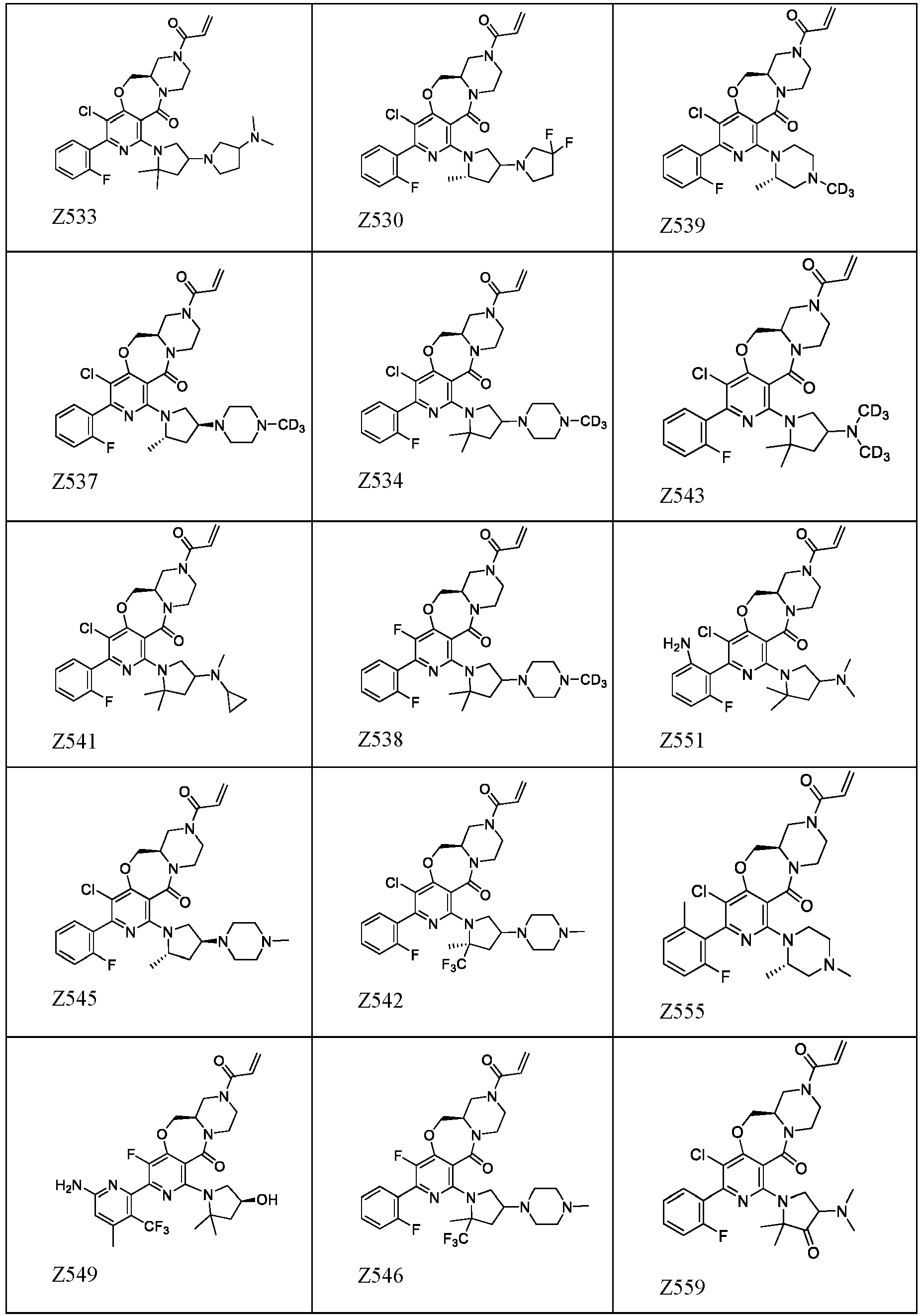

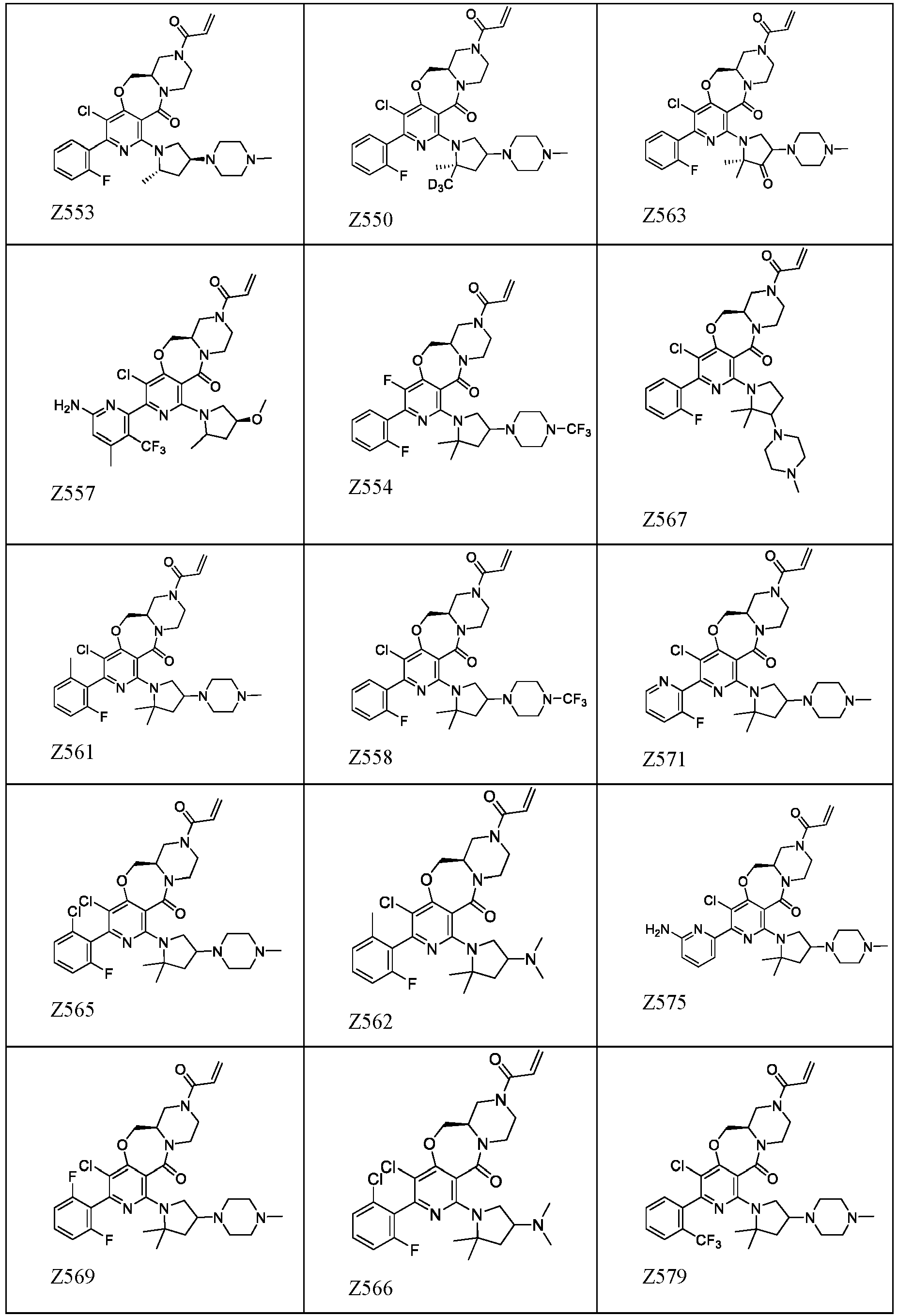

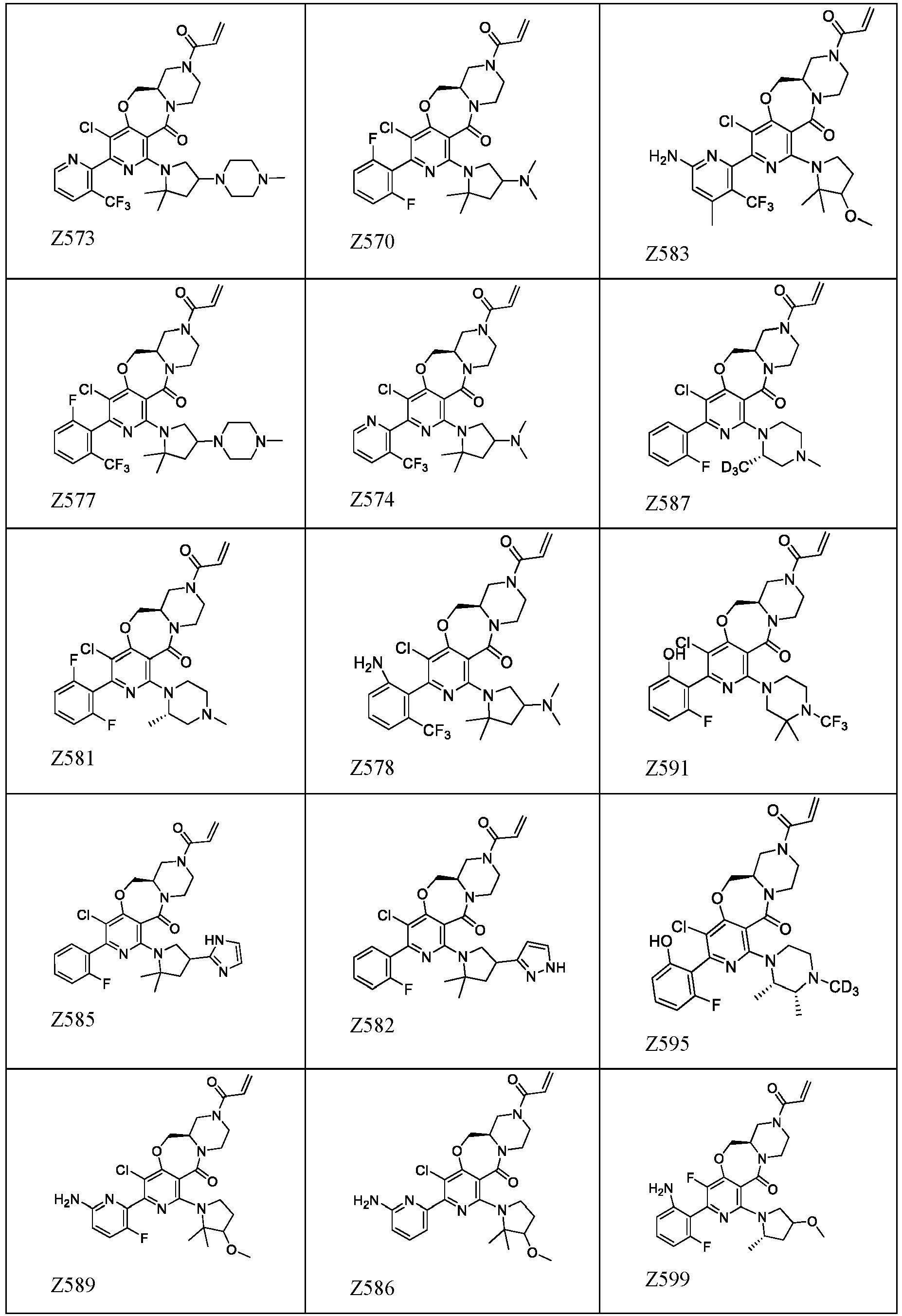

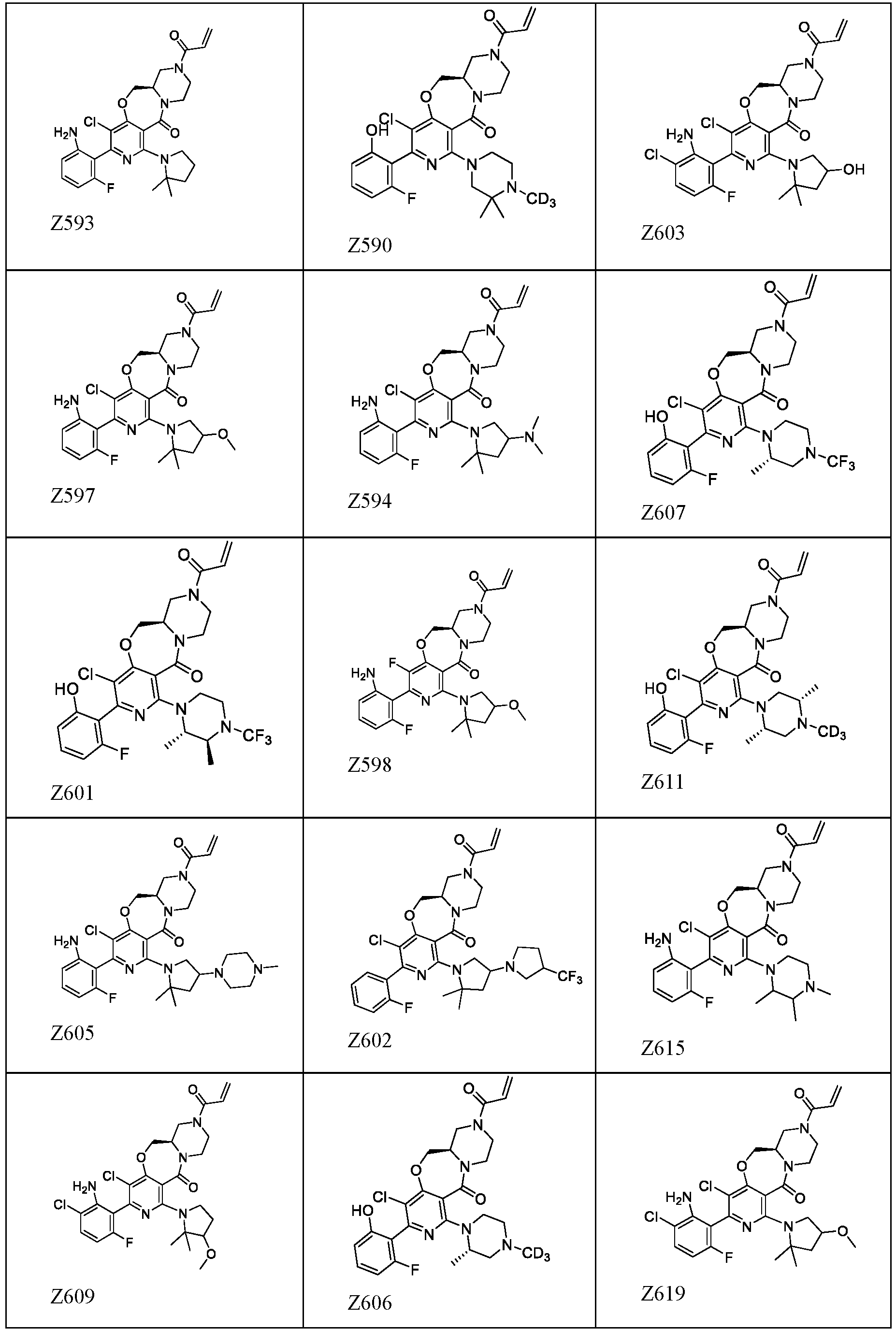

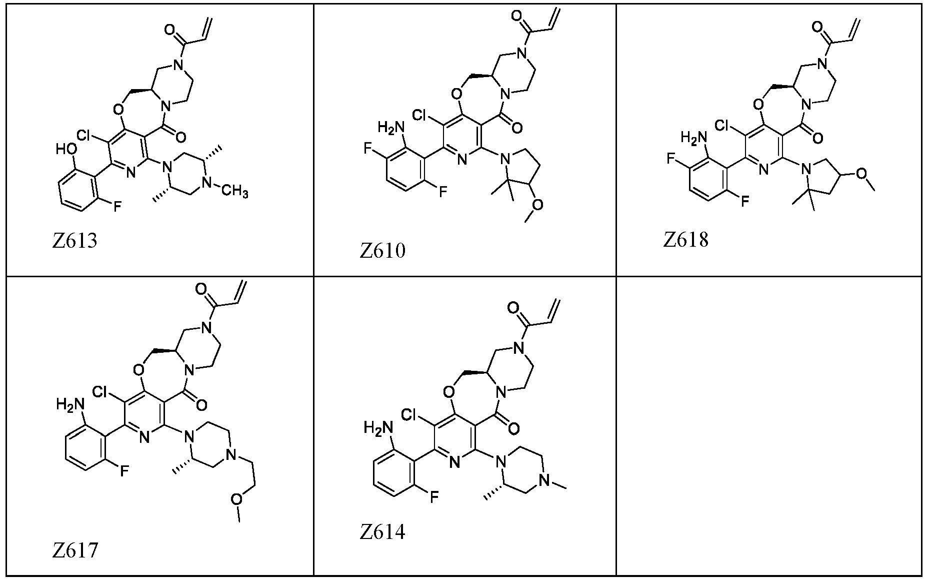

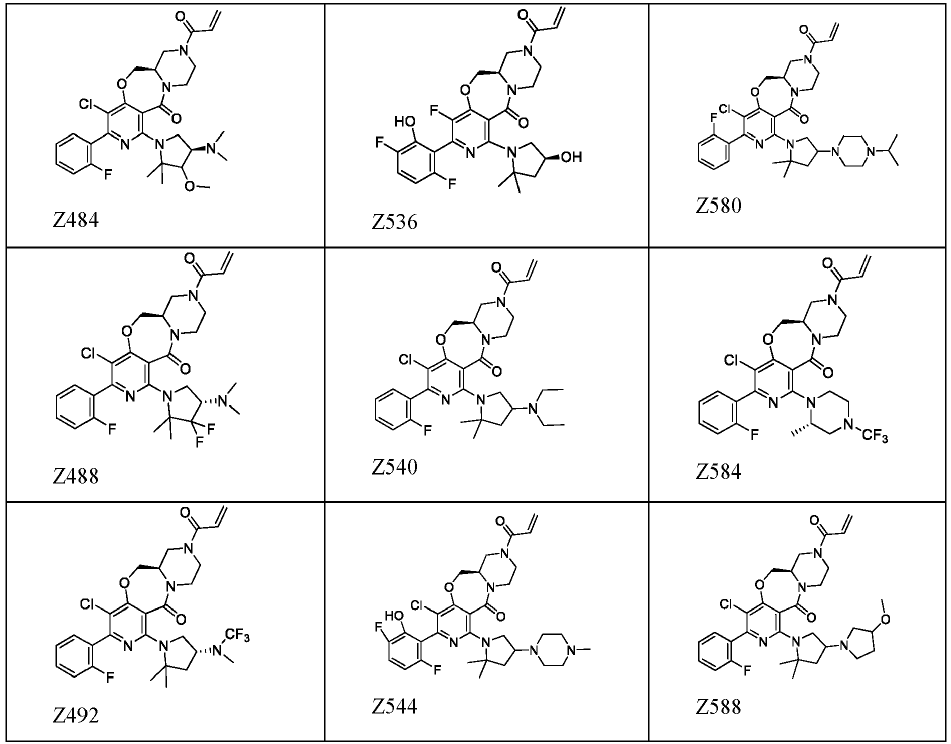

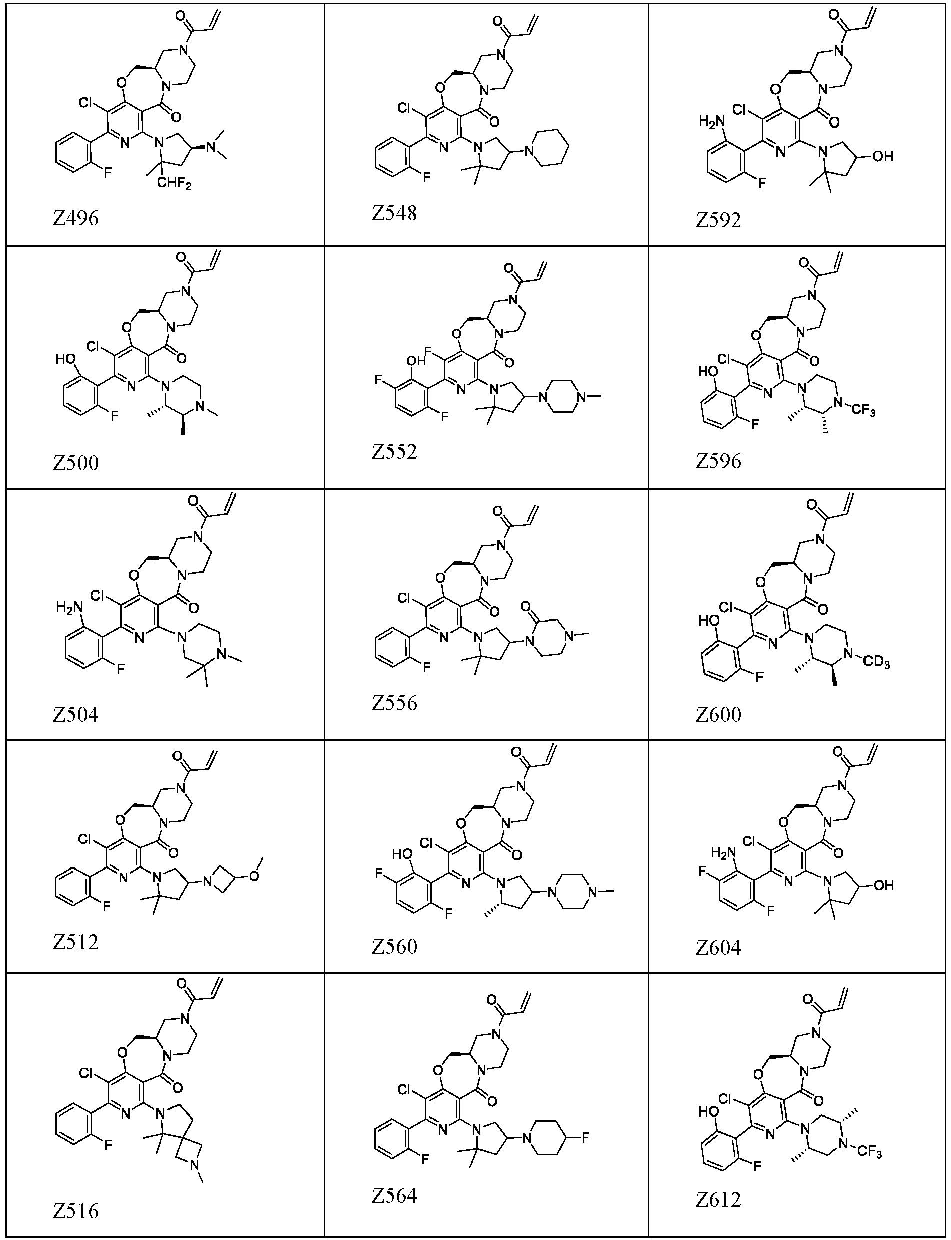

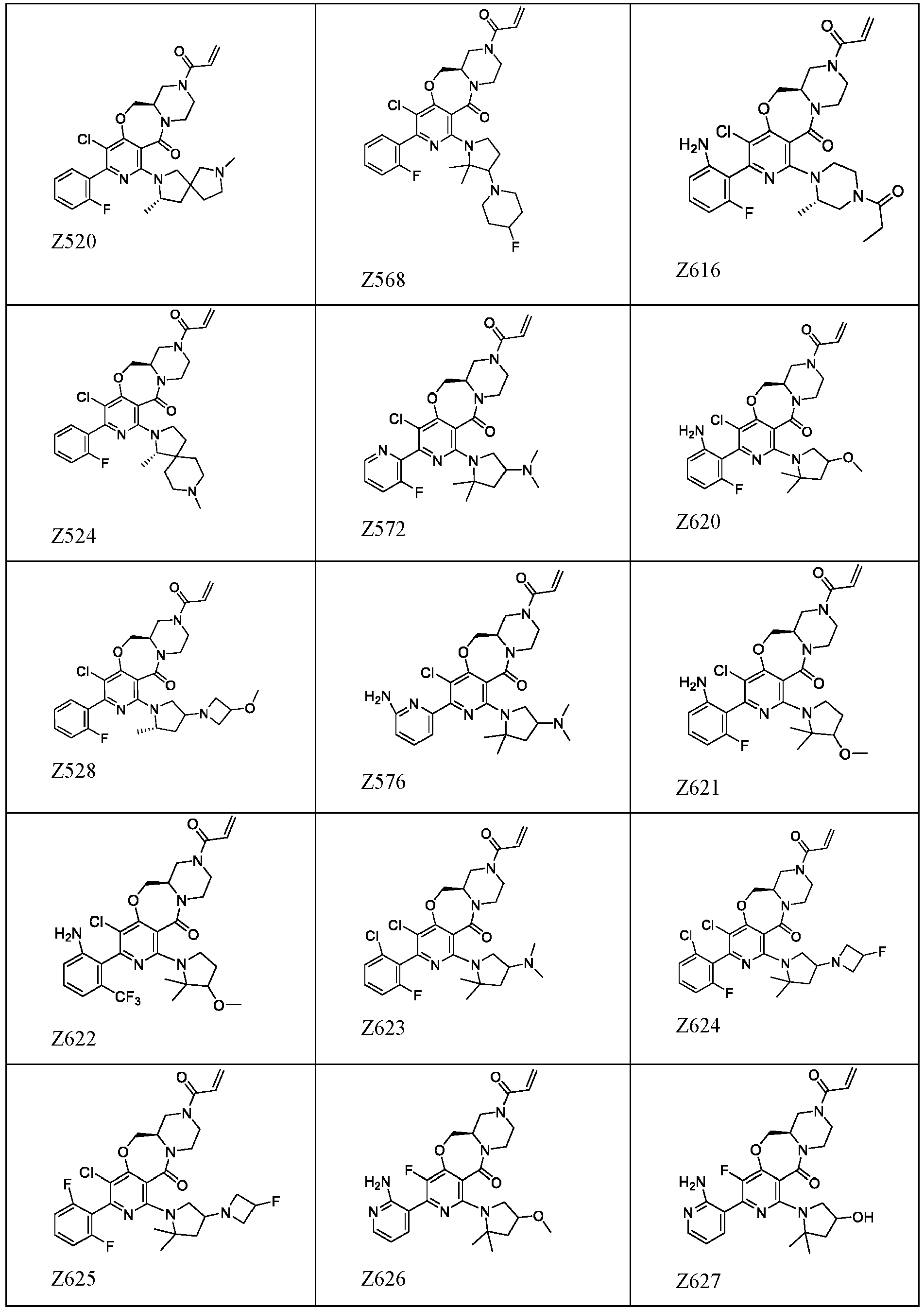

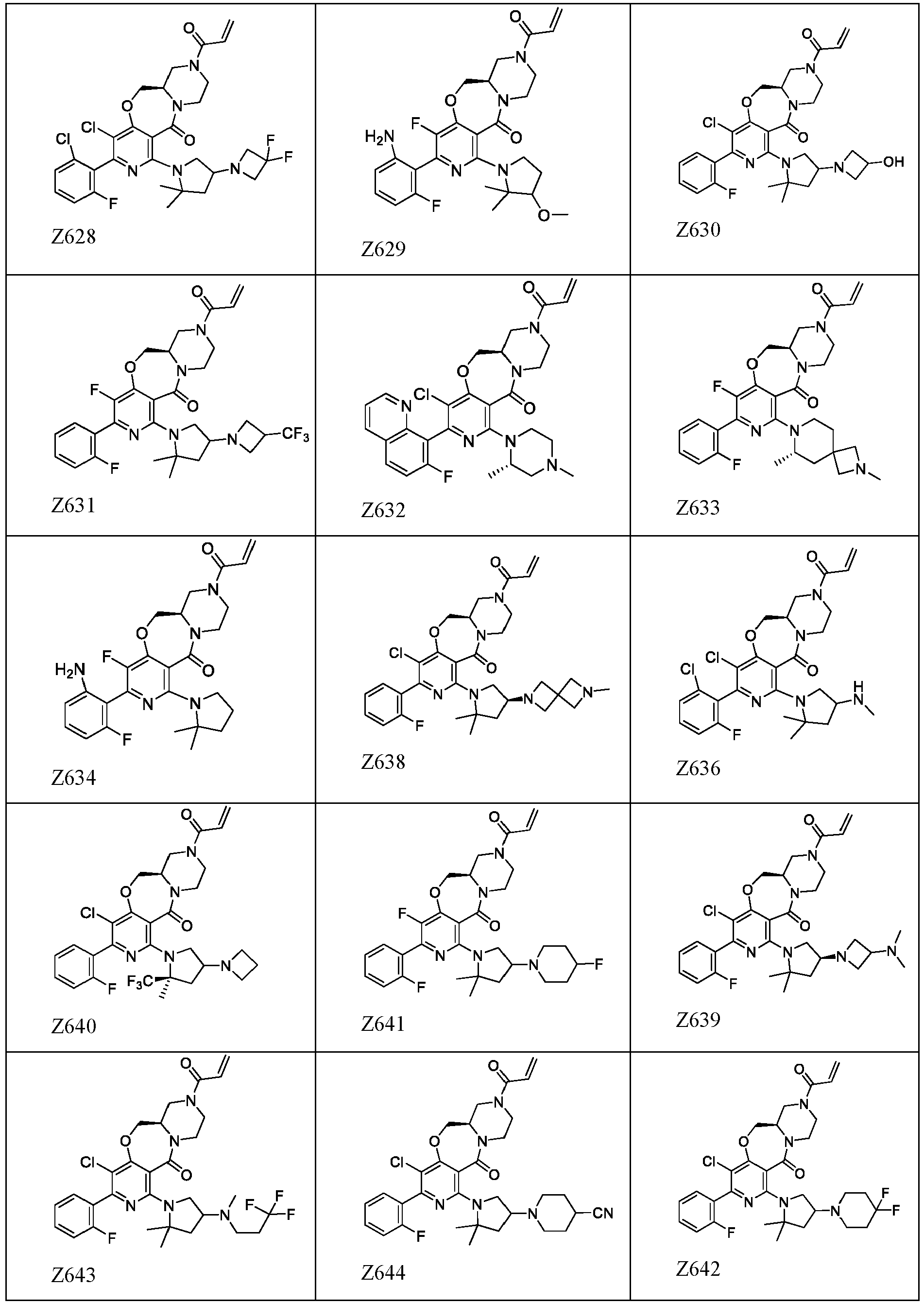

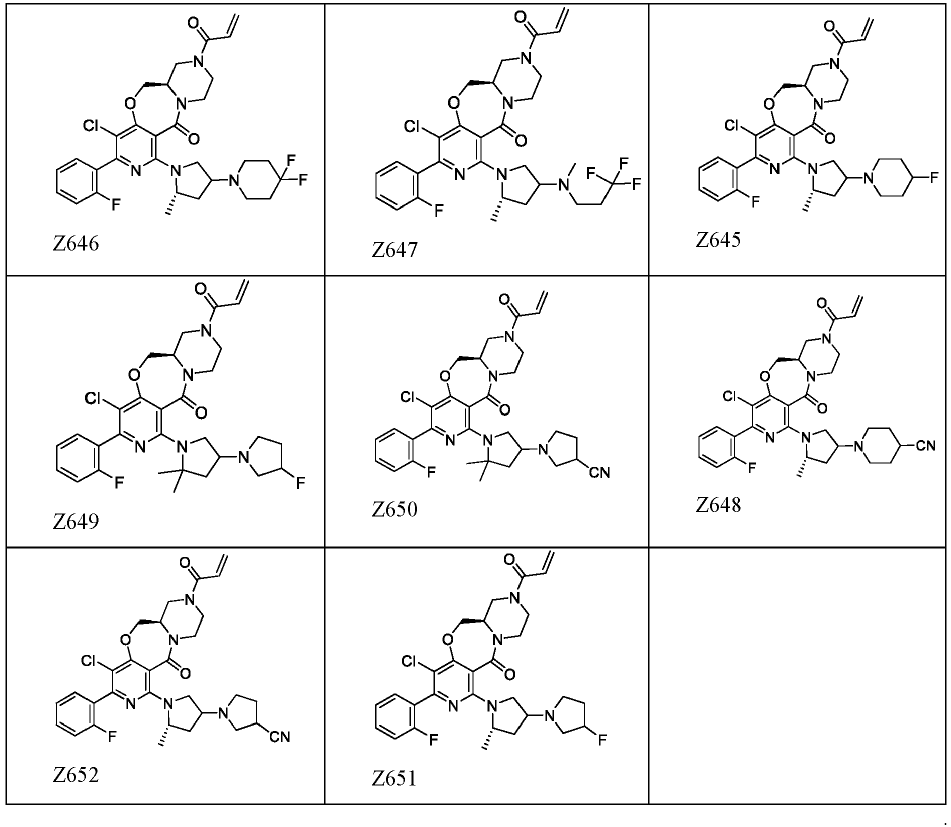

12. A pharmaceutical composition, comprising the compound, the pharmaceutically acceptable salt thereof, the stereoisomer thereof, the solvate thereof or the prodrug thereof as claimed in any one of claims 1-11, and a pharmaceutically acceptable carrier.

13. Use of the compound, the pharmaceutically acceptable salt thereof, the stereoisomer thereof, the solvate thereof or the prodrug thereof as claimed in any one of claims 1-11 or the pharmaceutical composition as claimed in claim 12 in the manufacture of a medicament for the prevention and/or treatment of cancer.

14. Use of the compound, the pharmaceutically acceptable salt thereof, the stereoisomer thereof, the solvate thereof or the prodrug thereof as claimed in any one of claims 1-11 or the pharmaceutical composition as claimed in claim 12 in the manufacture of a KRAS mutation inhibitor; the KRAS mutation is preferably a KRAS G12C mutation.
